(19) Europäisches Patentamt
European Patent Office
Office européen des brevets

(11) EP 1 892 306 A2

(12) **EUROPEAN PATENT APPLICATION**

(43) Date of publication:
**27.02.2008 Bulletin 2008/09**

(51) Int Cl.:
***C12Q 1/68*** *(2006.01)* ***G01N 33/50*** *(2006.01)*

(21) Application number: **07022919.0**

(22) Date of filing: **30.06.2004**

(84) Designated Contracting States:
**AT BE BG CH CY CZ DE DK EE ES FI FR GB GR HU IE IT LI LU MC NL PL PT RO SE SI SK TR**

(30) Priority: **06.10.2003 EP 03022587**

(62) Document number(s) of the earlier application(s) in accordance with Art. 76 EPC:
**04015374.4 / 1 522 594**

(71) Applicant: **Bayer HealthCare AG**
**51368 Leverkusen (DE)**

(72) Inventors:
- **Munnes, Marc, Dr.**
  **40699 Erkrath (DE)**
- **Bojar, Hans, Prof. Dr.**
  **40764 Langenfeld (DE)**

Remarks:
This application was filed on 27-11-2007 as a divisional application to the application mentioned under INID code 62.

(54) **Methods and kits for investigating cancer**

(57) The invention provides novel compositions, methods and uses, for the prediction, diagnosis, prognosis, prevention and treatment of malignant neoplasia and breast cancer. The invention further relates to genes that are differentially expressed in breast tissue of breast cancer patients versus those of normal "healthy" tissue. Differentially expressed genes for the identification of patients which are likely to respond to chemotherapy are also provided.

EP 1 892 306 A2

SEQUENCE LISTING

<110> BayerHealthcare AG

<120> METHODS AND KITS FOR INVESTIGATING CANCER

<130> BHC-0301001-03
<150>
<151>
<160> 571

<170> PatentIn version 3.1

<210> 1
<211> 1978
<212> DNA
<213> Homo sapiens
<400> 1
gggagggtac ttagggccgg ggctggccca ggctacggcg gctgcagggc tccggcaacc 60
gctccggcaa cgccaaccgc tccgctgcgc gcaggctggg ctgcaggctc tcggctgcag 120
cgctgggtgg atctaggatc cggcttccaa catgtggcag ctctgggcct ccctctgctg 180
cctgctggtg ttggccaatg cccggagcag gccctctttc catcccctgt cggatgagct 240
ggtcaactat gtcaacaaac ggaataccac gtggcaggcc gggcacaact tctacaacgt 300
ggacatgagc tacttgaaga ggctatgtgg taccttcctg ggtgggccca agccacccca 360
gagagttatg tttaccgagg acctgaagct gcctgcaagc ttcgatgcac gggaacaatg 420
gccacagtgt cccaccatca aagagatcag agaccagggc tcctgtggct cctgctgggc 480
cttcggggct gtggaagcca tctctgaccg gatctgcatc cacaccaatg cgcacgtcag 540
cgtggaggtg tcggcggagg acctgctcac atgctgtggc agcatgtgtg gggacggctg 600
taatggtggc tatcctgctg aagcttggaa cttctggaca agaaaaggcc tggtttctgg 660
tggcctctat gaatcccatg tagggtgcag accgtactcc atccctccct gtgagcacca 720
cgtcaacggc tcccggcccc catgcacggg ggagggagat acccccaagt gtagcaagat 780
ctgtgagcct ggctacagcc cgacctacaa acaggacaag cactacggat acaattccta 840
cagcgtctcc aatagcgaga aggacatcat ggccgagatc tacaaaaacg gccccgtgga 900
gggagctttc tctgtgtatt cggacttcct gctctacaag tcaggagtgt accaacacgt 960
caccggagag atgatgggtg gccatgccat ccgcatcctg ggctggggag tggagaatgg 1020
cacaccctac tggctggttg ccaactcctg gaacactgac tggggtgaca atggcttctt 1080
taaaatactc agaggacagg atcactgtgg aatcgaatca gaagtggtgg ctggaattcc 1140
acgcaccgat cagtactggg aaaagatcta atctgccgtg ggcctgtcgt gccagtcctg 1200
ggggcgagat cggggtagaa atgcatttta ttctttaagt tcacgtaaga tacaagtttc 1260
agacagggtc tgaaggactg gattggccaa acatcagacc tgtcttccaa ggagaccaag 1320
tcctggctac atcccagcct gtggttacag tgcagacagg ccatgtgagc caccgctgcc 1380
agcacagagc gtccttcccc ctgtagacta gtgccgtagg gagtacctgc tgccccagct 1440
gactgtggcc ccctccgtga tccatccatc tccagggagc aagacagaga cgcaggaatg 1500
gaaagcggag ttcctaacag gatgaaagtt cccccatcag ttcccccagt acctccaagc 1560
aagtagcttt ccacatttgt cacagaaatc agaggagaga tggtgttggg agccctttgg 1620
agaacgccag tctcccaggc cccctgcatc tatcgagttt gcaatgtcac aacctctctg 1680
atcttgtgct cagcatgatt ctttaataga agttttattt tttcgtgcac tctgctaatc 1740
atgtgggtga gccagtggaa cagcgggaga cctgtgctag ttttacagat tgcctcctaa 1800
tgacgcggct caaaaggaaa ccaagtggtc aggagttgtt tctgacccac tgatctctac 1860
taccacaagg aaaatagttt aggagaaacc agcttttact gtttttgaaa aattacagct 1920
tcaccctgtc aagttaacaa ggaatgcctg tgccaataaa agttttctcc aacttgaa 1978

<210> 2
<211> 3285
<212> DNA
<213> Homo sapiens
<400> 2
ctagaattca gcggccgctg aattctagac ccggatgaag agtaacgcca ttaccgcccg 60
agccgccgag agccttagcc gacggaaact ggacactgga ccggcagcgc catgagactc 120
ctcccccgct tgctgctgct tctcttactc gtgttccctg ccactgtctt gttccgaggc 180
ggccccagag gctcgttagc agtggcacaa gatcttacag aggatgaaga aacagtagaa 240
gattccataa ttgaggatga agatgatgaa gccgaggtag aagaagatga acccacagat 300
ttggtagaag ataaagagga agaagatgtg tctggtgaac ctgaagcttc accgagtgca 360
gatacaacta tactgtttgt aaaaggagaa gattttccag caaataacat tgtgaagttc 420
ctggtaggct ttaccaacaa gggtacagaa gattttattg ttgaatcctt agatgcctca 480
ttccgttatc ctcaggacca ccagttttat atccagaatt tcacagctct tcctctgaac 540
actgtagtgc caccccagag acaggcaact tttgagtact ctttcattcc tgcagagccc 600
atgggcggac gaccatttgg tttggtcatc aatctgaact acaaagattt gaacggcaat 660

gtattccaag atgcagtctt caatcaaaca gttacagtta ttgaaagaga ggatgggtta 720
gatggagaaa caatctttat gtatatgttc cttgctggtc ttgggcttct ggttattgtt 780
ggccttcatc aactcctaga atctagaaag cgtaagagac ccatacagaa agtagaaatg 840
ggtacatcaa gtcagaatga tgttgacatg agttggattc ctcaggaaac attgaatcaa 900
atcaataaag cttcaccaag aaggttgccc aggaaacggg cacagaagag atcagtggga 960
tctgatgagt aaatgttcct ttgtgcaaca attcggtctt tacttaacct gccctaatat 1020
ttttcggcct gatgggaatt agtgcagaga agccagtcac catagaaggc aactcctact 1080
tgtgtgtgga ctgagcaatc agagtctgtg gcgataatat tgctgaaaat gcactgcatt 1140
catttttcta aagtaacaaa tttggttttt ttttaaacca ttaaaatcta tgtgtgtgcg 1200
tgtgtatgta tgtgagcagt tggtcttacc agaatcattg ttgaactacc tgaaacaagt 1260
ctttagaata ctaaatataa tgctgttgtc tcttcctttt tgacattttc tgattttttc 1320
ccccaaaact cagttaatat ttacccacta tgattattga tgtcctgcct tgaacagttt 1380
taaagaaaac aatttttgga atagctcaaa tttcaattga tggcacaaat cagcattttg 1440
ttgttgttac tgtattacaa ttagtattct aaaggcagaa gcagaagtag ctgcttttta 1500
gcaatagaat tgtttcagta ttttgctgct gtttaatgcg catcttcaga aaacttccca 1560
gtggcttcaa ggaatttggg gatctctctg gcaacaaatt gtgaaacatg aaatttctgc 1620
tgactttaat atatgaaacc taatcctacc cccttttttta acaaaaagaa actagtacat 1680
ttgtgaaaat tgtgttgtgt tgtccattgt tgctctagtt ctgacccaga ggtagctctg 1740
gagtgatttt agacctactc actcagttgt gtgtaggttt ttttgttttg ttttgagaga 1800
gaatttttct ctccttaata gaagcatcct ttttaaagag aagttgcctt ggtccacaca 1860
ctaagcagaa aaccaagtta tcaggacaga gatatttccc agttactcct aatcaatgaa 1920
gaaagtgagt tggatatttt taaagcagtt aactaatttt ttcttaccta atcttttggg 1980
agttttgctt gttgatataa ccttttttagt taacctgaaa gattccaaaa attgttctta 2040
agtgcttgag actggaacca aaattaaatt gtacttcata aaatcctctt atagagttac 2100
tcttgcccta gattgtaaat taagtttggc attattgtca gactggatgg agggtgaagt 2160
aaaatagtat gaacaattaa gaggctctcc ccctcttgtc tttaagccat attctcctac 2220
atgtatttta taagaaaatg ttaagtcaaa ttttagtggc tctttaattc ctgacctctt 2280
cattctcctt ttcagtataa cctcccctat gctcatgccc acacagacaa aaaaacaaaa 2340
cgaaatacac acagaaaaaa gtctttccaa actgtttaag tatttaaaca tctgagccaa 2400
agcagataga agttattgta taattgttaa tcactttgca aataggggct atcaaattac 2460
ctatattggc attgctggat tataaactct atatctgtaa tataaagtgt ttgagttttt 2520
aattgggctg ttatgatcag taattgattt tgagaaagct ctatgagctc taagtaactg 2580
catggttttt tgtttaatgt aatataggag acccttcaca ttcccaagga atatattcca 2640
aaacattttt gtgaatatct aagtttgtga aactactagg gcatgataca gtaaggtgta 2700
attacagaat ttacgaaatg taaatggcct ctacagagtt ttatggaata cctggtacta 2760
acgtaggcag ctgcaaaacc acactgagtt acagctgtca gccctcctca ttcctaaata 2820
acttgcctta catatcagcc ctcccacttc tgaagttcaa attagtgcct cggaaatgta 2880
gaatttatta tttgtcattt tttttttta gcatagattg agaacagttg aactcttaaa 2940
tcctcagatg ccaggggtct gctctagcat cagtaagtat ttagcagaaa ctaactccgt 3000
aatgaatgga attcaattcc acacatggtt tgttcaagca cacttaataa gtagcctatt 3060
ttttaaatgt ctttttaaaa tgtaaatatt tggatgaagt ttttctttgt tttgatatat 3120
tcatttgcta caccaactat gttttcagaa ttcatctttt gaacaacttg gtttcagaat 3180
atgtaaaatg actttaagga tcttgtgtat caaacctatc cccggatgtg tgagaataat 3240
gtgttcataa agcatggatc tcgtaaaaaa aaaaaaaaaa aaaaa 3285

<210> 3
<211> 1545
<212> DNA
<213> Homo sapiens
<400> 3
gaagacacca ccggaagcaa ggaaggtgct gtgtaatcat taaggagcgg aggcttttgg 60
agctgctaaa atgccggatt acctcggtgc cgatcagcgg aagaccaaag aggatgagaa 120
ggacgacaag cccatccgag ctctggatga gggggatatt gccttgttga aaacttatgg 180
tcagagcact tactctaggc agatcaagca agttgaagat gacattcagc aacttctcaa 240
gaaaattaat gagctcactg gtattaaaga atctgacact ggcctggccc caccagcact 300
ctgggatttg gctgcagata agcagacact ccagagtgaa cagcctttac aggttgccag 360
gtgtacaaag ataatcaatg ctgattcgga ggacccaaaa tacattatca acgtaaagca 420
gtttgccaag tttgtggtgg accttagtga tcaggtggca cctactgaca ttgaagaagg 480
gatgagagtg ggcgtggata gaaataaata tcaaattcac attccattgc ctcctaagat 540
tgacccaaca gttaccatga tgcaggtgga agagaaacct gatgtcacat acagtgatgt 600
tggtggctgt aaggaacaga ttgagaaact gcgagaagta gttgaaaccc cattacttca 660
tccagagagg tttgtgaacc ttggcattga gcctcccaag ggcgtgctgc tctttggtcc 720
acccggtaca ggcaagacac tctgtgcgcg ggcagttgct aatcggactg atgcgtgctt 780
cattcgagtt attggatctg agcttgtaca gaaatacgtc ggtgaggggg ctcgaatggt 840
tcgtgaactc tttgaaatgg ccagaacaaa aaaagcctgc cttatcttct ttgatgaaat 900
tgatgctatt ggaggggctc gttttgatga tggtgctgga ggtgacaatg aagtgcagag 960
aacaatgttg gaactgatca atcagcttga tggttttgat cctcgaggca atattaaagt 1020
gctgatggcc actaacagac ctgatacttt ggatccagca ctgatgaggc cagggagatt 1080
ggatagaaaa attgaattta gcttgcccga tctagagggt cggacccaca tatttaagat 1140

```
tcacgctcgt tcaatgagtg ttgaaagaga tatcagattt gaactgttag cacgactgtg   1200
tccaaatagc actggtgctg agattagaag cgtctgcaca gaggctggta tgtttgccat   1260
cagagcacgg cgaaaaattg ctaccgagaa ggatttcttg gaagctgtaa ataaggtcat   1320
taagtcttat gccaaattca gtgctactcc tcgttacatg acatacaact gaaccctgaa   1380
ggctttcaag tgaaaacttt aaattggaat cctaacctta tatagacttg ttaataacca   1440
attcataaac aaataaatgg cttcaaaatt gtatgctttt ttccatatct cttcttgtaa   1500
tataataaaa ggtgatttct aatgttaaaa aaaaaaaaaa aaaaa                   1545

<210>  4
<211>  1976
<212>  DNA
<213>  Homo sapiens
<400>  4
gccacacggt ctttgagctg agtcgaggtg gaccctttga acgcagtcgc cctacagccg     60
ctgattcccc ccgcatcgcc tcccgtggaa gcccaggccc gcttcgcagc tttctccctt    120
tgtctcataa ccatgtccac caacgagaat gctaatacac cagctgcccg tcttcacaga    180
ttcaagaaca agggaaaaga cagtacagaa atgaggcgtc gcagaataga ggtcaatgtg    240
gagctgagga aagctaagaa ggatgaccag atgctgaaga ggagaaatgt aagctcattt    300
cctgatgatg ctacttctcc gctgcaggaa aaccgcaaca accagggcac tgtaaattgg    360
tctgttgatg acattgtcaa aggcataaat agcagcaatg tggaaaatca gctccaagct    420
actcaagctg ccaggaaact actttccaga gaaaaacagc cccccataga caacataatc    480
cgggctggtt tgattccgaa atttgtgtcc ttcttgggca gaactgattg tagtcccatt    540
cagtttgaat ctgcttgggc actcactaac attgcttctg ggacatcaga acaaaccaag    600
gctgtggtag atggaggtgc catcccagca ttcatttctc tgttggcatc tccccatgct    660
cacatcagtg aacaagctgt ctgggctcta ggaaacattg caggtgatgg ctcagtgttc    720
cgagacttgg ttattaagta cggtgcagtt gacccactgt tggctctcct tgcagttcct    780
gatatgtcat ctttagcatg tggctactta cgtaatctta cctggacact ttctaatctt    840
tgccgcaaca agaatcctgc acccccgata gatgctgttg agcagattct tcctacctta    900
gttcggctcc tgcatcatga tgatccagaa gtgttagcag atacctgctg ggctatttcc    960
taccttactg atggtccaaa tgaacgaatt ggcatggtgg tgaaaacagg agttgtgccc   1020
caacttgtga agcttctagg agcttctgaa ttgccaattg tgactcctgc cctaagagcc   1080
atagggaata ttgtcactgg tacagatgaa cagactcagg ttgtgattga tgcaggagca   1140
ctcgccgtct ttcccagcct gctcaccaac cccaaaacta acattcagaa ggaagctacg   1200
tggacaatgt caaacatcac agccggccgc caggaccaga tacagcaagt tgtgaatcat   1260
ggattagtcc cattccttgt cagtgttctc tctaaggcag attttaagac acaaaaggaa   1320
gctgtgtggg ccgtgaccaa ctataccagt ggtggaacag ttgaacagat tgtgtacctt   1380
gttcactgtg gcataataga accgttgatg aacctcttaa ctgcaaaaga taccaagatt   1440
attctggtta tcctggatgc catttcaaat atctttcagg ctgctgagaa actaggtgaa   1500
actgagaaac ttagtataat gattgaagaa tgtggaggct tagacaaaat tgaagctcta   1560
caaaaccatg aaaatgagtc tgtgtataag gcttcgttaa gcttaattga gaagtatttc   1620
tctgtagagg aagaggaaga tcaaaacgtt gtaccagaaa ctacctctga aggctacact   1680
ttccaagttc aggatggggc tcctgggacc tttaactttt agatcatgta gctgagacat   1740
aaatttgttg tgtactacgt ttggtatttt gtcttattgt ttctctacta agaactcttt   1800
cttaaatgtg gtttgttact gtagcacttt ttacactgaa actatacttg aacagttcca   1860
actgtacata catactgtat gaagcttgtc ctctgactag gtttctaatt tctatgtgga   1920
atttcctatc ttgcagcatc ctgtaaataa acattcaagt ccacccttaa aaaaaa       1976

<210>  5
<211>  3579
<212>  DNA
<213>  Homo sapiens
<400>  5
tcaggctcgc tgtcgcgcca ttttgccggg gtttgaatgt gaggcggagc ggcggcagga     60
gcgggtagtg ccagctacgg tccgcggctg gggttccctc ctccgtttct gtatccccac    120
gagatcctat agcaatggaa ctcagcgatg caaatctgca aacactaaca gaatatttaa    180
agaaaacact tgatcctgat cctgccatcc gacgtccagc tgagaaattt cttgaatctg    240
ttgaaggaaa tcagaattat ccactgttgc ttttgacatt actggagaag tcccaggata    300
atgttatcaa agtatgtgct tcagtaacat tcaaaaacta tattaaaagg aactggagaa    360
ttgttgaaga tgaaccaaac aaaatttgtg aagccgatcg agtggccatt aaagccaaca    420
tagtgcactt gatgcttagc agcccagagc aaattcagaa gcagttaagt gatgcaatta    480
gcattattgg cagagaagat tttccacaga aatggcctga cttgctgaca gaaatggtga    540
atcgctttca gagtggagat ttccatgtta ttaatggagt cctccgtaca gcacattcat    600
tatttaaaag ataccgtcat gaatttaagt caaacgagtt atggactgaa attaagcttg    660
ttctggatgc ctttgctttg cctttgacta atctttttaa ggccactatt gaactctgca    720
gtacccatgc aaatgatgcc tctgccctga ggattctgtt ttcttccctg atcctgatct    780
caaaattgtt ctatagttta aactttcagg atctccctga attttttgaa gataatatgg    840
aaacttggat gaataatttt catactctct taacattgga taataagctt ttacaaactg    900
atgatgaaga ggaagccggc ttattggagc tcttaaaatc ccagatttgt gataatgccg    960
cactctatgc acaaaagtac gatgaagaat tccagcgata cctgcctcgt tttgttacag   1020
```

ccatctggaa tttactagtt acaacgggtc aagaggttaa atatgatttg ttggtaagta 1080
atgcaattca atttctggct tcagtttgtg agagacctca ttataagaat ctatttgagg 1140
accagaacac gctgacaagt atctgtgaaa aggttattgt gcctaacatg gaatttagag 1200
ctgctgatga agaagcattt gaagataatt ctgaggagta cataaggaga gatttggaag 1260
gatctgatat tgatactaga cgcagggctg cttgtgatct ggtacgagga ttatgcaagt 1320
tttttgaggg acctgtgaca ggaatcttct ctggttatgt taattccatg ctgcaggaat 1380
acgcaaaaaa tccatctgtc aactggaaac acaaagatgc agccatctac ctagtgacat 1440
ctttggcatc aaaagcccaa acacagaagc atggaattac acaagcaaat gaacttgtaa 1500
acctaactga gttctttgtg aatcacatcc tccctgattt aaaatcagct aatgtgaatg 1560
aatttcctgt ccttaaagct gacggtatca aatatattat gatttttaga aatcaagtgc 1620
caaaagaaca tcttttagtc tcgattcctc tcttgattaa tcatcttcaa gctgaaagta 1680
ttgttgttca tacttacgca gctcatgctc ttgaacggct ctttactatg cgagggccta 1740
acaatgccac tctctttaca gctgcagaaa tcgcaccgtt tgttgagatt ctgctaacaa 1800
acctttcaa agctctcaca cttcctggct cttcagaaaa tgaatatatt atgaaagcta 1860
tcatgagaag tttttctctc ctacaagaag ccataatccc ctacatccct actctcatca 1920
ctcagcttac acagaagcta ttagctgtta gtaagaaccc aagcaaacct cactttaatc 1980
actacatgtt tgaagcaata tgtttatcca taagaataac ttgcaaagct aaccctgctg 2040
ctgttgtaaa ttttgaggag gctttgtttt tggtgtttac tgaaatctta caaaatgatg 2100
tgcaagaatt tattccatac gtctttcaag tgatgtcttt gcttctggaa acacacaaaa 2160
atgacatccc gtcttcctat atggccttat ttcctcatct ccttcagcca gtgctttggg 2220
aaagaacagg aaatattcct gctctagtga ggcttcttca agcattctta gaacgcggtt 2280
caaacacaat agcaagtgct gcagctgaca aaattcctgg gttactaggt gtctttcaga 2340
agctgattgc atccaaagca aatgaccacc aaggttttta tcttctaaac agtataatag 2400
agcacatgcc tcctgaatca gttgaccaat ataggaaaca aatcttcatt ctgctattcc 2460
agagacttca gaattccaaa acaaccaagt ttatcaagag tttttttagtc tttattaatt 2520
tgtattgcat aaaatatggg gcactagcac tacaagaaat atttgatggt atacaaccaa 2580
aaatgtttgg aatggttttg gaaaaaatta ttattcctga aattcagaag gtatctggaa 2640
atgtagagaa aaagatctgt gcggttggca taaccaaatt actaacagaa tgtcccccaa 2700
tgatggacac tgagtatacc aaactgtgga ctccattatt acagtctttg attggtcttt 2760
ttgagttacc cgaagatgat accattcctg atgaggaaca ttttattgac atagaagata 2820
caccaggata tcagactgcc ttctcacagt tggcatttgc tgggaaaaaa gagcatgatc 2880
ctgtaggtca aatggtgaat aaccccaaaa ttcacctggc acagtcactt cacaagttgt 2940
ctaccgcctg tccaggaagg gttccatcaa tggtgagcac cagcctgaat gcagaagcgc 3000
tccagtatct ccaagggtac cttcaggcag ccagtgtgac actgctttaa actgcatttt 3060
tctaatgggc taaacccaga tggtttccta ggaaatcaca ggcttctgag cacagctgca 3120
ttaaaacaaa ggaagttctc cttttgaact tgtcacgaat tccatcttgt aaaggatatt 3180
aaatgttgct ttaacctgaa ccttgagcaa attagttggt ttgtgtgatc atacagttat 3240
gtgggtggct tctagtttgc aacttcaagg gacaagtatt aatagttcag tgtatggcgt 3300
tggtttgtgt tgagcgtttg cacggtttgg ataatcttaa attttgacgg acactgtgga 3360
gactttctgt tactaaatcc ttttgttttg aagctgttgc tatttgtatt tctcttgtcc 3420
tttatatttt ttgtctgttt atttacgctt ttattggaaa tgtgaataag taaagaatta 3480
cttgtgttac ttgccaagca gtgcacattt catagtttca aatctgtaat cagcaataaa 3540
aatcctaaaa tatgtaccta aaaaaaaaaa aaaaaaaa 3579

<210> 6
<211> 1396
<212> DNA
<213> Homo sapiens
<400> 6

gcgtaattaa aaggcggcgg aagaaggtgg gagggtcatg acgcagcgag tttcagtcgt 60
gacttttctg ggggcatcgc ggcgtcccct tttttttgcc tttaaagtaa aacgtcgccc 120
cgacgcaccc cccgcgtatt tcggggggcg gaggcggcgg gccacggcgc gaagaggggc 180
ggtgctgacg ccggccggtc acgtgggcgt gttgtggggg ggagggggcgc cgccgcgcgg 240
tcggttccgg gcggttggga gcgcgcgagc tagcgagcga gaggcagccg cgcccgccgc 300
cgcccctgct ctgtatgccg ctctctcccg gcgcggccgc cgccgatcac agcagcagga 360
gccaccgccg ccgcggttga tgtggttggg ccggggctga ggaggccgcc aagatgccgc 420
agtccaagtc ccggaagatc gcgatcctgg gctaccggtc tgtggggaaa tcctcattga 480
cgattcaatt tgttgaaggc caatttgtgg actcctacga tccaaccata gaaaacactt 540
ttacaaagtt gatcacagta aatggacaag aatatcatct tcaacttgta gacacagccg 600
ggcaagatga atattctatc tttcctcaga catactccat agatattaat ggctatattc 660
ttgtgtattc tgttacatca atcaaaagtt ttgaagtgat taaagttatc catggcaaat 720
tgttggatat ggtggggaaa gtacaaatac ctattatgtt ggttgggaat aagaaagacc 780
tgcatatgga aagggtgatc agttatgaag aagggaaagc tttggcagaa tcttggaatg 840
cagcttttttt ggaatcttct gctaaagaaa atcagactgc tgtggatgtt tttcgaagga 900
taattttgga ggcagaaaaa atggacgggg cagcttcaca aggcaagtct tcatgctcgg 960
tgatgtgatt ctgctgcaaa gcctgaggac actgggaata tattctacct gaagaagcaa 1020
actgcccgtt ctccttgaag ataaactatg cttcttttttt cttctgttaa cctgaaagat 1080
atcatttggg tcagagctcc cctcccttca gattatgtta actctgagtc tgtccaaatg 1140
agttcacttc cattttcaaa ttttaagcaa tcatattttc aatttatata ttgtatttct 1200

taatattatg accaagaatt ttatcggcat taatttttca gtgtagtttg ttgtttaaaa 1260
taatgtaatc atcaaaatga tgcatattgt tacactacta ttaactaggc ttcagtatat 1320
cagtgtttat ttcattgtgt taaatgtata cttgtaaata aaatagctgc aaacctcaaa 1380
aaaaaaaaaa aaaaaa 1396

<210> 7
<211> 2465
<212> DNA
<213> Homo sapiens
<400> 7
gagcagcgcg gcctgacggg accaaggcgg cgggagtctg cggtcgttcc ctcggctgtg 60
gaccgggcgg cacgcacgcg gtgcagggta acatggcgga tgcggaagta attattttgc 120
caaagaaaca taagaagaaa aaggagcgga agtcattgcc agaagaagat gtagccgaaa 180
tacaacacgc tgaagaattt cttatcaaac ctgaatccaa agttgctaag ttggacacgt 240
ctcagtggcc ccttttgcta aagaattttg ataagctgaa tgtaaggaca acacactata 300
cacctcttgc atgtggttca aatcctctga agagagagat tggggactat atcaggacag 360
gtttcattaa tcttgacaag ccctctaacc cctcttccca tgaggtggta gcctggattc 420
gacggatact tcgggtggag aagacagggc acagtggtac tctggatccc aaggtgactg 480
gttgtttaat cgtgtgcata gaacgagcca ctcgcttggt gaagtcacaa cagagtgcag 540
gcaaagagta tgtggggatt gtccggctgc acaatgctat tgaagggggg acccagcttt 600
ctagggccct agaaactctg acaggtgcct tattccagcg acccccactt attgctgcag 660
taaagaggca gctccgagtg aggaccatct acgagagcaa aatgattgaa tacgatcctg 720
aaagaagatt aggaatcttt tgggtgagtt gtgaggctgg cacctacatt cggacattat 780
gtgtgcacct tggtttgtta ttgggagttg gtggtcagat gcaggagctt cggagggttc 840
gttctggagt catgagtgaa aaggaccaca tggtgacaat gcatgatgtg cttgatgctc 900
agtggctgta tgataaccac aaggatgaga gttacctgcg gcgagttgtt taccctttgg 960
aaaagctgtt gacatctcat aaacggctgg ttatgaaaga cagtgcagta aatgccatct 1020
gctatggggc caagattatg cttccaggtg ttcttcgata tgaggacggc attgaggtca 1080
atcaggagat tgtggttatc accaccaaag gagaagcaat ctgcatggct attgcattaa 1140
tgaccacagc ggtcatctct acctgcgacc atggtatagt agccaagatc aagagagtga 1200
tcatggagag agacacttac cctcggaagt ggggtttagg tccaaaggca agtcagaaga 1260
agctgatgat caagcagggc cttctggaca agcatgggaa gcccacagac agcacacctg 1320
ccacctggaa gcaggagtat gttgactaca gtgagtctgc caaaaaagag gtggttgctg 1380
aagtggtaaa agccccgcag gtagttgccg aagcagcaaa aactgcgaag cggaagcgag 1440
agagtgagag tgaaagtgac gagactcctc cagcagctcc tcagttgatc aagaaggaaa 1500
agaagaagag taagaaggac aagaaggcca aagctggtct ggagagcggg gccgagcctg 1560
gagatgggga cagtgatacc accaagaaga agaagaagaa gaagaaagca aaagaggtag 1620
aattggtttc tgagtagtga aggccacttg aagctggagg agaaactaaa gccttattga 1680
gaaaacatgt tatagatcct tttgttgctg agagagtgga acataggtcc tagacagggt 1740
gaagagttct ggcacatttt agctgctact ttgagacctc ggtgatgtta cctggtgtgg 1800
tcatcccatc ttgtcctgtt ttaaggatat gggtggtgaa agatgaaaga ggcagagttt 1860
atcccaatga cttctctgtt tgagttggga agcctcacct tcagacccag taactgtccg 1920
cagctgtctg ctagtggttg tcttaacatc gtagtcctag tttgcatttt ttaaatcccc 1980
tctgtttaaa aggtttgtaa aacaaaaaca aaaaactaag tctgctcagt gaaatgctgt 2040
agaaccctaa ataagtggta gaagagtgtc actgaatttt gtctctgaat tcagtataac 2100
tgagttttgt ccatgctggt gtctgggtta taggcctgat gggcctggta gttttccatc 2160
ttgttctggc ctagaggtca gtcctttgca cttcctcaaa gcttgtgtac agtgctcacc 2220
taaatccatc tgactacttg ttcctgtgcc ctcttgtttt aggcctcgtt tacttttaaa 2280
aaatgaaatt gttcattgct gggagaagaa tgttgtaatt tttacttatt aaagtcaact 2340
tgttaagttt tttatgtatt cctgttgggt tttcttgttg atctcatgct agcagagcaa 2400
aaattgtaaa atattttgat taaaaatcta gggaccttta tgtcctattt ggaattcgat 2460
atcaa 2465

<210> 8
<211> 2160
<212> DNA
<213> Homo sapiens
<400> 8
agccccctgc ccctcgccgc cccccgccgc ctgcctgggc cgggccgagg atgcggcgca 60
gcgcctcggc ggccaggctt gctcccctcc ggcacgcctg ctaacttccc ccgctacgtc 120
cccgttcgcc cgccgggccg ccccgtctcc ccgcggcctc cgggtccggg tcctccagga 180
cggccaggcc gtgccgccgt gtgccctccg ccgctcgccc gcgcgccgcg cgctccccgc 240
ctgcgcccag cgccccgcgc ccgcgcccca gtcctcgggc ggtccatgct gcccctctgc 300
ctcgtggccg ccctgctgct ggccgccggg cccgggccga gcctgggcga cgaagccatc 360
cactgcccgc cctgctccga ggagaagctg gcgcgctgcc gcccccccgt gggctgcgag 420
gagctggtgc gagaggcggg ctgcggctgt tgcgccactt gcgccctggg cttggggatg 480
ccctgcgggg tgtacacccc ccgttgcggc tcgggcctgc gctgctaccc gccccgaggg 540
gtggagaagc ccctgcacac actgatgcac gggcaaggcg tgtgcatgga gctggcggag 600
atcgaggcca tccaggaaag cctgcagccc tctgacaagg acgagggtga ccaccccaac 660

```
aacagcttca gcccctgtag cgcccatgac cgcaggtgcc tgcagaagca cttcgccaaa    720
attcgagacc ggagcaccag tgggggcaag atgaaggtca atggggcgcc ccgggaggat    780
gcccggcctg tgccccaggg ctcctgccag agcgagctgc accgggcgct ggagcggctg    840
gccgcttcac agagccgcac ccacgaggac ctctacttca tccccatccc caactgcgac    900
cgcaacggca acttccaccc caagcagtgt cacccagctc tggatgggca gcgtggcaag    960
tgctggtgtg tggaccggaa gacggggtg aagcttccgg ggggcctgga gccaaagggg    1020
gagctggact gccaccagct ggctgacagc tttcgagagt gaggcctgcc agcaggccag    1080
ggactcagcg tcccctgcta ctcctgtgct ctggaggctg cagagctgac ccagagtgga    1140
gtctgagtct gagtcctgtc tctgcctgcg gcccagaagt ttccctcaaa tgcgcgtgtg    1200
cacgtgtgcg tgtgcgtgcg tgtgtgtgtg tttgtgagca tgggtgtgcc cttggggtaa    1260
gccagagcct ggggtgttct ctttggtgtt acacagccca agaggactga gactggcact    1320
tagcccaaga ggtctgagcc ctggtgtgtt tccagatcga tcctggattc actcactcac    1380
tcattccttc actcatccag ccacctaaaa acatttactg accatgtact acgtgccagc    1440
tctagttttc agccttggga ggttttattc tgacttcctc tgattttggc atgtggagac    1500
actcctataa ggagagttca agcctgtggg agtagaaaaa tctcattccc agagtcagag    1560
gagaagagac atgtaccttg accatcgtcc ttcctctcaa gctagcccag agggtgggag    1620
cctaaggaag cgtggggtag cagatggagt aatggtcacg aggtccagac ccactcccaa    1680
agctcagact tgccaggctc cctttctctt cttccccagg tccttccttt aggtctggtt    1740
gttgcaccat ctgcttggtt ggctggcagc tgagagccct gctgtgggag agcgaagggg    1800
gtcaaaggaa gacttgaagc acagagggct agggaggtgg ggtacatttc tctgagcagt    1860
cagggtggga agaaagaatg caagagtgga ctgaatgtgc ctaatggaga agacccacgt    1920
gctaggggat gaggggcttc ctgggtcctg ttcccctacc ccatttgtgg tcacagccat    1980
gaagtcaccg ggatgaacct atccttccag tggctcgctc cctgtagctc tgcctccctc    2040
tccatatctc cttcccctac acctccctcc ccacacctcc ctactcccct gggcatcttc    2100
tggcttgact ggatggaagg agacttagga acctaccagt tggccatgat gtcttttctt    2160
```

<210> 9
<211> 9725
<212> DNA
<213> Homo sapiens
<400> 9

```
cgcgcgggct acctcagttc tcgggcgtac ggcgcggcct gtcctactgc cgccggcgcc     60
gcggccgtca tggggttcct gaaactgatt gagattgaga actttaagtc gtacaagggt    120
cgacagatta tcggaccatt tcagaggttc accgccatca ttggacccaa tggctctggt    180
aagtcaaatc tcatggatgc catcagcttt gtgctaggtg aaaaaaccag caacctgcgg    240
gtaaagaccc tgcgggacct gatccatgga gctcctgtgg gcaagccagc tgccaaccgg    300
gcctttgtca gcatggtcta ctctgaggag ggtgctgagg accgtacctt tgcccgtgtc    360
attgtaggag gttcttctga gtacaagatc aacaacaaag tggtccaact acatgagtac    420
agtgaggaat tagagaagtt gggcattctc atcaaagctc gtaacttcct cgttttccag    480
ggtgctgtgg aatctattgc catgaagaac cccaaagaga ggacagctct atttgaagag    540
attagtcgtt ctggggagct ggcgcaggag tatgacaagc gaaagaagga aatggtgaag    600
gctgaagagg acacacagtt taattaccat cgcaagaaaa atattgcggc tgaacgcaag    660
gaagcaaagc aggagaaaga agaggctgac cggtaccagc gcctgaagga tgaggtagta    720
cgggctcagg tacagctgca gctctttaag ctttaccata atgaagtgga aattgagaag    780
ctcaacaagg aactggcctc aaagaacaag gagatcgaga aggacaagaa gcgtatggac    840
aaggtggagg atgaactgaa ggagaagaag aaggagctgg gcaaaatgat gcgggagcag    900
cagcagattg agaaggagat caaggagaag gactcagaat tgaaccagaa gcggcctcag    960
tacatcaaag ccaaggagaa cacctcccac aaaatcaaga agctggaagc agccaagaag   1020
tctctgcaga atgctcagaa gcactacaag aagcgtaaag gtgacatgga tgagctggag   1080
aaggagatgc tgtcagtgga gaaggctcgg caggagtttg aagaacggat ggaagaagag   1140
agtcagagtc agggcagaga tttgacgttg gaggagaatc aggtgaagaa ataccaccgg   1200
ttgaaagaag aagccagcaa gagagcagct accctggccc aggagctgga gaaattcaat   1260
cgagaccaga aagctgacca ggaccgtctg gatctggaag aacggaagaa agtagagaca   1320
gaggccaaga tcaagcaaaa gctgcgggaa attgaagaga atcagaagcg gattgagaaa   1380
ctggaggaat acatcaccac tagcaagcag tccctagaag agcagaagaa gctagagggg   1440
gagctgacag aggaggtgga gatggccaag cggcgtattg atgaaatcaa taaggagctg   1500
aaccaggtga tggagcagct aggggatgcc cgcatcgacc gccaggagag cagccgccag   1560
cagcgaaagg cagagataat ggaaagcatc aagcgccttt accctggctc tgtgtacggc   1620
cgcctcattg acctatgcca gcccacacaa aagaagtatc agattgctgt aaccaaggtt   1680
ttgggcaaga acatggatgc cattattgtg gactcggaga agacaggccg ggactgtatt   1740
cagtatatca aggagcagcg tggggagcct gagaccttct tgcctcttga ctacctggag   1800
gtgaagccta cagatgagaa actccgggag ctgaaggggg ccaagctagt gattgatgtg   1860
attcgctatg agccacctca tatcaaaaag gccctgcagt atgcttgtgg caatgccctt   1920
gtctgtgaca acgtggaaga tgcccgccgc attgcctttg gaggccacca gcgccacaag   1980
acagtggcac tggatggaac cctattccag aagtcaggag tgatctctgg tggggccagt   2040
gacctgaagg ccaaggcacg gcgctgggat gagaaagcag tagacaagtt gaaagagaag   2100
aaggagcgct tgacagagga gctgaaagag cagatgaagg caaaacggaa agaggcagag   2160
ctgcgtcagg tgcagtctca ggcccatgga ctgcagatgc ggctcaagta ctcccagagt   2220
gacctagaac agaccaagac acgacatcta gccctgaatc tgcaggaaaa atccaagctg   2280
```

gagagtgagc tagccaactt tgggcctcgc attaatgata tcaagaggat cattcagagc 2340
cgagagaggg aaatgaaaga cttgaaggag aagatgaacc aggtagagga tgaggtgttt 2400
gaagagtttt gtcgggagat tggtgtgcgc aacatccggg agtttgagga agaaaaggtg 2460
aaacggcaga atgaaatcgc caagaagcgt ttggagtttg agaatcagaa gactcgcttg 2520
ggcattcagt tggattttga aaagaaccaa ctgaaggagg accaagataa agtacacatg 2580
tgggagcaga cagtgaaaaa agatgaaaat gagatagaaa agctcaaaaa ggaggaacaa 2640
agacacatga agatcataga tgagaccatg gctcagctac aagacctgaa gaatcagcat 2700
ctggccaaga agtcggaagt gaatgacaag aatcatgaga tggaggagat tcgtaagaaa 2760
ctcgggggcg ccaacaagga aatgacccat ttacagaagg aggtgacagc cattgagacc 2820
aagcttgaac agaagcgcag tgaccgtcac aacttgctac aggcctgtaa gatgcaggac 2880
attaagttgc cactgtcaaa aggcaccatg gatgatatta gtcaggaaga gggtagctcc 2940
caggggggagg actcagtgag tggttcacag agaatttcca gtatctatgc acgagaggcc 3000
ctcattgaga ttgactacgg tgatctgtgt gaggatctga aggatgccca ggctgaggaa 3060
gagatcaagc aagagatgaa cacactgcag cagaagctga atgagcagca gagtgtgctt 3120
cagcgtattg ccgcccccaa catgaaggcc atggaaaagc tggaaagtgt ccgagacaag 3180
ttccaggaga cctcagatga gtttgaagca gcccgaaagc gagcaaagaa ggccaagcag 3240
gcattcgaac agatcaagaa ggagcgcttt gaccgcttca atgcttgttt tgaatctgtg 3300
gctaccaaca ttgatgagat ctataaggcc ctgtcccgca atagcagtgc ccaggcattc 3360
ctgggccctg agaaccctga agagccctac ttggatggca tcaactacaa ctgtgtggct 3420
cctgggaaac gcttccggcc tatggacaac ttgtcaggcg gggagaagac agtggcagct 3480
ctggccctgc tctttgccat ccacagctac aagccagccc ccttcttcgt cctggatgag 3540
attgatgctg ccttggataa caccaacatt ggcaaggtgg caaattacat caaggagcag 3600
tcgacttgca acttccaggc catcgtcatc tctctcaagg aggagttcta caccaaggcc 3660
gagagcctca ttggagtcta tcctgagcaa ggggactgtg tgatcagcaa agtcctgacc 3720
ttcgacctca ccaagtaccc agatgccaac cccaacccca atgagcagta gcagtatttt 3780
tgccctcccg ccctgtctgg atccctaagc tgtccctctc ccaatctctg gatatttgac 3840
tcccaacctt ccccctacct cctggcccctt tttggtgtag tcatgggatt taggcactgc 3900
taatcaagca tgaagaggaa cagaggtgat gttaggtctg gagcaaaaat tcctgaacga 3960
cagggagtat tctggcctct gaaaggaggt gctgagctga acagggccat ctgttcatca 4020
cacacacccc cttcctcccc ctcatcaccc ataatcgtgg gccccttggg cctcttgccc 4080
actgtgtgtg tgggtatgta tgtgtgtatg tatgtatccg catgtgtgca tgtgagtatg 4140
tttgcaaaat aataaaggat attggagacc tgttttagaa ggagcctagg ctgaatttga 4200
ttccaagaga gcttaggatg acagcacccc tgagctgggc aaaggtactc aggacctcat 4260
aggagtctta ggcagttacc tgaaactgcc ttcattcact catttgtgta ttcattcatt 4320
tatgtattca tcagacacat accgaacacc ctctatttgt caggctctgt gcttggaata 4380
cagagttgaa tcagacatga tctctaccct cctagtaagg agatacagtg ggttcatgaa 4440
tgactatagt tagctgaatg tcatatgtac tttgaatttg agaagtgggt gatcccctct 4500
aggcttcctg gaggtcacat ttaagctaga ccttgacaaa ttggtaggat ttggtcaggc 4560
actaggagtg gagcatgagc tctggggaca gacagttatg ggttctggtc ccacttttta 4620
tcacttacta gttgtttgac cttgggcaag tcatttgacc ttctgtgcct cagtttcctc 4680
atctgtaaaa tggggctaac aatattacct acctcatagg atttaatgat gtcaagctcc 4740
tcactggagg ccttatccct tcgtggagcc cactaggtgc cgacccctca gaatataacc 4800
ctcatgcctg gacccctgag agcttctgat cccagctatt agggacagaa gaagcctcca 4860
aatctggaag gtgctgaatg ccctgctgac tgggaaagtt tcagggcact gatggggtct 4920
acctggtaag cggagggcct gaggaaacct gtagcttcaa tcatgtctgg taaccgggtg 4980
cctgagcccc aatctgggtt gtgaggaaat aggggagagg tatcctgggc cacatcccag 5040
cctaacacct gtgaggttca ttttaggaac taacctcatt agctataagg atcatgcaga 5100
ggcagcaaag ccgggtgcga tgagctcagc ctttactcat tcacatacac catcacactt 5160
taattccaat ctgtatattg ctttttaaaa gttaagtcca ttctaattac ccaaatatgc 5220
atgaattcat tctcctttg agaagttaga ttgttaaaga tagtctcatt cagctaccaa 5280
ccactccttg atccttccct tcttagtggc tgttgtttgt tgtacttccg tttagacttt 5340
gttttaatgc ttgtacgtac atatgtgaac tcattggaaa tattgtgtgt ttaatgcaaa 5400
tgatatattg aattgtttag caatttgttt tctttgctta acgatgtttt tgagatctgt 5460
gcatgttact taatgtagct caatccatct tctgtaattg ctgtatagat tgtcatcata 5520
tgattaccac attttactta cgcatttctt ttgtgatgga cattaagact gtttttaggt 5580
tttgctatta caaaatacta cacaggagca tcactatgcc tgtgtgaaag tatatgtatg 5640
aaagtttacc tagggttgat tcctagaagt ggaattgcaa agtcatagga tatttatata 5700
ttggttttta ataatacttc caaattgccc tcctgtacta tttactcagt atttttcttg 5760
aggttgatct gaggtctaac attgttatcc tatatcattt tcatcccaag tagtgatatc 5820
tgtgaaatca caggtttgat gtgtgctaat tatgtattct tctaatacat attaaaagac 5880
ataactatca aaacaaaata aatttgtctg ttttcaacca aagaagtcac gtaccactgg 5940
tggtactgtg tgccataatt tggcaaatgc tggcctttat ggacgagcac aattcggggg 6000
tcagacctgg ttcaaattct agctgtagaa acttgtgcaa gttacttcac ctctgagcct 6060
aagtttccac atctgtaaaa ggagataata aacacctacc ttgcagtagt gaagcaaaga 6120
gaaaattaaa tatatatgaa gcaatttggc tggcatctag atcattcaca gcccttttaaa 6180
ggtcaccttt gctgttctcc ccactttaca gataaggaaa ctgaggccca aaaaggtttg 6240
aacccaggtc ttccaagtca ttcaagtgct ttctccactg tacaggtggt tatcaacctt 6300
ggctgcgcat cagaatcgtt tgtaaagctt tttctttttc ctttttaaaa agtaaagcaa 6360
tatatacaca ggtaaaaaaa taaaatagta cagaagggct tataatgaga agcagcagtt 6420

ccctgcttgc acccccacat ccaaaggatg tggagctctt taaaaataaa ttgctctggt 6480
cccacctctg gaaatctgat tcagccagca tggataataa cccagataac taacccctac 6540
ctcacaggat aaaaaggatt acatgagatg ccttaggcta aggccctggc acacaggaac 6600
acatgtgcta caaaggagct ttggggactt aagtcctgag gatccaggag gtgaggtgac 6660
ttgtccaaga ttccactggt ttagtggcag agcctagact tccactcgga tctatttagt 6720
gcttgccccc tgctctctcc tgtcgtgccc caccacctcc tggcatcaca gggcaaccgt 6780
tgtcaaggct atgctcacgg gaggctgggc accacagtgt ttccaagagc aagctggatc 6840
cgagtagatt ccctagggct tgttggagga actagtttga ctcccttata ctgtggacgc 6900
agtagccttg ctgtagggag ttgaagagta ctccacaaca gtatcttaag tttaactggg 6960
cacttccctc tggaaatcac agtgttgtgc accaggaaca caaagatgag tcaaatcttt 7020
atcctgcctt tgaggagctc actgtttagt tggggaaacc atttgtaaaa cagccattaa 7080
ccatacagtg tgatcaacac tgacaggagc acaggaaaaa catctagctt atgtgaagat 7140
tcagagaagg catcctgtag tctaggtggt gatacctgaa ctgagtcttg agggacgggt 7200
aggaattagc cagttgagga agtagaagga atttccagat attggaaaca gtatgcatga 7260
agacatgaag gcaagaaaca gcaaaacaaa tactgaagca tgaagattcc tggggtgggg 7320
ggaaagcagc aagaaaaggt agagaggaac cagattggaa gagggtcgta aatgcatggc 7380
tacagaattc agatttgttt tgtaggacag tgtggttccc aaactggctg tataccacaa 7440
acaggtacgg cattctgggc cccggcccct aaaacattca ttaagtctgg ggtgaagatt 7500
tggaatcttg aatgcttata aaggttacca catgactagg gtacagccag atttggaaac 7560
catagcttga aggcagtgag ggagccatga aatggttttt aataggggga ctccagatca 7620
gatgtgaact taacctgttt ctggctggct agccaaccag catggaaaac agattaggtt 7680
agatgttcat gctgtatgtg cccgtgcctg tagcttccct gttaatcagc ttcttacact 7740
actatatttg cttattttgt ctctgaataa gctttaggca ccacaagggt gggcctgggg 7800
atattttgct taccagtata gcccctgcaa aaaagcacag tgcctgacac aaaacaggca 7860
cccagtaaag tttttgaatg aatgaatgca tgagtgaatc catttgtgag agagcgaatg 7920
gagatgacaa gattagctag gagactggaa aaagaccagg aggcctgcac tagggcaaag 7980
gccagtagga atagattgga ggtgttaagg tgtgaactgt taaggtaaga tgataactta 8040
atgactgatt attggatgtg gagggtgact gagaggatag aatgagtacc catgaatagc 8100
catgattcct accctgtccc agtcatctct ttccttatcc atctctgaaa caatctgctt 8160
acatcctcct cagcaactgg aattcctcaa gttagttaga cattctgtgt gctgtgtggt 8220
ctctcactgc ccccccactc cccacccctc cacaagccat tgattcattc atccagttca 8280
ataaatcttg gctaagcacc tccagtgtgc agtaaggctc ttccaagcca ggactctgac 8340
tccctctttc ctacctcaag agatgttttt gaggctttc ccaggtaaga gtcacatctc 8400
ttatacaata acttatagtg agatacccag aatgtcagac ttgtaaggga agactgccca 8460
aaccccttct gaggtcctca gaggggaatt aacttcctaa ggtccgactg ctaggaagtg 8520
ttggagccag aaatggaacc taggtttcct ttctatgtca tctctggagt cttgatcttg 8580
atctatccca ttgtagatca ggacaggcag aggtggtcag ggagaaggtg ggacttaggt 8640
tgaaccttga aggtcaatgt attggacagg tcaaacaaga tggttgccaa ttacactgcc 8700
cccttctgga aacccttagc aaacctgcca tgcttgcagt cccttctaag ggtttccctt 8760
agcataagtt gccatgctct gtaccatgtg acctcacaat cctggccaca gatagctaga 8820
tgtggatagt gtctggttca agggcaacca atctctaggc tggccagtgg cctgttagct 8880
ggactggcat aaggacttca ccttacaggg gtggcatgta tcaaatggca aatgtatgaa 8940
acaaccagat ctttcaggga ggcagaatgt gagctattca gaagaagtga acgttaatta 9000
gaatttaatg aggcattagt ggtggtggat gaggggtggc cagaaactaa acagcaaaag 9060
caaagagaaa gctgcagaaa ccataagtaa gcagaggtca tgagacattt gtataatgag 9120
atcacggagc cacagggtgg cagaagccat gaagcagcaa ggcaacaatg ggctagaagc 9180
catgaagcaa taggagccac gaggaacaga aaccgtgaga caaaactgac tatgagatcc 9240
acaaagcagc agaaggcttg aatagataag atcatgagac agtagaagcg atgagactgc 9300
aagaaccaca aggtagccag aaccatgtgg caacatggca acaggaatgg aagaggcagc 9360
aggagctaca atgcagaaaa gccatggatt aataggaact gaagcgccgg gagccatgaa 9420
gctgcaggac ccatgaggca gaaaaagcca tgggctagca tcgagggggg cagaaagaag 9480
ttagtcagta gcagtaggag gagtataaat acagccagaa aggagttgag tcaccaattt 9540
gggaagcact agagaaggga gcaacagatg cctgcagctg agggggtgac aagataagcc 9600
aggctctaga gctgctttgg atcatgaacc attttcaagt ttctgttctt ccatgaggct 9660
gcctgtgtag ctgttcttgt cttccttatt tccctgtgaa tgctttaata aatccccatc 9720
actaa 9725

<210> 10
<211> 1853
<212> DNA
<213> Homo sapiens
<400> 10
gatccctgag cgtgtggcag cagtgcggtc gtggtccctc cctatgcagc ctggtttcta 60
gcgtgacacg cccttgactt gaggaccatg aaccgcagcc gccaggtgac gtgcgtggcc 120
tgggtccgct gcggcgtggc caaagagaca ccagacaagg tagagctgag taaagaagaa 180
gtaaaacgcc tcattgctga ggcaaaggag aaattgcaag aagaaggtgg tggcagtgat 240
gaagaggaga caggcagtcc ttcagaagat ggcatgcaga gtgcacgcac ccaggcacgc 300
ccaagagagc ccctggagga tggtgaccca gaggatgaca ggacgcttga tgatgatgag 360
ctggctgagt acgacttaga taaatatgat gaggaaggtg acccagatgc tgagactctt 420

```
ggtgaatctc tcttgggtct tacggtctac gggagtaatg atcaagatcc ttacgttact     480
ctgaaagata cagaacaata tgaacgtgaa gatttcttga ttaagcccag tgataatctt     540
atagtttgtg gccgagctga acaggaccag tgcaatttag aggtgcatgt ttataatcaa     600
gaagaagact ctttttatgt acaccatgat atactcttgt ctgcatatcc tctgagtgtg     660
gaatggctga attttgatcc tagcccagat gattctactg gaaattacat tgctgtagga     720
aacatgaccc ctgttattga agtgtgggac cttgatatag tggactcttt agagccagtc     780
ttcacactcg gaagtaaact ttcaaaaaag aagaaaaaga aaggaaagaa gagttcctca     840
gcagaagggc ataccgatgc tgtccttgac ctttcatgga ataagctaat cagaaatgtt     900
ttagcaagtg catcagctga caacactgta attctgtggg atatgtcctt ggggaaacca     960
gcagctagcc tcgctgtaca cacagacaag gtccaaacac tgcagtttca tccatttgaa    1020
gcacagactc tgatttctgg ctcatatgat aagtcagtgg ctttgtatga ctgccgaagt    1080
ccagatgaaa gccatcgaat gtggcgattc agtgggcaga tagagagagt gacttggaat    1140
cacttttcac cttgtcattt cttggccagt acagatgacg gctttgtata taatttggat    1200
gcacgttcag ataagccaat ttttacactt aatgcacaca atgatgaaat ctctggtctt    1260
gatcttagca gtcaaatcaa gggctgtctc gtgactgctt cagctgacaa atacgtgaag    1320
atctgggaca tcttaggaga taggccaagt ctagttcatt ctagggacat gaaaatggga    1380
gttctcttct gttcttcatg ttgccctgat ttgccattta tttatgcctt tggaggtcaa    1440
aaagaagggc ttcgggtctg ggatataagc acagtctctt cagtaaatga agcatttgga    1500
agacgagaga ggcttgttct tgggagtgca agaaattcat ctattagtgg ccccttttggc   1560
agcaggagct cagatacacc catggagtct taatgaagat catctaattt cctgcttacc    1620
ttaactggga attttaaaaa gttggcctaa aaatgttcca tgcgtggcag caaccatgca    1680
gagtgactga aacacaattc atttctgact gacattcctt tctgcaactg cggtggcacc    1740
acaaatatcc ggtctttgtg cttgctcttc agatggatgg tttgtaaggc tcttgttgca    1800
tttcttaaaa aagagtaata aaaaggattt ttaaaaagta attccttaaa cat           1853
```

```
<210>  11
<211>  1985
<212>  DNA
<213>  Homo sapiens
<400>  11
```

```
cgccgagctt tcggcacctc tgccgggtgg taccgagcct tcccggcgcc ccctcctctc      60
ctcccaccgg cctgcccttc cccgcgggac tatcgccccc acgtttccct cagccctttt     120
ctctcccggc cgagccgcgg cggcagcagc agcagcagca gcagcaggag gaggagcccg     180
gtggcggcgg tggccgggga gcccatggcg tacagtcaag gaggcggcaa aaaaaaagtc     240
tgctactact acgacggtga tattggaaat tattattatg gacagggtca tcccatgaag     300
cctcatagaa tccgcatgac ccataacttg ctgttaaatt atggcttata cagaaaaatg     360
gaaatatata ggccccataa agccactgcc gaagaaatga caaaatatca cagtgatgag     420
tatatcaaat ttctacggtc aataagacca gataacatgt ctgagtatag taagcagatg     480
catatattta atgttggaga agattgtcca gcgtttgatg gactctttga gttttgtcag     540
ctctcaactg gcggttcagt tgctggagct gtgaagttaa accgacaaca gactgatatg     600
gctgttaatt gggctggagg attacatcat gctaagaaat acgaagcatc aggattctgt     660
tacgttaatg atattgtgct tgccatcctt gaattactaa agtatcatca gagagtctta     720
tatattgata tagatattca tcatggtgat ggtgttgaag aagcttttta tacaacagat     780
cgtgtaatga cggtatcatt ccataaatat ggggaatact ttcctggcac aggagacttg     840
agggatattg gtgctggaaa aggcaaatac tatgctgtca attttccaat gtgtgatggt     900
atagatgatg agtcatatgg gcagatattt aagcctatta tctcaaaggt gatggagatg     960
tatcaaccta gtgctgtggt attacagtgt ggtgcagact cattatctgg tgatagactg    1020
ggttgtttca atctaacagt caaaggtcat gctaaatgtg tagaagttgt aaaaactttt    1080
aacttaccat tactgatgct tggaggaggt ggctacacaa tccgtaatgt tgctcgatgt    1140
tggacatatg agactgcagt tgcccttgat tgtgagattc ccaatgagtt gccatataat    1200
gattactttg agtattttgg accagacttc aaactgcata ttagtccttc aaacatgaca    1260
aaccagaaca ctccagaata tatggaaaag ataaaacagc gtttgtttga aaatttgcgc    1320
atgttacctc atgcacctgg tgtccagatg caagctattc cagaagatgc tgttcatgaa    1380
gacagtggag atgaagatgg agaagatcca gacaagagaa tttctattcg agcatcagac    1440
aagcggatag cttgtgatga agaattctca gattctgagg atgaaggaga aggaggtcga    1500
agaaatgtgg ctgatcataa gaaaggagca aagaaagcta gaattgaaga agataagaaa    1560
gaaacagagg acaaaaaaac agacgttaag gaagaagata aatccaagga caacagtggt    1620
gaaaaaacag ataccaaagg aaccaaatca gaacagctca gcaacccctg aatttgacag    1680
tctcaccaat ttcagaaaat cattaaaaag aaaatattga aaggaaaatg ttttcttttt    1740
gaagacttct ggcttcattt tatactactt tggcatggac tgtatttatt ttcaaatggg    1800
actttttcgt ttttgttttt ctgggcaagt tttattgtga gattttctaa ttatgaagca    1860
aaatttcttt tctccaccat gctttatgtg atagtattta aaattgatgt gagttattat    1920
gtcaaaaaaa ctgatctatt aaagaagtaa ttggcctttc tgagctgaaa aaaaaaaaaa    1980
aaaag                                                                1985
```

```
<210>  12
<211>  2429
<212>  DNA
<213>  Homo sapiens
```

<400> 12
aagcggttgg gaaagtgtcg gtttatcttc gcgccccttg cgttcttgcc gcggcttgcc 60
tgggcaggta aagcgcgatt gcgagagctc ggcaaccctg ccgactcagc cggaaccggc 120
tcccggcccg aggggcgtgg tgtcctggtg ctccgactcc ttccgcaggc tccttgggac 180
ccgcggttcc gggagtccct tgctcagggt ccctttcctg cagtgaggcg ccgtccgcct 240
tccctgtgtc cccgcagacc cccatcatgg gcaataccag cagtgagcgc gccgcgctgg 300
agcggcatgg tggccataag acgccccgga gggacagctc gggggcacc aaggacgggg 360
acaggcccaa gatcctgatg gacagccccg aagacgccga cctcttccac tccgaggaaa 420
tcaaggcacc agagaaggag gaattcctgg cctggcagca tgatctggaa gtgaatgata 480
aagctcccgc ccaggctcgg ccaacggtgt ttcgatggac gggggcgga aaggaagttt 540
acttatctgg gtccttcaac aactggagta aacttcccct caccagaagc cacaataact 600
ttgtagccat cctggatctg ccggaaggag agcatcagta caagttcttt gtggatggtc 660
agtggacgca cgacccttcc gagcccatag taaccagcca gcttggcaca gttaacaaca 720
tcattcaagt gaagaaaact gactttgaag tatttgatgc tttaatggtg gattcccaaa 780
agtgctccga tgtgtctgag ctgtccagtt ctcccccagg accctaccat caggagccct 840
acgtctgcaa acccgaagag cgctttcggg caccccctat tctcccccca catctcctcc 900
aggtcatcct gaacaaggac acggggattt cctgtgatcc agctttgctt cctgagccca 960
atcacgtcat gctgaaccac ctatacgcgc tgtctatcaa ggatggagtg atggtgctca 1020
gcgcaaccca ccggtacaag aagaagtacg tcaccacctt gttatacaag cccatatgaa 1080
gagctggggg cggatggtgg cccaggagac agcacaccac caggctccac acgtgcatgc 1140
tttccccaag agggaatgga ctgtacattg ctcatttcac actcttcaga agacatttca 1200
tacctgccct ggtcctgctt gaaggtttgt ccaggcagag cagctcctgc agcgcctcgg 1260
tctgtgacag tcctcctagc acccccatgg ctttgagcct cggggactca tcaagtccaa 1320
gaaaagaggg aggggtggca gaggatctgc agccctggcc ccgcggtgca tgaggctggg 1380
tgcagttcta aacctacatt ctcgattttt cttaagccaa aaatgaatgc taactccttt 1440
gccagtaaaa ttctgggaaa cagggactga ggccacacat catttccagt catctgtgtg 1500
tttttaaggc cagccacttg tccctgttga ggcctggcta tggaactaaa tacagtgttg 1560
gtcttgcctg tccttcaaaa tcaacaacag attgtctctc ggctccaggg aggtgtcatt 1620
tctatagaaa ttagaagctt tctgatttct agatgaggtt ttacaattgt ttcttacagt 1680
catgtgcact aagtactctt tttgtaagca gaggtggctg gctctgcagc cttaaggcca 1740
ttttttaagt caccacgtct agaagtcaca tgaactctgc tcagcaataa tctgttctca 1800
gaacagactt ttcaacctgc tgccggattt ctccattcag ctggatgatc ctcaggactg 1860
accagttagc tggcaggttg tccagctttt tattccagtc ataataggtg acagtgttaa 1920
ccgtgaaaac ttgagaggca ctctgccctc ttccctataa aatcacacag cgtgatttta 1980
caaggtcccg tggcaccttg ctcaggacct ctgcccctag ttagcaagac tgcagcagtt 2040
gctgttgctt attctgaaag gaatgtagaa cttgacagca gccttctgag tctgggtcag 2100
gaagatgtcc tttggaccaa agcagacttc tgtatacgca gctcagtttc ccgggagtcg 2160
ccacagatgt acccactagc ccaggttgct gtgagtcagc ggaagctccc gttatgccct 2220
ttgctcctgg tgggagaggg aggagtgagc tccctgggtt ccagtattta cttggtatac 2280
ctgagtttgg gggtaccctt ttttgtgact tttcaaaaca gtgaattact gtcaccttga 2340
tggacaagtt tcaataaaac tttgtaaaaa aaaaaaaaaa aaaaaaaaaa aaaaaaaaaa 2400
aaaaaaaaaa aaaaaaaaaa aaaaaaaaa 2429

<210> 13
<211> 1654
<212> DNA
<213> Homo sapiens
<400> 13
gaattcgggc ggtcctcgga gacacgcggc ggtgtcctgt gttggccatg gccgactacc 60
tgattagtgg gggcacgtcc tacgtgccag acgacggact cacagcacag cagctcttca 120
actgcggaga cggcctcacc tacaatgact ttctcattct ccctgggtac atcgacttca 180
ctgcagacca ggtggacctg acttctgctc tgaccaagaa aatcactctt aagaccccac 240
tggtttcctc tcccatggac acagtcacag aggctgggat ggccatagca atggcgctta 300
caggcggtat tggcttcatc caccacaact gtacacctga attccaggcc aatgaagttc 360
ggaaagtgaa gaaatatgaa cagggattca tcacagaccc tgtggtcctc agccccaagg 420
atcgcgtgcg ggatgttttt gaggccaagg cccggcatgg tttctgcggt atcccaatca 480
cagacacagg ccggatgggg agccgcttgg tgggcatcat ctcctccagg gacattgatt 540
ttctcaaaga ggaggaacat gactgtttct tggaagagat aatgacaaag agggaagact 600
tggtggtagc cccccgcagc atcacactga aggaggcaaa tgaaattctg cagcgcagca 660
agaagggaaa gttgcccatt gtaaatgaag atgatgagct tgtggccatc attgcccgga 720
cagacctgaa gaagaatcgg gactacccac tagcctccaa agatgccaag aaacagctgc 780
tgtgtggggc agccattggc actcatgagg atgacaagta taggctggac ttgctcgccc 840
aggctggtgt ggatgtagtg gttttggact cttcccaggg aaattccatc ttccagatca 900
atatgatcaa gtacatcaaa gacaaatacc ctaatctcca agtcattgga ggcaatgtgg 960
tcactgctgc ccaggccaag aacctcattg atgcaggtgt ggatgccctg cgggtgggca 1020
tgggaagtgg ctccatctgc attacgcagg aagtgctggc ctgtgggcgg ccccaagcaa 1080
cagcagtgta caaggtgtca gagtatgcac ggcgctttgg tgttccggtc attgctgatg 1140
gaggaatcca aaatgtgggt catattgcga aagccttggc ccttggggcc tccacagtca 1200
tgatgggctc tctcctggct gccaccactg aggcccctgg tgaatacttc ttttccgatg 1260

ggatccggct aaagaaatat cgcggtatgg gttctctcga tgccatggac aagcacctca 1320
gcagccagaa cagatatttc agtgaagctg acaaaatcaa agtggcccag ggagtgtctg 1380
gtgctgtgca ggacaaaggg tcaatccaca aatttgtccc ttacctgatt gctggcatcc 1440
aacactcatg ccaggacatt ggtgccaaga gcttgaccca agtccgagcc atgatgtact 1500
ctggggagct taagtttgag aagagaacgt cctcagccca ggtggaaggt ggcgtccata 1560
gcctccattc gtatgagaag cggcttttct gaaaagggat ccagcacacc tcctcggttt 1620
ttttttcaat aaaagtttag aaagacccga attc 1654

<210> 14
<211> 1799
<212> DNA
<213> Homo sapiens
<400> 14
acactggggt ggtgcttagc cggcgccaga ccgaccctcg acttcggaga ggcagcgcgg 60
ttcctctggg tgcttccgcc tccccttctc ctgcttctcc agcctcttcg gcctcctcgc 120
ccgccgcggg aacccgagac cccagtgtat gccccacccc tgaccccgct cgcgacatgt 180
ccaccccggc tcggcggcgc ctcatgcggg acttcaagag gttgcaggag gatcctccag 240
ccggagtcag cggggctccg tccgagaaca acataatggt gtggaacgcg gtcattttcg 300
ggcctgaagg gaccccgttt gaggatggaa catttaaact tacaatagaa ttcactgaag 360
aatatccaaa taaaccacct acagttagat ttgtctctaa gatgttccat ccaaatgtct 420
atgcagatgg tagtatatgt ctggacatac ttcagaaccg ttggagtcca acctatgatg 480
tgtcttccat tctaacatcc atacagtctc tgttggatga acccaatccc aatagtccag 540
caaacagcca ggctgctcag ctgtaccagg agaacaaacg ggaatatgaa aagcgtgttt 600
ctgcaatagt agaacaaagc tggcgtgatt gttgaccccg ggtacagttt aaagaagctg 660
gccataagaa aaatatatat tgatgtgttt gtcacctccc tactcctgtc attacattta 720
ctttattaaa agcaaaataa ctgttgtgct gtttccatct tccttgccaa gttttcctac 780
cccttctacc ctctccttaa acatcagaaa acaccctcta tgaaatcaaa tgtactgtac 840
ctgggttact tgcaaaaatt actaatgctt cagtttttct gttgtatttc atttccagtt 900
ttcaggcagt tatttttatt attgtacttt aagcttttaa gatgaattgt tatacaagag 960
gtgcttatgc ttagcttgat gaccaggatg ttatttttaa caaaatgatt gctgaagtgt 1020
ttcatcctgg ctggtccttc acttgtgttg gatttagaag tgaatgtgtt tggaatatgg 1080
cctacagaga atagaaacaa atccatgtaa acaattttga aggaggcatg ggagctaaaa 1140
atcctgtgat actaagatct cagtcatatg aattacaacg tagtatttac tggcaagaag 1200
gagaaagttg aaggactcag ctaaaggagt acagcaattg tagtaactga cacatcctct 1260
ctttgcaagc tgctgactgg gcacactcat gccaagtttc agaattattg gtcttctggg 1320
tttttgcttt ttaaaagagg tgtgggagca gaggaatgga aacaatcgtg agtttttgag 1380
ctagggaaag ttggagctcc tttaatcttt ttaaaggatc agtgctgccc taagtgaata 1440
aactcaattg tccatcttta ttttagagtt ttaatgaatt caaggaaggg agcatagcat 1500
atctgtggca aactattttc cactcaaatc ctgagttatt gctgcatgct ttaatttctt 1560
ccctttcagc atctgagaac cttaaagcca atgtctgcga tcttttttttg gatatttata 1620
cttttagata tatagtacct ttaagtagca gtatgggaca aggcttgtaa atgttttgtc 1680
taatgttcta ttgtcacctt ttatgcattt atcacttcca aatctaactt tgcacaagta 1740
acccatgtaa aaaaaaatgt acatttttca aaagttgtaa ataaaaataa ccttaaaaa 1799

<210> 15
<211> 1105
<212> DNA
<213> Homo sapiens
<400> 15
ggggcttgtg ggtctttgag acccgaaaat tgagagcgtt ttcgcactcc agcggctgct 60
cctggcggct ctgcggccgt caccatgcca cagaatgaat atattgaatt acaccgtaaa 120
cgctatggat accgtttgga ttaccatgag aaaaagagaa agaaggaaag tcgagaggct 180
catgaacgtt caaagaaggc aaagaaaatg attggtctga aggctaagct ttaccataaa 240
cagcgtcatg ctgagaaaat acaaatgaaa aagactatca agatgcatga aaagagaaac 300
accaaacaaa agaatgatga aaagacacca cagggagcag tacctgccta tctgctggac 360
agagagggac aatctcgagc taaagtactt tccaatatga ttaaacagaa aagaaaagag 420
aaggcgggaa aatgggaagt ccctctgcct aaagtacgtg cccagggaga aacagaagta 480
ttaaaagtta ttcgaacagg aaagagaaag aagaaggcat ggaagagaat ggttactaaa 540
gtgtgctttg ttggagatgg ctttacaaga aaaccaccta aatatgaaag attcatcagg 600
ccaatgggct tgcgtttcaa gaaagcccat gtaacacatc ctgaactgaa agccaccttt 660
tgcctaccaa tacttggtgt aaagaagaat ccctcatccc cactgtatac aactttgggt 720
gttattacca aaggtactgt cattgaagta aatgtgagcg aattgggcct tgtgacgcaa 780
ggaggcaaag ttatttgggg aaaatatgcc caggttacca acaatcctga aaatgatgga 840
tgtataaatg cagtcttact ggtttgacag caatttcata tataattatt gaggactaca 900
caccaattga agaaactgcc attactgtga tgtttctgaa tactaccaaa cagccataca 960
tgtctgcaat gaagagattt attaaattgt aaacattaaa gtggtccagt tttataaatg 1020
gtctttattt tgaaatacgc tttgacccca tgttcataaa actgaatgat tgaaaaaaag 1080
caaatataca aatatcctac ttcat 1105

<210> 16
<211> 2905
<212> DNA
<213> Homo sapiens
<400> 16
cctggcgcgc ccggagttgc tggttctgtg aggtagcggc ggcaacgacc atggaggcca 60
cgtcccggga ggcggcgcca gcgaagagct cggcctcggg ccccaacgct ccccccgccc 120
tgttcgagct gtgcgggcgg gcggtgagcg cccatatggg ggttctggag agcggggtgt 180
gggccctccc aggcccaata cttcaaagca tcctacctct gctcaatata tattacttgg 240
agaggattga ggaaactgcc ctcaagaaag gcctctcaac tcaggccatc tggcgccgac 300
tctgggatga actgatgaag acaaggcctt ccagtttgga aagtgtgaca tgttggcgag 360
ccaagtttat ggaggccttt ttttcccatg ttctacgtgg gaccattgat gtgtcttctg 420
acaggcgtct ttgtgatcag cggttctcac ctcttctgca cagctcccgc catgtccgac 480
agctcaccat ctgtaacatg ctgcagggtg caaccgagct ggtggctgag cccaaccgca 540
gggttctgga gaccctggcc agctccctgc acactctcaa gttccgccac ctgctgttct 600
ctgatgtggc tgctcagcag tcacttcggc agctgttgca tcagctcatt caccatgggg 660
ctgtcagtca agtgtcgcta tactcctggc ctgtgcctga gtcagccctt ttcatcctta 720
ttctcaccat gagtgctggc ttctggcaac cagggcctgg tggcccaccc tgccgcctct 780
gtggagaggc ctcccgaggc cgggccccat cccgagatga agggtccctc ttattgggct 840
cacgtcggcc ccgccgggat gctgctgagc gatgtgctgc agccctgatg gccagccggc 900
gtaagagtga agccaagcag atgcccagag ctgcacctgc cactcgggta acacgccgga 960
gcacacagga gagcctgaca gcaggcggaa cagaccttaa gagggagctg cacccccag 1020
ccacctccca tgaggctcct ggcaccaagc ggtcaccttc tgctccagca gccacctcct 1080
ctgcctcttc ttctacatcc tcatacaaac gggcaccagc tagctcagcc ccacagccta 1140
agcccctaaa gcgtttcaag cgagctgcag ggaagaaggg tgctcgcacc cgtcaggggc 1200
ctggtgcaga gtctgaagac ctgtatgact tcgtttttat tgtggctggc gagaaggagg 1260
atggcgaaga gatggagatt ggggaagtgg cttgtggagc tttggatgga tcagatccca 1320
gctgcctggg gcttccagca ctggaagctt cacaaagatt ccgcagcatc tccaccttgg 1380
agctattcac agttccactc tccacagagg cagccctgac actatgccac ctgctgagct 1440
cctgggtgtc actggagagc ctcacactct cctacaatgg cctgggctct aacatcttcc 1500
gcctgctaga cagcctgcgg gccctgtcag gccaggctgg atgtcgcctc cgtgccctgc 1560
atctcagtga cctgttctca ccactgccca tcctggagct gacacgtgct atcgtgcgag 1620
cactgcccct gctacgggtc ctctctattc gtgttgacca cccaagccag cgggacaacc 1680
ctggtgtgcc agggaatgca gggcccccta gccacataat aggcgatgag gagataccag 1740
aaaactgcct ggagcagttg gagatgggat ttccacgggg agcccagcca gccccactgc 1800
tgtgctccgt tctgaaggcc tcgggttctc tgcagcagct gtccctggat agtgccacct 1860
ttgcctctcc ccaggatttt gggcttgttt tgcaaacact caaagagtac aacctagccc 1920
tgaaaagact gagcttccat gacatgaatc tcgctgactg tcagagcgag gtgctctttt 1980
tgctacagaa tctgactctg caagagatta ccttctcctt ctgccgtctg tttgagaagc 2040
gcccagccca atttctgcct gagatggttg ctgctatgaa gggcaactcc acactgaagg 2100
gcctccggct gccagggaac cgcctgggga atgctggcct gctggccttg gcagatgttt 2160
tctcagagga ttcatcctcc tctctctgtc agctggacat cagttccaac tgcatcaagc 2220
cagatgggct tctggagttc gccaagcggc tggagcgctg ggcccgtgga gcctttggtc 2280
acctgcgcct cttccaaaac tggctggacc aggatgcagt cacagccagg gaagccatcc 2340
ggcggctccg ggctacctgc catgtggtta gcgactcatg ggactcatcc caggccttcg 2400
cagattatgt tagcaccatg tgatggggcc cgtacctcac agtctcatgc tcggtaccat 2460
cagcttgcag ggctgaagc atgggctgcc cagaacccca accaccagtt ctatctttct 2520
ctttctgtca cctttttct ctttttcct tcttcccttg cactgaggtc ctggaggcct 2580
tgatgaggcc cagcaaacag gcattctcac agctgggttt atagtctttg ggcccccttac 2640
tcagtatcct gggaaccctg ggccaggagg ttacagtggt catcataatt gctgaagaga 2700
tcccctcccc tgcccctggg ttcctgcctt ccctcctcaa gcaggcaccc aggctttaga 2760
gaagtatagg gggcttcttc cctgctgggc ttaccacact gctctcaggc ctcaaaccct 2820
ttcatacctt tattcttttt tttaaccaaa aaagtttttc ttataaaata aattttgggc 2880
aaacatcaaa aaaaaaaaaa aaaaa 2905

<210> 17
<211> 7787
<212> DNA
<213> Homo sapiens
<400> 17
gagacaaagg ctgccgtcgg gacgggcgag ttagggactt gggtttgggc gaacaaaagg 60
tgagaaggac aagaagggac cgggcgatgg cagcagggga gccccgcggg cgcgcgtcct 120
cgggagtggc gccgtgacac gcatggtttc cccggacccg cggcggcgct gacttccgcg 180
agtcggagcg gcactcggcg agtccgggac tgcgctggaa caatggataa cttcttcacc 240
gagggaacac gggtctggct gagagaaaat ggccagcatt ttccaagtac tgtaaattcc 300
tgtgcagaag gcatcgtcgt cttccggaca gactatggtc aggtattcac ttacaagcag 360
agcacaatta cccaccagaa ggtgactgct atgcacccca cgaacgagga gggcgtggat 420
gacatggcgt ccttgacaga gctccatggc ggctccatca tgtataactt attccagcgg 480
tataagagaa atcaaatata tacctacatc ggctccatcc tggcctccgt gaacccctac 540

cagcccatcg ccgggctgta cgagcctgcc accatggagc agtacagccg gcgccacctg 600
ggcgagctgc ccccgcacat cttcgccatc gccaacgagt gctaccgctg cctgtggaag 660
cgctacgaca accagtgcat cctcatcagt ggtgaaagtg ggcaggtaa aaccgaaagc 720
actaaattga tcctcaagtt tctgtcagtc atcagtcaac agtctttgga attgtcctta 780
aaggagaaga catcctgtgt tgaacgagct attcttgaaa gcagccccat catggaagct 840
ttcggcaatg cgaagaccgt gtacaacaac aactctagtc gctttgggaa gtttgttcag 900
ctgaacatct gtcagaaagg aaatattcag ggcgggagaa ttgtagatta tttattagaa 960
aaaaaccgag tagtaaggca aaatcccggg gaaaggaatt atcacatatt ttatgcactg 1020
ctggcagggc tggaacatga agaaagagaa gaattttatt tatctacgcc agaaaactac 1080
cactacttga atcagtctgg atgtgtagaa gacaagacaa tcagtgacca ggaatccttt 1140
agggaagtta ttacggcaat ggacgtgatg cagttcagca aggaggaagt tcgggaagtg 1200
tcgaggctgc ttgctggtat actgcatctt gggaacatag aatttatcac tgctggtggg 1260
gcacaggttt ccttcaaaac agctttgggc agatctgcgg agttacttgg gctggaccca 1320
acacagctca cagatgcttt gacccagaga tcaatgttcc tcaggggaga agagatcctc 1380
acgcctctca atgttcaaca ggcagtagac agcagggact ccctggccat ggctctgtat 1440
gcgtgctgct ttgagtgggt aatcaagaag atcaacagca ggatcaaagg caatgaggac 1500
ttcaagtcta ttggcatcct cgacatcttt ggatttgaaa actttgaggt taatcacttt 1560
gaacagttca atataaacta tgcaaacgag aaacttcagg agtacttcaa caagcatatt 1620
ttttctttag aacaactaga atatagccgg gaaggattag tgtgggaaga tattgactgg 1680
atagacaatg gagaatgcct ggacttgatt gagaagaaac ttggcctcct agcccttatc 1740
aatgaagaaa gccatttttcc tcaagccaca gacagcacct tattggagaa gctacacagt 1800
cagcatgcga ataaccactt ttatgtgaag cccagagttg cagttaacaa ttttggagtg 1860
aagcactatg ctggagaggt gcaatatgat gtccgaggta tcttggagaa gaacagagat 1920
acatttcgag atgaccttct caatttgcta agagaaagcc gatttgactt tatctacgat 1980
ctttttgaac atgtttcaag ccgcaacaac caggatacct tgaaatgtgg aagcaaacat 2040
cggcggccta cagtcagctc acagttcaag gactcactgc attccttaat ggcaacgcta 2100
agctcctcta atcctttctt tgttcgctgt atcaagccaa acatgcagaa gatgccagac 2160
cagtttgacc aggcggttgt gctgaaccag ctgcggtact cagggatgct ggagactgtg 2220
agaatccgca aagctgggta tgcggtccga agacccttttc aggactttta caaaaggtat 2280
aaagtgctga tgaggaatct ggctctgcct gaggacgtcc gagggaagtg cacgagcctg 2340
ctgcagctct atgatgcctc caacagcgag tggcagctgg ggaagaccaa ggtctttctt 2400
cgagaatcct tggaacagaa actggagaag cggagggaag aggaagtgag ccacgcggcc 2460
atggtgattc gggcccatgt cttgggcttc ttagcacgaa aacaatacag aaaggtcctt 2520
tattgtgtgg tgataataca gaagaattac agagcattcc ttctgaggag gagatttttg 2580
cacctgaaaa aggcagccat agttttccag aagcaactca gaggtcagat tgctcggaga 2640
gtttacagac aattgctggc agagaaaagg gagcaagaag aaaagaagaa acaggaagag 2700
gaagaaaaga agaaacggga ggaagaagaa agagaaagag agagagagcg aagagaagcc 2760
gagctccgcg cccagcagga agaagaaacg aggaagcagc aagaactcga agccttgcag 2820
aagagccaga aggaagctga actgacccgt gaactggaga aacagaagga aaataagcag 2880
gtggaagaga tcctccgtct ggagaaagaa atcgaggacc tgcagcgcat gaaggagcag 2940
caggagctgt cgctgaccga ggcttccctg cagaagctgc aggagcggcg ggaccaggag 3000
ctccgcaggc tggaggagga agcgtgcagg gcggcccagg agttcctcga gtccctcaat 3060
ttcgacgaga tcgacgagtg tgtccggaat atcgagcggt ccctgtcggt gggaagcgaa 3120
ttttccagcg agctggctga gagcgcatgc gaggagaagc ccaacttcaa cttcagccag 3180
ccctacccag aggaggaggt cgatgagggc ttcgaagccg acgacgacgc cttcaaggac 3240
tcccccaacc ccagcgagca cggccactca gaccagcgaa caagtggcat ccggaccagc 3300
gatgactctt cagaggagga cccatacatg aacgacacgg tggtgcccac cagccccagt 3360
gcggacagca cggtgctgct cgccccatca gtgcaggact ccgggagcct acacaactcc 3420
tccagcggcg agtccaccta ctgcatgccc cagaacgctg ggacttgcc ctccccagac 3480
ggcgactacg actacgacca ggatgactat gaggacggtg ccatcacttc cggcagcagc 3540
gtgaccttct ccaactccta cggcagccag tggtcccccg actaccgctg ctctgtgggg 3600
acctacaaca gctcgggtgc ctaccggttc agctctgagg gggcgcagtc ctcgtttgaa 3660
gatagtgaag aggactttga ttccaggttt gatacagatg atgagctttc ataccggcgt 3720
gactctgtgt acagctgtgt cactctgccg tatttccaca gctttctgta catgaaaggt 3780
ggcctgatga actcttggaa acgccgctgg tgcgtcctca aggatgaaac cttcttgtgg 3840
ttccgctcca agcaggaggc cctcaagcaa ggctggctcc acaaaaaagg gggggctcc 3900
tccacgctgt ccaggagaaa ttggaagaag cgctggtttg tcctccgcca gtccaagctg 3960
atgtactttg aaaacgacag cgaggagaag ctcaagggca ccgtagaagt gcgaacggca 4020
aaagagatca tagataacac caccaaggag aatgggatcg acatcattat ggccgatagg 4080
actttccacc tgattgcaga gtccccagaa gatgccagcc agtggttcag cgtgctgagt 4140
caggtccacg cgtccacgga ccaggagatc caggagatgc atgatgagca ggcaaaccca 4200
cagaatgctg tgggcacctt ggatgtgggg ctgattgatt ctgtgtgtgc ctctgacagc 4260
cctgatagac ccaactcgtt tgtgatcatc acggccaacc gggtgctgca ctgcaacgcc 4320
gacacgccgg aggagatgca ccactggata accctgctgc agaggtccaa aggggacacc 4380
agagtggagg gccaggaatt catcgtgaga ggatggttgc acaaagaggt gaagaacagt 4440
ccgaagatgt cttcactgaa actgaagaaa cggtggtttg tactcaccca caattccctg 4500
gattactaca agagttcaga gaagaacgcg ctcaaactgg ggaccctggt cctcaacagc 4560
ctctgctctg tcgtcccccc agatgagaag atattcaaag agacaggcta ctggaacgtc 4620
accgtgtacg ggcgcaagca ctgttaccgg ctctacacca agctgctcaa cgaggccacc 4680

cggtggtcca gtgccattca aaacgtgact gacaccaagg ccccgatcga cacccccacc 4740
cagcagctga ttcaagatat caaggagaac tgcctgaact cggatgtggt ggaacagatt 4800
tacaagcgga acccgatcct tcgatacacc catcacccct tgcactcccc gctcctgccc 4860
cttccgtatg ggacataaa tctcaacttg ctcaaagaca aaggctatac caccccttcag 4920
gatgaggcca tcaagatatt caattccctg cagcaactgg agtccatgtc tgacccaatt 4980
ccaataatcc agggcatcct acagacaggg catgacctgc gacctctgcg ggacgagctg 5040
tactgccagc ttatcaaaca gaccaacaaa gtgccccacc ccggcagtgt gggcaacctg 5100
tacagctggc agatcctgac atgcctgagc tgcaccttcc tgccgagtcg agggattctc 5160
aagtatctca agttccatct gaaaaggata cgggaacagt ttccaggaac cgagatggaa 5220
aaatacgctc tcttcactta cgaatctctt aagaaaacca aatgccgaga gtttgtgcct 5280
tcccgagatg aaatagaagc tctgatccac aggcaggaaa tgacatccac ggtctattgc 5340
catggcggcg gctcctgcaa gatcaccatc aactcccaca ccactgctgg ggaggtggtg 5400
gagaagctga tccgaggcct ggccatggag gacagcagga acatgtttgc tttgtttgaa 5460
tacaacggcc acgtcgacaa agccattgaa agtcgaaccg tcgtagctga tgtcttagcc 5520
aagtttgaaa agctggctgc cacatccgag gttggggacc tgccatggaa attctacttc 5580
aaactttact gcttcctgga cacagacaac gtgccaaaag acagtgtgga gtttgcattt 5640
atgtttgaac aggcccacga agcggttatc catggccacc atccagcccc ggaagaaaac 5700
ctccaggttc ttgctgccct gcgactccag tatctgcagg gggattatac tctgcacgct 5760
gccatcccac ctctcgaaga ggtttattcc ctgcagagac tcaaggcccg catcagccag 5820
tcaaccaaaa ccttcacccc ttgtgaacgg ctggagaaga ggcggacgag cttcctagag 5880
gggaccctga ggcggagctt ccggacagga tccgtggtcc ggcagaaggt cgaggaggag 5940
cagatgctgg acatgtggat taaggaagaa gtctcctctg ctcgagccag tatcattgac 6000
aagtggagga aatttcaggg aatgaaccag gaacaggcca tggccaagta catggccttg 6060
atcaaggagt ggcctggcta tggctcgacg ctgtttgatg tggagtgcaa ggaaggtggc 6120
ttccctcagg aactctggtt gggtgtcagc gcggacgccg tctccgtcta caagcgtgga 6180
gagggaagac cactggaagt cttccagtat gaacacatcc tctcttttgg ggcacccctg 6240
gcgaatacgt ataagatcgt ggtcgatgag agggagctgc tctttgaaac cagtgaggtg 6300
gtggatgtgg ccaagctcat gaaagcctac atcagcatga tcgtgaagaa gcgctacagc 6360
acgacacgct ccgccagcag ccagggcagc tccaggtgaa ggcgggacag agcccacctg 6420
tctttgctac ctgaacgcac caccctctgg cctaggctgg ctccagtgtg ccatgcccag 6480
ccaaaacaaa cacagagctg cccaggcttt ctggaagctt ctggtctgag ggaggtgtct 6540
ccgaggatcc ttttgcctgc cgccttcatt gatcctgtat taagctgtca actttaacag 6600
tctgcacagt ttccaaagct ttactactct tagaggacac atgccttaaa aaaggagggg 6660
aggaaccacg ctgccaccaa agcagccgga agtgccttaa cttgtggaac caacactaat 6720
cgaccgtaac tgtgctactg aagggaactg cctttccccc ttctgggggga gacttaacag 6780
agcgtggaag gggggcattc tctgtcaatg atgcactaac ctcccaacct gatttccccg 6840
aatctgaggg aaggtgaggg agtgggaagg gggatggaga gctcgagggg acagtgtgtt 6900
tgagctggag tgctgcgggc agcctttctc atggaatgac atgaatcaac tttttttcttt 6960
gtttcatctt ttaagtgtac gtgcttgcct gttcgtgcat gtgttcataa actcaacact 7020
ttaatcatgg tttcatgagc attaaaaagc aaagggaaaa aggatgtgta atggtgtaca 7080
cagtctgtat attttaataa tgcagagcta tagtctcaat tgttacttta taaggtggtt 7140
ttattaacaa acccaaatcc tggattttcc tgtctttgct gtattttgaa aaacacgtgt 7200
tgactccatt gttttacatg tagcaaagtc tgccatctgt gtctgctgta ttataaacag 7260
ataagcagcc tacaagataa ctgtatttat aaaccactct tcaacagctg gctccagtgc 7320
tggttttaga acaagaatga agtcattttg gagtctttca tgtctaaaag atttaagtta 7380
aaaacaaagt gttacttgga aggttagctt ctatcattct ggatagatta cagatataat 7440
aaccatgttg actatggggg agagacgctg cattccagaa acgtcttaac acttgagtga 7500
atcttcaaag gaccctgaca ttaaatgctg aggctttaat acacacatat tttatcccaa 7560
gtttataatg gtggtctgaa caaggcacct gtaaataaat cagcatttat gaccagaaga 7620
aaaataatct ggtcttggac tttttatttt tatatggaaa agttttaagg acttgggcca 7680
actaagtcta cccacacgaa aaaagaaatt tgccttgtcc ctttgtgtac aaccatgcaa 7740
aactgtttgt tggctcacag aagttctgac aataaaagat actagct 7787

<210> 18
<211> 5512
<212> DNA
<213> Homo sapiens
<400> 18
gagctagccc cggcggccgc cgccgcccag accggacgac aggccacctc gtcggcgtcc 60
gcccgagtcc ccgcctcgcc gccaacgcca caaccaccgc gcacggcccc ctgactccgt 120
ccagtattga tcgggagagc cggagcgagc tcttcgggga gcagcgatgc gaccctccgg 180
gacggccggg gcagcgctcc tggcgctgct ggctgcgctc tgcccggcga gtcgggctct 240
ggaggaaaag aaagtttgcc aaggcacgag taacaagctc acgcagttgg gcacttttga 300
agatcatttt ctcagcctcc agaggatgtt caataactgt gaggtggtcc ttgggaattt 360
ggaaattacc tatgtgcaga ggaattatga tctttccttc ttaaagacca tccaggaggt 420
ggctggttat gtcctcattg ccctcaacac agtggagcga attcctttgg aaaacctgca 480
gatcatcaga ggaaatatgt actacgaaaa ttcctatgcc ttagcagtct tatctaacta 540
tgatgcaaat aaaaccggac tgaaggagct gcccatgaga aatttacagg aaatcctgca 600
tggcgccgtg cggttcagca acaaccctgc cctgtgcaac gtggagagca tccagtggcg 660

ggacatagtc agcagtgact ttctcagcaa catgtcgatg gacttccaga accacctggg 720
cagctgccaa aagtgtgatc caagctgtcc caatgggagc tgctggggtg caggagagga 780
gaactgccag aaactgacca aaatcatctg tgcccagcag tgctccgggc gctgccgtgg 840
caagtccccc agtgactgct gccacaacca gtgtgctgca ggctgcacag gcccccggga 900
gagcgactgc ctggtctgcc gcaaattccg agacgaagcc acgtgcaagg acacctgccc 960
cccactcatg ctctacaacc ccaccacgta ccagatggat gtgaaccccg agggcaaata 1020
cagctttggt gccacctgcg tgaagaagtg tccccgtaat tatgtggtga cagatcacgg 1080
ctcgtgcgtc cgagcctgtg gggccgacag ctatgagatg gaggaagacg gcgtccgcaa 1140
gtgtaagaag tgcgaagggc cttgccgcaa agtgtgtaac ggaataggta ttggtgaatt 1200
taaagactca ctctccataa atgctacgaa tattaaacac ttcaaaaact gcacctccat 1260
cagtggcgat ctccacatcc tgccggtggc atttaggggt gactccttca cacatactcc 1320
tcctctggat ccacaggaac tggatattct gaaaaccgta aaggaaatca cagggttttt 1380
gctgattcag gcttggcctg aaaacaggac ggacctccat gcctttgaga acctagaaat 1440
catacgcggc aggaccaagc aacatggtca gttttctctt gcagtcgtca gcctgaacat 1500
aacatccttg ggattacgct ccctcaagga gataagtgat ggagatgtga taatttcagg 1560
aaacaaaaat ttgtgctatg caaatacaat aaactggaaa aaactgtttg ggacctccgg 1620
tcagaaaacc aaaattataa gcaacagagg tgaaaacagc tgcaaggcca caggccaggt 1680
ctgccatgcc ttgtgctccc ccgagggctg ctggggcccg gagcccaggg actgcgtctc 1740
ttgccggaat gtcagccgag gcagggaatg cgtggacaag tgcaaccttc tggagggtga 1800
gccaagggag tttgtggaga actctgagtg catacagtgc cacccagagt gcctgcctca 1860
ggccatgaac atcacctgca caggacgggg accagacaac tgtatccagt gtgcccacta 1920
cattgacggc ccccactgcg tcaagacctg cccggcagga gtcatgggag aaaacaacac 1980
cctggtctgg aagtacgcag acgccggcca tgtgtgccac ctgtgccatc caaactgcac 2040
ctacggatgc actgggccag gtcttgaagg ctgtccaacg aatgggccta agatcccgtc 2100
catcgccact gggatggtgg gggccctcct cttgctgctg gtggtggccc tggggatcgg 2160
cctcttcatg cgaaggcgcc acatcgttcg gaagcgcacg ctgcggaggc tgctgcagga 2220
gagggagctt gtggagcctc ttacacccag tggagaagct cccaaccaag ctctcttgag 2280
gatcttgaag gaaactgaat tcaaaaagat caaagtgctg ggctccggtg cgttcggcac 2340
ggtgtataag ggactctgga tcccagaagg tgagaaagtt aaaattcccg tcgctatcaa 2400
ggaattaaga gaagcaacat ctccgaaagc caacaaggaa atcctcgatg aagcctacgt 2460
gatggccagc gtggacaacc cccacgtgtg ccgcctgctg ggcatctgcc tcacctccac 2520
cgtgcagctc atcacgcagc tcatgccctt cggctgcctc ctggactatg tccgggaaca 2580
caaagacaat attggctccc agtacctgct caactggtgt gtgcagatcg caaagggcat 2640
gaactacttg gaggaccgtc gcttggtgca ccgcgacctg gcagccagga acgtactggt 2700
gaaaacaccg cagcatgtca agatcacaga ttttgggctg gccaaactgc tgggtgcgga 2760
agagaaagaa taccatgcag aaggaggcaa agtgcctatc aagtggatgg cattggaatc 2820
aattttacac agaatctata cccaccagag tgatgtctgg agctacgggg tgaccgtttg 2880
ggagttgatg acctttggat ccaagccata tgacggaatc cctgccagcg agatctcctc 2940
catcctggag aaaggagaac gcctccctca gccaccccata tgtaccatcg atgtctacat 3000
gatcatggtc aagtgctgga tgatagacgc agatagtcgc ccaaagttcc gtgagttgat 3060
catcgaattc tccaaaatgg cccgagaccc ccagcgctac cttgtcattc aggggggatga 3120
aagaatgcat ttgccaagtc ctacagactc caacttctac cgtgccctga tggatgaaga 3180
agacatggac gacgtggtgg atgccgacga gtacctcatc ccacagcagg gcttcttcag 3240
cagcccctcc acgtcacgga ctcccctcct gagctctctg agtgcaacca gcaacaattc 3300
caccgtggct tgcattgata gaaatgggct gcaaagctgt cccatcaagg aagacagctt 3360
cttgcagcga tacagctcag accccacagg cgccttgact gaggacagca tagacgacac 3420
cttcctccca gtgcctgaat acataaacca gtccgttccc aaaaggcccg ctggctctgt 3480
gcagaatcct gtctatcaca atcagcctct gaaccccgcg cccagcagag acccacacta 3540
ccaggacccc cacagcactg cagtgggcaa ccccgagtat ctcaacactg tccagcccac 3600
ctgtgtcaac agcacattcg acagccctgc ccactgggcc cagaaaggca gccaccaaat 3660
tagcctggac aaccctgact accagcagga cttctttccc aaggaagcca agccaaatgg 3720
catctttaag ggctccacag ctgaaaatgc agaataccta agggtcgcgc cacaaagcag 3780
tgaatttatt ggagcatgac cacggaggat agtatgagcc ctaaaaatcc agactctttc 3840
gatacccagg accaagccac agcaggtcct ccatcccaac agccatgccc gcattagctc 3900
ttagacccac agactggttt tgcaacgttt acaccgacta gccaggaagt acttccacct 3960
cgggcacatt ttgggaagtt gcattccttt gtcttcaaac tgtgaagcat ttacagaaac 4020
gcatccagca agaatattgt ccctttgagc agaaatttat ctttcaaaga ggtatatttg 4080
aaaaaaaaaa aaaaagtata tgtgaggatt tttattgatt ggggatcttg gagtttttca 4140
ttgtcgctat tgatttttac ttcaatgggc tcttccaaca aggaagaagc ttgctggtag 4200
cacttgctac cctgagttca tccaggccca actgtgagca aggagcacaa gccacaagtc 4260
ttccagagga tgcttgattc cagtggttct gcttcaaggc ttccactgca aaacactaaa 4320
gatccaagaa ggccttcatg gccccagcag gccggatcgg tactgtatca agtcatggca 4380
ggtacagtag gataagccac tctgtccctt cctgggcaaa gaagaaacgg aggggatgaa 4440
ttcttcctta gacttacttt tgtaaaaatg tccccacggt acttactccc cactgatgga 4500
ccagtggttt ccagtcatga gcgttagact gacttgtttg tcttccattc cattgttttg 4560
aaactcagta tgccgcccct gtcttgctgt catgaaatca gcaagagagg atgacacatc 4620
aaataataac tcggattcca gcccacattg gattcatcag catttggacc aatagcccac 4680
agctgagaat gtggaatacc taaggataac accgcttttg ttctcgcaaa aacgtatctc 4740
ctaatttgag gctcagatga aatgcatcag gtcctttggg gcatagatca gaagactaca 4800

```
aaaatgaagc tgctctgaaa tctcctttag ccatcacccc aacccccccaa aattagtttg   4860
tgttacttat ggaagatagt tttctccttt tacttcactt caaaagcttt ttactcaaag   4920
agtatatgtt ccctccaggt cagctgcccc caaaccccct ccttacgctt tgtcacacaa   4980
aaagtgtctc tgccttgagt catctattca agcacttaca gctctggcca caacagggca   5040
ttttacaggt gcgaatgaca gtagcattat gagtagtgtg aattcaggta gtaaatatga   5100
aactagggtt tgaaattgat aatgctttca caacatttgc agatgtttta gaaggaaaaa   5160
agttccttcc taaaataatt tctctacaat tggaagattg gaagattcag ctagttagga   5220
gcccattttt tcctaatctg tgtgtgccct gtaacctgac tggttaacag cagtcctttg   5280
taaacagtgt tttaaactct cctagtcaat atccacccca tccaatttat caaggaagaa   5340
atggttcaga aaatattttc agcctacagt tatgttcagt cacacacaca tacaaaatgt   5400
tcctttttgct tttaaagtaa tttttgactc ccagatcagt cagagcccct acagcattgt   5460
taagaaagta tttgattttt gtctcaatga aaataaaact atattcattt cc           5512
```

<210> 19
<211> 1791
<212> DNA
<213> Homo sapiens
<400> 19

```
cctcgtgcca gagaaacatg tatcgttttc gatcacagct cttcacgggg atttctgctg     60
ccgccaccgc ccactcttac ccccgccgct tctcgactct gttgttagcc gaagactcgc    120
ctctcagccg cccgccgcac agacgcacga gtaaaaagtg cagctccatc ggctgatcct    180
cgctaagctc cgactctggg cggcaccggg cgtcccacga tgccgaagaa caagaagcgg    240
aacactcccc accgcggtag cagtgctggc ggcggcgggt caggagcagc cgcagcgacg    300
gcggcgacag caggtggcca gcatcgaaat gttcagcctt ttagtgatga agatgcatca    360
attgaaacaa tgagccattg cagtggttat agcgatcctt ccagttttgc tgaagatgga    420
ccagaagtcc ttgatgagga aggaactcaa gaagacctag agtacaagag aaagggatta    480
attgacctaa ccctggataa gagtgcgaag acaaggcaag cagctcttga aggtattaaa    540
aatgcactgg cttcaaaaat gctgtatgaa tttattctgg aaaggagaat gactttaact    600
gatagcattg aacgctgcct gaaaaaaggt aagagtgatg agcaacgtgc agctgcagcg    660
ttagcatctg ttctttgtat tcagctgggc cctggaattg aaagtgaaga gattttgaaa    720
actcttggac caatcctaaa gaaaatcatt tgtgatgggt cagctagtat gcaggctagg    780
caaacttgtg caacttgctt tggtgtttgc tgttttattg ccacagatga cattactgaa    840
ctatactcaa ctctggaatg tttggaaaat atcttcacta aatcctatct caaagagaaa    900
gacactactg ttatttgcag cactcctaat acagtgcttc atatcagctc tcttcttgca    960
tggacactac tgctgaccat atgcccaatc aatgaagtga agaaaaagct tgagatgcat   1020
ttccataagc ttccaagcct cctctcttgt gatgatgtaa acatgagaat agctgctggt   1080
gaatctttgg cacttctctt tgaattggcc agaggaatag agagtgactt tttttatgaa   1140
gacatggagt ccttgacgca gatgcttagg gccttggcaa cagatggaaa taaacaccgg   1200
gccaaagtgg acaagagaaa gcagcggtca gttttcagag atgtcctgag ggcagtggag   1260
gaacgggatt ttccaacaga aaccattaaa tttggtcctg aacgcatgta tattgattgc   1320
tgggtaaaaa aacacaccta tgacaccttt aaggaggttc ttggatcagg gatgcagtac   1380
ccacttgcag tcaaaatgga attcctcgaa aatgtatttg aaacttggac cccccagtgat   1440
gccttgatgc tgcaacgcct taaaacgatg aagatttctc gtttcgaaag gcatttatat   1500
aactctgcag ccttcaaagc tcgaaccaaa gctagaagca aatgtcgaga taagagagca   1560
gatgttggag aattcttcta gattttcaga acttgaagac tatttctaa tttctatttt   1620
tttttctatt tcaatgtatt taaactctag acacagtttt tatcttggat taacttagat   1680
aacttttgta gccagtggtt atattgctta taatttaatg tacaatacta ttgaaactgg   1740
tgagttctga ttattaaata ttctctgtaa atcagtaaac atgtataaag t             1791
```

<210> 20
<211> 1299
<212> DNA
<213> Homo sapiens
<400> 20

```
agtcctcttc cgggtgatgg cggcgggtgc cccggatgta gccctggcgc aagcatctct     60
tcttttttcc acctcgcctt ccgcggattc ccagcttgag aaacacctct ttgccccgtc    120
atgccaaaga ggaaagtgac cttccaaggc gtgggagatg aggaggatga ggatgaaatc    180
attgtcccca agaagaagct ggtggaccct gtggctgggt caggggggtcc tgggagccgc    240
tttaaaggca aacactcttt ggatagcgat gaggaggagg atgatgatga tgggggggtcc    300
agcaaatatg acatcttggc ctcagaggat gtagaaggtc aggaggcagc cacactcccc    360
agcgaggggg gtgttcggat cacacccttt aacctgcagg aggagatgga ggaaggccac    420
tttgatgccg atggcaacta cttcctgaac cgggatgctc agatccgaga cagctggctg    480
gacaacattg actgggtgaa gatccgggag cggccacctg gccagcgcca ggcctcagac    540
tcggaggagg aggacagctt gggccagacc tcaatgagtg cccaagccct cttggaggga    600
cttttggagc tcctattgcc tagagagaca gtggctgggg cactgaggcg tctgggggcc    660
cgaggaggag gcaaagggag aaaggggcct gggcaaccca gttcccctca gcgcctggac    720
cggctctccg ggttggccga ccagatggtg gcccggggca accttggtgt gtaccaggaa    780
acaagggaac ggttggctat gcgtctgaag ggtttggggt gtcagaccct aggaccccac    840
aatcccacac ccccaccctc cctggacatg ttcgctgagg agttggcgga ggaggaactg    900
```

gagaccccaa cccctaccca gagaggagaa gcagagtcgc ggggagatgg tctggtggat 960
gtgatgtggg aatataagtg ggagaacacg gggatgccg agctgtatgg gccccttcacc 1020
agcgcccaga tgcagacctg ggtgagtgaa ggctacttcc cggacggtgt ttattgccgg 1080
aagctggacc cccctggtgg tcagttctac aactccaaac gcattgactt tgacctctac 1140
acctgagcct gctgggggcc cagtttggtg ggccccttctt tcctggactt tgtggaggag 1200
gcaccaagtg tctcaggcag cgaggaaatt ggaggccatt tttcagtcaa tttccctttc 1260
ccaataaaag cctttagttg tgtaaaaaaa aaaaaaaaa 1299

<210> 21
<211> 900
<212> DNA
<213> Homo sapiens
<400> 21
gggactcggc ggaggatggg cttgctctca attttgcgca agttgaaaag tgcaccagac 60
caggaggtga gaatacttct cctgggcttg gataatgctg gcaagaccac tcttctgaag 120
cagcttgcat ctgaagacat cagccacatc acacctacac aggtttcaa catcaaaagt 180
gtacaatcac aaggttttaa actgaatgta tgggacattg gtggacagag gaaaatcaga 240
ccatactgga agaattattt tgaaaatacc gatattctta tatatgtaat cgacagtgca 300
gacagaaaaa gatttgaaga gacgggtcag gaactagcgg aattactgga ggaagaaaaa 360
ctaagttgtg tgccagtgct catctttgct aataagcagg atttgctcac agcagcccct 420
gcctctgaaa ttgcagaagg actgaacctg cataccatcc gcgaccgagt ctggcagatc 480
cagtcttgct cagctctcac aggagagggc gttcaggatg gcatgaactg ggtctgcaaa 540
aatgtcaatg caaagaagaa ataaaatcta gacgaatgga gatgcaggag ctgcgggagc 600
cgaattcggg ccttaaaaac actaatttgc tgctttctga ccaaatgttt ttcatctgtg 660
tacactccag ctgtttgaag agagggaaca acacggttta gaaagaatcc ccattccagc 720
agtagattta actgatctct gaggttcagt atcatttttc aaataaagga attatattat 780
ttcctctgca taattgaaat agtattaaat gtctaaagca catgattaga aaatgagatc 840
ttttaaatga gcaagagatt gcattgcagt ttagacaatt ccagtgggct tttttttcg 900

<210> 22
<211> 1530
<212> DNA
<213> Homo sapiens
<400> 22
aatcctggaa caaggctaca gcgtcgaaga tccccagcgc tgcgggctcg gagagcagtc 60
ctaacggcgc ctcgtacgct agtgtcctcc cttttcagtc cgcgtccctc cctgggccgg 120
gctggcactc ttgccttccc cgtccctcat ggcgctgctc cgacgcccga cggtgtccag 180
tgatttggag aatattgaca caggagttaa ttctaaagtt aagagtcatg tgactattag 240
gcgaactgtt ttagaagaaa ttggaaatag agttacaacc agagcagcac aagtagctaa 300
gaaagctcag aacaccaaag ttccagttca acccaccaaa acaacaaatg tcaacaaaca 360
actgaaacct actgcttctg tcaaaccagt acagatggaa aagttggctc caaagggtcc 420
ttctccccaca cctgaggatg tctccatgaa ggaagagaat ctctgccaag ctttttctga 480
tgccttgctc tgcaaaatcg aggacattga taacgaagat tgggagaacc ctcagctctg 540
cagtgactac gttaaggata tctatcagta tctcaggcag ctggaggttt tgcagtccat 600
aaacccacat ttcttagatg gaagagatat aaatggacgc atgcgtgcca tcctagtgga 660
ttggctggta caagtccact ccaagtttag gcttctgcag gagactctgt acatgtgcgt 720
tggcattatg gatcgatttt tacaggttca gccagtttcc cggaagaagc ttcaattagt 780
tgggattact gctctgctct tggcttccaa gtatgaggag atgttttctc caaatattga 840
agactttgtt tacatcacag acaatgctta taccagttcc caaatccgag aaatggaaac 900
tctaattttg aaagaattga aatttgagtt gggtcgaccc ttgccactac acttcttaag 960
gcgagcatca aaagccgggg aggttgatgt tgaacagcac actttagcca agtatttgat 1020
ggagctgact ctcatcgact atgatatggt gcattatcat ccttctaagg tagcagcagc 1080
tgcttcctgc ttgtctcaga aggttctagg acaaggaaaa tggaacttaa agcagcagta 1140
ttacacagga tacacagaga atgaagtatt ggaagtcatg cagcacatgg ccaagaatgt 1200
ggtgaaagta aatgaaaact taactaaatt catcgccatc aagaataagt atgcaagcag 1260
caaactcctg aagatcagca tgatccctca gctgaactca aaagccgtca aagaccttgc 1320
ctccccactg ataggaaggt cctaggctgc cgtgggccct ggggatgtgt gcttcattgt 1380
gccctttttc ttattggttt agaactcttg attttgtaca tagtcctctg gtctatctca 1440
tgaaacctct tctcagacca gttttctaaa catatattga ggaaaaataa agcgattggt 1500
ttttcttaag gtaaaaaaaa aaaaaaaaaa 1530

<210> 23
<211> 2863
<212> DNA
<213> Homo sapiens
<400> 23
cggaattcaa gaaacacaaa atgcagtggg cgtccctcct gctgctggca gggctcttct 60
ccctctccca ggcccagtat gaagatgacc ctcattggtg gttccactac ctccgcagcc 120
agcagtccac ctactacgat ccctatgacc cttacccgta tgagacctac gagccttacc 180

```
cctatggggt ggatgaaggg ccagcctaca cctacggctc tccatcccct ccagatcccc    240
gcgactgccc ccaggaatgc gactgcccac ccaacttcct cacggccatg tactgtgaca    300
atcgcaacct caagtacctg cccttcgttc cctcccgcat gaagtatgtg tacttccaga    360
acaaccagat cacctccatc caggaaggcg tctttgacaa tgccacaggg ctgctctgga    420
ttgctctcca cggcaaccag atcaccagtg ataaggtggg caggaaggtc ttctccaagc    480
tgaggcacct ggagaggctg tacctggacc acaacaacct gacccggatg cccggtcccc    540
tgcctcgatc cctgagagag ctccatctcg accacaacca gatctcacgg gtccccaaca    600
atgctctgga ggggctggag aacctcacgg ccttgtacct ccaacacgat gagatccagg    660
aagtgggcag ttccatgagg ggcctccggt cactgatctt gctggacctg agttataacc    720
accttcggaa ggtgcctgat gggctgccct cagctcttga gcagctgtac atggagcaca    780
acaatgtcta caccgtcccc gatagctact tccggggggc gcccaagctg ctgtatgtgc    840
ggctgtccca caacagtcta accaacaatg gcctggcctc caacaccttc aattccagca    900
gcctccttga gctagacctc tcctacaacc agctgcagaa gatcccccca gtcaacacca    960
acctggagaa cctctacctc caaggcaata ggatcaatga gttctccatc agcagcttct   1020
gcaccgtggt ggacgtcgtg aacttctcca agctgcaggt cgtgcgcctg gacgggaacg   1080
agatcaagcg cagcgccatg cctgccgacg cgcccctctg cctgcgcctt gccagcctca   1140
tcgagatctg agcagccctg gcaccgggta ctgggcggag agcccccgtg gcatttggct   1200
tgatggtttg gtttggctta tggaagatct gggacagacc gtgtgacaga agtccacggg   1260
caccctctgt agtcttcttt cctgtaggtg gggttagggg gggcgatcag ggacaggcag   1320
ccttctgctg aggacatagg cagaagctca ctcttttcca gggacagaag tggtggtaga   1380
tggaaggatc cctggatgtt ccaaccccat aaatctcacg gctcttaagt tcttcccaat   1440
gatctgaggt catggaactt caaaagtggc atgggcaata gtatataacc atacttttct   1500
aacaatccct ggctgtctgt gagcagcact tgacagctct ccctctgtgc tgggctggtc   1560
gtgcagttac tctgggctcc catttgttgc ttctcaaaat atacctcttg cccagctgcc   1620
tcttctgaaa tccacttcac ccactccact ttcctccaca gatgcctctt ctgtgcctta   1680
agcagagtca ggagacccca aggcatgtga gcatctgccc agcaacctgt ggagacaacc   1740
cacactgtgt ctgagggtga aaggacacca ggagtcactt ctatacctcc ctaacctcac   1800
ccctggaaag ccaccagatt ggaggtcacc agcatgatga taatattcat gacctgatgt   1860
gggaggagac agccaacctc aggcttagat caatgtatag ggctatattt tggcagctgg   1920
gtagctcttt gaaggtggat aagacttcag aagaggaaag gccagacttt gcttaccatc   1980
agcatctgca atgggccaaa cacacctcaa attggctgag ttgagaaagc agccccagta   2040
gttccattct tgcccagcac tttctgcatt ccaaacagca tcctacctgg gtttttatcc   2100
acaaaggtag cggccacatg gtttttaaag tatgagaaac acagtttgtc ctctcctttt   2160
atccaagcag gaagattcta tatcctgatg gtagagacag actccaggca gccctggact   2220
tgctagccca aagaaggagg atgtggttaa tctgtttcac ctggtttgtc ctaaggccat   2280
agttaaaaag taccagctct ggctggggtc cgtgaagccc aggccaggca gccaaatctt   2340
gcctgtgctg ggcatacaac cctctgcttt cacatctctg agctatatcc tcattagtga   2400
aggtggcttt tgctttatag tttggctggg gagcacttaa ttcttcccat ttcaaaaggt   2460
aatgttgcct ggggcttaac ccacctgccc tttgggcaag gttgggacaa agccatctgg   2520
gcagtcaggg gcaaggactg ttggaggaga gttagcccaa gtataggctc tgcccagatg   2580
ccatcacatc cctgatactg tgtatgcttt gaagcacctt ccctgagaag ggaagagggg   2640
atctttggac tacgttcttg gctccagacc tggaatccac aaaagccaaa ccagctcatt   2700
tcaacaaagg agctccgatg tgaggggcaa ggctgccccc tgccccaggg ctcttcagaa   2760
agcatctgca tgtgaacacc atcatgcctt tataaaggat ccttattaca ggaaaagcat   2820
gagtggtggc taacctgacc aataaagtta ttttatgatt gcc                     2863
```

```
<210>  24
<211>  4237
<212>  DNA
<213>  Homo sapiens
<400>  24
```

```
tcccgaaacc agagggatgg ggccggctgt gcagtagaac ggggatcgaa aagaggaaaa     60
caagggcacg aagaccagcg agaaagaaga ggacacctgg gaaaggcgga agcagaagac    120
ggggaaggga aaagaaaccc atagcaggtg gaaaccagat ctagagcaac accgtcaggt    180
tcacagtttg tttttctaga agagaagaaa gtacctgagg attgctcttt tttcctaccg    240
ttaatgaaaa ctacttttgt cttcatcata aaagaaaaaa ctaaggggag gtaaaggcag    300
tctcctgttt tattaggggg agaggtgaag ggaaatccag gctcactttc tgaataagcc    360
actgcctggt gcacagagca gaaccatcct ggtttctgaa gacacatccc tttcagcaga    420
attccagccg gagtcgctgg cacagttcta tttttatatt taaatgtatg tctcccctgg    480
cctttttttt tttttttttt tttagcaaca cttttcttgt ttgtaaacgc gagtgaccag    540
aaagtgtgaa tgcggagtag gaatattttt cgtgttctct tttatctgct tgcctttttt    600
agagagtagc agtggttcct atttcggaaa aggacgttct aattcaaagc tctctcccaa    660
tatatttaca cgaatacgca tttagaaagg gaggcagctt ttgaggttgc aatcctactg    720
agaaggatgg aagaaggagc caggcaccga aacaacaccg aaaagaaaca cccaggtggg    780
ggcgagtcgg acgccagccc cgaggctggt tccggagggg gcggagtagc cctgaagaaa    840
gagatcggat tggtcagtgc ctgtggtatc atcgtaggga acatcatcgg ctctggaatc    900
tttgtctcgc caaagggagt gctggagaat gctggttctg tgggccttgc tctcatcgtc    960
tggattgtga cgggcttcat cacagttgtg ggagccctct gctatgctga actcggggtc   1020
accatcccca aatctggagg tgactactcc tatgtcaagg acatcttcgg aggactggct   1080
```

gggttcctga ggctgtggat tgctgtgctg gtgatctacc ccaccaacca ggctgtcatc 1140
gccctcacct tctccaacta cgtgctgcag ccgctcttcc ccacctgctt ccccccagag 1200
tctggccttc ggctcctggc tgccatctgc ttattgctcc tcacatgggt caactgttcc 1260
agtgtgcggt gggccacccg ggttcaagac atcttcacag ctgggaagct cctggccttg 1320
gccctgatta tcatcatggg gattgtacag atatgcaaag gagagtactt ctggctggag 1380
cccaaagaatg catttgagaa tttccaggaa cctgacatcg gcctcgtcgc actggctttc 1440
cttcagggct cctttgccta tggaggctgg aactttctga attacgtgac tgaggagctt 1500
gttgatccct acaagaacct tcccagagcc atcttcatct ccatcccact ggtcacattt 1560
gtgtatgtct ttgccaatgt cgcttatgtc actgcaatgt ccccccagga gctgctggca 1620
tccaacgccg tcgctgtgac ttttggagag aagctcctag gagtcatggc ctggatcatg 1680
cccatttctg ttgccctgtc cacatttgga ggagttaatg ggtctctctt cacctcctct 1740
cggctgttct tcgctggagc ccgagagggc caccttccca gtgtgttggc catgatccac 1800
gtgaagcgct gcaccccaat cccagccctg ctcttcacat gcatctccac cctgctgatg 1860
ctggtcacca gcgacatgta cacactcatc aactatgtgg gcttcatcaa ctacctcttc 1920
tatggggtca cggttgctgg acagatagtc cttcgctgga agaagcctga tatcccccgc 1980
cccatcaaga tcaacctgct gttccccatc atctacttgc tgttctgggc cttcctgctg 2040
gtcttcagcc tgtggtcaga gccggtggtg tgtggcattg gcctggccat catgctgaca 2100
ggagtgcctg tctatttcct gggtgtttac tggcaacaca agcccaagtg tttcagtgac 2160
ttcattgagc tgctaaccct ggtgagccag aagatgtgtg tggtcgtgta ccccgaggtg 2220
gagcggggct cagggacaga ggaggctaat gaggacatgg aggagcagca gcagcccatg 2280
taccaaccca ctcccacgaa ggacaaggac gtggcggggc agccccagcc ctgaggacca 2340
ccattccctg gctactctct ccttcctccc cctttttatcc tacctccctg ccttggtcct 2400
gccaacacat gcgagtacac acacacccct ctctctgctt ttgtcaggca gtggtaggac 2460
tttggtgtgg gtggtgagaa attgtaaaca aaaactgaca ttcataccca aagaaccagc 2520
ctctcacccc agggtccatg tcccaggccc cactccagtg ctgcccacac tcccagctgc 2580
tggaggagag gggagatgcc aaggtgccct gcaggacctc cctccgggcc acaccctcag 2640
ctgcctcttc aggaaccgga gctcattact gccttccctc ccagggaggc cccttcagag 2700
aggagaggcc acaggagctg cattgtgggg ggacaggctc aagcaattct gtccccatca 2760
aggggtcagc tggagagacc caagacccta tctgttcacc agggacccaa aatccaaggg 2820
gatgcttccc tctgccctct ttcctgcccc tccccatcat acctgcaccc accccagcca 2880
gggctccctg tccagaattc ggttctcctc aggacgccaa ctcccagagc taaggaccaa 2940
ggagaagaac agcctctcca cccccaagcc aggcggttga ggaacatatt gagaaaggtt 3000
cagattgcag aaacccagcc ctgccctgc ctcctgcatc cagcccccaa catggtgcca 3060
aagcttccag aagccaaaaa gcttctgatt tttaaggtag tgggcatctc tctcctaatg 3120
acgaagctgc tcagcaactc cacctgcccg ccgcaggaag gagcagtccc ctgctatccc 3180
tgcagccact cccagcacac ccgcacacag ccagcaccac cgccccccacc gtgcacttct 3240
cctctctggg ccttggcttg ggaccaggta cgaaggatcc ccaagccctt caggcctgag 3300
atcagagcca gatcagcctt aagtcacctc ccatccaaga acttggccta aaaatactcc 3360
cctatttcta accctcagga cggatctgat attaaatgcc ttccctggga ggaagggtgc 3420
tttccccctc cctagaggtg cccattccat accctgggag actgaggaga gcattggctg 3480
aagcccagtt cctttcccat ccatccccaa ctccaataat cccccactcc tcgcaggtct 3540
cagtgtcatg ctgtcttggg gcagggtgaa agggtagtgg cagcagggcg cccactctgg 3600
agatcctcaa aaaaggccct cctctgtggc tggcagcctc tgacctttcc ctgggcttca 3660
aaggaaggct atggagtttg ctgtgggccc tgcaaccttc ccagccactc ctgctgcact 3720
aaggacttag gatcctttta tcacaaatcg ggattctctc cccccacccg aattctgtct 3780
gcttaaactg gaatacacag gagcccttcc tggcctggat ggtgtctccc agcttccccg 3840
cccagcttgc ccaccccata gttggtgaga tgccaagttt ggtctgagtt gtgacccctt 3900
cagagtagat gcccggcagg ctggggttgg cccctggagg gtcaggggac catcttctta 3960
ttccctcttt tctcattcct ccaacttcct cccctccttc aattattttt ttgtaaagtt 4020
gatgccttac tttttggata aatattttttg aagctggtat ttctatttct tttggatttt 4080
ttttaatgta aggttgtttt ggggatgga gttagaacct taatgataat ttctttcgtt 4140
tggtgtaggt tttagagatt tgttttgtgg agaggttttt ttcttttgat gtaataaaat 4200
ttaaaatgga aatgaaaaaa aaaaaaaaaa aaaaaaa 4237

<210> 25
<211> 890
<212> DNA
<213> Homo sapiens
<400> 25
ggcggaccga agaacgcagg aaggggggccg gggggacccg cccccggccg gccgcagcca 60
tgaactccaa cgtggagaac ctaccccccgc acatcatccg cctggtgtac aaggaggtga 120
cgacactgac cgcagaccca cccgatggca tcaaggtctt tcccaacgag gaggacctca 180
ccgacctcca ggtcaccatc gagggccctg aggggacccc atatgctgga ggtctgttcc 240
gcatgaaact cctgctgggg aaggacttcc ctgcctcccc acccaagggc tacttcctga 300
ccaagatctt ccacccgaac gtgggcgcca atggcgagat ctgcgtcaac gtgctcaaga 360
gggactggac ggctgagctg ggcatccgac acgtactgct gaccatcaag tgcctgctga 420
tccaccctaa ccccgagtct gcactcaacg aggaggcggg ccgcctgctc ttggagaact 480
acgaggagta tgcggctcgg gcccgtctgc tcacagagat ccacgggggc gccggcgggc 540
ccagcggcag ggccgaagcc ggtcgggccc tggccagtgg cactgaagct tcctccaccg 600

accctggggc cccaggggc ccgggagggg ctgagggtcc catggccaag aagcatgctg 660
gcgagcgcga taagaagctg gcggccaaga aaaagacgga caagaagcgg gcgctgcggg 720
cgctgcggcg gctgtagtgg gctctcttcc tccttccacc gtgaccccaa cctctcctgt 780
cccctccctc caactctgtc tctaagttat ttaaattatg gctggggtcg gggagggtac 840
agggggcact gggacctgga tttgtttttc taaataaagt tggaaaagca 890

<210> 26
<211> 2099
<212> DNA
<213> Homo sapiens
<400> 26
aagaagctgc cgttgttctg ggtactacag cagaagggta tgcggaagcg agcaccccag 60
tctgagatgg ctcctgccgg tgtgagcctg agggccacca tcctctgcct cctggcctgg 120
gctggcctgg ctgcaggtga ccgggtgtac atacacccct tccacctcgt catccacaat 180
gagagtacct gtgagcagct ggcaaaggcc aatgccggga agcccaaaga ccccaccttc 240
atacctgctc caattcaggc caagacatcc cctgtggatg aaaaggccct acaggaccag 300
ctggtgctag tcgctgcaaa acttgacacc gaagacaagt tgagggccgc aatggtcggg 360
atgctggcca acttcttggg cttccgtata tatggcatgc acagtgagct atggggcgtg 420
gtccatgggg ccaccgtcct ctccccaacg gctgtctttg gcaccctggc ctctctctat 480
ctgggagcct tggaccacac agctgacagg ctacaggcaa tcctgggtgt tccttggaag 540
gacaagaact gcacctcccg gctggatgcg cacaaggtcc tgtctgccct gcaggctgta 600
cagggcctgc tagtggccca gggcagggct gatagccagg cccagctgct gctgtccacg 660
gtggtgggcg tgttcacagc cccaggcctg cacctgaagc agccgtttgt gcagggcctg 720
gctctctata cccctgtggt cctcccacgc tctctggact tcacagaact ggatgttgct 780
gctgagaaga ttgacaggtt catgcaggct gtgacaggat ggaagactgg ctgctccctg 840
atgggagcca gtgtggacag caccctggct ttcaacacct acgtccactt ccaagggaag 900
atgaagggct tctccctgct ggccgagccc caggagttct gggtggacaa cagcacctca 960
gtgtctgttc ccatgctctc tggcatgggc accttccagc actggagtga catccaggac 1020
aacttctcgg tgactcaagt gcccttcact gagagcgcct gcctgctgct gatccagcct 1080
cactatgcct ctgacctgga caaggtggag ggtctcactt tccagcaaaa ctccctcaac 1140
tggatgaaga aactgtctcc ccggaccatc cacctgacca tgccccaact ggtgctgcaa 1200
ggatcttatg acctgcagga cctgctcgcc caggctgagc tgcccgccat tctgcacacc 1260
gagctgaacc tgcaaaaatt gagcaatgac cgcatcaggg tgggggaggt gctgaacagc 1320
atttttttttg agcttgaagc ggatgagaga gagcccacag agtctaccca acagcttaac 1380
aagcctgagg tcttggaggt gaccctgaac cgcccattcc tgtttgctgt gtatgatcaa 1440
agcgccactg ccctgcactt cctgggccgc gtggccaacc cgctgagcac agcatgaggc 1500
cagggcccca gaacacagtg cctggcaagg cctctgcccc tggcctttga ggcaaaggcc 1560
agcagcagat aacaaccccg gacaaatcag cgatgtgtca cccccagtct cccacctttt 1620
cttctaatga gtcgactttg agctggaaag cagccgtttc tccttggtct aagtgtgctg 1680
catggagtga gcagtagaag cctgcagcgg cacaaatgca cctcccagtt tgctgggttt 1740
attttagaga atgggggtgg ggaggcaaga accagtgttt agcgcgggac tactgttcca 1800
aaaagaattc caaccgacca gcttgtttgt gaaacaaaaa agtgttccct tttcaagttg 1860
agaacaaaaa ttgggtttta aaattaaagt atacattttt gcattgcctt cggtttgtat 1920
ttagtgtctt gaatgtaaga acatgacctc cgtgtagtgt ctgtaatacc ttagtttttt 1980
ccacagatgc ttgtgatttt tgaacaatac gtgaaagatg caagcacctg aatttctgtt 2040
tgaatgcgga acaatagctg gttatttctc ccttgtgtta gtaataaacg tcttgccac 2099

<210> 27
<211> 1892
<212> DNA
<213> Homo sapiens
<400> 27
agggtacggg ccgggaccgc cgcagcccgg ggcgggggca cggcaaccgc gaggcctggg 60
ggcgcccgcc ccccgcgccc cacgccccggt gccagcgagc cgaggcgtgc atctccttat 120
atggtcaaat gacacggcgg ggtttctcga gggcgggagc tgcgcagcgc tccactcggc 180
cggcagcgga gccgcagcca ccagccgccc gcgccctcca gccccgtccg ggagtccccg 240
gcccgctgcg gtgccgtgag tacctccaac cccctgcgcc ccggagggag gccgaggggc 300
ttagccacca gggctcggaa gtgggggccg aatccggtgc gagacccaag gagaggggag 360
cagagccgga gttggggaga ctgtggctga aaactgtgtc ttcctggaga ctaggctggc 420
attttgactt tgggacggag tctcgctttg tcgcccaggc tggagtgcag tggcacgatc 480
tcagctcact gcaagctcta cctcttggtt cacgccattc tcctgcccca gcctcccaag 540
tagctgggac tacaggttgc tgaaaagcca ggagtcaaaa tgactgagcg ctttgactgc 600
caccattgca acgaatctct ctttggcaag aagtacatcc tgcgggagga gagcccctac 660
tgcgtggtgt gctttgagac cctgttcgcc aacacctgcg aggagtgtgg gaagcccatc 720
ggctgtgact gcaaggactt gtcttacaag gaccggcact ggcatgaagc ctgtttccac 780
tgctcgcagt gcagaaactc actggtggac aagccctttg ctgccaagga ggaccagctg 840
ctctgtacag actgctattc caacgagtac tcatccaagt gccaggaatg caagaagacc 900
atcatgccag gtacccgcaa gatggagtac aagggcagca gctggcatga gacctgcttc 960
atctgccacc gctgccagca gccaattgga accaagagtt tcatccccaa agacaatcag 1020

aatttctgtg tgccctgcta tgagaaacaa catgccatgc agtgcgttca gtgcaaaaag 1080
cccatcacca cgggaggggt cacttaccgg gagcagccct ggcacaagga gtgcttcgtg 1140
tgcaccgcct gcaggaagca gctgtctggg cagcgcttca cagctcgcga tgactttgcc 1200
tactgcctga actgcttctg tgacttgtat gccaagaagt gtgctgggtg caccaacccc 1260
atcagcggac ttggtggcac aaaatacatc tcctttgagg aacggcagtg gcataacgac 1320
tgctttaact gtaagaagtg ctccctctca ctggtggggc gtggcttcct cacagagagg 1380
gacgacatcc tgtgccccga ctgtgggaaa gacatctgaa ttcaacacag agaagttgct 1440
gcttgtgatc tcacacacag atttttatgt tttctttctc acccaggcaa tcttgccttc 1500
tggtttcttc cagccacatt gagactttct tctagtgctt ttcagtgata ctcacgtttg 1560
cttaaaccct ttagtgcttt gtgatagttc agtcccaggg aaagagaaaa ctcgccctag 1620
gccctaggtg ggaagatggt ttgaaatttt tgtaatcgag taaggcacac ccaaatgtaa 1680
aaatcctttt gaatgatgcc tttataaatc tttctctcac tgtctattta agtgcaatta 1740
acatatgtca cgaacttgaa agttttctaa actcaataag gtaatgacca gttgttattt 1800
acagctctgt aacctcccgt tgcgtcaagt ctaaaccaag attatgtgac ttgcaataaa 1860
gttattcaga acagaaaaaa aaaaaaaaaa aa 1892

<210> 28
<211> 2904
<212> DNA
<213> Homo sapiens
<400> 28
ggaaactggc ggtggccgcg gccgccgagt cggtctgcgc atcctcctgc gttttctcgc 60
ttggatcttg gcactgagag gcggtggccg gcgggatgga gaaaagtagg atgaacctgc 120
ccaaggggcc ggacacgctc tgcttcgaca aggacgagtt catgaaggaa gatttcgatg 180
tcgatcattt tgtgtctgac tgtaggaagc gggtccagct ggaagaactg agagatgacc 240
tggagctcta ctataaactt cttaaaacag ccatggtcga actcatcaac aaggattatg 300
cagattttgt caatctttca acaaacttgg ttggcatgga caaagccctc aaccagcttt 360
ctgtgccttt gggacaatta cgagaagagg ttctgagcct tagatcgtct gtcagtgaag 420
gaattcgggc agttgatgaa cgaatgtcta aacaagagga cattaggaaa aaaaagatgt 480
gtgtattgag gcttatacaa gttattcggt cagttgagaa aattgaaaaa atcttaaact 540
ctcaaagttc taaagaaacc tctgcactag aagcaagcag cccccttttg actggacaaa 600
ttttggagag aattgccaca gaatttaatc agttacagtt tcatgctgtt caaagcaaag 660
gcatgcctct tttggacaaa gtaagaccgc gtatagctgg cattacagcc atgttacagc 720
agtcactgga aggtctccta ttagaaggcc ttcagacgtc tgacgtcgat ataatacggc 780
actgcttgcg gacttacgcc acgattgaca agacacggga cgcggaggcc ttagttggcc 840
aagtactagt gaaaccatac atagacgagg tgattataga gcagtttgtt gaatctcatc 900
ccaatggcct tcaggtcatg tataataaac tcctggagtt tgttcctcac cattgccgcc 960
ttcttcgaga agtcacagga ggtgccatct ccagtgaaaa aggcaatact gttcctggat 1020
atgacttttt ggtgaattct gtttggccac aaatagtaca aggattagaa gaaaagttac 1080
cctcgctttt taatcctggg aatcccgatg catttcatga gaaatatacc ataagtatgg 1140
attttgtcag aagattggaa cggcagtgtg gatcacaggc tagtgtaaag agattaagag 1200
cccatcctgc ctatcacagc ttcaataaga agtggaactt gcctgtttat tttcaaataa 1260
gatttagaga aatagcggga tccttagaag cagcacttac agatgtcctg gaagatgccc 1320
cagctgaaag tccgtattgc cttttggctt ctcatagaac ttggagcagc cttaggaggt 1380
gttggtcaga tgagatgttc ttgccattac tggtgcatcg cctgtggaga ctcactctgc 1440
agattttggc acgatactct gtgtttgtca atgagctttc actcaggccc atttctaatg 1500
aaagtcccaa ggagatcaag aaacctttgg taactggtag caaagaacct tccatcaccc 1560
aaggaaacac tgaagaccaa ggaagtggtc cttcggaaac aaagcctgtg gtttccattt 1620
cccgcactca gctcgtgtat gtggttgcag acctggacaa gcttcaggag cagcttccag 1680
aactcttgga aataatcaag ccaaaacttg aaatgattgg ctttaagaat ttttcttcta 1740
tctcagcagc cctggaggac tcccagagct ctttttcagc ctgtgtgccc tccttgagta 1800
gcaagatcat ccaggattta agtgactctt gcttcggttt cctaaaaagc gccctggagg 1860
ttcccaggct ttaccgaaga accaataagg aggtcccaac cacagcttcc tcctatgtgg 1920
acagtgctct gaagccctta ttccagcttc agagcggaca caaggataag ctcaaacaag 1980
caataattca gcagtggcta gaaggcactc tcagtgaaag cactcataag tactatgaaa 2040
ccgtgtcaga tgtattaaac tctgtgaaga agatggaaga gagcctgaaa aggctgaaac 2100
aagccagaaa aaccactccc gccaaccccg tcggtcccag tggtggcatg agcgacgacg 2160
acaaaatcag gctgcagttg gccctagatg ttgagtactt gggagagcag atacaaaagt 2220
tgggactaca agcaagtgac ataaaaagct tctcagctct cgcagagctt gttgctgctg 2280
ccaaggacca ggcaacagca gagcagcctt aagcatcttg gaagatcccg aggttagatt 2340
cttaagcaag agaagagttg gacttccagg ctgaagggga gaaagtgact ctgttctctt 2400
agcaaccgtc tgtagcaaag aagtgcttcc agcatcactc cagcaacacg cccatgcgtc 2460
ttctctcagc gtatttgggt cttctttgcc caaaagaaca caaaagcctt tttccattgt 2520
atggaagata gtttttaaga catttgaaac tttctactat agtttacaga acaaattatt 2580
ttatttttat tgtaaatctt agtgtggaag agctgatttc taaaatatga ttaaagtaaa 2640
tatataccta tgaatatcaa gagtcgtctc cctgagcctg tagttggaag tgacgactgt 2700
aatggaatga tgtcttgtat agaaatgccc ttctctgaaa taaagagaac tcctgggctt 2760
tctaaagagg ctgcgggaag ccatcctcca ctcccactgt gtgtgagagc agtgcttctg 2820
atcctgctgt caccccgacc tctggcagga gccggcgcca gtaggaaaga cctccttcct 2880

aaataaaaga agtgtctccc aaaa 2904

<210> 29
<211> 3611
<212> DNA
<213> Homo sapiens
<400> 29
aaaaaaaaaa aaaagggcgg ccgctcgcga tctagaacta gtcgtgactg ggaagatggc 60
cgtctttcct tggcactcca ggaataggaa ctacaaagct gaatttgcat catgccgact 120
ggaggctgta ccattggagt ttggggacta tcaccctctg aaacccataa ctgtcacaga 180
gtcaaagaca aagaaagtga accggaaagg aagcacttct tccacgtcct cctcctcctc 240
cagctccgtg gtggacccgc tgagcagcgt cctcgatggg actgaccccc tctccatgtt 300
tgcagccact gctgaccccg cagccttggc agctgccatg gacagctcca gaaggaaacg 360
tgatagagat gataactccg ttgtaggatc ggattttgag ccttggacca acaaacgggg 420
agaaatcctt gcccggtaca ccactaccga aaagctgtct attaatctgt ttatgggatc 480
tgaaaaaggc aaagctggga ctgccacatt ggcaatgtca gagaaggtgc ggacccggct 540
ggaggagctg gatgactttg aggagggttc ccaaaaggag ctgttgaact tgactcagca 600
ggattacgtg aaccgcatag aggagctcaa ccaatcgctg aaggatgcct gggcctcaga 660
ccagaaagtg aaggctctaa aaatagtcat ccagtgttca aagcttcttt cagacaccag 720
tgttattcag ttctacccaa gcaaatttgt ccttatcacc gacatacttg atacatttgg 780
aaagctcgtg tacgagcgca tcttttccat gtgtgtggat agccgcagcg tcttaccaga 840
tcacttttct ccagagaatg caaatgacac ggccaaggaa acatgcctaa attggttttt 900
caagattgcc tccatcaggg aactcattcc aagattttac gtggaggcat ccatcctgaa 960
atgtaacaaa ttcctctcca aaacgggaat ttcagagtgc ctgccccggt tgacatgcat 1020
gatcagaggg atcggagacc cactagtgtc ggtgtatgcc cgtgcctacc tgtgccgggt 1080
gggaatggaa gtggccccac atctcaaaga aaccctaaat aagaactttt ttgacttcct 1140
ccttacgttc aaacagattc atggggatac ggtccagaac cagctggtgg tccaaggagt 1200
ggagctccca tcttacctcc ccttgtaccc gcctgccatg gactggatct tccagtgcat 1260
ctcctaccat gcccccgagg ctctgctgac cgagatgatg gaaaggtgta agaaactagg 1320
aaacaatgcc ttgctgttga attctgtgat gtctgccttc cggcctgagt tcatcgccac 1380
aaggtctatg gatttcattg gcatgattaa agagtgtgat gaatctggtt tccccaagca 1440
tcttcttttt cgatcactgg gattaaactt ggccttggct gatcctcctg agagtgaccg 1500
acttcagatt ctcaacgaag cttggaaagt catcactaag ctgaagaacc cacaggacta 1560
cattaattgt gccgaagtgt gggtggaata cacctgcaag catttcacga aacgagaggt 1620
gaataccgtt ttggcagatg tcatcaagca catgactcca gatcgtgcat ttgaagattc 1680
ctacccccag cttcagttaa taattaagaa agttattgcc cacttccatg acttctcagt 1740
tcttttctca gtggaaaaat ttctgccgtt tctggacatg ttccaaaaag agagtgtgcg 1800
ggtggaggtt tgcaaatgca tcatggacgc ctttatcaag catcaacaag agcccaccaa 1860
ggacccggtc atcttgaatg ccctttttgca tgtttgcaag accatgcatg actctgtgaa 1920
tgcactcact cttgaggatg agaaaagaat gctgtcatat ttgattaatg gatttataaa 1980
aatggtttcc tttggccgtg attttgaaca acagctgagt ttttatgttg agtccaggtc 2040
gatgttttgc aatctggagc ctgttcttgt gcagttgatt catagtgtga accggttggc 2100
aatggagaca agaaaagtaa tgaaaggaaa tcattccaga aagacagctg catttgtccg 2160
ggcctgtgtt gcctactgct tcatcaccat cccctccctg gcgggcatct tcacacgtct 2220
caatctctac ctgcattctg gtcaggtggc cttggccaac cagtgcctct cccaagctga 2280
tgctttttc aaagccgcta taagccttgt tccggaagtt ccaaagatga ttaatattga 2340
tgggaagatg cggccatcgg aatcgttcct tctggaattc ctctgcaatt tcttttctac 2400
tttattaata gttccggatc atcctgaaca tggggtcctg tttcttgttc gagagcttct 2460
caacgtgatc caggactaca cctgggagga caacagcgat gagaaaatcc gcatctacac 2520
ctgcgtcctg catctcctct ccgccatgag ccaggagacg tacctttacc acatagacaa 2580
agtggactcc aacgacagcc tctacggggg agactccaag ttcctggcag aaaacaacaa 2640
gctgtgtgag acggtgatgg ctcagatcct agagcatctg aaaaccctgg ccaaggacga 2700
ggccctgaag cgccagagct cgttgggcct ttccttcttt aacagcatct tggcccatgg 2760
ggacctacgc aacaacaagc tcaaccagct ctccgtcaac ctgtggcacc tggcacagag 2820
gcacggctgt gcagacacca ggaccatggt gaaaacgcta gaatacatca agaagcaaag 2880
caaacaacca gacatgactc atctgacgga gctggccctc agactccctc tgcaaacaag 2940
gacctgaccc ccgggcccat ccccaggctc agggactctg gtgccaaatc cagaaagatc 3000
tgctctgctg ccctgaactc ttacggcaat ttaggtttct catttttctt ttctttttac 3060
atatgtacaa attgttttaa gctttggcct ctatccaggt tattctgaca atgaagaaat 3120
gggagttgtc agagcattaa aatgcaatct tcactaagaa gcagtctctg tgttgtcttt 3180
gcacaagtgg ccttcggtct actcagcccg atctgatggg ccttttttagc aagagagaaa 3240
caagaatgca agtaacatct ttcttctctg gaaggtgttt gtttttttcat agtttagaaa 3300
taaggacttt aaaagtggac tgcttttcaa agtgccactg ttccagaccc attccattcc 3360
agactttgta ccttaaagtt agagcacacc caaagtctgg aactgtgtta cctgaacccc 3420
tatggaggat ttataaaagg cagaaatagc actccattaa ctcttttttcc tatcaaaagc 3480
agctcttgat tggacttaga atctgtgttg gtggatcaaa ggagaaagcg aggtcaaatt 3540
tgagattctc tgtggcttca gtatacagta actgaataaa tgtcctgaag gagaaaaaaa 3600
aaaaaaaaaa a 3611

<210> 30
<211> 2950
<212> DNA
<213> Homo sapiens
<400> 30

```
gcggcccgta ggaaggtgct gtccgatcga tcgggatagg agcggtccct gcgcttgctg      60
ctgggaagtg gtacaatcat gtttgaaatt aagaagatct gttgcatcgg tgcaggctat     120
gttggaggac ccacatgtag tgtcattgct catatgtgtc ctgaaatcag ggtaacggtt     180
gttgatgtca atgaatcaag aatcaatgcg tggaattctc ctacacttcc tatttatgag     240
ccaggactaa aagaagtggt agaatcctgt cgaggaaaaa atcttttttt ttctaccaat     300
attgatgatg ccatcaaaga agctgatctt gtatttattt ctgtgaatac tccaacaaaa     360
acctatggaa tggggaaagg ccgggcagca gatctgaagt atattgaagc ttgtgctaga     420
cgcattgtgc aaaactcaaa tgggtacaaa attgtgactg agaaaagcac agttccagtg     480
cgggcagcag aaagtatccg tcgcatattt gatgcaaaca caaaacccaa cttgaattta     540
caggtgctgt ccaaccctga gtttctggca gagggaacag ccatcaagga cctaaagaac     600
ccagacagag tactgattgg aggggatgaa actccagagg gccagagagc tgtgcaggcc     660
ctgtgtgctg tatatgagca ctgggttccc agagaaaaga tcctcaccac taatacttgg     720
tcttcagagc tttccaaact ggcagcaaat gcttttcttg cccagagaat aagcagcatt     780
aactccataa gtgctctgtg tgaagcaaca ggagctgatg tagaagaggt agcaacagcg     840
attggaatgg accagagaat tggaaacaag tttctaaaag ccagtgttgg gtttggtggg     900
agctgtttcc aaaaggatgt tctgaatttg gtttatctct gtgaggctct gaatttgcca     960
gaagtagctc gttattggca gcaggtcata gacatgaatg actaccagag gaggaggttt    1020
gcttcccgga tcatagatag tctgtttaat acagtaactg ataagaagat agctattttg    1080
ggatttgcat tcaaaaagga cactggtgat acaagagaat cttctagtat atatattagc    1140
aaatatttga tggatgaagg tgcacatcta catatatatg atccaaaagt acctagggaa    1200
caaatagttg tggatctttc tcatccaggt gtttcagagg atgaccaagt gtcccggctc    1260
gtgaccattt ccaaggatcc atatgaagca tgtgatggtg cccatgctgt tgttatttgc    1320
actgagtggg acatgtttaa ggaattggat tatgaacgca ttcataaaaa aatgctaaag    1380
ccagccttta tcttcgatgg acggcgtgtc ctggatgggc tccacaatga actacaaacc    1440
attggcttcc agattgaaac aattggcaaa aaggtgtctt caaagagaat tccatatgct    1500
ccttctggtg aaattccgaa gtttagtctt caagatccac ctaacaagaa acctaaagtg    1560
tagagattgc cattttttatt tgtgattttt tttttttttt ggtacttcag gatagcaaat    1620
atctatctgc tattaaatgg taaatgaacc aagtgttttt ttttgttttt tttttgagac    1680
agagtctcac tgttgcccag gctggagtgc agtggtgcaa tctcggctca ctgcaagctc    1740
tgcttcccag gttcacgcca ttctcctggc tcagcctccc aagtagctgg gactacaggc    1800
acccgccaca gtgcctgggc taattttttg tatttttagt agagacaggg tttcaccatg    1860
tgagccagga tggtctcaat ctcctgacct tgtgaaccac ccgtctcggc ctcccaaagt    1920
gctgggatta caggtgtgag ccaccacgcc tgggcccatg aaccaagtgt ttttaaggaa    1980
acaaaactat tttttttaatc atcagattta tactagctat atggatatta gcatatctgg    2040
taattatgaa tctagaattt ttttacatat ttttataata ctgttagctc agttattgga    2100
tgagtgaaag ataatcatgt tggttttaat agtgtcaatt tttgtaaaat aaaaattaaa    2160
cttcaaactc tttactttat aaattgtcca taggccacac tttaatatca cattataaag    2220
ggaaggacag tcttcattcc tcctggttat tggtttgttt gtcattaaag atatattttg    2280
aatccatgaa attgctatgc taaacagcct ttacatgtat ggtctggtta aagttccttt    2340
gttccttttg ttttaataaa atgtgtcact gatttttttag ctcaaaatca tcactgttaa    2400
tttccagtca ccccaaatat ggttaaaaga tttttttttt taatcatgaa gagaaaatta    2460
gtagcatttc tttctctccc cattatttat tggttttcct cactaatctt ttttttttta    2520
gtccaaaagc caaaaatatt tatcttggtt ttacatttta atttccattc ttaattgtaa    2580
tttttttctt taaataagga aaccaatata atctcatgta taaaaactta aatattttac    2640
aagttacata tagcatcatt ctaaaataag aattttttttt gttttctgtc tgcttttttc    2700
ttatgtctct tgttgagttt tatattttca gtggttattt ttgcttgtgt tagatcatta    2760
ttaaaatata tccaatgtcc ctttgatact tgtgctctgc tgagaatgta cagtttgcat    2820
taaacatccc aggtctcatc cttcaggaat tttgcagttc aatgagaaga gggagacaaa    2880
tataaagatg aggacagaag catctctaca gatgaaaatt acataaataa aacattctcc    2940
atcaacaaaa                                                           2950
```

<210> 31
<211> 1390
<212> DNA
<213> Homo sapiens
<400> 31

```
gggaagtgct gttggagccg ctgtggttgc tgtccgcgga gtggaagcgc gtgcttttgt      60
ttgtgtccct ggccatggcg ctgcagctct cccgggagca gggaatcacc ctgcgcggga     120
gcgccgaaat cgtggccgag ttcttctcat tcggcatcaa cagcatttta tatcagcgtg     180
gcatatatcc atctgaaacc tttactcgag tgcagaaata cggactcacc ttgcttgtaa     240
ctactgatct tgagctcata aaatacctaa ataatgtggt ggaacaactg aaagattggt     300
tatacaagtg ttcagttcag aaactggttg tagttatctc aaatattgaa agtggtgagg     360
tcctggaaag atggcagttt gatattgagt gtgacaagac tgcaaaagat gacagtgcac     420
ccagagaaaa gtctcagaaa gctatccagg atgaaatccg ttcagtgatc agacagatca     480
```

```
cagctacggt gacatttctg ccactgttgg aagtttcttg ttcatttgat ctgctgattt     540
atacagacaa agatttggtt gtacctgaaa aatgggaaga gtcgggacca cagtttatta     600
ccaattctga ggaagtccgc cttcgttcat ttactactac aatccacaaa gtaaatagca     660
tggtggccta caaaattcct gtcaatgact gaggatgaca tgaggaaaat aatgtaattg     720
taattttgaa atgtggtttt cctgaaatca ggtcatctat agttgatatg ttttatttca     780
ttggttaatt tttacatgga gaaaaccaaa atgatactta ctgaactgtg tgtaattgtt     840
cctttatttt tttggtacct atttgactta ccatggagtt aacatcatga atttattgca     900
cattgttcaa aaggaaccag gaggtttttt tgtcaacatt gtgatgtata ttcctttgaa     960
gatagtaact gtagatggaa aaacttgtgc tataaagcta gatgctttcc taaatcagat    1020
gttttggtca agtagtttga ctcagtatag gtagggagat atttaagtat aaaatacaac    1080
aaaggaagtc taaatattca gaatctttgt taaggtcctg aaagtaactc ataatctata    1140
aacaatgaaa tattgctgta tagctccttt tgaccttcat ttcatgtata gttttcccta    1200
ttgaatcagt ttccaattat ttgactttaa tttatgtaac ttgaacctat gaagcaatgg    1260
atatttgtac tgtttaatgt tctgtgatac agaactctta aaaatgtttt ttcatgtgtt    1320
ttataaaatc aagttttaag tgaaagtgag gaaataaagt taagtttgtt ttaaaaaaaa    1380
aaaaaaaaaa                                                           1390
```

```
<210>  32
<211>  1820
<212>  DNA
<213>  Homo sapiens
<400>  32
gagctccaag ctggtttgaa caagccctgg gcatgtttgg cgggaagttg gcttagctcg      60
gctacctgtg gccccgcagt tttgtagtcc ccgccttgtt tctccccaga ggcctctcaa     120
tcctccctcc atgatcttcg catagagcac agtacccctt cacacggagg acgcgatggc     180
tcccaagaaa cgcccagaaa cccagaagac ctccgagatt gtattacgcc ccaggaacaa     240
gaggagcagg agtcccctgg agctggagcc cgaggccaag aagctctgtg cgaagggctc     300
cggtcctagc agaagatgtg actcagactg cctctgggtg gggctggctg gcccacagat     360
cctgccacca tgccgcagca tcgtcaggac cctccaccag cataagctgg gcagagcttc     420
ctggccatct gtccagcagg ggctccagca gtcctttttg cacactctgg attcttaccg     480
gatattacaa aaggctgccc cctttgacag gagggctaca tccttggcgt ggcacccaac     540
tcaccccagc accgtggctg tgggttccaa aggggggagat atcatgctct ggaattttgg    600
catcaaggac aaacccacct tcatcaaagg gattggagct ggagggagca tcactgggct     660
gaagtttaac cctctcaata ccaaccagtt ttacgcctcc tcaatggagg gaacaactag     720
gctgcaagac tttaaaggca acattctacg agtttttgcc agctcagaca ccatcaacat     780
ctggttttgt agcctggatg tgtctgctag tagccgaatg gtggtcacag gagacaacgt     840
ggggaacgtg atcctgctga acatggacgg caaagagctt tggaatctca gaatgcacaa     900
aaagaaagtg acgcatgtgg ccctgaaccc atgctgtgat tggttcctgg ccacagcctc     960
cgtagatcaa acagtgaaaa tttgggacct gcgccaggtt agagggaaag ccagcttcct    1020
ctactcgctg ccgcacaggc atcctgtcaa cgcagcttgt ttcagtcccg atggagcccg    1080
gctcctgacc acggaccaga agagcgagat ccgagtttac tctgcttccc agtgggactg    1140
cccccctgggc ctgatcccgc accctcaccg tcacttccag cacctcacac ccatcaaggc   1200
agcctggcat cctcgctaca acctcattgt tgtgggccga tacccagatc ctaatttcaa    1260
aagttgtacc ccttatgaat tgaggacgat cgacgtgttc gatggaaact cagggaagat    1320
gatgtgtcag ctctatgacc cagaatcttc tggcatcagt tcgcttaatg aattcaatcc    1380
catgggggac acgctggcct ctgcaatggg ttaccacatt ctcatctgga gccaggagga    1440
agccaggaca cggaagtgag agacactaaa gaaggtgtgg gccagacaag gccttggagc    1500
ccacacatgg gatcaagtcc tgcaagcaga ggtggtgatt tgttaaaggg ccaaaagtat    1560
ccaaggttag ggttggagca ggggtgctgg gacctggggc actgtgggac tgggacactt    1620
ttatgttaat gctctggact tgcctccaga gactgctcca gagttggtga cacagctgtc    1680
ccaagggccc ctctgtatct agcctggaac caaggttatc ttggaactaa atgacttttc    1740
tcctctcagt gggtggtagc agagggatca agcagttatt tgatttgtgc tcttttgata    1800
tggccaataa aaccataccg                                                1820
```

```
<210>  33
<211>  4833
<212>  DNA
<213>  Homo sapiens
<400>  33
gccatttcct cctcttgttt tcactccgga ttctccatgt tggacccaaa ctgaggagcc      60
cggagctgcc gctgggggat cggggccggg ggcacccggg ggagccgctg cccgggccgc     120
ccgccccttg tacaggccgc ctcccttccc ggtccgggga ggaaacgaga gggggggatgt    180
gaacagctgt ggaagtcgga gtctcgggag ccggagcggg cccccgccca ggcccccccag    240
cccagcccag cccgcgcgcc cgcccgtcct cccgtccagc cagcccgggc ccgcgggatt     300
gttagatgga acacggctcc atcatcaccc aggcgcggag ggaagacgcc ctggtgctca     360
ccaagcaagg cctggtctcc aagtcctctc ctaagaagcc tcgtggacgt aacatcttca     420
aggccctttt ctgctgtttt cgcgcccagc atgttggcca gtcaagttcc tccactgagc     480
tcgctgcgta taaggaggaa gcaaacacca ttgctaagtc ggatctgctc cagtgtctcc     540
agtaccagtt ctaccagatc ccagggacct gcctgctccc agaggtgaca gaggaagatc     600
```

aaggaaggat ctgtgtggtc attgacctcg atgaaaccct tgtgcatagc tcctttaagc 660
caatcaacaa tgctgacttc atagtgccta tagagattga ggggaccact caccaggtgt 720
atgtgctcaa gaggccttat gtggatgagt tcctgagacg catgggggaa ctctttgaat 780
gtgttctctt cactgccagc ctggccaagt atgccgaccc tgtgacagac ctgctggacc 840
ggtgtggggt gttccgggcc cgcctattcc gtgagtcttg cgtgttccac cagggctgct 900
acgtcaagga cctcagccgc ctggggaggg acctgagaaa gaccctcatc ctggacaact 960
cgcctgcttc ttacatattc caccccgaga atgcagtgcc tgtgcagtcc tggtttgatg 1020
acatggcaga cactgagttg ctgaacctga tcccaatctt tgaggagctg agcggagcag 1080
aggacgtcta caccagcctt ggggcagctg cgggcccctt agcctgccct gcttccaagc 1140
gacggccatc ccagtagggg actttcccac actgtgcctt tacgatcagc gtgacagagt 1200
agaagctgga gtgcctcacc acacggcccg gaaacagcgg gaagtaactg gaaagagctt 1260
taggacagct tagatgccga gtgggcgaat gccagaccaa tgatacccag agctacctgc 1320
cgccaacttg ttgagatgtg tgtttgactg tgagagagtg tgtgtttgtg tgtgtgtttt 1380
gccatgaact gtggccccag tgtatagtgt ttcagtgggg gagaagctga aagaccaaga 1440
ctcttcccaa gttagcttgt ctcctctcct gtcaccctaa gagccactga gttgtgtagg 1500
gatgaaract attgaagact ccattgccaa accatggcct ttcctcagtg ttgtaaggcc 1560
tatgccaagg ataaaggaag ggtatgcctt tgggtactcc aggcatacac ctttctgaaa 1620
tccttctcca gccagctgct gcagacaaaa gatcacattt ctgggaagat gagaacttgt 1680
ttccagacca gcatccagtg gccatcaggt cttgtggccc aaaggctatg cttgcctccg 1740
gctgagtgcc tgggataggc cttttctatg tctccccaag gctggggtgc tgagcctgcc 1800
ttcctcacca cctagccata gtctcaaacc tgtggggaag gaggttttct ccctgcccgg 1860
gaagaggaca gataactgat ttccgttctt ttgactgtgt tttaaaattc tctttctaaa 1920
cacagagtgt tgggcctggt ttgtttctga caaagttaca gtcctgggcc tgtaatgaat 1980
gtcggcggcg ctggggttgc agggaaaaga caaatcctca aagcgtggac gtgtgtcccc 2040
atggcttgtg gatcagctaa gctcgggatc atttccataa gtctgctttt caggggattct 2100
ctgctggtgc tggtgcaagg acttctgttc caaaggctgg gaaaaactaa gctgtcccag 2160
cccctcccat ttcttgggca gggctctttt cctgttgtgt cttcccccag ggcctgtcct 2220
gtaccgagct ctgtctgttc cagcctacat ccttcctggg tgttgctttt cctcttaagg 2280
gcctcagaac tcttgctctt cctggggtga ggggaatga gtgttcttga catgtgacag 2340
cctaatgcgc atgctttctg cctctggtaa caggagtgag tgagcccctc agacctgcac 2400
tctgggtgtc tcctgcttac aaaggttctt aatagtgaat gctttaaaat taaagtcatc 2460
acgaaatgga agttttccca gggtggaaaa taagaggaag tgctgctgta attgggagca 2520
caagggggcct cccaaaaagg agccccacct cagcatcact gccttaatcg tggcctccct 2580
ggggtgggtg gggttctctc ctccctccct ccctcctcct ggggtgggag ggcgctcctg 2640
ttcccatctc tgtgttccct ggaggcaggt atcacaaagc atttgtgaat tgctttaggt 2700
gcagggacac cacccactca ggactcttcc ccatcatccc ttccattgcc acaccctaga 2760
tccagcctca ggaactaaca agttktgaga aaagcaggtg gtagagcagc agcttcgtgc 2820
tctcagcggt ggctggctgg cattttttctc tagcgttgtg gtgccacctt cccttcttgt 2880
cccaaggtta taaggccttg tctttctctt tggaatcata aagtggaaca gagtccccag 2940
aactcatgtg ghcatttccg acagcatcac tccccggtgc ctatggggtc ccggtgtacc 3000
taaagggaga aggaccccat gtgctagcca gaaatatact gtctcttgaa ggaaagcagg 3060
agctcagact cttagagcca gctgtggctt cggacccaag gcctgaccta ggctgctatc 3120
ctaatattgg aggaggggcc tctcttccaa gccccaccct aagggttagc ccttggacaa 3180
atcttgtgcc gtctaggccc agccaggctt ttctgactaa ataagcaata agaggctcta 3240
agctgactga gttgcaagga ccctttccgc cctcccttgg atctccatgt ttctccagat 3300
ggcggaagag catgtgccac cccctttcct aacagacttg tccaagtgct tggcgtggga 3360
cccatgacca aagcccagga tggcttggtg ggagtgtccc tgctgcatct gcatgaagcc 3420
cctgcttttt aggcctcact cccatcagaa ccctgcctgc ccacctgcaa ctcccccccca 3480
acaatgccat tcccacttgc cccagagaag ctactcggcc aaacctagcc agggtctgtt 3540
cttgtggacc agagccagcc tagtcattat ttgctgtcgg gtttccagtt tcaccgtgtg 3600
ttagggtgag ggatgattgt aaaatttgct cctcaaagga atcaggccag actcaatttt 3660
gggagggcaa gacagggagg aggccgcttc atcccagact ctcttctagg gcttccccacc 3720
atcagcccct cccacttgag actggtcttt gggaggcaat aggccaccat gcctggtcag 3780
caccaattca agccatgcca ggaatctgcc tacctgccag gttcagttct tttaaggtgc 3840
ctcttcaggg acacagtgtg tctctctgat tgggcttcta aatcaaaagc ctgatgttcg 3900
tgtccctctc ataggggggag ctttggacac aggaccagtt tggaaaaggg tcaggtaagg 3960
gtttccactc tgcacattgt agagggaaca ctctgtaggc ccatgggtcc cttactagag 4020
aggttgagtg aatttgcctt cagttaacat gggaccttct gtttagcttc ctcttgcttc 4080
ccaaagattt taagcatttt gtaaatgtat aaactcacct ctggtaacag tggcccagac 4140
gctgctttgt gctaaaagca tgggaaatgt aaaggcagtc tttctctggg aaatggatgc 4200
tattctattc tgctgcccct acctgttcct gaggcctcat ttagaaagaa aatcccctca 4260
gaaggctgtc tggcacccag tgtcctagcc aggccaagta tatgagaaag gtaagtccat 4320
tttccccttc aggtcctcag tggattactt aaccactgct gtccctcggt cccttttttcc 4380
taaacgggtt tagttctgtc ttttttctcc ttttttctaa atgctggtaa atatttacat 4440
tcagccaggg aagaggaggc cagaggtcgg gccagctgcc ccattctttt aacgttgtag 4500
ggcctgccca tggagcggac cctcctcttt gggcctcgtg agcttttttg cttatcatgt 4560
tccatttcgt gccgctttcc cccttcaaga tgccatttgg agggtagggg atctgcttcc 4620
cactgtgact gggctatggg attctgacta ccttgcttac agattcatgg tttgataaat 4680
ttgttgtatt ccaaaacttg aaatgcagga cgccattaag tgtctgttta tatttttgga 4740

```
atatttgtat tacttacaat taattaataa aagtgggttt aaaaaacctt tccaggaaaa   4800
aaaaaaaaaa aaaaaaaaaa aaaaaaaaaa aaa                                4833

<210>  34
<211>  6030
<212>  DNA
<213>  Homo sapiens
<400>  34
gttggccccc gttacttttc ctctgggaaa tatggcgcac gctgggagaa cagggtacga     60
taaccgggag atagtgatga agtacatcca ttataagctg tcgcagaggg gctacgagtg    120
ggatgcggga gatgtgggcg ccgcgccccc gggggccgcc cccgcgccgg gcatcttctc    180
ctcgcagccc gggcacacgc cccatacagc cgcatcccgg gacccggtcg ccaggacctc    240
gccgctgcag accccggctg cccccggcgc cgccgcgggg cctgcgctca gcccggtgcc    300
acctgtggtc cacctgaccc tccgccaggc cggcgacgac ttctcccgcc gctaccgccg    360
cgacttcgcc gagatgtcca ggcagctgca cctgacgccc ttcaccgcgc ggggacgctt    420
tgccacggtg gtggaggagc tcttcaggga cggggtgaac tgggggagga ttgtggcctt    480
ctttgagttc ggtggggtca tgtgtgtgga gagcgtcaac cgggagatgt cgcccctggt    540
ggacaacatc gccctgtgga tgactgagta cctgaaccgg cacctgcaca cctggatcca    600
ggataacgga ggctgggatg cctttgtgga actgtacggc cccagcatgc ggcctctgtt    660
tgatttctcc tggctgtctc tgaagactct gctcagtttg gccctggtgg gagcttgcat    720
caccctgggt gcctatctgg gccacaagtg aagtcaacat gcctgcccca aacaaatatg    780
caaaaggttc actaaagcag tagaaataat atgcattgtc agtgatgttc catgaaacaa    840
agctgcaggc tgtttaagaa aaaataacac acatataaac atcacacaca cagacagaca    900
cacacacaca caacaattaa cagtcttcag gcaaaacgtc gaatcagcta tttactgcca    960
aagggaaata tcatttattt tttacattat taagaaaaaa agatttattt atttaagaca   1020
gtcccatcaa aactcctgtc tttggaaatc cgaccactaa ttgccaagca ccgcttcgtg   1080
tggctccacc tggatgttct gtgcctgtaa acatagattc gctttccatg ttgttggccg   1140
gatcaccatc tgaagagcag acggatggaa aaggacctg atcattgggg aagctggctt   1200
tctggctgct ggaggctggg gagaaggtgt tcattcactt gcatttcttt gccctggggg   1260
ctgtgatatt aacagaggga gggttcctgt gggggaagt ccatgcctcc ctggcctgaa    1320
gaagagactc tttgcatatg actcacatga tgcatacctg gtgggaggaa aagagttggg   1380
aacttcagat ggacctagta cccactgaga tttccacgcc gaaggacagc gatgggaaaa   1440
atgcccttaa atcataggaa agtatttttt taagctacca attgtgccga gaaaagcatt   1500
ttagcaattt atacaatatc atccagtacc ttaagccctg attgtgtata ttcatatatt   1560
ttggatacgc accccccaac tcccaatact ggctctgtct gagtaagaaa cagaatcctc   1620
tggaacttga ggaagtgaac atttcggtga cttccgcatc aggaaggcta gagttaccca   1680
gagcatcagg ccgccacaag tgcctgcttt taggagaccg aagtccgcag aacctgcctg   1740
tgtcccagct tggaggcctg gtcctggaac tgagccgggg ccctcactgg cctcctccag   1800
ggatgatcaa cagggcagtg tggtctccga atgtctggaa gctgatggag ctcagaattc   1860
cactgtcaag aaagagcagt agaggggtgt ggctgggcct gtcaccctgg ggccctccag   1920
gtaggcccgt tttcacgtgg agcatgggag ccacgaccct tcttaagaca tgtatcactg   1980
tagagggaag gaacagaggc cctgggccct tcctatcaga aggacatggt gaaggctggg   2040
aacgtgagga gaggcaatgg ccacggccca ttttggctgt agcacatggc acgttggctg   2100
tgtggccttg gcccacctgt gagtttaaag caaggcttta aatgactttg gagagggtca   2160
caaatcctaa aagaagcatt gaagtgaggt gtcatggatt aattgacccc tgtctatgga   2220
attacatgta aaacattatc ttgtcactgt agtttggttt tatttgaaaa cctgacaaaa   2280
aaaaagttcc aggtgtggaa tatgggggtt atctgtacat cctggggcat taaaaaaaaa   2340
atcaatggtg gggaactata aagaagtaac aaaagaagtg acatcttcag caaataaact   2400
aggaaatttt tttttcttcc agtttagaat cagccttgaa acattgatgg aataactctg   2460
tggcattatt gcattatata ccatttatct gtattaactt tggaatgtac tctgttcaat   2520
gtttaatgct gtggttgata tttcgaaagc tgctttaaaa aaatacatgc atctcagcgt   2580
tttttttgttt ttaattgtat ttagttatgg cctatacact atttgtgagc aaaggtgatc   2640
gttttctgtt tgagattttt atctcttgat tcttcaaaag cattctgaga aggtgagata   2700
agccctgagt ctcagctacc taagaaaaac ctggatgtca ctggccactg aggagctttg   2760
tttcaaccaa gtcatgtgca tttccacgtc aacagaattg tttattgtga cagttatatc   2820
tgttgtccct ttgaccttgt ttcttgaagg tttcctcgtc cctgggcaat tccgcattta   2880
attcatggta ttcaggatta catgcatgtt tggttaaacc catgagattc attcagttaa   2940
aaatccagat ggcaaatgac cagcagattc aaatctatgg tggtttgacc tttagagagt   3000
tgctttacgt ggcctgtttc aacacagacc cacccagagc cctcctgccc tccttccgcg   3060
ggggctttct catggctgtc cttcagggtc ttcctgaaat gcagtggtgc ttacgctcca   3120
ccaagaaagc aggaaacctg tggtatgaag ccagacctcc ccggcgggcc tcagggaaca   3180
gaatgatcag acctttgaat gattctaatt tttaagcaaa atattatttt atgaaaggtt   3240
tacattgtca aagtgatgaa tatggaatat ccaatcctgt gctgctatcc tgccaaaatc   3300
attttaatgg agtcagtttg cagtatgctc cacgtggtaa gatcctccaa gctgctttag   3360
aagtaacaat gaagaacgtg gacgctttta atataaagcc tgttttgtct tctgttgttg   3420
ttcaaacggg attcacagag tatttgaaaa atgtatatat attaagaggt cacgggggct   3480
aattgctggc tggctgcctt ttgctgtggg gttttgttac ctggttttaa taacagtaaa   3540
tgtgcccagc ctcttggccc cagaactgta cagtattgtg gctgcacttg ctctaagagt   3600
agttgatgtt gcattttcct tattgttaaa aacatgttag aagcaatgaa tgtatataaa   3660
```

```
agcctcaact agtcattttt ttctcctctt cttttttttc attatatcta attattttgc   3720
agttgggcaa cagagaacca tccctatttt gtattgaaga gggattcaca tctgcatctt   3780
aactgctctt tatgaatgaa aaaacagtcc tctgtatgta ctcctcttta cactggccag   3840
ggtcagagtt aaatagagta tatgcacttt ccaaattggg gacaagggct ctaaaaaaag   3900
ccccaaaagg agaagaacat ctgagaacct cctcggccct cccagtccct cgctgcacaa   3960
atactccgca agagaggcca gaatgacagc tgacagggtc tatggccatc gggtcgtctc   4020
cgaagatttg gcaggggcag aaaactctgg caggcttaag atttggaata aagtcacaga   4080
atcaaggaag cacctcaatt tagttcaaac aagacgccaa cattctctcc acagctcact   4140
tacctctctg tgttcagatg tggccttcca tttatatgtg atctttgttt tattagtaaa   4200
tgcttatcat ctaaagatgt agctctggcc cagtgggaaa aattaggaag tgattataaa   4260
tcgagaggag ttataataat caagattaaa tgtaaataat cagggcaatc ccaacacatg   4320
tctagctttc acctccagga tctattgagt gaacagaatt gcaaatagtc tctatttgta   4380
attgaactta tcctaaaaca aatagtttat aaatgtgaac ttaaactcta attaattcca   4440
actgtacttt taaggcagtg gctgttttta gactttctta tcacttatag ttagtaatgt   4500
acacctactc tatcagagaa aaacaggaaa ggctcgaaat acaagccatt ctaaggaaat   4560
tagggagtca gttgaaattc tattctgatc ttattctgtg gtgtcttttg cagcccagac   4620
aaatgtggtt acacactttt taagaaatac aattctacat tgtcaagctt atgaaggttc   4680
caatcagatc tttattgtta ttcaatttgg atctttcagg gatttttttt ttaaattatt   4740
atgggacaaa ggacatttgt tggagggtg ggagggagga acaattttta aatataaaac    4800
attcccaagt ttggatcagg gagttggaag ttttcagaat aaccagaact aagggtatga   4860
aggacctgta ttggggtcga tgtgatgcct ctgcgaagaa ccttgtgtga caaatgagaa   4920
acattttgaa gtttgtggta cgacctttag attccagaga catcagcatg gctcaaagtg   4980
cagctccgtt tggcagtgca atggtataaa tttcaagctg gatatgtcta atgggtattt   5040
aaacaataaa tgtgcagttt taactaacag gatatttaat gacaaccttc tggttggtag   5100
ggacatctgt ttctaaatgt ttattatgta caatacagaa aaaaatttta taaaattaag   5160
caatgtgaaa ctgaattgga gagtgataat acaagtcctt tagtcttacc cagtgaatca   5220
ttctgttcca tgtctttgga caaccatgac cttggacaat catgaaatat gcatctcact   5280
ggatgcaaag aaaatcagat ggagcatgaa tggtactgta ccggttcatc tggactgccc   5340
cagaaaaata acttcaagca aacatcctat caacaacaag gttgttctgc ataccaagct   5400
gagcacagaa gatgggaaca ctggtggagg atggaaaggc tcgctcaatc aagaaaattc   5460
tgagactatt aataaataag actgtagtgt agatactgag taaatccatg cacctaaacc   5520
ttttggaaaa tctgccgtgg gccctccaga tagctcattt cattaagttt ttccctccaa   5580
ggtagaattt gcaagagtga cagtggattg catttctttt ggggaagctt tcttttggtg   5640
gttttgttta ttataccttc ttaagttttc aaccaaggtt tgcttttgtt ttgagttact   5700
ggggttattt ttgttttaaa taaaaataag tgtacaataa gtgtttttgt attgaaagct   5760
tttgttatca agattttcat acttttacct tccatggctc tttttaagat tgatactttt   5820
aagaggtggc tgatattctg caacactgta cacataaaaa atacggtaag gatactttac   5880
atggttaagg taaagtaagt ctccagttgg ccaccattag ctataatggc actttgtttg   5940
tgttgttgga aaaagtcaca ttgccattaa actttccttg tctgtctagt taatattgtg   6000
aagaaaaata aagtacagtg tgagatactg                                    6030
```

<210> 35
<211> 5189
<212> DNA
<213> Homo sapiens
<400> 35

```
ggactgcgaa aggagcaggg ttgcggagct agggctccag cctgcggccg cgcattcttg     60
cgtctggcca gccgcgagct ctaagggtcg gccccgcccg gtccgccccc gcggctccct    120
gccaggctct cgcgggcgcg ctcggggtgg ggcctcgcgg ctggcggaga tgcggccggg    180
gctgcgcggt ggtgatgcga gcctgctggg cggcgcgccg gggcagccgg agccgcgcgc    240
cgcggcgctg taatcggaca ccaagagcgc tcgccccccgg cctccggcca ctttccattc   300
actccgaggt gcttgattga gcgacgcgga gaagagctcc gggtgccgcg gcactgcagc    360
gctgagattc ctttacaaag aaactcagag gaccgggaag aaagaatttc acctttgcga    420
cgtgctagaa aataaggtcg tctgggaaaa ggactggaga cacaagcgca tccaaccccg    480
gtagcaaact gatgactttt ccgtgctgat ttctttcaac ctcggtattt tcccttggat    540
attaacttgc atatctgaag aaatggcatt ccggacaatt tgcgtgttgg ttggagtatt    600
tatttgttct atctgtgtga aaggatcttc ccagccccaa gcaagagttt atttaacatt    660
tgatgaactt cgagaaacca agacctctga atacttcagc ctttcccacc atcctttaga    720
ctacaggatt ttattaatgg atgaagatca ggaccggata tatgtgggaa gcaaagatca    780
cattctttcc ctgaatatta acaatataag tcaagaagct ttgagtgttt tctggccagc    840
atctacaatc aaagttgaag aatgcaaaat ggctggcaaa gatcccacac acggctgtgg    900
gaactttgtc cgtgtaattc agactttcaa tcgcacacat ttgtatgtct gtgggagtgg    960
cgctttcagt cctgtctgta cttacttgaa cagagggagg agatcagagg accaagtttt   1020
catgattgac tccaagtgtg aatctggaaa aggacgctgc tctttcaacc ccaacgtgaa   1080
cacggtgtct gttatgatca atgaggagct tttctctgga atgtatatag atttcatggg   1140
gacagatgct gctatttttc gaagtttaac caagaggaat gcggtcagaa ctgatcaaca   1200
taattccaaa tggctaagtg aacctatgtt tgtagatgca catgtcatcc cagatggtac   1260
tgatccaaat gatgctaagg tgtacttctt cttcaaagaa aaactgactg acaataacag   1320
gagcacgaaa cagattcatt ccatgattgc tcgaatatgt cctaatgaca ctggtggact   1380
```

```
gcgtagcctt gtcaacaagt ggaccacttt cttaaaggcg aggctggtgt gctcggtaac    1440
agatgaagac ggcccagaaa cacactttga tgaattagag gatgtgtttc tgctggaaac    1500
tgataacccg aggacaacac tagtgtatgg catttttaca acatcaagct cagttttcaa    1560
aggatcagcc gtgtgtgtgt atcatttatc tgatatacag actgtgttta atgggccttt    1620
tgcccacaaa gaagggccca atcatcagct gatttcctat cagggcagaa ttccatatcc    1680
tcgccctgga acttgtccag gaggagcatt tacacccaat atgcgaacca ccaaggagtt    1740
cccagatgat gttgtcactt ttattcggaa ccatcctctc atgtacaatt ccatctaccc    1800
aatccacaaa aggcctttga ttgttcgtat tggcactgac tacaagtata caaagatagc    1860
tgtggatcga gtgaacgctg ctgatgggag ataccatgtc ctgtttctcg gaacagatcg    1920
gggtactgtg caaaaagtgg ttgttcttcc tactaacaac tctgtcagtg gcgagctcat    1980
tctggaggag ctggaagtct ttaagaatca tgctcctata acaacaatga aaatttcatc    2040
taaaaagcaa cagttgtatg tgagttccaa tgaaggggtt tcccaggtat ctctgcaccg    2100
ctgccacatc tatggtacag cctgtgctga ctgctgcctg gcgcgggacc cttattgcgc    2160
ctgggatggc cattcctgtt ccagattcta cccaactggg aaacggagga gccgaagaca    2220
agatgtgaga catggaaacc cactgactca atgcagagga tttaatctaa aagcatacag    2280
aaatgcagct gaaattgtgc agtatggagt aaaaaataac accacttttc tggagtgtgc    2340
ccccaagtct ccgcaggcat ctatcaagtg gctgttacag aaagacaaag acaggaggaa    2400
agaggttaag ctgaatgaac gaataatagc cacttcacag ggactcctga tccgctctgt    2460
tcagggttct gaccaaggac tttatcactg cattgctaca gaaaatagtt tcaagcagac    2520
catagccaag atcaacttca aagttttaga ttcagaaatg gtggctgttg tgacggacaa    2580
atggtcccca tggacctggg ccagctctgt gagggcttta cccttccacc cgaaggacat    2640
catgggggca ttcagccact cagaaatgca gatgattaac caatattgca aagacactcg    2700
gcagcaacat cagcagggag atgaatcaca gaaaatgaga ggggactatg gcaagttaaa    2760
ggccctcatc aatagtcgga aaagtagaaa caggaggaat cagttgccag agtcataata    2820
ttttcttatg tgggtcttat gcttccatta acaaatgctc tgtcttcaat gatcaaattt    2880
tgagcaaaga aacttgtgct ttaccaaggg gaattactga aaaaggtgat tactcctgaa    2940
gtgagtttta cacgaactga aatgagcatg cattttcttg tatgatagtg actagcacta    3000
gacatgtcat ggtcctcatg gtgcatataa atatatttaa cttaacccag attttattta    3060
tatctttatt caccttttct tcaaaatcga tatggtggct gcaaaactag aattgttgca    3120
tccctcaatt gaatgagggc catatccctg tggtattcct ttcctgcttt ggggctttag    3180
aattctaatt gtcagtgatt ttgtatatga aaacaagttc caaatccaca gcttttacgt    3240
agtaaaagtc ataaatgcat atgacagaat ggctatcaaa agaaatagaa aaggaagacg    3300
gcatttaaag ttgtataaaa acacgagtta ttcataaaga gaaaatgatg agtttttatg    3360
gttccaatga aatatgttgg ggttttttta agattgtaaa aataatcagt tactggtatc    3420
tgtcactgac ctttgtttcc ttattcagga agataaaaat cagtaaccta ccccatgaag    3480
atatttggtg ggagttatat cagtgaagca gtttggttta tattcttatg ttatcacctt    3540
ccaaacaaaa gcacttactt tttttggaag ttatttattt tagactcaaa gaatataatc    3600
ttgcactact cagttattac tgtttgttct cttattccct agtctgtgtg gcaaattaaa    3660
caatataaga aggaaaaatt tgaagtatta gacttctaaa taaggggtga aatcatcaga    3720
aagaaaaatc aaagtagaaa ctactaattt tttaagagga atttataaca aatatggcta    3780
gttttcaact tcagtactca aattcaatga ttcttccttt tattaaaacc agtctcagat    3840
atcatactga tttttaagtc aacactatat attttatgat cttttcagtg tgatggcaag    3900
gtgcttgtta tgtctagaaa gtaagaaaac aatatgagga gacattctgt ctttcaaaag    3960
gtaatggtac atacgttcac tggtctctaa gtgtaaaagt agtaaatttt gtgatgaata    4020
aaataattat ctcctaattg tatgttagaa taattttatt agaataattt catactgaaa    4080
ttatttctc caaataaaaa ttagatggaa aaatgtgaaa aaaattattc atgctctcat    4140
atatatttta aaaacactac ttttgctttt ttatttacct tttaagacat tttcatgctt    4200
ccaggtaaaa acagatattg taccatgtac ctaatccaaa tatcatataa acattttatt    4260
tatagttaat aatctatgat gaaggtaatt aaagtagatt atggcctttt taagtattgc    4320
agtctaaaac ttcaaaaact aaaatcattg tcaaaattaa tatgattatt aatcagaata    4380
tcagaatatg attcactatt taaactatga taaattatga taatatatga ggaggcctcg    4440
ctatagcaaa aatagttaaa atgctgacat aacaccaaac ttcatttttt aaaaaatctg    4500
ttgttccaaa tgtgtataat tttaaagtaa tttctaaagc agtttattat aatggtttgc    4560
ctgcttaaaa ggtataatta aacttctttt ctcttctaca ttgacacaca gaaatgtgtc    4620
aatgtaaagc caaaaccatc ttctgtgttt atggccaatc tattctcaaa gttaaaagta    4680
aaattgtttc agagtcacag ttccctttat ttcacataag cccaaactga tagacagtaa    4740
cggtgtttag ttttatacta tatttgtgct atttaattct ttctattttc acaattatta    4800
aattgtgtac actttcatta cttttaaaaa tgtagaaatt cttcatgaac ataactctgc    4860
tgaatgtaaa agaaaatttt ttttcaaaaa tgctgttaat gtatactact ggtggttgat    4920
tggttttatt ttatgtagct tgacaattca gtgacttaat atctattcca tttgtattgt    4980
acataaaatt ttctagaaat acactttttt ccaaagtgta agtttgtgaa tagattttag    5040
catgatgaaa ctgtcataat ggtgaatgtt caatctgtgt aagaaaacaa actaaatgta    5100
gttgtcacac taaaatttaa ttggatattg atgaaatcat tggcctggca aaataaaaca    5160
tgttgaattc cccaaaaaaa aaaaaaaaa                                      5189
```

<210> 36
<211> 2360
<212> DNA
<213> Homo sapiens

<400> 36

```
gctacgcggg ccacgctgct ggctggcctg acctaggcgc gcggggtcgg gcggccgcgc 60
gggcgggctg agtgagcaag acaagacact caagaagagc gagctgcgcc tgggtcccgg 120
ccaggcttgc acgcagaggc gggcggcaga cggtgcccgg cggaatctcc tgagctccgc 180
cgcccagctc tggtgccagc gcccagtggc cgccgcttcg aaagtgactg gtgcctcgcc 240
gcctcctctc ggtgcgggac catgaagctg ctgccgtcgg tggtgctgaa gctctttctg 300
gctgcagttc tctcggcact ggtgactggc gagagcctgg agcggcttcg gagagggcta 360
gctgctggaa ccagcaaccc ggaccctccc actgtatcca cggaccagct gctaccccta 420
ggaggcggcc gggaccggaa agtccgtgac ttgcaagagg cagatctgga cctttttgaga 480
gtcactttat cctccaagcc acaagcactg gccacaccaa acaaggagga gcacgggaaa 540
agaaagaaga aaggcaaggg gctagggaag aagagggacc catgtcttcg gaaatacaag 600
gacttctgca tccatggaga atgcaaatat gtgaaggagc tccgggctcc ctcctgcatc 660
tgccacccgg gttaccatgg agagaggtgt catgggctga gcctcccagt ggaaaatcgc 720
ttatatacct atgaccacac aaccatcctg gccgtggtgg ctgtggtgct gtcatctgtc 780
tgtctgctgg tcatcgtggg gcttctcatg tttaggtacc ataggagagg aggttatgat 840
gtggaaaatg aagagaaagt gaagttgggc atgactaatt cccactgaga gagacttgtg 900
ctcaaggaat cggctgggga ctgctacctc tgagaagaca caaggtgatt tcagactgca 960
gaggggaaag acttccatct agtcacaaag actccttcgt ccccagttgc cgtctaggat 1020
tgggcctccc ataattgctt tgccaaaata ccagagcctt caagtgccaa acagagtatg 1080
tccgatggta tctgggtaag aagaaagcaa aagcaaggga ccttcatgcc cttctgattc 1140
ccctccacca aaccccactt cccctcataa gtttgtttaa acacttatct tctggattag 1200
aatgccggtt aaattccata tgctccagga tctttgactg aaaaaaaaaa agaagaagaa 1260
gaaggagagc aagaaggaaa gatttgtgaa ctggaagaaa gcaacaaaga ttgagaagcc 1320
atgtactcaa gtaccaccaa gggatctgcc attgggaccc tccagtgctg gatttgatga 1380
gttaactgtg aaataccaca agcctgagaa ctgaattttg ggacttctac ccagatggaa 1440
aaataacaac tatttttgtt gttgttgttt gtaaatgcct cttaaattat atatttattt 1500
tattctatgt atgttaattt atttagtttt taacaatcta acaataatat ttcaagtgcc 1560
tagactgtta ctttggcaat ttcctggccc tccactcctc atccccacaa tctggcttag 1620
tgccacccac ctttgccaca aagctaggat ggttctgtga cccatctgta gtaatttatt 1680
gtctgtctac atttctgcag atcttccgtg gtcagagtgc cactgcggga gctctgtatg 1740
gtcaggatgt aggggttaac ttggtcagag ccactctatg agttggactt cagtcttgcc 1800
taggcgattt tgtctaccat ttgtgttttg aaagcccaag gtgctgatgt caaagtgtaa 1860
cagatatcag tgtctccccg tgtcctctcc ctgccaagtc tcagaagagg ttgggcttcc 1920
atgcctgtag ctttcctggt ccctcacccc catggcccca ggccacagcg tgggaactca 1980
ctttcccttg tgtcaagaca tttctctaac tcctgccatt cttctggtgc tactccatgc 2040
aggggtcagt gcagcagagg acagtctgga gaaggtatta gcaaagcaaa aggctgagaa 2100
ggaacaggga acattggagc tgactgttct tggtaactga ttacctgcca attgctaccg 2160
agaaggttgg aggtggggaa ggctttgtat aatcccaccc acctcaccaa aacgatgaag 2220
gtatgctgtc atggtccttt ctggaagttt ctggtgccat ttctgaactg ttacaacttg 2280
tatttccaaa cctggttcat atttatactt tgcaatccaa ataaagataa cccttattcc 2340
ataaaaaaaa aaaaaaaaaa 2360
```

<210> 37
<211> 4163
<212> DNA
<213> Homo sapiens
<400> 37

```
ttgatttggt atagtgggaa catttgcttt ggagacagat gaactggatt ctgatcgtga 60
ccctgctatt ttctccttgt gtgactttgg agccatgaga ccccagatcc tgctgctcct 120
ggccctgctg accctaggcc tggctgcaca acaccaagac aaagtgccct gtaagatggt 180
ggacaagaag gtctcgtgcc aggttctggg cctgctccag gtcccctcgg tgctcccgcc 240
agacactgag acccttgatc tatctgggaa ccagctgcgg agtatcctgg cctcacccct 300
gggcttctac acggcacttc gtcacctgga cctgagcacc aatgagatca gcttcctcca 360
gccaggagcc ttccaggccc tgacccacct ggagcacctc agcctggctc acaaccggct 420
ggcgatggcc actgcgctga gtgctggtgg cctgggcccc ctgccacgcg tgacctccct 480
ggacctgtct gggaacagcc tgtacagcgg cctgctggag cggctgctgg gggaggcacc 540
cagcctgcat accctctcac tggcggagaa cagtctgact cgcctcaccc gccacacctt 600
ccgggacatg cctgcgctgg agcagcttga cctgcatagc aacgtgctga tggacatcga 660
ggatggcgcc ttcgagggcc tgcccgcct gacccatctc aacctctcca ggaattccct 720
cacctgcatc tccgacttca gcctccagca gctgcgggtg ctagacctga gctgcaacag 780
catcgaggcc tttcagacgg cctcccagcc ccaggctgag ttccagctca cctggcttga 840
cctgcgggag aacaaactgc tccatttccc cgacctggcc gcgctcccga gactcatcta 900
cctgaacttg tccaacaacc tcatccggct ccccacaggg ccaccccagg acagcaaggg 960
catccacgca ccttccgagg gctggtcagc cctgcccctc tcagccccca gcgggaatgc 1020
cagcggccgc cccctttccc agctcttgaa tctggatttg agctacaatg agattgagct 1080
catccccgac agctttcttg agcacctgac ctccctgtgc ttcctgaacc tcagcagaaa 1140
ctgcttgcgg acctttgagg cccggcgctt aggctccctg ccctgcctga tgctccttga 1200
cttaagccac aatgccctgg agacactgga actgggcgcc agagccctgg ggtctctgcg 1260
gacgctgctc ctacagggca atgccctgcg ggacctgccc ccatacacct ttgccaatct 1320
```

```
ggccagcctg cagcggctca acctgcaggg gaaccgagtc agcccctgtg gggggccaga   1380
tgagcctggc ccctccggct gtgtggcctt ctccggcatc acctccctcc gcagcctgag   1440
cctggtggat aatgagatag agctgctcag ggcaggggcc ttcctccaca ccccactgac   1500
tgagctggac ctttcttcca atcctgggct ggaggtggcc acgggggcct tgggaggcct   1560
ggaggcctcc ttggaggtcc tggcactgca gggcaacggg ctgatggtcc tgcaggtgga   1620
cctgccctgc ttcatctgcc tcaagcggct caatcttgcc gagaaccgcc tgagccacct   1680
tcccgcctgg acacaggctg tgtcactgga ggtgctggac ctgcgaaaca acagcttcag   1740
cctcctgcca ggcagtgcca tgggtggcct ggagaccagc ctccggcgcc tctacctgca   1800
ggggaatcca ctcagctgct gcggcaatgg ctggctggca gcccagctgc accagggccg   1860
tgtggacgtg gacgccaccc aggacctgat ctgccgcttc agctcccagg aggaggtgtc   1920
cctgagccac gtgcgtcccg aggactgtga gaagggggga ctgaagaaca tcaacctcat   1980
catcatcctc accttcatac tggtctctgc catcctcctc accacgctgg ccgcctgctg   2040
ctgcgtccgc cggcagaagt ttaaccaaca gtataaagcc taaagaagcc gggagacact   2100
ctaggtcagt gggggagcct gaggtacaga gaagagtgag gactgactca aggtcacaca   2160
gtgatccgga tcccagaact ctggtctcca aattacagcc caggacacct ttctctgccg   2220
cctgctgcat cagtgggtga ccccccttccc gggctgcact ttgggtccag ctgtggaagc   2280
cagaagttgg gcggtttcag ggacagccga gaataatgtt gacctgtcag atcaacaaat   2340
cttcactgag catgtatttt gtgccacacc ctgctctggg cactgggaat gctgggaaat   2400
gagatacatt cccgccctca agaatctccc agtctggtag gagagagtgc tgcagagcca   2460
cgtggccgcc acgcagtgtg cttagggcct gaggtgtgaa agcccagggc tccagagctc   2520
ggcaggcccc gctggtttgg tgcggtgagt cctgccccgg ctgtgcaggg tgagggaggg   2580
ccaagccagg aggatttgtc tgagacattt ccaagcagac tgtttgtcac gtcttctgag   2640
aatgactttc agtctctctg aaaatgaaaa gcttaggacc ggaagagaga attggagctg   2700
tacgagtgtg tctcggatct ggtattgtta ggtgggccac ggcggctcca gcagggtctg   2760
gttaaggggt ccagcccagc actggaccat tccgtctcct gctctggact tgccctctcc   2820
cttcctggca ctctcatgtt gcataccctg accccagtgc tgctctaagc accgtccctg   2880
cccagcccca cttctccatc gcagccccac cttggctgct gagccaggag ctaaaacctt   2940
agatatctgg ttctgttttg cacccagctt ggcagatgtg gatttgaatc caagccttgt   3000
gtctgcccct atgtgacagc tctatatttt atccccgttt tataaaagag gaaactgaag   3060
ttctgaaaat ctccttccag ggccccagct aactaatgcc ataggtgaga ttcaaacctt   3120
catccttctg tctccagggc ctgatcttta ccactgcagg ggctgcaggc cgttaagtgg   3180
acaggaagtg gccccacata gcccgagcag ggtctggaag catcctgtgc tgtgcacacc   3240
tgctctctcc tctctcccag gcaggcagct gcaggcgctc tcctccttct ctgcctgttt   3300
ccctcctccc ttcctttcca ccctggtgtg ggttctcctg ttctctctgt gctcttgcat   3360
tctctcattc ccttttcctc tatggagcag agcctggagt ttgagactat ggaatccaac   3420
ctccccattg cacagatggg gaaactgagg cttaggaaga gaatgaaact tgtggagagc   3480
ttatacagaa cctctggggg aaaaaagagc ccttatttgt ggggtgagat tggggggttgg  3540
accagagtga tgtcctctct cagctatcac atcacaagat aatgctggct ccaaacttcc   3600
tttctgtgcc tcatcatgca aggatctttt ttccctctta caaaaacagg taaaaagcct   3660
cacccagatg acccccatcc ctcataccat ggagtcatga gctgtctggg aagaatggac   3720
gtgctgggac caactcaaga ccttgttttg ctgtcttcat catcttacct gtgcttggcc   3780
cacagtctgg ctcatgatgt gggctcagta atgtgcgaga aagtgaaaat gccactctct   3840
ccaccccatt ttacagagga gaacaccaag gcccagagga agttaaggga gagtcaatgg   3900
gcagagccag ggctaggccc tggtggtgtg tggagcaccc aggcagaccc agtcctggtt   3960
gggatcacac ccacgggtgc tactgcacgt aacactcctc cttaggcctg gaggccaagg   4020
tgtgggtccc cacgcctgat ctttgaaaac actacacagg gctgctgtca cttcccaggg   4080
cccaggcctc agcccaggcc tcgggaccaa ctctttgtat aacctacctg aatgtattaa   4140
aaactaattt tggaaaaaaa aaa                                            4163
```

<210> 38
<211> 4548
<212> DNA
<213> Homo sapiens
<400> 38

```
gaattccggg agcccgcgga ggctgctgca gcagcccgaa ccggcgtccg agtccgggga     60
aggcccccgc gagcgcaggg aggggccgga aagttccggc gaactggggg agcggcagtg    120
ggagagcgtc gggcgggagg cggtggtcct gggagcggcc ccggcgcccg aagccgcccc    180
gagctggtgg ggagggtccc ggccggtggg gccgggctgg cgggggaggg gcgcctgggg    240
gtgcaagagg cgaattggct gcccggggtg caggagggac aggtggtgcg ggctgtcagg    300
aaacctacct gaacgggaag gagccccagg agggagaggg acccggcgag ggctcaggac    360
ccggaggcgc cggcggagga ggaggtggtg actggcaggc ccgggcccca cggtacctcc    420
ggggctgaag gggacgcagg atgtaggggc atggggagtc gggcgcagaa gagtgcgggg    480
aacgctgagc tctgggagcc actgcctgag ggcaggccgc ggccggcggg aacctcttct    540
gccgtctcag cctgggcgtc gctgaagctg tgtctgcggg gaggcagcgg aaggcggcag    600
aggctgggag gcggcagaat gcagccagag gagggcacag gctggctgct ggagctgctg    660
tccgaggtgc agctgcaaca gtacttcctg cggctccgag atgacctcaa cgtcacccgc    720
ctgtcccact ttgagtacgt caagaatgag gacctggaga agatcggcat gggtcggcct    780
ggccagcggc ggctgtggga ggctgtgaag aggaggaagg ccttgtgcaa acgcaagtcg    840
tggatgagta aggtgttcag tggaaagcga ctggaggctg agttcccacc tcatcactct    900
```

cagagcacct tccggaagac ctcgcccgcc cctgggggcc cagcagggga ggggccccctg 960
cagagcctca cctgcctcat tggggagaag gacctgcgcc tcctggagaa gctgggtgat 1020
ggttcctttg gcgtggtgcg caggggcgag tgggacgcgc cctcagggaa gacggtgagt 1080
gtggctgtga agtgcctgaa gcccgatgtc ctgagccagc cagaagccat ggacgacttc 1140
atccgggagg tcaatgccat gcactcgctc gaccaccgaa acctcatccg cctctacggg 1200
gtggtgctca cgccgcccat gaagatggtg acagagctgg cacctctggg atcgttgttg 1260
gaccggctac gtaagcacca gggccacttc ctcctgggga ctctgagccg ctacgctgtg 1320
caggtggctg agggcatggg ctacctggag tccaagcgct ttattcaccg tgacctggct 1380
gcccgcaatc tgctgttggc tacccgcgac ctggtcaaga tcggggactt tgggctgatg 1440
cgagcactac ctcagaatga cgaccattac gtcatgcagg aacatcgcaa ggtgcccttc 1500
gcctggtgtg cccccgagag cctgaagaca cgcaccttct cccatgccag cgacacctgg 1560
atgttcgggg tgacactgtg ggaaatgttc acctacggcc aggagccctg gatcggcctc 1620
aacggcagtc agatcctgca taagatcgac aaggaggggg agcggctgcc ccggcccgag 1680
gactgtcccc aggacatcta caacgtcatg gtccagtgct gggctcacaa gccagaggac 1740
agacccacgt ttgtggccct gcgggacttc ctgctggagg cccagcccac agacatgcgg 1800
gcccttcagg actttgagga accggacaag ctgcacatcc agatgaatga tgtcatcacc 1860
gtcatcgagg gaagggccga gaactactgg tggcgtggcc agaacacacg gacgctgtgt 1920
gtggggccct tccctcgcaa cgtggtgacc tccgtggccg gcctgtcggc ccaggacatc 1980
agccagcccc tgcagaacag cttcatccac acagggcatg gcgacagtga cccccgccac 2040
tgctggggct tcccggacag gattgacgaa ctgtatctgg gaaaccccat ggaccccccc 2100
gacctcctga gcgtggaact gagcacctcc cggccccccc agcatctagg aggggtgaaa 2160
aaaccaacct atgaccctgt gagcgaggac caagacccct tgtccagcga cttcaagagg 2220
ctgggcctgc ggaagccagg cctgccccga gggctgtggc tggcgaagcc ctcggcgcgg 2280
gtgccgggca ccaaggccag ccgaggcagc ggggctgagg tcacgctcat cgacttcggt 2340
gaggagcccg tggtcccggc cctacggccc tgcccgccct ccctggcgca gctggccatg 2400
gacgcctgct ccctgctgga cgagaccccg cctcagagcc ccacgcgggc actgccccgg 2460
cccctgcacc ccacgcctgt ggtggactgg gacgcacgcc cgctgccccc cccgcccgcc 2520
tatgacgacg tggcccagga tgaggatgac tttgagatct gctccatcaa cagcaccctc 2580
gtgggcgcgg gggtccctgc cgggcccagc cagggccaga ccaactacgc ctttgtgcct 2640
gagcaggcgc ggccgccccc tcccctggag gacaacctgt tcctcccgcc ccagggtggg 2700
ggcaagccgc ccagctccgc acagaccgca gagatcttcc aggcgctaca gcaggagtgc 2760
atgaggcaac tgcaggctcc gggctccccg gccccctctc ccagcccggg gggtgacgac 2820
aagccccagg tgcctcctcg ggtacccatc ccccctcggc ccacgcgccc acacgtccag 2880
ctgtctccag cccccccggg cgaggaggag accagccagt ggcctggacc tgcttcccct 2940
ccccgggtgc ctccgcggga gcccctgtcc cctcaaggct cgaggacacc cagccccctg 3000
gtaccacctg gcagctcccc gctgccaccc cggctctcaa gctcacctgg gaagaccatg 3060
cccaccaccc agagctttgc ctcagacccc aagtacgcca cccccaggt gatccaggcc 3120
cctggcgcgg gtggtccctg catcctgccc atcgtccggg atggcaagaa ggtcagcagc 3180
acccactatt acttgctgcc cgagcgacca tcctacctgg agcgctacca gcgcttcctg 3240
cgtgaggccc agagccccga ggagcctacc cccctgcctg tgcctctgct gctgcccccca 3300
cccagcaccc cagcccccgc cgcccccacg gccaccgtgc ggccgatgcc ccaggctgcc 3360
ttggacccca aggccaactt ctccaccaac aacagcaacc caggggcccg gccaccaccc 3420
ccgagggcca ctgctcggct gccacagagg ggctgccctg gcgatgggcc agaggcgggc 3480
cggccagcag acaagatcca gatggccatg gtgcatgggg tgaccacaga ggagtgccag 3540
gcggccctgc agtgccacgg ctggagcgtg cagagggctg cccagtatct gaaggtggag 3600
cagctcttcg ggctgggtct gcggcccaga ggggagtgcc acaaagtgct ggagatgttc 3660
gactggaacc tggagcaggc cggctgccac cttctgggct cctggggccc tgcccaccac 3720
aagcgctgag atgcgtctgg agagccagag ggcctgcctg aaggaatcac ctgagcctgt 3780
ccgtccacca ggagtgggga gatgccccca tccagtcctg gaggacccgc tgctcctgct 3840
gctcccgggg atggagcaag gccaaggctg cgggaggctg ggagccctgc cctgcccatc 3900
cctcccgcac cagtgctgtc cctgcacact ttggttcagt cccggtgccc ctgccaagat 3960
gtggaagggg ccgggtgaag acaggcttga gggccgcccc agcaggctct gggtatgacc 4020
tgcctctggc cctggtcctg ggcggggcct gtgggtggag tagtaccccc aggccctgcc 4080
ctgggtgaca gactgggagg aaaccaggct ggacctgggc aggcgggatg tgttggccac 4140
agggagaggc ggaccggcac ccggtgggac ctcctaggac tgggccttct tccaggggc 4200
ccctggcagc agctggggtg tcgggcagaa tgtgacttgt ggccttacca tggacttgaa 4260
tgggacttgg ctggcctcag gatcttgtgc ctggaaatag cctgaggtgg ctcaggaagc 4320
ggagaaaggg tgccagacca ttctctggcg gggaccaggg cccaaggcca gggctggaag 4380
gagaccaagg ggcagcccct ggaggacatc agtgcttcct cttccaccca attcccccac 4440
gcggttccat gttttcccac cagcctgttg ggcgaagttg ctgctccggc attcagtcct 4500
gcttcttcca gagaaataaa gttagtttct attttatgtt aaaaaaa 4548

<210> 39
<211> 2687
<212> DNA
<213> Homo sapiens
<400> 39
acggcgcgct gggctcacac tgtcccgccg cggacgggct ttgtggttgg gggcgcgcgt 60
gcgagtgcca gtgagagtgt gggtgcgcgc tgtgggccgc ggcgcgggtg ggtggccgtg 120

```
cgttcttgcg agccggcctg caggaggcga ggctcccctg gcctcccgca cccagcggcg    180
gaccgagccc ctggagggaa gttgccgcag ccgcccgggc cgccggccct cctgtcccgc    240
gccaggtaca cagcttctcc tagcatgact tcgatctgat cagcaaacaa gaaaatttgt    300
ctcccgtagt tctggggcgt gttcaccacc tacaaccaca gagctgtcat ggctgccatc    360
tctacttcca tccctgtaat ttcacagccc cagttcacag ccatgaatga accacagtgc    420
ttctacaacg agtccattgc cttcttttat aaccgaagtg gaaagcatct tgccacagaa    480
tggaacacag tcagcaagct ggtgatggga cttggaatca ctgtttgtat cttcatcatg    540
ttggccaacc tattggtcat ggtggcaatc tatgtcaacc gccgcttcca ttttcctatt    600
tattacctaa tggctaatct ggctgctgca gacttctttg ctgggttggc ctacttctat    660
ctcatgttca acacaggacc caatactcgg agactgactg ttagcacatg gctcctgcgt    720
cagggcctca ttgacaccag cctgacggca tctgtggcca acttactggc tattgcaatc    780
gagaggcaca ttacggtttt ccgcatgcag ctccacacac ggatgagcaa ccggcgggta    840
gtggtggtca ttgtggtcat ctggactatg gccatcgtta tgggtgctat acccagtgtg    900
ggctggaact gtatctgtga tattgaaaat tgttccaaca tggcacccct ctacagtgac    960
tcttacttag tcttctgggc cattttcaac ttggtgacct ttgtggtaat ggtggttctc   1020
tatgctcaca tctttggcta tgttcgccag aggactatga gaatgtctcg gcatagttct   1080
ggaccccggc ggaatcggga taccatgatg agtcttctga agactgtggt cattgtgctt   1140
ggggccttta tcatctgctg gactcctgga ttggttttgt tacttctaga cgtgtgctgt   1200
ccacagtgcg acgtgctggc ctatgagaaa ttcttccttc tccttgctga attcaactct   1260
gccatgaacc ccatcattta ctcctaccgc gacaaagaaa tgagcgccac ctttaggcag   1320
atcctctgct gccagcgcag tgagaacccc accggcccca cagaaggctc agaccgctcg   1380
gcttcctccc tcaaccacac catcttggct ggagttcaca gcaatgacca ctctgtggtt   1440
tagaacggaa actgagatga ggaaccagcc gtcctctctt ggaggataaa cagcctcccc   1500
ctacccaatt gccagggcaa ggtggggtgt gagagaggag aaaagtcaac tcatgtactt   1560
aaacactaac caatgacagt atttgttcct ggaccccaca agacttgata tatattgaaa   1620
attagcttat gtgacaaccc tcatcttgat ccccatccct tctgaaagta ggaagttgga   1680
gctcttgcaa tggaattcaa gaacagactc tggagtgtcc atttagacta cactaactag   1740
acttttaaaa gattttgtgt ggtttggtgc aagtcagaat aaattctggc tagttgaatc   1800
cacaacttca tttatataca ggcttccctt ttttattttt aaaggatacg tttcacttaa   1860
taaacacgtt tatgcctatc agcatgtttg tgatggatga gactatggac tgcttttaaa   1920
ctaccataat tccatttttt cccttacata ggaaaactgt aagttggaat tatcttttgt   1980
ttagaaagca tgcatgtaat gtatgtatgc agtatgcctt acttaaaaag attaaaagga   2040
tactaatgtt aaatcttcta ggaaatagaa cctagacttc aaagccagta tttgtttagg   2100
tcatgaagca aacaatgctc taatcacaat attaactgtt taattaaaat gttgtaacaa   2160
gtataaaaca gggaatgtaa gtttattacc aaagtgatat gtattccaaa aaagtcatag   2220
aagatgaagc actataatat tgttcccata tatttaaaat acccaagtac attctaatta   2280
ccagtatatc agaggaaaat tttcgtagtc tttgtaaaat aatatactca tcatagaaaa   2340
cttgaaaaat gcagaaatgt ataaaaaagc aaaaatgatt actgataata tcacaaccca   2400
gaagtaacca cctttaaaaa gcaacccccca tgtatgccta tatgtgtatt gtatactttt   2460
tttacataat tggagtcata ctgtaaacag ttttataagt agatcttttt cattgcaaaa   2520
ttgccacatt ttcttatggc attaaaaatt ttacaaaaac ataattttaa tggctatatt   2580
atattccatt taatggatgc aactcagttt atttaaccat tcccatgttg ttaactattt   2640
aggttgtttc taattttcat tattataaag ttgcagaaat ttggtgt                  2687
```

<210> 40
<211> 768
<212> DNA
<213> Homo sapiens
<400> 40

```
ccttcagcat aaaagctgat ccacaaacaa gaggagcacc agacctcctc ttggcttcga     60
gatggcttcg ccacaccaag agcccaaacc tggagacctg attgagattt tccgccttgg    120
ctatgagcac tgggccctgt atataggaga tggctacgtg atccatctgg ctcctccaag    180
tgagtacccc ggggctggct cctccagtgt cttctcagtc ctgagcaaca gtgcagaggt    240
gaaacggggg cgcctggaag atgtggtggg aggctgttgc tatcgggtca acaacagctt    300
ggaccatgag taccaaccac ggcccgtgga ggtgatcatc agttctgcga aggagatggt    360
tggtcagaag atgaagtaca gtattgtgag caggaactgt gagcactttg tcgcccagct    420
gagatatggc aagtcccgct gtaaacaggt ggaaaaggcc aaggttgaag tcggtgtggc    480
cacggcgctt ggaatcctgg ttgttgctgg atgctctttt gcgattagga gataccaaaa    540
aaaagcaaca gcctgaagca gccacaaaat cctgtgttag aagcagctgt ggggggtccca   600
gtggagatga gcctcccccca tgcctcccagc agcctgaccc tcgtgccctg tctcaggcgt  660
tctctagatc ctttcctctg tttccctctc tcgctggcaa aagtatgatc taattgaaac    720
aagactgaag gatcaataaa cagccatctg cccccttcaaa aaaaaaa                 768
```

<210> 41
<211> 1398
<212> DNA
<213> Homo sapiens
<400> 41

```
agcggagggg cgccctaacg aacccgggat acggtctgtt cctagtccgc tccggaaatg     60
```

```
caactgcgta cggcctggcc gcgtaatcgt gacgacagcg cgccagcgcc ggctagccgg    120
acgccctagg cttccgcgag atcttcggtg ggggtacggg tgttttacgc caggacgctg    180
atgcgtttgg gttctcgtct gcagaccctc tggacctggt cacgattcca taatgtacca    240
caacagtagt cagaagcggc actggacctt ctccagcgag gagcagctgg caagactgcg    300
ggctgacgcc aaccgcaaat tcagatgcaa agccgtggcc aacgggaagg ttcttccgaa    360
tgatccagtc tttcttgagc ctcatgaaga aatgacactc tgcaaatact atgagaaaag    420
gttattggaa ttctgttcgg tgtttaagcc agcaatgcca agatctgttg tgggtacggc    480
ttgtatgtat ttcaaacgtt tttatcttaa taactcagta atggaatatc accccaggat    540
aataatgctc acttgtgcat ttttggcctg caaagtagat gaattcaatg tatctagtcc    600
tcagtttgtt ggaaacctcc gggagagtcc tcttggacag gagaaggcac ttgaacagat    660
actggaatat gaactacttc ttatacagca acttaatttc caccttattg tccacaatcc    720
ttacagacca tttgagggct tcctcatcga cttaaagacc cgctatccca tattggagaa    780
tccagagatt ttgaggaaaa cagctgatga ctttcttaat agaattgcat tgacggatgc    840
ttacctttta tacacacctt cccaaattgc cctgactgcc attttatcta gtgcctccag    900
ggctggaatt actatggaaa gttatttatc agagagtctg atgctgaaag agaacagaac    960
ttgcctgtca cagttactag atataatgaa aagcatgaga aacttagtaa agaagtatga   1020
accacccaga tctgaagaag ttgctgttct gaaacagaag ttggagcgat gtcattctgc   1080
tgagcttgca cttaacgtaa tcacgaagaa gaggaaaggc tatgaagatg atgattacgt   1140
ctcaaagaaa tccaaacatg aggaggaaga atggactgat gacgacctgg tagaatctct   1200
ctaaccattt gaagttgatt tctcaatgct aactaatcaa gagaagtagg aagcatatca   1260
aacgtttaac tttatttaaa aagtataatg tgaaaacata aaatatatta aaacttttct   1320
attgttttct ttccctttca cagtaacttt atgtaaaata aaccatcttc aaaagagcta   1380
gaataaaaaa aaaaaaa                                                  1398
```

<210> 42
<211> 2756
<212> DNA
<213> Homo sapiens
<400> 42

```
atgctgtcct tccagtaccc cgacgtgtac cgcgacgaga ccgccgtaca ggattatcat     60
ggtcataaaa tttgtgaccc ttacgcctgg cttgaagacc ccgacagtga acagactaag    120
gcctttgtgg aggcccagaa taagattact gtgccatttc ttgagcagtg tcccatcaga    180
ggtttataca aagagagaat gactgaacta tatgattatc ccaagtatag ttgccacttc    240
aagaaaggaa aacggtattt ttattttttac aatacaggtt tgcagaacca gcgagtatta    300
tatgtacagg attccttaga gggtgaggcc agagtgttcc tggaccccaa catactgtct    360
gacgatggca cagtggcact ccgaggttat gcgttcagcg aagatggtga atattttgcc    420
tatggtctga gtgccagtgg ctcagactgg gtgacaatca agttcatgaa agttgatggt    480
gccaaagagc ttccagatgt gcttgaaaga gtcaagttca gctgtatggc ctggacccat    540
gatgggaagg gaatgttcta caactcatac cctcaacagg atggaaaaag tgatggcaca    600
gagacatcta ccaatctcca ccaaaagctc tactaccatg tcttgggaac cgatcagtca    660
gaagatattt tgtgtgctga gtttcctgat gaacctaaat ggatgggtgg agctgagtta    720
tctgatgatg gccgctatgt cttgttatca ataagggaag gatgtgatcc agtaaaccga    780
ctctggtact gtgacctaca gcaggaatcc agtggcatcg cgggaatcct gaagtgggta    840
aaactgattg acaactttga aggggaatat gactacgtga ccaatgaggg ggcggtgttc    900
acattcaaga cgaatcgcca gtctcccaac tatcgcgtga tcaacattga cttcagggat    960
cctgaagagt ctaagtggaa agtacttgtt cctgagcatg agaaagatgt cttagaatgg   1020
atagcttgtg tcaggtccaa cttcttggtc ttatgctacc tccatgacgt caagaacatt   1080
ctgcagctcc atgacctgac tactggtgct ctccttaaga ccttcccgct cgatgtcggc   1140
agcattgtag ggtacagcgg tcagaagaag gacactgaaa tcttctatca gtttacttcc   1200
tttttatctc caggtatcat ttatcactgt gatcttacca aagaggagct ggagccaaga   1260
gttttccgag aggtgaccgt aaaaggaatt gatgcttctg attaccagac agtccagatt   1320
ttctacccta gcaaggatgg tacgaagatt ccaatgttca ttgtgcataa aaaaagcata   1380
aaattggatg gctctcatcc agctttctta tatggctatg gcggcttcaa catatccatc   1440
acacccaact acagtgtttc caggcttatt tttgtgagac acatgggtgg tatcctggca   1500
gtggccaaca tcagaggagg tggcgaatat ggagagacgt ggcataaagg tggtatcttg   1560
gccaacaaac aaaactgctt tgatgacttt cagtgtgctg ctgagtatct gatcaaggaa   1620
ggttacacat ctcccaagag gctgactatt aatggaggtt caaatggagg cctcttagtg   1680
gctgcttgtg caaatcagag acctgacctc tttggttgtg ttattgccca agttggagta   1740
atggacatgc tgaagtttca taaatatacc atcggccatg cttggaccac tgattatggg   1800
tgctcggaca gcaaacaaca ctttgaatgg cttgtcaaat actctccatt gcataatgtg   1860
aagttaccag aagcagatga catccagtac ccgtccatgc tgctcctcac tgctgaccat   1920
gatgaccgcg tggtcccgct tcactccctg aagttcattg ccacccttca gtacatcgtg   1980
ggccgcagca ggaagcaaag caaccccctg cttatccacg tggacaccaa ggcgggccac   2040
ggggcgggga agcccacagc caaagtgata gaggaagtct cagacatgtt tgcgttcatc   2100
gcgcggtgcc tgaacgtcga ctggattcca taaacagttt tcgtgcttcc tcctgacagc   2160
gacagaaaac ctcaagggct ttcccacgtt gacaccaaga aaccactggg cataatgctt   2220
ccccacggga acattattcc tggactgaca ggctacagtt gaacagaact gccgtgggaa   2280
ttttatcttt tttaggcttc tccttttttag caaggccttg gtgtttcttt ttccaccctg   2340
tctaggcaca tgtggttttt tggtgttttt tttaagggca tgttgggata aatagctaaa   2400
```

```
tggcaacaaa cacattgtga atattagatt gctgaattaa ggatcatagt cgggcatact   2460
tatctatatc cataacctct atatctttaa ataaatgtga gaactgttct catggagaag   2520
acttctttgc aacaataata aatgttattt aagaatgaca gggatttact tccggtttct   2580
tcatattgag gggcaactcc agaagtggag ttttctgtga gaataaagca tttcaccttt   2640
ctgcaacaag ttagttttca agcagttaag tcatagaatg tttgttagct gtgaaaataa   2700
gttgttcatc caaaaaaaaa aaaaaaaaaa aaaaaaaaaa aaaaaaaaag gaattc       2756

<210>  43
<211>  6319
<212>  DNA
<213>  Homo sapiens
<400>  43
acacagtact ctcagcttgt tggtggaagc ccctcatctg ccttcattct gaaggcaggg     60
cccggcagag gaaggatcag agggtcgcgg ccggagggtc ccggccggtg gggccaactc    120
agagggagag gaaaggccta gagacacgaa gaacgcaaac catcaaattt agaagaaaaa    180
gccctttgac tttttccccc tctccctccc caatggctgt gtagcaaaca tccctggcga    240
taccttggaa aggacgaagt tggtctgcag tcgcaatttc gtgggttgag ttcacagttg    300
tgagtgcggg gctcggagat ggagccgtgg tcctctaggt ggaaaacgaa acggtggctc    360
tgggatttca ccgtaacaac cctcgcattg accttcctct tccaagctag agaggtcaga    420
ggagctgctc cagttgatgt actaaaagca ctagattttc acaattctcc agagggaata    480
tcaaaaacaa cgggattttg cacaaacaga aagaattcta aaggctcaga tactgcttac    540
agagtttcaa agcaagcaca actcagtgcc ccaacaaaac agttatttcc aggtggaact    600
ttcccagaag acttttcaat actatttaca gtaaaaccaa aaaaaggaat tcagtctttc    660
cttttatcta tatataatga gcatggtatt cagcaaattg gtgttgaggt tgggagatca    720
cctgtttttc tgtttgaaga ccacactgga aaacctgccc cagaagacta tcccctcttc    780
agaactgtta acatcgctga cgggaagtgg catcgggtag caatcagcgt ggagaagaaa    840
actgtgacaa tgattgttga ttgtaagaag aaaaccacga aaccacttga tagaagtgag    900
agagcaattg ttgataccaa tggaatcacg gtttttggaa caaggatttt ggatgaagaa    960
gtttttgagg gggacattca gcagtttttg atcacaggtg atcccaaggc agcatatgac   1020
tactgtgagc attatagtcc agactgtgac tcttcagcac ccaaggctgc tcaagctcag   1080
gaacctcaga tagatgagta tgcaccagag gatataatcg aatatgacta tgagtatggg   1140
gaagcagagt ataaagaggc tgaaagtgta acagagggac ccactgtaac tgaggagaca   1200
atagcacaga cggaggcaaa catcgttgat gattttcaag aatacaacta tggaacaatg   1260
gaaagttacc agacagaagc tcctaggcat gtttctggga caaatgagcc aaatccagtt   1320
gaagaaatat ttactgaaga atatctaacg ggagaggatt atgattccca gaggaaaaat   1380
tctgaggata cactatatga aaacaaagaa atagacggca gggattctga tcttctggta   1440
gatggagatt taggcgaata tgatttttat gaatataaag aatatgaaga taaaccaaca   1500
agccccccta atgaagaatt tggtccaggt gtaccagcag aaactgatat tacagaaaca   1560
agcataaatg gccatggtgc atatggagag aaaggacaga aaggagaacc agcagtggtt   1620
gagcctggta tgcttgtcga aggaccacca ggaccagcag gacctgcagg tattatgggt   1680
cctccaggtc tacaaggccc cactggaccc cctggtgacc ctggcgatag gggcccccca   1740
ggacgtcctg gcttaccagg ggctgatggt ctacctggtc ctcctggtac tatgttgatg   1800
ttaccgttcc gttatggtgg tgatggttcc aaaggaccaa ccatctctgc tcaggaagct   1860
caggctcaag ctattcttca gcaggctcgg attgctctga gaggcccacc tggcccaatg   1920
ggtctaactg gaagaccagg tcctgtgggg gggcctggtt catctggggc caaaggtgag   1980
agtggtgatc caggtcctca gggccctcga ggcgtccagg gtccccctgg tccaacggga   2040
aaacctggaa aaaggggtcg tccaggtgca gatggaggaa gaggaatgcc aggagaacct   2100
ggggcaaagg gagatcgagg gtttgatgga cttccgggtc tgccaggtga caaaggtcac   2160
aggggtgaac gaggtcctca aggtcctcca ggtcctcctg gtgatgatgg aatgagggga   2220
gaagatggag aaattggacc aagaggtctt ccaggtgaag ctggcccacg aggtttgctg   2280
ggtccaaggg gaactccagg agctccaggg cagcctggta tggcaggtgt agatggcccc   2340
ccaggaccaa aagggaacat gggtccccaa ggggagcctg ggcctccagg tcaacaaggg   2400
aatccaggac ctcagggtct tcctggtcca caaggtccaa ttggtcctcc tggtgaaaaa   2460
ggaccacaag gaaaaccagg acttgctgga cttcctggtg ctgatgggcc tcctggtcat   2520
cctgggaaag aaggccagtc tggagaaaag ggggctctgg gtccccctgg tccacaaggt   2580
cctattggat acccgggccc ccggggagta aagggagcag atggtgtcag aggtctcaag   2640
ggatctaaag gtgaaaaggg tgaagatggt tttccaggat tcaaaggtga catgggtcta   2700
aaaggtgaca gaggagaagt tggtcaaatt ggcccaagag gggaagatgg ccctgaagga   2760
cccaaaggtc gagcaggccc aactggagac ccaggtcctt caggtcaagc aggagaaaag   2820
ggaaaacttg gagttccagg attaccagga tatccaggaa gacaaggtcc aaagggttcc   2880
actggattcc ctgggtttcc aggtgccaat ggagagaaag gtgcacgggg agtagctggc   2940
aaaccaggcc ctcggggtca gcgtggtcca acgggtcctc gaggttcaag aggtgcaaga   3000
ggtcccactg ggaaacctgg gccaaagggc acttcaggtg gcgatggccc tcctggccct   3060
ccaggtgaaa gaggtcctca aggacctcag ggtccagttg gattccctgg accaaaaggc   3120
cctcctggac caccaggaag gatgggctgc ccaggacacc ctgggcaacg tggggagact   3180
ggatttcaag gcaagaccgg ccctcctggg ccagggggag tggttggacc acagggacca   3240
accggtgaga ctggtccaat aggggaacgt gggcatcctg gccctcctgg ccctcctggt   3300
gagcaaggtc ttcctggtgc tgcaggaaaa gaaggtgcaa agggtgatcc aggtcctcaa   3360
ggtatctcag ggaaagatgg accagcagga ttacgtggtt tcccagggga aagaggtctt   3420
```

cctggagctc agggtgcacc tggactgaaa ggaggggaag gtccccaggg cccaccaggt 3480
ccagttggct caccaggaga acgtgggtca gcaggtacag ctggcccaat tggtttacca 3540
gggcgcccgg gacctcaggg tcctcctggt ccagctggag agaaaggtgc tcctggagaa 3600
aaaggtcccc aagggcctgc agggagagat ggagttcaag gtcctgttgg tctcccaggg 3660
ccagctggtc ctgccggctc ccctggggaa gacggagaca agggtgaaat tggtgagccg 3720
ggacaaaaag gcagcaaggg tgacaaggga gaaaatggcc ctcccggtcc cccaggtctt 3780
caaggaccag ttggtgcccc tggaattgct ggaggtgatg gtgaaccagg tcctagagga 3840
cagcagggga tgtttgggca aaaaggtgat gagggtgcca gaggcttccc tggacctcct 3900
ggtccaatag gtcttcaggg tctgccaggc ccacctggtg aaaaaggtga aaatggggat 3960
gttggtccat gggggccacc tggtcctcca ggcccaagag gccctcaagg tcccaatgga 4020
gctgatggac cacaaggacc cccaggttct gttggttcag ttggtggtgt tggagaaaag 4080
ggtgaacctg gagaagcagg aaacccaggg cctcctgggg aagcaggtgt aggcggtccc 4140
aaaggagaaa gaggagagaa aggggaagct ggtccacctg gagctgctgg acctccaggt 4200
gccaagggggc cgccaggtga tgatggccct aagggtaacc cgggtcctgt tggttttcct 4260
ggagatcctg gtcctcctgg ggaacttggc cctgcaggtc aagatggtgt tggtggtgac 4320
aagggtgaag atggagatcc tggtcaaccg ggtcctcctg gcccatctgg tgaggctggc 4380
ccaccaggtc ctcctggaaa acgaggtcct cctggagctg caggtgcaga gggaagacaa 4440
ggtgaaaaag gtgctaaggg ggaagcaggt gcagaaggtc ctcctggaaa aaccggccca 4500
gtcggtcctc agggacctgc aggaaagcct ggtccagaag gtcttcgggg catccctggt 4560
cctgtgggag aacaaggtct ccctggagct gcaggccaag atggaccacc tggtcctatg 4620
ggacctcctg gcttacctgg tctcaaaggt gaccctggct ccaagggtga aaagggacat 4680
cctggtttaa ttggcctgat tggtcctcca ggagaacaag gggaaaaagg tgaccgaggg 4740
ctccctggaa ctcaaggatc tccaggagca aaaggggatg gggaattcc tggtcctgct 4800
ggtcccttag gtccacctgg tcctccaggc ttaccaggtc ctcaaggccc aaagggtaac 4860
aaaggctcta ctggacccgc tggccagaaa ggtgacagtg gtcttccagg gcctcctggg 4920
cctccaggtc cacctggtga agtcattcag cctttaccaa tcttgtcctc caaaaaaacg 4980
agaagacata ctgaaggcat gcaagcagat gcagatgata atattcttga ttactcggat 5040
ggaatggaag aaatatttgg ttccctcaat tccctgaaac aagacatcga gcatatgaaa 5100
tttccaatgg gtactcagac caatccagcc cgaacttgta aagacctgca actcagccat 5160
cctgacttcc cagatggtga atattggatt gatcctaacc aaggttgctc aggagattcc 5220
ttcaaagttt actgtaattt cacatctggt ggtgagactt gcatttatcc agacaaaaaa 5280
tctgagggag taagaatttc atcatggcca aaggagaaac caggaagttg gtttagtgaa 5340
tttaagaggg gaaaactgct ttcatactta gatgttgaag gaaattccat caatatggtg 5400
caaatgacat tcctgaaact tctgactgcc tctgctcggc aaaatttcac ctaccactgt 5460
catcagtcag cagcctggta tgatgtgtca tcaggaagtt atgacaaagc acttcgcttc 5520
ctgggatcaa atgatgagga gatgtcctat gacaataatc cttttatcaa aacactgtat 5580
gatggttgta cgtccagaaa aggctatgaa aagactgtca ttgaaatcaa tacaccaaaa 5640
attgatcaag tacctattgt tgatgtcatg atcaatgact ttggtgatca gaatcagaag 5700
ttcggatttg aagttggtcc tgtttgtttt cttggctaag attaagacaa agaacatatc 5760
aaatcaacag aaaatatacc ttggtgccac caacccattt tgtgccacat gcaagttttg 5820
aataaggatg gtatagaaaa caacgctgca tatacaggta ccatttagga aataccgatg 5880
cctttgtggg ggcagaatca catggcaaaa gctttgaaaa tcataaagat ataagttggt 5940
gtggctaaga tggaaacagg gctgattctt gattcccaat tctcaactct cctttttccta 6000
tttgaatttc tttggtgctg tagaaaacaa aaaaagaaaa atatatattc ataaaaaata 6060
tggtgctcat tctcatccat ccaggatgta ctaaaacagt gtgtttaata aattgtaatt 6120
attttgtgta cagttctata ctgttatctg tgtccatttc caaaacttgc acgtgtccct 6180
gaattccatc tgactctaat tttatgagaa ttgcagaact ctgatggcaa taaatatatg 6240
tattatgaaa aaataaagtt gtaatttctg atgactctaa gtccctttct ttggttaata 6300
ataaaatgcc tttgtatat 6319

<210> 44
<211> 3986
<212> DNA
<213> Homo sapiens
<400> 44

atgctgggga agagccatgg taggaccact catggccctc ttcctttggc ggaccttgga 60
atccaccttc cctgcgttaa agtgctccac caggtgacgc cggaagagaa gccagcaggc 120
ggcggcggcg tcagcatcag cggcctcctg cccgtatcta tcgtggcggc gacgggaccc 180
gcctccctgg gcgccggagt catgtgaccc acacaatggc tgagtggcta ctctcggctt 240
cctggcaacg ccgagcgaaa gctatgactg cggccgcggg ttcggcgggc cgcgccgcgg 300
tgcccttgct gctgtgtgcg ctgctggcgc ccggcggcgc gtacgtgctc gacgactccg 360
acgggctggg ccgggagttc gacggcatcg gcgcggtcag cggcggcggg gcaacctccc 420
gacttctagt aaattaccca gagccctatc gttctcagat attggattat ctctttaagc 480
cgaattttgg tgcctctttg catattttaa aagtggaaat aggtggtgat gggcagacaa 540
cagacggcac tgagccctcc cacatgcatt atgcactaga tgagaattat ttccgaggat 600
acgagtggtg gttgatgaaa gaagctaaga agaggaatcc caatattaca ctcattgggt 660
tgccatggtc attccctgga tggctgggaa aaggtttcga ctggccttat gtcaatcttc 720
agctgactgc ctattatgtc gtgacctgga ttgtgggcgc caagcgttac catgatttgg 780
acattgatta tattggaatt tggaatgaga ggtcatataa tgccaattat attaagatat 840

```
taagaaaaat gctgaattat caaggtctcc agcgagtgaa aatcatagca agtgataatc    900
tctgggagtc catctctgca tccatgctcc ttgatgccga actcttcaag gtggttgatg    960
ttataggggc tcattatcct ggaacccatt cagcaaaaga tgcaaagttg actgggaaga   1020
agctttggtc ttctgaagac tttagcactt taaatagtga catgggtgca ggctgctggg   1080
gtcgcatttt aaatcagaat tatatcaatg gctatatgac ttccacaatc gcatggaatt   1140
tagtggctag ttactatgaa cagttgcctt atgggagatg cgggttgatg acggcccaag   1200
agccatggag tgggcactac gtggtagaat ctcctgtctg ggtatcagct cataccactc   1260
agtttactca acctggctgg tattacctga agacagttgg ccatttagag aaaggaggaa   1320
gctacgtagc tctgactgat ggcttaggga acctcaccat catcattgaa accatgagtc   1380
ataaacattc taagtgcata cggccatttc ttccttattt caatgtgtca caacaatttg   1440
ccacctttgt tcttaaggga tcttttagtg aaataccaga gctacaggta tggtatacca   1500
aacttggaaa aacatccgaa agatttcttt ttaagcagct ggattctcta tggctccttg   1560
acagcgatgg cagtttcaca ctgagcctgc atgaagatga gctgttcaca ctcaccactc   1620
tcaccactgg tcgcaaaggc agctacccgc ttcctccaaa atcccagccc ttcccaagta   1680
cctataagga tgatttcaat gttgattacc cattttttag tgaagctcca aactttgctg   1740
atcaaactgg tgtatttgaa tattttacaa atattgaaga ccctggcgag catcacttca   1800
cgctacgcca agttctcaac cagagaccca ttacgtgggc tgccgatgca tccaacacaa   1860
tcagtattat aggagactac aactggacca atctgactat aaagtgtgat gtttacatag   1920
agacccctga cacaggaggt gtgttcattg caggaagagt aaataaaggt ggtattttga   1980
ttagaagtgc cagaggaatt ttcttctgga tttttgcaaa tggatcttac agggttacag   2040
gtgatttagc tggatggatt atatatgctt taggacgtgt tgaagttaca gcaaaaaaat   2100
ggtatacact cacgttaact attaagggtc atttcgcctc tggcatgctg aatgacaagt   2160
ctctgtggac agacatccct gtgaattttc caaagaatgg ctgggctgca attggaactc   2220
actcctttga atttgcacag tttgacaact ttcttgtgga agccacacgc taatacttaa   2280
cagggcatca tagaatactc tggattttct tcccttcttt ttggttttgg ttcagagcca   2340
attcttgttt cattggaaca gtatatgagg cttttgagac taaaaataat gaagagtaaa   2400
aggggagaga aatttatttt taatttaccc tgtggaagat tttattagaa ttaattccaa   2460
ggggaaaact ggtgaatctt taacattacc tggtgtgttc cctaacattc aaactgtgca   2520
ttggccatac ccttaggagt ggtttgagta gtacagacct cgaagccttg ctgctaacac   2580
tgaggtagct ctcttcatct tatttgcaag cggtcctgta gatggcagta acttgatcat   2640
cactgagatg tatttatgca tgctgaccgt gtgtccaagt gagccagtgt cttcatcaca   2700
agatgatgct gccataatag aaagctgaag aacactagaa gtagcttttt gaaaaccact   2760
tcaacctgtt atgctttatg ctctaaaaag tattttttta ttttcctttt taagatgata   2820
cttttgaaat gcaggatatg atgagtggga tgattttaaa aacgcctctt taataaacta   2880
cctctaacac tatttctgcg gtaatagata ttagcagatt aattgggtta tttgcattat   2940
ttaatttttt tgattccaag ttttggtctt gtaaccacta taactctctg tgaacgtttt   3000
tccaggtggc tggaagaagg aagaaaacct gatatagcca atgctgttgt agtcgtttcc   3060
tcagcctcat ctcactgtgc tgtggtctgt cctcacatgt gcactggtaa cagactcaca   3120
cagctgatga atgcttttct ctccttatgt gtggaaggag gggagcactt agacatttgc   3180
taactcccag aattggatca tctcctaaga tgtacttact ttttaaagtc caaatatgtt   3240
tatatttaaa tatacgtgag catgttcatc atgttgtatg atttatacta agcattaatg   3300
tggctctatg tagcaaatca gttattcatg taggtaaagt aaatctagaa ttatttataa   3360
gaattactca ttgaactaat tctactattt aggaatttat aagagtctaa cataggctta   3420
gctacagtga agttttgcat tgcttttgaa gacaagaaaa gtgctagaat aaataagatt   3480
acagagaaaa ttttttgtta aaaccaagtg atttccagct gatgtatcta atattttttta   3540
aaacaaacat tatagaggtg taatttattt acaataaaat gttcctactt taaatataca   3600
attcagtgag ttttgataaa ttgatatacc catgtaacca acactccagt caagcttcag   3660
aatatttcca tcaccccaga aggttctctt gtatacctgc tcagtcagtt cctttcactc   3720
ccaattgttg gcagccattg ataggaattc tatcactata ggttagtttt ctttgttcca   3780
gaacatcatg aaagcggcgt catgtactgt gtattcttat gaatggtttc tttccatcag   3840
cataatgatt tgagattggt ccatgttgtg tgattcagtg gtttgttcct tcttatttct   3900
gaagagtttt ccattgtatg aatataccac aatttgtttc ctccccacca gtttctgata   3960
ctacaattaa aactgtctac atttac                                        3986
```

```
<210>  45
<211>  2058
<212>  DNA
<213>  Homo sapiens
<400>  45
cggtttctgc tgggtttctg aactgctggg tttctgcttg ctcctctgga gatgcagcgt     60
ctgttgactc cagtgaagcg cattctgcaa ctgacaagag cggtgcagga aacctccctc    120
acacctgctc gcctgctccc agtagcccac caaaggtttt ctacagcctc tgctgtcccc    180
ctggccaaaa cagatacttg gccaaaggac gtgggcatcc tggccctgga ggtctacttc    240
ccagcccaat atgtggacca aactgacctg gagaagtata acaatgtgga agcaggaaag    300
tatacagtgg gcttgggcca gacccgtatg ggcttctgct cagtccaaga ggacatcaac    360
tccctgtgcc tgacggtggt gcaacggctg atggagcgca tacagctccc atgggactct    420
gtgggcaggc tggaagtagg cactgagacc atcattgaca agtccaaagc tgtcaaaaca    480
gtgctcatgg aactcttcca ggattcaggc aatactgata ttgagggcat agataccacc    540
aatgcctgct acggtggtac tgcctccctc ttcaatgctg ccaactggat ggagtccagt    600
```

```
tcctgggatg gtcgttatgc catggtggtc tgtggagaca ttgccgtcta tcccagtggt    660
aatgctcgtc ccacaggtgg ggccggagct gtggctatgc tgattggccc aaaggcccct    720
ctggccctgg agcgagggct gaggggaacc catatggaga atgtgtatga cttctacaaa    780
ccaaatttgg cctcggagta cccaatagtg gatgggaagc tttccatcca gtgctacttg    840
cgggccttgg atcgatgtta cacatcatac cgtaaaaaaa tccagaatca gtggaagcaa    900
gctggcagcg atcgaccctt cacccttgac gatttacagt atatgatctt tcatacaccc    960
ttttgcaaga tggtccagaa gtctctggct cgcctgatgt tcaatgactt cctgtcagcc   1020
agcagtgaca cacaaaccag cttatataag gggctggagg ctttcggggg gctaaagctg   1080
gaagacacct acaccaacaa ggacctggat aaagcacttc taaaggcctc tcaggacatg   1140
ttcgacaaga aaaccaaggc ttccctttac ctctccactc acaatgggaa catgtacacc   1200
tcatccctgt acgggtgcct ggcctcgctt ctgtcccacc actctgccca agaactggct   1260
ggctccagga ttggtgcctt ctcttatggc tctggtttag cagcaagttt cttttcattt   1320
cgagtatccc aggatgctgc tccaggctct cccctggaca agttggtgtc cagcacatca   1380
gacctgccaa aacgcctagc ctcccgaaag tgtgtgtctc ctgaggagtt cacagaaata   1440
atgaaccaaa gagagcaatt ctaccataag gtgaatttct ccccacctgg tgacacaaac   1500
agccttttcc caggtacttg gtacctggag cgagtggacg agcagcatcg ccgaaagtat   1560
gccccggcgtc ccgtctaaag gtgttctgca gatccatgga aagcttcctg ggaaacgtat   1620
gctagcagag cttctccccg tgaatcatat ttttaagatc ccactcttag ctggtaaatg   1680
aatttgaatc gacatagtag ccccataagc atcagccctg tagagtgagg agccatctct   1740
agcgggccct tcattcctct ccatgctgca atcactgtcc tgggcttatg gtgcctatgg   1800
actaggggtc ctttgtgaaa gagcaagatg gagcaatgga gagaagacct cttcctgaat   1860
cactggactc cagaaatgtg catgcagatc agctgttgcc ttcaagatcc agataaactt   1920
tcctgtcatg tgttagaact ttattattat taatattgtt aaacttctgt gctgttcctg   1980
tgaatctcca aattttgtac cttgttctaa gctaatatat agcaattaaa aagagagaaa   2040
gagaaaaaaa aaaaaaa                                                  2058
```

<210> 46
<211> 2538
<212> DNA
<213> Homo sapiens
<400> 46

```
cgcccccacc gccacccaac caagcatcac ccattccagc acttccggcc tgacccctgt     60
ccagcgcctt tcctttctcc agcttctgct ctgccccaag agtggtcgcc ttcgtggcag    120
ggcacgactc tctcccagct cgggtcgccg cccacattag tgtggggccc tgcggcctag    180
cgtccctcac cagaggcctc cccttgccta gctggaccgc cgagggacat cgacgagtat    240
cctcctcctg ctgtccccgg cttcgcctgc cgcccctaac cggccagtca agatggccgc    300
cgctgggtga ggcaagctgg cgcgccgcgg gggcgtctgg gagttgtagt tcgggacggc    360
gggctgacgc acttcgccgc cggccgacgg gcgccattgt gcggcgcgcg ccggagtatc    420
ctgagctcca gctggaccct aaattggatc ctcttcctgc tgagagtccc ctaatgaaca    480
ttgaggttgt tgaggtcctc acactgaacc aggaggtggc tggtccccgg aatgcccaga    540
tccaggccct atatgctgaa gatggaagcc tgagtgcaga tgcccccagt gagcaggtcc    600
aacagcaggg caagcatcca ggtgaccctg aggccgcgcg ccagaggttc cggcagttcc    660
gttataagga catgacaggt ccccgggagg ccctggacca gctccgagag ctgtgtcacc    720
agtggctaca gcctaaggca cgctccaagg agcagatcct ggagctgctg gtgctggagc    780
agttcctagg tgcactgcct gtgaagctcc ggacatgggt ggaatcgcag cacccagaga    840
actgccaaga ggtggtggcc ctggtagagg gtgtgacctg gatgtctgag gaggaagtac    900
ttcctgcagg acaacctgcc gagggcacca cctgctgcct cgaggtcact gcccagcagg    960
aggagaagca ggaggatgca gccatctgcc cagtgacagt gctccctgag gagccagtga   1020
ccttccagga tgtggctgtg gacttcagcc gggaggagtg gggctgctg ggcccgacac    1080
agaggaccga gtaccgcgat gtgatgctgg agacctttgg gcacctggtc tctgtggggt   1140
gggagactac actggaaaat aaagagttag ctccaaattc tgacattcct gaggaagaac   1200
cagcccccag cctgaaagta caagaatcct caagggattg tgccttgtcc tctacattag   1260
aagatacctt gcagggtggg gtccaggaag tccaagacac agtgttgaag cagatggagt   1320
ctgctcagga aaaagacctt cctcagaaga agcactttga caaccgtgag tcccaggcaa   1380
acagtggtgc tcttgacaca aaccaagttt cgctccagaa aattgacaac cctgagtccc   1440
aggcaaacag tggcgctctt gacacaaacc aagttttgct ccacaaaatt cctcctagaa   1500
aacgattgcg caaacgtgac tcacaagtta aaagtatgaa acataattca cgtgtaaaaa   1560
ttcatcagaa gagctgtgaa aggcaaaagg ccaaggaagg caatggttgt aggaaaacct   1620
tcagtcggag tactaaacag attacgttta taagaattca caaggggagc caagtttgcc   1680
gatgcagtga atgtggtaaa atattccgga acccaagata cttttctgtg cataagaaaa   1740
tccataccgg agagaggccc tatgtgtgtc aagactgtgg gaaaggattt gttcagagct   1800
cttccctcac acagcatcag agagttcatt ctggagagag accatttgaa tgtcaggagt   1860
gtgggaggac cttcaatgat cgctcagcca tctcccagca cctgaggact cacactggcg   1920
ctaagcccta caagtgtcag gactgtggaa aagccttccg ccagagctcc cacctcatca   1980
gacatcagag gactcacacc ggggagcgcc catatgcatg caacaaatgt ggaaaggcct   2040
tcacccagag ctcacacctt attgggcacc agagaaccca caataggaca aagcgaaaga   2100
agaaacagcc tacctcatag ctctcaagcc agttgaagaa accttgcctt ttcagcttga   2160
ccctgcaata taacatgcac aggcctgctt gtgaatcagg actgaatgtg aaagggaagt   2220
attgagtgag gacattccca aaaccaaagg acaactgagg agactgccca gcacataatg   2280
```

aataaataag aaaatgagtg aggagttatt aacatcattt ggaaaaaaga tttcccattc 2340
acttgatatt gtttgttcac tcatttagtc attaaaagtg agattaataa aatctgaaaa 2400
tgttatataa taactttaaa aagccaggta attaataatc tgcactgata ttacatccac 2460
agtaccacag tatttatgtg tatgaattaa ggattaaaag ataatgtgga taaataaact 2520
attgatctat gtctgtgt 2538

<210> 47
<211> 540
<212> DNA
<213> Homo sapiens
<400> 47
atccctgact cggggtcgcc tttggagcag agaggaggca atggccacca tggagaacaa 60
ggtgatctgc gccctggtcc tggtgtccat gctggccctc ggcaccctgg ccgaggccca 120
gacagagacg tgtacagtgg ccccccgtga aagacagaat tgtggttttc ctggtgtcac 180
gccctcccag tgtgcaaata agggctgctg tttcgacgac accgttcgtg ggtcccctg 240
gtgcttctat cctaatacca tcgacgtccc tccagaagag gagtgtgaat tttagacact 300
tctgcaggga tctgcctgca tcctgacggg gtgccgtccc cagcacggtg attagtccca 360
gagctcggct gccacctcca ccggacacct cagacacgct tctgcagctg tgcctcggct 420
cacaacacag attgactgct ctgactttga ctactcaaaa ttggcctaaa aattaaaaga 480
gatcgatatt aaaaaaaaaa aaaaaaaaaa aaaaaaaaaa aaaaaaaaaa aaaaaaaaaa 540

<210> 48
<211> 2254
<212> DNA
<213> Homo sapiens
<400> 48
gggaattctg aaagccgctg gcggaccgcg cgcagcggcc agagaccgag ccctaaggag 60
agtgcggcgc ttcccgaggc gtgcagctgg gaactgcaac tcatctgggt tgtgcgcaga 120
aggctggggc aagcgagtag agaagtggag cgtaagccag gggcgttggg ggccgtgcgg 180
gtcgggcgcg tgccacgccc gcggggtgaa gtcggagcgc ggggcctgct ggagagagga 240
gcgctgcgga ccgagtaatg gcaatgcaga tgcagcttga agcaaatgca gatacttcag 300
tggaagaaga aagctttggc ccacaaccca tttcacggtt agagcagtgt ggcataaatg 360
ccaacgatgt gaagaaattg gaagaagctg gattccatac tgtggaggct gttgcctatg 420
cgccaaagaa ggagctaata aatattaagg gaattagtga agccaaagct gataaaattc 480
tggctgaggc agctaaatta gttccaatgg gtttcaccac tgcaactgaa ttccaccaaa 540
ggcggtcaga gatcatacag attactactg gctccaaaga gcttgacaaa ctacttcaag 600
gtggaattga gactggatct atcacagaaa tgtttggaga attccgaact gggaagaccc 660
agatctgtca tacgctagct gtcacctgcc agcttcccat tgaccggggt ggaggtgaag 720
gaaaggccat gtacattgac actgagggta cctttaggcc agaacggctg ctggcagtgg 780
ctgagaggta tggtctctct ggcagtgatg tcctggataa tgtagcatat gctcgagcgt 840
tcaacacaga ccaccagacc cagctccttt atcaagcatc agccatgatg gtagaatcta 900
ggtatgcact gcttattgta gacagtgcca ccgcccttta cagaacagac tactcgggtc 960
gaggtgagct ttcagccagg cagatgcact tggccaggtt tctgcggatg cttctgcgac 1020
tcgctgatga gtttggtgta gcagtggtaa tcactaatca ggtggtagct caagtggatg 1080
gagcagcgat gtttgctgct gatcccaaaa aacctattgg aggaaatatc atcgcccatg 1140
catcaacaac cagattgtat ctgaggaaag gaagagggga aaccagaatc tgcaaaatct 1200
acgactctcc ctgtcttcct gaagctgaag ctatgttcgc cattaatgca gatggagtgg 1260
gagatgccaa agactgaatc attgggtttt tcctctgtta aaaaccttaa gtgctgcagc 1320
ctaatgagag tgcactgctc cctggggttc tctacaggcc tcttcctgtt gtgactgcca 1380
ggataaagct tccgggaaaa cagctattat atcagctttt ctgatggtat aaacaggaga 1440
caggtcagta gtcacaaact gatctaaaat gtttattcct tctgtagtgt attaatctct 1500
gtgtgttttc tttggttttg gaggaggggt atgaagtatc tttgacatgg tgccttagga 1560
atgacttggg tttaacaagc tgtctactgg acaatcttat gtttccaaga gaactaaagc 1620
tggagagacc tgacccttct ctcacttcta aattaatggt aaaataaaat gcctcagcta 1680
tgtagcaaag ggaatgggtc tgcacagatt ctttttttct gtcagtaaaa ctctcaagca 1740
ggtttttaag ttgtctgtct gaatgatctt gtgtaagggt ttggttatgg agtcttgtgc 1800
caaacctact aggccattag cccttcacca tctacctgct tggtctttca ttgctaagac 1860
taactcaaga taatcctaga gtcttaaagc atttcaggcc agtgtggtgt cttgcgcctg 1920
tactcccagc actttgggag gccgaggcag gtggatcgct tgagccagga gttttaagtc 1980
cagcttggcc aagatggtga aatcccatct ctacaaaaaa tgcagaactt aatctggaca 2040
cactgttaca cgtgcctgta gtcccagcta ctctatagcc tgaggtggga gaatcactta 2100
agcctggaag gtggaagttg cagtgagtcg agattgcact gctgcattcc agccagggtg 2160
acagagtgag accatgtttc aaacaagaaa catttcagag ggcaagtaaa cagatttgat 2220
tgtgaggctt ctaataaagt agttattagt agtg 2254

<210> 49
<211> 1994
<212> DNA
<213> Homo sapiens

<400> 49
aaacttcccg cacgcgttac aggagccagg tcggtataag cgccagcggc ctcgccgccc 60
gtcaagctgt ccacatccct ggcctcagcc cgccacatca ccctgacctg cttacgccca 120
gattttcttc aatcacatct gaataaatca cttgaagaaa gcttatagct tcattgcacc 180
atgtgtggca tttgggcgct gtttggcagt gatgattgcc tttctgttca gtgtctgagt 240
gctatgaaga ttgcacacag aggtccagat gcattccgtt ttgagaatgt caatggatac 300
accaactgct gctttggatt tcaccggttg gcggtagttg acccgctgtt tggaatgcag 360
ccaattcgag tgaagaaata tccgtatttg tggctctgtt acaatggtga aatctacaac 420
cataagaaga tgcaacagca ttttgaattt gaataccaga ccaaagtgga tggtgagata 480
atccttcatc tttatgacaa aggaggaatt gagcaaacaa tttgtatgtt ggatggtgtg 540
tttgcatttg ttttactgga tactgccaat aagaaagtgt tcctgggtag agatacatat 600
ggagtcagac ctttgtttaa agcaatgaca gaagatggat ttttggctgt atgttcagaa 660
gctaaaggtc ttgttacatt gaagcactcc gcgactccct ttttaaaagt ggagcctttt 720
cttcctggac actatgaagt tttggattta aagccaaatg gcaaagttgc atccgtggaa 780
atggttaaat atcatcactg tcgggatgaa cccctgcacg ccctctatga caatgtggag 840
aaactctttc caggttttga gatagaaact gtgaagaaca acctcaggat ccttttttaat 900
aatgctgtaa agaaacgttt gatgacagac agaaggattg gctgcctttt atcagggggc 960
ttggactcca gcttggttgc tgccactctg ttgaagcagc tgaaagaagc ccaagtacag 1020
tatcctctcc agacatttgc aattggcatg gaagacagcc ccgatttact ggctgctaga 1080
aaggtggcag atcatattgg aagtgaacat tatgaagtcc tttttaactc tgaggaaggc 1140
attcaggctc tggatgaagt catattttcc ttggaaactt atgacattac aacagttcgt 1200
gcttcagtag gtatgtattt aatttccaag tatattcgga agaacacaga tagcgtggtg 1260
atcttctctg gagaaggatc agatgaactt acgcagggtt acatatattt tcacaaggct 1320
ccttctcctg aaaaagccga ggaggagagt gagaggcttc tgagggaact ctatttgttt 1380
gatgttctcc gcgcagatcg aactactgct gcccatggtc ttgaactgag agtcccattt 1440
ctagatcatc gattttcttc ctattacttg tctctgccac cagaaatgag aattccaaag 1500
aatgggatag aaaaacatct cctgagagag acgtttgagg attccaatct gatacccaaa 1560
gagattctct ggcgaccaaa agaagccttc agtgatggaa taacttcagt taagaattcc 1620
tggtttaaga ttttacagga atacgttgaa catcaggttg atgatgcaat gatggcaaat 1680
gcagcccaga aatttccctt caatactcct aaaaccaaag aaggatatta ctaccgtcaa 1740
gtctttgaac gccattaccc aggccgggct gactggctga gccattactg gatgcccaag 1800
tggatcaatg ccactgaccc ttctgcccgc acgctgaccc actacaagtc agctgtcaaa 1860
gcttaggtgg tctttatgct gtaatgtgaa agcaaatatt tcttcgtgtt ggatggggac 1920
tgtgggtaga taggggaaca atgagagtca actcaggcta acttgggtgt gaaaaaaata 1980
aaagtcctaa atct 1994

<210> 50
<211> 1599
<212> DNA
<213> Homo sapiens
<400> 50
actcttggga aaactgctgg gcaccgtcgt cgcgctgaag gtggttctgt acctgctccg 60
agtgtgctta gcgatggcct ggaaatccgg cggcgccagc cactcggagc taatccacaa 120
tctccgcaaa aatggaatca tcaagacaga taaagtattt gaagtgatgc tggctacaga 180
ccgctcccac tatgcaaaat gtaacccata catggattct ccacaatcaa taggtttcca 240
agcaacaatc agtgctccac acatgcatgc atatgcgcta gaacttctat ttgatcagtt 300
gcatgaagga gctaaagctc ttgatgtagg atctggaagt ggaatcctta ctgcatgttt 360
tgcacgtatg gttggatgta ctggaaaagt cataggaatt gatcacatta aagagctagt 420
agatgactca gtaaataatg tcaggaagga cgatccaaca cttctgtctt cagggagagt 480
acagcttgtt gtgggggatg gaagaatggg atatgctgaa gaagcccctt atgatgccat 540
tcatgtggga gctgcagccc ctgttgtacc ccaggcgcta atagatcagt taaagcccgg 600
aggaagattg atattgcctg ttggtcctgc aggcggaaac caaatgttgg agcagtatga 660
caagctacaa gatggcagca tcaaaatgaa gcctctgatg gggtgatat acgtgccttt 720
aacagataaa gaaaagcagt ggtccaggtg gaagtgattt tatcttctgc tctttcttct 780
tccacacatg caagggatga attgtaaaag caacatcagc ttgaccagta taaaattaca 840
gtggattgct catctcagtc ctcaaagctt tttgaaaacc aacaccatca cagcttgttt 900
tggactttgt tacactgtta ttttcagcat gaaaatgtgt gttttttttag ggtttctgat 960
tcttcaaaga ggcacagagc caaattggta gaggaaggat gcaaagtata aatttgtgta 1020
atattacttt aacatgccca tattttactt ggaaatatta aaagaaaggg ttctgtaaaa 1080
tggaaaactt agtttgtgaa ttgattttga ggagtggttt ttcttttctt ggacacttaa 1140
ttctgttctg atattaattt atcagattgc ttttgtgcat tggataacac caccattcac 1200
aagttaagat tcttggtatt tggatatctg ttagatgcta ctaagaaaat agagatgagc 1260
tttcttttta aagcttttga tgtggtgtca tagaatagca tgttgtagat acaatcagct 1320
gctttgttac cttaaaacta ggcatttgta aatattaaac cttaagatgg caggtgatgt 1380
cctgtaaaca ctcagctgtt cagattggac ataactgact tagttcttcc cttctctctc 1440
tctcaaaatt ataggagact tgtagtttag tgtggttttc tgtttctaat ttgctgctga 1500
taatgtatat gaacttaaca tgctgtgtaa gctgtgtcct agttcttgaa cagtttatgc 1560
agtgctgctt tgccaaataa agttaaaagt aaaagaggc 1599

<210> 51
<211> 6450
<212> DNA
<213> Homo sapiens
<400> 51
gagttgtgcc tggagtgatg tttaagccaa tgtcagggca aggcaacagt ccctggccgt 60
cctccagcac ctttgtaatg catatgagct cgggagacca gtacttaaag ttggaggccc 120
gggagcccag gagctggcgg agggcgttcg tcctgggagc tgcacttgct ccgtcgggtc 180
gccggcttca ccggaccgca ggctcccggg gcagggccgg ggccagagct cgcgtgtcgg 240
cgggacatgc gctgcgtcgc ctctaacctc gggctgtgct ctttttccag gtggcccgcc 300
ggtttctgag ccttctgccc tgcggggaca cggtctgcac cctgcccgcg gccacggacc 360
atgaccatga ccctccacac caaagcatct gggatggccc tactgcatca gatccaaggg 420
aacgagctgg agcccctgaa ccgtccgcag ctcaagatcc ccctggagcg gcccctgggc 480
gaggtgtacc tggacagcag caagcccgcc gtgtacaact accccgaggg cgccgcctac 540
gagttcaacg ccgcggccgc cgccaacgcg caggtctacg gtcagaccgg cctcccctac 600
ggcccccgggt ctgaggctgc ggcgttcggc tccaacggcc tgggggggttt cccccccactc 660
aacagcgtgt ctccgagccc gctgatgcta ctgcacccgc cgccgcagct gtcgcctttc 720
ctgcagcccc acggccagca ggtgccctac tacctggaga acgagcccag cggctacacg 780
gtgcgcgagg ccggcccgcc ggcattctac aggccaaatt cagataatcg acgccagggt 840
ggcagagaaa gattggccag taccaatgac aagggaagta tggctatgga atctgccaag 900
gagactcgct actgtgcagt gtgcaatgac tatgcttcag gctaccatta tggagtctgg 960
tcctgtgagg gctgcaaggc cttcttcaag agaagtattc aaggacataa cgactatatg 1020
tgtccagcca ccaaccagtg caccattgat aaaaacagga ggaagagctg ccaggcctgc 1080
cggctccgca aatgctacga agtgggaatg atgaaaggtg ggatacgaaa agaccgaaga 1140
ggagggagaa tgttgaaaca caagcgccag agagatgatg gggagggcag ggtgaagtg 1200
gggtctgctg gagacatgag agctgccaac ctttggccaa gcccgctcat gatcaaacgc 1260
tctaagaaga acagcctggc cttgtccctg acggccgacc agatggtcag tgccttgttg 1320
gatgctgagc cccccatact ctattccgag tatgatccta ccagaccctt cagtgaagct 1380
tcgatgatgg gcttactgac caacctggca gacagggagc tggttcacat gatcaactgg 1440
gcgaagaggg tgccaggctt tgtggatttg accctccatg atcaggtcca ccttctagaa 1500
tgtgcctggc tagagatcct gatgattggt ctcgtctggc gctccatgga gcacccagtg 1560
aagctactgt ttgctcctaa cttgctcttg gacaggaacc agggaaaatg tgtagagggc 1620
atggtggaga tcttcgacat gctgctggct acatcatctc ggttccgcat gatgaatctg 1680
cagggagagg agtttgtgtg cctcaaatct attattttgc ttaattctgg agtgtacaca 1740
tttctgtcca gcaccctgaa gtctctggaa gagaaggacc atatccaccg agtcctggac 1800
aagatcacag acactttgat ccacctgatg gccaaggcag gcctgaccct gcagcagcag 1860
caccagcggc tggcccagct cctcctcatc ctctcccaca tcaggcacat gagtaacaaa 1920
ggcatggagc atctgtacag catgaagtgc aagaacgtgg tgcccctcta tgacctgctg 1980
ctggagatgc tggacgccca ccgcctacat gcgcccacta gccgtggagg ggcatccgtg 2040
gaggagacgg accaaagcca cttggccact gcgggctcta cttcatcgca ttccttgcaa 2100
aagtattaca tcacgggggga ggcagagggt ttccctgcca cagtctgaga gctccctggc 2160
tcccacacgg ttcagataat ccctgctgca ttttaccctc atcatgcacc actttagcca 2220
aattctgtct cctgcataca ctccggcatg catccaacac caatggcttt ctagatgagt 2280
ggccattcat ttgcttgctc agttcttagt ggcacatctt ctgtcttctg ttgggaacag 2340
ccaaagggat tccaaggcta aatctttgta acagctctct ttcccccttg ctatgttact 2400
aagcgtgagg attcccgtag ctcttcacag ctgaactcag tctatgggtt ggggctcaga 2460
taactctgtg catttaagct acttgtagag acccaggcct ggagagtaga cattttgcct 2520
ctgataagca ctttttaaat ggctctaaga ataagccaca gcaaagaatt taaagtggct 2580
cctttaattg gtgacttgga gaaagctagg tcaagggttt attatagcac cctcttgtat 2640
tcctatggca atgcatcctt ttatgaaagt ggtacacctt aaagctttta tatgactgta 2700
gcagagtatc tggtgattgt caattcactt cccccctatag gaatacaagg ggccacacag 2760
ggaaggcaga tcccctagtt ggccaagact tattttaact tgatacactg cagattcaga 2820
gtgtcctgaa gctctgcctc tggctttccg gtcatgggtt ccagttaatt catgcctccc 2880
atggacctat ggagagcaac aagttgatct tagttaagtc tccctatatg agggataagt 2940
tcctgatttt tgtttttatt tttgtgttac aaaagaaagc cctccctccc tgaacttgca 3000
gtaaggtcag cttcaggacc tgttccagtg ggcactgtac ttggatcttc ccggcgtgtg 3060
tgtgccttac acaggggtga actgttcact gtggtgatgc atgatgaggg taaatggtag 3120
ttgaaaggag caggggccct ggtgttgcat ttagccctgg ggcatggagc tgaacagtac 3180
ttgtgcagga ttgttgtggc tactagagaa caagagggaa agtagggcag aaactggata 3240
cagttctgag cacagccaga cttgctcagg tggccctgca caggctgcag ctacctagga 3300
acattccttg cagaccccgc attgcctttg gggggtgccct gggatccctg gggtagtcca 3360
gctcttattc atttcccagc gtggccctgg ttggaagaag cagctgtcaa gttgtagaca 3420
gctgtgttcc tacaattggc ccagcaccct ggggcacggg agaagggtgg ggaccgttgc 3480
tgtcactact caggctgact ggggcctggt cagattacgt atgcccttgg tggtttagag 3540
ataatccaaa atcagggttt ggtttgggga agaaaatcct ccccccttcct cccccgcccc 3600
gttccctacc gcctccactc ctgccagctc atttccttca atttcctttg acctataggc 3660
taaaaaagaa aggctcattc cagccacagg gcagccttcc ctgggccttt gcttctctag 3720
cacaattatg ggttacttcc tttttcttaa caaaaaagaa tgtttgattt cctctgggtg 3780
accttattgt ctgtaattga aaccctattg agaggtgatg tctgtgttag ccaatgaccc 3840

```
aggtagctgc tcgggcttct cttggtatgt cttgtttgga aaagtggatt tcattcattt   3900
ctgattgtcc agttaagtga tcaccaaagg actgagaatc tgggagggca aaaaaaaaaa   3960
aaaaagtttt tatgtgcact taaatttggg gacaatttta tgtatctgtg ttaaggatat   4020
gcttaagaac ataattcttt tgttgctgtt tgtttaagaa gcaccttagt ttgtttaaga   4080
agcaccttat atagtataat atatattttt ttgaaattac attgcttgtt tatcagacaa   4140
ttgaatgtag taattctgtt ctggatttaa tttgactggg ttaacatgca aaaaccaagg   4200
aaaaatattt agtttttttt tttttttttg tatacttttc aagctacctt gtcatgtata   4260
cagtcattta tgcctaaagc ctggtgatta ttcatttaaa tgaagatcac atttcatatc   4320
aacttttgta tccacagtag acaaaatagc actaatccag atgcctattg ttggatattg   4380
aatgacagac aatcttatgt agcaaagatt atgcctgaaa aggaaaatta ttcagggcag   4440
ctaattttgc ttttaccaaa atatcagtag taatattttt ggacagtagc taatgggtca   4500
gtgggttctt tttaatgttt atacttagat tttcttttaa aaaaattaaa ataaaacaaa   4560
aaaaatttct aggactagac gatgtaatac cagctaaagc caaacaatta tacagtggaa   4620
ggttttacat tattcatcca atgtgtttct attcatgtta agatactact acatttgaag   4680
tgggcagaga acatcagatg attgaaatgt tcgcccaggg gtctccagca actttggaaa   4740
tctctttgta tttttacttg aagtgccact aatggacagc agatattttc tggctgatgt   4800
tggtattggg tgtaggaaca tgatttaaaa aaaaaactct tgcctctgct ttcccccact   4860
ctgaggcaag ttaaaatgta aaagatgtga tttatctggg gggctcaggt atggtgggga   4920
agtggattca ggaatctggg gaatggcaaa tatattaaga agagtattga aagtatttgg   4980
aggaaaatgg ttaattctgg gtgtgcacca aggttcagta gagtccactt ctgccctgga   5040
gaccacaaat caactagctc catttacagc catttctaaa atggcagctt cagttctaga   5100
gaagaaagaa caacatcagc agtaaagtcc atggaatagc tagtggtctg tgtttctttt   5160
cgccattgcc tagcttgccg taatgattct ataatgccat catgcagcaa ttatgagagg   5220
ctaggtcatc caaagagaag accctatcaa tgtaggttgc aaaatctaac ccctaaggaa   5280
gtgcagtctt tgatttgatt tccctagtaa ccttgcagat atgtttaacc aagccatagc   5340
ccatgccttt tgagggctga acaaataagg gacttactga taatttactt ttgatcacat   5400
taaggtgttc tcaccttgaa atcttataca ctgaaatggc cattgattta ggccactggc   5460
ttagagtact ccttcccctg catgacactg attacaaata ctttcctatt catactttcc   5520
aattatgaga tggactgtgg gtactgggag tgatcactaa caccatagta atgtctaata   5580
ttcacaggca gatctgcttg ggaagctag ttatgtgaaa ggcaaataaa gtcatacagt   5640
agctcaaaag gcaaccataa ttctctttgg tgcaagtctt gggagcgtga tctagattac   5700
actgcaccat tcccaagtta atcccctgaa aacttactct caactggagc aaatgaactt   5760
tggtcccaaa tatccatctt ttcagtagcg ttaattatgc tctgtttcca actgcatttc   5820
ctttccaatt gaattaaagt gtggcctcgt ttttagtcat ttaaaattgt tttctaagta   5880
attgctgcct ctattatggc acttcaattt tgcactgtct tttgagattc aagaaaaatt   5940
tctattcatt tttttgcatc caattgtgcc tgaactttta aaatatgtaa atgctgccat   6000
gttccaaacc catcgtcagt gtgtgtgttt agagctgtgc accctagaaa caacatactt   6060
gtcccatgag caggtgcctg agacacagac ccctttgcat tcacagagag gtcattggtt   6120
atagagactt gaattaataa gtgacattat gccagtttct gttctctcac aggtgataaa   6180
caatgctttt tgtgcactac atactcttca gtgtagagct cttgttttat gggaaaggc   6240
tcaaatgcca aattgtgttt gatggattaa tatgcccttt tgccgatgca tactattact   6300
gatgtgactc ggttttgtcg cagctttgct ttgtttaatg aaacacactt gtaaacctct   6360
tttgcacttt gaaaaagaat ccagcgggat gctcgagcac ctgtaaacaa ttttctcaac   6420
ctatttgatg ttcaaataaa gaattaaact                                    6450
```

<210> 52
<211> 1518
<212> DNA
<213> Homo sapiens
<400> 52

```
ggccgctagg ggtgcggggt tggggaggag gccgctagtc tacgcctgtg gagccgatac     60
tcagccctct gcgaccatgg ctgtgctggc ggcacttctg cgcagcggcg cccgcagccg    120
cagccccctg ctccggaggc tggtgcagga aataagatat gtggaacgga gttatgtatc    180
aaaacccact ttgaaggaag tggtcatagt aagtgctaca agaacaccca ttggatcttt    240
tttaggcagc ctttccttgc tgccagccac taagcttggt tccattgcaa ttcagggagc    300
cattgaaaag gcagggattc caaaagaaga agtgaaagaa gcatacatgg gtaatgttct    360
acaaggaggt gaaggacaag ctcctacaag gcaggcagta ttgggtgcag gcttacctat    420
ttctactcca tgtaccacca taaacaaagt ttgtgcttca ggaatgaaag ccatcatgat    480
ggcctctcaa agtcttatgt gtggacatca ggatgtgatg gtggcaggtg ggatggagag    540
catgtccaat gttccatatg taatgaacag aggatcaaca ccatatggtg gggtaaagct    600
tgaagatttg attgtaaaag acgggctaac tgatgtctac aataaaattc atatgggcag    660
ctgtgctgag aatacagcaa agaagctgaa tattgcacga aatgaacagg acgcttatgc    720
tattaattct tataccagaa gtaaagcagc atgggaagct gggaaatttg gaaatgaagt    780
tattcctgtc acagttacag taaaaggtca accagatgta gtggtgaaag aagatgaaga    840
atataaacgt gttgatttta gcaaagttcc aaagctgaag acagttttcc agaaagaaaa    900
tggcacagta acagctgcca atgccagtac actgaatgat ggagcagctg ctctggttct    960
catgacggca gatgcagcga agaggctcaa tgttacacca ctggcaagaa tagtagcatt   1020
tgctgacgct gctgtagaac ctattgattt tccaattgct cctgtatatg ctgcatctat   1080
ggttcttaaa gatgtgggat tgaaaaaaga agatattgca atgtgggaag taaatgaagc   1140
```

ctttagtctg gttgtactag caaacattaa aatgttggag attgatcccc aaaaagtgaa 1200
tatcaatgga ggagctgttt ctctgggaca tccaattggg atgtctggag ccaggattgt 1260
tggtcatttg actcatgcct tgaagcaagg agaatacggt cttgccagta tttgcaatgg 1320
aggaggaggt gcttctgcca tgctaattca gaagctgtag acaacctctg ctatttaagg 1380
agacaaccct atgtgaccag aaggcctgct gtaatcagtg tgactactgt gggtcagctt 1440
atattcagat aagctgtttc attttttatt attttctatg ttaactttta aaaatcaaaa 1500
tgatgaaatc ccaaaaaca 1518

<210> 53
<211> 1439
<212> DNA
<213> Homo sapiens
<400> 53
ggctcgcctc ggcgtgcagt gcgcgtgcgt ggagctggga gctaggtcct cggagtgggc 60
cggagatggc ggcggccgac ggggctttgc cggaggcggc ggctttagag caacccgcgg 120
agctgcctgc ctcggtgcgg gcgagtatcg agcggaagcg gcagcgggca ctgatgctgc 180
gccaggcccg gctggctgcc cggccctact cggcgacggc ggctgcggct actggaggca 240
tggctaatgt aaaagcagcc ccaaagataa ttgacacagg aggaggcttc attttagaag 300
aggaagaaga agaagaacag aaaattggaa aagttgttca tcaaccagga cctgttatgg 360
aatttgatta tgtaatatgc gaagaatgtg ggaaagaatt tatggattct tatcttatga 420
accactttga tttgccaact tgtgataact gcagagatgc tgatgataaa cacaagctta 480
taaccaaaac agaggcaaaa caagaatatc ttctgaaaga ctgtgattta gaaaaaagag 540
agccacctct taaatttatt gtgaagaaga atccacatca ttcacaatgg ggtgatatga 600
aactctactt aaagttacag attgtgaaga ggtctcttga agtttggggt agtcaagaag 660
cattagaaga agcaaaggaa gtccgacagg aaaaccgaga aaaaatgaaa cagaagaaat 720
ttgataaaaa agtaaaagaa ttgcggcgag cagtaagaag cagcgtgtgg aaaagggaga 780
cgattgttca tcaacatgag tatggaccag aagaaaacct agaagatgac atgtaccgta 840
agacttgtac tatgtgtggc catgaactga catatgaaaa aatgtgattt tttagttcag 900
tgacctgttt tatagaattt tatatttaaa taaaggaaat ttagattggt ccttttcaaa 960
attcaaaaaa aaaagcaaca tcttcataga tgaatgaaac ccttgtataa gtaatacttc 1020
agtaataatt atgtatgtta tggcttaaaa gcaagtttca gtgaaggtca cctggcctgg 1080
ttgtgtgcac aatgtcatgt ctgtgattgc cttcttacaa cagagatggg agctgagtgc 1140
tagagtaggt gcagaagtgg taggtcagct acaaatttga ggacaagata ccaaggcaaa 1200
ccctagattg ggtagagggg aaaagggttc aacaaaggct gaactggatt cttaaccaag 1260
aaacaaataa tagcaatggt ggtgcaccac tgtaccccag gttctagtca tgtgtttttt 1320
aggacgattt ctgtctccac gatggtggaa acagtgggga actactgctg gaaaaagccc 1380
taatagcaga aataaacatt gagttgtacg agtctgaaaa aaaaaaaaaa aaaaaaaaa 1439

<210> 54
<211> 3857
<212> DNA
<213> Homo sapiens
<400> 54
ggccgagcct cggcggcggc ggtagcggcg gcggcgacgc tgacacctcc caccatggac 60
agcttcgact tagccctgct ccaggaatgg gacctcgagt cactgtgtgt ctatgaacca 120
gatagaaatg cattacggag gaaagaacga gaaagaagaa atcaagaaac tcaacaggat 180
gatggcacgt ttaattctag ttactctctc ttcagtgagc cctacaagac taacaagggg 240
gatgaactct ccaaccggat ccagaacact ttaggcaatt atgatgaaat gaaagacttt 300
ttaactgata gaaccaatca gagtcatctc gttggagttc ccaaacctgg ggttcctcag 360
actcctgtga acaagatcga tgaacatttt gttgcagatt caagagccca gaaccagccc 420
tcgtctatct gtagcactac aacttccaca ccagcagctg tccccgtgca gcagagtaaa 480
agaggcacta tgggctggca gaaggctggg cacccacccct ctgacggcca acagagagca 540
acacaacagg gctctctcag gaccttgctt ggagatggtg ttggcagaca gcagcctcgg 600
gccaaacaag tgtgcaatgt ggaggtgggc cttcagaccc aggagaggcc acctgccatg 660
gcggccaagc acagcagcag cggacactgt gttcagaact ttcctccatc cctagcttca 720
aaacccagcc tggtccagca gaaaccgacc gcgtatgtga ggccaatgga cggccaagat 780
caggcccctg atgagtctcc taagctgaag tcgtcttcgg aaaccagcgt gcactgcaca 840
tcatacaggg gagtccctgc cagcaagccg gagcctgcca gagccaaggc caagctctcc 900
aagttcagca tccccaagca gggggaggag agtagatctg gagaaaccaa cagctgtgtt 960
gaagaaataa tccgggagat gacctggctt ccaccacttt ctgctattca agcacctggc 1020
aaagtggaac caaccaaatt tccatttcca aataaggact ctcagcttgt atcctctgga 1080
cacaataatc caaagaaagg tgatgcagag ccagagagtc cagacaatgg cacatcgaat 1140
acatcaatgc tggaagatga ccttaagcta agcagtgatg aagaggagaa tgaacagcag 1200
gcagctcaga gaacggctct ccgcgctctc tctgacagcg ccgtggtcca gcagcccaac 1260
tgcagaacct cggtgccttc cagcaagggc agcagcagca gcagcagcag cggcacgagc 1320
agctcctcca gcgactcaga gagcagctcc ggatctgact cggagaccga gagcagctcc 1380
agcgagagtg agggcagcaa gcccccccac ttctccagcc ccgaggctga accggcatcc 1440
tctaacaagt ggcagctgga taaatggcta aacaaagtta atccccacaa gcctcctatt 1500
ctgatccaaa atgaaagcca cgggtcagag agcaatcagt actacaaccc ggtgaaagag 1560

```
gacgtccagg actgtgggaa agtccccgac gtttgccagc ccagcctgag agagaaggag   1620
atcaagagca cttgcaagga ggagcaaagg ccaaggacag ccaacaaggc ccctgggagt   1680
aaaggcgtga agcagaagtc cccgcccgcg gccgtggccg tggcggtgag cgcagccgcc   1740
ccgccacccg cagtgccctg tgcgcccgcg gagaacgcgc ccgcgcctgc ccggaggtcc   1800
gcgggcaaga agcccaccag gcgcaccgag aggacctcag ccggggacgg cgccaactgc   1860
caccggcccg aggagcccgc ggccgcggac gcgctgggga cgagcgtggt ggtcccccg    1920
gagcccacca aaaccaggcc ctgtggcaac aacagagcga gccaccgcaa ggagctgcgc   1980
tcctccgtga cctgcgagaa gcgccgcacg cgggggctaa gcaggatcgt ccccaaatcc   2040
aaggagttca ttgagacaga gtcgtcatct tcatcctcct cctcggactc cgacctggag   2100
tccgagcagg aggagtaccc tctgtccaaa gcacagaccg tggctgcctc tgcctcctcc   2160
gggaatgatc agaggctgaa ggaggccgct gccaacgggg gcagtggtcc tagggcccct   2220
gtaggctcca tcaacgccag gaccaccagt gacatcgcca aggagctgga ggagcagttc   2280
tacacactgg tcccctttgg ccggaacgaa cttctctccc ctctaaagga cagtgatgag   2340
atcaggtctc tctgggtcaa aatcgacctg accctcctgt ccaggatccc agaacacctg   2400
ccccaggagc caggggtatt gagcgcccct gccaccaagg actctgagag cgcaccgccc   2460
agccacacct cggacacacc tgcagaaaag gctttgccaa aatccaagag gaaacgcaag   2520
tgtgacaacg aagacgacta cagggagatc aagaagtccc agggagagaa agacagctct   2580
tcaagactgg ccacctccac cagtaatact ttgtctgcaa accactgcaa catgaacatc   2640
aacagtgtgg caataccaat aaataaaaat gaaaaaatgc ttcggtcgcc catctcaccc   2700
ctctctgatg catctaaaca caaatacacc agcgaggact taacttcttc cagccgacct   2760
aatggcaaca gtttgtttac ttcagcctct tccagcaaaa agcctaaggc cgacagccag   2820
ctgcagcctc acggcggaga cctcacgaaa gcagctcaca acaattctga aaacattccc   2880
ctccacaagt cacggccgca gacgaagccg tggtctccag gctccaacgg ccacagggac   2940
tgcaagaggc agaaacttgt cttcgatgat atgcctcgca gtgccgatta ttttatgcaa   3000
gaagctaaac gaatgaagca taaagcagat gcaatggtgg aaaagtttgg aaaggctttg   3060
aactatgctg aagcagcatt gtcgtttatc gagtgtggaa atgcaatgga acaaggcccc   3120
atggaatcca aatctcctta ttacctgatg tattcagaaa cagtagagct catcaggtat   3180
gctatgagac taaaaaccca ctcaggcccc aatgccacac cagaagacaa acaactggct   3240
gcattatgtt accgatgcct ggccctcctg tactggcgga tgtttcgact caaaagggac   3300
cacgctgtaa agtattcaaa agcactaatc gactatttca agaactcatc taaagccgcc   3360
caagccccat ctccgtgggg ggccagtgga aagagcactg gaaccccatc cccccatttct  3420
cccaacccct ttcccggcag ctccgtgggg tctcagggca gcctctccaa cgccagcgcc   3480
ctgtccccgt cgaccatcgt cagcatccca cagcgcatcc accagatggc ggccaaccac   3540
gtcagcatca ccaacagcat cctgcacagc tacgactact gggagatggc cgacaacctg   3600
gccaaggaaa accgagaatt cttcaacgac ctggatctgc tcatggggcc ggtcaccctg   3660
cacagcagca tggagcacct ggtccagtac tcccaacagg gcctgcactg gctgcggaac   3720
agcgcccacc tgtcataggg acctcaccct ggggccagag tgggctctgg tctccacaga   3780
tggctcaacg tttttggaca ctgtgctact gaaactccca gccacagcat ttatagactg   3840
cggtgaacat ttcctca                                                 3857
```

```
<210>  55
<211>  3285
<212>  DNA
<213>  Homo sapiens
<400>  55
caccttctgc actgctcatc tgggcagagg aagcttcaga aagctgccaa ggcaccatct     60
ccaggaactc ccagcacgca gaatccatct gagaatatgc tgccacaaat acccttttg     120
ctgctagtat ccttgaactt ggttcatgga gtgttttacg ctgaacgata ccaaatgccc    180
acaggcataa aaggcccact acccaacacc aagacacagt tcttcattcc ctacaccata    240
aagagtaaag gtatagcagt aagaggagag caaggtactc ctggtccacc aggccctgct    300
ggacctcgag ggcacccagg tccttctgga ccaccaggaa aaccaggcta cggaagtcct    360
ggactccaag gagagccagg gttgccagga ccaccgggac catcagctgt agggaaacca    420
ggtgtgccag gactcccagg aaaaccagga gagagaggac catatggacc aaaaggagat    480
gttggaccag ctggcctacc aggaccccgg ggcccaccag gaccacctgg aatccctgga    540
ccggctggaa tttctgtgcc aggaaaacct ggacaacagg gacccacagg agccccagga    600
cccaggggct ttcctggaga aaagggtgca ccaggagtcc ctggtatgaa tggacagaaa    660
ggggaaatgg gatatggtgc tcctggtcgt ccaggtgaga ggggtcttcc aggccctcag    720
ggtcccacag gaccatctgg ccctcctgga gtgggaaaaa gaggtgaaaa tggggttcca    780
ggacagccag gcatcaaagg tgatagaggt tttccgggag aaatgggacc aattggccca    840
ccaggtcccc aaggccctcc tggggaacga gggccagaag gcattggaaa gccaggagct    900
gctggagccc caggccagcc aggattccca ggaacaaaag gtctccctgg ggctccagga    960
atagctgggc ccccagggcc tcctggcttt gggaaaccag gcttgccagg cctgaaggga   1020
gaaagaggac ctgctggcct tcctgggggt ccaggtgcca aaggggaaca aggccagca    1080
ggtcttcctg ggaagccagg tctgactgga ccccctggga atatgggacc ccaaggacca   1140
aaaggcatcc cgggtagcca tggtctccca ggccctaaag gtgagacagg gccagctggg   1200
cctgcaggat accctggggc taagggtgaa aggggttccc ctgggtcaga tggaaaacca   1260
gggtacccag gaaaaccagg tctcgatggt cctaagggta acccagggtt accaggtcca   1320
aaaggtgatc ctggagttgg aggacctcct ggtctcccag gccctgtggg cccagcagga   1380
gcaaagggaa tgcccggaca caatggagag gctggcccaa gaggtgcccc tggaatacca   1440
```

```
ggtactagag gccctattgg gccaccaggc attccaggat tccctgggtc taaaggggat   1500
ccaggaagtc ccggtcctcc tggcccagct ggcatagcaa ctaagggcct caatggaccc   1560
accgggccac cagggcctcc aggtccaaga ggccactctg gagagcctgg tcttccaggg   1620
ccccctgggc ctccaggccc accaggtcaa gcagtcatgc ctgagggttt tataaaggca   1680
ggccaaaggc ccagtctttc tgggacccct cttgttagtg ccaaccaggg ggtaacagga   1740
atgcctgtgt ctgcttttac tgttattctc tccaaagctt acccagcaat aggaactccc   1800
ataccatttg ataaaatttt gtataacagg caacagcatt atgacccaag gactggaatc   1860
tttacttgtc agataccagg aatatactat ttttcatacc acgtgcatgt gaaagggact   1920
catgtttggg taggcctgta taagaatggc accccctgtaa tgtacaccta tgatgaatac   1980
accaaaggct acctggatca ggcttcaggg agtgccatca tcgatctcac agaaaatgac   2040
caggtgtggc tccagcttcc caatgccgag tcaaatggcc tatactcctc tgagtatgtc   2100
cactcctctt tctcaggatt cctagtggct ccaatgtgag tacaccccac agagctaatc   2160
taaatcttgt gctagaaaaa gcattctcta actctacccc accctacaaa atgcatatgg   2220
aggtaggctg aaaagaatgt aatttttatt ttctgaaata cagatttgag ctatcagacc   2280
aacaaacctt cccccctgaaa agtgagcagc aacgtaaaaa cgtatgtgaa gcctctcttg   2340
aatttctagt tagcaatctt aaggctcttt aaggttttct ccaatattaa aaaatatcac   2400
caaagaagtc ctgctatgtt aaaaacaaac aacaaaaaac aaagcaacaa aaaaaaaaat   2460
taaaaaaaaa aacagaaata gagctctaag ttatgtgaaa tttgatttga gaaactcggc   2520
atttcctttt taaaaaagcc tgtttctaac tatgaatatg agaacttcta ggaaacatcc   2580
aggaggtatc atataacttt gtagaactta aatacttgaa tattcaaatt taaaagacac   2640
tgtatcccct aaaatatttc tgatggtgca ctactctgag gcctgtatgg cccctttcat   2700
caatatctat tcaaatatac aggtgcatat atacttgtta aagctcttat ataaaaaagc   2760
cccaaaatat tgaagttcat ctgaaatgca aggtgctttc atcaatgaac cttttcaaaa   2820
cttttctatg attgcagaga agctttttat atacccagca tagcttttgga acaggtatct   2880
gacctattct tatttagtta acacaagtgt gattaatttg atttctttaa ttccttattg   2940
aatcttatgt gatatgattt tctggattta cagaacatta gcacatgtac cttgtgcctc   3000
ccattcaagt gaagttataa tttacactga gggtttcaaa attcgactag aagtggagat   3060
atattattta tttatgcact gtactgtatt tttatattgc tgtttaaaac ttttaagctg   3120
tgcctcactt attaaagcac aaaatgtttt acctactcct tatttacgac acaataaaat   3180
aacatcaata gatttttagg ctgaattaat ttgaaagcag caatttgctg ttctcaacca   3240
ttctttcaag gcttttcatt cgacacaata aaataacatc aatag                   3285
```

```
<210>  56
<211>  5341
<212>  DNA
<213>  Homo sapiens
<400>  56
gcgagacgcc ctctctcttc cagccgcagc cgcgttgcgg gttcttccct gtggaacagt     60
agagacctag tctgccttct ttcacaagat aatccttcaa ttatttgaag gtgattattc    120
agcatatatc tcttgagtac ctgtgcatgc aagacactgt cctggatatt gtgagagatc    180
caaagtttag tatgagatca ttttccctttg cctgagtttt gagctgattg agaaataaga    240
acctacagat gaagaagcaa gacagtacct ttaaaaaaat tagcttttta ttcagattct    300
ctttgtggcc agatgggttt gtatggacag gcttgtccat ctgtaacttc attaaggatg    360
acatctgaac tggagagcag cctaacgtct atggactggt taccacagct caccatgaga    420
gcagccatcc aaaaatctga tgctacacaa aatgcacatg gaacaggaat ttctaagaag    480
aatgcactcc ttgacccaaa tacaactctg gaccaggaag aagtccaaca gcacaaagat    540
gggaaacctc catacagtta tgccagcctc attacatttg caattaatag ctcacccaaa    600
aagaaaatga ctttaagtga aatttatcag tggatttgtg ataacttccc atattataga    660
gaggctggca gtggttggaa gaattccata cgacataatc tgtcattgaa caaatgtttc    720
cttaaagtgc ctcgatctaa ggatgaccct ggaaagggggt cctactgggc aatagacacc    780
aatccgaagg aagatgcgct gcctactcgg ccaaagaaga gggcacgatc tgtagaacgg    840
gcctcaactc catatagcat agattcagat tctttgggaa tggagtgtat tatttcggga    900
agtgcctctc caactctggc aatcaacact gtgactaaca aagtaacatt gtataacact    960
gatcaggatg gtagtgatag cccacgcagt agccttaaca acagtctctc agaccagagt   1020
ttggcatctg ttaatttgaa cagtgttgga agtgtgcata gttatacacc ggtgacaagc   1080
catccagaat cagtctctca atcattaact cctcagcagc aaccacagta caaccttcca   1140
gaaagagaca agcaactact tttctcagaa tataattttg aagatcttag tgcctcattt   1200
cggagccttt ataagtcagt ttttgagcag tcacttagtc aacaaggttt gatgaacatc   1260
ccttctgaat cttcccagca gtcccacact tcatgtacct atcagcactc tcccagcagt   1320
acagtgagca ctcacccaca cagcaaccaa agcagcctgt ccaacagtca tggcagtggc   1380
ctcaacacca caggcagtaa ttcggttgca caggtctcac tgtctcaccc ccagatgcac   1440
ccacagccat ctccacatcc tccccatcga ccgcatggtt taccgcagca tccgcagcgt   1500
tccccacacc cagcaccaca cccacagcaa cacagccagc tccagtcccc tcacccccag   1560
catccctctc cacatcaaca catacagcac catccgaacc atcagcatca gacgttaaca   1620
catcaggcac ccccaccccc acaacaggta tcctgtaatt ctggtgtttc aaatgattgg   1680
tatgcgacac ttgatatgct aaaagaaagc tgtcgaattg ccagcagtgt taattggtca   1740
gatgtagacc tttcacagtt tcaaggtctg atggagagta tgagacaggc agatctcaag   1800
aactggtctt tagaccaggt tcagtttgcc gatctttgtt cttctcttaa tcagttcttt   1860
acacaaactg gccttataca ttcacagagt aatgttcaac aaaatgtttg tcatggtgcc   1920
```

atgcatccaa caaaaccttc ccaacacatt ggaacaggaa atttgtacat agattctagg 1980
caaaatctcc ctccttcagt gatgccaccc cctggttatc ctcatatccc acaggcactc 2040
agcactccag gaacaacgat ggcaggccat cacagagcca tgaaccagca gcacatgatg 2100
ccttcccaag ccttccagat gcggcgttcc ctgcctccag atgacatcca ggatgacttt 2160
gattgggatt caattgtgta gggcttgttt ctgcaagaca ccagacccta acgttacctt 2220
tctgtgcagt gaagggaaag gtttaagaga atccagttga gaaaacaaac ttgctaatca 2280
ctttaccaat gttatcaaaa ttacttttga agacaatcag aaggatttta gctggataac 2340
ttactgcttt tatctgaccc aagcaagtac tacatgtttg tctccctgcc agctgcccta 2400
tgtagctcct aactgttgtg tgatttggac ggctttttgc atatttgtgt cagtttgatg 2460
ttaaccacaa gtgccagact gatttttcag acggagccta ttttgctgca agcagtttat 2520
ataaagatac atatgtgtaa atatatgtac aaaaattact gaaaggcttc agttttttct 2580
aattggatta ttatgtcttg aaagttattg tcagttttta ttccttgtta ggctattttc 2640
tgcaggatgc ttttaactga tgtaggaaac tgaaaggaaa tagatttttt ccaaaaccca 2700
gttcccctta tttaatcttt tttagaaatg tgggtaatga attctatcta ataagtcaag 2760
gaaaccagaa tttgacacac tccaacaatc caaaggggca tgttgctcct gagcagcatg 2820
aagaactgac caaattggtt ttgatgcttg ggggatcata gagtatttat gtctgctttt 2880
ctaaatctgc attataatag ctctaaaatt tgttgattgg taagaaattg ggcattgctt 2940
ggctctttaa acacatcagt gcttccacat tcacctatgt atttattatt caaaagtgtc 3000
attttaatat ttattgctac cttctgtgaa tgcttagctc ctgtcgggtt cattaaggag 3060
aaatgtgtct gaaagcacag aactattatt atttttttct ttttttgaga tggagtcttg 3120
ccctgttgcc caggctggag tgcagtggca cgatctcggc tcactgcaac ctccgcctcc 3180
cgggttcaag caattctcct gcctcagcct cccgagtagc tgggagtgca ggtgcacacc 3240
gccatgcctg gctgattttt tgtattttag tagagatggg gtttcaccat gttgcctggg 3300
ctggttgcaa actcttgagc tcaggcagtc tgcctgcctc agcctcctag agtgctggga 3360
ttacagacat gagccactgc gcctggccca gcgttacttt tcttgataag aatttacaga 3420
taagacactc agagagacat tgtgtcattc tctatcatca ctccttttac catgtgaata 3480
gatttctctg tcttggcatt cttgctgatt cagacttatt agttaaatcg gatttttctg 3540
gaatttgaat cagatttcat ttgggcaaca acagcagggc agggtttcta aagcaggttt 3600
ccccactcat ctaggttgtc ttgaaaggag gatagagcca cttacagttt tatttgcttc 3660
agtgtttaat gtagatatta tgtggcctgc tgtttctgtt gtcttgtatg tctgtgtatg 3720
catgacattt ggtccctttc tttgaaatgc agcccctctt acgggttttt tagtggtggt 3780
agtttgtttt gtttcatatc atatcacagt ttgcatggac tgattatctt agttttatga 3840
taaaactaga agaaatgagg gttttttttta atgaatagat tttgaattga ttctttagac 3900
cccccaaaaa gtgtcagttc taatggggaa atatattcca tcaagtcaaa gagtaataac 3960
tgctcactag ttgcttttta gcatctctgg tcttatcagc catgctaaat cactttaatt 4020
agccttagtg attctgtggt tgagtaatct ctacttgaac taaacaaaca tcttttgttt 4080
ctgtgtgtgt gtgctgtgtg tgagagtgtg cgcgcgcgtg tgtgtgtgtg tgttttaatg 4140
gagtgttgcc ttgaatgaat cactgggaag ccagccatgg taagggctgg tgaggttggg 4200
gagaaaggaa gagctttatg tttctctgtt gtttggaccc tacttggcat gaaaaaggaa 4260
gctcagttcc agccccttgg atcaacgaaa atcagaggat tctggaaagg cagccaactt 4320
gcgcctctta gaaggatcag aggcaagatg agatggcagc ctgcagagta aatgcttgaa 4380
aaaagagggg tgattttcaa tggtttgctt aagtcactgt tttctagaca ccaaaatagc 4440
tgttttgaaa ctgttttaat tgcttgggta gcaatgtgca ctttaaacaa tttggatatt 4500
ggaatgcagt ctcatactga gtgatttgag tagaaccact gatgatgatt ttataaattg 4560
tgtgaagcga atttcccatt tggcaatcat ttactgattt gcagtgatga atatttttat 4620
gagaatttaa acttagcaag aatggccatg gaggcaaagc cttcacccag acccatccca 4680
ctctcctgtg atccaggtgg tccaggagcc caggacaggc cttttctgtg ggccctggcc 4740
agacagggtt acctggtgag gtgcagagag tccctctagt ggccattttg tatggtagtt 4800
gctaatgcag aacaagttct gtcttgggct taaattgact gaagacttta gggggaaaga 4860
atagtaaatg catgtaaaca aatggggaca ctctgttcag gagaataatc cgactggcat 4920
ttgtggcagt ttttgaaatg taaatgtatt catgtgtgtt cttgtaaata cgtgtcgctc 4980
agatgtcctt tgaagtggga gggaatcaat ccggggataa tttcaaatgg aatagagtat 5040
tttgatattg ttcattcaga gggtgatgtg tacatatcta tattgtatat atgtgatgaa 5100
aatgcattgg ctttttgtgc agatacaacc tgctctctgt actgctgttg gacagtcagt 5160
gttttaatgt ttctacagtt ttgctattgc acgatttcat attttgcctc tatgatgaac 5220
ggcaaccatt atttgtaact gtttagtgct gtaaagaaat attccaagtg tcattaggat 5280
tgttgctgcc agaactgata tgcatgaatg gcacttaaaa taaatatatt atgttaactc 5340
t 5341

<210> 57
<211> 1561
<212> DNA
<213> Homo sapiens
<400> 57
cgcacgccac ccgcccgccg cctgccagag ctgctcggcc cgcagccagg gggacagcgg 60
ctggtcggag gctcgcagtg ctgtcggcga gaagcagtcg ggtttggagc gcttgggtcg 120
cgttggtgcg cggtggacac gagggacccc agttcccgcg agcagctccg cgccggccct 180
gagagactaa gctgaaactg ctgctcagct cccaagatgg tgccacccaa attgcatgtg 240
cttttctgcc tctgcggctg cctggctgtg gtttatcctt ttgactggca atacataaat 300

cctgttgccc atatgaaatc atcagcatgg gtcaacaaaa tacaagtact gatggctgct 360
gcaagctttg gccaaactaa aatccccccgg ggaaatgggc cttattccgt tggttgtaca 420
gacttaatgt ttgatcacac taataagggc accttcttgc gtttatatta tccatcccaa 480
gataatgatc gccttgacac cctttggatc ccaaataaag aatatttttg gggtcttagc 540
aaatttcttg gaacacactg gcttatgggc aacatttga ggttactctt tggttcaatg 600
acaactcctg caaactggaa ttcccctctg aggcctggtg aaaaatatcc acttgttgtt 660
ttttctcatg gtcttggggc attcaggaca ctttattctg ctattggcat tgacctggca 720
tctcatgggt ttatagttgc tgctgtagaa cacagagata gatctgcatc tgcaacttac 780
tatttcaagg accaatctgc tgcagaaata ggggacaagt cttggctcta ccttagaacc 840
ctgaaacaag aggaggagac acatatacga aatgagcagg tacggcaaag agcaaaagaa 900
tgttcccaag ctctcagtct gattcttgac attgatcatg gaaagccagt gaagaatgca 960
ttagatttaa agtttgatat ggaacaactg aaggactcta ttgataggga aaaaatagca 1020
gtaattggac attcttttgg tggagcaacg gttattcaga ctcttagtga agatcagaga 1080
ttcagatgtg gtattgccct ggatgcatgg atgtttccac tgggtgatga agtatattcc 1140
agaattcctc agcccctctt ttttatcaac tctgaatatt tccaatatcc tgctaatatc 1200
ataaaaatga aaaaatgcta ctcacctgat aaagaaagaa agatgattac aatcaggggt 1260
tcagtccacc agaattttgc tgacttcact tttgcaactg gcaaaataat tggacacatg 1320
ctcaaattaa agggagacat agattcaaat gcagctattg atcttagcaa caaagcttca 1380
ttagcattct tacaaaagca tttaggactt cataaagatt ttgatcagtg ggactgcttg 1440
attgaaggag atgatgagaa tcttattcca gggaccaaca ttaacacaac caatcaacac 1500
atcatgttac agaactcttc aggaatagag aaatacaatt aggattaaaa taggtttttt 1560
a 1561

<210> 58
<211> 863
<212> DNA
<213> Homo sapiens
<400> 58
ccagcggctg cggcggcgga gctcctccga ggtccgggtc accagtctct gctcttccca 60
gcctctccgg cgcgctccaa gggcttcccg tcgggaccat gcgcggccgt gagctcccgc 120
tggtcctgct ggcgctggtc ctctgcctgg cgcccccgggg gcgagcggtc ccgctgcctg 180
cgggcggagg gaccgtgctg accaagatgt acccgcgcgg caaccactgg gcggtggggc 240
acttaatggg gaaaaagagc acaggggagt cttcttctgt ttctgagaga gggagcctga 300
agcagcagct gagagagtac atcaggtggg aagaagctgc aaggaatttg ctgggtctca 360
tagaagcaaa ggagaacaga aaccaccagc cacctcaacc caaggccctg ggcaatcagc 420
agccttcgtg ggattcagag gatagcagca acttcaaaga tgtaggttca aaaggcaaag 480
ttggtagact ctctgctcca ggttctcaac gtgaaggaag gaacccccag ctgaaccagc 540
aatgataatg atggcctctc tcaaaagaga aaaacaaaac ccctaagaga ctgcgttctg 600
caagcatcag ttctacggat catcaacaag atttccttgt gcaaaatatt tgactattct 660
gtatctttca tccttgacta aattcgtgat tttcaagcag catcttctgg tttaaacttg 720
tttgctgtga acaattgtcg aaaagagtct tccaattaat gctttttttat atctaggcta 780
cctgttggtt agattcaagg ccccgagctg ttaccattca caataaaagc ttaaacacat 840
tgtccaaaaa aaaaaaaaaa aaa 863

<210> 59
<211> 3052
<212> DNA
<213> Homo sapiens
<400> 59
caggaccatg gaactcagcg tcctcctctt ccttgcactc ctcacaggac tcttgctact 60
cctggttcag cgccacccta acacccatga ccgcctccca ccagggcccc gccctctgcc 120
ccttttggga aaccttctgc agatggatag aagaggccta ctcaaatcct ttctgaggtt 180
ccgagagaaa tatggggacg tcttcacggt acacctggga ccgaggcccg tggtcatgct 240
gtgtggagta gaggccatac gggaggccct tgtggacaag gctgaggcct tctctggccg 300
gggaaaaatc gccatggtcg acccattctt ccggggatat ggtgtgatct ttgccaatgg 360
aaaccgctgg aaggtgcttc ggcgattctc tgtgaccact atgagggact tcgggatggg 420
aaagcggagt gtggaggagc ggattcagga ggaggctcag tgtctgatag aggagcttcg 480
gaaatccaag ggggccctca tggaccccac cttcctcttc cagtccatta ccgccaacat 540
catctgctcc atcgtctttg gaaaacgatt ccactaccaa gatcaagagt tcctgaagat 600
gctgaacttg ttctaccaga ctttttcact catcagctct gtattcggcc agctgtttga 660
gctcttctct ggcttcttga aatactttcc tggggcacac aggcaagttt acaaaaacct 720
gcaggaaatc aatgcttaca ttggccacag tgtggagaag caccgtgaaa ccctggaccc 780
cagcgccccc aaggacctca tcgacaccta cctgctccac atggaaaaag agaaatccaa 840
cgcacacagt gaattcagcc accagaacct caacctcaac acgctctcgc tcttctttgc 900
tggcactgag accaccagca ccactctccg ctacggcttc ctgctcatgc tcaaatacco 960
tcatgttgca gagagagtct acagggagat tgaacaggtg attggcccac atcgccctcc 1020
agagcttcat gaccgagcca aaatgccata cacagaggca gtcatctatg agattcagag 1080
attttccgac cttctcccca tgggtgtgcc ccacattgtc acccaacaca ccagcttccg 1140
agggtacatc atccccaagg acacagaagt atttctcatc ctgagcactg ctctccatga 1200

```
cccacactac tttgaaaaac cagacgcctt caatcctgac cactttctgg atgccaatgg   1260
ggcactgaaa aagactgaag cttttatccc cttctcctta gggaagcgga tttgtcttgg   1320
tgaaggcatc gcccgtgcgg aattgttcct cttcttcacc accatcctcc agaacttctc   1380
catggccagc cccgtggccc cagaagacat cgatctgaca ccccaggagt gtggtgtggg   1440
caaaataccc ccaacatacc agatccgctt cctgccccgc tgaaggggct gagggaaggg   1500
ggtcaaagga ttccagggtc attcagtgtc cccgcctctg tagacaatgg ctctgactcc   1560
ccgcaacttc ctgcctctga gagacctgct acaagccagc ttccttcccc tccatggcac   1620
cagttgtctg aggtcacatt gcaagtgagt gcaggagtga gattatcgaa aattataata   1680
tacaaaatca tatatatata tatgttcttg ttttttgaga cagagtctca cactgttgcc   1740
caggctggag tgcagtggcg tgatctcggc tcactgcaac ctccaccccc ggggatcaag   1800
caactctcct gcctcagcct ccctagtagc tgggattaca ggcatgcact accacgcttg   1860
gctaattttt gtatttttag tagagatggg gtttcactgt gtaggccagg ctggtctcga   1920
actcctgaac tcaagtgatt cacccacctt agcctcccaa agtgctggga ttacaggcgt   1980
gagtcaccgt gcccagccat gtatatatat aattttaaaa attaagctga aattcacata   2040
acataaaatt agctgtttta aagtgtaaaa tttagtggcg tgtggttcat tcacaaagct   2100
gtacaaccac caccatctag ttccaaacat tttctttttt tctgagatgg agtctcactc   2160
tgtcacccag gttcgagttc agtggtgcca tctctgtcca ctgcaacctc cacatcctgg   2220
gttcaagtga ttctcctgcc tcagcctctg gaggagctgg tatcacaggc gtcccccacc   2280
acgcctggct aaattttgta tttttaggtg gtcttgaact cctgatgtca ggtgattctc   2340
ctagctccaa atgttttcat tatctctccc ccaacaaaac ccatacctat caagctgtca   2400
ctccccatac cccattctct ttttcatctc ggcccctgtc aatctggttt ttgtcactat   2460
ggacttacca attctgaata tttcccataa acagaatcat acaatatttg attttttttt   2520
tttttttgaa actaagcctt gctctgtctc ccaggctgga gtgctatggt gcaatttttg   2580
ttcactgcaa cctctgcctt ccaagatcaa gagattctcc agtctcagct cccaagtagc   2640
tgggattaca ggcatgtact accatgcctg gctaattttc ttgtagtttt agtagggaca   2700
tgttggccag gctggtggtg agctcctggc ctcaggtgat ccacccacct cagtgttcca   2760
aagtgctgat attacaggca taatatgtga tcttttgtgt ctggttgctt tcatgttgaa   2820
tgctattttt gaggttcatg cctgttgtag accacagtca cacactgctg tagtcttccc   2880
cagtcctcat tcccagctgc ctcttcctac tgcttccgtc tatcaaaaag ccccccttggc  2940
ccaggttccc tgagctgtgg gattctgcac tggtgctttg gattccctga tatgttcctt   3000
caaatctgct gagaattaaa taaacatctc taaagcctga cctccccacg tc           3052
```

<210> 60
<211> 1789
<212> DNA
<213> Homo sapiens
<400> 60

```
tcgatgctcc acgtaggagc tggctcggct gccggctgcg gtcagggcca ccgtataaag     60
agggcggcag acccgagcgc ctaggactcg ccgctgcccg cgccccgccg ccgctgctgc    120
ccccagcccc ggccccaggc gtcccagcca tggtccgccc aatgctcttg ctcagcctcg    180
gcctcctggc tggtctgctg ccggcgctgg ccgcctgccc ccagaactgc cactgccaca    240
gcgacctgca gcacgtcatc tgcgacaagg tggggctgca gaagatcccc aaggtgtcag    300
agaagaccaa gctgctcaac ctacagcgca acaacttccc ggtgctggct gccaattcgt    360
tccgggccat gccgaacctc gtgtcattgc acctgcagca ctgccagatc cgcgaggtgg    420
ccgccggtgc cttccgcggc ctcaagcaac ttatctactt gtacctgtcc cataacgaca    480
tccgcgtcgt gcgtgccggt gccttcgacg acctgaccga gctgacctac ctctacctgg    540
accacaacaa ggtcactgag ctgccccggg ggttgctctc cccgctggtc aacctcttca    600
tcttgcagct caacaacaac aagatccgtg agctgcgcgc agggcccttc cagggagcca    660
aggacctgcg ctggctctac ctgtcggaaa acgcgttgag ctccctgcag cccggggccc    720
tggacgacgt ggagaacctc gccaaattcc acgtggacag gaaccagctg tccagctacc    780
cctcagctgc cctgagcaag ctacgggtgg tggaggagct gaagctgtcc cacaaccccc    840
tgaaaagcat cccggacaat gccttccagt cctttggcag atacctggag accctctggc    900
tggacaacac caacctggag aagttctcag atggtgcctt cctgggtgta accacgctga    960
aacacgtcca tttggagaac aaccgcttga accagctacc ctcgaacttc cccttcgaca   1020
gcctggagac cctcgccctt accaataacc cctggaagtg tacctgccag ctccggggcc   1080
ttcggcggtg gctggaagcc aaggcctccc gcccagatgc cacctgtgcc tcacctgcca   1140
agttcaaggg ccagcacatc cgtgacacgg acgccttccg cagctgcaag ttccccacca   1200
agaggtccaa gaaagctggc cgccattaaa caggttctga cccagccagt cctggtgact   1260
ggcctctgcc ttccaccgga gagactactg accttctcac ctccgaccca taccttctcc   1320
ccacagcctc tgctgatgca cagagctgcc tacacctaga cacgtcctgg caggggggcct  1380
cgggcactcc actaccaacc cagctccacc cagcagtgtc ctggggagaa ggaaggctga   1440
gcctctcccc agccttcatg ccttccccac cctccagctc ctcttggaga agctgttgct   1500
gagacccccc cccccccccc aagtcaatca gaaccacaac aggttggtca tcaggatggc   1560
caccctccca ggatcatcct tcctctgttc tctttccctg ccacgtggaa acaatcatca   1620
gatccttgcc ccaccccttg cttccagaaa gggttttaaa gcccatgccc caactctgcc   1680
agccccccacc tgccaggacg ttctagcagg tcatcggtgc tttgctgtcc atcttcccat  1740
gctgcaattt cttcctgaga tttctataaa tataaatgta tgtatgtat              1789
```

<210> 61

<211> 3164
<212> DNA
<213> Homo sapiens
<400> 61

```
cacatttctt agcttctctg agcctccatt tcagcatctg taaaatgtta ctacctactt      60
tataggtttt ctgatgaggc ttaagtgtgg ttgtgcatgt gaccatctta gcatagtgtg     120
tggcaatggt aagcactcag tgctgtcagc tgtttttacc gctgttatta tattactgtg     180
ttttcccatg tcattaggtc tgtgatccag gcttcagcag acaatccagg ctgatgcttt     240
tgctgagtgc gctaagggat gctggcctgg cgggatggtg aactggaagc agaaacgtca     300
tcctctctct tccttcttgc catgcaggtg tggatgtgtg ggggccgcat ggaggacatc     360
ccctgctcca gggtgggcca tatctacagg aagtatgtgc cctacaaggt cccggccgga     420
gtcagcctgg cccgggtaag aaccttaagc gggtggccga agtgtggatg gatgagtacg     480
cagagtacat ttaccagcgc cggcctgaat accgccacct ctccgctggg gatgtcgcag     540
tccagaaaaa gctccgcagc tcccttaact gcaagagttt caagtggttt atgacgaaga     600
tagcctggga cctgcccaaa ttctacccac ccgtggagcc cccggctgca gcttgggggg     660
agatccgaaa tgtgggcaca gggctgtgtg cagacacaaa gcacggggcc ttgggctccc     720
cactaaggct agagggctgc gtccgaggcc gtggggaggc tgcctggaac aacatgcagg     780
tattcacctt cacctggaga gaggacatcc ggcctggaga cccccagcac accaagaagt     840
tctgctttga tgccatttcc cacaccagcc ctgtcacgct gtacgactgc cacagcatga     900
agggcaacca gctgtggaaa taccgcaaag acaagaccct gtaccaccct gtcagtggca     960
gctgcatgga ctgcagtgaa agtgaccata ggatcttcat gaacacctgc aacccatcct    1020
ctctcaccca gcagtggctg tttgaacaca ccaactcaac agtcttggaa aaattcaata    1080
ggaactgagc cctcatgtcc ccttggcagg ccccccaggg tctggcactc actgcagact    1140
tcctctttca agggaggcag ggcccctgtg ggcactaggt gtaaaaggtg ctggccaaat    1200
ggttcagggt gaagagggct cttgattcag gggctggggt ctgcctggtc cttgagcccc    1260
tgagttgtgg gggtagggtg aagagcatat cccacaagag gccccacagg gagcagagac    1320
tgctttaatc cctgctgaca tcacggaaaa gcaacagagc cttttcaact ttgtcactat    1380
gtccccttga acattatgtg ggagaacacc aaggtagcct aggccaccca aaagtgagtc    1440
ctgcgaggtt gcccagccct cagatggctc tcctacatga tggtgcttta gaaacaaagg    1500
taaaatttgc ctgtttgggg cagcttttag tatcgatgcc actcatctgc agcagaagag    1560
aaagaagtcc tcttggggct ttttagtttc tgccgtcctg gggggaacat tgcagttact    1620
gcacagcttc tgttctctgt cacaacccca ggtgatttgg tccggtcaaa ggccatactt    1680
ggggccctaa gagtgttcag tattgaatgc tgatcagctg ccaggtgagg agtcagaaga    1740
gggagccccc ctagacattt ctttgcagct atggacatgc gggatatctc cccctgctct    1800
ctgggtattt gaaatgtcaa ttttagcact ctccaggcac aaggacagcc cagcaccagc    1860
tttacagggc agtgtttcag atggccctga gcccacggaa aaggccaggt agacctccaa    1920
actagaaatg ctggctgatt tgccctgatc catgcttcca tttccctgtc tctcttcccc    1980
aggcaattac tggcctcaaa agaggaacag aggtgctgcg aggtgctcac ctcacagagt    2040
ctggaggcct ccaggatcaa ctgtgggcaa agtgcctgcc tctgacctca tcatggttct    2100
agttctcata cagaactcca gaattttaa agaactctat aattggattg caaactagga     2160
tgctacatag gattctggta ttccacatcc aatatggatt tctagaatgc tgtgattaaa    2220
ggagccagcc aggtgtaata cagtcaaggc agccccccagc ctagagacaa tctgtgaaat   2280
ccaaagttgg tggtgttggg aaagcagggg gacatgtgtc cctcagctca gcagaggctg    2340
tggtacaaca tggtccttgg tgaagacctg caccccctgga acctcccacc atcatcacaa   2400
ctgtagtctc atttgcagtg gagaaaagaa cccgacgtcc cacagccaga tatacaccca    2460
gctccatgcc agcccttcat gtttaccttt tgctttgtta attacatgtc agactcctag    2520
agggcctcca gactaatagg aagcatttct gtaaccaacc tgccacccac tgattcagaa    2580
atggaaatca cattccacaa tctatggctt ccaccagcta gcccaggaaa tacttgaaat    2640
cagcattcca attagtgttg agtctcttga ttgtgtcatt taccaattaa ataactgaga    2700
cctaagtctg ggaacagagc cacgaatctg cctttgagat gctggcagat ctcaaggcca    2760
tcaattattg ggggagggag ggacaaacac tcccaatcat ccaccagtca gactgaatgt    2820
gtagctggcg aggaattact tccacttctg gcccagcaca agccctgctt tggccacctg    2880
tctgcaagag aggcggcccc tgtgcttgca acgcttacgt gttgatccca gtgtcctttt    2940
ccaaatgagt gctgtagctt tagaagtggc cctctataga aagaagtcaa aagatgaggc    3000
cccttctaga atctaggata acaagagtgt tgacagtttg aggagtcgaa ttgagattca    3060
tcatcaaaga gcaatgcagc gtcgttaaaa taaaaactgt gccttttaaa aagaaaaatg    3120
caaatataga gcaaatccct aaacttgaaa aaaaaaaaaa aaaa                      3164
```

<210> 62
<211> 1121
<212> DNA
<213> Homo sapiens
<400> 62

```
ctagctctgt gggaaggttt tgggctctct ggctcggatt ttgcaatttc tccctgggga      60
ctgccgtgga gccgcatcca ctgtggatta taattgcaac atgacgctgg aagagctcgt     120
ggcgtgcgac aacgcggcgc agaagatgca gacggtgacc gccgcggtgg aggagctttt     180
ggtggccgct cagcgccagg atcgcctcac agtgggggtg tacgagtcgg ccaagttgat     240
gaatgtggac ccagacagcg tggtcctctg cctcttggcc attgacgagg aggaggagga     300
tgacatcgcc ctgcaaatcc acttcacgct catccagtcc ttctgctgtg acaacgacat     360
```

caacatcgtg cgggtgtcgg gcaatgcgcg cctggcgcag ctcctgggag agccggccga 420
gacccagggc accaccgagg cccgagacct ccactgtctt cccttcctac agaaccctca 480
cacggacgcc tggaagagcc acggcttggt ggaggtggcc agctactgcg aagaaagccg 540
gggcaacaac cagtgggtcc cctacatctc tcttcaggaa cgctgaggcc cttcccagca 600
gcagaatctg ttgagttgct gccaacaaac aaaaaataca ataaatattt gaaccccctc 660
ccccccagca caaccccccc aaaacaaccc aacccacgag gaccatcggg ggcaggtcgt 720
tggagactga agagaaagag agagaggaga agggagtgag gggccgctgc cgccttcccc 780
atcacggagg gtccagactg tccactcggg ggtggagtga gactgactgc aagccccacc 840
ctccttgaga ctggagctga gcgtctgcat acgagagact tggttgaaac ttggttggtc 900
cttgtctgca ccctcgacaa gaccacactt tgggacttgg gagctggggc tgaagttgct 960
ctgtacccat gaactcccag tttgcgaatt aataagagac aatctatttt gttacttgca 1020
cttgttattc gaaccactga gagcgagatg ggaagcatag atatctatat ttttatttct 1080
actatgaggg ccttgtaata aatttctaaa gcctcaaaaa a 1121

<210> 63
<211> 1258
<212> DNA
<213> Homo sapiens
<400> 63
gacataaaaa ccgggtgccg gcaggcgcca gtcgcaggtg tgctgctgag gcgtgagaat 60
ggcgtcccgc ggccggcgtc cggagcatgg cggaccccca gagctgtttt atgacgagac 120
agaagcccgg aaatacgttc gcaactcacg gatgattgat atccagacca ggatggctgg 180
gcgagcattg gagcttcttt atctgccaga gaataagccc tgttacctgc tggatattgg 240
ctgtggcact gggctgagtg gaagttatct gtcagatgaa gggcactatt gggtgggcct 300
ggatatcagc cctgccatgc tggatgaggc tgtggaccga gagatagagg gagacctgct 360
gctgggggat atgggccagg gcatccccatt caagccaggc acatttgatg gttgcatcag 420
catttctgct gtgcagtggc tctgtaatgc taacaagaag tctgaaaacc ctgccaagcg 480
cctgtactgc tttttttgctt ctcttttttc tgttctcgtc cggggatccc gagctgtcct 540
gcagctgtac cctgagaact cagagcagtt ggagctgatc acaacccagg ccacaaaggc 600
aggcttctcc ggtggcatgg tggtagacta ccctaacagt gccaaagcaa agaaattcta 660
cctctgcttg ttttctgggc cttcgacctt tataccagag ggctgagtg aaaatcagga 720
tgaagttgaa cccagggagt ctgtgttcac caatgagagg ttcccattaa ggatgtcgag 780
gcggggaatg gtgaggaaga gtcgggcatg ggtgctggag aagaaggagc ggcacaggcg 840
ccagggcagg gaagtcagac ctgacaccca gtacaccggc cgcaagcgca agccccgctt 900
ctaagtcacc acgcggttct ggaaaggcac ttgcctctgc acttttctat attgttcagc 960
tgacaaagta gtattttaga aaagttctaa agttataaaa atgttttctg cagtaaaaaa 1020
aaagttctct gggccgggcg tggtggctca cacctgtaat cccagcacct tgggaggctg 1080
aggtgggagg atcatttgag gccaggagtt tgagacctgc ctgggcaaca taatgaaact 1140
tcctttccag ggaggaaaaa aaaaaaaaaa aaaagctctg agagcatctt attttgttta 1200
aaggcaagaa ataaaatttc cttttgtgga aaaaaaaaaa aaaaaaaaaa aaaaaaa 1258

<210> 64
<211> 2668
<212> DNA
<213> Homo sapiens
<400> 64
atggcggcgg gtgggagcgg cgggcgtgcg tcgtgcccgc cgggggtcgg ggtcggcccg 60
ggcacggggg gcagtcccgg gcccagcgcc aacgccgccg ccaccccggc ccccggcaac 120
gcggccgccg ccgccgccgc cgccgccgcc gccgccgccg cccctgggcc gacgccgccc 180
gccccgccgg gccccgggac agacgcgcag gccgcgggcg cggagcgggc ggaggaggcg 240
gcgggcccgg gggcggcggc gctgcagcgc gaggccgcgt acaactggca ggccagcaag 300
cccaccgtgc aggagcgctt cgccttcctc ttcaacaacg aggtgctgtg cgacgtgcac 360
ttcctggtgg gcaaggggct cagctcgcag cgcatccccg cgcacaggtt cgtgctggcc 420
gtgggcagcg ccgtctttga tgccatgttc aacgggggaa tggccacaac atccacggag 480
attgagctgc ccgacgtgga acccgccgcc ttcctcgcac tgctcaagtt tctctactcg 540
gacgaggtgc agattggccc ggagacggtg atgaccacgc tatacaccgc caagaagtac 600
gcggtgccag cgctcgaggc ccattgcgtg gagttcctga agaagaacct gcgagccgac 660
aacgccttca tgctgctcac gcaggcgcga ctcttcgatg aaccgcagct ggccagcctg 720
tgcctggaga acatcgacaa aaacactgca gacgccatca ccgcggaggg cttcaccgac 780
attgacctgg acacgctggt ggctgtcctg gagcgcgaca cactgggcat ccgtgaggtg 840
cggctgttca atgccgttgt ccgctggtcc gaggccgagt gtcagcggca gcagctgcag 900
gtgacgccag agaacaggcg gaaggttctg ggcaaggccc tgggcctcat tcgcttcccg 960
ctcatgacca tcgaggagtt cgctgcaggt cccgcacagt cgggcatcct ggtggaccgc 1020
gaggtggtca gcctcttcct gcacttcacc gtcaacccca agccacgagt ggagttcatt 1080
gaccggcccc gctgctgcct gcgtgggaag gagtgcagca tcaaccgctt ccagcaggtg 1140
gagagtcgct ggggctacag cgggaccagt gaccgcatca ggttctcagt caacaagcgc 1200
atcttcgtgg tgggatttgg gctgtatgga tccatccacg ggcccaccga ctaccaagtg 1260
aacatccaga ttattcacac cgatagcaac accgtcttgg gccagaacga cacgggcttc 1320
agctgcgacg gctcagccag caccttccgc gtcatgttca aggagccggt ggaggtgctg 1380

cccaacgtca actacacggc ctgtgccacg ctcaagggcc cagactccca ctacggcacc 1440
aaaggcctgc gcaaggtgac acacgagtcg cccaccacgg gcgccaagac ctgcttcacc 1500
ttttgctacg cggccgggaa caacaatggc acatccgtgg aggacggcca gatccccgag 1560
gtcatcttct acacctaggc tgcccgacac cgacaccgcc ctccctccgc ggggatagcc 1620
gcagccccag gccatcatct gctgctgggg tccccccacc acgcggtgcc aggcccagtg 1680
tcccccaggc cgtctgtcca ctccatgcca cctttctcag catcaggacg ggttgccct 1740
gtgttcacca cgagtgtggc tgctggatca gggcagccgg ggaggtggcc aggccagtgg 1800
ccaggccctg tggagacaat ccctcaggac tagggacagg gctgtgccgg cctgggccag 1860
ggcccacgga cccgcagctc agggcgcctg cccacgtcgt ctgccggcgg tgcgccgcgg 1920
gcgtccctcg cgtctcttca ctgcacattg caatgcattt gcgattccca tttctctgct 1980
aggagccagc ctgggtggcg ctgctcccag agccgtgggt cccagacctt gcgttccttt 2040
tgttcctgtc cgtttatcag gacacgggcc ccacctgtca cgtgcccgag gccacccaag 2100
cccagcctgc ggggcgttcc cactgcctgg atgccggctt gagttctgcg cacgcaggat 2160
tcagtgtggg gacggcccct gccggatagg cctagccctg gcccaggtgg tgagcggttt 2220
gcagtgtccg ttctcatcca cctgatgggc ccagataaag gcccccgctg tccagcctcc 2280
ctggacggcc ctcgcggtcc ctgcagccca agatgggact cagaccctgt gccccagagc 2340
tcccctgccg cagaatgggg ccccagccgg ccccgaccgg gtccaggagc actgctcgcc 2400
tgtacatact gttgccctag cccacctggt gccgtgggag ccaccccag gtgctggggg 2460
cacagcccct ccccactccg gccacgcccc cacccacccc gcgtgtttct gccctgtgac 2520
tcctggaacc tgcgtcctcc ccaaagccat gggaggggtg tcctcctcag accatgcccc 2580
cagatgattt ttttaaataa agaaacaaat gcacctgcaa aaaaaaaaaa tagaaaataa 2640
aaaaaaaaaa aaaaaaaaaa aaaaaaa 2668

<210> 65
<211> 5003
<212> DNA
<213> Homo sapiens
<400> 65
ggatccattt tataagctca aagataatta cttttcagac taagaatatt tagggtaaaa 60
agtactgttc aacatctcta ctgaggatgt tatgatgtag cacactctat aagctggagc 120
taaaggaaac tttccttaaa gtgctattta ctaaaaattg gaacacattc cttaagacaa 180
atcgaagtgt ggcacacaac atccaaactt ccatcataga tacagaggtg ttaccatctc 240
ccactcccaa atttctttgt cacgctgagg atactcaaga ggagcaggac atgttggtcg 300
cagcaggaga aacttgaaag cattcacttt tatggaactc ataagggaga gaatctctta 360
tttagtatcg tccttgatac atttattatt ttaaaagata atgtagccaa atgtcttcct 420
ctgtgttaaa tctttacaaa actgaaatct taaaatggtg acaaaaattc tacttctgat 480
agaatctatt catttttcca attagatagg gcataattct taatttgcaa aacaaaacgt 540
aatatgctta tgaggttcca tcccaaagaa cctgctattg agagtagcat tcagaataac 600
gggtggaaat gccaactcca gagtttcaga tcctaccggt aattggggta gggaggggct 660
ttgggcgggg cctccctaga ggaggaggcg ttgttagaaa gctgtctggc cagtccacag 720
ctgtcactaa tcggggtaag ccttgttgta tttgcgcgtg tgggtggcat tctcaatgag 780
aactagcttc acttgtcatt tgagtgaaat ctacaacccg aggcggctag tgctcccgca 840
ctactgggat ctgagatctt cggagatgac tgtcgcccgc agtacggagc cagcagaagt 900
ccgacccttc ctgggaatgg gctgtaccga gaggtccgac tagccccagg gttttagtga 960
gggggcagtg gaactcagcg agggactgag agcttcacag catgcacgag tttgatgcca 1020
gagaaaaagt cgggagataa aggagccgcg tgtcactaaa ttgccgtcgc agccgcagcc 1080
actcaagtgc cggacttgtg agtactctgc gtctccagtc ctcggacaga agttggagaa 1140
ctctcttgga gaactccccg agttaggaga cgagatctcc taacaattac tactttttct 1200
tgcgctcccc acttgccgct cgctgggaca aacgacagcc acagttcccc tgacgacagg 1260
atggaggcca agggcaggag ctgaccagcg ccgccctccc ccgcccccga cccaggaggt 1320
ggagatcctc cggtccagcc acattcaaca cccactttct cctccctctg cccctatatt 1380
cccgaaaccc cctcctcctt ccctttttccc tcctccctgg agacggggga ggagaaaagg 1440
ggagtccagt cgtcatgact gagctgaagg caaagggtcc ccgggctccc cacgtggcgg 1500
gcggcccgcc ctccccccgag gtcggatccc cactgctgtg tcgcccagcc gcaggtccgt 1560
tcccggggag ccagacctcg gacaccttgc ctgaagtttc ggccatacct atctccctgg 1620
acgggctact cttccctcgg ccctgccagg gacaggaccc ctccgacgaa aagacgcagg 1680
accagcagtc gctgtcggac gtggagggcg catattccag agctgaagct acaaggggtg 1740
ctggaggcag cagttctagt cccccagaaa aggacagcgg actgctggac agtgtcttgg 1800
acactctgtt ggcgccctca ggtcccgggc agagccaacc cagccctccc gcctgcgagg 1860
tcaccagctc ttggtgcctg tttggccccg aacttcccga agatccaccg gctgccccccg 1920
ccacccagcg ggtgttgtcc ccgctcatga gccggtccgg gtgcaaggtt ggagacagct 1980
ccgggacggc agctgcccat aaagtgctgc cccggggcct gtcaccagcc cggcagctgc 2040
tgctcccggc ctctgagagc cctcactggt ccggggcccc agtgaagccg tctccgcagg 2100
ccgctgcggt ggaggttgag gaggaggata gctctgagtc cgaggagtct gcgggtccgc 2160
ttctgaaggg caaacctcgg gctctgggtg gcgcggcggc tggaggagga gccgcggctt 2220
gtccgccggg ggcggcagca ggaggcgtcg ccctggtccc caaggaagat tcccgcttct 2280
cagcgcccag ggtcgccctg gtggagcagg acgcgccgat ggcgcccggg cgctccccgc 2340
tggccaccac ggtgatggat ttcatccacg tgcctatcct gcctctcaat cacgccttat 2400
tggcagcccg cactcggcag ctgctggaag acgaaagtta cgacggcggg gccggggctg 2460

```
ccagcgcctt tgccccgccg cggacttcac cctgtgcctc gtccaccccg gtcgctgtag   2520
gcgacttccc cgactgcgcg tacccgcccg acgccgagcc caaggacgac gcgtaccctc   2580
tctatagcga cttccagccg cccgctctaa agataaagga ggaggaggaa ggcgcggagg   2640
cctccgcgcg ctccccgcgt tcctaccttg tggccggtgc caaccccgca gccttcccgg   2700
atttcccgtt ggggccaccg cccccgctgc cgccgcgagc gaccccatcc agacccgggg   2760
aagcggcggt gacggccgca cccgccagtg cctcagtctc gtctgcgtcc tcctcggggt   2820
cgaccctgga gtgcatcctg tacaaagcgg agggcgcgcc gccccagcag ggcccgttcg   2880
cgccgccgcc ctgcaaggcg ccgggcgcga gcggctgcct gctcccgcgg gacggcctgc   2940
cctccacctc cgcctctgcc gccgccgccg ggcggccccc cgcgctctac cctgcactcg   3000
gcctcaacgg gctcccgcag ctcggctacc aggccgccgt gctcaaggag ggcctgccgc   3060
aggtctaccc gccctatctc aactacctga ggccggattc agaagccagc cagagcccac   3120
aatacagctt cgagtcatta cctcagaaga tttgtttaat ctgtggggat gaagcatcag   3180
gctgtcatta tggtgtcctt acctgtggga gctgtaaggt cttctttaag agggcaatgg   3240
aagggcagca caactactta tgtgctggaa gaaatgactg catcgttgat aaaatccgca   3300
gaaaaaactg cccagcatgt cgccttagaa agtgctgtca ggctggcatg gtccttggag   3360
gtcgaaaatt taaaaagttc aataaagtca gagttgtgag agcactggat gctgttgctc   3420
tcccacagcc attgggcgtt ccaaatgaaa gccaagccct aagccagaga ttcacttttt   3480
caccaggtca agacatacag ttgattccac cactgatcaa cctgttaatg agcattgaac   3540
cagatgtgat ctatgcagga catgacaaca caaaacctga cacctccagt tctttgctga   3600
caagtcttaa tcaactaggc gagaggcaac ttctttcagt agtcaagtgg tctaaatcat   3660
tgccaggttt tcgaaactta catattgatg accagataac tctcattcag tattcttgga   3720
tgagcttaat ggtgtttggt ctaggatgga gatcctacaa acatgtcagt gggcagatgc   3780
tgtattttgc acctgatcta atactaaatg aacagcggat gaaagaatca tcattctatt   3840
cattatgcct taccatgtgg cagatcccac aggagtttgt caagcttcaa gttagccaag   3900
aagagttcct ctgtatgaaa gtattgttac ttcttaatac aattcctttg gaagggctac   3960
gaagtcaaac ccagtttgag gagatgaggt caagctacat tagagagctc atcaaggcaa   4020
ttggtttgag gcaaaaagga gttgtgtcga gctcacagcg tttctatcaa cttacaaaac   4080
ttcttgataa cttgcatgat cttgtcaaac agcttcatct gtactgcttg aatacattta   4140
tccagtcccg ggcactgagt gttgaatttc cagaaatgat gtctgaagtt attgctgcac   4200
aattacccaa gatattggca gggatggtga aacccccttct ctttcataaa aagtgaatgt   4260
catctttttc ttttaaagaa ttaaattttg tggcatgtct ttttgttttg gtcaggatta   4320
tgaggtcttg agtttttata atgttcttct gaaagcctta catttataac atcatagtgt   4380
gtaaatttaa aagaaaaatt gtgaggttct aattattttc ttttataaag tataattaga   4440
atgtttaact gttttgttta cccatatttt cttgaagaat ttacaagatt gaaaaagtac   4500
taaaattgtt aaagtaaact atatcttatc catattattt cataccatgt aggtgaggat   4560
ttttaacttt tgcatctaac aaatcatcga cttaagagaa aaaatcttac atgtaataac   4620
acaaagctat tatatgttat ttctaggtaa ctccctttgt gtcaattata tttccaaaaa   4680
tgaaccttta aaatggtatg caaaattttg tctatatata tttgtgtgag gaggaaattc   4740
ataactttcc tcagattttc aaaagtattt ttaatgcaaa aaatgtagaa agagtttaaa   4800
accactaaaa tagattgatg ttcttcaaac taggcaaaac aactcatatg ttaagaccat   4860
tttccagatt ggaaacacaa atctcttagg aagttaataa gtagattcat atcattatac   4920
aaatagtatt gtgggttttg taggttttta aaataacctt ttttggggag agaattgtcc   4980
tctaatgagg tattgcgagt ggc                                           5003
```

<210> 66
<211> 1340
<212> DNA
<213> Homo sapiens
<400> 66

```
gcactgtctc tatgctcccc agcgtctagt ctatttattg tcgcggggaa gctgcggccg     60
cctcgcaccc ggaacaacaa agcaaggaag acggagtccg agcctcgggg gctcctagca    120
acgggccggg gcgggagttc catggagact ggggagcgcg cccgtctcat cctcatcctt    180
gtcctccagc ttctccttcg catccgacgc aaccggcagc agcgctgccg ccgcgtcctc    240
agccaccgct ccctcttccc acggatgtga tcttcgtggt ggaaagctaa attttaaaac    300
caccccaatg gatgcagaca gtgatgttgc attggacatt ctaattacaa atgtagtctg    360
tgtttttaga acaagatgtc atttaaactt aaggaagatt gctttggaag gagcaaatgt    420
aatttataaa cgtgatgttg gaaaagtatt aatgaagctt agaaaaccta gaattacagc    480
tacaatttgg tcctcaggaa aaattatttg cactggagca acaagtgaag aagaagctaa    540
atttggtgcc agacgcttag cccgtagtct gcagaaacta ggttttcagg taatatttac    600
agattttaag gttgtaaacg ttctggcagt gtgtaacatg ccatttgaaa tccgtttgcc    660
agaattcaca aagaacaata gacctcatgc cagttacgaa cctgaacttc atcctgctgt    720
gtgctatcgg ataaaatctc taagagctac attacagatt ttttcaacag gaagtatcac    780
agtaacaggg cccaatgtaa aggctgttgc tactgctgtg gaacagattt acccatttgt    840
gtttgaaagc aggaaagaaa ttttataatt caccacttaa ttggttagaa tctctaactg    900
agcacctttt aaacctgctg cacattggac tcaaaaggaa aactggacca acaataattg    960
aggaaataga ctcttttatt cattcacggc tacagtgtaa gctccagtcc ctttggattt   1020
tattccaaac cttgctgtaa tataaaagga agtttacaag acatgatatt gctgctttta   1080
caaaaggaca ttctatttat tttcgcagta attctcatgt ccccataagc agagctgtca   1140
cagtgtgcac taccttagat tgttttattg tcgtcattgt tattttttttc catttgagc    1200
```

taatgtgttt tatttgtgaa tagtctttta catttttgta tgctgaatat gggcaccaaa 1260
gaacctgtaa aagttatctt tttcaattga atgtgcacaa ataaaagttt ggaaaagata 1320
aaaaaaaaaa aaaaaaaaaa 1340

<210> 67
<211> 5426
<212> DNA
<213> Homo sapiens
<400> 67
agaaggtagc agacagacag acggatctaa cctctcttgg atcctccagc catgaggctg 60
ctctgggggc tgatctgggc atccagcttc ttcaccttat ctctgcagaa gcccaggttg 120
ctcttgttct ctccttctgt ggttcatctg gggtccccc tatcggtggg ggtgcagctc 180
caggatgtgc cccgaggaca ggtagtgaaa ggatcagtgt tcctgagaaa cccatctcgt 240
aataatgtcc cctgctcccc aaaggtggac ttcaccctta gctcagaaag agacttcgca 300
ctcctcagtc tccaggtgcc cttgaaagat gcgaagagct gtggcctcca tcaactcctc 360
agaggccctg aggtccagct ggtggcccat tcgccatggc taaaggactc tctgtccaga 420
acgacaaaca tccagggtat caacctgctc ttctcctctc gccgggggca cctctttttg 480
cagacggacc agcccattta caaccctggc cagcgggttc ggtaccgggt ctttgctctg 540
gatcagaaga tgcgcccgag cactgacacc atcacagtca tggtggagaa ctctcacggc 600
ctccgcgtgc ggaagaagga ggtgtacatg ccctcgtcca tcttccagga tgactttgtg 660
atcccagaca tctcagagcc agggacctgg aagatctcag cccgattctc agatggcctg 720
gaatccaaca gcagcaccca gtttgaggtg aagaaatatg tccttcccaa ctttgaggtg 780
aagatcaccc ctggaaagcc ctacatcctg acggtgccag gccatcttga tgaaatgcag 840
ttagacatcc aggccaggta catctatggg aagccagtgc agggggtggc atatgtgcgc 900
tttgggctcc tagatgagga tggtaagaag actttctttc gggggctgga gagtcagacc 960
aagctggtga atggacagag ccacatttcc ctctcaaagg cagagttcca ggacgccctg 1020
gagaagctga atatgggcat tactgacctc caggggctgc gcctctacgt tgctgcagcc 1080
atcattgagt atccaggtgg ggagatggag gaggcagagc tcacatcctg gtattttgtg 1140
tcatctccct tctccttgga tcttagcaag accaagcgac accttgtgcc tggggccccc 1200
ttcctgctgc aggccttggt ccgtgagatg tcaggctccc cagcttctgg cattcctgtc 1260
aaagtttctg ccacggtgtc ttctcctggg tctgttcctg aagtccagga cattcagcaa 1320
aacacagacg ggagcggcca agtcagcatt ccaataatta tccctcagac catctcagag 1380
ctgcagctct cagtatctgc aggctcccca catccagcga tagccaggct cactgtggca 1440
gccccacctt caggaggccc cgggtttctg tctattgagc ggccggattc tcgacctcct 1500
cgtgttgggg acactctgaa cctgaacttg cgagccgtgg gcagtggggc caccttttct 1560
cattactact acatgatcct atcccgaggg cagatcgtgt tcatgaatcg agagcccaag 1620
aggaccctga cctcggtctc ggtgtttgtg gaccatcacc tggcaccctc cttctacttt 1680
gtggccttct actaccatgg agaccaccca gtggccaact ccctgcgagt ggatgtccag 1740
gctggggcct gcgagggcaa gctggagctc agcgtggacg gtgccaagca gtaccggaac 1800
ggggagtccg tgaagctcca cttagaaacc gactccctag ccctggtggc gctgggagcc 1860
ttggacacag ctctgtatgc tgcaggcagc aagtcccaca agcccctcaa catgggcaag 1920
gtctttgaag ctatgaacag ctatgacctc ggctgtggtc ctgggggtgg ggacagtgcc 1980
cttcaggtgt tccaggcagc gggcctggcc ttttctgatg gagaccagtg gaccttatcc 2040
agaaagagac taagctgtcc caaggagaag acaacccgga aaaagagaaa cgtgaacttc 2100
caaaaggcga ttaatgagaa attgggtcag tatgcttccc cgacagccaa gcgctgctgc 2160
caggatgggg tgacacgtct gcccatgatg cgttcctgcg agcagcgggc agcccgcgtg 2220
cagcagccgg actgccggga gcccttcctg tcctgctgcc aatttgctga gagtctgcgc 2280
aagaagagca gggacaaggg ccaggcgggc ctccaacgag ccctggagat cctgcaggag 2340
gaggacctga ttgatgagga tgacattccc gtgcgcagct tcttcccaga gaactggctc 2400
tggagagtgg aaacagtgga ccgctttcaa atattgacac tgtggctccc cgactctctg 2460
accacgtggg agatccatgg cctgagcctg tccaaaacca aaggcctatg tgtggccacc 2520
ccagtccagc tccgggtgtt ccgcgagttc cacctgcacc tccgcctgcc catgtctgtc 2580
cgccgctttg agcagctgga gctgcggcct gtcctctata actacctgga taaaaacctg 2640
actgtgagcg tccacgtgtc ccccagtggag gggctgtgcc tggctggggg cggagggctg 2700
gcccagcagg tgctggtgcc tgcgggctct gcccggcctg ttgccttctc tgtggtgccc 2760
acggcagccg ccgctgtgtc tctgaaggtg gtggctcgag ggtccttcga attccctgtg 2820
ggagatgcgg tgtccaaggt tctgcagatt gagaaggaag ggccatcca tagagaggag 2880
ctggtctatg aactcaaccc cttggaccac cgaggccgga ccttggaaat acctggcaac 2940
tctgatccca atatgatccc tgatggggac tttaacagct acgtcagggt tacagcctca 3000
gatccattgg acactttagg ctctgagggg gccttgtcac caggaggcgt ggcctccctc 3060
ttgaggcttc ctcgaggctg tggggagcaa accatgatct acttggctcc gacactggct 3120
gcttcccgct acctggacaa gacagagcag tggagcacac tgcctcccga gaccaaggac 3180
cacgccgtgg atctgatcca gaaaggctac atgcggatcc agcagtttcg gaaggcggat 3240
ggttcctatg cggcttggtt gtcacggggc agcagcacct ggctcacagc ctttgtgttg 3300
aaggtcctga gtttggccca ggagcaggta ggaggatcgc ctgagaaact gcaggagaca 3360
tctaactggc ttctgtccca gcagcaggct gacggctcgt tccaggacct ctctccagtg 3420
atacatagga gcatgcaggg gggtttggtg ggcaatgatg agactgtggc actcacagcc 3480
tttgtgacca tcgcccttca tcatggcctg gccgtcttcc aggatgaggg tgcagagcca 3540
ttgaagcaga gagtggaagc ctccatctca aaggcaagct catttttggg ggagaaagca 3600

agtgctgggc tcctgggtgc ccacgcagct gccatcacgg cctatgccct gacactgacc 3660
aaggcccctg cggacctgcg gggtgttgcc cacaacaacc tcatggcaat ggcccaggag 3720
actggagata acctgtactg gggctcagtc actggttctc agagcaatgc cgtgtcgccc 3780
accccggctc ctcgcaaccc atccgacccc atgccccagg ccccagccct gtggattgaa 3840
accacagcct acgccctgct gcacctcctg cttcacgagg gcaaagcaga gatggcagac 3900
caggctgcgg cctggctcac ccgtcagggc agcttccaag gggggattcg cagtacccaa 3960
gacacggtga ttgccctgga tgccctgtct gcctactgga ttgcctccca caccactgag 4020
gagaggggtc tcaatgtgac tctcagctcc acaggccgga atgggttcaa gtcccacgcg 4080
ctgcagctga acaaccgcca gattcgcggc ctggaggagg agctgcagtt ttccttgggc 4140
agcaagatca atgtgaaggt gggaggaaac agcaaaggaa ccctgaaggt ccttcgtacc 4200
tacaatgtcc tggacatgaa gaacacgacc tgccaggacc tacagataga agtgacagtc 4260
aaaggccacg tcgagtacac gatggaagca aacgaggact atgaggacta tgagtacgat 4320
gagcttccag ccaaggatga cccagatgcc cctctgcagc ccgtgacacc cctgcagctg 4380
tttgagggtc ggaggaaccg ccgcaggagg gaggcgccca aggtggtgga ggagcaggag 4440
tccagggtgc actacaccgt gtgcatctgg cggaacggca aggtggggct gtctggcatg 4500
gccatcgcgg acgtcaccct cctgagtgga ttccacgccc tgcgtgctga cctggagaag 4560
ctgacctccc tctctgaccg ttacgtgagt cactttgaga ccgaggggcc ccacgtcctg 4620
ctgtattttg actcggtccc cacctcccgg gagtgcgtgg gctttgaggc tgtgcaggaa 4680
gtgccggtgg ggctggtgca gccggccagc gcaaccctgt acgactacta caaccccgag 4740
cgcagatgtt ctgtgtttta cggggcacca agtaagagca gactcttggc caccttgtgt 4800
tctgctgaag tctgccagtg tgctgagggg aagtgccctc gccagcgtcg cgccctggag 4860
cggggtctgc aggacgagga tggctacagg atgaagtttg cctgctacta cccccgtgtg 4920
gagtacggct tccaggttaa ggttctccga gaagacagca gagctgcttt ccgcctcttt 4980
gagaccaaga tcacccaagt cctgcacttc accaaggatg tcaaggccgc tgctaatcag 5040
atgcgcaact tcctggttcg agcctcctgc cgccttcgct tggaacctgg gaaagaatat 5100
ttgatcatgg gtctggatgg ggccacctat gacctcgagg gacaccccca gtacctgctg 5160
gactcgaata gctggatcga ggagatgccc tctgaacgcc tgtgccggag cacccgccag 5220
cgggcagcct gtgcccagct caacgacttc ctccaggagt atggcactca ggggtgccag 5280
gtgtgagggc tgccctccca cctccgctgg gaggaacctg aacctgggaa ccatgaagct 5340
ggaagcactg ctgtgtccgc tttcatgaac acagcctggg accagggcat attaaaggct 5400
tttggcagca aagtgtcagt gttggc 5426

<210> 68
<211> 1578
<212> DNA
<213> Homo sapiens
<400> 68
gatcagggcc gagttgtctc ggcggcgctg ccgaggcctc cacccaggac agtccccctc 60
cccgggcctc tctcctcttg cctacgagtc ccctctcctc gtaggcctct cggatctgat 120
atcgtggggt gaggtgagca ggcccgggga gggtggttac cgctgaggag ctgcagtctc 180
tgtcaagatg atagaggtac tgacaacaac tgactctcag aaactgctac accagctgaa 240
tgccctgttg gaacaggagt ctagatgtca gccaaaggtc tgtggtttga gactaattga 300
gtctgcacac gataatggcc tcagaatgac tgcaagacta agggactttg aagtaaaaga 360
tcttcttagt ctaactcagt tctttggctt tgacacagag acattttctc tagctgtgaa 420
tttactggac agattcctgt ctaaaatgaa ggtacagccc aagcaccttg ggtgtgttgg 480
actgagctgc ttttatttgg ctgtaaaatc aatagaagag gaaaggaatg tcccattggc 540
aactgacttg atccgaataa gtcaatatag gtttacggtt tcagacttga tgagaatgga 600
aaagattgta ttggagaagg tgtgttggaa agtcaaagct actactgcct ttcaatttct 660
gcaactgtat tattcactcc ttcaagagaa cttgccactt gaaaggagaa atagcattaa 720
ttttgaaaga ctagaagctc aactgaaggc atgtcattgc aggatcatat ttctaaagc 780
aaagccttct gtgttggcat tgtctatcat tgcattagag atccaagcac agaagtgtgt 840
agagttaaca gaaggaatag aatgtcttca gaaacattcc aagataaatg gcagagatct 900
gaccttctgg caagagcttg tatccaaatg tttaactgaa tattcatcaa ataagtgttc 960
caaaccaaat gttcagaagt tgaaatggat tgtttctggg cgtactgcac ggcaattgaa 1020
gcatagctac tacagaataa ctcaccttcc aacaattcct gaaatggtcc cttaactgga 1080
ttattacagc accaaaaaac ttctctgaag cctttctcca caaccttgtt ctatggattc 1140
cataatgtta caatggattt aagctatgaa gcctcaaaac atcacgagat aagcatgatg 1200
gtctcagact tgggaaaact gcctaatatt atgctgtagt ggaattatgt ttatgatttg 1260
aattcatctg tgaaggcatt caaatcaaag ctaaaagcct aaatgtgaaa tgctaatgac 1320
aagcctgaga aggtaaacta tgaatcttca tttctatcat tgatctaact ttagatattg 1380
gatcaatata tttaggtggt attgaaaatg ctattggagg agtcacacta atactatcaa 1440
ctatcagtct tcccacagct tcaatcactg tcattattct aatcctactc ctacttaaat 1500
tttaagttat gaggtttatg tcgaaagcaa catttcacaa atgtactttt aaggcataat 1560
aagggttaac attctagg 1578

<210> 69
<211> 1501
<212> DNA
<213> Homo sapiens

<400> 69
atagaggaca cgaccaagat ggcggcggtg tctggcttgg tgcggagacc ccttcgggag 60
gtctccgggc tgctgaagag gcgctttcac tggaccgcgc cggctgcgct gcaggtgaca 120
gttcgtgatg ctataaatca gggtatggat gaggagctgg aaagagatga gaaggtattt 180
ctgcttggag aagaagttgc ccagtatgat ggggcataca aggttagtcg agggctgtgg 240
aagaaatatg gagacaagag gattattgac actcccatat cagagatggg ctttgctgga 300
attgctgtag gtgcagctat ggctgggttg cggcccattt gtgaatttat gaccttcaat 360
ttctccatgc aagccattga ccaggttata aactcagctg ccaagaccta ctacatgtct 420
ggtggccttc agcctgtgcc tatagtcttc aggggaccca atggtgcctc agcaggtgta 480
gctgcccagc actcacagtg ctttgctgcc tggtatgggc actgcccagg cttaaaggtg 540
gtcagtccct ggaattcaga ggatgctaaa ggacttatta aatcagccat tcgggataac 600
aatccagtgg tggtgctaga gaatgaattg atgtatgggg ttccttttga atttctcccg 660
gaagctcagt caaaagattt tctgattcct attggaaaag ccaaaataga aaggcaagga 720
acacatataa ctgtggtttc ccattcaaga cctgtgggcc actgcttaga agctgcagca 780
gtgctatcta aagaaggagt tgaatgtgag gtgataaata tgcgtaccat tagaccaatg 840
gacatggaaa ccatagaagc cagtgtcatg aagacaaatc atcttgtaac tgtggaagga 900
ggctggccac agtttggagt aggagctgaa atctgtgcca ggatcatgga aggtcctgcg 960
ttcaatttcc tggatgctcc tgctgttcgt gtcactggtg ctgatgtccc tatgccttat 1020
gcaaagattc tagaggacaa ctctatacct caggtcaaag acatcatatt tgcaataaag 1080
aaaacattaa atatttagtt tggacttgaa tatcaagtcg ttgaaattta tttgaaatac 1140
ttgctggcac tgcacctgga tttgtactgc aagacctgac tattcataaa ggaaaacgat 1200
ttctaaagca acagcaggta tttttgtaca gggaagttta aatgtgtttg tgtatggaaa 1260
actctccact ctcctcccct agatgccatg cttccttttg tctgttacgg ttgccatgtt 1320
ctttgaataa caaattatat cacatttctat cctctctcac cacaaggaca aagtatggat 1380
gtggcagagt cctgatgaaa gatgtatcca aacaagataa cttatatgta taaaattaaa 1440
gcatataata cacatttact gttagtttgt tttgataagg aataaaggaa tttctaacat 1500
g 1501

<210> 70
<211> 3514
<212> DNA
<213> Homo sapiens
<400> 70
gtcatctggc cagcccccgc ccctcctccc ggcgtcagcc cgccagaggc cgcgcggggc 60
ccgggcttcg gccgatcagc ccgggaggcc ccgccgcgcc ccccttggccc gcgcgcccgt 120
ggtcacagtg gaagaggcgc ccgcgctgcg ctgcccggag gagccgtcgc gcgcccgctt 180
cctgttcggc tggttcctgc cagctcgagg acaaaacacg cgtgcgcgcg gcgggcgagc 240
gcgctcgccg cctcagtcgc cagcgccggg cgcagtccgc ctttttccgg agcagactgg 300
ccgcggtgct agtcggtagc agcggccgcc gcagcggctc cgcactggcg aaccgagggc 360
agaaaaaggc ggggttgacg gctttttggt aggagtgggc tggaccggac gccagagaca 420
aaggctccca aggcaagagg gactgtggcc ctgcgtcggc tctgctcgga actgctgacc 480
ccaggaattt acgcccctc gtttttctct tctgattctt ctcttctccc aagcccgcgt 540
cccctcacgc gtggcctctc tccttgccgg gagggccgcg atggaggtcc cgcccaggct 600
ttcccatgtg ccgccgccat tgttcccctc cgctcccgct actttagcct cccgcagcct 660
ctcccattgg cggccgcggc cgccgcggca gctagccccg ctcctccctt cgctcgctcc 720
cagctccgcc cggcaggggg cgcgccgggc ccagcgccac gtcaccgccc agcagccctc 780
ccgattggcg ggcggggcgg ctataaaggg agggcgcagg cggcgcccgg atctcttccg 840
ccgccatttt aaatccagct ccatacaacg ctccgccgcc gctgctgccg cgacccggac 900
tgcgcgccag caccccccctg ccgacagctc cgtcactatg gaggatatga acgagtacag 960
caatatagag gaattcgcag agggatccaa gatcaacgcg agcaagaatc agcaggatga 1020
cggtaaaatg tttattggag gcttgagctg ggatacaagc aaaaaagatc tgacagagta 1080
cttgtctcga tttggggaag ttgtagactg cacaattaaa acagatccag tcactgggag 1140
atcaagagga tttggatttg tgcttttcaa agatgctgct agtgttgata aggttttgga 1200
actgaaagaa cacaaactgg atggcaaatt gatagatccc aaaagggcca aagctttaaa 1260
agggaaagaa cctcccaaaa aggtttttgt gggtggattg agcccggata cttctgaaga 1320
acaaattaaa gaatattttg gagcctttgg agagattgaa aatattgaac ttcccatgga 1380
tacaaaaaca aatgaaagaa gaggattttg ttttatcaca tatactgatg aagagccagt 1440
aaaaaaattg ttagaaagca gataccatca aattggttct gggaagtgtg aaatcaaagt 1500
tgcacaaccc aaagaggtat ataggcagca acagcaacaa caaaaaggtg gaagaggtgc 1560
tgcagctggt ggacgaggtg gtacgagggg tcgtggccga ggtcagggcc aaaactggaa 1620
ccaaggattt aataactatt atgatcaagg atatggaaat tacaatagtg cctatggtgg 1680
tgatcaaaac tatagtggct atggcggata tgattatact gggtataact atgggaacta 1740
tggatatgga cagggatatg cagactacag tggccaacag agcacttatg gcaaggcatc 1800
tcgagggggt ggcaatcacc aaaacaatta ccagccatac taaaggagaa cattggagaa 1860
aacagcggga acttcattgc aggccgtgtg tcaccctgac cacgtctatc tctgggggtc 1920
gcacgttgcg ggcagagcgc aaggcataca ccagaaaacg ctgtcctgtg gaggagatgt 1980
taaagtaacc catcttgcag gacgacattg aagattggtc ttctgttgat ctaagatgat 2040
tattttgtaa aagactttct agtgtacaag acaccattgt gtccaactgt atatagctgc 2100
caattagttt tctttgtttt tactttgtcc tttgctatct gtgttatgac tcaatgtgga 2160

tttgtttata cacattttat ttgtatcatt tcatgttaaa cctcaaataa atgcttcctt 2220
atgtgattgc ttttctgcgt caggtactac atagctctgt aaaaaatgta atttaaaata 2280
agcaataatt aaggcacagt tgattttgta gagtattggt ccatacagag aaactgtggt 2340
cctttataaa tagccagcca gcgtcaccct cttctccaat ttgtaggtgt attttatgct 2400
cttaaggctt catcttctcc ctgtaactga gatttctacc acacctttga acaatgttct 2460
ttcccttctg gttatctgaa gactgtcctg aaaggaagac ataagtgttg tgattagtag 2520
aagctttgta atcataacac aatgagtaat tcttgtataa aagttcagat acaaaaggag 2580
cactgtaaaa ctggtaggag ctatggttta agagcattgg aagtagttac aactcaagga 2640
ttttggtaga aaggtatgag tttggtcgaa aaattaaaat agtggcaaaa taagatttag 2700
ttgtgttttc tcagagccgc cacaagattg aacaaaatgt tttctgtttg ggcatcctga 2760
ggaagttgta ttagctgtta atgctctgtg agtttagaga aaagtcttga tagtaaatct 2820
agtttttgac acagtgcatg aactaagtag ttaaatattt acatattcag aaaggaatag 2880
tggaaaaggt atcttggtta tgacaaagtc attacaaatg tgactaagtc attacaaatg 2940
tgactgagtc attacagtgg accctctggg tgcattgaaa agaatccgtt ttatatccag 3000
gtttcagagg acctggaata ataataagct ttggattttg cattcagtgt agttggattt 3060
tgggaccttg gcctcagtgt tatttactgg gattggcata cgtgttcaca ggcagagtag 3120
ttgatctcac acaacgggtg atctcacaaa actggtaagt ttcttatgct catgagccct 3180
cccttttttt ttttaatttg gtgcctgcaa ctttcttaac aatgattcta cttcctgggc 3240
tatcacatta taatgctctt ggcctctttt ttgctgctgt tttgctattc ttaaacttag 3300
gccaagtacc aatgttggct gttagaaggg attctgttca ttcaacatgc aactttaggg 3360
aatggaagta agttcatttt taagttgtgt ggtcagtagg tgcggtgtct agggtagtga 3420
atcctgtaag ttcaaattta tgattaggtg acgagttgac attgagattg tccttttccc 3480
ctgatcaaaa aaatgaataa agccttttta aacg 3514

<210> 71
<211> 1599
<212> DNA
<213> Homo sapiens
<400> 71
aagatcctgg cctgtgcagc tcgggtttcc gagcttctgc ctcaggcatc tccgcgatct 60
cctctcccct ccaatcctat ccgtgatgga cgatgcccac gagtcgccct ccgacaaagg 120
tggagagaca ggggagtcgg atgagacggc cgctgtgccc ggggacccgg gggctaccga 180
caccgatgga atcccagagg aaactgacgg agacgcagat gtggacttga aagaagctgc 240
agcggaggaa ggcgagctcg agagtcagga tgtctcagat ttaacaacag ttgaaaggga 300
agactcatca ttacttaatc ctgcagccaa aaaactgaaa atagatacca aagaaaagaa 360
agagaaaaag cagaaagtag atgaagatga gattcagaag atgcaaatcc tggtttcttc 420
tttttctgag gagcagctga accgttatga aatgtatcgc cgctcagctt tccctaaggc 480
agccatcaaa aggctgatcc agtccatcac tggcacctct gtgtctcaga atgttgttat 540
tgctatgtct ggtatttcca aggttttcgt cggggaggtg gtagaagaag cactggatgt 600
gtgtgagaag tggggagaaa tgccaccact acaacccaaa catatgaggg aagccgttag 660
aaggttaaag tcaaaaggac agatccctaa ctcgaagcac aaaaaaatca tcttcttcta 720
gaccaaagtc tagaaaggcc tatgttactg acggaagaag tattggttcc agacttccta 780
taagactgtc tgcattggtg ctttagtatc tcaggcctcc aaggattcca tgatgatttt 840
aatgtctttc tcaaaactct gatatttgtc acacctagaa agtatgtagc ctgattgata 900
cttgccttga ctaaattttg ggacctcttg gggcattttg aagtatttaa ctgtcttgac 960
cagttggaag aagatacgtg ggccataagc atcttctgga caggggaact gctttcagag 1020
agaaaacctt tccaagagag ttttgttttg ttttggtttc gttttgtttg agatagggtc 1080
ttgctctatc acctaggctg gagtgcagcg gcatgactgc agccttgaac tcctgggctt 1140
aagtgaccct cccacctcag tctcctgagt agctaggact acaggcacac actactgtgc 1200
ccagctaact tattttttatt ttttatggag atggggtctt gctttgttgc ccaggctggt 1260
cgtgaactcc tggcttcaag cagtcctcct gcctcagcct cctaaagtgc cgagggcttt 1320
aatggtttca cattgaagcc tgaagttgct aagacttagg ttgtttctta tatctggttt 1380
taagtagatg aaacaaccag aaacttttac ttgtgatact ctaccatgaa ggatgcggta 1440
atggcaggaa tagcagaata attggtgctt gtaaacattt aagattctcc tgtggatttt 1500
ggtgagtgat cattaaactg ttttccaact tgcaaaaaaa aaaaaaaaaa aaaaaaaaaa 1560
aaaaaaaaaa aaaaaaaaaa aaaaaaaaaa aaaaaaaaa 1599

<210> 72
<211> 2068
<212> DNA
<213> Homo sapiens
<220>
<221> misc_feature
<222> (143)..(143)
<223> n = a, t, g, or c
<400> 72
ggcgccggga ttgggagggc ttcttgcagg ctgctgggct ggggctaagg gctgctcagt 60
ttccttcagc ggggcactgg gaagcgccat ggcactgcag ggcatctcgg tcgtggagct 120
gtccggcctg gccccgggcc gtntctgtgc tatggtcctg gctgacttcg ggcgcgtgt 180

ggtacgcgtg gaccggcccg gctcccgcta cgacgtgagc cgcttgggcc ggggcaagcg 240
ctcgctagtg ctggacctga agcagccgcg ggagccgcgt gctgcggcgt ctgtgcaagc 300
ggtcggatgt gctgctggag cccttccgcc gcggtgtcat ggagaaactc cagctgggcc 360
cagagattct gcagcgggaa aatccaaggc ttatttatgc caggctgagt ggatttggcc 420
agttcaggaa agcttctgcc ggttagctgg ccacgatatc aactatttgg ctttgtcagg 480
tgttctctca aaaattggca gaagtggtga gaatccgtat gccccgctga atctcgtggc 540
tgactttgct ggtggtggcc ttatgtgtgc actgggcatt ataatggctc tttttgaccg 600
cacacgcact gacaagggtc aggtcattga tgcaaatatg gtggaaggaa cagcatattt 660
aagttctttt ctgtggaaaa ctcagaaatc gagtctgtgg gaagcacctc gaggacagaa 720
catgttggat ggtggagcac ctttctatac gacttacagg acagcagatg ggaattcat 780
ggctgttgga gcaatagaac cccagttcta cgagctgctg atcaaaggac ttggactaaa 840
gtctgatgaa cttcccaatc agatgagcac ggatgattgg ccagaaatga agaagaagtt 900
tgcagatgta tttgcaaaga agacgaaggc agagtggtgt caaatctttg acggcacaga 960
tgcctgtgtg actccggttc tgacttttga ggaggttgtt catcatgatc acaacaagga 1020
acggggctcg tttatcacca gtgaggagca ggacgtgagc ccccgccttg cacctctgct 1080
gttaaacacc ccagccatcc cttcttccaa aggggatcct ttcataggag aacacactga 1140
ggagatactt gaagaatttg gattcagccg agaagagatt tatcagctta actcagataa 1200
aatcattgaa agtaataagg taaaagctag tctctaactt ccaggcccac ggctcaagtg 1260
aatttgaata ctgcatttac agtgtagagt aacacataac attgtatgca tggaaacatg 1320
gaggaacagt attacagtgt cctaccactc taatcaagaa aagaattaca gactctgatt 1380
ctacagtgat gattgaattc taaaaatggt tatcattagg gcttttgatt tataaaactt 1440
tgggtactta tactaaatta tggtagttat tctgccttcc agtttgcttg atatatttgt 1500
tgatattaag attcttgact tatattttga atgggttcta gtgaaaaagg aatgatatat 1560
tcttgaagac atcgatatac atttatttac actcttgatt ctacaatgta gaaaatgagg 1620
aaatgccaca aattgtatgg tgataaaagt cacgtgaaac agagtgattg gttgcatcca 1680
ggccttttgt cttggtgttc atgatctccc tctaagcaca ttccaaactt tagcaacagt 1740
tatcacactt tgtaatttgc aaagaaaagt ttcacctgta ttgaatcaga atgccttcaa 1800
ctgaaaaaaa catatccaaa ataatgagga aatgtgttgg ctcactacgt agagtccaga 1860
gggacagtca gttttagggt tgcctgtatc cagtaactcg ggcctgtttt ccccgtgggt 1920
ctctgggctg tcagctttcc tttctccatg tgtttgattt ctcctcaggc tggtagcaag 1980
ttctggatct tatacccaac acacagcaac atccagaaat aaagatctca ggaccccccca 2040
aaaaaaaaaa aaaaaaaaaa aaaaaaa 2068

<210> 73
<211> 1252
<212> DNA
<213> Homo sapiens
<400> 73
cggccggttt tggtaggccc gggccgccgc caggcctccg cctgagcccg cacccgccat 60
ggacaactac gcagatcttt cggataccga gctgaccacc ttgctgcgcc ggtacaacat 120
cccgcacggg cctgtagtag gatcaactcg taggctttac gagaagaaga tcttcgagta 180
cgagacccag aggcggcggc tctcgccccc cagctcgtcc gccgcctcct cttatagctt 240
ctctgacttg aattcgacta gaggggatgc agatatgtat gatcttccca agaaagagga 300
cgctttactc taccagagca agggctacaa tgacgactac tatgaagaga gctacttcac 360
caccaggact tatggggagc ccgagtctgc cggcccgtcc agggctgtcc gccagtcagt 420
gacttcattc ccagatgctg acgctttcca tcaccaggtg catgatgacg atcttttgtc 480
ttcttctgaa gaggagtgca aggataggga acgccccatg tacggccggg acagtgccta 540
ccagagcatc acgcactacc gccctgtttc agcctccagg agctccctgg acctgtccta 600
ttatcctact tcctcctcca cctcttttat gtcctcctca tcatcttcct cttcatggct 660
cacccgccgt gccatccggc ctgaaaaccg tgctcctggg gctgggctgg gccaggatcg 720
ccaggtcccg ctctggggcc agctgctgct tttcctggtc tttgtgatcg tcctcttctt 780
catttaccac ttcatgcagg ctgaagaagg caaccccttc tagagggagc catgagggtc 840
tgggcttcag agctaggtct ttggggaagt cctggctgac tgccttagca gtgggggtgg 900
gggtggggc aggggcaggg gctttatgtg tttttgcttg gggggcgctg ggcctagccc 960
agagtagtgc ttgctccccc tgccttgtcc caccagggag gcagcagact caggccctcc 1020
atggtcctct ttgtcatttt gttgacatgc attcctcctt ttgtcatctt gttgggggga 1080
ggggattaac caaaggccac cctgactttg tttttgtgga cacacaataa aagccccgtt 1140
tatttgttaa aaaaaaaaaa aaaaaaaaaa aaaaaaaaaa aaaaaaaaaa aaaaaaaaaa 1200
aaaaaaaaaa aaaaaaaaaa aaaaaaaaaa aaaaaaaaaa aaaaaaaaaa aa 1252

<210> 74
<211> 1658
<212> DNA
<213> Homo sapiens
<400> 74
cttccccttc ccctcccagc gcgcccgcgc gccccgcggc cctcggcgag cagctcggct 60
ccccccagcg ctccccgggc ccaaagatat ggcaatggta gttagcagct ggcgagatcc 120
gcaggacgac gtggccgggg gcaaccccgg cggccccaac cccgcagcgc aggcggcccg 180
cggcggcggc ggcggcgccg gcgagcagca gcagcaggcg ggctcgggcg cgccgcacac 240

```
gccgcagacc ccgggccagc ccggagcgcc cgccaccccc ggcacggcgg gggacaaggg    300
ccagggcccg cccggttcgg gccagagcca gcagcacatc gagtgcgtgg tgtgcgggga    360
caagtcgagc ggcaagcact acggccaatt cacctgcgag ggctgcaaaa gtttcttcaa    420
gaggagcgtc cgcaggaact taacttacac atgccgtgcc aacaggaact gtcccatcga    480
ccagcaccac cgcaaccagt gccaatactg ccgcctcaag aagtgcctca aagtgggcat    540
gaggcgggaa gcggttcagc gaggaagaat gcctccaacc cagcccaatc caggccagta    600
cgcactcacc aacggggacc ccctcaacgg ccactgctac ctgtccggct acatctcgct    660
gctgctgcgc gccgagccct accccacgtc gcgctacggc agccagtgca tgcagcccaa    720
caacattatg ggcatcgaga acatctgcga gctggccgcg cgcctgctct tcagcgccgt    780
cgagtgggcc cgcaacatcc ccttcttccc ggatctgcag atcaccgacc aggtgtccct    840
gctacgcctc acctggagcg agctgttcgt gctcaacgcg gcccagtgct ctatgccgct    900
gcacgtggcg ccgttgctgg ccgccgccgg cctgcatgcc tcgcccatgt ctgccgaccg    960
cgtcgtggcc ttcatggacc acatccgcat cttccaggag caggtggaga agctcaaggc   1020
gctacacgtc gactcagccg agtacagctg cctcaaagcc atcgtgctgt tcacgtcaga   1080
cgcctgtggc ctgtcggatg cggcccacat cgagagcctg caggagaagt cgcagtgcgc   1140
actggaggag tacgtgagga gccagtaccc caaccagccc agccgttttg gcaaactgct   1200
gctgcgactg ccctcgctgc gcaccgtgtc ctcctccgtc atcgagcagc tcttcttcgt   1260
ccgtttggta ggtaaaaccc ccatcgaaac tctcatccgc gatatgttac tgtctgggag   1320
cagcttcaac tggccttaca tgtccatcca gtgctcctag accttgggcg cttcccacct   1380
gccccgtccc cctagagact cagaggaccc acctgggcca aggactccaa agccgcgggg   1440
acaccgggaa gtgcagcggg ccaggcaggc tgggtgggag ggaggagggc cgagacagga   1500
gcagcccacc cagcagaaat acaatccgag ctacaaagca tgggaaaaag agactctttt   1560
aggatcagat ctgtgagcac gttggcgagg aaaaacaaaa caaacaaaaa aaagaacctt   1620
gtgtctgtct ggtgaaaaaa aaaaaaaaaa aaaaaaa                            1658
```

<210> 75
<211> 2444
<212> DNA
<213> Homo sapiens
<400> 75

```
gcgttctcgg gaagctgctg ccgtagctgc cgccgccgct accaccgcgt tcgggtgtag     60
aatttggaat ccctgcgccg cgttaacaat gaagcagagt tcgaacgtgc cggctttcct    120
cagcaagctg tggacgcttg tggaggaaac ccacactaac gagttcatca cctggagcca    180
gaatggccaa agttttctgg tcttggatga gcaacgattt gcaaaagaaa ttcttcccaa    240
atatttcaag cacaataata tggcaagctt tgtgaggcaa ctgaatatgt atggtttccg    300
taaagtagta catatcgact ctggaattgt aaagcaagaa agagatggtc ctgtagaatt    360
tcagcatcct tacttcaaac aaggacagga tgacttgttg gagaacatta aaggaaggt    420
ttcatcttca aaaccagaag aaaataaaat tcgtcaggaa gatttaacaa aaattataag    480
tagtgctcag aaggttcaga taaaacagga aactattgag tccaggcttt ctgaattaaa    540
aagtgagaat gagtcccttt ggaaggaggt gtcagaatta cgagcaaagc atgcacaaca    600
gcaacaagtt attcgaaaga ttgtccagtt tattgttaca ttggttcaaa ataaccaact    660
tgtgagttta aaacgtaaaa ggcctctact tctaaacact aatggagccc aaaagaagaa    720
cctgtttcag cacatagtca aagaaccaac tgataatcat catcataaag ttccacacag    780
taggactgaa ggtttaaagc caagggagag gatttcagat gacatcatta tttatgatgt    840
tactgatgat aatgcagatg aagaaaatat cccagttatt ccagaaacta atgaggatgt    900
tatatctgat ccctccaact gtagccagta ccctgatatt gtcatcgttg aagatgacaa    960
tgaagatgag tatgcacctg tcattcagag tggagagcag aatgaaccag ccagagaatc   1020
cctaagttca ggcagtgatg gcagcagccc tctcatgtct agtgctgtcc agctaaatgg   1080
ctcatccagt ctgacctcag aagatccagt gaccatgatg gattccattt tgaatgataa   1140
catcaatctt ttgggaaagg ttgagctgtt ggattatctt gacagtattg actgcagttt   1200
agaggacttc caggccatgc tatcaggaag acaatttagc atagacccag atctcctggt   1260
tgatcttttc actagttctg tgcagatgaa tcccacagat tacatcaata atacaaaatc   1320
tgagaataaa ggattagaaa ctaccaagaa caatgtagtt cagccagttt cggaagaggg   1380
aagaaaatct aaatccaaac cagataagca gcttatccag tataccgcct ttccacttct   1440
tgcattcctc gatgggaacc ctgcttcttc tgttgaacag gcgagtacaa cagcatcatc   1500
agaagttttg tcctctgtag ataaacccat agaagttgat gagcttctgg atagcagcct   1560
agacccagaa ccaacccaaa gtaagcttgt tcgcctggag ccattgactg aagctgaagc   1620
tagtgaagct acactgtttt atttatgtga acttgctcct gcacctctgg atagtgatat   1680
gccactttta gatagctaaa tccccaggaa gtggacttta catgtatata ttcatcaaaa   1740
tgatgaacta tttattttaa agtatcattt ggtacttttt ttgtaaattg ctttgttttg   1800
tttaatcaga tactgtggaa taaaagcacc ttttgctttt ctcactaacc acacactctt   1860
gcagagcttt caggtgttac tcagctgcat agttacgcag atgtaatgca cattattggc   1920
gtatctttaa gttggattca aatggccatt tttctccaat tttggtaaat tggatatctt   1980
tttttttacaa atacgaccat taacctcagt taaatttttg tttgttttcc tgtttgatgc   2040
tgtctatttg cattgagtgt aagtcatttg aactaatggt ataactccta aagcttctct   2100
gctccagtta tttttattaa atatttttca cttggcttat ttttaaaact gggaacataa   2160
agtgcctgta tcttgtaaaa cttcatttgt ttcttttggt tcagagaagt tcatttatgt   2220
tcaaagacgt ttattcatgt tcaacaggaa agacaaagtg tacgtgaatg ctcgctgtct   2280
gataggggttc cagctccata tatatagaaa gatcggggggt gggatgggat ggagtgagcc   2340
```

ccatccagtt agttggacta gttttaaata aaggttttcc ggtttgtgtt tttttgaacc 2400
atactgttta taaaataaat acaatgaatg ttgagtacta gtgg 2444

<210> 76
<211> 3263
<212> DNA
<213> Homo sapiens
<400> 76
gctcctgaga ccggcgggca cacgggggtc tgtggccccc gccgtagcag tggctgccgc 60
cgtcgcttgg ttcccgtcgg tctgcgggag gcgggttatg gcggcggcgg cagtgagagc 120
tgtgaatgaa ttctccgggt ggacgaggga agaagaaagg ctccggcggc gccagcaacc 180
cggtgcctcc caggcctccg ccccttgcc tggcccccgc ccctcccgcc gccgggccgg 240
cccctccgcc cgagtcgccg cataagcgga acctgtacta tttctcctac ccgctgtttg 300
taggcttcgc gctgctgcgt ttggtcgcct tccacctggg gctcctcttc gtgtggctct 360
gccagcgctt ctcccgcgcc ctcatggcag ccaagaggag ctccggggcc gcgccagcac 420
ctgcctcggc ctcggccccg gcgccggtgc cgggcggcga ggccgagcgc gtccgagtct 480
tccacaaaca ggccttcgag tacatctcca ttgccctgcg catcgatgag gatgagaaag 540
caggacagaa ggagcaagct gtggaatggt ataagaaagg tattgaagaa ctggaaaaag 600
gaatagctgt tatagttaca ggacaaggtg aacagtgtga aagagctaga cgccttcaag 660
ctaaaatgat gactaatttg gttatggcca aggaccgctt acaacttcta gagaagatgc 720
aaccagtttt gccattttcc aagtcacaaa cggacgtcta taatgacagt actaacttgg 780
catgccgcaa tggacatctc cagtcagaaa gtggagctgt tccaaaaaga aaagacccct 840
taacacacac tagtaattca ctgcctcgtt caaaaacagt tatgaaaact ggatctgcag 900
gcctttcagg ccaccataga gcacctagtt acagtggttt atccatggtt tctggagtga 960
aacagggatc tggtcctgct cctaccactc ataagggtac tccgaaaaca aataggacaa 1020
ataaaccttc taccctaca actgctactc gtaagaaaaa agacttgaag aattttagga 1080
atgtggacag caaccttgct aaccttataa tgaatgaaat tgtggacaat ggaacagctg 1140
ttaaatttga tgatatagct ggtcaagact tggcaaaaca agcattgcaa gaaattgtta 1200
ttcttccttc tctgaggcct gagttgttca cagggcttag agctcctgcc agagggctgt 1260
tactctttgg tccacctggg aatgggaaga caatgctggc taaagcagta gctgcagaat 1320
cgaatgcaac cttctttaat ataagtgctg caagtttaac ttcaaaatac gtgggagaag 1380
gagagaaatt ggtgagggct ctttttgctg tggctcgaga acttcaacct tctataattt 1440
ttatagatga agttgatagc cttttgtgtg aaagaagaga aggggagcac gatgctagta 1500
gacgcctaaa aactgaattt ctaatagaat ttgatggtgt acagtctgct ggagatgaca 1560
gagtacttgt aatgggtgca actaataggc cacaagagct tgatgaggct gttctcaggc 1620
gtttcatcaa acgggtatat gtgtctttac caaatgagga gacaagacta cttttgctta 1680
aaaatctgtt atgtaaacaa ggaagtccat tgacccaaaa agaactagca caacttgcta 1740
gaatgactga tggatactca ggaagtgacc taacagcttt ggcaaaagat gcagcactgg 1800
gtcctatccg agaactaaaa ccagaacagg tgaagaatat gtctgccagt gagatgagaa 1860
atattcgatt atctgacttc actgaatcct tgaaaaaaat aaaacgcagc gtcagccctc 1920
aaactttaga agcgtacata cgttggaaca aggactttgg agataccact gtttaaggaa 1980
ataccttgt aaacctgcag aacattttac ttaaaagagg aaacacaaga tcttcaatga 2040
acgtcatcgg ctacagaaac agcctaagtt tacaggactt tttagagtct tacatatttg 2100
tgcaccaaac ttgaagatga accagaaaac agacttaaac aaaatataca atgcaaatgt 2160
aatttttgt tgtttaaggc cttgccttga tggtcacagt tatcccaatg gacactaagt 2220
tagagcacaa caaaacctga ttctggtctt ctttaccaat ataatcataa tgtaaataat 2280
aatttgtata ttgtgttgca gatgaaagta ttccaggaac agtgaatggt agaagacaca 2340
agaacatttg tttgtttgtc ttctgatgtt ttttcttaaa atagtaattt ctcctacttt 2400
tcttttctac tgttgtctta actacaggtg attggaatgc caaacactct taagtttatt 2460
ttcttttttc gttttataaa ttcagtgtgc caaatgaaac ttttttccta agtaactgta 2520
ataggaaaaa gtttattttg agagtttctt cttcataaat ctacagacat taaacaattg 2580
ttgtgttctt tttacctttt atttttctat taccttgcta ccaaacagtt tagatagcaa 2640
tataatagca aaaaagcaaa tatggtaaaa tagagaaggt ttgaaggttt gagttactct 2700
gtcatataac atgtagatca gtcttcatgt gacctgcagt attttttttt ctaatgtatt 2760
tgtcagaaat ctgttgtaga ctgttaactt cttcctgatg gaatttattt tctgcaagaa 2820
ttattctgat atttaagaga gccaatttta actgctgtga aaatgtttcc agtgcaagag 2880
aagggaaata ctaggaacta agacatttct aatttattgc ttattacttt cttaatttta 2940
caggataatt ataagcaagt ggaactacca tcttttattc ttaataatta ttaatccctt 3000
caatgaaact ttaaaaaaac tgaattttta tacatggcat acatttttct agttccttct 3060
gcttgcttta ttaactcaaa agttctagtt ctagtctgtt gatctgcctt ttgttctccc 3120
aaaatgtaca gtaattccat ttgtttgtat aaatatgcct ggattttcat tataaaaatg 3180
tcattgtagg gagtagagac tcatatcatg gccttttaaa tattgtaata aaggcaaata 3240
gatatttgcc cttagtttac tgg 3263

<210> 77
<211> 6452
<212> DNA
<213> Homo sapiens
<400> 77

```
cgagcgagtg tcatggcggc cggcgtcgag ttggcaggag taacccacgg aactgaggaa      60
agtcattaga gctgagaaag aagtggccca atctggacgg tgggaattcg tgggaatgag     120
cagaaggccc tccgtagtga ctgtgtcact agaggcgggc ccctggtaaa attccaggcc     180
aggcctctgc gtttctaggc agaacctgga gtcggccttg cctgagaacc cagctttgtg     240
ttatcgtatc ctgtctcgcg aaggcaggcg ttcaaggata tttggtcgga tcgcccggcg     300
gcgctaaacg ttttcttttt tccgagcgga ccgggtcgtt ctctaaactc gccgcgatgt     360
cgtcctggct tgggggcctc ggctccggat tgggccagtc tctgggtcaa gtcgggggca     420
gcctggcttc cctcactggc cagatatcaa actttacaaa ggatatgctg atggagggca     480
cggaggaagt ggaagcagaa ttacctgatt ctaggacaaa ggaaattgaa gccattcatg     540
caatcttgag atcagagaat gaaaggctta agaaactttg tactgatcta gaagagaaac     600
atgaagcatc agagattcaa ataaagcagc aatctacaag ttaccgaaat caacttcaac     660
aaaaagaggt agaaatcagc catcttaaag ccagacagat tgcactccag gatcagttgc     720
tgaaactgca gtcagctgct cagtcagtac cttcaggagc tggtgtacca gcaaccactg     780
catcatcttc attcgcttat gggattagtc atcatccttc agctttccat gacgatgaca     840
tggactttgg tgatataatt tcatcccaac aagaaataaa ccgactctca aatgaagttt     900
caagacttga gtctgaagtt ggccattgga ggcatattgc tcagacttcc aaagcacaag     960
gaacagataa ctctgatcaa agtgaaatat gtaaactaca aaatatcatt aaggaactaa    1020
aacagaaccg aagtcaggaa attgatgacc atcaacatga aatgtcagta ctgcagaatg    1080
cacaccaaca gaaattgaca gaaataagtc gacgacatcg agaagaatta agtgactatg    1140
aagaacgaat tgaagaactt gaaaatctgt tacaacaagg tggctctgga gttatagaaa    1200
ctgatctctc taaaatctat gagatgcaaa aaactattca agttctacaa atagaaaaag    1260
tggagtctac caaaaaaatg gaacaacttg aggataaaat aaaagatata aataaaaaat    1320
tatcttctgc agaaaatgac agagatattt tgaggaggga acaagaacag ctaaatgtgg    1380
aaaagagaca aataatggaa gaatgtgaaa acttgaaatt ggaatgtagt aaattgcagc    1440
cttctgctgt gaagcaaagt gatactatga cagaaaagga aagaattctt gcccagagtg    1500
catcagtgga agaagtgttc agactacaac aagcactgtc tgatgccgaa aatgaaataa    1560
tgagattgag tagtttaaac caggataaca gtcttgctga agacaatctg aaacttaaaa    1620
tgcgtatcga agttttagaa aaagagaagt cattactgag tcaagaaaag gaagaacttc    1680
agatgtcact tttaaaattg aacaatgaat atgaagtaat taaaagtaca gctacaagag    1740
acataagttt ggattcagaa ttacatgact taagacttaa tttggaggca aaggaacaag    1800
aactcaatca gagtattagt gaaaaggaaa cactgatagc tgagatagaa gaattggaca    1860
gacagaatca agaagctaca aagcacatga ttttgataaa agatcagcta tcaaaacaac    1920
aaaatgaagg agatagcatc atcagtaaac tgaaacaaga tctaaatgat gaaaaaaaga    1980
gagttcatca acttgaagat gataaaatgg acattactaa agagttagat gtacagaaag    2040
aaaagctaat tcaaagtgaa gtggccctaa atgatttaca tttaaccaag cagaaacttg    2100
aggacaaagt agaaaattta gtagatcagc taaataaatc acaagaaagt aatgtaagca    2160
tccagaagga gaatttagaa cttaaggagc atattagaca aaatgaggag gagctttcta    2220
gaataaggaa tgagttaatg cagtctctaa atcaagactc taatagtaat tttaaggata    2280
ccttacttaa agaaagagaa gctgaagtta gaaacttaaa gcaaaatctt tcagaattag    2340
aacagctcaa tgaaaattta aagaaagttg cttttgatgt caaaatggaa aatgaaaagt    2400
tagttttagc atgtgaagat gtgaggcatc agttagaaga atgtcttgct ggtaacaatc    2460
agctttctct ggaaaaaaac actattgtgg agactctaaa aatggaaaaa ggagagatag    2520
aggcagaatt gtgttgggct aaaaagaggc tgttggaaga agcaaacaag tatgagaaaa    2580
ccattgaaga actgtcaaat gcacgtaatt tgaatacctc tgccttacag ctggaacatg    2640
agcatttaat taaactcaat caaaagaaag acatggaaat agcagaactc aaaaagaata    2700
ttgaacaaat ggatactgac cataaagaaa ctaaggacgt tttgtcatct agtttagaag    2760
agcagaagca gttgacacaa cttataaaca agaaagaaat ttttattgaa aagcttaaag    2820
aaagaagttc aaagctgcag gaggaattgg ataaatattc tcaggcctta agaaaaaatg    2880
aaattttaag acagaccata gaggaaaaag accgaagtct tggatccatg aaagaggaaa    2940
ataatcatct gcaagaagaa ttggaacgac tcagggaaga gcagagtcga accgcacctg    3000
tggctgaccc taaaaccctt gatagtgtta ctgaactagc atctgaggta tctcaactga    3060
acacgatcaa ggaacatctt gaagaggaaa ttaaacatca tcaaaagata attgaagatc    3120
aaaaccagag taagatgcaa ctacttcagt ctttacaaga gcaaaagaag gaaatggatg    3180
agtttagata ccagcatgag caaatgaacg ccacacacac ccagctcttt ttagagaagg    3240
atgaggaaat taagagtttg caaaaaacaa ttgaacaaat caaaacccag ttgcatgaag    3300
aaagacagga cattcaaaca gataactctg atatttttca agaaacaaaa gttcagagcc    3360
ttaatataga aaatggaagt gaaaagcatg atttatctaa agctgaaacg gaaagattag    3420
tgaaaggaat aaaagagcga gaactggaga ttaaacttct aaatgaaaag aatatatctt    3480
taactaaaca gattgatcag ttgtccaaag atgaagttgg taaactaact cagattattc    3540
agcagaaaga tttggagata caagctcttc atgctagaat ttcttcaact tcccatactc    3600
aagatgttgt ttaccttcaa cagcaactgc aggcttatgc tatggaaaga gaaaaggtat    3660
ttgctgtttt gaatgagaag actagggaaa atagccatct aaaaacagaa tatcacaaaa    3720
tgatggatat tgttgctgcc aaggaagcag ctcttatcaa actgcaagat gaaaataaaa    3780
aattgtccac tagatttgaa agtagtggcc aagatatgtt tagagaaact attcagaatt    3840
tatcacgtat cattcgagaa aaagacatcg aaatagatgc actaagtcag aaatgtcaga    3900
ctttattggc agttttacaa acatccagca ctggtaatga ggctggaggt gttaatagtc    3960
atcaatttga ggagcttcta caggaacgtg acaagttaaa acagcaagta aagaaaatgg    4020
aagagtggaa gcagcaggtg atgaccacag tacaaaatat gcaacacgag tcagcccagc    4080
ttcaggaaga gcttcaccaa cttcaagcac aggttttggt tgacagtgat aataattcta    4140
```

aattacaagt ggactatact ggcctgatcc aaagttatga gcagaatgaa accaaactca 4200
aaaattttgg gcaggaatta gcacaagttc agcacagcat tgggcagctt tgcaatacca 4260
aggatcttct tttaggaaaa cttgatatta tttcacccca gctgtcttct gcatcattgc 4320
ttactcccca gtctgcagag tgtcttagag caagtaagtc tgaagtattg agtgaatctt 4380
ctgaattgct tcagcaagag ttagaagagc taagaaaatc actacaggaa aaagatgcaa 4440
caattagaac tctccaggaa aataaccaca gattgtctga ttcgattgct gccacctcag 4500
agctagaaag aaaagaacac gaacaaaccg attcagaaat caagcagcta aaggagaaac 4560
aagatgtttt gcaaaagtta cttaaggaaa aagacctctt aatcaaagcc aaaagtgatc 4620
aactactttc ttccaatgaa aatttcacta acaaagtaaa tgaaaacgaa cttttgaggc 4680
aggcagtaac aaacctgaag gagagaatat taattctaga gatggacatt ggcaaactaa 4740
aaggagaaaa tgaaaaaata gtggaaacat acaggggaaa ggaaacagaa tatcaagcgt 4800
tacaagagac taacatgaag ttttctatga tgctgcgaga aaaagagttt gagtgccact 4860
caatgaagga gaaggctctt gcttttgaac agctattgaa agagaaagaa cagggcaaga 4920
ctggagagtt aaatcagctt ttaaatgcag ttaaatcaat gcaggagaag acagttgtgt 4980
ttcaacagga gagagaccaa gtcatgttgg ccctgaaaca aaaacaaatg gaaaatactg 5040
ccctacagaa tgaggttcaa cgtttacgtg acaaagaatt tcgttcaaac caagagctag 5100
agagattgcg taatcatctt ttagaatcag aagattctta tacccgtgaa gctttggctg 5160
cagaagatag agaggctaaa ctaagaaaga aagtcacagt attggaggaa aagctagttt 5220
catcctctaa tgcaatggaa aatgcaagcc atcaagccag tgtgcaggta gagtcattgc 5280
aagaacagtt gaatgtagtt tccaagcaaa gggatgaaac tgcgctgcag ctttctgtct 5340
ctcaggaaca agtaaagcag tatgctctgt cactggccaa cctgcagatg gtactagagc 5400
atttccaaca agaggaaaaa gctatgtatt ctgctgaact cgaaaagcaa aaacagctta 5460
tagctgaatg gaagaaaaac gcagaaaatc tggaaggaaa agtgatatca ttacaggaat 5520
gtttggatga agcaaatgct gcattggatt cagcatcaag acttacagaa cagttagatg 5580
taaaagaaga acaaattgaa gaacttaaaa gacaaaatga gctccgacaa gaaatgctgg 5640
atgatgtaca aaagaaattg atgagcttag caaacagctc agaaggaaaa gtagacaaag 5700
tcctaatgag aaacctcttc attggtcatt tccacacacc gaaaaatcag cgtcatgaag 5760
tgttacggtt aatggggagc atcctgggcg tcagaaggga ggagatggag cagttgtttc 5820
atgacgatca gggcagtgtt accaggtgga tgactgggtg gcttggagga ggatcaaaaa 5880
gtgttcccaa cacacctttg agaccaaatc agcaatctgt ggttaatagt tctttttcag 5940
aactttttgt taaatttcta gaaacagaat ctcatccatc cattccacca ccaaagcttt 6000
ctgttcatga tatgaaacct ctggattcac caggaagaag aaaaagagat acaaatgcac 6060
cagaaagttt taaagataca gcagaatcca ggtctggtag aagaacagat gtaaatccgt 6120
ttttggctcc tcgctcggca gctgtacctc ttattaaccc agctggactt ggacctggtg 6180
ggcccgggca tcttcttctg aaacccatct cagatgtttt gcccacattt acacctttgc 6240
cagcgttacc tgacaacagt gctggggttg tgctgaaaga ccttttaaag caatagatga 6300
ttctcaagcc agagacaatc tagcacttta aagaaaccat gaacactata tgtatgtact 6360
ttatcacaaa gtggcctttg ggagaaagt catgtatttg ttcgcaatta tgctttctct 6420
gaatttaata aaaatattcc taatgctttt ag 6452

<210> 78
<211> 2648
<212> DNA
<213> Homo sapiens
<400> 78

gcctctctcc atcagacacc ccaaggttcc atccgaagca ggcggagcac cgaacgcacc 60
ccggggtggt cagggacccc catccgtgct gccccctagg agcccgcgcc tctcctctgc 120
gccccgcctc tcgggccgca acatcgcgcg gttcctttaa cagcgcgctg gcagggtgtg 180
ggaagcagga ccgcgtcctc ccgccccctc ccatccgagt ttcaggtgaa ttggtcaccg 240
agggaggagg ccgacacacc acacctacac tcccgcgtcc acctctccct ccctgcttcc 300
tctggcggag gcggcaggaa ccgagagcca ggtccagagc gccgaggagc cggtctagga 360
cgcagcagat tggtttatct tggaagctaa agggcattgc tcatcctgaa gatcagctga 420
ccattgacaa tcagccatgt catccaggcc tcttgaaagt ccacctcctt acaggcctga 480
tgaattcaaa ccgaatcatt atgcaccaag caatgacata tatggtggag agatgcatgt 540
tcgaccaatg ctctctcagc cagcctactc tttttaccca gaagatgaaa ttcttcactt 600
ctacaaatgg acctctcctc caggagtgat tcggatcctg tctatgctca ttattgtgat 660
gtgcattgcc atctttgcct gtgtggcctc cacgcttgcc tgggacagag gctatggaac 720
ttccctttta ggaggtagtg taggctaccc ttatggagga agtggctttg gtagctacgg 780
aagtggctat ggctatggct atggttatgg ctatggctac ggaggctata cagacccaag 840
agcagcaaag ggcttcatgt tggccatggc tgccttttgt ttcattgccg cgttggtgat 900
ctttgttacc agtgttataa gatctgaaat gtccagaaca agaagatact acttaagtgt 960
gataatagtg agtgctatcc tgggcatcat ggtgtttatt gccacaattg tctatataat 1020
gggagtgaac ccaactgctc agtcttctgg atctctatat ggttcacaaa tatatgccct 1080
ctgcaaccaa ttttatacac ctgcagctac tggactctac gtggatcagt atttgtatca 1140
ctactgtgtt gtggatcccc aggaggccat tgccattgta ctggggttca tgattattgt 1200
ggcttttgct ttaataattt tctttgctgt gaaaactcga agaaagatgg acaggtatga 1260
caagtccaat attttgtggg acaaggaaca catttatgat gagcagcccc ccaatgtcga 1320
ggagtgggtt aaaaatgtgt ctgcaggcac acaggacgtg ccttcacccc catctgacta 1380
tgtggaaaga gttgacagtc ccatggcata ctcttccaat ggcaaagtga atgacaagcg 1440

gttttatcca gagtcttcct ataaatccac gccggttcct gaagtggttc aggagcttcc 1500
attaacttcg cctgtggatg acttcaggca gcctcgttac agcagcggtg gtaactttga 1560
gacaccttca aaaagagcac ctgcaaaggg aagagcagga aggtcaaaga gaacagagca 1620
agatcactat gagacagact acacaactgg cggcgagtcc tgtgatgagc tggaggagga 1680
ctggatcagg gaatatccac ctatcacttc agatcaacaa agacaactgt acaagaggaa 1740
ttttgacact ggcctacagg aatacaagag cttacaatca gaacttgatg agatcaataa 1800
agaactctcc cgtttggata aagaattgga tgactataga gaagaaagtg aagagtacat 1860
ggctgctgct gatgaataca atagactgaa gcaagtgaag ggatctgcag attacaaaag 1920
taagaagaat cattgcaagc agttaaagag caaattgtca cacatcaaga agatggttgg 1980
agactatgat agacagaaaa catagaaggc tgatgccaag ttgtttgaga aattaagtat 2040
ctgacatctc tgcaatcttc tcagaaggca aatgactttg gaccataacc ccggaagcca 2100
aacctctgtg agcatcacaa agttttggtt gctttaacat catcagtatt gaagcatttt 2160
ataaatcgct tttgataatc aactgggctg aacactccaa ttaaggattt tatgctttaa 2220
acattggttc ttgtattaag aatgaaatac tgtttgaggt ttttaagcct taaaggaagg 2280
ttctggtgtg aactaaactt tcacacccca gacgatgtct tcatacctac atgtatttgt 2340
ttgcataggt gatctcattt aatcctctca accacctttc agataactgt tatttataat 2400
cactttttc cacataagga aactgggttc ctgcaatgaa gtctctgaag tgaaactgct 2460
tgtttcctag cacacacttt tggttaagtc tgttttatga cttcattaat aataaattcc 2520
ctggcctttc atattttagc tactatatat gtgatgatct accagcctcc ctattttttt 2580
tctgttatat aaatggttaa aagaggtttt tcttaaataa taaagatcat gtaaaagtaa 2640
aaaaaaaa 2648

<210> 79
<211> 7193
<212> DNA
<213> Homo sapiens
<400> 79

agaataaggg cagggaccgc ggctcctatc tcttggtgat ccccttcccc attccgcccc 60
cgcctcaacg cccagcacag tgccctgcac acagtagtcg ctcaataaat gttcgtggat 120
gatgatgatg atgatgatga aaaaaatgca gcatcaacgg cagcagcaag cggaccacgc 180
gaacgaggca aactatgcaa gaggcaccag acttcctctt tctggtgaag gaccaacttc 240
tcagccgaat agctccaagc aaactgtcct gtcttggcaa gctgcaatcg atgctgctag 300
acaggccaag gctgcccaaa ctatgagcac ctctgcaccc ccacctgtag gatctctctc 360
ccaaagaaaa cgtcagcaat acgccaagag caaaaaacag ggtaactcgt ccaacagccg 420
acctgcccgc gcccttttct gtttatcact caataacccc atccgaagag cctgcattag 480
tatagtggaa tggaaaccat ttgacatatt tatattattg gctatttttg ccaattgtgt 540
ggccttagct atttacatcc cattccctga agatgattct aattcaacaa atcataactt 600
ggaaaaagta gaatatgcct tcctgattat ttttacagtc gagacatttt tgaagattat 660
agcgtatgga ttattgctac atcctaatgc ttatgttagg aatggatgga atttactgga 720
ttttgttata gtaatagtag gattgtttag tgtaattttg gaacaattaa ccaaagaaac 780
agaaggcggg aaccactcaa gcggcaaatc tggaggcttt gatgtcaaag ccctccgtgc 840
ctttcgagtg ttgcgaccac ttcgactagt gtcaggggtg cccagtttac aagttgtcct 900
gaactccatt ataaaagcca tggttcccct ccttcacata gccctttttgg tattatttgt 960
aatcataatc tatgctatta taggattgga actttttatt ggaaaaatgc acaaaacatg 1020
tttttttgct gactcagata tcgtagctga agaggaccca gctccatgtg cgttctcagg 1080
gaatggacgc cagtgtactg ccaatggcac ggaatgtagg agtggctggg ttggcccgaa 1140
cggaggcatc accaactttg ataactttgc ctttgccatg cttactgtgt ttcagtgcat 1200
caccatggag ggctggacag acgtgctcta ctgggtaaat gatgcgatag gatgggaatg 1260
gccatgggtg tattttgtta gtctgatcat ccttggctca tttttcgtcc ttaacctggt 1320
tcttggtgtc cttagtggag aattctcaaa ggaaagagag aaggcaaaag cacggggaga 1380
tttccagaag ctccgggaga agcagcagct ggaggaggat ctaaagggct acttggattg 1440
gatcacccaa gctgaggaca tcgatccgga gaatgaggaa gaaggaggag aggaaggcaa 1500
acgaaatact agcatgccca ccagcgagac tgagtctgtg aacacagaga acgtcagcgg 1560
tgaaggcgag aaccgaggct gctgtggaag tctctggtgc tggtggagac ggagaggcgc 1620
ggccaaggcg gggccctctg ggtgtcggcg gtggggtcaa gccatctcaa aatccaaact 1680
cagccgacgc tggcgtcgct ggaaccgatt caatcgcaga agatgtaggg ccgccgtgaa 1740
gtctgtcacg ttttactggc tggttatcgt cctggtgttt ctgaacacct taaccatttc 1800
ctctgagcac tacaatcagc cagattggtt gacacagatt caagatattg ccaacaaagt 1860
cctcttggct ctgttcacct gcgagatgct ggtaaaaatg tacagcttgg gcctccaagc 1920
atatttcgtc tctcttttca accggtttga ttgcttcgtg gtgtgtggtg gaatcactga 1980
gacgatcctg gtggaactgg aaatcatgtc tcccctgggg atctctgtgt ttcggtgtgt 2040
gcgcctctta agaatcttca aagtgaccag gcactggact tccctgagca acttagtggc 2100
atccttatta aactccatga agtccatcgc ttcgctgttg cttctgcttt ttctcttcat 2160
tatcatcttt tccttgcttg ggatgcagct gtttggcggc aagtttaatt ttgatgaaac 2220
gcaaaccaag cggagcacct ttgacaattt ccctcaagca cttctcacag tgttccagat 2280
cctgacaggc gaagactgga atgctgtgat gtacgatggc atcatggctt acgggggccc 2340
atcctcttca ggaatgatcg tctgcatcta cttcatcatc ctcttcattt gtggtaacta 2400
tattctactg aatgtcttct tggccatcgc tgtagacaat ttggctgatg ctgaaagtct 2460
gaacactgct cagaaagaag aagcggaaga aaaggagagg aaaaagattg ccagaaaaga 2520

```
gagcctagaa aataaaaaga acaacaaacc agaagtcaac cagatagcca acagtgacaa 2580
caaggttaca attgatgact atagagaaga ggatgaagac aaggacccct atccgccttg 2640
cgatgtgcca gtaggggaag aggaagagga agaggaggag gatgaacctg aggttcctgc 2700
cggaccccgt cctcgaagga tctcggagtt gaacatgaag gaaaaaattg cccccatccc 2760
tgaagggagc gctttcttca ttcttagcaa gaccaacccg atccgcgtag gctgccacaa 2820
gctcatcaac caccacatct tcaccaacct catccttgtc ttcatcatgc tgagcagcgc 2880
tgccctggcc gcagaggacc ccatccgcag ccactccttc cggaacacga tactgggtta 2940
ctttgactat gccttcacag ccatctttac tgttgagatc ctgttgaaga tgacaacttt 3000
tggagctttc ctccacaaag ggccttctgc caggaactac ttcaatttgc tggatatgct 3060
ggtggttggg gtgtctctgg tgtcatttgg gattcaatcc agtgccatct ccgttgtgaa 3120
gattctgagg gtcttaaggg tcctgcgtcc cctcagggcc atcaacagag caaaaggact 3180
taagcacgtg gtccagtgcg tcttcgtggc catccggacc atcggcaaca tcatgatcgt 3240
cactaccctc ctgcagttca tgtttgcctg tatcggggtc cagttgttca aggggaagtt 3300
ctatcgctgt acggatgaag ccaaaagtaa ccctgaagaa tgcaggggac ttttcatcct 3360
ctacaaggat ggggatgttg acagtcctgt ggtccgtgaa cggatctggc aaaacagtga 3420
tttcaacttc gacaacgtcc tctctgctat gatggcgctc ttcacagtct ccacgtttga 3480
gggctggcct gcgttgctgt ataaagccat cgactcgaat ggagagaaca tcggcccaat 3540
ctacaaccac cgcgtggaga tctccatctt cttcatcatc tacatcatca ttgtagcttt 3600
cttcatgatg aacatctttg tgggctttgt catcgttaca tttcaggaac aaggagaaaa 3660
agagtataag aactgtgagc tggacaaaaa tcagcgtcag tgtgttgaat acgccttgaa 3720
agcacgtccc ttgcggagat acatccccaa aaacccctac cagtacaagt tctggtacgt 3780
ggtgaactct tcgcctttcg aatacatgat gtttgtcctc atcatgctca acacactctg 3840
cttggccatg cagcactacg agcagtccaa gatgttcaat gatgccatgg acattctgaa 3900
catggtcttc accggggtgt tcaccgtcga gatggttttg aaagtcatcg catttaagcc 3960
taaggggtat tttagtgacg cctggaacac gtttgactcc ctcatcgtaa tcggcagcat 4020
tatagacgtg gccctcagcg aagcggaccc aactgaaagt gaaaatgtcc ctgtcccaac 4080
tgctacacct gggaactctg aagagagcaa tagaatctcc atcacctttt tccgtctttt 4140
ccgagtgatg cgattggtga agcttctcag caggggggaa ggcatccgga cattgctgtg 4200
gacttttatt aagtcctttc aggcgctccc gtatgtggcc ctcctcatag ccatgctgtt 4260
cttcatctat gcggtcattg gcatgcagat gtttgggaaa gttgccatga gagataacaa 4320
ccagatcaat aggaacaata acttccagac gtttccccag gcggtgctgc tgctcttcag 4380
gtgtgcaaca ggtgaggcct ggcaggagat catgctggcc tgtctcccag ggaagctctg 4440
tgaccctgag tcagattaca accccgggga ggagtataca tgtgggagca actttgccat 4500
tgtctatttc atcagttttt acatgctctg tgcatttctg atcatcaatc tgtttgtggc 4560
tgtcatcatg gataatttcg actatctgac ccgggactgg tctattttgg ggcctcacca 4620
tttagatgaa ttcaaaagaa tatggtcaga atatgaccct gaggcaaagg gaaggataaa 4680
acaccttgat gtggtcactc tgcttcgacg catccagcct cccctggggt ttgggaagtt 4740
atgtccacac agggtagcgt gcaagagatt agttgccatg aacatgcctc tcaacagtga 4800
cgggacagtc atgtttaatg caaccctgtt tgctttggtt cgaacggctc ttaagatcaa 4860
gaccgaaggg aacctggagc aagctaatga agaacttcgg gctgtgataa agaaaatttg 4920
gaagaaaacc agcatgaaat tacttgacca agttgtccct ccagctggtg atgatgaggt 4980
aaccgtgggg aagttctatg ccactttcct gatacaggac tactttagga aattcaagaa 5040
acggaaagaa caaggactgg tgggaaagta ccctgcgaag aacaccacaa ttgccctaca 5100
ggcgggatta aggacactgc atgacattgg gccagaaatc cggcgtgcta tatcgtgtga 5160
tttgcaagat gacgagcctg aggaaacaaa acgagaagaa gaagatgatg tgttcaaaag 5220
aaatggtgcc ctgcttggaa accatgtcaa tcatgttaat agtgatagga gagattccct 5280
tcagcagacc aataccaccc accgtcccct gcatgtccaa aggccttcaa ttccacctgc 5340
aagtgatact gagaaaccgc tgtttcctcc agcaggaaat tcggtgtgtc ataaccatca 5400
taaccataat tccataggaa agcaagttcc cacctcaaca aatgccaatc tcaataatgc 5460
caatatgtcc aaagctgccc atggaaagcg gcccagcatt gggaaccttg agcatgtgtc 5520
tgaaaatggg catcattctt cccacaagca tgaccgggag cctcagagaa ggtccagtgt 5580
gaaaagaacc cgctattatg aaacttacat taggtccgac tcaggagatg aacagctccc 5640
aactatttgc cgggaagacc cagagataca tggctatttc agggaccccc actgcttggg 5700
ggagcaggag tatttcagta gtgaggaatg ctacgaggat gacagctcgc ccacctggag 5760
caggcaaaac tatggctact acagcagata cccaggcaga aacatcgact ctgagaggcc 5820
ccgaggctac catcatcccc aaggattctt ggaggacgat gactcgcccg tttgctatga 5880
ttcacggaga tctccaagga gacgcctact acctcccacc ccagcatccc accggagatc 5940
ctccttcaac tttgagtgcc tgcgccggca gagcagccag gaagaggtcc cgtcgtctcc 6000
catcttcccc catcgcacgg ccctgcctct gcatctaatg cagcaacaga tcatggcagt 6060
tgccggccta gattcaagta aagcccagaa gtactcaccg agtcactcga cccggtcgtg 6120
ggccacccct ccagcaaccc ctccctaccg ggactggaca ccgtgctaca ccccccctgat 6180
ccaagtggag cagtcagagg ccctggacca ggtgaacggc agcctgccgt ccctgcaccg 6240
cagctcctgg tacacagacg agcccgacat ctcctaccgg actttcacac cagccagcct 6300
gactgtcccc agcagcttcc ggaacaaaaa cagcgacaag cagaggagtg cggacagctt 6360
ggtggaggca gtcctgatat ccgaaggctt gggacgctat gcaagggacc caaaatttgt 6420
gtcagcaaca aaacacgaaa tcgctgatgc ctgtgacctc accatcgacg agatggagag 6480
tgcagccagc accctgctta atggaacgt gcgtccccga gccaacgggg atgtgggccc 6540
cctctcacac cggcaggact atgagctaca ggactttggt cctggctaca gcgacgaaga 6600
gccagaccct gggagggatg aggaggacct ggcggatgaa atgatatgca tcaccacctt 6660
```

```
gtagccccca gcgaggggca gactggctct ggcctcaggt ggggcgcagg agagccaggg   6720
gaaaagtgcc tcatagttag gaaagtttag gcactagttg ggagtaatat tcaattaatt   6780
agacttttgt ataagagatg tcatgcctca agaaagccat aaacctggta ggaacaggtc   6840
ccaagcggtt gagcctggca gagtaccatg cgctcggccc cagctgcagg aaacagcagg   6900
ccccgccctc tcacagagga tgggtgagga ggccagacct gccctgcccc attgtccaga   6960
tgggcactgc tgtggagtct gcttctccca tgtaccaggg caccaggccc acccaactga   7020
aggcatggcg gcggggtgca ggggaaagtt aaaggtgatg acgatcatca cacctcgtgt   7080
cgttacctca gccatcggtc tagcatatca gtcactgggc ccaacatatc cattttttaaa   7140
ccctttcccc caaatacact gcgtcctggt tcctgtttag ctgttctgaa ata          7193
```

<210> 80
<211> 2907
<212> DNA
<213> Homo sapiens
<400> 80

```
ggaaccatgg agctcagcgt cctcctcttc cttgcactcc tcacaggcct cttgctactc     60
ctggttcagc gtcaccctaa ctcccatggc accctcccac cagggccccg ccctctgccc    120
cttttgggga accttctgca gatggacaga agaggcctac tcaaatcctt tctgaggttc    180
cgagagaaat atggggacgt cttcacggta cacctgggac cgaggcccgt ggtcatgctg    240
tgtggagtag aggccatacg ggaggccctg gtggacaacg ctgaggcctt ctctggccgg    300
ggaaaaatcg tcatcatgga cccagtctac cagggatatg gcatgctctt tgccaatgga    360
aaccgctgga aggtgcttcg gcgattctct gtgaccacca tgagggactt cgggatggga    420
aagcggagtg tggaggagcg gattcaggac gaggctcagt gtctgataga ggaacttcgg    480
aaatccaagg gagccctcgt ggaccccacc ttcctcttcc attccattac cgccaacatc    540
atctgctcca tcatctttgg aaaacgcttc cactaccaag atcaagagtt cctgaagacg    600
ctgaacttgt tctgccagag tttcttactc atcagctcta tatccagcca gctgtttgag    660
ctcttctctg gcttcttgaa atactttcct ggggcacaca ggcaagttta caaaaaccta    720
caggaaatca atgcttacat tggccacagt gtggagaagc accgtgaaac cctggacccc    780
agcgccccca gggacctcat cgacacctac ctgctccaca tggaaaaaga gaaatccaac    840
ccacacagtg aattcagcca ccagaacctc atcatcaaca cgctctcgct cttctttgct    900
ggcactgaga ccaccagcac cactctccgc tacggcttcc tgctcatgct caaataccct    960
catgtcgcag agagagtcta caaggagatt gaacaggtgg ttggcccaca tcgccctcca   1020
gcgcttgatg accgagccaa aatgccatac acagaggcag tcatccgtga gattcagaga   1080
tttgctgacc ttctccccat gggtgtgccc cacattgtca cccaacacac cagcttctga   1140
gggtacacca tccccaagga cacggaagta tttctcatcc tgagcactgc tctccgtgac   1200
ccacactact ttgaaaaacc agacgccttc aatcctgacc actttctgga tgccaatggg   1260
gcactgaaaa agaatgaagc ttttatcccc ttctccttag ggaagcggat ttgtcttggt   1320
gaaggcattg cccgtgcgga attgttcctc ttcttcacca ccatcctcca gaacttctcc   1380
gtggccagcc ccgtggctcc tgaagacatc gatctgacac cccaggagtg tggtgtgggc   1440
aaaatacccc caacatacca gatctgcttc ctgccccgct gaaggggctg agggaagggg   1500
gtcaaaggat tccagggtca ttcagtgtcc ccacctctgt agataatggc tctgactccc   1560
tgcaacttcc tgcctctgag agacctgctg caagccagct tccttccctt ccatggcacc   1620
agttgtctga ggtcgcagtg caaatgagtg gaggagtgag attattgaaa attataatat   1680
acaaaattat atatatatat tttgagacag agtctcactc agttgcccag gctggagtgc   1740
agtggcgtga tctcggctca ctgcaacctc cacccccggg gttcaagaaa ttctcctgcc   1800
tcagcctccc tagtagctgg gattacaggt gtgtgctacc atgcctggct aatttttgta   1860
tttttagtag agatggggtt tcaccgtgtt ggccaggctg atctcaaact cctgaactca   1920
agtgattcac ccaccttagc ctcccaaagt gctgggatta caggtgtgag tcaccatgcc   1980
cggccatgta tatatataat tttaaaaatt aagatgaaat tcacataaaa taaaattagc   2040
cattttaaag tgtacaattt agtggtgtgt ggttcattca caaagctgta caaccaccac   2100
catctagttc caaacatttt cttttttctt gagacggagt ctcactctgt cacccaggtt   2160
cgagttcagt ggtcttgaac tcctgatgtc aggtgattct cctagttcca aatgttttca   2220
ttatctctcc cccaacaaaa cccataccta tcaagctgtc actccccata ccccattctc   2280
tttttcatct cagcccctgt caatctggtt tttgtcctta tggacttacc aattctgaat   2340
atttcctata aacagaatca cacaatattt gatttttttt ttaaaactaa gccttgctct   2400
gtctcccagg ctggagtgct gtggcgtgat tttggttcac tgcaacctcc gccttccaag   2460
ttcaagagat tctcctgcct cagcttccaa gtagctggga ttacaggcat gtggtaccac   2520
gcctggctaa ttttcttgta tttttagtag ggacatgttg gccaggctgg ttgtgagctc   2580
ctggcctcag gtgatccaca cgcctcagtg tcccagagtg ctgatattac aggcgtaata   2640
tgtgatcttt tgtgtctggt tcctttcacg ttgaacgcta tttttgaggt tcgtgcctgt   2700
tgtagaccac agtcacacac tgctgtagtc ttccccccatc ctcattccca gctgcctcct   2760
cctactgttt ccctctatca aaaagcctcc ttggcgcagg ttccctgagc tgtgggattc   2820
tgcactggtg ctttggattc cctgatatgt tccttcaaat ccactgagaa ttaaataaac   2880
atcgctaaag cctgacctcc ccacgtc                                       2907
```

<210> 81
<211> 2398
<212> DNA
<213> Homo sapiens

<400> 81
cggaggggc tcagtccgca gccgccgccg ccaccgccgc gcctcggcct cggtgcaggc 60
agcggccgcc gccgccgaga cagctgcgcg ggcgagcatc cccacgcagc accttggaag 120
ttgttttcaa ccatatccag cctttgccga atacatccta tctgccacac atccagcgtg 180
aggtccctcc agctacaagg tgggcaccat ggcggagaag tttgactgcc actactgcag 240
ggatcccttg caggggaaga agtatgtgca aaaggatggc caccactgct gcctgaaatg 300
ctttgacaag ttctgtgcca acacctgtgt ggaatgccgc aagcccatcg gtgcggactc 360
caaggaggtg cactataaga accgcttctg gcatgacacc tgcttccgct gtgccaagtg 420
ccttcacccc ttggccaatg agacctttgt ggccaaggac aacaagatcc tgtgcaacaa 480
gtgcaccact cgggaggact cccccaagtg caaggggtgc ttcaaggcca ttgtggcagg 540
agatcaaaac gtggagtaca aggggaccgt ctggcacaaa gactgcttca cctgtagtaa 600
ctgcaagcaa gtcatcggga ctggaagctt cttccctaaa ggggaggact tctactgcgt 660
gacttgccat gagaccaagt ttgccaagca ttgcgtgaag tgcaacaagg ccatcacatc 720
tggaggaatc acttaccagg atcagccctg gcatgccgat tgctttgtgt gtgttacctg 780
ctctaagaag ctggctgggc agcgtttcac cgctgtggag gaccagtatt actgcgtgga 840
ttgctacaag aactttgtgg ccaagaagtg tgctggatgc aagaacccca tcactgggtt 900
tggtaaaggc tccagtgtgg tggcctatga aggacaatcc tggcacgact actgcttcca 960
ctgcaaaaaa tgctccgtga atctggccaa caagcgcttt gttttccacc aggagcaagt 1020
gtattgtccc gactgtgcca aaaagctgta aactgacagg ggctcctgtc ctgtaaaatg 1080
gcatttgaat ctcgttcttt gtgtccttac tttctgccct ataccatcaa taggggaaga 1140
gtggtccttc ccttctttaa agttctcctt ccgtcttttc tcccatttta cagtattact 1200
caaataaggg cacacagtga tcatattagc atttagcaaa aagcaaccct gcagcaaagt 1260
gaatttctgt ccggctgcaa tttaaaaatg aaaacttagg tagattgact cttctgcatg 1320
tttctcatag agcagaaaag tgctaatcat ttagccactt agtgatgtaa gcaagaagca 1380
taggagataa aacccccact gagatgcctc tcatgcctca gctgggaccc accgtgtaga 1440
cacacgacat gcaagagttg cagcggctgc tccaactcac tgctcaccct cttctgtgag 1500
caggaaaaga accctactga catgcatggt ttaacttcct catcagaact ctgcccttcc 1560
ttctgttctt ttgtgctttc aaataactaa cacgaacttc cagaaaatta acatttgaac 1620
ttagctgtaa ttctaaactg acctttcccc gtactaacgt ttggtttccc cgtgtggcat 1680
gttttctgag cgttcctact ttaaagcatg gaacatgcag gtgatttggg aagtgtagaa 1740
agacctgaga aaacgagcct gtttcagagg aacatcgtca caacgaatac ttctggaagc 1800
ttaacaaaac taaccctgct gtccttttta ttgtttttaa ttaatatttt tgttttaatt 1860
gatagcaaaa tagtttatgg gtttggaaac ttgcatgaaa atattttagc cccctcagat 1920
gttcctgcag tgctgaaatt catcctacgg aagtaaccgc aaaactctag aggggggagtt 1980
gagcaggcgc cagggctgtc atcaacatgg atatgacatt tcacaacagt gactagttga 2040
atcccttgta acgtagtagt tgtctgctct ttgtccatgt gttaatgagg actgcaaagt 2100
cccttctgtt gtgattccta ggacttttcc tcaagaggaa atctggattt ccacctaccg 2160
cttacctgaa atgcaggatc acctacttac tgtattctac attattatat gacatagtat 2220
aatgagacaa tatcaaaagt aaacatgtaa tgacaataca tactaacatt cttgtaggag 2280
tggttagaga agctgatgcc tcatttctac attctgtcat tagctattat catctaacgt 2340
ttcagtgtat ccttacagaa ataaagcagc atatgaaaaa aaaaaaaaaa aaaaaaa 2398

<210> 82
<211> 2197
<212> DNA
<213> Homo sapiens
<400> 82
gtcgccgctg ccgggttgcc agcggagtcg cgcgtcggga gctacgtagg gcagagaagt 60
catggcttct ccgtccaaag gcaatgactt gttttcgccc gacgaggagg gcccagcagt 120
ggtggccgga ccaggcccgg ggcctggggg cgccgagggg gccgcggagg agcgccgcgt 180
caaggtctcc agcctgccct tcagcgtgga ggcgctcatg tccgacaaga agccgcccaa 240
ggaggcgtcc ccgctgccgg ccgaaagcgc ctcggccggg gccaccctgc ggccactgct 300
gctgtcgggg cacggcgctc gggaagcgca cagcccccggg ccgctggtga agcccttcga 360
gaccgcctcg gtcaagtcgg aaaattcaga agatggagcg gcgtggatgc aggaacccgg 420
ccgatattcg ccgccgccaa gacatatgag ccctaccacc tgcaccctga ggaaacacaa 480
gaccaatcgg aagccgcgca cgccctttac cacatcccag ctcctcgccc tggagcgcaa 540
gttccgtcag aaacagtacc tctccattgc agagcgtgca gagttctcca gctctctgaa 600
cctcacagag acccaggtca aaatctggtt ccagaaccga agggccaagg cgaaaagact 660
gcaggaggca gaactggaaa agctgaaaat ggctgcaaaa cctatgctgc cctccagctt 720
cagtctccct ttccccatca gctcgcccct gcaggcagcg tccatatatg gagcatccta 780
cccgttccat agacctgtgc ttcccatccc gcctgtggga ctctatgcca cgccagtggg 840
atatggcatg taccacctgt cctaaggaag accagatcaa tagactccat gatggatgct 900
tgtttcaaag ggtttcctct ccctctccac gaaggcagta ccagccagta ctcctgctct 960
gctaaccctg cgtgcaccac cctaagcggc taggctgaca gggccacacg acatagctga 1020
aatttgttct gtaggcggag gcaccaagcc ctgttttctt ggtgtaatct tccagatgcc 1080
ccctttttcct ttcacaaaga ttggctctga tggtttttat gtataaatat atatatataa 1140
taaaatataa tacattttta tacagcagac gtaaaaattc aaattatttt aaaaggcaaa 1200
atttatatac atatgtgctt tttttctata tctcaccttc ccaaaagaca ctgtgtaagt 1260
ccatttgttg tattttctta aagagggaga caaattattt gcaaaatgtg ctaaagtcaa 1320

tgatttttac gggattattg acttctgctt atggaaaaca aagaaacaga cacaatgcac 1380
acagaaaata ttagatatgg agagattatt caaagtgaag gggacacatc atatttctgc 1440
attttacttg cattaaaaga aacctcttta tatactacag ttgttcctat ctctcccccg 1500
ccccccaccg ccccaccaca cacatatttt taaagttttt cctttttttaa gaatattttt 1560
gtaagaccaa tacctgggat gagaagaatc ctgagactgc ctggaggtga ggtagaaaat 1620
tagaaatact tcctaattct tctcaaggct gttggtaact ttatttcaga taattggaga 1680
gtaaaatgtt aaaacctgtt gagaggaatt gatggtttct gagaaatact aggtacattc 1740
atcctcacag attgcaaagg tgatttgggt ggggggtttag taattttctg cttaaaaaat 1800
gagtatcttg taaccattac ctatatgcta aatattcttg aacaattagt agatccagaa 1860
agaaaaaaaa atatgctttc tctgtgtgtg tacctgttgt atgtcctaaa cttattagaa 1920
aattttatat actttttttac atgttggggg gcagaaggta aagccatgtt ttgacttggt 1980
gaaaatggga ttgtcaaaca gcccattaag ttccctggta tttcaccttc ctgtccatct 2040
gtcccctccc tccggtatac ctttatccct ttgaaagggt gcttgtacaa tttgatatat 2100
tttattgaag agttatctct tattctgaat taaattaagc atttgtttta ttgcagtaaa 2160
gtttgtccaa actcacaatt aaaaaaaaaa aaaaaa 2197

<210> 83
<211> 2201
<212> DNA
<213> Homo sapiens
<400> 83
ggggtggga agagctgaag caggcgctct tggctcggcg cggcccgctg caatccgtgg 60
aggaacgcgc cgccgagcca ccatcatgcc tgggcactta caggaaggct tcggctgcgt 120
ggtcaccaac cgattcgacc agttatttga cgacgaatcg gacccccttcg aggtgctgaa 180
ggcagcagag aacaagaaaa aagaagccgg cgggggcggc gttgggggcc ctggggccaa 240
gagcgcagct caggccgcgg cccagaccaa ctccaacgcg gcaggcaaac agctgcgcaa 300
ggagtcccag aaagaccgca agaacccgct gcccccccagc gttggcgtgg ttgacaagaa 360
agaggagacg cagccgcccg tggcgcttaa gaaagaagga ataagacgag ttggaagaag 420
acctgatcaa caacttcagg gtgaagggaa aataattgat agaagaccag aaaggcgacc 480
acctcgtgaa cgaagattcg aaaagccact tgaagaaaag ggtgaaggag gcgaattttc 540
agttgataga ccgattattg accgacctat tcgaggtcgt ggtggtcttg gaagaggtcg 600
agggggccgt ggacgtggaa tgggccgagg agatggattt gattctcgtg gcaaacgtga 660
atttgatagg catagtggaa gtgatagatc tggcctgaag cacgaggaca aacgtggagg 720
tagcggatct cacaactggg gaactgtcaa agacgaatta actgacttgg atcaatcaaa 780
tgtgactgag gaaacacctg aaggtgaaga acatcatcca gtggcagaca ctgaaaataa 840
ggagaatgaa gttgaagagg taaaagagga gggtccaaaa gagatgactt tggatgagtg 900
gaaggctatt caaaataagg accgggcaaa agtagaattt aatatccgaa aaccaaatga 960
aggtgctgat gggcagtgga agaagggatt tgttcttcat aaatcaaaga gtgaagaggc 1020
tcatgctgaa gattcggtta tggaccatca tttccggaag ccagcaaatg atataacgtc 1080
tcagctggag atcaattttg gagaccttgg ccgcccagga cgtggcggca ggggaggacg 1140
aggtggacgt gggcgtggtg ggcgcccaaa ccgtggcagc aggaccgaca agtcaagtgc 1200
ttctgctcct gatgtggatg acccagaggc attcccagct ctggcttaac tggatgccat 1260
aagacaaccc tggttccttt gtgaaccctt ctgttcaaag cttttgcatg cttaaggatt 1320
ccaaacgact aagaaattaa aaaaaaaaag actgtcattc ataccattca cacctaaaga 1380
ctgaatttta tctgttttaa aaatgaactt ctcccgctac acagaagtaa caaatatggt 1440
agtcagtttt gtatttagaa atgtattggt agcagggatg ttttcataat tttcagagat 1500
tatgcattct tcatgaatac ttttgtattg ctgcttgcaa atatgcattt ccaaacttga 1560
aatataggtg tgaacagtgt gtaccagttt aaagctttca cttcatttgt gtttttttaat 1620
taaggactta gaagttcccc caattacaaa ctggttttaa atattggaca tactggtttt 1680
aatacctgct ttgcatattc acacatggtc aactgggaca tgttaaactt tgatttgtca 1740
aattttatgc tgtgtggaat actaactata tgtattttaa cttagtttta atattttcat 1800
tttgggggaa aaatcttttt tcacttctca tgatagctgt tatatatata tgctaaatct 1860
ttatatacag aaatatcagt acttgaacaa attcaaagca catttggttt attaaccctt 1920
gctccttgca tggctcatta ggttcaaatt ataactaatt tacattttca gctatattta 1980
ctttttaaat gcttgagttt cccattttaa aatctaaact agacatctta attggtgaaa 2040
gttgtttaaa ctacttattg ttggtaggca catcgtgtca agtgaagtag ttttataggt 2100
atgggttttt tctcccccctt caccagggtg ggtggaataa gttgatttgg ccaatgtgta 2160
atatttaaac tgttctgtaa aataaaaaaa aaaaaaaaaa a 2201

<210> 84
<211> 1233
<212> DNA
<213> Homo sapiens
<400> 84
gtttgcttca ccttctgcca ggattgtaag tttcctgagg cctcccccagt cctgcggaac 60
tggctccggc tggcacctga ggagcggcgt gacccccgagg gcccagggag ctgcccggct 120
ggcctaggca ggcagccgca ccatggccag cacggccgtg cagcttctgg gcttcctgct 180
cagcttcctg ggcatggtgg gcacgttgat caccaccatc ctgccgcact ggcggaggac 240
agcgcacgtg ggcaccaaca tcctcacggc cgtgtcctac ctgaaagggc tctggatgga 300

gtgtgtgtgg cacagcacag gcatctacca gtgccagatc taccgatccc tgctggcgct 360
gccccaagac ctccaggctg cccgcgccct catggtcatc tcctgcctgc tctcgggcat 420
agcctgcgcc tgcgccgtca tcgggatgaa gtgcacgcgc tgcgccaagg gcacacccgc 480
caagaccacc tttgccatcc tcggcggcac cctcttcatc ctggccggcc tcctgtgcat 540
ggtggccgtc tcctggacca ccaacgacgt ggtgcagaac ttctacaacc cgctgctgcc 600
cagcggcatg aagtttgaga ttggccaggc cctgtacctg ggcttcatct cctcgtccct 660
ctcgctcatt ggtggcaccc tgctttgcct gtcctgccag gacgaggcac cctacaggcc 720
ctaccaggcc ccgcccaggg ccaccacgac cactgcaaac accgcacctg cctaccagcc 780
accagctgcc tacaaagaca atcgggcccc ctcagtgacc tcggccacgc acagcgggta 840
caggctgaac gactacgtgt gagtccccac agcctgcttc tcccctgggc tgctgtgggc 900
tgggtccccg gcgggactgt caatggaggc aggggttcca gcacaaagtt tacttctggg 960
caatttttgt atccaaggaa ataatgtgaa tgcgaggaaa tgtctttaga gcacagggac 1020
agaggggggaa ataagaggag gagaaagctc tctataccaa agactgaaaa aaaaaatcct 1080
gtctgttttt gtatttatta tatatattta tgtgggtgat ttgataacaa gtttaatata 1140
aagtgacttg ggagtttggt cagtggggtt ggtttgtgat ccaggaataa accttgcgga 1200
tgtggctgtt tatgaaaaaa aaaaaaaaaa aaa 1233

<210> 85
<211> 3139
<212> DNA
<213> Homo sapiens
<400> 85
ggccgaggag gaagtggcgg cggcggcggc gggactgcgc gccccagctc cgatccccgt 60
tccgcgtccc cgccgccggg aggaggtgcc cactcgctcg cggcgcgcgc cggccgccag 120
actcggcctg tgggcgattt cctccggacc caggctcccc gcccgaggag gaagatgcag 180
acctttctga aagggaagag agttggctac tggctgagcg agaagaaaat caagaagctg 240
aatttccagg ccttcgccga gctgtgcagg aagcgaggga tggaggttgt gcagctgaac 300
cttagccggc cgatcgagga gcagggcccc ctggacgtca tcatccacaa gctgactgac 360
gtcatccttg aagccgacca gaatgatagc cagtccctgg agctggtgca caggttccag 420
gagtacatcg atgcccaccc tgagaccatc gtcctggacc cgctccctgc catcagaacc 480
ctgcttgacc gctccaagtc ctatgagctc atccggaaga ttgaggccta catggaagac 540
gacaggatct gctcgccacc cttcatggag ctcacgagcc tgtgcgggga tgacaccatg 600
cggctgctgg agaagaacgg cttgactttc ccattcattt gcaaaaccag agtggctcat 660
ggcaccaact ctcacgagat ggctatcgtg ttcaaccagg agggcctgaa cgccatccag 720
ccaccctgcg tggtccagaa tttcatcaac cacaacgccg tcctgtacaa ggtgttcgtg 780
gttggcgagt cctacaccgt ggtccagagg ccctcactca agaacttctc cgcaggcaca 840
tcagaccgtg agtccatctt cttcaacagc cacaacgtgt caaagccgga gtcgtcatcg 900
gtcctgacgg agctggacaa gatcgagggc gtgttcgagc ggccgagcga cgaggtcatc 960
cgggagctct cccgggccct gcggcaggca ctgggcgtgt cactcttcgg catcgacatc 1020
atcatcaaca accagacagg gcagcacgcc gtcattgaca tcaatgcctt cccaggctac 1080
gagggcgtga gcgagttctt cacagacctc ctgaaccaca tcgccactgt cctgcagggc 1140
cagagcacag ccatggcagc cacaggggac gtggccctgc tgaggcacag caagcttctg 1200
gccgagccgg cgggcggcct ggtgggcgag cggacatgca gcgccagccc cggctgctgc 1260
ggcagcatga tgggccagga cgcgccctgg aaggctgagg ccgacgcggg cggcaccgcc 1320
aagctgccgc accagagact cggctgcaac gccggcgtgt cgcccagctt ccagcagcat 1380
tgtgtggcct ccctggccac caaggcctcc tcccagtagc cacggagccg ggacccagag 1440
gggcagcgca gggcgcagag cacacccgct gggccagcag ctcccaacgg cgatgctact 1500
actaagaatc cccagtgatc tgattcttct gtttttttaat ttttaacctg attctctgat 1560
gtcatgatct aaatgagggg tagtggggga tggaagagag taccaggtgg tccaccgttg 1620
gggagtgggg ccgtccgcct gctctctact gtgcagacct cctaactgag tttacacacg 1680
cttgtgtttg caacactagg tctggatggg aggtgagggg tgtgcgtatg gctgccatgc 1740
cggtgtctgt gcacatccct gtctgttggt ctccatggcc actgtggacc gggacccttg 1800
ggggaagcct gcccatgtcg ggctgtggga ggctgatcgg tgcatgtgag agtggcttcc 1860
cttctgcctg actccccact ccctgacctg ccccttcctt gtttttcctc ctactggtct 1920
ccaccaaggc tttgttagcc cccaccctgc ctggtgtgca gctaacccct ccctccccac 1980
agccagagga ggccacagac ccctcagggg agttccgcgc tggtgtctgg gctgtgctcc 2040
ctcactaaag ggaaggaaag gaagctgggc gtcctctggg ccccccaaca cacgtcccat 2100
ttagccctgc acagcggtct ccttccccta agccagcact gctgctccct ggagcccggg 2160
aaggaggctg cctggctgga ggcccgagcc gatggcgcct gtgctgagga tttgtgctgt 2220
gatttgggca aatcattcca ggtctttggg cctccacccc cttgtctcta gtggacattt 2280
gagatcagag agcaccacag ggctggcttt gtgccctaac ccctgggatg cagcctgcct 2340
ttccataaag tcacctaggt gaggataggc gcgggagcct cggcatgaca ccatggagat 2400
cggggccctc ttcccagtgg gttcactcct tttcacacct gctgggtccc tcctcaccca 2460
gcaggcctgg tccacctctc attgcaagcc ccaagcactg agccagcagg tgccagggag 2520
ccacccgccc cccatcgctt ctgcacacct cagactcacc ccatcacctt ggcagcaaag 2580
cactggctct gccgtctgac ccctgatcca ggcagccccc tctgcagaga aaagggttgg 2640
ggagaagcct ctgcagtcct ggaagatgtg gggtgctggg tgagaggcat cagcccccac 2700
aagtatgttt ttgtgtctta agatagcagt ttactttgaa aaagtgaaaa aggcttccgg 2760
gctgtcctct gcccagtgag atggaggacg ctagagaaag tgctgagtgt cccgagagag 2820

gcccccgagc cagtgcatgg aggtccttcg gcctggctca gctgggctgc aggatgccca 2880
ctttgaggag ggaggcacag ggcttgggcg aggggcagag gccatcagaa ctgcccggct 2940
tttttggaaa ctgaggaccc aacaactaac cacgtttaca cgacttgagt tttgaacccc 3000
gattaatgtc tgtacgtcac ctttcctagt tctgaccctg agccctgggg aacaggaaag 3060
cgtggctggc ctcttgcact gctttgtctc caaaataaac tactgaaatc aaaccgcaaa 3120
aaaaaaaaaa aaaaaaaaa 3139

<210> 86
<211> 4530
<212> DNA
<213> Homo sapiens
<400> 86
aattctcgag ctcgtcgacc ggtcgacgag ctcgagggtc gacgagctcg agggcgcgcg 60
cccggccccc acccctcgca gcaccccgcg ccccgcgccc tcccagccgg gtccagccgg 120
agccatgggg ccggagccgc agtgagcacc atggagctgg cggccttgtg ccgctggggg 180
ctcctcctcg ccctcttgcc ccccggagcc gcgagcaccc aagtgtgcac cggcacagac 240
atgaagctgc ggctccctgc cagtcccgag acccacctgg acatgctccg ccacctctac 300
cagggctgcc aggtggtgca gggaaacctg gaactcacct acctgcccac caatgccagc 360
ctgtccttcc tgcaggatat ccaggaggtg cagggctacg tgctcatcgc tcacaaccaa 420
gtgaggcagg tcccactgca gaggctgcgg attgtgcgag gcacccagct ctttgaggac 480
aactatgccc tggccgtgct agacaatgga gacccgctga acaataccac ccctgtcaca 540
ggggcctccc caggaggcct gcgggagctg cagcttcgaa gcctcacaga gatcttgaaa 600
ggaggggtct tgatccagcg gaacccccag ctctgctacc aggacacgat tttgtggaag 660
gacatcttcc acaagaacaa ccagctggct ctcacactga tagacaccaa ccgctctcgg 720
gcctgccacc cctgttctcc gatgtgtaag ggctcccgct gctggggaga gagttctgag 780
gattgtcaga gcctgacgcg cactgtctgt gccggtggct gtgcccgctg caaggggcca 840
ctgcccactg actgctgcca tgagcagtgt gctgccggct gcacgggccc caagcactct 900
gactgcctgg cctgcctcca cttcaaccac agtggcatct gtgagctgca ctgcccagcc 960
ctggtcacct acaacacaga cacgtttgag tccatgccca atcccgaggg ccggtataca 1020
ttcggcgcca gctgtgtgac tgcctgtccc tacaactacc tttctacgga cgtgggatcc 1080
tgcaccctcg tctgccccct gcacaaccaa gaggtgacag cagaggatgg aacacagcgg 1140
tgtgagaagt gcagcaagcc ctgtgcccga gtgtgctatg gtctgggcat ggagcacttg 1200
cgagaggtga gggcagttac cagtgccaat atccaggagt ttgctggctg caagaagatc 1260
tttgggagcc tggcatttct gccggagagc tttgatgggg acccagcctc caacactgcc 1320
ccgctccagc cagagcagct ccaagtgttt gagactctgg aagagatcac aggttaccta 1380
tacatctcag catggccgga cagcctgcct gacctcagcg tcttccagaa cctgcaagta 1440
atccggggac gaattctgca caatggcgcc tactcgctga ccctgcaagg gctgggcatc 1500
agctggctgg ggctgcgctc actgagggaa ctgggcagtg gactggccct catccaccat 1560
aacacccacc tctgcttcgt gcacacggtg ccctgggacc agctctttcg gaacccgcac 1620
caagctctgc tccacactgc caaccggcca gaggacgagt gtgtgggcga gggcctggcc 1680
tgccaccagc tgtgcgcccg agggcactgc tggggtccag ggcccaccca gtgtgtcaac 1740
tgcagccagt tccttcgggg ccaggagtgc gtggaggaat gccgagtact gcaggggctc 1800
cccagggagt atgtgaatgc caggcactgt ttgccgtgcc accctgagtg tcagccccag 1860
aatggctcag tgacctgttt tggaccggag gctgaccagt gtgtggcctg tgcccactat 1920
aaggaccctc ccttctgcgt ggcccgctgc cccagcggtg tgaaacctga cctctcctac 1980
atgcccatct ggaagtttcc agatgaggag ggcgcatgcc agccttgccc catcaactgc 2040
acccactcct gtgtggacct ggatgacaag ggctgccccg ccgagcagag agccagccct 2100
ctgacgtcca tcgtctctgc ggtggttggc attctgctgg tcgtggtctt gggggtggtc 2160
tttgggatcc tcatcaagcg acggcagcag aagatccgga agtacacgat gcggagactg 2220
ctgcaggaaa cggagctggt ggagccgctg acacctagcg gagcgatgcc caaccaggcg 2280
cagatgcgga tcctgaaaga gacggagctg aggaaggtga aggtgcttgg atctggcgct 2340
tttggcacag tctacaaggg catctggatc cctgatgggg agaatgtgaa aattccagtg 2400
gccatcaaag tgttgaggga aaacacatcc cccaaagcca acaaagaaat cttagacgaa 2460
gcatacgtga tggctggtgt gggctcccca tatgtctccc gccttctggg catctgcctg 2520
acatccacgg tgcagctggt gacacagctt atgccctatg gctgcctctt agaccatgtc 2580
cgggaaaacc gcggacgcct gggctcccag gacctgctga actggtgtat gcagattgcc 2640
aaggggatga gctacctgga ggatgtgcgg ctcgtacaca gggacttggc cgctcggaac 2700
gtgctggtca agagtcccaa ccatgtcaaa attacagact tcgggctggc tcggctgctg 2760
gacattgacg agacagagta ccatgcagat gggggcaagg tgcccatcaa gtggatggcg 2820
ctggagtcca ttctccgccg gcggttcacc caccagagtg atgtgtggag ttatggtgtg 2880
actgtgtggg agctgatgac ttttggggcc aaaccttacg atgggatccc agcccgggag 2940
atccctgacc tgctggaaaa gggggagcgg ctgccccagc cccccatctg caccattgat 3000
gtctacatga tcatggtcaa atgttggatg attgactctg aatgtcggcc aagattccgg 3060
gagttggtgt ctgaattctc ccgcatggcc agggaccccc agcgctttgt ggtcatccag 3120
aatgaggact tgggcccagc cagtcccttg gacagcacct tctaccgctc actgctggag 3180
gacgatgaca tgggggacct ggtggatgct gaggagtatc tggtacccca gcagggcttc 3240
ttctgtccag accctgcccc gggcgctggg ggcatggtcc accacaggca ccgcagctca 3300
tctaccagga gtggcggtgg ggacctgaca ctagggctgg agccctctga agaggaggcc 3360
cccaggtctc cactggcacc ctccgaaggg gctggctccg atgtatttga tggtgacctg 3420

ggaatggggg cagccaaggg gctgcaaagc ctccccacac atgaccccag ccctctacag 3480
cggtacagtg aggaccccac agtacccctg ccctctgaga ctgatggcta cgttgccccc 3540
ctgacctgca gcccccagcc tgaatatgtg aaccagccag atgttcggcc ccagccccct 3600
tcgccccgag agggccctct gcctgctgcc cgacctgctg gtgccactct ggaaagggcc 3660
aagactctct ccccagggaa gaatggggtc gtcaaagacg tttttgcctt tgggggtgcc 3720
gtggagaacc ccgagtactt gacaccccag ggaggagctg cccctcagcc ccaccctcct 3780
cctgccttca gcccagcctt cgacaacctc tattactggg accaggaccc accagagcgg 3840
ggggctccac ccagcacctt caaagggaca cctacggcag agaacccaga gtacctgggt 3900
ctggacgtgc cagtgtgaac cagaaggcca agtccgcaga agccctgatg tgtcctcagg 3960
gagcagggaa ggcctgactt ctgctggcat caagaggtgg gagggccctc cgaccacttc 4020
caggggaacc tgccatgcca ggaacctgtc ctaaggaacc ttccttcctg cttgagttcc 4080
cagatggctg gaaggggtcc agcctcgttg gaagaggaac agcactgggg agtctttgtg 4140
gattctgagg ccctgcccaa tgagactcta gggtccagtg gatgccacag cccagcttgg 4200
ccctttcctt ccagatcctg ggtactgaaa gccttaggga agctggcctg agaggggaag 4260
cggccctaag ggagtgtcta agaacaaaag cgacccattc agagactgtc cctgaaacct 4320
agtactgccc cccatgagga aggaacagca atggtgtcag tatccaggct ttgtacagag 4380
tgcttttctg tttagttttt actttttttg ttttgttttt ttaaagacga aataaagacc 4440
caggggagaa tgggtgttgt atggggaggc aagtgtgggg ggtccttctc cacacccact 4500
ttgtccattt gcaaatatat tttggaaaac 4530

<210> 87
<211> 2629
<212> DNA
<213> Homo sapiens
<400> 87
acttgtcatg gcgactgtcc agctttgtgc caggagcctc gcaggggttg atgggattgg 60
ggttttcccc tcccatgtgc tcaagactgg cgctaaaagt tttgagcttc tcaaaagtct 120
agagccaccg tccagggagc aggtagctgc tgggctccgg ggacactttg cgttcgggct 180
gggagcgtgc tttccacgac ggtgacacgc ttccctggat tggcagccag actgccttcc 240
gggtcactgc catggaggag ccgcagtcag atcctagcgt cgagcccccct ctgagtcagg 300
aaacattttc agacctatgg aaactacttc ctgaaaacaa cgttctgtcc ccettgccgt 360
cccaagcaat ggatgatttg atgctgtccc cggacgatat tgaacaatgg ttcactgaag 420
acccaggtcc agatgaagct cccagaatgc cagaggctgc tccccgcgtg gcccctgcac 480
cagcagctcc tacaccggcg gcccctgcac cagccccctc ctggcccctg tcatcttctg 540
tcccttccca gaaaacctac cagggcagct acggtttccg tctgggcttc ttgcattctg 600
ggacagccaa gtctgtgact tgcacgtact cccctgccct caacaagatg ttttgccaac 660
tggccaagac ctgccctgtg cagctgtggg ttgattccac acccccgccc ggcacccgcg 720
tccgcgccat ggccatctac aagcagtcac agcacatgac ggaggttgtg aggcgctgcc 780
cccaccatga gcgctgctca gatagcgatg gtctggcccc tcctcagcat cttatccgag 840
tggaaggaaa tttgcgtgtg gagtatttgg atgacagaaa cacttttcga catagtgtgg 900
tggtgcccta tgagccgcct gaggttggct ctgactgtac caccatccac tacaactaca 960
tgtgtaacag ttcctgcatg ggcggcatga accggaggcc catcctcacc atcatcacac 1020
tggaagactc cagtggtaat ctactgggac ggaacagctt tgaggtgcgt gtttgtgcct 1080
gtcctgggag agaccggcgc acagaggaag agaatctccg caagaaaggg gagcctcacc 1140
acgagctgcc cccagggagc actaagcgag cactgcccaa caacaccagc tcctctcccc 1200
agccaaagaa gaaaccactg gatggagaat atttcaccct tcagatccgt gggcgtgagc 1260
gcttcgagat gttccgagag ctgaatgagg ccttggaact caaggatgcc caggctggga 1320
aggagccagg ggggagcagg gctcactcca gccacctgaa gtccaaaaag ggtcagtcta 1380
cctcccgcca taaaaaactc atgttcaaga cagaagggcc tgactcagac tgacattctc 1440
cacttcttgt tccccactga cagcctccca cccccatctc tccctcccct gccattttgg 1500
gttttgggtc tttgaaccct tgcttgcaat aggtgtgcgt cagaagcacc caggacttcc 1560
atttgctttg tcccggggct ccactgaaca agttggcctg cactggtgtt ttgttgtggg 1620
gaggaggatg gggagtagga cataccagct tagattttaa ggtttttact gtgagggatg 1680
tttgggagat gtaagaaatg ttcttgcagt taagggttag tttacaatca gccacattct 1740
aggtaggtag gggcccactt caccgtacta accagggaag ctgtccctca tgttgaattt 1800
tctctaactt caaggcccat atctgtgaaa tgctggcatt tgcacctacc tcacagagtg 1860
cattgtgagg gttaatgaaa taatgtacat ctggccttga aaccaccttt tattacatgg 1920
ggtctaaaac ttgacccccct tgagggtgcc tgttccctct ccctctccct gttggctggt 1980
gggttggtag tttctacagt tgggcagctg gttaggtaga gggagttgtc aagtcttgct 2040
ggcccagcca aaccctgtct gacaacctct tggtcgacct tagtacctaa aaggaaatct 2100
caccccatcc cacaccctgg aggatttcat ctcttgtata tgatgatctg gatccaccaa 2160
gacttgtttt atgctcaggg tcaatttctt ttttcttttt tttttttttt tttctttttc 2220
tttgagactg ggtctcgctt tgttgcccag gctggagtgg agtggcgtga tcttggctta 2280
ctgcagcctt tgcctccccg gctcgagcag tcctgcctca gcctccggag tagctgggac 2340
cacaggttca tgccaccatg gccagccaac ttttgcatgt tttgtagaga tggggtctca 2400
cagtgttgcc caggctggtc tcaaactcct gggctcaggc gatccacctg tctcagcctc 2460
ccagagtgct gggattacaa ttgtgagcca ccacgtggag ctggaagggt caacatcttt 2520
tacattctgc aagcacatct gcattttcac cccacccttc ccctccttct ccctttttat 2580
atcccatttt tatatcgatc tcttatttta caataaaact ttgctgcca 2629

<210> 88
<211> 2496
<212> DNA
<213> Homo sapiens
<400> 88

```
ctctcttgcc tgccgccatg tgcctttcgc cttctgccgt gattgtgagg cctccccagc      60
catatggaag ttcaggatgt ctgcccgtgg cccggctatc ggcatcgacc tgggcaccac     120
ctattcgtgc gtcggggtct tccaacatgg caaggtggag atcatcgcca acgaccaggg     180
caatcgcacc acccccagct acgtggcctt cacggacacc gagcgcctca tcggcgacgc     240
cgccaagaac caggtggcca tgaaccccac caacaccatc ttcgacgcca agaggctgat     300
tggacggaaa ttcgaggatg ccacagtgca gtcggatatg aaacactggc cgttccgggt     360
ggtgagcgag ggaggcaagc ccaaagtgca agtagagtac aagggggaga ccaagacctt     420
cttcccagag gagatatcct ccatggtcct cacgaagatg aaggagatcg cggaagccta     480
cctgggggc aaggtgcaca gcgcggtcat aacggtcccg gcctatttca acgactcgca      540
gcgccaggcc accaaggacg caggcaccat cacggggctc aatgtgctgc gcatcatcaa     600
cgagcccacg gcggcggcca tcgcctacgg cctggacaag aagggctgcg cgggcggcga     660
gaagaacgtg ctcatctttg acctgggcgg tggcactttc gacgtgtcca tcctgaccat     720
cgaggatggc atcttcgagg tgaagtccac ggccggcgac acccacctgg gcggtgagga     780
cttcgacaac cgcatggtga gccacctggc ggaggagttc aagcgcaagc acaagaagga     840
cattgggccc aacaagcgcg ccgtgaggcg gctgcgcacc gcttgcgagc gcgccaagcg     900
caccctgagc tcgtccacgc aggcgagcat cgagatcgac tcgctctacg agggcgtgga     960
cttctatacg tccatcacgc gcgcccgctt cgaggagctc aatgccgacc tctttcgcgg    1020
gaccctggag ccggtggaga aggcgctgcg cgacgccaag ctggacaagg gccagatcca    1080
ggagatcgtg ctggtgggcg gctccactcg tatccccaag atccagaagc tgctgcagga    1140
tttcttcaac ggcaaggagc tgaacaagag catcaacccc gacgaggcgg tggcctatgg    1200
cgccgcggtg caggcggcca tcctcatcgg cgacaaatca gagaatgtgc aggacctgct    1260
gctactcgac gtgaccccgt tgtcgctggg catcgagaca gctggcggtg tcatgacccc    1320
actcatcaag aggaacacca cgatccccac caagcagacg cagaccttca ccacctactc    1380
ggacaaccag agcagcgtac tggtgcaggt atacgagggc gaacgggcca tgaccaagga    1440
caataacctg ctgggcaagt tcgacctgac cgggattccc cctgcgcctc gcggggtccc    1500
ccaaatcgag gttaccttcg acattgacgc caatggcatc cttaacgtta ccgccgccga    1560
caagagcacc ggtaaggaaa acaaaatcac catcaccaat gacaaaggtc gtctgagcaa    1620
ggacgacatt gaccggatgg tgcaggaggc ggagcggtac aaatcggaag atgaggcgaa    1680
tcgcgaccga gtcgcggcca aaaacgccct ggagtcctat acctacaaca tcaagcagac    1740
ggtggaagac gagaaactga ggggcaagat tagcgagcag gacaaaaaca agatcctcga    1800
caagtgtcag gaggtgatca actggctcga ccgaaaccag atggcagaga aagatgagta    1860
tgaacacaag cagaaagagc tcgaaagagt ttgcaacccc atcatcagca aactttacca    1920
aggtggtcct ggcggcggca gcggcggcgg cggttcagga gcctccgggg gacccaccat    1980
cgaagaagtg gactaagctt gcactcaagt cagcgtaaac ctctttgcct ttctctctct    2040
ctcttttttt ttgtttgttt ctttgaaatg tccttgtgcc aagtacgaga tctattgttg    2100
gaagtctttg gtatatgcaa atgaaaggag aggtgcaaca acttagttta attataaaag    2160
ttccaaagtt tgttttttaa aaacattatt cgaggtttct ctttaatgca ttttgcgtgt    2220
ttgctgactt gagcattttt gattagttcg tgcatggaga tttgtttgag atgagaaacc    2280
ttaagtttgc acacctgttc tgtagaagct tggaaacagt aaaatatata ggagcttaaa    2340
ttgtttattt ttatgtacta ctttaaaact aaactgaaca ttgcagtaat gttaaggaca    2400
ggtatacttt ttgcaaacaa atgcataaat gcaaatgtaa agtaaagctg aaattgatct    2460
caaaaaaaaa aaaaaaaaaa aaaaaaaaaa aaaaaa                              2496
```

<210> 89
<211> 5287
<212> DNA
<213> Homo sapiens
<400> 89

```
ctggccgcgg cgtggggaca gcgaggcgca ctggggcctc ccagcgccgg gccggccgcg      60
ccgtccagcc cgaggtctac ggctttgcgc tccgagccca gaggaagatg cctgccggca     120
ccgagctcgg gctgcggggc tgaacgcctg tcttccaggc gcacggcagc actgccctcg     180
gccggtgtcg gtagcggcac tcggcgtgcc ccgggcggac gaagagcgca ggctgggtac     240
accttgcccg aatcggcgga gttcgcagct agcgagggcg ggccggccgg cccggatggg     300
cgcggggttt gcggccccg ccgggtgctc cggagcggcc cgggcaccgg gggcacgctg      360
agtgccggag ccgcggccgc agagagaact tggggcgggg gccatgcacc ggtgcggagt     420
ctagagccga gcggagcgcc ccgcgggccc gccccgcgtct gcggccgttc gggtcctcac    480
agtccccgcg cccggagttc gcagcgcgct ccagacaaga tggcgcggcc ggtccgggga     540
gggctcgggg ccccgcgccg ctcgccttgc cttctccttc tctggctgct tttgcttcgg     600
ctggagccgg tgaccgccgc ggccggcccg cgggcgccct gcgcggccgc ctgcacttgc     660
gctggggact cgctggactg cggtgggcgc gggctggctg cgttgcccgg ggacctgccc     720
tcctggacgc ggagcctaaa cctgagttac aacaaactct ctgagattga ccctgctggt     780
tttgaggact tgccgaacct acaggaagtg tacctcaata ataatgagtt gacagcggta     840
ccatccctgg gcgctgcttc atcacatgtc gtctctctct ttctgcagca caacaagatt     900
```

cgcagcgtgg aggggagcca gctgaaggcc tacctttcct tagaagtgtt agatctgagt 960
ttgaacaaca tcacggaagt gcggaacacc tgctttccac acggaccgcc tataaaggag 1020
ctcaacctgg caggcaatcg gattggcacc ctggagttgg gagcatttga tggtctgtca 1080
cggtcgctgc taactcttcg cctgagcaaa aacaggatca cccagcttcc tgtaagagca 1140
ttcaagctac ccaggctgac acaactggac ctcaatcgga acaggattcg gctgatagag 1200
ggcctcacct tccaggggct caacagcttg gaggtgctga agcttcagcg aaacaacatc 1260
agcaaactga cagatggggc cttctgggga ctgtccaaga tgcatgtgct gcacctggag 1320
tacaacagcc tggtagaagt gaacagcggc tcgctctacg gcctcacggc cctgcatcag 1380
ctccacctca gcaacaattc catcgctcgc attcaccgca agggctggag cttctgccag 1440
aagctgcatg agttggtcct gtccttcaac aacctgacac ggctggacga ggagagcctg 1500
gccgagctga gcagcctgag tgtcctgcgt ctcagccaca attccatcag ccacattgcg 1560
gagggtgcct tcaagggact caggagcctg cgagtcttgg atctggacca taacgagatt 1620
tcgggcacaa tagaggacac gagcggcgcc ttctcagggc tcgacagcct cagcaagctg 1680
actctgtttg gaaacaagat caagtctgtg gctaagagag cattctcggg gctggaaggc 1740
ctggagcacc tgaaccttgg agggaatgcg atcagatctg tccagtttga tgcctttgtg 1800
aagatgaaga atcttaaaga gctccatatc agcagcgaca gcttcctgtg tgactgccag 1860
ctgaagtggc tgcccccgtg gctaattggc aggatgctgc aggcctttgt gacagccacc 1920
tgtgcccacc cagaatcact gaagggtcag agcattttct ctgtgccacc agagagtttc 1980
gtgtgcgatg acttcctgaa gccacagatc atcacccagc cagaaaccac catggctatg 2040
gtgggcaagg acatccggtt tacatgctca gcagccagca gcagcagctc ccccatgacc 2100
tttgcctgga agaaagacaa tgaagtcctg accaatgcag acatggagaa ctttgtccac 2160
gtccacgcgc aggacgggga agtgatggag tacaccacca tcctgcacct ccgtcaggtc 2220
actttcgggc acgagggccg ctaccaatgt gtcatcacca accactttgg ctccacctat 2280
tcacataagg ccaggctcac tgtgaatgtg ttgccatcat tcaccaaaac gccccacgac 2340
ataaccatcc ggaccaccac catggcccgc ctcgaatgtg ctgccacagg tcacccaaac 2400
cctcagattg cctggcagaa ggatggaggc acggatttcc ccgctgcccg tgagcgacgc 2460
atgcatgtca tgccggatga cgacgtgttt ttcatcactg atgtgaaaat agatgacgca 2520
ggggtttaca gctgtactgc tcagaactca gccggttcta tttcagctaa tgccaccctg 2580
actgtcctag agaccccatc cttggtggtc cccttggaag accgtgtggt atctgtggga 2640
gaaacagtgg ccctccaatg caaagccacg gggaaccctc cgccccgcat cacctggttc 2700
aagggggacc gcccgctgag cctcactgag cggcaccacc tgacccctga caaccagctc 2760
ctggtggttc agaacgtggt ggcagaggat gcgggccgat atacctgtga gatgtccaac 2820
accctgggca cggagcgagc tcacagccag ctgagcgtcc tgcccgcagc aggctgcagg 2880
aaggatggga ccacggtagg catcttcacc attgctgtcg tgagcagcat cgtcctgacg 2940
tcactggtct gggtgtgcat catctaccag accaggaaga agagtgaaga gtacagtgtc 3000
accaacacag atgaaaccgt cgtgccacca gatgttccaa gctacctctc ttctcagggg 3060
acccttctg accgacaaga aaccgtggtc aggaccgagg gtgcgggccc tcaggccaat 3120
gggcacattg agagcaatgg tgtgtgtcca agagatgcaa gccactttcc agagcccgac 3180
actcacagcg ttgcctgcag gcagccaaag ctctgtgctg ggtctgcgta tcacaaagag 3240
ccgtggaaag cgattgagaa agctgaaggg acacctgggc cacataagat ggaacacggt 3300
ggccgggtcg tatgcagtga ctgcaacacc gaagtggact gttactccag ggacaagcc 3360
ttccaccccc agcctgtgtc cagagacagc gcacagccaa gtgcgccaaa tggcccggag 3420
ccgggtggga gtgaccaaga gcattctcca catcaccagt gcagcaggac tgccgctggg 3480
tcctgccccg agtgccaagg gtcgctctac cccagtaacc acgatagaat gctgacggct 3540
gtgaagaaaa agccaatggc atctctagat gggaaagggg attcttcctg gactttagca 3600
aggttgtatc acccggactc tacagagcta cagcctgcat cttcattaac ttcaggcagt 3660
ccagagcgcg cggaagccca gtacttgctt gtttccaatg gccacctccc caaagcatgt 3720
gacgccagtc ccgagtccac gccactgaca ggacagctcc ccgggaaaca gagggtgcca 3780
ctgctgttgg caccaaaaag ctaggttttg tctacctcag ttcttgtcat accaatctct 3840
acgggaaaga gaggtaggag aggctgcgag gaagcttggg ttcaagcgtc actcatctgt 3900
acatagttgt aactcccatg tggagtatca gtcgctcaca ggacttggat ctgaagcaca 3960
gtaaacgcaa gaggggattt gtgtacaaaa ggcaaaaaaa gtatttgata tcattgtaca 4020
taagagtttt cagagatttc atatatatct tttacagagg ctattttaat ctttagtgca 4080
tggttaacag aaaaaaatta tacaattttg acaatattat ttttcgtatc aggttgctgt 4140
ttaattttgg agggggtggg gaaatagttc tggtgcctta acgcatggct ggaatttata 4200
gaggctacaa ccacatttgt tcacaggagt tttggtgcg ggtggggaag gatggaaggc 4260
cttggattta tattgcactt catagacccc taggctgctg tgcggtggga ctccacatgc 4320
gccggaagga gcttcaggtg agcactgctc atgtgtggat gcccctgcaa caggcttccc 4380
tgtctgtaga gccaggggtg caagtgccat ccacacttgc agtgaatggc ttttcctttt 4440
aggtttaagt cctgtctgtc tgtaaggcgt agaatctgtc cgtctgtaag gcgtagaatg 4500
agggttgtta atccatcaca agcaaaaggt cagaacagtt aaacactgcc tttcctcctc 4560
ctcttatttt atgataaaag caaatgtggc cttctcagta tcattcgatt gctatttgag 4620
acttttaaat taaggtaaag gctgctggtg ttggtacctg tggatttttc tatactgatg 4680
ttttcgtttt gccaatataa tgagtattac attggccttg ggggacagaa aggaggaagt 4740
tctgactttt cagggctacc ttatttctac taaggaccca gagcaggcct gtccatgcca 4800
ttccttcgca cagatgaaac tgagctggga ctggaaagga cagcccttga cctgggttct 4860
gggtataatt tgcacttttg agactggtag ctaaccatct tatgagtgcc aatgtgtcat 4920
ttagtaaaac ttaaatagaa acaaggtcct tcaaatgttc ctttggccaa aagctgaagg 4980
gagttactga gaaaatagtt aacaattact gtcaggtgtc atcactgttc aaaaggtaag 5040

cacatttaga attttgttct tgacagttaa ctgactaatc ttacttccac aaaatatgtg 5100
aatttgctgc ttctgagagg caatgtgaaa gagggagtat tacttttatg tacaaagtta 5160
tttatttata gaaattttgg tacagtgtac attgaaaacc atgtaaaata ttgaagtgtc 5220
taacaaatgg cattgaagtg tctttaataa aggttcattt ataaatgtca aaaaaaaaaa 5280
aaaaaaa 5287

<210> 90
<211> 1918
<212> DNA
<213> Homo sapiens
<400> 90
gaggccccgc cccctgagcc tgggtagcgg cgcgagggcc gggagaaccg ttcgcggagg 60
aaaggcgaac tagtgttggg atggccacca actgggggag cctcttgcag gataaacagc 120
agctagagga gctggcacgg caggccgtgg accgggccct ggctgaggga gtattgctga 180
ggacctcaca ggagcccact tcctcggagg tggtgagcta tgccccattc acgctcttcc 240
cctcactggt ccccagtgcc ctgctggagc aagcctatgc tgtgcagatg gacttcaacc 300
tgctagtgga tgctgtcagc cagaacgctg ccttcctgga gcaaactctt tccagcacca 360
tcaaacagga tgactttacc gctcgtctct ttgacatcca caagcaagtc ctaaaagagg 420
gcattgccca gactgtgttc ctgggcctga atcgctcaga ctacatgttc cagcgcagcg 480
cagatggctc cccagccctg aaacagatcg aaatcaacac catctctgcc agctttgggg 540
gcctggcctc ccggacccca gctgtgcacc gacatgttct cagtgtcctg agtaagacca 600
aagaagctgg caagatcctc tctaataatc ccagcaaggg actggccctg ggaattgcca 660
aagcctggga gctctacggc tcacccaatg ctctggtgct actgattgct caagagaagg 720
aaagaaacat atttgaccag cgtgccatag agaatgagct actggccagg aacatccatg 780
tgatccgacg aacatttgaa gatatctctg aaaaggggtc tctggaccaa gaccgaaggc 840
tgtttgtgga tggccaggaa attgctgtgg tttacttccg ggatggctac atgcctcgtc 900
agtacagtct acagaattgg gaagcacgtc tactgctgga gaggtcacat gctgccaagt 960
gcccagacat tgccacccag ctggctggga ctaagaaggt gcagcaggag ctaagcaggc 1020
cgggcatgct ggagatgttg ctccctggcc agcctgaggc tgtggcccgc ctccgcgcca 1080
cctttgctgg cctctactca ctggatgtgg gtgaagaagg ggaccaggcc atcgccgagg 1140
cccttgctgc ccctagccgg tttgtgctaa agccccagag agagggtgga ggtaacaacc 1200
tatatgggga ggaaatggta caggccctga aacagctgaa ggacagtgag gagagggcct 1260
cctacatcct catggagaag atcgaacctg agccttttga gaattgcctg ctacggcctg 1320
gcagccctgc ccgagtggtc cagtgcattt cagagctggg catctttggg gtctatgtca 1380
ggcaggaaaa gacactcgtg atgaacaagc acgtggggca tctacttcga accaaagcca 1440
tcgagcatgc agatggtggt gtggcagcgg gagtggcagt cctggacaac ccataccctg 1500
tgtgagggca caaccaggcc acgggacctt ctatcctctg tatttgtcat tcctctccta 1560
gccctcctga ggggtatcct cctaaagacc tccaaagttt ttatggaagg gtaaatactg 1620
gtaccttccc ccagctttcc atctgaggac cagaaaagtt gtgtctccct tagatgagat 1680
ctagacgccc ccaaatcctt gagatgtggg tatagctcag ggtaagctgc tctgaggtaa 1740
aggtccatga accctgcccc actcctgtca gcccctcatc agccttttca gcaggttcca 1800
gtgcctgact tgggatagga ctgagtggta ggaggagggg gagtggaggg gcatagcctt 1860
tccctaattc tgccttaaat aaaactgcat tgctgattca aaaaaaaaaa aaaaaaa 1918

<210> 91
<211> 1268
<212> DNA
<213> Homo sapiens
<400> 91
ggcattaatc aatccctgtt tctattttaa taggggggac aaggtagggg agagggaaaa 60
acagttttgg atataactat tttggaaggag agtagacatg aagagggcaa accctagctt 120
ttcattatct acaatcaata gtttgttttt ctttttaaa aagaaagaa aaacccttc 180
aatcttcaat actctttaaa agcccacttc ttagctactg gccaatccac accaattatt 240
taaattcact tggtacacac ctttgtccac tgggtaaatt atattcatta tgcccactgc 300
tgcagcacgc ataaaccaac accctgcat ggctgaacag ggcctaatct aggactgatg 360
ggagaagggc ttgcaaacca agatcaaggt gtttctccgc taatactgtc taccaagctg 420
atccctacaa aaatgcatat aaaagcaggc aagtttagct actgtgttgc aagagaaacc 480
aggaccttgt taaatagttc tctccattac catttattct ctcaagggaa gcttaaaaaa 540
acaacaacaa caaaacaaca cattggtctg gccacctcat gaatccaaca agcattagtg 600
tggcatttca gtggagaagg aaacttgggg ggaaaaatcc catcaaggtt gtaagaaagg 660
ctcccaattt aactgtccct gtccctattt atccatcatc caagaccatc cattattcta 720
gagcactctg atcaataaaa ggggtccaag catcaggagc aggcaaggag aaccaaaaga 780
catcaagaaa ccgatttgct tgagaaaagc agcgattctt cctttcagag ttctccatgg 840
ctgagaaaat gcccaagaca tcatgtatgt gacttagata ctgctttttg ggaggttaag 900
agtagcatga agaacttaag atgatgataa gagtctaaat ttttagtttc aaggtttcaa 960
tagaatgtgg atatattcaa aactttcaaa aaggacagtg tttagaaagg gtaaaactag 1020
gacacagaaa acactgggaa ttaccacgac cccaagtgc ttccggctcc aggaaataac 1080
cattcatgtg tttgctggag gtcacacaat tttcccctat tacctggtgc aaaatgactc 1140
atcacttccc aaaagcttct tttcaaacca cgattttccc atttattttg gtccaatgca 1200

gtcccattct ttatggccta tagtctcact cccaactacc cccctggggg gttaaaaaaa 1260
aaaaaaaa 1268

<210> 92
<211> 12515
<212> DNA
<213> Homo sapiens
<400> 92
ctaccgggcg gaggtgagcg cggcgccggc tcctcctgcg gcggactttg ggtgcgactt 60
gacgagcggt ggttcgacaa gtggccttgc gggccggatc gtcccagtgg aagagttgta 120
aatttgcttc tggccttccc ctacggatta tacctggcct tcccctacgg attatactca 180
acttactgtt tagaaaatgt ggcccacgag acgcctggtt actatcaaaa ggagcggggt 240
cgacggtccc cactttcccc tgagcctcag cacctgcttg tttggaaggg gtattgaatg 300
tgacatccgt atccagcttc ctgttgtgtc aaaacaacat tgcaaaattg aaatccatga 360
gcaggaggca atattacata atttcagttc cacaaatcca acacaagtaa atgggtctgt 420
tattgatgag cctgtacggc taaaacatgg agatgtaata actattattg atcgttcctt 480
caggtatgaa aatgaaagtc ttcagaatgg aaggaagtca actgaatttc caagaaaaat 540
acgtgaacag gagccagcac gtcgtgtctc aagatctagc ttctcttctg accctgatga 600
gaaagctcaa gattccaagg cctattcaaa aatcactgaa ggaaaagttt caggaaatcc 660
tcaggtacat atcaagaatg tcaaagaaga cagtaccgca gatgactcaa aagacagtgt 720
tgctcaggga acaactaatg ttcattcctc agaacatgct ggacgtaatg gcagaaatgc 780
agctgatccc atttctgggg attttaaaga aatttccagc gttaaattag tgagccgtta 840
tggagaattg aagtctgttc ccactacaca atgtcttgac aatagcaaaa aaaatgaatc 900
tccctttttgg aagctttatg agtcagtgaa gaaagagttg gatgtaaaat cacaaaaaga 960
aaatgtccta cagtattgta gaaaatctgg attacaaact gattacgcaa cagagaaaga 1020
aagtgctgat ggtttacagg gggagaccca actgttggtc tcgcgtaagt caagaccaaa 1080
atctggtggg agcggccacg ctgtggcaga gcctgcttca cctgaacaag agcttgacca 1140
gaacaagggg aagggaagag acgtggagtc tgttcagact cccagcaagg ctgtgggcgc 1200
cagctttcct ctctatgagc cggctaaaat gaagacccct gtacaatatt cacagcaaca 1260
aaattctcca caaaaacata agaacaaaga cctgtatact actggtagaa gagaatctgt 1320
gaatctgggt aaaagtgaag gcttcaaggc tggtgataaa actcttactc ccaggaagct 1380
ttcaactaga aatcgaacac cagctaaagt tgaagatgca gctgactctg ccactaagcc 1440
agaaaatctc tcttccaaaa ccagaggaag tattcctaca gatgtggaag ttctgcctac 1500
ggaaactgaa attcacaatg agccattttt aactctgtgg ctcactcaag ttgagaggaa 1560
gatccaaaag gattccctca gcaagcctga gaaattgggc actacagctg gacagatgtg 1620
ctctgggtta cctggtctta gttcagttga tatcaacaac tttggtgatt ccattaatga 1680
gagtgaggga ataccttga aaagaaggcg tgtgtccttt ggtgggcacc taagacctga 1740
actatttgat gaaaacttgc ctcctaatac gcctctcaaa aggggagaag ccccaaccaa 1800
aagaaagtct ctggtaatgc acactccacc tgtcctgaag aaaatcatca aggaacagcc 1860
tcaaccatca ggaaaacaag agtcaggttc agaaatccat gtggaagtga aggcacaaag 1920
cttggttata agccctccag ctcctagtcc taggaaaact ccagttgcca gtgatcaacg 1980
ccgtaggtcc tgcaaaacag cccctgcttc cagcagcaaa tctcagacag aggttcctaa 2040
gagaggagga gaaagagtgg caacctgcct tcaaaagaga gtgtctatca gccgaagtca 2100
acatgatatt ttacagatga tatgttccaa aagaagaagt ggtgcttcgg aagcaaatct 2160
gattgttgca aaatcatggg cagatgtagt aaaacttggt gcaaaacaaa cacaaactaa 2220
agtcataaaa catggtcctc aaaggtcaat gaacaaaagg caaagaagac ctgctactcc 2280
aaagaagcct gtgggcgaag ttcacagtca atttagtaca ggccacgcaa actctccttg 2340
taccataata atagggaaag ctcatactga aaaagtacat gtgcctgctc gaccctacag 2400
agtgctcaac aacttcattt ccaaccaaaa aatggacttt aaggaagatc tttcaggaat 2460
agctgaaatg ttcaagaccc cagtgaagga gcaaccgcag ttgacaagca catgtcacat 2520
cgctatttca aattcagaga atttgcttgg aaaacagttt caaggaactg attcaggaga 2580
agaacctctg ctccccacct cagagagttt tggaggaaat gtgttcttca gtgcacagaa 2640
tgcagcaaaa cagccatctg ataaatgctc tgcaagccct cccttaagac ggcagtgtat 2700
tagagaaaat ggaaacgtag caaaaacgcc caggaacacc tacaaaatga cttctctgga 2760
gacaaaaact tcagatactg agacagagcc ttcaaaaaca gtatccactg taaacaggtc 2820
aggaaggtct acagagttca ggaatataca gaagctacct gtggaaagta agagtgaaga 2880
aacaaataca gaaattgttg agtgcatcct aaaaagaggt cagaaggcaa cactactaca 2940
acaaaggaga gaaggagaga tgaaggaaat agaaagacct tttgagacat ataaggaaaa 3000
tattgaatta aaagaaaacg atgaaaagat gaaagcaatg aagagatcaa gaacttgggg 3060
gcagaaatgt gcaccaatgt ctgacctgac agacctcaag agcttgcctg atacagaact 3120
catgaaagac acggcacgtg gccagaatct cctccaaacc caagatcatg ccaaggcacc 3180
aaagagtgag aaaggcaaaa tcactaaaat gccctgccag tcattacaac cagaaccaat 3240
aaacacccca acacacacaa aacaacagtt gaaggcatcc ctggggaaag taggtgtgaa 3300
agaagagctc ctagcagtcg gcaagttcac acggacgtca ggggagacca cgcacacgca 3360
cagagagcca gcaggagatg gcaagagcat cagaacgttt aaggagtctc caaagcagat 3420
cctggaccca gcagcccgtg taactggaat gaagaagtgg ccaagaacgc ctaaggaaga 3480
ggcccagtca ctagaagacc tggctggctt caaagagctc ttccagacac caggtccctc 3540
tgaggaatca atgactgatg agaaaactac caaaatagcc tgcaaatctc caccaccaga 3600
atcagtggac actccaacaa gcacaaagca atggcctaag agaagtctca ggaaagcaga 3660

tgtagaggaa gaattcttag cactcaggaa actaacacca tcagcaggga aagccatgct 3720
tacgcccaaa ccagcaggag gtgatgagaa agacattaaa gcatttatgg gaactccagt 3780
gcagaaactg gacctggcag gaactttacc tggcagcaaa agacagctac agactcctaa 3840
ggaaaaggcc caggctctag aagacctggc tggctttaaa gagctcttcc agactcctgg 3900
tcacaccgag gaattagtgg ctgctggtaa aaccactaaa ataccctgcg actctccaca 3960
gtcagaccca gtggacaccc caacaagcac aaagcaacga cccaagagaa gtatcaggaa 4020
agcagatgta gagggagaac tcttagcgtg caggaatcta atgccatcag caggcaaagc 4080
catgcacacg cctaaaccat cagtaggtga agagaaagac atcatcatat ttgtgggaac 4140
tccagtgcag aaactggacc tgacagagaa cttaaccggc agcaagagac ggccacaaac 4200
tcctaaggaa gaggcccagg ctctggaaga cctgactggc tttaaagagc tcttccagac 4260
ccctggtcat actgaagaag cagtggctgc tggcaaaact actaaaatgc cctgcgaatc 4320
ttctccacca gaatcagcag acaccccaac aagcacaaga aggcagccca agacaccttt 4380
ggagaaaagg gacgtacaga aggagctctc agccctgaag aagctcacac agacatcagg 4440
ggaaaccaca cacacagata aagtaccagg aggtgaggat aaaagcatca acgcgtttag 4500
ggaaactgca aaacagaaac tggaccccagc agcaagtgta actggtagca agaggcaccc 4560
aaaaactaag gaaaaggccc aacccctaga agacctggct ggctggaaag agctcttcca 4620
gacaccagta tgcactgaca agcccacgac tcacgagaaa actaccaaaa tagcctgcag 4680
atcacaacca gacccagtgg acacaccaac aagctccaag ccacagtcca agagaagtct 4740
caggaaagtg gacgtagaag aagaattctt cgcactcagg aaacgaacac catcagcagg 4800
caaagccatg cacacaccca aaccagcagt aagtggtgag aaaaacatct acgcatttat 4860
gggaactcca gtgcagaaac tggacctgac agagaactta actggcagca agagacggct 4920
acaaactcct aaggaaaagg cccaggctct agaagacctg gctggcttta aagagctctt 4980
ccagacacga ggtcacactg aggaatcaat gactaacgat aaaactgcca aagtagcctg 5040
caaatcttca caaccagacc tagacaaaaa cccagcaagc tccaagcgac ggctcaagac 5100
atccctgggg aaagtgggcg tgaaagaaga gctcctagca gttggcaagc tcacacagac 5160
atcaggagag actacacaca cacacacaga gccaacagga gatggtaaga gcatgaaagc 5220
atttatggag tctccaaagc agatcttaga ctcagcagca agtctaactg gcagcaagag 5280
gcagctgaga actcctaagg gaaagtctga agtccctgaa gacctggccg gcttcatcga 5340
gctcttccag acaccaagtc acactaagga atcaatgact aatgaaaaaa ctaccaaagt 5400
atcctacaga gcttcacagc cagacctagt ggacacccca acaagctcca agccacagcc 5460
caagagaagt ctcaggaaag cagacactga agaagaattt ttagcattta ggaaacaaac 5520
gccatcagca ggcaaagcca tgcacacacc caaaccagca gtaggtgaag agaaagacat 5580
caacacgttt ttgggaactc cagtgcagaa actggaccag ccaggaaatt tacctggcag 5640
caatagacgg ctacaaactc gtaaggaaaa ggcccaggct ctagaagaac tgactggctt 5700
cagagagctt ttccagacac catgcactga taaccccaca gctgatgaga aaactaccaa 5760
aaaaatactc tgcaaatctc cgcaatcaga cccagcggac accccaacaa acacaaagca 5820
acggcccaag agaagcctca agaaagcaga cgtagaggaa gaatttttag cattcaggaa 5880
actaacacca tcagcaggca aagccatgca cacgcctaaa gcagcagtag gtgaagagaa 5940
agacatcaac acatttgtgg ggactccagt ggagaaactg gacctgctag gaaatttacc 6000
tggcagcaag agacggccac aaactcctaa agaaaaggcc aaggctctag aagatctggc 6060
tggcttcaaa gagctcttcc agacaccagg tcacactgag gaatcaatga ccgatgacaa 6120
aatcacagaa gtatcctgca aatctccaca accagaccca gtcaaaaccc caacaagctc 6180
caagcaacga ctcaagatat ccttggggaa agtaggtgtg aaagaagagg tcctaccagt 6240
cggcaagctc acacagacgt cagggaagac cacacagaca cacagagaga cagcaggaga 6300
tggaaagagc atcaaagcgt ttaaggaatc tgcaaagcag atgctggacc cagcaaacta 6360
tggaactggg atggagaggt ggccaagaac acctaaggaa gaggcccaat cactagaaga 6420
cctggccggc ttcaaagagc tcttccagac accagaccac actgaggaat caacaactga 6480
tgacaaaact accaaaatag cctgcaaatc tccaccacca gaatcaatgg acactccaac 6540
aagcacaagg aggcggccca aaacaccttt ggggaaaagg gatatagtgg aagagctctc 6600
agccctgaag cagctcacac agaccacaca cacagacaaa gtaccaggag atgaggataa 6660
aggcatcaac gtgttcaggg aaactgcaaa acagaaactg gacccagcag caagtgtaac 6720
tggtagcaag aggcagccaa gaactcctaa gggaaaagcc caacccctag aagacttggc 6780
tggcttgaaa gagctcttcc agacaccagt atgcactgac aagcccacga ctcacgagaa 6840
aactaccaaa atagcctgca gatctccaca accagaccca gtgggtaccc caacaatctt 6900
caagccacag tccaagagaa gtctcaggaa agcagacgta gaggaagaat ccttagcact 6960
caggaaacga acaccatcag tagggaaagc tatggacaca cccaaaccag caggaggtga 7020
tgagaaagac atgaaagcat ttatgggaac tccagtgcag aaattggacc tgccaggaaa 7080
tttacctggc agcaaaagat ggccacaaac tcctaaggaa aaggcccagg ctctagaaga 7140
cctggctggc ttcaaagagc tcttccagac accaggcact gacaagccca cgactgatga 7200
gaaaactacc aaaatagcct gcaaatctcc acaaccagac ccagtggaca ccccagcaag 7260
cacaaagcaa cggcccaaga gaaacctcag gaaagcagac gtagaggaag aatttttagc 7320
actcaggaaa cgaacaccat cagcaggcaa agccatggac accccaaaac cagcagtaag 7380
tgatgagaaa aatatcaaca catttgtgga aactccagtg cagaaactgg acctgctagg 7440
aaatttacct ggcagcaaga gacagccaca gactcctaag gaaaaggctg aggctctaga 7500
ggacctggtt ggcttcaaag aactcttcca gacaccaggt cacactgagg aatcaatgac 7560
tgatgacaaa atcacagaag tatcctgtaa atctccacag ccagagtcat tcaaaacctc 7620
aagaagctcc aagcaaaggc tcaagatacc cctggtgaaa gtggacatga aagaagagcc 7680
cctagcagtc agcaagctca cacggacatc aggggagact acgcaaacac acacagagcc 7740
aacaggagat agtaagagca tcaaagcgtt taaggagtct ccaaagcaga tcctggaccc 7800

agcagcaagt gtaactggta gcaggaggca gctgagaact cgtaaggaaa aggcccgtgc 7860
tctagaagac ctggttgact tcaaagagct cttctcagca ccaggtcaca ctgaagagtc 7920
aatgactatt gacaaaaaca caaaaattcc ctgcaaatct cccccaccag aactaacaga 7980
cactgccacg agcacaaaga gatgccccaa gacacgtccc aggaaagaag taaaagagga 8040
gctctcagca gttgagaggc tcacgcaaac atcagggcaa agcacacaca cacacaaaga 8100
accagcaagc ggtgatgagg gcatcaaagt attgaagcaa cgtgcaaaga agaaaccaaa 8160
cccagtagaa gaggaaccca gcaggagaag gccaagagca cctaaggaaa aggcccaacc 8220
cctggaagac ctggccggct tcacagagct ctctgaaaca tcaggtcaca ctcaggaatc 8280
actgactgct ggcaaagcca ctaaaatacc ctgcgaatct cccccactag aagtggtaga 8340
caccacagca agcacaaaga ggcatctcag gacacgtgtg cagaaggtac aagtaaaaga 8400
agagccttca gcagtcaagt tcacacaaac atcaggggaa accacggatg cagacaaaga 8460
accagcaggt gaagataaag gcatcaaagc attgaaggaa tctgcaaaac agacaccggc 8520
tccagcagca agtgtaactg gcagcaggag acggccaaga gcacccaggg aaagtgccca 8580
agccatagaa gacctagctg gcttcaaaga cccagcagca ggtcacactg aagaatcaat 8640
gactgatgac aaaaccacta aaataccctg caaatcatca ccagaactag aagacaccgc 8700
aacaagctca aagagacggc ccaggacacg tgcccagaaa gtagaagtga aggaggagct 8760
gttagcagtt ggcaagctca cacaaacctc aggggagacc acgcacaccg acaaagagcc 8820
ggtaggtgag ggcaaaggca cgaaagcatt taagcaacct gcaaagcgga acgtggacgc 8880
agaagatgta attggcagca ggagacagcc aagagcacct aaggaaaagg cccaacccct 8940
ggaagacctg gccagcttcc aagagctctc tcaaacacca ggccacactg aggaactggc 9000
aaatggtgct gctgatagct ttacaagcgc tccaaagcaa acacctgaca gtggaaaacc 9060
tctaaaaata tccagaagag ttcttcgggc ccctaaagta gaacccgtgg gagacgtggt 9120
aagcaccaga gaccctgtaa aatcacaaag caaaagcaac acttccctgc ccccactgcc 9180
cttcaagagg ggaggtggca aagatggaag cgtcacggga accaagaggc tgcgctgcat 9240
gccagcacca gaggaaattg tggaggagct gccagccagc aagaagcaga gggttgctcc 9300
cagggcaaga ggcaaatcat ccgaacccgt ggtcatcatg aagagaagtt tgaggacttc 9360
tgcaaaaaga attgaacctg cggaagagct gaacagcaac gacatgaaaa ccaacaaaga 9420
ggaacacaaa ttacaagact cggtccctga aaataaggga atatccctgc gctccagacg 9480
ccaagataag actgaggcag aacagcaaat aactgaggtc tttgtattag cagaaagaat 9540
agaaataaac agaaatgaaa agaagcccat gaagacctcc ccagagatgg acattcagaa 9600
tccagatgat ggagcccgga aacccatacc tagagacaaa gtcactgaga acaaaaggtg 9660
cttgaggtct gctagacaga atgagagctc ccagcctaag gtggcagagg agagcggagg 9720
gcagaagagt gcgaaggttc tcatgcagaa tcagaaaggg aaaggagaag caggaaattc 9780
agactccatg tgcctgagat caagaaagac aaaaagccag cctgcagcaa gcactttgga 9840
gagcaaatct gtgcagagag taacgcggag tgtcaagagg tgtgcagaaa atccaaagaa 9900
ggctgaggac aatgtgtgtg tcaagaaaat aacaaccaga agtcataggg acagtgaaga 9960
tatttgacag aaaaatcgaa ctgggaaaaa tataataaag ttagttttgt gataagttct 10020
agtgcagttt ttgtcataaa ttacaagtga attctgtaag taaggctgtc agtctgctta 10080
agggaagaaa actttggatt tgctgggtct gaatcggctt cataaactcc actgggagca 10140
ctgctgggct cctggactga gaatagttga acaccggggg ctttgtgaag gagtctgggc 10200
caaggtttgc cctcagcttt gcagaatgaa gccttgaggt ctgtcaccac ccacagccac 10260
cctacagcag ccttaactgt gacacttgcc acactgtgtc gtcgtttgtt tgcctatgtt 10320
ctccagggca cggtggcagg aacaactatc ctcgtctgtc ccaacactga gcaggcactc 10380
ggtaaacacg aatgaatgga taagcgcacg gatgaatgga gcttacaaga tctgtctttc 10440
caatggccgg gggcatttgg tccccaaatt aaggctattg gacatctgca caggacagtc 10500
ctatttttga tgtcctttcc tttctgaaaa taaagttttg tgctttggag aatgactcgt 10560
gagcacatct ttagggacca agagtgactt tctgtaagga gtgactcgtg gcttgccttg 10620
gtctcttggg aatacttttc taactagggt tgctctcacc tgagacattc tccacccgcg 10680
gaatctcagg gtcccaggct gtgggccatc acgacctcaa actggctcct aatctccagc 10740
tttcctgtca ttgaaagctt cggaagttta ctggctctgc tcccgcctgt ttctttctg 10800
actctatctg gcagcccgat gccacccagt acaggaagtg acaccagtac tctgtaaagc 10860
atcatcatcc ttggagagac tgagcactca gcaccttcag ccacgatttc aggatcgctt 10920
ccttgtgagc cgctgcctcc gaaatctcct ttgaagccca gacatctttc tccagcttca 10980
gacttgtaga tataactcgt tcatcttcat ttactttcca ctttgccccc tgtcctctct 11040
gtgttcccca aatcagagaa tagcccgcca tcccccagat cacctgtctg gattcctccc 11100
cattcaccca ccttgccagg tgcaggtgag gatggtgcac cagacagggt agctgtcccc 11160
caaaatgtgc cctgtgcggg cagtgccctg tctccacgtt tgtttcccca gtgtctggcg 11220
gggagccagg tgacatcata aatacttgct gaatgaatgc agaaatcagc ggtactgact 11280
tgtactatat tggctgccat gatagggttc tcacagcgtc atccatgatc gtaagggaga 11340
atgacattct gcttgaggga gggaatagaa aggggcaggg aggggacatc tgaggccttc 11400
acagggctgc aaagggtaca gggattgcac cagggcagaa caggggaggg tgttcaagga 11460
agagtggctc ttagcagagg cactttggaa ggtgtgaggc ataaatgctt ccttctacgt 11520
aggccaacct caaaactttc agtaggaatg ttgctatgat caagttgttc taacacttta 11580
gacttagtag taattatgaa cctcacatag aaaaatttca tccagccata tgcctgtgga 11640
gtggaatatt ctgtttagta gaaaaatcct ttagagttca gctctaacca gaaatcttgc 11700
tgaagtatgt cagcaccttt tctcaccctg gtaagtacag tatttcaaga gcacgctaag 11760
ggtggttttc attttacagg gctgttgatg atgggttaaa aatgttcatt taagggctac 11820
ccccgtgttt aatagatgaa caccacttct acacaaccct ccttggtact gggggaggga 11880
gagatctgac aaatactgcc cattccccta ggctgactgg atttgagaac aaataccccac 11940

ccatttccac catggtatgg taacttctct gagcttcagt ttccaagtga atttccatgt 12000
aataggacat tcccattaaa tacaagctgt ttttactttt tcgcctccca gggcctgtgc 12060
gatctggtcc cccagcctct cttgggcttt cttacactaa ctctgtacct accatctcct 12120
gcctccctta ggcaggcacc tccaaccacc acacactccc tgctgttttc cctgcctgga 12180
actttcccac cagccccacc aagatcattt catccagtcc tgagctcagc ttaagggagg 12240
cttcttgcct gtgggttccc tcacccccat gcctgtcctc caggctgggg caggttctta 12300
gtttgcctgg aattgttctg tacctctttg tagcacgtag tgttgtgaaa ctaagccact 12360
aattgagttt ctggctcccc tcctggggtt gtaagttttg ttcattcatg agggccgact 12420
gtatttcctg gttactgtat cccagtgacc agccacagga gatgtccaat aaagtatgtg 12480
atgaaatggt cttaaaaaaa aaaaaaaaaa aaaaa 12515

<210> 93
<211> 963
<212> DNA
<213> Homo sapiens
<400> 93
gacacgaagc ctcccgggtg gcttacagac gctgccagca tcgccgccgc cagaggagaa 60
atgtctgaag taagacccct ctccagagac atcttgatgg agaccctcct gtatgagcag 120
ctcctggaac ccccgaccat ggaggttctt ggcatgactg actctgaaga ggacctggac 180
cctatggagg acttcgattc tttggaatgc atggagggca gtgacgcatt ggccctgcgg 240
ctggcctgca tcggggacga gatggacgtg agcctcaggg ccccgcgcct ggcccagctc 300
tccgaggtgg ccatgcacag cctgggtctg gctttcatct acgaccagac tgaggacatc 360
agggatgttc ttagaagttt catggacggt ttcaccacac ttaaggagaa cataatgagg 420
ttctggagat ccccgaaccc cgggtcctgg gtgtcctgcg aacaggtgct gctggcgctg 480
ctgctgctgc tggcgctgct gctgccgctg ctcagcgggg gcctgcacct gctgctcaag 540
tgaggccccg gcggctcagg gcggggctgg ccccaccccc atgaccactg ccctggaggt 600
ggcggcctgc tgctgttatc tttttaactg ttttctcatg atgccttttt atatttaaac 660
cccgagatag tgctggaaca ctgctgaggt tttatactca ggtttttttgt ttttttttta 720
ttccagtttt cgtttttttct aaaagatgaa ttcctatggc tctgcaattg tcaccggtta 780
actgtggcct gtgcccagga agagccattc actcctgccc ctgcccacac ggcaggtagc 840
agggggagtg ctggtcacac ccctgtgtga tatgtgatgc cctcggcaaa gaatctactg 900
gaatagattc cgaggagcag gagtgctcaa taaaatgttg gtttccagca aaaaaaaaaa 960
aaa 963

<210> 94
<211> 848
<212> DNA
<213> Homo sapiens
<220>
<221> misc_feature
<222> (671)..(671)
<223> n = a, t, g, or c
<220>
<221> misc_feature
<222> (677)..(677)
<223> n = a, t, g, or c
<220>
<221> misc_feature
<222> (737)..(737)
<223> n = a, t, g, or c
<400> 94
taaaaagtat ttattttta ctagtgagga tggaactagg ggaagaagtt tacagaaaag 60
atgtccatat acaaagaaag aaatagcaaa atatttttgg tcataattta gaaagaaact 120
aatctataaa atggagacaa ttctccccaa ttcatctctc tcatagaaga gcataagcac 180
gggctctcag ttccttgata tcctcaagag gccacggata cgtctgacaa gagcctgtat 240
gaaactatat cgagatcgaa cttttgaata taatccttca atgtccagaa gtttcttttg 300
tagtgattct ccaaaagcgt tgattgcatc agcctgaagc tggtctaaat catgttggct 360
cactatagcc agcccacttc taaaatctag attggtttct gaggcgaagg aatcttgcag 420
tcttctgctt ttaaacaagg ttctcgaggc aaataaggaa tcttgagaat ccgtgggcat 480
gcaatgtaac aagtagtact ccagatggcg ccaaagaata aataggcagg tctctatgat 540
aaaagaacaa agggaaagca gtttagctcg attggtgatc accttcacca agcgccgtct 600
tgctagaaca tatttctgag cagtggagaa tttatcacac cagcaggcat cacagactga 660
cacaactctt ntatctncat ctggggaagc tgctctacat tttgtagctt gctgacactc 720
tgtcgatgac tgtcatnata gctggagaaa tcatagcact ctgtttcagc aggagatgat 780
tatgctaaac caggcaggcg ccagtatgga acactggaag ctgggtatct tgcgcggtca 840
tctctatt 848

<210> 95
<211> 749

<212> DNA
<213> Homo sapiens
<400> 95
ttttttttt ttttttttt ttttttttt ttttttttcc agatgaaatt ccaggctttt 60
atcaaaagca aaagtttatc cctatccaaa aatgaaatta tacaaattca tacatatggt 120
aaagattcta gctgcctctt aaacgagaca aattttcagc agaggggccc aaaacaaaat 180
ggtcctagcc ctctgaagta aaaatattta tgggaaaaat cagtttttgt aagaaagaaa 240
aattaagaat agtctctctc ccaggccata gagcttcctt tgtccaatca ggttggatca 300
tgccagggga gatcagcttg ctcaaaagtc actatttaac gaagaaaaac ataaatcctt 360
tcagagaagg gacactcaga aagaacaggt gaggagtgtg taacatcaaa ccaaaccaac 420
ctcatttcaa ccccactcag ggcctctctg atgcaatccc aaagttagca gtggcaatgc 480
aactttcaga ccgggagagc tctgactgcc tgttggatac tcgagtcaca gtccatgggg 540
tgttttgtta ttggccaatg tctgttaacc attaactctc ttcatgattg caccagttag 600
accatttgcc ctgatggggg gaaaaagaag aagaaaaaac aaattagaag aaaaaaagtg 660
acaagagaaa gcaagctgct aatggccgat cccaacacac tttctgggag acagtagatg 720
aaatcacagc cacagtgacc acatgctgc 749

<210> 96
<211> 1865
<212> DNA
<213> Homo sapiens
<400> 96
gcgtggaggt cgacgactcc gtcgcagact acggacctgt ctgggtctca gccgccaaag 60
accccgtccg gtaggtgagt ggctcacttt gagggcaagc cttctcggat cgaggcttct 120
tcatggccgc tcagatcgtg agcggccggg gctgctctct ttgcggagga tggcgtctaa 180
tgagcgcagt tgattcgagg aagtactagc cggacatcat gagtggctgt cgggtattca 240
tcgggagact aaatccagcg gccagggaga aggacgtgga aagattcttc aagggatatg 300
gacggataag agatattgat ctgaaaagag gctttggttt tgtggaattt gaggatccaa 360
gggatgcaga tgatgctgtg tatgagcttg atggaaaaga actctgtagt gaaagggtta 420
ctattgaaca tgctagggct cggtcacgag gtggaagagg tagaggacga tactctgacc 480
gttttagtag tcgcagacct cgaaatgata gacggtatgt gaagggtgga tggctgcatt 540
gaacaattat tgtaggggta gcatttaaga ttcaggagtc attagcagtg atgattttgg 600
gacctgccgt ataatctgtt cttctattcc cacgttagcc aattgttctt gatgaatcta 660
tatgagtcat agaacacaaa tctattgacg gaagtcatta gaatggcttg tgatatctga 720
tggcttgaac ttgccccacag ttgaacacaa gtgctgtcat tgcatttctt ccattgtgaa 780
tacgaatttt cttcctcaga aatgctccac ctgtaagaac agaaaatcgt cttatagttg 840
agaatttatc ctcaagagtc agctggcagg atctcaaaga tttcatgaga caagctgggg 900
aagtaacgtt tgcggatgca caccgaccta aattaaatga aggggtggtt gagtttgcct 960
cttatggtga cttaaagaat gctattgaaa aactttctgg aaaggaaata aatgggagaa 1020
aaataaaatt aattgaaggc agcaaaaggc acaggtcaag aagcaggtct cgatcccgga 1080
ccagaagttc ctctaggtct cgtagccgat cccgttcccg tagtcgcaaa tcttacagcc 1140
ggtcaagaag caggagcagg agccggagcc ggagcaagtc ccgttctgtt agtaggtctc 1200
ccgtgcctga gaagagccag aaacgtggtt cttcaagtag atctaagtct ccagcatctg 1260
tggatcgcca gaggtcccgg tcccgatcaa ggtccagatc agttgacagt ggcaattaaa 1320
ctgtaaataa cttgccctgg gggccttttt ttttaaaaaa caaaaaccac aaaaattccc 1380
aaaccatact tgctaaaaat tctggtaagt atgtgctttt ctgtgggggt gggatttgga 1440
agggggggttg ggttgggctg gatatctttg tagatgtgga ccaccaaggg gttgttgaaa 1500
actaattgta ttaaatgtct tttgataagc cttctgctca catttttgtg aatgtctgaa 1560
gtatatagtt tgtgtatatt gacagagctc ttttataact aaagcaaatt taattttttt 1620
gtactagaaa aaaatttgaa catttagtt cttggttata aaaatgttaa ttcagaatta 1680
gtttaatgcc ttaattaaac taattaatag ctttggacac ttaaaagagc tctaaatttg 1740
cttgtacata aaggcttaat ttgttttcct tgttagggtc aagggtgtcc tccactcttt 1800
aacagctgct ggacagacac attagagcag ctgtttgtta ttgataataa aatattataa 1860
aacta 1865

<210> 97
<211> 898
<212> DNA
<213> Homo sapiens
<400> 97
tcctctttcc ctaagcagcc tgagggttga ctggattggt gaggcccgtg tggctacttc 60
tgtggaagca gtgctgtagt tactggaaga taaaagggaa agcaagccct tggtggggga 120
aagtatggct gcgatgatgg catttcttag gacacctttg gattaataat gaaaacaact 180
actctctgag cagctgttcg aatcatctga tatttatact gaatgagtta ctgtaagtac 240
gtattgacag aattacactg tactttcctc taggtgatct gtgaaaatgg ttcgctattc 300
acttgacccg gagaacccca cgaaatcatg caaatcaaga ggttccaatc ttcgtgttca 360
ctttaagaac actcgtgaaa ctgctcaggc catcaagggt atgcatatac gaaaagccac 420
gaagtatctg aaagatgtca ctttacagaa acagtgtgta ccattccgac gttacaatgg 480
tggagttggc aggtgtgcgc aggccaagca atggggctgg acacaaggtc ggtggcccaa 540

aaagagtgct gaatttttgc tgcacatgct taaaaacgca gagagtaatg ctgaacttaa 600
gggtttagat gtagattctc tggtcattga gcatatccaa gtgaacaaag cacctaagat 660
gcgccgccgg acctacagag ctcatggtcg gattaaccca tacatgagct ctccctgcca 720
cattgagatg atccttacgg aaaaggaaca gattgttcct aaaccagaag aggaggttgc 780
ccagaagaaa aagatatccc agaagaaact gaagaaacaa aaacttatgg cacgggagta 840
aattcagcat taaaataaat gtaattaaaa ggaaaaaaaa aaaaaaaaaa aaaaaaa 898

<210> 98
<211> 592
<212> DNA
<213> Homo sapiens
<400> 98
ttcggcacag ggccggagag caagagcggg gaggaggaga ggtcggaatg tctccaggag 60
cccttagaga ccgagtcccg gcggcgacgg cggggcagcg caccggcagg cggattcatt 120
ccacttaaaa cctgaaaaca ttggaccaca caaagtctta ctgatttcag gtaaaaacaa 180
taattgaaga tgtccagcaa aacagcaagc accaacaata tagcccaggc aaggagaact 240
gtgcagcagt taagattaga agcctccatt gaaagaataa aggtttcgaa ggcatcagcg 300
gacctcatgt cctactgtga ggaacatgcc aggagtgacc ctttgctgat aggaatacca 360
acttcagaaa accctttcaa ggataaaaaa acttgcatca tcttatagtg gaatagagaa 420
acagctcctc gcctcttccc aacaacgcaa atttatgagc agctccttga agaagattta 480
ccttcagctt attttggtaa ccactgctaa taactaaaat gttctcagct tggaataatg 540
gactctgagt ctctattttc caagttgtcc tttctcctta aaataccctt tt 592

<210> 99
<211> 3275
<212> DNA
<213> Homo sapiens
<400> 99
gtactacctt cggtctaggc agcggaggca gccgcgaccc caggaaaccg aggaaatgaa 60
gacgcgaagg actacccgcc ttcagcagca gcactcagag cagcctccgc tacagccgtc 120
tcctgttacg accaggagag ggctgcggga ctctcattcc tctgaagagg atgaagcatc 180
ttcccaaact gatttaagcc aaacgatctc aaagaaaact gtcaggagca tacaagaggc 240
tccagtgagt gaagatcttg taatcaggtt acgtcgaccc cctctaagat gcccaagata 300
tgaagccacc agtgtccaac agaaggtcaa tttctctgaa gaaggagaaa ctgaagaaga 360
tgatcaagac agctctcaca gcagtgtcac tactgttaag gccagatcca gggattctga 420
tgaatctgga gataaaacca ccagatcatc tagtcaatat atagaatcat tttggcagtc 480
atcacaaagt caaaacttca cagctcatga taagcaacgt tcagtgctaa gctcaggata 540
tcaaaaaact ccccaggaat gggccccaca aactgcaaga ataaggacca ggatgcaaaa 600
tgacagcatt ctgaaatcag agcttggaaa ccagtcacca tcaacctcca gccgacaagt 660
gactggacaa ccccaaaatg catcttttgt caagaggaac cggtggtggc tacttcctct 720
gatagctgct cttgcctctg ggagtttttg gttctttagt actcctgagg tagaaaccac 780
tgctgttcaa gagttccaga accagatgaa tcaacttaag aataagtacc aaggtcaaga 840
tgagaagctg tggaaaagga gccaaacatt cctggaaaaa catcttaata gctcccatcc 900
tcggtctcag cctgctatct tactgctcac tgctgcccga gatgctgaag aagcacttag 960
gtgtctgagt gaacaaattg ctgatgccta ttcttctttt cgtagtgtcc gtgccatccg 1020
gattgatggg acagataaag ctactcaaga cagtgatact gtcaaactag aggtagacca 1080
agaactgagc aatggattta agaatggcca gaatgcagct gtggtacacc gctttgagtc 1140
atttcccgca ggctctactt tgatcttcta caaatattgt gaccatgaaa acgcagcctt 1200
caaagatgta gccttagtcc tgactgtctt attggaggaa gagacacttg gaacaagtct 1260
aggcctaaag gaagttgaag aaaaagtaag agattttctt aaagtcaagt tcaccaattc 1320
taacacaccc aactcctaca atcatatgga cccagacaaa ctgaatggcc tctggagccg 1380
tatttctcac ttagttctgc ctgtgcaacc tgaaaatgcc ctgaaaaggg gcatctgctt 1440
ataagaagtg agagagaaga aaaatcatgt cccaagttct gagaattgtt cacactttct 1500
aaccagagac agaattcaga gctctttttg aaagaactag tttcttttta aagaagttaa 1560
gtgcttacat aaacatggaa catataaatc attcattcaa cctaattatc tgtgatatga 1620
gagaatcatt tcagtttcca ttgagagctc tgttaaaggt atcttaggag tgcagattat 1680
atgcagttcc ttagagaatc tgttttgatt ctgggctgag ttattacaat tatggtatga 1740
ccagtgagag cgatttgtgt cctttcttat gaaccttcct gattttttaa cttagatttc 1800
tcactaagtt tcctgagtta ttagtaagat tgctccctaa atgtaaccag ggatttttcc 1860
attatgttcc cttttatacc atttaggtgt gagttcctta gcttctgcct ataggaacat 1920
aagtaatgaa aggtcatcta ggtgtgtgtt tggaattttt ttctttttgtt ttttggaaaa 1980
tggaaagaac aaggcaagga aggaaaatta atggggaagc tgaagggagg aaatgttaca 2040
gtaatccact gagatgtagt ttagtcaaac ataggtatat gaactgttaa tcttggtgga 2100
ttccgttggg atttgttttt tacatctcct ttttccttcc ttgaagaaaa attcttggct 2160
gtcccaagga ttcttcatgt atattgcaaa atttaatata tttgttcact tgagtcttaa 2220
gagtaggtca ggccaaggca ggtggatcac ctgaggtcag aagttcaaga ccagcctggc 2280
caacatggtg aaaccccctc tctactaaaa atacaaaaat tagctgggcg tggtggtgca 2340
tgcctgaaat cccagctact tgggaggctg aggcaggaga attgcttgaa cccgggaggc 2400
ggaggttgca gtaagccgag accatgctta ttgccctcca gtctgggcaa caaaaatgaa 2460

actccgtctc aaaaaaaaaa aagtaggtca attcgtaaca gtttattttg gacacaccct 2520
gaagcttttg ttttgaatta agaaatgagt ttcaggaatt gacaaaccat ttgttaacca 2580
gctgtcctgg gctattgaag aagatttcat tttccgcgca tccacttgtt gcagtccaag 2640
tcctctagtg caacgccata gcaaatataa agatttacta tgcacgtgat acattttatg 2700
gagtgcctca agccaagata gtgaatttat atgaagggtg tgaaatgtat ttctttacta 2760
ctatagtagt ttagttataa ctttgcatct ataattgagc tttctcatct gtcaaatgct 2820
atggttttct taaaatgtta caattctaga tctatccacc ttgttttttt attgtctaca 2880
aaccattaaa tgtaaatatt gtttttagag tcagtcattg gctttgtcat ttaccctttg 2940
agagttccac aagtggtagt agagtggttt aacgtctttc ctctagtact accagtattc 3000
ataaatgtat acccccttact gtaatttgtt cctcttagaa gtcagatcat ctgatttata 3060
gaggattatg aaaaccagag tttttgaaaa ggcttatttt atacatacgt attatataga 3120
gaaacaaatg tttttattaa atgtttcatt gacccaagta atttaaaagt aatatgttac 3180
ctatgtcttt caggaaaaat aattatagca gctgatctgt aaatgacttt aaaagctatt 3240
ttagtaattt aaataaattt actttcttgg tttat 3275

<210> 100
<211> 2399
<212> DNA
<213> Homo sapiens
<400> 100
ttagaagaat tgcataaaac gtagtaaatg gggtctgtca ttagcaaagg caaatctaag 60
caatcatttt tccccccaga agttacttag aaggagaact gggaacactt ggggtctctc 120
taactgatgg cattcacttc acacagtcgt ctatgttatc cagagatttt tatttcattt 180
tacattttag ggcacagttc tttggggcta attaaaatgg ggtttgcagg ctttttatgg 240
tgaagaataa tatatctctg tctatagctt tcccatggta gcctgataag gctgagagag 300
aaaaatatgt gcagtatctc atcctccccc tgtaccaggc catagctttg aagtgtattt 360
tgtaaattca actataggtt agtcagaatg ctgtttttcg ttaattaact tagcctgtgt 420
tgatatctcc tccttcctgg tcacattcaa accttcccag agtacaaagg ggtatgtaga 480
aaggattcca gaagaagtaa tactttattc tctaatgtta atagcttttc tggatctctt 540
agtagggggga aagtagaaaa tgattgttta acagtatgtg tggtgatgtc attatctcca 600
gagaggcttc aagaaatcct ttggaaataa aaagttaaat gtttgcattt catgtggtat 660
ttcaggtctt caggttctga ttagcttact tttttccttt gtctttggct gatttctgct 720
ttgtagataa ataataatag ccctgagatg tttctaacat ttaaataaga aaaaaatcaa 780
atcgaagtca gcctgctgga aaagtgatca catggcagtt gcagtaactt gtatggaaag 840
agaaaatgca atgagcccag ttactgcact tgccactacc atgctgtcca tggaaggaat 900
aatcagcagt tcagttgtca caagccgccc ttgaagaaaa cgcagcaaaa tattttaaaa 960
tgaagatatt gcagtcccca gagccagtga aggtttcttt tggtaaaatg aaattgtgcc 1020
attgtcaaag taccccgtag tgatgagcac tgactggttc actggccaca ttttagttct 1080
tcataataat aggccacaaa agggctctgt ggtttgcctc catgtgcact ggcccctccc 1140
cacccctagg gggcactcag tagctgctga gaaggcctgt ccacgaggct gttggaaccc 1200
ctccaataaa tacttagagg tagtgtatct gatgcttgtt ttcgtggaga aaattgtatt 1260
ggagaactta aaacatcacg aatattttta ataggatccg cagacaccca aaggagaagc 1320
ttggtctttt ccaggtattt ccaacttgag ttcaacccaa agcctttgaa aggaatgcat 1380
taccccatga ccacatgctg agaccccatg gggtctaaca cgggacctaa gaaagtctct 1440
gcagccagat agtacatggt gtctccacaa aactaggcat tctggagatt gcccagaaag 1500
ggatgtgagg ggaccgttaa gatctgtctt gcttatctca tgcactcaca ttccttcagc 1560
ctcctggagt tcctgataaa aggaagccag ggtgtggaca ttttttagct attgatttcc 1620
caatagcttg tggatcagtt gtacacccac acttccttct ctgcctaatt ccgtttttct 1680
ggaaaaagta gtatgcccat gtatgtgtgt ttttcttaac acaggtccat gaaagtttgg 1740
cttcctggtt tgatgtctgt tgcgtggcct ggaaaccagg gagcagcaac tattgagatg 1800
gtttctgtgt tcagtgaaaa attctatttc attgagacaa ttttttcttt atccacagta 1860
attttttgac actgtcatca tgaaactacc cttaggaaaa taagattacc tgcaaaaaaa 1920
aaaaagaaat gagttatgga ataggaacag ttatgtgatg attctgaaac tttaacttag 1980
agcttcatta ctttaagaat ggaaaacaac ctctgagttt gatttcccaa agtttcataa 2040
agcccctaag ctcatgattt tcatcaactc tttgcccaca tagtcattta cctccacagc 2100
cgtttgttgt catagaaggg gtggtggtgt ttggatttga tttttttcaa cttgcagtga 2160
gaaataggat aggtgacaaa accttacttg ttttcttaag acaattcagt gcttgagcat 2220
ctctgtcaga aatggaatga aatactgtta gccaattaga attattttat gtattgttat 2280
tgtgttttgc tgatttttat atgaaaatat aattattcat tcttgatctc tggaagcaat 2340
cattattatg aggatcattt tactttggaa acactttcat aataaagata agtattaag 2399

<210> 101
<211> 6496
<212> DNA
<213> Homo sapiens
<400> 101
cgcactctcc gtccccgcgg ctggcgcagg acctcactcg agcggagcgc ccacggggag 60
cgggtcgcgg ggcggcggcg gcgaggagga ggcgagaagg agttggagga ggaggaggag 120
gaggcgaggg cgagcgagcc cagcggggtc ccggccgccc cgcgggccaa agtcgagccc 180

```
tcccgcccgt gggcgagcgc gccagccgcc ccttccagaa cagccgccgc cacaaagaag    240
aacgggggt gccgaggtcc ccatgacctc ctaaagtggt gcggtccctg ctgagtgcgc     300
tgcccgggcc gtgacccgcg cccctgtgcg tccccgcgcg cctccgagcg cccctgtgcg    360
ccccggcccg cgccccgccg gcatggacgt ccatacccgc tggaaagcgc gcagcgcgct    420
ccgcccgggc gccccgctgc tgcccccgct gctgctgctg ctgctgtggg cgccgcctcc    480
gagccgcgca gctcagccag cagatctcct gaaggttcta gattttcaca acttgcctga    540
tggaataaca aagacaacag gcttttgcgc cacgcggcga tcttccaaag gcccggatgt    600
cgcttacaga gtcaccaaag acgcgcagct cagcgcaccc accaagcagc tgtaccctgc    660
gtctgcattt cccgaggact tctccatcct aacaactgtg aaagccaaga aaggcagcca    720
ggccttcctg gtctccatct acaacgagca gggtatccag cagattgggc tggagctggg    780
ccgctctccc gtcttcctct acgaggacca cacggggaag cctggcccgg aagactaccc    840
cctcttccgg ggcatcaacc tgtcagatgg caagtggcac agaattgctc tcagcgtcca    900
caagaaaaat gtcaccttga tcctcgactg taaaaagaag accaccaaat tcctcgaccg    960
cagcgaccac cccatgatcg acatcaatgg catcatcgtg tttggcaccc ggatcctgga   1020
tgaggaggtg tttgagggtg acatccagca gctgctcttt gtctcggacc accgggcagc   1080
ttatgattac tgtgagcact acagccctga ctgtgacacc gcagtacctg acaccccaca   1140
gtcgcaggac cccaatccag atgaatatta cacggaagga gacggcgagg gtgagaccta   1200
ttactacgaa taccccctact acgaagaccc cgaagaccta gggaaggagc ccacccccag  1260
caagaagccc gtggaagctg ccaaagaaac cacagaggtc cccgaggagc tgaccccgac   1320
ccccacggaa gctgctccca tgcctgaaac cagtgaaggg gctgggaagg aagaggacgt   1380
cggcatcggg gactatgact acgtgcccag tgaggactac tacacgccct caccgtatga   1440
tgacctcacc tatggcgagg gggaggagaa ccctgaccag cccacagacc caggcgctgg   1500
ggccgaaatt cccaccagca ccgccgacac ctccaactcc tccaatccag ctccgcctcc   1560
aggggaaggt gcggatgact tggaggggga gttcactgag gaaacgatcc ggaaccttga   1620
cgagaactac tacgacccct actacgaccc caccagctcc ccgtcggaga tcgggccggg   1680
aatgccggcg aaccaggata ccatctatga agggattgga ggacctcggg gcgagaaagg   1740
ccaaaaggga gaaccagcga ttatcgagcc gggcatgctc atcgagggcc cgcctggccc   1800
agaaggcccc gcgggtcttc ccggacctcc aggaaccatg ggtcccactg gccaagtcgg   1860
ggaccctgga gaaaggggcc cccctggacg cccaggcctt cctggggccg atggcctgcc   1920
cggtcctcca ggaaccatgc tcatgctgcc cttccggttt ggaggtggcg gcgatgcggg   1980
ctccaaaggc cccatggtct cagcccagga gtcccaggcg caagccattc tccagcaggc   2040
caggttggca ctgaggggac cagctggccc gatgggtctc acagggagac ctggccctgt   2100
gggtccccct gggagcggag gtttgaaggg cgagccggga gacgtggggc ctcaggtcc    2160
tcgaggtgtg caaggcccgc ctggtccggc cgggaagccc ggaagacggg gtcggggctgg  2220
gagtgatgga gccagaggaa tgcctggaca aactggcccc aagggtgacc ggggtttcga   2280
cggcctggct gggttgccag gcgagaaggg ccacaggggt gaccctggtc cttccggccc   2340
accaggacct ccgggagacg atggagaaag ggtgacgac ggagaagttg ggcccagggg    2400
gctgcctggg gagcccgggc cacgtggtct gcttggccg aaagggcccc caggtcctcc    2460
cggacctccc ggtgtcacgg gtatggacgg ccagccgggg ccaaaaggaa atgtgggtcc   2520
ccagggagag cctggccccc caggacagca gggtaatcca ggcgcccagg gtcttccagg   2580
cccccagggt gcaattggtc ctccaggaga aaagggtccc ttggggaaac caggccttcc   2640
aggaatgccc ggtgctgacg gacccccggg acaccctggc aaagaaggcc ctccaggaga   2700
gaaaggaggt cagggtccac ctggccccca gggtccgatt ggctacccag gtcctcgagg   2760
agtcaagggg gccgatggca tccgtggtct gaagggcaca aagggcgaga agggtgaaga   2820
cggctttcct gggtttaaag gagacatggg catcaagggt gatcgggggg agatcggccc   2880
acccggtccc aggggagaag atggccctga aggcccaaag ggtcgcggag gtcccaatgg   2940
tgaccccggt cctctgggac cccctgggga gaagggaaaa ctcggagtcc cagggttacc   3000
agggtatcca ggaagacaag gaccaaaggg ctctattgga ttccctggat ttcctggcgc   3060
caatggagag aagggcggca gggggacccc tggaaagcca ggaccgcggg ggcagcgagg   3120
cccaacgggt ccgaggggtg aaagaggccc ccggggcatc actgggaagc ctggccccaa   3180
gggcaactcc ggaggtgacg gcccagctgg ccctcctggt gaacggggac ccaatggacc   3240
ccaaggaccc acaggatttc ctggaccaaa gggcccccct ggccctccag gcaaggatgg   3300
actcccagga caccctggac agagaggcga gactggtttc caaggcaaga ccggccctcc   3360
aggcccccccc ggcgtggtcg gccctcaggg tcccacggga gaaacgggcc caatgggtga  3420
gcgtggccac cctgggcccc ctggaccccc cggtgaacag gggcttccgg gccttgctgg   3480
aaaagaaggg acgaagggtg acccaggccc tgcaggcctc cctgggaaag atggccctcc   3540
aggattacgt ggtttccctg ggaccgaggg gcttcctggt ccagtgggag ctcttggact   3600
gaaaggcaat gaagggcccc ctggcccacc aggccctgcg ggatctccag ggagagagg    3660
tccagctgga gccgctgggc ccatcggaat tccagggaga cctgggcccc agggaccccc   3720
agggccggca ggagagaaag ggctcctgg cgagaaaggc ccacaaggcc cagctggccg    3780
agacggtctc caggggcctg tggggctccc gggtccagct ggccctgtgg gtccccctgg   3840
agaagacgga gataagggag agatcgggga gccggggcag aaaggaagca aggggacaa    3900
aggagaacag ggtcctcctg ggcctacagg tcctcaaggc cccatcggac agccaggccc   3960
ctctggagct gacggcgagc cggggcctcg ggccagcag ggcctttcg ggcagaaagg     4020
tgatgaaggt cccagaggct ttcctggacc ccctgggcca gtggggctgc agggtttgcc   4080
aggacctcca ggcgagaagg gtgagacagg agacgtgggc cagatgggcc ccccgggtcc   4140
ccctggcccc cgaggaccct ccggagctcc aggtgctgat ggcccacaag gtcccccagg   4200
tggaatagga aaccctggtg cagtgggaga gaagggcgag cctggcgaag caggtgagcc   4260
tggcccttcc gggagaagcg gcccccccggg acccaaagga gaaaggggag agaaggcgga 4320
```

gtcaggccct tcaggtgctg ccggaccccc tggacccaaa ggccctcccg gagatgatgg 4380
tcccaaaggc agccctggcc cagtgggttt tcctggagat cctggccccc ccggagagcc 4440
tggccccgcg ggtcaagatg gtccccctgg tgacaaagga gatgatggtg aacccgggca 4500
gacgggatcc cccggcccta ctggtgaacc aggtccatcg ggcctccag gaaaaagggg 4560
tcccccaggc cccgcaggcc ccgaaggcag acagggagag aaaggggcca agggagaagc 4620
cggcttggaa ggccctcctg ggaagactgg ccccatcggc ccccaggggg cccctgggaa 4680
gcccggaccg gatggccttc gagggatccc tggccctgtg ggagaacaag gtctcccagg 4740
atccccaggc ccggacggtc cccccggccc catgggtccc ccaggacttc ccggcctcaa 4800
aggagattct ggtcccaaag gtgaaaaggg tcatccaggc ctgatcgggc tcatcggtcc 4860
tccgggtgaa cagggtgaga agggcgaccg tggtctccct ggcccccagg gctcctccgg 4920
tcctaaggga gaacagggta tcactggtcc ttctggcccg attgggcctc ctgggccccc 4980
tggcctgccg ggtccgcctg gtccaaaagg tgctaagggc tcctcgggtc caactggccc 5040
gaagggtgag gcaggccacc caggaccccc aggcccccg ggcccccgg gagaggtcat 5100
ccagcccctg ccaatccagg catccaggac gcggcggaac atcgacgcca gccagctgct 5160
ggacgacggg aatggcgaga actacgtgga ctacgcggac ggcatggaag agatcttcgg 5220
ctctctcaac tctctgaagc tggagattga gcagatgaaa cggcccctgg gcacgcagca 5280
gaaccccgcc cgcacctgca aggacctgca gctctgccac cccgacttcc cagatggtga 5340
atactgggtc gatcctaacc aaggatgctc cagggattcc ttcaaggttt actgcaactt 5400
cacagccggg ggtcgacat gcgtcttccc tgacaagaag tccgaagggg ccagaatcac 5460
ttcttggccc aaagaaaacc cgggctcctg gttcagtgaa ttcaagcgtg ggaaactgct 5520
ctcctatgtg gacgccgagg gcaaccctgt gggtgtggta cagatgacct tcctgcggct 5580
gctgagcgcc tctgcccacc agaacgtcac ctaccactgc taccagtcag tggcctggca 5640
ggacgcagcc acgggcagct acgacaaggc cctccgcttc ctgggctcca acgacgagga 5700
gatgtcctat gacaacaacc cctacatccg cgccctggtg gacggctgtg ctaccaagaa 5760
aggctaccag aagacggttc tggagatcga cacccccaaa gtggagcagg tgcccatcgt 5820
ggacatcatg ttcaatgact tcggtgaagc gtcacagaaa tttggatttg aagtggggcc 5880
ggcttgcttc atgggctagg agccgccgag cccgggctcc cgagccgaat tcagcaacct 5940
cgtacctcag catgccattg cttcgtgagt gtcccgtgca cgtcctgatc ctggacagtg 6000
aaggcttctc cctcccctcc cacctgactt catctacgcc tcggcaccac ggggtgtggg 6060
accccagccc ggagagaaca gagggaagga gccgcgcccc cacctggagc tgaatcacat 6120
gacctagctg caccccagcg cctgggcccg ccccacgctc tgtccacacc catgcgcccc 6180
gggagcgggg ccatgcctcc agcccccag ctcgcccgac cccatcctgtt cgtgaatagg 6240
tctcaggggt tgggggaggg actgccagat ttggacacta tattttttc taaattcaac 6300
ttgaagatgt gtatttcccc tgaccttcaa aaaatgttcc aaggtaagcc tcgtaaaggt 6360
catcccacca tcaccaaagc ctccgttttt aacaacctcc aacacgatcc atttagaggc 6420
caaatgtcat tctgcaggtg ccttcccgat ggattaaagg tgcttatgtt tttgtgagtt 6480
ttaagtaaat atttgt 6496

<210> 102
<211> 1216
<212> DNA
<213> Homo sapiens
<400> 102
ttcggcactt gggagaagat gtttgaaaaa actgactctg ctaatgagcc tggactcaga 60
gctcaagtct gaactctacc tccagacaga atgaagttca tctcgacatc tctgcttctc 120
atgctgctgg tcagcagcct ctctccagtc caaggtgttc tggaggtcta ttacacaagc 180
ttgaggtgta gatgtgtcca agagagctca gtctttatcc ctagacgctt cattgatcga 240
attcaaatct tgccccgtgg gaatggttgt ccaagaaaag aaatcatagt ctggaagaag 300
aacaagtcaa ttgtgtgtgt ggaccctcaa gctgaatgga tacaaagaat gatggaagta 360
ttgagaaaaa gaagttcttc aactctacca gttccagtgt ttaagagaaa gattccctga 420
tgctgatatt tccactaaga acacctgcat tcttccctta tccctgctct ggattttagt 480
tttgtgctta gttaaatctt ttccagggag aaagaacttc cccatacaaa taaggcatga 540
ggactatgtg aaaaataacc ttgcaggagc tgatggggca aactcaagct tcttcactca 600
cagcacccta tatacacttg gagtttgcat tcttattcat cagggaggaa agtttctttg 660
aaaatagtta ttcagttata agtaatacag gattattttg attatatact tgttgtttaa 720
tgtttaaaat ttcttagaaa acaatggaat gagaatttaa gcctcaaatt tgaacatgtg 780
gcttgaatta agaagaaaat tatggcatat attaaaagca ggcttctatg aaagactcaa 840
aaagctgcct gggaggcaga tggaacttga gcctgtcaag aggcaaagga atccatgtag 900
tagatatcct ctgcttaaaa actcactacg gaggagaatt aagtcctact tttaaagaat 960
ttctttataa aatttactgt ctaagattaa tagcattcga agatccccag acttcataga 1020
atactcaggg aaagcattta aagggtgatg tacacatgta tcctttcaca catttgcctt 1080
gacaaacttc tttcactcac atctttttca ctgacttttt ttgtgggggc ggggccgggg 1140
ggactctggt atctaattct ttaatgattc ctataaatct aatgacattc aataaagttg 1200
agcaaacatt ttactt 1216

<210> 103
<211> 1197
<212> DNA
<213> Homo sapiens

<400> 103
acacatatct cagcaggtta agagaaacgg ggagggaagg gcagctgcaa agttcccagg 60
agtaaagggg cggtgggagt gtgctcgggc agcacagggg agggaagatt aaggcacagg 120
tgcgcggggc ctcagacggc ccagggggagg ggtgtggaaa cctcccctct cagatgcagc 180
tggtgagtgg ctggcgaggg ggcccacggc aaagacccct cttggcaact gtgagtcccc 240
tcatctcact gcgcagtggt aatggaggcg tctcaggcag ggttcctcgt agaggtcgg 300
gggtctcaca gccccatggg ccccgatcac gggccgggcc tcgggagcag gggtgctcag 360
caacgggggc aggcccggcc ggctggtgct cgcggggatg ctgggtccgc gggggcgtgg 420
agccgggtcg gctgggtgcg cgcatgctgc ggagcttcgg gctgggaagg ccacgttggt 480
gcagaagagg ccgaggcagc agctggcgct ggcactcctt cccactgggc cagagcgtcc 540
cccagcgaga ggtagtatgc gcatccaagc cgggcagtga ctactctgta caggcggggc 600
aggacagcag gctacggaca gggcagctac ttggacgcgg gagcagttcc aatcctgatc 660
cgagtagcct ggcacgggcg agtgccttgc caaggttcct cgtgtgcgcg gcataaccag 720
gtaatggcag atggtgcccg tctgctccct tcctccaagg ggattgtcct ccggaaccgt 780
cgtgggcaat acaatccagc gaggcggatg gatgaaggca aaaagggaca ttaaggggga 840
gagatgatag ggcgcactat gtgacgtggt ccatgagcgc acagcacacc attcgatcaa 900
ggatgagaca cggagggcca aggggcacaa tcgaaggcca aaaagagggg aacaaacgaa 960
ggaccgggac agggccaggc cccccgcacg gacacatagc aagggagcga ggaggaggaa 1020
cgcccgacta ggagagcacc ggggaggagg acaggacaag ggcaaaagcg ggtgcagcag 1080
aaagagacag ggcgcgagca agcaagaagc gagagagaga agaggcagag gaagaaaggc 1140
aggaacgcgg agcggcaaca atgagcgggg ataacgaccg agcacacaaa tcagtgg 1197

<210> 104
<211> 707
<212> DNA
<213> Homo sapiens
<220>
<221> misc_feature
<222> (513)..(513)
<223> n = a, t, g, or c
<220>
<221> misc_feature
<222> (553)..(553)
<223> n = a, t, g, or c
<220>
<221> misc_feature
<222> (562)..(562)
<223> n = a, t, g, or c
<220>
<221> misc_feature
<222> (631)..(631)
<223> n = a, t, g, or c
<220>
<221> misc_feature
<222> (670)..(670)
<223> n = a, t, g, or c
<220>
<221> misc_feature
<222> (679)..(679)
<223> n = a, t, g, or c
<220>
<221> misc_feature
<222> (696)..(696)
<223> n = a, t, g, or c
<400> 104
ttttactgga aggctgtatt tatgctaaac ctttattact tttagaaagc tgtacacttt 60
tttaaaaaat ttgcacttat aaataataga aaaaatatta gaatcaaaat ttcgtcatta 120
cagatgggaa aatatgtact ataaggaatt caagttaaca gaggcttgat ttatataaaa 180
gaaagctgca gttttaaagt tgtgttcctt aaagaccaga ttatagctta tctgaatggg 240
cttgacactt tcaaatggaa taaagttaca atattaaaca gattagggta agaaaatcta 300
aatctggcac atctctatta tttgacagtg tttaaccaaa tacatcatac agaatagaag 360
gtgagtgcca ggcccctgag gaggagctct cgtgttccac agcaaagcgc tgcccaagga 420
ctgcgggaag tgatccagct gccttcacac ggaggacacg agactgcttc ctcaagggct 480
cctgcctgcc tggacactgg tgggaggcgc tgnttagttg gctggtttca gaggggtctt 540
tcggagggac ctnctgctgc angctgaagt gtctttattc ctggcgggag accgcacatt 600
ccactgctga agctgtgggg gcggttatca ngcagtgata aaccatagat gtcatttcct 660
tgactccggn cttcatttnt ctctttgctg acgacngagt cccgggg 707

<210> 105

<211> 1656
<212> DNA
<213> Homo sapiens
<400> 105
atgcccaccc ctggggcagc ctccctgttg tcggctgtgc aagcccaaga acgcagactt 60
tatgtacctc tccctggagg cttctcggtg cctccagtga ccccgatgcc tcactgcctt 120
ctggacccca aagaggatct aaagggctgt ccacaggctt gctctggctg tagcatggga 180
acccctgctc cttcttatct ggtgcaagga gcccagggga tgcctttaag gaaggccgca 240
tccttagcaa ctaagttgga agaaatgggg ctctttggcc agcaatgtct tcccgccatg 300
ggctataccct gcaggtgccg ggagccactt ctgcttccat cccacctgat tggagtgggc 360
ttcccacagg cccccgctcc gcagctcctc agtgctctcc tcctcctccc tgctcccccg 420
agcccagccc agtctgaggt ccatcccccct gtgaggggag ctgagcagtg ctgcaaactc 480
agtgctcctg agtaccctgg ggatttgctg aactgcacat tctcattctg taaaccggag 540
ctgagcctct gcacgtctaa cctgctccct ggcaatgctg ctgttactga gcagagtgga 600
aaagtgctag acaatacatt ggggggaaat ttcagtctca aatgcattgg agcagaaacc 660
agaactccct gtcaagctgc tctgtgggtt tttgaagcga aggtctccag gctcatgaaa 720
tctgcctctt gccaaaggca gaggatccac tggcagcagg gagcactggc tgcagtcagg 780
cagggagatt tagggcttaa atacaaggaa atttcatgta aggatgcagg agtgtttcat 840
ggaacctcaa gctcaggaaa gctgcacagc catgggaaga ccctagagcc ggagtctgga 900
aaagcaatcc gagcttggat cttagaaatc ttgtcttctt gcaaaccggg ttctctggtc 960
caaatggcca aaaacatgac catggacagg gcctgtgctt atctcgtcac ctgcagagag 1020
gcaactctct ggtggggagt agttgcaggt agtcttgttt atgaacatgg caatcagaag 1080
cccatccctg tcactgtgtg gaggggaggg gagagaagag ggatggttcc aaccaaaaaa 1140
gatacatcca catctgtttt gttcatccag atacacgtgc taacattttc agctgagaat 1200
aaaattcaga gcaactgtaa ctcttcctct tgcatcagac caaccggcag aagggaacgg 1260
gatgtcacaa accagactca cgcatctgga aaacgttgtg tggctgggct ggctccagaa 1320
ggaagcgacg agggccttct accggccaca ctcccttgct tgggcttccc taagctgctt 1380
tctgcagagg aaagggccaa tcacggccag ccaaggaata caaggccttg tcagctggac 1440
cttgactgcg ctatattcgg ccacttccac acattgccta acaagcttct tctggtcagt 1500
gtgtctgaga tcctcacctc agaaattctc acattagcaa acctactcca aggatgccag 1560
catgaacctg aacggtatgg tcacgttaca gatccagaac acaaaacgac ttccgctttt 1620
acagtttcaa aggcctcaag gtggagaaaa aactga 1656

<210> 106
<211> 566
<212> DNA
<213> Homo sapiens
<400> 106
ttttgggatg cttcactttc tttattgccc atccagggga cagccaagcc agctccatct 60
gcattctggc tgcagcgtgt acattagggg actcaggggc cacagtgtgg gaccgtgcac 120
actggcaagg cactggcgga tgctggcagg ccagtggaca tggatagatg agaatgacaa 180
ctcacagatg tcctagcttc cgctggccca gctgccagcc actggccatc accctttgc 240
ccagcatgtg tgcattgtca cccaaaacat cttgaaactt gccattagtg aggcattcaa 300
caaagaagta agctaagtga gtaggaaaca gtgtttcctg gaatataccg cactctgcct 360
gaaataggaa aactatgttt gccgggaagc agcagcagca ggaaagaagt tataccaaaa 420
acgacttgta caccacagac attataaccc tttcctcaaa gaaacagtca tgttctgttg 480
ggtattatgg acaggtctct ggaaatttat ctaataaaga ccaacaaact tccccagcag 540
tgcctctgag taccgtgtga attctg 566

<210> 107
<211> 3088
<212> DNA
<213> Homo sapiens
<400> 107
ttgcttgagt catcttctga agctttaaaa acaattgatg aattggcctt caagatagac 60
ctaaatagca catcacatgt gaatattaca actcggaact tggctctcag cgtatcatcc 120
ctgttaccag ggacaaatgc aatttcaaat tttagcattg gtcttccaag caataatgaa 180
tcgtatttcc agatggattt tgagagtgga caagtggatc cactggcatc tgtaattttg 240
cctccaaact tacttgagaa tttaagtcca gaagattctg tattagttag aagagcacag 300
tttacttttct tcaacaaaac tggacttttc caggatgtag gaccccaaag aaaaacttta 360
gtgagttatg tgatggcgtg cagtattgga aacattacta tccagaatct gaaggatcct 420
gttcaaataa aaatcaaaca tacaagaact caggaagtgc atcatcccat ctgtgccttc 480
tgggatctga acaaaaacaa aagttttgga ggatggaaca cgtcaggatg tgttgcacac 540
agagattcag atgcaagtga gacagtctgc ctgtgtaacc acttcacaca ctttggagtt 600
ctgatggacc ttccaagaag tgcctcacag ttagatgcaa gaaacactaa agtcctcact 660
ttcatcagct atattgggtg tggaatatct gctatttttt cagcagcaac tctcctgaca 720
tatgttgctt ttgagaaatt gcgaagggat tatccctcca aatcttgat gaacctgagc 780
acagccctgc tgttcctgaa tctcctcttc ctcctagatg gctggatcac ctccttcaat 840
gtggatggac tttgcattgc tgttgcagtc ctgttgcatt tcttccttct ggcaaccttt 900

```
acctggatgg ggctagaagc aattcacatg tacattgctc tagttaaagt atttaacact    960
tacattcgcc gatacattct aaaattctgc atcattggct ggggtttgcc tgccttagtg   1020
gtgtcagttg ttctagcgag cagaaacaac aatgaagtct atggaaaaga aagttatggg   1080
aaagaaaaag gtgatgaatt ctgttggatt caagatccag tcatatttta tgtgacctgt   1140
gctgggtatt ttggagtcat gtttttctg aacattgcca tgttcattgt ggtaatggtg    1200
cagatctgtg ggaggaatgg caagagaagc aaccggaccc tgagagaaga agtgttaagg   1260
aacctgcgca gtgtggttag cttgaccttt ctgttgggca tgacatgggg ttttgcattc   1320
tttgcctggg gacccttaaa tatccccttc atgtacctct tctccatctt caattcatta   1380
caaggcttat ttatattcat cttccactgt gctatgaagg agaatgttca gaaacagtgg   1440
cggcggcatc tctgctgtgg tagatttcgg ttagcagata actcagattg gagtaagaca   1500
gctaccaata tcatcaagaa aagttctgat aatctaggaa aatctttgtc ttcaagctcc   1560
attggttcca actcaaccta tcttacatcc aaatctaaat ccagctctac cacctatttc   1620
aaaaggaata gccacacaga taatgtctcc tatgagcatt ccttcaacaa aagtggatca   1680
ctcagacagt gcttccatgg acaagtcctt gtcaaaactg gcccatgctg atggagatca   1740
aacatcaatc atccctgtcc atcaggtcat tgataaggtc aagggttatt gcaatgctca   1800
ttcagacaac ttctataaaa atattatcat gtcagacacc ttcagccaca gcacaaagtt   1860
ttaatgtctt taagaaaaag aaatcaatct gcagaaatgt gaagatttgc aagcagtgta   1920
aactgcaact agtgatgtaa atgtgctatt acctaggtaa ctgcatatat ataaggaatg   1980
tattttgtta agaaggcttt tgtgaaattc agaatttttc tttttaatat atttcttcca   2040
tggaagagtt gtcatcacta aaacttcagt actgagagta acatgactca gtagccacag   2100
aagctatgat ttgtaaaata tataattgaa tcagagtaat cataatgcag ggagagacatt  2160
caaattagag acaagggaga agcaatgctg aggaagaccc tagatagagc tcattttact   2220
ccacctaatc gttatatctg gatataccca ttttctgcat cttctttctc aacaataaac   2280
tgtccttgct ttggagactt taagacattt cctaaagcac aaataaaagc ctcgtatttc   2340
cccattgaga gttttgttcc aaggaatatg aagtgagaca tatgggtgag tcataataat   2400
caaaataatt tatgaagagc tgggtctgca atagctagtc taaaaactac ttgtgtgtca   2460
gtcctctggt tatagtatat aagagcctga ggaggtctgg caagatagat ggtgtattat   2520
ttatggatca ggctgctgca tacaaacctt gcatactatt atgcagctta cctaactctc   2580
agactattct gagtaatgct tgcttgctaa tgaatgtata ggagaccaca ttgtaattgt   2640
tcttagatga tggagtccat gcagtttctt agaaatcggt ctcagtgcat gctgtgcttt   2700
ttcacatttg ctctgggtta tctgggaagt atcaggttct gggaggcaac agcattaagt   2760
gataagaaaa ggagacattc tggcaaagcc aatctgctta aaggcaaagt ccagaacctg   2820
gaacctagag gcctttctct ctgcacgaaa aacaggtagt ttgcagtctg agatatggga   2880
gagcttttag gctacacagc aacccaaggg acctctcacc ttttgctgag cttcaatcag   2940
gaagctattt gcctggctcc agcagatgat gagataatga ggtagtgggt tttttattac   3000
tgttccattt tgcaacatcc tgcaacacca tcctgggaga caagagcatt acccagcttg   3060
gctttcacgg gggagggttg tattcagt                                      3088
```

<210> 108
<211> 1542
<212> DNA
<213> Homo sapiens
<400> 108

```
cgaccggcac gttcacccca tccctcaggc tttatttatt ttttttcgac aggttctttt     60
caaggctcca gtcaccgcag cagttgtcca tgctgtagtt tccactttcc tgtatgggcg    120
ggctggttag gattccactt tcccccaagt gcttagccca gggccagaca aaaagtagtt    180
gcttaagaaa tacttgttga aggaataaat taatgaatga atttgtgctt acagcggctg    240
gatggcagac aaacctatga ttataggaac atcaggatct catttggaac agattacgga    300
tgctgcattg tggaacttgg aaaaacaaga gttcttggac aggtttcctg tgaacttgtg    360
tctccaaaac tcaatcgggc aacagaaggt attctttttt taaccttgaa ctctctcaga    420
tggccgctcc agctttcgaa cctggcaggc agtcagatct cttggtgaag ttgaatcgac    480
tcatggaaag atgtctaaga aattcgaagt gtatagacac tgagtctctc tgtgttgttg    540
ctggtgaaaa ggtttggcaa atacgtgtag acctacattt attaaatcat gatggaaata    600
ttattgatgc tgccagcatt gctgcaatcg tggccttatg tcatttccga agacctgatg    660
tctctgtcca aggagatgaa gtaacactgt atacacctga agagcgtgat cctgtaccat    720
taagtatcca ccacatgccc atttgtgtca gttttgcctt tttccagcaa ggaacatatt    780
tattggtgga tcccaatgaa cgagaagaac gtgtgatgga tggcttgctg gtgattgcca    840
tgaacaaaca tcgagagatt tgtactatcc agtccagtgg tgggataatg ctactaaaag    900
atcaagttct gagatgcagt aaaatcgctg gtgtgaaagt agcagaaatt acagagctaa    960
tattgaaagc tttggagaat gaccaaaaag taaggaaaga aggtggaaag tttggttttg   1020
cagagtctat agcaaatcaa aggatcacag catttaaaat ggaaaaggcc cctattgata   1080
cctcggatgt agaagaaaaa gcagaagaaa tcattgctga agcagaacct ccttcagaag   1140
ttgtttctac acctgtgcta tggactcctg gaactgccca aattggagag ggagtagaaa   1200
actcctgggg tgatcttgaa gactctgaga aggaagatga tgaaggcggt ggtgatcaag   1260
ctatcattct tgatggtata aaaatggaca ctggagtaga agtctctgat attggaagcc   1320
aagatgctcc cataatactc tcagatagtg aagaagaaga aatgatcatt ttggaaccag   1380
acaagaatcc aaagaaaata agaacacaga ccaccagtgc aaaacaagaa aaagcaccaa   1440
gtaaaaagcc agtgaaaaga agaaaaaaga agagagctgc caattaaagc taacagttgt   1500
atatctgtat atataactat taaaagggat atttattcca tt                      1542
```

<210> 109
<211> 5504
<212> DNA
<213> Homo sapiens
<400> 109

```
cacaaatctg tgattacctg gcgggtgaac cgctccggtg gcggggagag cgggctccca     60
gcgctgggta ggggccgggt tccggcgagc gccatcccgg agcgtcagtt tcccagtttg    120
ggaagtgagg agaacctgcc tcgcccttcc ccgccaaggc ttagggaagg gactatggca    180
gttccaagag gaaatcaggg tctcgctctg ttgctcaggc tggattgcag tggcgtgatc    240
atgcctcact gcagcctcga cctccctggg ctcaagcaat cctcccactt cagcctccag    300
agtggctggg accacagtgt ggctgtccag ctgggctgag tcgcccaaga aggacgtgac    360
aggtgccgac gccaccgccg agcccatgat cctggaacag tacgtggtgg tgtccaacta    420
taagaagcag gagaactcgg agctgagcct ccaggccggg gaggtggtgg atgtcatcga    480
gaagaacgag agcggctggt ggttcgtgag cacttctgag gagcagggct gggtccctgc    540
cacctacctg gaggcccaga atggtactcg ggatgactcc gacatcaaca cctctaagac    600
tggagaagag gagaagtatg tcaccgtgca gccttacacc agccaaagca aggacgagat    660
tggctttgag aagggcgtca cagtggaggt gatccggaag aatctggaag gctggtggta    720
tatcagatac ctgggcaaag agggctgggc gccagcatcc tacctgaaga aggccaagga    780
tgacctgcca acccggaaga agaacctggc cggcccagtg gagatcattg ggaacatcat    840
ggagatcagc aacctgctga acaagaaggc gtctggggac aaggaaactc caccagccga    900
aggcgagggc catgaggccc ccattgccaa gaaggagatc agcctgccca tcctctgcaa    960
tgcctccaat ggcagtgccg tgggcgttcc tgacaggact gtctccaggc tggcccaggg   1020
ctctccagct gtggccagga ttgccctca gcgggcccag atcagctccc cgaacctacg   1080
gacaagacct ccaccacgca gagaatccag cctggggttc caactgccaa agccaccaga   1140
gccccccttct gttgaggtgg agtactacac cattgccgaa ttccagtcgt gcatttccga   1200
tggcatcagc tttcggggtg gacagaaggc agaggtcatt gataagaact caggtggctg   1260
gtggtacgtg cagatcggtg agaaggaggg ctgggccccc gcatcataca tcgataagcg   1320
caagaagccc aacctgagcc gccgcacaag cacgctgacc cggcccaagg tgcccccgcc   1380
agcacccccc agcaagccca aggaggccga ggagggccct acgggggcca gtgagagcca   1440
ggactccccg cggaagctca agtatgagga gcctgagtat gacatccctg cattcggctt   1500
tgactcagag cctgagctga gcgaggagcc cgtggaggac agagcctcag gggagaggcg   1560
gcctgcccag ccccaccggc cctcgccggc ctcttctctg cagcgggccc gcttcaaggt   1620
gggtgagtct tcagaggatg tggccctgga agaggagacc atctatgaga atgagggctt   1680
ccggccatat gcagaggaca ccctgtcagc cagaggctcc tccggggaca gcgactcccc   1740
aggcagctcc tcgctgtccc tgaccaggaa aaactccccc aaatcaggct cccccaagtc   1800
atcatcactc ctaaagctca aggcagagaa gaatgcccag gcagaaatgg ggaagaacca   1860
ctcctcagcc tcctttttcct catccatcac catcaacacc acttgctgct cctcctcttc   1920
ctcctcctcc tcttccttgt ccaaaaccag tggcgacctg aagccccgct ctgcttcgga   1980
cgcaggcatc cgcggcactc ccaaggtcag ggcaaagaag gatgctgatg cgaacgctgg   2040
gctgacctcc tgtccccggg ccaagccatc ggtccggccc aagccattcc taaaccgagc   2100
agagtcgcag agccaagaga agatggacat cagcacttta cggcgccagc tgagacccac   2160
aggccagctc cgtggagggc tcaagggctc caagagtgag gattcggagc tgccccccgca   2220
gacggcctcc gaggctccca gtgaggggtc taggagaagc tcatccgacc tcatcaccct   2280
cccagccacc actccccccat gtcccaccaa gaaggaatgg gaagggccag ccacctcgta   2340
catgacatgc agcgcctacc agaaggtcca ggactcggag atcagcttcc ccgcgggcgt   2400
ggaggtgcag gtgctggaga agcaggagag cgggtggtgg tatgtgaggt ttggggagct   2460
ggagggctgg gccccttccc actatttggt gctggatgag aacgagcaac ctgacccctc   2520
tggcaaagag ctggacacag tgcccgccaa gggcaggcag aacgaaggca agtcagacag   2580
cctggagaag atcgagaggc gcgtccaagc actgaacacc gtcaaccaga gcaagaaggc   2640
cacgcccccc atcccctcca aacctcccgg gggctttggc aagacctcag gcactccagc   2700
ggtgaagatg aggaacggag tgcggcaggt ggcggtcagg ccccagtcgg tgtttgtgtc   2760
cccgccaccc aaggacaaca acctgtcctg cgccctgcgg aggaatgagt cactcacggc   2820
cactgatggc ctccgaggcg tccgacggaa ctcctccttt agcactgctc gctccgctgc   2880
cgccgaggcc aagggccgcc tggccgaacg ggctgccagc cagggttcag actcacccct   2940
actgcccgcc cagcgcaaca gcatccccgt gtcccctgtg cgccccaagc ccatcgagaa   3000
gtctcagttc atccacaata acctcaaaga tgtgtacgtc tctatcgcag actacgaggg   3060
ggatgaggag acagcaggct tccaggaggg ggtgtccatg gaggttctgg agaggaaccc   3120
taatggctgg tggtactgcc agatcctgga tggtgtgaag cccttcaaag gctgggtgcc   3180
ttccaactac cttgagaaaa agaactagca gagggcctgg gctcttccag cctcagtgtg   3240
cctctctggc cgcccactgg atgagcggtg agacgaacaa aagggaaagg aaaaaatggg   3300
ggtggggggt gggggggtgga caacattcaa cactgcagaa tgggtgacct caaagatgcc   3360
ccctgtccaa gccatcccac agctggaagg taggggatgg gggtgcccac actgagtgag   3420
gaagggaatg gaccagggag tcccaggcct gggacccaga gccaagaaag ctgagatatc   3480
ctgtgcacca tagggacttc accaatggat tacatgccat ctgggacagg ccatgtggga   3540
gaccccagtt gtgcctttgc tacagatctg gaaaagacaa ggtcatgggg gcctccagtg   3600
tcctgcccct gcttggccca gttttgattg ctggcatctt gccaccccag gtatccctgg   3660
tattgtccta agctgtattt gtgaattgtg ctggtttcct gggcattgcc acgcctacca   3720
caggtgggta cattagaagc caccactggc tttcaggctt gggggtgtct tctgagctca   3780
```

agcctgcttc tgggccaggc cattgtcact gttagttgaa gaaaaagcag ttcccaggtg 3840
ccagcaaaga ccatctttca taactgtcac tgtcttggcc ttgagaagag agcccgctct 3900
ccgtggggca ccccatggag gacacagtac cagagtttac agagagggtg ggcgaagcca 3960
ccggtctctt cctaatctgc acagactatt ttgggtattt ctgggcgggc agttcctttg 4020
catgtttcgg gagaggtttg ttgatttggg gcttatatgt caggcctttg gtttgcgtct 4080
tattttaggg gttgtttggg ggcctgggtg gtcggcctca catgggaagg agatgggtag 4140
tggatggggt ttctgttgta tcttgtgggc gggtgatttt gcttttgttt ttgtttcaca 4200
ttcttccccc tccacaagcc aaagtcgttt catttggttt ccactgtgtg gactgtgctg 4260
gagcttggcg cctgccagaa aaatttgggg ctaggcaagc cccaggttgc agacatggtg 4320
aagcagagaa actgttcttc tggttcctgc acaacctcag aggggcaaaa accctcccca 4380
ggaaggagga gggtgttcag gagccagact tttggagaga aggcagctcc cagcctgctg 4440
ggtgaccgcc attctgcgtg tgttccccag ctgggcaggg ctggaagcct tacgtatgaa 4500
gcatggagaa gcagccattg tccccactat gggcagaggg gggacccggc tggccccttg 4560
ggtcagactg gagccaacac cgccagccac cccctctggc ctgctggcaa tgccacaggt 4620
gcccaagaag atggaggatc cctgtgccag gagccaacct ggtcttcccg agggtcagtg 4680
ccccagtgaa gacagaagcg agagaataaa gttccctgta ggtcctctgt cacctttggg 4740
ttgtgttttt caattgttga catttcagag gggaccctcc agaagcccag ccggcttccc 4800
ccaaggactc cccctttcgct gggagtggat ttccacacgt gcctttgatt tcggacagat 4860
tgggcctcac agccaccgat tcagctgcca gggtccctgg actgggggtt ggtgttttct 4920
atagaggagg aaaggccctc cctcaccctg ctccccaccc aggcagggca gcatgggacc 4980
cagtgtctca gtgccttcaa aacccacccc cacccctacc ctaccccacc acaccccatc 5040
ccagaggcct tgcctgggca accctaagcc cctgtccctc gccatacact gatgcctggc 5100
agctagagca aatggctcgt gttctttgtc gaaggcctgt ggtgagattg ttttgtttcc 5160
ttttgttttg tgagtttgtt taaaattgaa attagttatt ttcttctgct ggacagtatt 5220
aaatagagca ggatgttgag ttaatctgct agattgcagt actaatggta gtggtttagt 5280
gtcttcatgt taatattatt tgtacttatt tgaacaataa tgataaagaa gtggttcatt 5340
attttttaat taatgcactt taaataaggt agaatggaaa aaacccagag agcaaagtgc 5400
attacttaaa gatgcagtat atacttttct catttttaaa cagcacatat ttattaagag 5460
aaaaaaagta atttatgact atttaaaata aaatttaaaa gtag 5504

<210> 110
<211> 5699
<212> DNA
<213> Homo sapiens
<400> 110

ggagttctca gacctccagt ttcagccctg ccctcagcct ccaatccgta agagacaccc 60
agccccagca attggattgg gcagcccgtc ttgacacacc actgtgctga gtgcttgagg 120
acgtgtttca acagatggtt ggggttagtg tgtgtcatca cattcgagtg gggattaaga 180
gaaggaaggc tgccttgctg gagctgtgtg gtcttctcca agtgagagtc gcaggcaata 240
gaactacttt gcttttggag gaaaaggagg aattcatttt cagcagacac aagaaaagca 300
gtttttttttt cagggattct tcacttctct tgaacaagga actcactcag agactaacac 360
aaaggaagta atttcttacc tggtcattat ttagtctaca ataagttcat ccttcttcag 420
tgtgaccagt aaattcttcc catactcttg aagagagcat aattggaatg gagaggtggt 480
gctgacggcc acccaccatc atctaaagaa gataaacttg gcaaatgaca tgcaggttct 540
tcaaggcaga ataattgcag aaaatcttca aaggacccta tctgcagatg ttctgaatac 600
ctctgagaat agagattgat tattcaacca ggatacctaa ttcaagaact ccagaaatca 660
ggagacggag acattttgtc agttttgcaa cattggacca aatacaatga agtattcttg 720
ctgtgctctg gttttggctg tcctgggcac agaattgctg ggaagcctct gttcgactgt 780
cagatccccg aggttcagag gacggataca gcaggaacga aaaaacatcc gacccaacat 840
tattcttgtg cctaccgatg atcaagatgt ggagctgggg tccctgcaag tcatgaacaa 900
aacgagaaag attatggaac atgggggggc caccttcatc aatgcctttg tgactacacc 960
catgtgctgc ccgtcacggt cctccatgct caccgggaag tatgtgcaca atcacaatgt 1020
ctacaccaac aacgagaact gctcttcccc ctcgtggcag gccatgcatg agcctcggac 1080
ttttgctgta tatcttaaca acactggcta cagaacagcc tttttttggaa aatacctcaa 1140
tgaatataat ggcagctaca tcccccctgg gtggcgagaa tggcttggat taatcaagaa 1200
ttctcgcttc tataattaca ctgtttgtcg caatggcatc aaagaaaagc atggatttga 1260
ttatgcaaag gactacttca cagacttaat cactaacgag agcattaatt acttcaaaat 1320
gtctaagaga atgtatcccc ataggcccgt tatgatggtg atcagccacg ctgcgcccca 1380
cggccccgag gactcagccc cacagttttc taaactgtac ccaatgctt cccaacacat 1440
aactcctagt tataactatg caccaaatat ggataaacac tggattatgc agtacacagg 1500
accaatgctg cccatccaca tggaatttac aaacattcta cagcgcaaaa ggctccagac 1560
tttgatgtca gtggatgatt ctgtggagag gctgtataac atgctcgtgg agacggggga 1620
gctggagaat acttacatca tttacaccgc cgaccatggt taccatattg ggcagtttgg 1680
actggtcaag ggggaaatcca tgccatatga ctttgatatt cgtgtgcctt tttttattcg 1740
tggtccaagt gtagaaccag gatcaatagt cccacagatc gttctcaaca ttgacttggc 1800
cccccacgatc ctggatattg ctgggctcga cacacctcct gatgtggacg gcaagtctgt 1860
cctcaaactt ctggacccag aaaagccagg taacaggttt cgaacaaaca agaaggccaa 1920
aatttggcgt gatacattcc tagtggaaag aggcaaattt ctacgtaaga aggaagaatc 1980
cagcaagaat atccaacagt caaatcactt gcccaaatat gaacgggtca aagaactatg 2040

ccagcaggcc aggtaccaga cagcctgtga acaaccgggg cagaagtggc aatgcattga 2100
ggatacatct ggcaagcttc gaattcacaa gtgtaaagga cccagtgacc tgctcacagt 2160
ccggcagagc acgcggaacc tctacgctcg cggcttccat gacaaagaca aagagtgcag 2220
ttgtagggag tctggttacc gtgccagcag aagccaaaga aagagtcaac ggcaattctt 2280
gagaaaccag gggactccaa agtacaagcc cagatttgtc catactcggc agacacgttc 2340
cttgtccgtc gaatttgaag gtgaaatata tgacataaat ctggaagaag aagaagaatt 2400
gcaagtgttg caaccaagaa acattgctaa gcgtcatgat gaaggccaca aggggccaag 2460
agatctccag gcttccagtg gtggcaacag gggcaggatg ctggcagata gcagcaacgc 2520
cgtgggccca cctaccactg tccgagtgac acacaagtgt tttattcttc ccaatgactc 2580
tatccattgt gagagagaac tgtaccaatc ggccagagcg tggaaggacc ataaggcata 2640
cattgacaaa gagattgaag ctctgcaaga taaaattaag aatttaagag aagtgagagg 2700
acatctgaag agaaggaagc ctgaggaatg tagctgcagt aaacaaagct attacaataa 2760
agagaaaggt gtaaaaaagc aagagaaatt aaagagccat cttcacccat tcaaggaggc 2820
tgctcaggaa gtagatagca aactgcaact tttcaaggag aacaaccgta ggaggaagaa 2880
ggagaggaag gagaagagac ggcagaggaa gggggaagag tgcagcctgc ctggcctcac 2940
ttgcttcacg catgacaaca accactggca gacagccccg ttctggaacc tgggatcttt 3000
ctgtgcttgc acgagttcta acaataacac ctactggtgt ttgcgtacag ttaatgagac 3060
gcataatttt cttttctgtg agtttgctac tggctttttg gagtattttg atatgaatac 3120
agatccttat cagctcacaa atacagtgca cacggtagaa cgaggcattt tgaatcagct 3180
acacgtacaa ctaatggagc tcagaagctg tcaaggatat aagcagtgca acccaagacc 3240
taagaatctt gatgttggaa ataaagatgg aggaagctat gacctacaca gaggacagtt 3300
atgggatgga tgggaaggtt aatcagcccc gtctcactgc agacatcaac tggcaaggcc 3360
tagaggagct acacagtgtg aatgaaaaca tctatgagta cagacaaaac tacagactta 3420
gtctggtgga ctggactaat tacttgaagg atttagatag agtatttgca ctgctgaaga 3480
gtcactatga gcaaaataaa acaaataaga ctcaaactgc tcaaagtgac gggttcttgg 3540
ttgtctctgc tgagcacgct gtgtcaatgg agatggcctc tgctgactca gatgaagacc 3600
caaggcataa ggttgggaaa acacctcatt tgaccttgcc agctgacctt caaaccctgc 3660
atttgaaccg accaacatta agtccagaga gtaaacttga atggaataac gacattccag 3720
aagttaatca tttgaattct gaacactgga gaaaaaccga aaaatggacg gggcatgaag 3780
agactaatca tctggaaacc gatttcagtg gcgatggcat gacagagcta gagctcgggc 3840
ccagccccag gctgcagccc attcgcaggc acccgaaaga acttccccag tatggtggtc 3900
ctggaaagga catttttgaa gatcaactat atcttcctgt gcattccgat ggaatttcag 3960
ttcatcagat gttcaccatg gccaccgcag aacaccgaag taattccagc atagcgggga 4020
agatgttgac caaggtggag aagaatcacg aaaaggagaa gtcacagcac ctagaaggca 4080
gcgcctcctc ttcactctcc tctgattaga tgaaactgtt accttaccct aaacacagta 4140
tttcttttta actttttttat ttgtaaacta ataaaggtaa tcacagccac caacattcca 4200
agctaccctg ggtacctttg tgcagtagaa gctagtgagc atgtgagcaa gcggtgtgca 4260
cacggagact catcgttata atttactatc tgccaagagt agaaagaaag gctggggata 4320
tttgggttgg cttggttttg atttttttgct tgtttgtttg ttttgtacta aaacagtatt 4380
atcttttgaa tatcgtaggg acataagtat atacatgtta tccaatcaag atggctagaa 4440
tggtgccttt ctgagtgtct aaaacttgac accccctggta aatctttcaa cacacttcca 4500
ctgcctgcgt aatgaagttt tgattcattt ttaaccactg gaatttttca atgccgtcat 4560
tttcagttag atgatttttgc actttgagat taaaatgcca tgtctatttg attagtctta 4620
tttttttatt tttacaggct tatcagtctc actgttggct gtcattgtga caaagtcaaa 4680
taaaccccca aggacgacac acagtatgga tcacatattg tttgacatta agcttttgcc 4740
agaaaatgtt gcatgtgttt tacctcgact tgctaaaatc gattagcaga aaggcatggc 4800
taataatgtt ggtggtgaaa ataaataaat aagtaaacaa aatgaagatt gcctgctctc 4860
tctgtgccta gcctcaaagc gttcatcata catcatacct ttaagattgc tatattttgg 4920
gttattttct tgacaggaga aaaagatcta aagatctttt attttcatct tttttggttt 4980
tcttggcatg actaagaagc ttaaatgttg ataaaatatg actagttttg aatttacacc 5040
aagaacttct caataaaaga aaatcatgaa tgctccacaa tttcaacata ccacaagaga 5100
agttaatttc ttaacattgt gttctatgat tatttgtaag accttcacca agttctgata 5160
tcttttaaag acatagttca aaattgcttt tgaaaatctg tattcttgaa aatatccttg 5220
ttgtgtatta ggtttttaaa taccagctaa aggattacct cactgagtca tcagtaccct 5280
cctattcagc tccccaagat gatgtgtttt tgcttaccct aagagaggtt ttcttcttat 5340
ttttagataa ttcaagtgct tagataaatt atgttttctt taagtgttta tggtaaactc 5400
ttttaaagaa aatttaatat gttatagctg aatcttttttg gtaactttaa atctttatca 5460
tagactctgt acatatgttc aaattagctg cttgcctgat gtgtgtatca tcggtgggat 5520
gacagaacaa acatatttat gatcatgaat aatgtgcttt gtaaaaagat ttcaagttat 5580
taggaagcat actctgtttt ttaatcatgt ataatattcc atgatacttt tatagaacaa 5640
ttctggcttc aggaaagtct agaagcaata tttcttcaaa taaaaggtgt ttaaacttt 5699

<210> 111
<211> 5020
<212> DNA
<213> Homo sapiens
<400> 111

gacgcgaggc gggttcttgg actgagtgtg cggcgcggtg cgccgccttc cgaggctcct 60
cccgcgggtg gcagcggacg gggcgcgccc ctcggccagt cctcggtcct caggcttgtg 120

```
gctccgttga gcaccggccg ccgggcctct gggtccgtcg agtggagact ctctgaaaag    180
cgtgggctcc gtggcctccg gcgcggccgc ggcgggtcgg tctcctagat catccgggaa    240
gcccacggga ccctcaggcg ggcaggatga acgactggca caggatcttc acccaaaacg    300
tgcttgtccc tccccaccca cagagagcgc gccagccttg gaaggaatcc acggcattcc    360
agtgtgtcct caagtggctg gacggaccgg taattaggca gggcgtgctg gaggtactgt    420
cagaggttga atgccatctg cgagtgtctt tctttgatgt cacctaccgg cacttctttg    480
ggaggacgtg gaaaaccaca gtgaagccga cgaagagacc gccgtccagg atcgtcttta    540
atgagccctt gtattttcac acatccctaa accaccctca tatcgtggct gtggtggaag    600
tggtcgctga gggcaagaaa cgggatggga gcctccagac attgtcctgt gggtttggaa    660
ttcttcggat cttcagcaac cagccggact ctcctatctc tgcttcccag gacaaaaggt    720
tgcggctgta ccatggcacc cccagagccc tcctgcaccc gcttctccag gaccccgcag    780
agcaaaacag acacatgacc ctcattgaga actgcagcct gcagtacacg ctgaagccac    840
acccggccct ggagcctgcg ttccaccttc ttcctgagaa ccttctggtg tctggtctgc    900
agcagatacc tggcctgctt ccagctcatg gagaatccgg cgacgctctc cgaaagcctc    960
gcctccagaa gcccatcacg gggcacttgg atgacttatt cttcaccctg tacccctccc   1020
tggagaagtt tgaggaagag ctgctggagc tccacgtcca ggaccacttc caggagggat   1080
gtggcccact ggacggtggt gccctggaga tcctggagcg gcgcctgcgt gtgggcgtgc   1140
acaatggtct gggcttcgtg cagaggccgc aggtcgttgt actggtgcct gagatggatg   1200
tggccttgac gcgctcagct agcttcagca ggaaagtggt ctcctcttcc aagaccagct   1260
ccgggagcca agctctggtt ttgagaagcc gcctccgcct cccagagatg gtcggccacc   1320
ctgcatttgc ggtcatcttc cagctggagt acgtgttcag cagccctgca ggagtggacg   1380
gcaatgcagc ttcggtcacc tctctgtcca acctggcatg catgcacatg gtccgctggg   1440
ctgtttggaa ccccttgctg gaagctgatt ctggaagggt gaccctgcct ctgcagggtg   1500
ggatccagcc caacccctcg cactgtctgg tctacaaggt accctcagcc agcatgagct   1560
ctgaagaggt gaagcaggtg gagtcgggta cactccggtt ccagttctcg ctgggctcag   1620
aagaacacct ggatgcaccc acggagcctg tcagtggccc caaagtggag cggcggcctt   1680
ccaggaaacc acccacgtcc ccttcgagcc cgccagcgcc agtacctcga gttctcgctg   1740
ccccgcagaa ctcacctgtg ggaccagggt tgtcaatttc ccagctggcg gcctccccgc   1800
ggtccccgac tcagcactgc ttggccaggc ctacttcaca gctaccccat ggctctcagg   1860
cctccccggc ccaggcacag gagttcccgt tggaggccgg tatctcccac ctggaagccg   1920
acctgagcca gacctccctg gtcctggaaa catccattgc cgaacagtta caggagctgc   1980
cgttcacgcc tttgcatgcc cctattgttg tgggaaccca gaccaggagc tctgcagggc   2040
agccctcgag agcctccatg gtgctcctgc agtcctccgg ctttcccgag attctggatg   2100
ccaataaaca gccagccgag gctgtcagcg ctacagaacc tgtgacgttt aaccctcaga   2160
aggaagaatc agattgtcta caaagcaacg agatggtgct acagtttctt gcctttagca   2220
gagtggccca ggactgccga ggaacatcat ggccaaagac tgtgtatttc accttccagt   2280
tctaccgctt cccacccgca acgacgccac gactgcagct ggtccagctg gatgaggccg   2340
gccagcccag ctctggcgcc ctgacccaca tcctcgtgcc tgtgagcaga gatggcacct   2400
ttgatgctgg gtctcctggc ttccagctga ggtacatggt gggccctggg ttcctgaagc   2460
caggtgagcg gcgctgcttt gcccgctacc tggccgtgca gaccctgcag attgacgtct   2520
gggacggaga ctccctgctg ctcatcggat ctgctgccgt ccagatgaag catctcctcc   2580
gccaaggccg gccggctgtg caggcctccc acgagcttga ggtcgtggca actgaatacg   2640
agcaggacaa catggtggtg agtggagaca tgctggggtt tggccgcgtc aagcccatcg   2700
gcgtccactc ggtggtgaag ggccggctgc acctgacttt ggccaacgtg ggtcacccgt   2760
gtgaacagaa agtgagaggt tgtagcacat tgccaccgtc cagatctcgg gtcatctcaa   2820
acgatggagc cagccgcttc tctggaggca gcctcctcac gactggaagc tcaaggcgaa   2880
aacacgtggt gcaagcacag aagctggcgg acgtggacag tgagctggct gccatgctac   2940
tgacccatgc ccggcagggc aaggggcccc aggacgtcag ccgcgagtcg gatgccaccc   3000
gcaggcgtaa gctggagcgg atgaggtctg tgcgcctgca ggaggccggg ggagacttgg   3060
gccggcgcgg gacgagcgtg ttggcgcagc agagcgtccg cacacagcac ttgcgggacc   3120
tacaggtcat cgccgcctac cgggaacgca cgaaggccga gagcatcgcc agcctgctga   3180
gcctggccat caccacggag cacacgctcc acgccacgct ggggtcgcc gagttctttg    3240
agtttgtgct taagaacccc cacaacacac agcacacggt gactgtggag atcgacaacc   3300
ccgagctcag cgtcatcgtg gacagtcagg agtggaggga cttcaagggt gctgctggcc   3360
tgcacacacc ggtggaggag gacatgttcc acctgcgtgg cagcctggcc ccccagctct   3420
acctgcgccc ccacgagacc gcccacgtcc ccttcaagtt ccagagcttc tctgcagggc   3480
agctggccat ggtgcaggcc tctcctgggt tgagcaacga gaagggcatg gacgccgtgt   3540
caccttggaa gtccagcgca gtgcccacta aacacgccaa ggtcttgttc cgagcgagtg   3600
gtggcaagcc catcgccgtg ctctgcctga ctgtggagct gcagccccac gtggtggacc   3660
aggtcttccg cttctatcac ccggagctct ccttcctgaa gaaggccatc cgcctgccgc   3720
cctggcacac atttccaggt gctccggtgg gaatgcttgg tgaggacccc ccagtccatg   3780
ttcgctgcag cgacccgaac gtcatctgtg agacccagaa tgtgggcccc ggggaaccac   3840
gggacatatt tctgaaggtg gccagtggtc caagcccgga gatcaaagac ttctttgtca   3900
tcatttactc ggatcgctgg ctggcgacac ccacacagac gtggcaggtc tacctccact   3960
ccctgcagcg cgtggatgtc tcctgcgtcg caggccagct gacccgcctg tcccttgtcc   4020
ttcgggggac acagacagtg aggaaagtga gagctttcac ctctcatccc caggagctga   4080
agacagaccc caaaggtgtc ttcgtgctgc cgcctcgtgg ggtgcaggac ctgcatgttg   4140
gcgtgaggcc ccttagggcc ggcagccgct ttgtccatct caacctggtg gacgtggatt   4200
gccaccagct ggtggcctcc tggctcgtgt gcctctgctg ccgccagccg ctcatctcca   4260
```

```
aggcctttga gatcatgttg gctgcgggcg aagggaaggg tgtcaacaag aggatcacct   4320
acaccaaccc ctacccctcc cggaggacat tccacctgca cagcgaccac ccggagctgc   4380
tgcggttcag agaggactcc ttccaggtcg gggtggaga gacctacacc atcggcttgc   4440
agtttgcgcc tagtcagaga gtgggtgagg aggagatcct gatctacatc aatgaccatg   4500
aggacaaaaa cgaagaggca ttttgcgtga aggtcatcta ccagtgaggg cttgagggtg   4560
acgtccttcc tgcggcaccc agctggggcc tgtctgtgcc cctcctgccc tgcaggctgt   4620
cctccccgcc tctctgcagc ctttcacttc agtgcccacc tggctgacct gtgcacttgg   4680
ctgaggaagc agagaccgag cgctggtcat tttgtagtac ctgcatccag cttagctgct   4740
gctgacaccc agcaggcctg ggttccgtga gcgcgaactc cgtggtggtg ggtctggctc   4800
tggtgctgcc atctacgcat gtgggaccct cgttatcgct gttgctcaaa atgtatttta   4860
tgaatcatcc taaatgagaa aattatgttt ttcttactgg attttgtaca aacataatct   4920
attatttgct atgcaatatt ttatgctggt attatatctg tttttaaat tgttgaacaa   4980
aatactaaac ttttacacgt ctaaaaaaaa aaaaaaaaaa                         5020
```

<210> 112
<211> 3017
<212> DNA
<213> Homo sapiens
<400> 112

```
accgcgggca tttacccgtg ctttcccaag cctggaagaa ctcgtcatgc tctttgtagc     60
gtggtgcttc tgttgctcac agaggtgcct gcttcccctt ctgccatgat tggaagtttc    120
ctgaggcctc cccagccatg tggaactgac aacttgcctt tgatgatttt caagagagtt    180
gtgctatgat gtggcaaaag tatgcaggaa gcaggcggtc aatgcctctg ggagtaagga    240
tcctttcca cggtgtgttc tatgccgggg gctttgccat tgtgtattac ctcattcaaa     300
agtttcattc caggctttta tattacaagt tggcagtgga gcagctgcag agccatcccg    360
aggcacagga agctctgggc cctcctctca acatccatta tctcaagctc atcgacaggg    420
aaaacttcgt ggacattgtt gatgccaagt tgaagattcc tgtctctgga tccaaatcag    480
agggccttct ctacgtccac tcatccagag gtggcccctt tcagaggtgg caccttgacg    540
aggtcttttt agagctcaag gatggtcagc agattcctgt gttcaagctc agtggggaaa    600
acggtgatga agtgaaaaag gagtagagac gacccagaag acccagcttg cttctagtcc    660
atccttccct catctctacc atatggccac tggggtggtg gcccatctca gtgacagaca    720
ctcctgcaac ccagttttcc agccaccagt gggatgatgg cctccctatt ccctgagaca    780
caacagtatt gaaattgggc caattaataa ctccacagtg gcctctcact aaatgtgaga    840
ggtgaggaag ctgcaggaga aaatttgaag ctagcagtgt tcatgagatg taagtaaaga    900
agccatctcc ataaaagtac aacgtgacgc agcaagtgct gatggagaag ctgtagaagt    960
tacacagaag atctagcttg gaccatcgat gaaagtggct acactaaaca acagatttta   1020
aatgtagata aaacagcatt ttattgaaaa aaagatgcca tctaggactt tcatagttag   1080
agaaactcaa tgcctggctt caaaggacaa gctgactctt gtcaggggct agtgcagctg   1140
tgactttcag ttaaagccaa tgctcattga ctattctgaa aatcccaggg cccttaagat   1200
atgctgtata aacgcaacaa caaagcctgg atggcgttgg gtctgtttac agcctggtct   1260
actgagtaag cccactgttg agacctagtg ctcagaaaaa atagatttct ttcaaaatat   1320
ggctgggtgc agtggctcac accggtaatc ccagcacttt gggaggctgg ggcgggtgga   1380
ttgcttgatg ccaggagttc aagaccagcc tgggcaacat ggtgaaaccc tgtctcaaaa   1440
aacaaatcca aaaaaccaaa atattgctac tcattaacaa tgcacctggt tacccaagag   1500
ctctgatgga gatgtacaag gagatgaata ctgttttcat gcctgctaac acagtatcca   1560
ttctgctgct gatagatcaa gaagtacttt tgactttcaa aacttgttat ttaagaaata   1620
cattttgtgg tttctctgat ggttctgggc agtcagttca aaaccttctg gaaaggattc   1680
accattctag atgccatcaa gagcattcaa ttcacgggag gaggtcaaaa tatcaacatt   1740
aattggaatt tggaagaaat agattccaat cctcatggag gacttggagg gtttcaagac   1800
ttcagaggag gaaggaactg tagatgggct gaaaatagca agagaactag aagtggaggg   1860
gcctagagat gtgaccgaat tgctgtaacc tcaggatcaa acttgtatgg atgagaagtt   1920
ccttctcatg gacgagcaaa gaaggtgatt tcttgaaatg gaatctactc ctggtgaaga   1980
tgctatgaac attgttgaaa tgacaaaaga tttagaatat tccataaact tagctgtggc   2040
cagggtttga gaggattgac tcctaatttt gaagttctac tgtgggtaaa tgctgtcaaa   2100
cagcattgca tgccacagag gaatctctca tgaaagagtt aataaatgca gcaaacttca   2160
ctgttgtctt actttaagaa actgccacag tcactgcagg cttcagcaac ttatcttttc   2220
ctaccctcct ctcgcattta aaagtacttt taaattaatg tatgtacatt atttttaggc   2280
ataacactgc tgctcattta aaaaaactac agtatgaacc atgttatatg cagtggaaag   2340
gcaaaaaatt catgtgactt acttgcttgt atttggttta ttgcagtggt ctggaactga   2400
aagtgatgcg tatttctgag ttatgcctgt atttaagaag ctgagtgaaa cccaaagaag   2460
tccatgatag gacagacttc tgaaaactaa agacagaaaa aagcttgaaa gcatcaagaa   2520
agaagtgaaa tcttaactag agggaaaagt aactcagatg acagtggatt tctctctgga   2580
aaccatggag gccagaagga agtgagacaa gttcttttag tgccagaaga actatcaact   2640
tgtggctggg cacggtggct cacacctgta atcccagcac tttgggagac caaggtgggc   2700
agatcacttg aagtcaggag tttgagacca gcctgaccaa catggcaaaa ccctgtctct   2760
actaaaaata caaaaattag ccaggcatgg tggcacatgc ctgtaatctc agctacttgg   2820
gaggctgagg cacgagaatt acttgaaccc gggaggtgga ggttgcagtg agccaagatc   2880
ctgccactgc actccagcct gggcaacaga gcaagactgc ctcaaaacaa aactctgtca   2940
actccaaatt cctatttggt gaaatgatcc ttcaggattg atggggtaat aaaaacattc   3000
```

tcggttgata gaaaact 3017

<210> 113
<211> 1922
<212> DNA
<213> Homo sapiens
<400> 113
accagaggtt tcccagagag gaaggcgtgg ctccctcccg ggccagtgag ccctggcgcc 60
gccgcggccg cggtcccagc agcggagtag ggcggcggct gcgccccgca ccatgggggg 120
cagcccagcc ccagccgcgg taaacgccga cctccgccgc cgcccgcgcc gcgtctgccc 180
cctcccgctg cggctctctg gacgccatcc cctcctcacc tcgaagccaa catgaaggag 240
acccggggct acggagggga tgccccttc tgcacccgcc tcaaccactc ctacacaggc 300
atgtgggcgc ccgagcgttc cgcccgaggcg cggggcaacc tcacgcgccc tccagggtct 360
ggcgaggatt gcggatcggt gtccgtggcc ttcccgatca ccatgctgct cactggtttc 420
gtgggcaacg cactggccat gctgctcgtg tcgcgcagct accggcgccg ggagagcaag 480
cgcaagaagt ccttcctgct gtgcatcggc tggctggcgc tcaccgacct ggtcgggcag 540
cttctcacca ccccggtcgt catcgtcgtg tacctgtcca agcagcgttg ggagcacatc 600
gacccgtcgg ggcggctctg caccttttc gggctgacca tgactgtttt cggggctctcc 660
tcgttgttca tcgccagcgc catggccgtc gagcgggcgc tggccatcag ggcgccgcac 720
tggtatgcga gccacatgaa gacgcgtgcc acccgcgctg tgctgctcgg cgtgtggctg 780
gccgtgctcg ccttcgccct gctgccggtg ctgggcgtgg gccagtacac cgtccagtgg 840
cccgggacgt ggtgcttcat cagcaccggg cgagggggca acgggactag ctcttcgcat 900
aactggggca acctttctt cgcctctgcc tttgccttcc tggggctctt ggcgctgaca 960
gtcacctttt cctgcaacct ggccaccatt aaggccctgg tgtcccgctg ccgggccaag 1020
gccacggcat ctcagtccag tgcccagtgg ggccgcatca cgaccgagac ggccattcag 1080
cttatgggga tcatgtgcgt gctgtcggtc tgctggtctc cgctcctgat aatgatgttg 1140
aaaatgatct tcaatcagac atcagttgag cactgcaaga cacacacgga gaagcagaaa 1200
gaatgcaact tcttcttaat agctgttcgc ctggcttcac tgaaccagat cttggatcct 1260
tgggtttacc tgctgttaag aaagatcctt cttcgaaagt tttgccagat gagaaaaaga 1320
agactcagag agcaagagat ggggcctgat ggaaggtgtt tttgtcatgc atggaggcag 1380
gtccccagga cttggtgcag ttctcatgat agagaaccct gcagtgtcca gctaagctga 1440
tgacttgaag ataaatctgc ctaaccctgg gatgaagtat ctgtgaacta ttttgacagc 1500
agatgaggaa ttttggggaa attaaaacct gcctttctgc caggatcaca tcactggaag 1560
ctccatgact ctctttttgt aaaagaaaaa aaaatcacag aaacacccac ctcccaaact 1620
attctctttt acttcttccc ccaagcccac ccccaaatat aactgttatc cagaagctgt 1680
tatgtcctgt ttccatacat gtttttgtac ttttactata tctacataca tcaattaaac 1740
ttatgtccta ttgttttgtg aatttatatt tgcgtataca ttatcatatg taaaatttgc 1800
atttttttat tgaaaattat gtttcttgag atttatccac attgaaacat ggagctctaa 1860
atcgttaatt ttaaccgcta tagagtattc cataatttga ataaagcata atttgtttgt 1920
ac 1922

<210> 114
<211> 5417
<212> DNA
<213> Homo sapiens
<400> 114
agaaggtagc agacagacag acggatctaa cctctcttgg atcctccagc catgaggctg 60
ctctgggggc tgatctgggc atccagcttc ttcaccttat ctctgcagaa gcccaggttg 120
ctcttgttct ctccttctgt ggttcatctg gggtccccc tatcggtggg ggtgcagctc 180
caggatgtgc cccgaggaca ggtagtgaaa ggatcagtgt tcctgagaaa cccatctcgt 240
aataatgtcc cctgctcccc aaaggtggac ttcaccctta gctcagaaag agacttcgca 300
ctcctcagtc tccaggtgcc cttgaaagat gcgaagagct gtggcctcca tcaactcctc 360
agaggccctg aggtccagct ggtggcccat tcgccatggc taaaggactc tctgtccaga 420
acgacaaaca tccagggtat caacctgctc ttctcctctc gccgggggca cctcttttttg 480
cagacggacc agcccattta caaccctggc cagcgggttc ggtaccgggt ctttgctctg 540
gatcagaaga tgcgcccgag cactgacacc atcacagtca tggtggagaa ctctcacggc 600
ctccgcgtgc ggaagaagga ggtgtacatg ccctcgtcca tcttccagga tgactttgtg 660
atcccagaca tctcagagcc agggacctgg aagatctcag cccgattctc agatggcctg 720
gaatccaaca gcagcaccca gtttgaggtg aagaaatatg tccttcccaa ctttgaggtg 780
aagatcaccc ctggaaagcc ctacatcctg acggtgccag gccatcttga tgaaatgcag 840
ttagacatcc aggccaggta catctatggg aagccagtgc aggggggtggc atatgtgcgc 900
tttgggctcc tagatgagga tggtaagaag actttctttc ggggggctgga gagtcagacc 960
aagctggtga atggacagag ccacatttcc ctctcaaagg cagagttcca ggacgccctg 1020
gagaagctga atatgggcat tactgacctc caggggctgc gcctctacgt tgctgcagcc 1080
atcattgagt ctccaggtgg ggagatggag gaggcagagc tcacatcctg gtattttgtg 1140
tcatctccct tctccttgga tcttagcaag accaagcgac accttgtgcc tggggccccc 1200
ttcctgctgc aggccttggt ccgtgagatg tcaggctccc cagcttctgg cattcctgtc 1260
aaagtttctg ccacggtgtc ttctcctggg tctgttcctg aagcccagga cattcagcaa 1320
aacacagacg ggagcggcca agtcagcatt ccaataatta tccctcagac catctcagag 1380

ctgcagctct cagtatctgc aggctcccca catccagcga tagccaggct cactgtggca 1440
gccccacctt caggaggccc cgggtttctg tctattgagc ggccggattc tcgacctcct 1500
cgtgttgggg acactctgaa cctgaacttg cgagccgtgg gcagtggggc cacctttct 1560
cattactact acatgatcct atcccgaggg cagatcgtgt tcatgaatcg agagcccaag 1620
aggaccctga cctcggtctc ggtgtttgtg gaccatcacc tggcaccctc cttctacttt 1680
gtggccttct actaccatgg agaccaccca gtggccaact ccctgcgagt ggatgtccag 1740
gctggggcct gcgagggcaa gctggagctc agcgtggacg gtgccaagca gtaccggaac 1800
ggggagtccg tgaagctcca cttagaaacc gactccctag ccctggtggc gctgggagcc 1860
ttggacacag ctctgtatgc tgcaggcagc aagtcccaca agcccctcaa catgggcaag 1920
gtctttgaag ctatgaacag ctatgacctc ggctgtggtc ctgggggtgg ggacagtgcc 1980
cttcaggtgt tccaggcagc gggcctggcc ttttctgatg gagaccagtg gaccttatcc 2040
agaaagagac taagctgtcc caaggagaag acaacccgga aaaagagaaa cgtgaacttc 2100
caaaaggcga ttaatgagaa attgggtcag tatgcttccc cgacagccaa gcgctgctgc 2160
caggatgggg tgacacgtct gcccatgatg cgttcctgcg agcagcgggc agcccgcgtg 2220
cagcagccgg actgccggga gcccttcctg tcctgctgcc aatttgctga gagtctgcgc 2280
aagaagagca gggacaaggg ccaggcgggc ctccaacgag ccctggagat cctgcaggag 2340
gaggacctga ttgatgagga tgacattccc gtgcgcagct tcttcccaga gaactggctc 2400
tggagagtgg aaacagtgga ccgctttcaa atattgacac tgtggctccc cgactctctg 2460
accacgtggg agatccatgg cctgagcctg tccaaaacca aaggcctatg tgtggccacc 2520
ccagtccagc tccgggtgtt ccgcgagttc cacctgcacc tccgcctgcc catgtctgtc 2580
cgccgctttg agcagctgga gctgcggcct gtcctctata actacctgga taaaaacctg 2640
actgtgagcg tccacgtgtc cccagtggag gggctgtgcc tggctggggg cggagggctg 2700
gcccagcagg tgctggtgcc tgcgggctct gcccggcctg ttgccttctc tgtggtgccc 2760
acggcagccg ccgctgtgtc tctgaaggtg gtggctcgag ggtccttcga attccctgtg 2820
ggagatgcgg tgtccaaggt tctgcagatt gagaaggaag gggccatcca tagagaggag 2880
ctggtctatg aactcaaccc cttggaccac cgaggccgga ccttggaaat acctggcaac 2940
tctgatccca atatgatccc tgatggggac tttaacagct acgtcagggt tacagcctca 3000
gatccattgg acactttagg ctctgagggg gccttgtcac caggaggcgt ggcctccctc 3060
ttgaggcttc ctcgaggctg tggggagcaa accatgatct acttggctcc gacactggct 3120
gcttcccgct acctggacaa gacagagcag tggagcacac tgcctcccga gaccaaggac 3180
cacgccgtgg atctgatcca gaaaggctac atgcggatcc agcagtttcg gaaggcggat 3240
ggttcctatg cggcttggtt gtcacgggac agcagcacct ggctcacagc ctttgtgttg 3300
aaggtcctga gtttggccca ggagcaggta ggaggctcgc ctgagaaact gcaggagaca 3360
tctaactggc ttctgtccca gcagcaggct gacggctcgt tccaggaccc ctgtccagtg 3420
ttagacagga gcatgcaggg gggtttggtg ggcaatgatg agactgtggc actcacagcc 3480
tttgtgacca tcgcccttca tcatgggctg gccgtcttcc aggatgaggg tgcagagcca 3540
ttgaagcaga gagtggaagc ctccatctca aaggcaaact cattttggg ggagaaagca 3600
agtgctgggc tcctgggtgc ccacgcagct gccatcacgg cctatgccct gtcactgacc 3660
aaggcgcctg tggacctgct cggtgttgcc cacaacaacc tcatggcaat ggcccaggag 3720
actggagata acctgtactg gggctcagtc actggttctc agagcaatgc cgtgtcgccc 3780
accccggctc ctcgcaaccc atccgacccc atgccccagg ccccagccct gtggattgaa 3840
accacagcct acgccctgct gcacctcctg cttcacgagg gcaaagcaga gatggcagac 3900
caggcttcgg cctggctcac ccgtcagggc agcttccaag gggggattccg cagtacccaa 3960
gacacggtga ttgccctgga tgccctgtct gcctactgga ttgcctccca caccactgag 4020
gagaggggtc tcaatgtgac tctcagctcc acaggccgga atgggttcaa gtcccacgcg 4080
ctgcagctga acaaccgcca gattcgcggc ctggaggagg agctgcagtt ttccttgggc 4140
agcaagatca atgtgaaggt gggaggaaac agcaaaggaa ccctgaaggt ccttcgtacc 4200
tacaatgtcc tggacatgaa gaacacgacc tgccaggacc tacagataga agtgacagtc 4260
aaaggccacg tcgagtacac gatggaagca aacgaggact atgagtacga tgagcttcca 4320
gccaaggatg acccagatgc ccctctgcag cccgtgacac ccctgcagct gtttgagggt 4380
cggaggaacc gccgcaggag ggaggcgccc aaggtggtgg aggagcagga gtccagggtg 4440
cactacaccg tgtgcatctg gcggaacggc aaggtggggc tgtctggcat ggccatcgcg 4500
gacgtcaccc tcctgagtgg attccacgcc ctgcgtgctg acctggagaa gctgacctcc 4560
ctctctgacc gttacgtgag tcactttgag accgagggggc cccacgtcct gctgtatttt 4620
gactcggtcc ccacctcccg ggagtgcgtg ggctttgagg ctgtgcagga agtgccggtg 4680
gggctggtgc agccggccag cgcaaccctg tacgactact acaaccccga gcgcagatgt 4740
tctgtgtttt acggggcacc aagtaagagc agactcttgg ccaccttgtg ttctgctgaa 4800
gtctgccagt gtgctgaggg gaagtgccct cgccagcgtc gcgccctgga gcggggtctg 4860
caggacgagg atggctacag gatgaagttt gcctgctact acccccgtgt ggagtacggc 4920
ttccaggtta aggttctccg agaagacagc agagctgctt tccgcctctt tgagaccaag 4980
atcacccaag tcctgcactt caccaaggat gtcaaggccg ctgctaatca gatgcgcaac 5040
ttcctggttc gagcctcctg ccgccttcgc ttggaacctg ggaaagaata tttgatcatg 5100
ggtctggatg gggccaccta tgacctcgag ggacaccccc agtacctgct ggactcgaat 5160
agctggatcg aggagatgcc ctctgaacgc ctgtgccgga gcacccgcca gcgggcagcc 5220
tgtgcccagc tcaacgactt cctccaggag tatggcactc aggggtgcca ggtgtgaggg 5280
ctgccctccc acctccgctg ggaggaacct gaacctggga accatgaagc tggaagcact 5340
gctgtgtccg ctttcatgaa cacagcctgg gaccagggca tattaaaggc ttttggcagc 5400
aaagtgtcag tgttggc 5417

<210> 115
<211> 224931
<212> DNA
<213> Homo sapiens
<400> 115
gtttacacgc tccggggcct gtaggcgccg cgagttccgg ctgtcgccgt cgccgccgcg 60
gctcctggag gtcggttgcg acgagtaacg gcgccaggac gagccctgcg ccttcttttt 120
cgatatgtac ccgaattggg gccggtatgg cgggagcagc cactatccgc cgccaccggt 180
cccaccgccg ccgccagtgg cgcttcctga ggcctcgccg gggcccgggt actcgagctc 240
gacgactccc gcggccccct cctcctcggg cttcatgagc ttccgcgaac agcacttggc 300
gcagctccag cagctgcagc agatgcacca gaagcaaatg cagtgcgtgc ttcagcccca 360
ccaccttcct ccgccccctc tgccgccccc gccagtgatg ccgggggccg gctacggaga 420
ctggcagccg ccaccgccac cgatgccccc gccacccggg ccggccctca gctatcagaa 480
gcagcagcag tacaaacacc agatgctcca ccaccaacga gacgggcctc ctggtttggt 540
tccaatggag ctggaatccc cccctgaatc tccccctgtg ccgcctgggt cctatatgcc 600
cccatctcag tcttacatgc ccccacctca gccgccaccc tcttactacc ccccgacctc 660
atctcagccc tacctgcctc ctgctcagcc gtccccttcg cagtccccac cttcccaatc 720
ctacctggcg cccacccctt cttactcatc ctcctcctct tcctcgcaat cctatttgag 780
ccattcccag tcctacttgc cctcttctca ggcatctcct tcccgcccct cccagggcca 840
ttctaaatcc caactactag ctccaccacc accgtccgcc ccccctggaa ataagacaac 900
tgtccagcaa gagcctttgg agagtggggc caaaaacaag agtactgaac agcagcaagc 960
cgcccctgag ccagatccct ctacgatgac tccacaggaa cagcagcagt attggtatcg 1020
acagcacttg cttagtttgc aacagaggac aaaagttcat ttgccaggac acaaaaaggg 1080
tcctgtggta gcaaaggata caccagagcc ggtaaaagaa gaagttacag tacctgccac 1140
cagtcaagtt ccagaatctc cttcttctga ggagccccca ttgccacctc caaatgagga 1200
agtgccacct cctctcccac ctgaggaacc ccagtctgag gacccagaag aagatgccag 1260
gttaaagcag ttgcaggctg cagcagcaca ctggcagcag caccagcagc atcgagtcgg 1320
tttccagtat cagggaataa tgcagaagca cactcagtta cagcagattc tacaacagta 1380
tcagcagatt atacagcccc caccacatat acagaccatg tctgtagata tgcagctgcg 1440
gcattatgag atgcagcagc aacagtttca acatctttac caagaatggg agcgagagtt 1500
tcagctatgg gaggaacaac tccattccta tcctcataaa gatcagcttc aggagtatga 1560
gaagcagtgg aaaacatggc agggacatat gaaagccact cagagctatc tccaggagaa 1620
agtcaattca tttcagaaca tgaagaacca gtatatgggg aacatgtcaa tgccacctcc 1680
ttttgttcca tattctcaga tgcctccacc tctacctaca atgccccctc cagtgttgcc 1740
tccttcattg ccaccaccag tgatgccccc tgccctccct gctacagtgc caccacctgg 1800
catgccccca cctgttatgc caccttctct accaacctct gttcccccac cagggatgcc 1860
tccttctctc tcttctgcag ggccaccacc agttctcccc ccaccttccc tgtcttctgc 1920
agggccacca ccagttcttc cccaccatc tctctcttca acagcacctc cacctgtcat 1980
gcccctccca ccattgtctt cagctacacc tcctccagga atacctcccc ctggagttcc 2040
acaagggata cctcctcagt taacagcagc cccagttcca ccagcctcca gttcacagag 2100
ctcgcaagtt ccagagaaac ctagaccagc actgcttcct actcctgtgt cttttggttc 2160
tgccccaccg acaacttacc atcctccgtt gcaatcagct ggtccatcag aacaagtgaa 2220
ttcaaaagct cctttgagca agtctgctct gccatacagt tcattctcat ctgatcaagg 2280
acttggggag tcttcagctg ctccatctca gccaatcact gcagtgaagg acatgccagt 2340
gagatcaggt ggcctgcttc cagatcctcc tagaagtagt tacttggaaa gtccaagagg 2400
cccaagattt gatggtcctc gaagatttga ggatttaggg tcaaggtgtg aaggaccgag 2460
acccaaaggg cctcgttttg aaggaaatcg ccccgatggg ccaagaccca gatatgaagg 2520
tcacccagca gagggcacta aaagcaagtg gggaatgatt ccccgggggc cagcatctca 2580
attttatatt acccccagta catccctaag tcctcgacag agtggaccac agtggaaagg 2640
ccccaaacca gcttttggac agcagcatca gcagcaacct aagtcacaag cagaacctct 2700
ttcaggaaac aaagaaccat tagcagacac cagtagtaac cagcagaaga attttaaaat 2760
gcaatcagct gcattttcca ttgctgcaga tgtaaaggat gtcaaggcgg ctcagtcaaa 2820
tgagaatcta agcgactctc aacaagagcc acctaaaagt gaagtctcgg aagggcccgt 2880
agagccctct aattgggacc agaatgttca aagtatggag actcaaatcg acaaagccca 2940
agctgttact cagcctgtac cccttgcgaa taagcctgta cctgctcaat ctacttttcc 3000
ttcaaaaaca gggggatgg agggaggaac agcagtagca acatcatcat taacagcaga 3060
taatgatttt aaacctgtgg gtattggtct accccattca gaaaacaacc aagataaagg 3120
cctgcctcgg ccagataata gagataatag attagaaggc aatagaggca acagctcatc 3180
ttacagaggt cctgggcaaa gcagaatgga agacacacgg gataaaggtc tagtaaacag 3240
aggtcgcggc caggcaatca gtcgaggccc aggattggtc aagcaagaag actttcggga 3300
taagatgatg ggtagaagag aagatagtcg agagaagatg aacagaggag aaggtagccg 3360
ggacagaggg ttggtgaggc ctggaagcag tcgggagaaa gtgccaggtg gtcttcaagg 3420
gagccaggac aggggtgcag ctggcagccg agaaaggggga ccacctcgga gggctggcag 3480
tcaggagagg ggacctcttc gaagggctgg gagtagagag agaataccac cccgaagagc 3540
tgggagcagg gagagaggac cacctcgagg gcctggcagt cgagaaaggg gactgggaag 3600
atcagatttt ggtcgtgata gaggtccatt cagaccagaa ccaggagatg gtggggaaaa 3660
aatgtatcca tatcaccggg atgagcctcc tagggctcca tggaaccatg gagaagagcg 3720
agggcatgaa gagtttccat tagatggtag aaatgctcca atggaacgag aaagactcga 3780
tgactgggat agagagagat actggagaga atgtgaacgt gactatcaag atgatacact 3840

agagctctat aacagagagg acaggttctc agcaccacca tctcggtctc atgatggaga 3900
taggcgaggc ccttggtggg atgattggga gagagaccag gatatggatg aggactacaa 3960
tagggaaatg gaaagggaca tggacaggga tgtggatcgg atttcaagac ctatggatat 4020
gtatgataga agtttggata atgagtggga cagagattat gggagaccac tggatgaaca 4080
agaatcacag tttcgtgaac gggatattcc atctcttcca cctttaccgc ccctcccacc 4140
tcttccacct ttggatagat atcgggatga tagatggaga gaagaaagaa atcgagagca 4200
tgggtatgat cgagatttcc gtgatagggg tgagttgagg attcgagagt atccagaaag 4260
aggagataca tggcgggaaa agcgagatta tgttcctgac agaatggact gggaaagaga 4320
acggttgtca gacagatggt acccatctga tgtggataga cattccccca tggcggaaca 4380
tatgccctcc tcacatcatt cctcagaaat gatggggtcc gatgcaagct tagactctga 4440
ccaaggcctt ggaggggtaa tggttctcag tcagaggcag catgaaatca ttttgaaagc 4500
tgcacaagaa ctgaaaatgc ttcgggaaca gaaagaacag cttcaaaaga tgaaagactt 4560
cgggtctgag ccacagatgg ctgaccatct accacctcag gaatcaagat tgcagaatac 4620
atcttcaaga cctggaatgt atccgcctcc agggtcgtat agacctcccc ctcctatggg 4680
caaaccacca ggttcaattg taagaccctc tgctccacca gcaagatcat ctgttcctgt 4740
gaccaggcca cctgtcccaa taccaccacc tccacctcct ccacctctac ctcctcctcc 4800
tccagtgata aagccacaaa cttcagctgt agaacaggaa cgatgggatg aagattcttt 4860
ctatgggctc tgggatacaa atgatgaaca aggactgaat tcagaattta agtcagaaac 4920
tgcagcaatt ccatctgctc cagtattacc acccccacct gttcactctt ccattccccc 4980
tcctggccca gtgcctatgg gtatgccacc aatgtccaag ccaccaccag tacaacagac 5040
tgttgattat ggccatggcc gagatatatc cactaataaa gttgaacaga taccttatgg 5100
agaaagaata actctacgcc cagatccact acctgaaaga tcaacttttg agacagagca 5160
tgcaggccaa cgtgatcgtt atgatagaga aagagatcgt gagccttatt ttgatcgtca 5220
aagtaatgtc atagcagatc atcgagattt taaaagggat cgtgagacac atagagatcg 5280
agaccgggat cgtggtgtta ttgactatga ccgggatcga tttgacagag aacgccgacc 5340
ccgagatgat agagctcagt catatcgaga caaaaaagac cattcctcat ccagaagagg 5400
gggttttgat aggccatcct atgaccggaa gtctgaccga ccagtctatg aaggaccatc 5460
catgtttgga ggagaacgaa ggacttatcc tgaggagcga atgcctctgc cagctccttc 5520
actgagccac cagcctcctc cagctccacg agtcgagaag aagcctgaat caaagaatgt 5580
ggacgatatt ttgaaaccac cgggccggga gagcagacct gagagaattg ttgttataat 5640
gagaggatta cctggcagtg gaaagacaca tgttgcaaaa cttattcgag ataaggaggt 5700
agaatttgga ggacctgcac ccagagttct aagcctggat gattacttca tcactgaagt 5760
ggaaaaagaa gaaaaagatc cagattctgg aaagaaagtg aaaaagaagg taatggaata 5820
tgaatatgaa gctgagatgg aggagactta ccgcaccagc atgttcaaaa ctttcaaaaa 5880
gactctggat gatggctttt ttccccttcat catcctggat gccatcaatg acagagttag 5940
gcattttgac cagttttgga gtgcagcaaa aaccaaggga tttgaggtat atttggctga 6000
aatgagtgca gataaccaga cttgtggcaa gagaaatatt catggaagaa agcttaaaga 6060
aataaataag atggctgatc actgggaaac tgcacctcgt cacatgatgc gtctagatat 6120
tcgttctttg ctgcaagatg ctgctattga agaggtagag atggaagatt ttgatgcaaa 6180
tatcgaagaa cagaaagaag aaaagaaaga tgcagaggaa gaggaaagcg aactgggtta 6240
cattccgaaa agcaaatggg agatggacac atctgaggca aagctagaca agttggatgg 6300
cttgaggact ggtactaaaa ggaaacgtga ctgggaggcc attgccagca gaatggagga 6360
ttatcttcag ctccccgatg attatgatac tcgtgcttct gaagccaagg cctcgcggag 6420
cttctttgtg tgtcaccttg cttccacgtt tcagttcttg ttttgtttct actgctttag 6480
tttttttttaa agttctccag tgtccccaag aggtattaga atcttgctgt acccaagcaa 6540
gacgttaatt tttcttttaa ctgttttggg gagggaggga gtgatagctt aactgctgaa 6600
gccaggcggg ggtctgctgg aggattccaa cagagagtat ttcctccact gtacaatgtc 6660
acagactatc tctatcatca ttgctttgtg gctgtttctg ttttttactg tatgtaactg 6720
gtagctgatt gtactaggat taaaaacaat aaactttcat gataaagccg atgagattca 6780
tgggctatac agcgctccta aaatctttgt ggtaacctta ctctttttta atgtacaaat 6840
gagagtcctg gccaggtgca gtggctcaca cctgtaatcc cagcactttg ggaggccaag 6900
gttggaggat cactgggccc aggagtttga gatcagcctt ggcaagatgg tgagattcca 6960
tctctacaaa aaatcaaaat attattcagg cgtcgtagca cacacctgtg gtctcagcta 7020
cttgggaagc tgaggctgga gaactgctcg agcgcgggag ttgaagtctg ccatgagcca 7080
tgatctcggc acacactcca gcctgggtga cggagcgaga ccctgtctca aaaaataaat 7140
taataaataa caaaaaataa aaattagaat ccttattgct cataagaaaa gaactctagc 7200
tgggcacagt ggctcccacc tgtaatccca gcacttcggg aggccaagag gggtagatca 7260
cctgagatca ggaatttgag accagcttgg ccaacatggt gaaacaccat ctctattaaa 7320
aatacaaaag aaattagctg ggcattgtgg cgggcacctg tagtcccagc tactcaggag 7380
gctgaggcag gagaatcact tgaacccagg cggcagaggt tgcagtgagc taagattgtg 7440
ccactgcact ccagcctggg tgacagagtg agactctcaa aaaaaaaaaa aaaaaaaaaa 7500
aaaagaactc caatttatat accagctgag agagacaggc taggtggact ccagccctcc 7560
ctgctttgca atcctagggc catgggctca gctctcccac agcattcagt tcatgtagtc 7620
agcatggtgg gtttggctct gtttgctcaa caagactgca aacatgaaca gctgtatctc 7680
agacacctga ctcagaacct tacacacact ggaggtgctt agttgcatgg aattgtgatc 7740
ttacagcttg aggcaggtaa ctccagtggg aacaaaactt aatgagtatc aggggataat 7800
tcccagagaa ccgatataaa cagctgccca acagaccagg tctgggacaa ggcaattccc 7860
actgcatcca agtgacggat aagtgcaggg gagaaggggt cagggttcat ggcagggcgt 7920
tttagggaaa gtctactctg ccccctgaaa tctctctccc acctcttcac gaatacttct 7980

gatttctgaa acaagatgct aaggcactcg gtcagcactt agaaatgcat ctgcccaaga 8040
ggagatcaaa gagaatgttt tcatttactc tgaagtataa agattgggta aagacccagc 8100
acggtggctc acacctgtaa tcccagcact ttgggaggcc aaggcaggtg gatcatgaag 8160
tcaggagatc aagaccatcc tggccaacat ggtgaaaccc atctctacta aaaatacaaa 8220
aatcagctgg gcgtggtggc aggcacctgt aatcccagct actcgggagg ctgagacagg 8280
agaattgcct gaacgcagga gtcagaggtt gcagtgagct gagatcgtgg cactgcactc 8340
cagcctggcg acagagtgag actccctccc gccgcccccc ctcccaaaaa aaagattggg 8400
gatagactct ttcctaagcg ttcttgacag gggaaaaagg ttagggggca taaaggtttt 8460
cagggtgtgg aaagtaaaaa tggattttca ggtagaacat gagaaagaga aagggctgga 8520
aaagaaggct gggatacaag cgtatgggat acacatcagc tttggctgca agttacagaa 8580
gctggctctg gcaaacataa gtggcgaaag agaatttgtt ggaaagatac atggtactga 8640
aggaggcctt caacactcag gcctgagaag ggaccagggc agctttgggg gtctgggtag 8700
cgggaactca tgagctgtct ggctttggat gattctaaag caactgcctt ccattatggg 8760
taatgactcc aactcagatt caaattcccc aggaaaaagt ctgacagatc ttgggtctat 8820
gctcaccctt tgagaatgga gggttgggag agtcctgtgg ctggcagccc caccaggaat 8880
gcatggaatt ggggagtttg agtttcccaa aggaaagctg agtttctgtt actcaaggaa 8940
gggaagaagt gctgggcata tgtgcacagc agatgttctc tacacatggg atgtcaattg 9000
agagaggtgg gggatggatg gggtggggga tgggaatttc caggacgctg cagaacctgc 9060
catagggcc aaggctcctg ccacagccct ggattgcatc tccctctcct cctataaaag 9120
caagctcagg aaaagaacat tgtttctgtt cttgacagct ccatagaatt gtctccttag 9180
cttccagtca cgctcagctc ctgctagaat aattaccacc cacctgttct ccatacaaga 9240
attcacagcc agtggacaca catctggcct gtaaaagaca tgcttggaag tctcccaggc 9300
tgcttggaac aggcaggaga caacacaggg tcagccacta attcaaaggc aaggaaacag 9360
ctggcctgag agctatggtc actggagcct aagtggcagc gaactcctcc cctgccaggc 9420
agctcaatgg atgtgggaca ggtgtgtcct gagacaaaca aaccccctga gactcagagg 9480
aggtttgagt gctgggccct gtcctcttct tccagcacat ggctgtcttc ccccaccccca 9540
tctgcttctc tagtgggtgg agggggctaa taccctgcct ttcttggagt tcctttattt 9600
ttcttcccat tactgtttac atcactccct tctaaacacc atggaaatta agaaaaggcc 9660
aggtaaagtt ttaatgggga gggccagaaa tattttggtg gtgaaggtaa ttgcatccaa 9720
gttttaagga tgtggatgca gaattggaag ggagcagggc gcggtggctc acgcctgtaa 9780
tcccaacaat ttgggaggtc aaggcaagtg gatgtcttga gaccagaagt tcgagaccag 9840
cctggccaac atggtgaaac cccatctcta ctaaaaacaa aaattaacca ggcatggtgg 9900
cacacacccg taatcccagc tactcaggag gctgaggcag gagaatagca tgaacccatg 9960
aggcagaggt tgcagtgagc caagatcgca ccactgcact ccagcctggg caactgagcg 10020
agactgtctc aaagaaaaaa aaaatggaat tggaagggga ctgactgaga gagcctctag 10080
gccagcggtt ttcaactttt tttttttttt ttttttgagc aaaacctttg tgcagtgttt 10140
cagcattcaa caaatattta ctgaatgcca actctgtccc aggtgctgtg ctaggggata 10200
cagcagcatg caatccagac ctgattccag cccttgtgag catacagtgt agtggaggag 10260
acagacactg ggaaaataat tagataagta gatcaacagt gagctgcaat tgaagtagag 10320
ctaagaaaga gacgtatgca gctagatgac ctaatacaga cttgggaggg gatttgggga 10380
aggtatcccc aaaggaatga cattggagct gagatctgaa gggtgcacag aactcagaaa 10440
ggaggtatgg aggaggacgg tcaggaggag atgtcataag atcctggagg taggaaggct 10500
cttgtgctgg aggaactcac aggcaaggca agcagagtag tgtgggatgc aggggctagg 10560
gattcaggga ccagactgcc agggggttcag gagtcagatc caagatgtga acaggaaact 10620
ggggagctcc tgtggctgga aaggggcccg cccttgatct ctccccaaac accccaacac 10680
acatacttcc ctttgcctta tagcctctga ggcatcttgg aggaacgttg gggcttgtcc 10740
gaataccgct tgaaaaccac aaatccagcc ctattgccat tttcagatgt gaagactgag 10800
aatcaacaat tgccccctcc cagtgccctc tctgttaact ctgggcagct aatccagcct 10860
ccctggagag agtgtgatgc ggtgacagac tctgaggaca ctgcagacag ctcagtgttt 10920
caggaatgat ggggacagcc ttgagagcca gcccagggct ctggcatggg gaatggaccc 10980
caaagatgag aggttggccc agtctggggc actcagtctt ttctgcctgt gctgggagag 11040
tctctgaccc tcttagccca tccccatcat tcatccagag gcgcaggcag gccgcctgcc 11100
aggatagcag atcccactca gctctggcct caacattcct ctccccgccg gggggaaagg 11160
tcaagtgtag gatattttcc caagccctgg agaacagcag cacctcctga tatctacagc 11220
tactacccag cttgaagggc aactaccagg gcagacaggc aggacgggag agcagtcagc 11280
tggccctcta agcagggctg caggggggaag ggactgggga ggcagggcct gaaatatatc 11340
aacaacttca gccccaagaa gtagagtaga gagtgggtga tgggaaaagg gcttgcatgg 11400
caggggtctg aggctgaggc tccgtctgcc tcaggaaggc taaaacgttc ctgcttcaac 11460
ccagctggtc ctcatttaac taactccagg gtctggcctc tctggagacc ccctgctggc 11520
ccgtctgcag ctcctggagc agaagccaaa agcagccacc agcttagaga cagcagctga 11580
acccagagcc ccccaagcag gagacaggca gtgcaggcat tgcaggcccc tttgcaaccc 11640
tgcaaggagt caaaagccaa gtaggtgggg ctggggggaa ggcttcagag ataaagaagg 11700
cagaggcaat tgtctctaag ccagatcagg agcagctatg gagagaaaaa tctcttggag 11760
cctggaaggt ggtttcatca gtcaaggctg tcctaaggca aactatgcaa cagtagcctc 11820
ccttcttccc ccgtctccct tctgcccctc atccagcaca ctgctctgtg agcactgggc 11880
acttgccatc ctgccatcct cccccagggg aaggcaggta cagaggaaca agggagcagg 11940
tgccagaagg cctgaattca aatctagctg ctccctattc tagccgtgtg accttcggca 12000
aagatctgaa cctctgagac tctgctcctg cttctgtaaa aatgggatgg ataagagcta 12060
tgtcatagga ctgtagaaat gattgaatga gaaaatgaag gtaaagatct taggtcttat 12120

tgagtttgcc atggatactc ctcatgggac aagagagaga gaagggggaa gtggggaagt 12180
actggggact gttgaatgat ccaaatggga tgcctgatga atggggcagc tcccaaatgt 12240
ttcaaccctg tttccaaggt agaggctggg ccagagagtg ggtggtgagg gcctgaggta 12300
gggaaagtta ttctgtattg aaaagaaaac agagagagga agtcagatct cacgacgtgc 12360
gtcagccaag agcctgggca gacagtgtct cctccagagg aagggctggt actccggcac 12420
tctgttgccc tctggcagcc gctaagctcc tgttttcctc attaagctgt ccgcaacctg 12480
gccccctta cactgctgat aagggccaga aacaattcag aatccccaac agatggcatc 12540
tgtggttttc aatgtcacct cccgatggaa tcaacaaggt ggggctgaac tcaggtggac 12600
tgcagggagt cagggcagaa aagcaccaac tctgaggggt ggagtccagg attcgggaac 12660
ctcactgtgt gcccagcgat gttaggcctc gtggacacat aaaatggtaa aagctagtat 12720
ttgttgggca cacactattt gccaagcttt ggtgctaagc acataagttt attatctcat 12780
ttaatcccca taacaaccct acgaagttga tagcattatt attcctattt tataggcaga 12840
ggaactgagg cagagtgcct caggggctta taatttgttg gggagtcaag aacacaggtt 12900
aatacccca agggagaaga gaacaaaatc tgagctgttt ctcaccatcc caagtggccc 12960
tgtcctcagt tttgcatcac tgtctgcctc agtttcccca gatttccagc taccaccatg 13020
gtggctgagc tcaaaaggca gcctccaatc actgttctag ggatggacat gctcattttc 13080
ccaaagttca gccaaggcag aatttgtcaa aataagttta tctaggaaac gtagaagagg 13140
atggaacaaa ataatataaa aagcaaatgc tctgggaaga gagcagctta tgaggcagtt 13200
ttcatgtccc aagatgagga aaataataat agctattggt gtgccaagta tcagacactg 13260
tgctatgctc ttgattatgt tattttgttt aatcctcaag ggactgaag tattacacaa 13320
gcattattgt cctcatttca caaataaggg aacagatgtt cagagaggtt aagcaacttg 13380
tccaggtcac atagcttgta aggaatagag agctaggagt caagcttgcc tttgctttta 13440
acaatttaaa taaaggagac aggcatggtg agtacaacca gctgacaacc agacaggcct 13500
cttgcttcca ggccccattt aggtctaatg gagagatttg actgaacaac ctagatccta 13560
aatggcaggt tttcaggata gctctgctga tcctccatct gacagatttt cagatcttgc 13620
tcaggaaata atcagaacac ttttcctact ggacaagtct ggaaaaaacc ttggcaaatc 13680
tgtaccctcc agttctccta gtccccattt acacagccca aaacaaaaca aacaaacaaa 13740
acccaaaaca ccccaataaa ttaaaaccct gtatttgcac ttgcacttca tttcaaatga 13800
agtacacgcc tcatggccag gcacagtggc tcaggcctat aatcccagca ctttgggagg 13860
ctgaggtggg tggatcacga ggtcaggagt ccaagaccag cctggccaag atggtgaaac 13920
cccgtctcta ctaaaaatac aaaaaaatgt agccgggtgt ggtggtgggc acttgtaatc 13980
ccagctaccc gggaggctga ggcagagaac tgcttgaacc caggaggcgg aggttgcagt 14040
gagctgagat tgcaccacta aattccagcc tgggcaacaa agtgagactt tgtctcaaaa 14100
aacaaacaaa aaaccaaaac aaaacaacaa caacaacaaa caaacagaaa aaataccaaa 14160
ttaccctcat aacatgctac aatttccaca ctatttcact ggttaactga tggttcttca 14220
agcccagaga taatgtgagt aagcccgtaa cccagtgcct ggcaagagat gcgttgttgc 14280
tgcaattatc attaagagac ctcttcctgt gttcacatca gcaagtcatc cctgataccc 14340
ttggaataaa gcagtatgaa attaagtact aattgaatgg ttgatatcaa tagctattga 14400
taagtaatat ttattaagca gctactgcat gccagatttt agtctatata gctttacacg 14460
cattatctct tttaatcttt ataacaaccc catgagatag gaattattgt tactccctct 14520
gttttaaaga caatgaaact aaggcctgga agtactaaat aatttgctta tggtcacaca 14580
acacccagat ggcgggagtg ggatttgaac ccagacagct aagttcagaa cctgcaccag 14640
taacctcttt cctctcctgc ctcccgtgat gggtttgaca ataggataca gagggtcaaa 14700
gaaagaagat gtccttgtgt gctgggggag ttgggggaag cttcctggag ggaatgggct 14760
ctactggact tgaaagaagg acaagagaag tgaagcaaaa cagacatcaa ttttaggctg 14820
gaagatgatc ctgaagtctc atgggaccca aaaatgggac atcaggagag ctctcatctt 14880
tggaacatga gggattgctt atcctctgaa gcagtgtcat ttggccaact tgtcagtcca 14940
aggaaggact cagagcttat caggaacctg tggggtctgc actgggtctg tagcatcaaa 15000
atagaggagg aggacagcaa cagatctcct gggaggccct ggggataggg ccaggattag 15060
atcaactccc tgccatctcc atactgatca caggatttgc ttagaggaag ttgtgtttgg 15120
tttgctgtga ccatggcagg gttgccccag caggtatgga tgcagagggg ctcaaggacc 15180
cacaagtctg taggagttca cagaaatctt agagagaagc ttcaacttca cttaagccac 15240
aaaaatattg ggggctgggg aagaattaaa ttccaggcta ggtaggctcc cagctgacac 15300
catgttagag tcttcccgcc caccacccgc caaaggcatg tctgggaggg ccaaatgaga 15360
catacctggc acgtggcaag tgcccttaaa ggttcagcaa ctggctgggc gtggtggctc 15420
acacctgtaa tcccagcact ttgggaggct gaggcaggca catcacctga ggttacctga 15480
gtttgagacc agcctggcca acataatgaa accccgtctt tactaaaaat acaaaattta 15540
gctgggtgtg gtggtgcatg cctgtaatac cagctgcttg gtgggaggct gaggtgggaa 15600
aatcacttga attcgggagg tagaggttgc agtgaaccga gattgcacca ctgcactcca 15660
gcctgggcga cagagcaaga ctctgccaaa aaaaaaaaaa aaaaaaaaaa aaaagattca 15720
gcaaccttct ccaggcccca tctttcagag ctcacttgct tggctgtgga ttacaaatct 15780
gtcaaccaga ctcccagatg ccacctgcta cctgtttgtg aacacgcaca tcttcctgca 15840
cagtcccctg tgtgaatatg tgttacaaac ctttccagga agcagagtta aactgagaag 15900
attctatctg ttcgggtccc agcggcccac agtatggcct ccctggactt aaagtctaga 15960
atgactttgc atggattgag atgctatgag ggtggagtgt tatgagagct tgccaagctg 16020
agactggctg gggaggggac atcacaggta ctcttaggag gcctgagaat ccgaacaaaa 16080
cggtatcact aggcagaatg tggccgaagc tactagttgg gtgctgagca tagttcggca 16140
atttgtgag catatttgta agaacatata tgtttatcag tagaaataat gcactgtctg 16200
ggttgactgg ctctgtttgt gtacattctt ggatcccaga gagccctggg gagcaataca 16260

tcacacaagc atttctcgcc tgagagctgg tcagagggat cattccaggg aaagctgggc 16320
tggtgttgac cagctgaggt ctgagagaga acatgtgtgc accccaaggc caggcctggc 16380
tctcataagc actggtggaa tttctgagtg catcagttac tctgcacctg tccctttaca 16440
cttagcccca gcctggcctg gcccggccca gcaagtctgc agccagaggt agcctggaaa 16500
gaactggcca acgctttcga gaaccaggct gaggccagaa ggcacgcaaa gcctgaagga 16560
ttcctctctg ctcaggatcc catgtgagaa gtctggggct cctgtcacgt atgttctcag 16620
ctgtcaccgc acagctcact ttcttttatc atggccacgt gtgtgcatgt tttgctctcc 16680
tactgcactt caagccattg ttactcaatg tgtggtccgt gggcccatag catcagcgtc 16740
aaccaggagc ttgttagaaa cacagaatct cggggcccat cacagacata tcagatcaca 16800
atctgtatat taacaagaac cccagtgatt tgaatgcaca ttaaagtttg agaaacactg 16860
ctacttgaaa gcaggagtga accttgtcac ggtgatcttt gtacaactaa gcttttgtat 16920
cccagatgct tggtggtaat taggtgctca ataaatacat gttttaacat ttcattttga 16980
aataattata gacttagaga agagttgcaa aaatagtaca gtgatgtatg tgaaccacta 17040
atactaacag cttacataac cagagtacaa ttaccaaatt aacatgggaa attaacatag 17100
gtatcattaa ccaaactaca gggcctttc taatttcctc tgttttcccc atgtcccttt 17160
tctgctctca gattgctcag ggtatttagt tgttgggtct ccttagtctc ctctaatcta 17220
tggcagctct ctagtctatc cttgtctttc atgaccttga ctcttttaat gagtacccct 17280
cagttatttt gtagtctatc tttcaacttg ggtatgtctg ctgtcttctt gtgattagat 17340
tgaggttata cacagaatag aggtgatgct gtgacctcaa ggcatcgtct caggggatac 17400
gtaatgtcaa cacatcttat tcctggtgat gttaatcttg atctcttagt taagctcata 17460
tatgccggat ttctccactg tttctccact ctaaaattac tactatactt ccctctttag 17520
ttaataagta tattggggaa gataatttga gactgtgcaa atatcctgat tctcccactg 17580
atttgagtgg ccatcactgg tggatcttat caacaacttt gtttttgttt ttgatttttg 17640
ttcctggccg acaattatta ctattgtgtc tgcatagtgg tgattctgta tttccctcct 17700
ttcttctata tttattaatt gaaattcttc tgccaagaag agttgttact tcttccccat 17760
ttttttaatt caattgctta tttcagtagg ggctctcggg tatttattgt atcccagggg 17820
ttataattca atgttatcat tattttatgt tcttgcttaa accgttccag ccactgggag 17880
ctcctacaag ttggctgttg tgtcctttca acatgcccat ctttttccga gtacttcctt 17940
attccctggt accacaagat gtttcagact catcttgttc catatagttt gaatgaatga 18000
atgggagaat gaaaaaatag gctgtatgta taaactcagc cttcctgtga ttccccaact 18060
aagaagctgt atttaattca attctcagct gggaggaaga acgcatgttc agatgcccca 18120
tagaatcact cacctccagc tggacatggt ggctcacacc tgtaatccca gcagtttgag 18180
aggctgaggc tggtggatca cctgaggtca ggagttcaag accggccttg ccaacatagt 18240
gaaaccccat ctctactaaa aataaataaa taaataaata aataaataaa taaataaata 18300
aatagctggg tgtggtggca cgcacctgta gtcccagcta ctcaggaggc tgaggcagga 18360
gaatcgcttg agccctggag gcggaggttg cagtgagcca agatcatgcc attgcactcc 18420
aacccaggcg acagagcaag accctgtatt taaaaaataa taacaaacaa aaagcaacag 18480
aatcactcac ctccactgtt ctgtgagcct cgagtaaagc tggagaaact tttaacctaa 18540
agacaaagga aactgtatcc gtggaaactg taggctggaa aattggcttg ccctgaaagt 18600
ccgactcatg gacttggcac atctaccaag cagtatgaga tagtgcttaa gagtggctgt 18660
gctggagcca gatgccttgg tctaaatcct tatagccact tactgtgtaa ccacctcatg 18720
cctcagtttc cccatcagta aagtgcaggt gataatgaca gtcctggcca tggatttgtg 18780
taattactga gtttacatat acagattgct tagaacagcc cgtgatacat gtaaacattt 18840
aataaccatt agttaggagt ctgatatttc cagcaattag ctcatcctgc taaattcatt 18900
cagcaattgc ttattaagca tctacatcat agtacacact gttgcgagtc ctgggatctc 18960
agcagtgaac gaaagtgaca tcactgctct cacagagcat acattcttct ggaataaaaa 19020
taatctaacc aaataacaga ctacactact tttttttttt tttttgagat ggagtctcac 19080
tctgttgccc aggatggagt gcagtggtgc catcttggct cactgcaagc tctacctcct 19140
gggttcacac cattctcctg cctcagcctc ccaagtagct gggactacat gtgcccgcca 19200
ccatgcccag ctaatttttt ttttgtattt ttagtagaga aggggtttca ccatgttagc 19260
caggatggtc tcaatctcct gacctcatga tccgcccgcc tcagcctccc aaagtgctgg 19320
gattacaggc gtgagccaat gtgcccggcc acagactaca ctacttttta agacagtaga 19380
gggggacagg aacagctgca tttttgtcat ctagcaaaac cattcacccc tcttgacata 19440
agtatccatt ttcctctgta gacccacacc ctggtatcat gtgattcatg cgttggtcaa 19500
tcaaataact gtattgccct agccacagtg attggttcag gcaaggacac aagacctaat 19560
cagcactaga gagaatttg cagctggggc ttttcatggt aaaaccagtg agctaagagc 19620
ttgcggcttg agaataaagc caacacccct gaaggcagag ccgggagtgg aggggaaagt 19680
cctgatgggt tgtacaagcc cctgaatcaa gaatggccg aagttcatcc caggcttgac 19740
ttctcagtta cacgagacag tcaattccct tctactactt gttactgaga gagttctgag 19800
cttacttcaa tccctcagaa agctgacagc tgagcaggaa gcaggagctt gtcctgattc 19860
ccccactgtg cctgtgtcct gagcccctgg tcttgctcta gtacttctca gtttcctgcc 19920
ttcccctaac atacacacca ccaccacccc actgcccacc tcgggccacc cccttacttc 19980
ctctcaagga gattcctgcc ccgtcctcca cagagctgga gcctctccct cttgctcacc 20040
tggcttccct cccatattct tctgcccaag cctcttcttc ccaggcaagt ctggctgaat 20100
ccccagctct gtttcgcctc gtggtctggc ctcctcctct ccagaggcct gaggggctga 20160
tgcgtggttt cagatggcca gcagaatgct acccccaacc tcaggagtcc caccgaagct 20220
cacactgcgg agggtcccct ttttattacc taatctcctg cctctcatgt cttcctgtaa 20280
ctccaggcct gagcggcttc agggcagaat caaggatgtt cgagttctcc cacctctatg 20340
ttctgttaga gagccatgtc ctcagagcag acggggagac accaaactcc cacttccttt 20400

gggaagattt ccatttctct tttccaaaat cctagggaat gtactgcgta agccccttaa 20460
ttatacattg actcacgacg ttgcttgtgc tgttctctcc cctcaagcta tctttcattc 20520
actagaagat ttgcctctta tttgctgtaa gcctagcaca gtgctcaata aacactgaat 20580
gaagaaatga gtggttgaat aaatgaatta ttgaatgggt gctgaaaata gtaagtgaat 20640
gaataaataa atgaatagac taatgaggag aggcagtgaa aggcctggga cggagtggca 20700
gaagggtcaa aaaaaccctg gaggggatag gtcctgagcc cagggaaggg aagatggggc 20760
actcaccatg gcagaatggc aggagggaga cagatgttgt caagtctcta agccttgtgt 20820
ttacataaaa aactggagtt tgggcctggg ttgttttatc gaacccattt attagctttt 20880
taaaactttt tttaaaaaca catggccggg tgcggtggct cacgcctgta atcccagcac 20940
tttgggaggc caaggggggc gtggatcact tgaggtcagg agtttgagac cagcctggcc 21000
aagatagtaa aaccccatct ctaccaaaaa tataaaaaat tacccgggta tggtggcacg 21060
tgcctgtaat cccggctact cggcaggctg aggcagaaga attgcttgaa cccaggaggt 21120
agaggttgca gtgagccgag atcatgccac tgcactccag cctgggtgac agagcgagac 21180
tcagtctaag aaaaaaaaaa aaacataaac ataaaaaaaa catacaaacg ccattttcaa 21240
ggccattgca catcttgcaa tgacatggcc ggttgtggct ctgcctcagc accccacttc 21300
ccagatacag aaaggaccaa ttggccatgc cgacagcacg ccaggtgcag cctcggggga 21360
aggacctggg agatccagtg gacttggggt gcctcccaaa gcccttcagc aaggactcaa 21420
gttctctgca ggtcacaggc agagggcctc tttcttcccc cccgactccc ttctccttcc 21480
tctgccattg actctccatc ctgaatccac actggcactg ccgggagccc tgtggggcct 21540
caggcaaccc aagagagctg aagatgactc catggatttg cagctacaaa tgaaaaacca 21600
cggtttgttt tgaatggatt gtggctctgg cgagaaagcc ttgggatata aatccgccat 21660
gagtggggat gcagatggga agcagatgtg gggatgttga aagcccccgc tccagcacct 21720
aaggcccacc ttggagaaca catgccttcc actgaaaaat gcagatgggt gaggcagctc 21780
agacactgtg ggagccccag ctaagagccc agtctgctca gctgtcacct tctccttctt 21840
ccccctccct ctaggcaatg aagcactcct tctgcattct tggaggatat gcctcctctc 21900
cctgcctccc tctccctctg tcttcatacc actgcctgtc ttactgtctc ttgctctcag 21960
gcgttctctg actccatgtc tgtccttggt ctgtgcctct ccctccctcc ctcttcctgg 22020
ctgctgccca aggccagtgc ccctaccctt catgcttgga taagtctgcc aatcagcttg 22080
aacccctggg gtgaagaacg ggaagaacag tccaagggca gacggcagcc ccagtttcat 22140
gaaggaaggc gatttcaagc cagagacgcc tgggcaggat gaggccaaga actgctcact 22200
tacccacagg gctggatatt tagtttgagt tggggagggc aggtggcaac cagcctggag 22260
ctcccaagcc tgcaagagca gctctgcccc agcccctacc cagctttagg tttcattcag 22320
tgtggccttc tctccttcct ggcctggagc cagccctctc cagcaccagg aagaggcagc 22380
acagctgggt accatggtca caattacccc caaggacacg ggcccaggtg aaggctaggc 22440
aggacaggat ggagcatgtt ttgtatcctc cactgctctc cagagagcta ggagcagccc 22500
ttccctttca acccctgcag ggccccagac agcctccaaa ggcaaatgtg ctttttcttt 22560
ggcttgtata cagtctaaat actttatcca attgatacgg gctctatatc cagttgtctt 22620
attttccttt ttgggtctgt ccacagctct ctgagctctc aggcaggatt ttgggaccat 22680
ttgtggcaca ttccagaggc cactagcaat ctcacaaatt aattctaaac ttgtacacag 22740
gctgtgaatg ctatacaact tttagggggg acttgaggga cacaccaaaa accatccaaa 22800
aatagaatgt tgccattccc aggagactgg aatcatttag aggacaattt cacaaacgct 22860
tctgccgata tggaggaggc tgctgtcgga atgcagctgg gacccatctc cctggcactc 22920
tcgagtctct gcttaggggc tttcccttcc cagcaggcac tcattccagg atccacaggc 22980
accttgcggt ccccagcact gggcggcact gagggggtat cactgtggca ccataccgta 23040
aggcatgatc ccaggaggct tggcatttag tgtggaggct aagctcttgg ttctaggttc 23100
ctttatgcat tgagcatctt tttcacacat gcctgaggct gtgttaggca gtgagtgtga 23160
atggcgaaca aaccagacag ggagcctctg ctcgtgcaag aaacaaacag ggtgctgaga 23220
cctggaataa ctttcaggaa gactgaagag agcagcttgc agctgaggga gatctcaggc 23280
aggtctcagc agctgcctct gggcttcagt ttcttcaccc ataaaatggg ggtgctaaca 23340
gcgctacttt gaggattcaa tgagataatc aatacaggat aactcctagt cataacaagc 23400
cactaatgag gaccatacaa ccaaagcagt taggagcaga cagggcagga ggatagcaga 23460
cgcagtcagc actctgcccc catgtcttcc gatccccgta tcacttcagg acacatggtt 23520
cactcccaac agcccacacc tgcctgtttg tcagcgggct gccctcaggc tgttggaacc 23580
tgctctggcc acctgcatgg agggcaggcc agtgccgggg aggtttcgtc cctgagcccc 23640
tacaggcagc ccctgaccag tgactctgag tgtggaggta gaaatatctc ctccatctcc 23700
agagctcttg cagactgagc cacttaccct tcgtggcctt ttgcttgaca ttgtatcctt 23760
gcttggcctc ctcttttcag tccccttccc ctactcctgg gaagatttgg gaaaaaatca 23820
ccactttcac acaaaccctt gtttgctggg tctgcttttg gggaaaacat cttaagacac 23880
tgggaaatgc taaacattag gaggtgtctc ctaagctgtg tgaccctgtc ttcctgcaga 23940
gctgcccttg ggccaaggca agtcctccca agtttctaag gaattagtca caacaagata 24000
gccttctaac tcaggaaatc tgtgctcctt gtatttgaac atggcaaagg ccagcaccat 24060
aggaaccctg gatggcccct ggcatgtagt tctcggctct caactcagga aactgtctga 24120
gctgctgcct tcgggactgt gtactccctc caggaacctg cacagggagg ccaaaaaaag 24180
ggcataaaat tcatatttca ttattgttat tcttctctat tcctggaaac tccagtcatc 24240
ctgaagatga ttctttagcg tctctcttcc catgaaatgg tggactggtg aggcaggcac 24300
acaggcggcc aacagggcac atccaagatg caggtctgga ttatttgtct agactttatg 24360
atgtttgtgg tatttgccag ctttttaagt tttaggattt gttgtgattt ttttttctta 24420
tttcaaaatt ttccttcata tttaatttg tattcttttt tttttttaaa gggatcccaa 24480
aattatgtaa gctttaggca acagaagacc tcggtaggtt ttgagcatct gaggactgag 24540

aggcttttc acagactagc ttctagctcc atacttttct ttttaatata cagggtctca 24600
ctctgtcacc caggctggag tgcagtggca caagcacggc tcactgcagc ctcaacctcc 24660
caagctcagg tgacaggtga tcctcccacc tcagcctcat gagtagctgg gattacaggc 24720
gcctgccacc atgcccgtct aatttttgta ttttttgtaa agacagggtt tcaccatgtt 24780
gcccaggctg gtctcgaact cctgggctca agtgatctgc ccacctcatc ctcccaaagt 24840
gctgggatta taggcgggag ccaccacacc ccacctggct ccatacattt caactcttgt 24900
ttttcctctc ctgtcttcat acttctggtc ccaatgtgac agctgctgat cctgcacctg 24960
ccactgtgtc aggtgaatca gcacagtgta tggtggaatt atttctacaa gccatttata 25020
atcaggacag ttagcaataa atatactaca tgacatacac atattacata caacccaacg 25080
gagtgttttc ccaacataac caaatgaata tattacagaa ggcccaactg agtatattcc 25140
caactcaaat tcctttagtc atactcccaa atgccacagc cacctcacca cagcctgaca 25200
tgaagggagg tgtgacagat aagaaatcag ggtggagact gactacagtt aaattttttt 25260
gggaggagac agggtcttgc tatgttgccc aggctggagt gtggtggcta ttcacaggtg 25320
tgatcatagt gcactactct ctcaaactcc tgggctcaat tgatccacct gcctcagcct 25380
cccaagtgct gagactacag gtgcatacca ccacacctag ctatagtttt ttggtttttt 25440
ttgtttgttt gttgttttta ccttagcaaa tactatgact ttgaaaaaaa atatgacctt 25500
ggaaggaact cctgtaggtg agcggccctc aatgtaagcc tcattagctt ctcaggaaac 25560
tggcctctgg tagctggact tgctgtgagg cttgggcaca taatatttta aggtctgtaa 25620
gttttagcca catcctgggt gtgtgtgtaa gggaggaggg tggctcagaa ttgttctggg 25680
ctcttcattt cctcagtgcc cttcaactct gaatccatgt caggaagacc ctggcaagac 25740
tgaggaggga gagcaaatgg taactatggc cataactgac agtgagtttg gcccctgagg 25800
ttggacttag tagcaggaga catcagtatt ccaaaagccc aatcactcct cttcttttgc 25860
atttcctttt agtttagtcc tttaattttt taaaccaaag tttcttctga aaaaattctt 25920
ggtttgtctt tgctgaatgg aagagcaaag gaatttagct atacccaggt caggccaagg 25980
tcagtaccag tggacgtggg agaggcaatg ttggaattaa gaggatgttt tgtgatttac 26040
aaaacactat tcacatccat tgtctcattt aagctttacg tgccctctgt cgggtacact 26100
tgtacgttgt tagaatcact atttcccagt taaagttaag agcgactaag caatttatgc 26160
agagtataca cctaggcagt ggcaagtcag aaatttgaac ccagatcttc caaattcaaa 26220
tccacaacat aaccaaagac aactgtggat agtggtttac tattgctaac tactaatttt 26280
ttttttaca cttagtgtca gacacagtac cggcactttt catgagttct cttatttaat 26340
cctcatatcc tctggcacga gccaggtttg aaggatgggg aggtagcagg gaggaaggac 26400
gggatgggcc tcattccttg cagagtgcac agaacaagta aaggaacaaa ggcaggaacg 26460
agctgagatg gttcagacca cgttcactga gagctggtgg tgcttcacag ccaccgtcct 26520
tgggcctctg gcctggtcag gtaagcctcc tagaaccttt catcttagta aaaatgggct 26580
gtgcccatgg aaagtggcct ctggctcagc cctggaagca acagctggaa gaggcctgtg 26640
gcctgtcact atccacacgg ttgcttctct gcaagacaga ggcccgccac ggtgagctgg 26700
tctgtccaac cagctcctga gttgccagtc aaatgatggg ctcggcctca gcgctcagct 26760
ttgaagttta atggggatcc tggtagccca caggtcttgg ccagatcttg ccctttagta 26820
agtgtcttct tccacttccc atcaggggcc tggagcctgg ccaactccct gaggcatctc 26880
tcccagctta tttggattga gggatggagg gtgcctcctg ccctggagag ggaggtggag 26940
aatggaaccc gtaccctgat tgggaaacag aaacctcagg aggcaccagg cagcaggact 27000
ggacaaagaa gtgccggcca gactggctcc ctggggtccc agaagtcaca gacctacaca 27060
atgtctccct gggttagagg atgaacagaa cactagcaac tctcttacca gtgttctcca 27120
gacttgtctg tcctgcccct aactccacct cctttgggct tgtgtgagaa aacgtgtcag 27180
agccaatttg acacctacat ccatccactg gacatgtaat cctgcgcaac ttacttctct 27240
gtacttcagt ttcctcatct gtaaaatggg aataatgatt gtcttcagct tatagaatga 27300
tgtgaatatt aaatgaatta acacagacaa agtgcttaga atagggcctg gcacagagta 27360
agcatgtatt agatgttggc tgctattatt attattatta caagagttct gagattactc 27420
ccttaggccc cagtattttc ctctggggtg tttttgtca ctttctgtca cttaaaatgg 27480
attgagattt tagggcaaac actggagaag cttctcaatt cctcctaatg ggcctgctga 27540
gggtgccgtg gctgactgtg ggcaccggct tgctgctcct ctcctggccg cctccagttc 27600
caggctgctt ttgctgctct gcctcactgg tctgttcgga gtatccacag ctgctagctg 27660
gagaagcggt gttaatacct cattagcacc ttcggctgct ccttgtgggc actaatcact 27720
tcagtttgct gcctctggag tgactctgtg ctgcctttga aaagcaggcc atgtgatggc 27780
gcagagactt ctccagctgg tcccaattcc ctccctcaga ttgttacttt tacccaggaa 27840
gtgaaacaac ttactgttta cattttgtat acacacacac acacaaatat aaaatttttt 27900
aatagatcaa atccttactc cttcatcagg acctgaaact aaacctgtaa gcagcttctt 27960
ccagagcctt cctcttcttc cccagtccac acctgcagac ctgcccctca cccgtgccac 28020
ccgcggtttg tacaggaatc agacaagaag gcaagggccc gaattggaag cagatgtttg 28080
ggacgatgag tgaggttggg gtggagacaa taggaaggac gtagaccctg ctagagctgc 28140
ttgttgctct gacataccgc caaggctcag aaaaatagag tgactgttcc tggttaaata 28200
cccagctgtt actcgtgcca aactttcctg ggcctcaatc ctcagagtct ctggctctgc 28260
ttccaaagtt ctccatatcc tcatgccaac cactatcttg gccccttctt cccaccatgc 28320
caagcccccca agaccagcca taccccctaaa agtcaggact ggagttttac tcaccccgcc 28380
ccacaaagct aacccacgat gccatttttt tgaagcctaa actcaagata tggtctgaca 28440
aaagaggttt tgctgattct gggtgtgaga gtctactgga agacatggct tggacgccaa 28500
gaccacagag ttttcaaggt ttagagggat gataggtcag aattgttaaa ccagaccact 28560
gcggagtatg cagatgggtg gctgttgact gcacccactt tagaacatct ccagagctgc 28620
acatgagcct gccaggagcc aacagcacat ttgtctaccc tgactgtgac ataaaatgct 28680

```
agatctatag gagaacaccc aagtttaatt ccattgtttt gatttttcaa ggagacacaa 28740
gttggcatca tcatgaacaa ggtttcagag aaatcccatt ggaaagagag gctaaataac 28800
tggttttcta aaggcgatgg cctgacccaa cccctatcca gagctgtgat cccagtcaga 28860
agcttaggac atcaacttta agatctgccc aatccccagg aggaatagat gtcagctgct 28920
gtatacgaca gagagataac cagggactct agtttcctgg tttaatatta agggaaattc 28980
cactgaagct cagtgtttcc acccatagtg gtcacaacca accaatttca tgggtgttca 29040
taaaattgat ttgccttttc acgcctctga gtttattgga aatggtattc ctttgacttt 29100
cctctgccac actgattata accatagcac aaaccctttg attgctatta gtgttggaga 29160
tacctccctc tccccatcca ctcaagattc taatttcatt cactgagctg caggctattg 29220
gccagttccc tattgccaaa gtcaacactt ccggtctgct ttgtctggga gagcagatta 29280
aagggagagt gggccctaag attaccgagt ccccttcccc aggctgtccc aggacctgcc 29340
caagtgatgg cctatttatg gcacaataat gcgcttgctc cttgggtctc ttggtttgga 29400
atctatcttt acaagggttc tttcttctct gaaaatcaac agggagagag taacttccct 29460
ctctgtagga agctagacta atttcacttg tagcaacagc aactctataa tatagaaagt 29520
ctccaaatac ataaagaaat gctccccacc aagtaaacag gagaaactat tgaagggggg 29580
gacagtggtg atgggtttgg gtgcccaagc ttcaggagaa ggaaactcta aggaaaatag 29640
attgtttata tctgtctccc aagattgtaa gcttctcaga ggccaggatc acattctacc 29700
ttgtgtgtcc ctagctcccc gcacagtgct tgatacatag gaaacgtctg ctgaaatgca 29760
gagctgcaca gaactcaaga aaaaaaaagg gaggctacaa acccagggat ggaacagaaa 29820
tacctgactg ggaggggagg aggttaggaa atgaaatgac aacttagagc aggattggac 29880
gtaggagacc aagcacaaaa tgatgacaca cacataatct tcctcttgca caataacaca 29940
acattttgtt ttcaacataa cataaaagcc atcaaaggct ctctagctgg cttgcaaata 30000
ctctgatatc cctgactctg ctaatgggca aacagaggaa tgtcttggca actccctcaa 30060
gtgggagttc tgaggagaga ggaaacattg ttggtctgta gaagaaggga agattcagaa 30120
aaactccttt atgtgcaaat ccataatgca ggtgaagtgg ggggttgcag aaagagcgac 30180
ttcactcaag agagttacag aaaccccctc accctggaaa agatactatg cttatggttt 30240
caacagaata gtttcatagt atcttcacag tcttgtttag ggtgtgtgtt gtctcccttt 30300
aactctggca ttcttgaaaa tgtttgcttc ctcagtgaga tatccaccag gaatttggcc 30360
tttgccaaat ggtgctattt catatgagca gagtttagag acaaaagatt tattccaata 30420
agtctttttc tgcagactta attaccagag ataaacatca gggtattatg taacatatct 30480
gacctcatta tttcatttga tacctattga gtttccacca aagcaaggat actaaaattt 30540
cagggccttt ttcctcagtt atatcctcaa gggtgtcttg tattctttat atagaattca 30600
cttaaacttg gaagtcactt tgctcattag ttggtagtct aaggaacttt ctacatgcaa 30660
aagattctaa agccattact aatagctgta tctcttaaaa tctctctacc ctgcatgagt 30720
agggcctttt cccataaatc ccaaatcccc aagtgactga taggtaagcc aaggtataaa 30780
gtgttaatct gtcacacaca gcatcaccaa gtgaacaagg cagagtcagc gagcacagtg 30840
gcccagtgcc tgggctgttt cttagtctaa actaatctgc tagagacaaa ccagaaatgt 30900
ggagtttggg cttcgacacg aaacagaccc atgttcaaat acagctctac cacttgccag 30960
ctaagaaacc ttggcagtta cttatcctct gtgtgcctca actgtgtttt gtgtaaaatg 31020
gagaaaacaa gacatacctt gcagggttgt tgtgaatgag acaataaata ctaaagctcc 31080
tagcatatgg gaagagctaa aaaaaaaaaa aaaaaatggt agctattatc atgattcagt 31140
aatcaggtga attaaaggaa gtcaaggttc ctaccacatg acataggatt gagatacagc 31200
tccctcaaat acctcttccg tccttacagg atcatgcaaa ggaggaagtc tcttctgact 31260
tggtgtcttc ttcatgaatg tttgggccag gtgtcttgag tactcattct ctctggcctg 31320
cctggacacg gcaaggtgaa aaacagagtc accacccccct gggaatttcc acaccagtca 31380
tcgactctag cattctcaac tgccccccaga cccatacagc ctcctgctac agaaactacc 31440
agggccctaa gtcacagtca gcatggtaac acacatacta tatataggaa aactttttttc 31500
ccaagaactc ttaagttatt agtcgatttt cttaattggt taatagcatc attttgcagg 31560
tcaggaaact gatactgaaa gaaggttgtc ttgcccaaag ttgaaccacg ggagttaaaa 31620
ataaaaatat gaacatttgg ctgggcccca tggctcatgc ttgtgatccc agcactttgg 31680
gaggccgagg caggcggaat acctgaggtc aggagttcaa gaccagtctg gccaacatgg 31740
tgaaaccccg tctctactaa aaacacaaaa attagccaga catggtggca cacacctgtt 31800
attcgggagg ctgaggcagg agaatagctt gaacctggga ggtgaaggtt acggtgagct 31860
ctgagatcat ccccactgcac tcgagcctgg gtgaccaagt gagactctgt ctcaaaaaaa 31920
acaaacaaca aaacaaaaaa acaaaaaaaa ttcaagccca tcaattcatg gttttctatc 31980
ctttgagggt cccattttgc aaacttgatg caaacgtgtc caacggtacc ttcttcgaga 32040
aaacacccag tgacacgaga tgcctatcct gcaaggagtg ctggaggccc aggcccccta 32100
gagtcagaaa catagggatac aagtaaattc ctgtcaacag gatggaccat ctggtagagg 32160
acataatgag gtttataaag aagcctcctc tcctacctcc aagactgact tcttcctaga 32220
gtcatacctac aaagatgaac tcctaaaacc tcacttggaa atttgcctgg tttgccaggt 32280
gaacctggtc tcaggcattc acagccatca cagactacaa aagccatggt ggcctgctta 32340
ttattgggta ttccagggaa atagcaacac aggcctcatc tggataaact atccatcatc 32400
tccctgaagg gaggctacaa ggggaactag atggggagtc ccactctgtt cttgcaaggt 32460
gactggatgc tatgattctg tgctggtgtg tctgatccct atccctagag gtaccaacat 32520
tgccactatc ccatggttaa gaagctgctg ctggctagta ttgacaagtc agggctatat 32580
cgacagcctg aacaactgtg ctaaagccac ctctgatacc atattgaaaa acctacaggt 32640
atcattcctt gacttctgga aagagactgt gttctccaca tctccttcca ggaattaact 32700
gcccatctta tagagaccac acctgaattc actccagaga accaggtccc tgctgtggct 32760
atcataaaat gtccttaaat gagcaaaatt aaagcaaact aaatccggga ggggaaaaaa 32820
```

gggatattta taacaaggta tatctgaact ctaggcagca ccactctatt ggatacaaac 32880
caaggtcaac atgacctgca gatctctaaa tccaccctcc tgtactccaa ataaaagcta 32940
aaaaacaaaa acaaaaaaaa ctaagctcac caataacttt gggatagctg ttaggtggtg 33000
tgcagactca gtattctgag ttagaaatac tagcctattc cacaattttt gagacatttt 33060
agtgctgagt catttttaat actactacac atacatgata ctcaagaatt tcctgctgac 33120
cagcctacaa aaactgctta gggtttaggg tataaaaaca aaggagatcc aatttggagt 33180
atctggagga aaaatcttta cagaaataag atactttata gtcttagaat aattaatcag 33240
attcagcatc aggaatccag actagattct cacagggcaa ttatggcctc agaagacttt 33300
tcaaaccaat tatgaggtct agaaattact atggagaaac tgaccttggt tagcttctta 33360
tcatgacaca cgtgggctct ttttgcccct ctccactttt tttttttttt ttttttgag 33420
acacagtctt gctctgtcgt ccaagctaga gtgcagtggc gcgatctcgg ctcactgaaa 33480
gctccacctc ctgggttcac gccattctcc tgcctcagcc tcccgagtag ctgggactac 33540
agatgcccgc caccacgccc atttaatttt ttgtattttt agtacagatg ggtttcacc 33600
gtgttaccca ggatggtctt gatctcctga cctcatcatc cgcccgcctc ggcctcccaa 33660
agtgctggga ttacaggcgt gagccaccgc acccagccgc ccctccccac tattaacgtg 33720
aggacaggct gtttgaaaag gggcctggtg aaataatgat cataagtcag gacagtagtt 33780
acctttagtg ggatgggacg agagtgtgct ctgcagtgct gcaatgtttt atttcttgac 33840
ctggatggtg gctatttgtg catttcctat tactcataag ttacacatgt taataccttt 33900
tttatacata ctgtgtctca aaataaaaga aaaaaggaat tttagtatcc tgtaatactt 33960
ttactattcc acattatctg gaactggttt attatgcttg aaggtctatg tgaagccaga 34020
gtagagggaa ggagccctta gggttcaagc cacttgctgg agaacctcgc tgtggtcctg 34080
acagcgaaaa ctggggctgg agagaggttt caaagctcta tctacaatag ctcagaggca 34140
atagtataca agaagctgaa gctagtattt atggagtcat aagaatgact tgataggcta 34200
aaatcactca ttggatcaat gttcatagtg acctattcaa agccacaaca gcaagtgtat 34260
aagatacaaa taaccttaaa atgtacagtt agaaggacag aattttaaga cctttttgt 34320
ttttaagaga cagtgtctta gccaggcacg gtggctcatg ctaatcccag cacttttaga 34380
ggtgggtgga tcacttgagc ccaggagttc aagatcagcc tgggaaacat gccaaaaccc 34440
cacctttca aaaaaaaaga gaaagagaga cagtgtctcg ctctgtcacc caggctagag 34500
cacagtgaca caggcatagc tcactgcagc ctcgaactcc tgggttcaag tgaccctcct 34560
gccttgcagt gttccaaagt actgggatta tacatatgag tcactgtgcc tggcccttta 34620
agatatctca tatcactcag gagcttgatg aggtaagagg agaatgcact gtttggggtg 34680
ggggcaagga tctgttttgt tttgttttta aattcaaaag ggaatttttt taacattttc 34740
cagattggga accaatatac aagacatcat aattactttc ccaagaaagt cagggtcctt 34800
aatactactc cttctaggcc caaatcagag gtctgttttt tttcttctct tccaagctta 34860
cctttgtaag ctgaaaagaa tcggtaagac ttgccgccaa caaacttctt aggagcagta 34920
tttttgtttg ccactcccaa accccttgtg ggtgagggag atgttgggag acaaatgtgt 34980
gcttaaatat gaaaatcaaa aggctattta tcaaagtgtt aacggtgatt acctctggat 35040
agcaacttac ctatttccct tgttcttttt cttatttcca aattttctat attgaatttc 35100
taggtagtaa aacaacaatg aattattgtt taaagactga ttaaccggtt ccttcttcag 35160
aaactgagca taaacacttg tcctcgtctc cccatggctg agacagtggc cctgcctggc 35220
accatggaca gcaatcaggg aagggtgaaa gggcaagagt gggacaggcc aggactccag 35280
gtgtacactc taccccgagc cgaccacttc aggaggaatt cctggggaaa tgcaaaatca 35340
tttccaaatc ttttccttcc ccaccttcca gtcaagccat gaatctccat ttaaagcagc 35400
cactcactcc ttagttcttt taaacattta tttatctact gtacaaaata tttacatcat 35460
cagctgcaac tgcctggccc tttcacctgg cctgacgaat ctgcagcagg gcttggtctc 35520
tgccaatttt ccagaaacat gctgacactc tcctaggtat tcactcatgt ctggtctcct 35580
tcaaagacgc taaaaggcca gaagggtagc tggcccccca agtacctggg tcacaaggac 35640
ataaataaaa gaactggcca aaataagaaa cactaataga aaattgccca agaaataaca 35700
ctctctcatc tctttgacat attgtacctt ttccccacac tggctagtat gaaagcagga 35760
ttagaaaaaa aaaaaacaaa acagtaaaag aaaaggccca agagagcaaa gatacttttg 35820
aatagagttc agacagagaa tagaaaacca caatagtgcc aaagggtttt gtttaacaaa 35880
gactggtgcc taaggaccac cacagggatg cagcttccct ggttgggttg caatggtgcc 35940
taagatgctc tgaaaaagtg ctatgactag agcttaaaat gacaggtcta gccaagacaa 36000
tcaactcaat ctccaggatc agtctctgga aggctgccgg aggggagaat tcagaaaagc 36060
gactgccaaa aacagcagaa ccagtccacg tgccccccagc tggaaacccc tttctgggcc 36120
caaggggcag cattatgttt cccagggtgg tgataaacct tccggtacat atcccatggg 36180
aaaacatttg ggcaagcagc agcaccttgt cctggtccct gaggagagca gtgaccatgt 36240
ggcatggagg atcctggggt atagagaccc tgatgctgga tcccggaccc caaggatgag 36300
tgaggtcagc acccatgtgc caagaaagga caaatgatga ctgcgaaaga ctgaggtcag 36360
gcaggaactg tagcagctca ggagggcttg ttcctcatga tccctgcgaa caggagggct 36420
cagacatgct tccaggaggc caaggcattg ccaaagtcct gccttgtttc aggactctgt 36480
gtacttgctg gttttggcaa taaagatggc ttgtaatatt ctcagagttg actgccccat 36540
tgggaatggt agcttgcagc aaagccttct taaaaacaca aatttgggaa gtcaatgaga 36600
ttttgaatct tgaaattatt ttcaatcaaa cagtaatgaa aaaggatgtt gtcatcttcc 36660
aagatacagc agcaggtact cttcaatcat cattcaacag tgaacctaat aagcttcctc 36720
cctccttagt tctccttcct gataactgat ggagcaaagg ggagtacagg ggttggcagg 36780
tgtttactgt gtaacatatc acctccatgt tcatccctag tgtcctgacg ccaaggacct 36840
gtgataagca cgtaaactcc tagtccctgt tcctttgtct tagtgctgcc agagaacacc 36900
aagcagagag agaatcaggg attgctggca tactattctt actgttctaa tcttttgcta 36960

caaaattttc ttcagtagaa tgctaaagga tccccagctt ttagcagact acataatggg 37020
gaagtcagtg gcccaagccg gccatacttc agtgccaata acaaactcag ggaaaagcat 37080
cagtagtctc caagccagcc atatgcctag gcatgcccca ctctggaggc tacagctcag 37140
ctatccttca tattcttcag tctatctgct tttctcactg gctacaattc aagaaaaagc 37200
ttacagagtt cttctttgtg gattaataat catttccaga aatgcccgaa gggaaagtgc 37260
tttttccagt tctgacacca caaaaaaata ttctttttag cagtccttag aggcagtcca 37320
gtgccatggc ctatgcctac cattgtgcag cgggactgct cctatgggcc acctcctatc 37380
tgtcccccctc tctcctggct cctgatgaga agaggcaatc accccaccca tgagaaggac 37440
tcaagtacca gtctggtcaa gtagtgaggg ccaaagaggg tgtctccaag gagtatcagc 37500
agagggagtt ttggagcaca gtgtggattt aagggtctcc acaccagttt cccaacaggg 37560
ctgagccccg tgtgccatct ccctcagcta ctgagatctt caaaggacca aataaatgat 37620
agcagcatgg tcctcttctc atgacagaat gaagagctca gcttacatac catgccaaag 37680
tggcctgtgg tagatatggg cagggagcag gtgaggtaaa gacaaggctt gtaggtgaca 37740
aaatcctcca gacacagggg agcatgcggc atcttctggc tgatggttat gtgtgttagg 37800
atcagtggtt atgatgtctg taacttgcgc ccagaagctc cagagagcat gggagccaac 37860
tggatgacag gagagtggtc agagcatgcc aaagccctcg caaccctcgc tgatggccag 37920
ctgcagcatc ctgtgcacct cttcatagga gtcatatgta gggaggcaca gctggttaaa 37980
actggaaggg caagggagaa gtgattaact cataccacca aaaacactgg gcatgcatca 38040
ggcgggcaga tacctaccat gtgtgtgcag taggcagcgt gctatgggtc ggagcggcaa 38100
taatctgaaa tgagggacag agggcggcaa agcctccagg tggtagctga gaggagcctg 38160
ttgtgaactg aagtagccga gccaactcct cctgggtcag actggaaacc acagtccaaa 38220
accacctcat gacctggcag ggagagcaca aggctgggat cacacacagt aaacaagcta 38280
ggattagatc cctggggaag gaacaagata cactaaagtg gggctgtgcc ccagttaccg 38340
ttctatgagg acacagggta ggaacagttt gacaagcact tcaagaattt acactgtcac 38400
atgattagga gggtagtcac tgactgttgg ttctctttct tcgccatctc caatttacta 38460
ctcctgtggc agtcagcatg acagccctta gggatcaggg ctcccttctg cctggacaac 38520
cacctatctt tccccatgca tcattttttt ttcccagaaa acatatcaac tgctttgact 38580
cccctacttt tttcttcttt tcctttcttt tttttttttt tttttttttt gagaaagagt 38640
ctcgctctgt cacccaggct gcagtgcagt ggcgtgatct cagctcactc cacctcctgg 38700
gttcaagcga ttctcctgcc tcagcctctc gagtagctgg gactacaggc atgtgccacc 38760
acacccagct aatttttttt tttttttttt tgtattgtta gtagagtcgg gctttcacca 38820
tgtggccagg ctggtctcaa aactcctgac tttgtgatcc gcctgcctca gcctcccaaa 38880
gtgctgggat tacaggcatg agccactgcg cccggcctcc tctacttttt tcttctgaaa 38940
ctttttcctt ataacatggc acaaatccaa ctgaaagaag tttggtccat ttaccttttc 39000
tctgaaatgc catgagccac caacaactac tgcatgggct ttgaagtcag acacactgat 39060
gtctccagtc ccacacatca gcagctggaa aaagatggta aggtgttact atgacagtca 39120
gagagacaaa gacccaatat aaggtcaaaa ttacctaagt taacaggcaa aatgtagaag 39180
aaaacattac caggaaaaag acagaaggtg aggtcataat gccagaatac tgctttagga 39240
aaatcaatta catacaacag ggcaatagtt gctaaccagt gggtaaaaaa aagattcaca 39300
tggcttttaa aatatatatt tatacaagtg ttggtggccc tacctgggac ctctaaatct 39360
gaatctctcg gaacaggtta tgagcttata caggaagaaa aaaaattgct caggtatttc 39420
tttcttttct tttttttttt ttttttgaga cggagtctca ctgtctcggc tcactgcaac 39480
ctccacctct ccaccttgca tgctcaagtg attctcctgc ctcggcctcc tgagtagctg 39540
ggattacagg cgaggtattt ctgatgaaca ccctaagaac tgctactatg gattattttt 39600
aaatgaaaca aaggacaaaa cacacacaca caaactgttt cctgacagtc tgctctaaac 39660
ttagcctggg aatatcaagg agaaaaggca agctgtttcc ccaaaatccc caggcccatt 39720
tatgaatttg tcaatgacct ttcctttttc ttaggatgca ttcttttgtg gattgaactt 39780
ttactacctt cccttcagct tctgcctcta cttgatcaga tacatttaaa agtatgccaa 39840
aaaatgagaa cattaatgac atcagagcct taaacatctt gttaatgtcc taatttggtt 39900
caggatccag catgttactt agcagactgc aagtactcat ccaatagcta taaagaataa 39960
tacattctaa ccagagacaa gaagagccca aactgagcct gagaagcctt ggctccttca 40020
aaaagaactt gctaagctga ccaggaacta agagcaggat gtggaacaac tgtaagagac 40080
catgtccttc ctttctactt acttctacaa attgttacaa atgctttgct tcaaaatgta 40140
acaaaattta ccagtagtta ttcctcagcc caaaaaataa cagtgctagg taacaggttt 40200
atcagaaact ttaggctgag ctgtggagag ttcattttgt ttggatttca catctgaaga 40260
tataaaacat gaaaattaca gaagattttg attcttgttc tcaaatctac ttcagttaat 40320
agaaataaga attgggcaag accacctaaa attacttttt cccccatttt aaaagaaaac 40380
cttcctccct aaagaaacgt ttgcagaaag acactcctca tagaaactac gggaatttat 40440
aactagtaac ttgcactgca ccattttgta ctgatgtctc atctgtgagc aacaggccac 40500
aacaaattga tgttattctt tgcccacacc tgatacggac ttcagttctg catagaatgg 40560
tatttaccat cttagagcca ttttttagtt gttacaggat ctgaaataag aatgaattta 40620
ctatataaat aaaaatatat actatatact gttttggtct aatgcttttg aaatatgatc 40680
tcatcttttg gtttgtttcc ttcacttcat tagaatgtag cccatgaggg ctggaacttt 40740
gtcttattcg ttactctctc tgcagtgcct caggaggcac tcaatatata cctgaaaaaa 40800
taaattctat tactccctta aatgtcatag gattttatag acggtacatg atattaacat 40860
tctttactac tcattgattt tttttttttt ttttttgaga cagagtctta ctctgttgcc 40920
caggctggaa tgcagtggtg cgatctcgga tcactgcaac ttccgcctcc ggggttcaag 40980
taattctcat gcctcagctt cccaagtagc tgggattaca agcgtgcatc accacaccca 41040
gctaattttt gtattttcag tagagacggg gtttcaccat gttggccagg ctggtctcga 41100

actcctgacc tcaggtgatc cacccacctc aacttcccaa agtgctggga ttacagaagt 41160
gagccaccat gcccggcctc agtgatactt gatgaataaa tgagtattaa ttattactct 41220
tcataaataa tgagctactt gctactttca tttatttatc ccttaagcta atatctctgc 41280
actctttgct actaattttc acattctaat tttttaaagg ctgaagggtc aatgaatgga 41340
ggagcgacaa aagtgaaacc acctcttgca taaatggctt tccagctgca tctcataaga 41400
gaaggtacac caagctggtt cctgtaagct acaaaaatgc caagttaaga atgggagtct 41460
gaattgcaat cacttacctc aagctcattc tcatcaaaaa tagccaaaag gttctcaggg 41520
accaattcat tcaggcctga aacaaagtag gcaagttaaa gtcataccca caccatggat 41580
acaggtattt taacttcaag gccaagaaca gacaggatcc caccttttag gaaatgttcc 41640
acctcctctt tcacttgact ggccagccga tattgggcca gcaaatttaa atagaagatt 41700
ttattcgcat tggtgactgg agtttgagct ccacctgtca tgagttctac aacctgtaac 41760
aggcagcaaa agacagacaa gggagaggct gtggatggaa atttatgaga tgacatctgc 41820
ctggatgttt tatagacaca gattactgac ctccatcact gtcagtcaca agtttctgct 41880
gtgcattcag cttttcaaca cacttggttt aatttagttc ctaagcttca gggttcctac 41940
cactattgct gtatactagc tattatttct gaaatgggtg cctgggtaag gtcttctctg 42000
actcagtcaa gtagtagtga tgcaatagga agatactatg gctcctcgct agcaatgttt 42060
taatctatga tccaaaatga aaccactatt gacctcttct ctaagcaact aagagtaaaa 42120
tcagataagc agtaaggaag tctttttatg tctcagaaat tccaagatgg gaacaattgt 42180
tcccacacat tcttttatct ctaatgtgag tcaacagaac aagccacagt ttgatacttg 42240
aagatcctgg attctaatct cagccctgat gctagcttgc tacagacctt tggttaattg 42300
cccctccaga ccttagggga aattatttgg cctgaacttt cctctctgca aagaaaaagg 42360
tgaacaaact gtatgactct caaacagaat tggtatcccc caataaactc ctaagggaac 42420
tttgaaggag ccctgaagca cagcttaaaa ccatcatcca ggaatatttg tgtagagtga 42480
ccaaggttgt ctccacaggt gaatgaagtt acaagatcag attttaagac aacagcatgg 42540
tagcaatagc atgctactcc acagcaggga ttctcaatgg atctaaaaat gtaaaacatt 42600
ttttctgttg gtatgcacaa tcctggattt ttttttaat gtaagcattt ctatctagga 42660
gactgagaaa taaattatta agatttagta ttcatattaa acacaattaa aatgagcact 42720
accctgggtc tgaggaccct aattcagtgt cctattgact ctagctgcct taccatgcca 42780
gctcaggaag ttgggtctaa tcccatgtgt acaaaggcag acagctggga atcccataat 42840
ttccagaaag gatatctgat tatcagtata gtggtaccaa tggtcacaat ttttcaatca 42900
ttcctattct gcttttaact ttaaatttac tgcaacttta gcgtcatcat tatcatttga 42960
tgctgtttaa caccacaatg taaatagaag cacatgcatc ggaaatatct ctgggccatc 43020
attttctaat aaaaagtttt ggttttatta ttttgagata gggttttgct gtgtcaccca 43080
ggctggagtg cagtggtgca atcacagctt actgcagcct taacctcctg ggctcaagcg 43140
atgttccagc ttcagcgccc acttcctccc acactacccc agtagctagg accacaggca 43200
cacgccacca tacccagcta gatttttaaa atctaaaaga cagggtcttg ccaaagttgc 43260
ccaggctggt cttgaactcc tggtctcaag ccatcctcct gcctcagcct cccaaagtgt 43320
tgcaattaca ggccatgagc cactgcgccc agccaaaaag tttaaaaata cccaggcatt 43380
gtgttaaaat aggaatcacc tacatcacta ctgcaaatgt tgacacagac actgccatct 43440
cttccaaatc tgcttgtaca gtgacacttc accatcattc agttattttt caactggaag 43500
caggactcgg tgggatagat ggagatatgt gtcttttgct tcactcaaat gggctgagat 43560
cataaagcat aggatgcaat gtcctaaagc tgtgctgtcc aatacagcag ctgctagcca 43620
agtgtagtta tgtaagtata cattaactaa aatgagaagt tcacttatta tgtcacccac 43680
attttgggtg tttaatagac aacacaaata tggaacattt ccaccactgc agaaagttct 43740
cttagacagt cctgctctac aggaaactgg agaacatagg acccagaggc tttgtcctct 43800
ttctcacctt atccaattga cctgatttat tatatttctc ttctgcaaag accagctcca 43860
tctcactcat gtcattgttg aggataaaac aaactttaga tttgtagaat tctgggtcat 43920
ctgtttcaaa gtactgagga ggcagaaaga cacagaacag aacatgaata ctcttgccct 43980
gaaaggtgta actgcttaga accacttatc ccttcaacct taaaaattat ttacaagccc 44040
aggaggagag atttcagggt taatggccag gggccttagt aggaaatgca cacccaagat 44100
gttaccttgt aatgcatacg cagtcctatg atttgggcca ggaaagagcg ggtgaagcga 44160
gctcggacca actgcttgta ggctcctcct agagaggact catagagaca cttgccccacg 44220
agccgtcccg caaactcata catttcagg cgcagatgag cggggcgatt agggttggga 44280
tgcacctgtc caagaaagag actgaaaggc cctgggccat ttttcttcaa gcccagttct 44340
ggaaggaccc ttccttcccc aaccccatgc caaaggaaca agatgtcact gtgaggatca 44400
gagcccatcc aaatgttagt tttttggtag ccaccactga gtaattcaaa gtgactagaa 44460
gataaagggc aaaggtggac ataattaaga atcttatatt aagtttgtgg ttaatcagca 44520
gatgatgtga tgccttttcc ctaaaccaga acttctttca gccaaagtgt acttctctac 44580
tcctgacacc tttgtctaga caaagatcta gtacaggaat catttgcttc aggtatgttt 44640
tgagaaagag gcttaattat tataataatt agtagagtag acattatact ataatgtttt 44700
tagtcaaata ttcagctgcc tgcattttttg cttattttac agatgaaata ataaaggcaa 44760
agaggagaaa gacaatctga ttagcttata ttttaagcag taaaattaag aataggacac 44820
aggttgtcag gttcccagat cagtttcata attttgaatc cttgttagtc tacccatgat 44880
aacataatcc acattttcca cccacccgtc accaactcac taatgcttgg ttgttgtcac 44940
tgaaccgggt gaagagctga ttggtggtat caaatagtgc tttgcagatt agctcaaacc 45000
attcccggcg aggccctccc cagtccagag ctgaaaagca taatgaaaat aaaaaatgac 45060
tctgccctcc ctcctcttac cacttttgtt tattgagtca tcattttata ctctccacat 45120
tccctctatt gtactactct ggtgtttctc cgttgcgact catctaaact gtggtccttt 45180
agaaatccca ccccttttg taggattaat ggtattctga tcactaagga aaagcccct 45240

agtatggaaa caccagaggt cggagatccc aggaaatgga catttctctc aaggtaacca 45300
cggttcaaac acttagaact ctctctccaa gtccctgtat gtctgtgtac cacaagctcc 45360
tcaatgccat cctggccagt aaatactacc cttttttct tgactcatgg ttccctttc 45420
cttggggaaa aaaaaaccta ggtttttttt tttagggagt gagtaggagg taagagttgc 45480
ttttttttt ttttttttt ttttgtggag aaggtgcgag tggggaggag agttcaaaaa 45540
gatgaatata aaatttcaaa actgaccttc ttcatcctgg aaaacaacct caaagttctt 45600
gctccaatct gagatggaga aattccgagt ggctttcaga gactgaaaag aagtgaaagg 45660
aagggaattg tcagaacact ggctggtgtc ctctggatgt taaagacaac acaagagcac 45720
tgccattgtc tgctggacta actacgactg cctgaaggag atatgttact cataaacaag 45780
ctttaatgat accacctgac atttaaaatg cacataactt caaatttaaa gttttaaaaa 45840
ataatctgat ttgcctttac atcatcactt tgttggaaag ctgaagttgt ttttttcttt 45900
tcctttgtt ttaaacaatc cttgttttag caatgaaaac attcttgcat aaagaagtaa 45960
aaaatgactc aagtcagtaa gctgtaggca aaagaaccaa ggaatcctgg ttttcagcaa 46020
agtatgatct atccactaga catatttatc cacaaggcta ggttcagaca gcgagccatg 46080
ccacacggag aatatggctg agaaaacggg tttccagtca cacctaccag agccaaacat 46140
cacaaacact ttctggacag gaaccaagcc ttctcattcc tggggaatgt aagaactatg 46200
ccagtgtctg tgcagtggag acagacacac atcaggtgtt ccttttccat cagaccaaag 46260
tgacaacaaa aatcatttcc ttttaattca acaagcgctc tattccaggg attcaataac 46320
aagaagtttc ttctgtaatc actatttaca cctgccctac atgggagttc caagggaccc 46380
attcagtaga tttgaaggcc agtaagtaaa ctgctcttcc ctcgataaca tacctccaac 46440
aaaactgccc caaatttagt agaatcaaat agaagaacag acatatgtaa agagagaaat 46500
ttaggtttca gaaatttagt atatctgtct tttataagta tatctgtctt ttataagtat 46560
atctatatct ttacttaaac agagaaattt aagtttcatg aaaaagataa ccagatcagg 46620
catggtagct catgcctgta atcccaacac tttgggaggc tgaggcgggt gtatcacttg 46680
agtccaggag ttcgagacca gccggagcaa tatggcaaaa tcccatcttt acaaaaaata 46740
caaaaattag ccaggcatgg tgacacatac ctatagtccc agctatttgg gaggctgagt 46800
tgggaggatc acagcccggg aagatcgagg ctgcagtgag ccatgatcgc acctctgcac 46860
cccagcctgg gcaatagagt gagaccctat ctcccaaaca aaaacaaaaa caaaaacagt 46920
aacctgcaga atatagacat tcaattggaa aactttggta tctgctgaca gagatgaaat 46980
tttacctacc gattccaaca aggcatgtct gctgaccttc agggtgactt tggaatgtgg 47040
tcttttcata tgtacctgcc gaagctctcg ctggaaaaag ttcaccttgt cctgaaaggt 47100
ctcagagcct cctggggaac agcaagatcc atcattacag cctcattaca agccatctgt 47160
gtagtcctgc ctctccaccc tcatggatgt ggctgggctc actaccttcc ctatagaatc 47220
cctgtgtaac ctcacctatg ttcttatgca gggagcggat aaaagtggct gctagaatgt 47280
tcctctcctt acagctgagc tccacaggag gttgaatgcc atcatccacc accagtgtga 47340
gcagcttatg gacagggtca ggaccaaggt atgaaaactg gtaaagaaaa acaaagaaat 47400
taatctataa aaccctcaag gccagtaaaa gtcctccaca gctctatcct gtgtaccaac 47460
caacaacaat aagttaggct tgttttctcc aaatgaggac ctaccagaaa tacatttcat 47520
tatttttccc ttcagtgatt tacatttcaa catttaaaat tattttagtc acagagactg 47580
cctttagtta ggaccaccag caaaactaca gaccttctaa cataaaatat aagaagggtg 47640
atatgttaaa tagcaataaa tggcaatacg gtgatatatt aaatagcaat agtaaggata 47700
tattaaacag caataaatga ctactccaaa ctgatcagaa gaaaactatt tttataaaat 47760
ggcacattct agcctttggg caaaagttta gattccagaa gcccagaaaa gttaacaaca 47820
aacaaacaat gtattttaat atatgagagt ggtgcttcta aaaggagtta gcttcagcac 47880
actgggagac caagcttggg ccagagttac aaacatcttc tctattaact tagtcttgaa 47940
ggcaaaggat cactgttgaa gtattctgag ttgctgctaa ttcctgtttc tgtaggcatg 48000
cgtccttcca gaatttatat attcttcatg ccatttagtg aggagataat gatgctttat 48060
ttggcaacga atcctgtgta cactgtgaga tcttgcagaa acactaaaag tgcttctgag 48120
aaaaaataaa aggctgagat agtccagaga tgaagcatgc ttgatgtgaa ccagatgtaa 48180
ggttcagctt acttttgttc ctggacacac tcggaaggtg taaaggcgcc aggggatgat 48240
cttcaggtag aactccttca ctgagaattg ctggaggacc aacagacagg aaatgaagtg 48300
aatgataaat aaaaaggctc tatttgggta ttctaattca aatatcatag tccctattta 48360
catttctcat aacctttcct ctccatggac aaaagtaata atttagtgag tactaaagtc 48420
aaataaatac attaactctc tatttaaaaa ggcaaattaa aaatattacc ctgatggcca 48480
agtgtggatg gctcacatct ataatctcag cactttggga ggccgaggca ggcagatcac 48540
ttgaggccag gagttcaaga ccagcctggc caacatggca aaactcagtc tctactaaaa 48600
atacaaaaaa attagctggg tgcggtagct catgcctgta atcccaagta ctcgggaggc 48660
tgaggcacaa gaatcgcttg aacccgggag gcagagggtg cagtgagctg agattggacc 48720
atggcactcc agcatgggtg acaaagtgag accctgtctc aaaaaataaa ataaataaaa 48780
aaaatatgac tgacaatgca gtttcacaca gagatatttc ataattgtaa tactcaagtc 48840
tttctaaata tctgggatca ggttttcact ttatgagaac agaatatttc acctagaaac 48900
aatcaaaatc tccttctgct cagccccatg atgctcagaa tgacttcctc caagttactg 48960
gatagcaaac accagcaacc catacacact gctttatacc tatttgaatg gtgccaagga 49020
tctgccaggg aagggaggca agatggggtg agctggagtg gaatatgttg gtggtatagg 49080
ggaaggtcct cacacaaaaa agttcataca aaagaggaaa agacaatgca aaaatttttt 49140
aatcttaaaa aatccctttt atctgaatgt gcaggtcagt tgttttcttg ataaaaaaaa 49200
gtttaaagaa aatttcaaac acatactaaa gaagacaaag aagtataatg aaactatcag 49260
ctgcttcaac aattatcaat tcatagccaa tcttgtttca tttattcact tacccatttc 49320
cctcattacc atcacccta gtccaaatta aggctagatt tttaaatgaa gaatggtaca 49380

cagcaaagtg ttctgaagta gaacagaatt agaactcagt tttggttatg agattaaaga 49440
gatgcaatgg gatgtttcac agagtctcat ttttatgagg actgaatgaa taaggaaaaa 49500
caaatattaa aaagaaacta acacaaagtg cttcttagct caggggcttg ggaaattcac 49560
cttctttcct ctgccaaaat ttctttactc caatcctgta gcctaatagt gacatccatt 49620
tactgtggtt tcatgaatca cagaacaaaa gatcaaattg ttttgacaca gcacaagaag 49680
ccaacagtaa tttttatgct agcaagaaat ttttaacagt tgaaagtggc tttctataca 49740
actagttaac aatcttgccc tgattctcag actgttactt tgtggtttct gctaccaaat 49800
accaattaca cacaggttca agcaggggga ttttatgtcg agaatggaag gtccaacctt 49860
tggtgacaca tagcagtaca ccttcttcgg tttcttcacc ttctcagggg tgtggcactc 49920
agagggcgag tcttcatctt cctcgtcaac agcagtggat ggccggcgct gggaagaggt 49980
catgtgcatg ggtggcaggt gccatggagt gctgctacag ttggtagcat tataaagata 50040
agcctcaaag taaatgctca cgcctgaagt ggacacattg cgttcgacga tattcttctc 50100
atcctctgaa gtataataag gaataaggtt taaagtagaa gtcagagaag aaaaaaatta 50160
cctatcacag gcttttagtc cacaggacaa gccaaatgtg gcccaaatat caaatgtttg 50220
tgaggtcaga caaaagagtg caaacaggca aagaaacggc tgaagaacag cactaaatca 50280
tacttttctt ttaacttacc acttaggaca ataatgtcaa attcaccatt attgattggc 50340
tgattttggt atgaaatgca agcatggaag cagcctcgag aatgcagggt gagtcgcaag 50400
aacacctgga aagtctgcct gttggatgtt actgatttct caaatgagac accagtactc 50460
tcttcttctt gagggccgag ctataggaag gcaacagagc atcacttaac atatagtatt 50520
ttcccattta caagccactt tactccttac aaagagcttt cctatctatg atctaatcta 50580
aatcacaaaa ttactaatga gacaggtatt gtcagatgaa gatacagaca gcacagaagt 50640
caaggagaag gaaagagaga tcgaagtgaa ttaggtgtga gaataacaga gaaaggagcc 50700
agctctctct agcacacaga taaagaaggg agactgctta cctcatgaat ggacaaggtg 50760
taattgtgct catctctcaa ggacatggaa ttgttggtgg gattatcata ctcatctcgg 50820
ggtactattt gaagggtgtg cggctgccca caggtcaata caagagtaga aaagtggcac 50880
acaattttgg tcttagaagg aaccaccatt cctggaacaa agacaatgga atctaacatt 50940
tatttatttt atttttttat ttttattttt tttgagatgg agtctcgctc tttcgcccag 51000
gctggagtgc agtggcgcaa tctcggctca ctgcaagctc tgccttccgg gctcatgcca 51060
ttctcctgcc tcagcctccc gagtagctgg gactataggt gcccgccacc atgcccagag 51120
aatttttttt tgtattttta gtggagacgg ggtttcactg tgttagccag gatagtctcg 51180
atctcctgac ctcctgatcc gcccgccttg gcctcccaaa gtgctgggat tacaggcgtg 51240
agccaccacg cccggctgga atctaacatt taaaggtgct cttcagggca caaagatctg 51300
ctcagtgggt cctagaaagc taggtttcct cccaattcct agggataaat aacactatca 51360
agaggaagag aggccaggtg cggtggctca cacgtgtaat cccagcactt tgggaggcca 51420
aggccagaga atcccttaag gatcccttaa gaagttcaag accagcttga gcaacatagc 51480
aagaccatct ctacaaaaaa taaaaattag ccaggcatgg tggttcatgc ctgtgtccca 51540
gctactcagg aagctaaggc aagaggatca cttttgccca ggaggtaaag gctacagtga 51600
gctatgacca caccattgca ttctagcctg tgtgacaaag ggagaccctg tctctctcct 51660
tttttttttt tttttttttt tttgagacag agtcacgctc tgtcacccaa gctggaatgc 51720
agtggcacga tctcggctca ctgcaacctc tgcctgtcgg gttcaagcga ttctcctgcc 51780
tcagctgagt agctgggact acaggcaccc gccactacac ccagctaatt tttgtatttt 51840
tagtagagac agcgtttcac catattggcc aggctggtct caaactcccc accttgtaat 51900
ttgcccgcct cggcctccca aagtgctgga attacaggcg taagccacgg tgcccagcta 51960
accctgtctc tttattaaaa aaaaaaaaaa gaggaaaaaa cagaaataga gaaatttaaa 52020
tgaatttggg gaattatttt tgttacaaat tcttaaccac tgcaaacccct aagtaaaatg 52080
aaaaaaactt ggaagaaata aaacaaaaaa accaacaaac aactaaacaa atggagacat 52140
gtttatggaa gatccaacat agtacagatg tcaattttcc ccaaattgat atataggttt 52200
aacacaattc ttatcaaagt tccagcaaga ttatttatag atttagttaa gattattcta 52260
aaatttaaat ggaaaggcaa aggaactaga atactaaaac aattttgaaa aagaagaata 52320
aagtggagtc agcttaccaa tttcaagact tattataaga gctacagtaa tcaggctgtg 52380
tgacttggct cacgcctgta atcccaacac tttggaaggc caaagcagga agattacttg 52440
agtccaggag ttcgagacca ccctgggcaa catagtgaga ccctattcct acaaaaaaga 52500
taaaaaatta gctaagcacg gtggtgcacg cctgtagtcc cagctactcg ggaggttgag 52560
gcaggaggat cacttgaacc caggaggcca aggctgcagt gaatcatgat cgcaccactg 52620
cactcagcct gggtgacaga gtgagaccct gcctcaaaaa aaaaaaagaa aagaagaggg 52680
agctacagaa atcaagattc tgtggtactg gaggagggat aggtacacag attaatgaaa 52740
taagcccccca caaatatgtc caactgcttt ttgataaagg tacaaaagca atgtaataga 52800
aaatgaaaga tcacctttcc aacaaatgat gctggagtaa agacatgtat aagcaaaaaa 52860
aaaaaaaaaa aaaagaggac cttgacccaa atctattaaa attaactaaa aatggatcat 52920
gaccctaaat acacattttc aaactgtaaa aacttttggg aaaaaaagag aacatcttca 52980
ggatctgaga ccaaagattt cttagacacc aacagtttga ccataaaaga aaaaaatgga 53040
tgcactggat ttctttcttt ctttttttgag acagggtctc actttgtcac ccaggctgga 53100
gtgtagtggc aagatcacag ctcactgcag cctcaacctc ctgggcttgt tatcctacca 53160
cctcacttct ccaccctact ccccagtagc tgggactaaa ggtgcacgcc accacaccca 53220
gctaatttat aaattttttg tagagatgag gtctcactat gttgcccagg ccggtcttga 53280
actcctgggc tcaagtgatt ctcccacctc agaaagtgga tttcatcaaa attgaaaaca 53340
tgtgctctgt gaataactct gttaagggaa taagaagaaa agctcagcct gagcaacatg 53400
gcaaagcccc atctctacaa aaaatataaa aattagccag gtgtggtggc acatgccaca 53460
gtcccagcaa cttgggaggc tgagatggga gatcacctaa gccgggagag gtcaaggctg 53520

cagtgagcca tgactatgct actgcacgcc atcatgggtg acagagtgat accctgtctc 53580
caaaaagaaa taaagaaaag ctacagatgg ggagaaaata tttgcagacc atatgtttaa 53640
caaaggacta gcatatagaa tatatacaga actctcaaaa gtcaacagta aaaagccaaa 53700
atgggccggg cacggtggtt catgcctgta atcctagcac tttgggaggc caaggtggat 53760
ggatcacccg aggtcaggag tttgagacca gcctgtctaa cagggcaaaa ccccatctct 53820
actaaaaaca caaaaaacta gccaggcatg atagcagatg cctgtaatcc cagctacttg 53880
ggaggctgag gcaggagaat tgcttgaaca tgggaggcgg aggtcgcagt gagctgagat 53940
tgtgccattg cactctagct tgggcgtcag aacgagactc catctcaaaa aaaagaaaat 54000
aataataaat aaataaataa ataataataa tcccattaga taatgggcaa aagggccagg 54060
tgtggtggct cacgcctgta atcccagcac tttttggggc caaggggggtg gatcacttga 54120
gctcaggatt tcgagacctg cctggccaac atggcaaaac tctgtctcta caaaatatac 54180
aaaaattagc caggcgtggt ggtgcctgcc tgtaatccca gctactcagg aggctgacac 54240
aggagaattg ccagaacctg ggaagaggag gttggttgca gtgagtcaag gtagcaccac 54300
tgtacaccag cctgggcaag agcaaggatg tctaaaaaaa aaagaaaaaa agaaagaaag 54360
aaaagaaaag aaaagagaaa agaaaagaaa agaaaatgga caaaagacac gaagagacat 54420
ttcaccaaag gatacataga tgacagacag gcacatgaaa agatgttcaa tagcattaac 54480
tatttaagaa atgcaaatta aaagcacaat gagataccac tacacactta tcacaatggc 54540
taaaataaaa aataatgaca gcattaaatg ttagcaagga tgtgaagaaa ctggattatt 54600
catacattgc tggtgggaat gtaaaatgct acagccactc tggaaaagaa tttggcaatt 54660
taaaagaaac taagcataca actccaatac atctcagcaa ctgcatacta gagaaatgaa 54720
aatttatgtt cacataaaaa cctgtaagtg aatatttata gcagctttat tcatcatagc 54780
ccccaaatgg aaacaaccca gatgaccttt aacagatgaa tagttaaaca aactggtaca 54840
ttcagaccac agaatatacg tagagaatcc ctaatccaaa aaccagaaat ccaaaatgct 54900
ccaaaatcca aaacattttg ggtacctaca tgatactcaa aggaaatgct cactgaagca 54960
tttagggttt cagatttttg gcctagggtg ctgaagtggt aagtaagtat attgccaata 55020
ttccaaaacc cccaaaaatc caaaatccaa gacacttcta ggcagacata gtggctcatg 55080
cctgtaatcc cagcaccttg ggaggccaag gcaggaggat tgcttgaggc caggagttag 55140
aaaccagctt ggttaacata atgagactgt ctctacaaaa caaaaaatca aaaaattaac 55200
cgagcgtggt ggtgcacact tgtagtccca gctacttggg aggctgaggt ggaaggattg 55260
cttgggccca acaggtcaag gctacagtga gccatgatcc tgccactgca ttccagccta 55320
ggtgacagag caaggccctg tctcaaaaac aacaaaacat tcctatacca cgtttggaga 55380
gaaaaaaacc acaaaaccac acatctggtc ccaagcattt tggatgaaga atactcaacc 55440
tgtactactc agcagtaaaa aggaaccaac tactgataca ctcagtaacc tggatgaatc 55500
tccagaaaat tatgctgagt gaggaaaagt caaccccaac aggttatata ctatatgatt 55560
ctagttatat aatattcttc aaatgacaaa attatagaaa tggggaatag attcgtgggt 55620
tgccaggggt taagaacagg ttggggatgg ggggcaggta cggtggctca tgcctgtaat 55680
cccagcactt tgggaggctg aggcgagcgg atcatgaggt caggagatcg agaccatcct 55740
ggccaacatg gtgaaatctt gtctctacta aaaatacaaa aattagctgg gcgtggtggc 55800
acgtgcctgt aatcccagct actcagcgtg aggcaggaga atccactgaa ccagggagtt 55860
ggaggttgca gtgagccaag atggcaccac tacactccag cctggcgaca gggcgagacg 55920
ccatcaaaaa aaaaaagaaa aaaaaaaaaa gaaagaaaca ggttgggggg gataggtgtg 55980
gctgtaaaag ggcaacataa gggatccttg tgatgatgga aatgttctgt accttgactg 56040
tatcaatgtc aataacctgg ctgttatact gtactagtag ttttggaaaa tgttaccatt 56100
gcaggaaact gggaaaaggg tacaccagat ctctgcatta tttcttacaa atgcatatga 56160
atatacaatt atcaaaaaat aaaaagttta attttttttaa aaaggctggt acatgtaagg 56220
gtatgagctg ttctccactg gtggcaaaga ggtgggtgat gataattcat atgaaaagga 56280
gtatctgagt gaggcactta agcaaaggag aggatacatg gaaagagaga cggcttgaga 56340
gccactttgt ccagagcctt actctgctat cagagaaaga aaattaagga ctagtcaagc 56400
aaagatacac aaatcttaag gccccactca gtacggtttc acttcaaaag ttacccaggg 56460
accccaggtc ctattatttc tgtggatacc acagccctta gcccttaaga actaaccaaa 56520
ctaatgactc agcaatttta ctcccagagt aataaaaaca tgtcaacaca aaaacttgta 56580
cacaaatgtt cacagtagtg ttgcttacag tacccaaaag tagaaacaat tcgaccacca 56640
actgatgaaa ggataaataa aatacggcat gtacataaaa tagaatatta tttgtcaata 56700
aaaagaaatg aagtacagat tcacgctaca gcatgactga atctggttta gatatttgta 56760
ccctccaaat cgcatgttga aatgtgattc ccagtgttgg aggtggggcc tggtgggagg 56820
tgatggggtc atagggggagg atccctcatg aatggcttgg tccccctcctc atggtaatga 56880
ataagctctt gttctgagtt catgcaagat ctgtttgttt agaaaagccc agcacctcct 56940
ccctctctct ctctcttgct ccctctctca ccattaggca ctcctgctcc cgcttcgcct 57000
tccaccatga gtggaagctt cgcgaggcct caccagaagc agatgctggt gctatgcttt 57060
ttgtacaact tgcagaacca tgagccaaat aaacctcttt tctttataaa ttacccagtc 57120
tcaggtattt ctttacagca agacaaaagc agccaaacac agcaggcaaa tccacagagg 57180
cagcagatga gtggctgcct aggtctgggg gatttggggg aaaagtgact actaacatgt 57240
atgggggtttc tttttgaggt gatgaaaatg ctctaaaatc gactgtgatg atggctgcat 57300
ttaaagaacc attaaactgt atactttaaa aggatgaatt ttatggcatg ggaattaaat 57360
ctcaataaag gtattatttt taaaaaggaa agacaggatg gaaagagaga gagggaagga 57420
gacaaaggga aaaagaaagg aaaaaagaac ctaccaggtt gaaaaatttt gtagtaggga 57480
ctatatgcca catttaatcc accaagcttc actgtgattt cataacgccc agccttgcgc 57540
acagtgaagg ccacttttac tacgttggaa ttgggctcct gaaggacttc ctgggtcact 57600
ggaatttcca ctgctagctc gacatgagag atgtgaactc ttagtcccac aggccgatgt 57660

gcagggaaag gctgccgtt cttatagaat aactgccatg gggagaagaa ggacacctgt 57720
tagcaagcag aggaaaaaaa catttcctgt gtagagtgag tggggagaga ggaagtagct 57780
ggcagagacc acaccagtaa atcccagtac ctaccctgtt cctcacagtc ctgctcacaa 57840
aaacacagag agaaacaaat cccctcaata ctctaaagaa aaccattaag tcatgagcaa 57900
attcttaact tgttcagagg acaagggtag aatttaccac taaatcaaga aaatgtccat 57960
tttaacttgc cacttctgaa ccaccaatgc tgcctccttg gtcttaccag ccctttatat 58020
tatcaatgct aacccaaatt taaaggtaag ttagccggca gcactggcaa tgagtgaact 58080
aaagcgttac cagacaaagg taggaggggt tcagactctc aagccaagct taatgctaca 58140
gtggagaagt caatgcagga gttgggaaat tttttctgta aagtgccaga gagtaaatat 58200
tttggctttg tggaccgtac acaactactc aactgtcata actactcagg tgtgaaaaca 58260
gccatagtca atacatgatg aatgggtgtg gctgtgttcc aagacaaatg tatttaaaga 58320
aacagaaggt gggcctgatt tgctaacccc tatctataat acttactctt ttctgtaata 58380
tttttcttat tttgaaaagg taacattaag ctttatactt cttcaaagct aaggacctat 58440
taaatcaaaa tgacagagaa atttgtgact cttcagatag tagcaattca ctagttcctg 58500
gagggagttg aaaaacaaga gaaaatgtta acattccagg ccagcacctg ggctctggaa 58560
agcaagttac tcagaaaccc caatgggtat ggagacagaa acattccctt acatgcactc 58620
ggaaggccat gctgtggccc acctcatagg ggtccttcca atcccaggag actttgcaag 58680
accggggatc caggtaattt ccccgcacgt agtcataaat agtccggtcc cctcggcgct 58740
cgcggtcctc attctggagg aagctgacta cacgtgcggc aagctcaaag aggaacttaa 58800
ttgtgaagaa gaatgcaacc acagacactg tgattccacc tatgcaaaag agagaacaca 58860
caaaccgcct gccagttaca cattacagct tttcattatg caacaggcca gccatttctc 58920
aaaaattaaa ggaggtcagg atgagactgt aagactgcca aagaaacact gcactgggca 58980
ctacctctgt agataagaaa ggaacttcta caccaggggt cagtaactac agtcctcagg 59040
ccaaagccca cccactgcct attttggtaa ataaagttcc attgggatac agccacaccc 59100
atcatttata aattgtctat ggctactttt gcactacact ggcagagttg agtcattgaa 59160
atgagccttc ggaaccaaaa atatttactc tctggccttt tatagaaaaa gtttgcaaac 59220
tcttgctcta tatgagctgc atataatgtc cgccctgctc cctaccttcc tgcacttgct 59280
gggcacccca cagaagcagt caaaaggatg tgcctttggt tcccaccatt tccttcttaa 59340
agaatgcatt atttgggagc cctcccctcc cttgtgtact tggcaaaagt cttctccaat 59400
ccccaccaga gttagcagtt cccttttctg ggctccttca aactcctaca cattcatctg 59460
gtataacttt tctcctcctt gaccgttccc ctttatgtta tgaatgccta ctccttgaag 59520
gcagattttt gtcttattca actttagact ccacagtaaa cagtgtctgg cattgactat 59580
aattgctcaa taaacccaaa tgaatgaatt ccaatgccaa ctctgtgaag gtacattctg 59640
gcacgactag aaatattttc agctccattt tcaaagggaa aagaataggc agaaaggcaa 59700
aaaggaccca agcaaccttt acaaaaatat aatagagaga gctctttcca taacgtacca 59760
ataacgtaaa acatcaggtc ccgtcaatgc caacagacag ccgaggatca cagctgcagc 59820
tgttaaaaga aaacttgttt agtttttgtc tccaactctg cctcatagct atctcagagt 59880
aagacaaaga agagtgtatg gcgtgagcca aacaactctc tctagtccag agccttacta 59940
ttcctatcct ctttctagct tgacaggtgc cgtataatat atttcagaga aacaaagaga 60000
ctgcatcatt cccccttaaag acttccaatc tactgaccac tctaggaata ccacaaacag 60060
ccacttgctt tattgctaat ttcaaaatgc agtaacatgg ctttcatctt cttagatcca 60120
tagttcccaa gacagtttcc ctccctcatg caaagtggtg ctaattaaca catgcaaaga 60180
gggtaatgga gtcactttcc ccttatgttt gccttttggg agtaattcag ctaagtagaa 60240
atagctatat aaaatgtaga tttttttttt ttaaagcaag tatcattatc atcatgttgg 60300
cattatgtaa ttaaaatgaa ctctgggtca tcccaataat aagcagtggg tctgaaagtg 60360
aagtcattta tgctgttacc aatcagacaa actaatgggc cctctggaag atgagctcag 60420
cagaaagacc aaggagagga acaaaaggag actgtacttc tcatcctcag tcatgcctgt 60480
caaggtagga gtctgaggga agactttcac tgttgttaaa cttgtttcca gaataagact 60540
tcaatcttaa ttactcattt gatcatgtga accaataaaa gtactgccaa ggaaaaatgc 60600
aatgtatgct aacgttcata tcttatcact gagtctgacc ttagcacagg ctgtatatgc 60660
tattttacaa tgcatgaatg cactctttct tttcaagtca gcgtacacta aggctgtcaa 60720
tgttatcttg acccaacacc ttttatcagc actagaatat tacgtagaga caagacagct 60780
aaaaatttct aggctcagat ctgggaggga ccaaaacaac tttttttcag ggtgtgtttt 60840
tacatctgat tgtgcttgct tagttcctct gataacaata tacccagtgg aacccacaga 60900
gagtaggtaa tttgaggaat gaccgtcaaa agtttcatac aaatcctact tagagtgatt 60960
atctaaagtc ctactagaaa agaaatccac actgggaaca ttagaattgt cacgtggatg 61020
aaggtgctcc caaaagggtc tattacagac aatataaaga gccaaaattt gggatcacaa 61080
ctactgaacc tccttcccaa acaagtatct catctcttgg gaccctatgc tgaattagct 61140
ggggacccaa cagctatcat ctcaaaaatc tgctacgatc tctcaaaaag catctgtctg 61200
catcttacag gatgcactag atgacttggt gggaccaaaa ctggcagttt aactgcctta 61260
ggtacttcct cagcgggatg ccattcgatc tgaccctaga tgtgaaattt ctggtcctca 61320
gggaagttac agcactcagc aaagaaatga agtatatgtg gctggtgtta cagatttgtg 61380
gtcttcttag aggaataaaa gcagtgccaa atgtatggct tgagtcatga gggctagaaa 61440
aattcccacc tactatctct gacaactaca acaacccttc tgtgagagac agctgacatg 61500
cagtaattga aaagcctgga gaacaatccc aggactgcaa actgtgtgac ctatctaggc 61560
ttcagaatct cattgctgta aaaagagttg caatttaata atttctatgg tatcatctcc 61620
tttagtatga tatgacttca atgcaactcc ccatggccat caaagctcta gccaaacagc 61680
ataacaaata gcataagata gaggataagg gggcagactc tagagttaga ttacctggct 61740
tcaaatccat gccccatcat tcataatgtg accctggaca agtcatttaa tctttgtaaa 61800

ggagtgccag tttccttata tgtacctcac ggggtgctgt gaggattaaa tgagttaaca 61860
catgtaagca gcctacagta gtgcctggca cacagtgagt tctcaaagtt ttaatgatta 61920
ttacaattat tatcaatagt agcgccagta tcactactcc tttacttcaa agctgatagg 61980
aaactcctct ctactggccc acactttctt caaagagaaa tgcatctgaa tttcaaaagg 62040
catggcttct attactgtcc ctgaacagtc tgcatgggag ttctttatac tattctaatt 62100
ttatgcctaa aaaattttac aataaagggt taaaaactca tcctaaaata attcgtccaa 62160
caaatactga gtacttactt tttttttttt tttttttttt ttttgacgga gtctcacact 62220
gtcacccagg ctggagtgca gtggtgtgat ctcggctcac tgaaagctct gcctcccggg 62280
ttcgcgccat tctcctgcct cagcctcccg agtagctggc attacaggtg cctgccacca 62340
tgcccagcta atttttgta tttttggtag agacgaggtt tcactgtgtt agccaggatg 62400
gtctcgatct catgacctcg tgatccaccc gcctcagcct cccaaagtgc tgggattaca 62460
ggcgtgagcc accacgcccg gccctgagta cttactcaat gtgccaggta cagggaggac 62520
aaattagaac atgagagcca cagtccagtt atcatgaagc tggcagtaat gagctttacc 62580
ttgtggagtc ctcggccatt tttccctgt cacttatttc tcaatcaaat catcactact 62640
gattttcaag atcagctaaa ccttctccaa tgagtcagtt ctacatttct gccccacctc 62700
tattcatcat ttaaaactca acagaccaat tactactttt tccccaaaca actgtcttct 62760
tgaaacctcc tcatgaccag agagttagtt ctatcaattc atcattcgct gacatcaact 62820
ttacaaaatg tcccactgct ctacaacagc tctcaaacca ctcccccaccc ccatagtatt 62880
tactgacctc ccagtttcac ccatggtcac attctgaatt tgccttcgtt ctacaaattc 62940
ccccttttctg gttcctcctg aattctctca aaagcccttc tattttattt ttatttttat 63000
ttttattttt tttgagacag agtcttgctc tgttgcctag gatggagtgc agtggcataa 63060
tcttggctct ctgcaacctc cacctcccag gttcaagcaa ttctcctgcc tcagcctccc 63120
gagtagctgg gattacaggt gcccaccacc acgccaggct aatttttgt atttttttt 63180
tttttagta gagacagggt ttcaccatgt cagccaggct gctcttgaac tcctgacctc 63240
aagtgaacca cctgccttgg cttcccaaag tgctgggatt acaagcgtga gccaccacgc 63300
ccagccaggc ctccctattt ttgtcactca aatcgctttt attccagatc ttacctctgt 63360
tctgtaagca ttttctgtaa gcatgttccg accctgttgg gggctctctc tgtcctgtat 63420
ctactgctac ccctacccca cgctattctc cttggcccca gttgctataa taccttgtta 63480
ttcatcctat gatctatcca aattagatcc tttacacttt tacatacagc tctgaaatgt 63540
cccctctcca ttccttttga aaactcatct cctctgccta gaatatcctc cttcctttta 63600
ttctctatgc atggaaaatc tcctgatcct gaggtctagg aaaaatccta tccactctct 63660
ccaaaatctt ccctaatatt aaatttagaa attccttccc tcatgtgaat ctccatagaa 63720
cattgttttt atttatctta tagcactttt cttttttttt tttttttttt tttgggacgg 63780
agtttcactc ttgttgccca ggctggagtg caatggcgtg atctcagctc accgcaacct 63840
ctacctctgg gttcaagcga ttctcctgcc tcagcctccc gagtagctgg gattacaggc 63900
atgcaccacc acgcctggct aattttgtat ttttagtaga gacggggttt ctccatgttg 63960
gtcaggctga tctcaaactc ccaacctgag gtgatccgcc tgcctcagcc tcccaaagtg 64020
ctgggattac aggtgtgagc caccatgccc ggcctagcac ttttattctt ttagtctcgt 64080
tttttcattt tagagtggta atgagtagtc ttaaaaatcc tccaggctgg gcatggtggc 64140
tcatgtctgt aaccccaaca ctttgggagg ctgaggtggg aagatcactt gagctcagga 64200
gtttgagacc agtctgggca atatagtggg acctcacctc tactaaaact tgaaaacaaa 64260
ttagctgggc gtgctggcac acacctatat ccaagctact ggaggcaagg agggcatctg 64320
aggcagaagg atcccttgag cccaggaggt taaggctgca gtgagccctg atggcaccac 64380
tgcactgcac tccaactgca caacagagca agaccctgtc tccaaaaaaa aaaaaaaaaa 64440
aaaagctcca aagaaaccaa cataattgca tatagtaact tcaataaata tgaataagat 64500
caaatgtctg tccttttctt tgtccagaat gcctaaggat caggaatttt ccagatggga 64560
tcttccttgt agatagagtt tgaaaggatt tctctaacag tatattcact tgagccaggc 64620
gttgtaagtt cctcaaccta aaagttgcca atttaattca gttcaacaag caggtatcaa 64680
atgcccacca ctggggttgc agattcccat gcatggtatt tttctacagc agtttctaca 64740
gcatgctcta ctttttgcta ttcataattt tcctgtatga aattattttg gaactttccc 64800
aagtctaatt gcagtacaaa caagtatgtg aggctctact gtactatttc atcaaattgt 64860
tatgagtact ttgtgtgttt tcaagttcat ctctttagtg tccttgacct ttataaatat 64920
ggcatattgt gatactttaa gctagaagaa aaatatgaca gtagtaaatt aacagctttc 64980
tacccactta ttgagtcact tatcacaggc caaacactgc cttcagtgct ttacatatgt 65040
taacctaagt aactgtcacc acgacaactt ggtaagatga atactattat tctcgtttta 65100
tggatttttt tttttaagc tgggcttaag gttgctgaac atctcacatt ggggtaagtg 65160
acagagttga gattcaagcc gggctgggaa agcctagcag ggcatggcgg ctcatacctg 65220
taatcccagc actttggaag ccaaggcagg aggatcactt gagctcaaga gcttgggacc 65280
agcctgagca acatggcaaa accctgtctc tacaaaaaat acaaagaaaa aattaggatg 65340
tcatagtgca cgcctgcagt cccagttact tgggaggctg aggcgggaag actgcttgag 65400
cctaggaggt caaggctgca gtgagccatg attgtgctac tgcactccag cctggttgac 65460
agagaaagac cctgtctcca tttaaaaaaa aaaaaaaaaa aaaaaaagcc tacgtctaac 65520
tccgaagctc aatgctctta acctctacag tgaatcatga aaaagaataa agtcataagg 65580
atagtgaagt atttggggaa aaaaagattt atgaaaacag gtaaaacaaa gtatgagaaa 65640
acactatcca atcttactgt gaggaatctc atagcaaggc taaaattaga aagataaact 65700
cttatggctc ttttttaatc ttttgatata taaataaagc atgtcaaaga aaagtggaat 65760
gcaatgccaa ttctctagtg cagaaatatc acttgcttac gaaggttcct ggtctggttg 65820
cttaatacta cagcattttg gctcacatta cctaccaaag gggaaaaata cttcaaaata 65880
tctgtctcaa gggactgtta gaaagttgat taacatacat attctaaaat cctctgcttt 65940

ttcggagtat aaagattttt aattacagta gcaactaaat tttagcccca acattgaaaa 66000
ccagttgtga aaaagtcatt tcccaaaaat ggagaactgg tttcttcata tttaaaattg 66060
ctctgctggg gacaaagtca attctcactg caactgtata ctttaacagc tatcccttgc 66120
tgaaaaggct taggaattgg tcactggatc catgttgtac atcccaataa ctaaagctta 66180
actcagtctg ttaccttacc tttaaacgag ggcccatgtt ctgagaacca agtagagcac 66240
tcctattcac caaagcaggt aacttttggc ccctttcacc ttgtcttcca acttccacat 66300
gaaagaaaaa cgccacaagg tcaacagaaa gggtctatcc atcagacttt gtcacagtaa 66360
tcaggtcagt gtttctggga tgaactggat gaactccagg gtagagaaat acagcccaag 66420
aatatatcat tcctggactt ctctctagtg cagggtgcaa tcgactgcca aaggacgccc 66480
caggatcctg tccaaaaaag aaaacagggt tagttggaat aggtttttat ttctgaaaag 66540
gattccccag aaaaaaatca aagagaaggt agaacaggca gtggaaaccc tgggacacta 66600
aaaataacca aaaattggta aactctgacc aagcattctc aattaaaact ccacacagtt 66660
tccttgaggc ctctctccaa ctccataaga aaagtcaact ggcagcctca ctgtctataa 66720
ctatgtctcc ggctgagaag tgccagagct tccttcagtt cactgtcccc aaatacttcc 66780
ctatccttat gacattattc tttttcatga ttcagcatag aggttaagag cattgagctt 66840
cagaattaga cacagttttt ttgtttttgt ttttgttttt gagacagggt ctttctccat 66900
cacccaggtt ggagtgcagt ggcatgatta cagctcactg cagccttgac ctcctgggct 66960
caagcgatcc tcccgcctca gcctcccaag tagctgggac tacaggcgtg cactaccaca 67020
cctggctaat ttttttatttg tgtttttttgt agagacgggg ttttgccatg ttgctcaggc 67080
tagtcttgaa ctcctaagct caagaaatcc tcctgccttg gcctcccaaa gtgctgggat 67140
tacaggtgtg agtccattct tcagtagaag caaactaggc cacagactta aagagtaacc 67200
ttcagcacaa ccacataccc agtttgaata taatggggtt gcagcacagg gtctggagca 67260
accacaaaga caagtgagat gaacgcacag ccagaaccag gttacggaca gccttatatg 67320
ccatggtaag gaattgagac aatattcagc tggaaaggag aaacttccaa agagctttaa 67380
agcccaatct atcagcctat gcagtctgct cccttcgtgc ccacatatgc atttccaaga 67440
catgcttaaa taaagttgat tttatattta aaacagtaaa atctgctact acgagtcagg 67500
ggaaaatctg tgaactttca aaataaagtc actaaataaa gtccagtaaa gtcagtttat 67560
aactctcgaa gatattcatg aaccaaaaat gtagcagaaa gggggtttat ggcaatgcat 67620
acacaaacag ccaccataag acaaatgaga agagactact aattagtaaa tgaagagaca 67680
gtcttaccat atggcaggaa ctgttctcag tgcttcatac acataaactc acagatacaa 67740
ttatccctat tttaacacag atacaattat ccctatttta cagatgagga aactgggaca 67800
cagaaaggtt aaagtacttt tccaagaaaa cacacagtga gtaatagagc tgagtcaaac 67860
tcagacaacc tggacccaga atccctgctt ttaatgacta ctctactgcc tctccactat 67920
gggaaaccaa ggaagaaagc acaaaatgct aaaagaacag aatgagtgaa gaccagtaaa 67980
tgaaattaag ttggcaaact tttatcatta aaaataaata agtatatgta tttagtttaa 68040
aatacagcaa catgaccggg cacagtagtt cacgcctgta atcccagcac tttgggaggc 68100
caaggcaggc ggatcacttg agcccaggag ttcaagacca gcctgggcga cataacaaaa 68160
ccccgtctct acaaaaaata caaaaattac ccagacatgg cggcaggcgc ctgtggtccc 68220
agttacttgg aggctgaggt gggggttggc ttgagcccag gaggcagacg ttgacgtgag 68280
ccgagatcat gccactgcac tccaccctgg gcaacagagc cagacccagt ctcaaaataa 68340
ataaataaat aaataaataa ataatgccaa cagacttgcc aagtacaaga gccaaaacct 68400
gggtcaactg gctactggaa ccagcttaaa ggatccacaa acaacagact atatacgtaa 68460
gctataagag agcactatgt agaatactat ataaggaatt tttaaatgac acttttaagg 68520
aataaacaac atagggaaat gcttacaaat aaaatgttaa ataaaaaagt aggaacaaaa 68580
ctgtttatgt agcacaagtc caaatttaga aacataacat tacataaaga agactgaaag 68640
gaaaatataa aaatattaat agtgatcatc tcttggtata cggaattata aataattttt 68700
atttctacc tgtacctttt agtgtttttc aaattcttaa tgttgcaagg acatgtaaaa 68760
agtttctatt tctggagaac acatttttgg tggggactga ttttccttaa ataagggaga 68820
gaatgggcaa gttatccttt tttcaaataa gaaaatggta taaagaggct gggcacggtg 68880
gctcacgcct gtaatcccag cactttggga ggctgaagcg ggcggatcat gaggtcagga 68940
gatcaagacc atcctggcta acacggtgaa atcccgtctc tactaaaaat acaaaaaatt 69000
agccaggcac ggtggcgggt gcctgtagtc ctagctactt gggacgctga ggcaggagaa 69060
tggtgtgaac ccgggaggcg gagtttgccg tgagccaaga ttgcgccact gcactccagc 69120
ctgggcgata gagtgagact ctgtctcgga aaaaaaaaaa aaaaaaaaga aagaaagaaa 69180
atggtataaa gaaattaaga taacagatta tactgaactt ttctttcctt cttttttttt 69240
tttttttga gatagagtct tgttctgttg cccaggctgg agtgcactgg catgatcttg 69300
gctcactgcc tcactacaac ctccacctcc tgggttcaag cgattctctt gcctcagcct 69360
ccctagtggc tgggactaca ggtgcgtact actatgccca gctagttttt gtatttttgg 69420
tagaggcagg gtttcaccac gttggccagg ctggtcttga actcctgacc acaggtgatt 69480
cgcccgcctc agcttcccaa agtgccagga ttacaggaat gagccaccac acccagcctg 69540
aacttttatt tctattcact atgatgttag caggtttcca accctgtgat cagctatttg 69600
attattaaat caaataactc ctagttgtaa tataagcttt actatcccta ccgtgagcta 69660
ccatgctcac cgcaatgctt cagtacctct actgagcttc aaaactcaac tcaagcactc 69720
ccccttctct ggtcctgctc caaggcctct ctgaggactc ttcccacacc agatagaatc 69780
agccacttcc tcctttgtgg tgctcctctg ctgaagatac ctccgttata gcatttacta 69840
gtctgtattg aaattatttg tttatatagc agtctctcct aatagattgc ggactcccca 69900
aggaacactg accaggtcat aacattgcct ccccaaggct agtatggtgt tcagccctgc 69960
tatctcagag tctgtacttt cacaagatcc ccaggtgagt cctatgtatg ctgaagtttg 70020
agaaacatgg tttagactct agagcaatca gtatgagaag gggctcaaca ttctgtatct 70080

ctaacaggtt cacaggtaat gctgatgctg ctggtctcct agaccatgct ttcagaagtg 70140
agaatccaag ttaagagtca gcaagccaaa tcctaacagc cgcctgtttt tgtgaaaaca 70200
gccaagttca ttcatttaca tattatctgt ggctgctttt gcacaatggc agagctgagt 70260
agttgcagca gagacatatg gcccacaaaa ctttactatc tgatgcttta ttgaaaaagt 70320
ataccaattc caggtgtaga ggtcttagtc ttaaagctac acaactacct tatataaaca 70380
aatacatttt tctaaatcaa agaaagcata caaatcattt tcttccagtt ctaccatctc 70440
ttcttaggaa aagaattaca aaacttgcaa tgttatgcaa acacatctag tttcaactat 70500
gacttgatgg ctttattctt cccatacccc aattaggaat tttacagtca ttttccaaag 70560
tatgtatttc atacaatcat gcttgcattt gatcaaggct aatatttcct aaaactagta 70620
tctcggggac caaatactgt atccgggttg ctaaaattac tataacacat actactaact 70680
cagtctctac ttctaaagtg ctattctaaa aagctgtgaa aggccaggtg cagtagtgca 70740
cacctgtatt cccaacactt taggaggctg aactgggaag atcacttgag cctaggagtt 70800
gaaaccagac tgggccctct ctaaaaaaat aaaaataaat tggtcaggta tggtggctca 70860
cgcctgtaat cccagcactt tgggaggcca aagtgggcag atcacctgag gtcaggggtt 70920
tgagaaaagc ctggccaaca tggcaaaacc ccatcactac taaaatacga aaattagcca 70980
cgcatggtgg cacacacctg taatcccagc tactcaggag actgaggcag gagaatcact 71040
tgaacttggg aggcttaagt tgtagtgagc caagattgcg ccactgcact ccatcctggg 71100
tgacaaagca agacttcgtc tcaaaaaata aaataaaata ataaaataaa ataaattagc 71160
tgggcgtggt agcatgcccc tgttgtccta gctactcaga agctaaggca ggaggatctc 71220
ctaagcccag aagtttgagg ctacagtgag ctgtgatcac accacaccac tgtactctag 71280
cctgggtaac agagagagag atcctgtcac acatacacac acacacacac acacagccat 71340
gaaaaacatg atgtaaaagt ccatttcaaa attttggtgc atacagagct agtggcagca 71400
aacagaagtt ctgatatcac ctactgatgt ccaccaaatg acaagacaaa agaaagacgt 71460
ttgttttgta agatgcaatc tccagcattc tccacagaga agcaccagcc atgagtccag 71520
ggacatgaac ttaaaaaaac aaactattca attttcaaaa agtgcacagg ttagggtggt 71580
ctaactaagg aggttccaag aatatatcag acactttgga ttatcagttt acatttacta 71640
tggcttaatt ccactcttag tttatactcc ccaacccaaa atcccaccat aaaagattgc 71700
cctcctccat caagtttata ctcttgagat atttatacag ttgtctattt ctgctattgt 71760
ggtttagcca agttacatgg ttttcattta tactttcttc atcagtcagc ctctccagtc 71820
tcttaatcat tcctgtgggt cttctgtgaa ctctctccag ctgatcccca acgatctggt 71880
aatgaggtgc ctccaaatga ctgcagcctt ccagggacag cctcattagt cagcaggatg 71940
aactcactct cctcagttcc atgacatact actcttgagc aggcaatccc ccaaatcaca 72000
gaaatctact gtgctgccgt attagactgt acctcattct ataactccca gatttggaca 72060
ctggggcatg ctagaatgtg ctagaagcca gaggataaat gacccatgtt cctacagcat 72120
ctgtttcact agaggatgag tgactgggga gaaaaaaaaa tttcatgtta aatcaaactc 72180
atagataata aaacagaatg taatatttgc atgaatttct aaagataaaa catggatatg 72240
ttgaatatat tcatagatat tctgaacttg aagcagaaag gctgcttggg cctctgctcc 72300
cggcaccaag gagttaacat atcctttacc actagaggta cttgagccaa aaccctcact 72360
caacttctga tctactgtgc attaccttac tcataccata tactcacatt ttaaatgata 72420
ttatttttct gctatgtttc cccatcctag atatctatat ttattttctc tttgcctaaa 72480
atcttttgct gcaggaacac gtgtcattta tcttctggct tctagaacat tcttgcctgc 72540
cctgatgccc aaatctggga ggcatgaaat ggggttacag tctaattcac tcgataattc 72600
acagactacc aacatctata ggttcctctt catcgttcat tcttcattca tttcacattt 72660
cttgagcatt tgctatatac tgggctacgt gataaaagct aaagtcacaa ggataaataa 72720
gacagggtgc tttcttaacc tcaatgaact tggtccgcca tgggagaagg acaatgcaat 72780
gttgcaagtg ccataaaaga ggaataagca aagtatggag ggcagaggaa ggagaagtaa 72840
ctgtatgtgc aagtggaaaa gcagagacaa cagagctgat tacttaagaa tgagtaagca 72900
ggctgagcat ggtggttcat acctgtaatc ccagcacttt gggaggctga ggtgggagga 72960
tggcttgcgc ccaggagttc aagaccaatc tgggtaacat agtgagatcc catctctaaa 73020
aaaaaaaaaa aaaaaagctg ggtgtggtgg tatgcaccta tggtcccagc tactcaggag 73080
gctgaggtgg gaggatcatt tgagcccagg agggtgaggc tgcagggagc cctgactgct 73140
ccactacact tcagaccgtg tgaagagcaa gactctgtct ccaaaaaaaa ccaaggtgtt 73200
tagaaagacc acctggggct aaactaggat gactaccatg tagtggttaa gagcatgaat 73260
cttactactg aatgatctta aacaagttac agcaggtcct caaacgacat cagtttcatt 73320
caacactgtt ttcttatgat tttgaggagt aaaaaaatgg actcctggca ggcaccaatg 73380
tgtggagttc acacattctc tgtgtgggtt ttctctggat acttggattt cctcccacat 73440
cccaaagatg tgcacttcaa agttaactgg catgtctaaa gtgtcccagt ctaagtgaca 73500
gtgaggggtg tgtgtgtgtg tgtgtgtgtg tgtgtgagag agagagagag agagcaagag 73560
caagagagag agagagagac agcacacaag cgagagagcc ctgcaataga atggcgtccc 73620
atccagcgct ggttcccaac ttgcacccta agccctggcc accctcaacc gtggactgga 73680
ataattgggt aaatgttata attatctaac ttgtttttat caatctttct tatacatact 73740
gctctcattt attttaatat gtatattatg ttctggtctt tatttaaaat agtttggtga 73800
tatttttgtg accagaaata tgccatagga acttaattct tgtttatatc aattagccta 73860
tggtaaaact ggttatcaat atacatcatt ttgtttgaag gagaagtttc caagaaccta 73920
tcaacatcaa gtgaggactt gctgtaaatt tctcttcagt tgcctcatat gtaaaatgga 73980
gatgcactga gaaaagaaac gaatccgctg ctcttgggga gcaagacttg gagaataagg 74040
acaaggagaa ttgcttgaga ctgaaacttt acacccaggt gatatggagt gccttggggg 74100
acaaggctgt atgcacagac caggctgctg ggttgccagg ttatgagggt ggtttgtgcc 74160
aggccacaat agcccaaccc acagcaagtg aggaagatgg cttcaatcta cctgaaggac 74220

tcactggttt tccagagaga ttccatgtgg aaaaaactac agcaacaatc caaaataatg 74280
gattttccag tgttctctga aggatgatga taacaagaat caacctttat tgagaactta 74340
ctatgtgcca ggcattgtat aaagtgctac atctatatta tctcatttaa ctctcacatc 74400
aaccatatga ggaaagcata ctttatccat attttataga ttaggaaaca gaagcacaga 74460
aagctaaaat gacttgccta tagccatcta gtttataagt gaaggagtca agcaatgtga 74520
ttccagagtt tatattcttt ctctactctt aactggcaat actgatgtat caaaaagaac 74580
ttccagaagg ggggaaaaag atattattct tgggatagga agaattaagc tcagaaatat 74640
ttaatttgaa attttagccg ggcgcagtgg ctcacacctg taattccagc attttgggag 74700
gccaaggcag gtggatcacg agttcaggag atcgagacca ttctggctaa catggtgaaa 74760
ccctgtctct actaaatata caaaaaaaat tagccgggca tggtggcaca cgcctgtatt 74820
cccagctact ggggaagctg aggcaggaga attgcttgaa cccaggaggc agaggttgca 74880
gtaagctgag atcatgctac tgcactccag tctgggcgac agagcgagag tccctctcaa 74940
aacaaaacaa aacaaaacaa aaaggaatgt taaaggaata tgacttcatt ttagacttta 75000
agcaacaaca acaacaaaaa gatgtttttt aaccgagaga gaaaaaggat cacacatttt 75060
aaatgaagta actctgggga caatgtgaaa gactctgaag ggggacagac ccattaatga 75120
gcacaagata aggggctact ggtccaatgg agtagctata cactggccac aatatagaca 75180
gggtccaaca tagaccctca ttagtcacat tggttgaggg tggccagggc ttagggtgca 75240
agttgggagc caaccctgga tgggttgctc tcctgcttta tgctctcaga agcaacagga 75300
tcaatgatct gtgaggtcaa atgaataaac aagaggaaga agccaaggaa aaaggcaaat 75360
tttctatggt tgggagcctg gataaatgaa ggagaattaa ggaggcaaag caaacatgaa 75420
aaaaaagata tattagtcca atttcatgct gctgttaaag acgtatcaga gactggttaa 75480
tttacaaaga aaaagaggtt taatggactc acagtaccat gtggctgggg aggcctcaca 75540
aatcacggtg gaaggtgaaa ggcatgtctt acatggcagc aggcaagaga gaatgagagc 75600
caagcgaaaa gagaaatccc ttataaaatc atcagatctt gtgagactta ttcactacca 75660
caagaacagt atgtgggaaa ctgcctccgt gattcaatta tctcccactg gatccctccc 75720
ataacacatg agaattatgg gaactataat tcaagatgag atttggatgg ggacacagcc 75780
aaaccatatc attccacccc ggcccctccc aaatctcatg tccttacatt tcaaaaccaa 75840
tcatgccttc ccaacagtcc tccaaagtct taactcaaag ttcacaatcc aaagtatcat 75900
ctgagacaag gcaagtccct tctgcctatg accctgtaaa atcaaaatca agttagttac 75960
tttctagata caatgggagt acaggcaatg ggtaaatata cctgttccaa atgggagaaa 76020
ttggccaaaa caaaggggct gcagggccca tgcaagtcca aaatccagca gggcagtcaa 76080
atctcaaagc tccaaaatga tctcctttga ctccatgtct cacatccagg tcacgctgac 76140
acaagaggtg ggttcccatg gtctggggca gctccatccc tgtggctttg cagggtacag 76200
cctcctcccc agctgctttc gtgggctagc actgagtgcc tgaggctttt ccaggcacat 76260
ggtacaaact gtcagtgaat ctaccactct ggggtctgga ggacggtggc cctcttctca 76320
cagctccact agacagtgcc ccagtgggga ctctgtgtgg gagcttcaac ccccacatttc 76380
cccccaacat tgccctagca gaggttctcc atgagggccc caccccatag cagacttctg 76440
cctgaacatt caggagtttc catacatcct ctgaaatcca ggcagaggtt ctcaaacctc 76500
agttcttgac ttctgtgcac ccacaggctc aacatcacgt gtaagctgcc aaggcttggg 76560
gcttgcacct tctgaagcga tggcccaagc tgtaccttgg ctcctttcag ccacagctgg 76620
agcaactggg gcacagggca ccaagtccct acactgcaca cagcaggggg gacctgggcc 76680
ctgccttcaa aaccattttt tcctcctagg cctccaggtc tgtgatggga gtggctgcta 76740
tgaaaacctc tgacatgccc tggagacatt ttccccattg tcttggtgat ttggctcctc 76800
tttatttatg caaatttctg tagccagttt gaatttctcc tcaaaaaatg ggtttttctt 76860
ttctatcgcc actgtcaggc tgcaaatttt ccaaactttt atgctctgct tccctttttaa 76920
atgtaagttc caatttcaga tcatctctct caaattcaaa gttccacaga tttctagggc 76980
aggagcaaaa tgattccagt ctccttgcta aagcataaca agagtgacct ttgctccagt 77040
tcccaacaaa ttcctcatct ccatctgaga ccacctcagc ctggatttca atgtccatat 77100
catcaccatt ttgttcaaag tcattcaaca agtctccagg aagtttcaaa ctttcccaca 77160
ttttcctgtc ttcctctgag ccctccaaac tgttctaacc tctgcctgtt acccagttcc 77220
aaagttactt ccatattttc aggtatcttt acagcaccgc ctcactccca gtaccaattt 77280
actctattag tccattttca cactgctgat aaagacatac tagagactgg gtaacttcta 77340
aagaaaaaag gttgaatgga ctcacagttc cacatggctg ggaggcctca caaccatgtc 77400
agaaggcaaa aggcatgtct tacgtggcag caggcaagag acaatgagag ccaagcgaaa 77460
ggggaaatcc cttataaaat caacagatat tgtgagactt attcactacc atgagaacag 77520
tatgagggaa accaccccat gattcaatta tctcccactg gatccctccc acaacaatgg 77580
gaattatggg agctaaaatt caaggtgaga tttgggtggg gacacagcca aaccatatca 77640
aaagataatg agtagagaat taaacatgtt aagtttggag tgtctacagg atatccaggt 77700
gaatacattc aaaaggtaca tagaaataca tttgtatcac acgcaaaatg tagtccttcg 77760
tgtgtagcat tttttcactt agcataatgg ttcttagatt catacatgtt gttacacatt 77820
tcggtagttc actccttttt cctgcagagt agtactccat taaatggaca tactgcaatc 77880
tgtttatcca ttcaccagat gaaggacatt tgggttacat ccagtttggt ggtatcatag 77940
ataaagctgc tatgaacatt aagctacaag tctgtgtagg cacatgattt catttctctt 78000
gggtaaatat ctaagaatgg gattgctggg tcatatggta agtatctact taacttacaa 78060
gaaactgcca aaccgtttac caaagcggct ggaccacttt gcatttccac cagcaatgca 78120
caagaggcag gttgctctat atccaccaac acttggtact gtcttaatgt tacccattct 78180
aataggtgtg aagtggtatc tcactgtgat tttaatttgc atttccctaa ggactaacaa 78240
tattgagtat ttttttcatg tgtttattgg ttatttgtat tagtttcttt gaagtatcta 78300
ttcaaatctt ttctttttta aaaatcgagc tgtttgtctt cttactattg agttgtaaga 78360

gttctttata cattctaaat gtgaaccccg aatatctgag acaggtctca gttaatttag 78420
aaagtttatt ttgccaaggt tgaagacata cgcccaggac acggcctcat gaggtcctga 78480
cgacatgtgc ccaaggtggt tggggcacag cttgctttta tacattttag ggagacttga 78540
gacatcaatc aagatgtata ctggctcagt ccagaaaggc gggacaactt ggagtaggga 78600
ggggggcttc caggtgagaa aaatggttgc attatttga gtttctgatt agcccttcca 78660
aagaaggcaa tcagatatgc atctatctcg gtaggcaggg gatgactctg aacagaatga 78720
gacacagatt tgccctaagc agttcccagc tttacttttc cctttagctt agtgatttgg 78780
gggccccaag acttattttc ctttcacata aatataagtc ctttattaga taaacatttt 78840
gcaaatattt tctgtggctt gcctcttcat tttcttaaca gaaaatacac tttttaaaga 78900
gtacaagttt ctaactttga tgaaagccaa cataaatttt ttcttcatag tttgtggttt 78960
ttgtgttcta tttttaaaa ctctgcctag ctcaaggtca caaagatttt ctcctggttt 79020
ttttctagag ggttatctaa acgtttcaca agagaaaatc ctctcgtcaa attttttaac 79080
aagaattttt taaaactcct gcattccatt tatctcatag tcattaagtt ttattcccct 79140
tgtttctttc aaattcaatg ctgcacatta ccatgcacta tttaagttac agtcttctgt 79200
tgttttttttt tttttgagac ggagtttcgc tcttgttgcc caggctggag tgcaatggca 79260
ccatctcggc tcaccgcaac ctccaccttc caggttcaag cgattctcct gcctcggcct 79320
cccaagtagc tgggattata ggcatgcgtc accacgccca gctaattttg tatttttagt 79380
agagacgggg tttctccatg ttggtcaggc tggtctcgaa ctcccgacct caggtgattc 79440
gcccacctca gcctccctaa gtgctgggat tacaggcgtg agccaccatg cctggcccca 79500
gttatagtct atttctgaat ttggaaagat tttaatattt taacagattt ttcctctctt 79560
caatctagta aatggaatgc ttaagtttag ccaaggtttt agtccctcac ttctctccaa 79620
atccacataa gacctctgca cccctgatac tggttcttaa aacaacaact aaattccaca 79680
acccagaaca tgcaccttga agttaagcca gggacaataa actagttacc cttctggctt 79740
taacgagagt cttttataag atcatgccca ccttactgag tcactctaac ctgtgatgag 79800
actggaaaat gttgtcctat aatcactcac tggttaaatt aaaatttagg cttataaagg 79860
ttaagctaac caagacaaaa gtaattctgc atttcttaat tcagtttgga ttctctctat 79920
tgcaccattc tctctaaaac aagccagagt tcttcagacc cgtatttctt tttgtatagt 79980
atacacaaag gcatagctta gattcagctg tccctggagt tgtttgccat tcattctcag 80040
cacagtttag agaaagcagg aaaaagggat tcaaagctaa tgttgagtat aacagggaaa 80100
acaagaagtg agaatttacc attgctcatt ttgtaacctc atggggagaa aaacaaaaga 80160
gaaaactggc tcatccatgt cactgtccca ttcttaaccc taaatctaga aaatactagc 80220
ttcagtttcc tcttccctcc tatcttgatg tgttttctat atacaaaact gactctgcta 80280
tgcctgagcc acagaggaca ggcccccatg ttcccatctc atggcagctt ttatgcttag 80340
gtgactttgg accactgaat cttggctata acaggaagat tttaaaagta attctttgaa 80400
cctgacaagc cgtctccctc aataagtcta ggaagctatt attccattaa ctgcgcctga 80460
agtggcaggc acacagccag atctgcagcc cagtcgggtg gaactggaat ggtcacacgt 80520
atacttaaaa gctaactctg cctctactac catccattac tttcaattct gtgcttattt 80580
ttaaggaaaa taaattattt atataattga aaggaaaaac aatgttaatt aaatgagcat 80640
tttgtagtta agattccaag aagaaaaact caactagcca cagaaatggc accctaagat 80700
agctggattt gaaggagaaa ctcaacactt acctattcaa aacaaaattt ctgttgacct 80760
aataatcagt tttaccaaag aaaggtctat taatttcttc aaatgcatat taagaagcga 80820
ccacatgcca agcaatatgt tggaccctaa agattccaga atgagtaaaa tattctcaac 80880
actttagaaa cataaaagct tagtaaagaa gacagacaaa taaataatta cgtagttata 80940
cttccgtgta gtaaaatcta tgaggtgagc atagagggta tggatggata ccttaatcaa 81000
gatcagctct tagcttctca ggaaagttca catcaccctc ctggaagcaa agtcaactct 81060
tccatagagt taacaatgct ttgggggtca aggaatacaa aacactacct atgaagtatt 81120
acttattctc agggtcaatg actctcttca acactcgtca gaggggattc ttggaccaca 81180
tacatagcac agaaaaagca ctcactggaa ataccaactt atttatcaaa attatacacg 81240
atctggtcgg gcacagtgac tcatgcctat aatcccagca ctttgggagg ccgaggcggg 81300
cagatcacct gaggtcagaa tttcgagacc agcctggcca acatggtgaa atcccatctc 81360
tactaaaaat acaaaaatta gccaggcgtg gtggcaggcg cctgtaatcc cagctactcg 81420
ggaggctgag gccggagaat agtttgaacc caggaggcag aagtggcagt gagccaaaat 81480
ggtgccattg cactccagcc tgggtgacaa gagcgaaact ccattttaaa aaaaaaagag 81540
gccgggcaca gtggatcaca ccggtaatcc cagcactttg ggaggccaag gtgggcggat 81600
tacgaggtca ggagatcgag accaccctgg ctaaaatggt gaaatcccat ctctactaaa 81660
aatacaaaaa atggccgggc acagtggctc acacctgtaa tcccagcact ttgggaggcc 81720
caggcaggtg ggtcacaagg tcaggagatc gagaccttcc tggctaacat gtgaaacccc 81780
gtctctacta aaaatacaaa aaattagctg ggtgtggtgg tgggcgcctg tagtcccagc 81840
tacttgggag gctgaggcag gagaaaggtg tgaacccagg aggcggagct tgcagtgagc 81900
cgagatcgcg ccactgcact ccagcctggg cgacagagtg agattcgtct caaaaaaaaa 81960
aaaaaattag acgggtatgg tggcgggcgc ctatagtccc agctacttgg gaggctgagg 82020
cgggagaatg gcgtgaaccc gggaggcgga gcttgcagtg agccgagatg gcgccactgc 82080
actccagcct gggcgacaga gtgagacctt cgcctcagaa aaaaaaaaaa aaaaagaaat 82140
tacacatgat ctaactggaa tgtggtattc tagattcagt ttgggtatag aaaaaggaaa 82200
ttagtggaga aacttgtgaa atccaaataa agtctatagt ttagtaatag tattgtacca 82260
atgttaattt cttagatgtg acagtatgtg ccatggttat gtaagaagtt aacattagga 82320
gacaccggtg aaaggtatac aggaatactc tgtactatct ttgcaacttt tttataaatt 82380
taaaattatt ccacaattta aaacaattta attacagatg gtgctcccct ttacattctc 82440
actccccctt agaattctaa tcatgtctcc ctaatcattg ccccaattca ggtaatgcca 82500

gcctaaaact ttttaaatat cttttttaac ccctttctat gaactgtttg cacagcccac 82560
ctgggatttc ttctttacca agacatgaaa aactagatca cacttgggaa gttttaactg 82620
ttttctgttc ctaataggtt taccatatat accgggggta ccttgaattt gaacttccca 82680
aagcggacaa gagaggggtt aaaataacat gctctgggtc tctggataaa agcaagggga 82740
cattacagaa aatatgttgt atattaataa attatctaat ctctggccaa aaagtacaat 82800
atatagaaaa tcaattaaca gatgttaact attggcatct caacagtaac aagctgcact 82860
gaaataaatg ccttcgctct gaaaaacatg atgcaaattg ctcaactatt caagataaca 82920
aaaactcata aaatcatatg gaaccgttca attagactaa atgacaaaac atgtatctac 82980
catgactatt tagtttctaa tagctaagtg aatacccatc tttctctata aaccctatct 83040
tgtagaacag tcttcaaaag agccaccagg aaaacatcac tatggtgctg taacaccata 83100
tcaacagctc aggcatgcgt gtgtgtacac acacatacat acacacaaac tgcaataagg 83160
ttaaacaacc tcaaaagttt gaaaactact atatgaaaga acattagtga aggctaagca 83220
ttcagaaccc ttcaaagaaa aagaaaccaa agaaaaataa ctgaaattta aacccaagtt 83280
agttaatagc taaagtcatt taaaaggtaa aacggtcagg cacagtggct catgcctctt 83340
ctcccagcta ctcaggaagc tgaggtggga ggatggcttg aggccaggag ttcaagatca 83400
gcctgggcaa catagcaaga tctaataaat aataaataaa aggtaaaatg atacactaga 83460
gtccattaag gtcataaaat ctgatctatt acatggtact aatatgttga tagtattaaa 83520
taaatattac acaacagtac tctgacctgg accacttaca gaaagatgaa caagaagagt 83580
aattcctcta aaatatttca actcccacat ctctattcag ccaccaacca tactttacct 83640
atttacaagt ctttctgtaa tacaggactg tggtgagatt gcccaggaca gaatgggccc 83700
tcaataaatg ctttctgaat gaatgaccaa gcggcagtaa tctatctctg aggtattctt 83760
acaaaccaat tcaatatcaa gacctcagaa gcagaacatg ctgtatgcca tgctaccaca 83820
tttaggggca ggcaagttaa ttgaaaaata attaacagcg actaatgttt gttgggagaa 83880
aaaaagtcct atcatgtaag tcagaaaaat aaatgtaaaa actataactt gaaaatggac 83940
aaaaaagtga atctacttat aactttctaa cattgaaata caagatgttc tttaaggagt 84000
ctttcattat ccagaataat tttacaaaga aatttgatac caacttaaat tagtaagtta 84060
gaccaattag tgaatggaat gaagtccaag cccaattatg tgttcaaact gtgtgtccaa 84120
tgccttgggt gatttttttct cttcaagact gcagaaaaat gcaaaacaac cctcttgact 84180
gctgagaagg actaaaatca tttcttttcc tcacaagtat gtgcccaaaa gcaattactt 84240
tctgctccca tccatcacaa atcaacacta cctcccagct tcgcatcccc cactttccat 84300
ccccatcaaa gaatgctgta gagtatctga ccactttcat ttcacgctat aagaacctca 84360
gattgcaata aaattacaat gcaacaaaaa caaatttttc taagtatgtt cttatccccc 84420
atggtgttcc tacactaatt tagctcccta ttaggtatta acaatcaagg aaatcagaac 84480
actctcaata gaggttacaa tagaaattac aaatggcctt tttttaagtt gttgttgttc 84540
atggattcca aaattcaaag tacacattac cagaagagcg tttttctgtc agtgcctaaa 84600
tttaaatgca aattactatt taatgatatg caattttccc tctgaaccta tatataatac 84660
taatgagtca aatgtttaag aagaataact gaccatgtta ttttagcttt tagacaataa 84720
aaagcttatt gtctaatcac caagagatac atcaaaaaac tggttgctat tagctctaca 84780
catgaatggc agcttgacag agacaagctg ctccagaaag attaggaact tctagtgacc 84840
acaaatgggg taaagcagaa ggcacataca gtatctgtag ctaccatttc tacatcaaaa 84900
attcagtgcc cacagatatg taaactatta agccatctaa atgaatgtgt gcctgaaaga 84960
gaaaggagaa gcagaaaaag tttacaccca aatcaacaga agaaatcacc ctgcgattga 85020
gcatcccagg acctgaaaac ttaatgtttt gaggtttatg aacacgttca agttccagtg 85080
caactatgtt aaaataaaat tgggctggtg ccaggcacgg tggctcacgc ctgtaatccc 85140
agcactttgg gaggccaagg tgggcggatc acctgaggtc gggagttcaa gaccagtctg 85200
accaacatgg agaaaccccg tctctactaa aaatataaaa tcagccaggc gtgttggcgc 85260
acgcctgtaa tcccagctac tcaggaggct gaggcaggag aatcgcttaa acctgggagg 85320
tggaggttgc agtgagccga gattgcgcca ttgcactcca gcctgggtaa caagagcgaa 85380
actaactccg tctcaaaaaa aaaaaaaaaa aattggggct tggcacagtg gctcacgtct 85440
gtaatcccag aactttggga ggccgaggtg ggcagatcac ctgaggtcag gagtttgaga 85500
ccagccttgc caacatggtg aaacccatct ctattaaaaa tacaaaaatt agcaaggtat 85560
ggtggcacac acatgcctgc agtcccagct actcgggagg ctgaggcagg agaattgctt 85620
gaacctggga gggggaggtt gctgtgagcc gagattgcgc cactgcactc caacctgggc 85680
agcagaggga ggctctgtct caaaaataaa ataaaataaa ataaaataaa attggcataa 85740
aattccaacc ctgccacaca tcctcaagtt ggaagacaca caatggtaac aacagaaaac 85800
actctgagta gtctggttta cactcttcta gggacctcgc atgttaacta tgaataaaca 85860
taagtcctga taacatccca ataaaagagt gctactatga ttatctccat tttacagata 85920
aagaaactga gatgcagata gattaactca cccaatgtca tggcgagtta taaggcagag 85980
caaggatttt aaccccatca gtcagactcc aaagttcgtg ttaaacaata aacgtgtttt 86040
catcataagg gttatacaat ggtcaccttc ttttttaaac ctgataacat attttacttc 86100
caactctagg atagcatcat gcaataagaa aagtatgaca gaggtaccca ttccgttccc 86160
agcttgatca gtagctggag aatggaagac aaagtgcaaa aaaaaaaaaa aaaaaaaaaa 86220
aaaaaagaaa aaaaaagaaa gaaagaaaga aagaaaaaga aaaaaagaaa tacaagtaca 86280
gactttggaa tcacgagact caaacagacc cctgaacaaa gtctggggct caatttcccc 86340
atatgtaaaa taaaagaatt tatgttattg agaaggtcaa agaaggtaat atgaataaag 86400
tgacaagtac agtatacagg atgggggttt tcatttaaag tttacagaca cccagagggt 86460
ccacggatgg gcttcagagt ccaccaatct cattaagtgt attacaaact gtatgtgtgc 86520
ccattttttta aagaattgtt cctggctttc cacaaacttt caagaaataa gttatctcca 86580
aaaagttaag aaccactgga atacatagca ggtactcaaa gaaaaagct acctttcgct 86640

```
ccttgttctc acaaattctc cctttttact taaagatgac acaccaaggc ttcacaataa 86700
atgtgcattg tatctcacca ttttcttggc ttgggtaagt agtaacatat gcaagcttat 86760
gccccaattc tctagcgata gtctaggccc tcaaacactg cttcatttct ctcttcaccg 86820
agaggagtac acggtatata ctcgagaaca atcatccacc tagctgcaca aagcaccata 86880
tcggctagga aaaccaaata ggaaccacag cctcagggct ggaccataga caccggatac 86940
cccaacgcta cagggtgagg agaatgtgtt cccccaaact actgggcaaa ccaggagtct 87000
gaaccccccct accctttcc tccctctctc tctggaactc tggtaaaggc aggtcttctg 87060
ggctctgcct aaggctggag agaaacgtta cccgagccgg gggttgcagc gcgacgaagt 87120
tccacctccg ctgtcctggg aaggggcggc agcactcagc agaagacggg ctccccactc 87180
tcccaccaac agaccccaga gttggtctcc acccggcctg ggaaccggct cgggggattg 87240
ccctttcccc aaggagtttc cgcttccggg ctatcctttg ccggaagcag tatgtgaatg 87300
acgtagaagt attgcgccgt tggtgattac ggaagaacca ggagtttggc gtgaccatgg 87360
tgagagaaga cggtccaaga agggacgtta tcaggccact ttttgggtgg agaaggaggt 87420
agtcagaccg tggccaaatt tccttcacat tatcctagca tattttcatt ctggtcttag 87480
ctcttaaact tctccaagcg gtgactgtta tgctttacag agtggggaag agggtctggg 87540
ttatttgagg caagaaggga aggcaataga caagagaaaa gaagagactt taaaagatgc 87600
cgtgtgtttc caactttttt aattctaaaa tttctgtttc aaggggaagc aaaagaaaac 87660
aaggaagtat gcgaccatga agcgaatgct tagtctcaga gatcagaggc tgtgagtgtc 87720
tggaatcaac tgcccaggga tattctaata agagtctaga gaggataagt agtaaaaatg 87780
tttgttgtta ttattttgtt ttttgcttat tatcgtgtct tagatttata tagcatgaga 87840
tagccagtgt ttaacagtaa tttagctgct gagcgatcac actgggaaag gcgatgggaa 87900
ttaaacattt cttaagtgtt cacttttttct accgttatat gctttgtctg ccatatcgca 87960
tttaaacctc atgtcagttc ggtgaagtcc gtatagttct ctttatgttt ttaccattac 88020
actactacat tcccctgggg tggggcgagg ggagtgtttc ttgttgaaag atggcaaggt 88080
tagagacctt gtttgtctgg cgtttccagt gcctggtact caaatatttt tgtcagagaa 88140
aatgggaaaa ctccaagata tctccatttt ataagtctta ctaatataag aatccaaatc 88200
actgtggaag aagaaagtga agtgaaattc cctagtatga agaggatcaa agcggtgtcc 88260
ctgcttaagt gtagaataag catagctatt aaataaagac tgatcttcac tgagtttaag 88320
agtctcagaa agccgggcgc agtggctcac gcctgtaatc ccagcacttt gggaggctga 88380
ggcgggtgga tcacctgagg tgaggagttc aaaaccagcc tggccaacac ggtgaaaccc 88440
tgtctccact aaaaatacaa aaaagagtct cagaataaga gaatagtcgt taaaaccttc 88500
agaattatta ttctcagcct ttagtgtgga tgtattgtgt aatttttagg aatgacactc 88560
cattaataat aatttctttc tagttaaggg ttaagttctg tatatgcgtt tttacatttt 88620
agccctttat aagaagcata tggggtaata cttttccccc aaaagttaaa gcatctgaat 88680
cttcagaaga taactgtcac ccttttaccc attgcttgga agtggattgt gatccttgat 88740
gtgagagatt ctgactaaaa aataaatata gtataatcag cttttattta ctcattaaca 88800
attcttatgc attattggca ttactggtta attctgatac ttccaggtac tgtgctttgc 88860
actagagtta tttgctcaga aagagtatgc tgatattaac atgaggatat cacaaagtag 88920
gatttcttca gtagaccaaa aaaaaaaaaa aatggaactt tagattttg agattttaat 88980
tgctgtggtt tttcttctcc cttggattta atttaccttt ttcttcctag taaagaaaag 89040
gatagattaa aacctaaaaa gaaagaaaag aaggatccca gcgcattaaa ggaaagagaa 89100
gtgtgagtaa tcaaacgttt gaagttttcc tttaaaacca tagacctctt agaaatccag 89160
gctttccctg agctggtttg ctaacttatt tgttagtaag tctagcacaa ctatgtagat 89220
gaaagtcata tcgatgagca tatatcaatt ctagtggttt gaaggttttg ggattttatg 89280
gagcagtaaa atttccccta aaatcctgga gatattatct aggattatta actttttctt 89340
tgccagggat cttttaagag acataagtgc cattgtttaa cacaccagca gttcataaaa 89400
gagagactgt atcaccaaaa aatatttaaa atatattttc agaataaaga tatttatata 89460
aagatatgac ctttatataa agatataaag gtatttcatt ccttttaagt aagtacattt 89520
aactttatga aaactttacc acattttcct tatttttcta atttttccct ggattggtaa 89580
tggacatatt atcacaggta gatactgatc catggcctag catttggaat cactgtcata 89640
ttcaacccag acaacttggg ctagatgtga tacaggtcct gctgtagtat ataatcctaa 89700
cactagacaa actgttctct atcacattcc ataacagcag ttttagaatc ctttaagaat 89760
gacaaacgtg ttgttgtatc attctaatca cgataactgt attctacctc cctgtgccca 89820
tggtgattct acttttgcta gaggaagagc tagattggat aattgctttt tttgtttgtt 89880
tgtttttgat aattgctttt ttaaagttgg tgacaaaggt tgccagggga gctgagatta 89940
agataatgat attactttct tccttgcctt ccttttttagg atttatttcc aatgaacatg 90000
gacaaaagag tttacagcaa acagtcccaa gcagacttct tttttgtttt tgttaatact 90060
agggtatcaa tgtaaatttt tctttgttct tgttttcttt ttccttcagt ccccaacacc 90120
cttcctgctt atttttccaa tataatacac agctgggccc accttaccac atcctcgttg 90180
ataccaactt tatcaacttt tccataaaag ccaaactgga cttagtgcag tcaatgatgg 90240
actgtctgta tgccaagtgt gagtatcata cttttatgtt tccgtgacat ttgttcagaa 90300
taattatatt tatacaaata catgaaagga gggagatggg gttcttgtta actgaattgc 90360
tggccattta atttacagtg gtcaccatag cagtgaccat gtttaatgaa ttgaagtata 90420
atgcaaactt gactggaaaa aattaaactt tcacaattca gaaaacactt agaatgcttc 90480
gagtatcatt atggacatca attttgaaca taggggaatt ttctgtttta gaattctaga 90540
ttttaaaatt ctagataaat gaaaaatcta tttttcagta tctatattct tttatgttaa 90600
caaggatatg ttctcccttt tgccccagtg ttgaaacact aaataagaca ttctgccatt 90660
tatgatcaca gccctagaag ttagatagat gatggtcaat tcctaagttt attcagtttg 90720
gtatttattc gtagtacaga ggatctgagg tagtttattt acacctaggt atgcaattta 90780
```

ccattaatag atttcccttg tgtatttgta tatcccctcc tttaaatgat cttccttcac 90840
taccatgaac aggaaaaaga aaagtaaggc catctatgaa gcctatcata gtaagatctg 90900
tcccatttgt agcgggttta tatttatata tttttcagtc acccggttag aggtacgaat 90960
gaaaattaga cttaaacttt gccatagagg ataaattact ggaatcaaag ggaaaaattt 91020
gtacctccct ttttaagttt taaaggctac tatataatga acaacagttg cagagtgata 91080
gaaatggtaa atgttgttta ctgatttatt aaaatgagca agatgagaag atggggttaa 91140
ggaatgtgtg aaggggtaag aaagcagatt ctttagatgc ccaaaaatta attaagaatg 91200
gctgcagccg ggcgcagtgg ctcatacctg taatcccagc actcgcggtg gctcacgcct 91260
gtaatgccag cactttggga ggccaaagca ggcggatcat gaggtcgaga tcgagaccat 91320
tctggccaac aaggtgaaac cccatatcta ctaaaaatac aaaaattagc tgggcgtggt 91380
ggcacgtgac tgtagtccca gctacttggg aggctgaggc aggagaatcg ctggaacctg 91440
ggaggtggag cttgcagtga gccgagattg tgccactgca ctccagcctg gcaagagaaa 91500
ctccatctaa aaaaaaaaaa gaatggctgc atacatttat tagctgcata cgtttattaa 91560
gatgtgaagg tagccatcag atgagaaaaa aatggagcta atggaataaa atagtcttct 91620
ctacagatca gaattgattg ggtggcaatg gtcaggggac cattgttttt tcatataggc 91680
ctttctgtat tactttttaa agctatatac ataaactttt gaaataaatc ttttagaaat 91740
aatctaaagt tgaggtgcta ccaggcctct aagaatgagg cttgggtgac cccatatggg 91800
ggaagacact aaacttggta gtctctttgt atttcccatg gtatttctaa ttgagctgct 91860
tctccaggaa tgctgtgacc tacttatcaa tgaaacggat agagggtcaa agcatggccc 91920
tttgcttcct tgatgaattg cttccttgac tctatacctc caccagtaat atctagatag 91980
tgactgttag agctttaggc aggatagctt tagtcttatg gctttcttta aatatgtata 92040
cttttttttt tgagacaggg tcttgctttg tcacccaggc tggagtgcgt tggcgtgatc 92100
tcagctcact gcgacctccg cctcccaggt tcaagcagtt cttctgcctc agccacccca 92160
gtagctagga catgccacca cgcctatcta atttttgtat ttttcgtaga ggcgggtttc 92220
accatattgg tcaggctggt ctcaaactcc tgacctcagg tgatccaccc acctcggcct 92280
cccgaagtgc tgggattgca ggcgtgagcc accgtgcctg gccttgcttg cctttatact 92340
taaaggaagg cctttctctt cctcttacct tgaaaaggcc tgtttgatct ttacatggtt 92400
cctggtctct tctatttcca ctttatagtc caacaaggaa ggatagtttg cttcccattg 92460
gctttctctt ctgcctatga atcctgaggc tgttactttc cctttccttg tagtggtttt 92520
cctgagcctg cttccctact tcagttgcaa gcctttgctc acttcactgc tacatggctc 92580
tgaaattggt cctcttaaag aacacattgc cttgtttcat gatctcagta ttaaaatata 92640
tatatatatt gagacaaagt ttcactctgt ggcccgggct ggagtacagt ggggtgatct 92700
ctgctcactg caacctctgc ctcccaggtt caagtgattc tcctgcctca gcctcccgag 92760
tagctgggat tataggtgca tgctgccact cccagctaat ttttgtgtaa agtagagacg 92820
gggtttctct atgttggcca ggttggtatc gaactcctga cctcaagcat tccgcccact 92880
ttggcctccc aaagtgctgg gattataggc atgagccacc acgcctggcc agtattaata 92940
tacttctaaa aaattctagc ataaaaaaat ggaagttgaa gcctgggcaa cataacgaga 93000
ccccatgtct acacataatt taaaaattag ccaggggcc aggtgcggtg gttcacacct 93060
gtaatcctag tgcttttcag gagttcgaga tcagcccggc caacatggtg aaaccccatc 93120
tctactaaaa atacaaaaat tagccaggcg cagcaccatg tgcctgtaat cccagttacc 93180
tgtgaggctg agggaagaga atctctggaa cctgggaggc agaggctaca gtgagccgag 93240
atcgtgccac tgcactccag cctgggtgag agagcgagac tctgtctcaa aaaaaaaaaa 93300
aaaaaaaaaa agcatgcatc gtggcatgca cctgtggtcc tagctacaga tgctcaggtg 93360
ggaggatcac ttgagcctgg gaggctgagg ctacagtgaa ttgtgattat gccactacac 93420
tccagcccag gcaacagagc aagaccctgt ctttaaaaaa aaaaaaaaga agaagaagaa 93480
gaaaagaaag ttgggctggg cgagatcttc accccgtagt cccagcagtt tgggaggccg 93540
aggtgggagg agttctgggc ttgagctcag gagttcaaga ccagcctggg caacatggtg 93600
tgaccgcatc tctacaaaaa aaattttttt taaatcaagc tgggcatggt ggctcatgcc 93660
tgtaatccca acgctttggg aggccaaggt gggtggattg cttgagccca ggagtttgag 93720
accagcctgg ccaacatggc aaaaccctat ctctaccaaa agaaaccaca aaaattagct 93780
gagcatggtg gagcatgtct atggtcccag ctgcttggga ggctgaggtg ggaggatcac 93840
ttaagcctgg gaggtcgagg ctgcactgag ctatgtccat gtcactgccc tccagcctgg 93900
gtgacggaac aagaacctgt ctcaaaaggg ggaaaaaaag gaagttgctt ataatgcatt 93960
tcctcaaagg agcaattata aaaattcata aatctgtgtg agatggaggg ttacttttat 94020
ttattaatga aaatggcctg taatcccagc actttgggag gccgaagcac gcggactacc 94080
tgaggtctgg agttcaagac cagcatggcc aacatggcaa aactctgtct ctactaaaaa 94140
tccaaaaaaa attagccggg cgtggtggca ggtccctata attccagcta ctcgggaggc 94200
tgatgcagga aaatcgcttg aaccccagag gcagaaattg cagtgagcca agatcgtgcc 94260
attgcaagag cgaaactccg tctcaaaaaa ataaaaaaag aaaatggatc ctgtattatt 94320
ttttgcttta taaaacagat ttactgaaat ataattgact ccctttacaa ctatacaatt 94380
catcccttta aagtgtacag ttcagtgact tctagtggat tcacagagtt gtgcatcatc 94440
catcaccata gtcaatttta gaacttccat tatcccagga agaaacctgg agacccttag 94500
ccatcaccct gcaaccctca ccattctctc tatttctagg caaccactaa tctactttct 94560
gtttctataa atttccctat tctggacatt tcataaaaat agaatcttat aatatttggt 94620
cctttttcat ttaacgtact gttttcaagg atcatttata ttacagtttg tatcagtact 94680
tcatttcttt ttattactaa ataatattcc actgtatgga tagatcacat tttaatcatt 94740
tctacttgct ttttacattg aactatatat tttgaacatt tttccatggc atgtatatat 94800
aaatgggcct cattctttta ttttgagtca aggtcttgct atgtcatcca agctggaatg 94860
cagtggtctg agccaatagc ttacagcagc ctcaacctcc aggtctcaag tgatcctgca 94920

```
gccacagcct cccaagtagc tgagactact ggaaagagcc accatgcctg gctattttat   94980
ttatttatgt tttgtagaaa caaggtatca atcatgttgc ccaagttttt ggggtttgtt   95040
tttttttttt tttttgagac aggctgtcac tctgtcaccc aggctggagt gcagtggtgt   95100
aatctcagct cactacagcc tctgcctccc gggttcaagc aattctcatg cctcagcctc   95160
ccagtagccg ggattgcagg tgtgttccac cacacccacc taattttttt tttttttttt   95220
tgagacagag cctcactctg ttacccgggt tggagtgcag tggtgcaata tcggctcact   95280
gcaacctctg cctccaggct tcaagtgatt ctcctgcctc agcctcctga gtatctggga   95340
ctacaggcat gtgccaccac gcccagctaa ttttttttgta tttttagtag agatgaggtt   95400
tcaccatgtt gaccagactg gtcttgaact cctgacctca ggtgacctgc ctgccttggc   95460
ctcccaaagt gctgagatta cgggcatgag ccaccacacc cagccctaat ttttatattt   95520
ttagtagaga tggggtttttg ccatgttggc caggaattct tttttaaagt tatgtatggc   95580
cgggcacggt ggctcacgcc tataatccca tcattttggg agttcgaggc aggtggattg   95640
cttcaggtca ggagttcgaa accagcctga ccaacatggt gaaaccttgt ctttactaaa   95700
actacaaaaa ttagctgggc gtggtggagg gcgcctgtaa tcccagctac ttgggaggct   95760
gaggcaggag aatcgcttga acctgggagg cagagattac gccactgcac tccagcctgg   95820
gcaacaaagt gagacttcaa aaaaaaataa agaatgaata aagttacata ctataattgc   95880
ttctcagcct tttggctaag atcaggtgta aagttacata ctattctatt gtttaaatac   95940
ataatttttt aaacccagta tcttattagg tattttggtt atttccaggt tctacccaat   96000
taacaagtct gtagtaaata tccttatgaa tatgtctccc taagagtgta tttctttagg   96060
atacattgtc tggaagctgt attatgtatc aagggatttg tgtattttta ataggtattg   96120
caaatttgcc ctcaaatagt tgtaccaatg tatataccta caaacttagt atgagaatac   96180
ttgtttcctc aatattgggt attttctttt tttttttttt ttttgagatg gagtttcact   96240
cttgtcactc aggctagagt gcaatggcac gatcttggct cattgcaacc tccatctgct   96300
gggttgaact gatcctcctg cctcagcctc ccaagtaact gggattacag gtgcccaaca   96360
ccatgcccag ctattttttt tatttttagt agagacgggt ttcaccatgt tggccaggct   96420
ggtctcgaac tcctgacctc aggtgatccg cctgcctcag cctcccaaag tgctgggatt   96480
ataggcgtga gccactacgc ccggccaata ttggatattt tcatctgttt aatctttgcc   96540
actcttctag gtgccactct tcccagtctt tggcccaata gactccgttc aaatgttggc   96600
tttaccattt gctatctatt tagttaactt ctttgtgtct caggttcctc acctataaaa   96660
tagggatgat gatttttttt taaactatta tatggctttc tgtttgatag ttgtcaaaca   96720
gaagttttta aaatttttat gtgatcagat ttacattatc tggctaattt catggtttct   96780
tgtggttcac atttaaattt tatttcatct gagccaggca cagtggctca cacctgtaat   96840
cccagcactt tgggagccca agacaggagg aatgattgat cccaggagtt caagacagcc   96900
tgggcaacat ggcaaagccc catctctaca aaaaaaaaat gttttttttt aattagctgg   96960
gcatagtggt gcgtacctgt ggtcctcgct actcgggagg ctgaggtggg agaatcatta   97020
gaacccagga ggataactag aacccaggag tttgaggctg cagtgagttg agatcgtgcc   97080
actgcgctcc agcctgggca acagagcaag actctgtctc aaaaaaataa taaaaataaa   97140
taattttttt cccatcagga acttacatta atataaaaat aatgacagag ttcagcttag   97200
ttttttttcc ccagatatgt ttataacagg gaatctgcag tttggggttg ggacaatctt   97260
agtttcttag ggttttcttc tactctagtt cttcttgtat atttcagtgt gctaattctg   97320
ctgtcattgc aaaagtgtcc tgggtctttt taaaactctg agcagggata taattcagag   97380
ataatttcgt tacaagttct gttcttaatg tgaatttttt tccctggcag gtatcccatg   97440
tataaccgat tgtgtaatgg ctgaaattga gaaattgggg cagaagtatc gagtggctct   97500
aaggtaggaa ggaggtaaac tagatctgtt ctagattggt atactgaaga ttcatctgtt   97560
tgttgtttaa agatttcttg gccagttagt tgtgaaaatc atacaaaact atttatcatt   97620
tttggggggg ttgttttggt ctttctctca cacaaaaatc agttccagtt agagtgtcta   97680
aacttttaat agaaaataca gagaatatct tgtccgggct cggtggcctg taatcccagc   97740
actttgggag gccgaggcag gcagatcacc tgaggtcagg agtccgagac cagcctgacc   97800
aacatggaga aaccctatct cttctaaaaa tacaaaatta gccggctgtg gtggcaggtg   97860
cctgtaatcc cagctacttg ggaggcttaa gcaggagaat cgcttgaacc taggaggcgg   97920
aggttgcagt gagcctacat cacgccattg cactccagcc tgggcaacaa gagcaaaact   97980
ctgtctcaaa aaaaaaaaaa aagagaaaat acagagaata tcttaatgaa attgaatatt   98040
tgtgtatagc aaaagatatc attaacaaag taaacagaca tacataatca acaaagggct   98100
tattttcaaa atgcataaag aacttcacat ctgtaagaaa aacataattc ttaaattcac   98160
agtgagttgt catttagtgc ccattagatt ggcaaaaatt ttttaatgag cttgacaatg   98220
ccaagaatag ttaagaccat gaagcaatgg gaattctcat actgaactac taagggtata   98280
aattggtcat cactttggag ggcaacttga caatatctgg taaaatccag gttgttcata   98340
ctcttacaac acagtctttt aacctggaga tcacattcta caggtaatta aattggttta   98400
aaggatcaca atgcctggtg ggtttggggg gttttttgtg ttttgttttg ttctgttttt   98460
gaagcaaggt gttgctctgt cacccaggct ggagtgcagt ggcacaatca gggctcactg   98520
cagcctcgac ctcctgggct taagcagtcc tcctgcctca gcaccctcaa gtagctggga   98580
ctacaggcat gcaacacctc acatggccag ttttttaaaa tttcagtaga gatgaggtct   98640
caccatgttg cccaggctag tcttgaactc ctgggctcaa gtgatcctcc caccttggcc   98700
tctcaaagtg ctgggattat aggcatgagc caccacaccc aacccccgag tatatttttt   98760
agtgtaacag aagaaactat aggctgggca cggtggctca tgcctgtaat cccaacacct   98820
tgggaggctg aggcaagtgg attgtttcag cctaggattt ccagtccagc ctgggcatca   98880
tggtgaaacc ctgtctccac aaaaagtaaa aaaattaacc aggtgtggtg gcatacacct   98940
gtagtctcag ctattcggga ggctgaggtg ggaagattgc ttgagcccag gaggcagagg   99000
tagcagtgag ctgagatggt gccactgcac tttagcctgg gtgatagagt gagactctgt   99060
```

ctcaaaaaaa aaagaaagaa attataaaat attagcatga atatgtagaa aaaattgggt 99120
actgcttcac aaaacttttt tcgctgttat atgtgtgtat atgtatatgt ggtatatgtg 99180
tgcatactgt acgctagata aaatctatct cgtgctatgg gttctggtaa aaaaaaaaag 99240
aaagaaaaag attgctatag agaagttcag ctactattgg agtcaagtat tggcatcttc 99300
ttatgtacgt aatagcaaaa aattgcagac agccagaaga aatggctttt cttaagccat 99360
aaaggatggc caggcatggt ggctcatgcc tgtaatccca gaactttggg aggccgaggc 99420
aggcagatca tttgaggtca ggagtgatcc tcaacatggt gaaaccctgt ctctactaaa 99480
atatgaaaat tagccaggca tggtggcggg tgcccatatt cccagctact ctggaggctg 99540
aggcaggaga atcatttgag cccaggaggc ggaggtttca gtgagctgag gtcatgccac 99600
tgtactccag cctgggctac aaagcaagac cctgtctcca aaaaaaaaaa aaaaaaaaaa 99660
aaaaaaaact gggtacagaa gctcacgtat aattcccaac acttcgtgag gccaagtggg 99720
gaggatcgct ggagcccagg agtttgagac cagcctggcc aacataatga gaccctggct 99780
ctacaaaact ttaaaaattt gccgggcggc cagccaggcg cagtggctca cgcctgtaat 99840
cccagcactt tgggaggccg aggcgggtgg atcacgaggt caggagatcg agaccatcct 99900
ggctaacacg gtgaaacccct gtctctacta aaaatacaaa aaaattagcc aagcgtggta 99960
gtgggcgcct ggtagtccca gctgctgggg aggctgaggc aggagaatag catgaacccg 100020
ggaggcggag cttgcagtga gccgagatcg cgccactgca ctccagcctg ggtgacagag 100080
cgagactcca tctcaaaaaa aaaaaaaaga aaattagcca ggcatggtgg cttgtgcctc 100140
tacggagttc aaggttgcag tgagctatga tcacaccact ccactccagc cccaaaaaca 100200
aagtgagacc ttgtctcttt aaaaaagaaa gaaaagaata gataagctgt ggaatgctca 100260
tgaataaaat actatacaag agtgaagatg agtgaactag agttatgcgc caatgtggat 100320
aaatctcaaa aacaatataa actataaaaa aaaaacaaca gcagtcaggc actgtggctc 100380
actcctgtaa tcccagcagt attgggagcc cgaagcagga ggattccttg agcccaggag 100440
tttgagacca gcctgggcaa catagtgaga cccaatgtct atgaaacatt ttaaaattag 100500
ccaggcatgg tggcatgtac ttgtattccc agctacttgg gaggctgagg taggaggatt 100560
gcttgagcca gggaggttcg agatcatacc actgcattct agcctgggag acagcgagac 100620
cctgtctcaa aaaaaaagaa aaagaacaat gctgagcact gttaacgcac atctgtagtt 100680
ccagttactt gggaggctga ggcagaagga tcacctgagc ctaggagttt caagccagcc 100740
tggacaacaa gacccccatc tcttttttttt tttttttatt atactttaag ttttagggta 100800
catgtgcaca atgtgcaggt tagttacata tgtatacatg tgccatgctg gtgcgctgca 100860
cccactaact cgtcatctag cattaggtat atctcgcaat gctatccctc cctcctcccc 100920
ccaccccaca acaggcccca gagtgtgatg ttccccttcc tgtgtccatg tgttctcatt 100980
gttcagttcc cacctatgag tgagaatatg cggtgtttgg ttttttgttc ttgtgatagt 101040
ttactgagaa tgatgatttc caatttcatc catgtcccta caaaggacgt gaactcatca 101100
tttttttatgg ctgcatagta ttccatggtg tatatgtgcc acattttctt aatccagtct 101160
atcattgttg gacatttggg ttggttccaa gtctttacta ttgtgaataa tgccacaata 101220
aacatacatg tgcatgtgcc tttatagcag catgatttat agtcctttgg gtatatatcc 101280
agtaatggga tggctgggtc aaatggtatt tctagttcta gatccctgag gaatcaccac 101340
actgacttcc acaatggttg aactagttta cagtcctgcc aacagtgtaa aagtgttcct 101400
atttctccac atcctctcca gcacctgttg tttcctgact ttttaatgat tgccattcta 101460
actggtgtga gatggtatct cattgtggtt ttgatttgca tttctctgat ggccagtgat 101520
ggtgagcatt ttttcatgtg ttttttggct gcataaatgt cttcttttga gtagtgtctg 101580
ttcatgtcct tcgcccactt tttgatgggg ttgtttgttt ttttcttgta aatttgtttg 101640
agttcattgt agattctgaa tattagccct ttgtcagatg agtaggttgc gaaaattttc 101700
tcccattttg taggttgcct gttcactctg atggtagttt cttttgctgt gcagaagctc 101760
ttgagtttaa ttagatccca tttgtcaatt ttggcttttg ttgccattgc ttttggtgtt 101820
ttagacatga agtccttgcc catgcctatg tcctgaatgg taaagcctag gttttctttt 101880
agggttttta tggttttagg tctaacgttt aagtctttaa tccatcttga attgattttt 101940
gtataaagtg taagaaaggg atccagtttc agctttctac atatggctag ccagttttcc 102000
cagcaccatt tattaaatag ggaatccttt ccccattgct ttctcaggt ttgtcaaaga 102060
tcagatagtt gtagatatgc ggcgttattt ctgagggctc tgttccgttt cattgatcta 102120
tatttctgtt ttggtaccag taccatgctg ttttggttac tgtagccttg tagtatagtt 102180
tgaagtcagg tagtgtgatg cctccagctt tgttcttttg gcttaggatt gacttggcga 102240
tgtgggctct tttttggctc catatgaact ttaaagtagt tttttccaat tatgtgaaga 102300
aagtcattgg tagcttgatg gggatggtat tgaatctata aattaccttg ggcagtatgg 102360
ccattttcac gatattgatt cttcctatcc atgagcatgg aatgttcttc catttgtttg 102420
tgtcctcttt tatttcattg agcagtggtt tgtaattctc cttgaagagg tccttcacat 102480
cccttgtaag ttggattcct aggtatttta ttctctttga agcaattgtg aatgggagtt 102540
cactcatgat ttggctctct gtttgtctgt tgttggtgta taagaatgct tgtgattttt 102600
tacattgatt ttgtatcctg agactttgct gaagttgctt atcagcttaa ggagattttg 102660
ggctgagaca atggggtttt ctagatatac aatcatgtcg tctgcaaacg gacaatttga 102720
cttcctcttt tcctaattga ataccctttta tttccttctc ctgcctaatt gccctggcca 102780
gaacttccaa cactatgttg aataggagtg gtgagagagg gcatccctgt cttgtgccag 102840
ttttcaaagg gaatgctgcc agtttttgcc cattcagtat gatattggct atgggtttgt 102900
catagatagc tcttattatt ttgaaatacg tcccatcaat acctaattta ttgagagttt 102960
ttagcatgaa gcgatgttga cttttgccaa aggccttttc tgcatctatt gagataatca 103020
tgtggttttt gtctttggtt ctgtttatat gctggattac atttattgat ttgcatatat 103080
tgaaccagcc ttgcatccca gggatgaagc ccacttgatc atggtggata agctttttga 103140
tgtgctgctg gatttgattt tcccgtattt tattgaggat ttttgcatca gtgttcatca 103200

aggatattgg tctaaaattc tctttttgg ttgtgtctct gcccggcttt ggtatcagga 103260
tgatgctggc ctcataaaat gagttaggga gtattccctc tttttctgtt gattggaata 103320
gtttcagaag gaatggtacc agttcctcct tgtacctgtg gtagaattcg gctgtgaatc 103380
catctggtcc tggactcttt ttggttggta agctattgat tattgccaca atttcagctc 103440
ctgttattgg tctattcaga gattcaactt cttcctggtt tagtcttggg agagtgtatg 103500
tgtcgaggaa tttatccatt tcttctagat tttctagttt atttgcgtag aggtgtttgt 103560
agtattctct gatggtagtt tgtatttctg tgggatcggt gatgatatcc cctttatcat 103620
tttttattgc gtctatttga ttcttctgtc ttttttctt tattagtctt ggtagcggtt 103680
tatcagtttt gttgatcgtt tcaaaaaacc agctcctgga ttcattaatt ttttgaaggg 103740
ttttttgtgt ctctatttcc ttcagtcctg ctctgatttt agttatttct tgacttctgc 103800
tagcttttga atgcgtttgc tcttgttttt ctagttcttt taattgtgat gttagggtgt 103860
caattttcga tctttcctgc tttctcttgt gggcatttag tgctataaat ttccctctac 103920
acactgcttt gaatgcgtcc cagagattct ggtatgttgt gtctttgttc tcgttggttt 103980
caaagaacat atttatttct gccttcattt cgttatgtac ccagtagtca ttcaggagca 104040
ggttgttcag tttccatgta gttgtgcggt tttgagtgag attcttaatc ctgagttcta 104100
gtttgattgc actgtggtct gagagacagt ttgttataat ttctgttctt ttatatttgc 104160
tgaggagagc tttacttcca agtatgtggt caattttgga ataggtgtgg tgtggtgctg 104220
aaaaaaatgt atattctgtt gatttggggt ggagagttct gtagatgtct atgaaaaagc 104280
aagaacatat gtggagtata ccatttatct aaagtctaaa aacatgcaaa ccaatactgc 104340
attgtttata gatatgtgta tatgcttttt tttttttttt tttttttaa agacagattc 104400
tcactctgtc gcccaggctg gagtgcagtg atgctatcac agctcactgc agccttgacc 104460
tctccggctc aagtgatcct cctgctttgg cctcccaagt agctgggact acaggtgcat 104520
accactatgc atgtctgatt atttttttc ttttgcatgg ctaattttaa aaaaaaattt 104580
ttttggtaga gactgggcat ggtggttcac gtctgtaatc ctggcactta ggaaggctgt 104640
ggcgggcaga tcacctgagg ctaggagttc aagaccagtc tgaccaatgt ggtgaaacct 104700
catctctgct aaaaatacaa aaattagctg ggggtggtgg caggcaccta taatcccagc 104760
tactagggat gctgaggcag gagaattgct tgaggctggg aggcagaggc tgcaatgagc 104820
caagatcatg ccactgcatc cagcctaggc aacagagcaa gactccatct caaaaaggct 104880
gggtgcagtg gctaacgcct gtaatcctag cactttggga ggctgaggta ggcagatcat 104940
ctgaggtcgg gagttctaga ccagcctggc taacatggtg aaaccccgtc tctcctaaaa 105000
atacaaaatt agctgggcat ggtggcgcat gcctgtaatc ccagctactc aggaggctga 105060
agcaggagaa tcgcttgaac ccgggagcag aggttgcagt gagctgggat tgtgccaatg 105120
cactccagtc tgggtgacag aatgagaccc catctccaaa aaaaaaaaaa atttagtaga 105180
gatagggtct cactatgttg cccagctggt ctcaaactcc tggcctcaag tgatcttcct 105240
gctttggcct cctaaagtgc tgggattgta gtcatgatcc tccacaccca gcctggtaat 105300
ttttaaaaca ataaaaataa agaatgagag gggaaaaaat agaggcacca agtggaaaca 105360
ctaagtttgg ctgtcaagag atgcagaaat ggggtgttag ctggaggggg gtataggtaa 105420
aaagattttt ttgtcttaag gcagagaata tatgacagca tgttagtatg tttctaggaa 105480
agagctagta gagaggagac aagtgatttc aggagtaaag agataactga aagagcatag 105540
tcctagactg ttgcccacct agtttgtcag caaccagtcc atcctccctc tagtgtatcc 105600
tccccagcac agcctgctta gtttttccaa gacagagctt tgttcttatc actttgtccc 105660
ccagaaatct ttaatgactc ctcattacct gtgtcatcac actctgtcagc ctggcattgg 105720
agactctttc tagccccatt tcattcttct ctccacaaac actctgcccc cagccagaaa 105780
gatgtgttta ctcttcccca aataccaaga aaccatttta cagttggctg gtgtcctatt 105840
tggtaccctc acattgcttg gcacattctt taatcttatt tcaccccatg tcattttttt 105900
ctaaatattc ccaacccaca ttgacctagc gctgaactac tgcaatatgg aggagtattt 105960
gtgttgcact tggtacatat gtgctgtctt gtatttttct tcatttcagt gactcatcat 106020
cactgttaat agaaagagca tggctctcag aatcacacag agttggggtt tttctttttc 106080
aaatatgtat gaaaataata catgtttgag tgttttatgg caaacaatac aagcatatac 106140
atatagtaaa aggtaaaagt ctgcctctac attcatactt tgtccattct catacccaa 106200
gagtgacaac tgacaaaggt taatatattt tttcagacct ttttctgtgc aaatgcaaat 106260
ctaaataaag agtattggaa agaaggattt gtattttatt aattaaatca tattatatat 106320
atatatatgc atatgccctg ctttgtttat tcaacagttt agtgtaggtc cttccacatt 106380
ggtacgtaca gctctacctc actttttatt aataggataa gggcaccaaa atgggtttaa 106440
cttttacctt tctaaaggat atttttattt tcttcactta ctataaactg tgttgagtga 106500
tcatccttct gcctatattc ttgttcattg tgtttaagac aacttcttaa aagtgaaatg 106560
gttaggttaa aatgggtttt gtttttgaat cctggctttt cccagctgta tagctttgaa 106620
tgaagtaatt taatctctct aagcctactt cctcatctgt agaatagata tataataact 106680
caaagttttg taatgattaa atgacccaaa gcacactagc tgtcagcctt cattccctgc 106740
cccgtgaatt gtctttccag ttagaggata aagttcagga ggagaacagt gccttaaaat 106800
ctctgcatag atcatgttac ttttattgca ttaattaaga ctcagaaagt tatccagctg 106860
attgaatgtt ttctttaatc cacctacagg attgccaagg atccaagatt tgaacgatta 106920
ccatgtacac acaaaggaac ctatgcagat gactgcttag tacagagagt aactcaggta 106980
ttatcagttc ataactgttt actttgtgct taaaaaaaaa aaatgtaaac tattatctcc 107040
tgaatgttca tggtcagtaa aacatctagg aatatacatt atggttaaat taaaaaaaaa 107100
ttgcatagca ttccttcata agaagcatag gtgtcttcat ttacttgaga aagtggattg 107160
aaaatgtaca ctctaaccat aatggaaaga gcacaggctt ggggaggaga catgggtttc 107220
acgtctcagc acctgcattt actggctgtg tgatttggga catttaactg attctttatc 107280
tcacaaatct ctgtaaaatg attttaagtg agttaatgta gaacccattt atttttgatg 107340

taggaatttt gttcctcttc acgatgccta atccaaaagt gcttcttagt taaatctgag 107400
agtctcccaa gatgctgttt cacacatacc ctagaataag taggttttta attccttcat 107460
taagcaccac aaggacttct gctactgagt gcttttataa gggcctctct gctgatgata 107520
atcttcatct gctctgctga ccacagagcc atccttttag ccacttcacc ccatggggcg 107580
ggtgggtggg gccagtattt cccacagcac agctgacacc actctgccac tttttataaa 107640
actcagtagg agagtcagac gtgacagatg tgaagcttcc tataaaatag ctttcacctg 107700
acagctaaat attactgctt ttataactgc taggctctag tttcaggccc tgaatatcct 107760
acttaggctt cctgtttcgg ggatagctta cctctaacat cagtaggccc agagcacacc 107820
caagccccaa gatctaggga aaggagggag ctatggcctc caaattaagg cactttttat 107880
ctccatggag ttcctcggag cattttccca aaactaaaga attgtctgcc ccaagtgtga 107940
aagtcccttt catttatgag cagaaaacag tttgcagctt gtgaaaagtt ttctaccttc 108000
tcctcctcaa gattctaaat gctgcattca gcccaggcaa cttgtttatt ttatataaca 108060
agattaggag ctcctagatc atagtaggta ctcaacaaat tgttatagcc tgcccttcgc 108120
tacttactgt gaaaatgaac gattctcaga ctggtcagtc gttgtgccat tatccagtat 108180
tcactaagcg tgctcagtga cctcagtgtg aacatcactc catgtctctt acagcataag 108240
tgttacattg tggccacagt tgaccgggac ctgaaaagaa gaatccgtaa gattcctgga 108300
gttcctatca tgtacatttc taaccatagg tgagaaattt cccttggaga agggaataga 108360
aatatataat tgatatcaat tgaaaatgag gataagtgat aaggttataa aggatttgaa 108420
agaaattatt gtatcaaatg tttacatagt tatatttgaa gaaatgagac aaagcaagag 108480
caagagcaaa agagagagat aggatttaag gaatgaggca aacatttaaa ttgttagagt 108540
aagattactg gaagaaatag acgtattgtt ttaaataaaa gcagtaattt aaatcaagct 108600
aatctttttg gccagaaata cctgttttga aagatttttt tttattaaca cttcatctga 108660
gaaatggaaa gcttatttaa aaaaaaaaaa agctgttatc cttcttggct attttttgtc 108720
agtactcaga cttattttcc agcttttcag cttgaggaga gcacaatgtg gtgggaagtc 108780
tgtggactaa gaagttggac ttactgatct cagctcctca gttcccctct tgctgacttt 108840
gtgactttga aaaaacaatc atattgtatt atagtcagca ttttgctgta aatcattcat 108900
tatgatgttc ccctaaattg tttccactga caaaaaggat tcacagtggg ggtgagagta 108960
ttgcagtcta taaaggatgg gtttttagat gggttctctt cccatctttc ttaccggtgt 109020
tgatttttg tcctgatcag atacaacatt gaacggatgc cagatgatta tggagcccct 109080
cgattctaat tcttacaaga cacagttcct ctgcctttct tcgaccaact ttctcttgtt 109140
gccagttcat tacacaaaat gtagcgggat ttttaaggaa tcagagagac tgatggagtt 109200
cagggagata tttattattt aggtgcacca gcccagtcag attaacatcc aaaggactga 109260
accctgaaca gagttaagtt accttttaag cattttgtgg ggccgcgggg gttgggggga 109320
atctgtgcag ggggaagcat attacagaag caagaaagac agttattcaa ttaactgaga 109380
catgcattac atcatttctt acttttcaag gaaaatcatg ttttacgact tgagtttatc 109440
tgtctagtta ccttgcagct gcacagctag agaaacaggg tatttacaat gcctgggaaa 109500
ggaggagaga taaggctcac tagccacaga aaaacaggca gttaattttt aaaggactcc 109560
agctctttct ctttctcacg gggaattgga ttttcttaca tgcaactgaa tttctgctta 109620
cacattttta aatttctttt aattgctttt ccaatgcaat agcatgaatt attattctgt 109680
tgacctattt gccttactat gagctgaggg tagttcaata tgctcactct tttttttttt 109740
tttcttgaga tgaagttttg ctctgtcacc aggctggagt gcagtggcgt gatctgggct 109800
cactgcaacc cctgcctccc atccttcaag tgattctcct gcctcagcct cccgagtagc 109860
tgggattaca ggtacacacc accatgctca actaattttt ctttttgggg cgggggggggt 109920
ggatattttt agtagagaca aatacttttg tatttttaat acagaaaagt atgcctcggc 109980
ctcccaaagt gctggaatta cagacatgag ccactgtgcc tggcctgctc actttctgat 110040
gctgcttttt tgttgtttgt ttgtttattt gtttgttttg agacggagtc tcgctctgtc 110100
accaggctgg agtgcagtgg cgcaatctcg gctcattgca acctccgcct cccaggttca 110160
agcgattctc ctgcctcagc ctgcggagta gctgggacta taggtgcgtg ccaccacgtc 110220
cagccactga tgctgttttg ccactgatgc tgttttgaaa ttgatatttt gtctcataaa 110280
aactaggcca ggcatggtgg ctcattcctg taatcccagc actttgggag gccaaggtgg 110340
gtggatcacc tgaggtcagg agttggagat gagcctggac aacatggcaa aaccccacct 110400
ctactaaaaa tacaaaaaaa attagctggg tatggtgtca cgtgcctgta atcccagcca 110460
ctggggaggc tgaggcagga gaacctcttg aacccaggag gtggaggttg cagtgagctg 110520
agattgcgcc actgcattcc agcctgggtg acagagtgag actgtctcaa aaaaaaaaaa 110580
aaattaatga tggtcaaagg gtataaaatc tcagacagga agaatatgtt ttatgctttt 110640
tttgagttct gttgtacagt gtggtgccta tacttaataa tggggtatta tacatttcaa 110700
aattgatgtt ctcatcacag aaatgtattt gaagtattgc atatgttaac tagcttgatt 110760
taactattcc atattgtatc cacaatttat gatatcactt tgtaacccat acatttatac 110820
aattataaat tgtcaattta caataaaaaa tttttgttgc gtctttttag aaatcagata 110880
ttctgatgat tgctcacata atttggcggt cattgtttta tactgactta ctcaataaac 110940
atttattaaa tgtatactag gtgcttaata agagatggtc ctgccctcaa gacactcatt 111000
atggtgggaa agaaaaacat gtaaatgaat aaatgccgta aaatagcagt gctagggtaa 111060
aagtttcaga ggcatgtcat gggcacataa aggtggaatg atttttttct actggttggg 111120
atggagaggg aggaaaggcc agtggtcatg tggtttgagc taatcttaaa aatgaatagg 111180
tgcctcccca gtaaattagg aatggaagat gaagcatagg aaacagtaca ccatagaaaa 111240
tccattaacc tatgtataat cccagcactt tgggagactg aggtgggcag atcacttgag 111300
gtcaggagtt tgaaaccagc ctggccaaca tggtgaaacc ccacctctac taaaaattcc 111360
aaaattaact gggcgtggtg gtgcaagcct gtagtcccag ctacttggga ggctgaggca 111420
ggagaatcgc ttgaacccgg gaggcagagg ttgcaacgag ccgagatcac gccactgcac 111480

tccagcctgg gcgacagagt gaaactctgt ctcaaaaaca aacaaacaaa aaacctatgt 111540
atttgtgggg atttggtata ttattaaggt ggtatttcgt ttctgtggga aaaggaaatt 111600
tcattaacag gccaaatagt ctggagtcat tctgttagtg ttagaatact aattccacca 111660
gttaatatct gtatgagttg gacaaaccat ataaccatct gttgccttcc ttgtttcctc 111720
tttaaagtgt ttaatccgca aaacaaacct gataaagtga gattaaattg aatagtacct 111780
ggcccaagta agtgttcttc agtaaatgtt agtgattatt acacaatggc aaggtggcta 111840
tccatttgaa aaaaaattag gccgggcgtg gtggctcaca cctgtaatcc caacactttg 111900
ggaggccaag gtgggcggat cacctgaggt caggagccca agaccagcct gaccaacatg 111960
gagaaacccc atctctacta aaaatacaaa aaaacaatta ggcaggcatg gtggcaaatg 112020
cctgtattcc cagctactct ggaggctgag gcaggagaac gcttgaacct gggagatgga 112080
ggttgtgatg agccgagatc atgccattgc actcattcca gcctgggcaa caagagtgaa 112140
actctgtctc aaaaaaaaaa aaaaaaaaag agagagagaa aaaaattagt ttcctacttc 112200
acatgattca taaaaatatt tcaagtggat caaagaccta aatttttttt ttttttgaga 112260
cagtttctca ctctgtcacc caggctgcag tgcgttggca caatcatgac tcactgcagc 112320
cttgaccttc ccaggccctg atgatccacc ccagcctctc aagtagctgg gatttttgta 112380
tttcttacag agatgggatt ttgccttatt gcccaccttg gccttctaaa gtattgggat 112440
tatgggcatg agccactgca cccaccctaa acttatttct aagctatgga agcattagga 112500
aaataaatgt aaagaaaaac gtgtttatca cctttggata gaattcttaa gcagaatcta 112560
aaaagattga agagtttgaa tatattaaaa ttatagattt ctgtgcaaga cactcccctc 112620
ccctaaaaaa caagtgactg atgagatttt ccaatacatg taacaagggt ttagagaaat 112680
gagtactgat gtcatgctga cagaagtgca aatatttatg gccatttttt acagctgttg 112740
acaagatcca ctaaaataaa aattgcagat atcctgtgag cccaattcta ttttttttggt 112800
aactttatag tatcatacag gtgattcact ttaaaatacc tctgcatgga caacgtgata 112860
tgtatgcgaa gaaacattca catgtgtaca aggaagcagg tagaaggatg tttactgcac 112920
tgctgcttat aaaagagaaa aaacaaacta aatgtccatc aaaaggatct taactgtggc 112980
cgggcatggt gactcacacc tgtaatccta gcactttgga aggctgaggt gggtggattg 113040
cctgacctca ggagttcaag accaccctgg gcaacatggc gaaaacctgt ctctactaaa 113100
ataaaaaaat tagctgagtg tggtggtgcg cacctgtagt cccagctact caggaggctg 113160
aggcacaaga attgcttgaa cccaggagac agaggttgcg gtgagctgag atcacgccac 113220
tgcactccag cctggcgaca gagcgcgact ccgtctcaaa aaaaaaaaaa aaaaaaaagc 113280
ggtggggggt cttaactatg gaatgtcatg aacatctatc tacaaaaaat gaggtaggtt 113340
taaaatgggc aaaggactta aacatttctc caaagaagat atacacgtgg ccaagaaaca 113400
tgcaaaaaga tggtcaatat cactaatcat tagggaaatg caaattaaaa ccacaatgat 113460
atactacctc acaccccctta gccactatta aaagaaagaa gcaatagtta ttaaaaagta 113520
tttgtgagga tatgtagaaa ttgtaaccct tagctgtgca ctgcacatgc ccatagttcc 113580
agctactctg aaggctgagg tgagaggcct gagcccagga attcgaggct gcagtgagct 113640
atgattgcgc cattggactc cagcctgggc aacagagcaa gacaccgtct ctaaaagaag 113700
caaatcagaa cccttgtgcg ctgttcctgg aaatgtaaaa tggtatagcc acaaagaata 113760
tggcagttcc tcaaaaagtt aaaaatgaaa ttatcacatg attccagcaa ttccacttct 113820
tgacatgtat tcaaaagaat caaagatctt agagtatttg cccacccata ttcatcacgg 113880
cattatttac aatagccaag aggtggaagc aacccaagtg tccatcagca gacgaataga 113940
tcaacaaaat gtgctctcta catacaacag aatatgagtc acccttacaa aggaaagaaa 114000
ttctgacaca tgctgtaaca tcgatgaacc ttgaagccat tgtgctaagt gaaataagcc 114060
agtcacaaaa agacaaatac tgtgtgattc cgcttatatg aggtatctag agtagtcaaa 114120
ttcacagaga tggaaagtag tgtagtggtt gccagtggct gtggggattg gggaatagac 114180
agtttaatgg gtatggagtt tcagttttgc aagatgaaga gttctggaga ttggttgtac 114240
gacagtatga atgtactaaa gccactgaac tgtatgcttt aaaatggtta agatggccgg 114300
gcagggtggc tcatgcctgc aatcccagca ctttgggagg ccgggggcagg tggatcacct 114360
gaggtcagga gttcaagacc agcctggtca acatggcaaa acccggtatc tactaaaaat 114420
acaaaaaaaa tagctgggtg tggcagcacg cacctataat cccagctact caggaggctg 114480
aggcaggaga atcacttgaa cttgggaggc ggaggttgca gtgagccgag actgtgctcc 114540
agcctgggtg acagagaaga actctgtctc aaaataaata aaataaaata aaatggttaa 114600
gatggtaaat ttctgttat gtgtctttta ccgtaattaa tataaatgag attgagctat 114660
atcacttaaa tggaaataac tatgacctat tattaagaga aactaaatta tagaacaggt 114720
acagtgtgat attggtttta agttttaata tcacaaatat ttaattgttt atttagaaat 114780
atacagtata tatataaatg gatagaaaaa gttctgaaga taaagctttc tcttccgtct 114840
cttcactggt acagaattcc ttaaataaat taatgtcagg ctggacgcag tggctcatgc 114900
ctgtaatccc agcactttgg gaggccgagg caggtgaatc gcctcagctc aagagttcga 114960
gaccaccctg ggcaacatgg tgaaaccccca tctctactaa aatacaataa agtagccagg 115020
tgtggtagca ctcacctgta gtcccagcta ctcaggaggc tgaggcatga gagtcgcttg 115080
agcccaggag gaagaggttg caatgagctg agatcacgcc actgcactcc agcttgggct 115140
acagagtgag actctgtctc aaaagaaaaa aaaaagttat aggactggat tgtggctcac 115200
tcctcttgct gccataatat gagtggaatc atcctggata ggccaattat aatatcctta 115260
ttccattgcc attgtgatta tttcaaacct ggatatcaaa gacaagccaa ttagttgtaa 115320
ccagagcttt cactggagag ttacgctctt tttctatggg agaactgtta aaagctttaa 115380
atacaagctt taaaattgag agaccatatt tactaacaca ttggaagtgc ctacctgggt 115440
tttactttac atagccaaac ccaatcataa ttaatatcga atgtatgtct cctaaaccaa 115500
gagagaaaac agaaagaaat tcaattaatt aaaaaaaaag cagaatttgt caaaatgtat 115560
ggaactgcac cattggtgaa ctgtatgttt cattatgtgt aaattttttta aaaaactaaa 115620

aggaaaaata aatgtagaag aggagaggga agaagcagca acggtaaata caaaacaaag 115680
taagataagg gccgggcatg gtggctcatg cctgtaatcc cagcactttg ggaggccgag 115740
gcaggccgat cacaaggtca ggagatcgag accatcctgg ctaacatcct ggctaaaacc 115800
ccatctctac caaaaaaata caaaaaagtg agctgggcgt ggtggcgggt gcctgtagtc 115860
ccagctactc agaaggctga ggcaggagaa tggcgtgaac ccgggacggg gagcttgcag 115920
tgagccgaga tcgcaccact gcactccagt ctgggtgaca gagcgaaact ccatctcaaa 115980
aaaaaaaaaa agtaagatgg tgggaataag actagacatg cagtagtcat gatgaatgtg 116040
actgggttaa atagcctcat taaaagacag atgctcagat ttgatttttt tttttttaat 116100
cttgtggctg tgtgttattt acagacatag ctagaacaga atggcacagg acagctggaa 116160
ataaagggaa aaataaattt accatacaac tcaacaattc cactcctagg tatctaccca 116220
agagaaggaa aatacatgtc catacaaaga cttggataca aatgctcata ttttttttaa 116280
atttcttttt tttgagacag agtctcgctc tgtcaccagg ctggagtgca gtggtgcgtt 116340
ctcggctcac tgcagcctct gcctcccagg ctcaagtgat tctcctgctt cagcctcctg 116400
agagctggga ttacaggcac gtgccaccac acccggctaa tttttgtat ttttagtaga 116460
gatggggttt caccatgtta gccaggatgg tctcgatctc cggacctcgt gatccacctg 116520
cctcggcctc ccaaagtgct gggattatag gcatgagcca ccgcacccag ctgctcatat 116580
tatttttctt cataataaaa agtggaaaca aaccaagcaa ccataaacac agtggtcccc 116640
aacgtttttg gcaccaggga ctgtggaaga cagtttttcc acagatggca gtggggcatg 116700
atttctggat gattcaagca catcacattt attgtgcact ttattattat tacattgtaa 116760
tatataatga aataattata caactcacca taatgtagaa tcagtgggag ccctgagttt 116820
gttttcctgt aactagacag tcccatctgg ggttgatggg agacagggac agatcatcag 116880
gcattagctt ctcataagga gcacacaacc tagatccctt acatgtgcag ttcacattaa 116940
ggttcatact cctatgagac tctaacaccg ctgctgatct gacaggagct caggcggtaa 117000
tgtgagtgat gggtagtggc cgtaaataca gatgaagctt cacttacctg cccgccattc 117060
atctcctgct gtgtggtcca gttcctaaca ggccatgggc cactagtgat tcatggccca 117120
tgggttgggc acccctgcat aaccagataa atggataaac aaaatgtggt tcattcacac 117180
agtggaatac tgttcagcaa tgaaaggtat aaaccactga tagaacaaca tacatgaatc 117240
acaaaatcaa gtgaagcaaa agaagccaga tacaaaatat acagatggtg cctgacttag 117300
gatggtttga cttaggattt ttgactttac aatgggttta ctaggatatt acatgcattt 117360
tcagcttaca atatttcaat ttacacaaga tttattgggg ctgggcatgg tgggttcatg 117420
cctgtaatct ggcactttgg gaggccaagg caggaggatc acttatgccc aggagtttga 117480
gactagcctg ggcaacatag ggagacccca tctctataaa aaataaaata aaaagaaggt 117540
ttattgggat gtagctccaa cataagtcaa gaagcatcta cacactgtat gattccattt 117600
acatgaaact ctagaaaaga caaatctaat ctataatgat aaaaagtaaa tcgttatggt 117660
attaggggtg tccatgaaaa aaaagaaaaa gaaaagaaga aagtaaacat tgttcacctg 117720
aggttggagg tagggtattg gctggataca ggcacaatgg aattagtggg gagggaatt 117780
cattgtttct aaaaattatc tattttgatt gttgtgatga ttgcttgatg tattgattcg 117840
tcaaatcaaa atgcacacaa aatgatggca ttttattgtg tgtaaattat gcttcattaa 117900
ttaaaagatg gaaaaggaca cgcaaaagaa aaaataggcc aggtacagtg gctcattact 117960
ataatcccaa cactttggga ggctgagtgg gcggatcact tgagcctcgg agtttcagac 118020
cagcctgggc aacatggtga aactctgtct ctacaaaaaa tttaaaaatt tgctgggtgt 118080
ggtggtgcaa ttgtgtagtc ccagctacta gggaggctga ggcagaaaga tcgcttgagc 118140
caaggaggtc gaggctacag tgagctgtga tcgcgccact aaactccaac ctgaacaaca 118200
gagtgagacc ctgtctcaaa aaaagaagaa aaaaaagaaa aaagaaaaaa tggaaaaaga 118260
atatgctgtt tgaatatgca agatggaaaa tattgaggtt ttctttgtgg gctaacacat 118320
ggaaaatgtc atgaaagtac aatgtacact tgaaaaggag gtgcactctc tgttatgtag 118380
gtttaatatt tatccataag atctacccaa ttatttcggt cttctgtatg cttatacttt 118440
tttttttga gacggagttt cactttttt tttttttttt ttttttgaga cggagtctcg 118500
ctctgtggcc caggcgggag tgcagtggcg ctatctcggc tcgctgcaag ctccgcctcc 118560
tgggttcacg ccattctcct gcctcagcct cccgagtagc tgggactaca ggcgcccgtc 118620
atcacgcccg gctaattttt ttgtgttttt agtagagacg gggtttcacc gtgttagcca 118680
ggatggtctc gatctcctga cctcgtgatc tgcccgcctc agcctcccaa agtgctggga 118740
ttacaggcgt gagccaccgc gcccggcctt gagacggagt ttcgctctta ttgcccaggc 118800
tgcagtgcaa tcgcgtgatt ttggctcact gcaacctgcg ccacccaggt tcaagtgatt 118860
cttctgcttc agcctcccaa gtagctggga ttacaggcat gcaccaccat gcccggctaa 118920
ttttgtgttt ttagtagaga tggtgttttg ccatgttggc gaagctggtt ttgaacccct 118980
gacctcaggt gatccaccca cctcagcctc ccaaagtgct gggattacag gtgtgagcca 119040
cccatgccca gcctatgctt atacttttta tttttgactc tttcagaagt caaaagcaga 119100
aaaatacatt agagtgtcct aaaactcgtg cttttttttt tttttttttt tttgaggcaa 119160
agtcttgccc tgtcacccag gctggaatgc aatggcacaa tctcagctca ctgcaacctc 119220
cgcctcctgg attcaagcga ttctcctgcc ttagcctctc aagtagctag gattacaggc 119280
gtgcgccacc atgcctggct aatttttgt atctttagtg gagacggggt ttcaccatgt 119340
tgaccaggct ggtctcgaac tcctgatctc gtgatctgcc cacagcctcc caaagtgctg 119400
ggattacagg tgtgagccac cacacccagc tttttttttt ttttttaact acttagtgtt 119460
tccacttcat gaaagtcgct actgtattgt tttgtgcatg gatactcata actgatatag 119520
ttcttcctaa ttgtagcctt tggcattgtg ttagtccgtt ttcatgctgg tgataaagac 119580
gtatcagaga gtgggtaatt tataaacaaa aggaggttta atggactcac tgttccatat 119640
ggctggggag gcctcacaat catatgatgc aaagtgaaag gcatgtctta catggtggca 119700
gaggagagaa taagagcgaa gtgaaaagga aaactcctta taaaaccatc agatctaatc 119760

ccagcacttt gggaggctga ggcgggcaga tcacaaggtc aggagatcaa gaccatcctg 119820
gctaacacag tgaaaccccg tctctactaa aaatacaaaa aattagctgg acgtggtggc 119880
aggcgcctgt agtcccagct tcttgggagg ctgaggcaga agaatggcat gaacctggga 119940
ggtggagctt gcagtgagcc tagatcgtgc caccgcactc cagcctgggt gacagagcga 120000
gactccatct caaaaaaaaa aaaaaccaaa aaccaaaaaa ccatcagatc tcgtgagact 120060
tattcactac catgagaaca gtatggggga aattgccctc atgatttaat gaactctcac 120120
cgggtctctc ccacaacaca tgggaattat gggagctaaa ctcaggatga gatttgggtg 120180
gggacacagt caaaccatat caggcattat aatatatcct tttgccttat ttaatgattt 120240
ttggcctaaa ttctactttt tctgatgtta agaacaggac ttaaatgctt tctttttcta 120300
cttctttttg tattgcattt accttatata ttttgtccat cttttgattt ttacctttc 120360
taaattcatt atccccaaag tagggtgctc cagactagtc actgctgtgt gtcctttgtt 120420
ttctttttct attctttttt tttttttttt ttttgagaca gagtctcgct ttgtcaccag 120480
gctaaataaa gtgcagtggc gcgatctcag ctcactgcat cctttgcctc ccaggttcaa 120540
gggattctcc tgcctcagcc tcctgagtag ctgggattac aggcacgcgt caacacaccc 120600
agctaatttt tatattttta gtagagatga ggtttcacca tgttggccag gatggtcttg 120660
atatcctgac ctcgtaatcc acctgccttg gcttcccaaa gtattgggat tacaggcgtg 120720
agccactgcc cctgactgct tttttttttt tttttttttt aagagaaatc tccctctgtc 120780
acccaggctg gagtgcagtg gggtgatctt ggctcactgc agcctctgcc tcttgggttc 120840
aagcgattct cctgcctcag cctctggagt agctgggact ataggcatgc accaccacac 120900
ctggctaatt tttgtatttt tttagtagag tcagggtttt gccatgctgc ccaggctggt 120960
ttcgaactcc tgacctcaag tgatcctccc gcctcagccc cccaaagtgc tgggattaca 121020
ggcatgagcc accagaccca gctttttttc tttttaaaa cgggagtgtt cactgtggat 121080
gttctgttcc tattccgcca ttgtatatgt ggtgtttaaa ggcagattat ttgtcctttt 121140
gtcgcatagg tctctagatc aagtgaagcc acacctcctg agatagaaac tactacatat 121200
accccttgct tgcatcacct acatatacct ctactctgca tcacctagat gtggtggtcc 121260
ttgagcatga tgacattact ggacaggact ttaagattgt ctctgtgggt gagggaattg 121320
tgtatacttt gcctgtgatt gggaaagtga gttcaacatt tggtaaacag aagggtagag 121380
tgtagcaacg gcttgtgctg gtcaccaagt attctggctc tctgttttac ggaccaaggt 121440
agcattgctc ttccccacca cctttaaagt cagatgtggc aaagcgactt gctttggcca 121500
atgcaatgtg agtgaagatg acatgtatca tttcttgaca gaagctttaa gtcattgtgt 121560
gattagctgt gccccctttg ccctgtgttg gtaatcatga aagcctggaa atggaaactg 121620
acttggcctg ggtcccacag aacatctcag ccagttcatg gtggtgacat tagggtgaac 121680
aataagtaag catttcttgt tttaagccat tgatatttgg gttgttgttt gtttgtttgc 121740
ttttattact gcagcatgac cttggcctat accaactgat aaagtatatg ttaaaaagtt 121800
ggagttgaag caattagaaa agaaaaagaa ataaaagata tccaaattgg aaaggaagta 121860
aaatgatctc tattcacaga tgatatgatc ttacatatca aaatccctaa agaattcaca 121920
aaacactgtt acaatgaata aatgatttca gcaaagtttc aggatacaaa atcaacacac 121980
aaaaatcagt agcattttat atagtaataa tcacatgact gatctgaaaa agaaattaag 122040
gaaccaatcc catttgctat gcatcaaaaa ggataaaata cttagcagta aaactaacca 122100
aagaggtgac agacttgtac actgaaaatt atagaacatt gctaaaagaa attttttttaa 122160
aaagatacaa aaatatagaa agacatccca cactcatgga ttagaagacc ccatatcatt 122220
aaaatgtcta tactacccaa agcgatctac agattcaatg taatccttat gaaaatccca 122280
atggcaggtg gggcgtggtg gctcacgcct gtaatcccag cactttggga ggccaaggcg 122340
ggcagatcac taggtcagga gttcaagacc agcctgacca acatggtgac accccatctc 122400
tactaaaaaa tacaaaaatt acccaggcat ggtggtgcac tcctgtagtc ccagctactt 122460
gggaggctga ggcaggagaa ttgcttgaac ctgggagatg gaggttgcag tgagccaaga 122520
tcatgccact gcatcccagc ctgggcaaca gggcaagact ccatctcaga aaaagaaagg 122580
aaaaagaaaa aaacaaaaga aaatcccaat ggcacttttt gcggaaatag aaaaagccat 122640
cctaaaattc atacaaaatc tcaagggacc ccatatagcc aaaaaaaaaa aaatcttgaa 122700
aaagaagact gaagttggag gtctcacaat ttctgattgc gtactacaaa gcagcagtaa 122760
tcaagaaagc atggaactgg cataaagaca gatatgtaga ccaatagaat agaatagaga 122820
gcccagaagt aaacccttgg atatttggcc aaatgatttt taacaagggt aatgctggag 122880
aaaggtcact ctctttgaca aattctgttg agaaagttgg atgtccacat gcaaaataat 122940
gaagttggac cttacaccat atacaaaaat taactcaaaa tggattaaaa acctaaatat 123000
aaggactaaa actataaaac tcctagatta agctgggcat ggtggctcac gcctgtaatc 123060
acagcacttt tggaggccaa ggcaagcaga tcacctgagg tcgggagttc gagaccagcc 123120
tgaccaacat gttgaaaccc catctctact aaaagtacaa aattagctgg gtgtggtggc 123180
tcgtgcctgt aatcccagct actcgggagg ctgaggcagg agaattgctt gaacctggga 123240
ggtggaggtt gcagtgagcc gagattgcac cattgcactc cagcctgggc aacaagagca 123300
aaactgtgtc tcaaaaaaaa cccagaaatt tattttttca cagttctgga gtgtaaggaa 123360
agtccaagat gaaggcactg gcatctggtg agggccttct tgctgtgtcc tcacatggca 123420
aacaaatgac aagctagtca aaggctgcat aaagcttctt ttgtaagggc cttaatcctg 123480
ttaatgaggg aggagcccta ctggcctaat cacctcttaa agatctcacc tcttggccag 123540
gtgctgtggc tcattcctgt aatcccagta ttttgggagg ccaaagcgga tggatcatct 123600
gaggtcagga gttcaagacc agcctggcca acatgttgaa accccatttc tactaaaaat 123660
acaaaaaaat tagctgggca tggtagcgca tgcctgtaat cccagctact cacgaggtta 123720
aggctggagg attgcttaaa tccgggaggc agaggttgca gtgagccgag atgatgccac 123780
tacactcaag cctgagcaac aacagtgaaa ctccctctca aaaaaaaaaa aaaacaaatg 123840
ccacctctta atactattat agtgacatca tgtagatttt ggaggagaca cattcaaatc 123900

atagcagatg gtaaatttaa tattatcttt tttaaaaaaa acacaaagta aatatggata 123960
tgtagatcaa agcaatgcac agtgggaaat gaaagtttaa ttatcaaagg aactgttgat 124020
cagaacatca tatccattta aggttgtttt attggttttt gttttgtttt gttttgtttt 124080
gtttttgttt tttttaggca gggtctggct ctgtcaccca gactggagtg cagtggcatg 124140
atctcagctc actgcaactt ctgcctcctt ggctcaagcc atccttccat ctcagcctcc 124200
caggtatctg ggacaccaca tccagctaat ttttgtattt actgtagaga tggggtttcg 124260
ccatgttgcg taggttggtc tcaaacttct gagctcaagc gatcacccac ctcagccccc 124320
gaaagtactg ggattacagg cgtgagccac cgtggcttgc ctaagcttta ttttaaaggc 124380
tgttttcaat attatttgaa gattgggcag tacatgatgc aatcaaacaa aacacaggtt 124440
tcaggtatgt ataaatactt atgctttatt tcatatataa tgctgtgtgt tccttgacac 124500
acacagttct atgtattcca agtggtcact gtagtttatc acaggaaaat gttaatgccc 124560
tagaaatttt catctgaggt atctaagccc ataataggtc atcattaaat agtctctgaa 124620
ttattgttgt gtattatata ttgtgtgtat tttgtgtatt atatattgtg tattataagt 124680
tacaccacaa atactacaat atgttcgctc actctggaaa ttaaaaagtg ggagatacac 124740
tttaataagt ataatggatt gacccataga tgactttata ttacataaac tacttaagac 124800
atattctttg tgctgctttt tctaaaatag aatatgttaa tataaacata tttgaaattc 124860
aaaaagcttg gcctgtgtct gttttgtttc tcagtgcaat ttctgttatc aatgtgctta 124920
taagtaataa tttattaata ctactaataa aaaattaaca tgattgagag gaaaaaaatc 124980
tggattaatt agctaaagaa ataaagcaac ccaggagagg gaaaaataag ttataaaaaa 125040
gaaagtgggc caggtgcggt ggctcacact gtaatcccag caatttggga ggctgaggca 125100
ggtggatcaa ctgaggtcag gagttcgaga ccagcctggc caacatggtg aaaccccatc 125160
tctactaaaa atacaaaaat tagccggaca tggtggtgtg cacctgtaat cctagctatt 125220
cgggaggctg aggcaggaga atcatttgaa cccagtaggt ggaggttgca gtgggccaag 125280
atcgcactac tgcactccag cctgggtgac agagcaagac tccatctcag aaaaaaaaaa 125340
aagaaagaaa gaaactggct gagacaggag gatcatttga gccccaggag tttgagatca 125400
gcctgggtaa caaaacgaga ccctgtctct acaaaaaata caaaaattag ttgggagcag 125460
tagcatgcac ctgtagtctc agctacttgg gagactgagg caggaggatt ccaattcctt 125520
gatcccctga gttgaggatg caatgagcta taatcctgcc actgcactcc tgcctgggtg 125580
acagagtaag atcctgtctc aaataaatga gtaaataaat aaatggccag gcatggtggc 125640
tcatgcctgt aatcccagca ctttgcgagg cctaggcgga tcacccgagg tcaggagttt 125700
gagaccagcc tggccaacat ggtgaaaccc cgtctctact aaaaatacaa aaaaattagc 125760
caggcgtggt ggcaggcgcc tgtaatccta gctactcagg aggctgaggc aggagaattg 125820
cttgaaccca ggaggcggag gttgcagtga gccgagattg tgccactgca ctccagcctg 125880
ggcgactgag tgaaactcca tctcagaaaa aaaataaaaa ataaaaataa ataaatgtat 125940
ctgtctgttc tcacactgct ataaaaatct ataataccta agattgggta atttatgaag 126000
gaaagaggtt taattgactc acagttctgc aggctgtaca ggaagcatgg tgaggggagg 126060
cctcaggaaa cttacaatca tggcagaagg tgaaggggag gcaagcacgt cttaccatgg 126120
cagagcagga gagagacagt gagctaatag gggagtgcca cccactttta aaccagcaga 126180
tctcatgaga actcactcac tatcatgaga acatgggaga agtccgcctc catgatctaa 126240
tcacctccca ccaggtccct cccccaatat tgggaattac aattcaacat gagatttggg 126300
tggggacaca gagccaaatt acatcaatca atcaataaat aagaaactag caatctaaaa 126360
gagaggaaga gaagagaaaa agaaggccag gcactgtggc tcacgcctgt aatcccagca 126420
ctttgggagg ccgagatggg tggatcacga ggtcaggaga tcgacaccat cctggctaac 126480
atggtgaaac cccgtctctac taaaaaatac aaaaaaatta gccaggcgtg gtggcgggcg 126540
cctgtagtcc cagctacttg ggaggctgag gcaggagaat ggcatgaacc caggaggcgg 126600
agcttgcagt gagccaagat agcgccactg cactccagcc tgggagacag agcaagactc 126660
tgtctcaaaa aaaaaaaaaa aaaaaaagac aggtgcaaca actgaaaaag cacaaaataa 126720
aattgttaga tcttctcact taatctttat atatataaat atatatgtaa tatatatttt 126780
atttttgagt tggagtcttg ctctgtcacc caggctagag tgcagtggcg tgatcttggc 126840
tcactgaaac ctccacctcc caagttcaag caattcttgt gcctccacct cctgagtaac 126900
tgggattaca ggtacatacc accatgccta gctaaatgtg tattttcagt agagacaggg 126960
tttcaccatg ttggccaggc tggtctcaaa ctcctggcct caagtgatct gcccacctca 127020
gcctctcaag tgctgagatt acaggcatga gctaccacac ctggcctcaa tcttctaaat 127080
attttgacct cttttacata tttgccattt tcttctcttg tgtgcattct ggatctttcc 127140
tgaattctat ctttcagttt tctgattctc tcttcagcct tgtctaatat gctgctaaat 127200
tgttaaattc actcatttgg ttttttcttt cagttagatt ttttactact agaagttctg 127260
ttttgttctt tttcggatct gtgttttctt agctcatctt ttcaagcctt tttttttttt 127320
tttttttttt ttgagatgga gtctcgctct attgcccaga ctggactgca gtggtgcgat 127380
ctcagctcac tgcaaccttc gcctcccggg ttcaagtgat tctcctgcct caggctcccg 127440
agtagctgtg actacaggca tgcaccacca tgcccagcta atttttgtat ttttaataga 127500
ggtggggttt caccatgttg gccaggctgg tttccaactc ctgaccttag gtgatgtgcc 127560
tgtctcagcc ttccaaagtg ctgggattac aggcatgagc cactgcaccc agttcaaatt 127620
actctaagta tatatttata ctcccatcaa cagtacatgt gaatttcaat ttttccacaa 127680
cctcattaga acttggtatt gtcagtgtgg tagatactgc cagttgtccc aggatctact 127740
ttccccttct tcctttaata acagatttaa tgagtttcac ctggattgca gctgggcatg 127800
cccaaccaaa tcatagacaa gatgatgtgg ctgaaagtaa tatgcatcac ttctgggtca 127860
tgaccttagg aagcatgctc cttagaaggt atgcttccca aactcgagat ctttttgatg 127920
atgttctttt tgcatttggt ctatacttcc aaattccttt ttatgtccct gtatttgaac 127980
acatttgcct gacaatgaag gccccttccc caggtagtg aatatttatt gtttttgctt 128040

gcccacatcc actccttctt cctaatagta cctaaattat ttttcttgaa atgttacatt 128100
ttcctcaata tagtagtaga atttagatca gagtgcccag ccctttccct atagaagctg 128160
aaggggagca ctgaaggctc cttctttcag aacccttctc tcactatcat cccatatatc 128220
taagaaggca ttcagtggct tagggagaaa aaaaaaagag ttgaaccttg tttcttccag 128280
catccagagc cttgacggtg gaagggaaga agcacagcat gaatattgcc aaggttatat 128340
atgaccaggt tactctgagt ctggacacaa cccatcgaga atcctggtga aatgcctgcc 128400
taagggtaga ccccatagca gttaatctgc atctagatcc tgttgtttcc aattgcataa 128460
gctacaagag ctagcagagg ttttattcca gagaatggta tgtaaaccac cagccagtca 128520
gaatatgtcc tcaaaccact agctaataaa atgtcacgtc ctcttagagg cctcattcaa 128580
atcactctag taaccctaag aatgaaatgt aacaagtaag aaatgcagct aggttataaa 128640
cctagtctga aggagactaa tggtagttga ctttgacaga actgacaatt ggcagctgac 128700
ttctctgggg tctcccacac aaaaccttct gtgtatttgt gcttcaactc atcttttaga 128760
ccattctgaa tcagagcact ttatattttt attgttcttt acttttcatg ttctggattg 128820
gtgaaattaa tctctgaaaa atgggctggg ttggtcttta taatgtgaag tcaatgttac 128880
taagctgtca tgaggagagc attataccaa caatgaggct tcctttaaag aattttttgg 128940
actggatgtg gtcattcaca cctgtaatcc cagcaatttg ggaggccaag gcaggaggat 129000
tccttggagc caagagtttg agaccagcct gcgcaaatag tgagacccta tgtccaccaa 129060
aaagtaaaaa gttaaaaaat tagccaagaa tagtggcgca tgcctgtagt tccagctact 129120
ctggaggcta aggctgtggg atcacttgag cccaggaggt caaggctgca gtggctgtca 129180
tggcgccact gtactccagc ctgggcaaca gagtgagctc ctgtctctaa aaaaaattta 129240
aaaaggaggt tgggtgcagt gactcatgct tataatctca gcactttggg agactgatca 129300
cctgacctca ggagttcgtg accagcctgg gtaacatggt gaaaccccgt ctctactata 129360
aatgcaaaaa ttggccggat gcagtggctc aagcctgtaa tcccagcact ttgggaggcc 129420
gaggtgggtg gatcacctga ggtcaggagt tcgagaccag cccagccaac actgtgaaac 129480
cctgtctgta ctaaaactac aaaaattagc caggcgtgat ggtgcatgcc tgtaatccca 129540
gctactcggg aggctgaggc aggagaatcg cttgaacccg cgaggtggag gttgcagtga 129600
gctgagattg tgccactgca ctccaacttg ggcaacagag tgggactctt aaaacaaaac 129660
aaaaagcaaa aattagccac gcaaggtggc tcatgcttgt aatcccagct actcgggagg 129720
ctgaggcaag agaattgctt gaacccggga ggcggaggtt gcagtgagct gagattgtgc 129780
cactggactc caccctgggc gacagagcaa gacatctcaa aaaaaaataa taataattaa 129840
ttaatttttt aaaaattgcc tggcacagtg gctcacacct gtaatcccag caccttggga 129900
ggccaaggcc ggtggatcac ttgaggtcag gagtttgaga ccagccttgc caacatggtg 129960
aaaccccatc tctactaaaa atacaaaaat tagctgggcc tggtggtgca cacctgtaat 130020
cctagctact caggaggctg aggcaggaga atcgcttgaa cccaggaggc agaggctgca 130080
gtgagccgag attgcgccac tgcactccag cctgggcaac agaacgagac tccgtctcaa 130140
acaaaaaaaa aacttaaaaa aagaacaaat tttttggaga cggggattct gcataacaaa 130200
aatgactgct gggcgcggtg gctcacgcct gtaatcccag cactttggga ggccgaggcg 130260
ggtggatcac ctgaggtagg gagttcaaga ccagcctgac caacatggag aaaccccgtc 130320
tctactaaaa atacaaaatt agccggggtg gtggcgcatg cctgtaatcc tagcaactcg 130380
ggaggctgag gcaggagaat cgcttgaact cgggaggtgg aggttgcggt gagccgagat 130440
cgcgccattg cactccagcc tgggcaaaaa gggcaaaact ccatctccaa aaaaaaaaca 130500
aaaaacaaaa atgactatga tctttgaaaa ataattgtgc aggctagaaa ctttgagtgg 130560
ctgaagtata tgttagttcc ccgagagtaa gattctaggg gataggatcc ccatggcatc 130620
tagaacaatg cctaacttta tcttgaaaca atgaggggaa tgtgaaaggg cctgtttaaa 130680
cagtggtgct gtctcccaac taggtgctga aagacccttt gtgttgaggg cagggatctg 130740
gttagaggac ctgagagcct taagctatca ggccaagtgg gaaaggaaaa ggccaagaga 130800
agagtttcca cccacctcca cctaatctta cctttctctg tcttccacta ccatcacttt 130860
tttttctttt tcccaccttt tttttttcaa aactttttttc tttttttctt gatacagggt 130920
ctcactctgt tgcccaggtt ggagtgcagt ggtgtaatct cagctcactg caacctctgc 130980
ctcctgggct caagccatcc tcccacctca gcctctggaa tagctggaac tataggcatg 131040
caccactatg cctggctaat ttttgtattt tttgtagaga tggggttttg ccatgttgcc 131100
caggctggtc ttgaacttgt gagctcaagc aattggccca tctcggcctt ccaaagtgct 131160
gggataacag gtgtgagcca ctgcacccag cttaaaatgt tttttaaaat tgtgacatac 131220
agcacacaga caggaaagca caagggataa atgtacagct ccatacgttt taacaaagaa 131280
aatatttgtg aaattaccac ttagttcaag aaattggctg ggcgcagtgg ctcatgcctg 131340
taattccaac actttgggag gccaaggtgg gtggatcatc tgaggtcagg agttcaagac 131400
cagcctggcc aacatggtga aaccccatgt ctgctaaaaa taaaaaaatt agctggacat 131460
ggtagtgggc acctgtaatc ccagctactc aggaggttga ggcaggagaa tcacttgaac 131520
ctgggaggtg gaggttgcag tgagccgaga atgtgccact gtactccagc ctgggtgaca 131580
gtgctagact gcatctcaaa aaaaaaaaaa aaaaaagaaa gaaatagaac attgtcagca 131640
ccctagaaac ccactcatgt ctctcgccac caggcctact ctttcccttc ttcccaaagg 131700
caactactac cctcacttct aacattttaa tttgtttgct tatttttgaa atatatgtaa 131760
agttaattgt acaataaata ttcttctgaa ctactattgt tcaacatttt catgaaggta 131820
catttattaa attatagcat atgggaaaat gcacaaatca taagtggtac agcttgatga 131880
atttttcaca aactgaatgt atctatgtaa ctagcatgca gatcaagaaa tggagcatta 131940
tcagaagtcc cctttgtgcc tccttctagt cactatccct ctctcccaag agtagctact 132000
atcctgagtt ctaacagtat aaatcagttt tattatttta ccttatatac atgaaaatat 132060
acagtatata ctcttttctt cgtagctttt cttgctcaat attgtttgtg aggttcatct 132120
gcattgttgc acatagttgt agatcactca ttctcattgc tgttttgtat tccattgtat 132180

aaatgtacca caatttgacg attcattcta gtgtaggtgg atatttgggt tgttttttcc 132240
attttcagct attacaaata ctgccagtat gggcagacat ggtgccttac acctgtaacc 132300
cagcacattg ggaggcctag gcaggcagat cgtctgagct cagaagtttg agaccagccc 132360
gagcaacatg ctgaaactct gcctccacaa aaaatacaaa aattagctgt gcatggtgcc 132420
gtgcacagta agtagtagtc ccggctactt gggaggatga ggcgagaggg tcacttgtgc 132480
cagggaggtt gaggccacag tgagctgtga tggtgctact atttgtttgt atataaatag 132540
tatatttgtg gccggatgca gtggctcacg cctgtaatcc cagcactgag gaaggctgag 132600
gtaggcagat cacgaggtca agagatggag accatcctga ccaacatggt gaaaccctgt 132660
ctctgctaaa aatacaaaaa ttagccagcc gtggtggcgt gttcccgtaa tcccagctac 132720
tcgggaggct gaggcagaat tgcttgaact caggaggtgg aggttgtagt gagccgagat 132780
catgccactg ctctccagcc tggtgacaga gggagactct gtctcaaata aataaataaa 132840
taaataaata aataaataaa taaataaaaa taaataaata aatagtatat ttgtacactg 132900
ttagaactca ggatagtagt gggagaagat agtgactaga aggggcacaa aggggacttc 132960
tggcaatgct tcctttcttg atctgggtgc tagttaaata gatgcattca gtttgtgaaa 133020
aattcatcaa gctgtaccac ttatgatttg tgcacttttt ttttcttgag acggagtttc 133080
actcacacta tcgcccaggc tggagagcag tggcacgatc tcagcttaca gctcactgca 133140
acctctgcca cttgggttca agtgattctt ttgcgtcagc ctccccagta gctgggatta 133200
caagtgtgtg ccaccacgcc tggctaattt ttctattttt agtagagaca gggtttcacc 133260
attttgacca ggctggtttc aaactgctga cctcaagtga tccacccacc ttggcttccc 133320
aaagtgctgg gattacaggc ataagccacc gtgcccagcc tgtgcacttt tttatatgct 133380
atgatttaat aaatgtttct tcatgaaaat gttgaacaat gtgatcactc cagcctgggt 133440
gacacggcga gattctgcct caaaaacaaa aacaaacaaa tactgccagt ataaacattc 133500
ttagttatgt ctcccggtga acatatctat gcatttcttt tttttttttt tttttgagac 133560
ggagtcttgc tctgtcgcca ggctggagtg cagtggtgcg gtcttggctc actgcaacct 133620
ccacctcctg ggttcaagcg attctcctgc ctcagcctcc caagcagctg agattacagg 133680
catgcaccac catgctcggc taatttttgt atttttagta gagatggggt ttcaccatgt 133740
tggccaggct ggtcttgaac tcctgacctg gtgatctgcc cgcctcggcc tcccaaagtg 133800
ctgggattac aggcgtgagc cactgcaccc ggaccacacc tatgcatttc tgttggttat 133860
atgccttaga gtaagattcc tgggtcatgg tctgttcaga tttggtagat actaccagac 133920
agtatttcag agtagtcatc ccaatttaca ttcccaccag cagtgtatga gaatttcatt 133980
tgtttcacag ccttgccaac cttcaatatt acatctctta ttttagccat gttgatgagt 134040
tgtacatata ctggtatctc attttggctt tagttcacat aaccctggtt tctattgagg 134100
ttgaattcct ttcacatgta ttgataattt aacttttatt ttatgtgaaa taccttcaca 134160
tctcattttt ctcctgtaag ttgtcatttt caaattgatt tgtgggattt ctctacattg 134220
tctaaatgaa tcctttgcag gttaaatgcg tcacaaatat ttccttgtac tctgtagttt 134280
ctcagtttct ttatgaaagg tatctatgta tagattatat atgtacatgt ttatttgtat 134340
attggcttta tgcccagcaa tcttttgatt cccataattt acctgtagat tattttggat 134400
tttgaatgta cacaatataa gtaaacaatg acaattttgt ttcttcctct ccaatactta 134460
tttccttcct tccttccttt cttccctccc tccttcctct ctcccttcct ccctccctcc 134520
ttccctttc ctttcttttt atacccattg cattagtagg gcctgcatta caatgctgat 134580
aagatattgt attagtttcc taaggctgct gtaacaaagc accacaaact gggtgcctta 134640
aaacaacaga aatgtattct tcacagttct agggactaga agtctgaaat catggtattg 134700
gcagggtcat gctccctttg aaacctgtag gggaattctt tccagcttct tcttagcttc 134760
tggcggtttg cggccaatct tgggttttcc ttgtcttgta gcagcataat cccagaatcc 134820
cagtctctgc cttcatcatc acatagtgtt ctccctctgc ctctgtgtct tcacatgacc 134880
atcttatttt aaggacacca gatacattgg atttggggcc cactctaaca tgacctgatc 134940
ttaactaatt acatctataa ctacctgatt tccaaatgag gtcacattct gtagtactgg 135000
gtgttagaac ttcaacatat tcaactcata acagatgtga tgatagtgga cgtctgtact 135060
tgatcttagt ggcaaaactt tcaacttttc accattatgt gtgatgtcag ccgggtatgg 135120
tggctcacac ctgtaatcct agcactttgg gaggccgaag cgggtggata acttgagccc 135180
aggagttcaa gacaagcctg gccaacatgg tgaaacccca tgtgtatata tatatatata 135240
tttttttttc tttgtttttg agacagagtc ttgctctgtt gaccaggctg cagtgcagtg 135300
gcatgatctc agctcactgc aacctccgcc ccccgggttc aagcaattct cctgcctcag 135360
cctcctgagt agctgggatt acaggcatgt gccaccacac ccagctaatt tttgtatttt 135420
tagtggagat ggggtttcat catgtcggtc aggctgctct tgaactcctg acctcaagtg 135480
atccgcctgc cttggcctcc caaggctggg gttacaggtg tgagccacca tgcccggcct 135540
aaatttatac atttttcaaa agaaaatgtc agcccaaaat attaaaaaca caactaacta 135600
gggtttctgg gttgggttcc tgatctgggt gccgatttca caagtgtgat tggcttgtga 135660
aaatttacta tgctgtctac ttaagacatg tgttaaataa aatttatagc aagttgggca 135720
ttaggcccaa cagaacaaac caaaatggag tttattgatg ttgccatgcc aaaattaaaa 135780
tctttatctg accattcaat aaaccaggag agagagaaat aatagccaag tctgcaaaca 135840
gtccattttt agccagcatg ataaagaagt cctctctgct ttaaccttat aagaaaagaa 135900
actttgaaac aaacaatcca cttttagttt tctgtttctc tctttttttt ctttttttgag 135960
atggagtctc gctctgtcac ccaggctgga gtgcagtggc acgatctcag ctcactccaa 136020
cctctgcctc ctgggttcaa gtgattctcc tgcctcagcc tcctgagtag ctgagattac 136080
aggtgagcac caccacgcct ggatactttt tgtattttta gtaggcatgg ggtttcacca 136140
tgttgaccag gctggtcttg atctcctgac ctggtgatct gcctgcctca gcctcccaaa 136200
agtgttggga ttacaggagt gagcccccgc gccctgcctc tgcgtttctt ttctgtctat 136260
gaaattatcc cgattgttct gctcattgga acactgtttc tgctttatag aatgagacgt 136320

tgcccaattc tagaatagca ggtaaaagtt aattaagatc tttaaactaa atttgttgta 136380
attttgtctt ttgacatgta attttctgta tattcgttat acttcaataa aaagttaaaa 136440
gaaacataac taaatttcta tttgaaaaca gaagctcttc cttttgaagg atgccttgct 136500
gctccacttt ccattcacat tcacctcctg agagaggtga agatctgggt tgctaagatg 136560
gaagaaaggt caattatttg catgttactc aagtgatgca ctgacacaat attctcacag 136620
ttctccctcc ccccgccccc ccccacacac ccaaaagaat tcagtgtatt agctttgctt 136680
tttagtgatt cactcaagct aaagtaagag tttatgtctg gtattacgtt gagagctttt 136740
ggaagattat cttccaggtt gtctttagac catctttgta tctggttttt gctctctttg 136800
cttttgctct gagattctgc aacaccgatt tatatgtgtt gagacagtct aaaaaattcc 136860
atttctcccc ttctacaatt cctgcagatg ttactcacta tatgtctgca ggaattggtg 136920
taatcttaca ctctgccttg aaaacacctt tccattcttc acagctttgt tcccagttga 136980
atttctgggg agaattgagt taggtctatt catttaattt gggagaaact gacatcttta 137040
caatttttcc tcttttcacc caggatcatg gtaagcctct ccattatttg aagtctaatt 137100
tcatcctact tggatatgct tctttatgac tctgtagctt tctacatata agtcctacat 137160
ctttcttgtt agagtttttc ctagttgctt tatgtttttt tgttgttgtt gcgattgttg 137220
ctattgtgaa tggaaccttt tttttttttc attcaaaaat gaaccccgtgg agcgatagga 137280
tctctaccac ctctttcggc tgtgagactc tatggttttt cagttctatg agaacattct 137340
actggagcct acattatctg aacccaaagg aagatggcaa ccacgagtcg ccaagtaaag 137400
gaccgctcta tctcgccttt gttctcctct cggtggttac tgcgcagacg tagtcgccca 137460
agtcgcgtcc ccgccttccc ggtcgcgggc ccagctcggg agcgccggcg cactggcgcg 137520
ctccgtttac acgctccggg gcctgtaggc gccgcgagtt ccggctgtcg ccgtcgccgc 137580
cgcggctcct ggaggtcggt tgcgacgagt aacggcgcca ggacgagccc tgcgccttct 137640
ttttcgatat gtacccgaat tggggccggt atggcgggag cagccactat ccgccgccac 137700
cggtcccacc gccgccgcca gtggcgcttc ctgaggcctc gccggggccc gggtactcga 137760
gctcgacgac tcccgcggcc ccctcctcct cgggcttcat gagcttccgc gaacagcact 137820
tggcgcagct ccagcagctg cagcagatgc accagaagca aatgcagtgc gtgcttcagc 137880
cccaccacct tcctccgccc cctctgccgc ccccgccagt gatgccgggg ggcggctacg 137940
gagactggca gccgccaccg ccaccgatgc ccccgccacc cggccggcc ctcagctatc 138000
agaagcagca gcagtacaaa caccagatgc tccaccacca acgagacggg cctcctggtt 138060
tggttccaat ggagctggaa tcccccccctg aatctccccc tgtgccgcct gggtcctata 138120
tgccccccatc tcagtcttac atgccccccac ctcagccgcc accctcttac tacccccccga 138180
cctcatctca gccctacctg cctcctgctc agccgtcccc ttcgcagtcc ccaccttccc 138240
aatcctacct ggcgcccacc ccttcttact catcctcctc ctcttcctcg caatcctatt 138300
tgagccattc ccagtcctac ttgccctctt ctcaggcatc tccttcccgc ccctcccagg 138360
gccattctaa atcccaacta ctagctccac caccaccgtc cgccccccct ggaaataaga 138420
caactgtcca gcaagagcct ttggagagtg gggccaaaaa caagagtact gaacagcagc 138480
aagccgcccc tgagccagat ccctctacga tgactccaca ggtaagaaag catctgcctg 138540
aacctcatct ttcacctaga gggccctgaa tgagcaggcc tcagggcttt aaccagtggt 138600
tagtctaatt ccaacacaca atgactagct aacactccaa aggattccgt aagagtcaga 138660
gggctgacag ctcagctttt taattagcta gatgtgaatt accaaaaaaa cagcgtttgt 138720
agatgtgtat ggattacata cagtggtggg gtggctcaaa agttacctct cagatacatt 138780
ccttggtttc tttgaccatc ctcttagatt ttgttgctgt tgcaaataga ttttgttgca 138840
gagggtcttg ttttaaaatt gtattttaaa cgaaagtttc catttgagag aaagcatgaa 138900
aaagaggcct cgccaagggc tgacacttga atgtttgtaa actttatgat tagttagctt 138960
cttttaaaaa tattacttag gggccaggga tagttgtcag cttcctgaat taagaggttt 139020
cagaaaaagg atggaaatct tgtcttacca ggagcatcct aattatgctt ataggtacat 139080
atacaataca tagatgagtc ccgagaccct tttggggatc gattggtcca ttttaggaat 139140
tgacccagac atccttttct catattattt aaagattgtt ttaagataat gagcaatttt 139200
ctgcgtttca ttgtcattac cattcactcc aaagaaacag ccttgcaatt tggccaacgt 139260
aaaaaaagtt tggtgatatc ttttcatgag agagtcagcc attcttatcc cgcagaacag 139320
acaaattgac agggtaacag taataaacga gttccccctt gagagaggga aggtcaaact 139380
aaagcagaat aaatcaattg ttatgatttc ctaaagtaga cactttttca gcgattacta 139440
atttacctta gattcactaa ggaaaaaaga gaatcttgta atataaacgc tcagatttac 139500
ctataaaaga tttccaaaag atagggatgc agtattataa ttttacggtg gtttggcatt 139560
agaaattaag aacagaagca agttgtttta agaaatgatg cttggcacat cttctggaga 139620
aaacacttgg gatgctgaaa taaagtagta gaataatgat tctgttggct tacatcaaat 139680
ttgtaccatc tcaccagttt attttcccca gattgctcta ttatagagac agactttctg 139740
tcctctttcc caccccactc tgaagtcttt agtgcttttg ttgtcaaaca ttctcagtta 139800
gtctgagcca aaataggaca tttatattct cactctacta gactgttttt ctgaccttac 139860
agagagacaa aataattgta tttttggctt tttttgggag gagtgtgtgt gtcagatcag 139920
gccttaatat gtatagttac catgaacagt gcttttactt ttctgccttt gtaccctttt 139980
ctgtaatgaa ctcagtgtct tgaatattca gtgcctgcaa ttctttggtg agaggcagag 140040
tggtggaaaa tgagacttgt ggtgcacata gaccagatca agaagagcct tgaagcctgt 140100
ggctttcttt cttcttttta gaattcatcc taacctcctc aaccctctgt ttccccttag 140160
agttgtattg ctttcaaggc atatctcaaa tatcaaggtt tccatgaagc cctgcttgtg 140220
ctcacccatc tggtttggtt ctgttcttca gattgtaagt cccttgcaga caggaattat 140280
cttacttatc ttcttattac cgtagcacct aggacagtgc cttgcaaatt gtaggctctc 140340
cacagatttg ttgaataaaa tacgagagtt gtcatgttct agacttagga tatgttggtt 140400
tattagatga taaatatttc ttcatgccag taccagtctc tggcaatcct tctacatatt 140460

tattattctt tgattcatat tttgtttttg gaatgtgtca ttcaaaattt cttatcgtga 140520
aatattttta aaatacacaa aatagagaaa aaagtatgat tccccataca tacccatcat 140580
tgagattcaa cagctatcaa gattttgcca tgtttacttc acctaggcct tttttattat 140640
tgttgcatat atatatattg agacagggtc ttgctctgtc agtcacccag ggtggagtgt 140700
agtggcatag tctcagttca ctgcaacctc tgcctcccag gctcaagcca tcccaagtag 140760
ctgggactac aggtgcatgc caccatgcct ggctattttt tttttttttt tttttggta 140820
gagacggggt tttgctatgt tgcccaggct ggtcttgaac tcctgagctc aaacagtaca 140880
ctgccttgac ctctcaaagt gctgggatta tgggcatgaa ccaccacacc tggcctctgc 140940
tgaaatattt taaagcaaat cttagatctt atgtcatttc atccctacat tcttagtcaa 141000
gtacctctat aaaacaaaac aaaacaaaac caagaccttt tctttttttt tttttttttt 141060
gagatggagt ctcacactgt tgcccaggct ggagtgcagt ggcgcgatct tggctcattg 141120
caacctctgc ctcccgggtt caagcgattc tcctgcctca gcctcctgag tagctgggat 141180
tacaagtgca tgccaccacg cctggctaat tttgtatttt ttagtagaga cggggtttca 141240
ccatgttggt caggctggtc ttgaactcct gacctcatga tccagctgcc ttggcctccc 141300
aaagtgctgg gattacaggc gtgagccacc gtgcccggcc aagacccttt cttagataaa 141360
cacattgtta acacctaaca aagttaacag tttcttggta tcatctaata cctgatccgt 141420
tcagatttcc tttattgttt catcatacta atgaactaaa aataattctt tgataccatc 141480
catgttaaaa taccccaaat tgactcaaca gtgttgttgt tttctgtcct cacagtctgt 141540
tcccaaacaa tgtttttttg gtgtgttcaa gtcagagtcc aaacagaata ctgaaaagat 141600
tcttagtata gatcagggtt tctcagcttc ggcactatgg acattttggg caggatgatt 141660
ctttgttgtg gaggggcttg tcctatgcat tgtaggatgt ttaacagcgt ccctgacctc 141720
tacccattag atgccagtgg cagcccccag tccgacaacc aaaaatgttt ccacacattg 141780
ccaagtatcc cctggagaca aaattaccct ctgttgagaa ccccttgacct aggtcagtcc 141840
ctcctatttt ctttttctac ctcatgtctt tgaacttgat gaaaaatctg ttgtcagttt 141900
tcctgtagaa tgtctgacat tctgcattgt ctgctttttc atggtgtcac atttgacttg 141960
ttcctatgtc ttccacattt tttttttttt tgaattagaa gttaactctg gaaccttcat 142020
ttaagttcag ctttcttttg gcaagtgttg cattcctcat gggacacccg atgcctggtt 142080
gtcccactgt tagtaatgcc attggtccct ctactgtaaa gctttctatc aatcttgcat 142140
ctaatgtttc agtctactgg ttcatcaatt gaagaaagac cttgcctaag taaattattt 142200
cattagacag aggttgcaac ctggtgattt tttttttctg ccatttcttc cacagttact 142260
atatggagtt atgtaaagaa aaacttttgc cacagatatt tgattggaaa tacagttggt 142320
ataggaagga caagataaat acttgatatt tttccaggtg ttttctagtt tttcgggtaa 142380
agtgttgatg atttaatgga atgtgttttt ttgtttgttt gtttctgaga gagagtctca 142440
ctctgtcgct caggctggag tgcagtggtg aaatctcggc tcactgcaac ttccacttcc 142500
tgggttcaaa caattctgcc ctagcctccc aagtatatgg gattacaggt gcatgctacc 142560
atgcctggct agtttttgta tttttaatag agatggggtt tcaccatgtt ggtcaggctg 142620
gtctcgaact cctgacctca agtgatccac ctgcttcggc ctcccaaagt gctgggatta 142680
tagacgtgag ccactgcgcc cggtcggaat gtgtcttttt gaataattaa tttttctatg 142740
tgtttaaagg actgatttat ttagttaaag ctgttggctt tgattagttt ttagaggtat 142800
catttattta atgtttaaat acttacggac tagcaactat gtgccaggta ctatgagtac 142860
tgtgcttaat actggagatg aaatggttaa caagatagac atgggacctg ccctcattga 142920
cttcttgttt cagttgtggg ggaagggaga agacatttag taagtaatta agaggagacg 142980
tacagagtgc tatgggatta taaaatatca ctagattatt ccccaaggaa ctggcaaagg 143040
caagtttttt gtttttttgtt tttttttaa gttaccactg tattcacagt accttgcata 143100
ggatctagcg cataggaggt ggagaacaaa tatattttaa ataattgaat atagctggga 143160
gcgctaacct aattaaagag gattcccttt gtaaagtgat attttttatt ttttatttgt 143220
ttttatttta ttttatttat ttttattttt ttgagatgga gtctcgctct gttgcccagg 143280
ctggagtgca gtggcgcgat ctcggctcac tgcaacctcc gccttccggg tttaagcaat 143340
tttctgcctc agcctcccta gtaactggga ttacaggtgc ctgccaccac gcccggctaa 143400
tttttttgta tttttatctt tttttttttt tttttttttt tttttttgga gacgaagtct 143460
cactcttgtt gcccaggctg gggtgcaatg gcacgatctc ggctcactgc aacctctgcc 143520
tcccgggttc aaacaattct cctgcctcag cctgcctcag ctactcccag gagtagctgg 143580
gattacaggc gcctgccacc acgcttggct aatttttgta tttttagtag agacgaggtt 143640
tcaccatgtt ggctaggctg gtctcgaact cctgacccca ggtgatctgc ctgtctcagc 143700
ctcccaaagt gctggggtta caggtgtgag tcagagtgcc cggccatttt tttttgtatt 143760
tttagtacag aagaggtttc accatcttgg ccaggctggt cttgaactcc tgacctcgtg 143820
atccacctgc tacggcctcc caaagtgctg ggattacagg cgtaagctgc cacgccgggc 143880
ctgtaaagtg atatttaaat gaactctaaa gaataagtag gaggactggg tgctcacacc 143940
tgtaatccca gttctttggg ctcattagag gccgggagtt caaggccagc ctgggcaaca 144000
aagtgagaca cccccccccc cgccccccgc ccacaaaaag tttaaaaaac tagccaggtg 144060
gccgggcgca gtggctcacg cctgtaatcc cagcactttg ggaggcctag gcgggcagat 144120
cacgaggtca gaagatcaag accatcctgg ctaacatggt gaaaccccat ctctactaaa 144180
aatacaaaaa attagccggg cgtggtggtg ggcgcctgta gtcccaactt ctcaggaagc 144240
tgaggcagga gaatggtgtg aacccgggag gcggagcttg cagtgagccg agatcgcgcc 144300
actgcactcc agcctgggcg acagagtgag actccgactc aaaaaaaaaa aaaacctagc 144360
caggcatggt gttgcatgcc tgtagtgccg gctacttgcg ggtggctgag gtgggaggat 144420
tgtttaagcc caggagttct cgtgccactg cactctagcc tgggcaacag agtgagacct 144480
aaaaaaaaaa aaaatgagta ggccaggtgc agtggctgat gcctgtaatc tcagcacttt 144540
gggaggccaa ggcaggcgga tcacttgaag tcaggagttc aagaccagcc tggccaacat 144600

ggtgaaattc tgtctctact acaaatacaa aaaattagct gggtgtggtg gtgcacgctt 144660
ttaatctcag ctacttggga ggctgaggtg ggaggatcac ttgaacccag gaggcagagg 144720
ttgcagtgag ccaagattgc cccactgctc tctagcctgg gcgacagagt gggactccat 144780
ctcagttaaa aaaaaagaaa aagagtagaa attaaccaga agttagaatt caaagaatta 144840
agttcaataa ggctcactga aagctcaggg tgaggatggt aggtatgaac tagatcaggg 144900
aaggcctgta aggacttcat tctaagggca gttggaagcc attgaaggat tttacatagg 144960
tgaatgtgac agattggcat ttttgaaagc tcactctgct tgcagtgtgg agagcagaat 145020
taaaaggagc aagagtgatt tagagagaat agttattgaa gtaatccagg caagagataa 145080
tagtggttta gactttgatg gtattagttg agatggagag aagtgggaag agtggagaaa 145140
tatttagctg ctagaattca ctttgaggtt gagtagttat ggagagggag gcatcaagaa 145200
tgattcttag gtttctgggc tgggccgtag ggtggatggt ggtcaaattt actaaaacag 145260
aaaaacctaa aggaggagga ggaagaacat ttctgggggt gaggagaaaa tcatgagtta 145320
ggtttcagac atactgagtt tggggttctt gagacataga ttccaaatgg agatgtccac 145380
ttggaagtta gatatagctc tggagtctag atgtaagatc tgggagacat agatttataa 145440
gtcatcagca taaaaatggt atttgaagac acagaaatag atgaagcctc tcaaggaatg 145500
tgtgtagagt aagaactgag gttaaaggac attaagccta gctccacaaa tagcagtatc 145560
taagggaacc aatgaaagaa tggccaaata ggtattcata ttaggttggt gcaaaagtaa 145620
ttgcagtttt tgctattact tttaatggca aaaccacagt tactttggca ccagtgtgat 145680
aggtggaaaa tcaagatagt ggatagtgcg ttgccatgaa cgtcaaggaa cagactctag 145740
gaggagggag tcgtcagctg tgtgaagtac tgctgagacg tcaagcaaga taaagactgc 145800
gagttatctt ttgcacctac tgacatgtag attattgttg accttggcag gagcagtttc 145860
atagtgactg aagccatatt gcattgattt gaggagtgat ggaaggtgag gaaatgggga 145920
tggtgagtac agacaactct ttcaagaagt ttgactgaag gagagagaga atgagggaag 145980
gagctggagg aatagatgaa gtaaatgagc atttaaaata aaaataggtg gggactaagc 146040
ttatctggtg accctgattc ttacagtaga atgtgaatct caaaaatatt taagcctatt 146100
ttaaataaat ttctaaaatg gaaacccaat tcattatttt accaggtgtc ctccctgccc 146160
cctgctttag aatataagac atagaacatt gtcttatagg catttatgat aacctggata 146220
gggaaggtaa tcttctaaaa ttaatcttta aaacattaca cacaaacact cacctccttt 146280
atagttctcc acttggaaaa tcatttagta tctagttcat tactagcagc agtggtcagt 146340
aagtgcttcc tgagtaaatt aaaatcaaca tctttctttt tttttttttt ttctcagaga 146400
gatgggatct tgccatgttg cccaggctgg tctgaattcc tgggctcaag cgatattcgt 146460
gcctcggcag catctttctt aataacagta tttgtgacaa atagttctat attcttaatt 146520
tttttttttt ttttgagatg gagtctcgct ctcacccagg ctgaagtgca gtggcgcgag 146580
gatctgggct cactgcaacc tccgcctccc aggttcaagc gattctcctg cctcagcctc 146640
ccgagtagct gggattacag gcatgtgcca ccacacccag ctaagttttg tatttttagt 146700
agagacaggg tttcaccatg ttggtcaggc tggtctcgaa ctcctcacct tgtgatccgc 146760
ccgccttggc ctcccaaagt gctgggatta cagatgtgag ccaccgcacc cagccaatag 146820
ttctacattc tttatgtttg aaatcccttc attcaatctc gttttaaatg tgacctatat 146880
tatgatttcc ctctacctat aaaacaacat cccatatgaa atggagttat ctgatatgat 146940
gggacattca gaagagctgg catcattagc aagttcaggc attttttttc tttctaaaat 147000
agggatgatc acacttctct tagggtcagt aaggacattt gtgagtgata tcattattat 147060
ttatttgcag agcttcaaaa atgtgctgaa gcctaccata ggattgaatt agatctggtt 147120
actacttata gggttcatat taaatagaaa ttgacctaca aggcggccgg gcacggtggc 147180
tcacgcctgt aatcccagca ctttgggagg ctgaggtggg cggatcacaa ggtcaggaga 147240
tcgagaccat cctggctaac atggtgaaac cccgtctcta ctaaaaaata taaaaaatta 147300
gccgggcgtg gtggcgggcg cctgtagtcc caactactca ggaggctgag gcaggagaat 147360
ggcgtgaact cgggaggcgg agcttgcagt gagccgagat cgcgccactg cgctccagcc 147420
tgggcgacag agcgagactc catctcaaga aaaaaaaaag agaaattgac ctacaaggca 147480
tactgtcagt gaatcaacaa gtacttcagc ccttaacaag ttcttaaccc tggtctggat 147540
ctaagacaaa gcatgagaat aggtgatgaa gaagctgata aagaatctgg acctgtacag 147600
ttttcattct gtacagtctt catatctgta gcattggctt agtttgtgaa agatagtttg 147660
catctttgta tcttttaata atgctttcaa agcaacttta aaaaaggaaa cttttaactt 147720
ctacttgaaa gagaatgtac taattttttt aaaactgaag gataagtctg ttgatgtttt 147780
cagtattaaa ataatggaag gaagtgtttt aatgctacat tattattatt attggctttg 147840
gctttcatgg tcagtgtgct gttttctttc tttttttttt tttttttttt ttttttgag 147900
acagagtctt tctctgttgc ccaggctgga gtgcaatggc acgatctcgg ctcactgcaa 147960
cctccgcctc ccaggttcag gggattctcc tgcctcaccc tccggagtag ctgagattat 148020
aggtgtgtgc caccacaccc tactaatttt tgtattttta gtagaggcga ggtttcaccg 148080
tgttggccag gctggtctcg aactcctgac ctcaggtgac ctgcccgcct agacctccca 148140
aagtactggg attacaggca tgagccactg tgcctggcca agtgtgctgt tttcttacgt 148200
tgaatagtaa aagtacagat actctttgac ttacagtggg gttacatctc aataaaccca 148260
ttgtaagttg aggataatgt aagttgaaca tttaatacac ctagcctact gaataccata 148320
gtttagccta gctattaaat gtgctcagaa agtttacatt aggcaaagtc atctggccac 148380
acagtacaca gtagagtatt ggtttttttc ttgttttgtt ttgttttctt tgagatggag 148440
tcagtgagtg gtgcgatctt ggctcactgc aacctccacc tcctgggttc aagcgagtct 148500
cccgtctcag cctcccaagt agctgggatt acaggcacct gcgaccatgg cccagctaat 148560
ttttttgtat tttttttgtt ttgagacgga gtctcgctct gtcgcccagg ctggagtgca 148620
gtggcgtgat ctcggctcac tgcaacctcc gcctcctggg ttcatgccat tctcctgcct 148680
cagcctccca agtagctggg actacaggtg cctgccacca tgccaggtta atttttttat 148740

atttttagga gagatggggt ttcaccgtgt tagccaggat ggtcttgatc tcctgacctc 148800
atgatctacc tgcctcggcc tcccagagtg ctgggattac aggcgtgagc cactgcgctc 148860
cgcctttttt tttttgtatt tttgtagaga cggggtttca ccatgttggc caggctggtc 148920
ttgaactcct gaccttaggt gatcagccca ccttggcctc ccaaagtgct gggattacag 148980
gtgtgagcca ctgcacctgg ccgaggattg attgtttgcc ctcatgatgt gtggctgact 149040
aggagcctag ggctcgctgc tgctgtccag catcacaaga gagtatcata ctgaatattt 149100
tgagcctggg aaaagatcaa aattcatggt atggtttcca gtgaatacat atcactttca 149160
caccaccata aagtcgaaaa atcataagtt gaaccattgt taagtcaggg accatctgta 149220
attatatcct gggagctgat gattatatgt ataatagtta aaaacaaaaa atccagtcat 149280
gggttaaacg tcaagatgga gtttatgaga tggaatttat tcttcccaat agattggaac 149340
aagtttcagg taatagtaat gagtgaccga gagggaaggg cttcagttga tcaataacca 149400
tttattgaca tcctactgtg tgctctagca ctatgcttga ctaccagata ggatataaaa 149460
agaggggaag gcagctgtcc cctaagaaaa attacattct ttgagtgaaa agtaggtaac 149520
aaatcagtat gtttggtgag aaaaaggtat gtattatgta tatttaatgc atctgtttgc 149580
atatggaaaa ttaggggagg ttatactata aaatattaac agtgtaggaa atcctagtta 149640
tccagagata attggattag aaaataggca agtaaagtga attcatttaa cacaggaacc 149700
aaaagtttat tgtaaaacat aaaacatttg attgggaccc agtctggaaa aacaaagctc 149760
aaagtgataa ggataagcag attttatatt tgatggaatt gttataaatg aaatcatagc 149820
caagttttag aatgaacaaa gaataaacta tagattgggt ttctttgctg taatttttgt 149880
gggaaaatta tataacaaca ttatagatca tcttttattt catttttga catgttgaaa 149940
aaatatttta gggatttatc gatttttata cggactgttt ttcaaccttt ggtattcagg 150000
ccattctctt ttatagtttt tactgtaaag tactgttcgg ttgttaactg gtaggacttc 150060
tagcagatcc tcagctgcca gtaataagtt ccaatgttct ccaacatcac tttcttgaaa 150120
tactacatcc atacaattgt agtagaatca tatgcttttg ttagtcatat aagtatacag 150180
ggcagttcat cagtgaatat tttagtgtta tgataacttt tggttttctt tatgcaacct 150240
tgtaaccatg ggaaattata taatactagg ataatgacac tcctgtagtt tttttctgag 150300
tggtgagatt tcttttattg attttaatta attagttatt acttacctgt gtagtcagtt 150360
ttctttattt gagattttta aactcaccac agacactgaa ttagtgaata ctgaaccagt 150420
gctcctcggg gaaatacagg gttaggttcc tgtgagtctc ttgtcacatt tttatcaact 150480
gatgaacaca tagccttgtt tatgtgtgtt tctgtttaaa gaaccttatt tagtacatat 150540
tgttgattta ttaacattgt actcatggcc aacagcactg tagcacttgt ccgaacaaag 150600
ctgacctaac acagatattt tctctgtgag acataagacc gctttcttgc tcttgggaca 150660
ctaaacagca tctcagctct atgcctgggg gccattttaa atagtgaaat caacaaaaaa 150720
gcacaaaagt ttgacaaatg tgtctctaaa tagactgaaa atgacactta tttatagtat 150780
agagctggaa caagaaggca gagcatcacc ttgcacttca tctcggctgg gaatgcacac 150840
atgagtcagg ggactcatgt ttttcactgc tgtccacatg tttgtgaatg actgggaaag 150900
ctcagtgagt atggattttg ggtttagaaa taaattttag gtcgggtgtg gtggctcatg 150960
cctgtaatcc cagcactttg ggaggccaag gtggatcact tgagtccagg agtttgaaac 151020
cagcctgggc aacatggtga aacttgtctc tacaaaaaat acaaaaatta tccagatggt 151080
ggcacatacc tgtggtccca gttactcggg aggttgaagc gggaggatca tttgagcaca 151140
ggaggcggag gtggcagtga gctgaaactg caccactaca ctccagcctg ggtgacagag 151200
ggagatgacc ctgtctcaaa aaaagaaaaa aaaattttag caagtaggca aatttgcaaa 151260
tacagaatcc aagaataatg aggattggct atatttctca atatttccat actaagctta 151320
ttgttgtaat ttttaaatga aagcacaaaa taagctttca gaaaattaaa gtacagcata 151380
atttggaaga taaagctaag gcttttaaaa taaatagaaa gcattttaaa acacagtata 151440
ttggccaggt gcggtggctc acacctgtaa tcccagcact ttgggaggcc gaggctcgtg 151500
gatcacctga ggtcaggagt tcgagaccag cctggccaac atggtgaaac cccatctcta 151560
ctaaaaataa aaaaatttag ctgggcttgg tggtgggtgc ctgtaatccc agctactcgg 151620
gaagctgagg caggagaatc acttgaacct gggaggtgga ggttgcagtg agccgagatc 151680
acaccactgc actccagcct gggtgacaga gcaagactcc atctcaaaaa aaaaaaaata 151740
aataaaacac agcatattct cttatatgtg tgtacagcat attctcttat atgtgtgtac 151800
atctgagtat gggtagacta atgctacaac aggtgtttca aagtgggagg aggctgggtg 151860
cagtggttca cacctgtaat cccagcactt tgggaggctg aggtaagaga attgcttgag 151920
cccaggagtt tgagaccagc ctgggcaaca cagtaagacc ttgtgtctat aaaacataaa 151980
aaaattagct gggcgtgatg gtgcatgcct acgaacccag ctgctcagga ggctgaggtg 152040
ggaggattgc ttgagcctgg gaggttgagg cttcagtgag ccatgttcag gccattgtac 152100
tccatcctgg gtgagagagt gagatgcagt ctcaaaaaaa aaagaaagaa aagagggagg 152160
agtgcttgag gagaaagaga gaaggcagag aagagaggta ggactgttca gttatattca 152220
ttaattacaa gacccctgtc aataaatcgg aacaaactga agccaggtta aaaataagta 152280
gttgtgaata ctgaagagat ttaccctgat catttatcaa aaagatggct cttacctcca 152340
aaagcatgtg ttttattaga gtacagaagc atgttgaatt aatatgtatg gtttaattta 152400
aatgtataaa tttgggggag gaactgaaat ttttctgtag cctgtgttta agatcttgtt 152460
aacttatttg tcttcctcag tctagctttg ccttttttta catatagaca catgaacaca 152520
gacataaaga taggtccttg tcaacaccaa gttgcagaga tacatgattg tcaatcaaaa 152580
ttctcaaaaa ttgtttctgt tgtaggaaca gcagcagtat tggtatcgac agcacttgct 152640
tagtttgcaa cagaggacaa aagttcattt gccaggacac aaaaagggtc ctgtggtagc 152700
aaaggataca ccagagccgg taaaagaaga agttacagta cctgccacca gtcaagttcc 152760
agaatctcct tcttctgagg agcccccatt gccacctcca aatgaggaag tgccacctcc 152820
tctcccacct gaggaacccc aggtaaccat ataattaatt gtttgtgttt attaaattat 152880

ttagcttttt tctgattcat ttagtatact tttatcataa atggatatat atttttgttt 152940
ttttaggtac ctataaagtt gcatttgtga aatcagatgt ctgttgtatc aggtttattt 153000
gtttgtttat ttattttgag acagggtctc tctctgttta ccaggctgga gtgcaatggt 153060
gcgatcttgg cttactgcaa cttctgcctc ccaggttcag gcaattcttc tgcctcagcc 153120
tcctgagtag ctgggatgac aggcatgcac caccatgacc ggctaatttt tgtatttttt 153180
agtagagatg gggtttcacc atgttggcca ggctggtttc gaactcctga cctcaagtga 153240
tccacctgct tcggcctccc aaagtgcttg gattacaggc atgagccacc atgcctggcc 153300
tgttgtatca ggtttagaaa aggaaatttt tcctagtttg agtgaagagt tggatactct 153360
tctgaattga gtgcctctaa gcactaaagg aagatgccag catatgttca tagtgacagc 153420
acagggcaat agttgagtgc ttgagctttt agagtcaagc tacccaggct ataatccagg 153480
tactgccact tgccagtagt gactgggcaa atttgttaac cattctgtgc ttcaatttac 153540
tcctctgcaa aatgggtata ttcaatagta gctatcatat agaggtattc aagcattaaa 153600
tgtgataatt cttgtcactc agcttagtga ctggcacatg ggaggttctc atattggtcc 153660
aaaggtcatt gttctcccaa gagccttata tgaagggttt aatcaaacca tgatggttat 153720
attgtagtcc attggtgaaa agtgacaaaa aaagaaacgg atttttcata gagtgaaaag 153780
gtctataact taaggttcac tttttttttt tttttttcca gacagggtct cactctgttg 153840
cccaggctgg agtgcagtgg tgtgatctca actcactgca gcctcagcct cccaggctca 153900
agtgatcctc cttgcctcag ccttccaagt agctgggacc tctcttccct cccctcccct 153960
cccctccttt tttttttttt gagatggagt cttgctctgt cgcccaggct ggagtgcagt 154020
gacgcgatct tggctcactg caacctctgc ctccaaggtt caagtgattc tccggcctca 154080
gcctcccaag taggtgagat tacaggtacc tgccaccatg cctggctaat ttttgtattt 154140
ttagtagaga cggggtttca ccatgttgac caggctggtc ttggactcct gacttcaagt 154200
gatccacccg ccttggcctc ccaaagttct gggattacag gtgtgagcca tggtgcccgg 154260
cctgggacca ctttccactg ggaaaattgt tgtcatattt aggaataata agattattat 154320
tccagtgaag actatggaat ttataaactc ctttgtatta gtatccataa tagtatagat 154380
agagctatgt gttttatcta aagatagaat agctgttctc aatccaaaaa gaactgaaga 154440
tactgtgttt gacagttgga tatttctgag ttgtagggat tctggacttt ataatacatt 154500
tataaaatat ttttgcttgc tgttttatga cttacataaa gatgtgtcct cttttatctt 154560
tagtctgagg acccagaaga agatgccagg ttaaagcagt tgcaggctgc agcagcacac 154620
tggcagcagc accagcagca tcgagtcggt ttccagtatc agggaataat gcagaagcac 154680
actcagttac agcagattct acaacagtat cagcagatta tacagccccc accacatata 154740
caggcaagtg cttccatagt ccacaatagg aagttgcccc aagacattga aaggatttgt 154800
tattcttgaa agatttaagt gtaaaaaaca aaagatacca actgttgaca attagttgtt 154860
tcccaagggg tgcccaagta gttatctgcc acattacctg ggggactggg gaggggttgg 154920
ttatagtaga gtttcctgat catgtcaacc caggccagat gttagcttac tattgctaag 154980
ggtcttaaga gctggggatt tttaccatat agctacaggt taacctgtgt tttgcctgta 155040
aactacaaca gtaacagata agcaaagagg ccaaaaacag ccatcacaaa aaacctaagc 155100
aaatataccg taaaagataa agtgacttag atacttgttt gtgttgtatc tttgaggggt 155160
aatgttgtgt ctgtatatct ataataacag atcatttttc aagttcttga aattatccat 155220
attaaaaact tgaaaaatac ttattgctaa gataggtatt tgctttattg tttggttggt 155280
ttttccaact ctgtagtaag ttgtctaagc caaaggaagg aagaagaatg atcatcactc 155340
aaagaactta acgaacttat tcttagcctc ccaaagtctt tagtgttcta atttcctaaa 155400
gcgtcaaatg cccttgattc attaatattt taacctatga ttaattatct tatatgaatg 155460
gttttaaata gtttttatcc ctttatttgt agaccatgtc tgtagatatg cagctgcggc 155520
attatgagat gcagcagcaa cagtttcaac atctttacca agaatgggag cgagagtttc 155580
agctatggga ggaacaactc cattcctatc ctcataaaga tcagcttcag gagtatgaga 155640
agcagtggaa aacatggcag ggacatatga aagccactca gagctatctc caggagaaag 155700
tcaattcatt tcagaacatg aagaaccagt atatggggaa catgtcaatg ccacctcctt 155760
ttgttccata ttctcagatg cctccacctc tacctacaat gcccccctcca gtgttgcctc 155820
cttcattgcc accaccagtg atgcccccctg ccctccctgc tacagtgcca ccacctggca 155880
tgcccccacc tgttatgcca ccttctctac caacctctgt tcccccacca gggatgcctc 155940
cttctctctc ttctgcaggg ccaccaccag ttctccccc accttccctg tcttctgcag 156000
ggccaccacc agttcttccc ccaccatctc tctcttcaac agcacctcca cctgtcatgc 156060
ccctcccacc attgtcttca gctacacctc ctccaggaat acctccccct ggagttccac 156120
aagggatacc tcctcagtta acagcagccc cagttccacc agcctccagt tcacagagct 156180
cgcaagttcc agagaaacct agaccagcac tgcttcctac tcctgtgtct tttggttctg 156240
ccccaccgac aacttaccat cctccgttgc aatcagctgg tccatcagaa caagtgaatt 156300
caaaagctcc tttgagcaag tctgctctgc catacagttc attctcatct gatcaaggac 156360
ttggggagtc ttcagctgct ccatctcagc caatcactgc agtgaaggac atgccagtga 156420
gatcaggtgg cctgcttcca gatcctccta gaagtagtta cttggaaagt ccaagaggcc 156480
caaggtaggt ctgctttttt ttctcttttt ttttggtttg gctattttat tgacttaggc 156540
tatttggttc tcgatggtat aaaaagcttc atttatacaa tgtttatact ctctttgtac 156600
ttatattgtc tgcttgttaa gacatcacgt atgtaatcta gaaaaggtaa catgccccca 156660
tttctttgat gtctctgctt ttgtcctata tgtagagatt ataatccaga atgaatgtag 156720
tattgtagag taatgctaat catttgtat tgacttatta gctttaaaaa taatattaaa 156780
ctttatcttt tgtttgcttt ttcattcatt tgacaaataa ttgaattggc aattactatt 156840
tgttagataa tttgttagtt gttagagata aagatgaata agccaatatt ctactggctt 156900
gaggcagtga aatttagcat aaggtaagta ggacttttat tttatccatt cagcaaatat 156960
ttaagttcct gctcttgtac ttagagcagt gaatatagca gaacacataa aattcctgcc 157020

cccatggagc ttacatcctt tatattttcc ccagttaagg agatacttct gtttatcctg 157080
aacaacatat ttagaaagaa aaaaaattgc tggtgtgtaa gtttttttctc tttaccttct 157140
aaaaactgtt aaattcattt tttccaagtt tgtaaaaata gatttttctc cccaagttga 157200
cctaaatgat aacttttcta tggaaattat tattattttt tgagacaggg tctcggtttt 157260
tcacccaggc tagtgtgcag tgatagaatt atggctcagt gcagcctctt cctcctgggt 157320
tcaagcagtc ctcccacctc agcctcccag gtggctggga ccagaggtgc gcaccaacac 157380
atctggccag tttttaaaaa atacttcgta gagatgggga tcttcctatg ttgaccaggc 157440
tggtctcaaa ctcctgggct caagcgatcc tcctgccttg gcctccctgc ctgccgtggc 157500
ctcccaaagt gctgggatta caggtgtgag ccaccacgct cagccagaaa aatactttaa 157560
tcagtttttt tctcagttgg gaattgtagt agaaatgggt actccttata caaaattcaa 157620
acattacaga aaagtgtaga aaaaagtgaa agtcacctaa ttccaccatc tggagagaat 157680
cattttaaaa atatatgttg cagaaatggg atcatactga gtagattatc ttataactta 157740
tttttccacc aatctgcgta ttgcttagat tttcatctca gtatcagtgg atctacatca 157800
tcattttttt caacctcatt tttaacatttg aaaacaaaat atatctttta ttccccaagc 157860
ttataattct gaaagcagtg tggcacaaaa ctccatccac aatcaaagtt aagagaaagg 157920
gcaattaaca tgaattcctc tgtgtaccaa gaagccttgc tagagacttt acacacatta 157980
ttatctcatg gaagtcttct caacaacctg tgaagtaggc agattattct ccatttactc 158040
ttaacaaaat ggcactgaga ggactgcgta acttgctcag tcaggattat ttagcagagt 158100
gagtagaacc ccagaatagg tacagttgag tctagagggt ccccaaagca aaagtactat 158160
ttaagctctg tttactgcca tgattcaagc ctatactgtc agtgcttatt tggcatgtgt 158220
atcatcttga tttgaattcg ttcttaatct cctggaatga gcattgcctt ttagatctgg 158280
taactaattt gctatcagag aagtacattc ttaacacctc atgaaacata catttcctgc 158340
tcccttgatt tccgtcatgt taatattttc tctctttgcc tactaaatct tggctgtgtg 158400
aatttaggac ctattttaag ttttgtcttc agaagaaaat aacttctcaa gttaataatc 158460
tctgtgctgg gaggatatta gcaattatct aatatatctg tctacggcaa cgcacgtttg 158520
tattccattt cagtttgtca caatgagtct tgcacatatc atcttattta ttcctcttag 158580
tgatatcagg aggtaaataa atacataaga aaagctgaga ttcagagagg ttgtaaatac 158640
cttgcctcat ataacacagc tagtaaatgg tcaagatgag attagaatcc aaggacttct 158700
gacccacttt aagtccattg tactttctgt gatgagcagt tgtatgctta acattggcat 158760
tgtgattagg aacactgcca tataaggcat tgattctaga aagtttgttt tttacattga 158820
acctaaatgt gattctttaa cttttatctg ttgattctag atttgcctct ggagcaacac 158880
agaatgaatt atcctatgcc tcttctacag catgtcacta cagttattta aaaactatta 158940
ccctcatgtc tccctcaccc tacctcaccc agtttgctct gccaatcaca actcagattc 159000
ttttcatctt aacattgttt ctaacctcat cctgctgtac tgctcactgt cctttgaagg 159060
gcctttagtt tgttagtctc cttcagaatg aacacttact tcagatgcag aggagcatgg 159120
gcctagtacc aacttcatta taaataatgt gcttatattg tatagcttaa caaattgctt 159180
ttttgtttgt ttttttcagc actctcacag tgttgactct tgttgaactt aacatcaatc 159240
tagcttccca ggtcttttc acattagctt ttattaagcc aagtctctct tatcctgaac 159300
ttgtgtggtt ggttttttgt ttttgttttt cttgttcctt tttctaagac tttacatttc 159360
tctttagatt gttttctagt taggtttgca ccatcatttc agtctgctga aatcttggtc 159420
aatgatgatt atattgtttt aggtatgtga tatccctctt cagcattttc atctgcagct 159480
ttgataagga tggcttttta atccttgaat tggatcctac tcacattatc caccttgttt 159540
tgaaaacgtt tcccctaact agcttatgtc attgccattt cagtcacccc ctgctgcttt 159600
tgctcactca gcccttatag agtatagttt gggactgtta cccaggcata gttctttttc 159660
tccagtgtaa ctaggtaaaa aatcagtggg aagctggtta gtgagtgaac aagactgagg 159720
taagaataat ctatactatt aaggaaacct gtgaacttct tgtatttcta attcagattt 159780
tgctatcaaa catataactg atatgagaag ccactttttt taaataatca aagctgatta 159840
gaaaaacaag agtttagaac acaactaaac aaatttactg ataacatgct gttacaaata 159900
aagtactgta aatggctttg agtttttgca aaatccagat ttaagctgct ttataaaaac 159960
gagatttcta aaaagtcatg tgacaaaact gcatgacatg taaataacat atcacattgt 160020
tgagagtcta ggggagctgt tttgacactg tgtatatcat tggattcact cagtcttcca 160080
gaagactacc gttggtccag aaaactgtca tatcaatgga tgttggtttt ccaggacctt 160140
ttttttcaca tataataaaa cttattgaaa cgtaacagag aagtgcacaa ttttaagtgt 160200
tcagcttagt gaattttcac aaaataaaga cacacattgg ccaggtgcgg ttgctcatgc 160260
ctgtaatccc agcactttgg gaggctgagg ggggagcgga tcatgaggtt aagagttcga 160320
gaccagcctg gtcaatatgg tgaaaccccca tctctactaa aaaatactaa aattagctgg 160380
catggtaaca tgcgcctgta gtcccagcta cttgggaggc tgaggcagga gaattgcttg 160440
aacccgggag gcggaggttg cagtgagcca agatcgtgcc actctactcc atcctgggtg 160500
acagagtgag actccgtctc aaaaaaaaaa aaaaaaaaaa aggcacacat ggtatcacta 160560
ctagatcaag aaataggaat tactggtgcc caagaaatct ccctcatccc cctcagttat 160620
tttctaatac cccaaagata actgctatac tgagccatag attagttttg cctatttttg 160680
aacttcatgt gagtgaaata atactgtatg tactcatgag tctggcatct ttcacttgac 160740
attgtattat tgtgagagtc atccatgttg tgtgtagtag tagtttattc ccgttcctgt 160800
acaatagttc attgtatgaa tataacacaa tctgtttatc cattttaata ttaatggtca 160860
ttgaagtttt ttggttatta tgaaaagtgc tgctgtgtgt cttttgatgc tgtatgtgtc 160920
cctttagatg tatgtgtttg tttgtgtgtg tttaattttg tttggatatc tattaaggat 160980
tggaattgcc tgattataga gtatatacat gttctgcgtt aatgggtatt gttaaacatt 161040
tttccaaagt ggttgtttca gtttatattc tcaccagcag tgtttcagtg tttcagtttt 161100
tttgcatcct tatcaacact tggtattgtt aaacttttaa ggtttagcaa tcctagtgga 161160

tgtttagtgg tatcatgata atggatttaa tttacatttc cctgatgact aatgaggttt 161220
cacttttttt atttgattat tgttaatttg tatttcctct tttatgaaat gctgattcaa 161280
gtctttttcc catttttga ctaggtttta tgtcttactg attttgtagg agttctttgt 161340
gaattctaca catcagtttt ttatccatta tatgtattac aaatttctca ctgcaggact 161400
ttttattttc ttaatgagca cttgaatgta aacgctgcat gctgggtaca gtggctcatg 161460
cctgtaatcc cagcactttg ggaggctgac gcgggcagat cacttgaggt caggagtttg 161520
agaccagcgt ggccaacatg gtgaagcccc agctctacca aaaatacaaa aattagctag 161580
gcatggtggt gcacgcttgt aattccagct actcgggagg ccgaggcaca agaatcactt 161640
gaatgcagag gtggaggatg cagtgagccg agatcgagtc actacactcc agcctgggcg 161700
acagagtgac agagcgagaa cgagattccg tctcaaaaaa aaaaaagaaa ctaaacgctg 161760
cagtaagaca gtttgatctc agtatagtag aaaagtagtc tttaccgttt acctttttagg 161820
tccaatggac cctttgtatt ttaaagttta aagtaatttt acaaataatt tacagaattt 161880
aagaataaag gggccgggca tggtggctca catctgtaat ctcagtactt tgggaggcct 161940
aggcgagcgg atcacttgag gtcaggagtt tgagaccagc caggccaaca tgggaaaacc 162000
ctgtctttac taaaaataca aaaattagct gagcatggtt gtgcacacct gcaatctcag 162060
ctactcagga ggctgaggca tgagaattgc ttgaagctgg gtggcagagg ttgcagaact 162120
gcaccactgt actccagcct gggtgacgga gtgagagtct gtctcaaaaa taaaaataaa 162180
gggatagtga aaaaaatgtt aaagacaaac tcttttcatc cattaaattt gggcacacaa 162240
acatcttaaa agatgaaata attctgtcac ataccctttt ttttttctt ttttttgag 162300
atggagtctc gctctgtcgc ccaggctgga gtgcagtggc gcaatctcag ctcactacaa 162360
gctccacctc ccaggttcac gccattctcc tgcttcagcc tcccaagtag ctggaactac 162420
aggtgcccac cgccacgcct ggctaatttt ttgcattttt agtagagacg gggtttcacc 162480
gtgttagcca ggatggtctc gatctcctgg cctcgtgatc cacccgcctc ggcctctcaa 162540
agtgctggga ttacaggcgt gagccaccgc gcctggcctt ctgtcacata tccttttagc 162600
aaattgagat ggtccaggtt cttgccacaa aatatgtgat aagataaggt ccttcctgtt 162660
taataactaa gtgaattacc acaccacatt tcctttctgt tcttagaaat atattgtcag 162720
ctgggcatgg tggctcacac acctataatt ccagcccttt gcggggctga ggcaggagga 162780
ttgcttgagg ttaggaattt gagaccagct tgggcaacat agtgagaccc tgtctctaca 162840
aaaagtaaaa aattagccag gcttggtggc atgtgcctgt ggtcccagtt atgtgggagg 162900
ctgaggtggg aggatcgctt gagcctggga ggccaaggca gcagtgagct gtgcactgca 162960
ctccaacgta ggcaacatag tgagatcctg actcaaaaaa aaagaaagaa aagaaaccgt 163020
aaggctacaa gtattattat ttactttttc ttcttttatt tctttaacct tttattttta 163080
aatcaatact ccatataaaa ctgattttat atagcgtgag gtaggggtca atgtttattt 163140
tttccgaata actatccagt tcaccccaac accatttact gaagaagtca tccctactac 163200
tttgcagtga cttcatcata aattcaatgt ttaaatgagt ttgtttctgg actctccatt 163260
cctgtggtct atttgtctgt ctttgcatta gtgtcatatt ttaattactg tagctttaca 163320
ataaatttat ctgttagtgt aggtcctcca attctgtgct tctagataag ggattggcaa 163380
gctttctttt ttattttttc ttgagacgga gtctcagtct gtcacccagg ctggagtgca 163440
gtggcgctca ctgcaaccac tgcctcctgg gttcaagcga ttctcctgtc tcagcttccc 163500
aagtagctgg gattataggt gtgcaccacc atgcccggct aatttttgtg tttttagtac 163560
agatggggtt tcactgtttt ggccaggctg gtctcgaact cctgacctca ggtgatccat 163620
ctgccttggc ctccctgggt gctgggatta ccggtgtgag ccatcatccc tggcctggca 163680
agctttttat aaagggccag gtggtaaata tttgtaggcc ttgtgggtca catttggtct 163740
ctgttaaata tttttctttc ttttcttttt tttttttttt tcacatttaa aaaaaatttt 163800
attgaggcag ggtctttact ctgttgccca ggctagaatg cagtggcagg atcacaactc 163860
attacaactt tgacctcctg ggctcaagca gtcctcccac atcagcctcc catgtagctg 163920
gaactgtaga tgtgtcccac catgcttggc tacgttattt tttgtagaga tgggatctgc 163980
ctgtgttgtc caggctggtc tcaaactcct gggctcaggc catctgccca ccttggcctt 164040
ccaaagtgtt ggtattacag acatgagcca ccatgtgcag cctttttttt tttttaaacc 164100
ttcaagaatg taaaaaagat cacttttaac tgtatttgga acataggcca tagtttgctg 164160
acccctgttc tagattgtct tgattattct tattagaatc agcttgtcag ttttcaaaaa 164220
cctgctagaa ttttgatgag cattgcatag aatccattga ccatttggag gaagaattga 164280
aatctttata atatcaaggc ttctgatcag tcacaactat gttttgactg gctttaggat 164340
tggaattgtg gactttttcc attctatagc tatcttacta ggattagtaa gggactgtga 164400
gtgctatagg ttttctttat atttccattt gttatggttg ttcttgttta tagtgataac 164460
ttatctttgg gaattttaat tttaggacct tggttataca tatttagaaa acaggtgcta 164520
tttcacgtga aaagaaaaca ggaaggaatt taattccaaa atttggtatc ttgtaattcc 164580
catggtttgt taattttaat atgtttctgc aaatagtata atacataaaa taccttcttt 164640
aaaagtattt gtataactgc atcatacctc ttaggttttt acattttgga gttttatatc 164700
aaattttgaa atttttataa ttctaaggta tttataccta gttttagggg ttttttaaga 164760
gatttataac acacttaact atacaaataa acactttcta aatacttaaa accagatatt 164820
tgcttatttt tctttttatc ttaccaaggt acaatgcatg cgtttttatg tgattgtatt 164880
tagtgaattt atgtaatgtt tctactatat tttaaataca tgtttttata tgtcaaaagg 164940
gtcatagtat ttcattatat ttaaaggtgg cttattttca tacagatttt taatatgagt 165000
atctcttgat atatttgtta caaagcctta aaaggtctct agttttataa atgaaaacgg 165060
ttttgagtct gtcattttgt aacatctttg aactttgaaa atttgaaagt ccagtaatag 165120
acaggtgcag tggctcatgc ctgtaatcct agcgctgtgg gaggctgagg caggtggatt 165180
acttgagctc aggaatttga gaccagcctg ggcaacttgg tgaatccgca tctctaccaa 165240
aaatacaaaa attatccgag cctggtgaca taaaaaatta gccaggcatg gtggcaggca 165300

cctgtggtcc cagctactcg ggggactgag gtgggaggat cactggaacc caggaagtct 165360
aggctgcagt gagctaggat catgccactt cattccagcc tgggtgacag agtgagaccc 165420
tgtctcagaa aaggacaaaa aaaaatcaaa tagtgataaa tatttgccat ttatatgtag 165480
tgttgaaagt gtatttcaaa tggtttcaac aatttctggc ccccagaatg aaaataaaat 165540
gatgggcctt ggctgttgat cagcaatttg gtctgaaaac aaagcaccct accctttcaa 165600
cccttctgcc atgtcctctt atctgcacac cttttaatgc ctcctacttc ctttgtcctt 165660
tcttctggct tttacacatg ctcttttctt tgtctggaat tcccttcttg ccattctttg 165720
ctttatttat tcctattttt cctgccctgg ggcagcactt cagtaattac agattgggat 165780
tatttaatga aatcagacat tgggaatact ttggttagca agacaaacat gttccatatt 165840
tatcaaattc tgttctaatt gttgagttaa ttgtctcact cttcctgtgg acagtgaact 165900
ccttgaggtt aggcatgact ctctttttag caccagctca ttacctgaca atcagtatgt 165960
gttcagtgaa agttgcttct tccaaaatgt cagtcatgga taaaattctc aaggcattca 166020
caattttgtt aggaagggac tttgctcata tttagaattt gattagaata ctagataaca 166080
tgttaataag gtctctcatg gactcttgta cattgtggaa gatagtcttc ttttatcact 166140
tatgtgaaca aataattgct acatttaata catagcaaat gattttatac aactttaatg 166200
atctgatata ttattgaggc actttaaatt aataagtgta attccgagaa aggaaccaca 166260
ccttcacaag ccctaaagcg gcatactttt ttttccttct gtaaatatat actctccatc 166320
cttagcattg tgatgtatat actttgacaa gtttctggtc accttctttc gcctttgccc 166380
attatagctc tgccaagcct tcactacttc gcatagacca tctactctag tgctgctttc 166440
tcaatcttag caaacatttt tattttgcat tatatttata atttagcttc ctgccttaca 166500
ggaaatcaag tttacctatc aagcccagta aaacatctca cttctaaccc ttagctttac 166560
ttagcatgct cataatcttg tagtctcagg agaagcggcc cttctgatga gagctaatcc 166620
tctctctgtg ccctttaaag agatacagtt tacattttta tcaaagtgat tcatatgcat 166680
ggtttaaaaa aattgggcca aatggccacc cactgctcca tgccctccaa atgtttttgc 166740
caatggcatc ctctttcaat tatttagctg tttcttttgg tcgccatctt catagcacta 166800
aataattatg ttgccctttt cactacttaa acacttacac attttagata ttacatattt 166860
atttaactgt cctgaaaaat aaagttttcg ttcttcctca acactctgcc atatgggtgt 166920
taactacagc attccccttg ctgaccctcc ggaatgtgat ttgctgttta tcgaatctca 166980
tggcttcctg ttaccattca gtacctccat ttcctggagt gcatgctcaa gtaacttgct 167040
aaggaagtgt gggtgagggg taaattttct gagtcgtcct gtatctgaca atgtctttat 167100
tctaatctca cacttacttg ctaatttgga tataaaatat gatgttgaag ataattattc 167160
ctcaaggttt taggatcatt gttctatttt gttctatcac attgtgtagg tgttatactg 167220
ttctgattct tttcctgttt tggtgacctc ctctctccca aacttaaaaa atctctggta 167280
ttctgaaatt tcagtgtttt ttgtttgctt atttagtttt attattaatt gtactgagta 167340
gtcagtatgc tggccccttt ctgtctagag attggaatct cttaactttg ggacatttct 167400
ttgtgctatt tcttcattca tttttttct ctcgttttct gtttctggaa ctcttcatta 167460
gtaggatttt ggatctgttg gattgattat ctttgtgcct tttatcttac atttgttgt 167520
acttcctgag atactttaac ttctagtcct ttattgcatc ttttattttg agaattggaa 167580
atttttttt ctgtggttct tctggcgttg atcttttta taaatagcac attgttcttg 167640
atttatagat gcagtctctt taagaatctc tctgaggata acaatttgag ttgaaagttc 167700
ttttctgctt tttgcattct gttttctcca aattcagatc ctatttattt atctttagtt 167760
tttctttcat tttggacgtg ttcctcaaat atctagcaat ccttggctgt gcatttatgt 167820
ttaagcatgg aagacactga caaactgatt gggagctctg tgtatatgag aggggcttgt 167880
tacctgtcag gtttctgctt tatgtgaata gggtaaggaa ctgttactac tgctagggga 167940
ctctcaaatt caaggattta agagacattc agcctaagga aaggctggtt gatggttccg 168000
aatatagatt tacgtatttt tagccccaag ttttactcct ttccttccct agcacccccct 168060
agtgtctgag aatcctgacc ttctctgagg ttctctgggc aagccagcct tcttttgatt 168120
ttatcccttt ctgccatcag tactctaaac tggtttcttc ctgctctgta agttaccact 168180
catatttgtt ctttgtcttt cagaatgtac aaacaagatt ttcatcttct gattacctcc 168240
tctctctgtt tattatgggt ttgtgtattt ttgagttcct tgcaatgggt ttgtaaaagg 168300
tcaacttggc tgagctaaac tccatttccc aaaattccct ttctctctct ttttttttg 168360
agacaaagtc tcactttgtc acccaggctg gagtgcagtg gcaccacctc aggctcactg 168420
cattctccac ctcttgggtt caagtgattc tcctacctct gccttccgag tagctgggat 168480
tataggcaca cgccaccatg cccagcttaa gttttctatt agtagagaca gggtttcacc 168540
atgttggcca ggctggtctc taactcctga cctcaagtga tccactcgcc tccgcgtccc 168600
aaagtgctgg gattacaggc gtgagccacc gtgcctggca gtctttccca tatgtttctg 168660
attaggttgg gtcacaggga gattttcaca cctaacatga gacatttatt gctggacaac 168720
caaaaacatt ctctttcccc agaaaagcac acaaagtctt tttttttgt ctttaagata 168780
atattagttc atccttctcc acctgattca cactccctct ttcatgttct gtaacttaaa 168840
tactgaaata taaggttaac tcttattaac acatgttaga taataaggaa gaaaactaat 168900
gaaccaaaaa atatttggtt aatacacaca cacacacaca cacacatatt taaacacaaa 168960
tatatcaaat taaggaagaa atatttgtaa ctattgtagt ccatatttct gcaatggtca 169020
tgtggtcata gctggtatat atgttcttcc actactcatt ccatattccc tttgctctaa 169080
gtaagtactt cagccagttc ggattccttg cctcatgggg tgacccaggc tttcattctt 169140
gaaagatatg ggctattagc agttctttct gaattacttc tgctgtttgc agttctacaa 169200
aaccctttgc aattttccgt ggacttgaat ctcaggacat gggaaatact aagagatgtc 169260
ctagaggatc tcttgcattt cagacacact tcacttcacc tgtattgtgc aatagtcact 169320
caaattcctc ttgaaagtta gggtcagtta tccccagtga atacagtaac cctctccatt 169380
gcctcttgat ttggaacttc agagtaaagt tggggtctca acttcaggtt agtggaatca 169440

tttgttgtgt ccccctagtgg atgtgttcgt ctctttgaaa ctagactttt agaacaacaa 169500
agcctaaagt cacagggata ggaaataaat attttgccaa cactgaattt attagtcttc 169560
attgatttcc caaagaaatg aaagaaaaag cctcctagca ctcattaaaa gtcatgaaag 169620
gcatcactgt ctactactca gtcacctaag ccagaaacct tggaatcatc ttaaattctt 169680
cccttacaac tatcatttta tttctactgg tgacaaaatc ctatcaattc taccttattt 169740
tacatctctc acttttatcc cctcctctac attctcatat cagtcactgc cttagactag 169800
gcatcacctt cacttaccca aattgtaaag atgatcttta aactttttgt ctctgtccac 169860
tatttttctg ctaaagcaat cttttaaaaa tcagactata tttagtggct ctaagtgctt 169920
ataaggtaac accaaactcc ttaaccagct gaattaattt atctgttata atcgagatcc 169980
aagtcagatg gctttaacaa gttagaagtt tattctctct taggacttag gtgatggttc 170040
aggaatccag ttccttctga tttacctggt cttggatatt tcaccaccat gtcagaacca 170100
gcatgaaaaa ggaaatggag aagaggagga tgtgcccttt cctttaaggg gacaacccag 170160
aagatgtgca aatcatttc actctcagct ccttggccag aacttggctg ccagttctag 170220
ctgcaagaga tattgagaaa gagagtcttg tctgggggc cttgtgtcta gctacaggag 170280
gcctgttatt gtagataagt ggggaatggg tattggaaga ccaccagtag tctctgccac 170340
atgaggcata cacaatcttc tggagagtga tcctcgctta cttctttacc ttattttta 170400
gtgtgctgct tttccacatt tccctcttta tcagctctct tgactctgtt tccccttgc 170460
attagccatg ctaaactatt tgcacttcca aacatgctct gctgatacac gtttctccgc 170520
ctttcctctt gcttccttag cttgaggtgc ccttgccaca tactttcctc atctccaact 170580
cattctgtca gcttgatttc agagaagttg tcttttaaaa ctcaacataa gcctctccaa 170640
ttctgttttc cttattgtgt acacatagac ttgagacatt acatctcttg gtacttattt 170700
acacttagat tatctttata ttttgttgta aactctttga gggcacatat gacattttat 170760
tcttgtatct cttggatgta atgtaatgcc tgacagataa aggatgtgta ctttcttttt 170820
actggatgag caagtgggtt tatagcaaaa ataactgtgc agacttttta aaatgtcaga 170880
acctactctt aagatagggc tactgtttct ttttttttt tttttaactt ttgtcaatca 170940
aaatgttagt gctagctcta acaatattta taatcatcct atattttctt gttagtcttt 171000
tcatatttag tttttagtta caatttaatt ccagaagtgc tcagagttag ttatggaatt 171060
tgttaatttt tttctcttta aaatgagcag ccttttctgt ctcctaaggc acagtattta 171120
taattgcttt ttttttttt tttttttgag acggagtctc actcttgtca cccagtctag 171180
agtgcaatag cgtgatctca gctcactgca acctctgcct cccaggttca agtgattctc 171240
ctgcctcagc ctcccaagca gctgggattt gcaggcacct gccaccacac ctggctaaat 171300
ttttgtattt ttttttttt tttttttta gtagagatag ggtttcacca tgttggccag 171360
gctggtcttg aactcttgat ctcaggtgat ctgcccatct ctgcctccca aagtgctgtg 171420
attacaagtg tgagccactg cgccaggcct gtaattgctt tttaaccgtt cagtatatca 171480
gtgacttgcg gcaggagatg tgttgaagta agttagataa tactgacatg atttccaaag 171540
cctgtcctaa gaccttctga gcactgccac cttaacatac tggttctaag atattggtga 171600
ctcaggatta atttaatgac tgaaaagagt cattgttgta aacaaattct aaggcaagag 171660
tgccttaaat attcttacat ggagaatttt ttttttctgc tttactgtct tgagtaatat 171720
cttttgtttt acttgtagat ttgatggtcc tcgaagattt gaggatttag ggtcaaggtg 171780
tgaaggaccg agacccaaag ggcctcgttt tgaaggaaat cgcccccgatg ggccaagacc 171840
cagatatgaa ggtcacccag cagagggcac taaaagcaag tggggaatga ttccccgggg 171900
gccagcatct caattttata ttacccccag tacatcccta agtcctcgac agagtggacc 171960
acagtggaaa ggccccaaac cagcttttgg acagcagcat cagcagcaac ctaagtcaca 172020
agcagaacct ctttcaggaa acaaagaacc attagcagac accagtagta accagcagaa 172080
gaattttaaa atgcaatcag ctgcattttc cattgctgca gatgtaaagg atgtcaaggc 172140
ggctcagtca aatgagaatc taagcgactc tcaacaagag ccacctaaaa gtgaagtctc 172200
ggaagggccc gtagagccct ctaattggga ccagaatgtt caaagtatgg agactcaaat 172260
cgacaaagcc caagctgtta ctcagcctgt accccttgcg aataagcctg tacctgctca 172320
atctactttt ccttcaaaaa caggggggat ggagggagga acagcagtag caacatcatc 172380
attaacagca gataatgatt ttaaacctgt gggtattggt ctaccccatt cagaaaacaa 172440
ccaagataaa ggcctgcctc ggccagataa tagagataat agattagaag gcaatagagg 172500
caacagctca tcttacagag gtcctgggca aagcagaatg gaagacacac gggataaagg 172560
tctagtaaac agaggtcgcg gccaggcaat cagtcgaggc ccaggattgg tcaagcaaga 172620
agactttcgg gataagatga tgggtagaag agaagatagt cgagagaaga tgaacagagg 172680
agaaggtagc cgggacagag ggttggtgag gcctggaagc agtcgggaga aagtgccagg 172740
tggtcttcaa gggagccagg acaggggtgc agctggcagc cgagaaaggg gaccacctcg 172800
gagggctggc agtcaggaga ggggacctct tcgaagggct gggagtagag agagaatacc 172860
accccgaaga gctgggagca gggagagagg accacctcga gggcctggca gtcgagaaag 172920
gggactggga agatcagatt ttggtcgtga tagaggtcca ttcagaccag aaccaggaga 172980
tggtggggaa aaaatgtatc catatcaccg ggatgagcct cctagggctc catggaacca 173040
tggagaagag cgagggcatg aagagtttcc attagatggt agaaatgctc caatggaacg 173100
agaaagactc gatgactggg atagagagag atactggaga gaatgtgaac gtgactatca 173160
agatgataca ctagagctct ataacagaga ggacaggttc tcagcaccac catctcggtc 173220
tcatgatgga gataggcgag gccccttggtg ggatgattgg gagagagacc aggatatgga 173280
tgaggactac aatagggaaa tggaaaggga catggacagg gatgtggatc ggatttcaag 173340
acctatggat atgtatgata gaagtttgga taatgagtgg gacagagatt atgggagacc 173400
actggatgaa caagaatcac agtttcgtga acgggatatt ccatctcttc cacctttacc 173460
gcccctccca cctcttccac ctttggatag atatcgggat gatagatgga gagaagaaag 173520
aaatcgagag catgggtatg atcgagattt ccgtgatagg ggtgagttga ggattcgaga 173580

gtatccagaa agaggagata catggcggga aaagcgagat tatgttcctg acagaatgga 173640
ctgggaaaga gaacggttgt cagacagatg gtacccatct gatgtggata gacattcccc 173700
catggcggaa catatgccct cctcacatca ttcctcagaa atgatggggt ccgatgcaag 173760
cttagactct gaccaaggcc ttggaggggt aatggttctc agtcagaggc agcatgaaat 173820
cattttgaaa gctgcacaag aactgaaaat gcttcggtaa gttgactcct tcagatcctt 173880
tcttttaata aaaaccacat tcagactgct ctttttaaag tgatatgata tatttgaatg 173940
gaatcatttt acttcaagtt cttatcacat acttttaatt tatatttctg gtcattcagt 174000
gctgttttag ggttagaatg ggtatttagg aactgtgtta atgataaact tatgccctaa 174060
gatgtgaaaa ttgattgctg tcttggcctt tgtagaagtg ttgctggcat gccatgcctg 174120
tgcatgtttc aagtgtcaaa cttcaacttt tcagtgctct ggatttcaca gggttcatgt 174180
gtaaccttca ttttttccag taatcagttt gttctttggt attagatctg atagacttta 174240
tattcactaa tcattgataa aactttggaa agtgctgtat ttttctttgt tgaagctaag 174300
aatggtttgc ttaggagcat cctcagattt gtcttaacta ttgatacttt cttttggga 174360
tgttttatct ttcattataa ctaaaagcag atatttctcc ttgaggatgt tatttgtttc 174420
tacataacca aatgttttct ttgtgaaatg gaaaatagga tttcttgact tacaatcaac 174480
tgtgtatagg tatctacata atttcctctt ttcttgtgg taacaacaat aacaaaagct 174540
aacatttatt gcatgcttac tccgggccag gaactgtact aagtgtgttg tgtataataa 174600
actcatttaa tccttaaacc agctgtatct tgggatagga atttagaagt cagtgacaga 174660
ttgaaaactt ttttaaagca attttttga gaaagtcaat taatgattag gttttgataa 174720
attccaatgt atttcttcca tttttcaatt ctggtttaca tttgtggatc ttaacaataa 174780
attataactt aaactttaga actaggaaag aaaaatacgg tgaagaaaag gtaatgatga 174840
aagaccagat acggcaggtt tgtatggtct atagctaaga ggtttatttt ttccagatta 174900
ctcatgaaga aaatgtggaa agcatgctat agaatattta gctactacac tattcacttt 174960
taagttatag acacctttaa ttttaacacc taccgtgtaa ctgggcactt gctaataatt 175020
gagtgtatat tatgtcagtc tgtctatctc aaaggcctgc aaagataagg tagtattatc 175080
cccacttcac aaatgacact aaagttcaaa gaggttaagt tcccttggct agtaagtggc 175140
aaacccggcc tttgaactca atctgtctaa aactctaaaa tctgtgttat ttatggagaa 175200
taccaagctg attggtgttg ttaagctgcc aaggtttctt tttttttcct ttttaaattt 175260
ctttttaatt cgttattgct ttgcatggta atgctggata aaaggtgctg ctgctgcttc 175320
atcctttttt aaagctttct aggcaacatg aacataatta aacaatatat agggagtgat 175380
ggataatgtc atagccgaat acaagtcgtt tatgttgtgt cagtgtaaag tcgtaaggag 175440
tatcataagg gatgtgatga tgggatttac ctgagcagta aataatctta cacacttgac 175500
ccttactttg tggtccctca tttgtggacc ctcatttggg tcactttaat gtaaggagaa 175560
aattgtaaaa ggtcttaaga aattgtagtg gattttacct atgtttaagg taggcattgt 175620
gaaaaaaagt aaaaggaatt tcgtcagttt aaagaaaaaa ccgaaggatt acataattat 175680
cttcaggaat atgaaatgtt gtttttaaag gtatagactg acagttgttc tcaccatcta 175740
cagcaggggc cataaattaa aatgtcccca agaatcagat aacaaaaatg agtgaaagtg 175800
ggccaagtat aactctgtag agtacggtga agattgtgac aggttgtttc atctaacatg 175860
ggcagcctct actgcactca gctgattgtt ggcatgtggg aatgtaggcc tggtgttacc 175920
atatctcttt aagatttgaa tttatatatg aaatattttg ctttgtaaat atgacagcta 175980
atttcaaatt ttaaaaactg atgtgaaagc cacattttga aaaccttttt aggccatatt 176040
tggcccatgg gccaccattt tgcaacctct ggtctaaaga gttgaaaaaa ataggccggg 176100
cgtggtggct tacgcctgta atcctagcac tttgggaagc tgaggcaggc ggatcatgag 176160
gtcaggagtt cgagaccagc ctggccaata tagtgaaacc ccgtctctac taaaaaaata 176220
caaataatta gccgggcgcg gtggtgcatg cctgtagacc cagctactcg ggaggctgag 176280
gcaggagaat cacttgaacc cgggaggcag aggttgtagt gagctgagat cgcgccattg 176340
cactccagcc caggcgacag agtgagactc tgtctcaaaa aaaaaaaaaa aaaaatagca 176400
agagagcttt tggttggatt aaagaaaaac ataacaagta gatgagatag tagaaaacaa 176460
ctgaggcagg ttgtagaatc tattcttgaa tatcttcaat aatttataat tatttatatg 176520
tcttaaatga ctgatgagtt agctacataa aagcaaaaga gggtaatcct ggagctgctc 176580
agtgtatggc cactgcagag aattttagga caaggatttt ttaggtctcc ttttttttaat 176640
taaatattaa ttgtagtcac ttaggaatgg aataagcatg ctttatgtac tatgtcaatt 176700
ttatttgttt gcagggaaca gaaagaacag cttcaaaaga tgaaagactt cgggtctgag 176760
ccacagatgg ctgaccatct accacctcag gaatcaagat tgcagaatac atcttcaaga 176820
cctggaatgt atccggtatg ggagaatgtg tgctcagaaa atgaaatggt ttctactttg 176880
tatgtttcta tgttgtttga tttttttag aagttgaata aataacaaat ttcctctata 176940
aaatgtaaat tttggaaact taaaagttta tggtaattga actttgaaca ccttaaaagc 177000
atgttcaaaa gtgtggtaga gatgctcctt ttccccagat tttgataagt acaccaagta 177060
cacctgaact acatagcttc tgttcacaaa atggtagaat ttttctaata atttttatgt 177120
taaaatagtc ctgttggctg ggcatagtgg ctcacacctg taattctagc actttgggag 177180
gctaaggcga gaggattgct tgagcccagg agtttgacac cagccttcgc aaaatggtga 177240
gacccctatc tctaccaaaa aaaaaaaaaa gtcctgtttt ccatctaggc agggctagat 177300
gtgggcctcg tgatgcttcc tacctcttca ggctactagt ccttcagatg gggaaatgtc 177360
ataccccagt acatacttca gaactgttca ggcagtatga aaaggggtgt ttatgttaaa 177420
gacttgagta attaagttct cctttttttc ccaagagcat gccaggactt cttttatttt 177480
tgttatttta cctttaagcc agtaatgggc ttttcaaatg tttaaaatgt tatcttgcag 177540
gtagttttat gacagcataa tgaagaaaac agctctactg aacctctact gaaccatttc 177600
atcaaattaa cacttttcat ttctcagtgt tcccttccat cctggtctaa tacttaacag 177660
taagacatag ttgttgacat cagtgaccac catggtgttg ttgactcccc agatcggatc 177720

tcctgacctt tgtacgcatc tacctgtctg cagggtttct gtctgtggat gtgactcctg 177780
tcaaatgcag catattcaaa atggggctcg ttatcatacc ccattaaatt gtcttttttc 177840
ctcaccatca cctagtcacc ctaacttgaa acctgaaaat caaccctcca tcttgctcca 177900
tcttgctgat ttgtcctacc ctgctgctga ttgattttac ctcctttgaa tcaaattctc 177960
aagtataatt acacccattt taagcctata gttcgagttt tgacagatgt ataccaaaag 178020
ataaaaaaat tctcggccag gcgcggtggc tcacacctgt aatcccagca ctttgggagg 178080
ccaaggcggg tggatcactt gaggtcggga gtttgagatg agcctgacca acttggagaa 178140
accccgtctc tactaaaagt acaaaattag ccgggcgtgg tggcgcatgc ctgtaatccc 178200
agctactaag gaggccgagg caggagaatc acttgaaccc gggaggtaga ggttgcggtg 178260
agccgacatc gcgccattgc actccagcct gggcaacggg cgaaactcca tctcaaaatc 178320
aaaacaaaac aaaacaaaat tctcatcacc ctaaaaagtt ctctcatgcc cttttgagat 178380
tgggactacc cctatactcc acttcaggca gcgtcgatgt gatttgtggc cagatggaca 178440
ttttaaaaca aatctcacca tgtggctacc tggctttaaa acttcaatgt tctcccatgc 178500
cttcaggatg aaatttagaa tctcttgcat gactttcttg ttcttcctga gctggcctga 178560
cctctgctga ccattgcttg ctactccact ctctgatgct tcattttccc tccacataga 178620
gctacttaac aattttgtga atgtgctaga ttcttttact tcttcattcc tttgcgtatg 178680
catgttgttc tctaagccta gagtcttttc tttgccttcc catcccctcc tgcaactggc 178740
taatgtttgt ttatctttta caactcagct ctactatttt ctcttccacc tatgtagggc 178800
aattaccctt gtacataccc tgtaacatct tgtactagtc tttgttgtca tctttattat 178860
gttgcatttt gagtgtctgt tttcttgtct ttctttctaa attgagtcca taagataata 178920
actatatctt tcatctttgt gtgtcttagt aaatagaagc gccagattgt acatccacat 178980
ttctgcttta tttgcttaac aaatcataat catctttcca tattgttata tgctatatta 179040
ttttttgatt tacaataatt tattgaatta attcaatttc tgttggaatt tgtttctaat 179100
ctttcactgt tattcttaat attaccctaa tgaatgtctt tgtattgggt tcttatttta 179160
gataactcct tttctttttt cctttttttt ttttttgaaa tggagtctca ctctgttgtc 179220
caggctggag tgcagtggca tgatttcagc tcaccgaaac ctccacctct tgggtttaag 179280
cgattctcct gcctcagcct cccaagtagc tgggattaca ggtgcctggc accacacctg 179340
gctaaatttt ttgtattttt agtagagaca gggttttacc aattggccag gctggtcttg 179400
aactcctgac ctcaggtgat ccacctgcct cagcctccga aagtgctggg attacaggca 179460
tgaaccaccg cacccggcca ataacttctt aggagtaagt tttatgtttt tgttttgtct 179520
cttttttttt gctctgttgc ctgggaggct ggaatacagt ggtacaatca tagctcactg 179580
gagcctcaaa ctcctgggct caagtgattc tctcacctca gcctcctgag taactgggac 179640
tacaggcaca caccctacgc ctggctaata ataaaaaaaa aaaattgtta gtagggacag 179700
ggtctcgcgt tgttgcccag gctggtctcg aactcctggg cttaagtgat cctcatgcct 179760
tgtcctccca gagtgctggg attacaggtg tgagcccctg cacccagcca acagtgattt 179820
taacgtccca aagtgggaca cttcagtcaa agaataagca tacttttttt tttttttttt 179880
gaaacggaat gtagtctgtc acccaggctg gagtgcagtg gcacaatctc agctcactgt 179940
aatatccacc tcccgggttc aagtgattct cctgcctcat cctcctgagt agctgggatt 180000
acaggtgcct gccaccattc ctggctcatt ttttttgtat ttttaataga gatagggttt 180060
caccatgttg gccaggctgg ttttgaactc ctgacctcag gtgatccacc tgcctcagcc 180120
tcccaaagtg ctaggattac aggcatgagc caccacacct ggcccagaat aagcatactt 180180
tttaaatgat tcctgtcaca tagccaaaca gctctctata atagttgtat aatggccggg 180240
tgtggtggct catgcctgca atgccagcac tttgggaggc caaggcaggt ggatcacaag 180300
aggtcaggag ttccagacca gcctggccaa catggtgaaa ccccgtctct actaaaaata 180360
taaaaatcag ctgggcttgg tggcatgtgc ctgtaatcct agttacaggt gaggctgagg 180420
caggagaatc gcttgaacct gggaggcaga ggttgcagtg agccaagatt gcaccactgc 180480
actccagcct gggtgacaga tcaagactct gtctcaagtg catcacctga ggtcaggagt 180540
tcaagaccag cttggccaac atggtgaacc ctcgcctcta ctaaaaatac aaaaattagc 180600
caggcacgcg ccaggtggtg cgcacctgta atcccagcta ctagggagac tgaggcagga 180660
gaattgcttg aacctgagag gtagaggttg cacatagcgc cactgcgctc cagcctgggc 180720
aacaagagtg agactctgtc tcaaaaaaat atataaataa ataaatgaaa aaaaataatt 180780
gtataacatc tatactatag cctcgtaagc attagctact taatattttt ggtatattta 180840
ataattttaa tacagcattt ttgattacta gtgaacatga atattttccc atatttgtta 180900
attatacttt cctcttacag aaattctgtt tgtgtccttc acccatttat ctgtttgagt 180960
caggtttttt tttttaaat taactcatta tccttaatat aatatagata ttaacccttt 181020
ctcatataaa caataatttt aaaaaatgta tcttttactt tcactatata gaatgaagaa 181080
agcagttttt aaaaaattta taagtataca tctatgagat cttaagatat ttaaaacttg 181140
tcttgataga tctacccaga ctgaatagct tcatcctata ttggcatttt cttgacaaag 181200
tttaccatgc catactttaa aagtttacct ttaggtaata gccatttgtc tttaactgta 181260
gccagtagtt acctccagtc aaataatgtt ccttctgtat tagtcaactc cattttatta 181320
ttcagaaagt ttatttttaa agtactgtgt atatatggc aaatatataa ataatgtata 181380
ttgtgggctc tccaggatct aaaatagcgt caataaagaa aaaacatcct agaataacag 181440
agaagtaaaa aaccaaaaca aaacaaaaaa caaaaaaata aaaccaaacc agccagacac 181500
agtgaactaa gaaatgggaa gtaagtgttt gaatcacact tacaaagatt aaaatagaga 181560
ctaggcatgg tggctcacac ctgtaatccc agcactttgg gaggctgaag cgagaagatt 181620
acttgaggcc tggagttcaa gaccagcctg ggcaacatag caagaccccg tctttaccaa 181680
agaaagaata aaaataaaat gtatttggca actgggtttc aatattgagt agataggacc 181740
aatttgctaa gaagtcctac catctccatc atagacattt ctgatggtaa ctggtgatgc 181800
tttgggaaaa caaatggaag gagggcacac aacagtggca ctccttagat tactagatgt 181860

tcatttagtt tattttgttg tttcatagta ctcatgttct ctgttggtct cagcttcacc 181920
acagatgact attgtcaacc agataactga aaggaacaga agtgtggagg ctatctagga 181980
tttctctcac tgaaaattta ctgtttttgt ggaggagata ggtctctaga gtaggaagat 182040
agttcttatt tatttattca tttctctcct tcctgcacac tgagcatagg aagatagttc 182100
ttaaccgagg gttataaatc agaattattc atggagggtt tttttaaaat tttgaattac 182160
tggtgggacc ccacctacag aagttcagta gatttggtat tggcctggga catttgaatc 182220
ttacgtggct accaccctaa tttggagtat ggaaatttta aaacgttttc attacccctg 182280
taagatcact gcaccagttt acagttagtt aatcctcatt cctaccccag tcccagcact 182340
ttctgtctat atagatttgc ctttctgggc attttgtata aatgaattat ataatatgta 182400
gtcttttgtg tctatcttct tttcaaagtt tacccatgtt gtagcatgac tcagtacttc 182460
atttcttttt actgcttttt aaatttcttt ttatttataa cctgttacat gaatatacca 182520
cattttgttt aaccattcac cagttgttgg acattagggt tgttgaatag tacggctatg 182580
aatgtttgtg tgcaagtctt tggatggaca tatgttttta tttttcttgg atagataccg 182640
agaaatgaaa ttgctgagcc atatgataaa tttatgtttg gccgggtgcg gtggctcatg 182700
cctgtaatcc cagcattttg ggaggctgag gtgggtggat cgcctgaggt caggagttcg 182760
agaccagcct gaccaacata gtgaaacccc atctctacta aaaatacaaa aaatcagctg 182820
ggcgtggtgg cgggtgcctg taatcccagt tactcagaag gctgaggcag gagaatcact 182880
tgaacctggg aggcagaggt ggcagtgagc tgagatcgcg ccattgcagt ccagcctgag 182940
caacaagagt gaaactccgt ctcaaaaaaa aaaaaaaaaa attgtgtttg actttttgag 183000
agaaattggc aaagcatttt ctatagtggc accatttatt cccactaaca atctaaaagg 183060
gtttttcttt cctatatcct tgccatcatt tgtttttgtc tgtcttatta attataacca 183120
ttccagtgca tatgaaaatg gtatctcatt gggattttaa tttgtgtttc cctaatgact 183180
aatgatggtg agcatctttt catgtgctta ttgggcattt atatattttc tttggagaaa 183240
tatctattct catcttttgg ccatagtttt tttttttta tgatttgtct taccgagttt 183300
aagtgttttg tatattctga attcaagttc tttatcaaat ataagatttg cctatgtttt 183360
ctccctttct gtggcttgtc ctcatttttt ttgatggtat gttttgaagc acgaaatttt 183420
gaatgagtcc aaggtgttaa ctttttttctt ataaattgag aaccagattt taatttccac 183480
tgatctataa aaacatagtg gtatacttgt ccactaagct atttattctg ttctctagcc 183540
tccagggtcg tatagacctc cccctcctat gggcaaacca ccaggttcaa ttgtaagacc 183600
ctctgctcca ccagcaagat catctgttcc tgtgaccagg ccacctgtcc caataccacc 183660
acctccacct cctccacctc tacctcctcc tcctccagtg ataaagccac aaacttcagc 183720
tgtagaacag gaacgatggg atgaagattc tttctatggg ctctgggata caaatgatga 183780
acaaggactg aattcagaat ttaagtcaga aactgcagca attccatctg ctccagtatt 183840
accacccccca cctgttcact cttccattcc ccctcctggc ccagtgccta tgggtatgcc 183900
accaatgtcc aagccaccac cagtacaaca gactgttgat tatggccatg gccgaggtga 183960
gtaatgatag tgcacttgta tttgggattc tacaggaatt gttagcttca gctttagctt 184020
tctctagctt cactcttaca gtactttatc tctgattttg tttaccagat atatccacta 184080
ataaagttga acagatacct tatggagaaa gaataactct acgcccagat ccactacctg 184140
aaagatcaac ttttgagaca ggtaggattc ccagagaggt cagtattttt aaacatttta 184200
gggcctaatt atcacatgat tttcctttttg gtgagattat ctgagcctcc atttaataca 184260
gctaaaagat tcttagttga atttatggca aaaatttata ccagctctga agttagattt 184320
atagttttct gcttttattt ctataaaagg gatatagtta ttttgtacta attgtttttg 184380
tagagcatgc aggccaacgt gatcgttatg atagagaaag agatcgtgag ccttattttg 184440
atcgtcaaag taatgtcata gcagatcatc gagattttaa aagggatcgt gagacacata 184500
gagatcgaga ccgggatcgt ggtgttattg actatgaccg ggatcgattt gacagagaac 184560
gccgaccccg agatgatagg tatgctataa aacaatcttt gctaggtgta caaattactg 184620
tatacttaac agtaaaagtt taagagaaat ttaataagta acatattctt ctcatgcatg 184680
tgtatatgta atttggaggg ggaagaacaa ttctggacca gttttcagga aagtaggtcc 184740
ttgacttggt agcttgaatt ccagagaatt aaatgggaga tggagatagc cagaagggaa 184800
tggaaatggt attgtcaata aaggtgcagt catactcagt aggcaagcaa tttatttttg 184860
caattcatga gctttttatt tattattatt attattatta ttatacctag gggcattctg 184920
gcagccagcc aaggaaaagg gaaagggggg caagcccatg aactatttat ttatttattc 184980
attactattt tgtatggaga cggggtctct ctcagtctgt cacccaggct ggaatgcagt 185040
ggcgcagtct cagctcactg cagcctccac ctcctgggct caagtgatcc tcccacctca 185100
gtctcctgag tagctggggc cacaggcatg tgccaccatg cctggctaat tttttttttg 185160
tttttgatga cgatgtggtt tcaccatgtt gcccaggctg atctcaaact tctgagctca 185220
agtgatccac ctgcctcggc cttccgaagt gctgggatta taggcgtgag ccatcatgcc 185280
cagctttccc atgaacttct tagatgtatg tatataaagt aaatctaaat gaaaaagcaa 185340
agtcaaaatg taaatattgg ccgggcgtag tggctcacgc ctgtaatcct agcactttgg 185400
gaggccaagg caggtggatc tcttgagctc aggagttcaa gaccagcctg gccaacatgg 185460
cgaaacccca tctctacaaa aaataacaaa agttggctgg gtgtggttat gtgcacctgt 185520
agtcccagct actcaggagg ctgagatggg aggattgctt gaacactgga gatcaaggct 185580
gcagtgagcc gagatcacgc cactgcattc cagcctggtt gacaaagtaa gaccccaatc 185640
tcaaaaaaaa aaagtaaaca tttactgtgt ggagtacctt cctatagaga aaattgggca 185700
gttaatgctg ttgcgttata aagcagagaa ccccaaatat tttttatttg ctgaagcgct 185760
tcctattaca ccttctagag ctcagtcata tcgagacaaa aaagaccatt cctcatccag 185820
aagaggggt tttgataggc catcctatga ccggaagtct gaccgaccag tctatgaagg 185880
accatccatg tttggaggta gagtgatgcc ttattaacta caaggatgtt gagaatgtaa 185940
agcatgggag atgctttcca ttgtagctat gaatttaaac ttttagagta aaacgtattc 186000

acagatttct tagagctggt ggcattgtcc taaaaaatag cagtgtaatt aatgaaataa 186060
agcatgttta tttttaaaac aaagtagaat caaaattact gatagtctca ctacctacca 186120
tactacagct attataattt tgtagctttc ctctcattcg ctctttacag acatatttat 186180
gaagttgtaa tcaaagtata ataatacaat ttctttatcg tgattgttct gattgatatt 186240
gcaccataca catttttcat atgtaaacct attttattaa tggctctgtg acattccatt 186300
gagtggataa aatataagac caagaccctt attcttagtt atttaggttg cttttagttt 186360
ttcactgcta tgatttacac ttaaactaga aactcatacc tatttttagg aacaggctct 186420
cctaagatgg tataactctt actcttaaaa ccataaagca gaagttacag aacttaggac 186480
tatccagcaa agcatttatt attcttccat gtcctgttgc ttaactttcc tacatttaat 186540
acaaacaagg atgaattcag tgttttgcag aacattctat ttgcctgaaa tcatacttaa 186600
atcttctcat tttcttgact tcagtagatt ttacattacc ttaaattttc tctaattaaa 186660
gttaaaagtg gccccaaaat cactggactt ggtttgaatt atattacctt aggatgtgtg 186720
gggagagttg aatttcaaaa taattactct acaaatagta attttgttca actgctggaa 186780
tcctaggaga acgaaggact tatcctgagg agcgaatgcc tctgccagct ccttcactga 186840
gccaccagcc tcctccagct ccacgagtcg agaagaagcc tgaatcaaag aatgtggacg 186900
atattttgaa accaccgggc cgggagagca gacctgagag agtgagtcct atgaagttga 186960
ttcgtcttcg tgcaaaccag aagataagag atcttgttaa tatcctgaaa agaatttctt 187020
tatttttctg caacatagta ataatatcag attcaagacg ttttactatc tctaaatcac 187080
catataatct cataaatggc ctacactata gaatctttaa gaatttagca ctaaagtact 187140
tggcaaaaaa gatgttgaat tttaaaacat attaatgaga cctcaattaa ttgcgcgttg 187200
aagagaggaa aagtggattt ggttttatgg tgtgtggaat agaaagggag aattaggaga 187260
cttgattagc ttaggcttaa tttcctagtg acctccaaat gtgcgcattc tattaaattt 187320
gtcttattac agaataatta gactgactcc atttgaatta actggtactg gagatttcag 187380
ccagatattt taattccata tatacagaat taatagattg ttgttttaaa gtctttttgc 187440
ctactattac tatatagcta cttgaccttt cctgtggttg ctgtttgcat gataaatctt 187500
ttaccatttc cttcctttca ttctatttgt atcttttaat gtaaagtgtg tctcctgtag 187560
tatgcctatg gttggatctt tttttttttc cattcaggtt cccttttattt ctgacatttc 187620
ttcaccatcc ttgcaagggt aactccctaa tcactctaga aattcagatt cctgtttctg 187680
actccgtagg acacagcgga tcgtgttttt taatgcagtc tgaaaatctc tgccttttga 187740
ttggattgtt taatacattc acattttttt tttaaattat ggattcagga tacatgtgca 187800
ggttcattac acaggtattt tgtgtaatgc tggagtttgg gcttctaaag agcctgtcac 187860
ccaaaaagtg aacatagtac cctgtagtaa agttattatt gatggcttaa tttcattttt 187920
ggattgttcc ttgcaaatat atggaaatat ggtttatttt tgtttattcg tcttgtatcc 187980
tgcaataccca ctgaattcat ttattacttt tagtaggttt ttagttgatt cttaaggttt 188040
tctatgtata agatcatgtc atctgcaaat atacttttac ttctttcttt ctgaattgaa 188100
tgccttttat ttcttgccta attgccctgg ctagaacctc caatgcagtg ttgaatagac 188160
atggcaagag tggacattct tgtcttgtat ctgatttaga gagaaaaaaa tacaatcttt 188220
taccattaag aatgatgttg ctgggtgtgg tggctcacgc ctgtaatccc agcactttgg 188280
gaggctgaga tgggcggatc atgaggtcag gagattgaga ccatcctggc taacacggtg 188340
aaaccccgtc tgtactaaaa atacaaaaaa ttagctgggc gtggtggcag gcacctgtag 188400
tcccagctac tcaggaggct aaggcaggag aatggcatga acccaggagg cggagcttgc 188460
agtgagccaa gatcgcgcca ctgcactcca gcctgggcga cagagcgaga ctccgtcttt 188520
aaaaaaaaga agaatgatat tagctgtgga attttttttgt agatgcactt cattaggttg 188580
agaaagttcc cttctattcc tagtttgttg aatgttttta tcatgagagg atgttggatt 188640
tgttgaatgc tttttctgtg tcagttgaga tgatcgtgtg attttggttt ttagtctgtt 188700
gatatgatgt gttacattaa ttgatttcat atgttaactt gcattcctgg gataaatccc 188760
acttgatcat ggtgtgtaat aatttttata tgttgctgga ttcagcttgc taatattttg 188820
ttgaggattt ttgcatccat attcatagga gatattgtag ttttcttgtc tgtatgtggt 188880
ttggtgtaat gctggcctca taaagtaagt tagaaattat tccttcctat tctatgtttt 188940
tgaagacctt gggaagagtc ggtatttaaa tgtttggtga attcagtaat gaagtcatcc 189000
aggcttaggc ttttctttgt gtgtagtttt ttaaaattat tgtcttagat tcttcacttg 189060
ttataggtct attcagattg tttatttgtt cttgagtaaa ttttagtagt ctacatcttt 189120
ccaggaattt gtctgtttca cctaaattat ctaatttgtt ggcatacaat tgttcatagt 189180
attcctttgt aatctttttt ataaggttgg tagtaatgtc tcctccttca tttctttttt 189240
actcgaggca gagtctcact attgcccagg ctggggttgc actcctgggc tcaagtgacc 189300
ctcctgcctc agcctcctga ggaactggga ttataggcac atgccactga gcccagcttt 189360
catttctgat tctggtaatt tgaatcttct ttttttcta ttcagcctag gtaagagttc 189420
atcaattttg ttgatcttag tcaggcgtgg tggctcatgc ctgtcatccc agcactttgg 189480
gaggctgagg tgggcggatc acttgaggct aggagttcaa gaccagcctg gtcaacacgg 189540
tgaaacccca tctctactaa aaacacaaaa attaggcggg cgtggtggca ggcacctgta 189600
atcccagctg cttgggaggc tgaggcatga gaatcgctca aacctgggag gcagaggttg 189660
cgatgagcca agattgcacc actgcactcc agcctgggca acagagcaag actccatctc 189720
aaaaaaaaaa aaaaaagttg gtctttttaa agaaccactg ccaccgtttg gttttattga 189780
ctttttctct tttgttttc tgttttctat tttattaact tctgctttaa tctttattgt 189840
ttctttcctc tgtttcttta gtttgctctt ctttttcaag tgtcttaaag tggatggtta 189900
agttattgat ttgagaccta tttctttcct actataggca tttatagcta tcaatttcct 189960
tttaagcact gctttagctg tatcctgtaa gctttggtgt gttgtgtttt tttcattcat 190020
ctcagagtat tttctggttc ctcctggttt ctggctcctc ttgatttctg gttcctcttc 190080
attttttctt tgattcattg gttatttagg agtatgttgt ttagttttca cgtatttgtg 190140

agtttctcta attttttctt actattaatt tctaatttca ttccactgtg gtccagtgac 190200
atactttata ttatttctgt cctttcaaat ttatgaaggt ttgtttcatg gcctgttgta 190260
tagtctatcc tggaaaatgg taggtcattg ttaactgtta tctcattgca gaaaattttc 190320
tctcagtgat tttttttttt ttttggtcta ttctgtttca gattgttgtt ataatgagag 190380
gattacctgg cagtggaaag acacatgttg caaaacttat tcgagtgagt atggggaagc 190440
tgaaaaatca gctgcttgtg ctgcttgtgt tagtagtcac ttgccttctt attaatagca 190500
agcaacataa tgccaataga attgtcttcc atttgatcca ttacagttgg cccatgtggt 190560
agatgttgtc ccatatatat ttttatctac tcagagaaac atttttgcct ctgcttgatt 190620
tgaaaccacc agaagattgt atattattta gccacaagag ggaactttgg tgtgaacatt 190680
aatttattcc ataaattcgt tttaaccttc ttgactgtat gattcttagg ataaggaggt 190740
agaatttgga ggacctgcac ccagagttct aagcctggat gattacttca tcactgaagt 190800
ggaaaaagaa gaaaaagatc cagattctgg aaagaaagtg aaaaagaagg tatggtattc 190860
atctcagatc tcgttctgat tcattaatag tatttaaagg aaaatgtgtt tggagagaaa 190920
tgtggctttt ttgagggcag ggacctctct cttttccgaa cacagtactt tagtacttgg 190980
gtgtgaagga aagactacaa agtcatctct atccaaatac taaccaagga aaaggttttg 191040
gatgattctt tgcttttgct aattccatat ctcttttggg ggaccttagg taatggaata 191100
tgaatatgaa gctgagatgg aggagactta ccgcaccagc atgttcaaaa ctttcaaaaa 191160
gactctggat gatggctttt ttcccttcat catcctggat gccatcaatg acagagttag 191220
gcattttgac cagttttgga gtgcagcaaa aaccaaggga tttgaggtag aagcttaaag 191280
aactttaaag tactttgtgt tgtcatgtaa atgttatata tctttgccta attattattc 191340
attcttgctt aggtatattt ggctgaaatg agtgcagata accagacttg tggcaagaga 191400
aatattcatg gaagaaagct taaagaaata aataaggtga ttttaagaaa catagaataa 191460
tagagtacta tcatgcgctg cataatgatg ttttggttaa ggaaggactg catatatggt 191520
ggtcccataa gattataatg tatttttatt gtaccttttc tgtgtttaga tatgtttaag 191580
tacacaaata cttcattgtg ttgcagttgc ctacagtatt cagtacatta acatgctgta 191640
caagtttgta acctagatgt gcagtaggct ttatcatcta ggtttgtgta agtatactcc 191700
atgatgttca cacaatgaca aaattgccta atgacccatt cctcataata tatgcccatt 191760
gctaagtgat gtgtgactgt actctaaacc tcatattatc tgtatcagca ctcttgtctg 191820
gaagctcttg tttatctcct ttttttttca taataacttt cctatctgct ttagtaaatg 191880
tttctcttcc tttgtttctt ttcctagctc ttcttgaaat tagcctgtta tggcctggca 191940
tggtggctta ggcctgtaat cccagcactt tgggaggctg aggtgggcgg atcgcttgag 192000
cctgggagtt tgagaccaac ctgggcaaca tggcgaaacc ctgtctctac aaaaaaatac 192060
aaaaattagc tgggcatggt gtcatgtgcc tgtggtccca gctactgggg aggctgaagt 192120
gggaagatca cttgagccca ccatgtcaag gctgcagtga gccatgttgg cgccactaca 192180
ctccagcctg ggtgacagac cgagattctg tctcaaaaaa aaaaagaaat tagcccatta 192240
gactttacaa tattaaatta atatttaaca aatatttaat taatatttaa caatatttta 192300
aaatattgtt aaatgttgtt tatcttggat gcttacttgg tctttgagta gtttctagag 192360
ataactcaac catctgaatt tttcaataga tggctgatca ctgggaaact gcacctcgtc 192420
acatgatgcg tctagatatt cgttctttgc tgcaagatgc tgctattgaa gaggtgagta 192480
tcctttggtt caaatgcaat gcaaagtgat gttacttttt cacctttttta ctttgaaaaa 192540
cttaaaacct acagaaaagt tatatgaata gtattcatac aagaacacct actgatatgc 192600
ctttcattga gatcacggtt agtattttgc cacatttttc tctgaatgca tgtattctta 192660
ttaacattct tgttaatgat gtcactgttt ttgtggaacc atttgagagt taagttgcag 192720
atcttataaa ccttcaccct taaatacatc agtaggtatc ttgtaagaac aagtacattc 192780
tcttttgatt tgatgacagt tatcaaatgc aggaaattta gtattgataa aatactatat 192840
taagtaatat aaatttcctg aattaaattt cttgaattat ctcacatata gtttatattc 192900
acattttacc agttgtccct ataacgtggt atatagaaat tttcaaaaat ctaagattca 192960
atcctttaat ataaaatata gtttctcagc cttgtcattg tcatgaaaga taaagaattt 193020
atgaagagta taggcctgtt ttgtagaatg cccctcaatt tgaatttgtc tgtttgtttc 193080
cacatgctta ggttcaggtt aaacattagc gggaatgcta cataagtgat actgtgccct 193140
ctcagtgtgt cacatcagga gccatatggt gttaatttat ctcattattg gtgatttaag 193200
cactgattac ttggttaagg tgatatccat caaatttttc taccttttc tctttgtgat 193260
aagtaatctg tagggagata ctcttaatat cttttcccca acaaattttc attcactggt 193320
aattcttgcc tgaattaatt atttatttga tggaaggaaa aattttctat tttttaccag 193380
ttgtaggttt ctgtttgttt gacctcttcc atactcaata ttgtcattgt gctttttttt 193440
tttttttttt tgaggtggag tctcactctg tcacctagat tgtagtgcag tggtgtaatc 193500
tcggctcact gcaacctccg cctccagggt tcaggcgatt ctcctgcctc agcctcctta 193560
ttagctggaa ctacaggcat gcgccaccat gcctggctaa tttttgtatt tttagtagag 193620
atggagtttc accatgttgg ccaggctggt cttgaactcc tggcctcaag taatctgccc 193680
acctcagcct ccgaaagtgc tgggattaca gacgtgagcc accatgccca accgtcattg 193740
ttcttttatg tgtttttaat tcccatgcca tttctcaacc catttttttc tggcttctgc 193800
cactaccatt gtctggcaac tctttcacta atgatttccg tggcagtgga cagttctcat 193860
ttcctctatt gattaatttt gtagccacat gtaatatagt tgaccatgcc caccttctta 193920
aaaaacatat ttttctcttg gcttttgtga attcacattt ttctggtttt ccttttactt 193980
ttttggctat ctcttttcag tctccttcac tggcttctcc tgcttctcac tctttaaaag 194040
ggccccctta gggctccatc ctaggctctg tttttatttt gtaccctctg tatagatcat 194100
ctgaatcttt ccggtagctt agattgtcac ttacgtgcca ttaatgttat aaagtctaga 194160
ttgaattata ttactatcta aatagtagtg tttctgaatg gagggagtat aggtgatttt 194220
tataatcttc tttttactat tattttccaa attctctaca aaaaatatat attatcttag 194280

tatacctcaa actcaaccta tccaaaatat tgttcgtcca aacctgtttt tcatttagta 194340
gtctctgttt tagtaattag aaccctaacc acttgattgc tgaaaccggt gctctgggag 194400
ttatccctga ctcttttgtc attgttgaga cagagtctga ctctattgcc ctggctggag 194460
tgcggtggca tgatcatagc tcattgctgc ctcacctcct gggttcaaat gatcctctca 194520
cctcagcctc ctgagtagct gagactacag gcgcatgccc tgtgcccagc taattttta 194580
tttttttta gagatggggt ctcactatgt tgcccaggct ggtcttgaac tcctagactc 194640
aagcgatcct cctgccccaa cctcacaaag tgctgggatt ataggcgtga gccactgtgc 194700
ccaggcctga ctctttactc tgcccttgtg acctgtcatg tagtcaagaa agtttagtca 194760
gttgtacctt ttaatgacct ctgtaactca tctgcttatt tgcgttgcta ccactctggg 194820
ccaagccaat atcatcccac ttttcactat actttttctt atattttgaa atttgaatca 194880
ttgacatgtt tttacctgat tggaaaaata ctcgttaaag atttttaaaa gtctagtaat 194940
ccataggcat tcaaaatagg taaccatcta cttagcactt atacttaact ttacagggaa 195000
ttgtacagat ggatattcaa ggaaaattaa aggcagtagt atcctgtctt ttatcagttc 195060
tgtttcaaaa tttagggtgt taaaaattac ttggtatgta acagtgagcc tgtcttgctc 195120
tgggcaactg tattcattcc agatctcaat gagggagtca atattttgct aaatgttaac 195180
tcagtctttt tttttttttt taatggttgg tataggtaga gatggaagat tttgatgcaa 195240
atatcgaaga acagaaagaa gaaaagaaag atgcagagga agaggaaagc gaactggtag 195300
gagacagacc aaccactttg aacagtgtct ctttattaaa attcttaaag aaggtttaaa 195360
ttcagatctg tggttagaca actactgttg atatacactt tagtggtaaa agtttccata 195420
atcatcttca aggagacttt tggatactct ttatttttcc ccagaccaaa agaaccctct 195480
aatgtggtgt taagagaaag ttttaacaga gtgctaagaa acttaccctt gtactcatct 195540
atttgtgggt ggttttaggg gagtatacct tttaagaaaa tcaattacat atgaaagggc 195600
attcttacat tccagcagat atgtggcaca gcctacccaa catatgcata attgtctctt 195660
ctttgctaca agatctccaa atttgagttt ctgtcaacag aggcagaaga acttattgaa 195720
tggggaggca gagggttgcc actggctgct agatttgttc ttctgcattt tatcctaagt 195780
gacgggggaaa aattaaatga ctgtgtaggc aatggatttt gctcaggctg attagaggct 195840
tacatagtgt gagtttaaag tttctgtgta cctaatgtaa aatgctttcg atcacacctc 195900
tgtagagatg tttgcctttc cctgtcattc aagaatatgc gacctataga gttacgttga 195960
cttttcagtg tttactgaag tacagagatt agcaatgcct tggaatagtg ttgcttttta 196020
aatccagaat attactcggt ttatttaata cttgagccct tgctaaagtt gctcctcata 196080
ttcttttgaa aatgtacaaa atgagaactc tgattcctac cccctagagt atacagaatc 196140
tccagggagt ggtcccatcg aacccgtgtg tgatatagag gactcttgat aaaaactgtg 196200
tttatataaa gatggtgtca acctctacat tccaagattt caaatgtgga ttaaaaaata 196260
agttttaaag ttaatggcat ttgagtctca gtatgtgttt tttttaaatc tagttttctt 196320
aagttgtatt ttggcatata taatttaaaa tgtaggcagg atattcacta caaggtccac 196380
gctcttctgg aatatggcta atgacttctt tagccatttg cagcgtgtgt actagaaact 196440
gattacgtga gaagtctata atgttttagc tttctgaagc tccgtctttc ttttgtggag 196500
atttcagtgg ttcataacat ctgacttgct taggtgtagg tcaaaacttg ggaactttta 196560
attgaatgta ctattacttt ttaaagtgaa acacactgta atactatatt tttacatcag 196620
agtcatggca aggaggcctt aaaattctac cctgctggat tatctgtttt atttccaagt 196680
ggcctacaat ttcaagcaat gggagagtaa tatagtttaa aggaagcaca ggatgattcc 196740
agcaggggga aatgaataca cttgtctgcc tgacttgatt ataaaacttc tcttaaccca 196800
ttcaaagatg cagaattgaa atcttttact attggagtcc attccaagag taaaagattc 196860
ttggagaata tgttaaccaa gggttaagag caagtttcat accaaccact cagatcattt 196920
aaatggaatt ctcagtaaac cttgcatttt ccactctgca ctaattaata tagctggatg 196980
aaatccaaag ttttgttggt aaagataatc gtttgtaggg gatcattggt atgatagttt 197040
gcctccttta gtgcttcagt gaacctagta gataaaattt ataaatatat tattccagtg 197100
atgcttaaag ttttcagatg atttttaaga tgtaacttgt cttcagtact tttagcacta 197160
tcacttcagt atagctttgt cttggtttgt tctaaaaaat gtatttggga aatatataat 197220
ttgtaatcca aaatactacc tagtaagttg aaattgaatt attagttctg gctggtagac 197280
tagaaatgta tgaaacccat aggattatgc tctagtaaac tcttacacag aagctatcat 197340
catgaaatct tcaaaatgct ttgtgactta ctttacctgt gtgtcagttt gtttcatgaa 197400
cttgaacatt cccaacagta gagagataga ttgattcata aaagaggata ctgacacctt 197460
tgatgaatct gtcctattta ctaggttcca atattaacat ttctgtatgt taggtctaat 197520
ggaaaacagt ctaaatgaag gtaaagggga ataatgataa atcacatggt aaaagcatat 197580
acttgctagg tgaataaaca gaaatgatta ctgcatagtt caataggcac actgggaaat 197640
ttgaaaatct tttgggagtg actttgaaat aagccaccca gttattttat gaaatataag 197700
atgtaagttg ccagtggaga tttctggttt ttcatgtaag aggaaaacat aattttaaaa 197760
aacatcagaa ataataatct agttttcaac tgataaaaat gttctgaatt tgattcagta 197820
aaattagccg ttgtatgctc taactggtaa atgaattgag tcattttaaa atgaagttga 197880
acttcctaat cgggtaattg ggttaagtgc caccaattct tttcctttct tttgagtact 197940
taagattact gaaatataaa gtaactatct tgaagaacac tttttgcatc ttatagctac 198000
tatcacttga gaatagcagt tgtatttaga agataaccta gctagtatta caaattctct 198060
gatactctga gtaatattaa tattagcaca gtaactacct gtacattctt atcatatttg 198120
ctatacagct gttgttcaga ctacaattgt gagtcctgaa ctgtagctgc ctcaaagcgc 198180
cttcatgata ctattctgaa gtgactactc tgaaatgtgg acagtgaagt ttggtgaatt 198240
tggtcattta gtttcttcaa agataatgag attgtgtttc ccctaaggat gtatttacag 198300
ctagtcagtg gaaactgaat ccattcccag ttttaatgct tttccagttc aaacgtgata 198360
tgcatgtatg tgtctctttc taacccttcg agcttctctc tgtgtacgat ttagggttac 198420

attccgaaaa gcaaatggga gatggacaca tctgaggcaa agctaggtgg gtatttcctt 198480
tttcctgttt ttatatgtga tacctgatgg taatggtcat agatctttct gctataagag 198540
gaacaaattt cctaaaactt ctacttgaac agagatttct ggcctacctt atatcctcca 198600
atctattttg ctagttttta tatcttgtaa caaagtgata agtattttaa aagaactgct 198660
ttcaatacta tttaaagttt ttagagtcaa cagaaatatt tgataattta gcaacaaaat 198720
attgtcactt ataaaatcta ttcttttata ttaatcagaa atgttggtgc tgatgagatt 198780
tctttttttaa agaatattta aatgcagtcc aaagttaaac ctgattatga atactttaga 198840
taataaaaat aatataaagt ttaaaattaa ttaaaaggca tttattctcc ttgtataata 198900
aatgagagtt tctgagtaga gaggagtaga tatataattc aggtttttga ggagcctgct 198960
ttataagatt cagtttaaat ttttcttcaa attgttaatg ccaaattcca aggtaaacca 199020
gtttaaatga ttgtctaaat cagaatttgg agttgcttaa tgtgaagtta tctgtcaaac 199080
aaaaaagtca tacatatcca atggattccc tctattattt gttggtgaaa actgcaaaat 199140
attttttcag ttttacttcg cttaaagaga accatttaac atgttgatta agatgtatta 199200
ataaaaatct aaaacaatgt gctaaggact ttttgagatc agccttactg atgtatgaag 199260
tatgactgtc agtttatgtt tcttttcctg gtattatact ccaaaactaa aggaaccatc 199320
taagtatctt aaattgatac aggaagtttg agtcactgag ctgcttcatt gcttgggaca 199380
tgtgtttctg acatcagagg agtgcatttt acatgcaatt acataagttg tgcctttaat 199440
ttgcccccaac tacttgtgtt agtagttggt attgactagg ttctgtgtta aaagacagtt 199500
ccacggactt tattattaca ttactggtat aggtgctttg ttgtcaagtc taaaagattt 199560
tttttagttt atgtcatgtg aaaattatat gtggtttttg acatattctt ctagtagtct 199620
tccctctgtg ggaaatctta ataataacca attgtcatct tccagagacc cttaaagcac 199680
ttatgtgttt aaatgcaatt aaatatgtag gaaaataaaa attacaaaat acttttaaaa 199740
taataataat gctttagttc tgcttatttc gacgttagat taactctctg tgaagttctg 199800
tacattagaa tgacccataa tatgccaaga tcatttctct cttctggttc tcttaaatat 199860
tgattatcct ttagggcaaa gagagatcat aaataaagat agttaagggg aatgtaaaag 199920
tgataaatat taacttgtaa tctctagagg cctcatgtaa tacatgatct ggggtaatgc 199980
tgttttaaac tcaagccagt ccatctttta cttttctttc tgtttttaaa aggaaaaggg 200040
aatttagttg tgatgattat tcaagaatat atattaatat tggtatttct tggaatttct 200100
ggaaaacatt ctggcccgtc taccatttgc ttctcagaaa aaaacatttt atacaaattt 200160
tcagttttaa agacagaata gtagacaatg aattccagaa atggattttt aaaacaaacc 200220
atttcagtga aaggcactga agatctggta ataaatctag gaaataaatt ttaacatatt 200280
tacagtcggg tttttatttt ttaacaagag tgatagtgat gttgtatata aggacttgta 200340
tcttgcacca ctgtattgag agctaatccc accacagagg aaggcaaatt tagtgtgcat 200400
tcttgaaagt gcttagtact ttgaggttgg tccattgagc caactgagaa gattcttctc 200460
taaaacatga ggatcttgtg tcggatcttg tagcctattt atgttgcctt ccttatcata 200520
acaaatttaa tataatttga ttaccaagta caatttattt ttctgagtac tttaccaaca 200580
ttctccaatt actcttggaa aatgattata ctgggtggaa atgtattaag ttttagcagt 200640
gtcacatgtt gagcaaaaac agttgaatct gaaacagagg aattttgatt cacaaatgag 200700
tctgccagtg agaacatact tacctatagt ctttgaagca gctggttaga gcttattgca 200760
gattcttggt ctatgctcat taagtttagt acacagttca gcctgtgcct gaatgttaaa 200820
ttggaagtga tgtgattttt agaacagttc agaacaggcc tgagctacct ctggcatgga 200880
gttgttgaag gctgcctgtg gcccaccttc agtcccacca gactggttct tccctgctgg 200940
gacacaggca tcataatgca agggcaggtt gttacttgcc acatcaggtg gtgggattca 201000
gcattgaagc tacttaaact ccagtgacat gattttagtt tctcatactg tgaagctaga 201060
atatttgaga ctgttaccat cctaatttag gtttcttcat ataaagtact tttgcttttc 201120
tcatctcagt gtcatttgtc tctactcttg agttgtatat tggttttcta catgtcagac 201180
agattcatct cttcagagtt ggaatataat tattatttgt ataggtgcat aatcttgtat 201240
gtttacgttg aacagatttt cagaaatacc atttacattt agaaaataca tagatgtatc 201300
taaggaattt ttctgttgtg ctataataca tactgtttca aatatgtggt aaaattctgt 201360
gacctgccat attggattta aaacttcatc ttcatcttaa aacttcatct tttgaaatct 201420
ctgaaaatca ttagtgtgca tgtattgaac accagtcttt attctgtaat taacacccca 201480
gatttctttc ccctcacctt atgccatcca tctgtgtgtt tggtttccag tatgccatgt 201540
ggaagaggtg tgagcctttc ttcagcccaa gaaggaaact ttaaacatat ttgcacaata 201600
aaatttcaaa ttaaacattt caaaaagggt gctcagacta gaaatacatg ctcttctgaa 201660
attccatgtt gcaactgtaa ctcctgtcat atatacccag tgtatgagga aaagttcttg 201720
cagttttcac actgcccttc tgtattgctg cctggctgtg ctctgttgtt ggaactgaaa 201780
tatgaaattt ttactttgaa gtatgttaat gtcaaagttg atcgtattaa gtttggaaat 201840
cctttgaggt ttatctaata agtgtgttgg agcttctgtc tcttctggta atactgtacc 201900
ctgttgaacc aagaacagtt ttattgtttg tgggacttcg ttggttttct aataccataa 201960
cctgtgtccc tgtgcagtca gggggtcact tctttaagat catgtataat acggcccgtc 202020
atatacacgt agatagagcc atgtgattcc agaaattaga agactggatc tgtggaatcc 202080
atacatgtta aaattttgcc aaaatgagat gattaaaatt tttgtgagtt ttataaactg 202140
ttgcagttcg ccttactgat ttttcaatga taatcacttt tatgggaagg gggcttagga 202200
acaaaaaact ttgccaagaa tgcaaaatct tactggtttt taaagcttgt aacagttgtg 202260
tgtaaaactt ttatatttga aacgtaaact caccctttct gccactgctt tcattgcact 202320
tttcatacca agttctctcc aacgtggtgt ctgaaagatt tttattatat acactcttta 202380
tggaattcaa tgaagtgtgg ttatgctgtg tttctgaagt ttttaggctt ttcttcattg 202440
gcctgcctaa tactagtgtg tttctataac ttcagatgat tcaaaagttt agtgcttcat 202500
tgtagcaaaa aatgtatata actcataata tcctacatgt agtattcaaa atcaattatt 202560

aataaccaat aaaagactca acacattttc attgcgtgtt cttctttaag acacctaaac 202620
tcatatctca taatttctga atccgcaatc cctattcatt aattgattac agtttttgag 202680
ttgttggaaa gcctagccct ctcagattca gggttcagaa agaattacca ggtctggtaa 202740
aattgtctga ctagccctta gcctcagaat ggtcaacttc atagtataag caaagaaagt 202800
ggtgatctca tatagtcagc tttttcatga acattaattc atggtgaatg cactcacagc 202860
aaccaaaatc caaaaaaaaa aaatgttcat ctaaaacctt aaacattagc ttggctcatt 202920
gagttcttgg tacaacctgc ttttcatatg acacagtatc aaacatgatt tcagatgaaa 202980
tgggtggtgt taatattgtg ttaaagaaaa aaagaatgga agacactgtc tagaaaagcc 203040
caactttgca tttgccatca gaaaatgaga actgtggtgg gtgggaggac aaggaggcca 203100
ggagccaagt gccttttctt gccttgccca tatttgctct taaaccaaca tcgtaaacat 203160
aatggctacc agtgtgaata ccaaacaatg gaaattgcac agaacttgat attatctcag 203220
ccaagaagat agtttggatc tgcaagtgat gctgcactca aaaatgtgga tatgccctct 203280
gaaagcgttc cagccttctt ttcccctgtg tgtgtgtcgt gtgcaaactc acactcttcc 203340
ttaatgctac ggctctgtgt ttgtccctca gacaagttgg atggcttgag gactggtact 203400
aaaaggaaac gtgactggga ggccattgcc agcagaatgg aggattatct tcagctcccc 203460
gatgattatg atactcgtgc ttctgagcct gggaagaaga gggtaagaga ctttgtcaat 203520
aactgccaac aaatgaaggg cccatttagg catctggttt taggaagtta caggcagatg 203580
atcacatgga tgctattttt agaatgattt agtttacgct atgtcatgta tcccaagaag 203640
gagtcatacc ttataaatta tctcatcaaa atgtacaagt agcttaaaaa attggagaac 203700
agaacctcac atggctagca cgttgtcaac cttaaaggca ttgggagtgg ccagtcactt 203760
gtgctatttt gtcatggggc aaaaaaagcc aggtacagag tagggtattg aagagaacct 203820
gtggaacttc ttaatggatg ggatgtggag ggtccaaagg tagatcaagg gtgacccctt 203880
ggtactctgg ccatgagcac tgcatgtgtg tagtaccatt tagtgagaga gaaaactggg 203940
ggagtcatag gtcataattt ttggaactga gagaaatgat ttaagttctg tgagagaaat 204000
tctaattttt cttctaatta agttagaaat ataagtgaaa atgtcaaaat gactgttgga 204060
tttattagta taaaactcac cagagagatc agactgaact aacaaatttc aggaaatggt 204120
atttaaaacc gtgggactga atgatcttgc ccaaggaaaa agtataaatg gaaaaggtaa 204180
taggacgcca gacctccaaa atttagttca gatagaggtg tttcaagaag gaaagagtat 204240
agtcagtttt taaatgatgc agaaaagaca agtaagtttg gaacagataa atggctgcta 204300
gatgttggca acatggaggt cactgatgat tgtagtttgt catcagtcta cagtgatgag 204360
gcagcagaaa ccagtttgga gcagattaaa gaatgaataa gaaatgatga agtacagtac 204420
agcatatata ggcaactttt tgaagttttg ctgtgaaagg gaatagagaa atgaagctag 204480
gatattagat aggtctttac ataaatgttg aattcgatag tgatagatgt gtatatcctt 204540
taaaacaaaa taaagcaaag ggatcgtatt atatatactg ttgtgtagaa gtctttttca 204600
gctaatccat cttgatcttt ctatataagc atatacagaa ctaccattct ttttaaaggg 204660
ctacttggtg tcccattgtt tggttgtaca atcagttatt taactagtcc ctttgtgtta 204720
gtccgtttgc gttgctataa aggaatacgt gagattgggt aattcatgaa gataaaaggt 204780
ttattttggc tcactgtcct gcaggctgta caagaagcat ggtgctagca tcagctcctg 204840
gtgaggcctc agaaagtctt caattatggt ggaagacaag gggggagcca gcatgtgacg 204900
tggcgagaga ggaaccaagg ggagggggtc ccaactcctt ttaacaacca gatctcacat 204960
gaactcagag tgtgaactcg ctcattaccg tcaggagggc accaagacat tcacgaggat 205020
cctcctccat gacccaaaca ccttcagtat cggggattac aattcaacat gagatttgaa 205080
ggggacaaac atccaaacca taatgtcttt actgatggat gcttacagtg tttccattta 205140
ttgccagtac agatgtgaag gttgtggtgt gtatgtctct ctttacacgc ttacatgcaa 205200
ataattgcct gtttttctac agcagtgctg taatcaaact ctttaacttt tgacaatctg 205260
ataggtgaaa aaaatagtat ctcatttttc atttatttac taagaatgag gttgtgcatc 205320
atttatcttt atgcatcatt tctttgtatt tctttcttaa cgaatttttct cagtgcctta 205380
atgtgcttag tgtacttagt aatgggcacc attacaaaga atccaggttt caaagctttt 205440
taaaatactg cagatactcc aaagcctgcc cttgtctgtg tagcttcaaa ggatgggtaa 205500
gggagagccc actgccattt gcacccagcc tcaccaaagg actgcttata gtgatacctc 205560
ttactccagt tttaaggctg gagccaacag aggcagaagt ggcccttcct tgcaaataat 205620
aaagtgtagt tctggtaaca attagtaatc taaaatgaga aatattttct ctgtttttac 205680
tcaggtaact atcactgtct tcactgagga gtatgtaatc ccatgcagtt tattttatta 205740
ggttgggtga ccaaagagaa ctgagtggtg cacattctag ttctttaaag ttgctcctct 205800
ttaagtctcc tcccctgcct tataccttga cttcagatag tttttaactc ttgaaccact 205860
caccaaaacc aacattagag cacaggagat cctacaggta gcaaagaata cagaatgctg 205920
tacattactc attccttctt gggtttcttt gtttctgtga ttcagcttgt tacagatatc 205980
agatgggttc ttgggattaa cttcctctat aacaaaaacg tggttctgta gagttggtta 206040
tattagaatt ttgcctaaat ggctaggctt taaaatataa tttactttta aaatatttgc 206100
ttgcttgggt ataaaatggt agcttacttg gactttaatt tgtatttctt tgatgactat 206160
ggaaggtgaa cctttccctt gtttgtttgc tgaatttgta tattttagaa atacttatag 206220
ttacagtgga tgaaatataa gtacctcaaa cctttgaggg ctagttaaga tttttctgta 206280
ttataacaaa tttattttta caggaaaaaa caacagcaaa aatgtcgtaa gtctcaaaag 206340
tacctctagg aaaattaaaa gggacactga tattcaagaa gccatttcaa gctgaaagtc 206400
attattctgc tccttgtatg tttagtggtt cggtaatcat ttattcattc catatgttga 206460
atgtctttgt acgaagtcta tgaatgagtc ctcttaagga gctgacatcc agtggaggaa 206520
ttcaacatgt aaataaataa ttttactata gcatcagtat aaattagaga tattaacaaa 206580
atacaatagt ttccaaaata ttttaattcc aacttcattt gttttttgtt tttttgtttt 206640
ttatttttga gacagagtct cactctgttg cccagcctgg agtgcagtgg tgattttggc 206700

tcactgcaac ctctgcctcc caggttcaag ccattctcat gcctcagcct cctgagtagc 206760
tgggattaca ggcacccacc accacgtccg gctaattttg tttgtatttt tagtagagac 206820
ggggtttcgc catgttggcc aggctggtct caaactcctg acctcaggtg atctgtctgc 206880
tgcagcctcc caaagtgctg ggattacagg cgtgagccac tgtgcctggc ctaattccaa 206940
tgttaaaatc ttgaacattt attcgcaaac atactactca tttcaaaaac acctacacat 207000
tttttcttct aatcctatat tctttatttt atctgagttt aaaacttaga atttaaactg 207060
tagatttaaa agtagcaagg tgagattgat cagtatattt aactatgtgc tgaggttgat 207120
aagtatattt aactatgtgc tgaggatgcc gtgggaatgg atttaaattt gctgtgggct 207180
gtttactttt tctttattca atgagcctta ccatataagg ggcaaactgt ggccactgaa 207240
caaagaaaaa gtaaccagcc cacagcaaat ttaaatccat tcccacggca tcctgagcac 207300
agtattgtgt tttcacataa atgcccaaga cttagttcgt aatgatcttt cgctaaaatg 207360
tgtatccttg atccaggtgg agctgagaga atatacatga attaaattta attaattaat 207420
tattattgag gtagggtctc tctgtcaccc aggctggagt acagtggtgt gatcttggct 207480
cactgccact tctgcctccc aggctcaaac aattctccca cctcatcctc cctagtagct 207540
aggatttcag gcacccacca cacaccatgc ccagctaatt tttgtatttt tagtagagac 207600
acggtttcgc cttgttgccc aggctggtct tgaacaccta ggctgacgtg atctgccggc 207660
ctcggcctcc caaagtgctg gagttatagg tgtaagccac tgtgcctggc tgtatatatg 207720
aattaatcaa aataggtact tcagcattga tgagcagaat gcccaaagat ttttctatat 207780
tgaagcccac attatttatt aggcttctta ctaaagccca tgaaagacat tgagccatag 207840
gttttctata cactaaaaac ctactttctg gaaagtcaga tgttaccagt ttcttcccac 207900
tgctattact ttggctcctc cctttgacca ggtgatactg ttaaaagcct agttatttca 207960
ttgctagtca tgcaaatggt aattagtgta cattagagca ataatgcatt cccaggttca 208020
gtgtaaagag catggtgttt ccatgctctg gttctctctc cttgtcacag ataataaatc 208080
acaagccatg tttaaaatac attctctgca gtctttgata ctgtgtagtg caagcaacag 208140
attcatagtt tctttttaag aatctgaact tttacagaat atgtatatct ccatatactt 208200
ttaaaaacat attaacataa tatgtggctg ggcatggtgg ctcatgcttg taatcctagc 208260
actttgggcg gccaaggcag gaggattgct tgagtccagc agtttgagac cagcctgggc 208320
aacatagtga gactctgact ctaaaaaaaa aaaaaaaaat tacaaaaatt tttaaaaagc 208380
ataatatgta agaatatata tatattaaaa atcagaatac caataggaaa tatccctgat 208440
ccatatattt gtagttgtaa tcttgccatg aattatatca gttgagtctg tttcttggtc 208500
tctttatctc atgaatgaaa gtaataatac cagcttactg ttttctctaa gattaaattt 208560
caagaaatat ttttgtgtag agataaataa aattcagtta ttgatctagg gtgttgatag 208620
tctgttaggg aagacagatc tgtaagtgga taattatact acggtgtgga agttcaaaag 208680
cagacatatc aaaatccgga agttaactgt ggaaagcagt gattatttga ttggcaaagg 208740
aaaaaggggt aagtcaggga aggatgacta gactcatgcc aagtggataa ggttagggaa 208800
aagacatttc aggaagaatg actagtacat atatgcagag ttatagagac atgaaatggt 208860
gcgatgcgct ctgagtgcta gaaggaagtc tgtattgttt gtataaaatg ccataggcac 208920
tggccagaga tgttgcagga aagtgtagtt ggggccagat caagagggac ctgagttctt 208980
ggactttgcc tggtaggtag gggaaggagt taaagcaaat gaatcacaac tgtgtgtttt 209040
aggaagatca ctctggcagc atgaatctgg agcgggtgag tacagacagg aagactaggt 209100
agagattagt gctttgcttt caggtgaaaa aaaaatgagg tcagtgaatt attaaataaa 209160
aaggaatggc agaggagaaa gagaggacag gattgagctg aactgttcag gagctggaat 209220
ggttaggact tggattgtcc agagagtttt ctctttgtga cttaaaatat atggcttttc 209280
ccagttttcc tgggttagtt tttaattttt taaacttgcg gtttcaaatg agtcattcta 209340
ccccttagac tgtccagaga gggagggggc tctttgcata tttatttttc ctaaagctca 209400
gcctaatgct atgttctttt taggtcagat gggcagacct ggaagagaag aaggatgcag 209460
ataggaaaag ggccataggt tttgtggtcg gacagactga ttgggagaag atcacagatg 209520
aaagtggtca cctggctgaa aaagccctca atcgaaccaa atatatatga gacttagttt 209580
ttgaacggag tcattattcc tctaaggtaa gagtacacag tcatataaat agcctactgt 209640
gtggttgctt caaagggttt gagataaaga agacagcata ttttgctaaa aatgctctct 209700
tcttgtgtta ttttatagtt ctgaaagagt aagtatgtac tagttgttaa catttacaat 209760
actattagaa aaggaagcca ttttctgcaa gctggctcac ttataaaaat aaaaaccttg 209820
agaaaaactt gaattctata gatttgggaa ggactgaaat gatgttatac taagggctac 209880
tgaacttttt gcctgttctt tccaggtgtg acagcctttt cttttctttc caggtggttc 209940
gctttgaggt ggtctgaagc caaggcctcg cggagcttct ttgtgtgtca ccttgcttcc 210000
acgtttcagt tcttgttttg tttctactgc tttagttttt tttaaagttc tccagtgtcc 210060
ccaagaggta ttagaatctt gctgtaccca agcaagacgt taatttttct tttaactgtt 210120
ttggggaggg agggagtgat agcttaactg ctgaagccag gcgggggtct gctggaggat 210180
tccaacagag agtatttcct ccactgtaca atgtcacaga ctatctctat catcattgct 210240
ttgtggctgt ttctgttttt tactgtatgt aactggtagc tgattgtact aggattaaaa 210300
acaataaact ttcatgataa agccgatgag attcatgggc tatacagcat gggccctttt 210360
ctcttgtttt cttaatttta tttttaattg ctttttaaat atggtatgtc ctaattaaat 210420
gctagctgaa catcccaaaa ccttttttttc attgaatatt atatggctca atgattccgt 210480
agcacatgtt gtaaaacaca gggttcaaat gatgttcaaa acactcatct ttgttttata 210540
gctgatttct ctatctttgt gtcttgaaga ctgaccaagt tcaaatattc atcagttccc 210600
tggagcatat ccattctgta acatgatgct ttatttaaaa atttttttttg atcctctctc 210660
tgttcttccc tctcttcttt ccttccttct tcttttcact ttctgccttt atgctgttag 210720
gtttttattt tttcctgatc tggctttgaa ttctggtaga cagaactgat gtattctgtg 210780
gaactcaagc ttgttgcctt catttgaact tgaattgtgg atttttaaat gaagattgca 210840

gggaaagagt ataaagatgc agagcactct ggcatgtaac ctttaagcct gtcaggtttt 210900
caagttcttg ccagattgtt cattactgga aagcatggcc ttctgcacag aatttcctct 210960
cttgtggtta ataccaggtg gaacccagaa acatacagag ataaaaccca aatattattt 211020
ctatgtaaac acagaaaagg gactcttccc tttttcatcc tgcaaagaaa aaagtcatct 211080
tttagcatga aagaagatga gagagccttc ttcctcaagc ataatttgca ttttgtaatc 211140
caactcagta atttgtaaat gtgttcactg aagacttcaa tattttgaaa tattctcttt 211200
caggccacag atgaaaatcc tttactttta tgacctacat taaagactgt atgaatagag 211260
ctaatagtta ctagcatttg aatagtatca ctttgggttc aacaaattga tattatagta 211320
ctgaataccc ctaatttaat ggaaattcta acaactttct gtacttttat cttccctgaa 211380
aatctgaatt tttaagcaag tgactttaag ttcattaagt caatattgaa tgtataaatt 211440
gctacattaa ataacttctg ctgtttgtag tatttaataa gctattttca ttgcttattg 211500
gcctacaata catttctgtg tactgacctt tctcagcagc ctttattatg tagagtgaag 211560
atatgtcgca aaaatagcca aatagaatta ttgtttgtac aaagactcat ttactacttt 211620
ctaaagccac ccaatgccta ttatcaaaaa caaaacttcc ataatgtgtt ctctagttca 211680
gatgaaatgg aggccgatat gttcttctgc aggatgcttt atttcagttg tccttaacct 211740
tgtgatgttc tgacagtagt tagaatgcca atgaaatgag tggtgattat ccgtgagtca 211800
cttggaatca tgggtaattt tgcatatgtg tgttttcacc ttacagccat ggctttatgt 211860
ctgtgctctt ataccagggc agggtgggaa gctggggaag agttaacata gtcagaaacg 211920
gcctctgcca attttcagaa gatatagatt acgtgacctt agagaatgaa catagagtgc 211980
tcctacttta gaaaatgggt ataacaacct tcatgcaagg agattaatga aaaaagattt 212040
ggcaccaaaa agagtaatag agacagccag ctgataattg attttgctag tccaatgtgg 212100
gacttaacaa aattcttgca cttacattac tattagaagc agtatcgtat aggaagccca 212160
aggtatcttg aagaagaaat ttggtgtggg tagaaaatgc cttgataata caaatatttt 212220
aagtttgtta agaggtgtcc tggaaatgca gaggactaat tttggattcc attatttgct 212280
agcattttat tttaatttta ttaacattgt cttctcttag aaagttgctg gtttaaatca 212340
tatgagtttt attttaaatc ttttgtggtg tcatgttact taaaataatc ttatgttggg 212400
aaaggtttct gataccttgg tgggtatttc agcttttttt ttttttttgt cagctctgta 212460
aaggatgatt tcagacattt taaactgaaa gtgaaatctc tcacttaagt gatctaactt 212520
tacttgtaaa cttcctgtgt gaggaattca gtagacgaga cccagaaatg tttctgtttg 212580
agaatattta gagttaagtg ccgtatttga aatattcagg gactgattgt agaatcagaa 212640
gtcattaata ataatacaca agtagtctat tgacagttga ttcatataat tttctagaag 212700
tttgagtttg attcagatat cagtgttaaa tccagaagtt tattgacaaa gataaaatct 212760
taaattttct ggttggattt gttttcctcg tgaaagagtt ttgatcattg agcaaaccaa 212820
agtaggtgcc atgtcatcta gagatcgact attacatttt ttattagatt gactaagaat 212880
ctcaactgag gttttcttat tttgtaggcc cctcagtttt tgtgttaagt attttatcct 212940
gctaactgtg caaattgcag atattttggt aaagatgtct agagcctggc ttaattacta 213000
ggattataaa agcaacagat tctcagctga gataagtttt gctaagccat agaaggagaa 213060
tgatttctca tttttagtat tcacagaccc taatacaagt tcagattctt ctagaagctg 213120
tatgacctta agcaactttt tttttttttt tttttttttt ggagacaagg tctcactctg 213180
ttggctggag tgcagtgacg cgatatagct cactgcagcc tcaacttcct gggctcaggt 213240
gattctccca tctcagcctc ccaggtagct gggactacaa gtgtgcacca ccacacccag 213300
cctttttttt tttttttttt tttaagagat tggttgttgc catgtttcac aggctggtct 213360
tgaactcctg ggagcaaatg atcgcctatc tcagcctccc aaagtgctgg gattataggc 213420
gtgagccacc gtgcctggct gaaatatttt aacctatctg agcttcagtt tcctgcctat 213480
aaaatgaaaa taacaaaaca cacctattaa acattgagat aatgcatgta agctacttgg 213540
cacagtactg agcacatagt aaaagctcaa aaatcagtag tcaccatcat cgttactttt 213600
tgacttgttc tcatgtgttc tttttgaaat tcaaaatatc cagctggctt gaagatagtt 213660
gtcacgtttt atagtcagct atcatagact gagctatgtc atatgtaata gtacagtgtg 213720
ttgaaagagt aattattcag aagcattttc aggtaaccag tgacattgat tttcagaact 213780
agtacggagg cttgagaggc tcaggtgtaa aaggtaattt agtacttaga gggggttgag 213840
tagtaacaga aagtagttgt cagaccacag gtataaacat aggtgagggc acagaatcat 213900
tgatttgcct tgattttcac atattcaaga agaaagtaac tttgtgaata aataatgcgt 213960
tcataaaggt ataaaactca ggaggttcaa gtgtcccatc catcttaacc ctcagcctcc 214020
cacgactcca tctgtagagg caaccagtgt tgagaaaaat tgtgtggata agcattaagt 214080
attaatgttt ttacctggtt tttgtacaca cactacctta tttttccttt accagtatgt 214140
tttagaggtc attcagatca tatataaaat attcacattt tctctccccc ccacctcctt 214200
ctttggtttt ccaaaggtac cttcataata taaagaggtt tactgaaaaa tttcagaaat 214260
tgtttcagct gcatctctgt tcttttcgtg gcatggaata catggtatgg gtatatttgt 214320
atacaattct ttcatatatg taggttggtg taaccaccct ccacctttct cctagtcccc 214380
caaccccacc atctctaacc cttggcagcc actaatctgg tctccatttt tataatcttg 214440
tcatttaaag aatgttataa atatgaagtc atacagtatg taacctttttg ggattggcat 214500
tttgctttt ttcactcagc gtaattttct tgagattcat ttaagttgtt atgtgtgtca 214560
atagcttatt ccttttttatt gctgagtagc tttccaactc attctctttt gcagctgcag 214620
aatgttttat gtctttacta tcattttaac ttacagagtc tctattaatg ggtattcatg 214680
ctctcgttta ctgttataaa ttatgaattt cacatatact cagtcattct gcagcttcct 214740
gtacttgcct gatttctagc aatgaaggaa aataccgatg gcaccatttc atagacatac 214800
tcaaaggatg acaagcactt aaggaccagc acagcacagc ggtgaactct caagacagta 214860
tgacagtaat gagatgacga aggcacatct tgtttcctgg aaaattctgc atgataggag 214920
actgacaaag caaagaacca tgatgtgtct tagagaacca gaatggtcag agactctgat 214980

gatcccctgg tccacacgtg actgagagtt aataccttag gtcactgttc tcagaacctg 215040
gatatttata tgcacgtgca cctcagtgac tgatccattc tcagttttca ggtccattgc 215100
tttcacattt gaaatagtca aggagcatct ttatgactgg ggctgagctt ctgcattcat 215160
gtacagccct ctgcctgcca gcagtgcctt gcatttgtga tcctacaaag agcctgtgga 215220
cttaatggtt tactgtatag ggaaaggctt gactttgtgt cttctactta agaagcttga 215280
actttttacc aaatattcgt actccttttc ctgaaaatac tgatctaggc tacagatcca 215340
tctcttaacc atatttgaaa aattgaaata atatcttctg ccataacttg aaattccact 215400
ctcagatctg ttatttctga cagtgagttt gtatttctca agtagatatg tctgatgctt 215460
atttctttta cagcatctgg ctcagtgcat ggctcatata tctgtagagt gagtaaatac 215520
agggaatgtt tcctatgtgt atggcaaggg tcacagactc aggcaggtaa cagatgagca 215580
aggcagaggg tggagactgg taaactagac aactagatag gtcaggtcaa tcgggggcag 215640
cacctgctca gccatagcag attgtggcca tgtgggggata taggtctcat attgccagat 215700
ttcctgggtt ttcaagaaaa agtgaaaaat ctggattttt atgtgagatc ttccagtatt 215760
tcagcatcag gttctttttg tttgtgagtg ctttggccct cacagcctgc ctgccacatc 215820
tgacttgtgg actgccggct tgaaaccttt gatatttaga gaagtggtta gaccttagag 215880
ttggttctgg tgagatcctt cttgttttac agatgaggtt tctgagaccg aaagaaggga 215940
agtgacttgc tcaaagtctc actgttactc agtggcaaaa caagactaga atttgggttt 216000
cctggtccat aacctgtcac tttctttttt cttaactaca atacctcctt tatgaagacc 216060
ttcataaatt ctaggatggt gaattctcct ttattttgtg tgtcgtgtgc aagtggacat 216120
gtgtgtattt actaaactct gtggacctgg aaatacattc tgatatgact atcaggaaca 216180
atgtgattac ttctgaaacc aacttcctta aactctcatg tataaggtag ctatacacct 216240
aggatccctc ttaaacactt actacttggt tgggtaattc tcatgaataa cagaagcttg 216300
aaaattaaca gagtattgga ttcagtgatt gtacaagagc agtggagttt tctccacaaa 216360
gtagagaaaa tcatttgtgt attgttatgc aaatagctta cagaactatt aataattttg 216420
ttttctttaa tttgtaattt cttatgtaac tagtccattt gctttcaccc agtgatgagc 216480
atcttacctg cttcactcgg tagcgctcca ttcctaatct ttttaaggac aaattcatca 216540
acttggcgtt aaagtctatc agtaacatga tcccagctac ttctccagat tcctcttgag 216600
ctgtccttac tacatgtgca ctacactgca gccacactgt gtgacttatt ccctggggtt 216660
tctattgttt ctgccttgct tttctcatcc tttagatatt agcattattg gctgggcaca 216720
gtggttcaca cttgtaatcc tagcactttg ggaagctgag gcaggcagat cacttgagcc 216780
caggaattag agaccagcct gggcaacaaa aatgtcggct gcagtgagct gatactgctg 216840
tccaccctgg gcaacagggt gtgagacctc atctcaaaaa aaaaaaaaaa aaaaaaaagc 216900
caggcacggt ggctcatgcc tgtaatccca gcattttgag aggccgaggc aggtggatca 216960
tgcaaggtca ggagtttgag accagcctgg acaacatggt gaaaccccat ctctactaaa 217020
aatataaaaa ttttcagcta ctcgggaggc tgagccagga gaatcacttg aacccaggag 217080
gcagtgaccc aagattacac cactgcactc caacctgggt gacagagcaa gactccatct 217140
caaaaaaaag atcggtggtg ggagaaactg tccaaaggcc cagctcacat ggtactgcgt 217200
ccctgaatct gttcctcatc ttgttccctc ccagctctca caggctttgt ttgtacatgt 217260
cctaccatct tctcttaatt tgcctttcta gtagagtaac ttatatatcc ttcccccccca 217320
tccttaccca cttacataca agctcctaga aggtgagaat tatgttttac tcatcttcca 217380
tccttgtaac acataatatg cagtatctgt tacatatagt tgctactcaa gaagtatatt 217440
tagaattgat gacctggtga tttttaagta taaaattaat tcatgtaggt tcatatatgt 217500
ataatttata aaaccaattc ttgtgtttgc tttttcccta cctctgtatc ttccagctgg 217560
tggcatatca atacttaagg cgtagcacat tcaaatgcct tcaaaggaca ggaagataag 217620
tgtaaatgag agaattggcc agtaatttca taatatattt agttttattc tttcaacaga 217680
aacttacttt gtttgcagat tagacatgtg ggaatattct tcactcttat agaaaaatac 217740
cctgtttctc attctcaaaa cttttggcac tctcttagtt ttcccacctt tggtagagac 217800
ataagtacag tattattttt tctttactgt gagaaaaaca acagcagaag cagaataaaa 217860
cagggtactt ggccctctca gcctcagggt tcaggaagca gtagggtatg gtggaggctg 217920
ggatggtgca gagtgcttgt cctgtctcag gggacaattc tccagccagt ggttggaatg 217980
aagaaagaca gacccaatgt tactagatga agataaagtg gaagtctgca tttttatgtg 218040
aaatagtcac agtagaaaca aatcacatct ggaagccaca tgttaccaat aggctgtcat 218100
tttgggactt ttctgacatg ctctaaagga tgatgacaag tctgaaagac agttatggca 218160
aagaagatat ttgtaagaaa ggaaacatca ggttttatta catataaata ttttgagcag 218220
caccttttta ctagtatttt ggaaaagcat ttctgtcttc attgagcgaa gtggcaatac 218280
atatgtaata tactgatgct gaatttgcgg tgggttgatt tttgtccatt gttggctttc 218340
acatggaagc atgaatattg tctccccgct acccctttttg agtgacatcc ttccagtacc 218400
tccatttctc aagttgcagg taagcacctt gtgtatatac agttaaatag tgggcacaca 218460
tctatcccag gaaaagaaga cagtgagaat tccctttggt taagtggagc agttgaatgg 218520
ccactatacc tggatgcaaa tttgatccga attttctgct ctaaaccctc cactgctttc 218580
tgctgtttcc actgaggaaa gaggatgtgt gtcattaaag ctttgttggg acctgtcaca 218640
gtcaaggtgg gtataattat gtttaattta ttgaagtgtt tctgggcatc gtctttttca 218700
aattcatata ttctatccag aagatgtgaa ttcattttca ttattaaaaa aataaaatat 218760
tacagataaa gctcaacact cccccctccc ccttaagcac aaataccttg aatcttagag 218820
gtaattacta ttatttgga gtgtggcttt tccaaacctt tttgtgtgta tttgtataca 218880
tatgtacttg cctataaaaa tacagtttta ctgtttttta catgactgat attctttaca 218940
tactgttctg cagacctact atgtcttgga tacacacatg catgtatata tgtatatata 219000
ggtaaatata tatataggta gatataaata catgcattca tgtttgtgta tattccatga 219060
tatgtgtata atggcttact tttccttttt tttgtgagtt tttcactgtc ataccagtgt 219120

tgcattgaac atccttgtaa atgcctttg caggcttatt ttcaaaaatc ttccctgggg 219180
aagatacaga agaattgaat tactgggcta cagggaaacc atttctcttt ttattttctc 219240
ctaataatta tctacatgtg tgttttagaa acagtcttaa tttaatattg gggggtttcaa 219300
aattttttt ctcattttaa aattccagtt ttatatgctg tactcatttg aattctgcct 219360
tttgaatcta gatttggaat tgatatggat aagcaaaata ggttctaagt ggtagtgata 219420
gttcataacc agtttgcttt tggaataggt gctgtttcaa tcttggggaa ggtaaaatgt 219480
caattgagga aataatcttt aatcatgaag ttcttttttc caagtcattc tctgctccct 219540
ttggaattat gacaacttgg atccagaggt gctgtgaaaa tattttctta catcatacct 219600
cttcttttca tacaagtttg aatgatcccg gaaaatacag tttttgtatt ttcaaaaaaa 219660
aaaatgaagt ctggaacacc cacttttctt aataggatct agtttatttg tttaggtagt 219720
aaagctttta ttaaaagaac atactttgag gatttctgt ttctcacctg agccttctgt 219780
attcactaga tatgaaactg agtgcattgg cagggcctct actagacctg aaaggcagca 219840
ttcccagtgt ttattttcag gggccagagg gaaggctatt gactgacatt ggaggcctta 219900
acccctggat tggtaaggtt ctggtggaat gattgcttag attccataaa cactcgaata 219960
gatgattact cctttctgta tgtatgcatt tttggaagaa tgataaaata tgaaaagtgt 220020
gaagagttgt gcactcaagt ggtggttttg acctggggag cttttaaaac tcccaaagct 220080
caggctacac ctcatagcaa ttaaatcaga atctaggagt gggacccagg ccttggtaat 220140
tttttaaaag gccaccaggt cattctaata tgcagccaac tttgagaact ggtggggtaa 220200
aggggatttt aaattttatt tcatctctag ctgatgatag gttcattaag caaaactttc 220260
gaaggccctg gtctgttggt taactttgca ggtgtggaag atacagttgt tggaaggata 220320
cttgttaatg aaactcttta aatgtgcaaa agaaaatcca cctaggctga aagagttaat 220380
gcggtttcca aaggtgcttg atagccaaga aacagcttag gcacagggac agtctggctt 220440
cttgggagca aataaagcca agatttaaaa tgccttttaa tctaaaacat atgtttgccc 220500
gtgggcctgg aaacaagcct aggcctttgc agttgtgaag tttcctcacg atagacagac 220560
atgagttgtg gtccaatgtt atttaaactt gaaagcttag aggagaaatt tggtttgtgg 220620
aggaggttgt agatgggaga agagaagtgc agtgggcagg cagaacaaat tatttttctg 220680
tggtttgtcc tttctgattt taagaggaga actagttcat agttccagtt atcacataca 220740
aaatggaaca aacctcaaga aaatgagaat tgctattttc aataagctag aatgatctct 220800
ttaaattaat ctgctttgat acctaagata atctctattt gtggcataag cgtccttata 220860
aatgcattct atggtggtat ggactgtttt gcgtcttctt tagagtacgt gactccttca 220920
ttagaacttt tccccaaaga ttctgatttc tcattgtttt ttaaattttt attttgaaat 220980
agttacagat tcataggaag ttgtagaaaa aaatgtacag agaagtcctg gtgtattctt 221040
caaccagcca tccccaatgg taacatctta tacaatgaga agtgtgtgtc aaaactttta 221100
aaagccaaga agttaatgtt gatacactcc acagagctta ttcagatttt accagtttta 221160
caagcactca tgtgtatgtg tgtgtttggt tctgtgcagt tttatctaat gtaacttcat 221220
gtatttgtgt gtgtttggtt ctgtgcaatt ttatctcatg tagcttcatg taatcaccac 221280
caccataatc aagattcaga actccatgtg ggaatgtgaa gaactgttaa gagaatgagt 221340
atagaatgag tcactttgct tgtagtccca tcatgactta aattcaggac tttatattgt 221400
tggggactgc tgctttgagg gggtaagtag catagctttt gcaacataaa gtggcttttt 221460
agtttttttt ttttcatgag atcatcaata ttggtgcttt tctcagtaac tacgcttttg 221520
agtagttcaa gcatggccac ttgctttatt tgaagggagt aaaatgagaa gtatatgccc 221580
cagagcataa gaatctgttg ctgatcttaa tttttggaca aattgaatgc taggaagttc 221640
tttttctgcc aaacttcaga acatttgaga cctgctcagt aaacagtcat tgtatatttt 221700
tagtcctaaa acagtaagaa gactattggc ctcccagaga aatgccctga tgtatttgaa 221760
ttgaataggc atggctttat aataaagaaa tagtaacagt cactttaaat agcatattct 221820
aagcattaag tcaacctgtc aaaccctgcc tcactcacat tttttaggtg attactgtct 221880
ccagagcata aggatacata atcatgtcta ctgttcacat ggcataagga taaatcctca 221940
tggataggat aatttaaaaa ttatttttgc aatagtatgt caaaattaag gtgtttagga 222000
aagtccattg ttattaaact ttctgttccc aggacttgct gataaattac aatcaacaaa 222060
acagtttgta ttctgttctt gtacccacat ccccctgtct accttagaaa ttcttttttg 222120
gggcaccatt tttggcaaga attctatagg aaataagagt ttctggagtt tgttctgatt 222180
ggttcagagt cagcaagctc agctctgatg aagtaatggg atgtgtgaaa agaagtgtct 222240
aaacaccaag ataggaatat tgagcaaaag cagctttggg gtgtatatca aaaatgcatt 222300
ctgtctgatg taatggtgtt tttttgttag aaatttaata cagtaagaag tcagcaaaac 222360
ttcatgttga caatcccatt gtctcataaa gcttgatttg aaaaagacaa tgtagaaaca 222420
gactatccag aagacattta gagccttaaa tattctgata atcaccaacc cacactcatg 222480
gaagagcaga ggagaagata atcaacatta gtgactataa atgaaaagaa ccagaaaggc 222540
tggattactt tccatcaaaa aaagtaggtt gggtgtggtg cctcatgcct gtaatcccag 222600
tgctaggagg ctgaggtggg cggatctctt gagcccagga gttccacacc aaactgggca 222660
acatggtgag accccatctc tacaaaaaat agccaggcat ggtggtgcat gcctgtagtc 222720
tcagctactt gggaggttga ggtgggagga tcccttgagc ttaggaggtg gcggtttcag 222780
tgacccaaga ttttgccact gcactccagc ctgggtgaca gagcaagacc ctgtatgcaa 222840
aaaaaaaaaa aaaaaaaaaa aactaatctt tatgaaaaaa taatcctgaa tatagattta 222900
actgttaaga gggggggaaaa ccaggattcc agttatgttt ccctcgccac ccctgaccca 222960
gtcaagaagg caatgaacca gccagcccct tttaacaagt ggaacaacta cagagatgtt 223020
ttgaataact gtacagaagt taagaaaata ttcaagccaa aactagtcta ctcaatgaac 223080
atagatacag ccccaaaaag cagtggttag agatggcaag ttgcataatt ttagaaatac 223140
cacattagtc actgagcttt taattaagag gatgtcaaaa atgcagtgta ggccaggtgc 223200
agtggctcac tcttgtaatc ccagcacttt gggaggcaga ggtgggtgga tcacaaggtc 223260

```
aggagctcaa caccagcctg accaatatgg tgaaacccgt ctctactaaa aatacaaaaa 223320
ttagccaggt gtggtggcat gcgcctatag tcccagctac tcaggagact gaggcaggag 223380
gatcacttga acctgggagg cagagcttgc agtgagccac gattgcacca ctgcactcca 223440
gcctgggcca cagagcaaga ctctgtctca aaaataaata aataaaaaaa aaataaaaaa 223500
aatgcagtgt atccccctgt gtaaggaaaa agcaggtcag cgtctgagca accaaccaag 223560
aaacagcaat agtcgtaatg ccatttgtca aacatgtcaa catggggttg tatttaaaaa 223620
gtaaatgacg aaatgcatgt aaactttata acacaaaggt ttttagctgg gttttcttcc 223680
caaactatat taacatagtt taaagctaag ttttatggtt gtgtccgtgt tacatgtcag 223740
tgatgttgtt aagctaagaa ttctttttca ctgtgaagca gtacaaattc atgactttgc 223800
taacaatcta tgactgctga gcagtaatac agctaacaat tactgagcac atactatgct 223860
ctagaagtgt gttgagcatt tcacatgtat ggttttacct gaaccttaga acaatattat 223920
gaggtaggtg ctatattatt atctctgttt tacagatggc caagctgagg ccaagaagag 223980
taagtaattt agagagatcc gtagctgata ggtggttgag cccggatgaa aacacacccc 224040
aaagcccatg cacctaacaa actactcgat ttgggaaagg tattaggtga ggtggaaagc 224100
aaatagggtg ggtgaagcat tgttaggtca gatacctgag gataaagtgg ggaaagggta 224160
aagggaggaa caacattctt gccgacagct gaagctccag tccagaagca gtaactgagg 224220
gcatggaagt cagagccaca gaaatcagta aatagggggaa agagaaaggt tttcctaagg 224280
gaagaaaagg aattgacatt tgtttgaggt gtgactggag aattggaaat ggcaaaacaa 224340
gagtttctga gcatgaaggg gtgagatcgt gagaaatgaa atcaccctga agaactcttg 224400
gtgaaagaaa agcagacccc tagtgagcag atggacatgt aaaactggcg acggtttggg 224460
ttgttccttt actgtgttca ttgttcacct gccttcaaca tttcatcaga cactcccttc 224520
ctctggaggc tagcatctgt tttgcttttt tttttttttt tttttttaa ataggactga 224580
gcattcaact cttaggcact tggttatata tagtacttag gttattgaaa agagggccga 224640
gtgtggtggc tcattcctgt aatcccggca cttttggagg ccaaggcagg cggatcacct 224700
gaggtcagga gttcaagacc agcctggccg acatggcgag accccgtctc tactaaaaat 224760
acaaatatta gctgggcatg gtggtgtgca cctataatcc cagctacttg ggagactgag 224820
gcacaagaat cacttgaacc cagaaggtag aggctgcagt gagccaagat cgcatcactg 224880
cactccagcc tgggtgacag agcgagactc catctcaaaa aaaaaaagct t          224931
```

```
<210>  116
<211>  3244
<212>  DNA
<213>  Homo sapiens
<400>  116
gcggccgccg ccatgtcggt gctgggcgag tacgagcgac actgcgattc catcaactcg     60
gactttggga gcgagtccgg gggttgcggg gactcgagtc cggggcctag cgccagtcag    120
gggccgcgag ccggcggcgg cgcggcggag caggaggaac tgcactacat ccccatccgc    180
gtcctgggcc gcggcgcctt cggggaagcc acgctgtacc gccgcaccga ggatgactca    240
ctggttgtgt ggaaggaagt cgatttgacc cggctgtctg agaaggaacg tcgtgatgcc    300
ttgaatgaga tagttattct ggcactgctg cagcacgaca acattattgc ctactacaat    360
cacttcatgg acaataccac gctgctgatt gagctggaat attgtaatgg agggaacctg    420
tatgacaaaa tccttcgtca gaaggacaag ttgtttgagg aagagatggt ggtgtggtac    480
ctatttcaga ttgtttcagc agtgagctgc atccataaag ctggaatcct tcatagagat    540
ataaagacat taaatatttt tctgaccaag gcaaacctga taaaacttgg agattatggc    600
ctagcaaaga aacttaattc tgagtattcc atggctgaga cgcttgtggg aaccccatat    660
tacatgtctc cagagctctg tcaaggagta aagtacaatt tcaagtctga tatctgggca    720
gttggctgcg tcatttttga actgcttacc ttaaagagga cgtttgatgc tacaaaccca    780
cttaacctgt gtgtgaagat cgtgcaagga attcgggcca tggaagttga ctctagccag    840
tactctttgg aattgatcca aatggttcat tcgtgccttg accaggatcc tgagcagaga    900
cctactgcag atgaacttct agatcgccct cttctcagga aacgcaggag agagatggag    960
gaaaaagtca ctctgcttaa tgcacctaca aagagaccaa ggtcaagcac tgtgactgaa   1020
gcacccattg ctgtagtaac atcacgaacc agtgaagtct atatttgggg tggtggaaaa   1080
tccacccccc agaaactgga tgttatcaag agtggctgta gtgcccggca ggtctgtgca   1140
gggaataccc actttgctgt ggtcacagtg gagaaggaac tgtacacttg ggtgaacatg   1200
caaggaggca ctaaactcca tggtcagctg ggccatggag acaaagcctc ctatcgacag   1260
ccaaagcatg tggaaaagtt gcaaggcaaa gctatccatc aggtgtcatg tggtgatgat   1320
ttcactgtct gtgtgactga tgagggtcag ctctatgcct tcggatcaga ttattatggc   1380
tgcatggggg tggacaaagt tgctggccct gaagtgctag aacccatgca gctgaacttc   1440
ttcctcagca atccagtgga gcaggtctcc tgtggagata atcatgtggt ggttctgaca   1500
cgaaacaagg aagtctattc ttgggctgt ggcgaatatg gacgactggg tttggattca   1560
gaagaggatt attatacacc acaaaaggtg gatgttccca aggccttgat tattgttgca   1620
gttcaatgtg gctgtgatgg gacatttctg ttgacccagt caggcaaagt gctggcctgt   1680
ggactcaatg aattcaataa gctgggtctg aatcagtgca tgtcgggaat tatcaaccat   1740
gaagcatacc atgaagttcc ctacacaacg tcctttacct tggccaaaca gttgtccttt   1800
tataagatcc gtaccattgc cccaggcaag actcacacag ctgctattga tgagcgaggc   1860
cggctgctga cctttggctg caacaagtgt gggcagctgg gcgttgggaa ctacaagaag   1920
cgtctgggaa tcaacctgtt gggggggaccc cttggtggga agcaagtgat cagggtctcc   1980
tgcggtgatg agtttaccat tgctgccact gatgataatc acatttttgc ctggggcaat   2040
ggtggtaatg gccgcctggc aatgaccccc acagagagac cacatggctc tgatatctgt   2100
```

acctcatggc ctcggcctat ttttggatct ctgcatcatg tcccggacct gtcttgccgt 2160
ggatggcata ccattctcat cgttgagaaa gtattgaatt ctaagaccat ccgttccaat 2220
agcagtggct tatccattgg aactgtgttt cagagctcta gcccgggagg aggcggcggg 2280
ggcggcggtg gtgaagaaga ggacagtcag caggaatctg aaactcctga cccaagtgga 2340
ggcttccgag gaacaatgga agcagaccga ggaatggaag gtttaatcag tcccacagag 2400
gccatgggga acagtaatgg ggccagcagc tcctgtcctg gctggcttcg aaaggagctg 2460
gaaaatgcag aatttatccc catgcctgac agcccatctc ctctcagtgc agcgttttca 2520
gaatctgaga aagataccct gccctatgaa gagctgcaag gactcaaagt ggcctctgaa 2580
gctcctttgg aacacaaacc ccaagtagaa gcctcgtcac ctcggctgaa tcctgcagta 2640
acctgtgctg ggaagggaac accactgact cctcctgcgt gtgcgtgcag ctctctgcag 2700
gtggaggttg agagattgca gggtctggtg ttaaagtgtc tggctgaaca acagaagcta 2760
cagcaagaaa acctccagat tttacccaa ctgcagaagt tgaacaagaa attagaagga 2820
gggcagcagg tggggatgca ttccaaagga actcagacag caaaggaaga gatggaaatg 2880
gatccaaagc ctgacttaga ttcagattcc gggtgcctcc tgggaacaga ctcctgtaga 2940
cccagcctct agtctcctga gcctatagag cccccaggag actgggaccc aaagaacttc 3000
acagcacact taccgaatgc agagagcagc tttcctggct ttgttcactt gcagaaaagg 3060
agcgcaaggc agaggctctg aagcactttc cttgtacatt tggagagtgg cattgccttt 3120
tagataggat ctaggagtga ttttattgtt ttggagaatg gaagggcccc catggccctg 3180
gctttgtcat cagtgactgc catagcaaca gcagctctgt acctcatctg ttgatcccac 3240
cttt 3244

<210> 117
<211> 3128
<212> DNA
<213> Homo sapiens
<400> 117
tatttccatg caagtggaag acggtaccgt ctccccacat ttgagaagac tgttttgcat 60
cactctttgc tattgaagga agcagtgatg gtgaatttcc ttctgtttgg gttccttgtg 120
tctataactt cctttgtggt aaagccatca ggaagaatag tgggagtggg gtatatggtc 180
agggtgctca tatcctgctt tagcctagct gcttcttacg gagtgcaagg gagaactctg 240
agaagcagta tgtaaatacc agggagctga ttgctgaata ttgtggtctc atctgaatat 300
tgtggtctca tctaaatatt gacaccaggc aatgaagcag aaatagagta tgtgtccctt 360
tatgcgtggg taacttaagc ttctgtcatg tgggaagggg gaccgaatct tccctgggag 420
gaaggctcca aattctcact acttctgtgt tacttgaagg gggaagcata aggaacccag 480
tttgaaggca acattgtgtg ccatgaatct gcttattaat caacatgcct tgttaatgtc 540
ctctgccctg aacagccctt actcagttct catttggaaa gttatttttt ggggttacat 600
cctgtttgtt tcagaattta aaaccctcca tggtgggtca cttgaggtca ggagttcaag 660
attagcctgg ccaacttagt gaaactccgg ctctgctgaa gatgcaaaaa ttagccaggt 720
gtggcacacg cctgtaatcc cagttacttg ggaggccgag gcaggagaat cgcttgaacc 780
tgggaggcag aggtcgtagc gagccaagat cacaccattg cactccagcc tgggcaacag 840
agtgagactc tgtcaaaatc aaaaaaacca aaatacaaaa aactccattg ttctgagagt 900
ttcccctaaa ttatcggaag acactcacat tgaattggta ggacctatag tatagaggag 960
aatggtatgt atgtcagcag tagagttcca atccaaattg cgctatcgca ttagaaatac 1020
attaccctcc cactccccaa gttcagcaaa ttagaagaga atctgaagtt tgtaggaagg 1080
gaataaagct gctataaaca tttatgtgca ggtttttgtg tagactcgtt ttcagctcct 1140
ttgggtaaat accaaggaac caggttgctg gattgtaagt taagagtatg tttagttttg 1200
gaagaaactg ccaaactgtt ccaacatgac tattttgaat tcccatcgac aatgaataag 1260
agttcctgta gctcctcacc ctcattagta tttggtgatg tcactcttgc attttagcta 1320
atgggtgaat agtggtctca tcgttttaat tcccaatccc atagtgatgt ggagcacctt 1380
ttcatatact taatctgcca tccttatatc atcattggtg aggtgtctgg atttctgtc 1440
cagaatttta gttctagtgg ttttcttatt tttagaaagg aagtatagga aggaaggttt 1500
aatctgtctt aagcctgatt atatgaaaat caagaatagg ctaggtgtgg tggctgacat 1560
ctgtaatccc cgcactttgt gaggctgaag tgggaggact gcttgagcct gggaagtcaa 1620
gcctgcagtg agccatgact gttgctgcac tccagcctga gtgacagagt gagaccctgt 1680
ctcagaaaaa aaaaaagcag gctcatttta aagttgttta tattttattt tttgaaggaa 1740
ataaagttga agagatacaa agggtagtga agattctcct tcccctcctg taattcagct 1800
gtttagtttc tattctcgca cagccctgtt atcagtttct agtgtaagct tgcagtgact 1860
tttactttat ttataagcaa gtgttagtgg gtgttgtcat ttcttttaaa aatataaatt 1920
atactgtaca tgctattttg tattttggct tttctcccac ctaaacaccc taaagatcat 1980
tttctgtcag tatataaaat cttggtgatg ccaataccca tcccttagat tgtacaagca 2040
gcattttgcc atgctttatc ccatagcctg cctcctgcag tctcatcttt ttttctatat 2100
gcgtgtccaa gtaagttgta gacatttttc acttttaatc ctttcagcat acatgtcatg 2160
ggaatttgtt tttgtttttg ttttttgtgt ttgagacagt cgctctgtca cccaggctgg 2220
agttacagtg acgtgatccc agctcattac ggcctccacc tcccagtttc aagcaattct 2280
catgcctcgg attcccgagt agctgggatt acagatgtgc accaccacgc ctgactaatt 2340
tttgtatttt tagtagagat ggggtttcat catgttggcc aggctggtct ggaactcctg 2400
gcctccagtc atttgcctgc ctcggcctcc caaagtgctg ggattacagg catcagccac 2460
cacacccagc cgtcattgga gttttttga gggagatttg cagtaaggtg aataaatgtt 2520
aagtgtatca tttgatgagg tttttcttt ttttttttt ttttgagatg gagtcttgta 2580

tcgcccaggc tagagtgcag tggcctggtc ttggctcact gcaacctcca cctcctgggt 2640
tcaagggagt ctcctgcctc agcctcccga gtagctggga ttacaggtgt gagccaccat 2700
acccagactc atttgatgag ttttgatgta aaagcatgta actcaaaccc ctatgtctgg 2760
aacatctgta ttacatctga attttttcac ctagtcaatt cctgcacctc tctctcctgt 2820
cccttcttcc ctcccctggc agtcagctac tgttaggatt ttgttcactg tagattaatt 2880
ttgcctattc tagaacttca tataacggac tcacttatgc agtatgtatt ctgtaagttc 2940
cttttactca gcatgtttgt atgttcatca gtgtgtttaa gctttgttcc tttttatggc 3000
tgagcagtgt tctatgtatg aatgccacat tttaacgttt cacctgttag gtgaacaact 3060
aactggtgaa caatgggctg tttctagttt ggggctatca tgaataaagt tggtatgaac 3120
atgtatgt 3128

<210> 118
<211> 2326
<212> DNA
<213> Homo sapiens
<400> 118
ccagatcgca gctgaaggat ctgttgagcg cttcaggaaa ggcggacagg cgacactaac 60
aggtgaagat ctcgggagac catgactaag aaaagaattg ctgtgattgg gggaggagtg 120
agcgggctct cttccatcaa gtgctgcgta gaagaaggct tggaacctgt ctgctttgaa 180
aggactgatg acatcggagg gctctggagg ttccaggaaa atcctgaaga aggaagggcc 240
agtatttaca aatcagtgat catcaatact tctaaagaga tgatgtgctt cagtgactat 300
ccaatcccag atcattatcc caacttcatg cataatgccc aggtcctgga gtatttcagg 360
atgtatgcca aagaatttga ccttctaaag tatattcgat ttaagaccac tgtgtgcagt 420
gtgaagaagc agcctgattt tgccacttca ggccaatggg aagtggtcac tgaatctgaa 480
gggaaaaagg agatgaatgt ctttgatgga gtcatggttt gcactggcca tcacaccaat 540
gctcatctac ctctggaaag cttccctgga attgagaagt tcaaagggca gtacttccac 600
agtcgagact ataagaaccc agagggattc actggaaaga gagtcattat aattggcatt 660
gggaattctg gaggggatct ggctgtagag attagccaaa cagccaagca ggttttcctc 720
agcaccagga gaggggcttg gatcctgaat cgtgtagggg actacggata tcctgctgat 780
gtgttgttct cttctcgact tacacatttt atatggaaga tctgtggcca atcattagca 840
aacaaatatt tggaaaaaaa gataaaccaa aggtttgacc atgaaatgtt tggcctgaag 900
cctaaacaca gagctctgag tcagcatcca accttaaatg atgacctgcc aaatcgtatc 960
atttctggct tggtgaaagt gaaaggaaat gtgaaggaat tcacggagac agctgccata 1020
tttgaggatg gctccaggga ggatgacatt gatgctgtta tctttgccac aggctatagc 1080
tttgactttc catttctgga agattccgtc aaagtggtca aaaacaagat acccctgtat 1140
aaaaaggtct tccctcctaa cctggaaagg ccaactcttg caatcatagg cttgattcag 1200
cccttaggag ccattatgcc catttcagag ctccaaggac gctgggccac tcaggtattt 1260
aaaggtctaa agacattgcc ctcacagagt gaaatgatgg cagaaatatc taaagctcaa 1320
gaggaaattg acaaaaggta tgtggagagc caacgccata ccattcaggg agactacata 1380
gataccatgg aagagcttgc tgatttggtg ggggtcaggc ccaatctgct gtctctggcc 1440
ttcactgacc ccaagctggc attacactta ttactgggac cctgcactcc aatccactat 1500
cgtgtacagg gccctggaaa gtgggatggg gctcgaaaag ctatcctcac cacagatgat 1560
cgcatcagga agcctctgat gacaagagta gttgaaagga gtagttctat gacttcaaca 1620
atgacaatag gcaagtttat gctagctctt gccttctttg ctataattat agcttacttc 1680
tagttgtcct attgtcactg ccctgttttt cattgggaag cttatctaca gatgccttca 1740
gaatctgacg agattgactc tcagtttcat attgcccaga aatctacttt aatgtctctt 1800
tcgaaagcat taattcactt tccttttcc tacaatgaaa cctgttttcc atttgtatta 1860
actcatctcc cttccactca tgatccgtca ctcttccttg tggtaatccc tagactggga 1920
gctcaggtac tcttttagtc atctttgtat gtctttagca gagttcttga catgtggtag 1980
gtgcttaata aatgtttgtt gtttatcaaa ttttatggta gggagagtaa gtcagcatcg 2040
gtataaaatc gcttactcca cgtaactctt cttctgatag ggtttgattt tctattagaa 2100
gctcaatttt agtttttttt catattataa ctaaatatgt ttcctgagag ataagagaaa 2160
taatgttcct acaatagttg tatgtatcta agataagaca tatagatgct taagacattt 2220
tgtttcactt gctattcact agtgtacttg aaacatggtc atttttagcc cttttcctta 2280
ggaaccatgt ctttattttc tcaataaaga aattactttc aactca 2326

<210> 119
<211> 2439
<212> DNA
<213> Homo sapiens
<400> 119
cagcacccag ctccccgcca ccgccatggt ccccgacacc gcctgcgttc ttctgctcac 60
cctggctgcc ctcggcgcgt ccggacaggg ccagagcccg ttgggctcag acctgggccc 120
gcagatgctt cgggaactgc aggaaaccaa cgcggcgctg caggacgtgc gggactggct 180
gcggcagcag gtcagggaga tcacgttcct gaaaaacacg gtgatggagt gtgacgcgtg 240
cgggatgcag cagtcagtac gcaccggcct acccagcgtg cggcccctgc tccactgcgc 300
gcccggcttc tgcttccccg gcgtggcctg catccagacg gagagcggcg gccgctgcgg 360
cccctgcccc gcgggcttca cgggcaacgg ctcgcactgc accgacgtca acgagtgcaa 420
cgcccacccc tgcttccccc gagtccgctg tatcaacacc agcccggggt tccgctgcga 480

ggcttgcccg ccggggtaca gcggccccac ccaccagggc gtggggctgg ctttcgccaa 540
ggccaacaag caggtttgca cggacatcaa cgagtgtgag accgggcaac ataactgcgt 600
ccccaactcc gtgtgcatca acacccgggg ctccttccag tgcggcccgt gccagcccgg 660
cttcgtgggc gaccaggcgt ccggctgcca gcgcggcgca cagcgcttct gccccgacgg 720
ctcgcccagc gagtgccacg agcatgcaga ctgcgtccta gagcgcgatg gctcgcggtc 780
gtgcgtgtgt cgcgttggct gggccggcaa cgggatcctc tgtggtcgcg acactgacct 840
agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440
cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920
gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct ggggtcttc tgcttctccc aggagaacat 2220
catctggcc aacctgcgtt accgctgcaa tgacaccatc ccagaggact atgagaccca 2280
tcagctgcgg caagcctagg gaccagggtg aggacccgcc ggatgacagc caccctcacc 2340
gcggctggat gggggctctg cacccagccc aaggggtggc cgtcctgagg gggaagtgag 2400
aagggctcag agaggacaaa ataaagtgtg tgtgcaggg 2439

<210> 120
<211> 11185
<212> DNA
<213> Homo sapiens
<400> 120
gctgccccga gcctttctgg ggaagaactc caggcgtgcg gacgcaacag ccgagaacat 60
taggtgttgt ggacaggagc tgggaccaag atcttcggcc agccccgcat cctcccgcat 120
cttccagcac cgtcccgcac cctccgcatc cttccccggg ccaccacgct tcctatgtga 180
cccgcctggg caacgccgaa cccagtcgcg cagcgctgca gtgaattttc cccccaaact 240
gcaataagcc gccttccaag gccaagatgt tcataaatat aaagagcatc ttatggatgt 300
gttcaacctt aatagtaacc catgcgctac ataaagtcaa agtgggaaaa agcccaccgg 360
tgaggggctc cctctctgga aaagtcagcc taccttgtca tttttcaacg atgcctactt 420
tgccacccag ttacaacacc agtgaatttc tccgcatcaa atggtctaag attgaagtgg 480
acaaaaatgg aaaagatttg aaagagacta ctgtccttgt ggcccaaaat ggaaatatca 540
agattggtca ggactacaaa gggagagtgt ctgtgcccac acatcccgag gctgtgggcg 600
atgcctccct cactgtggtc aagctgctgg caagtgatgc gggtctttac cgctgtgacg 660
tcatgtacgg gattgaagac acacaagaca cggtgtcact gactgtggat ggggttgtgt 720
ttcactacag ggcggcaacc agcaggtaca cactgaattt tgaggctgct cagaaggctt 780
gtttggacgt tggggcagtc atagcaactc cagagcagct ctttgctgcc tatgaagatg 840
gatttgagca gtgtgacgca ggctggctgg ctgatcagac tgtcagatat cccatccggg 900
ctcccagagt aggctgttat ggagataaga tgggaaaggc aggagtcagg acttatggat 960
tccgttctcc ccaggaaact tacgatgtgt attgttatgt ggatcatctg gatggtgatg 1020
tgttccacct cactgtcccc agtaaattca ccttcgagga ggctgcaaaa gagtgtgaaa 1080
accaggatgc caggctggca acagtggggg aactccaggc ggcatggagg aacggctttg 1140
accagtgcga ttacgggtgg ctgtcggatg ccagcgtgcg ccaccctgtg actgtggcca 1200
gggcccagtg tggaggtggt ctacttgggg tgagaaccct gtatcgtttt gagaaccaga 1260
caggcttccc tccccctgat agcagatttg atgcctactg ctttaaacct aaagaggcta 1320
caaccatcga tttgagtatc ctcgcagaaa ctgcatcacc cagtttatcc aaagaaccac 1380
aaatggtttc tgatagaact acaccaatca tccctttagt tgatgaatta cctgtcattc 1440
caacagagtt ccctcccgtg ggaaatattg tcagttttga acagaaagcc acagtccaac 1500
ctcaggctat cacagatagt ttagccacca aattacccac acctactggc agtaccaaga 1560
agccctggga tatggatgac tactcacctt ctgcttcagg acctcttgga aagctagaca 1620
tatcagaaat taaggaagaa gtgctccaga gtacaactgg cgtctctcat tatgctacgg 1680
attcatggga tggtgtcgtg gaagataaac aaacacaaga atcggttaca cagattgaac 1740
aaatagaagt gggtcctttg gtaacatcta tggaaatctt aaagcacatt ccttccaagg 1800

aattccctgt aactgaaaca ccattggtaa ctgcaagaat gatcctggaa tccaaaactg 1860
aaaagaaaat ggtaagcact gtttctgaat tggtaaccac aggtcactat ggattcacct 1920
tgggagaaga ggatgatgaa gacagaacac ttacagttgg atctgatgag agcaccttga 1980
tctttgacca aattcctgaa gtcattacgg tgtcaaagac ttcagaagac accatccaca 2040
ctcatttaga agacttggag tcagtctcag catccacaac tgtttcccct ttaattatgc 2100
ctgataataa tggatcatcc atggatgact gggaagagag acaaactagt ggtaggataa 2160
cggaagagtt tcttggcaaa tatctgtcta ctacaccttt tccatcacag catcgtacag 2220
aaatagaatt gtttccttat tctggtgata aaatattagt agagggaatt tccacagtta 2280
tttatccttc tctacaaaca gaaatgacac atagaagaga aagaacagaa acactaatac 2340
cagagatgag aacagatact tatacagatg aaatacaaga agagatcact aaaagtccat 2400
ttatgggaaa aacagaagaa gaagtcttct ctgggatgaa actctctaca tctctctcag 2460
agccaattca tgttacagag tcttctgtgg aaatgaccaa gtcttttgat ttcccaacat 2520
tgataacaaa gttaagtgca gagccaacag aagtaagaga tatggaggaa gactttacag 2580
caactccagg tactacaaaa tatgatgaaa atattacaac agtgcttttg gcccatggta 2640
ctttaagtgt tgaagcagcc actgtatcaa aatggtcatg ggatgaagat aatacaacat 2700
ccaagccttt agagtctaca gaaccttcag cctcttcaaa attgccccct gccttactca 2760
caactgtggg gatgaatgga aaggataaag acatcccaag tttcactgaa gatggagcag 2820
atgaatttac tcttattcca gatagtactc aaaagcagtt agaggaggtt actgatgaag 2880
acatagcagc ccatggaaaa ttcacaatta gatttcagcc aactacatca actggtattg 2940
cagaaaagtc aactttgaga gattctacaa ctgaagaaaa agttccacct atcacaagca 3000
ctgaaggcca agtttatgca accatggaag gaagtgcttt gggtgaagta gaagatgtgg 3060
acctctctaa gccagtatct actgttcccc aatttgcaca cacttcagag gtggaaggat 3120
tagcatttgt tagttatagt agcacccaag agcctactac ttatgtagac tcttcccata 3180
ccattcctct ttctgtaatt cccaagacag actggggagt gttagtacct tctgttccat 3240
cagaagatga agttctaggt gaaccctctc aagacatact tgtcattgat cagactcgcc 3300
ttgaagcgac tatttctcca gaaactatga gaacaacaaa aatcacagag ggaacaactc 3360
aggaagaatt cccttggaaa gaacagactg cagagaaacc agttcctgct ctcagttcta 3420
cagcttggac tcccaaggag gcagtaacac cactggatga acaagagggc gatggatcag 3480
catatacagt ctctgaagat gaattgttga caggttctga gagggtccca gttttagaaa 3540
caactccagt tggaaaaatt gatcacagtg tgtcttatcc accaggtgct gtaactgagc 3600
acaaagtgaa aacagatgaa gtggtaacac taacaccacg cattgggcca aaagtatctt 3660
taagtccagg gcctgaacaa aaatatgaaa cagaaggtag tagtacaaca ggatttacat 3720
catctttgag tccttttagt acccacatta cccagcttat ggaagaaacc actactgaga 3780
aaacatccct agaggatatt gatttaggct caggattatt tgaaaagccc aaagccacag 3840
aactcataga atttcaaca atcaaagtca cagttccaag tgatattacc actgccttca 3900
gttcagtaga cagacttcac acaacttcag cattcaagcc atcttccgcg atcactaaga 3960
aaccacctct catcgacagg gaacctggtg aagaaacaac cagtgacatg gtaatcattg 4020
gagaatcaac atctcatgtt cctcccacta cccttgaaga tattgtagcc aaggaaacag 4080
aaaccgatat tgatagagag tatttcacga cttcaagtcc tcctgctaca cagccaacaa 4140
gaccacccac tgtggaagac aaagaggcct ttggacctca ggcgctttct acgccacagc 4200
cccagcaag cacaaaattt caccctgaca ttaatgttta tattattgag gtcagagaaa 4260
ataagacagg tcgaatgagt gatttgagtg taattggtca tccaatagat tcagaatcta 4320
aagaagatga accttgtagt gaagaaacag atccagtgca tgatctaatg gctgaaattt 4380
tacctgaatt ccctgacata attgaaatag acctatacca cagtgaagaa aatgaagaag 4440
aagaagaaga gtgtgcaaat gctactgatg tgacaaccac cccatctgtg cagtacataa 4500
atgggaagca tctcgttacc actgtgccca aggacccaga agctgcagaa gctaggcgtg 4560
gccagtttga aagtgttgca ccttctcaga atttctcgga cagctctgaa agtgatactc 4620
atccatttgt aatagccaaa acggaattgt ctactgctgt gcaacctaat gaatctacag 4680
aaacaactga gtctcttgaa gttacatgga agcctgagac ttaccctgaa acatcagaac 4740
atttttcagg tggtgagcct gatgttttcc ccacagtccc attccatgag gaatttgaaa 4800
gtggaacagc caaaaaaggg gcagaatcag tcacagagag agatactgaa gttggtcatc 4860
aggcacatga acatactgaa cctgtatctc tgtttcctga agagtcttca ggagagattg 4920
ccattgacca agaatctcag aaaatagcct ttgcaagggc tacagaagta acatttggtg 4980
aagaggtaga aaaaagtact tctgtcacat acactcccac tatagttcca agttctgcat 5040
cagcatatgt ttcagaggaa gaagcagtta ccctaatagg aaatccttgg ccagatgacc 5100
tgttgtctac caaagaaagc tgggtagaag caactcctag acaagttgta gagctctcag 5160
ggagttcttc gattccaatt acagaaggct ctggagaagc agaagaagat gaagatacaa 5220
tgttcaccat ggtaactgat ttatcacaga gaaatactac tgatacactc attactttag 5280
acactagcag gataatcaca gaaagctttt ttgaggttcc tgcaaccacc atttatccag 5340
tttctgaaca accttctgca aaagtggtgc ctaccaagtt tgtaagtgaa acagacactt 5400
ctgagtggat ttccagtacc actgttgagg aaaagaaaag gaaggaggag gagggaacta 5460
caggtacggc ttctacattt gaggtatatt catctacaca gagatcggat caattaattt 5520
taccctttga attagaaagt ccaaatgtag ctacatctag tgattcaggt accaggaaaa 5580
gttttatgtc cttgacaaca ccaacacagt ctgaaaggga aatgacagat tctactcctg 5640
tctttacaga aacaaataca ttagaaaatt tgggggcaca gaccactgag cacagcagta 5700
tccatcaacc tggggttcag gaagggctga ccactctccc acgtagtcct gcctctgtct 5760
ttatggagca gggctctgga gaagctgctg ccgacccaga aaccaccact gtttcttcat 5820
tttcattaaa cgtagagtat gcaattcaag ccgaaaagga agtagctggc actttgtctc 5880
cgcatgtgga aactacattc tccactgagc caacaggact ggttttgagt acagtaatgg 5940

acagagtagt tgctgaaaat ataacccaaa catccaggga aatagtgatt tcagagcgat 6000
taggagaacc aaattatggg gcagaaataa ggggcttttc cacaggtttt cctttggagg 6060
aagatttcag tggtgacttt agagaatact caacagtgtc tcatcccata gcaaaagaag 6120
aaacggtaat gatggaaggc tctggagatg cagcatttag ggacacccag acttcaccat 6180
ctacagtacc tacttcagtt cacatcagtc acatatctga ctcagaagga cccagtagca 6240
ccatggtcag cacttcagcc ttcccctggg aagagtttac atcctcagct gagggctcag 6300
gtgagcaact ggtcacagtc agcagctctg ttgttccagt gcttcccagt gctgtgcaaa 6360
agttttctgg tacagcttcc tccattatcg acgaaggatt gggagaagtg ggtactgtca 6420
atgaaattga tagaagatcc accattttac caacagcaga agtggaaggt acgaaagctc 6480
cagtagagaa ggaggaagta aaggtcagtg gcacagtttc aacaaacttt ccccaaacta 6540
tagagccagc caaattatgg tctaggcaag aagtcaaccc tgtaagacaa gaaattgaaa 6600
gtgaaacaac atcagaggaa caaattcaag aagaaaagtc atttgaatcc cctcaaaact 6660
ctcctgcaac agaacaaaca atctttgatt cacagacatt tactgaaact gaactcaaaa 6720
ccacagatta ttctgtacta acaacaaaga aaacttacag tgatgataaa gaaatgaagg 6780
aggaagacac ttctttagtt aacatgtcta ctccagatcc agatgcaaat ggcttggaat 6840
cttacacaac tctccctgaa gctactgaaa agtcacattt tttcttagct actgcattag 6900
taactgaatc tataccagct gaacatgtag tcacagattc accaatcaaa aaggaagaaa 6960
gtacaaaaca ttttccgaaa ggcatgagac caacaattca agagtcagat actgagctct 7020
tattctctgg actgggatca ggagaagaag ttttacctac tctaccaaca gagtcagtga 7080
attttactga agtggaacaa atcaataaca cattatatcc ccacacttct caagtggaaa 7140
gtacctcaag tgacaaaatt gaagacttta acagaatgga aaatgtggca aaagaagttg 7200
gaccactcgt atctcaaaca gacatctttg aaggtagtgg gtcagtaacc agcacaacat 7260
taatagaaat tttaagtgac actggagcag aaggacccac ggtggcacct ctccctttct 7320
ccacggacat cggacatcct caaaatcaga ctgtcaggtg ggcagaagaa atccagacta 7380
gtagaccaca aaccataact gaacaagact ctaacaagaa ttcttcaaca gcagaaatta 7440
acgaaacaac aacctcatct actgattttc tggctagagc ttatggtttt gaaatggcca 7500
aagaatttgt tacatcagca ccaaaaccat ctgacttgta ttatgaacct tctggagaag 7560
gatctggaga agtggatatt gttgattcat ttcacacttc tgcaactact caggcaacca 7620
gacaagaaag cagcaccaca tttgtttctg atgggtccct ggaaaaacat cctgaggtgc 7680
caagcgctaa agctgttact gctgatggat tcccaacagt ttcagtgatg ctgcctcttc 7740
attcagagca gaacaaaagc tcccctgatc caactagcac actgtcaaat acagtgtcat 7800
atgagaggtc cacagacggt agtttccaag accgtttcag ggaattcgag gattccacct 7860
taaaacctaa cagaaaaaaa cccactgaaa atattatcat agacctggac aaagaggaca 7920
aggatttaat attgacaatt acagagagta ccatccttga aattctacct gagctgacat 7980
cggataaaaa tactatcata gatattgatc atactaaacc tgtgtatgaa gacattcttg 8040
gaatgcaaac agatatagat acagaggtac catcagaacc acatgacagt aatgatgaaa 8100
gtaatgatga cagcactcaa gttcaagaga tctatgaggc agctgtcaac ctttctttaa 8160
ctgaggaaac atttgagggc tctgctgatg ttctggctag ctacactcag gcaacacatg 8220
atgaatcaat gacttatgaa gatagaagcc aactagatca catgggcttt cacttcacaa 8280
ctgggatccc tgctcctagc acagaaacag aattagacgt tttacttccc acggcaacat 8340
ccctgccaat tcctcgtaag tctgccacag ttattccaga gattgaagga ataaaagctg 8400
aagcaaaagc cctggatgac atgtttgaat caagcacttt gtctgatggt caagctattg 8460
cagaccaaag tgaaataata ccaacattgg gccaatttga aaggactcag gaggagtatg 8520
aagacaaaaa acatgctggt ccttcttttc agccagaatt ctcttcagga gctgaggagg 8580
cattagtaga ccatactccc tatctaagta ttgctactac ccaccttatg gatcagagtg 8640
taacagaggt gcctgatgtg atggaaggat ccaatccccc atattacact gatacaacat 8700
tagcagtttc aacatttgcg aagttgtctt ctcagacacc atcatctccc ctcactatct 8760
actcaggcag tgaagcctct ggacacacag agatccccca gcccagtgct ctgccaggaa 8820
tagacgtcgg ctcatctgta atgtccccac aggattcttt taaggaaatt catgtaaata 8880
ttgaagcaac tttcaaacca tcaagtgagg aataccttca cataactgag cctccctctt 8940
tatctcctga cacaaaatta gaaccttcag aagatgatgg taaacctgag ttattagaag 9000
aaatggaagc ttctcccaca gaacttattg ctgtggaagg aactgagatt ctccaagatt 9060
tccaaaacaa aaccgatggt caagtttctg gagaagcaat caagatgttt cccaccatta 9120
aaacacctga ggctggaact gttattacaa ctgccgatga aattgaatta gaaggtgcta 9180
cacagtggcc acactctact tctgcttctg ccacctatgg ggtcgaggca ggtgtggtgc 9240
cttggctaag tccacagact tctgagaggc ccacgctttc ttcttctcca gaaataaacc 9300
ctgaaactca agcagcttta atcagagggc aggattccac gatagcagca tcagaacagc 9360
aagtggcagc gagaattctt gattccaatg atcaggcaac agtaaaccct gtggaattta 9420
atactgaggt tgcaacacca ccattttccc ttctggagac ttctaatgaa acagatttcc 9480
tgattggcat taatgaagag tcagtggaag gcacggcaat ctatttacca ggacctgatc 9540
gctgcaaaat gaacccgtgc cttaacggag gcacctgtta tcctactgaa acttcctacg 9600
tatgcacctg tgtgccagga tacagcggag accagtgtga acttgatttt gatgaatgtc 9660
actctaatcc ctgtcgtaat ggagccactt gtgttgatgg ttttaacaca ttcaggtgcc 9720
tctgccttcc aagttatgtt ggtgcacttt gtgagcaaga taccgagaca tgtgactatg 9780
gctggcacaa attccaaggg cagtgctaca aatactttgc ccatcgacgc acatgggatg 9840
cagctgaacg ggaatgccgt ctgcagggtg cccatctcac aagcatcctg tctcacgaag 9900
aacaaatgtt tgttaatcgt gtgggccatg attatcagtg gataggcctc aatgacaaga 9960
tgtttgagca tgacttccgt tggactgatg gcagcacact gcaatacgag aattggagac 10020
ccaaccagcc agacagcttc ttttctgctg gagaagactg tgttgtaatc atttggcatg 10080

```
agaatggcca gtggaatgat gttccctgca attaccatct cacctatacg tgcaagaaag  10140
gaacagttgc ttgcggccag ccccctgttg tagaaaatgc caagaccttt ggaaagatga  10200
aacctcgtta tgaaatcaac tccctgatta gataccactg caaagatggt ttcattcaac  10260
gtcaccttcc aactatccgg tgcttaggaa atggaagatg ggctatacct aaaattacct  10320
gcatgaaccc atctgcatac caaaggactt attctatgaa atactttaaa aattcctcat  10380
cagcaaagga caattcaata aatacatcca aacatgatca tcgttggagc cggaggtggc  10440
aggagtcgag gcgctgatcc ctaaaatggc gaacatgtgt tttcatcatt tcagccaaag  10500
tcctaacttc ctgtgccttt cctatcacct cgagaagtaa ttatcagttg gtttggattt  10560
ttggaccacc gttcagtcat tttgggttgc cgtgctccca aaacatttta aatgaaagta  10620
ttggcattca aaaagacagc agacaaaatg aaagaaaatg agagcagaaa gtaagcattt  10680
ccagcctatc taatttcttt agttttctat ttgcctccag tgcagtccat ttcctaatgt  10740
ataccagcct actgtactat ttaaaatgct caatttcagc accgatggcc atgtaaataa  10800
gatgatttaa tgttgatttt aatcctgtat ataaaataaa aagtcacaat gagtttgggc  10860
atatttaatg atgattatgg agccttagag gtctttaatc attggttcgg ctgcttttat  10920
gtagtttagg ctggaaatgg tttcacttgc tctttgactg tcagcaagac tgaagatggc  10980
ttttcctgga cagctagaaa acacaaaatc ttgtaggtca ttgcacctat ctcagccata  11040
ggtgcagttt gcttctacat gatgctaaag gctgcgaatg ggatcctgat ggaactaagg  11100
actccaatgt cgaactcttc tttgctgcat tcctttttct tcacttacaa gaaaggcctg  11160
aatggaggac ttttctgtaa ccagg                                        11185
```

<210> 121
<211> 3187
<212> DNA
<213> Homo sapiens
<400> 121

```
gaaagaaatc tggtgcctgg gggtccctgt gttcacccct agagtttgtt ttaaaatttt    60
taattgaagc atgtgaagtg tacctgcaga aaagtgggaa catgatagtg tatggcttgg   120
tggattttca caaactgaac atacctgtgt aatcagcatc tagacccaga cccagagcgt   180
cacaaatatc ccccatcctg ggcttttccc agaggagatg ggggcttctg aagatggact   240
tacctgggac ctgcccccca tgagccagga cggtcccccc acagtcagcc tgtgcaaagg   300
ccccgtggcc aggggtggag gagaatatgt gggtgtggac aggatgggag actgtggcct   360
gaacaggaga ttttattata tctggagacc ctgagagacc ctgagacctg ggcaccctg    420
gctggccagg tcagaagcat cctgactgca gaggtccgtg cagccacacc ctcttccctg   480
ccagcaagct gtctgcggct catcggaggc ccctccgcct ggagccttct atggacgtga   540
tatgcctgta tctgttttta attttcattc ttcacttagg ggaagtgaaa tcgctcagag   600
atgagatcct ttaattgaaa acgaagtgta acggaatcta gtgtctttct aatgtggtaa   660
aattctccat caacatcaca gtcagctggc agctgaactt cagaatctca cttacagcag   720
gcgacacggg ggtacaccga tgggtcacac tgggtctggg ggctccctgg agctcctcct   780
gcgtgtggtc tggttaggag ttgagttgtt tgctccaggg ttattctcct cctcgagtca   840
cagtcacacg aatacctgcc ttctctggct ttcctgctat acacatattc acatggcgct   900
caagaagtta ggctcatggc aacgtgtgtc tttctctgga caactggccc agtttacagt   960
gaaatggaga atttcaggtc tccacgtctg cccaggaaag aacttcagct gactccacgg  1020
ggatctggaa atccacgacc aatcccgatc ggctcttatt agctccccgc tccacaagac  1080
acctgtgctt tggaaatcca ccaccaatcc cgatcggctc ttattagctc cccgctccac  1140
aagacacctg tgatctggaa atctaccacc aatcccgatc ggctcttatt agctccccgc  1200
tccacaagac acctgtgaca tcctccaggg ccacaggagc acgtgctgac cagttttccc  1260
ttccagttcc tgcacaaaaa gtgtccagag ggctgtttgc aaacactagt gcactttgta  1320
gcttttcacc ctctgtccca gggaatctag gagagatgag gcccgtcaga gtcaagagat  1380
gtcatccccc cagggtctcc aaggcatttc cacactattg gtggcacctg gaggacatgc  1440
accaaggctt gccagagcca acaggaagtg agcccagagc atggcacatg agcatcaccc  1500
gctgatggtg gcctgctgtg cctggtgcca acaggggcat cccggcccat acccctccag  1560
acaggaagca tgggtttgcc cacagacctg tcgggtgctc ctgtgagtgg cctccagatg  1620
tctttgtgca taggcacaag tgggccaggg ctggagggag gtgggaaacc tcatcatccg  1680
gtgggccctg ccaatcttaa cccagaaccc ttaggtattc ctggcagtag ccatgacatt  1740
ggagcacctt cctctccagc cagaggctga cctgagggcc actgtcctca gatgacacca  1800
cccaggagca ccctaggtga ggggtgaggg ccccccttatg tgaacctctt gcctcttcct  1860
ttctcccatc agagtggttg gatggagcca ttggcctcct ttcttcagc  gggcccttca  1920
acctctctgc accatgttgt ctggctgagg agctactaga aaagctgagt ggagtctcct  1980
ttccaacagg atgatgcatt tgctcaattc tcagggctgg aatgagccgg ctggtccccc  2040
agaaagctgg agtggggtac agagttcagt tttcctctct gtttacagct ccttgacagt  2100
cccacgccca tctggagtgg gagctgggag tcagtgttgg agaagaaaca acaaaagcca  2160
attagaacca ctatttttaa aaagtgctta ctgtgcacag atactcttca agcactggac  2220
gtggattctc tctctagccc tcagcacccc tgcggtagga gtgccgcctc tacccacttg  2280
tgatggggta cagaggcact tgctcttctg catggtgttc aataggctgg gagttttatt  2340
tatctcttca aactttgtac aagagctcat ggcttgtctt gggctttcgt cattaaacca  2400
aaggaaatgg aagccattcc cctgttgctc tccttagtct tggtcatcag aacctcactt  2460
ggtaccatat agatcaaaag ctttgtaacc acaggaaaaa ataaactctt ccatccctta  2520
aagaatagaa tagtttgtcc ctctcatggg aattgggctg tatgtatatt gttcttcctc  2580
cttagaattt agagatacaa gagttctact tagaactttt catggacaca atttccacaa  2640
```

cctttcagat gctgatgtag agctattggg aaagaacttc caaactcagg aagtttgcag 2700
agagcagaca gctagagata actcgggacc cagagttggt cgacagatgt tagatgtatc 2760
ctagctttta gctataaacc actcaaagat tcagccccca gatcccacag tcagaactga 2820
atctgcgttg ttgggaagcc agcagtggcc ttgggaagga agccatggct gtggttcaga 2880
gagggtgggc tggcaagcca cttccgggga aaactccttc cgccccaggt ttcttcttct 2940
cttaaggaga gattattctc accaacccgc tgccttcatg ctgccttcaa agctagatca 3000
tgtttgcctt gcttagagaa ttactgcaaa tcagccccag tgcttggcga tgcatttaca 3060
gatttctagg ccctcagggt tttgtagagt gtgagccctg gtgggcaggg ttggggggtc 3120
tgtcttctgc tggatgctgc ttgtaatcca tttggtgtac agaatcaaca ataaataata 3180
tacatgt 3187

<210> 122
<211> 1992
<212> DNA
<213> Homo sapiens
<400> 122
cagacatctg ctcctcacat gaatgcactc acctcctaga gaccaggctg ccatcatgct 60
ctggaagctt gtggagaatg tcaagtacga agatatctat gaggaccggc acgatggtgt 120
cccgagccac agctcgcggc tctcccagct gggctcggtg tcccaaggac cctactcgag 180
cgccccgccg ctgtcccaca ccccgtcgtc ggacttccag ccgccctact tcccaccccc 240
ctaccagccg ctcccctacc accagagcca ggacccctac tcccacgtca acgacccccta 300
ctccctgaac ccactgcacc agccccagca acatccctgg gggcaacggc agcggcaaga 360
agtgggttcg gaagccggct ctctcctgcc ccagcctcgg gccgccttgc cccagctctc 420
gggccttgac cccggaggg actaccactc ggtccgccgg ccggacgtgc tgctgcattc 480
ggcgcaccac ggcctggacg cgggcatggg tgacagcctc tcgctgcacg gcctcggcca 540
tcccggaatg gaagacgtcc agtcagttga agatgccaat aacagcggca tgaatctatt 600
ggaccagtct gtcattaaaa aagttccagt tcctcccaaa tcggtgactt ctctaatgat 660
gaataaagac ggcttcctgg gaggcatgtc tgtcaacacc ggcgaggtgt tttgctccgt 720
cccaggccgt ttgtctctgc tcagttcaac ttcgaagtac aaagtaactg tgggagaagt 780
tcagagacgg ctgtcgcccc ctgaatgcct caatgcatct ctcctcggcg gagtcctcag 840
aagagccaaa tcgaaaaatg gggggagatc tttgcgagaa aggctagaaa aaatcggttt 900
gaatttaccc gcgggcaggc gcaaagcagc aaatgtcacg ttactcacct ccctggtgga 960
aggagaagct gttcacttag ctagggattt tgggtacatt tgcgaaacgg agtttcccgc 1020
caaagccgtc tctgagtatt tgaaccggca gcacacagac ccgagtgacc tgcactcccg 1080
aaagaatatg ctgttggcca ccaagcaact ttgtaaagaa tttacggatc tactggcgca 1140
ggaccggaca ccgataggga acagccgacc cagccccatc ctggagccgg ggatccagag 1200
ctgcctcacg cacttcagcc tcatcacgca cggcttcggc gccccggcca tttgcgccgc 1260
gctcacggcc ctgcagaact atctcaccga ggcgctcaaa ggcatggaca agatgttctt 1320
gaacaacacc accactaaca ggcacacgtc tggggaaggc ccaggtagta aaactggcga 1380
caaggaggag aaacacagga aatgaaaaat ttttaaaaaa agaaggaaaa atgttttaaa 1440
tacaaaagga aaaacagaca aaaatttaat tttagcttta aaatattgga ttggctttgg 1500
aagaattata ttaggtagaa tacacataca atcaaaattt aaaaaaaaaa agctaaataa 1560
cttaaaaaaa aactgaggcg tacaacggag caacaatatc ggttctcagt gtctatttca 1620
agatacattt ggagacaacc gtccggattt tccacttcgg ttctttcgag tttagtaata 1680
ctgataataa aagaaaacca tgatttcccc ttccctttgg aaaataaaca taagactaaa 1740
catgagaaaa acgctaactt attggaagaa aatcggagaa acgttggtgt caatgctttg 1800
agagctggtt gactgagacg cacgaacttt ttaattttaa atatattttt aggaaactct 1860
cgcagtcccc gccctccatc tcacctcacc cgtctcccaa ccaccctttt ccatgttacc 1920
ctcccttcct cacattgtta cgggaatctt ctgggatgaa aatgagtgtg gttggaaaaa 1980
aaaaaaaaaa aa 1992

<210> 123
<211> 942
<212> DNA
<213> Homo sapiens
<400> 123
atgatacaga cccacagagt ctaacagatg tctctatatt cctcctcctc aaactctcag 60
aggatccaga actgcagcag gtcgtcgctg ggctgttcct gtccatgtgc ctggtcacgg 120
tgctggggaa cctactcatc atcctggccg tcagccctga ctcccacctc cacaccccca 180
tgtacttgtt cctctccaac ctgtcccttg cctgacatcg gtttcacctc caccacggtc 240
cccaagatga ttgtggacat ccagtctcac agcagagtca tctcctatgc aggctgcctg 300
actcagatgt ctctctttgc catttttgga ggtatggaag agagacatgc tcctgagtgt 360
gatggcctat gaccggtttg tagccatctg tcaccctcta tattgttcag ccatctttaa 420
cccgtgtttc tgtggcttcc tagatttgtt gtcttttttt ttttttctc agtctttcag 480
actcccagct gcacaacttg attgccttac aaatgacctg cttcaaggat gtggaaattc 540
ctaatttctt ctgggaacct tctcaactct cccatcttgc atgttgtgac accttcacca 600
ggaacatcag tatttccctg ctgccatatt tggttttctt cccatcttgg ggaccctttt 660
ctcttactgt aaaattgttt cctccattct gaggtttca tcatcaggtg ggaagtataa 720
accttctcca cctgtgggtc tcacctgtca gttgtttgct gattttatgg aacaggcatt 780

ggagggtacc tcggttcaga tgtgtcatct tccccgagaa agggtgcagt ggcctcagtg 840
atgtacatgg tggtcacccc catgctgaac cccttcatct acagcctgag aaacagggat 900
atgaaaagtg tcctgcggcg gccgcatggc agcacagtct aa 942

<210> 124
<211> 3969
<212> DNA
<213> Homo sapiens
<400> 124
ggcccagaaa tagcagcggc gcgttcccct cggcgcagag cgacgggcag aggcgagaga 60
cgccgcgggg ccgggcggcg gccccggaag gatgctgctg agccccggca ctgcctggct 120
gcgagcacat gatggcgata cgggagctca aagtgtgcct tctcggggac actggggttg 180
ggaaatcaag catcgtgtgt cgatttgtcc aggatcactt tgaccacaac atcagcccta 240
ctattggggc atcttttatg accaaaactg tgccttgtgg aaatgaactt cacaagttcc 300
tcatctggga cactgctggt caggaacggt ttcattcatt ggctcccatg tactatcgag 360
gctcagctgc agctgttatc gtgtatgata ttaccaagca ggattcattt tataccttga 420
agaaatgggt caaggagctg aaagaacatg gtccagaaaa cattgtaatg gccatcgctg 480
gaaacaagtg cgacctctca gatattaggg aggttcccct gaaggatgct aaggaatacg 540
ctgaatccat aggtgccatc gtggttgaga caagtgcaaa aaatgctatt aatatcgaag 600
agctctttca aggaatcagc cgccagatcc caccccttgga cccccatgaa aatggaaaca 660
atggaacaat caaagttgag aagccaacca tgcaagccag ccgccggtgc tgttgaccca 720
agggccgtgg tccacggtac ttgaagaagc cagagcccac atcctgtgca ctgctgaagg 780
accctacgct cggtggcctg gcacctcact ttgagaagag tgagcacact ggctttgcat 840
cctggaagac ctgcaggggg cggggcagga aatgtacctg aaaaggattt tagaaaaccc 900
tgggaaaacc caccacacca ccacaaaatg gcctttagtg tatgaaatgc acatggaggg 960
gatgtagttg cattttttgct aaaaaaaaaa aaaaaccttt aaaaattgtt ggatgtgtac 1020
aaaagtctta ctgccttatt atgtgtatgg gattctaaag tggcattcca cttggatttc 1080
ctgtgctacc tatcccaaat tcccagtaac tacttcagtg tcattgcctt tgttacctaa 1140
ccaaccttca ctgaaaggca aatttagttc aggaggttag tttttagcta gctttggaag 1200
taagccttta tttattactt tttggaggaa atcagagaag tgtcaatgga ccgtcactca 1260
gactgagact tgagttatta cagaagccag gaaaagtgta ttagaaactg ttgtctacac 1320
cacttttaat tggtgaacaa tttttctaag ttatggtcat atatacccaa acaaaccaaa 1380
tcaaactaaa ttactgcata taattttggg attgggtggc ctagtttgaa agagtgattt 1440
aagtaatcac tatgtaagtg gtgagagatg caggacatac acattattca agagaccacc 1500
tgacatgcat ctcctccgca ggaatacatt cgtcctctct tagagaagtt taacgcacat 1560
agtattattt tactaagaga atatctcttg gtgtcatatc taggggaaga gaattaacta 1620
gaattaaatt taatgtttga atctaaatca ttgggcaaac ttctaataat aacaattaat 1680
aataggttac aggaaagcca gccagaggaa gtgtcagcac tttaaaattc tagaccccaa 1740
aaaactacaa aatcagaaaa agtattttta tgtttctagc ttgaggagaa gggctttagg 1800
gctaaccaga ggtctgaccc tagaatgcca aggaactgag aatgggctcc gatgaaaacc 1860
ttccttttca gattccctgt ctgctcaatt aaagatgttt gaatccaaag gaagtcaagg 1920
aagaaaaagc atggaaagga agagaactga ttcctactga aaattcaaat tctattacca 1980
ttctaacttt cataaaaagt tgggatcaag aagcagctga tttcctgcca gggcttatat 2040
taggggggtga ttcttaaagg acattaggat tggtgctcag aaatggttaa tcatgctgtg 2100
tgctagccag ggccagctgg taccttcttt gccatgagca ttcaagggac agctaacctt 2160
tattgacaat ctatattgca aaagtcagga aagaggttgt gagctgattg gattaaagac 2220
ctggcacttc agtaactcag cacgcttcca cttcactcaa cttaagagag ttcattgaca 2280
gtgttaggat gtgaaggctg ggaaacactt attttgcttc aagagttcca cttggctctc 2340
ccaaataggt acctcaaaaa ctgttagcaa gcggcatttg gatgtcttga caggggcttt 2400
gcagggattt ttagggtttt ttccacattg tccacattaa tggttggcat gattgtgctt 2460
gcaggccaag aaatgatcat accccttgcc aaaggtaaaa aaaaaaaaaa aaaaatgagt 2520
tgaaaattga agtgacctct ttccagctga cttgcaggct tattttgtaa cctttcctca 2580
tccagttttc cctgagaacc tgggtttatc tctagatagc tgttcaggtt ttttagctga 2640
ggggtaagta tcctagctga gagttttgca tctttgggct gggtttgcag tggttgtgtt 2700
ttgcataaaa tgtctagtct ttgccacaga tagtgagcta cccactaatg agcccatggt 2760
tttatttcag aagcacatga gggtgtgaaa ccactctgtt acctttctgt attgtcttag 2820
ctattcaagc cagtcagagg ataatatata tattctcatc agcactcaga gtagtcagtg 2880
aagagagtag gatcacactt ggggcacacc aggattcaca taaacattgt atcttctctg 2940
tggatgctca ggccttgtct acaatgaggc tttacaacct tccttgtttg gctcgggatt 3000
acttcctggc tgtctaataa ttgaaccata accatgtaat attatgtaaa ggcctggaaa 3060
ttactgttgc taaaaaaagt catgtagttt catgtagtgt agcatccttg gcatcgtttt 3120
ccaaaatttg ttccttctcc cttttttttt tctttcgtgt gtggcatgag tgtgtatctg 3180
tgtaaatatg attgtatatg tgttactccg atatgtaatc catttcactg gctgagtttg 3240
gcccctagcc atgtgttaat ataaagtagg gcatgggctt cccaatggaa atctctgaga 3300
atgacagtgg agttgtgcaa gcattttaca ttgccacata attgacttgc cattttatgg 3360
ttaaaaacgg gcacattagg cagttgaata tgacgttacc ttgcagacta aaaggttgaa 3420
ggcccgaaac taacttttag ctaacaataa gggctgtgcc ccaatggaaa ctgagttcat 3480
tttctgagaa aggtttggat gactgaaata tttcctctac agtcaaggac tttggcatgc 3540
ggtggctgaa actgagcttt tttgtgtggg ctccagttct cactgttctg caatgctcat 3600

ggcaagttga atggtgagct agcttataaa ttaaagagct ctgaactgta ttcagaccga 3660
ctgggtatct agcttactgt tttaacatca ttgttgaaac cagaccctgt agtccagtgg 3720
tgctgccctg ttgtgcaaac tgctcctttt tctcgtgttt ttgtaaagag cttccatctg 3780
ggctggaccc agttcttgca catacaagac accgctgcag tcagctagga cctttccgcc 3840
atgtattcta ttctgtagta aagcatttcc atcaacaatg cctaattgta tctgttattt 3900
ttggtttaac acacactgat tcatactaat aaatattttc agttttaaaa aaaaaaaaaa 3960
aaaaaaaaa 3969

<210> 125
<211> 1424
<212> DNA
<213> Homo sapiens
<400> 125
ggccggtggc gggactctgg ggaaaatggc tgcgtcttcg agtggtgaga aggagaagga 60
gcggctggga ggcggtttgg gagtggcggg tggtaacagc acacgagagc ggctgctgtc 120
tgcgcttgag gacttggagg tcctgtctag ggaacttata gaaatgctgg caatttcaag 180
aaaccaaaag ttgttacagg ctggagagga aaaccaggtc ctggagttgt taattcaccg 240
agatggggaa tttcaagaac taatgaaatt ggcacttaat cagggaaaaa ttcatcatga 300
aatgcaagtt ttagaaaaag aagtagagaa gagagacagt gatattcagc agctacaaaa 360
acagctaaag gaagcagaac aaatactggc aacagctgtt taccaagcga aggagaaact 420
caagtcaata gaaaaagcaa gaaaaggtgc tatctcctct gaagaaataa ttaagtatgc 480
acataggatc agtgcaagta atgctgtatg tgctccactg acctgggttc caggggaccc 540
ccggagaccc tacccaactg atttagagat gagaagtggg ttactgggtc agatgaacaa 600
tccttccact aatggcgtga atggccattt accaggagat gcacttgcag caggaagatt 660
gccagatgtc cttgctccac agtatccatg gcagtcaaat gacatgtcga tgaatatgtt 720
accaccaaat catagtagtg actttttgtt ggaacctcct gggcataata aagaaaatga 780
agatgatgta gagattatgt caacggactc ctcaagcagt agtagtgagt ctgattgaaa 840
aacttaaaag acaatataca gaattgaata ctgtagaatt ctgtttcttt aacagtagca 900
gggaaatgta aactacaggt gacaaaaaat acccaggtaa acactggctt tggtagaatt 960
gtgcagtcat taaaagtcaa aatttttga ctttcttttt aaagccaaag accatagttt 1020
tagttttaag ccactaggta gatatttagg ggaatagtca aaatttactg ttgaaaaagc 1080
agttgctatg tgctttctta ccctgttctg ttccagtttt gctggatttg tacatagcca 1140
ttctagaaat agagttgagg gaaattatcc atatacatat cactaatagg cttcttggaa 1200
ttatttagaa aagcattttt aaactggcag tggatgactg aataggcatc atatttcttt 1260
ttgtgtgtca tttaaaagta acaaaaactg ccatttgaca gtaaaggctc ttggcttctg 1320
ttggaggcat gggaaattgt ctcaatttgt acagtttgta attgtaattt ttgtaaataa 1380
atttgtttgt acattgaaaa aaaaaaaaaa aaaaaaaaaa aaaa 1424

<210> 126
<211> 2836
<212> DNA
<213> Homo sapiens
<400> 126
ctccctcagc gtccggccga ggcgcggtgt atgctgagcc gctgccgcag cgggctgctc 60
cacgtcctgg gccttagctt cctgctgcag acccgccggc cgattctcct ctgctctcca 120
cgtctcatga agccgctggt cgtgttcgtc ctcggcggcc ccggcgccgg caaggggacc 180
cagtgcgccc gcatcgtcga gaaatatggc tacacacacc tttctgcagg agagctgctt 240
cgtgatgaaa ggaagaaccc agattcacag tatggtgaac ttattgaaaa gtacattaaa 300
gaaggaaaga ttgtaccagt tgagataacc atcagtttat taaagaggga aatggatcag 360
acaatggctg ccaatgctca gaagaataaa ttcttgattg atgggtttcc aagaaatcaa 420
gacaaccttc aaggatggaa caagaccatg gatgggaagg cagatgtatc tttcgttctc 480
tttttgact gtaataatga gatttgtatt gaacgatgtc ttgagagggg aaagagtagt 540
ggtaggagtg atgacaacag agagagcttg gaaaagagaa ttcagaccta ccttcagtca 600
acaaagccaa ttattgactt atatgaagaa atggggaaag tcaagaaaat agatgcttct 660
aaatctgttg atgaagtttt tgatgaagtt gtgcagattt ttgacaagga aggctaattc 720
taaacctgaa agcatccttg aaatcatgct tgaatattgc tttgatagct gctatcatga 780
cccctttta aggcaattct aatctttcat aactacatct caattagtgg ctggaaagta 840
catggtaaaa caaagtaaat ttttttatgt tcttttttg gtcacaggag tagacagtga 900
attcaggttt aacttcacct tagttatggt gctcaccaaa cgaagggtat cagctatttt 960
tttttaaatt caaaaagaat atccctttta tagtttgtgc cttctgtgag caaaactttt 1020
tagtacgcgt atatatccct ctagtaatca caacatttta ggatttaggg atacctgctt 1080
cctcttttc ttgcaagttt taaatttcca accttaagtg aatttgtgga ccaaatttca 1140
aaggaacttt ttgtgtagtc agttcttgca caatgtgttt ggtaaacaaa ctcaaaatgg 1200
attcttagga gcattttagt gtttattaaa taactgacca tttgctgtag aaagatgaga 1260
aaacttaagc tttgttttac tacaacttgt acaaagttgt atgacagggc atattctttg 1320
cttccaagat ttgggttggg ggcactaggg gttcagagcc tggcagaatt gtcagcttta 1380
gtctgacata atctaagggt atggggcaag gatcacatct aatgcttgtg tccttatact 1440
ctattatata gtgttattca tgattcagct gatcttaaca aaattcgtag cagtggaacc 1500
ttgaaatgca tgtggctaga tttatgctaa aatgattctc agttagcatt ttagtaacac 1560

ttcaaaggtt ttttttgtt tgttttctag acttaataaa agcttaggat taattagaag 1620
aagcaatcta gttaaatttc ccatttgtat tttattttct tgaatacttt tttcatagtt 1680
atttgtttaa aaagatttaa aaatcattgc actttggtca gaaaaataat aaatatatct 1740
tataaatgtt tgattccctt ccttgctatt tttattcagt agatttttgt ttggcatcat 1800
gttgaagcac cgaaagataa atgattttta aaaggctata gagtccaaag gaatattctt 1860
ttacaccaat tcttccttta aaaatctctg aggaatttgt tttcgcctta cttttttttc 1920
ttctgtcaca atgctaagtg gtatccgagg ttcttaatat gagatttaaa atcttaaaat 1980
gtttcttatt ttcagcactt acatcatttg gtacacaggg tcaaataggg caaataattt 2040
tgtctttgta taatagattt gatatttaaa gtcactggaa ataggacaag ttaatggatg 2100
tttttatatt ttaatagaat catttatttc tatgtgttat gaaattcact taatgataaa 2160
tttttcaaca tacttgccat tagaaaacaa agtattgcta agtactataa catattggcc 2220
actaaaattc atattgagat tatcttggtt tcttggaaga gataggaatg agttcttatc 2280
tagtgttgca ggccagcaaa tacagaggtg gtttaatcaa acagctctag tatgaagcaa 2340
gagtaaagac taaggtttcg agagcattcc tactcacata agtgaagaaa tctgtcagat 2400
aggaatctaa atatttatag tgagattgtg aaagcaacct taaagttttg aagaagactg 2460
atgagactag gtgctttgct tcctttcatc aggtatcttt ctgtggcatt tgagaacaga 2520
aaccaagaaa catggtaatt actaaattat gaggctttgc tttttgtttg cttttaagta 2580
gaaaaacatg ttggcaacat tgagttttgg agttgattga gataatatga cttaactagt 2640
tttgtcattc catttgttaa agatacagtc accaagaatg ttttgagttt tttgaaagac 2700
cccaatttaa gccttgctta tttttaaatt atttccattc agtgatgttg gatgtatatc 2760
agttatttag taaataatct caataaattt tgtgctgtgg cctttgctaa aaaaaaaaaa 2820
aaaaaaaaaa aaaaaa 2836

<210> 127
<211> 1677
<212> DNA
<213> Homo sapiens
<400> 127
ggcacgaggg ggtgagggtc tgcctgcctg tttctgtctg ggtgtctgag ttccaaaaaa 60
tgtgtgttgt ttgttcctcc tcatcctctt ctgagactgt tgttttttta gagcttgatt 120
gtgggagaaa agcttgtgtg aaattccctc ttcacctccc caccccccaa aaaataaaag 180
ggggcataaa ctttattcca ctggagaagc cctgggggtg gctggagcca gcctgctgag 240
aagcgtggtg cagctgggtc tgggacttca ctagagctta ctctggagca cctattttct 300
gtgcacatgg gagtgctcca ctccctagca gagcagaggg aagtctctgc caggtaccca 360
cgcccacaggc ctcaggatgg ctttttgtct gagggcctca ttaggcccag actgctgctg 420
ctggtaccag tctcctgaga cactgcctcc ctggagccca tgcatgccca gctgttctta 480
ctgcttagta gccttgaagc agcacatctc cactgtccct ggcaggattg tgggaggctt 540
cacaacccct gctttcctga cttcctcttc tgcccaacac tgggtgcccc ttcccagttt 600
cccagcaggg gcttcatggg ccaccgtcag tgggtctggg cctgccagag tcgtcttgct 660
cttcctcttg cttctaggca accacacttg gcaagttaaa tgtcccaagc accttgtctc 720
ccttcccaag aacaaaccat ttctgtgcac atccttggag gcgagatgag ctccttgcag 780
agggccagag taagtgcaag tcatggagtg caggcagagg ggtccatggc ttccccagcc 840
cagggagggt atcagtatca ttcattctct ctcccctcac tggtatgggt tcagagcaat 900
gcctagggcc cctctctgct cccctgccta gtggggtggt agtggctacc tctcagcccc 960
aatagtccct gctcctctta gaatcttcac caagtggcag gccacctctg ggcaggacca 1020
cctgcgtctg gcacccagga ggtgaggcag acaccatggt taggtgatag ggtctgaacc 1080
cagttgggga gacagagggg ttcagacttg ggaagaatct gtcagtgtgc atggagtgag 1140
gtgtgtgtgt gtggatggat gtgcacgtgt gcatgctttt tttctttttg ccgtgaggac 1200
agttgaattt ggggcatttt tctacaagaa gcagtcagcc ttctctcccc cataaggact 1260
ggctgtaacc ttaagcccat caaagttact tccagcctgg agagaacctc accaaacagc 1320
tggtgtggcc tgagggtggc catggtggga aatgggcatg agaataaata cctccccaaa 1380
ttcaatctga gcccagcagg agaggatggt agggttgcca gggctcagaa gtgcaagctg 1440
attactcacc ccaccctgcc tcgccgcacc tttcctttgt ttccttgggt gaatgcagga 1500
gcagcagtgg ctgcccttcc cacctggaca gtggtgtgtg tagaaagcca gctggatgtt 1560
tgtggtgggg cctcatggtg cctaggagga gataaagatg aggaggtttt tccttattgt 1620
ataaatgaat atttgtatga ttaaattaac acacacacca aaaaaaaaaa aaaaaaa 1677

<210> 128
<211> 3837
<212> DNA
<213> Homo sapiens
<400> 128
atcgcctcct ccctccccaa gatggcgtcc ttgctgcagt cggaccgggt tctctatcta 60
gtccagggag aaaagaaggt tcgggccccg ctctcgcaac tctacttctg ccgctattgt 120
agcgaactgc ggtcgctgga atgtgtgtct cacgaggtgg actcccatta ttgtcccagt 180
tgtttagaaa atatgccatc ggctgaagcc aaactaaaaa agaatagatg tgccaattgt 240
tttgactgtc ctggctgcat gcacaccctc tctactcggg ccacgagcat ctccacacag 300
cttccagatg acccagccaa gaccaccatg aagaaagcct attacctggc atgtggattt 360
tgtcgctgga cgtctagaga tgtgggcatg gcagacaaat ctgtagctag tggcggttgg 420

```
caggaacctg aaaatcctca cacacaacgg atgaacaaat tgattgaata ttaccagcag     480
cttgctcaga aagagaaggt tgagcgagat cgcaagaaac tggcacgacg tagaaactat     540
atgcctctgg ctttttcgga caaatatggt cttggaacca ggcttcagcg accacgagct     600
ggtgcatcca tcagtaccct tgccggactt tcccttaaag aaggagagga tcagaaagag     660
ataaagattg agccagctca ggctgtggat gaagtggaac ctctacctga agactattat     720
acaagaccag taaatttaac agaggtaaca acccttcagc agcgtctgtt acagcctgac     780
ttccagccag tctgtgcttc acagctctat cctcgccaca aacatcttct gatcaaacgg     840
tccctgcgct gccgtaaatg tgaacataat ttgagcaagc cagaatttaa cccaacgtca     900
atcaaattca aaatccagct ggtcgctgtc aattatattc cagaagtgag aatcatgtca     960
attcccaacc ttcgctacat gaaggagagc caggtcctcc tgactcttac aaatccagtt    1020
gagaacctca cccatgtgac tctcttcgag tgtgaggagg gggaccctga tgatatcaac    1080
agcactgcta aggtggtggt gcctcccaaa gagctcgttt tagctggcaa ggatgcagca    1140
gcagagtacg atgagttggc agaacctcaa gactttcagg acgatcctga cattatagcc    1200
ttcagaaagg ccaacaaagt gggtattttc atcaaagtta caccacagcg tgaggagggt    1260
gaagtgaccg tgtgcttcaa gatgaagcat gattttaaaa acctggcagc ccccattcgc    1320
cccattgaag aaagtgacca gggaacagaa gtcatctggc tcacccagca tgtggaactt    1380
agcttgggcc cacttcttcc ttaaaaggtt ccactggagg gcagatccca aaggacagta    1440
tcaccgtaaa cctgcgttaa aatgtggaag ctgctgcttc attaggcctt gtttataacg    1500
atgtacccat gcactacgga attctattgc taagaaagtg ggagcatagg caaggcattg    1560
ggaacacagg gtagctgctg ttgctcttgc tctcacccct gttgacacca gtaagtctgt    1620
gtcttcctca ctgaaccctg cacgttgagt aacagcagca taattccatc ctaggaaagg    1680
gggatgggtg ttccttggaa atggcattgt atttaccacc tgagaaactc tgtactgtct    1740
cttgatctga tctcactaag gatcacaatg tcacagatga aacttaaatg ataacccaaa    1800
ggtagacctg ctgttaatga tccagcattg gtcacaatgt accaactgct ttctgcattc    1860
cgttaaatat catctaacag tctaaaacat atcccttcat tgccataatg gctgccattt    1920
tgccatagat ttccatataa ctgaaaaact gaattgtcac tttatcttta gtatcatgat    1980
gattggaaaa acctgtgaag ttgttaaggc actctcattt gccctctttt tctaagtgaa    2040
tacaggacac gtattagttg ttcttaattt ttttcccagt aaaatatgga tcttttaaga    2100
agaatttgag aagcaaacaa ttacatgtca tgtcaagggg gtagcagatt ccattcgttt    2160
tcaatattgc cacaataccc agggattaat gctgccacag gggggcaatc tttatttgtc    2220
ttacttccta ccccttccct gttctgcctc tttaactcag ttaagttgtt ctgtttggga    2280
cctggaaaag aacccaaaga aaacctgagt ggacaggttc atttctggaa tgcagaaaac    2340
attttaaagg ctagattttt agaatattct caactagcat tctttccatt gatttgaagg    2400
ggaaattaac tattataatc tcttgaatcc aaaactggat attaagaact ttccccctta    2460
ctaagtttaa gacttttgtc atgtggtgag tcaaataaga ccattttgat tgtaaaccat    2520
aaaatagttc agcaagtagc ccacagttct ggcctaacag cagacttgct gttttcactt    2580
ggtatcctgg agttgggttg ctaaccttaa tttctatgat gttttctaaa atgaaacttg    2640
ataaagtaga ccaccagctg caccgtgttt tctgtaaaag tattgttagt aagtggccaa    2700
gagacttgag gaaaatacag attttttgtt taccttggtc ttgttttaag tcttaaaaaa    2760
ttaaagataa cattataatg tagaatacag atgggacata gtccttgtaa gcttcccttg    2820
aaaatgtttt aaatatttag gaagctttta aaagacacta aattgtactc taaaagacac    2880
taaattgtac taattgtaca aaggtcaagc caattttatg aaacagtcct acagagtaat    2940
atatgtgatg cagtgtaaga aggaaaatac tcatctctaa cattatggta ataacattta    3000
gcctcttagg agttggagca gggggatggg taattacaga tttgcagact atagaaagag    3060
tttcattttt ttgtgacccc acagagtctc aaatttttat tccactacct gctagagcct    3120
actgtgaaat cactgctcca tatttgccag tggaggaaat gggcatagag tagagaatag    3180
cttcatatgt ttacacgttt gcatagacta cacacatgtc atgcgtttat ggcaggtagc    3240
tggtatttat tccccaaagt aataatgttg aagtatgggt ctcatcattc ccatacacag    3300
aaacacaaaa cactttgatc ataaactttt ttcttcagaa gccaaactaa cttgcagaat    3360
aatagagcca ctggtttaat gtttcctcaa gataggtttt agtgtaagct agtattctgt    3420
gtgttcgtag aaatgattca atacctgcag ctggtgaatt aggaattgta tttgttgcct    3480
tttttatatt agatgaggtg caaaaatttt aatgctagtc agtatgcacc accacaggaa    3540
agttagatcc cattagcact tgagactaca gctttggaaa cttaggctaa gttaatttgg    3600
atttgttact tgattcacct actgaccttt tcttttgttt gaagtgctta tcagcataat    3660
gagctaagtg tcatgcatat ttgtgaagaa acacccttgt ttggtccctt ttgggacaga    3720
gaggtactcc ttgatcttta tgaatgaccg gttactgttt tgccttattg cttaacttaa    3780
tgtagtgaaa taaagcagac caagcttgaa aaaaaaaaaa aaaaaaaaaa aaaaaaa       3837
```

<210> 129
<211> 4020
<212> DNA
<213> Homo sapiens
<400> 129

```
gtcaattgcc ctttagtccc aggactaacc ggaagcttct gcaacaggag gacattgaaa      60
ataagatgga acccatccac ataaggattt gcctcaaagg gcactgcaaa aattgaacag     120
aggaatccca aggaagctgc ctgaatttgc ctgtatactc tcgttctgcg acttataaag     180
gaccagacaa atcaaattag tggttttggt ttccgccagc tgtggatgcc tttgacatta     240
tgaccgcaga ggattccacc gcagccatga gcagtgactc ggccgccggg tcctcggcca     300
aggtgcccga gggcgtggcg ggcgcgccca acgaggcagc actgctggcg ctgatggagc     360
```

```
gcacgggcta cagcatggtg caagagaacg ggcagcgcaa gtacggcggc ccaccgcccg   420
gctgggaggg cccgcacccg cagcgtggct gcgaggtctt cgtgggcaag atcccgcgcg   480
acgtgtacga ggacgagctg gtgcccgtgt tcgaggccgt gggccgcacc tacgagctgc   540
gcctcatgat ggactttgac ggcaagaacc gcggctacgc cttcgtcatg tactgccaca   600
agcacgaggc caagcgcgca gtgcgtgagc tcaacaacta cgagatccgc ccgggccgcc   660
tgctcggcgt gtgctgcagc gtggacaact gccgcctctt catcggcggg atccccaaga   720
tgaagaagcg cgaggaaatc ctggaggaga ttgccaaggt caccgagggc gtgctggacg   780
tgatcgtcta cgccagcgcg gccgacaaga tgaagaaccg cggcttcgcc ttcgtggagt   840
acgagagcca ccgcgcggct gccatggctc gccgcaagct catgcctggc cgcatccagc   900
tgtggggcca ccagatcgcc gtggactggg ccgagcctga gatcgacgtg gacgaggacg   960
tgatggagac cgtgaagatc ctctacgtgc gcaacctcat gatcgagacc accgaggaca  1020
ccatcaagaa gagcttcggc cagttcaacc ccggctgcgt ggagcgcgtc aagaagatcc  1080
gcgactacgc cttcgtgcac ttcaccagcc gcgaggatgc cgtgcatgcc atgaacaacc  1140
tcaacggcac tgagctggag ggctcgtgcc tggaggtcac gctggccaag cccgtggaca  1200
aggagcagta ctcgcgctac cagaaggcag ccagggggcgg cggcgcggct gaggcagcgc  1260
agcagcccag ctacgtgtac tcctgcgacc cctacacact ggcctactac ggctacccct  1320
acaacgcgct cattgggccc aacagggact actttgtgaa agtagccatc cctgccattg  1380
gggctcagta ttccatgttt ccagcagctc cagcccctaa aatgattgaa gatggcaaaa  1440
tccacacagt ggagcacatg atcagcccca ttgctgtgca gccagaccca gccagtgctg  1500
ctgccgccgc agccgcggcc gcagccgccg cagccgctgt cattcccact gtgtcgacgc  1560
caccaccttt ccagggccgc ccaataactc cagtatacac ggtggctcca aacgttcaga  1620
gaattcctac tgccgggatc tacggggcca gttacgtgcc atttgctgct ccagctacag  1680
ccacgatcgc cacactacag aagaacgcgg cagccgcggc cgccgtgtat ggaggatacg  1740
caggctacat acctcaggcc ttccctgctg ctgccattca ggtccccatc cccgacgtct  1800
accagacata ctgaggctgg tgaccagcac gaagacagac cacacaaaca ccactgaagg  1860
aacgcttgac tatttatgaa gaaggaacat gttggattca cacatgcaac ctgaaagtga  1920
agaatgttag cagatttatt tctgaattat tttatataca tgaagttttc actagttttt  1980
taagactatt ttcaacttag catgcctacg ttcatacatt tccaaaagac ttgcaatggt  2040
tcgtgccttc attccatctt ttaaaaattt gtatgctgta ctacatttgt atagaggttt  2100
ttgttgttgt tttttaagg atatattttc agtatgaagg ttattttctt aacttctgca  2160
ctccagagat ttctattttg tagtaccttc aataatatat caactatata ttaaaaaagc  2220
acacttgagg agctagggaa ctattttgaa aaatatatac aatatttaaa gatacaaaca  2280
gtagtgctta aaaatactac ataaagcatt attttaaagg ttatactgga aagtgcaatt  2340
ttaaaatgag taaaacctct gtatttctgc tggcattaag ggttgatggt gttaccatgt  2400
atcatcatgg cggtactatt ttttaaaaga aattaaacac tggatctctc cttaagccaa  2460
cattgaaaag acttgccgca cttctgagtc caaacactgg aaagctctcc ttgccaccgt  2520
tagccggggc tcattctcca tgtgccttag ccttaaacat gcccccactc ccacatctct  2580
caccctgtcc cctcctcccc agattcccaa tcccaccgca atgtttggca agcctaggac  2640
tgataagtag ctctgataga ggagctggtg gcttttatac ttcttcctgg gtttttgttg  2700
gggtttgttg tttcgttgtt ttttgttttt tttttgtttg gttggggaag tattgtcttc  2760
tacgtgtgct attttcagta gcagagtaag cacaaggttt taatcgagtt gcataagaca  2820
cctttgcata gctatttaat tgcccaatgt aaaactttaa tgccatttct aatgctttta  2880
ttcatttttg aagtatgagt ttgtagggac aaagaatgta tgttatcgta gacaagaccc  2940
ccagagactc ttttcagcag aaagttatgc ttctagttgc cttaccatgt ttcttgcaaa  3000
actgtccatg gtcctcaagg gtgttggaaa cattatgttt attaaatggg cctctcttcc  3060
tttgctgtgc acttgatggg tgaactggat tggggtgtgc acatccagga ggaggaggag  3120
agacctgtag aagtttaaag atagtttgta aatatcttct aatgcttgtt tttagtcctt  3180
ttatgttgga gaagttcatg gtatgtagtt taatgcaaaa tgaaaccatt ttatttcaat  3240
gttattaaaa aggtttgttt tattaggaag ttaatgtatt gttgcagtgt tttgtgcctg  3300
tttaaaggct tttgtttagc agagtgaatg taaaatacag taaaatgtta agattgtcat  3360
ctacttttta aaaaaaaata tcaacttgga attgtttttt aaaggctcaa tcaaggaagt  3420
gaggtgtgca ataaggtagc aagtaaaacg cagttgcgtt tttatgtcat gttagagatc  3480
catacaattt tccactcacg ggatttttgt tgatggctga attcttgtgg attcataaga  3540
ggatcatgcc cttagcaagt acttttgttt tgttttaaat taagagattc ccaaatgcct  3600
ttttcccccct catcttgaaa tgagatgagt ttttatgtgt aagcaatatt tatttaacta  3660
ttctataaaa ttattgagtg cctactgagg cctttaagca ccgctaacat tcctttccat  3720
cattcttttg aatgacataa aataattgtg caatgttcct gatgatgtac cccacagctg  3780
cattcaaact caaatctgtg ggaatgagtg actcgaccaa atgtaattcg gatcagatcc  3840
tcatcccctg actgtgtgaa aaaagtactc tccttctagt gaaggattgt cacagagttt  3900
cactggatga aactatgacc cagtattctt actgtatttt acatgtgcct gtaaattatt  3960
ttgccgaaat aagaagaaga agaggaagaa agaacagtag aaaaaaaaaa taaaaaaaaa  4020
```

<210> 130
<211> 4348
<212> DNA
<213> Homo sapiens
<400> 130

```
gggaggccca gggagaacgg ggaagggaca tttagtttga gacggtgctg agataggatc    60
atgaaggaag aggtgaaggg aattcctgta agagtggcgc tgcgttgtcg ccctctggtc   120
```

```
cccaaagaga ttagcgaggg ctgccagatg tgcctttcct tcgtgcccgg agagcctcag    180
gtggtggttg gtacagataa atccttcacc tacgattttg tatttgatcc ctctactgaa    240
caggaagaag tcttcaatac agcagtagcg ccactcataa aaggtgtatt taaaggatat    300
aatgcaacgg tcctggccta tgggcagact ggctctggaa aaacctattc aatgggaggt    360
gcatatactg cagagcaaga gaatgaacca acagttgggg ttattcctag ggtaatacaa    420
ctgctcttca aagaaattga taaaaagagt gactttgaat ttactctgaa agtgtcttac    480
ttagagattt acaatgaaga aattttggat cttctatgtc catctcgtga gaaagctcaa    540
ataaatatac gagaggatcc taaggaaggc ataaagattg tgggactcac tgagaagact    600
gttttggttg ccttggatac tgtttcctgt ttggaacagg gcaacaactc taggactgtg    660
gcctccacgg ctatgaactc ccagtcgtcc cgatctcatg ccatctttac aatctcctta    720
gagcaaggaa agaaaagtga caagaatagc agctttcgct ccaagctgca tcttgtagac    780
ctcgctggat cagaaagaca gaagaaaacc aaggctgaag gggatcgtct aaaagagggt    840
attaatatta accgaggcct cctatgcttg ggaaatgtaa tcagtgctct tggagatgac    900
aaaaagggtg gctttgcgcc ctacagagat tccaagttga ctcgactgct tcaagattct    960
ctaggaggta atagccatac tcttatgata gcctgtgtga gtcctgctga ctccaatcta   1020
gaggaaacat taaataccct tcgctatgct gacagagcaa gaaaaatcaa gaacaaacct   1080
attgttaata ttgatcccca gacagctgaa cttaatcatc taaagcaaca ggtacaacag   1140
ctacaagtct tgttgctaca ggcccatgga ggtaccctgc ctggatctat aactgtggaa   1200
ccatcagaga atctacaatc cctgatggag aagaatcagt ccctggtaga ggagaatgaa   1260
aaattaagtc gtggtctgag cgaggcagct ggtcagacag cccagatgtt ggagaggatc   1320
atttggacag agcaagcgaa tgaaaaaatg aacgccaagc tagaagagct caggcagcat   1380
gcggcctgca aactggatct tcaaaagcta gtggagactt tggaagacca ggaattgaaa   1440
gaaaatgtag agataatttg taacctgcag caattgatta cccagttatc ggatgaaact   1500
gttgcttgca tggctgcagc cattgatact gcggtggagc aagaagccca agtagaaacc   1560
agtccagaga cgagcaggtc ttctgacgct tttaccactc agcatgctct ccgtcaagcg   1620
cagatgtcta aggagctggt tgagttgaat aaagcgcttg cactgaaaga ggccctggct   1680
aggaagatga ctcagaatga cagccaactg cagcctattc agtaccaata ccaggataac   1740
ataaaagagc cagaattaga agtcatcaat ctgcaaaagg aaaaggaaga attggttctt   1800
gaacttcaga cagcaaagaa ggatgccaac caagccaagt tgagtgagcg ccgccgcaaa   1860
cgtctccagg agctggaggg tcaaattgct gatctgaaga agaaactgaa tgagcagtcc   1920
aaacttctga aactaaagga atccacagag cgtactgtct ccaaactgaa ccaggagata   1980
cggatgatga aaaaccagcg ggtacagtta atgcgtcaaa tgaaagaaga tgctgagaag   2040
tttagacagt ggaagcagaa aagagacaaa gaagtaatac agttaaaaga acgagaccgt   2100
aagaggcaat atgagctgct gaaacttgaa agaaacttcc agaaacaatc caatgtgctc   2160
agacgtaaaa cggaggaggc agcagctgcc aacaagcgtc tcaaggatgc tctccagaaa   2220
caacgggagg ttgcagataa gcggaaagag actcagagcc gtggaatgga aggcactgca   2280
gctcgagtga agaattggct tggaaacgaa attgaggtta tggtcagtac tgaggaagcc   2340
aaacgccatc tgaatgacct ccttgaagat agaaagatcc tggctcaaga tgtggctcaa   2400
ctcaaagaaa aaaaggaatc tggggagaat ccacctccta aactccggag gcgtacattc   2460
tcccttactg aagtgcgtgg tcaagtttcg gagtcagaag attctattac aaagcagatt   2520
gaaagcctag agactgaaat ggaattcagg agtgctcaga ttgctgacct acagcagaag   2580
ctgctggatg cagaaagtga agacagacca aaacaacgct gggagaatat tgccaccatt   2640
ctggaagcca agtgtgccct gaaatatttg attggagagc tggtctcctc caaaatacag   2700
gtcagcaaac ttgaaagcag cctgaaacag agcaagacca gctgtgctga catgcagaag   2760
atgctgtttg aggaacgaaa tcattttgcc gagatagaga cagagttaca agctgagctg   2820
gtcagaatgg agcaacagca ccaagagaag gtgctgtacc ttctcagcca gctgcagcaa   2880
agccaaatgg cagagaagca gttagaggaa tcagtcagtg aaaaggaaca gcagctgctg   2940
agcacactga agtgtcagga tgaagaactt gagaaaatgc gagaagtgtg tgagcaaaat   3000
cagcagcttc tccgagagaa tgaaatcatc aagcagaaac tgaccctcct ccaggtagcc   3060
agcagacaga aacatcttcc taaggatacc cttctatctc cagactcttc ttttgaatat   3120
gtccagccta agccaaaacc ttctcgtgtt aaagaaaagt tcctggagca aagcatggac   3180
atcgaggatc taaaatattg ttcagagcat tctgtgaatg agcatgagga tggtgatggt   3240
gatgatgatg agggggatga cgaggaatgg aagccaacaa aattagttaa tgtgtccagg   3300
aagaacatcc aagggtgttc ctgcaagggc tggtgtggaa acaagcaatg tgggtgcagg   3360
aagcaaaagt cagactgtgg tgtggactgt tgctgtgacc ccacaaagtg tcggaaccgc   3420
cagcaaggca aggatagctt gggcactgtt gaacggaccc aggattcaga aagctccttc   3480
aaactggagg atcctaccga ggtgacccca ggattgagct tctttaatcc cgtctgtgcc   3540
acccccaata gcaagatcct gaaagagatg tgcgatgtgg agcaggtgct gtcaaagaag   3600
actcccccag ctccctcccc ttttgacctc ccagagttga aacatgtagc aacagaatac   3660
caagaaaaca aggctccagg gaagaaaaag aaacgggctc tggccagcaa caccagcttc   3720
ttctctggct gctcccctat cgaagaagag gcccactgaa gttggagtca tcatctctac   3780
ccccagtctg gcttgggaga tgctttcagg ttgcagccag aaggggtttt ttaaatgact   3840
tctctggatt tcaggtttct tgctgttgaa aaaaggaaca aagcgttact gaaaagaagg   3900
taacctttgt tggatgtggg ccttagcctc caggtccaga ctactactct atgttctcca   3960
gaagggtgct aagtcaccta ctgaagagag aaccaactga ctttcctatt gactcatcag   4020
gaaccagtcc tcagtctggt caagttgttt cttatttgtg agcagttcag gctatctcct   4080
gatggggatg aggccaaggc tttcttatct tttggttgtc tctgcttaat ggaggagcct   4140
ggcctaggat ggaggcctgg cttagatctt tcattccacc tcaggaatga ggttgtgatc   4200
tttcctgtcc tgaccctctc tgaattatgt ttcaatagta ctcttgattg tctgccatgt   4260
```

tgttgaagca aatgaattat ttttaaatgt taagtaagta aataaacctt agcccgtctt 4320
tttttttttt tttttttttt tttttttt 4348

<210> 131
<211> 1919
<212> DNA
<213> Homo sapiens
<400> 131
ggcgtgggct ccccttccccc tctgtgggtc ccgcgaggag actctcgggc tttgaggtga 60
gacctgaagt tccgctggcc ggtagtgtag caggaaaggg caggtcctcc cgggtcgtga 120
gccagtagcc tcctggggtg gcaaggtgta gagagggggg cgttgaaagg acacccgcta 180
cccggcctgc tttctagggg tctctttgga ttgaggacat cagcagcagt ggaagggatt 240
ttactggaga cctgtcactg tcagagcctt aaaatatcac cgacggggcc ttaatgtcac 300
cgaggtagag agaaaagggc agtagcccta gagactattg cgacacagtg tgcccctcat 360
aagtttttcc agggaggggt tctgtactga gttgacgccc caggagctga gcaccaggct 420
ttgcatcctt gggaactcag caaacgtttg ttcagccaat tgcaggtagc atggcccagg 480
gcttgattga ggtggagcga aagttccttc cagggcctgg cacagaggag cggctgcagg 540
agttgggggg caccctggag taccgggtca ccttccgaga cacctactat gacacccctg 600
agctgagcct catgcaggct gaccactggc tgcgacgacg agaggatagt ggatgggagc 660
tcaaatgtcc tggagcagca ggtgtcttag gaccccacac ggagtataag gaactcacag 720
cggaacctac aattgtggcc caactctgta aggtgctgcg ggctgacggc ctgggggctg 780
gagatgtggc tgctgtgctg ggcccactgg ggctgcagga agtagctagt tttgtgacta 840
agcggagtgc ctggaagctg gtgctcttgg gagctgatga agaggagcca cagctcaggg 900
tggacttgga tacagccgac tttggctacg ctgtgggtga ggtagaggcc ctggtgcatg 960
aggaggctga agtaccaact gccctagaga agatccacag gctcagcagc atgcttggtg 1020
tgcctgcaca ggagacagca ccagccaagc tgattgtgta tctacagcgt ttccggcctc 1080
aagactatca gcgcctgcta gaagtgaaca gctccagaga gaggccacag gagactgaag 1140
atcctgacca ctgcctgggc taggggtgtc acttcctaga aggggaaggg aactctgggt 1200
ctaacggagt ccactcctgg gcccactgtg cctctcccct cagtgtccct tctgacagtg 1260
actcctctct ctccagcgct gcctgtttct tccccctcct ctaagctact cttccttgag 1320
ccctccctgg ctgcttcctc tcgctcatcc gtcagatgca atctgtgggc cgcccctctc 1380
ccctggccgc taagcagcct ccattgattt ccgctcgtgt ttataggatt tccacttagc 1440
cgtgatcagt agttaagcac aggaaaatcc cttgccaccc cccctccctt ggtcgatgcc 1500
attgattctg ccagcggctc ctaaaccgcc ttacagctga gttagaagat gaggagaggc 1560
agcagggatt tccctgcctt gggatgtggg aaacagaaga aaagttgagg aaatggttgg 1620
gaatcgctgt tagaactcta gaaattctaa tctgactttg ccactgtgcc cagctctggc 1680
ccagagggta gagtgcctcc tgtggaagtt taggggcaaa tttgttccct gacctggaga 1740
gggttagaaa gaaccaacag ctgcccccctc cccgcccccg tgttgagaca ggttctcaag 1800
gctcagggga agatgcatac ctcctatgta agaatgctct tcccttgctg ttattcatac 1860
acactttcca cttcaaatat acccaaatat ggctttcctc aaaaaaaaaa aaaaaaaaa 1919

<210> 132
<211> 3206
<212> DNA
<213> Homo sapiens
<400> 132
gtttataact tgaaaaatcc tctccgtctc ccttccctgc ctcctttcct ttccctttcc 60
tctgccagta caactagacc cggcgtctgg cgtccccggt gcccagcatt ctgcggggca 120
ggcggattaa ttggaattct tcaaaatgtc aggtgtggta cccacagccc ctgaacagcc 180
tgcaggtgaa atggaaaatc aaacaaaacc accagatcca aggcctgatg ctcctcctga 240
atacagttct catttttac caggaccccc tggaacagct gtccctccac ctactggcta 300
cccaggaggc ttgcctatgg gatactacag tccacagcaa cccagtacct tccctttgta 360
ccagccagtt ggtggtatcc atcctgtccg gtatcagcct ggcaaatatc ctatgccaaa 420
tcagtctgtt ccaataacat ggatgccagg gccaactcct atggcaaact gccctcctgg 480
tctggaatac ttagttcagt tggacaacat acatgttctt cagcattttg agcctctgga 540
aatgatgaca tgttttgaaa ctaataatag atatgatatt aaaaacaact cagaccagat 600
ggtttacgtt gtaaccgaag acacagatga ctttaccagg aatgcctatc ggacactaag 660
gcccttcgtc ctccgggtca ctgattgtat gggccgagaa atcatgacaa tgcagagacc 720
cttcagatgc acctgctgtt gcttctgttg cccctctgcc agacaagagc tggaggtgca 780
gtgtcctcct ggtgtcacca ttggctttgt tgcggaacat tggaacctgt gcaggcggt 840
gtacagcatc caaaatgaga agaaagaaaa tgtgatgaga gttcgtgggc catgctcaac 900
ctatggctgt ggttcagatt ctgtttttga ggtcaaatcc cttgatggca tatccaacat 960
cggcagtatt atccggaagt ggaatggttt gttatcagca atggcagatg ctgaccattt 1020
tgacattcac ttcccactag acctggatgt gaagatgaaa gccatgattt ttggagcttg 1080
cttcctcatt gacttcatgt attttgaaag atctccacca caacgttcaa gatagagaga 1140
cacagcaagc catcaactat ggttaatttt gaaaaatgga aaagttggat tgggcttaca 1200
gtcagcactc agttatttgc aagtgtattt ctttgctttg tagagtattt ttattgggtg 1260
ttaactttga cagctgagag tgggcttgca agaacacaat ctaaaagtgt gtttcaattg 1320
agtatctctc tagtagaata ggagttcatc ctgaaaagct gtgactcatt aacccagtaa 1380

acatatacaa agtaagctta aaacactata aacatgagat aagggaaaat gaatccagag 1440
ttctcatatt aataggtagt gaaacaataa ggcttttag agcagacttt gttggcataa 1500
aataacctgg cttctatccc taacccttc ctaccctttcc tctccgtcaa catgtcctca 1560
tactgaagac aaacttgttt caatgatagt cttcatttt aaaaacaaaa aggcaggcag 1620
acagaaataa tgatgttttc ttgcactaag aaggtactac ttgtacacat atatcaaaac 1680
ctcattctgc aaagtttttg aaggtttcaa tgggaaattt gattttatta caaaataaaa 1740
catttttaa tgttaaagtt tatatattcc atgcttgttt tctcattcac tggcatggat 1800
gatcaggagc tgcctatata tgaaggcaga atcagactat caggaaagga gctggccagg 1860
gccacagcca gtcaagatct ctgagcaact tagagacatt ggtgtcatta tatgaagctt 1920
gcatttaata catttataca taatacattt gtacatttaa ttcataacgt ctcttggtca 1980
cagatgcctt atatataaaa taagttgcca gatctctaag attgcctagt acacctttgt 2040
atctcatttg atgtgatacc cagaagagat cattgttttt tgtttttgtt tttgtttttt 2100
tcaagaagat ccttcgtgat caccatgctg ttctcatggt aagaactgga gttatgtttt 2160
taaatttgaa aatatgacat tttatgtagc actatataaa aagtgaaagc gacaaattcc 2220
accgctgctt aatactgctt tgcttctttt tattgacatg atagatacat atgtatctac 2280
acagagtaat aataataaaa cacagtaaac attctatttc tctatggtct acagcatgcc 2340
agtaaataat atgtaggacc aataataaat tatcaattac acatttttgt gttaactaat 2400
taaaagcata gtgtataagt gagtacactc taattaactt gcttctgttg cactttagtt 2460
ttctacctgc atatggactg catttttttt tttaacacag tcagtatgta gaatgggatg 2520
tattcttctg ctgctgctta ttaaataaag aaagcctgag tgttcttaga tggggttatt 2580
ctgagatgag ggtcttagcc tacagttctt tttgaaatga aaggtgcttt gtttttttaat 2640
tatattcatc ttttcagggt aaatttgttt ttctgagttt ctcgtaatgc tcatttttac 2700
atgctgctac tagctttttt ttttaaaaaa agtaaaagtt gctgctttct aaaatattaa 2760
ttgccttata tttgaaagtg ccattgcaat cgtaagtaga ctatgtattt cctataatga 2820
tgtctgatat ttaaatagga aatcagacaa acaatattca gaaagtttaa gcatataaac 2880
tttttatttt taacttgcct agatccctgt attccaaaac ctgctgcatc ataataaata 2940
tatctatata tatttagcat aagacgtgat atttttaatt tctttttttaa aaaattatat 3000
ttgtctctta gagttaaaat tttctttata taatattgtc atatgtcata gttttaatac 3060
aattcacatg atttctatgt ttcttaatga tattttgttg tgtaaaattg atcggattga 3120
ttaaaaaaca aattctctgg aatttgtgcg ttcatgcttt ttcgtattct ttatggcttt 3180
taaataaata tacaatggtt aatagt 3206

<210> 133
<211> 2087
<212> DNA
<213> Homo sapiens
<400> 133
gtttttttagg aagagtgtcc cgcagagacc cggcgggagc tgccaggagc tctgggattc 60
cagcggctgg aagccacctg ggaagcctgg cctcagtgtg gaagagaagg cagcaggatt 120
attacagaac cttgtgaagc caacgcgggc agccgccagg agctgcagac cgagaggatc 180
tcgtcctttc ttgcggccca gggagaccag gcctttcatt ctgggctcga gaccaacaat 240
tcgaattccg aactccccct gcgtgtggga ctcaaggttg cccagggctc acctctgatg 300
ggtgggcagg tgagcgcttc caacagcttc tcgaggctgc actgcagaaa tgccaacgag 360
gactggatgt cggcactgtg tccccggctc tgggatgtgc ccctccacca cctctccatc 420
ccagggagcc acgacacgat gacgtactgc ctgaacaaga agtcccccat ttctcacgag 480
gagtcccggc tgctgcagct gctgaacaag gccttgccct gcatcacgcg ccctgtcgtg 540
ctgaaatggt ccgtcaccca ggcactggac gtcacagagc agctggatgc cggggtgcgg 600
tacctggacc tgcggatagc ccacatgctg gagggctcgg agaagaacct gcactttgtc 660
catatggtgt acacaacggc gctggtggag gacacactca cggaaatctc ggagtggctg 720
gagcggcatc cacgcgaggt ggtcatcctg gcctgcagaa acttcgaggg gctgagcgag 780
gacctgcacg agtacctggt cgcctgtatc aagaacatct tcggggacat gctgtgtcct 840
cgtggggagg tgccgacact gcggcagctg tggtcccggg gccaacaggt catcgtctcc 900
tatgaagacg agagctcctt gcgccggcac cacgagctgt ggccaggagt cccctactgg 960
tggggaaaca gggtgaagac cgaggccctc atccgatacc tggagaccat gaagagctgc 1020
ggccgcccag gagggttgtt cgtggccggc atcaacctca cggagaacct gcagtacgtt 1080
ctggcgcacc cgtccgagtc cctggagaag atgacgctgc ccaaccttcc gcggctgagc 1140
gcgtgggtcc gagagcagtg cccggggccg ggttcacggt gcaccaacat catcgcgggg 1200
gacttcatcg gcgcagacgg cttcgtcagt gacgtcatcg cgctcaatca gaagctgctg 1260
tggtgctgac gggacccttc tgaagttcgg gacgcggcgg ctgcagtttc acccccgaat 1320
ttccaaatgg agtttcgctc tcgttgccga ggctggagtg tagtagtgtg atcctggctc 1380
actgcaacct ccacctcccg ggttccagca aattctcctg cctcagcctc ccaagtagct 1440
gggattgcag gcgcccgcca ccacgcccgg ataatttttg tgtgtttagc agagacgggg 1500
tttcaccatg ttggccaggc tggtctcgat ctcctgacct caggtgatcc acccgcctcg 1560
gcctcccaaa gtgctgggat gacaggcgtg agccaccgcg cccggcctat acctcatttt 1620
ctacatgtcg cttgttggag ctgctggttc aagttcccag ccagccaatg gatgccagca 1680
ccatttttac tcccctttcc caagcaaatc gtgcattttt gtctaacgag agacatcagt 1740
ttctcaggat gatcctcaag aacgttatgg agtccatgtt gcaataggtt ctctttggga 1800
cctaatgact cattttccaa aatccgcttt ctacttttgg tacccggttg ctacggtgaa 1860
atgaaggtgc cccgcatcca gaaagacgca ctcctggacc acaaccggcg gctacctcag 1920

ccccacggct ctgcaggatc agggctcggg caggccccgc ggagatgaag aatttgcagg 1980
gagcctccct gacttccgtc ggctgtgaat ccttgtctgt caggggcgta tccacaaaat 2040
caccaaattc atacagatcg tttaaataaa tgaacatcat taaagtc 2087

<210> 134
<211> 1401
<212> DNA
<213> Homo sapiens
<400> 134
ccgagtctca ccctcccagg cagctcctac actcaactgc ttctctagga aaggtctcac 60
ctccagcctg gagcagtcgg gattacagaa agccccatcc ttggcttagg gagcgccatg 120
acgactgaaa ttggttggtg gaagctgact ttcctccgga aaaagaaatc cactcccaaa 180
gtgctgtatg agatccctga cacctatgcc caaacagagg gagatgcaga acccccgagg 240
cctgacgctg gaggccccaa cagcgacttt aacacccgcc tggagaagat tgtggacaag 300
agcacaaagg gcaagcacgt caaggtctcc aactcaggac gcttcaagga gaagaagaaa 360
gtgagagcca cgctggcaga gaaccctaac ctctttgatg atcacgagga aggacggtca 420
tcaaagtgaa gggctgagga gggtgctagc acctcttggc tccctgccat cagccagatc 480
tgagacagga ccttgccacg ctggcctctt tggccatagc tgaagctgtg ggccagttg 540
atacctgctg gcaggaaatg gctgtttttt aggtttgtat ttatgtgccg ccacttttgt 600
aaggcctggg agatcccagg gtcctccacc ctccccctga ccacatacaa aggcactcta 660
gttcaagagt gaaaaatctc acccaggagg aacagccctc cttgaagcaa tggcagggcc 720
agcagggagg tgggcatggc agggaatgga gagagtgagc cagacagact tcacctcctt 780
actggacaca gggtcaaggg cgagtttcaa ttgctgctcc ctttactttc tctacctgtg 840
actactccct ggaccaatcc tgaggagggc acattttcca gaagccacgt gatagggggct 900
ggtttctgtg gagccagagg cagagacact gaacttgagc tcacctccta acaccggcag 960
taaacttcct ggaactttgc cctcaggtgc ggaggggaca gaggaccctg gcactctgtt 1020
agggtgctgt agaagactag attgatggta gtttggcctg ttagttcctg ttttggccat 1080
gacttttgca gatggcaagt cacacaccct caaagggaag ctacacgggc caaatcgggg 1140
gagtgggtgg ggaattttct cctctccctt tcctactata atagtattta agacatatca 1200
gctccagaga tgagtcctgg agccttgaat tttgtttaac aaaataattg taggtttctc 1260
tctgtaataa caacgctgga aaggccgaga acctctttta tgctcatgtc ttgcatttat 1320
tgagatgact gtttctcatg cctttatgtt ccttcatgta agtaaagtgg acctttgtgc 1380
tcaaaaaaaa aaaaaaaaaa a 1401

<210> 135
<211> 3737
<212> DNA
<213> Homo sapiens
<400> 135
ggcgtccgcg cacacctccc cgcgccgccg ccgccaccgc ccgcactccg ccgcctctgc 60
ccgcaaccgc tgagccatcc atgggggtcg cgggccgcaa ccgtcccggg gcggcctggg 120
cggtgctgct gctgctgctg ctgctgccgc cactgctgct gctggcgggg gccgtcccgc 180
cgggtcgggg ccgtgccgcg gggccgcagg aggatgtaga tgagtgtgcc caagggctag 240
atgactgcca tgccgacgcc ctgtgtcaga acacacccac ctcctacaag tgctcctgca 300
agcctggcta ccaaggggaa ggcaggcagt gtgaggacat cgatgaatgt ggaaatgagc 360
tcaatggagg ctgtgtccat gactgtttga atattccagg caattatcgt tgcacttgtt 420
ttgatggctt catgttggct catgacggtc ataattgtct tgatgtggac gagtgcctgg 480
agaacaatgg cggctgccag catacctgtg tcaacgtcat ggggagctat gagtgctgct 540
gcaaggaggg gtttttcctg agtgacaatc agcacacctg cattcaccgc tcggaagagg 600
gcctgagctg catgaataag gatcacggct gtagtcacat ctgcaaggag gccccaaggg 660
gcagcgtcgc ctgtgagtgc aggcctggtt ttgagctggc caagaaccag agagactgca 720
tcttgacctg taaccatggg aacggtgggt gccagcactc ctgtgacgat acagccgatg 780
gcccagagtg cagctgccat ccacagtaca agatgcacac agatgggagg agctgccttg 840
agcgagagga cactgtcctg gaggtgacag agagcaacac cacatcagtg gtggatgggg 900
ataaacgggt gaaacggcgg ctgctcatgg aaacgtgtgc tgtcaacaat ggaggctgtg 960
accgcacctg taaggatact tcgacaggtg tccactgcag ttgtcctgtt ggattcactc 1020
tccagttgga tgggaagaca tgtaaagata ttgatgagtg ccagacccgc aatggaggtt 1080
gtgatcattt ctgcaaaaac atcgtgggca gttttgactg cggctgcaag aaaggattta 1140
aattattaac agatgagaag tcttgccaag atgtggatga gtgctctttg gataggacct 1200
gtgaccacag ctgcatcaac caccctggca catttgcttg tgcttgcaac cgagggtaca 1260
ccctgtatgg cttcacccac tgtggagaca ccaatgagtg cagcatcaac aacggaggct 1320
gtcagcaggt ctgtgtgaac acagtgggca gctatgaatg ccagtgccac cctgggtaca 1380
agctccactg gaataaaaaa gactgtgtgg aagtgaaggg gctcctgccc acaagtgtgt 1440
caccccgtgt gtccctgcac tgcggtaaga gtggtggagg agacgggtgc ttcctcagat 1500
gtcactctgg cattcacctc tcttcagatg tcaccaccat caggacaagt gtaaccttta 1560
agctaaatga aggcaagtgt agtttgaaaa atgctgagct gtttcccgag ggtctgcgac 1620
cagcactacc agagaagcac agctcagtaa aagagagctt ccgctacgta aaccttacat 1680
gcagctctgg caagcaagtc ccaggagccc ctggccgacc aagcacccct aaggaaatgt 1740
ttatcactgt tgagtttgag cttgaaacta accaaaagga ggtgacagct tcttgtgacc 1800

```
tgagctgcat cgtaaagcga accgagaagc ggctccgtaa agccatccgc acgctcagaa 1860
aggccgtcca cagggagcag tttcacctcc agctctcagg catgaacctc gacgtggcta 1920
aaaagcctcc cagaacatct gaacgccagg cagagtcctg tggagtgggc cagggtcatg 1980
cagaaaacca atgtgtcagt tgcagggctg ggacctatta tgatggagca cgagaacgct 2040
gcattttatg tccaaatgga accttccaaa atgaggaagg acaaatgact tgtgaaccat 2100
gcccaagacc aggaaattct ggggccctga agaccccaga agcttggaat atgtctgaat 2160
gtggaggtct gtgtcaacct ggtgaatatt ctgcagatgg ctttgcacct tgccagctct 2220
gtgccctggg cacgttccag cctgaagctg gtcgaacttc ctgcttcccc tgtggaggag 2280
gccttgccac caaacatcag ggagctactt cctttcagga ctgtgaaacc agagttcaat 2340
gttcacctgg acatttctac aacaccacca ctcaccgatg tattcgttgc ccagtgggaa 2400
cataccagcc tgaatttgga aaaaataatt gtgtttcttg cccaggaaat actacgactg 2460
actttgatgg ctccacaaac ataacccagt gtaaaaacag aagatgtgga ggggagctgg 2520
gagatttcac tgggtacatt gaatccccaa actacccagg caattaccca gccaacaccg 2580
agtgtacgtg gaccatcaac ccaccccccca agcgccgcat cctgatcgtg gtccctgaga 2640
tcttcctgcc catagaggac gactgtgggg actatctggt gatgcggaaa acctcttcat 2700
ccaattctgt gacaacatat gaaacctgcc agacctacga acgccccatc gccttcacct 2760
ccaggtcaaa gaagctgtgg attcagttca agtccaatga agggaacagc gctagagggt 2820
tccaggtccc atacgtgaca tatgatgagg actaccagga actcattgaa gacatagttc 2880
gagatggcag gctctatgca tctgagaacc atcaggaaat acttaaggat aagaaactta 2940
tcaaggctct gtttgatgtc ctggcccatc cccagaacta tttcaagtac acagcccagg 3000
agtcccgaga gatgtttcca agatcgttca tccgattgct acgttccaaa gtgtccaggt 3060
ttttgagacc ttacaaatga ctcagcccac gtgccactca atacaaatgt tctgctatag 3120
ggttggtggg acagagctgt cttccttctg catgtcagca cagtcgggta ttgctgcctc 3180
ccgtatcagt gactcattag agttcaattt ttatagataa tacagatatt ttggtaaatt 3240
gaacttggtt tttctttccc agcatcgtgg atgtagactg agaatggctt tgagtggcat 3300
cagcttctca ctgctgtggg cggatgtctt ggatagatca cgggctggct gagctggact 3360
ttggtcagcc taggtgagac tcacctgtcc ttctggggtc ttactcctcc tcaaggagtc 3420
tgtagtggaa aggaggccac agaataagct gcttattctg aaacttcagc ttcctctagc 3480
ccggccctct ctaagggagc cctctgcact cgtgtgcagg ctctgaccag gcagaacagg 3540
caagagggga gggaaggaga cccctgcagg ctccctccac ccaccttgag acctgggagg 3600
actcagtttc tccacagcct tctccagcct gtgtgataca agtttgatcc caggaacttg 3660
agttctaagc agtgctcgtg aaaaaaaaaa gcagaaagaa ttagaaataa ataaaaacta 3720
agcacttctg gagacat 3737
```

<210> 136
<211> 2477
<212> DNA
<213> Homo sapiens
<400> 136

```
gcagaggtgc ggccggggag gcgcgcggag gctggagctg gaggcgcggc gccggtgagc 60
tgagaaccat gtgtgctcag tattgcatct cctttgctga tgttgaaaaa gctcatatca 120
acattcgaga ttctatccac ctcacaccag tgctaacaag ctccattttg aatcaactaa 180
cagggcgcaa tcttttcttc aaatgtgaac tcttccagaa aacaggatct tttaagattc 240
gtggtgctct caatgccgtc agaagcttgg ttcctgatgc tttagaaagg aagccgaaag 300
ctgttgttac tcacagcagt ggaaaccatg gccaggctct cacctatgct gccaaattgg 360
aaggaattcc tgcttatatt gtggtgcccc agacagctcc agactgtaaa aaacttgcaa 420
tacaagccta cggagcgtca attgtatact gtgaacctag tgatgagtcc agagaaaatg 480
ttgcaaaaag agttacagaa gaaacagaag gcatcatggt acatcccaac caggagcctg 540
cagtgatagc tggacaaggg acaattgccc tggaagtgct gaaccaggtt cctttggtgg 600
atgcactggt ggtacctgta ggtggaggag gaatgcttgc tggaatagca attacagtta 660
aggctctgaa acctagtgtg aaggtatatg ctgctgaacc ctcaaatgca gatgactgct 720
accagtccaa gctgaagggg aaactgatgc ccaatcttta tcctccagaa accatagcag 780
atggtgtcaa atccagcatt ggcttgaaca cctggcctat tatcagggac cttgtggatg 840
atatcttcac tgtcacagag gatgaaatta agtgtgcaac ccagctggtg tgggagagga 900
tgaaactact cattgaacct acagctggtg ttggagtggc tgctgtgctg tctcaacatt 960
ttcaaactgt ttccccagaa gtaaagaaca tttgtattgt gctcagtggt ggaaatgtag 1020
acttaacctc ctccataact tgggtgaagc aggctgaaag gccagcttct tatcagtctg 1080
tttctgttta atttacagaa aaggaaatgg tgggaattca gtgtctttag atactgaaga 1140
cattttgttt cctagtattg tcaactctta gttatcagat tcttaatgga gagtggctat 1200
ttcattaaga tttaatagtt ttttttggac taagtagtgg aaaaactttt atacttaact 1260
gagacatttt gtcaaggcta aaaaaaagtc ttgcaaaatg gggcagtgga ctgacaggct 1320
gacatagaaa ataaactttg cccaatcaca acttgtgcct cccatccctg gagtactgac 1380
tggcaccggt aagacagaat ctctctgaat ccattactcc atgccccctt gaggcactgt 1440
tgaagaaatc tcacttttca gccagggtac tggttctggt acatatggat cataagtcca 1500
tttggggaag actcgtttat acaggttcat cagtactgtg tcttgagatt ttagcttccc 1560
atcaaagctg catttcatgt ggccatgggt acctagaaag acatcagaac aagtcggtca 1620
aattaaaagt agaaaatttt aaagcaatga cttccaaccc aacagtcatt tagcaacact 1680
gcagaaatgc agacatggtc tcaaatcccg tgtttcctta cctaaaggtt ccttgatatg 1740
tcctctccgg ccccacttcg ttctcagttc cactggttta aaccacagca catcctctta 1800
```

gaatcaaaca cattaaagac caagatgaaa catttaccca caaaatgtaa acccaacctt 1860
tataccacaa aggcaatcag atcccatcct cctccttcat acccacctct gttgaagaac 1920
atgtaacgta ctactgccat cttagtaaaa attttgaaag gatgaccact cagaacaact 1980
ctcttgatga ccattctgtc tggatctact gacataagat ggcctgtagc aatgaggctg 2040
tgcattccta aggacaaaa gcaaagaagc tatttaggaa tttacaggcc aaagtcttca 2100
tttattgccc agtccattta aagacccatg caagagcctg gtttgtcatc cctgccctag 2160
cccaatctga ggctaagatt ggtaaactgt aagcccacac ttaaccttgt caataggttc 2220
ttgaaaactt gtacttcaag agaaatgatg tataacaaaa ccatactttt tctcatcagt 2280
tgttacaagg aaaggatgtt gaacaaaagg cagttatttg aggactggct atacactgtt 2340
tcacgtaaaa gttggagttt tcattgttct attaacaatg ttaaatgaag acttactgta 2400
ttttgaaaac catcattacc ttctccatac catttggctc ccaaatttaa ataaacaaga 2460
gaaaacggtg ttcatct 2477

<210> 137
<211> 1341
<212> DNA
<213> Homo sapiens
<400> 137
aggtgtttgg gtttcttcgc ggctgctcaa gatgaaccga ctcttcggga aagcgaaacc 60
caaggctccg cgccccagcc tgactgactg cattggcacg gtggacagta gagcagaatc 120
cattgacaag aagatttctc gattggatgc tgagctagtg aagtataagg atcagatcaa 180
gaagatgaga gagggtcctg caaagaatat ggtcaagcag aaagccttgc gagttttaaa 240
gcaaaagagg atgtatgagc agcagcggga caatcttgcc aacagtcatt caacatggac 300
aggccattat accatccagt ctttgaagga caccaagacc acggttgatg ctatgaaact 360
gggagtaaag gaaatgaaga aggcatacaa gccagtgaag atcgaccaga ttgaggattt 420
acaagaccag ctagaggata tgatggaaga tgcaaatgaa atccaagaag cactgagtcg 480
cagttatggc accccagaac tggatgaaga tgatttagaa gcagagttgg atgcactagg 540
tgatgagctt ctggctgatg aagacagttc ttatttggat gaggcagcat ctgcacctgc 600
aattccagaa ggtgttccca ctgatacaaa aaacaaggat ggagttctgg tggatgaatt 660
tggattgcca cagatccctg cttcatagat ttgcatcatt caagcatatc ttgtaaaaca 720
aacacatatt atgggactag gaaatattta tctttccaaa tttgccataa cagatttagg 780
tttctttcct ttctttgaag gaaagtttaa ttacattgct cttttatttt ttccattaag 840
agactcattg cttgggaaat gctttcttcg tactaaaatt tgattccttt ttttcttatg 900
aaaaacgaac tcagtttaaa agtattttta gctcgtatga cttgttttca ttcattaata 960
ataatttgaa ataaaactaa ggaaatggaa tcttaaaagt ctatgacagt gtaactctac 1020
agtctcaaaa tgacctgata aattgataag acaaagatga gattattggg gctgttcata 1080
ttatgattca gaatcatttt ctattgtggt attataggtt ggttaaagtg atggcctttt 1140
tgatgggttt tgttgtgtct tgtgaacaag tcgttactgt gtccattatt ggaatggaat 1200
tatcactact gtatcatgag tgggtatttt gattctatgg ttccctcagt attacatctt 1260
gacttgtaat caattatgaa tatttcttga tatttaatgt ataggacatt tatttatact 1320
caataaatat ttttcaaaag g 1341

<210> 138
<211> 2449
<212> DNA
<213> Homo sapiens
<400> 138
cttgtcccca gcgcctggac tccccttaa ctgcttggga aatgtgacct ttgctctggg 60
gggcctggcc ctgcaggccc caaccttccc tcatctctgg cggccctctt gggcctctga 120
cccagcccct ccccgggcca ggctcacaga agctggcttc tgggactgtc ctgggcccaa 180
gtgggcacct gcgccagccc cacctgtgcc tgggctgtgg ccccttccta cagggcgctc 240
accatggccc cgccgctcct gctgctgctg ctggccagtg gagcggcgc ctgcccgctg 300
ccctgcgtct gccagaacct gtccgagtcg ctcagcaccc tctgtgccca ccgaggcctg 360
ctgtttgtgc cgcccaacgt ggaccggcgc acagtggagc tgcggctggc tgacaacttc 420
atccaggccc tggggccccc tgacttccgc aacatgacgg gactggtgga cctgacactg 480
tctcgcaatg ccatcacccg cattggggcc cgcgcctttg gggacctcga gagcctgcgt 540
tccctccacc ttgacggcaa caggctggtg gagctgggca ccgggagcct ccggggcccc 600
gtcaatctgc agcacctcat cctcagcggc aaccagctgg gccgcatcgc gccgggagcc 660
ttcgacgact tcctagagag cctggaggac ctggacctgt cctacaacaa cctccggcag 720
gtgccctggg ccggcatcgg cgccatgcct gccctgcaca ccctcaacct ggaccataac 780
cttattgacg cactgccccc aggcgccttc gcccagctcg gtcagctctc ccgcctggac 840
ctcacctcca accgcctggc cacgctggct ccggacccgc ttttctctcg tgggcgtgat 900
gcagaggcct ctcccgcccc cctggtgctg agctttagcg ggaacccccct gcactgcaac 960
tgtgagctgc tgtggctgcg gcggctggcg cggccggacg acctggaaac gtgcgcctcc 1020
ccgcccggcc tggccggccg ctacttctgg gcagtgcccg agggcgagtt ctcctgtgag 1080
ccgcccctca ttgcccgcca cacgcagcgc ctctgggtgc tggaaggcca gcgggccacg 1140
ctgcggtgcc gggccctggg tgaccccgcg cctaccatgc actgggtcgg tcctgacgac 1200
cggttggttg gcaactcctc ccgagcccgg gctttcccca acgggacctt agagattggg 1260
gtgaccggcg ctggggacgc tgggggctac acctgcatcg ccaccaaccc tgctggtgag 1320

gccacagccc gagtagaact gcgggtgctg gccttgcccc atggtgggaa cagcagtgcc 1380
gagggggccc gccccgggcc ctcggacatc gccgcctccg ctcgcactgc tgccgagggt 1440
gaggggacgc tggagtctga gccagccgtg caggtgacgg aggtgaccgc cacctcaggg 1500
ctggtgagct ggggtcccgg gcggccagcc gacccagtgt ggatgttcca aatccagtac 1560
aacagcagcg aagatgagac cctcatctac cggattgtcc cagcctccag ccaccacttc 1620
ctgctgaagc acctcgtccc cggcgctgac tatgacctct gcctgctggc cttgtcaccg 1680
gccgctgggc cctctgacct cacggccacc aggctgctgg gctgtgccca tttctccacg 1740
ctgccggcct cgcccctgtg ccacgccctg caggcccacg tgctgggcgg gaccctgacc 1800
gtggccgtgg ggggtgtgct ggtggctgcc ttactggtct tcactgtggc cttgctggtt 1860
cggggccggg gggccggaaa tggccgcctc cccctcaagc tcagccacgt ccagtcccag 1920
accaatggag gccccagccc cacacccaag gcccacccgc cgcggagccc cccgccccgg 1980
ccgcagcgca gctgctctct ggacctggga gatgccgggt gctacggtta tgccaggcgc 2040
ctgggaggag cttgggcccg acggagccac tctgtgcatg gggggctgct cggggcaggg 2100
tgccgggggg taggaggcag cgccgagcgg ctggaagaga gtgtggtgtg atggacgggc 2160
agcttcctgt gtgctccaag ggatgagcct cgtggggcag agggcccggg gccgccgcct 2220
ggcctgggag tccctccctg gtttttattc tcagtacctc aggctcccct gtgtacttgg 2280
aggggcaggg agccctttcc tcggttctgg cctccagacc agggtaaggg caggcccctc 2340
caacaggtgc tcacagccac cgaggcaggg gctgcagcca cccactggga gtcttgtttt 2400
tatttataat aaaattgttg gggacacctc aaaaaaaaaa aaaaaaaaa 2449

<210> 139
<211> 2175
<212> DNA
<213> Homo sapiens
<400> 139
gcacactgaa gagtacgtct tcgggtctac ccctaatcac ataatggctg tgtttaatca 60
gaagtctgtc tcggacatga ttaaagagtt tcgaaaaaat tggcgtgctc tttgtaactc 120
tgagagaact actctatgtg gtgcagactc catgctcttg gcattgcagc tttctatggc 180
agagaacaac aaacagtgga gaatttacag tctctctcag tgatgtttta ttgacatgga 240
aatacttgct ccatgagaaa ttgaacttac cagttgaaaa catggacgtg actgaccatt 300
atgaggacgt taggaagatt tatgatgatt tcttgaagaa cagtaatatg ttagatctga 360
ttgatgttta tcaaaaatgt agggctttga cttctaattg tgaaaattat aacacagtat 420
ctcctagtca actactggat ttctgtctg gcaaacagta tgcagtaggt gatgaaactg 480
atctttctat accaacatca ccaacaagta aatacaaccg tgataatgaa aaggtgcagc 540
tgctagcaag gaaaattatc ttttcatatt taaatctgct agtgaattca aagaatgacc 600
tggctgtggc ttatattctc aatattcctg atagaggact aggaagagaa gccttcactg 660
atttgaaaca tgctgctcga gagaaacaaa tgtctatctt tttggtggcc acgtctttta 720
ttagaacaat agagcttgga gggaaaggat atgcaccacc accatcagat cctttaagga 780
cacatgtaaa gggattgtct aattttatta atttcattga caaattagat gagattcttg 840
gagaaatacc aaacccaagc attgcagggg gtcaaatact gtcagtgata aagatgcaac 900
tgattaaagg ccaaaacagc agggatcctt tttgcaaagc aatagaggaa gttgctcagg 960
atttggattt gaggattaaa aatattatca attctcaaga aggtgttgta gctcttagca 1020
ccactgacat cagtcctgct cggccaaaat ctcatgccat aaaccatggt actgcatact 1080
gtggcagaga tactgtgaaa gcctttattag ttcttttgga cgaagaagca gctaatgctc 1140
ctaccaaaaa caaagcagag cttttatatg atgaggaaga cacaatccat catcatggaa 1200
cgtctattct tacacttttt aggtctccca cacaggtgaa taatttgata aaacccctaa 1260
gagaacgcat ctgtgtgtca atgcaagaga aaaaaattaa gatgaagcaa actttaatta 1320
gatcccaatt tgcttgtact tataaagatg actacatgat aagcaaggat aattggaata 1380
atgttaattt agcatcaaag cctttgtgtg ttctttacat ggaaaatgac ctttctgagg 1440
gtgtaaatcc atctgttgga agatcaacaa ttggaacgag ttttggaaat gttcatctgg 1500
acagaagtaa aaatgaaaaa gtatcaagaa aatcaaccag tcagacagga aataaaagct 1560
caaaaaggaa acaggtggat ttggatggtg aaaatattct ctgtgataat agaaatgaac 1620
cacctcaaca taaaaatgct aaaataccta agaaatcaaa tgattcacag aatagattgt 1680
acggcaaact agctaaagta gcaaaaagta ataaatgtac tgccaaggac aagttgattt 1740
ctggccaggc aaagttaact cagtttttta gactataaat ttgtgtctta tatgctttag 1800
gtttatgcat ctataaacca ttcaccaaag acatgcttaa tttttaagag atcaaggtgt 1860
aaattatgat gatttattat tttggtctac agtgtatgta aggttagtat gttaagcact 1920
gtttttgact ttttaaaaat accttagatg caaatttata ggagaaaaaa cactttcaga 1980
taagaggtgt ttgctgggat ggaagaacta cctggcatgt aagaaatatc gtcagtcgtc 2040
ctaatgcata ttgtgactgt ttgcatatac ttctgtttat aaaagtatca gtttttacttt 2100
tcagaggatt tgtaagaatc atttaaattt tcattgaaat aaacgacaag tcacattgaa 2160
aaaaaaaaaa aaaaa 2175

<210> 140
<211> 1834
<212> DNA
<213> Homo sapiens
<400> 140
ggaagttgca ggcaatgagg gacattgcta tattaaagga aaagcaggag aaagaaatac 60

```
agacattaca ggaggagaca aagaaagtcc aagctgagac agcttcaaag acacgggaag    120
tacaggccca gctcctccag gagaaaagat tactggagaa acaactgagc gagccagaca    180
ggaggctact gggaaagaga aaaagaagag agcttaatat gaaggcccag gccttgaagt    240
tggcagcaaa gcggtttatt tttgaatact cctgtggcat caacagagag aaccagcagt    300
tcaagaagga attactgcag ctaattgagc aagcccagaa actaacggct actcaaagcc    360
acttagaaaa caggaagcag cagctgcagc aggaacagtg gtatctggag tccttaatcc    420
aggcgaggca gagactgcaa ggaagtcata atcagtgcct aaatagacag gatgttccaa    480
agaccacacc cagtcttccc caaggcacca aatcaaggat taatccaaag taacttctaa    540
aataacactg attaaataag aactggagca agtactctta agtgctacat taacctggtt    600
agaaaggctg ttggattcca gattgctatt gtaaaatctc catcatgatg tgttggagtg    660
aaggattaga tggttttatc caacagtcct actagatatt tggtaaccag cttcccttaa    720
ctagcttttt ctttaaatac tcgttaataa gctattccac aaacctccag ttaacctaac    780
acatgaccct aacctagcca tttaccatac atcaaactag ctaaaggaaa ccaacctaag    840
gaagtgaaaa cagttgtgat ttatttcatc tagctaaatt gtatttcttt atagagaaag    900
tacctttaag gatagcattc caaatagact ttgaatagcg ttctgccagt ttatcctcat    960
tccttttgac caacttagca gacaaaagca gtttttacaa gctctttgtg agtttgtgcc   1020
agtgaccagg tagctccttc tagttttctc atgagtgaaa aagcattctg ataacagcaa   1080
gtccagtaag tgctaggcag agtgaccttt cacctgatgc taagccccta caagtttgag   1140
aaggtaagaa aagatgaagg agacatatat taggtcagct cttacttttg aaaatgtttt   1200
atttgaagaa acacctgtag cattgaggtg actgaatgcc tccacttatt tcaggaaaac   1260
gtatccaaaa aaagttgaaa tatttggaca actttttttt taagtgccat cgatttccct   1320
agcagcattc taaaagatag caagtaaaat gatgtttgtt atcctaaatg ctttagtttt   1380
aggtcattta ttaattttct tacaggtgca ctttctagta catgaagtat cctttgtaat   1440
taatgtgtgc catatgttta ttcccattta gtataactat aaattatatt ttaaattata   1500
tatttttagg atagttatat tttttttggg ttctacgaca ttgaagttgg actagtgatt   1560
tatttgaatg ctgaatccta gtatagggga atataatctt atattttaac aggggtcctc   1620
tatgggaaaa taggatgaac tttgtttccc agaaattgtt aagtgatgaa aaacttcaaa   1680
ataatttcc tgcattttct gctttattta catgtaaagt gaattccctg aaaattggat    1740
ttaaaaagca ttctccttca atgtgccttt accttgtagc tttaacaact tttctgttaa   1800
atatgtagtt ttttattaaa caatgttatt aaat                                1834
```

<210> 141
<211> 1286
<212> DNA
<213> Homo sapiens
<400> 141

```
ggagcgcgcg gtccgggcac acggagcagg ttgggaccgc ggcgggtacc ggggccgggg     60
cgccatgcgg aggccgagcg tgcgcgcggc cgggctggtc ctgtgcaccc tgtgttacct    120
gctggtgggc gctgctgtct tcgacgcgct cgagtccgag gcggaaagcg gccgccagcg    180
actgctggtc cagaagcggg gcgctctccg gaggaagttc ggcttctcgg ccgaggacta    240
ccgcgagctg gagcgcctgg cgctccaggc tgagccccac cgcgccggcc gccagtggaa    300
gttccccggc tccttctact tcgccatcac cgtcatcact accatcgggt acggccacgc    360
cgcgccgggt acggactccg gcaaggtctt ctgcatgttc tacgcgctcc tgggcatccc    420
gctgacgctg gtcactttcc agagcctggg cgaacggctg aacgcggtgg tgcggcgcct    480
cctgttggcg gccaagtgct gcctgggcct gcggtggacg tgcgtgtcca cggagaacct    540
ggtggtggcc gggctgctgg cgtgtgccgc caccctggcc ctcggggccg tcgccttctc    600
gcacttcgag ggctggacct tcttccacgc ctactactac tgcttcatca ccctcaccac    660
catcggcttc ggcgacttcg tggcactgca gagcggcgag gcgctgcaga ggaagctccc    720
ctacgtggcc ttcagcttcc tctacatcct cctggggctc acggtcattg gcgccttcct    780
caacctggtg gtcctgcgct tcctcgttgc cagcgccgac tggcccgagc gcgctgcccg    840
cccccccagc ccgcgccccc cggggcgccc cgagagccgt ggcctctggc tgccccgccg    900
cccggcccgc tccgtgggct ccgcctctgt cttctgccac gtgcacaagc tggagaggtg    960
cgcccgcgac aacctgggct tttcgccccc ctcgagcccg ggggtcgtgc gtggcgggca   1020
ggctcccagg cctggggccc ggtggaagtc catctgacaa ccccacccag gccagggtcg   1080
aatctggaat gggagggtct ggcttcagct atcagggcac cctccccagg gattggaaac   1140
ggatgacggg cctctaggcg gtcttctgcc acgagcagtt tctcattact gtctgtggct   1200
aagtcccctc cctcctttcc aaaaatatat tacagtcaca ccataaaaaa aaaaaaaaaa   1260
aaaaaaaaaa aaaaaaaaaa aaaaaa                                         1286
```

<210> 142
<211> 4166
<212> DNA
<213> Homo sapiens
<400> 142

```
caggtggaat gtcagaagac tgagaacatt gttccttctt catactgctg ctctgttgcc     60
agagaatccc aatttacact caaagcttct ttgattaagt gctaggagat aaatttgcat    120
tttctcaagg aaaaggctaa aagtggtagc aggtggcatt taccgtcatg gagagcaggg    180
atcataacaa cccccaggag ggacccacgt cctccagcgg tagaagggct gcagtggaag    240
acaatcactt gctgattaaa gctgttcaaa acgaagatgt tgacctggtc cagcaattgc    300
```

tggaaggtgg agccaatgtt aatttccagg aagaggaagg gggctggaca cctctgcata 360
acgcagtaca aatgagcagg gaggacattg tggaacttct gcttcgtcat ggtgctgacc 420
ctgttctgag gaagaagaat ggggccacgc cttttatcct cgcagcgatt gcggggagcg 480
tgaagctgct gaaacttttc ctttctaaag gagcagatgt caatgagtgt gatttttatg 540
gcttcacagc cttcatggaa gccgctgtgt atggtaaggt caaagcccta aaattccttt 600
ataagagagg agcaaatgtg aatttgaggc gaaagacaaa ggaggatcaa gagcggctga 660
ggaaaggagg ggccacagct ctcatggacg ctgctgaaaa aggacacgta gaggtcttga 720
agattctcct tgatgagatg ggggcagatg taaacgcctg tgacaatatg ggcagaaatg 780
ccttgatcca tgctctcctg agctctgacg atagtgatgt ggaggctatt acgcatctgc 840
tgctggacca tggggctgat gtcaatgtga ggggagaaag agggaagact cccctgatcc 900
tggcagtgga gaagaagcac ttgggtttgg tgcagaggct tctggagcaa gagcacatag 960
agattaatga cacagacagt gatggcaaaa cagcactgct gcttgctgtt gaactcaaac 1020
tgaagaaaat cgccgagttg ctgtgcaaac gtggagccag tacagattgt ggggatcttg 1080
ttatgacagc gaggcggaat tatgaccatt cccttgtgaa ggttcttctc tctcatggag 1140
ccaaagaaga ttttcaccct cctgctgaag actggaagcc tcagagctca cactgggggg 1200
cagccctgaa ggatctccac agaatatacc gccctatgat tggcaaactc aagttcttta 1260
ttgatgaaaa atacaaaatt gctgatactt cagaaggagg catctacctg ggtttctatg 1320
agaagcaaga agtagctgtg aagacgttct gtgagggcag cccacgtgca cagcgggaag 1380
tctcttgtct gcaaagcagc cgagagaaca gtcacttggt gacattctat gggagtgaga 1440
gccacagggg ccacttgttt gtgtgtgtca ccctctgtga gcagactctg gaagcgtgtt 1500
tggatgtgca cagaggggaa gatgtggaaa atgaggaaga tgaatttgcc cgaaatgtcc 1560
tgtcatctat atttaaggct gttcaagaac tacacttgtc ctgtggatac acccaccagg 1620
atctgcaacc acaaaacatc ttaatagatt ctaagaaagc tgctcacctg gcagattttg 1680
ataagagcat caagtgggct ggagatccac aggaagtcaa gagagatcta gaggaccttg 1740
gacggctggt cctctatgtg gtaaagaagg gaagcatctc atttgaggat ctgaaagctc 1800
aaagtaatga agaggtggtt caactttctc cagatgagga aactaaggac ctcattcatc 1860
gtctcttcca tcctggggaa catgtgaggg actgtctgag tgacctgctg ggtcatccct 1920
tcttttggac ttgggagagc cgctatagga cgcttcggaa tgtgggaaat gaatccgaca 1980
tcaaaacacg aaaatctgaa agtgagatcc tcagactact gcaacctggg ccttctgaac 2040
attccaaaag ttttgacaag tggacgacta agattaatga atgtgttatg aaaaaaatga 2100
ataagtttta tgaaaaaaga ggcaatttct accagaacac tgtgggtgat ctgctaaagt 2160
tcatccggaa tttgggagaa cacattgatg aagaaaagca taaaaagatg aaattaaaaa 2220
ttggagaccc ttccctgtat tttcagaaga catttccaga tctggtgatc tatgtctaca 2280
caaaactaca gaacacagaa tatagaaagc atttccccca aacccacagt ccaaacaagc 2340
ctcagtgtga tggagctggt ggggccagtg ggttggccag ccctgggtgc tgatggactg 2400
atttgctgga gttcagggaa ctacttatta gctgtagagt ccttggcaaa tcacaacatt 2460
ctgggccttt taactcacca ggttgcttgt gagggatgag ttgcatagct gatatgtcag 2520
tccctggcat cgtgtattcc atatgtctat aacaaaagca atatataccc agactacact 2580
agtccataag ctttacccac taactgggag gacattctgc taagattcct tttgtcaatt 2640
gcaccaaaag aatgagtgcc ttgaccccta atgctgcata tgttacaatt ctctcactta 2700
attttcccaa tgatcttgca aaacagggat tatcatcccc atttaagaac tgaggaacct 2760
gagactcaga gagtgtgagc tactggccca agattattca atttatacct agcactttat 2820
aaatttatgt ggtgttattg gtacctctca tttgggcacc ttaaaactta actatccttc 2880
cagggctctt ccagatgagg cccaaaacat atatagggt tccaggaatc tcattcattc 2940
attcagtatt tattgagcat ctagtataag tctgggcact ggatgcatga attccactcc 3000
ttccagaacc aactgcattg gttttccatg accttaaggc agtagttctc aactgggggg 3060
caatttgca ctgaagagag catttggcag agtctgaaga agtttttggt gtcacagctt 3120
tgtggggagc atgctatggc atttagtggg taaagaccag ggatgctgcc aaacctgcct 3180
tgcacaggac agcccctgca acaaagaatt atccagacaa aaatatcaat ggtgctgagg 3240
ttgagaaaac ctgacttaag gggctgggat gcttttgaac tagcttaagg cccaggactg 3300
tggagtgtgt ggaccacccc acagaggagg gactcagatt tatttactct tgctggatct 3360
gtagtgatgg agttccttct ggtgtcagcc ccacaggagg ctcccaggcc tccctcactt 3420
cccataccca gtctaggagc tccttctggc tcccaagcac ccagagcttt cctccgcctt 3480
ttagttttgg ttcctccact ggaatgtagg ctcctcacgg gcgatggctg tcttttcttg 3540
actttgtatc ttcactgcca agcaaaaagt ctgccaagtg ggaatgttta ataaatattc 3600
attgaataat gaatgaacca tcttcgtaca tgaataataa tactgtctta cgtttttctg 3660
gtgctttata atgtatacat tacatctgag tattttattt tatttaattt tcaaaacaat 3720
cctttaaggt caacattgtt atccctattt tgctgatgag gaaactaagg ttagaaacat 3780
tttgatttcc tctaggacgt atagctagga agtgttacta tcttgatttg aacaaatttt 3840
ctggtgctaa gtctgatgtt ctttccatga atcattgtgg tggttgagat ggagctttgt 3900
aatgggaata aaacagtacc ttaggttctt tctgaaaagg aggtatctag caatggataa 3960
atagatacca ctgaatgaaa ttaaatgttg attaggaaca aatttaaggc ttaaaaaata 4020
ctttatgagc agcaagattg ctttaacttt taaaatgaag ctttggttct ctgatttgta 4080
atgagcacct ggacatgtca attaaaatgc ccatttgtga agcttactca ataaaacttt 4140
aaattgtcaa aaaaaaaaaa aaaaaa 4166

<210> 143
<211> 3412
<212> DNA

<213> Homo sapiens
<400> 143
agttttgctc cgaaagactt accgaggagg gagcttgcgg tgcgttctgg gaaagttgct 60
gggccagctc ctttgtttcc agtctgagcg ttgcgttcgg tttcccgagg gtcttctgag 120
gcaccgcggc tgcgggcttc tgagttcccg gctctccgca gggaagcctc ctcttcgtac 180
ctcgtttttt ggctcgtggg ggtcctccc accgctggcc gacgcagcca gcatgtccgg 240
ggtgcgcgca gtgcggatca gcatcgaatc ggcctgcgag aagcaggtcc atgaggtggg 300
cctggatggc accgagacgt acctgccccc gctgtccatg tcgcagaatc tggcgcgtct 360
ggcccagcgg atagacttca gccagggttc gggctccgag gaggaggagg cggcggggac 420
cgagggcgac gcgcaggagt ggccgggcgc cgggtccagc gcagaccagg acgacgagga 480
aggagtggta aaatttcagc cttccctttg gccttgggac tcagtgagga acaatttgag 540
aagtgccctg acagagatgt gtgttctcta tgatgttctc agtattgtta gggataaaaa 600
atttatgact cttgatcctg tctctcagga tgcacttcct ccaaaacaga atcctcagac 660
gttgcaattg atatctaaaa agaagtcact tgctggagca gcacaaatct tattgaaggg 720
ggcagaaaga ctgactaaat cagttaccga aaaccaagaa aacaagctac aaagagactt 780
caattctgag cttttgcgat tacggcaaca ctggaaactt cgaaaagttg gagataaaat 840
tctcggagat ctgagctaca gaagtgcagg atctctcttt cctcatcatg gtacatttga 900
agtaataaag aatacagatc tcgatctgga taaaaagata cctgaagatt actgtcctct 960
tgatgtccaa attcctagtg atttagaggg gtctgcatat atcaaggttt caatacaaaa 1020
acaggctcca gatataggtg acctcggcac agttaacctc ttcaaacgac ctttgcccaa 1080
atccaaacca ggttccccac attggcagac aaaattagaa gcggcacaga atgttctctt 1140
atgtaaagaa atttttgcac agctctctcg ggaagctgtt caaattaaat cacaagtccc 1200
tcacattgtg gtgaaaaacc agattatctc tcagcccttt ccgagcttgc agttatctat 1260
ttctttgtgc cattcctcaa atgataagaa atcccaaaaa tttgctactg agaagcaatg 1320
tccggaggac cacctttatg tcctagagca taatttgcat ctactgatta gagagtttca 1380
taaacagacc ttgagttcca tcatgatgcc tcatccagca agtgcacctt ttggccacaa 1440
gagaatgaga ctttcgggtc ctcaagcttt tgataaaaat gaaattaatt cattacagtc 1500
cagtgaaggg cttctggaaa aaataattaa acaagcaaag catattttc taaggagtag 1560
agctgctgca accattgaca gcttagcaag ccgaattgag gatcctcaga tacaggctca 1620
ttggtcaaat atcaatgatg tttatgaatc tagtgtgaaa gttttaatca catcacaagg 1680
ctatgaacaa atatgcaagt ccattcaact gcaattgaat attggagttg agcagattcg 1740
agttgtacat agagatggaa gagtaattac actgtcttat caggagcagg agctacagga 1800
ttttcttctg tctcagatgt cacagcacca ggtacatgca gttcagcaac tcgccaaggt 1860
tatgggctgg caagtactga gcttcagtaa tcatgtggga cttggaccta tagagagcat 1920
tggtaatgca tctgccatca cggtggcctc cccaagtggt gactatgcta tttcagttcg 1980
taatggacct gaaagtggca gcaagattat ggttcagttt cctcgtaacc aatgtaaaga 2040
ccttccaaaa agtgatgttt tacaagataa caaatggagt catcttcgtg ggccattcaa 2100
agaagttcag tggaataaaa tggaaggtcg aaattttgtt tataaaatgg agctgcttat 2160
gtctgcactt agcccttgtc tactatgatt ttttccagat gtttcctaaa gaagtttcca 2220
gaaactttga cttgaaatgt ttgcagatca actataagca caaagaagag ataacttcca 2280
aaagagtgct gtttttaaaa ataataatta ggaaatgttt atttagcact ttcaaacttt 2340
tcactttata aatgacaagt gctttgaaat gcagaagttt atgtacagtt gtatatacag 2400
tatgacaaga tgtaaaataa tatgttttc atgcagttta aaatattact aacttaaggg 2460
tttctatgtg ctttttaaaa tattccttct ttgatgttga catcaaataa agtatgtggt 2520
ttaaaaaaat ctccaaatac ctttttttcc ccccaaatac tttctaaact tttttttttt 2580
gagatggtat ctcactctgt agcccagtct ggagtgcagt ggtgtgatca tggttcactg 2640
cagtcttgac ctcccaggct taggtgatcc ttctgtctca gccttccgag tagctgggac 2700
cacaggcatg cacaaccacg cctggctaat ttttgtattt tttataaaga cagggttttt 2760
ccatgttgcc caggctggtt tcgaactcgg ctcaagtgat ctacctgcct ctgcctccca 2820
aagtgctagg attacaggcg tgagccacca tgcccagcct actctaaatt attgataacc 2880
tcttcctcca gttgtctcct ttaagctttc ctgggtctaa cctacatagg taatttaaga 2940
acatcctcag aaaggacagc tgaaggcaat aggaggcaga ttatctcttt agggcgtcct 3000
caagtttttt tggtctgttc tccacttgac ttgacctcac cagttgagac acctagtgta 3060
tggctcatgc ccagccttcc acctgggatt ctccagcctc cacccagcag ccctggattg 3120
ctttctccaa ttaaggcctt tccatcagct ctctgctttt tcaaagcgaa aaaactaatg 3180
gattagtggg ttatcttttc caaggaacag gtttgcactt cttggaaaaa gtgcctaaag 3240
tgtgcccatt aatatgagga tagatttagg ctcataagcc ttttggtaac actgaaagta 3300
gtatcatata ggcaagctct ccttataagt aaggctttca atttttaaaa cagacatcct 3360
gctttaacaa tttgtaagat gactgtgcag taataaaagt cctttgtatt tc 3412

<210> 144
<211> 6217
<212> DNA
<213> Homo sapiens
<400> 144
cgcagactgt gctggagctg gtgctgaaaa aggggtttg cagaggctgc cctggggctg 60
gtgctgaaag aagagcccac agctgacttc atggtgctac aataacctca gaatctactt 120
ttcactctca ggagaaccca cagtctaata tttagacatg atggcaaact gggcggaagc 180
aagacctctc ctcattctta ttgttttatt agggcaattt gtctcaataa aagcccagga 240

agaagacgag gatgaaggat atggtgaaga aatagcctgc actcagaatg gccagatgta 300
cttaaacagg gacatttgga aacctgcccc ttgtcagatc tgtgtctgtg acaatggagc 360
cattctctgt gacaagatag aatgccagga tgtgctggac tgtgccgacc ctgtaacgcc 420
ccctggggaa tgctgtcctg tctgttcaca aacacctgga ggtggcaata caaattttgg 480
tagaggaaga aagggacaaa agggagaacc aggattagtg cctgttgtaa caggcatacg 540
tggtcgtcca ggaccggcag gacctccagg atcacaggga ccaagaggag agcgagggcc 600
aaaaggaaga cctggccctc gtggacctca gggaattgat ggagaaccag gtgttcctgg 660
tcaacctggt gctccaggac ctcctggaca tccgtcccac ccaggacccg atggcttgag 720
caggccgttt tcagctcaaa tggctgggtt ggatgaaaaa tctggacttg ggagtcaagt 780
aggactaatg cctggctctg tgggtcctgt tggcccaagg ggaccacagg gtttacaagg 840
acagcaaggt ggtgcaggac ctacaggacc tcctggtgaa cctggtgatc ctggaccaat 900
gggtccgatt ggttcacgtg gaccagaggg ccctcctggt aaacctgggg aagatggtga 960
acctggcaga aatggaaatc ctggtgaagt gggatttgca ggatctccgg gagctcgtgg 1020
atttcctggg gctcctggtc ttccaggtct gaagggtcac cgaggacaca aaggtcttga 1080
aggccctaaa ggtgaagttg gagcacctgg ttccaagggt gaagctggcc ccactggtcc 1140
aatgggtgcc atgggtcctc tgggtccgag gggaatgcca ggagagagag ggagacttgg 1200
gccacagggt gctcctggac aacgaggtgc acatggtatg cctggaaaac ctggaccaat 1260
gggtcctctt gggataccag gctcttctgg ttttccagga aatcctggaa tgaagggaga 1320
agcaggtcct acaggggcgc gaggccctga aggtcctcag ggcagagag gtgaaactgg 1380
gcccccaggt ccagttggct ctccaggtct tcctggtgca ataggaactg atggtactcc 1440
tggtcccaaa ggcccaacgg gctctccggg tacctctggt cctcctggct cagcagggcc 1500
tcctggatct ccaggacctc agggtagcac tggtcctcag gggaattcgg gccttccggg 1560
tgatccaggt ttcaaaggag aagctggccc aaaaggggaa ccagggccac atggtattca 1620
gggtccgata ggcccacccg gtgaagaagg caaaagaggt cccagaggtg acccaggaac 1680
acttggtcct ccagggccag tgggagaaag gggtgctcct ggcaatcgtg gttttccagg 1740
ctctgatggt ttacctgggc caaagggtgc tcaaggagaa cggggtcctg taggttcttc 1800
aggacccaaa ggaagccagg gggatccagg acgtccaggg gaacctgggc ttccaggtgc 1860
tcggggtttg acaggaaatc ctggtgttca aggtcctgaa ggaaaacttg gacctttggg 1920
tgcgccaggg gaagatggcc gtccaggtcc tccaggctcc ataggaatca aagggcagcc 1980
cgggaccatg ggccttccag gccccaaagg tagcaatggt gaccctggga aacctggaga 2040
agcaggaaat cctggagttc ctgggcaaag gggagctcct ggaaaagatg gtaaagttgg 2100
tccttatggt cctcctgggc cgccgggtct acgtggtgaa agaggagaac aaggacctcc 2160
agggcccaca ggttttcagg ggcatcctgg tcctccaggt cctcctggag aaggtggaaa 2220
accaggtgat caaggtgttc ctggaggtcc cggagcagtt ggcccgttag gacctagagg 2280
agaacgagga aatcctgggg aaagaggaga acctgggata actggactcc ctggtgagaa 2340
gggaatggct ggaggacatg gtcctgatgg cccaaaaggc agtccaggtc catctgggac 2400
ccctggagat acaggcccac caggtcttca aggtatgccg ggagaaagag gaattgcagg 2460
aactcctggc cccaagggtg acagaggtgg cataggagaa aaaggtgctg aaggcacagc 2520
tggaaatgat ggtgcaggag gtcttccagg tcctttgggc cctccaggtc cggcaggcct 2580
actgggagaa aagggtgaac ctggtcctcg aggtttagtt ggtcctcctg gctcccgggg 2640
caatcctggt tctcgaggtg aaaatgggcc aactggagct gttggttttg ccggaccccca 2700
ggggtctgac ggacagcctg gagtaaaagg tgaacctgga gagccaggac agaagggaga 2760
tgctggttct cctggaccac aaggtttagc aggatcccct ggccctcatg gtcctaatgg 2820
tgttcctgga ctaaaaggtg gtcgaggaac ccaaggtccg cctggtgcta caggatttcc 2880
tggttctgcg ggcagagttg gacctccagg ccctgctgga gctccaggac ctgcgggacc 2940
cctaggggaa cccgggaagg agggacctcc aggtcctcgt ggggaccctg gctctcatgg 3000
gcgtgtggga gtccgaggac cagctggccc ccctggtggc ccaggagaca aaggggaccc 3060
aggagaagat gggcaacctg gtccagatgg ccccccctggt ccagctggaa cgaccgggca 3120
gagaggaatt gttggcatgc ctgggcaacg tggagagaga ggcatgcccg gcctaccagg 3180
cccagcggga acaccaggaa aagtaggacc aactggtgca acaggagata aaggtccacc 3240
tggacctgtg gggcccccag gctccaatgg tcctgtaggg gaacctggac cagaaggtcc 3300
agctggcaat gatggtaccc caggacggga tggtgctgtt ggagaacgtg gtgatcgtgg 3360
agaccctggg cctgcaggtc tgccaggctc tcagggtgcc cctggaactc ctggccctgt 3420
gggtgctcca ggagatgcag gacaaagagg agatccgggt tctcggggtc ctataggaca 3480
cctgggtcga gctggaaaac gtggattacc tggaccccaa ggacctcgtg gtgacaaagg 3540
tgatcatgga gaccgaggcg acagaggtca gaagggccac agaggcttta ctggtcttca 3600
gggtcttcct ggccctcctg gtccaaatgg tgaacaagga agtgctggaa tccctggacc 3660
atttggccca agaggtcctc caggcccagt tggtccttca ggtaaagaag gaaaccctgg 3720
gccacttggg ccattgggac ctccaggtgt acgaggcagt gtaggagaag caggacctga 3780
gggccctcct ggtgagcctg gcccacctgg ccctccgggt ccccctggcc accttacagc 3840
tgctcttggg gatatcatgg ggcactatga tgaaagcatg ccagatccac ttcctgagtt 3900
tactgaagat caggcggctc ctgatgacaa aaacaaaacg gacccagggg ttcatgctac 3960
cctgaagtca ctcagtagtc agattgaaac catgcgcagc cccgatggct cgaaaaagca 4020
cccagcccgc acgtgtgatg acctaaagct ttgccattcc gcaaagcaga gtggtgaata 4080
ctggattgat cctaaccaag gatctgttga agatgccatc aaagtttact gcaacatgga 4140
aacaggagaa acatgtattt cagcaaaccc atccagtgta ccacgtaaaa cctggtgggc 4200
cagtaaatct cctgacaata aacctgtttg gtatggtctt gatatgaaca gagggtctca 4260
gttcgcttat ggagaccacc aatcacctaa tacagccatt actcagatga ctttttgcg 4320
ccttttatca aaagaagcct cccagaacat cacttacatc tgtaaaaaca gtgtaggata 4380

catggacgat caagctaaga acctcaaaaa agctgtggtt ctcaaagggg caaatgactt 4440
agatatcaaa gcagagggaa atattagatt ccggtatatc gttcttcaag acacttgctc 4500
taagcggaat ggaaatgtgg gcaagactgt ctttgaatat agaacacaga atgtggcacg 4560
cttgcccatc atagatcttg ctcctgtgga tgttggcggc acagaccagg aattcggcgt 4620
tgaaattggg ccagtttgtt ttgtgtaaag taagccaaga cacatcgaca atgagcacca 4680
ccatcaatga ccaccgccat tcacaagaac tttgactgtt tgaagttgat cctgagactc 4740
ttgaagtaat ggctgatcct gcatcagcat tgtatatatg gtcttaagtg cctggcctcc 4800
ttatccttca gaatatttat tttacttaca atcctcaagt tttaattgat tttaaatatt 4860
tttcaataca acagtttagg tttaagatga ccaatgacaa tgaccacctt tgcagaaagt 4920
aaactgattg aataaataaa tctccgtttt cttcaattta tttcagtgta atgaaaaagt 4980
tgcttagtat ttatgaggaa attcttcttc ctggcaggta gcttaaagag tggggtatat 5040
agagccacaa cacatgttta ttttgcttgg ctgcagttga aaaatagaaa ttagtgccct 5100
tttgtgacct ctcattccaa gattgtcaat taaaaatgag tttaaaatgt ttaacttgtg 5160
atcgagacct acatgcatgt cttgatattg tgtaactata atagagactc tttaaggaga 5220
atcttaaaaa aaaaaaacgt ttctcactgt cttaaataga atttttaaat agtatatatt 5280
cagtggcatt ttggagaaca aagtgaattt acttcgactt cttaaatttt tgtaaaagac 5340
tataagttta gacatctttc tcattcaaat ttaaagatat ctttctcctc ttgatcaatc 5400
tatcaatatt gatagaagtc acactagtat ataccattta atacatttac actttcttat 5460
ttaagaagat attgaatgca aaataattga catatagaac tttacaaaca tatgtccaag 5520
gactctaaat tgagactctt ccacatgtac aatctcatca tcctgaagcc tataatgaag 5580
aaaaagatct agaaactgag ttgtggagct gactctaatc aaatgtgatg attggaatta 5640
gaccatttgg cctttgaact ttcataggaa aaatgaccca acatttctta gcatgagcta 5700
cctcatctct agaagctggg atggacttac tattcttgtt tatattttag atactgaaag 5760
gtgctatgct tctgttatta ttccaagact ggagataggc agggctaaaa aggtattatt 5820
atttttcctt taatgatggt gctaaaattc ttcctataaa attccttaaa aataaagatg 5880
gtttaatcac taccattgtg aaaacataac tgttagactt cccgtttctg aaagaaagag 5940
catcgttcca atgcttgttc actgttcctc tgtcatactg tatctggaat gctttgtaat 6000
acttgcatgc ttcttagacc agaacatgta ggtccccttg tgtctcaata cttttttttt 6060
cttaattgca tttgttggct ctattttaat ttttttcttt taaaataaac agctgggacc 6120
atcccaaaag acaagccatg catacaactt tggtcatgta tctctgcaaa gcatcaaatt 6180
aaatgcacgc ttttgtcatg tcaaaaaaaa aaaaaaa 6217

<210> 145
<211> 851
<212> DNA
<213> Homo sapiens
<400> 145
cccgcaagtg tacctcaatg gcgagtttgt aggggggctgt gacattcttc tgcagatgca 60
ccagaatggg gacttggtgg aagaactgaa aaagctgggg atccactccg ccctttttaga 120
tgaaaagaaa gaccaagact ccaagtgagg gcggccaagt cctcgctgag cagagaggga 180
gccgttcatg tcagagactc actgccagaa aagccttacc cattttggtt ttcactattg 240
agaccgcaac tgcttgcact gatcattttg gttcatgagc agttggtgat tttagttggt 300
ctggtgttcg ggctaagaat attttattgt ggacttaatt acaaccactg cactgtaatg 360
attcaatgct gtattatgat attgctgtaa acaaaattca ttcttatatt gtcacttatt 420
ctttgcctga ttcagaagtt aaataggagc tttggaatca ttattcatga cccctctgca 480
aatgtgtcag tctccaaaga gagtatctcc ccccaaattt tgtgtagctt cttttgttat 540
ggaaaatggt ggacaaaaaa agaaactgtg ataactgggg cgttgttttt taaaataaac 600
tccagcacag ggatgctgtg catgcctgag ttgattccga aaaaaaaaaa aaaaaaaaaa 660
aaaaaaaaaa aaaaaaaaaa aaaaaaaaaa aaaaaaaaaa aaaaaaaaaa aaaaaaaaaa 720
aaaaaaaaaa aaaaaaaaaa aaaaaaaaaa aaaaaaaaaa aaaaaaaaaa aaaaaaaaaa 780
aaaaaaaaaa aaaaaaaaaa aaaaaaaaaa aaaaaaaaaa aaaaaaaaaa aaaaaaaaaa 840
aaaaaaaaaa a 851

<210> 146
<211> 4268
<212> DNA
<213> Homo sapiens
<400> 146
ctggagcaga gccgctgggc gccggagccg aggcgagcgc cgcgcgcacc actggttgga 60
gttgctgtgg gtgagctgct gtggtctgta gccaagcatg ctgtggtcgg atctgcccag 120
ccgtggaaca gaaacatttg ctggatggaa aatccataaa agaaagctcc tgtgaaaagc 180
tgaggctgac aataatttaa gcaaaatcag atcgatctct ttgggctgcc tgacctcctt 240
gggtgcttgc tattaattaa cagactttgt ggggaaaaaa aggagcttgc cttctgagct 300
ttgtaccaaa gacctgggaa aactaaccat ctcagtcttt cctgaggact tgggaactgc 360
cgaggcctct gccaatgtgt tgactgtcgc tatgggctca ctgttgtcca ggcagctcat 420
atttcaaatt ataacctatt tcctgcacca ttgctgacgc ctggtgatcc atgtcagaag 480
tacttccagc tgactcaggt gttgacacct tggcagtgtt tatggccagc agcggaacta 540
cagacgtcac aaatcggaac agcccagcca caccaccaaa caccccttaac ctccgatcct 600
cccacaatga actgttgaac gctgaaataa aacacacaga aaccaagaac agcacacctc 660

ccaaatgcag gaaaaaatat gcactaacta acatccaggc ggccatgggc ctctcggatc 720
cagctgcaca gcccctgctg ggaaatggct ctgccaacat caagctggtg aaaaatgggg 780
agaaccagct ccgtaaggct gcagagcaag ggcagcagga ccccaacaaa aacctgagcc 840
ccactgcagt catcaacata acttctgaga agttagaggg taaagagccc cacccacagg 900
attcctcgag ctgtgagatt ttaccctccc agcccaggag aactaagagc ttcctaaatt 960
actatgcaga tctggaaacc tcagccagag aactagagca gaaccgaggc aatcaccatg 1020
ggactgcgga agagaaatcc cagccagtcc agggccaggc ctccaccatc attgggaatg 1080
gcgatttgct gctgcagaaa ccaaacagac cccagtccag ccctgaagac ggccaagtag 1140
ccacagtgtc atccagccca gaaaccaaga aggatcatcc gaaaacaggg gccaaaaccg 1200
actgtgcact gcaccggatc cagaacctgg caccgagcga tgaggagtcc agctggacaa 1260
cgttgtccca agacagtgcc tcacccagct ccccggatga aacagatata tggagtgatc 1320
actcatttca gactgatcca gatttgccgc ctggctggaa aagagtcagt gacattgccg 1380
ggacctatta ttggcacatc ccaacaggaa cgactcagtg ggaacggccc gtctccatcc 1440
cagcagatct ccagggttct aggaaagggt cacttagttc tgtaacgcca tctcccaccc 1500
cagagaacga ggatttgcat gcagccactg ttaacccgga ccccagttta aaagagtttg 1560
aaggagcaac cctacgctat gcatctttga aactcagaaa tgccccacac cctgatgatg 1620
atgattcttg tagtatcaac agtgacccag aagccaagtg ttttgctgtg cgttctctgg 1680
gatgggtaga gatggcagaa gaggacctcg cccccggtaa aagtagtgtt gcggtcaaca 1740
actgcatcag gcaactttcc tactgcaaaa atgacatccg agacacagtc gggatttggg 1800
gagaggggaa agacatgtac ctgatcctgg agaatgacat gctcagcctg gtggacccca 1860
tggaccgcag cgtgctgcac tcgcagccca tcgtcagcat ccgcgtgtgg ggcgtgggcc 1920
gcgacaatgg ccgggatttt gcttatgtag caagagataa agatacaaga attttgaaat 1980
gtcatgtatt tcgatgtgac acaccagcaa aagccattgc cacaagtctc cacgagatct 2040
gctccaagat tatggctgaa cggaagaatg ccaaagcgct ggcctgcagc tccttacagg 2100
aaagggccaa tgtgaacctc gatgtccctt tgcaagtaga ttttccaaca ccaaagactg 2160
agctggtcca gaagttccac gtgcagtact tgggcatgtt acctgtagac aaaccagtcg 2220
gaatggatat tttgaacagt gccatagaaa atcttatgac ctcatccaac aaggaggact 2280
ggctgtcagt gaacatgaac gtggctgatg ccactgtgac tgtcatcagt gaaaagaatg 2340
aagaggaagt cttagtggaa tgtcgtgtgc gattcctgtc cttcatgggt gttgggaagg 2400
acgtccacac atttgccttc atcatggaca cggggaacca gcgctttgag tgccacgttt 2460
tctggtgcga gcctaatgct ggtaacgtgt ctgaggcggt gcaggccgcc tgcatgttac 2520
gatatcagaa gtgcttggta gccaggccgc cttctcagaa agttcgacca cctccaccgc 2580
cagcagattc agtaaccaga agagtcacaa ccaatgtaaa acgaggggtc ttatccctca 2640
ttgacacttt gaaacagaaa cgccctgtca ccgaaatgcc atagctgcac atgcaaaagg 2700
actcggctat ttacctgaag attgactagc tacactaaag aaaatgaact ccgccatccg 2760
accttccatc cagttgctga tgctttgtct tcagagaatt tacccttaac caagcagtgt 2820
tagacaagca tgttctctcg tcttgccacc atcatgtgat atgaaaagaa gcatgaataa 2880
tttttttgc tgtaagttac atcatgcgca gtggaaggtc tttttcttat tgtaaatatt 2940
gtgaacatta cttaacttca cacacacaca gagaagagtg tggccccacc cctcctagtg 3000
aactaacgct gcgtccttgg aatgaatgat gcgtgagtta gtttcactgt cttcttggct 3060
ggacctgtca caagcaacct ttaagtccta cagcactttg ccctgttttc aacattggag 3120
taggcactgc atagcagata ccattgaatt gctgtaaaaa taggatggcg agtttgtgtt 3180
ttaatttttc ataaaattga acctgttggt tgacaaaatt ggctgttggc atcagtatag 3240
aaaccaactg gcagctttcc ctgacaagct ctttgacaca tggacaccat ttcatgtcta 3300
cagctgtttg tgggatgttg gaaaaaaatg aaacttcaaa attgatgaaa aactaaattc 3360
gaggaattaa aatcgaacaa aacatagcct ttcttttccg atggttttca aactgattat 3420
ttttaaaaga gattaataaa atcataatgc atttgggtg ggacatattt caaacttctg 3480
ccttatattg tacggtgcag ctagagaatt atagttcact atggccattc tctacataaa 3540
cattaagatg aaatactcct catcagcctt tcatccttag tttgagaatt agctgatatg 3600
caatttgaag ttgaggaaat atcattgata tttctatcat gcacgattat tttagatttc 3660
taccaccgtg tgatttttgc tagtccatgt gctagaggta aacgttctgc tggaattctg 3720
catccagctc tatccccctc tgatgctttt tgcccagaaa gctgtctgtc catcatgtat 3780
tgtccatggc aacaaattac attaggttga acctttcctt gattttatgt atttaatatt 3840
agaatttgtt ggactcaact agatatattt tttaatttat attttttcca ttttactttg 3900
aagatttgaa atgttcatac ctgagcaaag tctacacagg agtaatggac tgtttaacaa 3960
gtttcccaaa acagcatttt cctgctcctt cgtatgtagg tgagaaactt agctggaaag 4020
acatacaaat ttagactctc gttgacattg tcgttttaaa aggaagttgc taaggcgatc 4080
aatctcaata ttagtcttgt ttacttcttc ttaatgtcaa aattaacatt tacaacatcc 4140
aattataaaa gtaatgcttt atgtttataa aaaaaaaaaa aaaaaaaaaa aaaaaaaaaa 4200
aaaaaaaaaa aaaaaaaaaa aaaaaaaaaa aaaaaaaaaa aaaaaaaaaa aaaaaaaaaa 4260
aaaaaaaa 4268

<210> 147
<211> 1167
<212> DNA
<213> Homo sapiens
<220>
<221> misc_feature
<222> (415)..(415)

<223> n = a, t, g, or c
<220>
<221> misc_feature
<222> (559)..(559)
<223> n = a, t, g, or c
<220>
<221> misc_feature
<222> (602)..(602)
<223> n = a, t, g, or c
<220>
<221> misc_feature
<222> (612)..(612)
<223> n = a, t, g, or c
<220>
<221> misc_feature
<222> (621)..(621)
<223> n = a, t, g, or c
<400> 147
gaagcgctgg gcggaagtgg ctgtggcttt gccctttggc ctttgctggc tgtgtggcgg 60
ctccgcggtt cgcaggtcgt tcgctgagcg tctctgctta gccgcggtca tgagccggca 120
cagccggctg cagaggcagg ttctgagcct gtaccgcgat ctgctgcgcg ccgggcgtgg 180
gaagccgggc gccgaggcgc gagtgcgggc agagttccgg cagcatgcgg gcctgccgcg 240
gtccgacgtg ctgcgcatcg agtacctgta ccgccgcggg cggcgccagc tgcagctgct 300
acgctcgggc cacgccaccg ccatgggcgc cttcgtacgc ccgcgggccc cgaccgggga 360
gcctggcggc gtgggttgcc agcctgacga cggcgacagt ccaaggaacc cccacgacag 420
cacgggggca ccggagaccc gccccgacgg acggtgacag gcgaagagcc gaactcgctc 480
gatggcgtgg tggagccagg aggctcgcct gactgcatgg ggggactggg gaacccgcct 540
aaggtgagag gtcttaagag actagcttga cgaattgggg atgtcagaga ctcctccttg 600
gcgacgcagg gggcctagag agccccgtga tggacggcaa gggaggcccg ccttttccga 660
tgcttggaga caggtcggtg ctcctccccc atgagggctt ggggcggcct gggacgctgg 720
cgggctggac agtgtcaagc caagagctac ttgcccgaag gtacggggag ccaggacgac 780
ccccggtgga cagggagagc ctgagacgcc cttctcttga cccctgagaa catacccact 840
tctggctcct caaggagtct cccctctcct gtatttaact ctgagaagtg cagacttttt 900
gctgagaacg ttttgggaag gtgccctgat gagcggtgag aagcccggaa tccccttctg 960
gaaaactttc ccccattaat tgtgacaagc caggaccatg aggaaggggt aggggtctat 1020
caccctggtt gatcaactga agacccccaa aggcccctac ttgatggttt tgaggggcaa 1080
cattgactca tttgcccctt ccctctcgga atgttggaca aagggaataa aattggggat 1140
atgtctttaa cttagggggt tccattc 1167

<210> 148
<211> 2509
<212> DNA
<213> Homo sapiens
<400> 148
ctgaagcctg ggtcagcag gcgctgcggg cgcagctccg gtgcaagcga ggacacgaca 60
catgcagtgg cttctggact gcgcgatgac tggacgcaag taacttctag gtctgcagac 120
aagaggaaga gaagatgaag gaagactgtc tgccgagttc tcacgtgccc atcagtgaca 180
gcaagtccat tcagaagtcg gagctcttag gcctgctgaa aacctacaac tgctaccatg 240
agggcaagag cttccagctg agacaccgtg aggaagaagg gactctgatc atcgaggggc 300
tcctcaacat tgcctggggg ctgaggcggc ccatccggct gcagatgcag gatgaccggg 360
agcaggtgca cctcccctcc acctcatgga tgcccagacg gcctagctgc cctctaaagg 420
agccatcgcc ccagaacggg aacatcacag cccaggggcc aagcattcag ccagtgcaca 480
aggctgagag ttccacagac agctcggggc ccctggagga ggcagaggag gccccccagc 540
tgatgcggac caagagcgac gccagttgca tgagccagag gaggcccaag tgccgcgccc 600
ccggtgaggc ccagcgcatc cggcgacacc ggttctctat caacggccac ttctacaatc 660
ataagacctc cgtgtttact ccagcctatg gatccgtgac caatgtgagg gtcaacagca 720
ccatgacaac cctgcaggtg ctcaccctgc tgctgaacaa atttagggtg gaagatggcc 780
ccagtgagtt cgcactctac atcgttcacg agtctgggga gcggacaaaa ttaaaagact 840
gcgagtaccc gctgatttcc agaatcctgc atgggccatg tgagaagatc gccaggatct 900
tcctgatgga agctgacttg ggcgtggaag tcccccatga agtcgctcag tacattaagt 960
ttgaaatgcc ggtgctggac agttttgttg aaaaattaaa agaagaggaa gaaagagaaa 1020
taatcaaact gaccatgaag ttccaagccc tgcgtctgac gatgctgcag cgcctggagc 1080
agctggtgga ggccaagtaa ctggccaaca cctgcctctt ccaaagtccc cagcagtggc 1140
aggtgtacac tgagccctgg ttgctggccc cggccggtca cattgactga tggccaccgc 1200
ctgacgaatc gagtgcctgt gtgtctacct ctctgaagcc tgagcaccat gattcccaca 1260
gccagctctt ggctccaaga tgagcaccca caggaagccg acccaggcct gaggggccag 1320
gaacttgctg ggtcagatct gtgtggccag ccctgtccac accatgcctc tcctgcactg 1380
gagagcagtg ctggcccagc ccctgcggct taggcttcat ctgcttgcac attgcctgtc 1440
ccagagcccc tgtgggtcca caagcccctg tcctcttcct tcatatgaga ttcttgtctg 1500

```
ccctcatatc acgctgcccc acaggaatgc tgctgggaaa agcagggcct gccagcaggt   1560
atgagatcta gcctgctttc agccatcacc ttgccacagt gtccccggct tctaagcctc   1620
caatatcacc ctgtgagcct cgcacagctc agccccaaca cagaggtgag accaggaata   1680
aggccacaag tatctcactt tctctgcaga aatcaatctt tacttcatca gagagaccta   1740
aagcgattct tacaaggagc ttgctgcaag aaacacggtc attcaatcac attgaggagg   1800
gtccacatgg cattgagagg gtgctgcccg ctcaatgccc agcagcagct ctggaaggca   1860
gtgctcagcc ccatcaccac tgtcccgtgg atgcctgtgt acctcttgcc ttttctgggc   1920
ttgcgtttct ctcctctagt gggtggggat gactttcaat gactttcaat acttcccctg   1980
aaggaagaat gataaggaga aatgtctgtt ttgaggaaag ggctttgaat tccccagata   2040
ctgaacaatt tgtgtttgtg actgatggag aatttcagga atgaatgaga aagcctttgc   2100
gaaactatgc aacagtttac atcagtcatg tgaagtattt gtctaaaaca gagcaaactg   2160
aagaccaaat tattctcctg ttgaggtccg tggatggcag atttaaaggg aagaaccaca   2220
aaggcttgca aagataggag aggctccatc tctaatgcat gtagaagctc cttacgggtg   2280
cccatcaaga gcatagcttg gaagccacca tgctgtgcgg aactgcgtca gggcaaatgt   2340
cacagcagga tttccccaac ccagctccat catcacagac acagagagct gcaggggagg   2400
cctgcccact gttttgtcga ctctgccctc ctctggcagc atagatcctt aggtgctcaa   2460
taaaggtgtg ctgtattgaa ctgaagaagt gagaaaaaaa aaaaaaaaa              2509

<210>  149
<211>  2387
<212>  DNA
<213>  Homo sapiens
<220>
<221>  misc_feature
<222>  (541)..(541)
<223>  n = a, t, g, or c
<220>
<221>  misc_feature
<222>  (706)..(706)
<223>  n = a, t, g, or c
<220>
<221>  misc_feature
<222>  (805)..(805)
<223>  n = a, t, g, or c
<400>  149
gtccccgccg gggaggtcgg gacgggcagg ggctgaggac cccgcgccag cttgggagcc     60
ctggagttct ggcgggaggg aagctgtcaa atggaagccg gtagaaatga gacagacgcc    120
cggagaagcg gggagccccg cccctccgcc atagcagcct cgggcgccgc ctcttccttt    180
ccagcctccc gccctcgtct gcttccggcc ctgtggcctg gtggggctct gcaggctccc    240
tcgggagtgg tccttgggcc gtggcccctc tgggaggcct gagggagctc aatcctggta    300
gcaacacccc tgaattcctg gtggtgaaag gatgtggccc caggacccat cccggaagga    360
ggtgctgagg tttgcagtca gctgccgtat cctgactctg atgctgcagg ccctcttcaa    420
tgccatcatc ccagatcacc atgcagaagc cttctctcct cctcgcctgg ccccctcagg    480
ctttgtggac caactcgtgg aaggtcttct gggcggcctg tctcactggg atgctgaaca    540
cttcttgttc attgctgagc atggctacct gtatgagcac aactttgcct tctttcctgg    600
tttccccttg gccctgctgg tggggactga actgttgaga cccttacggg ggttactgag    660
tctacgcagt tgcctgctga tttcggtagc atcactcaat ttcttgttct tcatgttggc    720
tgcagttgca cttcatgacc tgggttgtct ggttttgcac tgtccccacc agtcctttta    780
tgcagctctg cttttctgtc tcagccctgc caatgtcttc ctggcagctg gttactcaga    840
agctttgttt gccctcctga cattcagtgc catggggcag ctggagaggg gccgagtctg    900
gactagtgta ctcctctttg cctttgccac tggggtacgc tccaacgggc tggtcagtgt    960
tggcttcctc atgcattctc aatgccaagg ctttttctct tctctaacga tgctgaatcc   1020
tctgagacag ctctttaagc tgatggcctc tctgtttctg tcggtgttca cacttggcct   1080
tccctttgcc ctctttcagt attatgccta cacccaattc tgtctgccag gctcagcccg   1140
ccccattcct gagcctttgg tacagttagc tgtagacaag ggctaccgga ttgcagaggg   1200
aaatgaaccg ccttggtgct tctgggatgt tccactaata tacagctata tccaggatgt   1260
ctactggaat gttggctttt tgaaatacta tgagctcaag caggtgccca atttctact    1320
ggctgcacca gtggctatac tggttgcctg ggcaacttgg acatacgtga ccactcaccc   1380
ttggctctgc cttacacttg ggctgcaaag gagcaagaac aataagaccc tagagaagcc   1440
cgatcttgga ttcctcagtc ctcaggtgtt tgtgtacgtg gtccacgctg cagtgctgct   1500
gctgtttgga ggtctgtgca tgcatgttca ggttctcacc aggtttttgg gctcctccac   1560
tcctattatg tactggtttc cagctcactt gcttcaggat caagagccgc tgttgagatc   1620
cttaaagact gtgccttgga agcctcttgc agaggactcc ccaccaggac aaaaggtccc   1680
cagaaatcct atcatgggac ttttgtatca ctggaaaacc tgttctccag tcacacgata   1740
cattctaggc tacttcctga cttactggct cctgggacta ctcctacatt gcaacttcct   1800
gccttggaca tgacctggac tctccaggga caggttggaa gccaacttaa cccaggggtc   1860
tgaaagtaaa aatacacatt ggaactgcct ctgctgccct gggatcatta ctgtgtccat   1920
tataatcttt ctctttctct ttgaaagctg gtcaggaatg ggagaagtgt cagacactag   1980
agagcccctt ctggtcctgg ctagggcaaa ttttagacaa ctattttctc tgtaagtgaa   2040
```

gattgtcgta ttccaagtct aaaatacacc tggatctgtc tagtcaatca acatagcaga 2100
gacagtctta aacctaccat tgacctgtgt gtaaatttaa atgtcaattt attgaagtgt 2160
aaatttcatc aaaggcatta gctgacaggc tggtaacagt ccacacaaga tggtataggc 2220
ctgaacagtg tagtggcagt aataaagtgg gaccattttt tccaaatgcg acattgtttc 2280
tgatgttttt ttttatgatt tgtgcatgta tttttatatg aaaaaaactt acaaagatat 2340
gcaaattaaa cttctttcag ttataaaaaa aaaaaaaaaa ccgcttg 2387

<210> 150
<211> 4917
<212> DNA
<213> Homo sapiens
<400> 150
agaagatgtc ttcgcggacg gtgctggccc cgggcaacga tcggaactcg gacacgcatg 60
gcaccttggg cagtggccgc tcctcggaca aaggcccgtc ctggtccagc cgctcactgg 120
gtgcccgttg ccggaactcc atcgcctcct gtcccgagga gcagccccac gtgggcaact 180
accgcctgct gaggaccatt gggaagggca actttgccaa agtcaagctg gctcggcaca 240
tcctcactgg tcgggaggtt gccatcaaga ttatcgacaa aacccagctg aatcccagca 300
gcctgcagaa gctgttccga gaagtccgca tcatgaaggg cctaaaccac cccaacatcg 360
tgaagctctt tgaggtgatt gagactgaga agacgctgta cctggtgatg gagtacgcaa 420
gtgctggaga agtgtttgac tacctcgtgt cgcatggccg catgaaggag aaggaagctc 480
gagccaagtt ccgacagatt gtttcggctg tgcactattg tcaccagaaa aatattgtac 540
acagggacct gaaggctgag aacctcttgc tggatgccga ggccaacatc aagattgctg 600
actttggctt cagcaacgag ttcacgctgg gatcgaagct ggacacgttc tgcgggagcc 660
ccccatatgc cgccccggag ctgtttcagg gcaagaagta cgacgggccg gaggtggaca 720
tctggagcct gggagtcatc ctgtacaccc tcgtcagcgg ctccctgccc ttcgacgggc 780
acaacctcaa ggagctgcgg gagcgagtac tcagagggaa gtaccgggtc cctttctaca 840
tgtcaacaga ctgtgagagc atcctgcgga gatttttggt gctgaaccca gctaaacgct 900
gtactctcga gcaaatcatg aaagacaaat ggatcaacat cggctatgag ggtgaggagt 960
tgaagccata cacagagccc gaggaggact tcggggacac caagagaatt gaggtgatgg 1020
tgggtatggg ctacacacgg gaagaaatca aagagtcctt gaccagccag aagtacaacg 1080
aagtgaccgc cacctacctc ctgctgggca ggaagactga ggagggtggg gaccgggcg 1140
ccccagggct ggccctggca cgggtgcggg cgcccagcga caccaccaac ggaacaagtt 1200
ccagcaaagg caccagccac agcaaagggc agcggagttc ctcttccacc taccaccgcc 1260
agcgcaggca tagcgatttc tgtggcccat cccctgcacc cctgcacccc aaacgcagcc 1320
cgacgagcac ggggaggcg gagctgaagg aggagcggct gccaggccgg aaggcgagct 1380
gcagcaccgc ggggagtggg agtcgagggc tgccccccctc cagccccatg gtcagcagcg 1440
cccacaaccc caacaaggca gagatcccag agcggcggaa ggacagcacg agcaccccca 1500
acaacctccc tcctagcatg atgacccgca gaaacaccta cgtttgcaca gaacgcccgg 1560
gggctgagcg cccgtcactg ttgccaaatg ggaaagaaaa cagctcaggc accccacggg 1620
tgccccctgc ctccccctcc agtcacagcc tggcaccccc atcaggggag cggagccgcc 1680
tggcacgcgg ttccaccatc cgcagcacct tccatggtgg ccaggtccgg gaccggcggg 1740
caggggtgg gggtggtggg ggtgtgcaga atgggccccc tgcctctccc acactggccc 1800
atgaggctgc acccctgccc gccgggcggc cccgccccac caccaacctc ttcaccaagc 1860
tgacctccaa actgacccga agggttaccc tcgatccctc taaacggcag aactctaacc 1920
gctgtgtttc gggcgcctct ctgccccagg gatccaagat caggtcgcag acgaacctga 1980
gagaatcggg ggacctgagg tcacaagttg ccatctacct tgggatcaaa cggaaaccgc 2040
cccccggctg ctccgattcc cctggagtgt gaagctgacc agctcgcgcc ctcctgaggc 2100
cctgatggca gctctgcgcc aggccacagc agccgcccgc tgccgctgcc gccagccaca 2160
gccgttcctg ctggcctgcc tgcacggggg tgcgggcggg cccgagcccc tgtcccactt 2220
cgaagtggag gtctgccagc tgccccggcc aggcttgcgg ggagttctct tccgccgtgt 2280
ggcgggcacc gccctggcct tccgcaccct cgtcacccgc atctccaacg acctcgagct 2340
ctgagccacc acggtcccag ggcccttact cttcctctcc cttgtcgcct tcacttctac 2400
aggaggggaa ggggccaggg aggggattct ccctttatca tcacctcagt ttccctgaat 2460
tatatttggg ggcaaagatt gtcccctctg ctgttctctg ggccgctca gcacagaaga 2520
aggatgaggg ggctcagcgg ggggagctgg caccttcctg gagcctccag ccagtcctgt 2580
cctccctcgc cctaccaaga gggcacctga ggagactttg gggacagggc aggggcaggg 2640
agggaaactg aggaaatctt ccattcctcc caacagctca aaattaggcc ttgggcaggg 2700
gcagggagag ctgctgagcc taaagactgg agaatctggg ggactgggag tgggggtcag 2760
agaggcagat tccttcccct cccgtcccct cacgctcaaa cccccacttc ctgccccagg 2820
ctggcgcggg gcactttgta caaatccttg taaatacccc acaccctccc ctctgcaaag 2880
gtctcttgag gagctgccgc tgtcacctac ggtttttaag ttattacacc ccgaccctcc 2940
tcctgtcagc cccctcacct gcagcctgtt gcccaataaa tttaagagag tccccccctc 3000
cccaatgctg accctaggat tttccttccc tgccctcacc tgcaaatgag ttaaagaaga 3060
ggcgtgggaa tccaggcagt ggtttttcct ttcggagcct cggttttctc atctgcagaa 3120
tgggagcggt gggggtggga aggtaaggat ggtcgtggaa gaaggcagga tggaactcgg 3180
cctcatcccc gaggccccag ttcctatatc gggcccccca ttcatccact cacactccca 3240
gccaccatgt tacactggac tctaagccac ttcttactcc agtagtaaat ttattcaata 3300
aacaatcatt gacccatgcc tactccatgc caggcccagt gctggacaca gagacatgaa 3360
gctctgtctg tgggagacag ggattctgac acagacaccg gacaaaccat tgtcttgggg 3420

```
agcccagaag agaaagtggg cagggtgggg tcattgggga agatgctcta gaggaattaa   3480
tgctggaatg ggggtgttgaa ggatgagtag gagttagtta ggcattgagt ttgccctggg   3540
caaaagccca gaagtgggag tatgtggtat atcttcagag aactgggtaa tttcagtgtg   3600
gctgctgtgt tgggcatgga tggagaatca gcaagagaaa tgctgtatta ggactaataa   3660
tccatctacg ctgcttaagc aaaaaggtat ttgttggttt atgttactta atagtccagg   3720
ggcacctggc ttcaggtagg tttgatccag gcatcaggcc attgcatcta ttttttcagt   3780
gtaagttgaa ttctagtaat ttttatcaag taagggctcc tttcctggtg gcacagatga   3840
cttcagcagt tagaagtttc tatccctcca gctttctgca gcagaaagac cctcattgtc   3900
agtttcccag caaaagtccc agggcagact ctcattggcc caaatgggcc atgtgatttt   3960
ctctaaacca atcactgtga ctctagagtg gccagactca gagctgcact tagtaggggt   4020
tcctcaaagg aaggtcaagt gtcatgagca ggagaaaagg catgggagct ggacagatta   4080
tagtggttga agtctgtgca gtacagaagg gcggagctta ttcacacagc acctttgggg   4140
ccaaaatgaa taagctggac tttctcccca tggcactggg gaaccatgga agttcaggga   4200
acttcaggga agaggcttgg tcaattcctg agagcatcct ctgtgctggg gacacagtgg   4260
taatcaagac agccccaaca ctgccctcat agagctcaca gtccaatgga ggaggcagat   4320
gtgtcctcag gcagcgactg ggcagggctg gtatagggga gtccagaggt gatgcctgcc   4380
tcagccaggg agggcttcct ggaggagaag gagccagcta gacatggata ggagtgcgtt   4440
ttaggcacag caaatggcac atacaagggc cagggagcaa gagagaggac aggtcctcaa   4500
caaatggcat gtgactttgt aagtgtagaa ttgctgtgag gtatggggct aggggcgtca   4560
gtagggcctt gaaggttatg gacaggggcc tgggctttct tccaagggca ctgggggagc   4620
catggcaagg ttgtaggtag ggtagagatg ggcgggtttg tgctatgtgc agggtggaag   4680
ggagggaagt tgacaggtca gaagatcagg aaagaggtcg gggctggaca gatggggaga   4740
gcgcagatag atttaagaga gtcctgtgag gcaaagtggg caggacctgg taacaggtgt   4800
ctggactgtg gctttggctg gctcagaagg tccccactgg cgtgtgtggt ctatgtagcc   4860
tctgggtgtg gagctgggat cttcaactgg ggacagtaca gtaaagaaca tcacagc      4917
```

<210> 151
<211> 1804
<212> DNA
<213> Homo sapiens
<400> 151

```
gtatcactca gaatctggca gccagttccg tcctgacaga gttcacagca tatattggtg     60
gattcttgtc catagtgcat ctgctttaag aattaacgaa agcagtgtca agacagtaag    120
gattcaaacc atttgccaaa aatgagtcta agtgcattta ctctcttcct ggcattgatt    180
ggtggtacca gtggccagta ctatgattat gattttcccc tatcaattta tgggcaatca    240
tcaccaaact gtgcaccaga atgtaactgc cctgaaagct acccaagtgc catgtactgt    300
gatgagctga aattgaaaag tgtaccaatg gtgcctcctg gaatcaagta tctttacctt    360
aggaataacc agattgacca tattgatgaa aaggcctttg agaatgtaac tgatctgcag    420
tggctcattc tagatcacaa ccttctagaa aactccaaga taaaagggag agttttctct    480
aaattgaaac aactgaagaa gctgcatata aaccacaaca acctgacaga gtctgtgggc    540
ccacttccca aatctctgga ggatctgcag cttactcata acaagatcac aaagctgggc    600
tcttttgaag gattggtaaa cctgaccttc atccatctcc agcacaatcg gctgaaagag    660
gatgctgttt cagctgcttt taaaggtctt aaatcactcg aataccttga cttgagcttc    720
aatcagatag ccagactgcc ttctggtctc cctgtctctc ttctaactct ctacttagac    780
aacaataaga tcagcaacat ccctgatgag tatttcaagc gttttaatgc attgcagtat    840
ctgcgtttat ctcacaacga actggctgat agtggaatac ctggaaattc tttcaatgtg    900
tcatccctgg ttgagctgga tctgtcctat aacaagctta aaaacatacc aactgtcaat    960
gaaaaccttg aaaactatta cctggaggtc aatcaacttg agaagtttga cataaagagc   1020
ttctgcaaga tcctggggcc attatcctac tccaagatca agcatttgcg tttggatggc   1080
aatcgcatct cagaaaccag tcttccaccg gatatgtatg aatgtctacg tgttgctaac   1140
gaagtcactc ttaattaata tctgtatcct ggaacaatat tttatggtta tgtttttctg   1200
tgtgtcagtt ttcatagtat ccatatttta ttactgttta ttacttccat gaattttaaa   1260
atctgaggga aatgttttgt aaacatttat ttttttttaaa gaaaagatga aaggcaggcc   1320
tatttcatca caagaacaca cacatataca cgaatagaca tcaaactcaa tgctttattt   1380
gtaaatttag tgttttttta tttctactgt caaatgatgt gcaaaacctt ttactggttg   1440
catggaaatc agccaagttt tataatcctt aaatcttaat gttcctcaaa gcttggatta   1500
aatacatatg gatgttactc tcttgcacca aattatcttg atacattcaa atttgtctgg   1560
ttaaaaaata ggtggtagat attgaggcca agaatattgc aaaatacatg aagcttcatg   1620
cacttaaaga agtattttta gaataagaat ttgcatactt acctagtgaa acttttctag   1680
aattattttt cactctaagt catgtatgtt tctctttgat tatttgcatg ttatgtttaa   1740
taagctacta gcaaaataaa acatagcaaa tgaaaaaaaa aaaaaaaaaa aaaaaaaaaa   1800
aaaa                                                                1804
```

<210> 152
<211> 5489
<212> DNA
<213> Homo sapiens
<400> 152

```
ggctgagttt tatgacgggc ccggtgctga agggcaggga acaacttgat ggtgctactt     60
```

tgaactgctt ttcttttctc ctttttgcac aaagagtctc atgtctgata tttagacatg 120
atgagctttg tgcaaaaggg gagctggcta cttctcgctc tgcttcatcc cactattatt 180
ttggcacaac aggaagctgt tgaaggagga tgttcccatc ttggtcagtc ctatgcggat 240
agagatgtct ggaagccaga accatgccaa atatgtgtct gtgactcagg atccgttctc 300
tgcgatgaca taatatgtga cgatcaagaa ttagactgcc ccaacccaga aattccattt 360
ggagaatgtt gtgcagtttg cccacagcct ccaactgctc ctactcgccc tcctaatggt 420
caaggacctc aaggccccaa gggagatcca ggccctcctg gtattcctgg gagaaatggt 480
gaccctggta ttccaggaca accagggtcc cctggttctc ctggcccccc tggaatctgt 540
gaatcatgcc ctactggtcc tcagaactat tctccccagt atgattcata tgatgtcaag 600
tctggagtag cagtaggagg actcgcaggc tatcctggac cagctggccc cccaggccct 660
cccggtcccc ctggtacatc tggtcatcct ggttcccctg gatctccagg ataccaagga 720
ccccctggtg aacctgggca agctggtcct tcaggccctc caggacctcc tggtgctata 780
ggtccatctg gtcctgctgg aaaagatgga gaatcaggta gacccggacg acctggagag 840
cgaggattgc ctggacctcc aggtatcaaa ggtccagctg ggatacctgg attccctggt 900
atgaaaggac acagaggctt cgatggacga aatggagaaa agggtgaaac aggtgctcct 960
ggattaaagg gtgaaaatgg tcttccaggc gaaaatggag ctcctggacc catgggtcca 1020
agagggggctc ctggtgagcg aggacggcca ggacttcctg gggctgcagg tgctcggggt 1080
aatgacggtg ctcgaggcag tgatggtcaa ccaggccctc ctggtcctcc tggaactgcc 1140
ggattccctg gatcccctgg tgctaagggt gaagttggac ctgcagggtc tcctggttca 1200
aatggtgccc ctggacaaag aggagaacct ggacctcagg gacacgctgg tgctcaaggt 1260
cctcctggcc ctcctgggat taatggtagt cctggtggta aaggcgaaat gggtcccgct 1320
ggcattcctg gagctcctgg actgatggga gcccggggtc ctccaggacc agccggtgct 1380
aatggtgctc ctggactgcg aggtggtgca ggtgagcctg gtaagaatgg tgccaaagga 1440
gagcccggac cacgtggtga acgcggtgag gctggtattc caggtgttcc aggagctaaa 1500
ggcgaagatg gcaaggatgg atcacctgga gaacctggtg caaatgggct tccaggagct 1560
gcaggagaaa ggggtgcccc tgggttccga ggacctgctg gaccaaatgg catcccagga 1620
gaaaagggtc ctgctggaga gcgtggtgct ccaggccctg cagggcccag aggagctgct 1680
ggagaacctg gcagagatgg cgtccctgga ggtccaggaa tgaggggcat gcccggaagt 1740
ccaggaggac caggaagtga tgggaaacca gggcctcccg gaagtcaagg agaaagtggt 1800
cgaccaggtc ctcctgggcc atctggtccc cgaggtcagc ctggtgtcat gggcttcccc 1860
ggtcctaaag gaaatgatgg tgctcctggt aagaatggag aacgaggtgg ccctggagga 1920
cctggccctc agggtcctcc tggaaagaat ggtgaaactg gacctcaagg acccccaggg 1980
cctactgggc ctggtggtga caaaggagac acaggacccc ctggtccaca aggattacaa 2040
ggcttgcctg gtacaggtgg tcctccagga gaaaatggaa aacctgggga accaggtcca 2100
aagggtgatg ccggtgcacc tggagctcca ggaggcaagg gtgatgctgg tgcccctggt 2160
gaacgtggac ctcctggatt ggcagggggcc ccaggactta gaggtggagc tggtcccccct 2220
ggtcccgaag gaggaaaggg tgctgctggt cctcctgggc cacctggtgc tgctggtact 2280
cctggtctgc aaggaatgcc tggagaaaga ggaggtcttg gaagtcctgg tccaaagggt 2340
gacaagggtg aaccaggcgg cccaggtgct gatggtgtcc cagggaaaga tggcccaagg 2400
ggtcctactg gtcctattgg tcctcctggc ccagctggcc agcctggaga taagggtgaa 2460
ggtggtgccc ccggacttcc aggtatagct ggacctcgtg gtagccctgg tgagagaggt 2520
gaaactggcc ctccaggacc tgctggtttc cctggtgctc ctggacagaa tggtgaacct 2580
ggtggtaaag gagaaagagg ggctccgggt gagaaaggtg aaggaggccc tcctggagtt 2640
gcaggacccc ctggaggttc tggacctgct ggtcctcctg gtccccaagg tgtcaaaggt 2700
gaacgtggca gtcctggtgg acctggtgct gctggcttcc ctggtgctcg tggtcttcct 2760
ggtcctcctg gtagtaatgg taacccagga cccccaggtc ccagcggttc tccaggcaag 2820
gatgggcccc caggtcctgc gggtaacact ggtgctcctg gcagccctgg agtgtctgga 2880
ccaaaaggtg atgctggcca accaggagag aagggatcgc ctggtgccca gggcccacca 2940
ggagctccag gcccacttgg gattgctggg atcactggag cacggggtct tgcaggacca 3000
ccaggcatgc caggtcctag gggaagccct ggccctcagg gtgtcaaggg tgaaagtggg 3060
aaaccaggag ctaacggtct cagtggagaa cgtggtcccc ctggacccca gggtcttcct 3120
ggtctggctg gtacagctgg tgaacctgga agagatggaa accctggatc agatggtctt 3180
ccaggccgag atggatctcc tggtggcaag ggtgatcgtg gtgaaaatgg ctctcctggt 3240
gcccctggcg ctcctggtca tccaggccca cctggtcctg tcggtccagc tggaaagagt 3300
ggtgacagag gagaaagtgg ccctgctggc cctgctggtg ctcccggtcc tgctggttcc 3360
cgaggtgctc ctggtcctca aggcccacgt ggtgacaaag gtgaaacagg tgaacgtgga 3420
gctgctggca tcaaaggaca tcgaggattc cctggtaatc caggtgcccc aggttctcca 3480
ggccctgctg gtcagcaggg tgcaatcggc agtccaggac ctgcaggccc cagaggacct 3540
gttggaccca gtggacctcc tggcaaagat ggaaccagtg gacatccagg tcccattgga 3600
ccaccagggc ctcgaggtaa cagaggtgaa agaggatctg agggctcccc aggccaccca 3660
gggcaaccag gccctcctgg acctcctggt gcccctggtc cttgctgtgg tggtgttgga 3720
gccgctgcca ttgctgggat tggaggtgaa aaagctggcg gttttgcccc gtattatgga 3780
gatgaaccaa tggatttcaa aatcaacacc gatgagatta tgacttcact caagtctgtt 3840
aatggacaaa tagaaagcct cattagtcct gatggttctc gtaaaaaccc cgctagaaac 3900
tgcagagacc tgaaattctg ccatcctgaa ctcaagagtg gagaatactg ggttgaccct 3960
aaccaaggat gcaaattgga tgctatcaag gtattctgta atatggaaac tggggaaaca 4020
tgcataagtg ccaatccttt gaatgttcca cggaaacact ggtggacaga ttctagtgct 4080
gagaagaaac acgtttggtt tggagagtcc atggatggtg gttttcagtt tagctacggc 4140
aatcctgaac ttcctgaaga tgtccttgat gtgcagctgg cattccttcg acttctctcc 4200

agccgagctt cccagaacat cacatatcac tgcaaaaata gcattgcata catggatcag 4260
gccagtggaa atgtaaagaa ggccctgaag ctgatggggt caaatgaagg tgaattcaag 4320
gctgaaggaa atagcaaatt cacctacaca gttctggagg atggttgcac gaaacacact 4380
ggggaatgga gcaaaacagt ctttgaatat cgaacacgca aggctgtgag actacctatt 4440
gtagatattg caccctatga cattggtggt cctgatcaag aatttggtgt ggacgttggc 4500
cctgtttgct ttttataaac caaactctat ctgaaatccc aacaaaaaaa atttaactcc 4560
atatgtgttc ctcttgttct aatcttgtca accagtgcaa gtgaccgaca aaattccagt 4620
tatttatttc caaaatgttt ggaaacagta taatttgaca aagaaaaatg atacttctct 4680
ttttttgctg ttccaccaaa tacaattcaa atgctttttg ttttattttt ttaccaattc 4740
caatttcaaa atgtctcaat ggtgctataa taaataaact tcaacactct ttatgataac 4800
aacactgtgt tatattcttt gaatcctagc ccatctgcag agcaatgact gtgctcacca 4860
gtaaaagata acctttcttt ctgaaatagt caaatacgaa attagaaaag ccctccctat 4920
tttaactacc tcaactggtc agaaacacag attgtattct atgagtccca gaagatgaaa 4980
aaaattttat acgttgataa aacttataaa tttcattgat taatctcctg gaagattggt 5040
ttaaaaagaa aagtgtaatg caagaattta aagaaatatt tttaaagcca caattatttt 5100
aatattggat atcaactgct tgtaaaggtg ctcctctttt ttcttgtcat tgctggtcaa 5160
gattactaat atttgggaag gctttaaaga cgcatgttat ggtgctaatg tactttcact 5220
tttaaactct agatcagaat tgttgacttg cattcagaac ataaatgcac aaaatctgta 5280
catgtctccc atcagaaaga ttcattggca tgccacaggg attctcctcc ttcatcctgt 5340
aaaggtcaac aataaaaacc aaattatggg gctgcttttg tcacactagc atagagaatg 5400
tgttgaaatt taactttgta agcttgtatg tggttgttga tctttttttt ccttacagac 5460
acccataata aaatatcata ttaaaattc 5489

<210> 153
<211> 5921
<212> DNA
<213> Homo sapiens
<400> 153
agcagacggg agtttctcct cggggtcgga gcaggaggca cgcggagtgt gaggccacgc 60
atgagcggac gctaaccccc tccccagcca caaagagtct acatgtctag ggtctagaca 120
tgttcagctt tgtggacctc cggctcctgc tcctcttagc ggccaccgcc ctcctgacgc 180
acggccaaga ggaaggccaa gtcgagggcc aagacgaaga catcccacca atcacctgcg 240
tacagaacgg cctcaggtac catgaccgag acgtgtggaa acccgagccc tgccggatct 300
gcgtctgcga caacggcaag gtgttgtgcg atgacgtgat ctgtgacgag accaagaact 360
gccccggcgc cgaagtcccc gagggcgagt gctgtcccgt ctgccccgac ggctcagagt 420
cacccaccga ccaagaaacc accggcgtcg agggacccaa gggagacact ggcccccgag 480
gcccaagggg acccgcaggc ccccctggcc gagatggcat ccctggacag cctggacttc 540
ccggaccccc cggacccccc ggacctcccg gacccccctgg cctcggagga aactttgctc 600
cccagctgtc ttatggctat gatgagaaat caaccggagg aatttccgtg cctggcccca 660
tgggtccctc tggtcctcgt ggtctccctg gcccccctgg tgcacctggt ccccaaggct 720
tccaaggtcc ccctggtgag cctggcgagc ctggagcttc aggtcccatg ggtccccgag 780
gtcccccagg tccccctgga aagaatggag atgatgggga agctggaaaa cctggtcgtc 840
ctggtgagcg tgggcctcct gggcctcagg gtgctcgagg attgcccgga acagctggcc 900
tccctggaat gaagggacac agaggtttca gtggtttgga tggtgccaag ggagatgctg 960
gtcctgctgg tcctaagggt gagcctggca gccctggtga aaatggagct cctggtcaga 1020
tgggcccccg tggcctgcct ggtgagagag gtcgccctgg agcccctggc cctgctggtg 1080
ctcgtggaaa tgatggtgct actggtgctg ccgggccccc tggtcccacc ggccccgctg 1140
gtcctcctgg cttccctggt gctgttggtg ctaagggtga agctggtccc caagggcccc 1200
gaggctctga aggtccccag ggtgtgcgtg gtgagcctgg ccccccctggc cctgctggtg 1260
ctgctggccc tgctggaaac cctggtgctg atggacagcc tggtgctaaa ggtgccaatg 1320
gtgctcctgg tattgctggt gctcctggct tccctggtgc ccgaggcccc tctggacccc 1380
agggccccgg cggccctcct ggtcccaagg gtaacagcgg tgaacctggt gctcctggca 1440
gcaaaggaga cactggtgct aagggagagc ctggccctgt tggtgttcaa ggaccccctg 1500
gccctgctgg agaggaagga aagcgaggag ctcgaggtga acccggaccc actggcctgc 1560
ccggaccccc tggcgagcgt ggtggacctg gtagccgtgg tttccctggc gcagatggtg 1620
ttgctggtcc caagggtccc gctggtgaac gtggttctcc tggccccgct ggccccaaag 1680
gatctcctgg tgaagctggt cgtcccggtg aagctggtct gcctggtgcc aagggtctga 1740
ctggaagccc tggcagccct ggtcctgatg gcaaaactgg ccccccctggt cccgccggtc 1800
aagatggtcg ccccggaccc ccaggcccac ctggtgcccg tggtcaggct ggtgtgatgg 1860
gattccctgg acctaaaggt gctgctggag agcccggcaa ggctggagag cgaggtgttc 1920
ccggaccccc tggcgctgtc ggtcctgctg gcaaagatgg agaggctgga gctcagggac 1980
cccctggccc tgctggtccc gctggcgaga gaggtgaaca aggccctgct ggctcccccg 2040
gattccaggg tctccctggt cctgctggtc ctccaggtga agcaggcaaa cctggtgaac 2100
agggtgttcc tggagacctt ggcgcccctg gcccctctgg agcaagaggc gagagaggtt 2160
tccctggcga gcgtggtgtg caaggtcccc ctggtcctgc tggaccccga ggggccaacg 2220
gtgctcccgg caacgatggt gctaagggtg atgctggtgc ccctggagct cccggtagcc 2280
agggcgcccc tggccttcag ggaatgcctg gtgaacgtgg tgcagctggt cttccagggc 2340
ctaagggtga cagaggtgat gctggtccca aaggtgctga tggctctcct ggcaaagatg 2400
gcgtccgtgg tctgaccggc cccattggtc ctcctggccc tgctggtgcc cctggtgaca 2460

agggtgaaag tggtcccagc ggccctgctg gtcccactgg agctcgtggt gcccccggag 2520
accgtggtga gcctggtccc cccggccctg ctggctttgc tggcccccct ggtgctgacg 2580
gccaacctgg tgctaaaggc gaacctggtg atgctggtgc caaaggcgat gctggtcccc 2640
ctgggcctgc cggacccgct ggacccccctg gccccattgg taatgttggt gctcctggag 2700
ccaaaggtgc tcgcggcagc gctggtcccc ctggtgctac tggtttccct ggtgctgctg 2760
gccgagtcgg tcctcctggc ccctctggaa atgctggacc ccctggccct cctggtcctg 2820
ctggcaaaga aggcggcaaa ggtccccgtg gtgagactgg ccctgctgga cgtcctggtg 2880
aagttggtcc ccctggtccc cctggccctg ctggcgagaa aggatcccct ggtgctgatg 2940
gtcctgctgg tgctcctggt actcccgggc ctcaaggtat tgctggacag cgtggtgtgg 3000
tcggcctgcc tggtcagaga ggagagagag gcttccctgg tcttcctggc ccctctggtg 3060
aacctggcaa acaaggtccc tctggagcaa gtggtgaacg tggtcccccc ggtcccatgg 3120
gcccccctgg attggctgga ccccctggtg aatctggacg tgagggggct cctgctgccg 3180
aaggttcccc tggacgagac ggttctcctg gcgccaaggg tgaccgtggt gagaccggcc 3240
ccgctggacc ccctggtgct cctggtgctc ctggtgcccc tggccccgtt ggccctgctg 3300
gcaagagtgg tgatcgtggt gagactggtc ctgctggtcc cgcccggtccc gtcggccccg 3360
tcggcgcccg tggccccgcc ggaccccaag gcccccgtgg tgacaagggt gagacaggcg 3420
aacagggcga cagaggcata aagggtcacc gtggcttctc tggcctccag ggtccccctg 3480
gccctcctgg ctctcctggt gaacaaggtc cctctggagc ctctggtcct gctggtcccc 3540
gaggtccccc tggctctgct ggtgctcctg gcaaagatgg actcaacggt ctccctggcc 3600
ccattgggcc ccctggtcct cgcggtcgca ctggtgatgc tggtcctgtt ggtccccccg 3660
gccctcctgg acctcctggt ccccctggtc ctcccagcgc tggtttcgac ttcagcttcc 3720
tgccccagcc acctcaagag aaggctcacg atggtggccg ctactaccgg gctgatgatg 3780
ccaatgtggt tcgtgaccgt gacctcgagg tggacaccac cctcaagagc ctgagccagc 3840
agatcgagaa catccggagc ccagagggaa gccgcaagaa ccccgcccgc acctgccgtg 3900
acctcaagat gtgccactct gactggaaga gtggagagta ctggattgac cccaaccaag 3960
gctgcaacct ggatgccatc aaagtcttct gcaacatgga gactggtgag acctgcgtgt 4020
accccactca gcccagtgtg gcccagaaga actggtacat cagcaagaac cccaaggaca 4080
agaggcatgt ctggttcggc gagagcatga ccgatggatt ccagttcgag tatggcggcc 4140
agggctccga ccctgccgat gtggccatcc agctgacctt cctgcgcctg atgtccaccg 4200
aggcctccca gaacatcacc taccactgca agaacagcgt ggcctacatg gaccagcaga 4260
ctggcaacct caagaaggcc ctgctcctca agggctccaa cgagatcgag atccgcgccg 4320
agggcaacag ccgcttcacc tacagcgtca ctgtcgatgg ctgcacgagt cacaccggag 4380
cctggggcaa gacagtgatt gaatacaaaa ccaccaagtc ctcccgcctg cccatcatcg 4440
atgtggcccc cttggacgtt ggtgccccag accaggaatt cggcttcgac gttggccctg 4500
tctgcttcct gtaaactccc tccatcccaa cctggctccc tcccacccaa ccaactttcc 4560
ccccaacccg gaaacagaca agcaacccaa actgaacccc cccaaaagcc aaaaaatggg 4620
agacaatttc acatggactt tggaaaatat ttttttcctt tgcattcatc tctcaaactt 4680
agtttttatc tttgaccaac cgaacatgac caaaaaccaa aagtgcattc aaccttacca 4740
aaaaaaaaaa aaaaaaaaaa agaataaata aataagtttt taaaaaagga agcttggtcc 4800
acttgcttga agacccatgc gggggtaagt ccctttctgc ccgttgggtt atgaaacccc 4860
aatgctgccc tttctgctcc tttctccaca cccccttgg cctcccctcc actccttccc 4920
aaatctgtct ccccagaaga cacaggaaac aatgtattgt ctgcccagca atcaaaggca 4980
atgctcaaac acccaagtgg cccccaccct cagcccgctc ctgcccgccc agcaccccca 5040
ggccctgggg acctggggtt ctcagactgc caaagaagcc ttgccatctg gcgctcccat 5100
ggctcttgca acatctcccc ttcgtttttg aggggtcat gccgggggag ccaccagccc 5160
ctcactgggt tcggaggaga gtcaggaagg gccacgacaa agcagaaaca tcggatttgg 5220
ggaacgcgtg tcatcccttg tgccgcaggc tgggcgggag agactgttct gttctgttcc 5280
ttgtgtaact gtgttgctga aagactacct cgttcttgtc ttgatgtgtc accggggcaa 5340
ctgcctgggg gcggggatgg gggcagggtg gaagcggctc ccccattttta taccaaaggt 5400
gctacatcta tgtgatgggt ggggtgggga gggaatcact ggtgctatag aaattgagat 5460
gcccccccag gccagcaaat gttccttttt gttcaaagtc tattttttatt ccttgatatt 5520
ttttctttct tttttttttt ttttgtggat ggggacttgt gaatttttct aaaggtgcta 5580
tttaacatgg gaggagagcg tgtgcgctcc agcccagccc gctgctcact ttccaccctc 5640
tctccacctg cctctggctt ctcaggcctc tgctctccga cctctctcct ctgaaaccct 5700
cctccacagc tgcagcccat cctcccggct ccctcctagt ctgtcctgcg tcctctgtcc 5760
ccgggtttca gagacaactt cccaaagcac aaagcagttt ttccctaggg gtgggaggaa 5820
gcaaaagact ctgtacctat tttgtatgtg tataataatt tgagatgttt ttaattattt 5880
tgattgctgg aataaagcat gtggaaatga cccaaacata a 5921

<210> 154
<211> 2565
<212> DNA
<213> Homo sapiens
<400> 154
ccgggaaagt gatgtgggta ggacaggcgg ggcgagccgc aggtgccaga acacagattg 60
tataaaaggc tgggggctgg tggggagcag gggaagggaa tgtgaccagg tctaggtctg 120
gagtttcagc ttggacactg agccaagcag acaagcaaag caagccagga cacaccatcc 180
tgccccaggc ccagcttctc tcctgccttc caacgccatg gggagcaatc tcagccccca 240
actctgcctg atgcccttta tcttgggcct cttgtctgga ggtgtgacca ccactccatg 300

```
gtctttggcc cagccccagg gatcctgctc tctggagggg gtagagatca aaggcggctc    360
cttccgactt ctccaagagg gccaggcact ggagtacgtg tgtccttctg gcttctaccc    420
gtaccctgtg cagacacgta cctgcagatc tacggggtcc tggagcaccc tgaagactca    480
agaccaaaag actgtcagga aggcagagtg cagagcaatc cactgtccaa gaccacacga    540
cttcgagaac ggggaatact ggccccggtc tccctactac aatgtgagtg atgagatctc    600
tttccactgc tatgacggtt acactctccg gggctctgcc aatcgcacct gccaagtgaa    660
tggccggtgg agtgggcaga cagcgatctg tgacaacgga gcggggtact gctccaaccc    720
gggcatcccc attggcacaa ggaaggtggg cagccagtac cgccttgaag acagcgtcac    780
ctaccactgc agccgggggc ttaccctgcg tggctcccag cggcgaacgt gtcaggaagg    840
tggctcttgg agcgggacgg agccttcctg ccaagactcc ttcatgtacg acacccctca    900
agaggtggcc gaagctttcc tgtcttccct gacagagacc atagaaggag tcgatgctga    960
ggatgggcac ggcccagggg aacaacagaa gcggaagatc gtcctggacc cttcaggctc   1020
catgaacatc tacctggtgc tagatggatc agacagcatt ggggccagca acttcacagg   1080
agccaaaaag tgtctagtca acttaattga gaaggtggca agttatggtg tgaagccaag   1140
atatggtcta gtgacatatg ccacataccc caaaatttgg gtcaaagtgt ctgaagcaga   1200
cagcagtaat gcagactggg tcacgaagca gctcaatgaa atcaattatg aagaccacaa   1260
gttgaagtca gggactaaca ccaagaaggc cctccaggca gtgtacagca tgatgagctg   1320
gccagatgac gtccctcctg aaggctggaa ccgcacccgc catgtcatca tcctcatgac   1380
tgatggattg cacaacatgg gcggggaccc aattactgtc attgatgaga tccgggactt   1440
gctatacatt ggcaaggatc gcaaaaaccc aagggaggat tatctggatg tctatgtgtt   1500
tggggtcggg cctttggtga accaagtgaa catcaatgct ttggcttcca agaaagacaa   1560
tgagcaacat gtgttcaaag tcaaggatat ggaaaacctg gaagatgttt tctaccaaat   1620
gatcgatgaa agccagtctc tgagtctctg tggcatggtt tgggaacaca ggaagggtac   1680
cgattaccac aagcaaccat ggcaggccaa gatctcagtc attcgccctt caaagggaca   1740
cgagagctgt atggggggctg tggtgtctga gtactttgtg ctgacagcag cacattgttt   1800
cactgtggat gacaaggaac actcaatcaa ggtcagcgta ggagggggaga agcgggacct   1860
ggagatagaa gtagtcctat ttcaccccaa ctacaacatt aatgggaaaa aagaagcagg   1920
aattcctgaa ttttatgact atgacgttgc cctgatcaag ctcaagaata agctgaaata   1980
tggccagact atcaggccca tttgtctccc ctgcaccgag ggaacaactc gagctttgag   2040
gcttcctcca actaccactt gccagcaaca aaaggaagag ctgctccctg cacaggatat   2100
caaagctctg tttgtgtctg aggaggagaa aaagctgact cggaaggagg tctacatcaa   2160
gaatggggat aagaaaggca gctgtgagag agatgctcaa tatgccccag gctatgacaa   2220
agtcaaggac atctcagagg tggtcacccc tcggttcctt tgtactggag gagtgagtcc   2280
ctatgctgac cccaatactt gcagaggtga ttctggcggc cccttgatag ttcacaagag   2340
aagtcgtttc attcaagttg gtgtaatcag ctggggagta gtggatgtct gcaaaaacca   2400
gaagcggcaa aagcaggtac ctgctcacgc ccgagacttt cacatcaacc tctttcaagt   2460
gctgccctgg ctgaaggaga aactccaaga tgaggatttg ggttttctat aaggggtttc   2520
ctgctggaca ggggcgtggg attgaattaa aacagctgcg acaac                   2565
```

<210> 155
<211> 5062
<212> DNA
<213> Homo sapiens
<400> 155

```
cactaacgct cttcctagtc cccgggccaa ctcggacagt ttgctcattt attgcaacgg     60
tcaaggctgg cttgtgccag aacggcgcgc gcgcgacgca cgcacacaca cgggggggaaa    120
ctttttttaaa aatgaaaggc tagaagagct cagcggcggc gcgggccgtg cgcgagggct    180
ccggagctga ctcgccgagg caggaaatcc ctccggtcgc gacgcccggc cccgctcggc    240
gcccgcgtgg gatggtgcag cgctcgccgc cgggcccgag agctgctgca ctgaaggccg    300
gcgacgatgg cagcgcgccc gctgcccgtg tcccccgccc gcgccctcct gctcgccctg    360
gccggtgctc tgctcgcgcc ctgcgaggcc cgaggggtga gcttatggaa ccaaggaaga    420
gctgatgaag ttgtcagtgc ctctgttcgg agtggggacc tctggatccc agtgaagagc    480
ttcgactcca agaatcatcc agaagtgctg aatattcgac tacaacggga aagcaaagaa    540
ctgatcataa atctggaaag aaatgaaggt ctcattgcca gcagtttcac ggaaacccac    600
tatctgcaag acggtactga tgtctccctc gctcgaaatt acacggtaat tctgggtcac    660
tgttactacc atggacatgt acggggatat tctgattcag cagtcagtct cagcacgtgt    720
tctggtctca ggggacttat tgtgtttgaa aatgaaagct atgtcttaga accaatgaaa    780
agtgcaacca acagatacaa actcttccca gcgaagaagc tgaaaagcgt ccggggatca    840
tgtggatcac atcacaacac accaaacctc gctgcaaaga atgtgtttcc accaccctct    900
cagacatggg caagaaggca taaaagagag accctcaagg caactaagta tgtggagctg    960
gtgatcgtgg cagacaaccg agagtttcag aggcaaggaa aagatctgga aaaagttaag   1020
cagcgattaa tagagattgc taatcacgtt gacaagtttt acagaccact gaacattcgg   1080
atcgtgttgg taggcgtgga agtgtggaat gacatggaca aatgctctgt aagtcaggac   1140
ccattcacca gcctccatga atttctggac tggaggaaga tgaagcttct acctcgcaaa   1200
tcccatgaca atgcgcagct tgtcagtggg gtttatttcc aagggaccac catcggcatg   1260
gccccaatca tgagcatgtg cacggcagac cagtctgggg gaattgtcat ggaccattca   1320
gacaatcccc ttggtgcagc cgtgaccctg gcacatgagc tgggccacaa tttcgggatg   1380
aatcatgaca cactggacag ggctgtagc tgtcaaatgg cggttgagaa aggaggctgc   1440
atcatgaacg cttccaccgg gtacccattt cccatggtgt tcagcagttg cagcaggaag   1500
```

gacttggaga ccagcctgga gaaaggaatg ggggtgtgcc tgtttaacct gccggaagtc 1560
agggagtctt tcgggggcca gaagtgtggg aacagatttg tggaagaagg agaggagtgt 1620
gactgtgggg agccagagga atgtatgaat cgctgctgca atgccaccac ctgtaccctg 1680
aagccggacg ctgtgtgcgc acatgggctg tgctgtgaag actgccagct gaagcctgca 1740
ggaacagcgt gcagggactc cagcaactcc tgtgacctcc cagagttctg cacaggggcc 1800
agccctcact gcccagccaa cgtgtacctg cacgatgggc actcatgtca ggatgtggac 1860
ggctactgct acaatggcat ctgccagact cacgagcagc agtgtgtcac actctgggga 1920
ccaggtgcta aacctgcccc tgggatctgc tttgagagag tcaattctgc aggtgatcct 1980
tatggcaact gtggcaaagt ctcgaagagt tcctttgcca aatgcgagat gagagatgct 2040
aaatgtggaa aaatccagtg tcaaggaggt gccagccggc cagtcattgg taccaatgcc 2100
gtttccatag aaacaaacat ccccctgcag caaggaggcc ggattctgtg ccggggggacc 2160
cacgtgtact tgggcgatga catgccggac ccagggcttg tgcttgcagg cacaaagtgt 2220
gcagatggaa aaatctgcct gaatcgtcaa tgtcaaaata ttagtgtctt tggggttcac 2280
gagtgtgcaa tgcagtgcca cggcagaggg gtgtgcaaca acaggaagaa ctgccactgc 2340
gaggcccact gggcacctcc cttctgtgac aagtttggct ttggaggaag cacagacagc 2400
ggccccatcc ggcaagcaga taaccaaggt ttaaccatag gaattctggt gaccatcctg 2460
tgtcttcttg ctgccggatt tgtggtttat ctcaaaagga agaccttgat acgactgctg 2520
tttacaaata agaagaccac cattgaaaaa ctaaggtgtg tgcgcccttc ccggccaccc 2580
cgtggcttcc aaccctgtca ggctcacctc ggccaccttg gaaaaggcct gatgaggaag 2640
ccgccagatt cctacccacc gaaggacaat cccaggagat tgctgcagtg tcagaatgtt 2700
gacatcagca gacccctcaa cggcctgaat gtccctcagc cccagtcaac tcagcgagtg 2760
cttcctcccc tccaccgggc cccacgtgca cctagcgtcc ctgccagacc cctgccagcc 2820
aagcctgcac ttaggcaggc ccaggggacc tgtaagccaa acccccctca gaagcctctg 2880
cctgcagatc ctctggccag aacaactcgg ctcactcatg ccttggccag gaccccagga 2940
caatgggaga ctgggctccg cctggcaccc ctcagacctg ctccacaata tccacaccaa 3000
gtgcccagat ccacccacac cgcctatatt aagtgagaag ccgacacctt ttttcaacag 3060
tgaagacaga agtttgcact atctttcagc tccagttgga gtttttttgta ccaactttta 3120
ggattttttt taatgtttaa aacatcatta ctataagaac tttgagctac tgccgtcagt 3180
gctgtgctgt gctatggtgc tctgtctact tgcacaggta cttgtaaatt attaatttat 3240
gcagaatgtt gattacagtg cagtgcgctg tagtaggcat ttttaccatc actgagtttt 3300
ccatggcagg aaggcttgtt gtgcttttag tattttagtg aacttgaaat atcctgcttg 3360
atgggattct ggacaggatg tgtttgcttt ctgatcaagg ccttattgga aagcagtccc 3420
ccaactaccc ccagctgtgc ttatggtacc agatgcagct caagagatcc caagtagaat 3480
ctcagttgat ttctggatt ccccatctca ggccagagcc aaggggcttc aggtccaggc 3540
tgtgtttggc tttcagggag gccctgtgcc ccttgacaac tggcaggcag gctcccaggg 3600
acacctggga gaaatctggc ttctggccag gaagctttgg tgagaacctg ggttgcagac 3660
aggaatctta aggtgtagcc acaccaggat agagactgga acactagaca agccagaact 3720
tgaccctgag ctgaccagcc gtgagcatgt ttggaagggg tctgtagtgt cactcaaggc 3780
ggtgcttgat agaaatgcca agcacttctt tttctcgctg tcctttctag agcactgcca 3840
ccagtaggtt atttagcttg ggaaaggtgg tgtttctgta agaaacctac tgcccaggca 3900
ctgcaaaccg ccacctccct atactgcttg gagctgagca aatcaccaca aactgtaata 3960
caatgatcct gtattcagac agatgaggac tttccatggg accacaacta ttttcagatg 4020
tgaaccatta accagatcta gtcaatcaag tctgtttact gcaaggttca acttattaac 4080
aattaggcag actctttatg cttgcaaaaa ctacaaccaa tggaatgtga tgttcatggg 4140
tatagttcat gtctgctatc attattcgta gatattggac aaagaacctt ctctatgggg 4200
catcctcttt ttccaacttg gctgcaggaa tctttaaaag atgcttttaa cagagtctga 4260
acctatttct taaacacttg caacctacct gttgagcatc acagaatgtg ataaggaaat 4320
caacttgctt atcaacttcc taaatattat gagatgtggc ttgggcagca tccccttgaa 4380
ctcttcactc ttcaaatgcc tgactaggga gccatgtttc acaaggtctt taaagtgact 4440
aatggcatga gaaatacaaa aatactcaga taaggtaaaa tgccatgatg cctctgtctt 4500
ctggactggt tttcacatta gaagacaatt gacaacagtt acataattca ctctgagtgt 4560
tttatgagaa agccttcttt tggggtcaac agttttccta tgctttgaaa cagaaaaata 4620
tgtaccaaga atcttggttt gccttccaga aaacaaaact gcatttcact ttcccggtgt 4680
tccccactgt atctaggcaa catagtattc atgactatgg ataaactaaa cacgtgacac 4740
aaacacacac aaaagggaac ccagctctaa tacattccaa ctcgtatagc atgcatctgt 4800
ttattctata gttattaagt tctttaaaat gtaaagccat gctggaaaat aatactgctg 4860
agatacatac agaattactg taactgatta cacttggtaa ttgtactaaa gccaaacata 4920
tatatactat taaaaaggtt tacagaattt tatggtgcat tacgtgggca ttgtcttttt 4980
agatgcccaa atccttagat ctggcatgtt agcccttcct ccaattataa gaggatatga 5040
accaaaaaaa aaaaaaaaaa aa 5062

<210> 156
<211> 2328
<212> DNA
<213> Homo sapiens
<400> 156
gccagccgag cggccagcca gtgcggggct ggccatgtaa ggcccacagg cggtcctgcc 60
cgcccggtgc cctgcggaga gcctcgtgca gccctgggca ccgcccctgc cctgccctga 120
cccctggcc ttgaaatgct gtcatcggag gagccgtccc gctcgggaca aggccagcat 180

ggacaaagct agagctgggg caagcaagga gccttcctgt cctcgaggcc gtgggaagag 240
aagcacgccc aggggggccac tcctgagagc ctctctgtcc accaggcctc tgcagagggg 300
tcaccatggc tctggcccga ggcagccggc agctgggggc cctggtgtgg ggcgcctgcc 360
tgtgcgtgct ggtgcacggg cagcaggcgc agcccgggca gggctcggac cccgcccgct 420
ggcggcagct gatccagtgg gagaacaacg ggcaggtgta cagcttgctc aactcgggct 480
cagagtacgt gccggccgga cctcagcgct ccgagagtag ctcccgggtg ctgctggccg 540
gcgcgcccca ggcccagcag cggcgcagcc acgggagccc ccggcgtcgg caggcgccgt 600
ccctgcccct gccggggcgc gtgggctcgg acaccgtgcg cggccaggcg cggcacccat 660
tcggctttgg ccaggtgccc gacaactggc gcgaggtggc cgtcggggac agcacgggca 720
tggccctggc ccgcacctcc gtctcccagc aacggcacgg gggctccgcc tcctcggtct 780
cggcttcggc cttcgccagc acctaccgcc agcagccctc ctacccgcag cagttcccct 840
acccgcaggc gcccttcgtc agccagtacg agaactacga ccccgcgtcg cggacctacg 900
accagggttt cgtgtactac cggcccgcgg gcggcggcgt gggcgcgggg gcggcggccg 960
tggcctcggc gggggtcatc taccccctacc agccccgggc gcgctacgag gagtacggcg 1020
gcggcgaaga gctgcccgag tacccgcctc agggcttcta cccggccccc gagaggccct 1080
acgtgccgcc gccgccgccg ccccccgacg gcctggaccg ccgctactcg cacagtctgt 1140
acagcgaggg cacccccggc ttcgagcagg cctaccctga ccccggtccc gaggcggcgc 1200
aggcccatgg cggagaccca cgcctgggct ggtacccgcc ctacgccaac ccgccgcccg 1260
aggcgtacgg gccgccgcgc gcgctggagc cgccctacct gccggtgcgc agctccgaca 1320
cgcccccgcc gggtggggag cggaacggcg cgcagcaggg ccgcctcagc gtaggcagcg 1380
tgtaccggcc caaccagaac ggccgcggtc tccctgactt ggtcccagac cccaactatg 1440
tgcaagcatc cacttatgtg cagagagccc acctgtactc cctgcgctgt gctgcggagg 1500
agaagtgtct ggccagcaca gcctatgccc ctgaggccac cgactacgat gtgcgggtgc 1560
tactgcgctt cccccagcgc gtgaagaacc agggcacagc agacttcctc cccaaccggc 1620
cacggcacac ctgggagtgg cacagctgcc accagcatta ccacagcatg gacgagttca 1680
gccactacga cctactggat gcagccacag gcaagaaggt ggccgagggc cacaaggcca 1740
gtttctgcct ggaggacagc acctgtgact tcggcaacct caagcgctat gcatgcacct 1800
ctcataccca gggcctgagc ccaggctgct atgacaccta caatgcggac atcgactgcc 1860
agtggatcga cataaccgac gtgcagcctg ggaactacat cctcaaggtg cacgtgaacc 1920
caaagtatat tgttttggag tctgacttca ccaacaacgt ggtgagatgc aacattcact 1980
acacaggtcg ctacgtttct gcaacaaact gcaaaattgt ccaatcctga tctccgggag 2040
ggacagatgg ccaatctctc cccttccaaa gcaggccctg ctccccgggc agcctcccgc 2100
cgaggggccc agcccccaac ccacaggcag ggaggggcat ccctccctgc cggcctcagg 2160
gagcgaacgt ggatgaaaac cacagggatt ccggatgcca gaccccattt tatacttcac 2220
ttttctctac agtgttgttt tgttgttgtt ggtttttatt ttttatactt tggccatacc 2280
acagagctag attgcccagg tctgggctga ataaaacaag gtttttct 2328

<210> 157
<211> 2249
<212> DNA
<213> Homo sapiens
<400> 157

ctcctctaca aagaggtgga cagagaagac agcagagacc atgggacccc cctcagcccc 60
tccctgcaga ttgcatgtcc cctggaagga ggtcctgctc acagcctcac ttctaacctt 120
ctggaaccca cccaccactg ccaagctcac tattgaatcc acgccattca atgtcgcaga 180
ggggaaggag gttcttctac tcgcccacaa cctgccccag aatcgtattg gttacagctg 240
gtacaaaggc gaaagagtgg atggcaacag tctaattgta ggatatgtaa taggaactca 300
acaagctacc ccagggcccg catacagtgg tcgagagaca atataccccca atgcatccct 360
gctgatccag aacgtcaccc agaatgacac aggattctat accctacaag tcataaagtc 420
agatcttgtg aatgaagaag caaccggaca gttccatgta tacccggagc tgcccaagcc 480
ctccatctcc agcaacaact ccaaccccgt ggaggacaag gatgctgtgg ccttcacctg 540
tgaacctgag gttcagaaca caacctacct gtggtgggta aatggtcaga gcctcccggt 600
cagtcccagg ctgcagctgt ccaatggcaa catgaccctc actctactca gcgtcaaaag 660
gaacgatgca ggatcctatg aatgtgaaat acagaaccca gcgagtgcca accgcagtga 720
cccagtcacc ctgaatgtcc tctatggccc agatgtcccc accatttccc cctcaaaggc 780
caattaccgt ccaggggaaa atctgaacct ctcctgccac gcagcctcta acccacctgc 840
acagtactct tggtttatca atgggacgtt ccagcaatcc acacaagagc tctttatccc 900
caacatcact gtgaataata gcggatccta tatgtgccaa gcccataact cagccactgg 960
cctcaatagg accacagtca cgatgatcac agtctctgga agtgctcctg tcctctcagc 1020
tgtggccacc gtcggcatca cgattggagt gctggccagg gtggctctga tatagcagcc 1080
ctggtgtatt ttcgatattt caggaagact ggcagattgg accagaccct gaattcttct 1140
agctcctcca atcccatttt atcccatgga accactaaaa acaaggtctg ctctgctcct 1200
gaagccctat atgctggaga tggacaactc aatgaaaatt taaagggaaa accctcaggc 1260
ctgaggtgtg tgccactcag agacttcacc taactagaga cagtcaaact gcaaaccatg 1320
gtgagaaatt gacgacttca cactatggac agcttttccc aagatgtcaa aacaagactc 1380
ctcatcatga taaggctctt accccctttt aatttgtcct tgcttatgcc tgcctctttc 1440
gcttggcagg atgatgctgt cattagtatt tcacaagaag tagcttcaga gggtaactta 1500
acagagtgtc agatctatct tgtcaatccc aacgttttac ataaaataag agatccttta 1560
gtgcacccag tgactgacat tagcagcatc tttaacacag ccgtgtgttc aaatgtacag 1620

tggtcctttt cagagttgga cttctagact cacctgttct cactccctgt tttaattcaa 1680
cccagccatg caatgccaaa taatagaatt gctccctacc agctgaacag ggaggagtct 1740
gtgcagtttc tgacacttgt tgttgaacat ggctaaatac aatgggtatc gctgagacta 1800
agttgtagaa attaacaaat gtgctgcttg gttaaaatgg ctacactcat ctgactcatt 1860
ctttattcta ttttagttgg tttgtatctt gcctaaggtg cgtagtccaa ctcttggtat 1920
taccctccta atagtcatac tagtagtcat actccctggt gtagtgtatt ctctaaaagc 1980
tttaaatgtc tgcatgcagc cagccatcaa atagtgaatg gtctctcttt ggctggaatt 2040
acaaaactca gagaaatgtg tcatcaggag aacatcataa cccatgaagg ataaaagccc 2100
caaatggtgg taactgataa tagcactaat gctttaagat ttggtcacac tctcacctag 2160
gtgagcgcat tgagccagtg gtgctaaatg ctacatactc caactgaaat gttaaggaag 2220
aagatagatc caaaaaaaaa aaaaaaaaa 2249

<210> 158
<211> 2260
<212> DNA
<213> Homo sapiens
<400> 158
aagcccagca gccccggggc ggatggctcc ggccgcctgg ctccgcagcg cggccgcgcg 60
cgccctcctg cccccgatgc tgctgctgct gctccagccg ccgccgctgc tggcccgggc 120
tctgccgccg gacgtccacc acctccatgc cgagaggagg gggccacagc cctggcatgc 180
agccctgccc agtagcccgg cacctgcccc tgccacgcag gaagcccccc ggcctgccag 240
cagcctcagg cctccccgct gtggcgtgcc cgacccatct gatgggctga gtgcccgcaa 300
ccgacagaag aggttcgtgc tttctggcgg gcgctgggag aagacggacc tcacctacag 360
gatccttcgg ttcccatggc agttggtgca ggagcaggtg cggcagacga tggcagaggc 420
cctaaaggta tggagcgatg tgacgccact caccttact gaggtgcacg agggccgtgc 480
tgacatcatg atcgacttcg ccaggtactg gcatggggac gacctgccgt ttgatgggcc 540
tggggggcatc ctggcccatg ccttcttccc caagactcac cgagaagggg atgtccactt 600
cgactatgat gagacctgga ctatcgggga tgaccagggc acagacctgc tgcaggtggc 660
agcccatgaa tttggccacg tgctggggct gcagcacaca acagcagcca aggccctgat 720
gtccgccttc tacacctttc gctacccact gagtctcagc ccagatgact gcaggggcgt 780
tcaacaccta tatggccagc cctggcccac tgtcacctcc aggaccccag ccctgggccc 840
ccaggctggg atagacacca atgagattgc accgctggag ccagacgccc cgccagatgc 900
ctgtgaggcc tcctttgacg cggtctccac catccgaggc gagctctttt tcttcaaagc 960
gggctttgtg tggcgcctcc gtgggggcca gctgcagccc ggctacccag cattggcctc 1020
tcgccactgg cagggactgc ccagccctgt ggacgctgcc ttcgaggatg cccagggcca 1080
catttggttc ttccaaggtg ctcagtactg ggtgtacgac ggtgaaaagc cagtcctggg 1140
ccccgcaccc ctcaccgagc tgggcctggt gaggttcccg gtccatgctg ccttggtctg 1200
gggtcccgag aagaacaaga tctacttctt ccgaggcagg gactactggc gtttccaccc 1260
cagcacccgg cgtgtagaca gtcccgtgcc ccgcagggcc actgactgga gaggggtgcc 1320
ctctgagatc gacgctgcct tccaggatgc tgatggctat gcctacttcc tgcgcggccg 1380
cctctactgg aagtttgacc ctgtgaaggt gaaggctctg gaaggcttcc cccgtctcgt 1440
gggtcctgac ttctttggct gtgccgagcc tgccaacact ttcctctgac catggcttgg 1500
atgccctcag ggtgctgac ccctgccagg ccacgaatat caggctagag acccatggcc 1560
atctttgtgg ctgtgggcac caggcatggg actgagccca tgtctcctgc aggggggatgg 1620
ggtggggtac aaccaccatg acaactgccg ggagggccac gcaggtcgtg gtcacctgcc 1680
agcgactgtc tcagactggg cagggaggct ttggcatgac ttaagaggaa gggcagtctt 1740
gggacccgct atgcaggtcc tggcaaacct ggctgccctg tctcatccct gtccctcagg 1800
gtagcaccat ggcaggactg gggaactgg agtgtccttg ctgtatccct gttgtgaggt 1860
tccttccagg ggctggcact gaagcaaggg tgctggggcc ccatggcctt cagccctggc 1920
tgagcaactg ggctgtaggg cagggccact tcctgaggtc aggtcttggt aggtgcctgc 1980
atctgtctgc cttctggctg acaatcctgg aaatctgttc tccagaatcc aggccaaaaa 2040
gttcacagtc aaatggggag gggtattctt catgcaggag accccaggcc ctggaggctg 2100
caacatacct caatcctgtc ccaggccgga tcctcctgaa gccctttcg cagcactgct 2160
atcctccaaa gccattgtaa atgtgtgtac agtgtgtata aaccttcttc ttcttttttt 2220
tttttaaact gaggattgtc attaaacaca gttgttttct 2260

<210> 159
<211> 1973
<212> DNA
<213> Homo sapiens
<400> 159
gggatattgg agtagcaaga ggctgggaag ccatcactta ccttgcactg agaaagaaga 60
caaaggccag tatgcacagc tttcctccac tgctgctgct gctgttctgg ggtgtggtgt 120
ctcacagctt cccagcgact ctagaaacac aagagcaaga tgtggactta gtccagaaat 180
acctggaaaa atactacaac ctgaagaatg atgggaggca agttgaaaag cggagaaata 240
gtggcccagt ggttgaaaaa ttgaagcaaa tgcaggaatt ctttgggctg aaagtgactg 300
ggaaaccaga tgctgaaacc ctgaaggtga tgaagcagcc cagatgtgga gtgcctgatg 360
tggctcagtt tgtcctcact gaggggaacc ctcgctggga gcaaacacat ctgacctaca 420
ggattgaaaa ttacacgcca gatttgccaa gagcagatgt ggaccatgcc attgagaaag 480

```
ccttccaact ctggagtaat gtcacacctc tgacattcac caaggtctct gagggtcaag     540
cagacatcat gatatctttt gtcaggggag atcatcggga caactctcct tttgatggac     600
ctggaggaaa tcttgctcat gcttttcaac caggcccagg tattggaggg gatgctcatt     660
ttgatgaaga tgaaaggtgg accaacaatt tcagagagta caacttacat cgtgttgcgg     720
ctcatgaact cggccattct cttggactct cccattctac tgatatcggg gctttgatgt     780
accctagcta caccttcagt ggtgatgttc agctagctca ggatgacatt gatggcatcc     840
aagccatata tggacgttcc caaaatcctg tccagcccat cggcccacaa accccaaaag     900
cgtgtgacag taagctaacc tttgatgcta taactacgat tcggggagaa gtgatgttct     960
ttaaagacag attctacatg cgcacaaatc ccttctaccc ggaagttgag ctcaatttca    1020
tttctgtttt ctggccacaa ctgccaaatg ggcttgaagc tgcttacgaa tttgccgaca    1080
gagatgaagt ccggtttttc aaagggaata agtactgggc tgttcaggga cagaatgtgc    1140
tacacggata ccccaaggac atctacagct cctttggctt ccctagaact gtgaagcata    1200
tcgatgctgc tctttctgag gaaaacactg gaaaaaccta cttctttgtt gctaacaaat    1260
actggaggta tgatgaatat aaacgatcta tggatccagg ttatcccaaa atgatagcac    1320
atgactttcc tggaattggc cacaaagttg atgcagtttt catgaaagat ggatttttct    1380
atttctttca tggaacaaga caatacaaat ttgatcctaa aacgaagaga attttgactc    1440
tccagaaagc taatagctgg ttcaactgca ggaaaaattg aacattacta atttgaatgg    1500
aaaacacatg gtgtgagtcc aaagaaggtg ttttcctgaa gaactgtcta ttttctcagt    1560
catttttaac ctctagagtc actgatacac agaatataat cttatttata cctcagtttg    1620
catatttttt tactatttag aatgtagccc tttttgtact gatataattt agttccacaa    1680
atggtgggta caaaaagtca agtttgtggc ttatggattc atataggcca gagttgcaaa    1740
gatcttttcc agagtatgca actctgacgt tgatcccaga gagcagcttc agtgacaaac    1800
atatcctttc aagacagaaa gagacaggag acatgagtct ttgccggagg aaaagcagct    1860
caagaacaca tgtgcagtca ctggtgtcac cctggatagg caagggataa ctcttctaac    1920
acaaaataag tgttttatgt ttggaataaa gtcaaccttg tttctactgt ttt           1973
```

<210> 160
<211> 2722
<212> DNA
<213> Homo sapiens
<400> 160

```
caacagtccc caggcatcac cattcaagat gcatccaggg gtcctggctg ccttcctctt      60
cttgagctgg actcattgtc gggccctgcc ccttcccagt ggtggtgatg aagatgattt     120
gtctgaggaa gacctccagt ttgcagagcg ctacctgaga tcatactacc atcctacaaa     180
tctcgcggga atcctgaagg agaatgcagc aagctccatg actgagaggc tccgagaaat     240
gcagtctttc ttcggcttag aggtgactgg caaacttgac gataacacct tagatgtcat     300
gaaaaagcca agatgcgggg ttcctgatgt gggtgaatac aatgttttcc ctcgaactct     360
taaatggtcc aaaatgaatt taacctacag aattgtgaat tacacccctg atatgactca     420
ttctgaagtc gaaaaggcat tcaaaaaagc cttcaaagtt tggtccgatg taactcctct     480
gaattttacc agacttcacg atggcattgc tgacatcatg atctcttttg gaattaagga     540
gcatggcgac ttctacccat ttgatgggcc ctctggcctg ctggctcatg cttttcctcc     600
tgggccaaat tatggaggag atgcccattt tgatgatgat gaaacctgga caagtagttc     660
caaaggctac aacttgtttc ttgttgctgc gcatgagttc ggccactcct taggtcttga     720
ccactccaag gaccctggag cactcatgtt tcctatctac acctacaccg gcaaaagcca     780
ctttatgctt cctgatgacg atgtacaagg gatccagtct ctctatggtc caggagatga     840
agaccccaac cctaaacatc caaaaacgcc agacaaatgt gacccttcct tatcccttga     900
tgccattacc agtctccgag gagaaacaat gatctttaaa gacagattct tctggcgcct     960
gcatcctcag caggttgatg cggagctgtt tttaacgaaa tcattttggc cagaacttcc    1020
caaccgtatt gatgctgcat atgagcaccc ttctcatgac ctcatcttca tcttcagagg    1080
tagaaaattt tgggctctta atggttatga cattctggaa ggttatccca aaaaaatatc    1140
tgaactgggt cttccaaaag aagttaagaa gataagtgca gctgttcact ttgaggatac    1200
aggcaagact ctcctgttct caggaaacca ggtctggaga tatgatgata ctaaccatat    1260
tatggataaa gactatccga gactaataga agaagacttc ccaggaattg gtgataaagt    1320
agatgctgtc tatgagaaaa atggttatat ctattttttc aacggaccca tacagtttga    1380
atacagcatc tggagtaacc gtattgttcg cgtcatgcca gcaaattcca ttttgtggtg    1440
ttaagtgtct ttttaaaaat tgttatttaa atcctgaaga gcatttgggg taatacttcc    1500
agaagtgcgg ggtaggggaa gaagagctat caggagaaag cttggttctg tgaacaagct    1560
tcagtaagtt atctttgaat atgtagtatc tatatgacta tgcgtggctg gaaccacatt    1620
gaagaatgtt agagtaatga aatggaggat ctctaaagag catctgattc ttgttgctgt    1680
acaaaagcaa tggttgatga tacttcccac accacaaatg ggacacatgg tctgtcaatg    1740
agagcataat ttaaaaatat atttataagg aaattttaca agggcataaa gtaaatacat    1800
gcatataatg aataaatcat tcttactaaa aagtataaaa tagtatgaaa atggaaattt    1860
gggagagcca tacataaaag aaataaacca aaggaaaatg tctgtaataa tagactgtaa    1920
cttccaaata aataattttc attttgcact gaggatattc agatgtatgt gcccttcttc    1980
acacagacac taacgaaata tcaaagtcat taaagacagg agacaaaaga gcagtggtaa    2040
gaatagtaga tgtggccttt gaattctgtt taattttcac ttttggcaat gactcaaagt    2100
ctgctctcat ataagacaaa tattcctttg catattataa aggataaaga aggatgatgt    2160
ctttttatta aaatatttca ggttcttcag aagtcacaca ttacaaagtt aaaattgtta    2220
tcaaaatagt ctaaggccat ggcatccctt tttcataaat tatttgatta tttaagacta    2280
```

aaagttgcat tttaacccta ttttacctag ctaattattt aattgtccgg tttgtcttgg 2340
atatataggc tattttctaa agacttgtat agcatgaaat aaaatatatc ttataaagtg 2400
gaagtatgta tattaaaaaa gagacatcca aatttttttt taaagcagtc tactagattg 2460
tgatcccttg agatatggaa ggatgccttt ttttctctgc atttaaaaaa atcccccagc 2520
acttcccaca gtgcctattg atacttgggg agggtgcttg gcacttattg aatatatgat 2580
cggccatcaa gggaagaact attgtgctca gagacactgt tgataaaaac tcaggcaaag 2640
aaaatgaaat gcatatttgc aaagtgtatt aggaagtgtt tatgttgttt ataataaaaa 2700
tatattttca acagaaaaaa aa 2722

<210> 161
<211> 2208
<212> DNA
<213> Homo sapiens
<400> 161
tctttggctt tttttggcgg agctggggcg ccctccggaa gcgtttccaa ctttccagaa 60
gtttctcggg acgggcagga gggggtgggg actgccatat atagatcccg ggagcagggg 120
agcgggctaa gagtagaatc gtgtcgcggc tcgagagcga gagtcacgtc ccggcgctag 180
cccagcccga cccaggccca ccgtggtgca cgcaaaccac ttcctggcca tgcgctccct 240
cctgcttctc agcgccttct gcctcctgga ggcggccctg gccgccgagg tgaagaaacc 300
tgcagccgca gcagctcctg gcactgcgga gaagttgagc cccaaggcgg ccacgcttgc 360
cgagcgcagc gccggcctgg ccttcagctt gtaccaggcc atggccaagg accaggcagt 420
ggagaacatc ctggtgtcac ccgtggtggt ggcctcgtcg ctagggctcg tgtcgctggg 480
cggcaaggcg accacggcgt cgcaggccaa ggcagtgctg agcgccgagc agctgcgcga 540
cgaggaggtg cacgccggcc tgggcgagct gctgcgctca ctcagcaact ccacggcgcg 600
caacgtgacc tggaagctgg gcagccgact gtacggaccc agctcagtga gcttcgctga 660
tgacttcgtg cgcagcagca agcagcacta caactgcgag cactccaaga tcaacttccg 720
cgacaagcgc agcgcgctgc agtccatcaa cgagtgggcc gcgcagacca ccgacggcaa 780
gctgcccgag gtcaccaagg acgtggagcg cacggacggc gccctgctag tcaacgccat 840
gttcttcaag ccacactggg atgagaaatt ccaccacaag atggtggaca accgtggctt 900
catggtgact cggtcctata ccgtgggtgt catgatgatg caccggacag gcctctacaa 960
ctactacgac gacgagaagg aaaagctgca aatcgtggag atgcccctgg cccacaagct 1020
ctccagcctc atcatcctca tgccccatca cgtggagcct ctcgagcgcc ttgaaaagct 1080
gctaaccaaa gagcagctga agatctggat ggggaagatg cagaagaagg ctgttgccat 1140
ctccttgccc aagggtgtgg tggaggtgac ccatgacctg cagaaacacc tggctgggct 1200
gggcctgact gaggccattg acaagaacaa ggccgacttg tcacgcatgt caggcaagaa 1260
ggacctgtac ctggccagcg tgttccacgc caccgccttt gagttggaca cagatggcaa 1320
cccctttgac caggacatct acgggcgcga ggagctgcgc agccccaagc tgttctacgc 1380
cgaccacccc ttcatcttcc tagtgcggga cacccaaagc ggctccctgc tattcattgg 1440
gcgcctggtc cggcctaagg gtgacaagat gcgagacgag ttatagggcc tcagggtgca 1500
cacaggatgg caggaggcat ccaaaggctc ctgagacaca tgggtgctat tggggttggg 1560
ggggaggtga ggtaccagcc ttggatactc catggggtgg gggtggaaaa acagaccggg 1620
gttcccgtgt gcctgagcgg accttcccag ctagaattca ctccacttgg acatgggccc 1680
cagataccat gatgctgagc ccggaaactc cacatcctgt gggacctggg ccatagtcat 1740
tctgcctgcc ctgaaagtcc cagatcaagc ctgcctcaat cagtattcat atttatagcc 1800
aggtaccttc tcacctgtga gaccaaattg agctaggggg gtcagccagc cctcttctga 1860
cactaaaaca cctcagctgc ctccccagct ctatcccaac ctctcccaac tataaaacta 1920
ggtgctgcag cccctgggac caggcacccc cagaatgacc tggccgcagt gaggcggatt 1980
gagaaggagc tcccaggagg ggcttctggg cagactctgg tcaagaagca tcgtgtctgg 2040
cgttgtgggg atgaactttt tgttttgttt cttccttttt tagttcttca aagataggga 2100
gggaaggggg aacatgagcc tttgttgcta tcaatccaag aacttatttg tacatttttt 2160
ttttcaataa aactttttcca atgacatttt gttggagcgt ggaaaaaa 2208

<210> 162
<211> 2373
<212> DNA
<213> Homo sapiens
<400> 162
cccaggccca ccccacccag cacccctggc gcagggactg ctggaacctg gctgtgcgcg 60
ctgtcgcttt aagacagact ctgccggcgc cgtccggagc cttagaaacc ggccccggat 120
cgcgagccgg agccggagcc ggagccgggg ccggccgggc tgctgaggcc cgagcggcag 180
gagcgcagcg cggagcgctg agccaggcgc ccagtcgcga gaagctgccg ccgcctctgc 240
ccgcccggcg ccgcagcccc gggcggtcca tggggcgggc acggcgtcgc tgcaggcgcc 300
ggcagccctg gagggcagcc gcttaggcgc tgcgctcttg tccccgcagg tcgcagccag 360
ggcggcgggg cgcgcccagc cccggcccct ggagcgcccg ccgcggtccc cacctccatg 420
gacgccttca agggggggcat gagcctggag cggctgccgg aggggctccg gccgccgccg 480
ccgccacccc atgacatggg gcccgccttc cacctggccc ggcccgccga cccccgcgag 540
ccgctcgaga actccgccag cgagtcgtct gacacggagc tgccagagaa ggagcgcggc 600
ggggaaccca aggggcccga ggacagtggt gcgggaggca cggctgcggg cggcgcagac 660
gacccagcca agaagaagaa gcagcggcgg caacgtacgc acttcacaag ccagcagttg 720

caagagctag aggccacgtt ccagaggaac cgctaccccg acatgagcat gagggaggag 780
atcgccgtgt ggaccaacct caccgagccg cgcgtgcggg tctggttcaa gaaccggcga 840
gccaagtggc gtaagcgcga gcgtaaccag cagctggacc tgtgcaaggg tggctacgtg 900
ccgcagttca gcggcctagt gcagccctac gaggacgtgt acgccgccgg ctactcctac 960
aacaactggg ccgccaagag cctggcgcca gcgccgctct ccaccaagag cttcaccttc 1020
ttcaactcca tgagcccgct gtcgtcgcag tccatgttct cagcacccag ctccatctcc 1080
tccatgacca tgccgtccag catgggccca ggcgccgtgc ctggcatgcc caactcgggc 1140
ctcaacaaca tcaacaacct caccggctcc tcgctcaact cggccatgtc gccgggcgct 1200
tgcccgtacg gcactcccgc ctcgccctac agcgtctacc gggacacgtg caactcgagc 1260
ctagccagcc tgcggctcaa gtccaaacag cactcgtcgt ttggctacgg cgccctgcag 1320
ggcccggcct cgggcctcaa cgcgtgccag tacaacagct gaccgccccg ccgcaccacg 1380
cgggccggcg gccggagcgg ggaagggcgc gggcgcggag gacgcacgcg gggccccggc 1440
tcgcaagccc cagctcaccg cgccgcggac ctcacacctg cgcagccccc tcctcccact 1500
tcccactccg ggttggtttt gtgtttgctt ttccggaccc cactctgccc tccaaaaaga 1560
caaaaaaaaa aaaaaaaaaa aaagcaaaaa gacgtcggag aaaagtgccg cgaaaaaatg 1620
gatgagttgc aatttctctc gggatggcgc gggtggtgtg tgtgtgttcc cacgggcccc 1680
ggaggcccac tccgcggagg gcacgcggcg cggtaggcga gcgccgaggc ccagcggccg 1740
ggggaggacg acctcgtatc ccgcgtcccc gccgcgctgg atccggactg agtggccggg 1800
cctgcggact ggatgtgcgg ggcctggact tgcctaggat ttcccgaccc cgtacaaacc 1860
aagttgccct ctccgagcta ggcccggccg agagcgcctt agctcgagtc ggatccgtgt 1920
tggggcgggc gttgggtttg gggggacggt gcccccagcc caggatcggg cactcagtgg 1980
agccgcacac ggccccggcg cgcctggtag agcctcgctg gccccgcgcc ccggagccct 2040
atattaaggc cacggagcga cagcgggcag tgcgggcctg gcgggaggtg ggggaggtcc 2100
atctcagaac accccagcct tgagcttagc tgcaggccca ggccctctgc tctgctcccg 2160
ggctaggagg tggccctctg tctgggcgaa cagccccctc ctcaccgccc gccgtgcaag 2220
agtcgagccg gcagagcaag gggcgcggcc ccagggccct gcgcccactt tgcacacccg 2280
ctctccggcc cgcgccccctg tttacagcgt ccctgtgtat gttggactga ctgtaataaa 2340
tctgtctata tcgactaaaa aaaaaaaaaa aaa 2373

<210> 163
<211> 9645
<212> DNA
<213> Homo sapiens
<400> 163

atgcccaagc gcgcgcactg gggggccctc tccgtggtgc tgatcctgct ttggggccat 60
ccgcgagtgg cgctggcctg cccgcatcct tgtgcctgct acgtccccag cgaggtccac 120
tgcacgttcc gatccctggc ttccgtgccc gctggcattg ctagacacgt ggaaagaatc 180
aatttggggt ttaatagcat acaggccctg tcagaaacct catttgcagg actgaccaag 240
ttggagctac ttatgattca cggcaatgag atcccaagca tccccgatgg agctttaaga 300
gacctcagct ctcttcaggt tttcaagttc agctacaaca agctgagagt gatcacagga 360
cagaccctcc agggtctctc taacttaatg aggctgcaca ttgaccacaa caagatcgag 420
tttatccacc ctcaagcttt caacggctta acgtctctga ggctactcca tttggaagga 480
aatctcctcc accagctgca ccccagcacc ttctccacgt tcacattttt ggattatttc 540
agactctcca ccataaggca cctctactta gcagagaaca tggttagaac tcttcctgcc 600
agcatgcttc ggaacatgcc gcttctggag aatctttact tgcagggaaa tccgtggacc 660
tgcgattgtg agatgagatg gtttttggaa tgggatgcaa aatccagagg aattctgaag 720
tgtaaaaagg acaaagctta tgaaggcggt cagttgtgtg caatgtgctt cagtccaaag 780
aagttgtaca aacatgagat acacaagctg aaggacatga cttgtctgaa gccttcaata 840
gagtcccctc tgagacagaa caggagcagg agtattgagg aggagcaaga acaggaagag 900
gatggtggca gccagctcat cctggagaaa ttccaactgc cccagtggag catctctttg 960
aatatgaccg acgagcacgg gaacatggtg aacttggtct gtgacatcaa gaaaccaatg 1020
gatgtgtaca agattcactt gaaccaaacg gatcctccag atattgacat aaatgcaaca 1080
gttgccttgg actttgagtg tccaatgacc cgagaaaact atgaaaagct atggaaattg 1140
atagcatact acagtgaagt tcccgtgaag ctacacagag agctcatgct cagcaaagac 1200
cccagagtca gctaccagta caggcaggat gctgatgagg aagctcttta ctacacaggt 1260
gtgagagccc agattcttgc agaaccagaa tgggtcatgc agccatccat agatatccag 1320
ctgaaccgac gtcagagtac ggccaagaag gtgctacttt cctactacac ccagtattct 1380
caaacaatat ccaccaaaga tacaaggcag gctcggggca gaagctgggt aatgattgag 1440
cctagtggag ctgtgcaaag agatcagact gtcctggaag gggtccatg ccagttgagc 1500
tgcaacgtga aagcttctga gagtccatct atcttctggg tgcttccaga tggctccatc 1560
ctgaaagcgc ccatggatga cccagacagc aagttctcca ttctcagcag tggctggctg 1620
aggatcaagt ccatggagcc atctgactca ggcttgtacc agtgcattgc tcaagtgagg 1680
gatgaaatgg accgcatggt atatagggta cttgtgcagt ctcccctccac tcagccagcc 1740
gagaaagaca cagtgacaat tggcaagaac ccaggggagt cggtgacatt gccttgcaat 1800
gctttagcaa tacccgaagc ccaccttagc tggattcttc caaacagaag gataattaat 1860
gatttggcta acacatcaca tgtatacatg ttgccaaatg gaactctttc catcccaaag 1920
gtccaagtca gtgatagtgg ttactacaga tgtgtggctg tcaaccagca aggggcagac 1980
cattttacgg tgggaatcac agtgaccaag aaagggtctg gcttgccatc caaaagaggc 2040
agacgcccag gtgcaaaggc tctttccaga gtcagagaag acatcgtgga ggatgaaggg 2100

ggctcgggca tgggagatga agagaacact tcaaggagac ttctgcatcc aaaggaccaa 2160
gaggtgttcc tcaaaacaaa ggatgatgcc atcaatggag acaagaaagc caagaaaggg 2220
agaagaaagc tgaaactctg gaagcattcg gaaaaagaac cagagaccaa tgttgcagaa 2280
ggtcgcagag tgtttgaatc tagacgaagg ataaacatgg caaacaaaca gattaatccg 2340
gagcgctggg ctgatatttt agccaaagtc cgtgggaaaa atctccctaa gggcacagaa 2400
gtacccccgt tgattaaaac cacaagtcct ccatccttga gcctagaagt cacaccacct 2460
tttcctgctg tttctccccc ctcagcatct cctgtgcaga cagtaaccag tgctgaagaa 2520
tcctcagcag atgtacctct acttggtgaa gaagagcacg ttttgggtac catttcctca 2580
gccagcatgg ggctagaaca caaccacaat ggagttattc ttgttgaacc tgaagtaaca 2640
agcacacctc tggaggaagt tgttgatgac ctttctgaga agactgagga gataacttcc 2700
actgaaggag acctgaaggg gacagcagcc cctacactta tatctgagcc ttatgaacca 2760
tctcctactc tgcacacatt agacacagtc tatgaaaagc ccacccatga agagacggca 2820
acagagggtt ggtctgcagc agatgttgga tcgtcaccag agcccacatc cagtgagtat 2880
gagcctccat tggatgctgt ctccttggct gagtctgagc ccatgcaata ctttgaccca 2940
gatttggaga ctaagtcaca accagatgag gataagatga aagaagacac ctttgcacac 3000
cttactccaa cccccaccat ctgggttaat gactccagta catcacagtt atttgaggat 3060
tctactatag gggaaccagg tgtcccaggc caatcacatc tacaaggact gacagacaac 3120
atccaccttg tgaaaagtag tctaagcact caagacacct tactgattaa aaagggtatg 3180
aaagagatgt ctcagacact acagggagga aatatgctag agggagaccc cacacactcc 3240
agaagttctg agagtgaggg ccaagagagc aaatccatca ctttgcctga ctccacactg 3300
ggtataatga gcagtatgtc tccagttaag aagcctgcgg aaaccacagt tggtaccctc 3360
ctagacaaag acaccacaac agtaacaaca acaccaaggc aaaaagttgc tccgtcatcc 3420
accatgagca ctcacccttc tcgaaggaga cccaacggga gaaggagatt acgccccaac 3480
aaattccgcc accggcacaa gcaaacccca cccacaactt ttgccccatc agagactttt 3540
tctactcaac caactcaagc acctgacatt aagatttcaa gtcaagtgga gagttctctg 3600
gttcctacag cttgggtgga taacacagtt aataccccca aacagttgga aatggagaag 3660
aatgcagaac ccacatccaa gggaacacca cggagaaaac acgggaagag gccaaacaaa 3720
catcgatata ccccttctac agtgagctca agagcgtccg gatccaagcc cagcccttct 3780
ccagaaaata aacatagaaa cattgttact cccagttcag aaactatact tttgcctaga 3840
actgtttctc tgaaaactga gggcccttat gattccttag attacatgac aaccaccaga 3900
aaaatatatt catcttaccc taaagtccaa gagacacttc cagtcacata taaacccaca 3960
tcagatggaa aagaaattaa ggatgatgtt gccacaaatg ttgacaaaca taaaagtgac 4020
attttagtca ctggtgaatc aattactaat gccataccaa cttctcgctc cttggtctcc 4080
actatgggag aatttaagga agaatcctct cctgtaggct ttccaggaac tccaacctgg 4140
aatccctcaa ggacggccca gcctgggagg ctacagacag acatacctgt taccacttct 4200
ggggaaaatc ttacagaccc tccccttctt aaagagcttg aggatgtgga tttcacttcc 4260
gagtttttgt cctctttgac agtctccaca ccatttcacc aggaagaagc tggttcttcc 4320
acaactctct caagcataaa agtggaggtg gcttcaagtc aggcagaaac caccaccctt 4380
gatcaagatc atcttgaaac cactgtggct attctccttt ctgaaactag accacagaat 4440
cacacccccta ctgctgcccg gatgaaggag ccagcatcct cgtccccatc cacaattctc 4500
atgtctttgg gacaaaccac caccactaag ccagcacttc ccagtccaag aatatctcaa 4560
gcatctagag attccaagga aaatgttttc ttgaattatg tggggaatcc agaaacagaa 4620
gcaaccccag tcaacaatga aggaacacag catatgtcag ggccaaatga attatcaaca 4680
ccctcttccg accgggatgc atttaacttg tctacaaagc tggaattgga aaagcaagta 4740
tttggtagta ggagtctacc acgtggccca gatagccaac gccaggatgg aagagttcat 4800
gcttctcatc aactaaccag agtccctgcc aaacccatcc taccaacagc aacagtgagg 4860
ctacctgaaa tgtccacaca aagcgcttcc agatactttg taacttccca gtcacctcgt 4920
cactggacca acaaaccgga aataactaca tatccttctg ggggctttgcc agagaacaaa 4980
cagtttacaa ctccaagatt atcaagtaca acaattcctc tcccattgca catgtccaaa 5040
cccagcattc ctagtaagtt tactgaccga agaactgacc aattcaatgg ttactccaaa 5100
gtgtttggaa ataacaacat ccctgaggca agaaacccag ttggaaagcc tcccagtcca 5160
agaattcctc attattccaa tggaagactc cctttcttta ccaacaagac tctttctttt 5220
ccacagttgg gagtcacccg gagaccccag atacccactt ctcctgcccc agtaatgaga 5280
gagagaaaag ttattccagg ttcctacaac aggatacatt cccatagcac cttccatctg 5340
gactttggcc ctccggcacc tccgttgttg cacactccgc agaccacggg atcaccctca 5400
actaacttac agaatatccc tatggtctct tccacccaga gttctatctc ctttataaca 5460
tcttctgtcc agtcctcagg aagcttccac cagagcagct caaagttctt tgcaggagga 5520
cctcctgcat ccaaattctg gtctcttggg gaaaagcccc aaatcctcac caagtcccca 5580
cagactgtgt ccgtcaccgc tgagacagac actgtgttcc cctgtgaggc aacaggaaaa 5640
ccaaagcctt tcgttacttg gacaaaggtt tccacaggag ctcttatgac tccgaatacc 5700
aggatacaac ggtttgaggt tctcaagaac ggtaccttag tgatacggaa ggttcaagta 5760
caagatcgag gccagtatat gtgcaccgcc agcaacctgc acggcctgga caggatggtg 5820
gtcttgcttt cggtcaccgt gcagcaacct caaatcctag cctcccacta ccaggacgtc 5880
actgtctacc tgggagacac cattgcaatg gagtgtctgg ccaaagggac cccagccccc 5940
caaatttcct ggatcttccc tgacaggagg gtgtggcaaa ctgtgtcccc cgtggagagc 6000
cgcatcaccc tgcacgaaaa ccggaccctt tccatcaagg aggcgtcctt ctcagacaga 6060
ggcgtctata agtgcgtggc cagcaatgca gccggggcgg acagcctggc catccgcctg 6120
cacgtggcgg cactgccccc cgttatccac caggagaagc tggagaacat ctcgctgccc 6180
ccggggctca gcattcacat tcactgcact gccaaggctg cgcccctgcc cagcgtgcgc 6240

tgggtgctcg gggacggtac ccagatccgc ccctcgcagt tcctccacgg gaacttgttt 6300
gttttcccca acgggacgct ctacatccgc aacctcgcgc ccaaggacag cgggcgctat 6360
gagtgcgtgg ccgccaacct ggtaggctcc gcgcgcagga cggtgcagct gaacgtgcag 6420
cgtgcagcag ccaacgcgcg catcacgggc acctccccgc ggaggacgga cgtcaggtac 6480
ggaggaaccc tcaagctgga ctgcagcgcc tcgggggacc cctggccgcg catcctctgg 6540
aggctgccgt ccaagaggat gatcgacgcg ctcttcagtt ttgatagcag aatcaaggtg 6600
tttgccaatg ggaccctggt ggtgaaatca gtgacggaca aagatgccgg agattacctg 6660
tgcgtagctc gaaataaggt tggtgatgac tacgtggtgc tcaaagtgga tgtggtgatg 6720
aaaccggcca agattgaaca caaggaggag aacgaccaca aagtcttcta cgggggtgac 6780
ctgaaagtgg actgtgtggc caccgggctt cccaatcccg agatctcctg gagcctccca 6840
gacgggagtc tggtgaactc cttcatgcag tcggatgaca gcggtggacg caccaagcgc 6900
tatgtcgtct tcaacaatgg gacactctac tttaacgaag tggggatgag ggaggaagga 6960
gactacacct gctttgctga aaatcaggtc gggaaggacg agatgagagt cagagtcaag 7020
gtggtgacag cgcccgccac catccggaac aagacttact tggcggttca ggtgccctat 7080
ggagacgtgg tcactgtagc ctgtgaggcc aaaggagaac ccatgcccaa ggtgacttgg 7140
ttgtccccaa ccaacaaggt gatccccacc tcctctgaga agtatcagat ataccaagat 7200
ggcactctcc ttattcagaa agcccagcgt tctgacagcg gcaactacac ctgcctggtc 7260
aggaacagcg cgggagagga taggaagacg gtgtggattc acgtcaacgt ccagccaccc 7320
aagatcaacg gtaaccccaa ccccatcacc accgtgcggg agatagcagc cgggggcagt 7380
cggaaactga ttgactgcaa agctgaaggc atccccaccc cgagggtgtt atgggctttt 7440
cccgagggtg tggttctgcc agctccatac tatggaaacc ggatcactgt ccatggcaac 7500
ggttccctgg acatcaggag tttgaggaag agcgactccg tccagctggt atgcatggca 7560
cgcaacgagg gaggggaggc gaggttgatc gtgcagctca ctgtcctgga gcccatggag 7620
aaacccatct tccacgaccc gatcagcgag aagatcacgg ccatggcggg ccacaccatc 7680
agcctcaact gctctgccgc ggggaccccg acacccagcc tggtgtgggt ccttcccaat 7740
ggcaccgatc tgcagagtgg acagcagctg cagcgcttct accacaaggc tgacggcatg 7800
ctacacatta gcggtctctc ctcggtggac gctgggggcct accgctgcgt ggcccgcaat 7860
gccgctggcc acacggagag gctggtctcc ctgaaggtgg gactgaagcc agaagcaaac 7920
aagcagtatc ataacctggt cagcatcatc aatggtgaga ccctgaagct cccctgcacc 7980
cctcccgggg ctgggcaggg acgtttctcc tggacgctcc ccaatggcat gcatctggag 8040
ggcccccaaa ccctgggacg cgtttctctt ctggacaatg gcaccctcac ggttcgtgag 8100
gcctcggtgt ttgacagggg tacctatgta tgcaggatgg agacggagta cggcccttcg 8160
gtcaccagca tccccgtgat tgtgatcgcc tatcctcccc ggatcaccag cgagcccacc 8220
ccggtcatct acacccggcc cgggaacacc gtgaaactga actgcatggc tatggggatt 8280
cccaaagctg acatcacgtg ggagttaccg gataagtcgc atctgaaggc aggggttcag 8340
gctcgtctgt atggaaacag atttcttcac cccccagggat cactgaccat ccagcatgcc 8400
acacagagag atgccggctt ctacaagtgc atggcaaaaa acattctcgg cagtgactcc 8460
aaaacaactt acatccacgt cttctgaaat gtggattcca gaatgattgc ttaggaactg 8520
acaacaaagc ggggtttgta agggaagcca ggttggggaa taggagctct taaataatgt 8580
gtcacagtgc atggtggcct ctggtgggtt tcaagttgag gttgatcttg atctacaatt 8640
gttgggaaaa ggaagcaatg cagacacgag aaggagggct cagccttgct gagacacttt 8700
cttttgtgtt tacatcatgc caggggcttc attcagggtg tctgtgctct gactgcaatt 8760
tttcttcttt tgcaaatgcc actcgactgc cttcataagc gtccatagga tatctgagga 8820
acattcatca aaaataagcc atagacatga acaacacctc actaccccat tgaagacgca 8880
tcacctagtt aacctgctgc agtttttaca tgatagactt tgttccagat tgacaagtca 8940
tctttcagtt atttcctctg tcacttcaaa actccagctt gcccaataag gatttagaac 9000
cagagtgact gatatatata tatatatttt aattcagagt tacatacata cagctaccat 9060
tttatatgaa aaaagaaaaa catttcttcc tggaactcac tttttatata atgttttata 9120
tatatatttt ttcctttcaa atcagacgat gagactagaa ggagaaatac tttctgtctt 9180
attaaaatta ataaattatt ggtctttaca agacttggat acattacagc agacatggaa 9240
atataatttt aaaaaatttc tctccaacct ccttcaaatt cagtcaccac tgttatatta 9300
ccttctccag gaaccctcca gtggggaagg ctgcgatatt agatttcctt gtatgcaaag 9360
tttttgttga aagctgtgct cagaggaggt gagaggagag gaaggagaaa actgcatcat 9420
aactttacag aattgaatct agagtcttcc ccgaaaagcc cagaaacttc tctgcagtat 9480
ctggcttgtc catctggtct aaggtggctg cttcttcccc agccatgagt cagtttgtgc 9540
ccatgaataa tacacgacct gttatttcca tgactgcttt actgtatttt taaggtcaat 9600
atactgtaca tttgataata aaataatatt ctcccaaaaa aaaaa 9645

<210> 164
<211> 1840
<212> DNA
<213> Homo sapiens
<400> 164

tccacacaca caaaaaacct gcgcgtgagg ggggaggaaa agcagggcct ttaaaaaggc 60
aatcacaaca acttttgctg ccaggatgcc cttgctttgg ctgagaggat ttctgttggc 120
aagttgctgg attatagtga ggagttcccc caccccagga tccgaggggc acagcgcggc 180
ccccgactgt ccgtcctgtg cgctggccgc cctcccaaag gatgtaccca actctcagcc 240
agagatggtg gaggccgtca agaagcacat tttaaacatg ctgcacttga agaagagacc 300
cgatgtcacc cagccggtac ccaaggcggc gcttctgaac gcgatcagaa agcttcatgt 360

gggcaaagtc ggggagaacg ggtatgtgga gatagaggat gacattggaa ggagggcaga 420
aatgaatgaa cttatggagc agacctcgga gatcatcacg tttgccgagt caggaacagc 480
caggaagacg ctgcacttcg agatttccaa ggaaggcagt gacctgtcag tggtggagcg 540
tgcagaagtc tggctcttcc taaaagtccc caaggccaac aggaccagga ccaaagtcac 600
catccgcctc ttccagcagc agaagcaccc gcagggcagc ttggacacag gggaagaggc 660
cgaggaagtg ggcttaaagg gggagaggag tgaactgttg ctctctgaaa aagtagtaga 720
cgctcggaag agcacctggc atgtcttccc tgtctccagc agcatccagc ggttgctgga 780
ccagggcaag agctccctgg acgttcggat tgcctgtgag cagtgccagg agagtggcgc 840
cagcttggtt ctcctgggca agaagaagaa gaaagaagag gagggggaag ggaaaaagaa 900
gggcggaggt gaaggtgggg caggagcaga tgaggaaaag gagcagtcgc acagaccttt 960
cctcatgctg caggcccggc agtctgaaga ccaccctcat cgccggcgtc ggcggggctt 1020
ggagtgtgat ggcaaggtca acatctgctg taagaaacag ttctttgtca gtttcaagga 1080
catcggctgg aatgactgga tcattgctcc ctctggctat catgccaact actgcgaggg 1140
tgagtgcccg agccatatag caggcacgtc cgggtcctca ctgtccttcc actcaacagt 1200
catcaaccac taccgcatgc ggggccatag ccccttttgcc aacctcaaat cgtgctgtgt 1260
gcccaccaag ctgagaccca tgtccatgtt gtactatgat gatggtcaaa acatcatcaa 1320
aaaggacatt cagaacatga tcgtggagga gtgtgggtgc tcatagagtt gcccagccca 1380
gggggaaagg gagcaagagt tgtccagaga agacagtggc aaaatgaaga aatttttaag 1440
gtttctgagt taaccagaaa aatagaaatt aaaaacaaaa caaaacaaaa aaaaaaacaa 1500
aaaaaaacaa aagtaaatta aaaacaaacc tgatgaaaca gatgaaacag atgaaggaag 1560
atgtggaaat cttagcctgc cttagccagg gctcagagat gaagcagtga agagacagat 1620
tgggagggaa agggagaatg gtgtaccctt tatttcttct gaaatcacac tgatgacatc 1680
agttgtttaa acggggtatt gtcctttccc cccttgaggt tcccttgtga gcttgaatca 1740
accaatctga tctgcagtag tgtggactag aacaacccaa atagcatcta gaaagccatg 1800
agtttgaaag ggcccatcac aggcactttc ctagcctaat 1840

<210> 165
<211> 11185
<212> DNA
<213> Homo sapiens
<400> 165

gctgccccga gcctttctgg ggaagaactc caggcgtgcg gacgcaacag ccgagaacat 60
taggtgttgt ggacaggagc tgggaccaag atcttcggcc agccccgcat cctcccgcat 120
cttccagcac cgtcccgcac cctccgcatc cttccccggg ccaccacgct tcctatgtga 180
cccgcctggg caacgccgaa cccagtcgcg cagcgctgca gtgaatttc cccccaaact 240
gcaataagcc gccttccaag gccaagatgt tcataaatat aaagagcatc ttatggatgt 300
gttcaacctt aatagtaacc catgcgctac ataaagtcaa agtgggaaaa agcccaccgg 360
tgaggggctc cctctctgga aaagtcagcc taccttgtca tttttcaacg atgcctactt 420
tgccacccag ttacaacacc agtgaatttc tccgcatcaa atggtctaag attgaagtgg 480
acaaaaatgg aaaagatttg aaagagacta ctgtccttgt ggcccaaaat ggaaatatca 540
agattggtca ggactacaaa gggagagtgt ctgtgcccac acatcccgag gctgtgggcg 600
atgcctccct cactgtggtc aagctgctgg caagtgatgc gggtctttac cgctgtgacg 660
tcatgtacgg gattgaagac acacaagaca cggtgtcact gactgtggat ggggttgtgt 720
ttcactacag ggcggcaacc agcaggtaca cactgaattt tgaggctgct cagaaggctt 780
gtttggacgt tggggcagtc atagcaactc cagagcagct ctttgctgcc tatgaagatg 840
gatttgagca gtgtgacgca ggctggctgg ctgatcagac tgtcagatat cccatccggg 900
ctcccagagt aggctgttat ggagataaga tgggaaaggc aggagtcagg acttatggat 960
tccgttctcc ccaggaaact tacgatgtgt attgttatgt ggatcatctg gatggtgatg 1020
tgttccacct cactgtcccc agtaaattca ccttcgagga ggctgcaaaa gagtgtgaaa 1080
accaggatgc caggctggca acagtggggg aactccaggc ggcatggagg aacggctttg 1140
accagtgcga ttacgggtgg ctgtcggatg ccagcgtgcg ccaccctgtg actgtggcca 1200
gggcccagtg tggaggtggt ctacttgggg tgagaaccct gtatcgtttt gagaaccaga 1260
caggcttccc tccccctgat agcagatttg atgcctactg ctttaaacct aaagaggcta 1320
caaccatcga tttgagtatc ctcgcagaaa ctgcatcacc cagtttatcc aaagaaccac 1380
aaatggtttc tgatagaact acaccaatca tccctttagt tgatgaatta cctgtcattc 1440
caacagagtt ccctcccgtg ggaaatattg tcagttttga acagaaagcc acagtccaac 1500
ctcaggctat cacagatagt ttagccacca aattacccac acctactggc agtaccaaga 1560
agccctggga tatggatgac tactcacctt ctgcttcagg acctcttgga aagctagaca 1620
tatcagaaat taaggaagaa gtgctccaga gtacaactgg cgtctctcat tatgctacgg 1680
attcatggga tggtgtcgtg gaagataaac aaacacaaga atcggttaca cagattgaac 1740
aaatagaagt gggtcctttg gtaacatcta tggaaatctt aaagcacatt ccttccaagg 1800
aattccctgt aactgaaaca ccattggtaa ctgcaagaat gatcctggaa tccaaaactg 1860
aaaagaaaat ggtaagcact gtttctgaat tggtaaccac aggtcactat ggattcacct 1920
tgggagaaga ggatgatgaa gacagaacac ttacagttgg atctgatgag agcaccttga 1980
tctttgacca aattcctgaa gtcattacgg tgtcaaagac ttcagaagac accatccaca 2040
ctcatttaga agacttggag tcagtctcag catccacaac tgtttcccct ttaattatgc 2100
ctgataataa tggatcatcc atggatgact gggaagagag acaaactagt ggtaggataa 2160
cggaagagtt tcttggcaaa tatctgtcta ctacaccttt tccatcacag catcgtacag 2220
aaatagaatt gtttccttat tctggtgata aaatattagt agagggaatt tccacagtta 2280

tttatccttc tctacaaaca gaaatgacac atagaagaga aagaacagaa acactaatac 2340
cagagatgag aacagatact tatacagatg aaatacaaga agagatcact aaaagtccat 2400
ttatgggaaa aacagaagaa gaagtcttct ctgggatgaa actctctaca tctctctcag 2460
agccaattca tgttacagag tcttctgtgg aaatgaccaa gtcttttgat ttcccaacat 2520
tgataacaaa gttaagtgca gagccaacag aagtaagaga tatggaggaa gactttacag 2580
caactccagg tactacaaaa tatgatgaaa atattacaac agtgcttttg gcccatggta 2640
ctttaagtgt tgaagcagcc actgtatcaa aatggtcatg ggatgaagat aatacaacat 2700
ccaagccttt agagtctaca gaaccttcag cctcttcaaa attgcccccct gccttactca 2760
caactgtggg gatgaatgga aaggataaag acatcccaag tttcactgaa gatggagcag 2820
atgaatttac tcttattcca gatagtactc aaaagcagtt agaggaggtt actgatgaag 2880
acatagcagc ccatggaaaa ttcacaatta gatttcagcc aactacatca actggtattg 2940
cagaaaagtc aactttgaga gattctacaa ctgaagaaaa agttccacct atcacaagca 3000
ctgaaggcca agtttatgca accatggaag gaagtgcttt gggtgaagta gaagatgtgg 3060
acctctctaa gccagtatct actgttcccc aatttgcaca cacttcagag gtggaaggat 3120
tagcatttgt tagttatagt agcacccaag agcctactac ttatgtagac tcttcccata 3180
ccattcctct ttctgtaatt cccaagacag actgggggagt gttagtacct tctgttccat 3240
cagaagatga agttctaggt gaaccctctc aagacatact tgtcattgat cagactcgcc 3300
ttgaagcgac tatttctcca gaaactatga gaacaacaaa aatcacagag ggaacaactc 3360
aggaagaatt cccttggaaa gaacagactg cagagaaacc agttcctgct ctcagttcta 3420
cagcttggac tcccaaggag gcagtaacac cactggatga acaagagggc gatggatcag 3480
catatacagt ctctgaagat gaattgttga caggttctga gagggtccca gttttagaaa 3540
caactccagt tggaaaaatt gatcacagtg tgtcttatcc accaggtgct gtaactgagc 3600
acaaagtgaa aacagatgaa gtggtaacac taacaccacg cattgggcca aaagtatctt 3660
taagtccagg gcctgaacaa aaatatgaaa cagaaggtag tagtacaaca ggatttacat 3720
catctttgag tccttttagt acccacatta cccagcttat ggaagaaacc actactgaga 3780
aaacatccct agaggatatt gatttaggct caggattatt tgaaaagccc aaagccacag 3840
aactcataga attttcaaca atcaaagtca cagttccaag tgatattacc actgccttca 3900
gttcagtaga cagacttcac acaacttcag cattcaagcc atcttccgcg atcactaaga 3960
aaccacctct catcgacagg gaacctggtg aagaaacaac cagtgacatg gtaatcattg 4020
gagaatcaac atctcatgtt cctcccacta cccttgaaga tattgtagcc aaggaaacag 4080
aaaccgatat tgatagagag tatttcacga cttcaagtcc tcctgctaca cagccaacaa 4140
gaccacccac tgtggaagac aaagaggcct ttggacctca ggcgctttct acgccacagc 4200
ccccagcaag cacaaaattt caccctgaca ttaatgttta tattattgag gtcagagaaa 4260
ataagacagg tcgaatgagt gatttgagtg taattggtca tccaatagat tcagaatcta 4320
aagaagatga accttgtagt gaagaaacag atccagtgca tgatctaatg gctgaaattt 4380
tacctgaatt ccctgacata attgaaatag acctatacca cagtgaagaa aatgaagaag 4440
aagaagaaga gtgtgcaaat gctactgatg tgacaaccac cccatctgtg cagtacataa 4500
atgggaagca tctcgttacc actgtgccca aggacccaga agctgcagaa gctaggcgtg 4560
gccagtttga aagtgttgca ccttctcaga atttctcgga cagctctgaa agtgatactc 4620
atccatttgt aatagccaaa acggaattgt ctactgctgt gcaacctaat gaatctacag 4680
aaacaactga gtctcttgaa gttacatgga agcctgagac ttaccctgaa acatcagaac 4740
atttttcagg tggtgagcct gatgttttcc ccacagtccc attccatgag gaatttgaaa 4800
gtggaacagc caaaaaaggg gcagaatcag tcacagagag agatactgaa gttggtcatc 4860
aggcacatga acatactgaa cctgtatctc tgtttcctga agagtcttca ggagagattg 4920
ccattgacca agaatctcag aaaatagcct ttgcaagggc tacagaagta acatttggtg 4980
aagaggtaga aaaaagtact tctgtcacat acactcccac tatagttcca agttctgcat 5040
cagcatatgt ttcagaggaa gaagcagtta ccctaatagg aaatccttgg ccagatgacc 5100
tgttgtctac caaagaaagc tgggtagaag caactcctag acaagttgta gagctctcag 5160
ggagttcttc gattccaatt acagaaggct ctggagaagc agaagaagat gaagatacaa 5220
tgttcaccat ggtaactgat ttatcacaga gaaatactac tgatacactc attactttag 5280
acactagcag gataatcaca gaaagctttt ttgaggttcc tgcaaccacc atttatccag 5340
tttctgaaca accttctgca aaagtggtgc ctaccaagtt tgtaagtgaa acagacactt 5400
ctgagtggat ttccagtacc actgttgagg aaaagaaaag gaaggaggag gagggaacta 5460
caggtacggc ttctacattt gaggtatatt catctacaca gagatcggat caattaattt 5520
taccctttga attagaaagt ccaaatgtag ctacatctag tgattcaggt accaggaaaa 5580
gttttatgtc cttgacaaca ccaacacagt ctgaaaggga aatgacagat tctactcctg 5640
tctttacaga aacaaataca ttagaaaatt tgggggcaca gaccactgag cacagcagta 5700
tccatcaacc tggggttcag gaagggctga ccactctccc acgtagtcct gcctctgtct 5760
ttatggagca gggctctgga gaagctgctg ccgacccaga aaccaccact gtttcttcat 5820
tttcattaaa cgtagagtat gcaattcaag ccgaaaagga agtagctggc actttgtctc 5880
cgcatgtgga aactacattc tccactgagc caacaggact ggttttgagt acagtaatgg 5940
acagagtagt tgctgaaaat ataacccaaa catccaggga aatagtgatt tcagagcgat 6000
taggagaacc aaattatggg gcagaaataa ggggcttttc cacaggtttt cctttggagg 6060
aagatttcag tggtgacttt agagaatact caacagtgtc tcatcccata gcaaaagaag 6120
aaacggtaat gatggaaggc tctggagatg cagcatttag ggacacccag acttcaccat 6180
ctacagtacc tacttcagtt cacatcagtc acatatctga ctcagaagga cccagtagca 6240
ccatggtcag cacttcagcc ttcccctggg aagagtttac atcctcagct gagggctcag 6300
gtgagcaact ggtcacagtc agcagctctg ttgttccagt gcttcccagt gctgtgcaaa 6360
agttttctgg tacagcttcc tccattatcg acgaaggatt gggagaagtg ggtactgtca 6420

atgaaattga tagaagatcc accattttac caacagcaga agtggaaggt acgaaagctc 6480
cagtagagaa ggaggaagta aaggtcagtg gcacagtttc aacaaacttt ccccaaacta 6540
tagagccagc caaattatgg tctaggcaag aagtcaaccc tgtaagacaa gaaattgaaa 6600
gtgaaacaac atcagaggaa caaattcaag aagaaaagtc atttgaatcc cctcaaaact 6660
ctcctgcaac agaacaaaca atctttgatt cacagacatt tactgaaact gaactcaaaa 6720
ccacagatta ttctgtacta acaacaaaga aaacttacag tgatgataaa gaaatgaagg 6780
aggaagacac ttctttagtt aacatgtcta ctccagatcc agatgcaaat ggcttggaat 6840
cttacacaac tctccctgaa gctactgaaa agtcacattt tttcttagct actgcattag 6900
taactgaatc tataccagct gaacatgtag tcacagattc accaatcaaa aaggaagaaa 6960
gtacaaaaca ttttccgaaa ggcatgagac caacaattca agagtcagat actgagctct 7020
tattctctgg actgggatca ggagaagaag ttttacctac tctaccaaca gagtcagtga 7080
atttactga agtggaacaa atcaataaca cattatatcc ccacacttct caagtggaaa 7140
gtacctcaag tgacaaaatt gaagacttta acagaatgga aaatgtggca aaagaagttg 7200
gaccactcgt atctcaaaca gacatctttg aaggtagtgg gtcagtaacc agcacaacat 7260
taatagaaat tttaagtgac actggagcag aaggacccac ggtggcacct ctccctttct 7320
ccacggacat cggacatcct caaaatcaga ctgtcaggtg ggcagaagaa atccagacta 7380
gtagaccaca aaccataact gaacaagact ctaacaagaa ttcttcaaca gcagaaatta 7440
acgaaacaac aacctcatct actgattttc tggctagagc ttatggtttt gaaatggcca 7500
aagaatttgt tacatcagca ccaaaaccat ctgacttgta ttatgaacct tctggagaag 7560
gatctggaga agtggatatt gttgattcat ttcacacttc tgcaactact caggcaacca 7620
gacaagaaag cagcaccaca tttgtttctg atgggtccct ggaaaaacat cctgaggtgc 7680
caagcgctaa agctgttact gctgatggat tcccaacagt ttcagtgatg ctgcctcttc 7740
attcagagca gaacaaaagc tcccctgatc caactagcac actgtcaaat acagtgtcat 7800
atgagaggtc cacagacggt agtttccaag accgtttcag ggaattcgag gattccacct 7860
taaaacctaa cagaaaaaaa cccactgaaa atattatcat agacctggac aaagaggaca 7920
aggatttaat attgacaatt acagagagta ccatccttga aattctacct gagctgacat 7980
cggataaaaa tactatcata gatattgatc atactaaacc tgtgtatgaa gacattcttg 8040
gaatgcaaac agatatagat acagaggtac catcagaacc acatgacagt aatgatgaaa 8100
gtaatgatga cagcactcaa gttcaagaga tctatgaggc agctgtcaac ctttctttaa 8160
ctgaggaaac atttgagggc tctgctgatg ttctggctag ctacactcag gcaacacatg 8220
atgaatcaat gacttatgaa gatagaagcc aactagatca catgggcttt cacttcacaa 8280
ctgggatccc tgctcctagc acagaaacag aattagacgt tttacttccc acggcaacat 8340
ccctgccaat tcctcgtaag tctgccacag ttattccaga gattgaagga ataaaagctg 8400
aagcaaaagc cctggatgac atgtttgaat caagcacttt gtctgatggt caagctattg 8460
cagaccaaag tgaaataata ccaacattgg gccaatttga aaggactcag gaggagtatg 8520
aagacaaaaa acatgctggt ccttcttttc agccagaatt ctcttcagga gctgaggagg 8580
cattagtaga ccatactccc tatctaagta ttgctactac ccaccttatg gatcagagtg 8640
taacagaggt gcctgatgtg atggaaggat ccaatccccc atattacact gatacaacat 8700
tagcagtttc aacatttgcg aagttgtctt ctcagacacc atcatctccc ctcactatct 8760
actcaggcag tgaagcctct ggacacacag agatccccca gcccagtgct ctgccaggaa 8820
tagacgtcgg ctcatctgta atgtccccac aggattcttt taaggaaatt catgtaaata 8880
ttgaagcaac tttcaaacca tcaagtgagg aataccttca cataactgag cctccctctt 8940
tatctcctga cacaaaatta gaaccttcag aagatgatgg taaacctgag ttattagaag 9000
aaatggaagc ttctcccaca gaacttattg ctgtggaagg aactgagatt ctccaagatt 9060
tccaaaacaa aaccgatggt caagtttctg gagaagcaat caagatgttt cccaccatta 9120
aaacacctga ggctggaact gttattacaa ctgccgatga aattgaatta gaaggtgcta 9180
cacagtggcc acactctact tctgcttctg ccacctatgg ggtcgaggca ggtgtggtgc 9240
cttggctaag tccacagact tctgagaggc ccacgctttc ttcttctcca gaaataaacc 9300
ctgaaactca agcagcttta atcagagggc aggattccac gatagcagca tcagaacagc 9360
aagtggcagc gagaattctt gattccaatg atcaggcaac agtaaaccct gtggaattta 9420
atactgaggt tgcaacacca ccattttccc ttctggagac ttctaatgaa acagatttcc 9480
tgattggcat taatgaagag tcagtggaag gcacggcaat ctatttacca ggacctgatc 9540
gctgcaaaat gaacccgtgc cttaacggag gcacctgtta tcctactgaa acttcctacg 9600
tatgcacctg tgtgccagga tacagcggag accagtgtga acttgatttt gatgaatgtc 9660
actctaatcc ctgtcgtaat ggagccactt gtgttgatgg ttttaacaca ttcaggtgcc 9720
tctgccttcc aagttatgtt ggtgcacttt gtgagcaaga taccgagaca tgtgactatg 9780
gctggcacaa attccaaggg cagtgctaca aatactttgc ccatcgacgc acatgggatg 9840
cagctgaacg ggaatgccgt ctgcagggtg cccatctcac aagcatcctg tctcacgaag 9900
aacaaatgtt tgttaatcgt gtgggccatg attatcagtg gataggcctc aatgacaaga 9960
tgtttgagca tgacttccgt tggactgatg gcagcacact gcaatacgag aattggagac 10020
ccaaccagcc agacagcttc ttttctgctg gagaagactg tgttgtaatc atttggcatg 10080
agaatggcca gtggaatgat gttccctgca attaccatct cacctatacg tgcaagaaag 10140
gaacagttgc ttgcggccag ccccctgttg tagaaaatgc caagaccttt ggaaagatga 10200
aacctcgtta tgaaatcaac tccctgatta gataccactg caaagatggt ttcattcaac 10260
gtcaccttcc aactatccgg tgcttaggaa atggaagatg ggctatacct aaaattacct 10320
gcatgaaccc atctgcatac caaaggactt attctatgaa atactttaaa aattcctcat 10380
cagcaaagga caattcaata aatacatcca aacatgatca tcgttggagc cggaggtggc 10440
aggagtcgag gcgctgatcc ctaaaatggc gaacatgtgt tttcatcatt tcagccaaag 10500
tcctaacttc ctgtgccttt cctatcacct cgagaagtaa ttatcagttg gtttggattt 10560

ttggaccacc gttcagtcat tttgggttgc cgtgctccca aaacatttta aatgaaagta 10620
ttggcattca aaaagacagc agacaaaatg aaagaaaatg agagcagaaa gtaagcattt 10680
ccagcctatc taatttcttt agttttctat ttgcctccag tgcagtccat ttcctaatgt 10740
ataccagcct actgtactat ttaaaatgct caatttcagc accgatggcc atgtaaataa 10800
gatgatttaa tgttgatttt aatcctgtat ataaaataaa aagtcacaat gagtttgggc 10860
atatttaatg atgattatgg agccttagag gtctttaatc attggttcgg ctgcttttat 10920
gtagtttagg ctggaaatgg tttcacttgc tctttgactg tcagcaagac tgaagatggc 10980
ttttcctgga cagctagaaa acacaaaatc ttgtaggtca ttgcacctat ctcagccata 11040
ggtgcagttt gcttctacat gatgctaaag gctgcgaatg ggatcctgat ggaactaagg 11100
actccaatgt cgaactcttc tttgctgcat tcctttttct tcacttacaa gaaaggcctg 11160
aatggaggac ttttctgtaa ccagg 11185

<210> 166
<211> 339
<212> PRT
<213> Homo sapiens
<400> 166

Met Trp Gln Leu Trp Ala Ser Leu Cys Cys Leu Leu Val Leu Ala Asn
1               5                   10                  15
Ala Arg Ser Arg Pro Ser Phe His Pro Leu Ser Asp Glu Leu Val Asn
            20                  25                  30
Tyr Val Asn Lys Arg Asn Thr Thr Trp Gln Ala Gly His Asn Phe Tyr
        35                  40                  45
Asn Val Asp Met Ser Tyr Leu Lys Arg Leu Cys Gly Thr Phe Leu Gly
    50                  55                  60
Gly Pro Lys Pro Pro Gln Arg Val Met Phe Thr Glu Asp Leu Lys Leu
65                  70                  75                  80
Pro Ala Ser Phe Asp Ala Arg Glu Gln Trp Pro Gln Cys Pro Thr Ile
                85                  90                  95
Lys Glu Ile Arg Asp Gln Gly Ser Cys Gly Ser Cys Trp Ala Phe Gly
            100                 105                 110
Ala Val Glu Ala Ile Ser Asp Arg Ile Cys Ile His Thr Asn Ala His
        115                 120                 125
Val Ser Val Glu Val Ser Ala Glu Asp Leu Leu Thr Cys Cys Gly Ser
    130                 135                 140
Met Cys Gly Asp Gly Cys Asn Gly Gly Tyr Pro Ala Glu Ala Trp Asn
145                 150                 155                 160
Phe Trp Thr Arg Lys Gly Leu Val Ser Gly Gly Leu Tyr Glu Ser His
                165                 170                 175
Val Gly Cys Arg Pro Tyr Ser Ile Pro Pro Cys Glu His His Val Asn
            180                 185                 190
Gly Ser Arg Pro Pro Cys Thr Gly Glu Gly Asp Thr Pro Lys Cys Ser
        195                 200                 205
Lys Ile Cys Glu Pro Gly Tyr Ser Pro Thr Tyr Lys Gln Asp Lys His
    210                 215                 220
Tyr Gly Tyr Asn Ser Tyr Ser Val Ser Asn Ser Glu Lys Asp Ile Met
225                 230                 235                 240
Ala Glu Ile Tyr Lys Asn Gly Pro Val Glu Gly Ala Phe Ser Val Tyr
                245                 250                 255
Ser Asp Phe Leu Leu Tyr Lys Ser Gly Val Tyr Gln His Val Thr Gly
            260                 265                 270
Glu Met Met Gly Gly His Ala Ile Arg Ile Leu Gly Trp Gly Val Glu
        275                 280                 285
Asn Gly Thr Pro Tyr Trp Leu Val Ala Asn Ser Trp Asn Thr Asp Trp
    290                 295                 300
Gly Asp Asn Gly Phe Phe Lys Ile Leu Arg Gly Gln Asp His Cys Gly
305                 310                 315                 320
Ile Glu Ser Glu Val Val Ala Gly Ile Pro Arg Thr Asp Gln Tyr Trp
                325                 330                 335
Glu Lys Ile

<210> 167
<211> 286
<212> PRT
<213> Homo sapiens
<400> 167

Met Arg Leu Leu Pro Arg Leu Leu Leu Leu Leu Leu Leu Val Phe Pro
1               5                   10                  15

Ala Thr Val Leu Phe Arg Gly Gly Pro Arg Gly Ser Leu Ala Val Ala
20 25 30
Gln Asp Leu Thr Glu Asp Glu Glu Thr Val Glu Asp Ser Ile Ile Glu
35 40 45
Asp Glu Asp Asp Glu Ala Glu Val Glu Glu Asp Glu Pro Thr Asp Leu
50 55 60
Val Glu Asp Lys Glu Glu Glu Asp Val Ser Gly Glu Pro Glu Ala Ser
65 70 75 80
Pro Ser Ala Asp Thr Thr Ile Leu Phe Val Lys Gly Glu Asp Phe Pro
85 90 95
Ala Asn Asn Ile Val Lys Phe Leu Val Gly Phe Thr Asn Lys Gly Thr
100 105 110
Glu Asp Phe Ile Val Glu Ser Leu Asp Ala Ser Phe Arg Tyr Pro Gln
115 120 125
Asp His Gln Phe Tyr Ile Gln Asn Phe Thr Ala Leu Pro Leu Asn Thr
130 135 140
Val Val Pro Pro Gln Arg Gln Ala Thr Phe Glu Tyr Ser Phe Ile Pro
145 150 155 160
Ala Glu Pro Met Gly Gly Arg Pro Phe Gly Leu Val Ile Asn Leu Asn
165 170 175
Tyr Lys Asp Leu Asn Gly Asn Val Phe Gln Asp Ala Val Phe Asn Gln
180 185 190
Thr Val Thr Val Ile Glu Arg Glu Asp Gly Leu Asp Gly Glu Thr Ile
195 200 205
Phe Met Tyr Met Phe Leu Ala Gly Leu Gly Leu Leu Val Ile Val Gly
210 215 220
Leu His Gln Leu Leu Glu Ser Arg Lys Arg Lys Arg Pro Ile Gln Lys
225 230 235 240
Val Glu Met Gly Thr Ser Ser Gln Asn Asp Val Asp Met Ser Trp Ile
245 250 255
Pro Gln Glu Thr Leu Asn Gln Ile Asn Lys Ala Ser Pro Arg Arg Leu
260 265 270
Pro Arg Lys Arg Ala Gln Lys Arg Ser Val Gly Ser Asp Glu
275 280 285

<210> 168
<211> 433
<212> PRT
<213> Homo sapiens
<400> 168
Met Pro Asp Tyr Leu Gly Ala Asp Gln Arg Lys Thr Lys Glu Asp Glu
1 5 10 15
Lys Asp Asp Lys Pro Ile Arg Ala Leu Asp Glu Gly Asp Ile Ala Leu
20 25 30
Leu Lys Thr Tyr Gly Gln Ser Thr Tyr Ser Arg Gln Ile Lys Gln Val
35 40 45
Glu Asp Asp Ile Gln Gln Leu Leu Lys Lys Ile Asn Glu Leu Thr Gly
50 55 60
Ile Lys Glu Ser Asp Thr Gly Leu Ala Pro Pro Ala Leu Trp Asp Leu
65 70 75 80
Ala Ala Asp Lys Gln Thr Leu Gln Ser Glu Gln Pro Leu Gln Val Ala
85 90 95
Arg Cys Thr Lys Ile Ile Asn Ala Asp Ser Glu Asp Pro Lys Tyr Ile
100 105 110
Ile Asn Val Lys Gln Phe Ala Lys Phe Val Val Asp Leu Ser Asp Gln
115 120 125
Val Ala Pro Thr Asp Ile Glu Glu Gly Met Arg Val Gly Val Asp Arg
130 135 140
Asn Lys Tyr Gln Ile His Ile Pro Leu Pro Pro Lys Ile Asp Pro Thr
145 150 155 160
Val Thr Met Met Gln Val Glu Glu Lys Pro Asp Val Thr Tyr Ser Asp
165 170 175
Val Gly Gly Cys Lys Glu Gln Ile Glu Lys Leu Arg Glu Val Val Glu
180 185 190
Thr Pro Leu Leu His Pro Glu Arg Phe Val Asn Leu Gly Ile Glu Pro
195 200 205
Pro Lys Gly Val Leu Leu Phe Gly Pro Pro Gly Thr Gly Lys Thr Leu
210 215 220
Cys Ala Arg Ala Val Ala Asn Arg Thr Asp Ala Cys Phe Ile Arg Val

```
225                 230                 235                 240
Ile Gly Ser Glu Leu Val Gln Lys Tyr Val Gly Glu Gly Ala Arg Met
                245                 250                 255
Val Arg Glu Leu Phe Glu Met Ala Arg Thr Lys Lys Ala Cys Leu Ile
            260                 265                 270
Phe Phe Asp Glu Ile Asp Ala Ile Gly Gly Ala Arg Phe Asp Asp Gly
        275                 280                 285
Ala Gly Gly Asp Asn Glu Val Gln Arg Thr Met Leu Glu Leu Ile Asn
    290                 295                 300
Gln Leu Asp Gly Phe Asp Pro Arg Gly Asn Ile Lys Val Leu Met Ala
305                 310                 315                 320
Thr Asn Arg Pro Asp Thr Leu Asp Pro Ala Leu Met Arg Pro Gly Arg
                325                 330                 335
Leu Asp Arg Lys Ile Glu Phe Ser Leu Pro Asp Leu Glu Gly Arg Thr
            340                 345                 350
His Ile Phe Lys Ile His Ala Arg Ser Met Ser Val Glu Arg Asp Ile
        355                 360                 365
Arg Phe Glu Leu Leu Ala Arg Leu Cys Pro Asn Ser Thr Gly Ala Glu
    370                 375                 380
Ile Arg Ser Val Cys Thr Glu Ala Gly Met Phe Ala Ile Arg Ala Arg
385                 390                 395                 400
Arg Lys Ile Ala Thr Glu Lys Asp Phe Leu Glu Ala Val Asn Lys Val
                405                 410                 415
Ile Lys Ser Tyr Ala Lys Phe Ser Ala Thr Pro Arg Tyr Met Thr Tyr
            420                 425                 430
Asn


<210>  169
<211>  529
<212>  PRT
<213>  Homo sapiens
<400>  169
Met Ser Thr Asn Glu Asn Ala Asn Thr Pro Ala Ala Arg Leu His Arg
1               5                   10                  15
Phe Lys Asn Lys Gly Lys Asp Ser Thr Glu Met Arg Arg Arg Arg Ile
            20                  25                  30
Glu Val Asn Val Glu Leu Arg Lys Ala Lys Lys Asp Asp Gln Met Leu
        35                  40                  45
Lys Arg Arg Asn Val Ser Ser Phe Pro Asp Asp Ala Thr Ser Pro Leu
    50                  55                  60
Gln Glu Asn Arg Asn Asn Gln Gly Thr Val Asn Trp Ser Val Asp Asp
65                  70                  75                  80
Ile Val Lys Gly Ile Asn Ser Ser Asn Val Glu Asn Gln Leu Gln Ala
                85                  90                  95
Thr Gln Ala Ala Arg Lys Leu Leu Ser Arg Glu Lys Gln Pro Pro Ile
            100                 105                 110
Asp Asn Ile Ile Arg Ala Gly Leu Ile Pro Lys Phe Val Ser Phe Leu
        115                 120                 125
Gly Arg Thr Asp Cys Ser Pro Ile Gln Phe Glu Ser Ala Trp Ala Leu
    130                 135                 140
Thr Asn Ile Ala Ser Gly Thr Ser Glu Gln Thr Lys Ala Val Val Asp
145                 150                 155                 160
Gly Gly Ala Ile Pro Ala Phe Ile Ser Leu Leu Ala Ser Pro His Ala
                165                 170                 175
His Ile Ser Glu Gln Ala Val Trp Ala Leu Gly Asn Ile Ala Gly Asp
            180                 185                 190
Gly Ser Val Phe Arg Asp Leu Val Ile Lys Tyr Gly Ala Val Asp Pro
        195                 200                 205
Leu Leu Ala Leu Leu Ala Val Pro Asp Met Ser Ser Leu Ala Cys Gly
    210                 215                 220
Tyr Leu Arg Asn Leu Thr Trp Thr Leu Ser Asn Leu Cys Arg Asn Lys
225                 230                 235                 240
Asn Pro Ala Pro Pro Ile Asp Ala Val Glu Gln Ile Leu Pro Thr Leu
                245                 250                 255
Val Arg Leu Leu His His Asp Asp Pro Glu Val Leu Ala Asp Thr Cys
            260                 265                 270
Trp Ala Ile Ser Tyr Leu Thr Asp Gly Pro Asn Glu Arg Ile Gly Met
        275                 280                 285
```

Val Val Lys Thr Gly Val Val Pro Gln Leu Val Lys Leu Leu Gly Ala
290 295 300
Ser Glu Leu Pro Ile Val Thr Pro Ala Leu Arg Ala Ile Gly Asn Ile
305 310 315 320
Val Thr Gly Thr Asp Glu Gln Thr Gln Val Val Ile Asp Ala Gly Ala
325 330 335
Leu Ala Val Phe Pro Ser Leu Leu Thr Asn Pro Lys Thr Asn Ile Gln
340 345 350
Lys Glu Ala Thr Trp Thr Met Ser Asn Ile Thr Ala Gly Arg Gln Asp
355 360 365
Gln Ile Gln Gln Val Val Asn His Gly Leu Val Pro Phe Leu Val Ser
370 375 380
Val Leu Ser Lys Ala Asp Phe Lys Thr Gln Lys Glu Ala Val Trp Ala
385 390 395 400
Val Thr Asn Tyr Thr Ser Gly Gly Thr Val Glu Gln Ile Val Tyr Leu
405 410 415
Val His Cys Gly Ile Ile Glu Pro Leu Met Asn Leu Leu Thr Ala Lys
420 425 430
Asp Thr Lys Ile Ile Leu Val Ile Leu Asp Ala Ile Ser Asn Ile Phe
435 440 445
Gln Ala Ala Glu Lys Leu Gly Glu Thr Glu Lys Leu Ser Ile Met Ile
450 455 460
Glu Glu Cys Gly Gly Leu Asp Lys Ile Glu Ala Leu Gln Asn His Glu
465 470 475 480
Asn Glu Ser Val Tyr Lys Ala Ser Leu Ser Leu Ile Glu Lys Tyr Phe
485 490 495
Ser Val Glu Glu Glu Glu Asp Gln Asn Val Val Pro Glu Thr Thr Ser
500 505 510
Glu Gly Tyr Thr Phe Gln Val Gln Asp Gly Ala Pro Gly Thr Phe Asn
515 520 525
Phe

<210> 170
<211> 971
<212> PRT
<213> Homo sapiens
<400> 170
Met Glu Leu Ser Asp Ala Asn Leu Gln Thr Leu Thr Glu Tyr Leu Lys
1 5 10 15
Lys Thr Leu Asp Pro Asp Pro Ala Ile Arg Arg Pro Ala Glu Lys Phe
20 25 30
Leu Glu Ser Val Glu Gly Asn Gln Asn Tyr Pro Leu Leu Leu Thr
35 40 45
Leu Leu Glu Lys Ser Gln Asp Asn Val Ile Lys Val Cys Ala Ser Val
50 55 60
Thr Phe Lys Asn Tyr Ile Lys Arg Asn Trp Arg Ile Val Glu Asp Glu
65 70 75 80
Pro Asn Lys Ile Cys Glu Ala Asp Arg Val Ala Ile Lys Ala Asn Ile
85 90 95
Val His Leu Met Leu Ser Ser Pro Glu Gln Ile Gln Lys Gln Leu Ser
100 105 110
Asp Ala Ile Ser Ile Ile Gly Arg Glu Asp Phe Pro Gln Lys Trp Pro
115 120 125
Asp Leu Leu Thr Glu Met Val Asn Arg Phe Gln Ser Gly Asp Phe His
130 135 140
Val Ile Asn Gly Val Leu Arg Thr Ala His Ser Leu Phe Lys Arg Tyr
145 150 155 160
Arg His Glu Phe Lys Ser Asn Glu Leu Trp Thr Glu Ile Lys Leu Val
165 170 175
Leu Asp Ala Phe Ala Leu Pro Leu Thr Asn Leu Phe Lys Ala Thr Ile
180 185 190
Glu Leu Cys Ser Thr His Ala Asn Asp Ala Ser Ala Leu Arg Ile Leu
195 200 205
Phe Ser Ser Leu Ile Leu Ile Ser Lys Leu Phe Tyr Ser Leu Asn Phe
210 215 220
Gln Asp Leu Pro Glu Phe Phe Glu Asp Asn Met Glu Thr Trp Met Asn
225 230 235 240
Asn Phe His Thr Leu Leu Thr Leu Asp Asn Lys Leu Leu Gln Thr Asp

```
                245                 250                 255
Asp Glu Glu Glu Ala Gly Leu Leu Glu Leu Leu Lys Ser Gln Ile Cys
            260                 265                 270
Asp Asn Ala Ala Leu Tyr Ala Gln Lys Tyr Asp Glu Glu Phe Gln Arg
        275                 280                 285
Tyr Leu Pro Arg Phe Val Thr Ala Ile Trp Asn Leu Leu Val Thr Thr
    290                 295                 300
Gly Gln Glu Val Lys Tyr Asp Leu Leu Val Ser Asn Ala Ile Gln Phe
305                 310                 315                 320
Leu Ala Ser Val Cys Glu Arg Pro His Tyr Lys Asn Leu Phe Glu Asp
                325                 330                 335
Gln Asn Thr Leu Thr Ser Ile Cys Glu Lys Val Ile Val Pro Asn Met
            340                 345                 350
Glu Phe Arg Ala Ala Asp Glu Glu Ala Phe Glu Asp Asn Ser Glu Glu
        355                 360                 365
Tyr Ile Arg Arg Asp Leu Glu Gly Ser Asp Ile Asp Thr Arg Arg Arg
    370                 375                 380
Ala Ala Cys Asp Leu Val Arg Gly Leu Cys Lys Phe Phe Glu Gly Pro
385                 390                 395                 400
Val Thr Gly Ile Phe Ser Gly Tyr Val Asn Ser Met Leu Gln Glu Tyr
                405                 410                 415
Ala Lys Asn Pro Ser Val Asn Trp Lys His Lys Asp Ala Ala Ile Tyr
            420                 425                 430
Leu Val Thr Ser Leu Ala Ser Lys Ala Gln Thr Gln Lys His Gly Ile
        435                 440                 445
Thr Gln Ala Asn Glu Leu Val Asn Leu Thr Glu Phe Phe Val Asn His
    450                 455                 460
Ile Leu Pro Asp Leu Lys Ser Ala Asn Val Asn Glu Phe Pro Val Leu
465                 470                 475                 480
Lys Ala Asp Gly Ile Lys Tyr Ile Met Ile Phe Arg Asn Gln Val Pro
                485                 490                 495
Lys Glu His Leu Leu Val Ser Ile Pro Leu Leu Ile Asn His Leu Gln
            500                 505                 510
Ala Glu Ser Ile Val Val His Thr Tyr Ala Ala His Ala Leu Glu Arg
        515                 520                 525
Leu Phe Thr Met Arg Gly Pro Asn Asn Ala Thr Leu Phe Thr Ala Ala
    530                 535                 540
Glu Ile Ala Pro Phe Val Glu Ile Leu Leu Thr Asn Leu Phe Lys Ala
545                 550                 555                 560
Leu Thr Leu Pro Gly Ser Ser Glu Asn Glu Tyr Ile Met Lys Ala Ile
                565                 570                 575
Met Arg Ser Phe Ser Leu Leu Gln Glu Ala Ile Ile Pro Tyr Ile Pro
            580                 585                 590
Thr Leu Ile Thr Gln Leu Thr Gln Lys Leu Leu Ala Val Ser Lys Asn
        595                 600                 605
Pro Ser Lys Pro His Phe Asn His Tyr Met Phe Glu Ala Ile Cys Leu
    610                 615                 620
Ser Ile Arg Ile Thr Cys Lys Ala Asn Pro Ala Ala Val Val Asn Phe
625                 630                 635                 640
Glu Glu Ala Leu Phe Leu Val Phe Thr Glu Ile Leu Gln Asn Asp Val
                645                 650                 655
Gln Glu Phe Ile Pro Tyr Val Phe Gln Val Met Ser Leu Leu Leu Glu
            660                 665                 670
Thr His Lys Asn Asp Ile Pro Ser Ser Tyr Met Ala Leu Phe Pro His
        675                 680                 685
Leu Leu Gln Pro Val Leu Trp Glu Arg Thr Gly Asn Ile Pro Ala Leu
    690                 695                 700
Val Arg Leu Leu Gln Ala Phe Leu Glu Arg Gly Ser Asn Thr Ile Ala
705                 710                 715                 720
Ser Ala Ala Ala Asp Lys Ile Pro Gly Leu Leu Gly Val Phe Gln Lys
                725                 730                 735
Leu Ile Ala Ser Lys Ala Asn Asp His Gln Gly Phe Tyr Leu Leu Asn
            740                 745                 750
Ser Ile Ile Glu His Met Pro Pro Glu Ser Val Asp Gln Tyr Arg Lys
        755                 760                 765
Gln Ile Phe Ile Leu Leu Phe Gln Arg Leu Gln Asn Ser Lys Thr Thr
    770                 775                 780
Lys Phe Ile Lys Ser Phe Leu Val Phe Ile Asn Leu Tyr Cys Ile Lys
785                 790                 795                 800
```

Tyr Gly Ala Leu Ala Leu Gln Glu Ile Phe Asp Gly Ile Gln Pro Lys
805 810 815
Met Phe Gly Met Val Leu Glu Lys Ile Ile Ile Pro Glu Ile Gln Lys
820 825 830
Val Ser Gly Asn Val Glu Lys Lys Ile Cys Ala Val Gly Ile Thr Lys
835 840 845
Leu Leu Thr Glu Cys Pro Pro Met Met Asp Thr Glu Tyr Thr Lys Leu
850 855 860
Trp Thr Pro Leu Leu Gln Ser Leu Ile Gly Leu Phe Glu Leu Pro Glu
865 870 875 880
Asp Asp Thr Ile Pro Asp Glu Glu His Phe Ile Asp Ile Glu Asp Thr
885 890 895
Pro Gly Tyr Gln Thr Ala Phe Ser Gln Leu Ala Phe Ala Gly Lys Lys
900 905 910
Glu His Asp Pro Val Gly Gln Met Val Asn Asn Pro Lys Ile His Leu
915 920 925
Ala Gln Ser Leu His Lys Leu Ser Thr Ala Cys Pro Gly Arg Val Pro
930 935 940
Ser Met Val Ser Thr Ser Leu Asn Ala Glu Ala Leu Gln Tyr Leu Gln
945 950 955 960
Gly Tyr Leu Gln Ala Ala Ser Val Thr Leu Leu
965 970

<210> 171
<211> 184
<212> PRT
<213> Homo sapiens
<400> 171
Met Pro Gln Ser Lys Ser Arg Lys Ile Ala Ile Leu Gly Tyr Arg Ser
1 5 10 15
Val Gly Lys Ser Ser Leu Thr Ile Gln Phe Val Glu Gly Gln Phe Val
20 25 30
Asp Ser Tyr Asp Pro Thr Ile Glu Asn Thr Phe Thr Lys Leu Ile Thr
35 40 45
Val Asn Gly Gln Glu Tyr His Leu Gln Leu Val Asp Thr Ala Gly Gln
50 55 60
Asp Glu Tyr Ser Ile Phe Pro Gln Thr Tyr Ser Ile Asp Ile Asn Gly
65 70 75 80
Tyr Ile Leu Val Tyr Ser Val Thr Ser Ile Lys Ser Phe Glu Val Ile
85 90 95
Lys Val Ile His Gly Lys Leu Leu Asp Met Val Gly Lys Val Gln Ile
100 105 110
Pro Ile Met Leu Val Gly Asn Lys Lys Asp Leu His Met Glu Arg Val
115 120 125
Ile Ser Tyr Glu Glu Gly Lys Ala Leu Ala Glu Ser Trp Asn Ala Ala
130 135 140
Phe Leu Glu Ser Ser Ala Lys Glu Asn Gln Thr Ala Val Asp Val Phe
145 150 155 160
Arg Arg Ile Ile Leu Glu Ala Glu Lys Met Asp Gly Ala Ala Ser Gln
165 170 175
Gly Lys Ser Ser Cys Ser Val Met
180

<210> 172
<211> 514
<212> PRT
<213> Homo sapiens
<400> 172
Met Ala Asp Ala Glu Val Ile Ile Leu Pro Lys Lys His Lys Lys Lys
1 5 10 15
Lys Glu Arg Lys Ser Leu Pro Glu Glu Asp Val Ala Glu Ile Gln His
20 25 30
Ala Glu Glu Phe Leu Ile Lys Pro Glu Ser Lys Val Ala Lys Leu Asp
35 40 45
Thr Ser Gln Trp Pro Leu Leu Leu Lys Asn Phe Asp Lys Leu Asn Val
50 55 60
Arg Thr Thr His Tyr Thr Pro Leu Ala Cys Gly Ser Asn Pro Leu Lys
65 70 75 80
Arg Glu Ile Gly Asp Tyr Ile Arg Thr Gly Phe Ile Asn Leu Asp Lys

85 90 95
Pro Ser Asn Pro Ser Ser His Glu Val Val Ala Trp Ile Arg Arg Ile
100 105 110
Leu Arg Val Glu Lys Thr Gly His Ser Gly Thr Leu Asp Pro Lys Val
115 120 125
Thr Gly Cys Leu Ile Val Cys Ile Glu Arg Ala Thr Arg Leu Val Lys
130 135 140
Ser Gln Gln Ser Ala Gly Lys Glu Tyr Val Gly Ile Val Arg Leu His
145 150 155 160
Asn Ala Ile Glu Gly Gly Thr Gln Leu Ser Arg Ala Leu Glu Thr Leu
165 170 175
Thr Gly Ala Leu Phe Gln Arg Pro Pro Leu Ile Ala Ala Val Lys Arg
180 185 190
Gln Leu Arg Val Arg Thr Ile Tyr Glu Ser Lys Met Ile Glu Tyr Asp
195 200 205
Pro Glu Arg Arg Leu Gly Ile Phe Trp Val Ser Cys Glu Ala Gly Thr
210 215 220
Tyr Ile Arg Thr Leu Cys Val His Leu Gly Leu Leu Leu Gly Val Gly
225 230 235 240
Gly Gln Met Gln Glu Leu Arg Arg Val Arg Ser Gly Val Met Ser Glu
245 250 255
Lys Asp His Met Val Thr Met His Asp Val Leu Asp Ala Gln Trp Leu
260 265 270
Tyr Asp Asn His Lys Asp Glu Ser Tyr Leu Arg Arg Val Val Tyr Pro
275 280 285
Leu Glu Lys Leu Leu Thr Ser His Lys Arg Leu Val Met Lys Asp Ser
290 295 300
Ala Val Asn Ala Ile Cys Tyr Gly Ala Lys Ile Met Leu Pro Gly Val
305 310 315 320
Leu Arg Tyr Glu Asp Gly Ile Glu Val Asn Gln Glu Ile Val Val Ile
325 330 335
Thr Thr Lys Gly Glu Ala Ile Cys Met Ala Ile Ala Leu Met Thr Thr
340 345 350
Ala Val Ile Ser Thr Cys Asp His Gly Ile Val Ala Lys Ile Lys Arg
355 360 365
Val Ile Met Glu Arg Asp Thr Tyr Pro Arg Lys Trp Gly Leu Gly Pro
370 375 380
Lys Ala Ser Gln Lys Lys Leu Met Ile Lys Gln Gly Leu Leu Asp Lys
385 390 395 400
His Gly Lys Pro Thr Asp Ser Thr Pro Ala Thr Trp Lys Gln Glu Tyr
405 410 415
Val Asp Tyr Ser Glu Ser Ala Lys Lys Glu Val Val Ala Glu Val Val
420 425 430
Lys Ala Pro Gln Val Val Ala Glu Ala Ala Lys Thr Ala Lys Arg Lys
435 440 445
Arg Glu Ser Glu Ser Glu Ser Asp Glu Thr Pro Pro Ala Ala Pro Gln
450 455 460
Leu Ile Lys Lys Glu Lys Lys Lys Ser Lys Lys Asp Lys Lys Ala Lys
465 470 475 480
Ala Gly Leu Glu Ser Gly Ala Glu Pro Gly Asp Gly Asp Ser Asp Thr
485 490 495
Thr Lys Lys Lys Lys Lys Lys Lys Lys Ala Lys Glu Val Glu Leu Val
500 505 510
Ser Glu

<210> 173
<211> 258
<212> PRT
<213> Homo sapiens
<400> 173
Met Leu Pro Leu Cys Leu Val Ala Ala Leu Leu Leu Ala Ala Gly Pro
1 5 10 15
Gly Pro Ser Leu Gly Asp Glu Ala Ile His Cys Pro Pro Cys Ser Glu
20 25 30
Glu Lys Leu Ala Arg Cys Arg Pro Pro Val Gly Cys Glu Glu Leu Val
35 40 45
Arg Glu Ala Gly Cys Gly Cys Cys Ala Thr Cys Ala Leu Gly Leu Gly
50 55 60

Met Pro Cys Gly Val Tyr Thr Pro Arg Cys Gly Ser Gly Leu Arg Cys
65 70 75 80
Tyr Pro Pro Arg Gly Val Glu Lys Pro Leu His Thr Leu Met His Gly
85 90 95
Gln Gly Val Cys Met Glu Leu Ala Glu Ile Glu Ala Ile Gln Glu Ser
100 105 110
Leu Gln Pro Ser Asp Lys Asp Glu Gly Asp His Pro Asn Asn Ser Phe
115 120 125
Ser Pro Cys Ser Ala His Asp Arg Arg Cys Leu Gln Lys His Phe Ala
130 135 140
Lys Ile Arg Asp Arg Ser Thr Ser Gly Gly Lys Met Lys Val Asn Gly
145 150 155 160
Ala Pro Arg Glu Asp Ala Arg Pro Val Pro Gln Gly Ser Cys Gln Ser
165 170 175
Glu Leu His Arg Ala Leu Glu Arg Leu Ala Ala Ser Gln Ser Arg Thr
180 185 190
His Glu Asp Leu Tyr Phe Ile Pro Ile Pro Asn Cys Asp Arg Asn Gly
195 200 205
Asn Phe His Pro Lys Gln Cys His Pro Ala Leu Asp Gly Gln Arg Gly
210 215 220
Lys Cys Trp Cys Val Asp Arg Lys Thr Gly Val Lys Leu Pro Gly Gly
225 230 235 240
Leu Glu Pro Lys Gly Glu Leu Asp Cys His Gln Leu Ala Asp Ser Phe
245 250 255
Arg Glu

<210> 174
<211> 1233
<212> PRT
<213> Homo sapiens
<400> 174
Met Gly Phe Leu Lys Leu Ile Glu Ile Glu Asn Phe Lys Ser Tyr Lys
1 5 10 15
Gly Arg Gln Ile Ile Gly Pro Phe Gln Arg Phe Thr Ala Ile Ile Gly
20 25 30
Pro Asn Gly Ser Gly Lys Ser Asn Leu Met Asp Ala Ile Ser Phe Val
35 40 45
Leu Gly Glu Lys Thr Ser Asn Leu Arg Val Lys Thr Leu Arg Asp Leu
50 55 60
Ile His Gly Ala Pro Val Gly Lys Pro Ala Ala Asn Arg Ala Phe Val
65 70 75 80
Ser Met Val Tyr Ser Glu Glu Gly Ala Glu Asp Arg Thr Phe Ala Arg
85 90 95
Val Ile Val Gly Gly Ser Ser Glu Tyr Lys Ile Asn Asn Lys Val Val
100 105 110
Gln Leu His Glu Tyr Ser Glu Glu Leu Glu Lys Leu Gly Ile Leu Ile
115 120 125
Lys Ala Arg Asn Phe Leu Val Phe Gln Gly Ala Val Glu Ser Ile Ala
130 135 140
Met Lys Asn Pro Lys Glu Arg Thr Ala Leu Phe Glu Glu Ile Ser Arg
145 150 155 160
Ser Gly Glu Leu Ala Gln Glu Tyr Asp Lys Arg Lys Lys Glu Met Val
165 170 175
Lys Ala Glu Glu Asp Thr Gln Phe Asn Tyr His Arg Lys Lys Asn Ile
180 185 190
Ala Ala Glu Arg Lys Glu Ala Lys Gln Glu Lys Glu Glu Ala Asp Arg
195 200 205
Tyr Gln Arg Leu Lys Asp Glu Val Val Arg Ala Gln Val Gln Leu Gln
210 215 220
Leu Phe Lys Leu Tyr His Asn Glu Val Glu Ile Glu Lys Leu Asn Lys
225 230 235 240
Glu Leu Ala Ser Lys Asn Lys Glu Ile Glu Lys Asp Lys Lys Arg Met
245 250 255
Asp Lys Val Glu Asp Glu Leu Lys Glu Lys Lys Lys Glu Leu Gly Lys
260 265 270
Met Met Arg Glu Gln Gln Gln Ile Glu Lys Glu Ile Lys Glu Lys Asp
275 280 285
Ser Glu Leu Asn Gln Lys Arg Pro Gln Tyr Ile Lys Ala Lys Glu Asn

```
    290                 295                 300
Thr Ser His Lys Ile Lys Lys Leu Glu Ala Ala Lys Lys Ser Leu Gln
305                 310                 315                 320
Asn Ala Gln Lys His Tyr Lys Lys Arg Lys Gly Asp Met Asp Glu Leu
                325                 330                 335
Glu Lys Glu Met Leu Ser Val Glu Lys Ala Arg Gln Glu Phe Glu Glu
            340                 345                 350
Arg Met Glu Glu Glu Ser Gln Ser Gln Gly Arg Asp Leu Thr Leu Glu
        355                 360                 365
Glu Asn Gln Val Lys Lys Tyr His Arg Leu Lys Glu Glu Ala Ser Lys
    370                 375                 380
Arg Ala Ala Thr Leu Ala Gln Glu Leu Glu Lys Phe Asn Arg Asp Gln
385                 390                 395                 400
Lys Ala Asp Gln Asp Arg Leu Asp Leu Glu Glu Arg Lys Lys Val Glu
                405                 410                 415
Thr Glu Ala Lys Ile Lys Gln Lys Leu Arg Glu Ile Glu Glu Asn Gln
            420                 425                 430
Lys Arg Ile Glu Lys Leu Glu Glu Tyr Ile Thr Thr Ser Lys Gln Ser
        435                 440                 445
Leu Glu Glu Gln Lys Lys Leu Glu Gly Glu Leu Thr Glu Glu Val Glu
    450                 455                 460
Met Ala Lys Arg Arg Ile Asp Glu Ile Asn Lys Glu Leu Asn Gln Val
465                 470                 475                 480
Met Glu Gln Leu Gly Asp Ala Arg Ile Asp Arg Gln Glu Ser Ser Arg
                485                 490                 495
Gln Gln Arg Lys Ala Glu Ile Met Glu Ser Ile Lys Arg Leu Tyr Pro
            500                 505                 510
Gly Ser Val Tyr Gly Arg Leu Ile Asp Leu Cys Gln Pro Thr Gln Lys
        515                 520                 525
Lys Tyr Gln Ile Ala Val Thr Lys Val Leu Gly Lys Asn Met Asp Ala
    530                 535                 540
Ile Ile Val Asp Ser Glu Lys Thr Gly Arg Asp Cys Ile Gln Tyr Ile
545                 550                 555                 560
Lys Glu Gln Arg Gly Glu Pro Glu Thr Phe Leu Pro Leu Asp Tyr Leu
                565                 570                 575
Glu Val Lys Pro Thr Asp Glu Lys Leu Arg Glu Leu Lys Gly Ala Lys
            580                 585                 590
Leu Val Ile Asp Val Ile Arg Tyr Glu Pro Pro His Ile Lys Lys Ala
        595                 600                 605
Leu Gln Tyr Ala Cys Gly Asn Ala Leu Val Cys Asp Asn Val Glu Asp
    610                 615                 620
Ala Arg Arg Ile Ala Phe Gly Gly His Gln Arg His Lys Thr Val Ala
625                 630                 635                 640
Leu Asp Gly Thr Leu Phe Gln Lys Ser Gly Val Ile Ser Gly Gly Ala
                645                 650                 655
Ser Asp Leu Lys Ala Lys Ala Arg Arg Trp Asp Glu Lys Ala Val Asp
            660                 665                 670
Lys Leu Lys Glu Lys Lys Glu Arg Leu Thr Glu Glu Leu Lys Glu Gln
        675                 680                 685
Met Lys Ala Lys Arg Lys Glu Ala Glu Leu Arg Gln Val Gln Ser Gln
    690                 695                 700
Ala His Gly Leu Gln Met Arg Leu Lys Tyr Ser Gln Ser Asp Leu Glu
705                 710                 715                 720
Gln Thr Lys Thr Arg His Leu Ala Leu Asn Leu Gln Glu Lys Ser Lys
                725                 730                 735
Leu Glu Ser Glu Leu Ala Asn Phe Gly Pro Arg Ile Asn Asp Ile Lys
            740                 745                 750
Arg Ile Ile Gln Ser Arg Glu Arg Glu Met Lys Asp Leu Lys Glu Lys
        755                 760                 765
Met Asn Gln Val Glu Asp Glu Val Phe Glu Glu Phe Cys Arg Glu Ile
    770                 775                 780
Gly Val Arg Asn Ile Arg Glu Phe Glu Glu Glu Lys Val Lys Arg Gln
785                 790                 795                 800
Asn Glu Ile Ala Lys Lys Arg Leu Glu Phe Glu Asn Gln Lys Thr Arg
                805                 810                 815
Leu Gly Ile Gln Leu Asp Phe Glu Lys Asn Gln Leu Lys Glu Asp Gln
            820                 825                 830
Asp Lys Val His Met Trp Glu Gln Thr Val Lys Lys Asp Glu Asn Glu
        835                 840                 845
```

Ile Glu Lys Leu Lys Lys Glu Glu Gln Arg His Met Lys Ile Ile Asp
850 855 860
Glu Thr Met Ala Gln Leu Gln Asp Leu Lys Asn Gln His Leu Ala Lys
865 870 875 880
Lys Ser Glu Val Asn Asp Lys Asn His Glu Met Glu Glu Ile Arg Lys
885 890 895
Lys Leu Gly Gly Ala Asn Lys Glu Met Thr His Leu Gln Lys Glu Val
900 905 910
Thr Ala Ile Glu Thr Lys Leu Glu Gln Lys Arg Ser Asp Arg His Asn
915 920 925
Leu Leu Gln Ala Cys Lys Met Gln Asp Ile Lys Leu Pro Leu Ser Lys
930 935 940
Gly Thr Met Asp Asp Ile Ser Gln Glu Glu Gly Ser Ser Gln Gly Glu
945 950 955 960
Asp Ser Val Ser Gly Ser Gln Arg Ile Ser Ser Ile Tyr Ala Arg Glu
965 970 975
Ala Leu Ile Glu Ile Asp Tyr Gly Asp Leu Cys Glu Asp Leu Lys Asp
980 985 990
Ala Gln Ala Glu Glu Glu Ile Lys Gln Glu Met Asn Thr Leu Gln Gln
995 1000 1005
Lys Leu Asn Glu Gln Gln Ser Val Leu Gln Arg Ile Ala Ala Pro
1010 1015 1020
Asn Met Lys Ala Met Glu Lys Leu Glu Ser Val Arg Asp Lys Phe
1025 1030 1035
Gln Glu Thr Ser Asp Glu Phe Glu Ala Ala Arg Lys Arg Ala Lys
1040 1045 1050
Lys Ala Lys Gln Ala Phe Glu Gln Ile Lys Lys Glu Arg Phe Asp
1055 1060 1065
Arg Phe Asn Ala Cys Phe Glu Ser Val Ala Thr Asn Ile Asp Glu
1070 1075 1080
Ile Tyr Lys Ala Leu Ser Arg Asn Ser Ser Ala Gln Ala Phe Leu
1085 1090 1095
Gly Pro Glu Asn Pro Glu Glu Pro Tyr Leu Asp Gly Ile Asn Tyr
1100 1105 1110
Asn Cys Val Ala Pro Gly Lys Arg Phe Arg Pro Met Asp Asn Leu
1115 1120 1125
Ser Gly Gly Glu Lys Thr Val Ala Ala Leu Ala Leu Leu Phe Ala
1130 1135 1140
Ile His Ser Tyr Lys Pro Ala Pro Phe Phe Val Leu Asp Glu Ile
1145 1150 1155
Asp Ala Ala Leu Asp Asn Thr Asn Ile Gly Lys Val Ala Asn Tyr
1160 1165 1170
Ile Lys Glu Gln Ser Thr Cys Asn Phe Gln Ala Ile Val Ile Ser
1175 1180 1185
Leu Lys Glu Glu Phe Tyr Thr Lys Ala Glu Ser Leu Ile Gly Val
1190 1195 1200
Tyr Pro Glu Gln Gly Asp Cys Val Ile Ser Lys Val Leu Thr Phe
1205 1210 1215
Asp Leu Thr Lys Tyr Pro Asp Ala Asn Pro Asn Pro Asn Glu Gln
1220 1225 1230

<210> 175
<211> 501
<212> PRT
<213> Homo sapiens
<400> 175
Met Asn Arg Ser Arg Gln Val Thr Cys Val Ala Trp Val Arg Cys Gly
1 5 10 15
Val Ala Lys Glu Thr Pro Asp Lys Val Glu Leu Ser Lys Glu Glu Val
20 25 30
Lys Arg Leu Ile Ala Glu Ala Lys Glu Lys Leu Gln Glu Glu Gly Gly
35 40 45
Gly Ser Asp Glu Glu Glu Thr Gly Ser Pro Ser Glu Asp Gly Met Gln
50 55 60
Ser Ala Arg Thr Gln Ala Arg Pro Arg Glu Pro Leu Glu Asp Gly Asp
65 70 75 80
Pro Glu Asp Asp Arg Thr Leu Asp Asp Asp Glu Leu Ala Glu Tyr Asp
85 90 95
Leu Asp Lys Tyr Asp Glu Glu Gly Asp Pro Asp Ala Glu Thr Leu Gly

100 105 110
Glu Ser Leu Leu Gly Leu Thr Val Tyr Gly Ser Asn Asp Gln Asp Pro
115 120 125
Tyr Val Thr Leu Lys Asp Thr Glu Gln Tyr Glu Arg Glu Asp Phe Leu
130 135 140
Ile Lys Pro Ser Asp Asn Leu Ile Val Cys Gly Arg Ala Glu Gln Asp
145 150 155 160
Gln Cys Asn Leu Glu Val His Val Tyr Asn Gln Glu Glu Asp Ser Phe
165 170 175
Tyr Val His His Asp Ile Leu Leu Ser Ala Tyr Pro Leu Ser Val Glu
180 185 190
Trp Leu Asn Phe Asp Pro Ser Pro Asp Asp Ser Thr Gly Asn Tyr Ile
195 200 205
Ala Val Gly Asn Met Thr Pro Val Ile Glu Val Trp Asp Leu Asp Ile
210 215 220
Val Asp Ser Leu Glu Pro Val Phe Thr Leu Gly Ser Lys Leu Ser Lys
225 230 235 240
Lys Lys Lys Lys Lys Gly Lys Lys Ser Ser Ser Ala Glu Gly His Thr
245 250 255
Asp Ala Val Leu Asp Leu Ser Trp Asn Lys Leu Ile Arg Asn Val Leu
260 265 270
Ala Ser Ala Ser Ala Asp Asn Thr Val Ile Leu Trp Asp Met Ser Leu
275 280 285
Gly Lys Pro Ala Ala Ser Leu Ala Val His Thr Asp Lys Val Gln Thr
290 295 300
Leu Gln Phe His Pro Phe Glu Ala Gln Thr Leu Ile Ser Gly Ser Tyr
305 310 315 320
Asp Lys Ser Val Ala Leu Tyr Asp Cys Arg Ser Pro Asp Glu Ser His
325 330 335
Arg Met Trp Arg Phe Ser Gly Gln Ile Glu Arg Val Thr Trp Asn His
340 345 350
Phe Ser Pro Cys His Phe Leu Ala Ser Thr Asp Asp Gly Phe Val Tyr
355 360 365
Asn Leu Asp Ala Arg Ser Asp Lys Pro Ile Phe Thr Leu Asn Ala His
370 375 380
Asn Asp Glu Ile Ser Gly Leu Asp Leu Ser Ser Gln Ile Lys Gly Cys
385 390 395 400
Leu Val Thr Ala Ser Ala Asp Lys Tyr Val Lys Ile Trp Asp Ile Leu
405 410 415
Gly Asp Arg Pro Ser Leu Val His Ser Arg Asp Met Lys Met Gly Val
420 425 430
Leu Phe Cys Ser Ser Cys Cys Pro Asp Leu Pro Phe Ile Tyr Ala Phe
435 440 445
Gly Gly Gln Lys Glu Gly Leu Arg Val Trp Asp Ile Ser Thr Val Ser
450 455 460
Ser Val Asn Glu Ala Phe Gly Arg Arg Glu Arg Leu Val Leu Gly Ser
465 470 475 480
Ala Arg Asn Ser Ser Ile Ser Gly Pro Phe Gly Ser Arg Ser Ser Asp
485 490 495
Thr Pro Met Glu Ser
500

<210> 176
<211> 488
<212> PRT
<213> Homo sapiens
<400> 176
Met Ala Tyr Ser Gln Gly Gly Gly Lys Lys Lys Val Cys Tyr Tyr Tyr
1 5 10 15
Asp Gly Asp Ile Gly Asn Tyr Tyr Tyr Gly Gln Gly His Pro Met Lys
20 25 30
Pro His Arg Ile Arg Met Thr His Asn Leu Leu Leu Asn Tyr Gly Leu
35 40 45
Tyr Arg Lys Met Glu Ile Tyr Arg Pro His Lys Ala Thr Ala Glu Glu
50 55 60
Met Thr Lys Tyr His Ser Asp Glu Tyr Ile Lys Phe Leu Arg Ser Ile
65 70 75 80
Arg Pro Asp Asn Met Ser Glu Tyr Ser Lys Gln Met His Ile Phe Asn
85 90 95

Val Gly Glu Asp Cys Pro Ala Phe Asp Gly Leu Phe Glu Phe Cys Gln
100 105 110
Leu Ser Thr Gly Gly Ser Val Ala Gly Ala Val Lys Leu Asn Arg Gln
115 120 125
Gln Thr Asp Met Ala Val Asn Trp Ala Gly Gly Leu His His Ala Lys
130 135 140
Lys Tyr Glu Ala Ser Gly Phe Cys Tyr Val Asn Asp Ile Val Leu Ala
145 150 155 160
Ile Leu Glu Leu Leu Lys Tyr His Gln Arg Val Leu Tyr Ile Asp Ile
165 170 175
Asp Ile His His Gly Asp Gly Val Glu Glu Ala Phe Tyr Thr Thr Asp
180 185 190
Arg Val Met Thr Val Ser Phe His Lys Tyr Gly Glu Tyr Phe Pro Gly
195 200 205
Thr Gly Asp Leu Arg Asp Ile Gly Ala Gly Lys Gly Lys Tyr Tyr Ala
210 215 220
Val Asn Phe Pro Met Cys Asp Gly Ile Asp Asp Glu Ser Tyr Gly Gln
225 230 235 240
Ile Phe Lys Pro Ile Ile Ser Lys Val Met Glu Met Tyr Gln Pro Ser
245 250 255
Ala Val Val Leu Gln Cys Gly Ala Asp Ser Leu Ser Gly Asp Arg Leu
260 265 270
Gly Cys Phe Asn Leu Thr Val Lys Gly His Ala Lys Cys Val Glu Val
275 280 285
Val Lys Thr Phe Asn Leu Pro Leu Leu Met Leu Gly Gly Gly Gly Tyr
290 295 300
Thr Ile Arg Asn Val Ala Arg Cys Trp Thr Tyr Glu Thr Ala Val Ala
305 310 315 320
Leu Asp Cys Glu Ile Pro Asn Glu Leu Pro Tyr Asn Asp Tyr Phe Glu
325 330 335
Tyr Phe Gly Pro Asp Phe Lys Leu His Ile Ser Pro Ser Asn Met Thr
340 345 350
Asn Gln Asn Thr Pro Glu Tyr Met Glu Lys Ile Lys Gln Arg Leu Phe
355 360 365
Glu Asn Leu Arg Met Leu Pro His Ala Pro Gly Val Gln Met Gln Ala
370 375 380
Ile Pro Glu Asp Ala Val His Glu Asp Ser Gly Asp Glu Asp Gly Glu
385 390 395 400
Asp Pro Asp Lys Arg Ile Ser Ile Arg Ala Ser Asp Lys Arg Ile Ala
405 410 415
Cys Asp Glu Glu Phe Ser Asp Ser Glu Asp Glu Gly Glu Gly Gly Arg
420 425 430
Arg Asn Val Ala Asp His Lys Lys Gly Ala Lys Lys Ala Arg Ile Glu
435 440 445
Glu Asp Lys Lys Glu Thr Glu Asp Lys Lys Thr Asp Val Lys Glu Glu
450 455 460
Asp Lys Ser Lys Asp Asn Ser Gly Glu Lys Thr Asp Thr Lys Gly Thr
465 470 475 480
Lys Ser Glu Gln Leu Ser Asn Pro
485

<210> 177
<211> 270
<212> PRT
<213> Homo sapiens
<400> 177
Met Gly Asn Thr Ser Ser Glu Arg Ala Ala Leu Glu Arg His Gly Gly
1 5 10 15
His Lys Thr Pro Arg Arg Asp Ser Ser Gly Gly Thr Lys Asp Gly Asp
20 25 30
Arg Pro Lys Ile Leu Met Asp Ser Pro Glu Asp Ala Asp Leu Phe His
35 40 45
Ser Glu Glu Ile Lys Ala Pro Glu Lys Glu Glu Phe Leu Ala Trp Gln
50 55 60
His Asp Leu Glu Val Asn Asp Lys Ala Pro Ala Gln Ala Arg Pro Thr
65 70 75 80
Val Phe Arg Trp Thr Gly Gly Gly Lys Glu Val Tyr Leu Ser Gly Ser
85 90 95
Phe Asn Asn Trp Ser Lys Leu Pro Leu Thr Arg Ser His Asn Asn Phe

```
            100                 105                 110
Val Ala Ile Leu Asp Leu Pro Glu Gly Glu His Gln Tyr Lys Phe Phe
        115                 120                 125
Val Asp Gly Gln Trp Thr His Asp Pro Ser Glu Pro Ile Val Thr Ser
    130                 135                 140
Gln Leu Gly Thr Val Asn Asn Ile Ile Gln Val Lys Lys Thr Asp Phe
145                 150                 155                 160
Glu Val Phe Asp Ala Leu Met Val Asp Ser Gln Lys Cys Ser Asp Val
                165                 170                 175
Ser Glu Leu Ser Ser Ser Pro Pro Gly Pro Tyr His Gln Glu Pro Tyr
            180                 185                 190
Val Cys Lys Pro Glu Glu Arg Phe Arg Ala Pro Pro Ile Leu Pro Pro
        195                 200                 205
His Leu Leu Gln Val Ile Leu Asn Lys Asp Thr Gly Ile Ser Cys Asp
    210                 215                 220
Pro Ala Leu Leu Pro Glu Pro Asn His Val Met Leu Asn His Leu Tyr
225                 230                 235                 240
Ala Leu Ser Ile Lys Asp Gly Val Met Val Leu Ser Ala Thr His Arg
                245                 250                 255
Tyr Lys Lys Lys Tyr Val Thr Thr Leu Leu Tyr Lys Pro Ile
            260                 265                 270

<210>  178
<211>  514
<212>  PRT
<213>  Homo sapiens
<400>  178
Met Ala Asp Tyr Leu Ile Ser Gly Gly Thr Ser Tyr Val Pro Asp Asp
1               5                   10                  15
Gly Leu Thr Ala Gln Gln Leu Phe Asn Cys Gly Asp Gly Leu Thr Tyr
            20                  25                  30
Asn Asp Phe Leu Ile Leu Pro Gly Tyr Ile Asp Phe Thr Ala Asp Gln
        35                  40                  45
Val Asp Leu Thr Ser Ala Leu Thr Lys Lys Ile Thr Leu Lys Thr Pro
    50                  55                  60
Leu Val Ser Ser Pro Met Asp Thr Val Thr Glu Ala Gly Met Ala Ile
65                  70                  75                  80
Ala Met Ala Leu Thr Gly Gly Ile Gly Phe Ile His His Asn Cys Thr
                85                  90                  95
Pro Glu Phe Gln Ala Asn Glu Val Arg Lys Val Lys Lys Tyr Glu Gln
            100                 105                 110
Gly Phe Ile Thr Asp Pro Val Val Leu Ser Pro Lys Asp Arg Val Arg
        115                 120                 125
Asp Val Phe Glu Ala Lys Ala Arg His Gly Phe Cys Gly Ile Pro Ile
    130                 135                 140
Thr Asp Thr Gly Arg Met Gly Ser Arg Leu Val Gly Ile Ile Ser Ser
145                 150                 155                 160
Arg Asp Ile Asp Phe Leu Lys Glu Glu Glu His Asp Cys Phe Leu Glu
                165                 170                 175
Glu Ile Met Thr Lys Arg Glu Asp Leu Val Val Ala Pro Arg Ser Ile
            180                 185                 190
Thr Leu Lys Glu Ala Asn Glu Ile Leu Gln Arg Ser Lys Lys Gly Lys
        195                 200                 205
Leu Pro Ile Val Asn Glu Asp Asp Glu Leu Val Ala Ile Ile Ala Arg
    210                 215                 220
Thr Asp Leu Lys Lys Asn Arg Asp Tyr Pro Leu Ala Ser Lys Asp Ala
225                 230                 235                 240
Lys Lys Gln Leu Leu Cys Gly Ala Ala Ile Gly Thr His Glu Asp Asp
                245                 250                 255
Lys Tyr Arg Leu Asp Leu Leu Ala Gln Ala Gly Val Asp Val Val Val
            260                 265                 270
Leu Asp Ser Ser Gln Gly Asn Ser Ile Phe Gln Ile Asn Met Ile Lys
        275                 280                 285
Tyr Ile Lys Asp Lys Tyr Pro Asn Leu Gln Val Ile Gly Gly Asn Val
    290                 295                 300
Val Thr Ala Ala Gln Ala Lys Asn Leu Ile Asp Ala Gly Val Asp Ala
305                 310                 315                 320
Leu Arg Val Gly Met Gly Ser Gly Ser Ile Cys Ile Thr Gln Glu Val
                325                 330                 335
```

Leu Ala Cys Gly Arg Pro Gln Ala Thr Ala Val Tyr Lys Val Ser Glu
340 345 350
Tyr Ala Arg Arg Phe Gly Val Pro Val Ile Ala Asp Gly Gly Ile Gln
355 360 365
Asn Val Gly His Ile Ala Lys Ala Leu Ala Leu Gly Ala Ser Thr Val
370 375 380
Met Met Gly Ser Leu Leu Ala Ala Thr Thr Glu Ala Pro Gly Glu Tyr
385 390 395 400
Phe Phe Ser Asp Gly Ile Arg Leu Lys Lys Tyr Arg Gly Met Gly Ser
405 410 415
Leu Asp Ala Met Asp Lys His Leu Ser Ser Gln Asn Arg Tyr Phe Ser
420 425 430
Glu Ala Asp Lys Ile Lys Val Ala Gln Gly Val Ser Gly Ala Val Gln
435 440 445
Asp Lys Gly Ser Ile His Lys Phe Val Pro Tyr Leu Ile Ala Gly Ile
450 455 460
Gln His Ser Cys Gln Asp Ile Gly Ala Lys Ser Leu Thr Gln Val Arg
465 470 475 480
Ala Met Met Tyr Ser Gly Glu Leu Lys Phe Glu Lys Arg Thr Ser Ser
485 490 495
Ala Gln Val Glu Gly Gly Val His Ser Leu His Ser Tyr Glu Lys Arg
500 505 510
Leu Phe

<210> 179
<211> 152
<212> PRT
<213> Homo sapiens
<400> 179
Met Ser Thr Pro Ala Arg Arg Arg Leu Met Arg Asp Phe Lys Arg Leu
1 5 10 15
Gln Glu Asp Pro Pro Ala Gly Val Ser Gly Ala Pro Ser Glu Asn Asn
20 25 30
Ile Met Val Trp Asn Ala Val Ile Phe Gly Pro Glu Gly Thr Pro Phe
35 40 45
Glu Asp Gly Thr Phe Lys Leu Thr Ile Glu Phe Thr Glu Glu Tyr Pro
50 55 60
Asn Lys Pro Pro Thr Val Arg Phe Val Ser Lys Met Phe His Pro Asn
65 70 75 80
Val Tyr Ala Asp Gly Ser Ile Cys Leu Asp Ile Leu Gln Asn Arg Trp
85 90 95
Ser Pro Thr Tyr Asp Val Ser Ser Ile Leu Thr Ser Ile Gln Ser Leu
100 105 110
Leu Asp Glu Pro Asn Pro Asn Ser Pro Ala Asn Ser Gln Ala Ala Gln
115 120 125
Leu Tyr Gln Glu Asn Lys Arg Glu Tyr Glu Lys Arg Val Ser Ala Ile
130 135 140
Val Glu Gln Ser Trp Arg Asp Cys
145 150

<210> 180
<211> 260
<212> PRT
<213> Homo sapiens
<400> 180
Met Pro Gln Asn Glu Tyr Ile Glu Leu His Arg Lys Arg Tyr Gly Tyr
1 5 10 15
Arg Leu Asp Tyr His Glu Lys Lys Arg Lys Lys Glu Ser Arg Glu Ala
20 25 30
His Glu Arg Ser Lys Lys Ala Lys Lys Met Ile Gly Leu Lys Ala Lys
35 40 45
Leu Tyr His Lys Gln Arg His Ala Glu Lys Ile Gln Met Lys Lys Thr
50 55 60
Ile Lys Met His Glu Lys Arg Asn Thr Lys Gln Lys Asn Asp Glu Lys
65 70 75 80
Thr Pro Gln Gly Ala Val Pro Ala Tyr Leu Leu Asp Arg Glu Gly Gln
85 90 95
Ser Arg Ala Lys Val Leu Ser Asn Met Ile Lys Gln Lys Arg Lys Glu

```
            100                 105                 110
Lys Ala Gly Lys Trp Glu Val Pro Leu Pro Lys Val Arg Ala Gln Gly
        115                 120                 125
Glu Thr Glu Val Leu Lys Val Ile Arg Thr Gly Lys Arg Lys Lys Lys
    130                 135                 140
Ala Trp Lys Arg Met Val Thr Lys Val Cys Phe Val Gly Asp Gly Phe
145                 150                 155                 160
Thr Arg Lys Pro Pro Lys Tyr Glu Arg Phe Ile Arg Pro Met Gly Leu
                165                 170                 175
Arg Phe Lys Lys Ala His Val Thr His Pro Glu Leu Lys Ala Thr Phe
            180                 185                 190
Cys Leu Pro Ile Leu Gly Val Lys Lys Asn Pro Ser Ser Pro Leu Tyr
        195                 200                 205
Thr Thr Leu Gly Val Ile Thr Lys Gly Thr Val Ile Glu Val Asn Val
    210                 215                 220
Ser Glu Leu Gly Leu Val Thr Gln Gly Gly Lys Val Ile Trp Gly Lys
225                 230                 235                 240
Tyr Ala Gln Val Thr Asn Asn Pro Glu Asn Asp Gly Cys Ile Asn Ala
                245                 250                 255
Val Leu Leu Val
            260

<210>  181
<211>  790
<212>  PRT
<213>  Homo sapiens
<400>  181
Met Glu Ala Thr Ser Arg Glu Ala Ala Pro Ala Lys Ser Ser Ala Ser
1               5                   10                  15
Gly Pro Asn Ala Pro Pro Ala Leu Phe Glu Leu Cys Gly Arg Ala Val
            20                  25                  30
Ser Ala His Met Gly Val Leu Glu Ser Gly Val Trp Ala Leu Pro Gly
        35                  40                  45
Pro Ile Leu Gln Ser Ile Leu Pro Leu Leu Asn Ile Tyr Tyr Leu Glu
    50                  55                  60
Arg Ile Glu Glu Thr Ala Leu Lys Lys Gly Leu Ser Thr Gln Ala Ile
65                  70                  75                  80
Trp Arg Arg Leu Trp Asp Glu Leu Met Lys Thr Arg Pro Ser Ser Leu
                85                  90                  95
Glu Ser Val Thr Cys Trp Arg Ala Lys Phe Met Glu Ala Phe Phe Ser
            100                 105                 110
His Val Leu Arg Gly Thr Ile Asp Val Ser Ser Asp Arg Arg Leu Cys
        115                 120                 125
Asp Gln Arg Phe Ser Pro Leu Leu His Ser Ser Arg His Val Arg Gln
    130                 135                 140
Leu Thr Ile Cys Asn Met Leu Gln Gly Ala Thr Glu Leu Val Ala Glu
145                 150                 155                 160
Pro Asn Arg Arg Val Leu Glu Thr Leu Ala Ser Ser Leu His Thr Leu
                165                 170                 175
Lys Phe Arg His Leu Leu Phe Ser Asp Val Ala Ala Gln Gln Ser Leu
            180                 185                 190
Arg Gln Leu Leu His Gln Leu Ile His His Gly Ala Val Ser Gln Val
        195                 200                 205
Ser Leu Tyr Ser Trp Pro Val Pro Glu Ser Ala Leu Phe Ile Leu Ile
    210                 215                 220
Leu Thr Met Ser Ala Gly Phe Trp Gln Pro Gly Pro Gly Gly Pro Pro
225                 230                 235                 240
Cys Arg Leu Cys Gly Glu Ala Ser Arg Gly Arg Ala Pro Ser Arg Asp
                245                 250                 255
Glu Gly Ser Leu Leu Leu Gly Ser Arg Arg Pro Arg Arg Asp Ala Ala
            260                 265                 270
Glu Arg Cys Ala Ala Ala Leu Met Ala Ser Arg Arg Lys Ser Glu Ala
        275                 280                 285
Lys Gln Met Pro Arg Ala Ala Pro Ala Thr Arg Val Thr Arg Arg Ser
    290                 295                 300
Thr Gln Glu Ser Leu Thr Ala Gly Gly Thr Asp Leu Lys Arg Glu Leu
305                 310                 315                 320
His Pro Pro Ala Thr Ser His Glu Ala Pro Gly Thr Lys Arg Ser Pro
                325                 330                 335
```

Ser Ala Pro Ala Ala Thr Ser Ser Ala Ser Ser Ser Thr Ser Ser Tyr
340 345 350
Lys Arg Ala Pro Ala Ser Ser Ala Pro Gln Pro Lys Pro Leu Lys Arg
355 360 365
Phe Lys Arg Ala Ala Gly Lys Lys Gly Ala Arg Thr Arg Gln Gly Pro
370 375 380
Gly Ala Glu Ser Glu Asp Leu Tyr Asp Phe Val Phe Ile Val Ala Gly
385 390 395 400
Glu Lys Glu Asp Gly Glu Glu Met Glu Ile Gly Glu Val Ala Cys Gly
405 410 415
Ala Leu Asp Gly Ser Asp Pro Ser Cys Leu Gly Leu Pro Ala Leu Glu
420 425 430
Ala Ser Gln Arg Phe Arg Ser Ile Ser Thr Leu Glu Leu Phe Thr Val
435 440 445
Pro Leu Ser Thr Glu Ala Ala Leu Thr Leu Cys His Leu Leu Ser Ser
450 455 460
Trp Val Ser Leu Glu Ser Leu Thr Leu Ser Tyr Asn Gly Leu Gly Ser
465 470 475 480
Asn Ile Phe Arg Leu Leu Asp Ser Leu Arg Ala Leu Ser Gly Gln Ala
485 490 495
Gly Cys Arg Leu Arg Ala Leu His Leu Ser Asp Leu Phe Ser Pro Leu
500 505 510
Pro Ile Leu Glu Leu Thr Arg Ala Ile Val Arg Ala Leu Pro Leu Leu
515 520 525
Arg Val Leu Ser Ile Arg Val Asp His Pro Ser Gln Arg Asp Asn Pro
530 535 540
Gly Val Pro Gly Asn Ala Gly Pro Pro Ser His Ile Ile Gly Asp Glu
545 550 555 560
Glu Ile Pro Glu Asn Cys Leu Glu Gln Leu Glu Met Gly Phe Pro Arg
565 570 575
Gly Ala Gln Pro Ala Pro Leu Leu Cys Ser Val Leu Lys Ala Ser Gly
580 585 590
Ser Leu Gln Gln Leu Ser Leu Asp Ser Ala Thr Phe Ala Ser Pro Gln
595 600 605
Asp Phe Gly Leu Val Leu Gln Thr Leu Lys Glu Tyr Asn Leu Ala Leu
610 615 620
Lys Arg Leu Ser Phe His Asp Met Asn Leu Ala Asp Cys Gln Ser Glu
625 630 635 640
Val Leu Phe Leu Leu Gln Asn Leu Thr Leu Gln Glu Ile Thr Phe Ser
645 650 655
Phe Cys Arg Leu Phe Glu Lys Arg Pro Ala Gln Phe Leu Pro Glu Met
660 665 670
Val Ala Ala Met Lys Gly Asn Ser Thr Leu Lys Gly Leu Arg Leu Pro
675 680 685
Gly Asn Arg Leu Gly Asn Ala Gly Leu Leu Ala Leu Ala Asp Val Phe
690 695 700
Ser Glu Asp Ser Ser Ser Ser Leu Cys Gln Leu Asp Ile Ser Ser Asn
705 710 715 720
Cys Ile Lys Pro Asp Gly Leu Leu Glu Phe Ala Lys Arg Leu Glu Arg
725 730 735
Trp Gly Arg Gly Ala Phe Gly His Leu Arg Leu Phe Gln Asn Trp Leu
740 745 750
Asp Gln Asp Ala Val Thr Ala Arg Glu Ala Ile Arg Arg Leu Arg Ala
755 760 765
Thr Cys His Val Val Ser Asp Ser Trp Asp Ser Ser Gln Ala Phe Ala
770 775 780
Asp Tyr Val Ser Thr Met
785 790

<210> 182
<211> 2058
<212> PRT
<213> Homo sapiens
<400> 182
Met Asp Asn Phe Phe Thr Glu Gly Thr Arg Val Trp Leu Arg Glu Asn
1 5 10 15
Gly Gln His Phe Pro Ser Thr Val Asn Ser Cys Ala Glu Gly Ile Val
20 25 30
Val Phe Arg Thr Asp Tyr Gly Gln Val Phe Thr Tyr Lys Gln Ser Thr

35 40 45
Ile Thr His Gln Lys Val Thr Ala Met His Pro Thr Asn Glu Glu Gly
50 55 60
Val Asp Asp Met Ala Ser Leu Thr Glu Leu His Gly Gly Ser Ile Met
65 70 75 80
Tyr Asn Leu Phe Gln Arg Tyr Lys Arg Asn Gln Ile Tyr Thr Tyr Ile
85 90 95
Gly Ser Ile Leu Ala Ser Val Asn Pro Tyr Gln Pro Ile Ala Gly Leu
100 105 110
Tyr Glu Pro Ala Thr Met Glu Gln Tyr Ser Arg Arg His Leu Gly Glu
115 120 125
Leu Pro Pro His Ile Phe Ala Ile Ala Asn Glu Cys Tyr Arg Cys Leu
130 135 140
Trp Lys Arg Tyr Asp Asn Gln Cys Ile Leu Ile Ser Gly Glu Ser Gly
145 150 155 160
Ala Gly Lys Thr Glu Ser Thr Lys Leu Ile Leu Lys Phe Leu Ser Val
165 170 175
Ile Ser Gln Gln Ser Leu Glu Leu Ser Leu Lys Glu Lys Thr Ser Cys
180 185 190
Val Glu Arg Ala Ile Leu Glu Ser Ser Pro Ile Met Glu Ala Phe Gly
195 200 205
Asn Ala Lys Thr Val Tyr Asn Asn Asn Ser Ser Arg Phe Gly Lys Phe
210 215 220
Val Gln Leu Asn Ile Cys Gln Lys Gly Asn Ile Gln Gly Gly Arg Ile
225 230 235 240
Val Asp Tyr Leu Leu Glu Lys Asn Arg Val Val Arg Gln Asn Pro Gly
245 250 255
Glu Arg Asn Tyr His Ile Phe Tyr Ala Leu Leu Ala Gly Leu Glu His
260 265 270
Glu Glu Arg Glu Glu Phe Tyr Leu Ser Thr Pro Glu Asn Tyr His Tyr
275 280 285
Leu Asn Gln Ser Gly Cys Val Glu Asp Lys Thr Ile Ser Asp Gln Glu
290 295 300
Ser Phe Arg Glu Val Ile Thr Ala Met Asp Val Met Gln Phe Ser Lys
305 310 315 320
Glu Glu Val Arg Glu Val Ser Arg Leu Leu Ala Gly Ile Leu His Leu
325 330 335
Gly Asn Ile Glu Phe Ile Thr Ala Gly Gly Ala Gln Val Ser Phe Lys
340 345 350
Thr Ala Leu Gly Arg Ser Ala Glu Leu Leu Gly Leu Asp Pro Thr Gln
355 360 365
Leu Thr Asp Ala Leu Thr Gln Arg Ser Met Phe Leu Arg Gly Glu Glu
370 375 380
Ile Leu Thr Pro Leu Asn Val Gln Gln Ala Val Asp Ser Arg Asp Ser
385 390 395 400
Leu Ala Met Ala Leu Tyr Ala Cys Cys Phe Glu Trp Val Ile Lys Lys
405 410 415
Ile Asn Ser Arg Ile Lys Gly Asn Glu Asp Phe Lys Ser Ile Gly Ile
420 425 430
Leu Asp Ile Phe Gly Phe Glu Asn Phe Glu Val Asn His Phe Glu Gln
435 440 445
Phe Asn Ile Asn Tyr Ala Asn Glu Lys Leu Gln Glu Tyr Phe Asn Lys
450 455 460
His Ile Phe Ser Leu Glu Gln Leu Glu Tyr Ser Arg Glu Gly Leu Val
465 470 475 480
Trp Glu Asp Ile Asp Trp Ile Asp Asn Gly Glu Cys Leu Asp Leu Ile
485 490 495
Glu Lys Lys Leu Gly Leu Leu Ala Leu Ile Asn Glu Glu Ser His Phe
500 505 510
Pro Gln Ala Thr Asp Ser Thr Leu Leu Glu Lys Leu His Ser Gln His
515 520 525
Ala Asn Asn His Phe Tyr Val Lys Pro Arg Val Ala Val Asn Asn Phe
530 535 540
Gly Val Lys His Tyr Ala Gly Glu Val Gln Tyr Asp Val Arg Gly Ile
545 550 555 560
Leu Glu Lys Asn Arg Asp Thr Phe Arg Asp Asp Leu Leu Asn Leu Leu
565 570 575
Arg Glu Ser Arg Phe Asp Phe Ile Tyr Asp Leu Phe Glu His Val Ser
580 585 590

Ser Arg Asn Asn Gln Asp Thr Leu Lys Cys Gly Ser Lys His Arg Arg
595 600 605
Pro Thr Val Ser Ser Gln Phe Lys Asp Ser Leu His Ser Leu Met Ala
610 615 620
Thr Leu Ser Ser Ser Asn Pro Phe Phe Val Arg Cys Ile Lys Pro Asn
625 630 635 640
Met Gln Lys Met Pro Asp Gln Phe Asp Gln Ala Val Val Leu Asn Gln
645 650 655
Leu Arg Tyr Ser Gly Met Leu Glu Thr Val Arg Ile Arg Lys Ala Gly
660 665 670
Tyr Ala Val Arg Arg Pro Phe Gln Asp Phe Tyr Lys Arg Tyr Lys Val
675 680 685
Leu Met Arg Asn Leu Ala Leu Pro Glu Asp Val Arg Gly Lys Cys Thr
690 695 700
Ser Leu Leu Gln Leu Tyr Asp Ala Ser Asn Ser Glu Trp Gln Leu Gly
705 710 715 720
Lys Thr Lys Val Phe Leu Arg Glu Ser Leu Glu Gln Lys Leu Glu Lys
725 730 735
Arg Arg Glu Glu Glu Val Ser His Ala Ala Met Val Ile Arg Ala His
740 745 750
Val Leu Gly Phe Leu Ala Arg Lys Gln Tyr Arg Lys Val Leu Tyr Cys
755 760 765
Val Val Ile Ile Gln Lys Asn Tyr Arg Ala Phe Leu Leu Arg Arg Arg
770 775 780
Phe Leu His Leu Lys Lys Ala Ala Ile Val Phe Gln Lys Gln Leu Arg
785 790 795 800
Gly Gln Ile Ala Arg Arg Val Tyr Arg Gln Leu Leu Ala Glu Lys Arg
805 810 815
Glu Gln Glu Glu Lys Lys Lys Gln Glu Glu Glu Glu Lys Lys Lys Arg
820 825 830
Glu Glu Glu Glu Arg Glu Arg Glu Arg Glu Arg Arg Glu Ala Glu Leu
835 840 845
Arg Ala Gln Gln Glu Glu Glu Thr Arg Lys Gln Gln Glu Leu Glu Ala
850 855 860
Leu Gln Lys Ser Gln Lys Glu Ala Glu Leu Thr Arg Glu Leu Glu Lys
865 870 875 880
Gln Lys Glu Asn Lys Gln Val Glu Glu Ile Leu Arg Leu Glu Lys Glu
885 890 895
Ile Glu Asp Leu Gln Arg Met Lys Glu Gln Gln Glu Leu Ser Leu Thr
900 905 910
Glu Ala Ser Leu Gln Lys Leu Gln Glu Arg Arg Asp Gln Glu Leu Arg
915 920 925
Arg Leu Glu Glu Glu Ala Cys Arg Ala Ala Gln Glu Phe Leu Glu Ser
930 935 940
Leu Asn Phe Asp Glu Ile Asp Glu Cys Val Arg Asn Ile Glu Arg Ser
945 950 955 960
Leu Ser Val Gly Ser Glu Phe Ser Ser Glu Leu Ala Glu Ser Ala Cys
965 970 975
Glu Glu Lys Pro Asn Phe Asn Phe Ser Gln Pro Tyr Pro Glu Glu Glu
980 985 990
Val Asp Glu Gly Phe Glu Ala Asp Asp Asp Ala Phe Lys Asp Ser Pro
995 1000 1005
Asn Pro Ser Glu His Gly His Ser Asp Gln Arg Thr Ser Gly Ile
1010 1015 1020
Arg Thr Ser Asp Asp Ser Ser Glu Glu Asp Pro Tyr Met Asn Asp
1025 1030 1035
Thr Val Val Pro Thr Ser Pro Ser Ala Asp Ser Thr Val Leu Leu
1040 1045 1050
Ala Pro Ser Val Gln Asp Ser Gly Ser Leu His Asn Ser Ser Ser
1055 1060 1065
Gly Glu Ser Thr Tyr Cys Met Pro Gln Asn Ala Gly Asp Leu Pro
1070 1075 1080
Ser Pro Asp Gly Asp Tyr Asp Tyr Asp Gln Asp Asp Tyr Glu Asp
1085 1090 1095
Gly Ala Ile Thr Ser Gly Ser Ser Val Thr Phe Ser Asn Ser Tyr
1100 1105 1110
Gly Ser Gln Trp Ser Pro Asp Tyr Arg Cys Ser Val Gly Thr Tyr
1115 1120 1125
Asn Ser Ser Gly Ala Tyr Arg Phe Ser Ser Glu Gly Ala Gln Ser

1130 1135 1140
Ser Phe Glu Asp Ser Glu Glu Asp Phe Asp Ser Arg Phe Asp Thr
1145 1150 1155
Asp Asp Glu Leu Ser Tyr Arg Arg Asp Ser Val Tyr Ser Cys Val
1160 1165 1170
Thr Leu Pro Tyr Phe His Ser Phe Leu Tyr Met Lys Gly Gly Leu
1175 1180 1185
Met Asn Ser Trp Lys Arg Arg Trp Cys Val Leu Lys Asp Glu Thr
1190 1195 1200
Phe Leu Trp Phe Arg Ser Lys Gln Glu Ala Leu Lys Gln Gly Trp
1205 1210 1215
Leu His Lys Lys Gly Gly Gly Ser Ser Thr Leu Ser Arg Arg Asn
1220 1225 1230
Trp Lys Lys Arg Trp Phe Val Leu Arg Gln Ser Lys Leu Met Tyr
1235 1240 1245
Phe Glu Asn Asp Ser Glu Glu Lys Leu Lys Gly Thr Val Glu Val
1250 1255 1260
Arg Thr Ala Lys Glu Ile Ile Asp Asn Thr Thr Lys Glu Asn Gly
1265 1270 1275
Ile Asp Ile Ile Met Ala Asp Arg Thr Phe His Leu Ile Ala Glu
1280 1285 1290
Ser Pro Glu Asp Ala Ser Gln Trp Phe Ser Val Leu Ser Gln Val
1295 1300 1305
His Ala Ser Thr Asp Gln Glu Ile Gln Glu Met His Asp Glu Gln
1310 1315 1320
Ala Asn Pro Gln Asn Ala Val Gly Thr Leu Asp Val Gly Leu Ile
1325 1330 1335
Asp Ser Val Cys Ala Ser Asp Ser Pro Asp Arg Pro Asn Ser Phe
1340 1345 1350
Val Ile Ile Thr Ala Asn Arg Val Leu His Cys Asn Ala Asp Thr
1355 1360 1365
Pro Glu Glu Met His His Trp Ile Thr Leu Leu Gln Arg Ser Lys
1370 1375 1380
Gly Asp Thr Arg Val Glu Gly Gln Glu Phe Ile Val Arg Gly Trp
1385 1390 1395
Leu His Lys Glu Val Lys Asn Ser Pro Lys Met Ser Ser Leu Lys
1400 1405 1410
Leu Lys Lys Arg Trp Phe Val Leu Thr His Asn Ser Leu Asp Tyr
1415 1420 1425
Tyr Lys Ser Ser Glu Lys Asn Ala Leu Lys Leu Gly Thr Leu Val
1430 1435 1440
Leu Asn Ser Leu Cys Ser Val Val Pro Pro Asp Glu Lys Ile Phe
1445 1450 1455
Lys Glu Thr Gly Tyr Trp Asn Val Thr Val Tyr Gly Arg Lys His
1460 1465 1470
Cys Tyr Arg Leu Tyr Thr Lys Leu Leu Asn Glu Ala Thr Arg Trp
1475 1480 1485
Ser Ser Ala Ile Gln Asn Val Thr Asp Thr Lys Ala Pro Ile Asp
1490 1495 1500
Thr Pro Thr Gln Gln Leu Ile Gln Asp Ile Lys Glu Asn Cys Leu
1505 1510 1515
Asn Ser Asp Val Val Glu Gln Ile Tyr Lys Arg Asn Pro Ile Leu
1520 1525 1530
Arg Tyr Thr His His Pro Leu His Ser Pro Leu Leu Pro Leu Pro
1535 1540 1545
Tyr Gly Asp Ile Asn Leu Asn Leu Leu Lys Asp Lys Gly Tyr Thr
1550 1555 1560
Thr Leu Gln Asp Glu Ala Ile Lys Ile Phe Asn Ser Leu Gln Gln
1565 1570 1575
Leu Glu Ser Met Ser Asp Pro Ile Pro Ile Ile Gln Gly Ile Leu
1580 1585 1590
Gln Thr Gly His Asp Leu Arg Pro Leu Arg Asp Glu Leu Tyr Cys
1595 1600 1605
Gln Leu Ile Lys Gln Thr Asn Lys Val Pro His Pro Gly Ser Val
1610 1615 1620
Gly Asn Leu Tyr Ser Trp Gln Ile Leu Thr Cys Leu Ser Cys Thr
1625 1630 1635
Phe Leu Pro Ser Arg Gly Ile Leu Lys Tyr Leu Lys Phe His Leu
1640 1645 1650

Lys Arg Ile Arg Glu Gln Phe Pro Gly Thr Glu Met Glu Lys Tyr
1655 1660 1665
Ala Leu Phe Thr Tyr Glu Ser Leu Lys Lys Thr Lys Cys Arg Glu
1670 1675 1680
Phe Val Pro Ser Arg Asp Glu Ile Glu Ala Leu Ile His Arg Gln
1685 1690 1695
Glu Met Thr Ser Thr Val Tyr Cys His Gly Gly Gly Ser Cys Lys
1700 1705 1710
Ile Thr Ile Asn Ser His Thr Thr Ala Gly Glu Val Val Glu Lys
1715 1720 1725
Leu Ile Arg Gly Leu Ala Met Glu Asp Ser Arg Asn Met Phe Ala
1730 1735 1740
Leu Phe Glu Tyr Asn Gly His Val Asp Lys Ala Ile Glu Ser Arg
1745 1750 1755
Thr Val Val Ala Asp Val Leu Ala Lys Phe Glu Lys Leu Ala Ala
1760 1765 1770
Thr Ser Glu Val Gly Asp Leu Pro Trp Lys Phe Tyr Phe Lys Leu
1775 1780 1785
Tyr Cys Phe Leu Asp Thr Asp Asn Val Pro Lys Asp Ser Val Glu
1790 1795 1800
Phe Ala Phe Met Phe Glu Gln Ala His Glu Ala Val Ile His Gly
1805 1810 1815
His His Pro Ala Pro Glu Glu Asn Leu Gln Val Leu Ala Ala Leu
1820 1825 1830
Arg Leu Gln Tyr Leu Gln Gly Asp Tyr Thr Leu His Ala Ala Ile
1835 1840 1845
Pro Pro Leu Glu Glu Val Tyr Ser Leu Gln Arg Leu Lys Ala Arg
1850 1855 1860
Ile Ser Gln Ser Thr Lys Thr Phe Thr Pro Cys Glu Arg Leu Glu
1865 1870 1875
Lys Arg Arg Thr Ser Phe Leu Glu Gly Thr Leu Arg Arg Ser Phe
1880 1885 1890
Arg Thr Gly Ser Val Val Arg Gln Lys Val Glu Glu Glu Gln Met
1895 1900 1905
Leu Asp Met Trp Ile Lys Glu Glu Val Ser Ser Ala Arg Ala Ser
1910 1915 1920
Ile Ile Asp Lys Trp Arg Lys Phe Gln Gly Met Asn Gln Glu Gln
1925 1930 1935
Ala Met Ala Lys Tyr Met Ala Leu Ile Lys Glu Trp Pro Gly Tyr
1940 1945 1950
Gly Ser Thr Leu Phe Asp Val Glu Cys Lys Glu Gly Gly Phe Pro
1955 1960 1965
Gln Glu Leu Trp Leu Gly Val Ser Ala Asp Ala Val Ser Val Tyr
1970 1975 1980
Lys Arg Gly Glu Gly Arg Pro Leu Glu Val Phe Gln Tyr Glu His
1985 1990 1995
Ile Leu Ser Phe Gly Ala Pro Leu Ala Asn Thr Tyr Lys Ile Val
2000 2005 2010
Val Asp Glu Arg Glu Leu Leu Phe Glu Thr Ser Glu Val Val Asp
2015 2020 2025
Val Ala Lys Leu Met Lys Ala Tyr Ile Ser Met Ile Val Lys Lys
2030 2035 2040
Arg Tyr Ser Thr Thr Arg Ser Ala Ser Ser Gln Gly Ser Ser Arg
2045 2050 2055

<210> 183
<211> 1210
<212> PRT
<213> Homo sapiens
<400> 183
Met Arg Pro Ser Gly Thr Ala Gly Ala Ala Leu Leu Ala Leu Leu Ala
1 5 10 15
Ala Leu Cys Pro Ala Ser Arg Ala Leu Glu Glu Lys Lys Val Cys Gln
20 25 30
Gly Thr Ser Asn Lys Leu Thr Gln Leu Gly Thr Phe Glu Asp His Phe
35 40 45
Leu Ser Leu Gln Arg Met Phe Asn Asn Cys Glu Val Val Leu Gly Asn
50 55 60
Leu Glu Ile Thr Tyr Val Gln Arg Asn Tyr Asp Leu Ser Phe Leu Lys

```
65                  70                  75                  80
Thr Ile Gln Glu Val Ala Gly Tyr Val Leu Ile Ala Leu Asn Thr Val
                85                  90                  95
Glu Arg Ile Pro Leu Glu Asn Leu Gln Ile Ile Arg Gly Asn Met Tyr
            100                 105                 110
Tyr Glu Asn Ser Tyr Ala Leu Ala Val Leu Ser Asn Tyr Asp Ala Asn
        115                 120                 125
Lys Thr Gly Leu Lys Glu Leu Pro Met Arg Asn Leu Gln Glu Ile Leu
    130                 135                 140
His Gly Ala Val Arg Phe Ser Asn Asn Pro Ala Leu Cys Asn Val Glu
145                 150                 155                 160
Ser Ile Gln Trp Arg Asp Ile Val Ser Ser Asp Phe Leu Ser Asn Met
                165                 170                 175
Ser Met Asp Phe Gln Asn His Leu Gly Ser Cys Gln Lys Cys Asp Pro
            180                 185                 190
Ser Cys Pro Asn Gly Ser Cys Trp Gly Ala Gly Glu Glu Asn Cys Gln
        195                 200                 205
Lys Leu Thr Lys Ile Ile Cys Ala Gln Gln Cys Ser Gly Arg Cys Arg
    210                 215                 220
Gly Lys Ser Pro Ser Asp Cys Cys His Asn Gln Cys Ala Ala Gly Cys
225                 230                 235                 240
Thr Gly Pro Arg Glu Ser Asp Cys Leu Val Cys Arg Lys Phe Arg Asp
                245                 250                 255
Glu Ala Thr Cys Lys Asp Thr Cys Pro Pro Leu Met Leu Tyr Asn Pro
            260                 265                 270
Thr Thr Tyr Gln Met Asp Val Asn Pro Glu Gly Lys Tyr Ser Phe Gly
        275                 280                 285
Ala Thr Cys Val Lys Lys Cys Pro Arg Asn Tyr Val Val Thr Asp His
    290                 295                 300
Gly Ser Cys Val Arg Ala Cys Gly Ala Asp Ser Tyr Glu Met Glu Glu
305                 310                 315                 320
Asp Gly Val Arg Lys Cys Lys Lys Cys Glu Gly Pro Cys Arg Lys Val
                325                 330                 335
Cys Asn Gly Ile Gly Ile Gly Glu Phe Lys Asp Ser Leu Ser Ile Asn
            340                 345                 350
Ala Thr Asn Ile Lys His Phe Lys Asn Cys Thr Ser Ile Ser Gly Asp
        355                 360                 365
Leu His Ile Leu Pro Val Ala Phe Arg Gly Asp Ser Phe Thr His Thr
    370                 375                 380
Pro Pro Leu Asp Pro Gln Glu Leu Asp Ile Leu Lys Thr Val Lys Glu
385                 390                 395                 400
Ile Thr Gly Phe Leu Leu Ile Gln Ala Trp Pro Glu Asn Arg Thr Asp
                405                 410                 415
Leu His Ala Phe Glu Asn Leu Glu Ile Ile Arg Gly Arg Thr Lys Gln
            420                 425                 430
His Gly Gln Phe Ser Leu Ala Val Val Ser Leu Asn Ile Thr Ser Leu
        435                 440                 445
Gly Leu Arg Ser Leu Lys Glu Ile Ser Asp Gly Asp Val Ile Ile Ser
    450                 455                 460
Gly Asn Lys Asn Leu Cys Tyr Ala Asn Thr Ile Asn Trp Lys Lys Leu
465                 470                 475                 480
Phe Gly Thr Ser Gly Gln Lys Thr Lys Ile Ile Ser Asn Arg Gly Glu
                485                 490                 495
Asn Ser Cys Lys Ala Thr Gly Gln Val Cys His Ala Leu Cys Ser Pro
            500                 505                 510
Glu Gly Cys Trp Gly Pro Glu Pro Arg Asp Cys Val Ser Cys Arg Asn
        515                 520                 525
Val Ser Arg Gly Arg Glu Cys Val Asp Lys Cys Asn Leu Leu Glu Gly
    530                 535                 540
Glu Pro Arg Glu Phe Val Glu Asn Ser Glu Cys Ile Gln Cys His Pro
545                 550                 555                 560
Glu Cys Leu Pro Gln Ala Met Asn Ile Thr Cys Thr Gly Arg Gly Pro
                565                 570                 575
Asp Asn Cys Ile Gln Cys Ala His Tyr Ile Asp Gly Pro His Cys Val
            580                 585                 590
Lys Thr Cys Pro Ala Gly Val Met Gly Glu Asn Asn Thr Leu Val Trp
        595                 600                 605
Lys Tyr Ala Asp Ala Gly His Val Cys His Leu Cys His Pro Asn Cys
    610                 615                 620
```

```
Thr Tyr Gly Cys Thr Gly Pro Gly Leu Glu Gly Cys Pro Thr Asn Gly
625                 630                 635                 640
Pro Lys Ile Pro Ser Ile Ala Thr Gly Met Val Gly Ala Leu Leu Leu
                645                 650                 655
Leu Leu Val Val Ala Leu Gly Ile Gly Leu Phe Met Arg Arg Arg His
            660                 665                 670
Ile Val Arg Lys Arg Thr Leu Arg Arg Leu Leu Gln Glu Arg Glu Leu
        675                 680                 685
Val Glu Pro Leu Thr Pro Ser Gly Glu Ala Pro Asn Gln Ala Leu Leu
    690                 695                 700
Arg Ile Leu Lys Glu Thr Glu Phe Lys Lys Ile Lys Val Leu Gly Ser
705                 710                 715                 720
Gly Ala Phe Gly Thr Val Tyr Lys Gly Leu Trp Ile Pro Glu Gly Glu
                725                 730                 735
Lys Val Lys Ile Pro Val Ala Ile Lys Glu Leu Arg Glu Ala Thr Ser
            740                 745                 750
Pro Lys Ala Asn Lys Glu Ile Leu Asp Glu Ala Tyr Val Met Ala Ser
        755                 760                 765
Val Asp Asn Pro His Val Cys Arg Leu Leu Gly Ile Cys Leu Thr Ser
    770                 775                 780
Thr Val Gln Leu Ile Thr Gln Leu Met Pro Phe Gly Cys Leu Leu Asp
785                 790                 795                 800
Tyr Val Arg Glu His Lys Asp Asn Ile Gly Ser Gln Tyr Leu Leu Asn
                805                 810                 815
Trp Cys Val Gln Ile Ala Lys Gly Met Asn Tyr Leu Glu Asp Arg Arg
            820                 825                 830
Leu Val His Arg Asp Leu Ala Ala Arg Asn Val Leu Val Lys Thr Pro
        835                 840                 845
Gln His Val Lys Ile Thr Asp Phe Gly Leu Ala Lys Leu Leu Gly Ala
    850                 855                 860
Glu Glu Lys Glu Tyr His Ala Glu Gly Gly Lys Val Pro Ile Lys Trp
865                 870                 875                 880
Met Ala Leu Glu Ser Ile Leu His Arg Ile Tyr Thr His Gln Ser Asp
                885                 890                 895
Val Trp Ser Tyr Gly Val Thr Val Trp Glu Leu Met Thr Phe Gly Ser
            900                 905                 910
Lys Pro Tyr Asp Gly Ile Pro Ala Ser Glu Ile Ser Ser Ile Leu Glu
        915                 920                 925
Lys Gly Glu Arg Leu Pro Gln Pro Pro Ile Cys Thr Ile Asp Val Tyr
    930                 935                 940
Met Ile Met Val Lys Cys Trp Met Ile Asp Ala Asp Ser Arg Pro Lys
945                 950                 955                 960
Phe Arg Glu Leu Ile Ile Glu Phe Ser Lys Met Ala Arg Asp Pro Gln
                965                 970                 975
Arg Tyr Leu Val Ile Gln Gly Asp Glu Arg Met His Leu Pro Ser Pro
            980                 985                 990
Thr Asp Ser Asn Phe Tyr Arg Ala  Leu Met Asp Glu Glu  Asp Met Asp
        995                 1000                 1005
Asp Val  Val Asp Ala Asp Glu  Tyr Leu Ile Pro Gln  Gln Gly Phe
    1010                 1015                 1020
Phe Ser  Ser Pro Ser Thr Ser  Arg Thr Pro Leu Leu  Ser Ser Leu
    1025                 1030                 1035
Ser Ala  Thr Ser Asn Asn Ser  Thr Val Ala Cys Ile  Asp Arg Asn
    1040                 1045                 1050
Gly Leu  Gln Ser Cys Pro Ile  Lys Glu Asp Ser Phe  Leu Gln Arg
    1055                 1060                 1065
Tyr Ser  Ser Asp Pro Thr Gly  Ala Leu Thr Glu Asp  Ser Ile Asp
    1070                 1075                 1080
Asp Thr  Phe Leu Pro Val Pro  Glu Tyr Ile Asn Gln  Ser Val Pro
    1085                 1090                 1095
Lys Arg  Pro Ala Gly Ser Val  Gln Asn Pro Val Tyr  His Asn Gln
    1100                 1105                 1110
Pro Leu  Asn Pro Ala Pro Ser  Arg Asp Pro His Tyr  Gln Asp Pro
    1115                 1120                 1125
His Ser  Thr Ala Val Gly Asn  Pro Glu Tyr Leu Asn  Thr Val Gln
    1130                 1135                 1140
Pro Thr  Cys Val Asn Ser Thr  Phe Asp Ser Pro Ala  His Trp Ala
    1145                 1150                 1155
Gln Lys  Gly Ser His Gln Ile  Ser Leu Asp Asn Pro  Asp Tyr Gln
```

1160 1165 1170
Gln Asp Phe Phe Pro Lys Glu Ala Lys Pro Asn Gly Ile Phe Lys
1175 1180 1185
Gly Ser Thr Ala Glu Asn Ala Glu Tyr Leu Arg Val Ala Pro Gln
1190 1195 1200
Ser Ser Glu Phe Ile Gly Ala
1205 1210

<210> 184
<211> 453
<212> PRT
<213> Homo sapiens
<400> 184
Met Pro Lys Asn Lys Lys Arg Asn Thr Pro His Arg Gly Ser Ser Ala
1 5 10 15
Gly Gly Gly Gly Ser Gly Ala Ala Ala Ala Thr Ala Ala Thr Ala Gly
20 25 30
Gly Gln His Arg Asn Val Gln Pro Phe Ser Asp Glu Asp Ala Ser Ile
35 40 45
Glu Thr Met Ser His Cys Ser Gly Tyr Ser Asp Pro Ser Ser Phe Ala
50 55 60
Glu Asp Gly Pro Glu Val Leu Asp Glu Glu Gly Thr Gln Glu Asp Leu
65 70 75 80
Glu Tyr Lys Arg Lys Gly Leu Ile Asp Leu Thr Leu Asp Lys Ser Ala
85 90 95
Lys Thr Arg Gln Ala Ala Leu Glu Gly Ile Lys Asn Ala Leu Ala Ser
100 105 110
Lys Met Leu Tyr Glu Phe Ile Leu Glu Arg Arg Met Thr Leu Thr Asp
115 120 125
Ser Ile Glu Arg Cys Leu Lys Lys Gly Lys Ser Asp Glu Gln Arg Ala
130 135 140
Ala Ala Ala Leu Ala Ser Val Leu Cys Ile Gln Leu Gly Pro Gly Ile
145 150 155 160
Glu Ser Glu Glu Ile Leu Lys Thr Leu Gly Pro Ile Leu Lys Lys Ile
165 170 175
Ile Cys Asp Gly Ser Ala Ser Met Gln Ala Arg Gln Thr Cys Ala Thr
180 185 190
Cys Phe Gly Val Cys Cys Phe Ile Ala Thr Asp Asp Ile Thr Glu Leu
195 200 205
Tyr Ser Thr Leu Glu Cys Leu Glu Asn Ile Phe Thr Lys Ser Tyr Leu
210 215 220
Lys Glu Lys Asp Thr Thr Val Ile Cys Ser Thr Pro Asn Thr Val Leu
225 230 235 240
His Ile Ser Ser Leu Leu Ala Trp Thr Leu Leu Leu Thr Ile Cys Pro
245 250 255
Ile Asn Glu Val Lys Lys Lys Leu Glu Met His Phe His Lys Leu Pro
260 265 270
Ser Leu Leu Ser Cys Asp Asp Val Asn Met Arg Ile Ala Ala Gly Glu
275 280 285
Ser Leu Ala Leu Leu Phe Glu Leu Ala Arg Gly Ile Glu Ser Asp Phe
290 295 300
Phe Tyr Glu Asp Met Glu Ser Leu Thr Gln Met Leu Arg Ala Leu Ala
305 310 315 320
Thr Asp Gly Asn Lys His Arg Ala Lys Val Asp Lys Arg Lys Gln Arg
325 330 335
Ser Val Phe Arg Asp Val Leu Arg Ala Val Glu Glu Arg Asp Phe Pro
340 345 350
Thr Glu Thr Ile Lys Phe Gly Pro Glu Arg Met Tyr Ile Asp Cys Trp
355 360 365
Val Lys Lys His Thr Tyr Asp Thr Phe Lys Glu Val Leu Gly Ser Gly
370 375 380
Met Gln Tyr Pro Leu Ala Val Lys Met Glu Phe Leu Glu Asn Val Phe
385 390 395 400
Glu Thr Trp Thr Pro Ser Asp Ala Leu Met Leu Gln Arg Leu Lys Thr
405 410 415
Met Lys Ile Ser Arg Phe Glu Arg His Leu Tyr Asn Ser Ala Ala Phe
420 425 430
Lys Ala Arg Thr Lys Ala Arg Ser Lys Cys Arg Asp Lys Arg Ala Asp
435 440 445

Val Gly Glu Phe Phe
    450

<210> 185
<211> 341
<212> PRT
<213> Homo sapiens
<400> 185
Met Pro Lys Arg Lys Val Thr Phe Gln Gly Val Gly Asp Glu Glu Asp
1               5                   10                  15
Glu Asp Glu Ile Ile Val Pro Lys Lys Lys Leu Val Asp Pro Val Ala
            20                  25                  30
Gly Ser Gly Gly Pro Gly Ser Arg Phe Lys Gly Lys His Ser Leu Asp
        35                  40                  45
Ser Asp Glu Glu Glu Asp Asp Asp Asp Gly Gly Ser Ser Lys Tyr Asp
    50                  55                  60
Ile Leu Ala Ser Glu Asp Val Glu Gly Gln Glu Ala Ala Thr Leu Pro
65                  70                  75                  80
Ser Glu Gly Gly Val Arg Ile Thr Pro Phe Asn Leu Gln Glu Glu Met
                85                  90                  95
Glu Glu Gly His Phe Asp Ala Asp Gly Asn Tyr Phe Leu Asn Arg Asp
            100                 105                 110
Ala Gln Ile Arg Asp Ser Trp Leu Asp Asn Ile Asp Trp Val Lys Ile
        115                 120                 125
Arg Glu Arg Pro Pro Gly Gln Arg Gln Ala Ser Asp Ser Glu Glu Glu
    130                 135                 140
Asp Ser Leu Gly Gln Thr Ser Met Ser Ala Gln Ala Leu Leu Glu Gly
145                 150                 155                 160
Leu Leu Glu Leu Leu Leu Pro Arg Glu Thr Val Ala Gly Ala Leu Arg
                165                 170                 175
Arg Leu Gly Ala Arg Gly Gly Gly Lys Gly Arg Lys Gly Pro Gly Gln
            180                 185                 190
Pro Ser Ser Pro Gln Arg Leu Asp Arg Leu Ser Gly Leu Ala Asp Gln
        195                 200                 205
Met Val Ala Arg Gly Asn Leu Gly Val Tyr Gln Glu Thr Arg Glu Arg
    210                 215                 220
Leu Ala Met Arg Leu Lys Gly Leu Gly Cys Gln Thr Leu Gly Pro His
225                 230                 235                 240
Asn Pro Thr Pro Pro Pro Ser Leu Asp Met Phe Ala Glu Glu Leu Ala
                245                 250                 255
Glu Glu Glu Leu Glu Thr Pro Thr Pro Thr Gln Arg Gly Glu Ala Glu
            260                 265                 270
Ser Arg Gly Asp Gly Leu Val Asp Val Met Trp Glu Tyr Lys Trp Glu
        275                 280                 285
Asn Thr Gly Asp Ala Glu Leu Tyr Gly Pro Phe Thr Ser Ala Gln Met
    290                 295                 300
Gln Thr Trp Val Ser Glu Gly Tyr Phe Pro Asp Gly Val Tyr Cys Arg
305                 310                 315                 320
Lys Leu Asp Pro Pro Gly Gly Gln Phe Tyr Asn Ser Lys Arg Ile Asp
                325                 330                 335
Phe Asp Leu Tyr Thr
            340

<210> 186
<211> 182
<212> PRT
<213> Homo sapiens
<400> 186
Met Gly Leu Leu Ser Ile Leu Arg Lys Leu Lys Ser Ala Pro Asp Gln
1               5                   10                  15
Glu Val Arg Ile Leu Leu Leu Gly Leu Asp Asn Ala Gly Lys Thr Thr
            20                  25                  30
Leu Leu Lys Gln Leu Ala Ser Glu Asp Ile Ser His Ile Thr Pro Thr
        35                  40                  45
Gln Gly Phe Asn Ile Lys Ser Val Gln Ser Gln Gly Phe Lys Leu Asn
    50                  55                  60
Val Trp Asp Ile Gly Gly Gln Arg Lys Ile Arg Pro Tyr Trp Lys Asn
65                  70                  75                  80
Tyr Phe Glu Asn Thr Asp Ile Leu Ile Tyr Val Ile Asp Ser Ala Asp

85 90 95
Arg Lys Arg Phe Glu Glu Thr Gly Gln Glu Leu Ala Glu Leu Leu Glu
100 105 110
Glu Glu Lys Leu Ser Cys Val Pro Val Leu Ile Phe Ala Asn Lys Gln
115 120 125
Asp Leu Leu Thr Ala Ala Pro Ala Ser Glu Ile Ala Glu Gly Leu Asn
130 135 140
Leu His Thr Ile Arg Asp Arg Val Trp Gln Ile Gln Ser Cys Ser Ala
145 150 155 160
Leu Thr Gly Glu Gly Val Gln Asp Gly Met Asn Trp Val Cys Lys Asn
165 170 175
Val Asn Ala Lys Lys Lys
180

<210> 187
<211> 398
<212> PRT
<213> Homo sapiens
<400> 187
Met Ala Leu Leu Arg Arg Pro Thr Val Ser Ser Asp Leu Glu Asn Ile
1 5 10 15
Asp Thr Gly Val Asn Ser Lys Val Lys Ser His Val Thr Ile Arg Arg
20 25 30
Thr Val Leu Glu Glu Ile Gly Asn Arg Val Thr Thr Arg Ala Ala Gln
35 40 45
Val Ala Lys Lys Ala Gln Asn Thr Lys Val Pro Val Gln Pro Thr Lys
50 55 60
Thr Thr Asn Val Asn Lys Gln Leu Lys Pro Thr Ala Ser Val Lys Pro
65 70 75 80
Val Gln Met Glu Lys Leu Ala Pro Lys Gly Pro Ser Pro Thr Pro Glu
85 90 95
Asp Val Ser Met Lys Glu Glu Asn Leu Cys Gln Ala Phe Ser Asp Ala
100 105 110
Leu Leu Cys Lys Ile Glu Asp Ile Asp Asn Glu Asp Trp Glu Asn Pro
115 120 125
Gln Leu Cys Ser Asp Tyr Val Lys Asp Ile Tyr Gln Tyr Leu Arg Gln
130 135 140
Leu Glu Val Leu Gln Ser Ile Asn Pro His Phe Leu Asp Gly Arg Asp
145 150 155 160
Ile Asn Gly Arg Met Arg Ala Ile Leu Val Asp Trp Leu Val Gln Val
165 170 175
His Ser Lys Phe Arg Leu Leu Gln Glu Thr Leu Tyr Met Cys Val Gly
180 185 190
Ile Met Asp Arg Phe Leu Gln Val Gln Pro Val Ser Arg Lys Lys Leu
195 200 205
Gln Leu Val Gly Ile Thr Ala Leu Leu Leu Ala Ser Lys Tyr Glu Glu
210 215 220
Met Phe Ser Pro Asn Ile Glu Asp Phe Val Tyr Ile Thr Asp Asn Ala
225 230 235 240
Tyr Thr Ser Ser Gln Ile Arg Glu Met Glu Thr Leu Ile Leu Lys Glu
245 250 255
Leu Lys Phe Glu Leu Gly Arg Pro Leu Pro Leu His Phe Leu Arg Arg
260 265 270
Ala Ser Lys Ala Gly Glu Val Asp Val Glu Gln His Thr Leu Ala Lys
275 280 285
Tyr Leu Met Glu Leu Thr Leu Ile Asp Tyr Asp Met Val His Tyr His
290 295 300
Pro Ser Lys Val Ala Ala Ala Ala Ser Cys Leu Ser Gln Lys Val Leu
305 310 315 320
Gly Gln Gly Lys Trp Asn Leu Lys Gln Gln Tyr Tyr Thr Gly Tyr Thr
325 330 335
Glu Asn Glu Val Leu Glu Val Met Gln His Met Ala Lys Asn Val Val
340 345 350
Lys Val Asn Glu Asn Leu Thr Lys Phe Ile Ala Ile Lys Asn Lys Tyr
355 360 365
Ala Ser Ser Lys Leu Leu Lys Ile Ser Met Ile Pro Gln Leu Asn Ser
370 375 380
Lys Ala Val Lys Asp Leu Ala Ser Pro Leu Ile Gly Arg Ser
385 390 395

<210> 188
<211> 376
<212> PRT
<213> Homo sapiens
<400> 188
Met Gln Trp Ala Ser Leu Leu Leu Leu Ala Gly Leu Phe Ser Leu Ser
1 5 10 15
Gln Ala Gln Tyr Glu Asp Asp Pro His Trp Trp Phe His Tyr Leu Arg
20 25 30
Ser Gln Gln Ser Thr Tyr Tyr Asp Pro Tyr Asp Pro Tyr Pro Tyr Glu
35 40 45
Thr Tyr Glu Pro Tyr Pro Tyr Gly Val Asp Glu Gly Pro Ala Tyr Thr
50 55 60
Tyr Gly Ser Pro Ser Pro Pro Asp Pro Arg Asp Cys Pro Gln Glu Cys
65 70 75 80
Asp Cys Pro Pro Asn Phe Leu Thr Ala Met Tyr Cys Asp Asn Arg Asn
85 90 95
Leu Lys Tyr Leu Pro Phe Val Pro Ser Arg Met Lys Tyr Val Tyr Phe
100 105 110
Gln Asn Asn Gln Ile Thr Ser Ile Gln Glu Gly Val Phe Asp Asn Ala
115 120 125
Thr Gly Leu Leu Trp Ile Ala Leu His Gly Asn Gln Ile Thr Ser Asp
130 135 140
Lys Val Gly Arg Lys Val Phe Ser Lys Leu Arg His Leu Glu Arg Leu
145 150 155 160
Tyr Leu Asp His Asn Asn Leu Thr Arg Met Pro Gly Pro Leu Pro Arg
165 170 175
Ser Leu Arg Glu Leu His Leu Asp His Asn Gln Ile Ser Arg Val Pro
180 185 190
Asn Asn Ala Leu Glu Gly Leu Glu Asn Leu Thr Ala Leu Tyr Leu Gln
195 200 205
His Asp Glu Ile Gln Glu Val Gly Ser Ser Met Arg Gly Leu Arg Ser
210 215 220
Leu Ile Leu Leu Asp Leu Ser Tyr Asn His Leu Arg Lys Val Pro Asp
225 230 235 240
Gly Leu Pro Ser Ala Leu Glu Gln Leu Tyr Met Glu His Asn Asn Val
245 250 255
Tyr Thr Val Pro Asp Ser Tyr Phe Arg Gly Ala Pro Lys Leu Leu Tyr
260 265 270
Val Arg Leu Ser His Asn Ser Leu Thr Asn Asn Gly Leu Ala Ser Asn
275 280 285
Thr Phe Asn Ser Ser Ser Leu Leu Glu Leu Asp Leu Ser Tyr Asn Gln
290 295 300
Leu Gln Lys Ile Pro Pro Val Asn Thr Asn Leu Glu Asn Leu Tyr Leu
305 310 315 320
Gln Gly Asn Arg Ile Asn Glu Phe Ser Ile Ser Ser Phe Cys Thr Val
325 330 335
Val Asp Val Val Asn Phe Ser Lys Leu Gln Val Val Arg Leu Asp Gly
340 345 350
Asn Glu Ile Lys Arg Ser Ala Met Pro Ala Asp Ala Pro Leu Cys Leu
355 360 365
Arg Leu Ala Ser Leu Ile Glu Ile
370 375

<210> 189
<211> 535
<212> PRT
<213> Homo sapiens
<400> 189
Met Glu Glu Gly Ala Arg His Arg Asn Asn Thr Glu Lys Lys His Pro
1 5 10 15
Gly Gly Gly Glu Ser Asp Ala Ser Pro Glu Ala Gly Ser Gly Gly Gly
20 25 30
Gly Val Ala Leu Lys Lys Glu Ile Gly Leu Val Ser Ala Cys Gly Ile
35 40 45
Ile Val Gly Asn Ile Ile Gly Ser Gly Ile Phe Val Ser Pro Lys Gly
50 55 60
Val Leu Glu Asn Ala Gly Ser Val Gly Leu Ala Leu Ile Val Trp Ile

65 70 75 80
Val Thr Gly Phe Ile Thr Val Val Gly Ala Leu Cys Tyr Ala Glu Leu
85 90 95
Gly Val Thr Ile Pro Lys Ser Gly Gly Asp Tyr Ser Tyr Val Lys Asp
100 105 110
Ile Phe Gly Gly Leu Ala Gly Phe Leu Arg Leu Trp Ile Ala Val Leu
115 120 125
Val Ile Tyr Pro Thr Asn Gln Ala Val Ile Ala Leu Thr Phe Ser Asn
130 135 140
Tyr Val Leu Gln Pro Leu Phe Pro Thr Cys Phe Pro Pro Glu Ser Gly
145 150 155 160
Leu Arg Leu Leu Ala Ala Ile Cys Leu Leu Leu Leu Thr Trp Val Asn
165 170 175
Cys Ser Ser Val Arg Trp Ala Thr Arg Val Gln Asp Ile Phe Thr Ala
180 185 190
Gly Lys Leu Leu Ala Leu Ala Leu Ile Ile Ile Met Gly Ile Val Gln
195 200 205
Ile Cys Lys Gly Glu Tyr Phe Trp Leu Glu Pro Lys Asn Ala Phe Glu
210 215 220
Asn Phe Gln Glu Pro Asp Ile Gly Leu Val Ala Leu Ala Phe Leu Gln
225 230 235 240
Gly Ser Phe Ala Tyr Gly Gly Trp Asn Phe Leu Asn Tyr Val Thr Glu
245 250 255
Glu Leu Val Asp Pro Tyr Lys Asn Leu Pro Arg Ala Ile Phe Ile Ser
260 265 270
Ile Pro Leu Val Thr Phe Val Tyr Val Phe Ala Asn Val Ala Tyr Val
275 280 285
Thr Ala Met Ser Pro Gln Glu Leu Leu Ala Ser Asn Ala Val Ala Val
290 295 300
Thr Phe Gly Glu Lys Leu Leu Gly Val Met Ala Trp Ile Met Pro Ile
305 310 315 320
Ser Val Ala Leu Ser Thr Phe Gly Gly Val Asn Gly Ser Leu Phe Thr
325 330 335
Ser Ser Arg Leu Phe Phe Ala Gly Ala Arg Glu Gly His Leu Pro Ser
340 345 350
Val Leu Ala Met Ile His Val Lys Arg Cys Thr Pro Ile Pro Ala Leu
355 360 365
Leu Phe Thr Cys Ile Ser Thr Leu Leu Met Leu Val Thr Ser Asp Met
370 375 380
Tyr Thr Leu Ile Asn Tyr Val Gly Phe Ile Asn Tyr Leu Phe Tyr Gly
385 390 395 400
Val Thr Val Ala Gly Gln Ile Val Leu Arg Trp Lys Lys Pro Asp Ile
405 410 415
Pro Arg Pro Ile Lys Ile Asn Leu Leu Phe Pro Ile Ile Tyr Leu Leu
420 425 430
Phe Trp Ala Phe Leu Leu Val Phe Ser Leu Trp Ser Glu Pro Val Val
435 440 445
Cys Gly Ile Gly Leu Ala Ile Met Leu Thr Gly Val Pro Val Tyr Phe
450 455 460
Leu Gly Val Tyr Trp Gln His Lys Pro Lys Cys Phe Ser Asp Phe Ile
465 470 475 480
Glu Leu Leu Thr Leu Val Ser Gln Lys Met Cys Val Val Val Tyr Pro
485 490 495
Glu Val Glu Arg Gly Ser Gly Thr Glu Glu Ala Asn Glu Asp Met Glu
500 505 510
Glu Gln Gln Gln Pro Met Tyr Gln Pro Thr Pro Thr Lys Asp Lys Asp
515 520 525
Val Ala Gly Gln Pro Gln Pro
530 535

<210> 190
<211> 225
<212> PRT
<213> Homo sapiens
<400> 190
Met Asn Ser Asn Val Glu Asn Leu Pro Pro His Ile Ile Arg Leu Val
1 5 10 15
Tyr Lys Glu Val Thr Thr Leu Thr Ala Asp Pro Pro Asp Gly Ile Lys
20 25 30

Val Phe Pro Asn Glu Glu Asp Leu Thr Asp Leu Gln Val Thr Ile Glu
35 40 45
Gly Pro Glu Gly Thr Pro Tyr Ala Gly Gly Leu Phe Arg Met Lys Leu
50 55 60
Leu Leu Gly Lys Asp Phe Pro Ala Ser Pro Pro Lys Gly Tyr Phe Leu
65 70 75 80
Thr Lys Ile Phe His Pro Asn Val Gly Ala Asn Gly Glu Ile Cys Val
85 90 95
Asn Val Leu Lys Arg Asp Trp Thr Ala Glu Leu Gly Ile Arg His Val
100 105 110
Leu Leu Thr Ile Lys Cys Leu Leu Ile His Pro Asn Pro Glu Ser Ala
115 120 125
Leu Asn Glu Glu Ala Gly Arg Leu Leu Leu Glu Asn Tyr Glu Glu Tyr
130 135 140
Ala Ala Arg Ala Arg Leu Leu Thr Glu Ile His Gly Gly Ala Gly Gly
145 150 155 160
Pro Ser Gly Arg Ala Glu Ala Gly Arg Ala Leu Ala Ser Gly Thr Glu
165 170 175
Ala Ser Ser Thr Asp Pro Gly Ala Pro Gly Gly Pro Gly Gly Ala Glu
180 185 190
Gly Pro Met Ala Lys Lys His Ala Gly Glu Arg Asp Lys Lys Leu Ala
195 200 205
Ala Lys Lys Lys Thr Asp Lys Lys Arg Ala Leu Arg Ala Leu Arg Arg
210 215 220
Leu
225

<210> 191
<211> 485
<212> PRT
<213> Homo sapiens
<400> 191
Met Arg Lys Arg Ala Pro Gln Ser Glu Met Ala Pro Ala Gly Val Ser
1 5 10 15
Leu Arg Ala Thr Ile Leu Cys Leu Leu Ala Trp Ala Gly Leu Ala Ala
20 25 30
Gly Asp Arg Val Tyr Ile His Pro Phe His Leu Val Ile His Asn Glu
35 40 45
Ser Thr Cys Glu Gln Leu Ala Lys Ala Asn Ala Gly Lys Pro Lys Asp
50 55 60
Pro Thr Phe Ile Pro Ala Pro Ile Gln Ala Lys Thr Ser Pro Val Asp
65 70 75 80
Glu Lys Ala Leu Gln Asp Gln Leu Val Leu Val Ala Ala Lys Leu Asp
85 90 95
Thr Glu Asp Lys Leu Arg Ala Ala Met Val Gly Met Leu Ala Asn Phe
100 105 110
Leu Gly Phe Arg Ile Tyr Gly Met His Ser Glu Leu Trp Gly Val Val
115 120 125
His Gly Ala Thr Val Leu Ser Pro Thr Ala Val Phe Gly Thr Leu Ala
130 135 140
Ser Leu Tyr Leu Gly Ala Leu Asp His Thr Ala Asp Arg Leu Gln Ala
145 150 155 160
Ile Leu Gly Val Pro Trp Lys Asp Lys Asn Cys Thr Ser Arg Leu Asp
165 170 175
Ala His Lys Val Leu Ser Ala Leu Gln Ala Val Gln Gly Leu Leu Val
180 185 190
Ala Gln Gly Arg Ala Asp Ser Gln Ala Gln Leu Leu Leu Ser Thr Val
195 200 205
Val Gly Val Phe Thr Ala Pro Gly Leu His Leu Lys Gln Pro Phe Val
210 215 220
Gln Gly Leu Ala Leu Tyr Thr Pro Val Val Leu Pro Arg Ser Leu Asp
225 230 235 240
Phe Thr Glu Leu Asp Val Ala Ala Glu Lys Ile Asp Arg Phe Met Gln
245 250 255
Ala Val Thr Gly Trp Lys Thr Gly Cys Ser Leu Met Gly Ala Ser Val
260 265 270
Asp Ser Thr Leu Ala Phe Asn Thr Tyr Val His Phe Gln Gly Lys Met
275 280 285
Lys Gly Phe Ser Leu Leu Ala Glu Pro Gln Glu Phe Trp Val Asp Asn

290 295 300
Ser Thr Ser Val Ser Val Pro Met Leu Ser Gly Met Gly Thr Phe Gln
305 310 315 320
His Trp Ser Asp Ile Gln Asp Asn Phe Ser Val Thr Gln Val Pro Phe
325 330 335
Thr Glu Ser Ala Cys Leu Leu Leu Ile Gln Pro His Tyr Ala Ser Asp
340 345 350
Leu Asp Lys Val Glu Gly Leu Thr Phe Gln Gln Asn Ser Leu Asn Trp
355 360 365
Met Lys Lys Leu Ser Pro Arg Thr Ile His Leu Thr Met Pro Gln Leu
370 375 380
Val Leu Gln Gly Ser Tyr Asp Leu Gln Asp Leu Leu Ala Gln Ala Glu
385 390 395 400
Leu Pro Ala Ile Leu His Thr Glu Leu Asn Leu Gln Lys Leu Ser Asn
405 410 415
Asp Arg Ile Arg Val Gly Glu Val Leu Asn Ser Ile Phe Phe Glu Leu
420 425 430
Glu Ala Asp Glu Arg Glu Pro Thr Glu Ser Thr Gln Gln Leu Asn Lys
435 440 445
Pro Glu Val Leu Glu Val Thr Leu Asn Arg Pro Phe Leu Phe Ala Val
450 455 460
Tyr Asp Gln Ser Ala Thr Ala Leu His Phe Leu Gly Arg Val Ala Asn
465 470 475 480
Pro Leu Ser Thr Ala
485

<210> 192
<211> 279
<212> PRT
<213> Homo sapiens
<400> 192
Met Thr Glu Arg Phe Asp Cys His His Cys Asn Glu Ser Leu Phe Gly
1 5 10 15
Lys Lys Tyr Ile Leu Arg Glu Glu Ser Pro Tyr Cys Val Val Cys Phe
20 25 30
Glu Thr Leu Phe Ala Asn Thr Cys Glu Glu Cys Gly Lys Pro Ile Gly
35 40 45
Cys Asp Cys Lys Asp Leu Ser Tyr Lys Asp Arg His Trp His Glu Ala
50 55 60
Cys Phe His Cys Ser Gln Cys Arg Asn Ser Leu Val Asp Lys Pro Phe
65 70 75 80
Ala Ala Lys Glu Asp Gln Leu Leu Cys Thr Asp Cys Tyr Ser Asn Glu
85 90 95
Tyr Ser Ser Lys Cys Gln Glu Cys Lys Lys Thr Ile Met Pro Gly Thr
100 105 110
Arg Lys Met Glu Tyr Lys Gly Ser Ser Trp His Glu Thr Cys Phe Ile
115 120 125
Cys His Arg Cys Gln Gln Pro Ile Gly Thr Lys Ser Phe Ile Pro Lys
130 135 140
Asp Asn Gln Asn Phe Cys Val Pro Cys Tyr Glu Lys Gln His Ala Met
145 150 155 160
Gln Cys Val Gln Cys Lys Lys Pro Ile Thr Thr Gly Gly Val Thr Tyr
165 170 175
Arg Glu Gln Pro Trp His Lys Glu Cys Phe Val Cys Thr Ala Cys Arg
180 185 190
Lys Gln Leu Ser Gly Gln Arg Phe Thr Ala Arg Asp Asp Phe Ala Tyr
195 200 205
Cys Leu Asn Cys Phe Cys Asp Leu Tyr Ala Lys Lys Cys Ala Gly Cys
210 215 220
Thr Asn Pro Ile Ser Gly Leu Gly Gly Thr Lys Tyr Ile Ser Phe Glu
225 230 235 240
Glu Arg Gln Trp His Asn Asp Cys Phe Asn Cys Lys Lys Cys Ser Leu
245 250 255
Ser Leu Val Gly Arg Gly Phe Leu Thr Glu Arg Asp Asp Ile Leu Cys
260 265 270
Pro Asp Cys Gly Lys Asp Ile
275

<210> 193

<211> 738
<212> PRT
<213> Homo sapiens
<400> 193

```
Met Glu Lys Ser Arg Met Asn Leu Pro Lys Gly Pro Asp Thr Leu Cys
1               5                   10                  15
Phe Asp Lys Asp Glu Phe Met Lys Glu Asp Phe Asp Val Asp His Phe
            20                  25                  30
Val Ser Asp Cys Arg Lys Arg Val Gln Leu Glu Glu Leu Arg Asp Asp
        35                  40                  45
Leu Glu Leu Tyr Tyr Lys Leu Leu Lys Thr Ala Met Val Glu Leu Ile
    50                  55                  60
Asn Lys Asp Tyr Ala Asp Phe Val Asn Leu Ser Thr Asn Leu Val Gly
65                  70                  75                  80
Met Asp Lys Ala Leu Asn Gln Leu Ser Val Pro Leu Gly Gln Leu Arg
                85                  90                  95
Glu Glu Val Leu Ser Leu Arg Ser Ser Val Ser Glu Gly Ile Arg Ala
            100                 105                 110
Val Asp Glu Arg Met Ser Lys Gln Glu Asp Ile Arg Lys Lys Lys Met
        115                 120                 125
Cys Val Leu Arg Leu Ile Gln Val Ile Arg Ser Val Glu Lys Ile Glu
    130                 135                 140
Lys Ile Leu Asn Ser Gln Ser Ser Lys Glu Thr Ser Ala Leu Glu Ala
145                 150                 155                 160
Ser Ser Pro Leu Leu Thr Gly Gln Ile Leu Glu Arg Ile Ala Thr Glu
                165                 170                 175
Phe Asn Gln Leu Gln Phe His Ala Val Gln Ser Lys Gly Met Pro Leu
            180                 185                 190
Leu Asp Lys Val Arg Pro Arg Ile Ala Gly Ile Thr Ala Met Leu Gln
        195                 200                 205
Gln Ser Leu Glu Gly Leu Leu Leu Glu Gly Leu Gln Thr Ser Asp Val
    210                 215                 220
Asp Ile Ile Arg His Cys Leu Arg Thr Tyr Ala Thr Ile Asp Lys Thr
225                 230                 235                 240
Arg Asp Ala Glu Ala Leu Val Gly Gln Val Leu Val Lys Pro Tyr Ile
                245                 250                 255
Asp Glu Val Ile Ile Glu Gln Phe Val Glu Ser His Pro Asn Gly Leu
            260                 265                 270
Gln Val Met Tyr Asn Lys Leu Leu Glu Phe Val Pro His His Cys Arg
        275                 280                 285
Leu Leu Arg Glu Val Thr Gly Gly Ala Ile Ser Ser Glu Lys Gly Asn
    290                 295                 300
Thr Val Pro Gly Tyr Asp Phe Leu Val Asn Ser Val Trp Pro Gln Ile
305                 310                 315                 320
Val Gln Gly Leu Glu Glu Lys Leu Pro Ser Leu Phe Asn Pro Gly Asn
                325                 330                 335
Pro Asp Ala Phe His Glu Lys Tyr Thr Ile Ser Met Asp Phe Val Arg
            340                 345                 350
Arg Leu Glu Arg Gln Cys Gly Ser Gln Ala Ser Val Lys Arg Leu Arg
        355                 360                 365
Ala His Pro Ala Tyr His Ser Phe Asn Lys Lys Trp Asn Leu Pro Val
    370                 375                 380
Tyr Phe Gln Ile Arg Phe Arg Glu Ile Ala Gly Ser Leu Glu Ala Ala
385                 390                 395                 400
Leu Thr Asp Val Leu Glu Asp Ala Pro Ala Glu Ser Pro Tyr Cys Leu
                405                 410                 415
Leu Ala Ser His Arg Thr Trp Ser Ser Leu Arg Arg Cys Trp Ser Asp
            420                 425                 430
Glu Met Phe Leu Pro Leu Leu Val His Arg Leu Trp Arg Leu Thr Leu
        435                 440                 445
Gln Ile Leu Ala Arg Tyr Ser Val Phe Val Asn Glu Leu Ser Leu Arg
    450                 455                 460
Pro Ile Ser Asn Glu Ser Pro Lys Glu Ile Lys Lys Pro Leu Val Thr
465                 470                 475                 480
Gly Ser Lys Glu Pro Ser Ile Thr Gln Gly Asn Thr Glu Asp Gln Gly
                485                 490                 495
Ser Gly Pro Ser Glu Thr Lys Pro Val Val Ser Ile Ser Arg Thr Gln
            500                 505                 510
Leu Val Tyr Val Val Ala Asp Leu Asp Lys Leu Gln Glu Gln Leu Pro
```

515 520 525
Glu Leu Leu Glu Ile Ile Lys Pro Lys Leu Glu Met Ile Gly Phe Lys
530 535 540
Asn Phe Ser Ser Ile Ser Ala Ala Leu Glu Asp Ser Gln Ser Ser Phe
545 550 555 560
Ser Ala Cys Val Pro Ser Leu Ser Ser Lys Ile Ile Gln Asp Leu Ser
565 570 575
Asp Ser Cys Phe Gly Phe Leu Lys Ser Ala Leu Glu Val Pro Arg Leu
580 585 590
Tyr Arg Arg Thr Asn Lys Glu Val Pro Thr Thr Ala Ser Ser Tyr Val
595 600 605
Asp Ser Ala Leu Lys Pro Leu Phe Gln Leu Gln Ser Gly His Lys Asp
610 615 620
Lys Leu Lys Gln Ala Ile Ile Gln Gln Trp Leu Glu Gly Thr Leu Ser
625 630 635 640
Glu Ser Thr His Lys Tyr Tyr Glu Thr Val Ser Asp Val Leu Asn Ser
645 650 655
Val Lys Lys Met Glu Glu Ser Leu Lys Arg Leu Lys Gln Ala Arg Lys
660 665 670
Thr Thr Pro Ala Asn Pro Val Gly Pro Ser Gly Gly Met Ser Asp Asp
675 680 685
Asp Lys Ile Arg Leu Gln Leu Ala Leu Asp Val Glu Tyr Leu Gly Glu
690 695 700
Gln Ile Gln Lys Leu Gly Leu Gln Ala Ser Asp Ile Lys Ser Phe Ser
705 710 715 720
Ala Leu Ala Glu Leu Val Ala Ala Ala Lys Asp Gln Ala Thr Ala Glu
725 730 735
Gln Pro

<210> 194
<211> 963
<212> PRT
<213> Homo sapiens
<400> 194
Met Ala Val Phe Pro Trp His Ser Arg Asn Arg Asn Tyr Lys Ala Glu
1 5 10 15
Phe Ala Ser Cys Arg Leu Glu Ala Val Pro Leu Glu Phe Gly Asp Tyr
20 25 30
His Pro Leu Lys Pro Ile Thr Val Thr Glu Ser Lys Thr Lys Lys Val
35 40 45
Asn Arg Lys Gly Ser Thr Ser Ser Thr Ser Ser Ser Ser Ser Ser Ser
50 55 60
Val Val Asp Pro Leu Ser Ser Val Leu Asp Gly Thr Asp Pro Leu Ser
65 70 75 80
Met Phe Ala Ala Thr Ala Asp Pro Ala Ala Leu Ala Ala Ala Met Asp
85 90 95
Ser Ser Arg Arg Lys Arg Asp Arg Asp Asp Asn Ser Val Val Gly Ser
100 105 110
Asp Phe Glu Pro Trp Thr Asn Lys Arg Gly Glu Ile Leu Ala Arg Tyr
115 120 125
Thr Thr Thr Glu Lys Leu Ser Ile Asn Leu Phe Met Gly Ser Glu Lys
130 135 140
Gly Lys Ala Gly Thr Ala Thr Leu Ala Met Ser Glu Lys Val Arg Thr
145 150 155 160
Arg Leu Glu Glu Leu Asp Asp Phe Glu Glu Gly Ser Gln Lys Glu Leu
165 170 175
Leu Asn Leu Thr Gln Gln Asp Tyr Val Asn Arg Ile Glu Glu Leu Asn
180 185 190
Gln Ser Leu Lys Asp Ala Trp Ala Ser Asp Gln Lys Val Lys Ala Leu
195 200 205
Lys Ile Val Ile Gln Cys Ser Lys Leu Leu Ser Asp Thr Ser Val Ile
210 215 220
Gln Phe Tyr Pro Ser Lys Phe Val Leu Ile Thr Asp Ile Leu Asp Thr
225 230 235 240
Phe Gly Lys Leu Val Tyr Glu Arg Ile Phe Ser Met Cys Val Asp Ser
245 250 255
Arg Ser Val Leu Pro Asp His Phe Ser Pro Glu Asn Ala Asn Asp Thr
260 265 270

Ala Lys Glu Thr Cys Leu Asn Trp Phe Phe Lys Ile Ala Ser Ile Arg
275 280 285
Glu Leu Ile Pro Arg Phe Tyr Val Glu Ala Ser Ile Leu Lys Cys Asn
290 295 300
Lys Phe Leu Ser Lys Thr Gly Ile Ser Glu Cys Leu Pro Arg Leu Thr
305 310 315 320
Cys Met Ile Arg Gly Ile Gly Asp Pro Leu Val Ser Val Tyr Ala Arg
325 330 335
Ala Tyr Leu Cys Arg Val Gly Met Glu Val Ala Pro His Leu Lys Glu
340 345 350
Thr Leu Asn Lys Asn Phe Phe Asp Phe Leu Leu Thr Phe Lys Gln Ile
355 360 365
His Gly Asp Thr Val Gln Asn Gln Leu Val Val Gln Gly Val Glu Leu
370 375 380
Pro Ser Tyr Leu Pro Leu Tyr Pro Pro Ala Met Asp Trp Ile Phe Gln
385 390 395 400
Cys Ile Ser Tyr His Ala Pro Glu Ala Leu Leu Thr Glu Met Met Glu
405 410 415
Arg Cys Lys Lys Leu Gly Asn Asn Ala Leu Leu Leu Asn Ser Val Met
420 425 430
Ser Ala Phe Arg Ala Glu Phe Ile Ala Thr Arg Ser Met Asp Phe Ile
435 440 445
Gly Met Ile Lys Glu Cys Asp Glu Ser Gly Phe Pro Lys His Leu Leu
450 455 460
Phe Arg Ser Leu Gly Leu Asn Leu Ala Leu Ala Asp Pro Pro Glu Ser
465 470 475 480
Asp Arg Leu Gln Ile Leu Asn Glu Ala Trp Lys Val Ile Thr Lys Leu
485 490 495
Lys Asn Pro Gln Asp Tyr Ile Asn Cys Ala Glu Val Trp Val Glu Tyr
500 505 510
Thr Cys Lys His Phe Thr Lys Arg Glu Val Asn Thr Val Leu Ala Asp
515 520 525
Val Ile Lys His Met Thr Pro Asp Arg Ala Phe Glu Asp Ser Tyr Pro
530 535 540
Gln Leu Gln Leu Ile Ile Lys Lys Val Ile Ala His Phe His Asp Phe
545 550 555 560
Ser Val Leu Phe Ser Val Glu Lys Phe Leu Pro Phe Leu Asp Met Phe
565 570 575
Gln Lys Glu Ser Val Arg Val Glu Val Cys Lys Cys Ile Met Asp Ala
580 585 590
Phe Ile Lys His Gln Gln Glu Pro Thr Lys Asp Pro Val Ile Leu Asn
595 600 605
Ala Leu Leu His Val Cys Lys Thr Met His Asp Ser Val Asn Ala Leu
610 615 620
Thr Leu Glu Asp Glu Lys Arg Met Leu Ser Tyr Leu Ile Asn Gly Phe
625 630 635 640
Ile Lys Met Val Ser Phe Gly Arg Asp Phe Glu Gln Gln Leu Ser Phe
645 650 655
Tyr Val Glu Ser Arg Ser Met Phe Cys Asn Leu Glu Pro Val Leu Val
660 665 670
Gln Leu Ile His Ser Val Asn Arg Leu Ala Met Glu Thr Arg Lys Val
675 680 685
Met Lys Gly Asn His Ser Arg Lys Thr Ala Ala Phe Val Arg Ala Cys
690 695 700
Val Ala Tyr Cys Phe Ile Thr Ile Pro Ser Leu Ala Gly Ile Phe Thr
705 710 715 720
Arg Leu Asn Leu Tyr Leu His Ser Gly Gln Val Ala Leu Ala Asn Gln
725 730 735
Cys Leu Ser Gln Ala Asp Ala Phe Phe Lys Ala Ala Ile Ser Leu Val
740 745 750
Pro Glu Val Pro Lys Met Ile Asn Ile Asp Gly Lys Met Arg Pro Ser
755 760 765
Glu Ser Phe Leu Leu Glu Phe Leu Cys Asn Phe Phe Ser Thr Leu Leu
770 775 780
Ile Val Pro Asp His Pro Glu His Gly Val Leu Phe Leu Val Arg Glu
785 790 795 800
Leu Leu Asn Val Ile Gln Asp Tyr Thr Trp Glu Asp Asn Ser Asp Glu
805 810 815
Lys Ile Arg Ile Tyr Thr Cys Val Leu His Leu Leu Ser Ala Met Ser

```
            820                     825                     830
Gln Glu Thr Tyr Leu Tyr His Ile Asp Lys Val Asp Ser Asn Asp Ser
        835                     840                     845
Leu Tyr Gly Gly Asp Ser Lys Phe Leu Ala Glu Asn Asn Lys Leu Cys
    850                     855                     860
Glu Thr Val Met Ala Gln Ile Leu Glu His Leu Lys Thr Leu Ala Lys
865                     870                     875                 880
Asp Glu Ala Leu Lys Arg Gln Ser Ser Leu Gly Leu Ser Phe Phe Asn
                885                     890                     895
Ser Ile Leu Ala His Gly Asp Leu Arg Asn Asn Lys Leu Asn Gln Leu
            900                     905                     910
Ser Val Asn Leu Trp His Leu Ala Gln Arg His Gly Cys Ala Asp Thr
        915                     920                     925
Arg Thr Met Val Lys Thr Leu Glu Tyr Ile Lys Lys Gln Ser Lys Gln
    930                     935                     940
Pro Asp Met Thr His Leu Thr Glu Leu Ala Leu Arg Leu Pro Leu Gln
945                     950                     955                 960
Thr Arg Thr


<210>  195
<211>  494
<212>  PRT
<213>  Homo sapiens
<400>  195
Met Phe Glu Ile Lys Lys Ile Cys Cys Ile Gly Ala Gly Tyr Val Gly
1               5                   10                  15
Gly Pro Thr Cys Ser Val Ile Ala His Met Cys Pro Glu Ile Arg Val
            20                  25                  30
Thr Val Val Asp Val Asn Glu Ser Arg Ile Asn Ala Trp Asn Ser Pro
        35                  40                  45
Thr Leu Pro Ile Tyr Glu Pro Gly Leu Lys Glu Val Val Glu Ser Cys
    50                  55                  60
Arg Gly Lys Asn Leu Phe Phe Ser Thr Asn Ile Asp Asp Ala Ile Lys
65                  70                  75                  80
Glu Ala Asp Leu Val Phe Ile Ser Val Asn Thr Pro Thr Lys Thr Tyr
                85                  90                  95
Gly Met Gly Lys Gly Arg Ala Ala Asp Leu Lys Tyr Ile Glu Ala Cys
            100                 105                 110
Ala Arg Arg Ile Val Gln Asn Ser Asn Gly Tyr Lys Ile Val Thr Glu
        115                 120                 125
Lys Ser Thr Val Pro Val Arg Ala Ala Glu Ser Ile Arg Arg Ile Phe
    130                 135                 140
Asp Ala Asn Thr Lys Pro Asn Leu Asn Leu Gln Val Leu Ser Asn Pro
145                 150                 155                 160
Glu Phe Leu Ala Glu Gly Thr Ala Ile Lys Asp Leu Lys Asn Pro Asp
                165                 170                 175
Arg Val Leu Ile Gly Gly Asp Glu Thr Pro Glu Gly Gln Arg Ala Val
            180                 185                 190
Gln Ala Leu Cys Ala Val Tyr Glu His Trp Val Pro Arg Glu Lys Ile
        195                 200                 205
Leu Thr Thr Asn Thr Trp Ser Ser Glu Leu Ser Lys Leu Ala Ala Asn
    210                 215                 220
Ala Phe Leu Ala Gln Arg Ile Ser Ser Ile Asn Ser Ile Ser Ala Leu
225                 230                 235                 240
Cys Glu Ala Thr Gly Ala Asp Val Glu Glu Val Ala Thr Ala Ile Gly
                245                 250                 255
Met Asp Gln Arg Ile Gly Asn Lys Phe Leu Lys Ala Ser Val Gly Phe
            260                 265                 270
Gly Gly Ser Cys Phe Gln Lys Asp Val Leu Asn Leu Val Tyr Leu Cys
        275                 280                 285
Glu Ala Leu Asn Leu Pro Glu Val Ala Arg Tyr Trp Gln Gln Val Ile
    290                 295                 300
Asp Met Asn Asp Tyr Gln Arg Arg Arg Phe Ala Ser Arg Ile Ile Asp
305                 310                 315                 320
Ser Leu Phe Asn Thr Val Thr Asp Lys Lys Ile Ala Ile Leu Gly Phe
                325                 330                 335
Ala Phe Lys Lys Asp Thr Gly Asp Thr Arg Glu Ser Ser Ser Ile Tyr
            340                 345                 350
```

Ile Ser Lys Tyr Leu Met Asp Glu Gly Ala His Leu His Ile Tyr Asp
355 360 365
Pro Lys Val Pro Arg Glu Gln Ile Val Val Asp Leu Ser His Pro Gly
370 375 380
Val Ser Glu Asp Asp Gln Val Ser Arg Leu Val Thr Ile Ser Lys Asp
385 390 395 400
Pro Tyr Glu Ala Cys Asp Gly Ala His Ala Val Val Ile Cys Thr Glu
405 410 415
Trp Asp Met Phe Lys Glu Leu Asp Tyr Glu Arg Ile His Lys Lys Met
420 425 430
Leu Lys Pro Ala Phe Ile Phe Asp Gly Arg Arg Val Leu Asp Gly Leu
435 440 445
His Asn Glu Leu Gln Thr Ile Gly Phe Gln Ile Glu Thr Ile Gly Lys
450 455 460
Lys Val Ser Ser Lys Arg Ile Pro Tyr Ala Pro Ser Gly Glu Ile Pro
465 470 475 480
Lys Phe Ser Leu Gln Asp Pro Pro Asn Lys Lys Pro Lys Val
485 490

<210> 196
<211> 205
<212> PRT
<213> Homo sapiens
<400> 196
Met Ala Leu Gln Leu Ser Arg Glu Gln Gly Ile Thr Leu Arg Gly Ser
1 5 10 15
Ala Glu Ile Val Ala Glu Phe Phe Ser Phe Gly Ile Asn Ser Ile Leu
20 25 30
Tyr Gln Arg Gly Ile Tyr Pro Ser Glu Thr Phe Thr Arg Val Gln Lys
35 40 45
Tyr Gly Leu Thr Leu Leu Val Thr Thr Asp Leu Glu Leu Ile Lys Tyr
50 55 60
Leu Asn Asn Val Val Glu Gln Leu Lys Asp Trp Leu Tyr Lys Cys Ser
65 70 75 80
Val Gln Lys Leu Val Val Val Ile Ser Asn Ile Glu Ser Gly Glu Val
85 90 95
Leu Glu Arg Trp Gln Phe Asp Ile Glu Cys Asp Lys Thr Ala Lys Asp
100 105 110
Asp Ser Ala Pro Arg Glu Lys Ser Gln Lys Ala Ile Gln Asp Glu Ile
115 120 125
Arg Ser Val Ile Arg Gln Ile Thr Ala Thr Val Thr Phe Leu Pro Leu
130 135 140
Leu Glu Val Ser Cys Ser Phe Asp Leu Leu Ile Tyr Thr Asp Lys Asp
145 150 155 160
Leu Val Val Pro Glu Lys Trp Glu Glu Ser Gly Pro Gln Phe Ile Thr
165 170 175
Asn Ser Glu Glu Val Arg Leu Arg Ser Phe Thr Thr Thr Ile His Lys
180 185 190
Val Asn Ser Met Val Ala Tyr Lys Ile Pro Val Asn Asp
195 200 205

<210> 197
<211> 427
<212> PRT
<213> Homo sapiens
<400> 197
Met Ala Pro Lys Lys Arg Pro Glu Thr Gln Lys Thr Ser Glu Ile Val
1 5 10 15
Leu Arg Pro Arg Asn Lys Arg Ser Arg Ser Pro Leu Glu Leu Glu Pro
20 25 30
Glu Ala Lys Lys Leu Cys Ala Lys Gly Ser Gly Pro Ser Arg Arg Cys
35 40 45
Asp Ser Asp Cys Leu Trp Val Gly Leu Ala Gly Pro Gln Ile Leu Pro
50 55 60
Pro Cys Arg Ser Ile Val Arg Thr Leu His Gln His Lys Leu Gly Arg
65 70 75 80
Ala Ser Trp Pro Ser Val Gln Gln Gly Leu Gln Gln Ser Phe Leu His
85 90 95
Thr Leu Asp Ser Tyr Arg Ile Leu Gln Lys Ala Ala Pro Phe Asp Arg

100 105 110
Arg Ala Thr Ser Leu Ala Trp His Pro Thr His Pro Ser Thr Val Ala
115 120 125
Val Gly Ser Lys Gly Gly Asp Ile Met Leu Trp Asn Phe Gly Ile Lys
130 135 140
Asp Lys Pro Thr Phe Ile Lys Gly Ile Gly Ala Gly Gly Ser Ile Thr
145 150 155 160
Gly Leu Lys Phe Asn Pro Leu Asn Thr Asn Gln Phe Tyr Ala Ser Ser
165 170 175
Met Glu Gly Thr Thr Arg Leu Gln Asp Phe Lys Gly Asn Ile Leu Arg
180 185 190
Val Phe Ala Ser Ser Asp Thr Ile Asn Ile Trp Phe Cys Ser Leu Asp
195 200 205
Val Ser Ala Ser Ser Arg Met Val Val Thr Gly Asp Asn Val Gly Asn
210 215 220
Val Ile Leu Leu Asn Met Asp Gly Lys Glu Leu Trp Asn Leu Arg Met
225 230 235 240
His Lys Lys Lys Val Thr His Val Ala Leu Asn Pro Cys Cys Asp Trp
245 250 255
Phe Leu Ala Thr Ala Ser Val Asp Gln Thr Val Lys Ile Trp Asp Leu
260 265 270
Arg Gln Val Arg Gly Lys Ala Ser Phe Leu Tyr Ser Leu Pro His Arg
275 280 285
His Pro Val Asn Ala Ala Cys Phe Ser Pro Asp Gly Ala Arg Leu Leu
290 295 300
Thr Thr Asp Gln Lys Ser Glu Ile Arg Val Tyr Ser Ala Ser Gln Trp
305 310 315 320
Asp Cys Pro Leu Gly Leu Ile Pro His Pro His Arg His Phe Gln His
325 330 335
Leu Thr Pro Ile Lys Ala Ala Trp His Pro Arg Tyr Asn Leu Ile Val
340 345 350
Val Gly Arg Tyr Pro Asp Pro Asn Phe Lys Ser Cys Thr Pro Tyr Glu
355 360 365
Leu Arg Thr Ile Asp Val Phe Asp Gly Asn Ser Gly Lys Met Met Cys
370 375 380
Gln Leu Tyr Asp Pro Glu Ser Ser Gly Ile Ser Ser Leu Asn Glu Phe
385 390 395 400
Asn Pro Met Gly Asp Thr Leu Ala Ser Ala Met Gly Tyr His Ile Leu
405 410 415
Ile Trp Ser Gln Glu Glu Ala Arg Thr Arg Lys
420 425

<210> 198
<211> 283
<212> PRT
<213> Homo sapiens
<400> 198
Met Glu His Gly Ser Ile Ile Thr Gln Ala Arg Arg Glu Asp Ala Leu
1 5 10 15
Val Leu Thr Lys Gln Gly Leu Val Ser Lys Ser Ser Pro Lys Lys Pro
20 25 30
Arg Gly Arg Asn Ile Phe Lys Ala Leu Phe Cys Cys Phe Arg Ala Gln
35 40 45
His Val Gly Gln Ser Ser Ser Ser Thr Glu Leu Ala Ala Tyr Lys Glu
50 55 60
Glu Ala Asn Thr Ile Ala Lys Ser Asp Leu Leu Gln Cys Leu Gln Tyr
65 70 75 80
Gln Phe Tyr Gln Ile Pro Gly Thr Cys Leu Leu Pro Glu Val Thr Glu
85 90 95
Glu Asp Gln Gly Arg Ile Cys Val Val Ile Asp Leu Asp Glu Thr Leu
100 105 110
Val His Ser Ser Phe Lys Pro Ile Asn Asn Ala Asp Phe Ile Val Pro
115 120 125
Ile Glu Ile Glu Gly Thr Thr His Gln Val Tyr Val Leu Lys Arg Pro
130 135 140
Tyr Val Asp Glu Phe Leu Arg Arg Met Gly Glu Leu Phe Glu Cys Val
145 150 155 160
Leu Phe Thr Ala Ser Leu Ala Lys Tyr Ala Asp Pro Val Thr Asp Leu
165 170 175

Leu Asp Arg Cys Gly Val Phe Arg Ala Arg Leu Phe Arg Glu Ser Cys
180 185 190
Val Phe His Gln Gly Cys Tyr Val Lys Asp Leu Ser Arg Leu Gly Arg
195 200 205
Asp Leu Arg Lys Thr Leu Ile Leu Asp Asn Ser Pro Ala Ser Tyr Ile
210 215 220
Phe His Pro Glu Asn Ala Val Pro Val Gln Ser Trp Phe Asp Asp Met
225 230 235 240
Ala Asp Thr Glu Leu Leu Asn Leu Ile Pro Ile Phe Glu Glu Leu Ser
245 250 255
Gly Ala Glu Asp Val Tyr Thr Ser Leu Gly Ala Ala Ala Gly Pro Leu
260 265 270
Ala Cys Pro Ala Ser Lys Arg Arg Pro Ser Gln
275 280

<210> 199
<211> 239
<212> PRT
<213> Homo sapiens
<400> 199
Met Ala His Ala Gly Arg Thr Gly Tyr Asp Asn Arg Glu Ile Val Met
1 5 10 15
Lys Tyr Ile His Tyr Lys Leu Ser Gln Arg Gly Tyr Glu Trp Asp Ala
20 25 30
Gly Asp Val Gly Ala Ala Pro Pro Gly Ala Ala Pro Ala Pro Gly Ile
35 40 45
Phe Ser Ser Gln Pro Gly His Thr Pro His Thr Ala Ala Ser Arg Asp
50 55 60
Pro Val Ala Arg Thr Ser Pro Leu Gln Thr Pro Ala Ala Pro Gly Ala
65 70 75 80
Ala Ala Gly Pro Ala Leu Ser Pro Val Pro Pro Val Val His Leu Thr
85 90 95
Leu Arg Gln Ala Gly Asp Asp Phe Ser Arg Arg Tyr Arg Arg Asp Phe
100 105 110
Ala Glu Met Ser Arg Gln Leu His Leu Thr Pro Phe Thr Ala Arg Gly
115 120 125
Arg Phe Ala Thr Val Val Glu Glu Leu Phe Arg Asp Gly Val Asn Trp
130 135 140
Gly Arg Ile Val Ala Phe Phe Glu Phe Gly Gly Val Met Cys Val Glu
145 150 155 160
Ser Val Asn Arg Glu Met Ser Pro Leu Val Asp Asn Ile Ala Leu Trp
165 170 175
Met Thr Glu Tyr Leu Asn Arg His Leu His Thr Trp Ile Gln Asp Asn
180 185 190
Gly Gly Trp Asp Ala Phe Val Glu Leu Tyr Gly Pro Ser Met Arg Pro
195 200 205
Leu Phe Asp Phe Ser Trp Leu Ser Leu Lys Thr Leu Leu Ser Leu Ala
210 215 220
Leu Val Gly Ala Cys Ile Thr Leu Gly Ala Tyr Leu Gly His Lys
225 230 235

<210> 200
<211> 751
<212> PRT
<213> Homo sapiens
<400> 200
Met Ala Phe Arg Thr Ile Cys Val Leu Val Gly Val Phe Ile Cys Ser
1 5 10 15
Ile Cys Val Lys Gly Ser Ser Gln Pro Gln Ala Arg Val Tyr Leu Thr
20 25 30
Phe Asp Glu Leu Arg Glu Thr Lys Thr Ser Glu Tyr Phe Ser Leu Ser
35 40 45
His His Pro Leu Asp Tyr Arg Ile Leu Leu Met Asp Glu Asp Gln Asp
50 55 60
Arg Ile Tyr Val Gly Ser Lys Asp His Ile Leu Ser Leu Asn Ile Asn
65 70 75 80
Asn Ile Ser Gln Glu Ala Leu Ser Val Phe Trp Pro Ala Ser Thr Ile
85 90 95
Lys Val Glu Glu Cys Lys Met Ala Gly Lys Asp Pro Thr His Gly Cys

```
                    100                 105                 110
Gly Asn Phe Val Arg Val Ile Gln Thr Phe Asn Arg Thr His Leu Tyr
        115                 120                 125
Val Cys Gly Ser Gly Ala Phe Ser Pro Val Cys Thr Tyr Leu Asn Arg
    130                 135                 140
Gly Arg Arg Ser Glu Asp Gln Val Phe Met Ile Asp Ser Lys Cys Glu
145                 150                 155                 160
Ser Gly Lys Gly Arg Cys Ser Phe Asn Pro Asn Val Asn Thr Val Ser
                165                 170                 175
Val Met Ile Asn Glu Glu Leu Phe Ser Gly Met Tyr Ile Asp Phe Met
            180                 185                 190
Gly Thr Asp Ala Ala Ile Phe Arg Ser Leu Thr Lys Arg Asn Ala Val
        195                 200                 205
Arg Thr Asp Gln His Asn Ser Lys Trp Leu Ser Glu Pro Met Phe Val
    210                 215                 220
Asp Ala His Val Ile Pro Asp Gly Thr Asp Pro Asn Asp Ala Lys Val
225                 230                 235                 240
Tyr Phe Phe Phe Lys Glu Lys Leu Thr Asp Asn Asn Arg Ser Thr Lys
                245                 250                 255
Gln Ile His Ser Met Ile Ala Arg Ile Cys Pro Asn Asp Thr Gly Gly
            260                 265                 270
Leu Arg Ser Leu Val Asn Lys Trp Thr Thr Phe Leu Lys Ala Arg Leu
        275                 280                 285
Val Cys Ser Val Thr Asp Glu Asp Gly Pro Glu Thr His Phe Asp Glu
    290                 295                 300
Leu Glu Asp Val Phe Leu Leu Glu Thr Asp Asn Pro Arg Thr Thr Leu
305                 310                 315                 320
Val Tyr Gly Ile Phe Thr Thr Ser Ser Ser Val Phe Lys Gly Ser Ala
                325                 330                 335
Val Cys Val Tyr His Leu Ser Asp Ile Gln Thr Val Phe Asn Gly Pro
            340                 345                 350
Phe Ala His Lys Glu Gly Pro Asn His Gln Leu Ile Ser Tyr Gln Gly
        355                 360                 365
Arg Ile Pro Tyr Pro Arg Pro Gly Thr Cys Pro Gly Gly Ala Phe Thr
    370                 375                 380
Pro Asn Met Arg Thr Thr Lys Glu Phe Pro Asp Asp Val Val Thr Phe
385                 390                 395                 400
Ile Arg Asn His Pro Leu Met Tyr Asn Ser Ile Tyr Pro Ile His Lys
                405                 410                 415
Arg Pro Leu Ile Val Arg Ile Gly Thr Asp Tyr Lys Tyr Thr Lys Ile
            420                 425                 430
Ala Val Asp Arg Val Asn Ala Ala Asp Gly Arg Tyr His Val Leu Phe
        435                 440                 445
Leu Gly Thr Asp Arg Gly Thr Val Gln Lys Val Val Val Leu Pro Thr
    450                 455                 460
Asn Asn Ser Val Ser Gly Glu Leu Ile Leu Glu Glu Leu Glu Val Phe
465                 470                 475                 480
Lys Asn His Ala Pro Ile Thr Thr Met Lys Ile Ser Ser Lys Lys Gln
                485                 490                 495
Gln Leu Tyr Val Ser Ser Asn Glu Gly Val Ser Gln Val Ser Leu His
            500                 505                 510
Arg Cys His Ile Tyr Gly Thr Ala Cys Ala Asp Cys Cys Leu Ala Arg
        515                 520                 525
Asp Pro Tyr Cys Ala Trp Asp Gly His Ser Cys Ser Arg Phe Tyr Pro
    530                 535                 540
Thr Gly Lys Arg Arg Ser Arg Arg Gln Asp Val Arg His Gly Asn Pro
545                 550                 555                 560
Leu Thr Gln Cys Arg Gly Phe Asn Leu Lys Ala Tyr Arg Asn Ala Ala
                565                 570                 575
Glu Ile Val Gln Tyr Gly Val Lys Asn Asn Thr Thr Phe Leu Glu Cys
            580                 585                 590
Ala Pro Lys Ser Pro Gln Ala Ser Ile Lys Trp Leu Leu Gln Lys Asp
        595                 600                 605
Lys Asp Arg Arg Lys Glu Val Lys Leu Asn Glu Arg Ile Ile Ala Thr
    610                 615                 620
Ser Gln Gly Leu Leu Ile Arg Ser Val Gln Gly Ser Asp Gln Gly Leu
625                 630                 635                 640
Tyr His Cys Ile Ala Thr Glu Asn Ser Phe Lys Gln Thr Ile Ala Lys
                645                 650                 655
```

Ile Asn Phe Lys Val Leu Asp Ser Glu Met Val Ala Val Val Thr Asp
660 665 670
Lys Trp Ser Pro Trp Thr Trp Ala Ser Ser Val Arg Ala Leu Pro Phe
675 680 685
His Pro Lys Asp Ile Met Gly Ala Phe Ser His Ser Glu Met Gln Met
690 695 700
Ile Asn Gln Tyr Cys Lys Asp Thr Arg Gln Gln His Gln Gln Gly Asp
705 710 715 720
Glu Ser Gln Lys Met Arg Gly Asp Tyr Gly Lys Leu Lys Ala Leu Ile
725 730 735
Asn Ser Arg Lys Ser Arg Asn Arg Arg Asn Gln Leu Pro Glu Ser
740 745 750

<210> 201
<211> 208
<212> PRT
<213> Homo sapiens
<400> 201
Met Lys Leu Leu Pro Ser Val Val Leu Lys Leu Phe Leu Ala Ala Val
1 5 10 15
Leu Ser Ala Leu Val Thr Gly Glu Ser Leu Glu Arg Leu Arg Arg Gly
20 25 30
Leu Ala Ala Gly Thr Ser Asn Pro Asp Pro Pro Thr Val Ser Thr Asp
35 40 45
Gln Leu Leu Pro Leu Gly Gly Gly Arg Asp Arg Lys Val Arg Asp Leu
50 55 60
Gln Glu Ala Asp Leu Asp Leu Leu Arg Val Thr Leu Ser Ser Lys Pro
65 70 75 80
Gln Ala Leu Ala Thr Pro Asn Lys Glu Glu His Gly Lys Arg Lys Lys
85 90 95
Lys Gly Lys Gly Leu Gly Lys Lys Arg Asp Pro Cys Leu Arg Lys Tyr
100 105 110
Lys Asp Phe Cys Ile His Gly Glu Cys Lys Tyr Val Lys Glu Leu Arg
115 120 125
Ala Pro Ser Cys Ile Cys His Pro Gly Tyr His Gly Glu Arg Cys His
130 135 140
Gly Leu Ser Leu Pro Val Glu Asn Arg Leu Tyr Thr Tyr Asp His Thr
145 150 155 160
Thr Ile Leu Ala Val Val Ala Val Val Leu Ser Ser Val Cys Leu Leu
165 170 175
Val Ile Val Gly Leu Leu Met Phe Arg Tyr His Arg Arg Gly Gly Tyr
180 185 190
Asp Val Glu Asn Glu Glu Lys Val Lys Leu Gly Met Thr Asn Ser His
195 200 205

<210> 202
<211> 662
<212> PRT
<213> Homo sapiens
<400> 202
Met Arg Pro Gln Ile Leu Leu Leu Leu Ala Leu Leu Thr Leu Gly Leu
1 5 10 15
Ala Ala Gln His Gln Asp Lys Val Pro Cys Lys Met Val Asp Lys Lys
20 25 30
Val Ser Cys Gln Val Leu Gly Leu Leu Gln Val Pro Ser Val Leu Pro
35 40 45
Pro Asp Thr Glu Thr Leu Asp Leu Ser Gly Asn Gln Leu Arg Ser Ile
50 55 60
Leu Ala Ser Pro Leu Gly Phe Tyr Thr Ala Leu Arg His Leu Asp Leu
65 70 75 80
Ser Thr Asn Glu Ile Ser Phe Leu Gln Pro Gly Ala Phe Gln Ala Leu
85 90 95
Thr His Leu Glu His Leu Ser Leu Ala His Asn Arg Leu Ala Met Ala
100 105 110
Thr Ala Leu Ser Ala Gly Gly Leu Gly Pro Leu Pro Arg Val Thr Ser
115 120 125
Leu Asp Leu Ser Gly Asn Ser Leu Tyr Ser Gly Leu Leu Glu Arg Leu
130 135 140
Leu Gly Glu Ala Pro Ser Leu His Thr Leu Ser Leu Ala Glu Asn Ser

145 150 155 160
Leu Thr Arg Leu Thr Arg His Thr Phe Arg Asp Met Pro Ala Leu Glu
165 170 175
Gln Leu Asp Leu His Ser Asn Val Leu Met Asp Ile Glu Asp Gly Ala
180 185 190
Phe Glu Gly Leu Pro Arg Leu Thr His Leu Asn Leu Ser Arg Asn Ser
195 200 205
Leu Thr Cys Ile Ser Asp Phe Ser Leu Gln Gln Leu Arg Val Leu Asp
210 215 220
Leu Ser Cys Asn Ser Ile Glu Ala Phe Gln Thr Ala Ser Gln Pro Gln
225 230 235 240
Ala Glu Phe Gln Leu Thr Trp Leu Asp Leu Arg Glu Asn Lys Leu Leu
245 250 255
His Phe Pro Asp Leu Ala Ala Leu Pro Arg Leu Ile Tyr Leu Asn Leu
260 265 270
Ser Asn Asn Leu Ile Arg Leu Pro Thr Gly Pro Pro Gln Asp Ser Lys
275 280 285
Gly Ile His Ala Pro Ser Glu Gly Trp Ser Ala Leu Pro Leu Ser Ala
290 295 300
Pro Ser Gly Asn Ala Ser Gly Arg Pro Leu Ser Gln Leu Leu Asn Leu
305 310 315 320
Asp Leu Ser Tyr Asn Glu Ile Glu Leu Ile Pro Asp Ser Phe Leu Glu
325 330 335
His Leu Thr Ser Leu Cys Phe Leu Asn Leu Ser Arg Asn Cys Leu Arg
340 345 350
Thr Phe Glu Ala Arg Arg Leu Gly Ser Leu Pro Cys Leu Met Leu Leu
355 360 365
Asp Leu Ser His Asn Ala Leu Glu Thr Leu Glu Leu Gly Ala Arg Ala
370 375 380
Leu Gly Ser Leu Arg Thr Leu Leu Leu Gln Gly Asn Ala Leu Arg Asp
385 390 395 400
Leu Pro Pro Tyr Thr Phe Ala Asn Leu Ala Ser Leu Gln Arg Leu Asn
405 410 415
Leu Gln Gly Asn Arg Val Ser Pro Cys Gly Gly Pro Asp Glu Pro Gly
420 425 430
Pro Ser Gly Cys Val Ala Phe Ser Gly Ile Thr Ser Leu Arg Ser Leu
435 440 445
Ser Leu Val Asp Asn Glu Ile Glu Leu Leu Arg Ala Gly Ala Phe Leu
450 455 460
His Thr Pro Leu Thr Glu Leu Asp Leu Ser Ser Asn Pro Gly Leu Glu
465 470 475 480
Val Ala Thr Gly Ala Leu Gly Gly Leu Glu Ala Ser Leu Glu Val Leu
485 490 495
Ala Leu Gln Gly Asn Gly Leu Met Val Leu Gln Val Asp Leu Pro Cys
500 505 510
Phe Ile Cys Leu Lys Arg Leu Asn Leu Ala Glu Asn Arg Leu Ser His
515 520 525
Leu Pro Ala Trp Thr Gln Ala Val Ser Leu Glu Val Leu Asp Leu Arg
530 535 540
Asn Asn Ser Phe Ser Leu Leu Pro Gly Ser Ala Met Gly Gly Leu Glu
545 550 555 560
Thr Ser Leu Arg Arg Leu Tyr Leu Gln Gly Asn Pro Leu Ser Cys Cys
565 570 575
Gly Asn Gly Trp Leu Ala Ala Gln Leu His Gln Gly Arg Val Asp Val
580 585 590
Asp Ala Thr Gln Asp Leu Ile Cys Arg Phe Ser Ser Gln Glu Glu Val
595 600 605
Ser Leu Ser His Val Arg Pro Glu Asp Cys Glu Lys Gly Gly Leu Lys
610 615 620
Asn Ile Asn Leu Ile Ile Leu Thr Phe Ile Leu Val Ser Ala Ile
625 630 635 640
Leu Leu Thr Thr Leu Ala Ala Cys Cys Cys Val Arg Arg Gln Lys Phe
645 650 655
Asn Gln Gln Tyr Lys Ala
660

<210> 203
<211> 1036
<212> PRT

<213> Homo sapiens
<400> 203
Met Gln Pro Glu Glu Gly Thr Gly Trp Leu Leu Glu Leu Leu Ser Glu
1 5 10 15
Val Gln Leu Gln Gln Tyr Phe Leu Arg Leu Arg Asp Asp Leu Asn Val
20 25 30
Thr Arg Leu Ser His Phe Glu Tyr Val Lys Asn Glu Asp Leu Glu Lys
35 40 45
Ile Gly Met Gly Arg Pro Gly Gln Arg Arg Leu Trp Glu Ala Val Lys
50 55 60
Arg Arg Lys Ala Leu Cys Lys Arg Lys Ser Trp Met Ser Lys Val Phe
65 70 75 80
Ser Gly Lys Arg Leu Glu Ala Glu Phe Pro Pro His His Ser Gln Ser
85 90 95
Thr Phe Arg Lys Thr Ser Pro Ala Pro Gly Gly Pro Ala Gly Glu Gly
100 105 110
Pro Leu Gln Ser Leu Thr Cys Leu Ile Gly Glu Lys Asp Leu Arg Leu
115 120 125
Leu Glu Lys Leu Gly Asp Gly Ser Phe Gly Val Val Arg Arg Gly Glu
130 135 140
Trp Asp Ala Pro Ser Gly Lys Thr Val Ser Val Ala Val Lys Cys Leu
145 150 155 160
Lys Pro Asp Val Leu Ser Gln Pro Glu Ala Met Asp Asp Phe Ile Arg
165 170 175
Glu Val Asn Ala Met His Ser Leu Asp His Arg Asn Leu Ile Arg Leu
180 185 190
Tyr Gly Val Val Leu Thr Pro Pro Met Lys Met Val Thr Glu Leu Ala
195 200 205
Pro Leu Gly Ser Leu Leu Asp Arg Leu Arg Lys His Gln Gly His Phe
210 215 220
Leu Leu Gly Thr Leu Ser Arg Tyr Ala Val Gln Val Ala Glu Gly Met
225 230 235 240
Gly Tyr Leu Glu Ser Lys Arg Phe Ile His Arg Asp Leu Ala Ala Arg
245 250 255
Asn Leu Leu Leu Ala Thr Arg Asp Leu Val Lys Ile Gly Asp Phe Gly
260 265 270
Leu Met Arg Ala Leu Pro Gln Asn Asp Asp His Tyr Val Met Gln Glu
275 280 285
His Arg Lys Val Pro Phe Ala Trp Cys Ala Pro Glu Ser Leu Lys Thr
290 295 300
Arg Thr Phe Ser His Ala Ser Asp Thr Trp Met Phe Gly Val Thr Leu
305 310 315 320
Trp Glu Met Phe Thr Tyr Gly Gln Glu Pro Trp Ile Gly Leu Asn Gly
325 330 335
Ser Gln Ile Leu His Lys Ile Asp Lys Glu Gly Glu Arg Leu Pro Arg
340 345 350
Pro Glu Asp Cys Pro Gln Asp Ile Tyr Asn Val Met Val Gln Cys Trp
355 360 365
Ala His Lys Pro Glu Asp Arg Pro Thr Phe Val Ala Leu Arg Asp Phe
370 375 380
Leu Leu Glu Ala Gln Pro Thr Asp Met Arg Ala Leu Gln Asp Phe Glu
385 390 395 400
Glu Pro Asp Lys Leu His Ile Gln Met Asn Asp Val Ile Thr Val Ile
405 410 415
Glu Gly Arg Ala Glu Asn Tyr Trp Trp Arg Gly Gln Asn Thr Arg Thr
420 425 430
Leu Cys Val Gly Pro Phe Pro Arg Asn Val Val Thr Ser Val Ala Gly
435 440 445
Leu Ser Ala Gln Asp Ile Ser Gln Pro Leu Gln Asn Ser Phe Ile His
450 455 460
Thr Gly His Gly Asp Ser Asp Pro Arg His Cys Trp Gly Phe Pro Asp
465 470 475 480
Arg Ile Asp Glu Leu Tyr Leu Gly Asn Pro Met Asp Pro Pro Asp Leu
485 490 495
Leu Ser Val Glu Leu Ser Thr Ser Arg Pro Pro Gln His Leu Gly Gly
500 505 510
Val Lys Lys Pro Thr Tyr Asp Pro Val Ser Glu Asp Gln Asp Pro Leu
515 520 525
Ser Ser Asp Phe Lys Arg Leu Gly Leu Arg Lys Pro Gly Leu Pro Arg

530 535 540
Gly Leu Trp Leu Ala Lys Pro Ser Ala Arg Val Pro Gly Thr Lys Ala
545 550 555 560
Ser Arg Gly Ser Gly Ala Glu Val Thr Leu Ile Asp Phe Gly Glu Glu
565 570 575
Pro Val Val Pro Ala Leu Arg Pro Cys Pro Pro Ser Leu Ala Gln Leu
580 585 590
Ala Met Asp Ala Cys Ser Leu Leu Asp Glu Thr Pro Pro Gln Ser Pro
595 600 605
Thr Arg Ala Leu Pro Arg Pro Leu His Pro Thr Pro Val Val Asp Trp
610 615 620
Asp Ala Arg Pro Leu Pro Pro Pro Pro Ala Tyr Asp Asp Val Ala Gln
625 630 635 640
Asp Glu Asp Asp Phe Glu Ile Cys Ser Ile Asn Ser Thr Leu Val Gly
645 650 655
Ala Gly Val Pro Ala Gly Pro Ser Gln Gly Gln Thr Asn Tyr Ala Phe
660 665 670
Val Pro Glu Gln Ala Arg Pro Pro Pro Pro Leu Glu Asp Asn Leu Phe
675 680 685
Leu Pro Pro Gln Gly Gly Gly Lys Pro Pro Ser Ser Ala Gln Thr Ala
690 695 700
Glu Ile Phe Gln Ala Leu Gln Gln Glu Cys Met Arg Gln Leu Gln Ala
705 710 715 720
Pro Gly Ser Pro Ala Pro Ser Pro Ser Pro Gly Gly Asp Asp Lys Pro
725 730 735
Gln Val Pro Pro Arg Val Pro Ile Pro Pro Arg Pro Thr Arg Pro His
740 745 750
Val Gln Leu Ser Pro Ala Pro Pro Gly Glu Glu Glu Thr Ser Gln Trp
755 760 765
Pro Gly Pro Ala Ser Pro Pro Arg Val Pro Pro Arg Glu Pro Leu Ser
770 775 780
Pro Gln Gly Ser Arg Thr Pro Ser Pro Leu Val Pro Pro Gly Ser Ser
785 790 795 800
Pro Leu Pro Pro Arg Leu Ser Ser Ser Pro Gly Lys Thr Met Pro Thr
805 810 815
Thr Gln Ser Phe Ala Ser Asp Pro Lys Tyr Ala Thr Pro Gln Val Ile
820 825 830
Gln Ala Pro Gly Ala Gly Gly Pro Cys Ile Leu Pro Ile Val Arg Asp
835 840 845
Gly Lys Lys Val Ser Ser Thr His Tyr Tyr Leu Leu Pro Glu Arg Pro
850 855 860
Ser Tyr Leu Glu Arg Tyr Gln Arg Phe Leu Arg Glu Ala Gln Ser Pro
865 870 875 880
Glu Glu Pro Thr Pro Leu Pro Val Pro Leu Leu Leu Pro Pro Pro Ser
885 890 895
Thr Pro Ala Pro Ala Ala Pro Thr Ala Thr Val Arg Pro Met Pro Gln
900 905 910
Ala Ala Leu Asp Pro Lys Ala Asn Phe Ser Thr Asn Asn Ser Asn Pro
915 920 925
Gly Ala Arg Pro Pro Pro Pro Arg Ala Thr Ala Arg Leu Pro Gln Arg
930 935 940
Gly Cys Pro Gly Asp Gly Pro Glu Ala Gly Arg Pro Ala Asp Lys Ile
945 950 955 960
Gln Met Ala Met Val His Gly Val Thr Thr Glu Glu Cys Gln Ala Ala
965 970 975
Leu Gln Cys His Gly Trp Ser Val Gln Arg Ala Ala Gln Tyr Leu Lys
980 985 990
Val Glu Gln Leu Phe Gly Leu Gly Leu Arg Pro Arg Gly Glu Cys His
995 1000 1005
Lys Val Leu Glu Met Phe Asp Trp Asn Leu Glu Gln Ala Gly Cys
1010 1015 1020
His Leu Leu Gly Ser Trp Gly Pro Ala His His Lys Arg
1025 1030 1035

<210> 204
<211> 364
<212> PRT
<213> Homo sapiens
<400> 204

```
Met Ala Ala Ile Ser Thr Ser Ile Pro Val Ile Ser Gln Pro Gln Phe
1               5                   10                  15
Thr Ala Met Asn Glu Pro Gln Cys Phe Tyr Asn Glu Ser Ile Ala Phe
            20                  25                  30
Phe Tyr Asn Arg Ser Gly Lys His Leu Ala Thr Glu Trp Asn Thr Val
        35                  40                  45
Ser Lys Leu Val Met Gly Leu Gly Ile Thr Val Cys Ile Phe Ile Met
    50                  55                  60
Leu Ala Asn Leu Leu Val Met Val Ala Ile Tyr Val Asn Arg Arg Phe
65                  70                  75                  80
His Phe Pro Ile Tyr Tyr Leu Met Ala Asn Leu Ala Ala Ala Asp Phe
                85                  90                  95
Phe Ala Gly Leu Ala Tyr Phe Tyr Leu Met Phe Asn Thr Gly Pro Asn
            100                 105                 110
Thr Arg Arg Leu Thr Val Ser Thr Trp Leu Leu Arg Gln Gly Leu Ile
        115                 120                 125
Asp Thr Ser Leu Thr Ala Ser Val Ala Asn Leu Leu Ala Ile Ala Ile
    130                 135                 140
Glu Arg His Ile Thr Val Phe Arg Met Gln Leu His Thr Arg Met Ser
145                 150                 155                 160
Asn Arg Arg Val Val Val Val Ile Val Val Ile Trp Thr Met Ala Ile
                165                 170                 175
Val Met Gly Ala Ile Pro Ser Val Gly Trp Asn Cys Ile Cys Asp Ile
            180                 185                 190
Glu Asn Cys Ser Asn Met Ala Pro Leu Tyr Ser Asp Ser Tyr Leu Val
        195                 200                 205
Phe Trp Ala Ile Phe Asn Leu Val Thr Phe Val Val Met Val Val Leu
    210                 215                 220
Tyr Ala His Ile Phe Gly Tyr Val Arg Gln Arg Thr Met Arg Met Ser
225                 230                 235                 240
Arg His Ser Ser Gly Pro Arg Arg Asn Arg Asp Thr Met Met Ser Leu
                245                 250                 255
Leu Lys Thr Val Val Ile Val Leu Gly Ala Phe Ile Ile Cys Trp Thr
            260                 265                 270
Pro Gly Leu Val Leu Leu Leu Leu Asp Val Cys Cys Pro Gln Cys Asp
        275                 280                 285
Val Leu Ala Tyr Glu Lys Phe Phe Leu Leu Leu Ala Glu Phe Asn Ser
    290                 295                 300
Ala Met Asn Pro Ile Ile Tyr Ser Tyr Arg Asp Lys Glu Met Ser Ala
305                 310                 315                 320
Thr Phe Arg Gln Ile Leu Cys Cys Gln Arg Ser Glu Asn Pro Thr Gly
                325                 330                 335
Pro Thr Glu Gly Ser Asp Arg Ser Ala Ser Ser Leu Asn His Thr Ile
            340                 345                 350
Leu Ala Gly Val His Ser Asn Asp His Ser Val Val
        355                 360

<210>  205
<211>  164
<212>  PRT
<213>  Homo sapiens
<400>  205
Met Ala Ser Pro His Gln Glu Pro Lys Pro Gly Asp Leu Ile Glu Ile
1               5                   10                  15
Phe Arg Leu Gly Tyr Glu His Trp Ala Leu Tyr Ile Gly Asp Gly Tyr
            20                  25                  30
Val Ile His Leu Ala Pro Pro Ser Glu Tyr Pro Gly Ala Gly Ser Ser
        35                  40                  45
Ser Val Phe Ser Val Leu Ser Asn Ser Ala Glu Val Lys Arg Gly Arg
    50                  55                  60
Leu Glu Asp Val Val Gly Gly Cys Cys Tyr Arg Val Asn Asn Ser Leu
65                  70                  75                  80
Asp His Glu Tyr Gln Pro Arg Pro Val Glu Val Ile Ile Ser Ser Ala
                85                  90                  95
Lys Glu Met Val Gly Gln Lys Met Lys Tyr Ser Ile Val Ser Arg Asn
            100                 105                 110
Cys Glu His Phe Val Ala Gln Leu Arg Tyr Gly Lys Ser Arg Cys Lys
        115                 120                 125
Gln Val Glu Lys Ala Lys Val Glu Val Gly Val Ala Thr Ala Leu Gly
```

130 135 140
Ile Leu Val Val Ala Gly Cys Ser Phe Ala Ile Arg Arg Tyr Gln Lys
145 150 155 160
Lys Ala Thr Ala

<210> 206
<211> 323
<212> PRT
<213> Homo sapiens
<400> 206
Met Tyr His Asn Ser Ser Gln Lys Arg His Trp Thr Phe Ser Ser Glu
1 5 10 15
Glu Gln Leu Ala Arg Leu Arg Ala Asp Ala Asn Arg Lys Phe Arg Cys
20 25 30
Lys Ala Val Ala Asn Gly Lys Val Leu Pro Asn Asp Pro Val Phe Leu
35 40 45
Glu Pro His Glu Glu Met Thr Leu Cys Lys Tyr Tyr Glu Lys Arg Leu
50 55 60
Leu Glu Phe Cys Ser Val Phe Lys Pro Ala Met Pro Arg Ser Val Val
65 70 75 80
Gly Thr Ala Cys Met Tyr Phe Lys Arg Phe Tyr Leu Asn Asn Ser Val
85 90 95
Met Glu Tyr His Pro Arg Ile Ile Met Leu Thr Cys Ala Phe Leu Ala
100 105 110
Cys Lys Val Asp Glu Phe Asn Val Ser Ser Pro Gln Phe Val Gly Asn
115 120 125
Leu Arg Glu Ser Pro Leu Gly Gln Glu Lys Ala Leu Glu Gln Ile Leu
130 135 140
Glu Tyr Glu Leu Leu Leu Ile Gln Gln Leu Asn Phe His Leu Ile Val
145 150 155 160
His Asn Pro Tyr Arg Pro Phe Glu Gly Phe Leu Ile Asp Leu Lys Thr
165 170 175
Arg Tyr Pro Ile Leu Glu Asn Pro Glu Ile Leu Arg Lys Thr Ala Asp
180 185 190
Asp Phe Leu Asn Arg Ile Ala Leu Thr Asp Ala Tyr Leu Leu Tyr Thr
195 200 205
Pro Ser Gln Ile Ala Leu Thr Ala Ile Leu Ser Ser Ala Ser Arg Ala
210 215 220
Gly Ile Thr Met Glu Ser Tyr Leu Ser Glu Ser Leu Met Leu Lys Glu
225 230 235 240
Asn Arg Thr Cys Leu Ser Gln Leu Leu Asp Ile Met Lys Ser Met Arg
245 250 255
Asn Leu Val Lys Lys Tyr Glu Pro Pro Arg Ser Glu Glu Val Ala Val
260 265 270
Leu Lys Gln Lys Leu Glu Arg Cys His Ser Ala Glu Leu Ala Leu Asn
275 280 285
Val Ile Thr Lys Lys Arg Lys Gly Tyr Glu Asp Asp Asp Tyr Val Ser
290 295 300
Lys Lys Ser Lys His Glu Glu Glu Glu Trp Thr Asp Asp Asp Leu Val
305 310 315 320
Glu Ser Leu

<210> 207
<211> 710
<212> PRT
<213> Homo sapiens
<400> 207
Met Leu Ser Phe Gln Tyr Pro Asp Val Tyr Arg Asp Glu Thr Ala Val
1 5 10 15
Gln Asp Tyr His Gly His Lys Ile Cys Asp Pro Tyr Ala Trp Leu Glu
20 25 30
Asp Pro Asp Ser Glu Gln Thr Lys Ala Phe Val Glu Ala Gln Asn Lys
35 40 45
Ile Thr Val Pro Phe Leu Glu Gln Cys Pro Ile Arg Gly Leu Tyr Lys
50 55 60
Glu Arg Met Thr Glu Leu Tyr Asp Tyr Pro Lys Tyr Ser Cys His Phe
65 70 75 80

Lys Lys Gly Lys Arg Tyr Phe Tyr Phe Tyr Asn Thr Gly Leu Gln Asn
85 90 95
Gln Arg Val Leu Tyr Val Gln Asp Ser Leu Glu Gly Glu Ala Arg Val
100 105 110
Phe Leu Asp Pro Asn Ile Leu Ser Asp Asp Gly Thr Val Ala Leu Arg
115 120 125
Gly Tyr Ala Phe Ser Glu Asp Gly Glu Tyr Phe Ala Tyr Gly Leu Ser
130 135 140
Ala Ser Gly Ser Asp Trp Val Thr Ile Lys Phe Met Lys Val Asp Gly
145 150 155 160
Ala Lys Glu Leu Pro Asp Val Leu Glu Arg Val Lys Phe Ser Cys Met
165 170 175
Ala Trp Thr His Asp Gly Lys Gly Met Phe Tyr Asn Ser Tyr Pro Gln
180 185 190
Gln Asp Gly Lys Ser Asp Gly Thr Glu Thr Ser Thr Asn Leu His Gln
195 200 205
Lys Leu Tyr Tyr His Val Leu Gly Thr Asp Gln Ser Glu Asp Ile Leu
210 215 220
Cys Ala Glu Phe Pro Asp Glu Pro Lys Trp Met Gly Gly Ala Glu Leu
225 230 235 240
Ser Asp Asp Gly Arg Tyr Val Leu Leu Ser Ile Arg Glu Gly Cys Asp
245 250 255
Pro Val Asn Arg Leu Trp Tyr Cys Asp Leu Gln Gln Glu Ser Ser Gly
260 265 270
Ile Ala Gly Ile Leu Lys Trp Val Lys Leu Ile Asp Asn Phe Glu Gly
275 280 285
Glu Tyr Asp Tyr Val Thr Asn Glu Gly Ala Val Phe Thr Phe Lys Thr
290 295 300
Asn Arg Gln Ser Pro Asn Tyr Arg Val Ile Asn Ile Asp Phe Arg Asp
305 310 315 320
Pro Glu Glu Ser Lys Trp Lys Val Leu Val Pro Glu His Glu Lys Asp
325 330 335
Val Leu Glu Trp Ile Ala Cys Val Arg Ser Asn Phe Leu Val Leu Cys
340 345 350
Tyr Leu His Asp Val Lys Asn Ile Leu Gln Leu His Asp Leu Thr Thr
355 360 365
Gly Ala Leu Leu Lys Thr Phe Pro Leu Asp Val Gly Ser Ile Val Gly
370 375 380
Tyr Ser Gly Gln Lys Lys Asp Thr Glu Ile Phe Tyr Gln Phe Thr Ser
385 390 395 400
Phe Leu Ser Pro Gly Ile Ile Tyr His Cys Asp Leu Thr Lys Glu Glu
405 410 415
Leu Glu Pro Arg Val Phe Arg Glu Val Thr Val Lys Gly Ile Asp Ala
420 425 430
Ser Asp Tyr Gln Thr Val Gln Ile Phe Tyr Pro Ser Lys Asp Gly Thr
435 440 445
Lys Ile Pro Met Phe Ile Val His Lys Lys Ser Ile Lys Leu Asp Gly
450 455 460
Ser His Pro Ala Phe Leu Tyr Gly Tyr Gly Gly Phe Asn Ile Ser Ile
465 470 475 480
Thr Pro Asn Tyr Ser Val Ser Arg Leu Ile Phe Val Arg His Met Gly
485 490 495
Gly Ile Leu Ala Val Ala Asn Ile Arg Gly Gly Gly Glu Tyr Gly Glu
500 505 510
Thr Trp His Lys Gly Gly Ile Leu Ala Asn Lys Gln Asn Cys Phe Asp
515 520 525
Asp Phe Gln Cys Ala Ala Glu Tyr Leu Ile Lys Glu Gly Tyr Thr Ser
530 535 540
Pro Lys Arg Leu Thr Ile Asn Gly Gly Ser Asn Gly Gly Leu Leu Val
545 550 555 560
Ala Ala Cys Ala Asn Gln Arg Pro Asp Leu Phe Gly Cys Val Ile Ala
565 570 575
Gln Val Gly Val Met Asp Met Leu Lys Phe His Lys Tyr Thr Ile Gly
580 585 590
His Ala Trp Thr Thr Asp Tyr Gly Cys Ser Asp Ser Lys Gln His Phe
595 600 605
Glu Trp Leu Val Lys Tyr Ser Pro Leu His Asn Val Lys Leu Pro Glu
610 615 620
Ala Asp Asp Ile Gln Tyr Pro Ser Met Leu Leu Leu Thr Ala Asp His

625 630 635 640
Asp Asp Arg Val Val Pro Leu His Ser Leu Lys Phe Ile Ala Thr Leu
645 650 655
Gln Tyr Ile Val Gly Arg Ser Arg Lys Gln Ser Asn Pro Leu Leu Ile
660 665 670
His Val Asp Thr Lys Ala Gly His Gly Ala Gly Lys Pro Thr Ala Lys
675 680 685
Val Ile Glu Glu Val Ser Asp Met Phe Ala Phe Ile Ala Arg Cys Leu
690 695 700
Asn Val Asp Trp Ile Pro
705 710

<210> 208
<211> 1806
<212> PRT
<213> Homo sapiens
<400> 208
Met Glu Pro Trp Ser Ser Arg Trp Lys Thr Lys Arg Trp Leu Trp Asp
1 5 10 15
Phe Thr Val Thr Thr Leu Ala Leu Thr Phe Leu Phe Gln Ala Arg Glu
20 25 30
Val Arg Gly Ala Ala Pro Val Asp Val Leu Lys Ala Leu Asp Phe His
35 40 45
Asn Ser Pro Glu Gly Ile Ser Lys Thr Thr Gly Phe Cys Thr Asn Arg
50 55 60
Lys Asn Ser Lys Gly Ser Asp Thr Ala Tyr Arg Val Ser Lys Gln Ala
65 70 75 80
Gln Leu Ser Ala Pro Thr Lys Gln Leu Phe Pro Gly Gly Thr Phe Pro
85 90 95
Glu Asp Phe Ser Ile Leu Phe Thr Val Lys Pro Lys Lys Gly Ile Gln
100 105 110
Ser Phe Leu Leu Ser Ile Tyr Asn Glu His Gly Ile Gln Gln Ile Gly
115 120 125
Val Glu Val Gly Arg Ser Pro Val Phe Leu Phe Glu Asp His Thr Gly
130 135 140
Lys Pro Ala Pro Glu Asp Tyr Pro Leu Phe Arg Thr Val Asn Ile Ala
145 150 155 160
Asp Gly Lys Trp His Arg Val Ala Ile Ser Val Glu Lys Lys Thr Val
165 170 175
Thr Met Ile Val Asp Cys Lys Lys Lys Thr Thr Lys Pro Leu Asp Arg
180 185 190
Ser Glu Arg Ala Ile Val Asp Thr Asn Gly Ile Thr Val Phe Gly Thr
195 200 205
Arg Ile Leu Asp Glu Glu Val Phe Glu Gly Asp Ile Gln Gln Phe Leu
210 215 220
Ile Thr Gly Asp Pro Lys Ala Ala Tyr Asp Tyr Cys Glu His Tyr Ser
225 230 235 240
Pro Asp Cys Asp Ser Ser Ala Pro Lys Ala Ala Gln Ala Gln Glu Pro
245 250 255
Gln Ile Asp Glu Tyr Ala Pro Glu Asp Ile Ile Glu Tyr Asp Tyr Glu
260 265 270
Tyr Gly Glu Ala Glu Tyr Lys Glu Ala Glu Ser Val Thr Glu Gly Pro
275 280 285
Thr Val Thr Glu Glu Thr Ile Ala Gln Thr Glu Ala Asn Ile Val Asp
290 295 300
Asp Phe Gln Glu Tyr Asn Tyr Gly Thr Met Glu Ser Tyr Gln Thr Glu
305 310 315 320
Ala Pro Arg His Val Ser Gly Thr Asn Glu Pro Asn Pro Val Glu Glu
325 330 335
Ile Phe Thr Glu Glu Tyr Leu Thr Gly Glu Asp Tyr Asp Ser Gln Arg
340 345 350
Lys Asn Ser Glu Asp Thr Leu Tyr Glu Asn Lys Glu Ile Asp Gly Arg
355 360 365
Asp Ser Asp Leu Leu Val Asp Gly Asp Leu Gly Glu Tyr Asp Phe Tyr
370 375 380
Glu Tyr Lys Glu Tyr Glu Asp Lys Pro Thr Ser Pro Pro Asn Glu Glu
385 390 395 400
Phe Gly Pro Gly Val Pro Ala Glu Thr Asp Ile Thr Glu Thr Ser Ile
405 410 415

```
Asn Gly His Gly Ala Tyr Gly Glu Lys Gly Gln Lys Gly Glu Pro Ala
            420                 425                 430
Val Val Glu Pro Gly Met Leu Val Glu Gly Pro Pro Gly Pro Ala Gly
        435                 440                 445
Pro Ala Gly Ile Met Gly Pro Pro Gly Leu Gln Gly Pro Thr Gly Pro
    450                 455                 460
Pro Gly Asp Pro Gly Asp Arg Gly Pro Pro Gly Arg Pro Gly Leu Pro
465                 470                 475                 480
Gly Ala Asp Gly Leu Pro Gly Pro Pro Gly Thr Met Leu Met Leu Pro
                485                 490                 495
Phe Arg Tyr Gly Gly Asp Gly Ser Lys Gly Pro Thr Ile Ser Ala Gln
            500                 505                 510
Glu Ala Gln Ala Gln Ala Ile Leu Gln Gln Ala Arg Ile Ala Leu Arg
        515                 520                 525
Gly Pro Pro Gly Pro Met Gly Leu Thr Gly Arg Pro Gly Pro Val Gly
    530                 535                 540
Gly Pro Gly Ser Ser Gly Ala Lys Gly Glu Ser Gly Asp Pro Gly Pro
545                 550                 555                 560
Gln Gly Pro Arg Gly Val Gln Gly Pro Pro Gly Pro Thr Gly Lys Pro
                565                 570                 575
Gly Lys Arg Gly Arg Pro Gly Ala Asp Gly Gly Arg Gly Met Pro Gly
            580                 585                 590
Glu Pro Gly Ala Lys Gly Asp Arg Gly Phe Asp Gly Leu Pro Gly Leu
        595                 600                 605
Pro Gly Asp Lys Gly His Arg Gly Glu Arg Gly Pro Gln Gly Pro Pro
    610                 615                 620
Gly Pro Pro Gly Asp Asp Gly Met Arg Gly Glu Asp Gly Glu Ile Gly
625                 630                 635                 640
Pro Arg Gly Leu Pro Gly Glu Ala Gly Pro Arg Gly Leu Leu Gly Pro
                645                 650                 655
Arg Gly Thr Pro Gly Ala Pro Gly Gln Pro Gly Met Ala Gly Val Asp
            660                 665                 670
Gly Pro Pro Gly Pro Lys Gly Asn Met Gly Pro Gln Gly Glu Pro Gly
        675                 680                 685
Pro Pro Gly Gln Gln Gly Asn Pro Gly Pro Gln Gly Leu Pro Gly Pro
    690                 695                 700
Gln Gly Pro Ile Gly Pro Pro Gly Glu Lys Gly Pro Gln Gly Lys Pro
705                 710                 715                 720
Gly Leu Ala Gly Leu Pro Gly Ala Asp Gly Pro Pro Gly His Pro Gly
                725                 730                 735
Lys Glu Gly Gln Ser Gly Glu Lys Gly Ala Leu Gly Pro Pro Gly Pro
            740                 745                 750
Gln Gly Pro Ile Gly Tyr Pro Gly Pro Arg Gly Val Lys Gly Ala Asp
        755                 760                 765
Gly Val Arg Gly Leu Lys Gly Ser Lys Gly Glu Lys Gly Glu Asp Gly
    770                 775                 780
Phe Pro Gly Phe Lys Gly Asp Met Gly Leu Lys Gly Asp Arg Gly Glu
785                 790                 795                 800
Val Gly Gln Ile Gly Pro Arg Gly Glu Asp Gly Pro Glu Gly Pro Lys
                805                 810                 815
Gly Arg Ala Gly Pro Thr Gly Asp Pro Gly Pro Ser Gly Gln Ala Gly
            820                 825                 830
Glu Lys Gly Lys Leu Gly Val Pro Gly Leu Pro Gly Tyr Pro Gly Arg
        835                 840                 845
Gln Gly Pro Lys Gly Ser Thr Gly Phe Pro Gly Phe Pro Gly Ala Asn
    850                 855                 860
Gly Glu Lys Gly Ala Arg Gly Val Ala Gly Lys Pro Gly Pro Arg Gly
865                 870                 875                 880
Gln Arg Gly Pro Thr Gly Pro Arg Gly Ser Arg Gly Ala Arg Gly Pro
                885                 890                 895
Thr Gly Lys Pro Gly Pro Lys Gly Thr Ser Gly Gly Asp Gly Pro Pro
            900                 905                 910
Gly Pro Pro Gly Glu Arg Gly Pro Gln Gly Pro Gln Gly Pro Val Gly
        915                 920                 925
Phe Pro Gly Pro Lys Gly Pro Pro Gly Pro Pro Gly Arg Met Gly Cys
    930                 935                 940
Pro Gly His Pro Gly Gln Arg Gly Glu Thr Gly Phe Gln Gly Lys Thr
945                 950                 955                 960
Gly Pro Pro Gly Pro Gly Gly Val Val Gly Pro Gln Gly Pro Thr Gly
```

965 970 975
Glu Thr Gly Pro Ile Gly Glu Arg Gly His Pro Gly Pro Pro Gly Pro
980 985 990
Pro Gly Glu Gln Gly Leu Pro Gly Ala Ala Gly Lys Glu Gly Ala Lys
995 1000 1005
Gly Asp Pro Gly Pro Gln Gly Ile Ser Gly Lys Asp Gly Pro Ala
1010 1015 1020
Gly Leu Arg Gly Phe Pro Gly Glu Arg Gly Leu Pro Gly Ala Gln
1025 1030 1035
Gly Ala Pro Gly Leu Lys Gly Gly Glu Gly Pro Gln Gly Pro Pro
1040 1045 1050
Gly Pro Val Gly Ser Pro Gly Glu Arg Gly Ser Ala Gly Thr Ala
1055 1060 1065
Gly Pro Ile Gly Leu Pro Gly Arg Pro Gly Pro Gln Gly Pro Pro
1070 1075 1080
Gly Pro Ala Gly Glu Lys Gly Ala Pro Gly Glu Lys Gly Pro Gln
1085 1090 1095
Gly Pro Ala Gly Arg Asp Gly Val Gln Gly Pro Val Gly Leu Pro
1100 1105 1110
Gly Pro Ala Gly Pro Ala Gly Ser Pro Gly Glu Asp Gly Asp Lys
1115 1120 1125
Gly Glu Ile Gly Glu Pro Gly Gln Lys Gly Ser Lys Gly Asp Lys
1130 1135 1140
Gly Glu Asn Gly Pro Pro Gly Pro Pro Gly Leu Gln Gly Pro Val
1145 1150 1155
Gly Ala Pro Gly Ile Ala Gly Gly Asp Gly Glu Pro Gly Pro Arg
1160 1165 1170
Gly Gln Gln Gly Met Phe Gly Gln Lys Gly Asp Glu Gly Ala Arg
1175 1180 1185
Gly Phe Pro Gly Pro Pro Gly Pro Ile Gly Leu Gln Gly Leu Pro
1190 1195 1200
Gly Pro Pro Gly Glu Lys Gly Glu Asn Gly Asp Val Gly Pro Trp
1205 1210 1215
Gly Pro Pro Gly Pro Pro Gly Pro Arg Gly Pro Gln Gly Pro Asn
1220 1225 1230
Gly Ala Asp Gly Pro Gln Gly Pro Pro Gly Ser Val Gly Ser Val
1235 1240 1245
Gly Gly Val Gly Glu Lys Gly Glu Pro Gly Glu Ala Gly Asn Pro
1250 1255 1260
Gly Pro Pro Gly Glu Ala Gly Val Gly Gly Pro Lys Gly Glu Arg
1265 1270 1275
Gly Glu Lys Gly Glu Ala Gly Pro Pro Gly Ala Ala Gly Pro Pro
1280 1285 1290
Gly Ala Lys Gly Pro Pro Gly Asp Asp Gly Pro Lys Gly Asn Pro
1295 1300 1305
Gly Pro Val Gly Phe Pro Gly Asp Pro Gly Pro Pro Gly Glu Leu
1310 1315 1320
Gly Pro Ala Gly Gln Asp Gly Val Gly Gly Asp Lys Gly Glu Asp
1325 1330 1335
Gly Asp Pro Gly Gln Pro Gly Pro Pro Gly Pro Ser Gly Glu Ala
1340 1345 1350
Gly Pro Pro Gly Pro Pro Gly Lys Arg Gly Pro Pro Gly Ala Ala
1355 1360 1365
Gly Ala Glu Gly Arg Gln Gly Glu Lys Gly Ala Lys Gly Glu Ala
1370 1375 1380
Gly Ala Glu Gly Pro Pro Gly Lys Thr Gly Pro Val Gly Pro Gln
1385 1390 1395
Gly Pro Ala Gly Lys Pro Gly Pro Glu Gly Leu Arg Gly Ile Pro
1400 1405 1410
Gly Pro Val Gly Glu Gln Gly Leu Pro Gly Ala Ala Gly Gln Asp
1415 1420 1425
Gly Pro Pro Gly Pro Met Gly Pro Pro Gly Leu Pro Gly Leu Lys
1430 1435 1440
Gly Asp Pro Gly Ser Lys Gly Glu Lys Gly His Pro Gly Leu Ile
1445 1450 1455
Gly Leu Ile Gly Pro Pro Gly Glu Gln Gly Glu Lys Gly Asp Arg
1460 1465 1470
Gly Leu Pro Gly Thr Gln Gly Ser Pro Gly Ala Lys Gly Asp Gly
1475 1480 1485

Gly Ile Pro Gly Pro Ala Gly Pro Leu Gly Pro Pro Gly Pro Pro
1490 1495 1500
Gly Leu Pro Gly Pro Gln Gly Pro Lys Gly Asn Lys Gly Ser Thr
1505 1510 1515
Gly Pro Ala Gly Gln Lys Gly Asp Ser Gly Leu Pro Gly Pro Pro
1520 1525 1530
Gly Pro Pro Gly Pro Pro Gly Glu Val Ile Gln Pro Leu Pro Ile
1535 1540 1545
Leu Ser Ser Lys Lys Thr Arg Arg His Thr Glu Gly Met Gln Ala
1550 1555 1560
Asp Ala Asp Asp Asn Ile Leu Asp Tyr Ser Asp Gly Met Glu Glu
1565 1570 1575
Ile Phe Gly Ser Leu Asn Ser Leu Lys Gln Asp Ile Glu His Met
1580 1585 1590
Lys Phe Pro Met Gly Thr Gln Thr Asn Pro Ala Arg Thr Cys Lys
1595 1600 1605
Asp Leu Gln Leu Ser His Pro Asp Phe Pro Asp Gly Glu Tyr Trp
1610 1615 1620
Ile Asp Pro Asn Gln Gly Cys Ser Gly Asp Ser Phe Lys Val Tyr
1625 1630 1635
Cys Asn Phe Thr Ser Gly Gly Glu Thr Cys Ile Tyr Pro Asp Lys
1640 1645 1650
Lys Ser Glu Gly Val Arg Ile Ser Ser Trp Pro Lys Glu Lys Pro
1655 1660 1665
Gly Ser Trp Phe Ser Glu Phe Lys Arg Gly Lys Leu Leu Ser Tyr
1670 1675 1680
Leu Asp Val Glu Gly Asn Ser Ile Asn Met Val Gln Met Thr Phe
1685 1690 1695
Leu Lys Leu Leu Thr Ala Ser Ala Arg Gln Asn Phe Thr Tyr His
1700 1705 1710
Cys His Gln Ser Ala Ala Trp Tyr Asp Val Ser Ser Gly Ser Tyr
1715 1720 1725
Asp Lys Ala Leu Arg Phe Leu Gly Ser Asn Asp Glu Glu Met Ser
1730 1735 1740
Tyr Asp Asn Asn Pro Phe Ile Lys Thr Leu Tyr Asp Gly Cys Thr
1745 1750 1755
Ser Arg Lys Gly Tyr Glu Lys Thr Val Ile Glu Ile Asn Thr Pro
1760 1765 1770
Lys Ile Asp Gln Val Pro Ile Val Asp Val Met Ile Asn Asp Phe
1775 1780 1785
Gly Asp Gln Asn Gln Lys Phe Gly Phe Glu Val Gly Pro Val Cys
1790 1795 1800
Phe Leu Gly
1805

<210> 209
<211> 669
<212> PRT
<213> Homo sapiens
<400> 209
Met Thr Ala Ala Ala Gly Ser Ala Gly Arg Ala Ala Val Pro Leu Leu
1 5 10 15
Leu Cys Ala Leu Leu Ala Pro Gly Gly Ala Tyr Val Leu Asp Asp Ser
20 25 30
Asp Gly Leu Gly Arg Glu Phe Asp Gly Ile Gly Ala Val Ser Gly Gly
35 40 45
Gly Ala Thr Ser Arg Leu Leu Val Asn Tyr Pro Glu Pro Tyr Arg Ser
50 55 60
Gln Ile Leu Asp Tyr Leu Phe Lys Pro Asn Phe Gly Ala Ser Leu His
65 70 75 80
Ile Leu Lys Val Glu Ile Gly Gly Asp Gly Gln Thr Thr Asp Gly Thr
85 90 95
Glu Pro Ser His Met His Tyr Ala Leu Asp Glu Asn Tyr Phe Arg Gly
100 105 110
Tyr Glu Trp Trp Leu Met Lys Glu Ala Lys Lys Arg Asn Pro Asn Ile
115 120 125
Thr Leu Ile Gly Leu Pro Trp Ser Phe Pro Gly Trp Leu Gly Lys Gly
130 135 140
Phe Asp Trp Pro Tyr Val Asn Leu Gln Leu Thr Ala Tyr Tyr Val Val

```
145                 150                 155                 160
Thr Trp Ile Val Gly Ala Lys Arg Tyr His Asp Leu Asp Ile Asp Tyr
                165                 170                 175
Ile Gly Ile Trp Asn Glu Arg Ser Tyr Asn Ala Asn Tyr Ile Lys Ile
            180                 185                 190
Leu Arg Lys Met Leu Asn Tyr Gln Gly Leu Gln Arg Val Lys Ile Ile
        195                 200                 205
Ala Ser Asp Asn Leu Trp Glu Ser Ile Ser Ala Ser Met Leu Leu Asp
    210                 215                 220
Ala Glu Leu Phe Lys Val Val Asp Val Ile Gly Ala His Tyr Pro Gly
225                 230                 235                 240
Thr His Ser Ala Lys Asp Ala Lys Leu Thr Gly Lys Lys Leu Trp Ser
                245                 250                 255
Ser Glu Asp Phe Ser Thr Leu Asn Ser Asp Met Gly Ala Gly Cys Trp
            260                 265                 270
Gly Arg Ile Leu Asn Gln Asn Tyr Ile Asn Gly Tyr Met Thr Ser Thr
        275                 280                 285
Ile Ala Trp Asn Leu Val Ala Ser Tyr Tyr Glu Gln Leu Pro Tyr Gly
    290                 295                 300
Arg Cys Gly Leu Met Thr Ala Gln Glu Pro Trp Ser Gly His Tyr Val
305                 310                 315                 320
Val Glu Ser Pro Val Trp Val Ser Ala His Thr Thr Gln Phe Thr Gln
                325                 330                 335
Pro Gly Trp Tyr Tyr Leu Lys Thr Val Gly His Leu Glu Lys Gly Gly
            340                 345                 350
Ser Tyr Val Ala Leu Thr Asp Gly Leu Gly Asn Leu Thr Ile Ile Ile
        355                 360                 365
Glu Thr Met Ser His Lys His Ser Lys Cys Ile Arg Pro Phe Leu Pro
    370                 375                 380
Tyr Phe Asn Val Ser Gln Gln Phe Ala Thr Phe Val Leu Lys Gly Ser
385                 390                 395                 400
Phe Ser Glu Ile Pro Glu Leu Gln Val Trp Tyr Thr Lys Leu Gly Lys
                405                 410                 415
Thr Ser Glu Arg Phe Leu Phe Lys Gln Leu Asp Ser Leu Trp Leu Leu
            420                 425                 430
Asp Ser Asp Gly Ser Phe Thr Leu Ser Leu His Glu Asp Glu Leu Phe
        435                 440                 445
Thr Leu Thr Thr Leu Thr Thr Gly Arg Lys Gly Ser Tyr Pro Leu Pro
    450                 455                 460
Pro Lys Ser Gln Pro Phe Pro Ser Thr Tyr Lys Asp Asp Phe Asn Val
465                 470                 475                 480
Asp Tyr Pro Phe Phe Ser Glu Ala Pro Asn Phe Ala Asp Gln Thr Gly
                485                 490                 495
Val Phe Glu Tyr Phe Thr Asn Ile Glu Asp Pro Gly Glu His His Phe
            500                 505                 510
Thr Leu Arg Gln Val Leu Asn Gln Arg Pro Ile Thr Trp Ala Ala Asp
        515                 520                 525
Ala Ser Asn Thr Ile Ser Ile Ile Gly Asp Tyr Asn Trp Thr Asn Leu
    530                 535                 540
Thr Ile Lys Cys Asp Val Tyr Ile Glu Thr Pro Asp Thr Gly Gly Val
545                 550                 555                 560
Phe Ile Ala Gly Arg Val Asn Lys Gly Gly Ile Leu Ile Arg Ser Ala
                565                 570                 575
Arg Gly Ile Phe Phe Trp Ile Phe Ala Asn Gly Ser Tyr Arg Val Thr
            580                 585                 590
Gly Asp Leu Ala Gly Trp Ile Ile Tyr Ala Leu Gly Arg Val Glu Val
        595                 600                 605
Thr Ala Lys Lys Trp Tyr Thr Leu Thr Leu Thr Ile Lys Gly His Phe
    610                 615                 620
Ala Ser Gly Met Leu Asn Asp Lys Ser Leu Trp Thr Asp Ile Pro Val
625                 630                 635                 640
Asn Phe Pro Lys Asn Gly Trp Ala Ala Ile Gly Thr His Ser Phe Glu
                645                 650                 655
Phe Ala Gln Phe Asp Asn Phe Leu Val Glu Ala Thr Arg
            660                 665
```

<210> 210
<211> 508
<212> PRT

<213> Homo sapiens
<400> 210
Met Gln Arg Leu Leu Thr Pro Val Lys Arg Ile Leu Gln Leu Thr Arg
1 5 10 15
Ala Val Gln Glu Thr Ser Leu Thr Pro Ala Arg Leu Leu Pro Val Ala
20 25 30
His Gln Arg Phe Ser Thr Ala Ser Ala Val Pro Leu Ala Lys Thr Asp
35 40 45
Thr Trp Pro Lys Asp Val Gly Ile Leu Ala Leu Glu Val Tyr Phe Pro
50 55 60
Ala Gln Tyr Val Asp Gln Thr Asp Leu Glu Lys Tyr Asn Asn Val Glu
65 70 75 80
Ala Gly Lys Tyr Thr Val Gly Leu Gly Gln Thr Arg Met Gly Phe Cys
85 90 95
Ser Val Gln Glu Asp Ile Asn Ser Leu Cys Leu Thr Val Val Gln Arg
100 105 110
Leu Met Glu Arg Ile Gln Leu Pro Trp Asp Ser Val Gly Arg Leu Glu
115 120 125
Val Gly Thr Glu Thr Ile Ile Asp Lys Ser Lys Ala Val Lys Thr Val
130 135 140
Leu Met Glu Leu Phe Gln Asp Ser Gly Asn Thr Asp Ile Glu Gly Ile
145 150 155 160
Asp Thr Thr Asn Ala Cys Tyr Gly Gly Thr Ala Ser Leu Phe Asn Ala
165 170 175
Ala Asn Trp Met Glu Ser Ser Ser Trp Asp Gly Arg Tyr Ala Met Val
180 185 190
Val Cys Gly Asp Ile Ala Val Tyr Pro Ser Gly Asn Ala Arg Pro Thr
195 200 205
Gly Gly Ala Gly Ala Val Ala Met Leu Ile Gly Pro Lys Ala Pro Leu
210 215 220
Ala Leu Glu Arg Gly Leu Arg Gly Thr His Met Glu Asn Val Tyr Asp
225 230 235 240
Phe Tyr Lys Pro Asn Leu Ala Ser Glu Tyr Pro Ile Val Asp Gly Lys
245 250 255
Leu Ser Ile Gln Cys Tyr Leu Arg Ala Leu Asp Arg Cys Tyr Thr Ser
260 265 270
Tyr Arg Lys Lys Ile Gln Asn Gln Trp Lys Gln Ala Gly Ser Asp Arg
275 280 285
Pro Phe Thr Leu Asp Asp Leu Gln Tyr Met Ile Phe His Thr Pro Phe
290 295 300
Cys Lys Met Val Gln Lys Ser Leu Ala Arg Leu Met Phe Asn Asp Phe
305 310 315 320
Leu Ser Ala Ser Ser Asp Thr Gln Thr Ser Leu Tyr Lys Gly Leu Glu
325 330 335
Ala Phe Gly Gly Leu Lys Leu Glu Asp Thr Tyr Thr Asn Lys Asp Leu
340 345 350
Asp Lys Ala Leu Leu Lys Ala Ser Gln Asp Met Phe Asp Lys Lys Thr
355 360 365
Lys Ala Ser Leu Tyr Leu Ser Thr His Asn Gly Asn Met Tyr Thr Ser
370 375 380
Ser Leu Tyr Gly Cys Leu Ala Ser Leu Leu Ser His His Ser Ala Gln
385 390 395 400
Glu Leu Ala Gly Ser Arg Ile Gly Ala Phe Ser Tyr Gly Ser Gly Leu
405 410 415
Ala Ala Ser Phe Phe Ser Phe Arg Val Ser Gln Asp Ala Ala Pro Gly
420 425 430
Ser Pro Leu Asp Lys Leu Val Ser Ser Thr Ser Asp Leu Pro Lys Arg
435 440 445
Leu Ala Ser Arg Lys Cys Val Ser Pro Glu Glu Phe Thr Glu Ile Met
450 455 460
Asn Gln Arg Glu Gln Phe Tyr His Lys Val Asn Phe Ser Pro Pro Gly
465 470 475 480
Asp Thr Asn Ser Leu Phe Pro Gly Thr Trp Tyr Leu Glu Arg Val Asp
485 490 495
Glu Gln His Arg Arg Lys Tyr Ala Arg Arg Pro Val
500 505

<210> 211
<211> 548

<212> PRT
<213> Homo sapiens
<400> 211

```
Met Asn Ile Glu Val Val Glu Val Leu Thr Leu Asn Gln Glu Val Ala
1               5                   10                  15
Gly Pro Arg Asn Ala Gln Ile Gln Ala Leu Tyr Ala Glu Asp Gly Ser
            20                  25                  30
Leu Ser Ala Asp Ala Pro Ser Glu Gln Val Gln Gln Gln Gly Lys His
        35                  40                  45
Pro Gly Asp Pro Glu Ala Ala Arg Gln Arg Phe Arg Gln Phe Arg Tyr
    50                  55                  60
Lys Asp Met Thr Gly Pro Arg Glu Ala Leu Asp Gln Leu Arg Glu Leu
65                  70                  75                  80
Cys His Gln Trp Leu Gln Pro Lys Ala Arg Ser Lys Glu Gln Ile Leu
                85                  90                  95
Glu Leu Leu Val Leu Glu Gln Phe Leu Gly Ala Leu Pro Val Lys Leu
            100                 105                 110
Arg Thr Trp Val Glu Ser Gln His Pro Glu Asn Cys Gln Glu Val Val
        115                 120                 125
Ala Leu Val Glu Gly Val Thr Trp Met Ser Glu Glu Glu Val Leu Pro
    130                 135                 140
Ala Gly Gln Pro Ala Glu Gly Thr Thr Cys Cys Leu Glu Val Thr Ala
145                 150                 155                 160
Gln Gln Glu Glu Lys Gln Glu Asp Ala Ala Ile Cys Pro Val Thr Val
                165                 170                 175
Leu Pro Glu Glu Pro Val Thr Phe Gln Asp Val Ala Val Asp Phe Ser
            180                 185                 190
Arg Glu Glu Trp Gly Leu Leu Gly Pro Thr Gln Arg Thr Glu Tyr Arg
        195                 200                 205
Asp Val Met Leu Glu Thr Phe Gly His Leu Val Ser Val Gly Trp Glu
    210                 215                 220
Thr Thr Leu Glu Asn Lys Glu Leu Ala Pro Asn Ser Asp Ile Pro Glu
225                 230                 235                 240
Glu Glu Pro Ala Pro Ser Leu Lys Val Gln Glu Ser Ser Arg Asp Cys
                245                 250                 255
Ala Leu Ser Ser Thr Leu Glu Asp Thr Leu Gln Gly Gly Val Gln Glu
            260                 265                 270
Val Gln Asp Thr Val Leu Lys Gln Met Glu Ser Ala Gln Glu Lys Asp
        275                 280                 285
Leu Pro Gln Lys Lys His Phe Asp Asn Arg Glu Ser Gln Ala Asn Ser
    290                 295                 300
Gly Ala Leu Asp Thr Asn Gln Val Ser Leu Gln Lys Ile Asp Asn Pro
305                 310                 315                 320
Glu Ser Gln Ala Asn Ser Gly Ala Leu Asp Thr Asn Gln Val Leu Leu
                325                 330                 335
His Lys Ile Pro Pro Arg Lys Arg Leu Arg Lys Arg Asp Ser Gln Val
            340                 345                 350
Lys Ser Met Lys His Asn Ser Arg Val Lys Ile His Gln Lys Ser Cys
        355                 360                 365
Glu Arg Gln Lys Ala Lys Glu Gly Asn Gly Cys Arg Lys Thr Phe Ser
    370                 375                 380
Arg Ser Thr Lys Gln Ile Thr Phe Ile Arg Ile His Lys Gly Ser Gln
385                 390                 395                 400
Val Cys Arg Cys Ser Glu Cys Gly Lys Ile Phe Arg Asn Pro Arg Tyr
                405                 410                 415
Phe Ser Val His Lys Lys Ile His Thr Gly Glu Arg Pro Tyr Val Cys
            420                 425                 430
Gln Asp Cys Gly Lys Gly Phe Val Gln Ser Ser Ser Leu Thr Gln His
        435                 440                 445
Gln Arg Val His Ser Gly Glu Arg Pro Phe Glu Cys Gln Glu Cys Gly
    450                 455                 460
Arg Thr Phe Asn Asp Arg Ser Ala Ile Ser Gln His Leu Arg Thr His
465                 470                 475                 480
Thr Gly Ala Lys Pro Tyr Lys Cys Gln Asp Cys Gly Lys Ala Phe Arg
                485                 490                 495
Gln Ser Ser His Leu Ile Arg His Gln Arg Thr His Thr Gly Glu Arg
            500                 505                 510
Pro Tyr Ala Cys Asn Lys Cys Gly Lys Ala Phe Thr Gln Ser Ser His
        515                 520                 525
```

Leu Ile Gly His Gln Arg Thr His Asn Arg Thr Lys Arg Lys Lys Lys
530 535 540
Gln Pro Thr Ser
545

<210> 212
<211> 84
<212> PRT
<213> Homo sapiens
<400> 212
Met Ala Thr Met Glu Asn Lys Val Ile Cys Ala Leu Val Leu Val Ser
1 5 10 15
Met Leu Ala Leu Gly Thr Leu Ala Glu Ala Gln Thr Glu Thr Cys Thr
20 25 30
Val Ala Pro Arg Glu Arg Gln Asn Cys Gly Phe Pro Gly Val Thr Pro
35 40 45
Ser Gln Cys Ala Asn Lys Gly Cys Cys Phe Asp Asp Thr Val Arg Gly
50 55 60
Val Pro Trp Cys Phe Tyr Pro Asn Thr Ile Asp Val Pro Pro Glu Glu
65 70 75 80
Glu Cys Glu Phe

<210> 213
<211> 339
<212> PRT
<213> Homo sapiens
<400> 213
Met Ala Met Gln Met Gln Leu Glu Ala Asn Ala Asp Thr Ser Val Glu
1 5 10 15
Glu Glu Ser Phe Gly Pro Gln Pro Ile Ser Arg Leu Glu Gln Cys Gly
20 25 30
Ile Asn Ala Asn Asp Val Lys Lys Leu Glu Glu Ala Gly Phe His Thr
35 40 45
Val Glu Ala Val Ala Tyr Ala Pro Lys Lys Glu Leu Ile Asn Ile Lys
50 55 60
Gly Ile Ser Glu Ala Lys Ala Asp Lys Ile Leu Ala Glu Ala Ala Lys
65 70 75 80
Leu Val Pro Met Gly Phe Thr Thr Ala Thr Glu Phe His Gln Arg Arg
85 90 95
Ser Glu Ile Ile Gln Ile Thr Thr Gly Ser Lys Glu Leu Asp Lys Leu
100 105 110
Leu Gln Gly Gly Ile Glu Thr Gly Ser Ile Thr Glu Met Phe Gly Glu
115 120 125
Phe Arg Thr Gly Lys Thr Gln Ile Cys His Thr Leu Ala Val Thr Cys
130 135 140
Gln Leu Pro Ile Asp Arg Gly Gly Gly Glu Gly Lys Ala Met Tyr Ile
145 150 155 160
Asp Thr Glu Gly Thr Phe Arg Pro Glu Arg Leu Leu Ala Val Ala Glu
165 170 175
Arg Tyr Gly Leu Ser Gly Ser Asp Val Leu Asp Asn Val Ala Tyr Ala
180 185 190
Arg Ala Phe Asn Thr Asp His Gln Thr Gln Leu Leu Tyr Gln Ala Ser
195 200 205
Ala Met Met Val Glu Ser Arg Tyr Ala Leu Leu Ile Val Asp Ser Ala
210 215 220
Thr Ala Leu Tyr Arg Thr Asp Tyr Ser Gly Arg Gly Glu Leu Ser Ala
225 230 235 240
Arg Gln Met His Leu Ala Arg Phe Leu Arg Met Leu Leu Arg Leu Ala
245 250 255
Asp Glu Phe Gly Val Ala Val Val Ile Thr Asn Gln Val Val Ala Gln
260 265 270
Val Asp Gly Ala Ala Met Phe Ala Ala Asp Pro Lys Lys Pro Ile Gly
275 280 285
Gly Asn Ile Ile Ala His Ala Ser Thr Thr Arg Leu Tyr Leu Arg Lys
290 295 300
Gly Arg Gly Glu Thr Arg Ile Cys Lys Ile Tyr Asp Ser Pro Cys Leu
305 310 315 320
Pro Glu Ala Glu Ala Met Phe Ala Ile Asn Ala Asp Gly Val Gly Asp

325 330 335
Ala Lys Asp

<210> 214
<211> 561
<212> PRT
<213> Homo sapiens
<400> 214
Met Cys Gly Ile Trp Ala Leu Phe Gly Ser Asp Asp Cys Leu Ser Val
1 5 10 15
Gln Cys Leu Ser Ala Met Lys Ile Ala His Arg Gly Pro Asp Ala Phe
20 25 30
Arg Phe Glu Asn Val Asn Gly Tyr Thr Asn Cys Cys Phe Gly Phe His
35 40 45
Arg Leu Ala Val Val Asp Pro Leu Phe Gly Met Gln Pro Ile Arg Val
50 55 60
Lys Lys Tyr Pro Tyr Leu Trp Leu Cys Tyr Asn Gly Glu Ile Tyr Asn
65 70 75 80
His Lys Lys Met Gln Gln His Phe Glu Phe Glu Tyr Gln Thr Lys Val
85 90 95
Asp Gly Glu Ile Ile Leu His Leu Tyr Asp Lys Gly Gly Ile Glu Gln
100 105 110
Thr Ile Cys Met Leu Asp Gly Val Phe Ala Phe Val Leu Leu Asp Thr
115 120 125
Ala Asn Lys Lys Val Phe Leu Gly Arg Asp Thr Tyr Gly Val Arg Pro
130 135 140
Leu Phe Lys Ala Met Thr Glu Asp Gly Phe Leu Ala Val Cys Ser Glu
145 150 155 160
Ala Lys Gly Leu Val Thr Leu Lys His Ser Ala Thr Pro Phe Leu Lys
165 170 175
Val Glu Pro Phe Leu Pro Gly His Tyr Glu Val Leu Asp Leu Lys Pro
180 185 190
Asn Gly Lys Val Ala Ser Val Glu Met Val Lys Tyr His His Cys Arg
195 200 205
Asp Glu Pro Leu His Ala Leu Tyr Asp Asn Val Glu Lys Leu Phe Pro
210 215 220
Gly Phe Glu Ile Glu Thr Val Lys Asn Asn Leu Arg Ile Leu Phe Asn
225 230 235 240
Asn Ala Val Lys Lys Arg Leu Met Thr Asp Arg Arg Ile Gly Cys Leu
245 250 255
Leu Ser Gly Gly Leu Asp Ser Ser Leu Val Ala Ala Thr Leu Leu Lys
260 265 270
Gln Leu Lys Glu Ala Gln Val Gln Tyr Pro Leu Gln Thr Phe Ala Ile
275 280 285
Gly Met Glu Asp Ser Pro Asp Leu Leu Ala Ala Arg Lys Val Ala Asp
290 295 300
His Ile Gly Ser Glu His Tyr Glu Val Leu Phe Asn Ser Glu Glu Gly
305 310 315 320
Ile Gln Ala Leu Asp Glu Val Ile Phe Ser Leu Glu Thr Tyr Asp Ile
325 330 335
Thr Thr Val Arg Ala Ser Val Gly Met Tyr Leu Ile Ser Lys Tyr Ile
340 345 350
Arg Lys Asn Thr Asp Ser Val Val Ile Phe Ser Gly Glu Gly Ser Asp
355 360 365
Glu Leu Thr Gln Gly Tyr Ile Tyr Phe His Lys Ala Pro Ser Pro Glu
370 375 380
Lys Ala Glu Glu Glu Ser Glu Arg Leu Leu Arg Glu Leu Tyr Leu Phe
385 390 395 400
Asp Val Leu Arg Ala Asp Arg Thr Thr Ala Ala His Gly Leu Glu Leu
405 410 415
Arg Val Pro Phe Leu Asp His Arg Phe Ser Ser Tyr Tyr Leu Ser Leu
420 425 430
Pro Pro Glu Met Arg Ile Pro Lys Asn Gly Ile Glu Lys His Leu Leu
435 440 445
Arg Glu Thr Phe Glu Asp Ser Asn Leu Ile Pro Lys Glu Ile Leu Trp
450 455 460
Arg Pro Lys Glu Ala Phe Ser Asp Gly Ile Thr Ser Val Lys Asn Ser
465 470 475 480

Trp Phe Lys Ile Leu Gln Glu Tyr Val Glu His Gln Val Asp Asp Ala
485 490 495
Met Met Ala Asn Ala Ala Gln Lys Phe Pro Phe Asn Thr Pro Lys Thr
500 505 510
Lys Glu Gly Tyr Tyr Tyr Arg Gln Val Phe Glu Arg His Tyr Pro Gly
515 520 525
Arg Ala Asp Trp Leu Ser His Tyr Trp Met Pro Lys Trp Ile Asn Ala
530 535 540
Thr Asp Pro Ser Ala Arg Thr Leu Thr His Tyr Lys Ser Ala Val Lys
545 550 555 560
Ala

<210> 215
<211> 227
<212> PRT
<213> Homo sapiens
<400> 215
Met Ala Trp Lys Ser Gly Gly Ala Ser His Ser Glu Leu Ile His Asn
1 5 10 15
Leu Arg Lys Asn Gly Ile Ile Lys Thr Asp Lys Val Phe Glu Val Met
20 25 30
Leu Ala Thr Asp Arg Ser His Tyr Ala Lys Cys Asn Pro Tyr Met Asp
35 40 45
Ser Pro Gln Ser Ile Gly Phe Gln Ala Thr Ile Ser Ala Pro His Met
50 55 60
His Ala Tyr Ala Leu Glu Leu Leu Phe Asp Gln Leu His Glu Gly Ala
65 70 75 80
Lys Ala Leu Asp Val Gly Ser Gly Ser Gly Ile Leu Thr Ala Cys Phe
85 90 95
Ala Arg Met Val Gly Cys Thr Gly Lys Val Ile Gly Ile Asp His Ile
100 105 110
Lys Glu Leu Val Asp Asp Ser Val Asn Asn Val Arg Lys Asp Asp Pro
115 120 125
Thr Leu Leu Ser Ser Gly Arg Val Gln Leu Val Val Gly Asp Gly Arg
130 135 140
Met Gly Tyr Ala Glu Glu Ala Pro Tyr Asp Ala Ile His Val Gly Ala
145 150 155 160
Ala Ala Pro Val Val Pro Gln Ala Leu Ile Asp Gln Leu Lys Pro Gly
165 170 175
Gly Arg Leu Ile Leu Pro Val Gly Pro Ala Gly Gly Asn Gln Met Leu
180 185 190
Glu Gln Tyr Asp Lys Leu Gln Asp Gly Ser Ile Lys Met Lys Pro Leu
195 200 205
Met Gly Val Ile Tyr Val Pro Leu Thr Asp Lys Glu Lys Gln Trp Ser
210 215 220
Arg Trp Lys
225

<210> 216
<211> 595
<212> PRT
<213> Homo sapiens
<400> 216
Met Thr Met Thr Leu His Thr Lys Ala Ser Gly Met Ala Leu Leu His
1 5 10 15
Gln Ile Gln Gly Asn Glu Leu Glu Pro Leu Asn Arg Pro Gln Leu Lys
20 25 30
Ile Pro Leu Glu Arg Pro Leu Gly Glu Val Tyr Leu Asp Ser Ser Lys
35 40 45
Pro Ala Val Tyr Asn Tyr Pro Glu Gly Ala Ala Tyr Glu Phe Asn Ala
50 55 60
Ala Ala Ala Ala Asn Ala Gln Val Tyr Gly Gln Thr Gly Leu Pro Tyr
65 70 75 80
Gly Pro Gly Ser Glu Ala Ala Ala Phe Gly Ser Asn Gly Leu Gly Gly
85 90 95
Phe Pro Pro Leu Asn Ser Val Ser Pro Ser Pro Leu Met Leu Leu His
100 105 110
Pro Pro Pro Gln Leu Ser Pro Phe Leu Gln Pro His Gly Gln Gln Val

115 120 125
Pro Tyr Tyr Leu Glu Asn Glu Pro Ser Gly Tyr Thr Val Arg Glu Ala
130 135 140
Gly Pro Pro Ala Phe Tyr Arg Pro Asn Ser Asp Asn Arg Arg Gln Gly
145 150 155 160
Gly Arg Glu Arg Leu Ala Ser Thr Asn Asp Lys Gly Ser Met Ala Met
165 170 175
Glu Ser Ala Lys Glu Thr Arg Tyr Cys Ala Val Cys Asn Asp Tyr Ala
180 185 190
Ser Gly Tyr His Tyr Gly Val Trp Ser Cys Glu Gly Cys Lys Ala Phe
195 200 205
Phe Lys Arg Ser Ile Gln Gly His Asn Asp Tyr Met Cys Pro Ala Thr
210 215 220
Asn Gln Cys Thr Ile Asp Lys Asn Arg Arg Lys Ser Cys Gln Ala Cys
225 230 235 240
Arg Leu Arg Lys Cys Tyr Glu Val Gly Met Met Lys Gly Gly Ile Arg
245 250 255
Lys Asp Arg Arg Gly Gly Arg Met Leu Lys His Lys Arg Gln Arg Asp
260 265 270
Asp Gly Glu Gly Arg Gly Glu Val Gly Ser Ala Gly Asp Met Arg Ala
275 280 285
Ala Asn Leu Trp Pro Ser Pro Leu Met Ile Lys Arg Ser Lys Lys Asn
290 295 300
Ser Leu Ala Leu Ser Leu Thr Ala Asp Gln Met Val Ser Ala Leu Leu
305 310 315 320
Asp Ala Glu Pro Pro Ile Leu Tyr Ser Glu Tyr Asp Pro Thr Arg Pro
325 330 335
Phe Ser Glu Ala Ser Met Met Gly Leu Leu Thr Asn Leu Ala Asp Arg
340 345 350
Glu Leu Val His Met Ile Asn Trp Ala Lys Arg Val Pro Gly Phe Val
355 360 365
Asp Leu Thr Leu His Asp Gln Val His Leu Leu Glu Cys Ala Trp Leu
370 375 380
Glu Ile Leu Met Ile Gly Leu Val Trp Arg Ser Met Glu His Pro Val
385 390 395 400
Lys Leu Leu Phe Ala Pro Asn Leu Leu Leu Asp Arg Asn Gln Gly Lys
405 410 415
Cys Val Glu Gly Met Val Glu Ile Phe Asp Met Leu Leu Ala Thr Ser
420 425 430
Ser Arg Phe Arg Met Met Asn Leu Gln Gly Glu Glu Phe Val Cys Leu
435 440 445
Lys Ser Ile Ile Leu Leu Asn Ser Gly Val Tyr Thr Phe Leu Ser Ser
450 455 460
Thr Leu Lys Ser Leu Glu Glu Lys Asp His Ile His Arg Val Leu Asp
465 470 475 480
Lys Ile Thr Asp Thr Leu Ile His Leu Met Ala Lys Ala Gly Leu Thr
485 490 495
Leu Gln Gln Gln His Gln Arg Leu Ala Gln Leu Leu Leu Ile Leu Ser
500 505 510
His Ile Arg His Met Ser Asn Lys Gly Met Glu His Leu Tyr Ser Met
515 520 525
Lys Cys Lys Asn Val Val Pro Leu Tyr Asp Leu Leu Leu Glu Met Leu
530 535 540
Asp Ala His Arg Leu His Ala Pro Thr Ser Arg Gly Gly Ala Ser Val
545 550 555 560
Glu Glu Thr Asp Gln Ser His Leu Ala Thr Ala Gly Ser Thr Ser Ser
565 570 575
His Ser Leu Gln Lys Tyr Tyr Ile Thr Gly Glu Ala Glu Gly Phe Pro
580 585 590
Ala Thr Val
595

<210> 217
<211> 427
<212> PRT
<213> Homo sapiens
<400> 217
Met Ala Val Leu Ala Ala Leu Leu Arg Ser Gly Ala Arg Ser Arg Ser
1 5 10 15

Pro Leu Leu Arg Arg Leu Val Gln Glu Ile Arg Tyr Val Glu Arg Ser
20 25 30
Tyr Val Ser Lys Pro Thr Leu Lys Glu Val Val Ile Val Ser Ala Thr
35 40 45
Arg Thr Pro Ile Gly Ser Phe Leu Gly Ser Leu Ser Leu Leu Pro Ala
50 55 60
Thr Lys Leu Gly Ser Ile Ala Ile Gln Gly Ala Ile Glu Lys Ala Gly
65 70 75 80
Ile Pro Lys Glu Glu Val Lys Glu Ala Tyr Met Gly Asn Val Leu Gln
85 90 95
Gly Gly Glu Gly Gln Ala Pro Thr Arg Gln Ala Val Leu Gly Ala Gly
100 105 110
Leu Pro Ile Ser Thr Pro Cys Thr Thr Ile Asn Lys Val Cys Ala Ser
115 120 125
Gly Met Lys Ala Ile Met Met Ala Ser Gln Ser Leu Met Cys Gly His
130 135 140
Gln Asp Val Met Val Ala Gly Gly Met Glu Ser Met Ser Asn Val Pro
145 150 155 160
Tyr Val Met Asn Arg Gly Ser Thr Pro Tyr Gly Gly Val Lys Leu Glu
165 170 175
Asp Leu Ile Val Lys Asp Gly Leu Thr Asp Val Tyr Asn Lys Ile His
180 185 190
Met Gly Ser Cys Ala Glu Asn Thr Ala Lys Lys Leu Asn Ile Ala Arg
195 200 205
Asn Glu Gln Asp Ala Tyr Ala Ile Asn Ser Tyr Thr Arg Ser Lys Ala
210 215 220
Ala Trp Glu Ala Gly Lys Phe Gly Asn Glu Val Ile Pro Val Thr Val
225 230 235 240
Thr Val Lys Gly Gln Pro Asp Val Val Val Lys Glu Asp Glu Glu Tyr
245 250 255
Lys Arg Val Asp Phe Ser Lys Val Pro Lys Leu Lys Thr Val Phe Gln
260 265 270
Lys Glu Asn Gly Thr Val Thr Ala Ala Asn Ala Ser Thr Leu Asn Asp
275 280 285
Gly Ala Ala Ala Leu Val Leu Met Thr Ala Asp Ala Ala Lys Arg Leu
290 295 300
Asn Val Thr Pro Leu Ala Arg Ile Val Ala Phe Ala Asp Ala Ala Val
305 310 315 320
Glu Pro Ile Asp Phe Pro Ile Ala Pro Val Tyr Ala Ala Ser Met Val
325 330 335
Leu Lys Asp Val Gly Leu Lys Lys Glu Asp Ile Ala Met Trp Glu Val
340 345 350
Asn Glu Ala Phe Ser Leu Val Val Leu Ala Asn Ile Lys Met Leu Glu
355 360 365
Ile Asp Pro Gln Lys Val Asn Ile Asn Gly Gly Ala Val Ser Leu Gly
370 375 380
His Pro Ile Gly Met Ser Gly Ala Arg Ile Val Gly His Leu Thr His
385 390 395 400
Ala Leu Lys Gln Gly Glu Tyr Gly Leu Ala Ser Ile Cys Asn Gly Gly
405 410 415
Gly Gly Ala Ser Ala Met Leu Ile Gln Lys Leu
420 425

<210> 218
<211> 273
<212> PRT
<213> Homo sapiens
<400> 218
Met Ala Ala Ala Asp Gly Ala Leu Pro Glu Ala Ala Ala Leu Glu Gln
1 5 10 15
Pro Ala Glu Leu Pro Ala Ser Val Arg Ala Ser Ile Glu Arg Lys Arg
20 25 30
Gln Arg Ala Leu Met Leu Arg Gln Ala Arg Leu Ala Ala Arg Pro Tyr
35 40 45
Ser Ala Thr Ala Ala Ala Ala Thr Gly Gly Met Ala Asn Val Lys Ala
50 55 60
Ala Pro Lys Ile Ile Asp Thr Gly Gly Gly Phe Ile Leu Glu Glu Glu
65 70 75 80
Glu Glu Glu Glu Gln Lys Ile Gly Lys Val Val His Gln Pro Gly Pro

85 90 95
Val Met Glu Phe Asp Tyr Val Ile Cys Glu Glu Cys Gly Lys Glu Phe
100 105 110
Met Asp Ser Tyr Leu Met Asn His Phe Asp Leu Pro Thr Cys Asp Asn
115 120 125
Cys Arg Asp Ala Asp Asp Lys His Lys Leu Ile Thr Lys Thr Glu Ala
130 135 140
Lys Gln Glu Tyr Leu Leu Lys Asp Cys Asp Leu Glu Lys Arg Glu Pro
145 150 155 160
Pro Leu Lys Phe Ile Val Lys Lys Asn Pro His His Ser Gln Trp Gly
165 170 175
Asp Met Lys Leu Tyr Leu Lys Leu Gln Ile Val Lys Arg Ser Leu Glu
180 185 190
Val Trp Gly Ser Gln Glu Ala Leu Glu Glu Ala Lys Glu Val Arg Gln
195 200 205
Glu Asn Arg Glu Lys Met Lys Gln Lys Lys Phe Asp Lys Lys Val Lys
210 215 220
Glu Leu Arg Arg Ala Val Arg Ser Ser Val Trp Lys Arg Glu Thr Ile
225 230 235 240
Val His Gln His Glu Tyr Gly Pro Glu Glu Asn Leu Glu Asp Asp Met
245 250 255
Tyr Arg Lys Thr Cys Thr Met Cys Gly His Glu Leu Thr Tyr Glu Lys
260 265 270
Met

<210> 219
<211> 1227
<212> PRT
<213> Homo sapiens
<400> 219
Met Asp Ser Phe Asp Leu Ala Leu Leu Gln Glu Trp Asp Leu Glu Ser
1 5 10 15
Leu Cys Val Tyr Glu Pro Asp Arg Asn Ala Leu Arg Arg Lys Glu Arg
20 25 30
Glu Arg Arg Asn Gln Glu Thr Gln Gln Asp Asp Gly Thr Phe Asn Ser
35 40 45
Ser Tyr Ser Leu Phe Ser Glu Pro Tyr Lys Thr Asn Lys Gly Asp Glu
50 55 60
Leu Ser Asn Arg Ile Gln Asn Thr Leu Gly Asn Tyr Asp Glu Met Lys
65 70 75 80
Asp Phe Leu Thr Asp Arg Thr Asn Gln Ser His Leu Val Gly Val Pro
85 90 95
Lys Pro Gly Val Pro Gln Thr Pro Val Asn Lys Ile Asp Glu His Phe
100 105 110
Val Ala Asp Ser Arg Ala Gln Asn Gln Pro Ser Ser Ile Cys Ser Thr
115 120 125
Thr Thr Ser Thr Pro Ala Ala Val Pro Val Gln Gln Ser Lys Arg Gly
130 135 140
Thr Met Gly Trp Gln Lys Ala Gly His Pro Pro Ser Asp Gly Gln Gln
145 150 155 160
Arg Ala Thr Gln Gln Gly Ser Leu Arg Thr Leu Leu Gly Asp Gly Val
165 170 175
Gly Arg Gln Gln Pro Arg Ala Lys Gln Val Cys Asn Val Glu Val Gly
180 185 190
Leu Gln Thr Gln Glu Arg Pro Pro Ala Met Ala Ala Lys His Ser Ser
195 200 205
Ser Gly His Cys Val Gln Asn Phe Pro Pro Ser Leu Ala Ser Lys Pro
210 215 220
Ser Leu Val Gln Gln Lys Pro Thr Ala Tyr Val Arg Pro Met Asp Gly
225 230 235 240
Gln Asp Gln Ala Pro Asp Glu Ser Pro Lys Leu Lys Ser Ser Ser Glu
245 250 255
Thr Ser Val His Cys Thr Ser Tyr Arg Gly Val Pro Ala Ser Lys Pro
260 265 270
Glu Pro Ala Arg Ala Lys Ala Lys Leu Ser Lys Phe Ser Ile Pro Lys
275 280 285
Gln Gly Glu Glu Ser Arg Ser Gly Glu Thr Asn Ser Cys Val Glu Glu
290 295 300

Ile Ile Arg Glu Met Thr Trp Leu Pro Pro Leu Ser Ala Ile Gln Ala
305 310 315 320
Pro Gly Lys Val Glu Pro Thr Lys Phe Pro Phe Pro Asn Lys Asp Ser
325 330 335
Gln Leu Val Ser Ser Gly His Asn Asn Pro Lys Lys Gly Asp Ala Glu
340 345 350
Pro Glu Ser Pro Asp Asn Gly Thr Ser Asn Thr Ser Met Leu Glu Asp
355 360 365
Asp Leu Lys Leu Ser Ser Asp Glu Glu Glu Asn Glu Gln Gln Ala Ala
370 375 380
Gln Arg Thr Ala Leu Arg Ala Leu Ser Asp Ser Ala Val Val Gln Gln
385 390 395 400
Pro Asn Cys Arg Thr Ser Val Pro Ser Ser Lys Gly Ser Ser Ser Ser
405 410 415
Ser Ser Ser Gly Thr Ser Ser Ser Ser Ser Asp Ser Glu Ser Ser Ser
420 425 430
Gly Ser Asp Ser Glu Thr Glu Ser Ser Ser Ser Glu Ser Glu Gly Ser
435 440 445
Lys Pro Pro His Phe Ser Ser Pro Glu Ala Glu Pro Ala Ser Ser Asn
450 455 460
Lys Trp Gln Leu Asp Lys Trp Leu Asn Lys Val Asn Pro His Lys Pro
465 470 475 480
Pro Ile Leu Ile Gln Asn Glu Ser His Gly Ser Glu Ser Asn Gln Tyr
485 490 495
Tyr Asn Pro Val Lys Glu Asp Val Gln Asp Cys Gly Lys Val Pro Asp
500 505 510
Val Cys Gln Pro Ser Leu Arg Glu Lys Glu Ile Lys Ser Thr Cys Lys
515 520 525
Glu Glu Gln Arg Pro Arg Thr Ala Asn Lys Ala Pro Gly Ser Lys Gly
530 535 540
Val Lys Gln Lys Ser Pro Pro Ala Ala Val Ala Val Ala Val Ser Ala
545 550 555 560
Ala Ala Pro Pro Pro Ala Val Pro Cys Ala Pro Ala Glu Asn Ala Pro
565 570 575
Ala Pro Ala Arg Arg Ser Ala Gly Lys Lys Pro Thr Arg Arg Thr Glu
580 585 590
Arg Thr Ser Ala Gly Asp Gly Ala Asn Cys His Arg Pro Glu Glu Pro
595 600 605
Ala Ala Ala Asp Ala Leu Gly Thr Ser Val Val Val Pro Pro Glu Pro
610 615 620
Thr Lys Thr Arg Pro Cys Gly Asn Asn Arg Ala Ser His Arg Lys Glu
625 630 635 640
Leu Arg Ser Ser Val Thr Cys Glu Lys Arg Arg Thr Arg Gly Leu Ser
645 650 655
Arg Ile Val Pro Lys Ser Lys Glu Phe Ile Glu Thr Glu Ser Ser Ser
660 665 670
Ser Ser Ser Ser Ser Asp Ser Asp Leu Glu Ser Glu Gln Glu Glu Tyr
675 680 685
Pro Leu Ser Lys Ala Gln Thr Val Ala Ala Ser Ala Ser Ser Gly Asn
690 695 700
Asp Gln Arg Leu Lys Glu Ala Ala Ala Asn Gly Gly Ser Gly Pro Arg
705 710 715 720
Ala Pro Val Gly Ser Ile Asn Ala Arg Thr Thr Ser Asp Ile Ala Lys
725 730 735
Glu Leu Glu Glu Gln Phe Tyr Thr Leu Val Pro Phe Gly Arg Asn Glu
740 745 750
Leu Leu Ser Pro Leu Lys Asp Ser Asp Glu Ile Arg Ser Leu Trp Val
755 760 765
Lys Ile Asp Leu Thr Leu Leu Ser Arg Ile Pro Glu His Leu Pro Gln
770 775 780
Glu Pro Gly Val Leu Ser Ala Pro Ala Thr Lys Asp Ser Glu Ser Ala
785 790 795 800
Pro Pro Ser His Thr Ser Asp Thr Pro Ala Glu Lys Ala Leu Pro Lys
805 810 815
Ser Lys Arg Lys Arg Lys Cys Asp Asn Glu Asp Asp Tyr Arg Glu Ile
820 825 830
Lys Lys Ser Gln Gly Glu Lys Asp Ser Ser Ser Arg Leu Ala Thr Ser
835 840 845
Thr Ser Asn Thr Leu Ser Ala Asn His Cys Asn Met Asn Ile Asn Ser

850 855 860
Val Ala Ile Pro Ile Asn Lys Asn Glu Lys Met Leu Arg Ser Pro Ile
865 870 875 880
Ser Pro Leu Ser Asp Ala Ser Lys His Lys Tyr Thr Ser Glu Asp Leu
885 890 895
Thr Ser Ser Ser Arg Pro Asn Gly Asn Ser Leu Phe Thr Ser Ala Ser
900 905 910
Ser Ser Lys Lys Pro Lys Ala Asp Ser Gln Leu Gln Pro His Gly Gly
915 920 925
Asp Leu Thr Lys Ala Ala His Asn Asn Ser Glu Asn Ile Pro Leu His
930 935 940
Lys Ser Arg Pro Gln Thr Lys Pro Trp Ser Pro Gly Ser Asn Gly His
945 950 955 960
Arg Asp Cys Lys Arg Gln Lys Leu Val Phe Asp Asp Met Pro Arg Ser
965 970 975
Ala Asp Tyr Phe Met Gln Glu Ala Lys Arg Met Lys His Lys Ala Asp
980 985 990
Ala Met Val Glu Lys Phe Gly Lys Ala Leu Asn Tyr Ala Glu Ala Ala
995 1000 1005
Leu Ser Phe Ile Glu Cys Gly Asn Ala Met Glu Gln Gly Pro Met
1010 1015 1020
Glu Ser Lys Ser Pro Tyr Tyr Leu Met Tyr Ser Glu Thr Val Glu
1025 1030 1035
Leu Ile Arg Tyr Ala Met Arg Leu Lys Thr His Ser Gly Pro Asn
1040 1045 1050
Ala Thr Pro Glu Asp Lys Gln Leu Ala Ala Leu Cys Tyr Arg Cys
1055 1060 1065
Leu Ala Leu Leu Tyr Trp Arg Met Phe Arg Leu Lys Arg Asp His
1070 1075 1080
Ala Val Lys Tyr Ser Lys Ala Leu Ile Asp Tyr Phe Lys Asn Ser
1085 1090 1095
Ser Lys Ala Ala Gln Ala Pro Ser Pro Trp Gly Ala Ser Gly Lys
1100 1105 1110
Ser Thr Gly Thr Pro Ser Pro Ile Ser Pro Asn Pro Phe Pro Gly
1115 1120 1125
Ser Ser Val Gly Ser Gln Gly Ser Leu Ser Asn Ala Ser Ala Leu
1130 1135 1140
Ser Pro Ser Thr Ile Val Ser Ile Pro Gln Arg Ile His Gln Met
1145 1150 1155
Ala Ala Asn His Val Ser Ile Thr Asn Ser Ile Leu His Ser Tyr
1160 1165 1170
Asp Tyr Trp Glu Met Ala Asp Asn Leu Ala Lys Glu Asn Arg Glu
1175 1180 1185
Phe Phe Asn Asp Leu Asp Leu Leu Met Gly Pro Val Thr Leu His
1190 1195 1200
Ser Ser Met Glu His Leu Val Gln Tyr Ser Gln Gln Gly Leu His
1205 1210 1215
Trp Leu Arg Asn Ser Ala His Leu Ser
1220 1225

<210> 220
<211> 680
<212> PRT
<213> Homo sapiens
<400> 220
Met Leu Pro Gln Ile Pro Phe Leu Leu Leu Val Ser Leu Asn Leu Val
1 5 10 15
His Gly Val Phe Tyr Ala Glu Arg Tyr Gln Met Pro Thr Gly Ile Lys
20 25 30
Gly Pro Leu Pro Asn Thr Lys Thr Gln Phe Phe Ile Pro Tyr Thr Ile
35 40 45
Lys Ser Lys Gly Ile Ala Val Arg Gly Glu Gln Gly Thr Pro Gly Pro
50 55 60
Pro Gly Pro Ala Gly Pro Arg Gly His Pro Gly Pro Ser Gly Pro Pro
65 70 75 80
Gly Lys Pro Gly Tyr Gly Ser Pro Gly Leu Gln Gly Glu Pro Gly Leu
85 90 95
Pro Gly Pro Pro Gly Pro Ser Ala Val Gly Lys Pro Gly Val Pro Gly
100 105 110

```
Leu Pro Gly Lys Pro Gly Glu Arg Gly Pro Tyr Gly Pro Lys Gly Asp
        115                 120                 125
Val Gly Pro Ala Gly Leu Pro Gly Pro Arg Gly Pro Pro Gly Pro Pro
    130                 135                 140
Gly Ile Pro Gly Pro Ala Gly Ile Ser Val Pro Gly Lys Pro Gly Gln
145                 150                 155                 160
Gln Gly Pro Thr Gly Ala Pro Gly Pro Arg Gly Phe Pro Gly Glu Lys
                165                 170                 175
Gly Ala Pro Gly Val Pro Gly Met Asn Gly Gln Lys Gly Glu Met Gly
            180                 185                 190
Tyr Gly Ala Pro Gly Arg Pro Gly Glu Arg Gly Leu Pro Gly Pro Gln
        195                 200                 205
Gly Pro Thr Gly Pro Ser Gly Pro Pro Gly Val Gly Lys Arg Gly Glu
    210                 215                 220
Asn Gly Val Pro Gly Gln Pro Gly Ile Lys Gly Asp Arg Gly Phe Pro
225                 230                 235                 240
Gly Glu Met Gly Pro Ile Gly Pro Pro Gly Pro Gln Gly Pro Pro Gly
                245                 250                 255
Glu Arg Gly Pro Glu Gly Ile Gly Lys Pro Gly Ala Ala Gly Ala Pro
            260                 265                 270
Gly Gln Pro Gly Ile Pro Gly Thr Lys Gly Leu Pro Gly Ala Pro Gly
        275                 280                 285
Ile Ala Gly Pro Pro Gly Pro Pro Gly Phe Gly Lys Pro Gly Leu Pro
    290                 295                 300
Gly Leu Lys Gly Glu Arg Gly Pro Ala Gly Leu Pro Gly Gly Pro Gly
305                 310                 315                 320
Ala Lys Gly Glu Gln Gly Pro Ala Gly Leu Pro Gly Lys Pro Gly Leu
                325                 330                 335
Thr Gly Pro Pro Gly Asn Met Gly Pro Gln Gly Pro Lys Gly Ile Pro
            340                 345                 350
Gly Ser His Gly Leu Pro Gly Pro Lys Gly Glu Thr Gly Pro Ala Gly
        355                 360                 365
Pro Ala Gly Tyr Pro Gly Ala Lys Gly Glu Arg Gly Ser Pro Gly Ser
    370                 375                 380
Asp Gly Lys Pro Gly Tyr Pro Gly Lys Pro Gly Leu Asp Gly Pro Lys
385                 390                 395                 400
Gly Asn Pro Gly Leu Pro Gly Pro Lys Gly Asp Pro Gly Val Gly Gly
                405                 410                 415
Pro Pro Gly Leu Pro Gly Pro Val Gly Pro Ala Gly Ala Lys Gly Met
            420                 425                 430
Pro Gly His Asn Gly Glu Ala Gly Pro Arg Gly Ala Pro Gly Ile Pro
        435                 440                 445
Gly Thr Arg Gly Pro Ile Gly Pro Pro Gly Ile Pro Gly Phe Pro Gly
    450                 455                 460
Ser Lys Gly Asp Pro Gly Ser Pro Gly Pro Pro Gly Pro Ala Gly Ile
465                 470                 475                 480
Ala Thr Lys Gly Leu Asn Gly Pro Thr Gly Pro Pro Gly Pro Pro Gly
                485                 490                 495
Pro Arg Gly His Ser Gly Glu Pro Gly Leu Pro Gly Pro Pro Gly Pro
            500                 505                 510
Pro Gly Pro Pro Gly Gln Ala Val Met Pro Glu Gly Phe Ile Lys Ala
        515                 520                 525
Gly Gln Arg Pro Ser Leu Ser Gly Thr Pro Leu Val Ser Ala Asn Gln
    530                 535                 540
Gly Val Thr Gly Met Pro Val Ser Ala Phe Thr Val Ile Leu Ser Lys
545                 550                 555                 560
Ala Tyr Pro Ala Ile Gly Thr Pro Ile Pro Phe Asp Lys Ile Leu Tyr
                565                 570                 575
Asn Arg Gln Gln His Tyr Asp Pro Arg Thr Gly Ile Phe Thr Cys Gln
            580                 585                 590
Ile Pro Gly Ile Tyr Tyr Phe Ser Tyr His Val His Val Lys Gly Thr
        595                 600                 605
His Val Trp Val Gly Leu Tyr Lys Asn Gly Thr Pro Val Met Tyr Thr
    610                 615                 620
Tyr Asp Glu Tyr Thr Lys Gly Tyr Leu Asp Gln Ala Ser Gly Ser Ala
625                 630                 635                 640
Ile Ile Asp Leu Thr Glu Asn Asp Gln Val Trp Leu Gln Leu Pro Asn
                645                 650                 655
Ala Glu Ser Asn Gly Leu Tyr Ser Ser Glu Tyr Val His Ser Ser Phe
```

```
        660                 665                 670
Ser Gly Phe Leu Val Ala Pro Met
        675                 680

<210>  221
<211>  622
<212>  PRT
<213>  Homo sapiens
<400>  221
Met Gly Leu Tyr Gly Gln Ala Cys Pro Ser Val Thr Ser Leu Arg Met
1               5                   10                  15
Thr Ser Glu Leu Glu Ser Ser Leu Thr Ser Met Asp Trp Leu Pro Gln
            20                  25                  30
Leu Thr Met Arg Ala Ala Ile Gln Lys Ser Asp Ala Thr Gln Asn Ala
        35                  40                  45
His Gly Thr Gly Ile Ser Lys Lys Asn Ala Leu Leu Asp Pro Asn Thr
    50                  55                  60
Thr Leu Asp Gln Glu Glu Val Gln Gln His Lys Asp Gly Lys Pro Pro
65                  70                  75                  80
Tyr Ser Tyr Ala Ser Leu Ile Thr Phe Ala Ile Asn Ser Ser Pro Lys
                85                  90                  95
Lys Lys Met Thr Leu Ser Glu Ile Tyr Gln Trp Ile Cys Asp Asn Phe
            100                 105                 110
Pro Tyr Tyr Arg Glu Ala Gly Ser Gly Trp Lys Asn Ser Ile Arg His
        115                 120                 125
Asn Leu Ser Leu Asn Lys Cys Phe Leu Lys Val Pro Arg Ser Lys Asp
    130                 135                 140
Asp Pro Gly Lys Gly Ser Tyr Trp Ala Ile Asp Thr Asn Pro Lys Glu
145                 150                 155                 160
Asp Ala Leu Pro Thr Arg Pro Lys Lys Arg Ala Arg Ser Val Glu Arg
                165                 170                 175
Ala Ser Thr Pro Tyr Ser Ile Asp Ser Asp Ser Leu Gly Met Glu Cys
            180                 185                 190
Ile Ile Ser Gly Ser Ala Ser Pro Thr Leu Ala Ile Asn Thr Val Thr
        195                 200                 205
Asn Lys Val Thr Leu Tyr Asn Thr Asp Gln Asp Gly Ser Asp Ser Pro
    210                 215                 220
Arg Ser Ser Leu Asn Asn Ser Leu Ser Asp Gln Ser Leu Ala Ser Val
225                 230                 235                 240
Asn Leu Asn Ser Val Gly Ser Val His Ser Tyr Thr Pro Val Thr Ser
                245                 250                 255
His Pro Glu Ser Val Ser Gln Ser Leu Thr Pro Gln Gln Gln Pro Gln
            260                 265                 270
Tyr Asn Leu Pro Glu Arg Asp Lys Gln Leu Leu Phe Ser Glu Tyr Asn
        275                 280                 285
Phe Glu Asp Leu Ser Ala Ser Phe Arg Ser Leu Tyr Lys Ser Val Phe
    290                 295                 300
Glu Gln Ser Leu Ser Gln Gln Gly Leu Met Asn Ile Pro Ser Glu Ser
305                 310                 315                 320
Ser Gln Gln Ser His Thr Ser Cys Thr Tyr Gln His Ser Pro Ser Ser
                325                 330                 335
Thr Val Ser Thr His Pro His Ser Asn Gln Ser Ser Leu Ser Asn Ser
            340                 345                 350
His Gly Ser Gly Leu Asn Thr Thr Gly Ser Asn Ser Val Ala Gln Val
        355                 360                 365
Ser Leu Ser His Pro Gln Met His Pro Gln Pro Ser Pro His Pro Pro
    370                 375                 380
His Arg Pro His Gly Leu Pro Gln His Pro Gln Arg Ser Pro His Pro
385                 390                 395                 400
Ala Pro His Pro Gln Gln His Ser Gln Leu Gln Ser Pro His Pro Gln
                405                 410                 415
His Pro Ser Pro His Gln His Ile Gln His His Pro Asn His Gln His
            420                 425                 430
Gln Thr Leu Thr His Gln Ala Pro Pro Pro Pro Gln Gln Val Ser Cys
        435                 440                 445
Asn Ser Gly Val Ser Asn Asp Trp Tyr Ala Thr Leu Asp Met Leu Lys
    450                 455                 460
Glu Ser Cys Arg Ile Ala Ser Ser Val Asn Trp Ser Asp Val Asp Leu
465                 470                 475                 480
```

Ser Gln Phe Gln Gly Leu Met Glu Ser Met Arg Gln Ala Asp Leu Lys
485 490 495
Asn Trp Ser Leu Asp Gln Val Gln Phe Ala Asp Leu Cys Ser Ser Leu
500 505 510
Asn Gln Phe Phe Thr Gln Thr Gly Leu Ile His Ser Gln Ser Asn Val
515 520 525
Gln Gln Asn Val Cys His Gly Ala Met His Pro Thr Lys Pro Ser Gln
530 535 540
His Ile Gly Thr Gly Asn Leu Tyr Ile Asp Ser Arg Gln Asn Leu Pro
545 550 555 560
Pro Ser Val Met Pro Pro Pro Gly Tyr Pro His Ile Pro Gln Ala Leu
565 570 575
Ser Thr Pro Gly Thr Thr Met Ala Gly His His Arg Ala Met Asn Gln
580 585 590
Gln His Met Met Pro Ser Gln Ala Phe Gln Met Arg Arg Ser Leu Pro
595 600 605
Pro Asp Asp Ile Gln Asp Asp Phe Asp Trp Asp Ser Ile Val
610 615 620

<210> 222
<211> 441
<212> PRT
<213> Homo sapiens
<400> 222
Met Val Pro Pro Lys Leu His Val Leu Phe Cys Leu Cys Gly Cys Leu
1 5 10 15
Ala Val Val Tyr Pro Phe Asp Trp Gln Tyr Ile Asn Pro Val Ala His
20 25 30
Met Lys Ser Ser Ala Trp Val Asn Lys Ile Gln Val Leu Met Ala Ala
35 40 45
Ala Ser Phe Gly Gln Thr Lys Ile Pro Arg Gly Asn Gly Pro Tyr Ser
50 55 60
Val Gly Cys Thr Asp Leu Met Phe Asp His Thr Asn Lys Gly Thr Phe
65 70 75 80
Leu Arg Leu Tyr Tyr Pro Ser Gln Asp Asn Asp Arg Leu Asp Thr Leu
85 90 95
Trp Ile Pro Asn Lys Glu Tyr Phe Trp Gly Leu Ser Lys Phe Leu Gly
100 105 110
Thr His Trp Leu Met Gly Asn Ile Leu Arg Leu Leu Phe Gly Ser Met
115 120 125
Thr Thr Pro Ala Asn Trp Asn Ser Pro Leu Arg Pro Gly Glu Lys Tyr
130 135 140
Pro Leu Val Val Phe Ser His Gly Leu Gly Ala Phe Arg Thr Leu Tyr
145 150 155 160
Ser Ala Ile Gly Ile Asp Leu Ala Ser His Gly Phe Ile Val Ala Ala
165 170 175
Val Glu His Arg Asp Arg Ser Ala Ser Ala Thr Tyr Tyr Phe Lys Asp
180 185 190
Gln Ser Ala Ala Glu Ile Gly Asp Lys Ser Trp Leu Tyr Leu Arg Thr
195 200 205
Leu Lys Gln Glu Glu Glu Thr His Ile Arg Asn Glu Gln Val Arg Gln
210 215 220
Arg Ala Lys Glu Cys Ser Gln Ala Leu Ser Leu Ile Leu Asp Ile Asp
225 230 235 240
His Gly Lys Pro Val Lys Asn Ala Leu Asp Leu Lys Phe Asp Met Glu
245 250 255
Gln Leu Lys Asp Ser Ile Asp Arg Glu Lys Ile Ala Val Ile Gly His
260 265 270
Ser Phe Gly Gly Ala Thr Val Ile Gln Thr Leu Ser Glu Asp Gln Arg
275 280 285
Phe Arg Cys Gly Ile Ala Leu Asp Ala Trp Met Phe Pro Leu Gly Asp
290 295 300
Glu Val Tyr Ser Arg Ile Pro Gln Pro Leu Phe Phe Ile Asn Ser Glu
305 310 315 320
Tyr Phe Gln Tyr Pro Ala Asn Ile Ile Lys Met Lys Lys Cys Tyr Ser
325 330 335
Pro Asp Lys Glu Arg Lys Met Ile Thr Ile Arg Gly Ser Val His Gln
340 345 350
Asn Phe Ala Asp Phe Thr Phe Ala Thr Gly Lys Ile Ile Gly His Met

```
        355                 360                 365
Leu Lys Leu Lys Gly Asp Ile Asp Ser Asn Ala Ala Ile Asp Leu Ser
    370                 375                 380
Asn Lys Ala Ser Leu Ala Phe Leu Gln Lys His Leu Gly Leu His Lys
385                 390                 395                 400
Asp Phe Asp Gln Trp Asp Cys Leu Ile Glu Gly Asp Asp Glu Asn Leu
                405                 410                 415
Ile Pro Gly Thr Asn Ile Asn Thr Thr Asn Gln His Ile Met Leu Gln
            420                 425                 430
Asn Ser Ser Gly Ile Glu Lys Tyr Asn
        435                 440

<210>  223
<211>  148
<212>  PRT
<213>  Homo sapiens
<400>  223
Met Arg Gly Arg Glu Leu Pro Leu Val Leu Leu Ala Leu Val Leu Cys
1               5                   10                  15
Leu Ala Pro Arg Gly Arg Ala Val Pro Leu Pro Ala Gly Gly Gly Thr
            20                  25                  30
Val Leu Thr Lys Met Tyr Pro Arg Gly Asn His Trp Ala Val Gly His
        35                  40                  45
Leu Met Gly Lys Lys Ser Thr Gly Glu Ser Ser Ser Val Ser Glu Arg
    50                  55                  60
Gly Ser Leu Lys Gln Gln Leu Arg Glu Tyr Ile Arg Trp Glu Glu Ala
65                  70                  75                  80
Ala Arg Asn Leu Leu Gly Leu Ile Glu Ala Lys Glu Asn Arg Asn His
                85                  90                  95
Gln Pro Pro Gln Pro Lys Ala Leu Gly Asn Gln Gln Pro Ser Trp Asp
            100                 105                 110
Ser Glu Asp Ser Ser Asn Phe Lys Asp Val Gly Ser Lys Gly Lys Val
        115                 120                 125
Gly Arg Leu Ser Ala Pro Gly Ser Gln Arg Glu Gly Arg Asn Pro Gln
    130                 135                 140
Leu Asn Gln Gln
145

<210>  224
<211>  491
<212>  PRT
<213>  Homo sapiens
<400>  224
Met Glu Leu Ser Val Leu Leu Phe Leu Ala Leu Leu Thr Gly Leu Leu
1               5                   10                  15
Leu Leu Leu Val Gln Arg His Pro Asn Thr His Asp Arg Leu Pro Pro
            20                  25                  30
Gly Pro Arg Pro Leu Pro Leu Leu Gly Asn Leu Leu Gln Met Asp Arg
        35                  40                  45
Arg Gly Leu Leu Lys Ser Phe Leu Arg Phe Arg Glu Lys Tyr Gly Asp
    50                  55                  60
Val Phe Thr Val His Leu Gly Pro Arg Pro Val Val Met Leu Cys Gly
65                  70                  75                  80
Val Glu Ala Ile Arg Glu Ala Leu Val Asp Lys Ala Glu Ala Phe Ser
                85                  90                  95
Gly Arg Gly Lys Ile Ala Met Val Asp Pro Phe Phe Arg Gly Tyr Gly
            100                 105                 110
Val Ile Phe Ala Asn Gly Asn Arg Trp Lys Val Leu Arg Arg Phe Ser
        115                 120                 125
Val Thr Thr Met Arg Asp Phe Gly Met Gly Lys Arg Ser Val Glu Glu
    130                 135                 140
Arg Ile Gln Glu Glu Ala Gln Cys Leu Ile Glu Glu Leu Arg Lys Ser
145                 150                 155                 160
Lys Gly Ala Leu Met Asp Pro Thr Phe Leu Phe Gln Ser Ile Thr Ala
                165                 170                 175
Asn Ile Ile Cys Ser Ile Val Phe Gly Lys Arg Phe His Tyr Gln Asp
            180                 185                 190
Gln Glu Phe Leu Lys Met Leu Asn Leu Phe Tyr Gln Thr Phe Ser Leu
        195                 200                 205
```

Ile Ser Ser Val Phe Gly Gln Leu Phe Glu Leu Phe Ser Gly Phe Leu
210 215 220
Lys Tyr Phe Pro Gly Ala His Arg Gln Val Tyr Lys Asn Leu Gln Glu
225 230 235 240
Ile Asn Ala Tyr Ile Gly His Ser Val Glu Lys His Arg Glu Thr Leu
245 250 255
Asp Pro Ser Ala Pro Lys Asp Leu Ile Asp Thr Tyr Leu Leu His Met
260 265 270
Glu Lys Glu Lys Ser Asn Ala His Ser Glu Phe Ser His Gln Asn Leu
275 280 285
Asn Leu Asn Thr Leu Ser Leu Phe Phe Ala Gly Thr Glu Thr Thr Ser
290 295 300
Thr Thr Leu Arg Tyr Gly Phe Leu Leu Met Leu Lys Tyr Pro His Val
305 310 315 320
Ala Glu Arg Val Tyr Arg Glu Ile Glu Gln Val Ile Gly Pro His Arg
325 330 335
Pro Pro Glu Leu His Asp Arg Ala Lys Met Pro Tyr Thr Glu Ala Val
340 345 350
Ile Tyr Glu Ile Gln Arg Phe Ser Asp Leu Leu Pro Met Gly Val Pro
355 360 365
His Ile Val Thr Gln His Thr Ser Phe Arg Gly Tyr Ile Ile Pro Lys
370 375 380
Asp Thr Glu Val Phe Leu Ile Leu Ser Thr Ala Leu His Asp Pro His
385 390 395 400
Tyr Phe Glu Lys Pro Asp Ala Phe Asn Pro Asp His Phe Leu Asp Ala
405 410 415
Asn Gly Ala Leu Lys Lys Thr Glu Ala Phe Ile Pro Phe Ser Leu Gly
420 425 430
Lys Arg Ile Cys Leu Gly Glu Gly Ile Ala Arg Ala Glu Leu Phe Leu
435 440 445
Phe Phe Thr Thr Ile Leu Gln Asn Phe Ser Met Ala Ser Pro Val Ala
450 455 460
Pro Glu Asp Ile Asp Leu Thr Pro Gln Glu Cys Gly Val Gly Lys Ile
465 470 475 480
Pro Pro Thr Tyr Gln Ile Arg Phe Leu Pro Arg
485 490

<210> 225
<211> 359
<212> PRT
<213> Homo sapiens
<400> 225
Met Val Arg Pro Met Leu Leu Leu Ser Leu Gly Leu Leu Ala Gly Leu
1 5 10 15
Leu Pro Ala Leu Ala Ala Cys Pro Gln Asn Cys His Cys His Ser Asp
20 25 30
Leu Gln His Val Ile Cys Asp Lys Val Gly Leu Gln Lys Ile Pro Lys
35 40 45
Val Ser Glu Lys Thr Lys Leu Leu Asn Leu Gln Arg Asn Asn Phe Pro
50 55 60
Val Leu Ala Ala Asn Ser Phe Arg Ala Met Pro Asn Leu Val Ser Leu
65 70 75 80
His Leu Gln His Cys Gln Ile Arg Glu Val Ala Ala Gly Ala Phe Arg
85 90 95
Gly Leu Lys Gln Leu Ile Tyr Leu Tyr Leu Ser His Asn Asp Ile Arg
100 105 110
Val Val Arg Ala Gly Ala Phe Asp Asp Leu Thr Glu Leu Thr Tyr Leu
115 120 125
Tyr Leu Asp His Asn Lys Val Thr Glu Leu Pro Arg Gly Leu Leu Ser
130 135 140
Pro Leu Val Asn Leu Phe Ile Leu Gln Leu Asn Asn Asn Lys Ile Arg
145 150 155 160
Glu Leu Arg Ala Gly Pro Phe Gln Gly Ala Lys Asp Leu Arg Trp Leu
165 170 175
Tyr Leu Ser Glu Asn Ala Leu Ser Ser Leu Gln Pro Gly Ala Leu Asp
180 185 190
Asp Val Glu Asn Leu Ala Lys Phe His Val Asp Arg Asn Gln Leu Ser
195 200 205
Ser Tyr Pro Ser Ala Ala Leu Ser Lys Leu Arg Val Val Glu Glu Leu

210 215 220
Lys Leu Ser His Asn Pro Leu Lys Ser Ile Pro Asp Asn Ala Phe Gln
225 230 235 240
Ser Phe Gly Arg Tyr Leu Glu Thr Leu Trp Leu Asp Asn Thr Asn Leu
245 250 255
Glu Lys Phe Ser Asp Gly Ala Phe Leu Gly Val Thr Thr Leu Lys His
260 265 270
Val His Leu Glu Asn Asn Arg Leu Asn Gln Leu Pro Ser Asn Phe Pro
275 280 285
Phe Asp Ser Leu Glu Thr Leu Ala Leu Thr Asn Asn Pro Trp Lys Cys
290 295 300
Thr Cys Gln Leu Arg Gly Leu Arg Arg Trp Leu Glu Ala Lys Ala Ser
305 310 315 320
Arg Pro Asp Ala Thr Cys Ala Ser Pro Ala Lys Phe Lys Gly Gln His
325 330 335
Ile Arg Asp Thr Asp Ala Phe Arg Ser Cys Lys Phe Pro Thr Lys Arg
340 345 350
Ser Lys Lys Ala Gly Arg His
355

<210> 226
<211> 276
<212> PRT
<213> Homo sapiens
<400> 226
Met Leu Ala Trp Arg Asp Gly Glu Leu Glu Ala Glu Thr Ser Ser Ser
1 5 10 15
Leu Phe Leu Leu Ala Met Gln Val Trp Met Cys Gly Gly Arg Met Glu
20 25 30
Asp Ile Pro Cys Ser Arg Val Gly His Ile Tyr Arg Lys Tyr Val Pro
35 40 45
Tyr Lys Val Pro Ala Gly Val Ser Leu Ala Arg Val Arg Thr Leu Lys
50 55 60
Arg Val Ala Glu Val Trp Met Asp Glu Tyr Ala Glu Tyr Ile Tyr Gln
65 70 75 80
Arg Arg Pro Glu Tyr Arg His Leu Ser Ala Gly Asp Val Ala Val Gln
85 90 95
Lys Lys Leu Arg Ser Ser Leu Asn Cys Lys Ser Phe Lys Trp Phe Met
100 105 110
Thr Lys Ile Ala Trp Asp Leu Pro Lys Phe Tyr Pro Pro Val Glu Pro
115 120 125
Pro Ala Ala Ala Trp Gly Glu Ile Arg Asn Val Gly Thr Gly Leu Cys
130 135 140
Ala Asp Thr Lys His Gly Ala Leu Gly Ser Pro Leu Arg Leu Glu Gly
145 150 155 160
Cys Val Arg Gly Arg Gly Glu Ala Ala Trp Asn Asn Met Gln Val Phe
165 170 175
Thr Phe Thr Trp Arg Glu Asp Ile Arg Pro Gly Asp Pro Gln His Thr
180 185 190
Lys Lys Phe Cys Phe Asp Ala Ile Ser His Thr Ser Pro Val Thr Leu
195 200 205
Tyr Asp Cys His Ser Met Lys Gly Asn Gln Leu Trp Lys Tyr Arg Lys
210 215 220
Asp Lys Thr Leu Tyr His Pro Val Ser Gly Ser Cys Met Asp Cys Ser
225 230 235 240
Glu Ser Asp His Arg Ile Phe Met Asn Thr Cys Asn Pro Ser Ser Leu
245 250 255
Thr Gln Gln Trp Leu Phe Glu His Thr Asn Ser Thr Val Leu Glu Lys
260 265 270
Phe Asn Arg Asn
275

<210> 227
<211> 161
<212> PRT
<213> Homo sapiens
<400> 227
Met Thr Leu Glu Glu Leu Val Ala Cys Asp Asn Ala Ala Gln Lys Met
1 5 10 15

Gln Thr Val Thr Ala Ala Val Glu Glu Leu Leu Val Ala Ala Gln Arg
20 25 30
Gln Asp Arg Leu Thr Val Gly Val Tyr Glu Ser Ala Lys Leu Met Asn
35 40 45
Val Asp Pro Asp Ser Val Val Leu Cys Leu Leu Ala Ile Asp Glu Glu
50 55 60
Glu Glu Asp Asp Ile Ala Leu Gln Ile His Phe Thr Leu Ile Gln Ser
65 70 75 80
Phe Cys Cys Asp Asn Asp Ile Asn Ile Val Arg Val Ser Gly Asn Ala
85 90 95
Arg Leu Ala Gln Leu Leu Gly Glu Pro Ala Glu Thr Gln Gly Thr Thr
100 105 110
Glu Ala Arg Asp Leu His Cys Leu Pro Phe Leu Gln Asn Pro His Thr
115 120 125
Asp Ala Trp Lys Ser His Gly Leu Val Glu Val Ala Ser Tyr Cys Glu
130 135 140
Glu Ser Arg Gly Asn Asn Gln Trp Val Pro Tyr Ile Ser Leu Gln Glu
145 150 155 160
Arg

<210> 228
<211> 281
<212> PRT
<213> Homo sapiens
<400> 228
Met Ala Ser Arg Gly Arg Arg Pro Glu His Gly Gly Pro Pro Glu Leu
1 5 10 15
Phe Tyr Asp Glu Thr Glu Ala Arg Lys Tyr Val Arg Asn Ser Arg Met
20 25 30
Ile Asp Ile Gln Thr Arg Met Ala Gly Arg Ala Leu Glu Leu Leu Tyr
35 40 45
Leu Pro Glu Asn Lys Pro Cys Tyr Leu Leu Asp Ile Gly Cys Gly Thr
50 55 60
Gly Leu Ser Gly Ser Tyr Leu Ser Asp Glu Gly His Tyr Trp Val Gly
65 70 75 80
Leu Asp Ile Ser Pro Ala Met Leu Asp Glu Ala Val Asp Arg Glu Ile
85 90 95
Glu Gly Asp Leu Leu Leu Gly Asp Met Gly Gln Gly Ile Pro Phe Lys
100 105 110
Pro Gly Thr Phe Asp Gly Cys Ile Ser Ile Ser Ala Val Gln Trp Leu
115 120 125
Cys Asn Ala Asn Lys Lys Ser Glu Asn Pro Ala Lys Arg Leu Tyr Cys
130 135 140
Phe Phe Ala Ser Leu Phe Ser Val Leu Val Arg Gly Ser Arg Ala Val
145 150 155 160
Leu Gln Leu Tyr Pro Glu Asn Ser Glu Gln Leu Glu Leu Ile Thr Thr
165 170 175
Gln Ala Thr Lys Ala Gly Phe Ser Gly Gly Met Val Val Asp Tyr Pro
180 185 190
Asn Ser Ala Lys Ala Lys Lys Phe Tyr Leu Cys Leu Phe Ser Gly Pro
195 200 205
Ser Thr Phe Ile Pro Glu Gly Leu Ser Glu Asn Gln Asp Glu Val Glu
210 215 220
Pro Arg Glu Ser Val Phe Thr Asn Glu Arg Phe Pro Leu Arg Met Ser
225 230 235 240
Arg Arg Gly Met Val Arg Lys Ser Arg Ala Trp Val Leu Glu Lys Lys
245 250 255
Glu Arg His Arg Arg Gln Gly Arg Glu Val Arg Pro Asp Thr Gln Tyr
260 265 270
Thr Gly Arg Lys Arg Lys Pro Arg Phe
275 280

<210> 229
<211> 525
<212> PRT
<213> Homo sapiens
<400> 229
Met Ala Ala Gly Gly Ser Gly Gly Arg Ala Ser Cys Pro Pro Gly Val

```
Gly Val Gly Pro Gly Thr Gly Gly Ser Pro Gly Pro Ser Ala Asn Ala
1               5                   10                  15
Ala Ala Thr Pro Ala Pro Gly Asn Ala Ala Ala Ala Ala Ala Ala Ala
            20                  25                  30
Ala Ala Ala Ala Ala Ala Pro Gly Pro Thr Pro Pro Ala Pro Pro Gly
        35                  40                  45
Pro Gly Thr Asp Ala Gln Ala Ala Gly Ala Glu Arg Ala Glu Glu Ala
    50                  55                  60
Ala Gly Pro Gly Ala Ala Ala Leu Gln Arg Glu Ala Ala Tyr Asn Trp
65                  70                  75                  80
Gln Ala Ser Lys Pro Thr Val Gln Glu Arg Phe Ala Phe Leu Phe Asn
                85                  90                  95
Asn Glu Val Leu Cys Asp Val His Phe Leu Val Gly Lys Gly Leu Ser
            100                 105                 110
Ser Gln Arg Ile Pro Ala His Arg Phe Val Leu Ala Val Gly Ser Ala
        115                 120                 125
Val Phe Asp Ala Met Phe Asn Gly Gly Met Ala Thr Thr Ser Thr Glu
    130                 135                 140
Ile Glu Leu Pro Asp Val Glu Pro Ala Ala Phe Leu Ala Leu Leu Lys
145                 150                 155                 160
Phe Leu Tyr Ser Asp Glu Val Gln Ile Gly Pro Glu Thr Val Met Thr
                165                 170                 175
Thr Leu Tyr Thr Ala Lys Lys Tyr Ala Val Pro Ala Leu Glu Ala His
            180                 185                 190
Cys Val Glu Phe Leu Lys Lys Asn Leu Arg Ala Asp Asn Ala Phe Met
        195                 200                 205
Leu Leu Thr Gln Ala Arg Leu Phe Asp Glu Pro Gln Leu Ala Ser Leu
    210                 215                 220
Cys Leu Glu Asn Ile Asp Lys Asn Thr Ala Asp Ala Ile Thr Ala Glu
225                 230                 235                 240
Gly Phe Thr Asp Ile Asp Leu Asp Thr Leu Val Ala Val Leu Glu Arg
                245                 250                 255
Asp Thr Leu Gly Ile Arg Glu Val Arg Leu Phe Asn Ala Val Val Arg
            260                 265                 270
Trp Ser Glu Ala Glu Cys Gln Arg Gln Gln Leu Gln Val Thr Pro Glu
        275                 280                 285
Asn Arg Arg Lys Val Leu Gly Lys Ala Leu Gly Leu Ile Arg Phe Pro
    290                 295                 300
Leu Met Thr Ile Glu Glu Phe Ala Ala Gly Pro Ala Gln Ser Gly Ile
305                 310                 315                 320
Leu Val Asp Arg Glu Val Val Ser Leu Phe Leu His Phe Thr Val Asn
                325                 330                 335
Pro Lys Pro Arg Val Glu Phe Ile Asp Arg Pro Arg Cys Cys Leu Arg
            340                 345                 350
Gly Lys Glu Cys Ser Ile Asn Arg Phe Gln Gln Val Glu Ser Arg Trp
        355                 360                 365
Gly Tyr Ser Gly Thr Ser Asp Arg Ile Arg Phe Ser Val Asn Lys Arg
    370                 375                 380
Ile Phe Val Val Gly Phe Gly Leu Tyr Gly Ser Ile His Gly Pro Thr
385                 390                 395                 400
Asp Tyr Gln Val Asn Ile Gln Ile Ile His Thr Asp Ser Asn Thr Val
                405                 410                 415
Leu Gly Gln Asn Asp Thr Gly Phe Ser Cys Asp Gly Ser Ala Ser Thr
            420                 425                 430
Phe Arg Val Met Phe Lys Glu Pro Val Glu Val Leu Pro Asn Val Asn
        435                 440                 445
Tyr Thr Ala Cys Ala Thr Leu Lys Gly Pro Asp Ser His Tyr Gly Thr
    450                 455                 460
Lys Gly Leu Arg Lys Val Thr His Glu Ser Pro Thr Thr Gly Ala Lys
465                 470                 475                 480
Thr Cys Phe Thr Phe Cys Tyr Ala Ala Gly Asn Asn Asn Gly Thr Ser
                485                 490                 495
Val Glu Asp Gly Gln Ile Pro Glu Val Ile Phe Tyr Thr
            500                 505                 510
        515                 520                 525
```

<210> 230
<211> 933
<212> PRT

<213> Homo sapiens
<400> 230
Met Thr Glu Leu Lys Ala Lys Gly Pro Arg Ala Pro His Val Ala Gly
1 5 10 15
Gly Pro Pro Ser Pro Glu Val Gly Ser Pro Leu Leu Cys Arg Pro Ala
20 25 30
Ala Gly Pro Phe Pro Gly Ser Gln Thr Ser Asp Thr Leu Pro Glu Val
35 40 45
Ser Ala Ile Pro Ile Ser Leu Asp Gly Leu Leu Phe Pro Arg Pro Cys
50 55 60
Gln Gly Gln Asp Pro Ser Asp Glu Lys Thr Gln Asp Gln Gln Ser Leu
65 70 75 80
Ser Asp Val Glu Gly Ala Tyr Ser Arg Ala Glu Ala Thr Arg Gly Ala
85 90 95
Gly Gly Ser Ser Ser Ser Pro Pro Glu Lys Asp Ser Gly Leu Leu Asp
100 105 110
Ser Val Leu Asp Thr Leu Leu Ala Pro Ser Gly Pro Gly Gln Ser Gln
115 120 125
Pro Ser Pro Pro Ala Cys Glu Val Thr Ser Ser Trp Cys Leu Phe Gly
130 135 140
Pro Glu Leu Pro Glu Asp Pro Pro Ala Ala Pro Ala Thr Gln Arg Val
145 150 155 160
Leu Ser Pro Leu Met Ser Arg Ser Gly Cys Lys Val Gly Asp Ser Ser
165 170 175
Gly Thr Ala Ala Ala His Lys Val Leu Pro Arg Gly Leu Ser Pro Ala
180 185 190
Arg Gln Leu Leu Leu Pro Ala Ser Glu Ser Pro His Trp Ser Gly Ala
195 200 205
Pro Val Lys Pro Ser Pro Gln Ala Ala Ala Val Glu Val Glu Glu Glu
210 215 220
Asp Ser Ser Glu Ser Glu Glu Ser Ala Gly Pro Leu Leu Lys Gly Lys
225 230 235 240
Pro Arg Ala Leu Gly Gly Ala Ala Ala Gly Gly Gly Ala Ala Ala Cys
245 250 255
Pro Pro Gly Ala Ala Ala Gly Gly Val Ala Leu Val Pro Lys Glu Asp
260 265 270
Ser Arg Phe Ser Ala Pro Arg Val Ala Leu Val Glu Gln Asp Ala Pro
275 280 285
Met Ala Pro Gly Arg Ser Pro Leu Ala Thr Thr Val Met Asp Phe Ile
290 295 300
His Val Pro Ile Leu Pro Leu Asn His Ala Leu Leu Ala Ala Arg Thr
305 310 315 320
Arg Gln Leu Leu Glu Asp Glu Ser Tyr Asp Gly Gly Ala Gly Ala Ala
325 330 335
Ser Ala Phe Ala Pro Pro Arg Thr Ser Pro Cys Ala Ser Ser Thr Pro
340 345 350
Val Ala Val Gly Asp Phe Pro Asp Cys Ala Tyr Pro Pro Asp Ala Glu
355 360 365
Pro Lys Asp Asp Ala Tyr Pro Leu Tyr Ser Asp Phe Gln Pro Pro Ala
370 375 380
Leu Lys Ile Lys Glu Glu Glu Glu Gly Ala Glu Ala Ser Ala Arg Ser
385 390 395 400
Pro Arg Ser Tyr Leu Val Ala Gly Ala Asn Pro Ala Ala Phe Pro Asp
405 410 415
Phe Pro Leu Gly Pro Pro Pro Pro Leu Pro Pro Arg Ala Thr Pro Ser
420 425 430
Arg Pro Gly Glu Ala Ala Val Thr Ala Ala Pro Ala Ser Ala Ser Val
435 440 445
Ser Ser Ala Ser Ser Ser Gly Ser Thr Leu Glu Cys Ile Leu Tyr Lys
450 455 460
Ala Glu Gly Ala Pro Pro Gln Gln Gly Pro Phe Ala Pro Pro Pro Cys
465 470 475 480
Lys Ala Pro Gly Ala Ser Gly Cys Leu Leu Pro Arg Asp Gly Leu Pro
485 490 495
Ser Thr Ser Ala Ser Ala Ala Ala Ala Gly Ala Ala Pro Ala Leu Tyr
500 505 510
Pro Ala Leu Gly Leu Asn Gly Leu Pro Gln Leu Gly Tyr Gln Ala Ala
515 520 525
Val Leu Lys Glu Gly Leu Pro Gln Val Tyr Pro Pro Tyr Leu Asn Tyr

530 535 540
Leu Arg Pro Asp Ser Glu Ala Ser Gln Ser Pro Gln Tyr Ser Phe Glu
545 550 555 560
Ser Leu Pro Gln Lys Ile Cys Leu Ile Cys Gly Asp Glu Ala Ser Gly
565 570 575
Cys His Tyr Gly Val Leu Thr Cys Gly Ser Cys Lys Val Phe Phe Lys
580 585 590
Arg Ala Met Glu Gly Gln His Asn Tyr Leu Cys Ala Gly Arg Asn Asp
595 600 605
Cys Ile Val Asp Lys Ile Arg Arg Lys Asn Cys Pro Ala Cys Arg Leu
610 615 620
Arg Lys Cys Cys Gln Ala Gly Met Val Leu Gly Gly Arg Lys Phe Lys
625 630 635 640
Lys Phe Asn Lys Val Arg Val Val Arg Ala Leu Asp Ala Val Ala Leu
645 650 655
Pro Gln Pro Leu Gly Val Pro Asn Glu Ser Gln Ala Leu Ser Gln Arg
660 665 670
Phe Thr Phe Ser Pro Gly Gln Asp Ile Gln Leu Ile Pro Pro Leu Ile
675 680 685
Asn Leu Leu Met Ser Ile Glu Pro Asp Val Ile Tyr Ala Gly His Asp
690 695 700
Asn Thr Lys Pro Asp Thr Ser Ser Ser Leu Leu Thr Ser Leu Asn Gln
705 710 715 720
Leu Gly Glu Arg Gln Leu Leu Ser Val Val Lys Trp Ser Lys Ser Leu
725 730 735
Pro Gly Phe Arg Asn Leu His Ile Asp Asp Gln Ile Thr Leu Ile Gln
740 745 750
Tyr Ser Trp Met Ser Leu Met Val Phe Gly Leu Gly Trp Arg Ser Tyr
755 760 765
Lys His Val Ser Gly Gln Met Leu Tyr Phe Ala Pro Asp Leu Ile Leu
770 775 780
Asn Glu Gln Arg Met Lys Glu Ser Ser Phe Tyr Ser Leu Cys Leu Thr
785 790 795 800
Met Trp Gln Ile Pro Gln Glu Phe Val Lys Leu Gln Val Ser Gln Glu
805 810 815
Glu Phe Leu Cys Met Lys Val Leu Leu Leu Leu Asn Thr Ile Pro Leu
820 825 830
Glu Gly Leu Arg Ser Gln Thr Gln Phe Glu Glu Met Arg Ser Ser Tyr
835 840 845
Ile Arg Glu Leu Ile Lys Ala Ile Gly Leu Arg Gln Lys Gly Val Val
850 855 860
Ser Ser Ser Gln Arg Phe Tyr Gln Leu Thr Lys Leu Leu Asp Asn Leu
865 870 875 880
His Asp Leu Val Lys Gln Leu His Leu Tyr Cys Leu Asn Thr Phe Ile
885 890 895
Gln Ser Arg Ala Leu Ser Val Glu Phe Pro Glu Met Met Ser Glu Val
900 905 910
Ile Ala Ala Gln Leu Pro Lys Ile Leu Ala Gly Met Val Lys Pro Leu
915 920 925
Leu Phe His Lys Lys
930

<210> 231
<211> 186
<212> PRT
<213> Homo sapiens
<400> 231
Met Asp Ala Asp Ser Asp Val Ala Leu Asp Ile Leu Ile Thr Asn Val
1 5 10 15
Val Cys Val Phe Arg Thr Arg Cys His Leu Asn Leu Arg Lys Ile Ala
20 25 30
Leu Glu Gly Ala Asn Val Ile Tyr Lys Arg Asp Val Gly Lys Val Leu
35 40 45
Met Lys Leu Arg Lys Pro Arg Ile Thr Ala Thr Ile Trp Ser Ser Gly
50 55 60
Lys Ile Ile Cys Thr Gly Ala Thr Ser Glu Glu Glu Ala Lys Phe Gly
65 70 75 80
Ala Arg Arg Leu Ala Arg Ser Leu Gln Lys Leu Gly Phe Gln Val Ile
85 90 95

Phe Thr Asp Phe Lys Val Val Asn Val Leu Ala Val Cys Asn Met Pro
100 105 110
Phe Glu Ile Arg Leu Pro Glu Phe Thr Lys Asn Asn Arg Pro His Ala
115 120 125
Ser Tyr Glu Pro Glu Leu His Pro Ala Val Cys Tyr Arg Ile Lys Ser
130 135 140
Leu Arg Ala Thr Leu Gln Ile Phe Ser Thr Gly Ser Ile Thr Val Thr
145 150 155 160
Gly Pro Asn Val Lys Ala Val Ala Thr Ala Val Glu Gln Ile Tyr Pro
165 170 175
Phe Val Phe Glu Ser Arg Lys Glu Ile Leu
180 185

<210> 232
<211> 1744
<212> PRT
<213> Homo sapiens
<400> 232
Met Arg Leu Leu Trp Gly Leu Ile Trp Ala Ser Ser Phe Phe Thr Leu
1 5 10 15
Ser Leu Gln Lys Pro Arg Leu Leu Leu Phe Ser Pro Ser Val Val His
20 25 30
Leu Gly Val Pro Leu Ser Val Gly Val Gln Leu Gln Asp Val Pro Arg
35 40 45
Gly Gln Val Val Lys Gly Ser Val Phe Leu Arg Asn Pro Ser Arg Asn
50 55 60
Asn Val Pro Cys Ser Pro Lys Val Asp Phe Thr Leu Ser Ser Glu Arg
65 70 75 80
Asp Phe Ala Leu Leu Ser Leu Gln Val Pro Leu Lys Asp Ala Lys Ser
85 90 95
Cys Gly Leu His Gln Leu Leu Arg Gly Pro Glu Val Gln Leu Val Ala
100 105 110
His Ser Pro Trp Leu Lys Asp Ser Leu Ser Arg Thr Thr Asn Ile Gln
115 120 125
Gly Ile Asn Leu Leu Phe Ser Ser Arg Arg Gly His Leu Phe Leu Gln
130 135 140
Thr Asp Gln Pro Ile Tyr Asn Pro Gly Gln Arg Val Arg Tyr Arg Val
145 150 155 160
Phe Ala Leu Asp Gln Lys Met Arg Pro Ser Thr Asp Thr Ile Thr Val
165 170 175
Met Val Glu Asn Ser His Gly Leu Arg Val Arg Lys Lys Glu Val Tyr
180 185 190
Met Pro Ser Ser Ile Phe Gln Asp Asp Phe Val Ile Pro Asp Ile Ser
195 200 205
Glu Pro Gly Thr Trp Lys Ile Ser Ala Arg Phe Ser Asp Gly Leu Glu
210 215 220
Ser Asn Ser Ser Thr Gln Phe Glu Val Lys Lys Tyr Val Leu Pro Asn
225 230 235 240
Phe Glu Val Lys Ile Thr Pro Gly Lys Pro Tyr Ile Leu Thr Val Pro
245 250 255
Gly His Leu Asp Glu Met Gln Leu Asp Ile Gln Ala Arg Tyr Ile Tyr
260 265 270
Gly Lys Pro Val Gln Gly Val Ala Tyr Val Arg Phe Gly Leu Leu Asp
275 280 285
Glu Asp Gly Lys Lys Thr Phe Phe Arg Gly Leu Glu Ser Gln Thr Lys
290 295 300
Leu Val Asn Gly Gln Ser His Ile Ser Leu Ser Lys Ala Glu Phe Gln
305 310 315 320
Asp Ala Leu Glu Lys Leu Asn Met Gly Ile Thr Asp Leu Gln Gly Leu
325 330 335
Arg Leu Tyr Val Ala Ala Ala Ile Ile Glu Tyr Pro Gly Gly Glu Met
340 345 350
Glu Glu Ala Glu Leu Thr Ser Trp Tyr Phe Val Ser Ser Pro Phe Ser
355 360 365
Leu Asp Leu Ser Lys Thr Lys Arg His Leu Val Pro Gly Ala Pro Phe
370 375 380
Leu Leu Gln Ala Leu Val Arg Glu Met Ser Gly Ser Pro Ala Ser Gly
385 390 395 400
Ile Pro Val Lys Val Ser Ala Thr Val Ser Ser Pro Gly Ser Val Pro

```
                        405                     410                     415
Glu Val Gln Asp Ile Gln Gln Asn Thr Asp Gly Ser Gly Gln Val Ser
                420                     425                     430
Ile Pro Ile Ile Ile Pro Gln Thr Ile Ser Glu Leu Gln Leu Ser Val
            435                     440                     445
Ser Ala Gly Ser Pro His Pro Ala Ile Ala Arg Leu Thr Val Ala Ala
    450                     455                     460
Pro Pro Ser Gly Gly Pro Gly Phe Leu Ser Ile Glu Arg Pro Asp Ser
465                     470                     475                     480
Arg Pro Pro Arg Val Gly Asp Thr Leu Asn Leu Asn Leu Arg Ala Val
                485                     490                     495
Gly Ser Gly Ala Thr Phe Ser His Tyr Tyr Tyr Met Ile Leu Ser Arg
            500                     505                     510
Gly Gln Ile Val Phe Met Asn Arg Glu Pro Lys Arg Thr Leu Thr Ser
        515                     520                     525
Val Ser Val Phe Val Asp His His Leu Ala Pro Ser Phe Tyr Phe Val
    530                     535                     540
Ala Phe Tyr Tyr His Gly Asp His Pro Val Ala Asn Ser Leu Arg Val
545                     550                     555                     560
Asp Val Gln Ala Gly Ala Cys Glu Gly Lys Leu Glu Leu Ser Val Asp
                565                     570                     575
Gly Ala Lys Gln Tyr Arg Asn Gly Glu Ser Val Lys Leu His Leu Glu
            580                     585                     590
Thr Asp Ser Leu Ala Leu Val Ala Leu Gly Ala Leu Asp Thr Ala Leu
        595                     600                     605
Tyr Ala Ala Gly Ser Lys Ser His Lys Pro Leu Asn Met Gly Lys Val
    610                     615                     620
Phe Glu Ala Met Asn Ser Tyr Asp Leu Gly Cys Gly Pro Gly Gly Gly
625                     630                     635                     640
Asp Ser Ala Leu Gln Val Phe Gln Ala Ala Gly Leu Ala Phe Ser Asp
                645                     650                     655
Gly Asp Gln Trp Thr Leu Ser Arg Lys Arg Leu Ser Cys Pro Lys Glu
            660                     665                     670
Lys Thr Thr Arg Lys Lys Arg Asn Val Asn Phe Gln Lys Ala Ile Asn
        675                     680                     685
Glu Lys Leu Gly Gln Tyr Ala Ser Pro Thr Ala Lys Arg Cys Cys Gln
    690                     695                     700
Asp Gly Val Thr Arg Leu Pro Met Met Arg Ser Cys Glu Gln Arg Ala
705                     710                     715                     720
Ala Arg Val Gln Gln Pro Asp Cys Arg Glu Pro Phe Leu Ser Cys Cys
                725                     730                     735
Gln Phe Ala Glu Ser Leu Arg Lys Lys Ser Arg Asp Lys Gly Gln Ala
            740                     745                     750
Gly Leu Gln Arg Ala Leu Glu Ile Leu Gln Glu Glu Asp Leu Ile Asp
        755                     760                     765
Glu Asp Asp Ile Pro Val Arg Ser Phe Phe Pro Glu Asn Trp Leu Trp
    770                     775                     780
Arg Val Glu Thr Val Asp Arg Phe Gln Ile Leu Thr Leu Trp Leu Pro
785                     790                     795                     800
Asp Ser Leu Thr Thr Trp Glu Ile His Gly Leu Ser Leu Ser Lys Thr
                805                     810                     815
Lys Gly Leu Cys Val Ala Thr Pro Val Gln Leu Arg Val Phe Arg Glu
            820                     825                     830
Phe His Leu His Leu Arg Leu Pro Met Ser Val Arg Arg Phe Glu Gln
        835                     840                     845
Leu Glu Leu Arg Pro Val Leu Tyr Asn Tyr Leu Asp Lys Asn Leu Thr
    850                     855                     860
Val Ser Val His Val Ser Pro Val Glu Gly Leu Cys Leu Ala Gly Gly
865                     870                     875                     880
Gly Gly Leu Ala Gln Gln Val Leu Val Pro Ala Gly Ser Ala Arg Pro
                885                     890                     895
Val Ala Phe Ser Val Val Pro Thr Ala Ala Ala Ala Val Ser Leu Lys
            900                     905                     910
Val Val Ala Arg Gly Ser Phe Glu Phe Pro Val Gly Asp Ala Val Ser
        915                     920                     925
Lys Val Leu Gln Ile Glu Lys Glu Gly Ala Ile His Arg Glu Glu Leu
    930                     935                     940
Val Tyr Glu Leu Asn Pro Leu Asp His Arg Gly Arg Thr Leu Glu Ile
945                     950                     955                     960
```

Pro Gly Asn Ser Asp Pro Asn Met Ile Pro Asp Gly Asp Phe Asn Ser
965 970 975
Tyr Val Arg Val Thr Ala Ser Asp Pro Leu Asp Thr Leu Gly Ser Glu
980 985 990
Gly Ala Leu Ser Pro Gly Gly Val Ala Ser Leu Leu Arg Leu Pro Arg
995 1000 1005
Gly Cys Gly Glu Gln Thr Met Ile Tyr Leu Ala Pro Thr Leu Ala
1010 1015 1020
Ala Ser Arg Tyr Leu Asp Lys Thr Glu Gln Trp Ser Thr Leu Pro
1025 1030 1035
Pro Glu Thr Lys Asp His Ala Val Asp Leu Ile Gln Lys Gly Tyr
1040 1045 1050
Met Arg Ile Gln Gln Phe Arg Lys Ala Asp Gly Ser Tyr Ala Ala
1055 1060 1065
Trp Leu Ser Arg Gly Ser Ser Thr Trp Leu Thr Ala Phe Val Leu
1070 1075 1080
Lys Val Leu Ser Leu Ala Gln Glu Gln Val Gly Gly Ser Pro Glu
1085 1090 1095
Lys Leu Gln Glu Thr Ser Asn Trp Leu Leu Ser Gln Gln Gln Ala
1100 1105 1110
Asp Gly Ser Phe Gln Asp Leu Ser Pro Val Ile His Arg Ser Met
1115 1120 1125
Gln Gly Gly Leu Val Gly Asn Asp Glu Thr Val Ala Leu Thr Ala
1130 1135 1140
Phe Val Thr Ile Ala Leu His His Gly Leu Ala Val Phe Gln Asp
1145 1150 1155
Glu Gly Ala Glu Pro Leu Lys Gln Arg Val Glu Ala Ser Ile Ser
1160 1165 1170
Lys Ala Ser Ser Phe Leu Gly Glu Lys Ala Ser Ala Gly Leu Leu
1175 1180 1185
Gly Ala His Ala Ala Ala Ile Thr Ala Tyr Ala Leu Thr Leu Thr
1190 1195 1200
Lys Ala Pro Ala Asp Leu Arg Gly Val Ala His Asn Asn Leu Met
1205 1210 1215
Ala Met Ala Gln Glu Thr Gly Asp Asn Leu Tyr Trp Gly Ser Val
1220 1225 1230
Thr Gly Ser Gln Ser Asn Ala Val Ser Pro Thr Pro Ala Pro Arg
1235 1240 1245
Asn Pro Ser Asp Pro Met Pro Gln Ala Pro Ala Leu Trp Ile Glu
1250 1255 1260
Thr Thr Ala Tyr Ala Leu Leu His Leu Leu Leu His Glu Gly Lys
1265 1270 1275
Ala Glu Met Ala Asp Gln Ala Ala Ala Trp Leu Thr Arg Gln Gly
1280 1285 1290
Ser Phe Gln Gly Gly Phe Arg Ser Thr Gln Asp Thr Val Ile Ala
1295 1300 1305
Leu Asp Ala Leu Ser Ala Tyr Trp Ile Ala Ser His Thr Thr Glu
1310 1315 1320
Glu Arg Gly Leu Asn Val Thr Leu Ser Ser Thr Gly Arg Asn Gly
1325 1330 1335
Phe Lys Ser His Ala Leu Gln Leu Asn Asn Arg Gln Ile Arg Gly
1340 1345 1350
Leu Glu Glu Glu Leu Gln Phe Ser Leu Gly Ser Lys Ile Asn Val
1355 1360 1365
Lys Val Gly Gly Asn Ser Lys Gly Thr Leu Lys Val Leu Arg Thr
1370 1375 1380
Tyr Asn Val Leu Asp Met Lys Asn Thr Thr Cys Gln Asp Leu Gln
1385 1390 1395
Ile Glu Val Thr Val Lys Gly His Val Glu Tyr Thr Met Glu Ala
1400 1405 1410
Asn Glu Asp Tyr Glu Asp Tyr Glu Tyr Asp Glu Leu Pro Ala Lys
1415 1420 1425
Asp Asp Pro Asp Ala Pro Leu Gln Pro Val Thr Pro Leu Gln Leu
1430 1435 1440
Phe Glu Gly Arg Arg Asn Arg Arg Arg Arg Glu Ala Pro Lys Val
1445 1450 1455
Val Glu Glu Gln Glu Ser Arg Val His Tyr Thr Val Cys Ile Trp
1460 1465 1470
Arg Asn Gly Lys Val Gly Leu Ser Gly Met Ala Ile Ala Asp Val

1475 1480 1485
Thr Leu Leu Ser Gly Phe His Ala Leu Arg Ala Asp Leu Glu Lys
1490 1495 1500
Leu Thr Ser Leu Ser Asp Arg Tyr Val Ser His Phe Glu Thr Glu
1505 1510 1515
Gly Pro His Val Leu Leu Tyr Phe Asp Ser Val Pro Thr Ser Arg
1520 1525 1530
Glu Cys Val Gly Phe Glu Ala Val Gln Glu Val Pro Val Gly Leu
1535 1540 1545
Val Gln Pro Ala Ser Ala Thr Leu Tyr Asp Tyr Tyr Asn Pro Glu
1550 1555 1560
Arg Arg Cys Ser Val Phe Tyr Gly Ala Pro Ser Lys Ser Arg Leu
1565 1570 1575
Leu Ala Thr Leu Cys Ser Ala Glu Val Cys Gln Cys Ala Glu Gly
1580 1585 1590
Lys Cys Pro Arg Gln Arg Arg Ala Leu Glu Arg Gly Leu Gln Asp
1595 1600 1605
Glu Asp Gly Tyr Arg Met Lys Phe Ala Cys Tyr Tyr Pro Arg Val
1610 1615 1620
Glu Tyr Gly Phe Gln Val Lys Val Leu Arg Glu Asp Ser Arg Ala
1625 1630 1635
Ala Phe Arg Leu Phe Glu Thr Lys Ile Thr Gln Val Leu His Phe
1640 1645 1650
Thr Lys Asp Val Lys Ala Ala Ala Asn Gln Met Arg Asn Phe Leu
1655 1660 1665
Val Arg Ala Ser Cys Arg Leu Arg Leu Glu Pro Gly Lys Glu Tyr
1670 1675 1680
Leu Ile Met Gly Leu Asp Gly Ala Thr Tyr Asp Leu Glu Gly His
1685 1690 1695
Pro Gln Tyr Leu Leu Asp Ser Asn Ser Trp Ile Glu Glu Met Pro
1700 1705 1710
Ser Glu Arg Leu Cys Arg Ser Thr Arg Gln Arg Ala Ala Cys Ala
1715 1720 1725
Gln Leu Asn Asp Phe Leu Gln Glu Tyr Gly Thr Gln Gly Cys Gln
1730 1735 1740
Val

<210> 233
<211> 295
<212> PRT
<213> Homo sapiens
<400> 233
Met Ile Glu Val Leu Thr Thr Thr Asp Ser Gln Lys Leu Leu His Gln
1 5 10 15
Leu Asn Ala Leu Leu Glu Gln Glu Ser Arg Cys Gln Pro Lys Val Cys
20 25 30
Gly Leu Arg Leu Ile Glu Ser Ala His Asp Asn Gly Leu Arg Met Thr
35 40 45
Ala Arg Leu Arg Asp Phe Glu Val Lys Asp Leu Leu Ser Leu Thr Gln
50 55 60
Phe Phe Gly Phe Asp Thr Glu Thr Phe Ser Leu Ala Val Asn Leu Leu
65 70 75 80
Asp Arg Phe Leu Ser Lys Met Lys Val Gln Pro Lys His Leu Gly Cys
85 90 95
Val Gly Leu Ser Cys Phe Tyr Leu Ala Val Lys Ser Ile Glu Glu Glu
100 105 110
Arg Asn Val Pro Leu Ala Thr Asp Leu Ile Arg Ile Ser Gln Tyr Arg
115 120 125
Phe Thr Val Ser Asp Leu Met Arg Met Glu Lys Ile Val Leu Glu Lys
130 135 140
Val Cys Trp Lys Val Lys Ala Thr Thr Ala Phe Gln Phe Leu Gln Leu
145 150 155 160
Tyr Tyr Ser Leu Leu Gln Glu Asn Leu Pro Leu Glu Arg Arg Asn Ser
165 170 175
Ile Asn Phe Glu Arg Leu Glu Ala Gln Leu Lys Ala Cys His Cys Arg
180 185 190
Ile Ile Phe Ser Lys Ala Lys Pro Ser Val Leu Ala Leu Ser Ile Ile
195 200 205

Ala Leu Glu Ile Gln Ala Gln Lys Cys Val Glu Leu Thr Glu Gly Ile
    210                 215                 220
Glu Cys Leu Gln Lys His Ser Lys Ile Asn Gly Arg Asp Leu Thr Phe
225                 230                 235                 240
Trp Gln Glu Leu Val Ser Lys Cys Leu Thr Glu Tyr Ser Ser Asn Lys
                245                 250                 255
Cys Ser Lys Pro Asn Val Gln Lys Leu Lys Trp Ile Val Ser Gly Arg
            260                 265                 270
Thr Ala Arg Gln Leu Lys His Ser Tyr Tyr Arg Ile Thr His Leu Pro
        275                 280                 285
Thr Ile Pro Glu Met Val Pro
    290                 295

<210> 234
<211> 359
<212> PRT
<213> Homo sapiens
<400> 234
Met Ala Ala Val Ser Gly Leu Val Arg Arg Pro Leu Arg Glu Val Ser
1               5                   10                  15
Gly Leu Leu Lys Arg Arg Phe His Trp Thr Ala Pro Ala Ala Leu Gln
            20                  25                  30
Val Thr Val Arg Asp Ala Ile Asn Gln Gly Met Asp Glu Glu Leu Glu
        35                  40                  45
Arg Asp Glu Lys Val Phe Leu Leu Gly Glu Glu Val Ala Gln Tyr Asp
    50                  55                  60
Gly Ala Tyr Lys Val Ser Arg Gly Leu Trp Lys Lys Tyr Gly Asp Lys
65                  70                  75                  80
Arg Ile Ile Asp Thr Pro Ile Ser Glu Met Gly Phe Ala Gly Ile Ala
                85                  90                  95
Val Gly Ala Ala Met Ala Gly Leu Arg Pro Ile Cys Glu Phe Met Thr
            100                 105                 110
Phe Asn Phe Ser Met Gln Ala Ile Asp Gln Val Ile Asn Ser Ala Ala
        115                 120                 125
Lys Thr Tyr Tyr Met Ser Gly Gly Leu Gln Pro Val Pro Ile Val Phe
    130                 135                 140
Arg Gly Pro Asn Gly Ala Ser Ala Gly Val Ala Ala Gln His Ser Gln
145                 150                 155                 160
Cys Phe Ala Ala Trp Tyr Gly His Cys Pro Gly Leu Lys Val Val Ser
                165                 170                 175
Pro Trp Asn Ser Glu Asp Ala Lys Gly Leu Ile Lys Ser Ala Ile Arg
            180                 185                 190
Asp Asn Asn Pro Val Val Val Leu Glu Asn Glu Leu Met Tyr Gly Val
        195                 200                 205
Pro Phe Glu Phe Leu Pro Glu Ala Gln Ser Lys Asp Phe Leu Ile Pro
    210                 215                 220
Ile Gly Lys Ala Lys Ile Glu Arg Gln Gly Thr His Ile Thr Val Val
225                 230                 235                 240
Ser His Ser Arg Pro Val Gly His Cys Leu Glu Ala Ala Ala Val Leu
                245                 250                 255
Ser Lys Glu Gly Val Glu Cys Glu Val Ile Asn Met Arg Thr Ile Arg
            260                 265                 270
Pro Met Asp Met Glu Thr Ile Glu Ala Ser Val Met Lys Thr Asn His
        275                 280                 285
Leu Val Thr Val Glu Gly Gly Trp Pro Gln Phe Gly Val Gly Ala Glu
    290                 295                 300
Ile Cys Ala Arg Ile Met Glu Gly Pro Ala Phe Asn Phe Leu Asp Ala
305                 310                 315                 320
Pro Ala Val Arg Val Thr Gly Ala Asp Val Pro Met Pro Tyr Ala Lys
                325                 330                 335
Ile Leu Glu Asp Asn Ser Ile Pro Gln Val Lys Asp Ile Ile Phe Ala
            340                 345                 350
Ile Lys Lys Thr Leu Asn Ile
        355

<210> 235
<211> 420
<212> PRT
<213> Homo sapiens

<400> 235
Met Glu Val Pro Pro Arg Leu Ser His Val Pro Pro Pro Leu Phe Pro
1               5                   10                  15
Ser Ala Pro Ala Thr Leu Ala Ser Arg Ser Leu Ser His Trp Arg Pro
            20                  25                  30
Arg Pro Pro Arg Gln Leu Ala Pro Leu Leu Pro Ser Leu Ala Pro Ser
        35                  40                  45
Ser Ala Arg Gln Gly Ala Arg Arg Ala Gln Arg His Val Thr Ala Gln
    50                  55                  60
Gln Pro Ser Arg Leu Ala Gly Gly Ala Ala Ile Lys Gly Gly Arg Arg
65                  70                  75                  80
Arg Arg Pro Asp Leu Phe Arg Arg His Phe Lys Ser Ser Ser Ile Gln
                85                  90                  95
Arg Ser Ala Ala Ala Ala Ala Ala Thr Arg Thr Ala Arg Gln His Pro
            100                 105                 110
Pro Ala Asp Ser Ser Val Thr Met Glu Asp Met Asn Glu Tyr Ser Asn
        115                 120                 125
Ile Glu Glu Phe Ala Glu Gly Ser Lys Ile Asn Ala Ser Lys Asn Gln
    130                 135                 140
Gln Asp Asp Gly Lys Met Phe Ile Gly Gly Leu Ser Trp Asp Thr Ser
145                 150                 155                 160
Lys Lys Asp Leu Thr Glu Tyr Leu Ser Arg Phe Gly Glu Val Val Asp
                165                 170                 175
Cys Thr Ile Lys Thr Asp Pro Val Thr Gly Arg Ser Arg Gly Phe Gly
            180                 185                 190
Phe Val Leu Phe Lys Asp Ala Ala Ser Val Asp Lys Val Leu Glu Leu
        195                 200                 205
Lys Glu His Lys Leu Asp Gly Lys Leu Ile Asp Pro Lys Arg Ala Lys
    210                 215                 220
Ala Leu Lys Gly Lys Glu Pro Pro Lys Lys Val Phe Val Gly Gly Leu
225                 230                 235                 240
Ser Pro Asp Thr Ser Glu Glu Gln Ile Lys Glu Tyr Phe Gly Ala Phe
                245                 250                 255
Gly Glu Ile Glu Asn Ile Glu Leu Pro Met Asp Thr Lys Thr Asn Glu
            260                 265                 270
Arg Arg Gly Phe Cys Phe Ile Thr Tyr Thr Asp Glu Glu Pro Val Lys
        275                 280                 285
Lys Leu Leu Glu Ser Arg Tyr His Gln Ile Gly Ser Gly Lys Cys Glu
    290                 295                 300
Ile Lys Val Ala Gln Pro Lys Glu Val Tyr Arg Gln Gln Gln Gln Gln
305                 310                 315                 320
Gln Lys Gly Gly Arg Gly Ala Ala Ala Gly Gly Arg Gly Gly Thr Arg
                325                 330                 335
Gly Arg Gly Arg Gly Gln Gly Gln Asn Trp Asn Gln Gly Phe Asn Asn
            340                 345                 350
Tyr Tyr Asp Gln Gly Tyr Gly Asn Tyr Asn Ser Ala Tyr Gly Gly Asp
        355                 360                 365
Gln Asn Tyr Ser Gly Tyr Gly Gly Tyr Asp Tyr Thr Gly Tyr Asn Tyr
    370                 375                 380
Gly Asn Tyr Gly Tyr Gly Gln Gly Tyr Ala Asp Tyr Ser Gly Gln Gln
385                 390                 395                 400
Ser Thr Tyr Gly Lys Ala Ser Arg Gly Gly Gly Asn His Gln Asn Asn
                405                 410                 415
Tyr Gln Pro Tyr
            420

<210> 236
<211> 211
<212> PRT
<213> Homo sapiens
<400> 236
Met Asp Asp Ala His Glu Ser Pro Ser Asp Lys Gly Gly Glu Thr Gly
1               5                   10                  15
Glu Ser Asp Glu Thr Ala Ala Val Pro Gly Asp Pro Gly Ala Thr Asp
            20                  25                  30
Thr Asp Gly Ile Pro Glu Glu Thr Asp Gly Asp Ala Asp Val Asp Leu
        35                  40                  45
Lys Glu Ala Ala Ala Glu Glu Gly Glu Leu Glu Ser Gln Asp Val Ser
    50                  55                  60

Asp Leu Thr Thr Val Glu Arg Glu Asp Ser Ser Leu Leu Asn Pro Ala
65 70 75 80
Ala Lys Lys Leu Lys Ile Asp Thr Lys Glu Lys Lys Glu Lys Lys Gln
85 90 95
Lys Val Asp Glu Asp Glu Ile Gln Lys Met Gln Ile Leu Val Ser Ser
100 105 110
Phe Ser Glu Glu Gln Leu Asn Arg Tyr Glu Met Tyr Arg Arg Ser Ala
115 120 125
Phe Pro Lys Ala Ala Ile Lys Arg Leu Ile Gln Ser Ile Thr Gly Thr
130 135 140
Ser Val Ser Gln Asn Val Val Ile Ala Met Ser Gly Ile Ser Lys Val
145 150 155 160
Phe Val Gly Glu Val Val Glu Glu Ala Leu Asp Val Cys Glu Lys Trp
165 170 175
Gly Glu Met Pro Pro Leu Gln Pro Lys His Met Arg Glu Ala Val Arg
180 185 190
Arg Leu Lys Ser Lys Gly Gln Ile Pro Asn Ser Lys His Lys Lys Ile
195 200 205
Ile Phe Phe
210

<210> 237
<211> 382
<212> PRT
<213> Homo sapiens
<220>
<221> misc_feature
<222> (19)..(19)
<223> X = A, C, D, E, F, G, H, I, K, L, M, N, P, Q, R, S, T, V, W, or Y
<400> 237
Met Ala Leu Gln Gly Ile Ser Val Val Glu Leu Ser Gly Leu Ala Pro
1 5 10 15
Gly Arg Xaa Cys Ala Met Val Leu Ala Asp Phe Gly Ala Arg Val Val
20 25 30
Arg Val Asp Arg Pro Gly Ser Arg Tyr Asp Val Ser Arg Leu Gly Arg
35 40 45
Gly Lys Arg Ser Leu Val Leu Asp Leu Lys Gln Pro Arg Glu Pro Arg
50 55 60
Ala Ala Ala Ser Val Gln Ala Val Gly Cys Ala Ala Gly Ala Leu Pro
65 70 75 80
Pro Arg Cys His Gly Glu Thr Pro Ala Gly Pro Arg Asp Ser Ala Ala
85 90 95
Gly Lys Ser Lys Ala Tyr Leu Cys Gln Ala Glu Trp Ile Trp Pro Val
100 105 110
Gln Glu Ser Phe Cys Arg Leu Ala Gly His Asp Ile Asn Tyr Leu Ala
115 120 125
Leu Ser Gly Val Leu Ser Lys Ile Gly Arg Ser Gly Glu Asn Pro Tyr
130 135 140
Ala Pro Leu Asn Leu Val Ala Asp Phe Ala Gly Gly Gly Leu Met Cys
145 150 155 160
Ala Leu Gly Ile Ile Met Ala Leu Phe Asp Arg Thr Arg Thr Asp Lys
165 170 175
Gly Gln Val Ile Asp Ala Asn Met Val Glu Gly Thr Ala Tyr Leu Ser
180 185 190
Ser Phe Leu Trp Lys Thr Gln Lys Ser Ser Leu Trp Glu Ala Pro Arg
195 200 205
Gly Gln Asn Met Leu Asp Gly Gly Ala Pro Phe Tyr Thr Thr Tyr Arg
210 215 220
Thr Ala Asp Gly Glu Phe Met Ala Val Gly Ala Ile Glu Pro Gln Phe
225 230 235 240
Tyr Glu Leu Leu Ile Lys Gly Leu Gly Leu Lys Ser Asp Glu Leu Pro
245 250 255
Asn Gln Met Ser Thr Asp Asp Trp Pro Glu Met Lys Lys Lys Phe Ala
260 265 270
Asp Val Phe Ala Lys Lys Thr Lys Ala Glu Trp Cys Gln Ile Phe Asp
275 280 285
Gly Thr Asp Ala Cys Val Thr Pro Val Leu Thr Phe Glu Glu Val Val
290 295 300
His His Asp His Asn Lys Glu Arg Gly Ser Phe Ile Thr Ser Glu Glu

305 310 315 320
Gln Asp Val Ser Pro Arg Leu Ala Pro Leu Leu Leu Asn Thr Pro Ala
325 330 335
Ile Pro Ser Ser Lys Gly Asp Pro Phe Ile Gly Glu His Thr Glu Glu
340 345 350
Ile Leu Glu Glu Phe Gly Phe Ser Arg Glu Glu Ile Tyr Gln Leu Asn
355 360 365
Ser Asp Lys Ile Ile Glu Ser Asn Lys Val Lys Ala Ser Leu
370 375 380

<210> 238
<211> 254
<212> PRT
<213> Homo sapiens
<400> 238
Met Asp Asn Tyr Ala Asp Leu Ser Asp Thr Glu Leu Thr Thr Leu Leu
1 5 10 15
Arg Arg Tyr Asn Ile Pro His Gly Pro Val Val Gly Ser Thr Arg Arg
20 25 30
Leu Tyr Glu Lys Lys Ile Phe Glu Tyr Glu Thr Gln Arg Arg Arg Leu
35 40 45
Ser Pro Pro Ser Ser Ser Ala Ala Ser Ser Tyr Ser Phe Ser Asp Leu
50 55 60
Asn Ser Thr Arg Gly Asp Ala Asp Met Tyr Asp Leu Pro Lys Lys Glu
65 70 75 80
Asp Ala Leu Leu Tyr Gln Ser Lys Gly Tyr Asn Asp Asp Tyr Tyr Glu
85 90 95
Glu Ser Tyr Phe Thr Thr Arg Thr Tyr Gly Glu Pro Glu Ser Ala Gly
100 105 110
Pro Ser Arg Ala Val Arg Gln Ser Val Thr Ser Phe Pro Asp Ala Asp
115 120 125
Ala Phe His His Gln Val His Asp Asp Asp Leu Leu Ser Ser Ser Glu
130 135 140
Glu Glu Cys Lys Asp Arg Glu Arg Pro Met Tyr Gly Arg Asp Ser Ala
145 150 155 160
Tyr Gln Ser Ile Thr His Tyr Arg Pro Val Ser Ala Ser Arg Ser Ser
165 170 175
Leu Asp Leu Ser Tyr Tyr Pro Thr Ser Ser Ser Thr Ser Phe Met Ser
180 185 190
Ser Ser Ser Ser Ser Ser Ser Trp Leu Thr Arg Arg Ala Ile Arg Pro
195 200 205
Glu Asn Arg Ala Pro Gly Ala Gly Leu Gly Gln Asp Arg Gln Val Pro
210 215 220
Leu Trp Gly Gln Leu Leu Leu Phe Leu Val Phe Val Ile Val Leu Phe
225 230 235 240
Phe Ile Tyr His Phe Met Gln Ala Glu Glu Gly Asn Pro Phe
245 250

<210> 239
<211> 423
<212> PRT
<213> Homo sapiens
<400> 239
Met Ala Met Val Val Ser Ser Trp Arg Asp Pro Gln Asp Asp Val Ala
1 5 10 15
Gly Gly Asn Pro Gly Gly Pro Asn Pro Ala Ala Gln Ala Ala Arg Gly
20 25 30
Gly Gly Gly Gly Ala Gly Glu Gln Gln Gln Gln Ala Gly Ser Gly Ala
35 40 45
Pro His Thr Pro Gln Thr Pro Gly Gln Pro Gly Ala Pro Ala Thr Pro
50 55 60
Gly Thr Ala Gly Asp Lys Gly Gln Gly Pro Pro Gly Ser Gly Gln Ser
65 70 75 80
Gln Gln His Ile Glu Cys Val Val Cys Gly Asp Lys Ser Ser Gly Lys
85 90 95
His Tyr Gly Gln Phe Thr Cys Glu Gly Cys Lys Ser Phe Phe Lys Arg
100 105 110
Ser Val Arg Arg Asn Leu Thr Tyr Thr Cys Arg Ala Asn Arg Asn Cys
115 120 125

Pro Ile Asp Gln His His Arg Asn Gln Cys Gln Tyr Cys Arg Leu Lys
130 135 140
Lys Cys Leu Lys Val Gly Met Arg Arg Glu Ala Val Gln Arg Gly Arg
145 150 155 160
Met Pro Pro Thr Gln Pro Asn Pro Gly Gln Tyr Ala Leu Thr Asn Gly
165 170 175
Asp Pro Leu Asn Gly His Cys Tyr Leu Ser Gly Tyr Ile Ser Leu Leu
180 185 190
Leu Arg Ala Glu Pro Tyr Pro Thr Ser Arg Tyr Gly Ser Gln Cys Met
195 200 205
Gln Pro Asn Asn Ile Met Gly Ile Glu Asn Ile Cys Glu Leu Ala Ala
210 215 220
Arg Leu Leu Phe Ser Ala Val Glu Trp Ala Arg Asn Ile Pro Phe Phe
225 230 235 240
Pro Asp Leu Gln Ile Thr Asp Gln Val Ser Leu Leu Arg Leu Thr Trp
245 250 255
Ser Glu Leu Phe Val Leu Asn Ala Ala Gln Cys Ser Met Pro Leu His
260 265 270
Val Ala Pro Leu Leu Ala Ala Ala Gly Leu His Ala Ser Pro Met Ser
275 280 285
Ala Asp Arg Val Val Ala Phe Met Asp His Ile Arg Ile Phe Gln Glu
290 295 300
Gln Val Glu Lys Leu Lys Ala Leu His Val Asp Ser Ala Glu Tyr Ser
305 310 315 320
Cys Leu Lys Ala Ile Val Leu Phe Thr Ser Asp Ala Cys Gly Leu Ser
325 330 335
Asp Ala Ala His Ile Glu Ser Leu Gln Glu Lys Ser Gln Cys Ala Leu
340 345 350
Glu Glu Tyr Val Arg Ser Gln Tyr Pro Asn Gln Pro Ser Arg Phe Gly
355 360 365
Lys Leu Leu Leu Arg Leu Pro Ser Leu Arg Thr Val Ser Ser Ser Val
370 375 380
Ile Glu Gln Leu Phe Phe Val Arg Leu Val Gly Lys Thr Pro Ile Glu
385 390 395 400
Thr Leu Ile Arg Asp Met Leu Leu Ser Gly Ser Ser Phe Asn Trp Pro
405 410 415
Tyr Met Ser Ile Gln Cys Ser
420

<210> 240
<211> 536
<212> PRT
<213> Homo sapiens
<400> 240
Met Lys Gln Ser Ser Asn Val Pro Ala Phe Leu Ser Lys Leu Trp Thr
1 5 10 15
Leu Val Glu Glu Thr His Thr Asn Glu Phe Ile Thr Trp Ser Gln Asn
20 25 30
Gly Gln Ser Phe Leu Val Leu Asp Glu Gln Arg Phe Ala Lys Glu Ile
35 40 45
Leu Pro Lys Tyr Phe Lys His Asn Asn Met Ala Ser Phe Val Arg Gln
50 55 60
Leu Asn Met Tyr Gly Phe Arg Lys Val Val His Ile Asp Ser Gly Ile
65 70 75 80
Val Lys Gln Glu Arg Asp Gly Pro Val Glu Phe Gln His Pro Tyr Phe
85 90 95
Lys Gln Gly Gln Asp Asp Leu Leu Glu Asn Ile Lys Arg Lys Val Ser
100 105 110
Ser Ser Lys Pro Glu Glu Asn Lys Ile Arg Gln Glu Asp Leu Thr Lys
115 120 125
Ile Ile Ser Ser Ala Gln Lys Val Gln Ile Lys Gln Glu Thr Ile Glu
130 135 140
Ser Arg Leu Ser Glu Leu Lys Ser Glu Asn Glu Ser Leu Trp Lys Glu
145 150 155 160
Val Ser Glu Leu Arg Ala Lys His Ala Gln Gln Gln Gln Val Ile Arg
165 170 175
Lys Ile Val Gln Phe Ile Val Thr Leu Val Gln Asn Asn Gln Leu Val
180 185 190
Ser Leu Lys Arg Lys Arg Pro Leu Leu Leu Asn Thr Asn Gly Ala Gln

```
        195                 200                 205
Lys Lys Asn Leu Phe Gln His Ile Val Lys Glu Pro Thr Asp Asn His
    210                 215                 220
His His Lys Val Pro His Ser Arg Thr Glu Gly Leu Lys Pro Arg Glu
225                 230                 235                 240
Arg Ile Ser Asp Asp Ile Ile Ile Tyr Asp Val Thr Asp Asp Asn Ala
                245                 250                 255
Asp Glu Glu Asn Ile Pro Val Ile Pro Glu Thr Asn Glu Asp Val Ile
            260                 265                 270
Ser Asp Pro Ser Asn Cys Ser Gln Tyr Pro Asp Ile Val Ile Val Glu
        275                 280                 285
Asp Asp Asn Glu Asp Glu Tyr Ala Pro Val Ile Gln Ser Gly Glu Gln
    290                 295                 300
Asn Glu Pro Ala Arg Glu Ser Leu Ser Ser Gly Ser Asp Gly Ser Ser
305                 310                 315                 320
Pro Leu Met Ser Ser Ala Val Gln Leu Asn Gly Ser Ser Ser Leu Thr
                325                 330                 335
Ser Glu Asp Pro Val Thr Met Met Asp Ser Ile Leu Asn Asp Asn Ile
            340                 345                 350
Asn Leu Leu Gly Lys Val Glu Leu Leu Asp Tyr Leu Asp Ser Ile Asp
        355                 360                 365
Cys Ser Leu Glu Asp Phe Gln Ala Met Leu Ser Gly Arg Gln Phe Ser
    370                 375                 380
Ile Asp Pro Asp Leu Leu Val Asp Leu Phe Thr Ser Ser Val Gln Met
385                 390                 395                 400
Asn Pro Thr Asp Tyr Ile Asn Asn Thr Lys Ser Glu Asn Lys Gly Leu
                405                 410                 415
Glu Thr Thr Lys Asn Asn Val Val Gln Pro Val Ser Glu Glu Gly Arg
            420                 425                 430
Lys Ser Lys Ser Lys Pro Asp Lys Gln Leu Ile Gln Tyr Thr Ala Phe
        435                 440                 445
Pro Leu Leu Ala Phe Leu Asp Gly Asn Pro Ala Ser Ser Val Glu Gln
    450                 455                 460
Ala Ser Thr Thr Ala Ser Ser Glu Val Leu Ser Ser Val Asp Lys Pro
465                 470                 475                 480
Ile Glu Val Asp Glu Leu Leu Asp Ser Ser Leu Asp Pro Glu Pro Thr
                485                 490                 495
Gln Ser Lys Leu Val Arg Leu Glu Pro Leu Thr Glu Ala Glu Ala Ser
            500                 505                 510
Glu Ala Thr Leu Phe Tyr Leu Cys Glu Leu Ala Pro Ala Pro Leu Asp
        515                 520                 525
Ser Asp Met Pro Leu Leu Asp Ser
    530                 535
```

<210> 241
<211> 616
<212> PRT
<213> Homo sapiens
<400> 241

```
Met Asn Ser Pro Gly Gly Arg Gly Lys Lys Lys Gly Ser Gly Gly Ala
1               5                   10                  15
Ser Asn Pro Val Pro Pro Arg Pro Pro Pro Pro Cys Leu Ala Pro Ala
            20                  25                  30
Pro Pro Ala Ala Gly Pro Ala Pro Pro Pro Glu Ser Pro His Lys Arg
        35                  40                  45
Asn Leu Tyr Tyr Phe Ser Tyr Pro Leu Phe Val Gly Phe Ala Leu Leu
    50                  55                  60
Arg Leu Val Ala Phe His Leu Gly Leu Leu Phe Val Trp Leu Cys Gln
65                  70                  75                  80
Arg Phe Ser Arg Ala Leu Met Ala Ala Lys Arg Ser Ser Gly Ala Ala
                85                  90                  95
Pro Ala Pro Ala Ser Ala Ser Ala Pro Ala Pro Val Pro Gly Gly Glu
            100                 105                 110
Ala Glu Arg Val Arg Val Phe His Lys Gln Ala Phe Glu Tyr Ile Ser
        115                 120                 125
Ile Ala Leu Arg Ile Asp Glu Asp Glu Lys Ala Gly Gln Lys Glu Gln
    130                 135                 140
Ala Val Glu Trp Tyr Lys Lys Gly Ile Glu Glu Leu Glu Lys Gly Ile
145                 150                 155                 160
```

Ala Val Ile Val Thr Gly Gln Gly Glu Gln Cys Glu Arg Ala Arg Arg
165 170 175
Leu Gln Ala Lys Met Met Thr Asn Leu Val Met Ala Lys Asp Arg Leu
180 185 190
Gln Leu Leu Glu Lys Met Gln Pro Val Leu Pro Phe Ser Lys Ser Gln
195 200 205
Thr Asp Val Tyr Asn Asp Ser Thr Asn Leu Ala Cys Arg Asn Gly His
210 215 220
Leu Gln Ser Glu Ser Gly Ala Val Pro Lys Arg Lys Asp Pro Leu Thr
225 230 235 240
His Thr Ser Asn Ser Leu Pro Arg Ser Lys Thr Val Met Lys Thr Gly
245 250 255
Ser Ala Gly Leu Ser Gly His His Arg Ala Pro Ser Tyr Ser Gly Leu
260 265 270
Ser Met Val Ser Gly Val Lys Gln Gly Ser Gly Pro Ala Pro Thr Thr
275 280 285
His Lys Gly Thr Pro Lys Thr Asn Arg Thr Asn Lys Pro Ser Thr Pro
290 295 300
Thr Thr Ala Thr Arg Lys Lys Lys Asp Leu Lys Asn Phe Arg Asn Val
305 310 315 320
Asp Ser Asn Leu Ala Asn Leu Ile Met Asn Glu Ile Val Asp Asn Gly
325 330 335
Thr Ala Val Lys Phe Asp Asp Ile Ala Gly Gln Asp Leu Ala Lys Gln
340 345 350
Ala Leu Gln Glu Ile Val Ile Leu Pro Ser Leu Arg Pro Glu Leu Phe
355 360 365
Thr Gly Leu Arg Ala Pro Ala Arg Gly Leu Leu Leu Phe Gly Pro Pro
370 375 380
Gly Asn Gly Lys Thr Met Leu Ala Lys Ala Val Ala Ala Glu Ser Asn
385 390 395 400
Ala Thr Phe Phe Asn Ile Ser Ala Ala Ser Leu Thr Ser Lys Tyr Val
405 410 415
Gly Glu Gly Glu Lys Leu Val Arg Ala Leu Phe Ala Val Ala Arg Glu
420 425 430
Leu Gln Pro Ser Ile Ile Phe Ile Asp Glu Val Asp Ser Leu Leu Cys
435 440 445
Glu Arg Arg Glu Gly Glu His Asp Ala Ser Arg Arg Leu Lys Thr Glu
450 455 460
Phe Leu Ile Glu Phe Asp Gly Val Gln Ser Ala Gly Asp Asp Arg Val
465 470 475 480
Leu Val Met Gly Ala Thr Asn Arg Pro Gln Glu Leu Asp Glu Ala Val
485 490 495
Leu Arg Arg Phe Ile Lys Arg Val Tyr Val Ser Leu Pro Asn Glu Glu
500 505 510
Thr Arg Leu Leu Leu Leu Lys Asn Leu Leu Cys Lys Gln Gly Ser Pro
515 520 525
Leu Thr Gln Lys Glu Leu Ala Gln Leu Ala Arg Met Thr Asp Gly Tyr
530 535 540
Ser Gly Ser Asp Leu Thr Ala Leu Ala Lys Asp Ala Ala Leu Gly Pro
545 550 555 560
Ile Arg Glu Leu Lys Pro Glu Gln Val Lys Asn Met Ser Ala Ser Glu
565 570 575
Met Arg Asn Ile Arg Leu Ser Asp Phe Thr Glu Ser Leu Lys Lys Ile
580 585 590
Lys Arg Ser Val Ser Pro Gln Thr Leu Glu Ala Tyr Ile Arg Trp Asn
595 600 605
Lys Asp Phe Gly Asp Thr Thr Val
610 615

<210> 242
<211> 1979
<212> PRT
<213> Homo sapiens
<400> 242
Met Ser Ser Trp Leu Gly Gly Leu Gly Ser Gly Leu Gly Gln Ser Leu
1 5 10 15
Gly Gln Val Gly Gly Ser Leu Ala Ser Leu Thr Gly Gln Ile Ser Asn
20 25 30
Phe Thr Lys Asp Met Leu Met Glu Gly Thr Glu Glu Val Glu Ala Glu

```
        35                  40                  45
Leu Pro Asp Ser Arg Thr Lys Glu Ile Glu Ala Ile His Ala Ile Leu
    50                  55                  60
Arg Ser Glu Asn Glu Arg Leu Lys Lys Leu Cys Thr Asp Leu Glu Glu
65                  70                  75                  80
Lys His Glu Ala Ser Glu Ile Gln Ile Lys Gln Gln Ser Thr Ser Tyr
                85                  90                  95
Arg Asn Gln Leu Gln Gln Lys Glu Val Glu Ile Ser His Leu Lys Ala
            100                 105                 110
Arg Gln Ile Ala Leu Gln Asp Gln Leu Leu Lys Leu Gln Ser Ala Ala
        115                 120                 125
Gln Ser Val Pro Ser Gly Ala Gly Val Pro Ala Thr Thr Ala Ser Ser
    130                 135                 140
Ser Phe Ala Tyr Gly Ile Ser His His Pro Ser Ala Phe His Asp Asp
145                 150                 155                 160
Asp Met Asp Phe Gly Asp Ile Ile Ser Ser Gln Gln Glu Ile Asn Arg
                165                 170                 175
Leu Ser Asn Glu Val Ser Arg Leu Glu Ser Glu Val Gly His Trp Arg
            180                 185                 190
His Ile Ala Gln Thr Ser Lys Ala Gln Gly Thr Asp Asn Ser Asp Gln
        195                 200                 205
Ser Glu Ile Cys Lys Leu Gln Asn Ile Ile Lys Glu Leu Lys Gln Asn
    210                 215                 220
Arg Ser Gln Glu Ile Asp Asp His Gln His Glu Met Ser Val Leu Gln
225                 230                 235                 240
Asn Ala His Gln Gln Lys Leu Thr Glu Ile Ser Arg Arg His Arg Glu
                245                 250                 255
Glu Leu Ser Asp Tyr Glu Glu Arg Ile Glu Glu Leu Glu Asn Leu Leu
            260                 265                 270
Gln Gln Gly Gly Ser Gly Val Ile Glu Thr Asp Leu Ser Lys Ile Tyr
        275                 280                 285
Glu Met Gln Lys Thr Ile Gln Val Leu Gln Ile Glu Lys Val Glu Ser
    290                 295                 300
Thr Lys Lys Met Glu Gln Leu Glu Asp Lys Ile Lys Asp Ile Asn Lys
305                 310                 315                 320
Lys Leu Ser Ser Ala Glu Asn Asp Arg Asp Ile Leu Arg Arg Glu Gln
                325                 330                 335
Glu Gln Leu Asn Val Glu Lys Arg Gln Ile Met Glu Glu Cys Glu Asn
            340                 345                 350
Leu Lys Leu Glu Cys Ser Lys Leu Gln Pro Ser Ala Val Lys Gln Ser
        355                 360                 365
Asp Thr Met Thr Glu Lys Glu Arg Ile Leu Ala Gln Ser Ala Ser Val
    370                 375                 380
Glu Glu Val Phe Arg Leu Gln Gln Ala Leu Ser Asp Ala Glu Asn Glu
385                 390                 395                 400
Ile Met Arg Leu Ser Ser Leu Asn Gln Asp Asn Ser Leu Ala Glu Asp
                405                 410                 415
Asn Leu Lys Leu Lys Met Arg Ile Glu Val Leu Glu Lys Glu Lys Ser
            420                 425                 430
Leu Leu Ser Gln Glu Lys Glu Glu Leu Gln Met Ser Leu Leu Lys Leu
        435                 440                 445
Asn Asn Glu Tyr Glu Val Ile Lys Ser Thr Ala Thr Arg Asp Ile Ser
    450                 455                 460
Leu Asp Ser Glu Leu His Asp Leu Arg Leu Asn Leu Glu Ala Lys Glu
465                 470                 475                 480
Gln Glu Leu Asn Gln Ser Ile Ser Glu Lys Glu Thr Leu Ile Ala Glu
                485                 490                 495
Ile Glu Glu Leu Asp Arg Gln Asn Gln Glu Ala Thr Lys His Met Ile
            500                 505                 510
Leu Ile Lys Asp Gln Leu Ser Lys Gln Gln Asn Glu Gly Asp Ser Ile
        515                 520                 525
Ile Ser Lys Leu Lys Gln Asp Leu Asn Asp Glu Lys Lys Arg Val His
    530                 535                 540
Gln Leu Glu Asp Asp Lys Met Asp Ile Thr Lys Glu Leu Asp Val Gln
545                 550                 555                 560
Lys Glu Lys Leu Ile Gln Ser Glu Val Ala Leu Asn Asp Leu His Leu
                565                 570                 575
Thr Lys Gln Lys Leu Glu Asp Lys Val Glu Asn Leu Val Asp Gln Leu
            580                 585                 590
```

```
Asn Lys Ser Gln Glu Ser Asn Val Ser Ile Gln Lys Glu Asn Leu Glu
        595                 600                 605
Leu Lys Glu His Ile Arg Gln Asn Glu Glu Glu Leu Ser Arg Ile Arg
    610                 615                 620
Asn Glu Leu Met Gln Ser Leu Asn Gln Asp Ser Asn Ser Asn Phe Lys
625                 630                 635                 640
Asp Thr Leu Leu Lys Glu Arg Glu Ala Glu Val Arg Asn Leu Lys Gln
                645                 650                 655
Asn Leu Ser Glu Leu Glu Gln Leu Asn Glu Asn Leu Lys Lys Val Ala
            660                 665                 670
Phe Asp Val Lys Met Glu Asn Glu Lys Leu Val Leu Ala Cys Glu Asp
        675                 680                 685
Val Arg His Gln Leu Glu Glu Cys Leu Ala Gly Asn Asn Gln Leu Ser
    690                 695                 700
Leu Glu Lys Asn Thr Ile Val Glu Thr Leu Lys Met Glu Lys Gly Glu
705                 710                 715                 720
Ile Glu Ala Glu Leu Cys Trp Ala Lys Lys Arg Leu Leu Glu Glu Ala
                725                 730                 735
Asn Lys Tyr Glu Lys Thr Ile Glu Glu Leu Ser Asn Ala Arg Asn Leu
            740                 745                 750
Asn Thr Ser Ala Leu Gln Leu Glu His Glu His Leu Ile Lys Leu Asn
        755                 760                 765
Gln Lys Lys Asp Met Glu Ile Ala Glu Leu Lys Lys Asn Ile Glu Gln
    770                 775                 780
Met Asp Thr Asp His Lys Glu Thr Lys Asp Val Leu Ser Ser Ser Leu
785                 790                 795                 800
Glu Glu Gln Lys Gln Leu Thr Gln Leu Ile Asn Lys Lys Glu Ile Phe
                805                 810                 815
Ile Glu Lys Leu Lys Glu Arg Ser Ser Lys Leu Gln Glu Glu Leu Asp
            820                 825                 830
Lys Tyr Ser Gln Ala Leu Arg Lys Asn Glu Ile Leu Arg Gln Thr Ile
        835                 840                 845
Glu Glu Lys Asp Arg Ser Leu Gly Ser Met Lys Glu Glu Asn Asn His
    850                 855                 860
Leu Gln Glu Glu Leu Glu Arg Leu Arg Glu Glu Gln Ser Arg Thr Ala
865                 870                 875                 880
Pro Val Ala Asp Pro Lys Thr Leu Asp Ser Val Thr Glu Leu Ala Ser
                885                 890                 895
Glu Val Ser Gln Leu Asn Thr Ile Lys Glu His Leu Glu Glu Glu Ile
            900                 905                 910
Lys His His Gln Lys Ile Ile Glu Asp Gln Asn Gln Ser Lys Met Gln
        915                 920                 925
Leu Leu Gln Ser Leu Gln Glu Gln Lys Lys Glu Met Asp Glu Phe Arg
    930                 935                 940
Tyr Gln His Glu Gln Met Asn Ala Thr His Thr Gln Leu Phe Leu Glu
945                 950                 955                 960
Lys Asp Glu Glu Ile Lys Ser Leu Gln Lys Thr Ile Glu Gln Ile Lys
                965                 970                 975
Thr Gln Leu His Glu Glu Arg Gln Asp Ile Gln Thr Asp Asn Ser Asp
            980                 985                 990
Ile Phe Gln Glu Thr Lys Val Gln  Ser Leu Asn Ile Glu  Asn Gly Ser
        995                 1000                1005
Glu Lys  His Asp Leu Ser Lys  Ala Glu Thr Glu Arg  Leu Val Lys
    1010                1015                1020
Gly Ile  Lys Glu Arg Glu Leu  Glu Ile Lys Leu Leu  Asn Glu Lys
    1025                1030                1035
Asn Ile  Ser Leu Thr Lys Gln  Ile Asp Gln Leu Ser  Lys Asp Glu
    1040                1045                1050
Val Gly  Lys Leu Thr Gln Ile  Ile Gln Gln Lys Asp  Leu Glu Ile
    1055                1060                1065
Gln Ala  Leu His Ala Arg Ile  Ser Ser Thr Ser His  Thr Gln Asp
    1070                1075                1080
Val Val  Tyr Leu Gln Gln Gln  Leu Gln Ala Tyr Ala  Met Glu Arg
    1085                1090                1095
Glu Lys  Val Phe Ala Val Leu  Asn Glu Lys Thr Arg  Glu Asn Ser
    1100                1105                1110
His Leu  Lys Thr Glu Tyr His  Lys Met Met Asp Ile  Val Ala Ala
    1115                1120                1125
Lys Glu  Ala Ala Leu Ile Lys  Leu Gln Asp Glu Asn  Lys Lys Leu
```

1130 1135 1140
Ser Thr Arg Phe Glu Ser Ser Gly Gln Asp Met Phe Arg Glu Thr
1145 1150 1155
Ile Gln Asn Leu Ser Arg Ile Ile Arg Glu Lys Asp Ile Glu Ile
1160 1165 1170
Asp Ala Leu Ser Gln Lys Cys Gln Thr Leu Leu Ala Val Leu Gln
1175 1180 1185
Thr Ser Ser Thr Gly Asn Glu Ala Gly Gly Val Asn Ser His Gln
1190 1195 1200
Phe Glu Glu Leu Leu Gln Glu Arg Asp Lys Leu Lys Gln Gln Val
1205 1210 1215
Lys Lys Met Glu Glu Trp Lys Gln Gln Val Met Thr Thr Val Gln
1220 1225 1230
Asn Met Gln His Glu Ser Ala Gln Leu Gln Glu Glu Leu His Gln
1235 1240 1245
Leu Gln Ala Gln Val Leu Val Asp Ser Asp Asn Asn Ser Lys Leu
1250 1255 1260
Gln Val Asp Tyr Thr Gly Leu Ile Gln Ser Tyr Glu Gln Asn Glu
1265 1270 1275
Thr Lys Leu Lys Asn Phe Gly Gln Glu Leu Ala Gln Val Gln His
1280 1285 1290
Ser Ile Gly Gln Leu Cys Asn Thr Lys Asp Leu Leu Leu Gly Lys
1295 1300 1305
Leu Asp Ile Ile Ser Pro Gln Leu Ser Ser Ala Ser Leu Leu Thr
1310 1315 1320
Pro Gln Ser Ala Glu Cys Leu Arg Ala Ser Lys Ser Glu Val Leu
1325 1330 1335
Ser Glu Ser Ser Glu Leu Leu Gln Gln Glu Leu Glu Glu Leu Arg
1340 1345 1350
Lys Ser Leu Gln Glu Lys Asp Ala Thr Ile Arg Thr Leu Gln Glu
1355 1360 1365
Asn Asn His Arg Leu Ser Asp Ser Ile Ala Ala Thr Ser Glu Leu
1370 1375 1380
Glu Arg Lys Glu His Glu Gln Thr Asp Ser Glu Ile Lys Gln Leu
1385 1390 1395
Lys Glu Lys Gln Asp Val Leu Gln Lys Leu Leu Lys Glu Lys Asp
1400 1405 1410
Leu Leu Ile Lys Ala Lys Ser Asp Gln Leu Leu Ser Ser Asn Glu
1415 1420 1425
Asn Phe Thr Asn Lys Val Asn Glu Asn Glu Leu Leu Arg Gln Ala
1430 1435 1440
Val Thr Asn Leu Lys Glu Arg Ile Leu Ile Leu Glu Met Asp Ile
1445 1450 1455
Gly Lys Leu Lys Gly Glu Asn Glu Lys Ile Val Glu Thr Tyr Arg
1460 1465 1470
Gly Lys Glu Thr Glu Tyr Gln Ala Leu Gln Glu Thr Asn Met Lys
1475 1480 1485
Phe Ser Met Met Leu Arg Glu Lys Glu Phe Glu Cys His Ser Met
1490 1495 1500
Lys Glu Lys Ala Leu Ala Phe Glu Gln Leu Leu Lys Glu Lys Glu
1505 1510 1515
Gln Gly Lys Thr Gly Glu Leu Asn Gln Leu Leu Asn Ala Val Lys
1520 1525 1530
Ser Met Gln Glu Lys Thr Val Val Phe Gln Gln Glu Arg Asp Gln
1535 1540 1545
Val Met Leu Ala Leu Lys Gln Lys Gln Met Glu Asn Thr Ala Leu
1550 1555 1560
Gln Asn Glu Val Gln Arg Leu Arg Asp Lys Glu Phe Arg Ser Asn
1565 1570 1575
Gln Glu Leu Glu Arg Leu Arg Asn His Leu Leu Glu Ser Glu Asp
1580 1585 1590
Ser Tyr Thr Arg Glu Ala Leu Ala Ala Glu Asp Arg Glu Ala Lys
1595 1600 1605
Leu Arg Lys Lys Val Thr Val Leu Glu Glu Lys Leu Val Ser Ser
1610 1615 1620
Ser Asn Ala Met Glu Asn Ala Ser His Gln Ala Ser Val Gln Val
1625 1630 1635
Glu Ser Leu Gln Glu Gln Leu Asn Val Val Ser Lys Gln Arg Asp
1640 1645 1650

Glu Thr Ala Leu Gln Leu Ser Val Ser Gln Glu Gln Val Lys Gln
1655 1660 1665
Tyr Ala Leu Ser Leu Ala Asn Leu Gln Met Val Leu Glu His Phe
1670 1675 1680
Gln Gln Glu Glu Lys Ala Met Tyr Ser Ala Glu Leu Glu Lys Gln
1685 1690 1695
Lys Gln Leu Ile Ala Glu Trp Lys Lys Asn Ala Glu Asn Leu Glu
1700 1705 1710
Gly Lys Val Ile Ser Leu Gln Glu Cys Leu Asp Glu Ala Asn Ala
1715 1720 1725
Ala Leu Asp Ser Ala Ser Arg Leu Thr Glu Gln Leu Asp Val Lys
1730 1735 1740
Glu Glu Gln Ile Glu Glu Leu Lys Arg Gln Asn Glu Leu Arg Gln
1745 1750 1755
Glu Met Leu Asp Asp Val Gln Lys Lys Leu Met Ser Leu Ala Asn
1760 1765 1770
Ser Ser Glu Gly Lys Val Asp Lys Val Leu Met Arg Asn Leu Phe
1775 1780 1785
Ile Gly His Phe His Thr Pro Lys Asn Gln Arg His Glu Val Leu
1790 1795 1800
Arg Leu Met Gly Ser Ile Leu Gly Val Arg Arg Glu Glu Met Glu
1805 1810 1815
Gln Leu Phe His Asp Asp Gln Gly Ser Val Thr Arg Trp Met Thr
1820 1825 1830
Gly Trp Leu Gly Gly Gly Ser Lys Ser Val Pro Asn Thr Pro Leu
1835 1840 1845
Arg Pro Asn Gln Gln Ser Val Val Asn Ser Ser Phe Ser Glu Leu
1850 1855 1860
Phe Val Lys Phe Leu Glu Thr Glu Ser His Pro Ser Ile Pro Pro
1865 1870 1875
Pro Lys Leu Ser Val His Asp Met Lys Pro Leu Asp Ser Pro Gly
1880 1885 1890
Arg Arg Lys Arg Asp Thr Asn Ala Pro Glu Ser Phe Lys Asp Thr
1895 1900 1905
Ala Glu Ser Arg Ser Gly Arg Arg Thr Asp Val Asn Pro Phe Leu
1910 1915 1920
Ala Pro Arg Ser Ala Ala Val Pro Leu Ile Asn Pro Ala Gly Leu
1925 1930 1935
Gly Pro Gly Gly Pro Gly His Leu Leu Leu Lys Pro Ile Ser Asp
1940 1945 1950
Val Leu Pro Thr Phe Thr Pro Leu Pro Ala Leu Pro Asp Asn Ser
1955 1960 1965
Ala Gly Val Val Leu Lys Asp Leu Leu Lys Gln
1970 1975

<210> 243
<211> 522
<212> PRT
<213> Homo sapiens
<400> 243
Met Ser Ser Arg Pro Leu Glu Ser Pro Pro Pro Tyr Arg Pro Asp Glu
1 5 10 15
Phe Lys Pro Asn His Tyr Ala Pro Ser Asn Asp Ile Tyr Gly Gly Glu
20 25 30
Met His Val Arg Pro Met Leu Ser Gln Pro Ala Tyr Ser Phe Tyr Pro
35 40 45
Glu Asp Glu Ile Leu His Phe Tyr Lys Trp Thr Ser Pro Pro Gly Val
50 55 60
Ile Arg Ile Leu Ser Met Leu Ile Ile Val Met Cys Ile Ala Ile Phe
65 70 75 80
Ala Cys Val Ala Ser Thr Leu Ala Trp Asp Arg Gly Tyr Gly Thr Ser
85 90 95
Leu Leu Gly Gly Ser Val Gly Tyr Pro Tyr Gly Gly Ser Gly Phe Gly
100 105 110
Ser Tyr Gly Ser Gly Tyr Gly Tyr Gly Tyr Gly Tyr Gly Tyr Gly Tyr
115 120 125
Gly Gly Tyr Thr Asp Pro Arg Ala Ala Lys Gly Phe Met Leu Ala Met
130 135 140
Ala Ala Phe Cys Phe Ile Ala Ala Leu Val Ile Phe Val Thr Ser Val

145 150 155 160
Ile Arg Ser Glu Met Ser Arg Thr Arg Arg Tyr Tyr Leu Ser Val Ile
165 170 175
Ile Val Ser Ala Ile Leu Gly Ile Met Val Phe Ile Ala Thr Ile Val
180 185 190
Tyr Ile Met Gly Val Asn Pro Thr Ala Gln Ser Ser Gly Ser Leu Tyr
195 200 205
Gly Ser Gln Ile Tyr Ala Leu Cys Asn Gln Phe Tyr Thr Pro Ala Ala
210 215 220
Thr Gly Leu Tyr Val Asp Gln Tyr Leu Tyr His Tyr Cys Val Val Asp
225 230 235 240
Pro Gln Glu Ala Ile Ala Ile Val Leu Gly Phe Met Ile Ile Val Ala
245 250 255
Phe Ala Leu Ile Ile Phe Phe Ala Val Lys Thr Arg Arg Lys Met Asp
260 265 270
Arg Tyr Asp Lys Ser Asn Ile Leu Trp Asp Lys Glu His Ile Tyr Asp
275 280 285
Glu Gln Pro Pro Asn Val Glu Glu Trp Val Lys Asn Val Ser Ala Gly
290 295 300
Thr Gln Asp Val Pro Ser Pro Pro Ser Asp Tyr Val Glu Arg Val Asp
305 310 315 320
Ser Pro Met Ala Tyr Ser Ser Asn Gly Lys Val Asn Asp Lys Arg Phe
325 330 335
Tyr Pro Glu Ser Ser Tyr Lys Ser Thr Pro Val Pro Glu Val Val Gln
340 345 350
Glu Leu Pro Leu Thr Ser Pro Val Asp Asp Phe Arg Gln Pro Arg Tyr
355 360 365
Ser Ser Gly Gly Asn Phe Glu Thr Pro Ser Lys Arg Ala Pro Ala Lys
370 375 380
Gly Arg Ala Gly Arg Ser Lys Arg Thr Glu Gln Asp His Tyr Glu Thr
385 390 395 400
Asp Tyr Thr Thr Gly Gly Glu Ser Cys Asp Glu Leu Glu Glu Asp Trp
405 410 415
Ile Arg Glu Tyr Pro Pro Ile Thr Ser Asp Gln Gln Arg Gln Leu Tyr
420 425 430
Lys Arg Asn Phe Asp Thr Gly Leu Gln Glu Tyr Lys Ser Leu Gln Ser
435 440 445
Glu Leu Asp Glu Ile Asn Lys Glu Leu Ser Arg Leu Asp Lys Glu Leu
450 455 460
Asp Asp Tyr Arg Glu Glu Ser Glu Glu Tyr Met Ala Ala Ala Asp Glu
465 470 475 480
Tyr Asn Arg Leu Lys Gln Val Lys Gly Ser Ala Asp Tyr Lys Ser Lys
485 490 495
Lys Asn His Cys Lys Gln Leu Lys Ser Lys Leu Ser His Ile Lys Lys
500 505 510
Met Val Gly Asp Tyr Asp Arg Gln Lys Thr
515 520

<210> 244
<211> 2181
<212> PRT
<213> Homo sapiens
<400> 244
Met Met Met Met Met Met Met Lys Lys Met Gln His Gln Arg Gln Gln
1 5 10 15
Gln Ala Asp His Ala Asn Glu Ala Asn Tyr Ala Arg Gly Thr Arg Leu
20 25 30
Pro Leu Ser Gly Glu Gly Pro Thr Ser Gln Pro Asn Ser Ser Lys Gln
35 40 45
Thr Val Leu Ser Trp Gln Ala Ala Ile Asp Ala Ala Arg Gln Ala Lys
50 55 60
Ala Ala Gln Thr Met Ser Thr Ser Ala Pro Pro Pro Val Gly Ser Leu
65 70 75 80
Ser Gln Arg Lys Arg Gln Gln Tyr Ala Lys Ser Lys Lys Gln Gly Asn
85 90 95
Ser Ser Asn Ser Arg Pro Ala Arg Ala Leu Phe Cys Leu Ser Leu Asn
100 105 110
Asn Pro Ile Arg Arg Ala Cys Ile Ser Ile Val Glu Trp Lys Pro Phe
115 120 125

```
Asp Ile Phe Ile Leu Leu Ala Ile Phe Ala Asn Cys Val Ala Leu Ala
    130                 135                 140
Ile Tyr Ile Pro Phe Pro Glu Asp Asp Ser Asn Ser Thr Asn His Asn
145                 150                 155                 160
Leu Glu Lys Val Glu Tyr Ala Phe Leu Ile Ile Phe Thr Val Glu Thr
                165                 170                 175
Phe Leu Lys Ile Ile Ala Tyr Gly Leu Leu Leu His Pro Asn Ala Tyr
            180                 185                 190
Val Arg Asn Gly Trp Asn Leu Leu Asp Phe Val Ile Val Ile Val Gly
        195                 200                 205
Leu Phe Ser Val Ile Leu Glu Gln Leu Thr Lys Glu Thr Glu Gly Gly
    210                 215                 220
Asn His Ser Ser Gly Lys Ser Gly Gly Phe Asp Val Lys Ala Leu Arg
225                 230                 235                 240
Ala Phe Arg Val Leu Arg Pro Leu Arg Leu Val Ser Gly Val Pro Ser
                245                 250                 255
Leu Gln Val Val Leu Asn Ser Ile Ile Lys Ala Met Val Pro Leu Leu
            260                 265                 270
His Ile Ala Leu Leu Val Leu Phe Val Ile Ile Ile Tyr Ala Ile Ile
        275                 280                 285
Gly Leu Glu Leu Phe Ile Gly Lys Met His Lys Thr Cys Phe Phe Ala
    290                 295                 300
Asp Ser Asp Ile Val Ala Glu Glu Asp Pro Ala Pro Cys Ala Phe Ser
305                 310                 315                 320
Gly Asn Gly Arg Gln Cys Thr Ala Asn Gly Thr Glu Cys Arg Ser Gly
                325                 330                 335
Trp Val Gly Pro Asn Gly Gly Ile Thr Asn Phe Asp Asn Phe Ala Phe
            340                 345                 350
Ala Met Leu Thr Val Phe Gln Cys Ile Thr Met Glu Gly Trp Thr Asp
        355                 360                 365
Val Leu Tyr Trp Val Asn Asp Ala Ile Gly Trp Glu Trp Pro Trp Val
    370                 375                 380
Tyr Phe Val Ser Leu Ile Ile Leu Gly Ser Phe Phe Val Leu Asn Leu
385                 390                 395                 400
Val Leu Gly Val Leu Ser Gly Glu Phe Ser Lys Glu Arg Glu Lys Ala
                405                 410                 415
Lys Ala Arg Gly Asp Phe Gln Lys Leu Arg Glu Lys Gln Gln Leu Glu
            420                 425                 430
Glu Asp Leu Lys Gly Tyr Leu Asp Trp Ile Thr Gln Ala Glu Asp Ile
        435                 440                 445
Asp Pro Glu Asn Glu Glu Glu Gly Gly Glu Glu Gly Lys Arg Asn Thr
    450                 455                 460
Ser Met Pro Thr Ser Glu Thr Glu Ser Val Asn Thr Glu Asn Val Ser
465                 470                 475                 480
Gly Glu Gly Glu Asn Arg Gly Cys Cys Gly Ser Leu Trp Cys Trp Trp
                485                 490                 495
Arg Arg Arg Gly Ala Ala Lys Ala Gly Pro Ser Gly Cys Arg Arg Trp
            500                 505                 510
Gly Gln Ala Ile Ser Lys Ser Lys Leu Ser Arg Arg Trp Arg Arg Trp
        515                 520                 525
Asn Arg Phe Asn Arg Arg Arg Cys Arg Ala Ala Val Lys Ser Val Thr
    530                 535                 540
Phe Tyr Trp Leu Val Ile Val Leu Val Phe Leu Asn Thr Leu Thr Ile
545                 550                 555                 560
Ser Ser Glu His Tyr Asn Gln Pro Asp Trp Leu Thr Gln Ile Gln Asp
                565                 570                 575
Ile Ala Asn Lys Val Leu Leu Ala Leu Phe Thr Cys Glu Met Leu Val
            580                 585                 590
Lys Met Tyr Ser Leu Gly Leu Gln Ala Tyr Phe Val Ser Leu Phe Asn
        595                 600                 605
Arg Phe Asp Cys Phe Val Val Cys Gly Gly Ile Thr Glu Thr Ile Leu
    610                 615                 620
Val Glu Leu Glu Ile Met Ser Pro Leu Gly Ile Ser Val Phe Arg Cys
625                 630                 635                 640
Val Arg Leu Leu Arg Ile Phe Lys Val Thr Arg His Trp Thr Ser Leu
                645                 650                 655
Ser Asn Leu Val Ala Ser Leu Leu Asn Ser Met Lys Ser Ile Ala Ser
            660                 665                 670
Leu Leu Leu Leu Leu Phe Leu Phe Ile Ile Ile Phe Ser Leu Leu Gly
```

```
        675                 680                 685
Met Gln Leu Phe Gly Gly Lys Phe Asn Phe Asp Glu Thr Gln Thr Lys
    690                 695                 700
Arg Ser Thr Phe Asp Asn Phe Pro Gln Ala Leu Leu Thr Val Phe Gln
705                 710                 715                 720
Ile Leu Thr Gly Glu Asp Trp Asn Ala Val Met Tyr Asp Gly Ile Met
                725                 730                 735
Ala Tyr Gly Gly Pro Ser Ser Ser Gly Met Ile Val Cys Ile Tyr Phe
            740                 745                 750
Ile Ile Leu Phe Ile Cys Gly Asn Tyr Ile Leu Leu Asn Val Phe Leu
        755                 760                 765
Ala Ile Ala Val Asp Asn Leu Ala Asp Ala Glu Ser Leu Asn Thr Ala
    770                 775                 780
Gln Lys Glu Glu Ala Glu Glu Lys Glu Arg Lys Lys Ile Ala Arg Lys
785                 790                 795                 800
Glu Ser Leu Glu Asn Lys Lys Asn Asn Lys Pro Glu Val Asn Gln Ile
                805                 810                 815
Ala Asn Ser Asp Asn Lys Val Thr Ile Asp Asp Tyr Arg Glu Glu Asp
            820                 825                 830
Glu Asp Lys Asp Pro Tyr Pro Pro Cys Asp Val Pro Val Gly Glu Glu
        835                 840                 845
Glu Glu Glu Glu Glu Glu Asp Glu Pro Glu Val Pro Ala Gly Pro Arg
    850                 855                 860
Pro Arg Arg Ile Ser Glu Leu Asn Met Lys Glu Lys Ile Ala Pro Ile
865                 870                 875                 880
Pro Glu Gly Ser Ala Phe Phe Ile Leu Ser Lys Thr Asn Pro Ile Arg
                885                 890                 895
Val Gly Cys His Lys Leu Ile Asn His His Ile Phe Thr Asn Leu Ile
            900                 905                 910
Leu Val Phe Ile Met Leu Ser Ser Ala Ala Leu Ala Ala Glu Asp Pro
        915                 920                 925
Ile Arg Ser His Ser Phe Arg Asn Thr Ile Leu Gly Tyr Phe Asp Tyr
    930                 935                 940
Ala Phe Thr Ala Ile Phe Thr Val Glu Ile Leu Leu Lys Met Thr Thr
945                 950                 955                 960
Phe Gly Ala Phe Leu His Lys Gly Ala Phe Cys Arg Asn Tyr Phe Asn
                965                 970                 975
Leu Leu Asp Met Leu Val Val Gly Val Ser Leu Val Ser Phe Gly Ile
            980                 985                 990
Gln Ser Ser Ala Ile Ser Val Val  Lys Ile Leu Arg Val  Leu Arg Val
        995                 1000                1005
Leu Arg  Pro Leu Arg Ala Ile  Asn Arg Ala Lys Gly  Leu Lys His
    1010                1015                1020
Val Val  Gln Cys Val Phe Val  Ala Ile Arg Thr Ile  Gly Asn Ile
    1025                1030                1035
Met Ile  Val Thr Thr Leu Leu  Gln Phe Met Phe Ala  Cys Ile Gly
    1040                1045                1050
Val Gln  Leu Phe Lys Gly Lys  Phe Tyr Arg Cys Thr  Asp Glu Ala
    1055                1060                1065
Lys Ser  Asn Pro Glu Glu Cys  Arg Gly Leu Phe Ile  Leu Tyr Lys
    1070                1075                1080
Asp Gly  Asp Val Asp Ser Pro  Val Val Arg Glu Arg  Ile Trp Gln
    1085                1090                1095
Asn Ser  Asp Phe Asn Phe Asp  Asn Val Leu Ser Ala  Met Met Ala
    1100                1105                1110
Leu Phe  Thr Val Ser Thr Phe  Glu Gly Trp Pro Ala  Leu Leu Tyr
    1115                1120                1125
Lys Ala  Ile Asp Ser Asn Gly  Glu Asn Ile Gly Pro  Ile Tyr Asn
    1130                1135                1140
His Arg  Val Glu Ile Ser Ile  Phe Phe Ile Ile Tyr  Ile Ile Ile
    1145                1150                1155
Val Ala  Phe Phe Met Met Asn  Ile Phe Val Gly Phe  Val Ile Val
    1160                1165                1170
Thr Phe  Gln Glu Gln Gly Glu  Lys Glu Tyr Lys Asn  Cys Glu Leu
    1175                1180                1185
Asp Lys  Asn Gln Arg Gln Cys  Val Glu Tyr Ala Leu  Lys Ala Arg
    1190                1195                1200
Pro Leu  Arg Arg Tyr Ile Pro  Lys Asn Pro Tyr Gln  Tyr Lys Phe
    1205                1210                1215
```

```
Trp Tyr  Val Val Asn Ser Ser  Pro Phe Glu Tyr Met  Met Phe Val
    1220                 1225                 1230
Leu Ile  Met Leu Asn Thr Leu  Cys Leu Ala Met Gln  His Tyr Glu
    1235                 1240                 1245
Gln Ser  Lys Met Phe Asn Asp  Ala Met Asp Ile Leu  Asn Met Val
    1250                 1255                 1260
Phe Thr  Gly Val Phe Thr Val  Glu Met Val Leu Lys  Val Ile Ala
    1265                 1270                 1275
Phe Lys  Pro Lys Gly Tyr Phe  Ser Asp Ala Trp Asn  Thr Phe Asp
    1280                 1285                 1290
Ser Leu  Ile Val Ile Gly Ser  Ile Ile Asp Val Ala  Leu Ser Glu
    1295                 1300                 1305
Ala Asp  Pro Thr Glu Ser Glu  Asn Val Pro Val Pro  Thr Ala Thr
    1310                 1315                 1320
Pro Gly  Asn Ser Glu Glu Ser  Asn Arg Ile Ser Ile  Thr Phe Phe
    1325                 1330                 1335
Arg Leu  Phe Arg Val Met Arg  Leu Val Lys Leu Leu  Ser Arg Gly
    1340                 1345                 1350
Glu Gly  Ile Arg Thr Leu Leu  Trp Thr Phe Ile Lys  Ser Phe Gln
    1355                 1360                 1365
Ala Leu  Pro Tyr Val Ala Leu  Leu Ile Ala Met Leu  Phe Phe Ile
    1370                 1375                 1380
Tyr Ala  Val Ile Gly Met Gln  Met Phe Gly Lys Val  Ala Met Arg
    1385                 1390                 1395
Asp Asn  Asn Gln Ile Asn Arg  Asn Asn Asn Phe Gln  Thr Phe Pro
    1400                 1405                 1410
Gln Ala  Val Leu Leu Leu Phe  Arg Cys Ala Thr Gly  Glu Ala Trp
    1415                 1420                 1425
Gln Glu  Ile Met Leu Ala Cys  Leu Pro Gly Lys Leu  Cys Asp Pro
    1430                 1435                 1440
Glu Ser  Asp Tyr Asn Pro Gly  Glu Glu Tyr Thr Cys  Gly Ser Asn
    1445                 1450                 1455
Phe Ala  Ile Val Tyr Phe Ile  Ser Phe Tyr Met Leu  Cys Ala Phe
    1460                 1465                 1470
Leu Ile  Ile Asn Leu Phe Val  Ala Val Ile Met Asp  Asn Phe Asp
    1475                 1480                 1485
Tyr Leu  Thr Arg Asp Trp Ser  Ile Leu Gly Pro His  His Leu Asp
    1490                 1495                 1500
Glu Phe  Lys Arg Ile Trp Ser  Glu Tyr Asp Pro Glu  Ala Lys Gly
    1505                 1510                 1515
Arg Ile  Lys His Leu Asp Val  Val Thr Leu Leu Arg  Arg Ile Gln
    1520                 1525                 1530
Pro Pro  Leu Gly Phe Gly Lys  Leu Cys Pro His Arg  Val Ala Cys
    1535                 1540                 1545
Lys Arg  Leu Val Ala Met Asn  Met Pro Leu Asn Ser  Asp Gly Thr
    1550                 1555                 1560
Val Met  Phe Asn Ala Thr Leu  Phe Ala Leu Val Arg  Thr Ala Leu
    1565                 1570                 1575
Lys Ile  Lys Thr Glu Gly Asn  Leu Glu Gln Ala Asn  Glu Glu Leu
    1580                 1585                 1590
Arg Ala  Val Ile Lys Lys Ile  Trp Lys Lys Thr Ser  Met Lys Leu
    1595                 1600                 1605
Leu Asp  Gln Val Val Pro Pro  Ala Gly Asp Asp Glu  Val Thr Val
    1610                 1615                 1620
Gly Lys  Phe Tyr Ala Thr Phe  Leu Ile Gln Asp Tyr  Phe Arg Lys
    1625                 1630                 1635
Phe Lys  Lys Arg Lys Glu Gln  Gly Leu Val Gly Lys  Tyr Pro Ala
    1640                 1645                 1650
Lys Asn  Thr Thr Ile Ala Leu  Gln Ala Gly Leu Arg  Thr Leu His
    1655                 1660                 1665
Asp Ile  Gly Pro Glu Ile Arg  Arg Ala Ile Ser Cys  Asp Leu Gln
    1670                 1675                 1680
Asp Asp  Glu Pro Glu Glu Thr  Lys Arg Glu Glu Glu  Asp Asp Val
    1685                 1690                 1695
Phe Lys  Arg Asn Gly Ala Leu  Leu Gly Asn His Val  Asn His Val
    1700                 1705                 1710
Asn Ser  Asp Arg Arg Asp Ser  Leu Gln Gln Thr Asn  Thr Thr His
    1715                 1720                 1725
Arg Pro  Leu His Val Gln Arg  Pro Ser Ile Pro Pro  Ala Ser Asp
```

1730 1735 1740
Thr Glu Lys Pro Leu Phe Pro Pro Ala Gly Asn Ser Val Cys His
1745 1750 1755
Asn His His Asn His Asn Ser Ile Gly Lys Gln Val Pro Thr Ser
1760 1765 1770
Thr Asn Ala Asn Leu Asn Asn Ala Asn Met Ser Lys Ala Ala His
1775 1780 1785
Gly Lys Arg Pro Ser Ile Gly Asn Leu Glu His Val Ser Glu Asn
1790 1795 1800
Gly His His Ser Ser His Lys His Asp Arg Glu Pro Gln Arg Arg
1805 1810 1815
Ser Ser Val Lys Arg Thr Arg Tyr Tyr Glu Thr Tyr Ile Arg Ser
1820 1825 1830
Asp Ser Gly Asp Glu Gln Leu Pro Thr Ile Cys Arg Glu Asp Pro
1835 1840 1845
Glu Ile His Gly Tyr Phe Arg Asp Pro His Cys Leu Gly Glu Gln
1850 1855 1860
Glu Tyr Phe Ser Ser Glu Glu Cys Tyr Glu Asp Asp Ser Ser Pro
1865 1870 1875
Thr Trp Ser Arg Gln Asn Tyr Gly Tyr Tyr Ser Arg Tyr Pro Gly
1880 1885 1890
Arg Asn Ile Asp Ser Glu Arg Pro Arg Gly Tyr His His Pro Gln
1895 1900 1905
Gly Phe Leu Glu Asp Asp Asp Ser Pro Val Cys Tyr Asp Ser Arg
1910 1915 1920
Arg Ser Pro Arg Arg Arg Leu Leu Pro Pro Thr Pro Ala Ser His
1925 1930 1935
Arg Arg Ser Ser Phe Asn Phe Glu Cys Leu Arg Arg Gln Ser Ser
1940 1945 1950
Gln Glu Glu Val Pro Ser Ser Pro Ile Phe Pro His Arg Thr Ala
1955 1960 1965
Leu Pro Leu His Leu Met Gln Gln Gln Ile Met Ala Val Ala Gly
1970 1975 1980
Leu Asp Ser Ser Lys Ala Gln Lys Tyr Ser Pro Ser His Ser Thr
1985 1990 1995
Arg Ser Trp Ala Thr Pro Pro Ala Thr Pro Pro Tyr Arg Asp Trp
2000 2005 2010
Thr Pro Cys Tyr Thr Pro Leu Ile Gln Val Glu Gln Ser Glu Ala
2015 2020 2025
Leu Asp Gln Val Asn Gly Ser Leu Pro Ser Leu His Arg Ser Ser
2030 2035 2040
Trp Tyr Thr Asp Glu Pro Asp Ile Ser Tyr Arg Thr Phe Thr Pro
2045 2050 2055
Ala Ser Leu Thr Val Pro Ser Ser Phe Arg Asn Lys Asn Ser Asp
2060 2065 2070
Lys Gln Arg Ser Ala Asp Ser Leu Val Glu Ala Val Leu Ile Ser
2075 2080 2085
Glu Gly Leu Gly Arg Tyr Ala Arg Asp Pro Lys Phe Val Ser Ala
2090 2095 2100
Thr Lys His Glu Ile Ala Asp Ala Cys Asp Leu Thr Ile Asp Glu
2105 2110 2115
Met Glu Ser Ala Ala Ser Thr Leu Leu Asn Gly Asn Val Arg Pro
2120 2125 2130
Arg Ala Asn Gly Asp Val Gly Pro Leu Ser His Arg Gln Asp Tyr
2135 2140 2145
Glu Leu Gln Asp Phe Gly Pro Gly Tyr Ser Asp Glu Glu Pro Asp
2150 2155 2160
Pro Gly Arg Asp Glu Glu Asp Leu Ala Asp Glu Met Ile Cys Ile
2165 2170 2175
Thr Thr Leu
2180

<210> 245
<211> 377
<212> PRT
<213> Homo sapiens
<400> 245
Met Glu Leu Ser Val Leu Leu Phe Leu Ala Leu Leu Thr Gly Leu Leu
1 5 10 15

```
Leu Leu Leu Val Gln Arg His Pro Asn Ser His Gly Thr Leu Pro Pro
            20                  25                  30
Gly Pro Arg Pro Leu Pro Leu Leu Gly Asn Leu Leu Gln Met Asp Arg
        35                  40                  45
Arg Gly Leu Leu Lys Ser Phe Leu Arg Phe Arg Glu Lys Tyr Gly Asp
    50                  55                  60
Val Phe Thr Val His Leu Gly Pro Arg Pro Val Val Met Leu Cys Gly
65                  70                  75                  80
Val Glu Ala Ile Arg Glu Ala Leu Val Asp Asn Ala Glu Ala Phe Ser
                85                  90                  95
Gly Arg Gly Lys Ile Val Ile Met Asp Pro Val Tyr Gln Gly Tyr Gly
            100                 105                 110
Met Leu Phe Ala Asn Gly Asn Arg Trp Lys Val Leu Arg Arg Phe Ser
        115                 120                 125
Val Thr Thr Met Arg Asp Phe Gly Met Gly Lys Arg Ser Val Glu Glu
    130                 135                 140
Arg Ile Gln Asp Glu Ala Gln Cys Leu Ile Glu Glu Leu Arg Lys Ser
145                 150                 155                 160
Lys Gly Ala Leu Val Asp Pro Thr Phe Leu Phe His Ser Ile Thr Ala
                165                 170                 175
Asn Ile Ile Cys Ser Ile Ile Phe Gly Lys Arg Phe His Tyr Gln Asp
            180                 185                 190
Gln Glu Phe Leu Lys Thr Leu Asn Leu Phe Cys Gln Ser Phe Leu Leu
        195                 200                 205
Ile Ser Ser Ile Ser Ser Gln Leu Phe Glu Leu Phe Ser Gly Phe Leu
    210                 215                 220
Lys Tyr Phe Pro Gly Ala His Arg Gln Val Tyr Lys Asn Leu Gln Glu
225                 230                 235                 240
Ile Asn Ala Tyr Ile Gly His Ser Val Glu Lys His Arg Glu Thr Leu
                245                 250                 255
Asp Pro Ser Ala Pro Arg Asp Leu Ile Asp Thr Tyr Leu Leu His Met
            260                 265                 270
Glu Lys Glu Lys Ser Asn Pro His Ser Glu Phe Ser His Gln Asn Leu
        275                 280                 285
Ile Ile Asn Thr Leu Ser Leu Phe Phe Ala Gly Thr Glu Thr Thr Ser
    290                 295                 300
Thr Thr Leu Arg Tyr Gly Phe Leu Leu Met Leu Lys Tyr Pro His Val
305                 310                 315                 320
Ala Glu Arg Val Tyr Lys Glu Ile Glu Gln Val Val Gly Pro His Arg
                325                 330                 335
Pro Pro Ala Leu Asp Asp Arg Ala Lys Met Pro Tyr Thr Glu Ala Val
            340                 345                 350
Ile Arg Glu Ile Gln Arg Phe Ala Asp Leu Leu Pro Met Gly Val Pro
        355                 360                 365
His Ile Val Thr Gln His Thr Ser Phe
    370                 375

<210>  246
<211>  280
<212>  PRT
<213>  Homo sapiens
<400>  246
Met Ala Glu Lys Phe Asp Cys His Tyr Cys Arg Asp Pro Leu Gln Gly
1               5                   10                  15
Lys Lys Tyr Val Gln Lys Asp Gly His His Cys Cys Leu Lys Cys Phe
            20                  25                  30
Asp Lys Phe Cys Ala Asn Thr Cys Val Glu Cys Arg Lys Pro Ile Gly
        35                  40                  45
Ala Asp Ser Lys Glu Val His Tyr Lys Asn Arg Phe Trp His Asp Thr
    50                  55                  60
Cys Phe Arg Cys Ala Lys Cys Leu His Pro Leu Ala Asn Glu Thr Phe
65                  70                  75                  80
Val Ala Lys Asp Asn Lys Ile Leu Cys Asn Lys Cys Thr Thr Arg Glu
                85                  90                  95
Asp Ser Pro Lys Cys Lys Gly Cys Phe Lys Ala Ile Val Ala Gly Asp
            100                 105                 110
Gln Asn Val Glu Tyr Lys Gly Thr Val Trp His Lys Asp Cys Phe Thr
        115                 120                 125
Cys Ser Asn Cys Lys Gln Val Ile Gly Thr Gly Ser Phe Phe Pro Lys
```

130 135 140
Gly Glu Asp Phe Tyr Cys Val Thr Cys His Glu Thr Lys Phe Ala Lys
145 150 155 160
His Cys Val Lys Cys Asn Lys Ala Ile Thr Ser Gly Gly Ile Thr Tyr
165 170 175
Gln Asp Gln Pro Trp His Ala Asp Cys Phe Val Cys Val Thr Cys Ser
180 185 190
Lys Lys Leu Ala Gly Gln Arg Phe Thr Ala Val Glu Asp Gln Tyr Tyr
195 200 205
Cys Val Asp Cys Tyr Lys Asn Phe Val Ala Lys Lys Cys Ala Gly Cys
210 215 220
Lys Asn Pro Ile Thr Gly Phe Gly Lys Gly Ser Ser Val Val Ala Tyr
225 230 235 240
Glu Gly Gln Ser Trp His Asp Tyr Cys Phe His Cys Lys Lys Cys Ser
245 250 255
Val Asn Leu Ala Asn Lys Arg Phe Val Phe His Gln Glu Gln Val Tyr
260 265 270
Cys Pro Asp Cys Ala Lys Lys Leu
275 280

<210> 247
<211> 267
<212> PRT
<213> Homo sapiens
<400> 247
Met Ala Ser Pro Ser Lys Gly Asn Asp Leu Phe Ser Pro Asp Glu Glu
1 5 10 15
Gly Pro Ala Val Val Ala Gly Pro Gly Pro Gly Pro Gly Gly Ala Glu
20 25 30
Gly Ala Ala Glu Glu Arg Arg Val Lys Val Ser Ser Leu Pro Phe Ser
35 40 45
Val Glu Ala Leu Met Ser Asp Lys Lys Pro Pro Lys Glu Ala Ser Pro
50 55 60
Leu Pro Ala Glu Ser Ala Ser Ala Gly Ala Thr Leu Arg Pro Leu Leu
65 70 75 80
Leu Ser Gly His Gly Ala Arg Glu Ala His Ser Pro Gly Pro Leu Val
85 90 95
Lys Pro Phe Glu Thr Ala Ser Val Lys Ser Glu Asn Ser Glu Asp Gly
100 105 110
Ala Ala Trp Met Gln Glu Pro Gly Arg Tyr Ser Pro Pro Pro Arg His
115 120 125
Met Ser Pro Thr Thr Cys Thr Leu Arg Lys His Lys Thr Asn Arg Lys
130 135 140
Pro Arg Thr Pro Phe Thr Thr Ser Gln Leu Leu Ala Leu Glu Arg Lys
145 150 155 160
Phe Arg Gln Lys Gln Tyr Leu Ser Ile Ala Glu Arg Ala Glu Phe Ser
165 170 175
Ser Ser Leu Asn Leu Thr Glu Thr Gln Val Lys Ile Trp Phe Gln Asn
180 185 190
Arg Arg Ala Lys Ala Lys Arg Leu Gln Glu Ala Glu Leu Glu Lys Leu
195 200 205
Lys Met Ala Ala Lys Pro Met Leu Pro Ser Ser Phe Ser Leu Pro Phe
210 215 220
Pro Ile Ser Ser Pro Leu Gln Ala Ala Ser Ile Tyr Gly Ala Ser Tyr
225 230 235 240
Pro Phe His Arg Pro Val Leu Pro Ile Pro Pro Val Gly Leu Tyr Ala
245 250 255
Thr Pro Val Gly Tyr Gly Met Tyr His Leu Ser
260 265

<210> 248
<211> 387
<212> PRT
<213> Homo sapiens
<400> 248
Met Pro Gly His Leu Gln Glu Gly Phe Gly Cys Val Val Thr Asn Arg
1 5 10 15
Phe Asp Gln Leu Phe Asp Asp Glu Ser Asp Pro Phe Glu Val Leu Lys
20 25 30

Ala Ala Glu Asn Lys Lys Lys Glu Ala Gly Gly Gly Gly Val Gly Gly
35 40 45
Pro Gly Ala Lys Ser Ala Ala Gln Ala Ala Ala Gln Thr Asn Ser Asn
50 55 60
Ala Ala Gly Lys Gln Leu Arg Lys Glu Ser Gln Lys Asp Arg Lys Asn
65 70 75 80
Pro Leu Pro Pro Ser Val Gly Val Val Asp Lys Lys Glu Glu Thr Gln
85 90 95
Pro Pro Val Ala Leu Lys Lys Glu Gly Ile Arg Arg Val Gly Arg Arg
100 105 110
Pro Asp Gln Gln Leu Gln Gly Glu Gly Lys Ile Ile Asp Arg Arg Pro
115 120 125
Glu Arg Arg Pro Pro Arg Glu Arg Arg Phe Glu Lys Pro Leu Glu Glu
130 135 140
Lys Gly Glu Gly Gly Glu Phe Ser Val Asp Arg Pro Ile Ile Asp Arg
145 150 155 160
Pro Ile Arg Gly Arg Gly Gly Leu Gly Arg Gly Arg Gly Gly Arg Gly
165 170 175
Arg Gly Met Gly Arg Gly Asp Gly Phe Asp Ser Arg Gly Lys Arg Glu
180 185 190
Phe Asp Arg His Ser Gly Ser Asp Arg Ser Gly Leu Lys His Glu Asp
195 200 205
Lys Arg Gly Gly Ser Gly Ser His Asn Trp Gly Thr Val Lys Asp Glu
210 215 220
Leu Thr Asp Leu Asp Gln Ser Asn Val Thr Glu Glu Thr Pro Glu Gly
225 230 235 240
Glu Glu His His Pro Val Ala Asp Thr Glu Asn Lys Glu Asn Glu Val
245 250 255
Glu Glu Val Lys Glu Glu Gly Pro Lys Glu Met Thr Leu Asp Glu Trp
260 265 270
Lys Ala Ile Gln Asn Lys Asp Arg Ala Lys Val Glu Phe Asn Ile Arg
275 280 285
Lys Pro Asn Glu Gly Ala Asp Gly Gln Trp Lys Lys Gly Phe Val Leu
290 295 300
His Lys Ser Lys Ser Glu Glu Ala His Ala Glu Asp Ser Val Met Asp
305 310 315 320
His His Phe Arg Lys Pro Ala Asn Asp Ile Thr Ser Gln Leu Glu Ile
325 330 335
Asn Phe Gly Asp Leu Gly Arg Pro Gly Arg Gly Gly Arg Gly Gly Arg
340 345 350
Gly Gly Arg Gly Arg Gly Gly Arg Pro Asn Arg Gly Ser Arg Thr Asp
355 360 365
Lys Ser Ser Ala Ser Ala Pro Asp Val Asp Asp Pro Glu Ala Phe Pro
370 375 380
Ala Leu Ala
385

<210> 249
<211> 239
<212> PRT
<213> Homo sapiens
<400> 249
Met Ala Ser Thr Ala Val Gln Leu Leu Gly Phe Leu Leu Ser Phe Leu
1 5 10 15
Gly Met Val Gly Thr Leu Ile Thr Thr Ile Leu Pro His Trp Arg Arg
20 25 30
Thr Ala His Val Gly Thr Asn Ile Leu Thr Ala Val Ser Tyr Leu Lys
35 40 45
Gly Leu Trp Met Glu Cys Val Trp His Ser Thr Gly Ile Tyr Gln Cys
50 55 60
Gln Ile Tyr Arg Ser Leu Leu Ala Leu Pro Gln Asp Leu Gln Ala Ala
65 70 75 80
Arg Ala Leu Met Val Ile Ser Cys Leu Leu Ser Gly Ile Ala Cys Ala
85 90 95
Cys Ala Val Ile Gly Met Lys Cys Thr Arg Cys Ala Lys Gly Thr Pro
100 105 110
Ala Lys Thr Thr Phe Ala Ile Leu Gly Gly Thr Leu Phe Ile Leu Ala
115 120 125
Gly Leu Leu Cys Met Val Ala Val Ser Trp Thr Thr Asn Asp Val Val

```
    130                 135                 140
Gln Asn Phe Tyr Asn Pro Leu Leu Pro Ser Gly Met Lys Phe Glu Ile
145                 150                 155                 160
Gly Gln Ala Leu Tyr Leu Gly Phe Ile Ser Ser Ser Leu Ser Leu Ile
                165                 170                 175
Gly Gly Thr Leu Leu Cys Leu Ser Cys Gln Asp Glu Ala Pro Tyr Arg
            180                 185                 190
Pro Tyr Gln Ala Pro Pro Arg Ala Thr Thr Thr Thr Ala Asn Thr Ala
        195                 200                 205
Pro Ala Tyr Gln Pro Pro Ala Ala Tyr Lys Asp Asn Arg Ala Pro Ser
    210                 215                 220
Val Thr Ser Ala Thr His Ser Gly Tyr Arg Leu Asn Asp Tyr Val
225                 230                 235
```

```
<210>  250
<211>  414
<212>  PRT
<213>  Homo sapiens
<400>  250
Met Gln Thr Phe Leu Lys Gly Lys Arg Val Gly Tyr Trp Leu Ser Glu
1               5                   10                  15
Lys Lys Ile Lys Lys Leu Asn Phe Gln Ala Phe Ala Glu Leu Cys Arg
            20                  25                  30
Lys Arg Gly Met Glu Val Val Gln Leu Asn Leu Ser Arg Pro Ile Glu
        35                  40                  45
Glu Gln Gly Pro Leu Asp Val Ile Ile His Lys Leu Thr Asp Val Ile
    50                  55                  60
Leu Glu Ala Asp Gln Asn Asp Ser Gln Ser Leu Glu Leu Val His Arg
65                  70                  75                  80
Phe Gln Glu Tyr Ile Asp Ala His Pro Glu Thr Ile Val Leu Asp Pro
                85                  90                  95
Leu Pro Ala Ile Arg Thr Leu Leu Asp Arg Ser Lys Ser Tyr Glu Leu
            100                 105                 110
Ile Arg Lys Ile Glu Ala Tyr Met Glu Asp Asp Arg Ile Cys Ser Pro
        115                 120                 125
Pro Phe Met Glu Leu Thr Ser Leu Cys Gly Asp Asp Thr Met Arg Leu
    130                 135                 140
Leu Glu Lys Asn Gly Leu Thr Phe Pro Phe Ile Cys Lys Thr Arg Val
145                 150                 155                 160
Ala His Gly Thr Asn Ser His Glu Met Ala Ile Val Phe Asn Gln Glu
                165                 170                 175
Gly Leu Asn Ala Ile Gln Pro Pro Cys Val Val Gln Asn Phe Ile Asn
            180                 185                 190
His Asn Ala Val Leu Tyr Lys Val Phe Val Val Gly Glu Ser Tyr Thr
        195                 200                 205
Val Val Gln Arg Pro Ser Leu Lys Asn Phe Ser Ala Gly Thr Ser Asp
    210                 215                 220
Arg Glu Ser Ile Phe Phe Asn Ser His Asn Val Ser Lys Pro Glu Ser
225                 230                 235                 240
Ser Ser Val Leu Thr Glu Leu Asp Lys Ile Glu Gly Val Phe Glu Arg
                245                 250                 255
Pro Ser Asp Glu Val Ile Arg Glu Leu Ser Arg Ala Leu Arg Gln Ala
            260                 265                 270
Leu Gly Val Ser Leu Phe Gly Ile Asp Ile Ile Ile Asn Asn Gln Thr
        275                 280                 285
Gly Gln His Ala Val Ile Asp Ile Asn Ala Phe Pro Gly Tyr Glu Gly
    290                 295                 300
Val Ser Glu Phe Phe Thr Asp Leu Leu Asn His Ile Ala Thr Val Leu
305                 310                 315                 320
Gln Gly Gln Ser Thr Ala Met Ala Ala Thr Gly Asp Val Ala Leu Leu
                325                 330                 335
Arg His Ser Lys Leu Leu Ala Glu Pro Ala Gly Gly Leu Val Gly Glu
            340                 345                 350
Arg Thr Cys Ser Ala Ser Pro Gly Cys Cys Gly Ser Met Met Gly Gln
        355                 360                 365
Asp Ala Pro Trp Lys Ala Glu Ala Asp Ala Gly Gly Thr Ala Lys Leu
    370                 375                 380
Pro His Gln Arg Leu Gly Cys Asn Ala Gly Val Ser Pro Ser Phe Gln
385                 390                 395                 400
```

Gln His Cys Val Ala Ser Leu Ala Thr Lys Ala Ser Ser Gln
405 410

<210> 251
<211> 1255
<212> PRT
<213> Homo sapiens
<400> 251
Met Glu Leu Ala Ala Leu Cys Arg Trp Gly Leu Leu Leu Ala Leu Leu
1 5 10 15
Pro Pro Gly Ala Ala Ser Thr Gln Val Cys Thr Gly Thr Asp Met Lys
20 25 30
Leu Arg Leu Pro Ala Ser Pro Glu Thr His Leu Asp Met Leu Arg His
35 40 45
Leu Tyr Gln Gly Cys Gln Val Val Gln Gly Asn Leu Glu Leu Thr Tyr
50 55 60
Leu Pro Thr Asn Ala Ser Leu Ser Phe Leu Gln Asp Ile Gln Glu Val
65 70 75 80
Gln Gly Tyr Val Leu Ile Ala His Asn Gln Val Arg Gln Val Pro Leu
85 90 95
Gln Arg Leu Arg Ile Val Arg Gly Thr Gln Leu Phe Glu Asp Asn Tyr
100 105 110
Ala Leu Ala Val Leu Asp Asn Gly Asp Pro Leu Asn Asn Thr Thr Pro
115 120 125
Val Thr Gly Ala Ser Pro Gly Gly Leu Arg Glu Leu Gln Leu Arg Ser
130 135 140
Leu Thr Glu Ile Leu Lys Gly Gly Val Leu Ile Gln Arg Asn Pro Gln
145 150 155 160
Leu Cys Tyr Gln Asp Thr Ile Leu Trp Lys Asp Ile Phe His Lys Asn
165 170 175
Asn Gln Leu Ala Leu Thr Leu Ile Asp Thr Asn Arg Ser Arg Ala Cys
180 185 190
His Pro Cys Ser Pro Met Cys Lys Gly Ser Arg Cys Trp Gly Glu Ser
195 200 205
Ser Glu Asp Cys Gln Ser Leu Thr Arg Thr Val Cys Ala Gly Gly Cys
210 215 220
Ala Arg Cys Lys Gly Pro Leu Pro Thr Asp Cys Cys His Glu Gln Cys
225 230 235 240
Ala Ala Gly Cys Thr Gly Pro Lys His Ser Asp Cys Leu Ala Cys Leu
245 250 255
His Phe Asn His Ser Gly Ile Cys Glu Leu His Cys Pro Ala Leu Val
260 265 270
Thr Tyr Asn Thr Asp Thr Phe Glu Ser Met Pro Asn Pro Glu Gly Arg
275 280 285
Tyr Thr Phe Gly Ala Ser Cys Val Thr Ala Cys Pro Tyr Asn Tyr Leu
290 295 300
Ser Thr Asp Val Gly Ser Cys Thr Leu Val Cys Pro Leu His Asn Gln
305 310 315 320
Glu Val Thr Ala Glu Asp Gly Thr Gln Arg Cys Glu Lys Cys Ser Lys
325 330 335
Pro Cys Ala Arg Val Cys Tyr Gly Leu Gly Met Glu His Leu Arg Glu
340 345 350
Val Arg Ala Val Thr Ser Ala Asn Ile Gln Glu Phe Ala Gly Cys Lys
355 360 365
Lys Ile Phe Gly Ser Leu Ala Phe Leu Pro Glu Ser Phe Asp Gly Asp
370 375 380
Pro Ala Ser Asn Thr Ala Pro Leu Gln Pro Glu Gln Leu Gln Val Phe
385 390 395 400
Glu Thr Leu Glu Glu Ile Thr Gly Tyr Leu Tyr Ile Ser Ala Trp Pro
405 410 415
Asp Ser Leu Pro Asp Leu Ser Val Phe Gln Asn Leu Gln Val Ile Arg
420 425 430
Gly Arg Ile Leu His Asn Gly Ala Tyr Ser Leu Thr Leu Gln Gly Leu
435 440 445
Gly Ile Ser Trp Leu Gly Leu Arg Ser Leu Arg Glu Leu Gly Ser Gly
450 455 460
Leu Ala Leu Ile His His Asn Thr His Leu Cys Phe Val His Thr Val
465 470 475 480
Pro Trp Asp Gln Leu Phe Arg Asn Pro His Gln Ala Leu Leu His Thr

485 490 495
Ala Asn Arg Pro Glu Asp Glu Cys Val Gly Glu Gly Leu Ala Cys His
500 505 510
Gln Leu Cys Ala Arg Gly His Cys Trp Gly Pro Gly Pro Thr Gln Cys
515 520 525
Val Asn Cys Ser Gln Phe Leu Arg Gly Gln Glu Cys Val Glu Glu Cys
530 535 540
Arg Val Leu Gln Gly Leu Pro Arg Glu Tyr Val Asn Ala Arg His Cys
545 550 555 560
Leu Pro Cys His Pro Glu Cys Gln Pro Gln Asn Gly Ser Val Thr Cys
565 570 575
Phe Gly Pro Glu Ala Asp Gln Cys Val Ala Cys Ala His Tyr Lys Asp
580 585 590
Pro Pro Phe Cys Val Ala Arg Cys Pro Ser Gly Val Lys Pro Asp Leu
595 600 605
Ser Tyr Met Pro Ile Trp Lys Phe Pro Asp Glu Glu Gly Ala Cys Gln
610 615 620
Pro Cys Pro Ile Asn Cys Thr His Ser Cys Val Asp Leu Asp Asp Lys
625 630 635 640
Gly Cys Pro Ala Glu Gln Arg Ala Ser Pro Leu Thr Ser Ile Val Ser
645 650 655
Ala Val Val Gly Ile Leu Leu Val Val Val Leu Gly Val Val Phe Gly
660 665 670
Ile Leu Ile Lys Arg Arg Gln Gln Lys Ile Arg Lys Tyr Thr Met Arg
675 680 685
Arg Leu Leu Gln Glu Thr Glu Leu Val Glu Pro Leu Thr Pro Ser Gly
690 695 700
Ala Met Pro Asn Gln Ala Gln Met Arg Ile Leu Lys Glu Thr Glu Leu
705 710 715 720
Arg Lys Val Lys Val Leu Gly Ser Gly Ala Phe Gly Thr Val Tyr Lys
725 730 735
Gly Ile Trp Ile Pro Asp Gly Glu Asn Val Lys Ile Pro Val Ala Ile
740 745 750
Lys Val Leu Arg Glu Asn Thr Ser Pro Lys Ala Asn Lys Glu Ile Leu
755 760 765
Asp Glu Ala Tyr Val Met Ala Gly Val Gly Ser Pro Tyr Val Ser Arg
770 775 780
Leu Leu Gly Ile Cys Leu Thr Ser Thr Val Gln Leu Val Thr Gln Leu
785 790 795 800
Met Pro Tyr Gly Cys Leu Leu Asp His Val Arg Glu Asn Arg Gly Arg
805 810 815
Leu Gly Ser Gln Asp Leu Leu Asn Trp Cys Met Gln Ile Ala Lys Gly
820 825 830
Met Ser Tyr Leu Glu Asp Val Arg Leu Val His Arg Asp Leu Ala Ala
835 840 845
Arg Asn Val Leu Val Lys Ser Pro Asn His Val Lys Ile Thr Asp Phe
850 855 860
Gly Leu Ala Arg Leu Leu Asp Ile Asp Glu Thr Glu Tyr His Ala Asp
865 870 875 880
Gly Gly Lys Val Pro Ile Lys Trp Met Ala Leu Glu Ser Ile Leu Arg
885 890 895
Arg Arg Phe Thr His Gln Ser Asp Val Trp Ser Tyr Gly Val Thr Val
900 905 910
Trp Glu Leu Met Thr Phe Gly Ala Lys Pro Tyr Asp Gly Ile Pro Ala
915 920 925
Arg Glu Ile Pro Asp Leu Leu Glu Lys Gly Glu Arg Leu Pro Gln Pro
930 935 940
Pro Ile Cys Thr Ile Asp Val Tyr Met Ile Met Val Lys Cys Trp Met
945 950 955 960
Ile Asp Ser Glu Cys Arg Pro Arg Phe Arg Glu Leu Val Ser Glu Phe
965 970 975
Ser Arg Met Ala Arg Asp Pro Gln Arg Phe Val Val Ile Gln Asn Glu
980 985 990
Asp Leu Gly Pro Ala Ser Pro Leu Asp Ser Thr Phe Tyr Arg Ser Leu
995 1000 1005
Leu Glu Asp Asp Asp Met Gly Asp Leu Val Asp Ala Glu Glu Tyr
1010 1015 1020
Leu Val Pro Gln Gln Gly Phe Phe Cys Pro Asp Pro Ala Pro Gly
1025 1030 1035

Ala Gly Gly Met Val His His Arg His Arg Ser Ser Ser Thr Arg
1040 1045 1050
Ser Gly Gly Gly Asp Leu Thr Leu Gly Leu Glu Pro Ser Glu Glu
1055 1060 1065
Glu Ala Pro Arg Ser Pro Leu Ala Pro Ser Glu Gly Ala Gly Ser
1070 1075 1080
Asp Val Phe Asp Gly Asp Leu Gly Met Gly Ala Ala Lys Gly Leu
1085 1090 1095
Gln Ser Leu Pro Thr His Asp Pro Ser Pro Leu Gln Arg Tyr Ser
1100 1105 1110
Glu Asp Pro Thr Val Pro Leu Pro Ser Glu Thr Asp Gly Tyr Val
1115 1120 1125
Ala Pro Leu Thr Cys Ser Pro Gln Pro Glu Tyr Val Asn Gln Pro
1130 1135 1140
Asp Val Arg Pro Gln Pro Pro Ser Pro Arg Glu Gly Pro Leu Pro
1145 1150 1155
Ala Ala Arg Pro Ala Gly Ala Thr Leu Glu Arg Ala Lys Thr Leu
1160 1165 1170
Ser Pro Gly Lys Asn Gly Val Val Lys Asp Val Phe Ala Phe Gly
1175 1180 1185
Gly Ala Val Glu Asn Pro Glu Tyr Leu Thr Pro Gln Gly Gly Ala
1190 1195 1200
Ala Pro Gln Pro His Pro Pro Pro Ala Phe Ser Pro Ala Phe Asp
1205 1210 1215
Asn Leu Tyr Tyr Trp Asp Gln Asp Pro Pro Glu Arg Gly Ala Pro
1220 1225 1230
Pro Ser Thr Phe Lys Gly Thr Pro Thr Ala Glu Asn Pro Glu Tyr
1235 1240 1245
Leu Gly Leu Asp Val Pro Val
1250 1255

<210> 252
<211> 393
<212> PRT
<213> Homo sapiens
<400> 252
Met Glu Glu Pro Gln Ser Asp Pro Ser Val Glu Pro Pro Leu Ser Gln
1 5 10 15
Glu Thr Phe Ser Asp Leu Trp Lys Leu Leu Pro Glu Asn Asn Val Leu
20 25 30
Ser Pro Leu Pro Ser Gln Ala Met Asp Asp Leu Met Leu Ser Pro Asp
35 40 45
Asp Ile Glu Gln Trp Phe Thr Glu Asp Pro Gly Pro Asp Glu Ala Pro
50 55 60
Arg Met Pro Glu Ala Ala Pro Arg Val Ala Pro Ala Pro Ala Ala Pro
65 70 75 80
Thr Pro Ala Ala Pro Ala Pro Ala Pro Ser Trp Pro Leu Ser Ser Ser
85 90 95
Val Pro Ser Gln Lys Thr Tyr Gln Gly Ser Tyr Gly Phe Arg Leu Gly
100 105 110
Phe Leu His Ser Gly Thr Ala Lys Ser Val Thr Cys Thr Tyr Ser Pro
115 120 125
Ala Leu Asn Lys Met Phe Cys Gln Leu Ala Lys Thr Cys Pro Val Gln
130 135 140
Leu Trp Val Asp Ser Thr Pro Pro Pro Gly Thr Arg Val Arg Ala Met
145 150 155 160
Ala Ile Tyr Lys Gln Ser Gln His Met Thr Glu Val Val Arg Arg Cys
165 170 175
Pro His His Glu Arg Cys Ser Asp Ser Asp Gly Leu Ala Pro Pro Gln
180 185 190
His Leu Ile Arg Val Glu Gly Asn Leu Arg Val Glu Tyr Leu Asp Asp
195 200 205
Arg Asn Thr Phe Arg His Ser Val Val Val Pro Tyr Glu Pro Pro Glu
210 215 220
Val Gly Ser Asp Cys Thr Thr Ile His Tyr Asn Tyr Met Cys Asn Ser
225 230 235 240
Ser Cys Met Gly Gly Met Asn Arg Arg Pro Ile Leu Thr Ile Ile Thr
245 250 255
Leu Glu Asp Ser Ser Gly Asn Leu Leu Gly Arg Asn Ser Phe Glu Val

```
            260                 265                 270
Arg Val Cys Ala Cys Pro Gly Arg Asp Arg Arg Thr Glu Glu Glu Asn
        275                 280                 285
Leu Arg Lys Lys Gly Glu Pro His His Glu Leu Pro Pro Gly Ser Thr
    290                 295                 300
Lys Arg Ala Leu Pro Asn Asn Thr Ser Ser Ser Pro Gln Pro Lys Lys
305                 310                 315                 320
Lys Pro Leu Asp Gly Glu Tyr Phe Thr Leu Gln Ile Arg Gly Arg Glu
                325                 330                 335
Arg Phe Glu Met Phe Arg Glu Leu Asn Glu Ala Leu Glu Leu Lys Asp
            340                 345                 350
Ala Gln Ala Gly Lys Glu Pro Gly Gly Ser Arg Ala His Ser Ser His
        355                 360                 365
Leu Lys Ser Lys Lys Gly Gln Ser Thr Ser Arg His Lys Lys Leu Met
    370                 375                 380
Phe Lys Thr Glu Gly Pro Asp Ser Asp
385                 390

<210>  253
<211>  639
<212>  PRT
<213>  Homo sapiens
<400>  253
Met Ser Ala Arg Gly Pro Ala Ile Gly Ile Asp Leu Gly Thr Thr Tyr
1               5                   10                  15
Ser Cys Val Gly Val Phe Gln His Gly Lys Val Glu Ile Ile Ala Asn
            20                  25                  30
Asp Gln Gly Asn Arg Thr Thr Pro Ser Tyr Val Ala Phe Thr Asp Thr
        35                  40                  45
Glu Arg Leu Ile Gly Asp Ala Ala Lys Asn Gln Val Ala Met Asn Pro
    50                  55                  60
Thr Asn Thr Ile Phe Asp Ala Lys Arg Leu Ile Gly Arg Lys Phe Glu
65                  70                  75                  80
Asp Ala Thr Val Gln Ser Asp Met Lys His Trp Pro Phe Arg Val Val
                85                  90                  95
Ser Glu Gly Gly Lys Pro Lys Val Gln Val Glu Tyr Lys Gly Glu Thr
            100                 105                 110
Lys Thr Phe Phe Pro Glu Glu Ile Ser Ser Met Val Leu Thr Lys Met
        115                 120                 125
Lys Glu Ile Ala Glu Ala Tyr Leu Gly Gly Lys Val His Ser Ala Val
    130                 135                 140
Ile Thr Val Pro Ala Tyr Phe Asn Asp Ser Gln Arg Gln Ala Thr Lys
145                 150                 155                 160
Asp Ala Gly Thr Ile Thr Gly Leu Asn Val Leu Arg Ile Ile Asn Glu
                165                 170                 175
Pro Thr Ala Ala Ala Ile Ala Tyr Gly Leu Asp Lys Lys Gly Cys Ala
            180                 185                 190
Gly Gly Glu Lys Asn Val Leu Ile Phe Asp Leu Gly Gly Gly Thr Phe
        195                 200                 205
Asp Val Ser Ile Leu Thr Ile Glu Asp Gly Ile Phe Glu Val Lys Ser
    210                 215                 220
Thr Ala Gly Asp Thr His Leu Gly Gly Glu Asp Phe Asp Asn Arg Met
225                 230                 235                 240
Val Ser His Leu Ala Glu Glu Phe Lys Arg Lys His Lys Lys Asp Ile
                245                 250                 255
Gly Pro Asn Lys Arg Ala Val Arg Arg Leu Arg Thr Ala Cys Glu Arg
            260                 265                 270
Ala Lys Arg Thr Leu Ser Ser Ser Thr Gln Ala Ser Ile Glu Ile Asp
        275                 280                 285
Ser Leu Tyr Glu Gly Val Asp Phe Tyr Thr Ser Ile Thr Arg Ala Arg
    290                 295                 300
Phe Glu Glu Leu Asn Ala Asp Leu Phe Arg Gly Thr Leu Glu Pro Val
305                 310                 315                 320
Glu Lys Ala Leu Arg Asp Ala Lys Leu Asp Lys Gly Gln Ile Gln Glu
                325                 330                 335
Ile Val Leu Val Gly Gly Ser Thr Arg Ile Pro Lys Ile Gln Lys Leu
            340                 345                 350
Leu Gln Asp Phe Phe Asn Gly Lys Glu Leu Asn Lys Ser Ile Asn Pro
        355                 360                 365
```

Asp Glu Ala Val Ala Tyr Gly Ala Ala Val Gln Ala Ala Ile Leu Ile
370 375 380
Gly Asp Lys Ser Glu Asn Val Gln Asp Leu Leu Leu Leu Asp Val Thr
385 390 395 400
Pro Leu Ser Leu Gly Ile Glu Thr Ala Gly Gly Val Met Thr Pro Leu
405 410 415
Ile Lys Arg Asn Thr Thr Ile Pro Thr Lys Gln Thr Gln Thr Phe Thr
420 425 430
Thr Tyr Ser Asp Asn Gln Ser Ser Val Leu Val Gln Val Tyr Glu Gly
435 440 445
Glu Arg Ala Met Thr Lys Asp Asn Asn Leu Leu Gly Lys Phe Asp Leu
450 455 460
Thr Gly Ile Pro Pro Ala Pro Arg Gly Val Pro Gln Ile Glu Val Thr
465 470 475 480
Phe Asp Ile Asp Ala Asn Gly Ile Leu Asn Val Thr Ala Ala Asp Lys
485 490 495
Ser Thr Gly Lys Glu Asn Lys Ile Thr Ile Thr Asn Asp Lys Gly Arg
500 505 510
Leu Ser Lys Asp Asp Ile Asp Arg Met Val Gln Glu Ala Glu Arg Tyr
515 520 525
Lys Ser Glu Asp Glu Ala Asn Arg Asp Arg Val Ala Ala Lys Asn Ala
530 535 540
Leu Glu Ser Tyr Thr Tyr Asn Ile Lys Gln Thr Val Glu Asp Glu Lys
545 550 555 560
Leu Arg Gly Lys Ile Ser Glu Gln Asp Lys Asn Lys Ile Leu Asp Lys
565 570 575
Cys Gln Glu Val Ile Asn Trp Leu Asp Arg Asn Gln Met Ala Glu Lys
580 585 590
Asp Glu Tyr Glu His Lys Gln Lys Glu Leu Glu Arg Val Cys Asn Pro
595 600 605
Ile Ile Ser Lys Leu Tyr Gln Gly Gly Pro Gly Gly Gly Ser Gly Gly
610 615 620
Gly Gly Ser Gly Ala Ser Gly Gly Pro Thr Ile Glu Glu Val Asp
625 630 635

<210> 254
<211> 1094
<212> PRT
<213> Homo sapiens
<400> 254
Met Ala Arg Pro Val Arg Gly Gly Leu Gly Ala Pro Arg Arg Ser Pro
1 5 10 15
Cys Leu Leu Leu Leu Trp Leu Leu Leu Leu Arg Leu Glu Pro Val Thr
20 25 30
Ala Ala Ala Gly Pro Arg Ala Pro Cys Ala Ala Ala Cys Thr Cys Ala
35 40 45
Gly Asp Ser Leu Asp Cys Gly Gly Arg Gly Leu Ala Ala Leu Pro Gly
50 55 60
Asp Leu Pro Ser Trp Thr Arg Ser Leu Asn Leu Ser Tyr Asn Lys Leu
65 70 75 80
Ser Glu Ile Asp Pro Ala Gly Phe Glu Asp Leu Pro Asn Leu Gln Glu
85 90 95
Val Tyr Leu Asn Asn Asn Glu Leu Thr Ala Val Pro Ser Leu Gly Ala
100 105 110
Ala Ser Ser His Val Val Ser Leu Phe Leu Gln His Asn Lys Ile Arg
115 120 125
Ser Val Glu Gly Ser Gln Leu Lys Ala Tyr Leu Ser Leu Glu Val Leu
130 135 140
Asp Leu Ser Leu Asn Asn Ile Thr Glu Val Arg Asn Thr Cys Phe Pro
145 150 155 160
His Gly Pro Pro Ile Lys Glu Leu Asn Leu Ala Gly Asn Arg Ile Gly
165 170 175
Thr Leu Glu Leu Gly Ala Phe Asp Gly Leu Ser Arg Ser Leu Leu Thr
180 185 190
Leu Arg Leu Ser Lys Asn Arg Ile Thr Gln Leu Pro Val Arg Ala Phe
195 200 205
Lys Leu Pro Arg Leu Thr Gln Leu Asp Leu Asn Arg Asn Arg Ile Arg
210 215 220
Leu Ile Glu Gly Leu Thr Phe Gln Gly Leu Asn Ser Leu Glu Val Leu

225 230 235 240
Lys Leu Gln Arg Asn Asn Ile Ser Lys Leu Thr Asp Gly Ala Phe Trp
245 250 255
Gly Leu Ser Lys Met His Val Leu His Leu Glu Tyr Asn Ser Leu Val
260 265 270
Glu Val Asn Ser Gly Ser Leu Tyr Gly Leu Thr Ala Leu His Gln Leu
275 280 285
His Leu Ser Asn Asn Ser Ile Ala Arg Ile His Arg Lys Gly Trp Ser
290 295 300
Phe Cys Gln Lys Leu His Glu Leu Val Leu Ser Phe Asn Asn Leu Thr
305 310 315 320
Arg Leu Asp Glu Glu Ser Leu Ala Glu Leu Ser Ser Leu Ser Val Leu
325 330 335
Arg Leu Ser His Asn Ser Ile Ser His Ile Ala Glu Gly Ala Phe Lys
340 345 350
Gly Leu Arg Ser Leu Arg Val Leu Asp Leu Asp His Asn Glu Ile Ser
355 360 365
Gly Thr Ile Glu Asp Thr Ser Gly Ala Phe Ser Gly Leu Asp Ser Leu
370 375 380
Ser Lys Leu Thr Leu Phe Gly Asn Lys Ile Lys Ser Val Ala Lys Arg
385 390 395 400
Ala Phe Ser Gly Leu Glu Gly Leu Glu His Leu Asn Leu Gly Gly Asn
405 410 415
Ala Ile Arg Ser Val Gln Phe Asp Ala Phe Val Lys Met Lys Asn Leu
420 425 430
Lys Glu Leu His Ile Ser Ser Asp Ser Phe Leu Cys Asp Cys Gln Leu
435 440 445
Lys Trp Leu Pro Pro Trp Leu Ile Gly Arg Met Leu Gln Ala Phe Val
450 455 460
Thr Ala Thr Cys Ala His Pro Glu Ser Leu Lys Gly Gln Ser Ile Phe
465 470 475 480
Ser Val Pro Pro Glu Ser Phe Val Cys Asp Asp Phe Leu Lys Pro Gln
485 490 495
Ile Ile Thr Gln Pro Glu Thr Thr Met Ala Met Val Gly Lys Asp Ile
500 505 510
Arg Phe Thr Cys Ser Ala Ala Ser Ser Ser Ser Ser Pro Met Thr Phe
515 520 525
Ala Trp Lys Lys Asp Asn Glu Val Leu Thr Asn Ala Asp Met Glu Asn
530 535 540
Phe Val His Val His Ala Gln Asp Gly Glu Val Met Glu Tyr Thr Thr
545 550 555 560
Ile Leu His Leu Arg Gln Val Thr Phe Gly His Glu Gly Arg Tyr Gln
565 570 575
Cys Val Ile Thr Asn His Phe Gly Ser Thr Tyr Ser His Lys Ala Arg
580 585 590
Leu Thr Val Asn Val Leu Pro Ser Phe Thr Lys Thr Pro His Asp Ile
595 600 605
Thr Ile Arg Thr Thr Thr Met Ala Arg Leu Glu Cys Ala Ala Thr Gly
610 615 620
His Pro Asn Pro Gln Ile Ala Trp Gln Lys Asp Gly Gly Thr Asp Phe
625 630 635 640
Pro Ala Ala Arg Glu Arg Arg Met His Val Met Pro Asp Asp Asp Val
645 650 655
Phe Phe Ile Thr Asp Val Lys Ile Asp Asp Ala Gly Val Tyr Ser Cys
660 665 670
Thr Ala Gln Asn Ser Ala Gly Ser Ile Ser Ala Asn Ala Thr Leu Thr
675 680 685
Val Leu Glu Thr Pro Ser Leu Val Val Pro Leu Glu Asp Arg Val Val
690 695 700
Ser Val Gly Glu Thr Val Ala Leu Gln Cys Lys Ala Thr Gly Asn Pro
705 710 715 720
Pro Pro Arg Ile Thr Trp Phe Lys Gly Asp Arg Pro Leu Ser Leu Thr
725 730 735
Glu Arg His His Leu Thr Pro Asp Asn Gln Leu Leu Val Val Gln Asn
740 745 750
Val Val Ala Glu Asp Ala Gly Arg Tyr Thr Cys Glu Met Ser Asn Thr
755 760 765
Leu Gly Thr Glu Arg Ala His Ser Gln Leu Ser Val Leu Pro Ala Ala
770 775 780

Gly Cys Arg Lys Asp Gly Thr Thr Val Gly Ile Phe Thr Ile Ala Val
785 790 795 800
Val Ser Ser Ile Val Leu Thr Ser Leu Val Trp Val Cys Ile Ile Tyr
805 810 815
Gln Thr Arg Lys Lys Ser Glu Glu Tyr Ser Val Thr Asn Thr Asp Glu
820 825 830
Thr Val Val Pro Pro Asp Val Pro Ser Tyr Leu Ser Ser Gln Gly Thr
835 840 845
Leu Ser Asp Arg Gln Glu Thr Val Val Arg Thr Glu Gly Ala Gly Pro
850 855 860
Gln Ala Asn Gly His Ile Glu Ser Asn Gly Val Cys Pro Arg Asp Ala
865 870 875 880
Ser His Phe Pro Glu Pro Asp Thr His Ser Val Ala Cys Arg Gln Pro
885 890 895
Lys Leu Cys Ala Gly Ser Ala Tyr His Lys Glu Pro Trp Lys Ala Ile
900 905 910
Glu Lys Ala Glu Gly Thr Pro Gly Pro His Lys Met Glu His Gly Gly
915 920 925
Arg Val Val Cys Ser Asp Cys Asn Thr Glu Val Asp Cys Tyr Ser Arg
930 935 940
Gly Gln Ala Phe His Pro Gln Pro Val Ser Arg Asp Ser Ala Gln Pro
945 950 955 960
Ser Ala Pro Asn Gly Pro Glu Pro Gly Gly Ser Asp Gln Glu His Ser
965 970 975
Pro His His Gln Cys Ser Arg Thr Ala Ala Gly Ser Cys Pro Glu Cys
980 985 990
Gln Gly Ser Leu Tyr Pro Ser Asn His Asp Arg Met Leu Thr Ala Val
995 1000 1005
Lys Lys Lys Pro Met Ala Ser Leu Asp Gly Lys Gly Asp Ser Ser
1010 1015 1020
Trp Thr Leu Ala Arg Leu Tyr His Pro Asp Ser Thr Glu Leu Gln
1025 1030 1035
Pro Ala Ser Ser Leu Thr Ser Gly Ser Pro Glu Arg Ala Glu Ala
1040 1045 1050
Gln Tyr Leu Leu Val Ser Asn Gly His Leu Pro Lys Ala Cys Asp
1055 1060 1065
Ala Ser Pro Glu Ser Thr Pro Leu Thr Gly Gln Leu Pro Gly Lys
1070 1075 1080
Gln Arg Val Pro Leu Leu Leu Ala Pro Lys Ser
1085 1090

<210> 255
<211> 474
<212> PRT
<213> Homo sapiens
<400> 255
Met Ala Thr Asn Trp Gly Ser Leu Leu Gln Asp Lys Gln Gln Leu Glu
1 5 10 15
Glu Leu Ala Arg Gln Ala Val Asp Arg Ala Leu Ala Glu Gly Val Leu
20 25 30
Leu Arg Thr Ser Gln Glu Pro Thr Ser Ser Glu Val Val Ser Tyr Ala
35 40 45
Pro Phe Thr Leu Phe Pro Ser Leu Val Pro Ser Ala Leu Leu Glu Gln
50 55 60
Ala Tyr Ala Val Gln Met Asp Phe Asn Leu Leu Val Asp Ala Val Ser
65 70 75 80
Gln Asn Ala Ala Phe Leu Glu Gln Thr Leu Ser Ser Thr Ile Lys Gln
85 90 95
Asp Asp Phe Thr Ala Arg Leu Phe Asp Ile His Lys Gln Val Leu Lys
100 105 110
Glu Gly Ile Ala Gln Thr Val Phe Leu Gly Leu Asn Arg Ser Asp Tyr
115 120 125
Met Phe Gln Arg Ser Ala Asp Gly Ser Pro Ala Leu Lys Gln Ile Glu
130 135 140
Ile Asn Thr Ile Ser Ala Ser Phe Gly Gly Leu Ala Ser Arg Thr Pro
145 150 155 160
Ala Val His Arg His Val Leu Ser Val Leu Ser Lys Thr Lys Glu Ala
165 170 175
Gly Lys Ile Leu Ser Asn Asn Pro Ser Lys Gly Leu Ala Leu Gly Ile

180 185 190
Ala Lys Ala Trp Glu Leu Tyr Gly Ser Pro Asn Ala Leu Val Leu Leu
195 200 205
Ile Ala Gln Glu Lys Glu Arg Asn Ile Phe Asp Gln Arg Ala Ile Glu
210 215 220
Asn Glu Leu Leu Ala Arg Asn Ile His Val Ile Arg Arg Thr Phe Glu
225 230 235 240
Asp Ile Ser Glu Lys Gly Ser Leu Asp Gln Asp Arg Arg Leu Phe Val
245 250 255
Asp Gly Gln Glu Ile Ala Val Val Tyr Phe Arg Asp Gly Tyr Met Pro
260 265 270
Arg Gln Tyr Ser Leu Gln Asn Trp Glu Ala Arg Leu Leu Leu Glu Arg
275 280 285
Ser His Ala Ala Lys Cys Pro Asp Ile Ala Thr Gln Leu Ala Gly Thr
290 295 300
Lys Lys Val Gln Gln Glu Leu Ser Arg Pro Gly Met Leu Glu Met Leu
305 310 315 320
Leu Pro Gly Gln Pro Glu Ala Val Ala Arg Leu Arg Ala Thr Phe Ala
325 330 335
Gly Leu Tyr Ser Leu Asp Val Gly Glu Glu Gly Asp Gln Ala Ile Ala
340 345 350
Glu Ala Leu Ala Ala Pro Ser Arg Phe Val Leu Lys Pro Gln Arg Glu
355 360 365
Gly Gly Gly Asn Asn Leu Tyr Gly Glu Glu Met Val Gln Ala Leu Lys
370 375 380
Gln Leu Lys Asp Ser Glu Glu Arg Ala Ser Tyr Ile Leu Met Glu Lys
385 390 395 400
Ile Glu Pro Glu Pro Phe Glu Asn Cys Leu Leu Arg Pro Gly Ser Pro
405 410 415
Ala Arg Val Val Gln Cys Ile Ser Glu Leu Gly Ile Phe Gly Val Tyr
420 425 430
Val Arg Gln Glu Lys Thr Leu Val Met Asn Lys His Val Gly His Leu
435 440 445
Leu Arg Thr Lys Ala Ile Glu His Ala Asp Gly Gly Val Ala Ala Gly
450 455 460
Val Ala Val Leu Asp Asn Pro Tyr Pro Val
465 470

<210> 256
<211> 96
<212> PRT
<213> Homo sapiens
<400> 256
Met Trp Ile Tyr Ser Lys Leu Ser Lys Arg Thr Val Phe Arg Lys Gly
1 5 10 15
Lys Thr Arg Thr Gln Lys Thr Leu Gly Ile Thr Thr Thr Pro Lys Cys
20 25 30
Phe Arg Leu Gln Glu Ile Thr Ile His Val Phe Ala Gly Gly His Thr
35 40 45
Ile Phe Pro Tyr Tyr Leu Val Gln Asn Asp Ser Ser Leu Pro Lys Ser
50 55 60
Phe Phe Ser Asn His Asp Phe Pro Ile Tyr Phe Gly Pro Met Gln Ser
65 70 75 80
His Ser Leu Trp Pro Ile Val Ser Leu Pro Thr Thr Pro Leu Gly Gly
85 90 95

<210> 257
<211> 3256
<212> PRT
<213> Homo sapiens
<400> 257
Met Trp Pro Thr Arg Arg Leu Val Thr Ile Lys Arg Ser Gly Val Asp
1 5 10 15
Gly Pro His Phe Pro Leu Ser Leu Ser Thr Cys Leu Phe Gly Arg Gly
20 25 30
Ile Glu Cys Asp Ile Arg Ile Gln Leu Pro Val Val Ser Lys Gln His
35 40 45
Cys Lys Ile Glu Ile His Glu Gln Glu Ala Ile Leu His Asn Phe Ser
50 55 60

```
Ser Thr Asn Pro Thr Gln Val Asn Gly Ser Val Ile Asp Glu Pro Val
65                  70                  75                  80
Arg Leu Lys His Gly Asp Val Ile Thr Ile Ile Asp Arg Ser Phe Arg
                85                  90                  95
Tyr Glu Asn Glu Ser Leu Gln Asn Gly Arg Lys Ser Thr Glu Phe Pro
            100                 105                 110
Arg Lys Ile Arg Glu Gln Glu Pro Ala Arg Arg Val Ser Arg Ser Ser
        115                 120                 125
Phe Ser Ser Asp Pro Asp Glu Lys Ala Gln Asp Ser Lys Ala Tyr Ser
    130                 135                 140
Lys Ile Thr Glu Gly Lys Val Ser Gly Asn Pro Gln Val His Ile Lys
145                 150                 155                 160
Asn Val Lys Glu Asp Ser Thr Ala Asp Asp Ser Lys Asp Ser Val Ala
                165                 170                 175
Gln Gly Thr Thr Asn Val His Ser Ser Glu His Ala Gly Arg Asn Gly
            180                 185                 190
Arg Asn Ala Ala Asp Pro Ile Ser Gly Asp Phe Lys Glu Ile Ser Ser
        195                 200                 205
Val Lys Leu Val Ser Arg Tyr Gly Glu Leu Lys Ser Val Pro Thr Thr
    210                 215                 220
Gln Cys Leu Asp Asn Ser Lys Lys Asn Glu Ser Pro Phe Trp Lys Leu
225                 230                 235                 240
Tyr Glu Ser Val Lys Lys Glu Leu Asp Val Lys Ser Gln Lys Glu Asn
                245                 250                 255
Val Leu Gln Tyr Cys Arg Lys Ser Gly Leu Gln Thr Asp Tyr Ala Thr
            260                 265                 270
Glu Lys Glu Ser Ala Asp Gly Leu Gln Gly Glu Thr Gln Leu Leu Val
        275                 280                 285
Ser Arg Lys Ser Arg Pro Lys Ser Gly Gly Ser Gly His Ala Val Ala
    290                 295                 300
Glu Pro Ala Ser Pro Glu Gln Glu Leu Asp Gln Asn Lys Gly Lys Gly
305                 310                 315                 320
Arg Asp Val Glu Ser Val Gln Thr Pro Ser Lys Ala Val Gly Ala Ser
                325                 330                 335
Phe Pro Leu Tyr Glu Pro Ala Lys Met Lys Thr Pro Val Gln Tyr Ser
            340                 345                 350
Gln Gln Gln Asn Ser Pro Gln Lys His Lys Asn Lys Asp Leu Tyr Thr
        355                 360                 365
Thr Gly Arg Arg Glu Ser Val Asn Leu Gly Lys Ser Glu Gly Phe Lys
    370                 375                 380
Ala Gly Asp Lys Thr Leu Thr Pro Arg Lys Leu Ser Thr Arg Asn Arg
385                 390                 395                 400
Thr Pro Ala Lys Val Glu Asp Ala Ala Asp Ser Ala Thr Lys Pro Glu
                405                 410                 415
Asn Leu Ser Ser Lys Thr Arg Gly Ser Ile Pro Thr Asp Val Glu Val
            420                 425                 430
Leu Pro Thr Glu Thr Glu Ile His Asn Glu Pro Phe Leu Thr Leu Trp
        435                 440                 445
Leu Thr Gln Val Glu Arg Lys Ile Gln Lys Asp Ser Leu Ser Lys Pro
    450                 455                 460
Glu Lys Leu Gly Thr Thr Ala Gly Gln Met Cys Ser Gly Leu Pro Gly
465                 470                 475                 480
Leu Ser Ser Val Asp Ile Asn Asn Phe Gly Asp Ser Ile Asn Glu Ser
                485                 490                 495
Glu Gly Ile Pro Leu Lys Arg Arg Arg Val Ser Phe Gly Gly His Leu
            500                 505                 510
Arg Pro Glu Leu Phe Asp Glu Asn Leu Pro Pro Asn Thr Pro Leu Lys
        515                 520                 525
Arg Gly Glu Ala Pro Thr Lys Arg Lys Ser Leu Val Met His Thr Pro
    530                 535                 540
Pro Val Leu Lys Lys Ile Ile Lys Glu Gln Pro Gln Pro Ser Gly Lys
545                 550                 555                 560
Gln Glu Ser Gly Ser Glu Ile His Val Glu Val Lys Ala Gln Ser Leu
                565                 570                 575
Val Ile Ser Pro Pro Ala Pro Ser Pro Arg Lys Thr Pro Val Ala Ser
            580                 585                 590
Asp Gln Arg Arg Arg Ser Cys Lys Thr Ala Pro Ala Ser Ser Ser Lys
        595                 600                 605
Ser Gln Thr Glu Val Pro Lys Arg Gly Gly Glu Arg Val Ala Thr Cys
```

610 615 620
Leu Gln Lys Arg Val Ser Ile Ser Arg Ser Gln His Asp Ile Leu Gln
625 630 635 640
Met Ile Cys Ser Lys Arg Arg Ser Gly Ala Ser Glu Ala Asn Leu Ile
645 650 655
Val Ala Lys Ser Trp Ala Asp Val Val Lys Leu Gly Ala Lys Gln Thr
660 665 670
Gln Thr Lys Val Ile Lys His Gly Pro Gln Arg Ser Met Asn Lys Arg
675 680 685
Gln Arg Arg Pro Ala Thr Pro Lys Lys Pro Val Gly Glu Val His Ser
690 695 700
Gln Phe Ser Thr Gly His Ala Asn Ser Pro Cys Thr Ile Ile Ile Gly
705 710 715 720
Lys Ala His Thr Glu Lys Val His Val Pro Ala Arg Pro Tyr Arg Val
725 730 735
Leu Asn Asn Phe Ile Ser Asn Gln Lys Met Asp Phe Lys Glu Asp Leu
740 745 750
Ser Gly Ile Ala Glu Met Phe Lys Thr Pro Val Lys Glu Gln Pro Gln
755 760 765
Leu Thr Ser Thr Cys His Ile Ala Ile Ser Asn Ser Glu Asn Leu Leu
770 775 780
Gly Lys Gln Phe Gln Gly Thr Asp Ser Gly Glu Glu Pro Leu Leu Pro
785 790 795 800
Thr Ser Glu Ser Phe Gly Gly Asn Val Phe Phe Ser Ala Gln Asn Ala
805 810 815
Ala Lys Gln Pro Ser Asp Lys Cys Ser Ala Ser Pro Pro Leu Arg Arg
820 825 830
Gln Cys Ile Arg Glu Asn Gly Asn Val Ala Lys Thr Pro Arg Asn Thr
835 840 845
Tyr Lys Met Thr Ser Leu Glu Thr Lys Thr Ser Asp Thr Glu Thr Glu
850 855 860
Pro Ser Lys Thr Val Ser Thr Val Asn Arg Ser Gly Arg Ser Thr Glu
865 870 875 880
Phe Arg Asn Ile Gln Lys Leu Pro Val Glu Ser Lys Ser Glu Glu Thr
885 890 895
Asn Thr Glu Ile Val Glu Cys Ile Leu Lys Arg Gly Gln Lys Ala Thr
900 905 910
Leu Leu Gln Gln Arg Arg Glu Gly Glu Met Lys Glu Ile Glu Arg Pro
915 920 925
Phe Glu Thr Tyr Lys Glu Asn Ile Glu Leu Lys Glu Asn Asp Glu Lys
930 935 940
Met Lys Ala Met Lys Arg Ser Arg Thr Trp Gly Gln Lys Cys Ala Pro
945 950 955 960
Met Ser Asp Leu Thr Asp Leu Lys Ser Leu Pro Asp Thr Glu Leu Met
965 970 975
Lys Asp Thr Ala Arg Gly Gln Asn Leu Leu Gln Thr Gln Asp His Ala
980 985 990
Lys Ala Pro Lys Ser Glu Lys Gly Lys Ile Thr Lys Met Pro Cys Gln
995 1000 1005
Ser Leu Gln Pro Glu Pro Ile Asn Thr Pro Thr His Thr Lys Gln
1010 1015 1020
Gln Leu Lys Ala Ser Leu Gly Lys Val Gly Val Lys Glu Glu Leu
1025 1030 1035
Leu Ala Val Gly Lys Phe Thr Arg Thr Ser Gly Glu Thr Thr His
1040 1045 1050
Thr His Arg Glu Pro Ala Gly Asp Gly Lys Ser Ile Arg Thr Phe
1055 1060 1065
Lys Glu Ser Pro Lys Gln Ile Leu Asp Pro Ala Ala Arg Val Thr
1070 1075 1080
Gly Met Lys Lys Trp Pro Arg Thr Pro Lys Glu Glu Ala Gln Ser
1085 1090 1095
Leu Glu Asp Leu Ala Gly Phe Lys Glu Leu Phe Gln Thr Pro Gly
1100 1105 1110
Pro Ser Glu Glu Ser Met Thr Asp Glu Lys Thr Thr Lys Ile Ala
1115 1120 1125
Cys Lys Ser Pro Pro Pro Glu Ser Val Asp Thr Pro Thr Ser Thr
1130 1135 1140
Lys Gln Trp Pro Lys Arg Ser Leu Arg Lys Ala Asp Val Glu Glu
1145 1150 1155

Glu Phe Leu Ala Leu Arg Lys Leu Thr Pro Ser Ala Gly Lys Ala
1160 1165 1170
Met Leu Thr Pro Lys Pro Ala Gly Gly Asp Glu Lys Asp Ile Lys
1175 1180 1185
Ala Phe Met Gly Thr Pro Val Gln Lys Leu Asp Leu Ala Gly Thr
1190 1195 1200
Leu Pro Gly Ser Lys Arg Gln Leu Gln Thr Pro Lys Glu Lys Ala
1205 1210 1215
Gln Ala Leu Glu Asp Leu Ala Gly Phe Lys Glu Leu Phe Gln Thr
1220 1225 1230
Pro Gly His Thr Glu Glu Leu Val Ala Ala Gly Lys Thr Thr Lys
1235 1240 1245
Ile Pro Cys Asp Ser Pro Gln Ser Asp Pro Val Asp Thr Pro Thr
1250 1255 1260
Ser Thr Lys Gln Arg Pro Lys Arg Ser Ile Arg Lys Ala Asp Val
1265 1270 1275
Glu Gly Glu Leu Leu Ala Cys Arg Asn Leu Met Pro Ser Ala Gly
1280 1285 1290
Lys Ala Met His Thr Pro Lys Pro Ser Val Gly Glu Glu Lys Asp
1295 1300 1305
Ile Ile Ile Phe Val Gly Thr Pro Val Gln Lys Leu Asp Leu Thr
1310 1315 1320
Glu Asn Leu Thr Gly Ser Lys Arg Arg Pro Gln Thr Pro Lys Glu
1325 1330 1335
Glu Ala Gln Ala Leu Glu Asp Leu Thr Gly Phe Lys Glu Leu Phe
1340 1345 1350
Gln Thr Pro Gly His Thr Glu Glu Ala Val Ala Ala Gly Lys Thr
1355 1360 1365
Thr Lys Met Pro Cys Glu Ser Ser Pro Pro Glu Ser Ala Asp Thr
1370 1375 1380
Pro Thr Ser Thr Arg Arg Gln Pro Lys Thr Pro Leu Glu Lys Arg
1385 1390 1395
Asp Val Gln Lys Glu Leu Ser Ala Leu Lys Lys Leu Thr Gln Thr
1400 1405 1410
Ser Gly Glu Thr Thr His Thr Asp Lys Val Pro Gly Gly Glu Asp
1415 1420 1425
Lys Ser Ile Asn Ala Phe Arg Glu Thr Ala Lys Gln Lys Leu Asp
1430 1435 1440
Pro Ala Ala Ser Val Thr Gly Ser Lys Arg His Pro Lys Thr Lys
1445 1450 1455
Glu Lys Ala Gln Pro Leu Glu Asp Leu Ala Gly Trp Lys Glu Leu
1460 1465 1470
Phe Gln Thr Pro Val Cys Thr Asp Lys Pro Thr Thr His Glu Lys
1475 1480 1485
Thr Thr Lys Ile Ala Cys Arg Ser Gln Pro Asp Pro Val Asp Thr
1490 1495 1500
Pro Thr Ser Ser Lys Pro Gln Ser Lys Arg Ser Leu Arg Lys Val
1505 1510 1515
Asp Val Glu Glu Glu Phe Phe Ala Leu Arg Lys Arg Thr Pro Ser
1520 1525 1530
Ala Gly Lys Ala Met His Thr Pro Lys Pro Ala Val Ser Gly Glu
1535 1540 1545
Lys Asn Ile Tyr Ala Phe Met Gly Thr Pro Val Gln Lys Leu Asp
1550 1555 1560
Leu Thr Glu Asn Leu Thr Gly Ser Lys Arg Arg Leu Gln Thr Pro
1565 1570 1575
Lys Glu Lys Ala Gln Ala Leu Glu Asp Leu Ala Gly Phe Lys Glu
1580 1585 1590
Leu Phe Gln Thr Arg Gly His Thr Glu Glu Ser Met Thr Asn Asp
1595 1600 1605
Lys Thr Ala Lys Val Ala Cys Lys Ser Ser Gln Pro Asp Leu Asp
1610 1615 1620
Lys Asn Pro Ala Ser Ser Lys Arg Arg Leu Lys Thr Ser Leu Gly
1625 1630 1635
Lys Val Gly Val Lys Glu Glu Leu Leu Ala Val Gly Lys Leu Thr
1640 1645 1650
Gln Thr Ser Gly Glu Thr Thr His Thr His Thr Glu Pro Thr Gly
1655 1660 1665
Asp Gly Lys Ser Met Lys Ala Phe Met Glu Ser Pro Lys Gln Ile

1670 1675 1680
Leu Asp Ser Ala Ala Ser Leu Thr Gly Ser Lys Arg Gln Leu Arg
1685 1690 1695
Thr Pro Lys Gly Lys Ser Glu Val Pro Glu Asp Leu Ala Gly Phe
1700 1705 1710
Ile Glu Leu Phe Gln Thr Pro Ser His Thr Lys Glu Ser Met Thr
1715 1720 1725
Asn Glu Lys Thr Thr Lys Val Ser Tyr Arg Ala Ser Gln Pro Asp
1730 1735 1740
Leu Val Asp Thr Pro Thr Ser Ser Lys Pro Gln Pro Lys Arg Ser
1745 1750 1755
Leu Arg Lys Ala Asp Thr Glu Glu Glu Phe Leu Ala Phe Arg Lys
1760 1765 1770
Gln Thr Pro Ser Ala Gly Lys Ala Met His Thr Pro Lys Pro Ala
1775 1780 1785
Val Gly Glu Glu Lys Asp Ile Asn Thr Phe Leu Gly Thr Pro Val
1790 1795 1800
Gln Lys Leu Asp Gln Pro Gly Asn Leu Pro Gly Ser Asn Arg Arg
1805 1810 1815
Leu Gln Thr Arg Lys Glu Lys Ala Gln Ala Leu Glu Glu Leu Thr
1820 1825 1830
Gly Phe Arg Glu Leu Phe Gln Thr Pro Cys Thr Asp Asn Pro Thr
1835 1840 1845
Ala Asp Glu Lys Thr Thr Lys Lys Ile Leu Cys Lys Ser Pro Gln
1850 1855 1860
Ser Asp Pro Ala Asp Thr Pro Thr Asn Thr Lys Gln Arg Pro Lys
1865 1870 1875
Arg Ser Leu Lys Lys Ala Asp Val Glu Glu Glu Phe Leu Ala Phe
1880 1885 1890
Arg Lys Leu Thr Pro Ser Ala Gly Lys Ala Met His Thr Pro Lys
1895 1900 1905
Ala Ala Val Gly Glu Glu Lys Asp Ile Asn Thr Phe Val Gly Thr
1910 1915 1920
Pro Val Glu Lys Leu Asp Leu Leu Gly Asn Leu Pro Gly Ser Lys
1925 1930 1935
Arg Arg Pro Gln Thr Pro Lys Glu Lys Ala Lys Ala Leu Glu Asp
1940 1945 1950
Leu Ala Gly Phe Lys Glu Leu Phe Gln Thr Pro Gly His Thr Glu
1955 1960 1965
Glu Ser Met Thr Asp Asp Lys Ile Thr Glu Val Ser Cys Lys Ser
1970 1975 1980
Pro Gln Pro Asp Pro Val Lys Thr Pro Thr Ser Ser Lys Gln Arg
1985 1990 1995
Leu Lys Ile Ser Leu Gly Lys Val Gly Val Lys Glu Glu Val Leu
2000 2005 2010
Pro Val Gly Lys Leu Thr Gln Thr Ser Gly Lys Thr Thr Gln Thr
2015 2020 2025
His Arg Glu Thr Ala Gly Asp Gly Lys Ser Ile Lys Ala Phe Lys
2030 2035 2040
Glu Ser Ala Lys Gln Met Leu Asp Pro Ala Asn Tyr Gly Thr Gly
2045 2050 2055
Met Glu Arg Trp Pro Arg Thr Pro Lys Glu Glu Ala Gln Ser Leu
2060 2065 2070
Glu Asp Leu Ala Gly Phe Lys Glu Leu Phe Gln Thr Pro Asp His
2075 2080 2085
Thr Glu Glu Ser Thr Thr Asp Asp Lys Thr Thr Lys Ile Ala Cys
2090 2095 2100
Lys Ser Pro Pro Pro Glu Ser Met Asp Thr Pro Thr Ser Thr Arg
2105 2110 2115
Arg Arg Pro Lys Thr Pro Leu Gly Lys Arg Asp Ile Val Glu Glu
2120 2125 2130
Leu Ser Ala Leu Lys Gln Leu Thr Gln Thr Thr His Thr Asp Lys
2135 2140 2145
Val Pro Gly Asp Glu Asp Lys Gly Ile Asn Val Phe Arg Glu Thr
2150 2155 2160
Ala Lys Gln Lys Leu Asp Pro Ala Ala Ser Val Thr Gly Ser Lys
2165 2170 2175
Arg Gln Pro Arg Thr Pro Lys Gly Lys Ala Gln Pro Leu Glu Asp
2180 2185 2190

Leu Ala Gly Leu Lys Glu Leu Phe Gln Thr Pro Val Cys Thr Asp
2195 2200 2205
Lys Pro Thr Thr His Glu Lys Thr Thr Lys Ile Ala Cys Arg Ser
2210 2215 2220
Pro Gln Pro Asp Pro Val Gly Thr Pro Thr Ile Phe Lys Pro Gln
2225 2230 2235
Ser Lys Arg Ser Leu Arg Lys Ala Asp Val Glu Glu Glu Ser Leu
2240 2245 2250
Ala Leu Arg Lys Arg Thr Pro Ser Val Gly Lys Ala Met Asp Thr
2255 2260 2265
Pro Lys Pro Ala Gly Gly Asp Glu Lys Asp Met Lys Ala Phe Met
2270 2275 2280
Gly Thr Pro Val Gln Lys Leu Asp Leu Pro Gly Asn Leu Pro Gly
2285 2290 2295
Ser Lys Arg Trp Pro Gln Thr Pro Lys Glu Lys Ala Gln Ala Leu
2300 2305 2310
Glu Asp Leu Ala Gly Phe Lys Glu Leu Phe Gln Thr Pro Gly Thr
2315 2320 2325
Asp Lys Pro Thr Thr Asp Glu Lys Thr Thr Lys Ile Ala Cys Lys
2330 2335 2340
Ser Pro Gln Pro Asp Pro Val Asp Thr Pro Ala Ser Thr Lys Gln
2345 2350 2355
Arg Pro Lys Arg Asn Leu Arg Lys Ala Asp Val Glu Glu Glu Phe
2360 2365 2370
Leu Ala Leu Arg Lys Arg Thr Pro Ser Ala Gly Lys Ala Met Asp
2375 2380 2385
Thr Pro Lys Pro Ala Val Ser Asp Glu Lys Asn Ile Asn Thr Phe
2390 2395 2400
Val Glu Thr Pro Val Gln Lys Leu Asp Leu Leu Gly Asn Leu Pro
2405 2410 2415
Gly Ser Lys Arg Gln Pro Gln Thr Pro Lys Glu Lys Ala Glu Ala
2420 2425 2430
Leu Glu Asp Leu Val Gly Phe Lys Glu Leu Phe Gln Thr Pro Gly
2435 2440 2445
His Thr Glu Glu Ser Met Thr Asp Asp Lys Ile Thr Glu Val Ser
2450 2455 2460
Cys Lys Ser Pro Gln Pro Glu Ser Phe Lys Thr Ser Arg Ser Ser
2465 2470 2475
Lys Gln Arg Leu Lys Ile Pro Leu Val Lys Val Asp Met Lys Glu
2480 2485 2490
Glu Pro Leu Ala Val Ser Lys Leu Thr Arg Thr Ser Gly Glu Thr
2495 2500 2505
Thr Gln Thr His Thr Glu Pro Thr Gly Asp Ser Lys Ser Ile Lys
2510 2515 2520
Ala Phe Lys Glu Ser Pro Lys Gln Ile Leu Asp Pro Ala Ala Ser
2525 2530 2535
Val Thr Gly Ser Arg Arg Gln Leu Arg Thr Arg Lys Glu Lys Ala
2540 2545 2550
Arg Ala Leu Glu Asp Leu Val Asp Phe Lys Glu Leu Phe Ser Ala
2555 2560 2565
Pro Gly His Thr Glu Glu Ser Met Thr Ile Asp Lys Asn Thr Lys
2570 2575 2580
Ile Pro Cys Lys Ser Pro Pro Pro Glu Leu Thr Asp Thr Ala Thr
2585 2590 2595
Ser Thr Lys Arg Cys Pro Lys Thr Arg Pro Arg Lys Glu Val Lys
2600 2605 2610
Glu Glu Leu Ser Ala Val Glu Arg Leu Thr Gln Thr Ser Gly Gln
2615 2620 2625
Ser Thr His Thr His Lys Glu Pro Ala Ser Gly Asp Glu Gly Ile
2630 2635 2640
Lys Val Leu Lys Gln Arg Ala Lys Lys Lys Pro Asn Pro Val Glu
2645 2650 2655
Glu Glu Pro Ser Arg Arg Arg Pro Arg Ala Pro Lys Glu Lys Ala
2660 2665 2670
Gln Pro Leu Glu Asp Leu Ala Gly Phe Thr Glu Leu Ser Glu Thr
2675 2680 2685
Ser Gly His Thr Gln Glu Ser Leu Thr Ala Gly Lys Ala Thr Lys
2690 2695 2700
Ile Pro Cys Glu Ser Pro Pro Leu Glu Val Val Asp Thr Thr Ala

2705 2710 2715
Ser Thr Lys Arg His Leu Arg Thr Arg Val Gln Lys Val Gln Val
2720 2725 2730
Lys Glu Glu Pro Ser Ala Val Lys Phe Thr Gln Thr Ser Gly Glu
2735 2740 2745
Thr Thr Asp Ala Asp Lys Glu Pro Ala Gly Glu Asp Lys Gly Ile
2750 2755 2760
Lys Ala Leu Lys Glu Ser Ala Lys Gln Thr Pro Ala Pro Ala Ala
2765 2770 2775
Ser Val Thr Gly Ser Arg Arg Arg Pro Arg Ala Pro Arg Glu Ser
2780 2785 2790
Ala Gln Ala Ile Glu Asp Leu Ala Gly Phe Lys Asp Pro Ala Ala
2795 2800 2805
Gly His Thr Glu Glu Ser Met Thr Asp Asp Lys Thr Thr Lys Ile
2810 2815 2820
Pro Cys Lys Ser Ser Pro Glu Leu Glu Asp Thr Ala Thr Ser Ser
2825 2830 2835
Lys Arg Arg Pro Arg Thr Arg Ala Gln Lys Val Glu Val Lys Glu
2840 2845 2850
Glu Leu Leu Ala Val Gly Lys Leu Thr Gln Thr Ser Gly Glu Thr
2855 2860 2865
Thr His Thr Asp Lys Glu Pro Val Gly Glu Gly Lys Gly Thr Lys
2870 2875 2880
Ala Phe Lys Gln Pro Ala Lys Arg Asn Val Asp Ala Glu Asp Val
2885 2890 2895
Ile Gly Ser Arg Arg Gln Pro Arg Ala Pro Lys Glu Lys Ala Gln
2900 2905 2910
Pro Leu Glu Asp Leu Ala Ser Phe Gln Glu Leu Ser Gln Thr Pro
2915 2920 2925
Gly His Thr Glu Glu Leu Ala Asn Gly Ala Ala Asp Ser Phe Thr
2930 2935 2940
Ser Ala Pro Lys Gln Thr Pro Asp Ser Gly Lys Pro Leu Lys Ile
2945 2950 2955
Ser Arg Arg Val Leu Arg Ala Pro Lys Val Glu Pro Val Gly Asp
2960 2965 2970
Val Val Ser Thr Arg Asp Pro Val Lys Ser Gln Ser Lys Ser Asn
2975 2980 2985
Thr Ser Leu Pro Pro Leu Pro Phe Lys Arg Gly Gly Gly Lys Asp
2990 2995 3000
Gly Ser Val Thr Gly Thr Lys Arg Leu Arg Cys Met Pro Ala Pro
3005 3010 3015
Glu Glu Ile Val Glu Glu Leu Pro Ala Ser Lys Lys Gln Arg Val
3020 3025 3030
Ala Pro Arg Ala Arg Gly Lys Ser Ser Glu Pro Val Val Ile Met
3035 3040 3045
Lys Arg Ser Leu Arg Thr Ser Ala Lys Arg Ile Glu Pro Ala Glu
3050 3055 3060
Glu Leu Asn Ser Asn Asp Met Lys Thr Asn Lys Glu Glu His Lys
3065 3070 3075
Leu Gln Asp Ser Val Pro Glu Asn Lys Gly Ile Ser Leu Arg Ser
3080 3085 3090
Arg Arg Gln Asp Lys Thr Glu Ala Glu Gln Gln Ile Thr Glu Val
3095 3100 3105
Phe Val Leu Ala Glu Arg Ile Glu Ile Asn Arg Asn Glu Lys Lys
3110 3115 3120
Pro Met Lys Thr Ser Pro Glu Met Asp Ile Gln Asn Pro Asp Asp
3125 3130 3135
Gly Ala Arg Lys Pro Ile Pro Arg Asp Lys Val Thr Glu Asn Lys
3140 3145 3150
Arg Cys Leu Arg Ser Ala Arg Gln Asn Glu Ser Ser Gln Pro Lys
3155 3160 3165
Val Ala Glu Glu Ser Gly Gly Gln Lys Ser Ala Lys Val Leu Met
3170 3175 3180
Gln Asn Gln Lys Gly Lys Gly Glu Ala Gly Asn Ser Asp Ser Met
3185 3190 3195
Cys Leu Arg Ser Arg Lys Thr Lys Ser Gln Pro Ala Ala Ser Thr
3200 3205 3210
Leu Glu Ser Lys Ser Val Gln Arg Val Thr Arg Ser Val Lys Arg
3215 3220 3225

Cys Ala Glu Asn Pro Lys Lys Ala Glu Asp Asn Val Cys Val Lys
3230 3235 3240
Lys Ile Thr Thr Arg Ser His Arg Asp Ser Glu Asp Ile
3245 3250 3255

<210> 258
<211> 160
<212> PRT
<213> Homo sapiens
<400> 258
Met Ser Glu Val Arg Pro Leu Ser Arg Asp Ile Leu Met Glu Thr Leu
1 5 10 15
Leu Tyr Glu Gln Leu Leu Glu Pro Pro Thr Met Glu Val Leu Gly Met
20 25 30
Thr Asp Ser Glu Glu Asp Leu Asp Pro Met Glu Asp Phe Asp Ser Leu
35 40 45
Glu Cys Met Glu Gly Ser Asp Ala Leu Ala Leu Arg Leu Ala Cys Ile
50 55 60
Gly Asp Glu Met Asp Val Ser Leu Arg Ala Pro Arg Leu Ala Gln Leu
65 70 75 80
Ser Glu Val Ala Met His Ser Leu Gly Leu Ala Phe Ile Tyr Asp Gln
85 90 95
Thr Glu Asp Ile Arg Asp Val Leu Arg Ser Phe Met Asp Gly Phe Thr
100 105 110
Thr Leu Lys Glu Asn Ile Met Arg Phe Trp Arg Ser Pro Asn Pro Gly
115 120 125
Ser Trp Val Ser Cys Glu Gln Val Leu Leu Ala Leu Leu Leu Leu Leu
130 135 140
Ala Leu Leu Leu Pro Leu Leu Ser Gly Gly Leu His Leu Leu Leu Lys
145 150 155 160

<210> 259
<211> 1844
<212> PRT
<213> Homo sapiens
<400> 259
Met Thr Thr Arg Phe Thr Asp Glu Leu Met Glu Gln Gly Leu Thr Tyr
1 5 10 15
Lys Val Leu Thr Leu Val Ser Gln Ile Asp Val Asn Asn Glu Phe Glu
20 25 30
Lys Leu Gln Arg Glu Arg Gly Leu Gly Ser Glu Lys His Arg Lys Glu
35 40 45
Val Ser Asp Leu Ile Lys Glu Cys Arg Gln Ser Leu Ala Glu Ser Leu
50 55 60
Phe Ala Trp Ala Cys Gln Ser Pro Leu Gly Lys Glu Asp Thr Leu Leu
65 70 75 80
Leu Ile Gly His Leu Glu Arg Val Thr Val Glu Ala Asn Gly Ser Leu
85 90 95
Asp Ala Val Asn Leu Ala Leu Leu Met Ala Leu Leu Tyr Cys Phe Asp
100 105 110
Ile Ser Phe Ile Glu Gln Ser Thr Glu Glu Arg Asp Asp Met Ile His
115 120 125
Gln Leu Pro Leu Leu Thr Glu Lys Gln Tyr Ile Ala Thr Ile His Ser
130 135 140
Arg Leu Gln Asp Ser Gln Leu Trp Lys Leu Pro Gly Leu Gln Ala Thr
145 150 155 160
Val Arg Leu Ala Trp Ala Leu Ala Leu Arg Gly Ile Ser Gln Leu Pro
165 170 175
Asp Val Thr Ala Leu Ala Glu Phe Thr Glu Ala Asp Glu Ala Met Ala
180 185 190
Glu Leu Ala Ile Ala Asp Asn Val Phe Leu Phe Leu Met Glu Ser Val
195 200 205
Val Val Ser Glu Tyr Phe Tyr Gln Glu Glu Phe Tyr Ile Arg Arg Val
210 215 220
His Asn Leu Ile Thr Asp Phe Leu Ala Leu Met Pro Met Lys Val Lys
225 230 235 240
Gln Leu Arg Asn Arg Ala Asp Glu Asp Ala Arg Met Ile His Met Ser
245 250 255
Met Gln Met Gly Asn Glu Pro Pro Ile Ser Leu Arg Arg Asp Leu Glu

```
            260                     265                     270
His Leu Met Leu Leu Ile Gly Glu Leu Tyr Lys Lys Asn Pro Phe His
        275                     280                     285
Leu Glu Leu Ala Leu Glu Tyr Trp Cys Pro Thr Glu Pro Leu Gln Thr
    290                     295                     300
Pro Thr Ile Met Gly Ser Tyr Leu Gly Val Ala His Gln Arg Pro Pro
305                     310                     315                     320
Gln Arg Gln Val Val Leu Ser Lys Phe Val Arg Gln Met Gly Asp Leu
                325                     330                     335
Leu Pro Pro Thr Ile Tyr Ile Pro Tyr Leu Lys Met Leu Gln Gly Leu
            340                     345                     350
Ala Asn Gly Pro Gln Cys Ala His Tyr Cys Phe Ser Leu Leu Lys Val
        355                     360                     365
Asn Gly Ser Ser His Val Glu Asn Ile Gln Gly Ala Gly Gly Ser Pro
    370                     375                     380
Val Ser Trp Glu His Phe Phe His Ser Leu Met Leu Tyr His Glu His
385                     390                     395                     400
Leu Arg Lys Asp Leu Pro Ser Ala Asp Ser Val Gln Tyr Arg His Leu
                405                     410                     415
Pro Ser Arg Gly Ile Thr Gln Lys Glu Gln Asp Gly Leu Ile Ala Phe
            420                     425                     430
Leu Gln Leu Thr Ser Thr Ile Ile Thr Trp Ser Glu Asn Ala Arg Leu
        435                     440                     445
Ala Leu Cys Glu His Pro Gln Trp Thr Pro Val Val Ile Leu Gly
    450                     455                     460
Leu Leu Gln Cys Ser Ile Pro Pro Val Leu Lys Ala Glu Leu Leu Lys
465                     470                     475                     480
Thr Leu Ala Ala Phe Gly Lys Ser Pro Glu Ile Ala Ala Ser Leu Trp
                485                     490                     495
Gln Ser Leu Glu Tyr Thr Gln Ile Leu Gln Thr Val Arg Ile Pro Ser
            500                     505                     510
Gln Arg Gln Ala Ile Gly Ile Glu Val Glu Leu Asn Glu Ile Glu Ser
        515                     520                     525
Arg Cys Glu Glu Tyr Pro Leu Thr Arg Ala Phe Cys Gln Leu Ile Ser
    530                     535                     540
Thr Leu Val Glu Ser Ser Phe Pro Ser Asn Leu Gly Ala Gly Leu Arg
545                     550                     555                     560
Pro Pro Gly Phe Asp Pro Tyr Leu Gln Phe Leu Arg Asp Ser Val Phe
                565                     570                     575
Leu Arg Phe Arg Thr Arg Ala Tyr Arg Arg Ala Ala Glu Lys Trp Glu
            580                     585                     590
Val Ala Glu Val Val Leu Glu Val Phe Tyr Lys Leu Leu Arg Asp Tyr
        595                     600                     605
Glu Pro Gln Leu Glu Asp Phe Val Asp Gln Phe Val Glu Leu Gln Gly
    610                     615                     620
Glu Glu Ile Ile Ala Tyr Lys Pro Pro Gly Phe Ser Leu Met Tyr His
625                     630                     635                     640
Leu Leu Asn Glu Ser Pro Met Leu Glu Leu Ala Leu Ser Leu Leu Glu
                645                     650                     655
Glu Gly Val Lys Gln Leu Asp Thr Tyr Ala Pro Phe Pro Gly Lys Lys
            660                     665                     670
His Leu Glu Lys Ala Val Gln His Cys Leu Ala Leu Leu Asn Leu Thr
        675                     680                     685
Leu Gln Lys Glu Asn Leu Phe Met Asp Leu Leu Arg Glu Ser Gln Leu
    690                     695                     700
Ala Leu Ile Val Cys Pro Leu Glu Gln Leu Leu Gln Gly Ile Asn Pro
705                     710                     715                     720
Arg Thr Lys Lys Ala Asp Asn Val Val Asn Ile Ala Arg Tyr Leu Tyr
                725                     730                     735
His Gly Asn Thr Asn Pro Glu Leu Ala Phe Glu Ser Ala Lys Ile Leu
            740                     745                     750
Cys Cys Ile Ser Cys Asn Ser Asn Ile Gln Ile Lys Leu Val Gly Asp
        755                     760                     765
Phe Thr His Asp Gln Ser Ile Ser Gln Lys Leu Met Ala Gly Phe Val
    770                     775                     780
Glu Cys Leu Asp Cys Glu Asp Ala Glu Glu Phe Val Arg Leu Glu Glu
785                     790                     795                     800
Gly Ser Glu Leu Glu Lys Lys Leu Val Ala Ile Arg His Glu Thr Arg
                805                     810                     815
```

Ile His Ile Leu Asn Leu Leu Ile Thr Ser Leu Glu Cys Asn Pro Pro
820 825 830
Asn Leu Ala Leu Tyr Leu Leu Gly Phe Glu Leu Lys Lys Pro Val Ser
835 840 845
Thr Thr Asn Leu Gln Asp Pro Gly Val Leu Gly Cys Pro Arg Thr Cys
850 855 860
Leu His Ala Ile Leu Asn Ile Leu Glu Lys Gly Thr Glu Gly Arg Thr
865 870 875 880
Gly Pro Val Ala Val Arg Glu Ser Pro Gln Leu Ala Glu Leu Cys Tyr
885 890 895
Gln Val Ile Tyr Gln Leu Cys Ala Cys Ser Asp Thr Ser Gly Pro Thr
900 905 910
Met Arg Tyr Leu Arg Thr Ser Gln Asp Phe Leu Phe Ser Gln Leu Gln
915 920 925
Tyr Leu Pro Phe Ser Asn Lys Glu Tyr Glu Ile Ser Met Leu Asn Gln
930 935 940
Met Ser Trp Leu Met Lys Thr Ala Ser Ile Glu Leu Arg Val Thr Ser
945 950 955 960
Leu Asn Arg Gln Arg Ser His Thr Gln Arg Leu Leu His Leu Leu Leu
965 970 975
Asp Asp Met Pro Val Lys Pro Tyr Ser Asp Gly Glu Gly Gly Ile Glu
980 985 990
Asp Glu Asn Arg Ser Val Ser Gly Phe Leu His Phe Asp Thr Ala Thr
995 1000 1005
Lys Val Arg Arg Lys Ile Leu Asn Ile Leu Asp Ser Ile Asp Phe
1010 1015 1020
Ser Gln Glu Ile Pro Glu Pro Leu Gln Leu Asp Phe Phe Asp Arg
1025 1030 1035
Ala Gln Ile Glu Gln Val Ile Ala Asn Cys Glu His Lys Asn Leu
1040 1045 1050
Arg Gly Gln Thr Val Cys Asn Val Lys Leu Leu His Arg Val Leu
1055 1060 1065
Val Ala Glu Val Asn Ala Leu Gln Gly Met Ala Ala Ile Gly Gln
1070 1075 1080
Arg Pro Leu Leu Met Glu Glu Ile Ser Thr Val Leu Gln Tyr Val
1085 1090 1095
Val Gly Arg Asn Lys Leu Leu Gln Cys Leu His Ala Lys Arg His
1100 1105 1110
Ala Leu Glu Ser Trp Arg Gln Leu Val Glu Ile Ile Leu Thr Ala
1115 1120 1125
Cys Pro Gln Asp Leu Ile Gln Ala Glu Asp Arg Gln Leu Ile Ile
1130 1135 1140
Arg Asp Ile Leu Gln Asp Val His Asp Lys Ile Leu Asp Asp Glu
1145 1150 1155
Ala Ala Gln Glu Leu Met Pro Val Val Ala Gly Ala Val Phe Thr
1160 1165 1170
Leu Thr Ala His Leu Ser Gln Ala Val Leu Thr Glu Gln Lys Glu
1175 1180 1185
Thr Ser Val Leu Gly Pro Ala Glu Ala His Tyr Ala Phe Met Leu
1190 1195 1200
Asp Ser Cys Phe Thr Ser Pro Pro Pro Glu Glu Asn Pro Leu Val
1205 1210 1215
Gly Phe Ala Ser Ile Gly Asp Ser Ser Leu Tyr Ile Ile Leu Lys
1220 1225 1230
Lys Leu Leu Asp Phe Ile Leu Lys Thr Gly Gly Gly Phe Gln Arg
1235 1240 1245
Val Arg Thr His Leu Tyr Gly Ser Leu Leu Tyr Tyr Leu Gln Ile
1250 1255 1260
Ala Gln Arg Pro Asp Glu Pro Asp Thr Leu Glu Ala Ala Lys Lys
1265 1270 1275
Thr Met Trp Glu Arg Leu Thr Ala Pro Glu Asp Val Phe Ser Lys
1280 1285 1290
Leu Gln Arg Glu Asn Ile Ala Ile Ile Glu Ser Tyr Gly Ala Ala
1295 1300 1305
Leu Met Glu Val Val Cys Arg Asp Ala Cys Asp Gly His Glu Ile
1310 1315 1320
Gly Arg Met Leu Ala Leu Ala Leu Leu Asp Arg Ile Val Ser Val
1325 1330 1335
Asp Lys Gln Gln Gln Trp Leu Leu Tyr Leu Ser Asn Ser Gly Tyr

1340 1345 1350
Leu Lys Val Leu Val Asp Ser Leu Val Glu Asp Asp Arg Thr Leu
1355 1360 1365
Gln Ser Leu Leu Thr Pro Gln Pro Pro Leu Leu Lys Ala Leu Tyr
1370 1375 1380
Thr Tyr Glu Ser Lys Met Ala Phe Leu Thr Arg Val Ala Lys Ile
1385 1390 1395
Gln Gln Gly Ala Leu Glu Leu Leu Arg Ser Gly Val Ile Val Arg
1400 1405 1410
Leu Ala Gln Cys Gln Val Tyr Asp Met Arg Pro Glu Thr Asp Pro
1415 1420 1425
Gln Ser Met Phe Gly Met Arg Asp Pro Pro Met Phe Ile Pro Thr
1430 1435 1440
Pro Val Asp Arg Tyr Arg Gln Ile Leu Leu Pro Ala Leu Gln Leu
1445 1450 1455
Cys Gln Val Ile Leu Thr Ser Ser Met Ala Gln His Leu Gln Ala
1460 1465 1470
Ala Gly Gln Val Leu Gln Phe Leu Ile Ser His Ser Asp Thr Ile
1475 1480 1485
Gln Ala Ile Leu Arg Cys Gln Asp Val Ser Ala Gly Ser Leu Gln
1490 1495 1500
Glu Leu Ala Leu Leu Thr Gly Ile Ile Ser Lys Ala Ala Leu Pro
1505 1510 1515
Gly Ile Leu Ser Glu Leu Asp Val Asp Val Asn Glu Gly Ser Leu
1520 1525 1530
Met Glu Leu Gln Gly His Ile Gly Arg Phe Gln Arg Gln Cys Leu
1535 1540 1545
Gly Leu Leu Ser Arg Phe Gly Gly Ser Asp Arg Leu Arg Gln Phe
1550 1555 1560
Lys Phe Gln Asp Asp Asn Val Glu Gly Asp Lys Val Ser Lys Lys
1565 1570 1575
Asp Glu Ile Glu Leu Ala Met Gln Gln Ile Cys Ala Asn Val Met
1580 1585 1590
Glu Tyr Cys Gln Ser Leu Met Leu Gln Ser Ser Pro Thr Phe Gln
1595 1600 1605
His Ala Val Cys Leu Phe Thr Pro Ser Leu Ser Glu Thr Val Asn
1610 1615 1620
Arg Asp Gly Pro Arg Gln Asp Thr Gln Ala Pro Val Val Pro Tyr
1625 1630 1635
Trp Arg Leu Pro Gly Leu Gly Ile Ile Ile Tyr Leu Leu Lys Gln
1640 1645 1650
Ser Ala Asn Asp Phe Phe Ser Tyr Tyr Asp Ser His Arg Gln Ser
1655 1660 1665
Val Ser Lys Leu Gln Asn Val Glu Gln Leu Pro Pro Asp Glu Ile
1670 1675 1680
Lys Glu Leu Cys Gln Ser Val Met Pro Ala Gly Val Asp Lys Ile
1685 1690 1695
Ser Thr Ala Gln Lys Tyr Val Leu Ala Arg Arg Arg Leu Val Lys
1700 1705 1710
Val Ile Asn Asn Arg Ala Lys Leu Leu Ser Leu Cys Ser Phe Ile
1715 1720 1725
Ile Glu Thr Cys Leu Phe Ile Leu Trp Arg His Leu Glu Tyr Tyr
1730 1735 1740
Leu Leu His Cys Met Pro Thr Asp Ser Gln Asp Ser Leu Phe Ala
1745 1750 1755
Ser Arg Thr Leu Phe Lys Ser Arg Arg Leu Gln Asp Ser Phe Ala
1760 1765 1770
Ser Glu Thr Asn Leu Asp Phe Arg Ser Gly Leu Ala Ile Val Ser
1775 1780 1785
Gln His Asp Leu Asp Gln Leu Gln Ala Asp Ala Ile Asn Ala Phe
1790 1795 1800
Gly Glu Ser Leu Gln Lys Lys Leu Leu Asp Ile Glu Gly Leu Tyr
1805 1810 1815
Ser Lys Val Arg Ser Arg Tyr Ser Phe Ile Gln Ala Leu Val Arg
1820 1825 1830
Arg Ile Arg Gly Leu Leu Arg Ile Ser Arg Asn
1835 1840

<210> 260

<211> 1299
<212> PRT
<213> Homo sapiens
<400> 260

```
Met Ala Ala Glu Thr Gln Thr Leu Asn Phe Gly Pro Glu Trp Leu Arg
1               5                   10                  15
Ala Leu Ser Ser Gly Gly Ser Ile Thr Ser Pro Pro Leu Ser Pro Ala
            20                  25                  30
Leu Pro Lys Tyr Lys Leu Ala Asp Tyr Arg Tyr Gly Arg Glu Glu Met
        35                  40                  45
Leu Ala Leu Phe Leu Lys Asp Asn Lys Ile Pro Ser Asp Leu Leu Asp
    50                  55                  60
Lys Glu Phe Leu Pro Ile Leu Gln Glu Glu Pro Leu Pro Pro Leu Ala
65                  70                  75                  80
Leu Val Pro Phe Thr Glu Glu Glu Gln Arg Asn Phe Ser Met Ser Val
                85                  90                  95
Asn Ser Ala Ala Val Leu Arg Leu Thr Gly Arg Gly Gly Gly Gly Thr
            100                 105                 110
Val Val Gly Ala Pro Arg Gly Arg Ser Ser Ser Arg Gly Arg Gly Arg
        115                 120                 125
Gly Arg Gly Glu Cys Gly Phe Tyr Gln Arg Ser Phe Asp Glu Val Glu
    130                 135                 140
Gly Val Phe Gly Arg Gly Gly Gly Arg Glu Met His Arg Ser Gln Ser
145                 150                 155                 160
Trp Glu Glu Arg Gly Asp Arg Arg Phe Glu Lys Pro Gly Arg Lys Asp
                165                 170                 175
Val Gly Arg Pro Asn Phe Glu Glu Gly Gly Pro Thr Ser Val Gly Arg
            180                 185                 190
Lys His Glu Phe Ile Arg Ser Glu Ser Glu Asn Trp Arg Ile Phe Arg
        195                 200                 205
Glu Glu Gln Asn Gly Glu Asp Glu Asp Gly Gly Trp Arg Leu Ala Gly
    210                 215                 220
Ser Arg Arg Asp Gly Glu Arg Trp Arg Pro His Ser Pro Asp Gly Pro
225                 230                 235                 240
Arg Ser Ala Gly Trp Arg Glu His Met Glu Arg Arg Arg Arg Phe Glu
                245                 250                 255
Phe Asp Phe Arg Asp Arg Asp Asp Glu Arg Gly Tyr Arg Arg Val Arg
            260                 265                 270
Ser Gly Ser Gly Ser Ile Asp Asp Asp Arg Asp Ser Leu Pro Glu Trp
        275                 280                 285
Cys Leu Glu Asp Ala Glu Glu Glu Met Gly Thr Phe Asp Ser Ser Gly
    290                 295                 300
Ala Phe Leu Ser Leu Lys Lys Val Gln Lys Glu Pro Ile Pro Glu Glu
305                 310                 315                 320
Gln Glu Met Asp Phe Arg Pro Val Asp Glu Gly Glu Glu Cys Ser Asp
                325                 330                 335
Ser Glu Gly Ser His Asn Glu Glu Ala Lys Glu Pro Asp Lys Thr Asn
            340                 345                 350
Lys Lys Glu Gly Glu Lys Thr Asp Arg Val Gly Val Glu Ala Ser Glu
        355                 360                 365
Glu Thr Pro Gln Thr Ser Ser Ser Ser Ala Arg Pro Gly Thr Pro Ser
    370                 375                 380
Asp His Gln Ser Gln Glu Ala Ser Gln Phe Glu Arg Lys Asp Glu Pro
385                 390                 395                 400
Lys Thr Glu Gln Thr Glu Lys Ala Glu Glu Glu Thr Arg Met Glu Asn
                405                 410                 415
Ser Leu Pro Ala Lys Val Pro Ser Arg Gly Asp Glu Met Val Ala Asp
            420                 425                 430
Val Gln Gln Pro Leu Ser Gln Ile Pro Ser Asp Thr Ala Ser Pro Leu
        435                 440                 445
Leu Ile Leu Pro Pro Pro Val Pro Asn Pro Ser Pro Thr Leu Arg Pro
    450                 455                 460
Val Glu Thr Pro Val Val Gly Ala Pro Gly Met Gly Ser Val Ser Thr
465                 470                 475                 480
Glu Pro Asp Asp Glu Glu Gly Leu Lys His Leu Glu Gln Gln Ala Glu
                485                 490                 495
Lys Met Val Ala Tyr Leu Gln Asp Ser Ala Leu Asp Asp Glu Arg Leu
            500                 505                 510
Ala Ser Lys Leu Gln Glu His Arg Ala Lys Gly Val Ser Ile Pro Leu
```

515 520 525
Met His Glu Ala Met Gln Lys Trp Tyr Tyr Lys Asp Pro Gln Gly Glu
530 535 540
Ile Gln Gly Pro Phe Asn Asn Gln Glu Met Ala Glu Trp Phe Gln Ala
545 550 555 560
Gly Tyr Phe Thr Met Ser Leu Leu Val Lys Arg Ala Cys Asp Glu Ser
565 570 575
Phe Gln Pro Leu Gly Asp Ile Met Lys Met Trp Gly Arg Val Pro Phe
580 585 590
Ser Pro Gly Pro Ala Pro Pro Pro His Met Gly Glu Leu Asp Gln Glu
595 600 605
Arg Leu Thr Arg Gln Gln Glu Leu Thr Ala Leu Tyr Gln Met Gln His
610 615 620
Leu Gln Tyr Gln Gln Phe Leu Ile Gln Gln Gln Tyr Ala Gln Val Leu
625 630 635 640
Ala Gln Gln Gln Lys Ala Ala Leu Ser Ser Gln Gln Gln Gln Gln Leu
645 650 655
Ala Leu Leu Leu Gln Gln Phe Gln Thr Leu Lys Met Arg Ile Ser Asp
660 665 670
Gln Asn Ile Ile Pro Ser Val Thr Arg Ser Val Ser Val Pro Asp Thr
675 680 685
Gly Ser Ile Trp Glu Leu Gln Pro Thr Ala Ser Gln Pro Thr Val Trp
690 695 700
Glu Gly Gly Ser Val Trp Asp Leu Pro Leu Asp Thr Thr Thr Pro Gly
705 710 715 720
Pro Ala Leu Glu Gln Leu Gln Gln Leu Glu Lys Ala Lys Ala Ala Lys
725 730 735
Leu Glu Gln Glu Arg Arg Glu Ala Glu Met Arg Ala Lys Arg Glu Glu
740 745 750
Glu Glu Arg Lys Arg Gln Glu Glu Leu Arg Arg Gln Gln Glu Glu Ile
755 760 765
Leu Arg Arg Gln Gln Glu Glu Glu Arg Lys Arg Arg Glu Glu Glu Glu
770 775 780
Leu Ala Arg Arg Lys Gln Glu Gly Ala Leu Arg Arg Gln Arg Glu Gln
785 790 795 800
Glu Ile Ala Leu Arg Arg Gln Arg Glu Glu Glu Glu Arg Gln Gln Gln
805 810 815
Glu Glu Ala Leu Arg Arg Leu Glu Glu Arg Arg Arg Glu Glu Glu Glu
820 825 830
Arg Arg Lys Gln Glu Glu Leu Leu Arg Lys Gln Glu Glu Glu Ala Ala
835 840 845
Lys Trp Ala Arg Glu Glu Glu Glu Ala Gln Arg Arg Leu Glu Glu Asn
850 855 860
Arg Leu Arg Met Glu Glu Glu Ala Ala Arg Leu Arg His Glu Glu Glu
865 870 875 880
Glu Arg Lys Arg Lys Glu Leu Glu Val Gln Arg Gln Lys Glu Leu Met
885 890 895
Arg Gln Arg Gln Gln Gln Gln Glu Ala Leu Arg Arg Leu Gln Gln Gln
900 905 910
Gln Gln Gln Gln Gln Leu Ala Gln Met Lys Leu Pro Ser Ser Ser Thr
915 920 925
Trp Gly Gln Gln Ser Asn Thr Thr Ala Cys Gln Ser Gln Ala Thr Leu
930 935 940
Ser Leu Ala Glu Ile Gln Lys Leu Glu Glu Glu Arg Glu Arg Gln Leu
945 950 955 960
Arg Glu Glu Gln Arg Arg Gln Gln Arg Glu Leu Met Lys Ala Leu Gln
965 970 975
Gln Gln Gln Gln Gln Gln Gln Gln Lys Leu Ser Gly Trp Gly Asn Val
980 985 990
Ser Lys Pro Ser Gly Thr Thr Lys Ser Leu Leu Glu Ile Gln Gln Glu
995 1000 1005
Glu Ala Arg Gln Met Gln Lys Gln Gln Gln Gln Gln Gln Gln His
1010 1015 1020
Gln Gln Pro Asn Arg Ala Arg Asn Asn Thr His Ser Asn Leu His
1025 1030 1035
Thr Ser Ile Gly Asn Ser Val Trp Gly Ser Ile Asn Thr Gly Pro
1040 1045 1050
Pro Asn Gln Trp Ala Ser Asp Leu Val Ser Ser Ile Trp Ser Asn
1055 1060 1065

Ala Asp Thr Lys Asn Ser Asn Met Gly Phe Trp Asp Asp Ala Val
1070 1075 1080
Lys Glu Val Gly Pro Arg Asn Ser Thr Asn Lys Asn Lys Asn Asn
1085 1090 1095
Ala Ser Leu Ser Lys Ser Val Gly Val Ser Asn Arg Gln Asn Lys
1100 1105 1110
Lys Val Glu Glu Glu Glu Lys Leu Leu Lys Leu Phe Gln Gly Val
1115 1120 1125
Asn Lys Ala Gln Asp Gly Phe Thr Gln Trp Cys Glu Gln Met Leu
1130 1135 1140
His Ala Leu Asn Thr Ala Asn Asn Leu Gly Val Pro Thr Phe Val
1145 1150 1155
Ser Phe Leu Lys Glu Val Glu Ser Pro Tyr Glu Val His Asp Tyr
1160 1165 1170
Ile Arg Ala Tyr Leu Gly Asp Thr Ser Glu Ala Lys Glu Phe Ala
1175 1180 1185
Lys Gln Phe Leu Glu Arg Arg Ala Lys Gln Lys Ala Asn Gln Gln
1190 1195 1200
Arg Gln Gln Gln Gln Leu Pro Gln Gln Gln Gln Gln Gln Pro Pro
1205 1210 1215
Gln Gln Pro Pro Gln Gln Pro Gln Gln Gln Asp Ser Val Trp Gly
1220 1225 1230
Met Asn His Ser Thr Leu His Ser Val Phe Gln Thr Asn Gln Ser
1235 1240 1245
Asn Asn Gln Gln Ser Asn Phe Glu Ala Val Gln Ser Gly Lys Lys
1250 1255 1260
Lys Lys Lys Gln Lys Met Val Arg Ala Asp Pro Ser Leu Leu Gly
1265 1270 1275
Phe Ser Val Asn Ala Ser Ser Glu Arg Leu Asn Met Gly Glu Ile
1280 1285 1290
Glu Thr Leu Asp Asp Tyr
1295

<210> 261
<211> 107
<212> PRT
<213> Homo sapiens
<400> 261
Met Ser Gly Cys Arg Val Phe Ile Gly Arg Leu Asn Pro Ala Ala Arg
1 5 10 15
Glu Lys Asp Val Glu Arg Phe Phe Lys Gly Tyr Gly Arg Ile Arg Asp
20 25 30
Ile Asp Leu Lys Arg Gly Phe Gly Phe Val Glu Phe Glu Asp Pro Arg
35 40 45
Asp Ala Asp Asp Ala Val Tyr Glu Leu Asp Gly Lys Glu Leu Cys Ser
50 55 60
Glu Arg Val Thr Ile Glu His Ala Arg Ala Arg Ser Arg Gly Gly Arg
65 70 75 80
Gly Arg Gly Arg Tyr Ser Asp Arg Phe Ser Ser Arg Arg Pro Arg Asn
85 90 95
Asp Arg Arg Tyr Val Lys Gly Gly Trp Leu His
100 105

<210> 262
<211> 184
<212> PRT
<213> Homo sapiens
<400> 262
Met Val Arg Tyr Ser Leu Asp Pro Glu Asn Pro Thr Lys Ser Cys Lys
1 5 10 15
Ser Arg Gly Ser Asn Leu Arg Val His Phe Lys Asn Thr Arg Glu Thr
20 25 30
Ala Gln Ala Ile Lys Gly Met His Ile Arg Lys Ala Thr Lys Tyr Leu
35 40 45
Lys Asp Val Thr Leu Gln Lys Gln Cys Val Pro Phe Arg Arg Tyr Asn
50 55 60
Gly Gly Val Gly Arg Cys Ala Gln Ala Lys Gln Trp Gly Trp Thr Gln
65 70 75 80
Gly Arg Trp Pro Lys Lys Ser Ala Glu Phe Leu Leu His Met Leu Lys

```
                85                      90                      95
Asn Ala Glu Ser Asn Ala Glu Leu Lys Gly Leu Asp Val Asp Ser Leu
            100                     105                     110
Val Ile Glu His Ile Gln Val Asn Lys Ala Pro Lys Met Arg Arg Arg
        115                     120                     125
Thr Tyr Arg Ala His Gly Arg Ile Asn Pro Tyr Met Ser Ser Pro Cys
    130                     135                     140
His Ile Glu Met Ile Leu Thr Glu Lys Glu Gln Ile Val Pro Lys Pro
145                     150                     155                     160
Glu Glu Glu Val Ala Gln Lys Lys Lys Ile Ser Gln Lys Lys Leu Lys
                165                     170                     175
Lys Gln Lys Leu Met Ala Arg Glu
            180

<210>  263
<211>  72
<212>  PRT
<213>  Homo sapiens
<400>  263
Met Ser Ser Lys Thr Ala Ser Thr Asn Asn Ile Ala Gln Ala Arg Arg
1               5                   10                      15
Thr Val Gln Gln Leu Arg Leu Glu Ala Ser Ile Glu Arg Ile Lys Val
            20                  25                      30
Ser Lys Ala Ser Ala Asp Leu Met Ser Tyr Cys Glu Glu His Ala Arg
        35                  40                      45
Ser Asp Pro Leu Leu Ile Gly Ile Pro Thr Ser Glu Asn Pro Phe Lys
    50                  55                      60
Asp Lys Lys Thr Cys Ile Ile Leu
65                  70

<210>  264
<211>  462
<212>  PRT
<213>  Homo sapiens
<400>  264
Met Lys Thr Arg Arg Thr Thr Arg Leu Gln Gln Gln His Ser Glu Gln
1               5                   10                      15
Pro Pro Leu Gln Pro Ser Pro Val Thr Thr Arg Arg Gly Leu Arg Asp
            20                  25                      30
Ser His Ser Ser Glu Glu Asp Glu Ala Ser Ser Gln Thr Asp Leu Ser
        35                  40                      45
Gln Thr Ile Ser Lys Lys Thr Val Arg Ser Ile Gln Glu Ala Pro Val
    50                  55                      60
Ser Glu Asp Leu Val Ile Arg Leu Arg Arg Pro Pro Leu Arg Cys Pro
65                  70                      75                      80
Arg Tyr Glu Ala Thr Ser Val Gln Gln Lys Val Asn Phe Ser Glu Glu
                85                      90                      95
Gly Glu Thr Glu Glu Asp Asp Gln Asp Ser Ser His Ser Ser Val Thr
            100                     105                     110
Thr Val Lys Ala Arg Ser Arg Asp Ser Asp Glu Ser Gly Asp Lys Thr
        115                     120                     125
Thr Arg Ser Ser Ser Gln Tyr Ile Glu Ser Phe Trp Gln Ser Ser Gln
    130                     135                     140
Ser Gln Asn Phe Thr Ala His Asp Lys Gln Arg Ser Val Leu Ser Ser
145                     150                     155                     160
Gly Tyr Gln Lys Thr Pro Gln Glu Trp Ala Pro Gln Thr Ala Arg Ile
                165                     170                     175
Arg Thr Arg Met Gln Asn Asp Ser Ile Leu Lys Ser Glu Leu Gly Asn
            180                     185                     190
Gln Ser Pro Ser Thr Ser Ser Arg Gln Val Thr Gly Gln Pro Gln Asn
        195                     200                     205
Ala Ser Phe Val Lys Arg Asn Arg Trp Trp Leu Leu Pro Leu Ile Ala
    210                     215                     220
Ala Leu Ala Ser Gly Ser Phe Trp Phe Phe Ser Thr Pro Glu Val Glu
225                     230                     235                     240
Thr Thr Ala Val Gln Glu Phe Gln Asn Gln Met Asn Gln Leu Lys Asn
                245                     250                     255
Lys Tyr Gln Gly Gln Asp Glu Lys Leu Trp Lys Arg Ser Gln Thr Phe
            260                     265                     270
```

Leu Glu Lys His Leu Asn Ser Ser His Pro Arg Ser Gln Pro Ala Ile
275 280 285
Leu Leu Leu Thr Ala Ala Arg Asp Ala Glu Glu Ala Leu Arg Cys Leu
290 295 300
Ser Glu Gln Ile Ala Asp Ala Tyr Ser Ser Phe Arg Ser Val Arg Ala
305 310 315 320
Ile Arg Ile Asp Gly Thr Asp Lys Ala Thr Gln Asp Ser Asp Thr Val
325 330 335
Lys Leu Glu Val Asp Gln Glu Leu Ser Asn Gly Phe Lys Asn Gly Gln
340 345 350
Asn Ala Ala Val Val His Arg Phe Glu Ser Phe Pro Ala Gly Ser Thr
355 360 365
Leu Ile Phe Tyr Lys Tyr Cys Asp His Glu Asn Ala Ala Phe Lys Asp
370 375 380
Val Ala Leu Val Leu Thr Val Leu Leu Glu Glu Thr Leu Gly Thr
385 390 395 400
Ser Leu Gly Leu Lys Glu Val Glu Glu Lys Val Arg Asp Phe Leu Lys
405 410 415
Val Lys Phe Thr Asn Ser Asn Thr Pro Asn Ser Tyr Asn His Met Asp
420 425 430
Pro Asp Lys Leu Asn Gly Leu Trp Ser Arg Ile Ser His Leu Val Leu
435 440 445
Pro Val Gln Pro Glu Asn Ala Leu Lys Arg Gly Ile Cys Leu
450 455 460

<210> 265
<211> 192
<212> PRT
<213> Homo sapiens
<400> 265
Met Phe Ser Gln Phe Thr Asp Ile Lys Arg Lys Asp Phe Gly Ile Met
1 5 10 15
Phe Leu His His Leu Val Ser Ile Phe Leu Ile Thr Phe Ser Tyr Val
20 25 30
Asn Asn Met Ala Arg Val Gly Thr Leu Val Leu Cys Leu His Asp Ser
35 40 45
Ala Asp Ala Leu Leu Glu Ala Ala Lys Met Ala Asn Tyr Ala Lys Phe
50 55 60
Gln Lys Met Cys Asp Leu Leu Phe Val Met Phe Ala Val Val Phe Ile
65 70 75 80
Thr Thr Arg Leu Gly Ile Phe Pro Leu Trp Val Leu Asn Thr Thr Leu
85 90 95
Phe Glu Ser Trp Glu Ile Val Gly Pro Tyr Pro Ser Trp Trp Val Phe
100 105 110
Asn Leu Leu Leu Leu Leu Val Gln Gly Leu Asn Cys Phe Trp Ser Tyr
115 120 125
Leu Ile Val Lys Ile Ala Cys Lys Ala Val Ser Arg Gly Lys Val Ser
130 135 140
Lys Asp Asp Arg Ser Asp Ile Glu Ser Ser Ser Asp Glu Glu Asp Ser
145 150 155 160
Glu Pro Pro Gly Lys Asn Pro His Thr Ala Thr Thr Thr Asn Gly Thr
165 170 175
Ser Gly Thr Asn Gly Tyr Leu Leu Thr Gly Ser Cys Ser Met Asp Asp
180 185 190

<210> 266
<211> 1838
<212> PRT
<213> Homo sapiens
<400> 266
Met Asp Val His Thr Arg Trp Lys Ala Arg Ser Ala Leu Arg Pro Gly
1 5 10 15
Ala Pro Leu Leu Pro Pro Leu Leu Leu Leu Leu Leu Trp Ala Pro Pro
20 25 30
Pro Ser Arg Ala Ala Gln Pro Ala Asp Leu Leu Lys Val Leu Asp Phe
35 40 45
His Asn Leu Pro Asp Gly Ile Thr Lys Thr Thr Gly Phe Cys Ala Thr
50 55 60
Arg Arg Ser Ser Lys Gly Pro Asp Val Ala Tyr Arg Val Thr Lys Asp

```
65                  70                  75                  80
Ala Gln Leu Ser Ala Pro Thr Lys Gln Leu Tyr Pro Ala Ser Ala Phe
                85                  90                  95
Pro Glu Asp Phe Ser Ile Leu Thr Thr Val Lys Ala Lys Lys Gly Ser
            100                 105                 110
Gln Ala Phe Leu Val Ser Ile Tyr Asn Glu Gln Gly Ile Gln Gln Ile
        115                 120                 125
Gly Leu Glu Leu Gly Arg Ser Pro Val Phe Leu Tyr Glu Asp His Thr
    130                 135                 140
Gly Lys Pro Gly Pro Glu Asp Tyr Pro Leu Phe Arg Gly Ile Asn Leu
145                 150                 155                 160
Ser Asp Gly Lys Trp His Arg Ile Ala Leu Ser Val His Lys Lys Asn
                165                 170                 175
Val Thr Leu Ile Leu Asp Cys Lys Lys Lys Thr Thr Lys Phe Leu Asp
            180                 185                 190
Arg Ser Asp His Pro Met Ile Asp Ile Asn Gly Ile Ile Val Phe Gly
        195                 200                 205
Thr Arg Ile Leu Asp Glu Glu Val Phe Glu Gly Asp Ile Gln Gln Leu
    210                 215                 220
Leu Phe Val Ser Asp His Arg Ala Ala Tyr Asp Tyr Cys Glu His Tyr
225                 230                 235                 240
Ser Pro Asp Cys Asp Thr Ala Val Pro Asp Thr Pro Gln Ser Gln Asp
                245                 250                 255
Pro Asn Pro Asp Glu Tyr Tyr Thr Glu Gly Asp Gly Glu Gly Glu Thr
            260                 265                 270
Tyr Tyr Tyr Glu Tyr Pro Tyr Tyr Glu Asp Pro Glu Asp Leu Gly Lys
        275                 280                 285
Glu Pro Thr Pro Ser Lys Lys Pro Val Glu Ala Ala Lys Glu Thr Thr
    290                 295                 300
Glu Val Pro Glu Glu Leu Thr Pro Thr Pro Thr Glu Ala Ala Pro Met
305                 310                 315                 320
Pro Glu Thr Ser Glu Gly Ala Gly Lys Glu Glu Asp Val Gly Ile Gly
                325                 330                 335
Asp Tyr Asp Tyr Val Pro Ser Glu Asp Tyr Tyr Thr Pro Ser Pro Tyr
            340                 345                 350
Asp Asp Leu Thr Tyr Gly Glu Gly Glu Glu Asn Pro Asp Gln Pro Thr
        355                 360                 365
Asp Pro Gly Ala Gly Ala Glu Ile Pro Thr Ser Thr Ala Asp Thr Ser
    370                 375                 380
Asn Ser Ser Asn Pro Ala Pro Pro Pro Gly Glu Gly Ala Asp Asp Leu
385                 390                 395                 400
Glu Gly Glu Phe Thr Glu Glu Thr Ile Arg Asn Leu Asp Glu Asn Tyr
                405                 410                 415
Tyr Asp Pro Tyr Tyr Asp Pro Thr Ser Ser Pro Ser Glu Ile Gly Pro
            420                 425                 430
Gly Met Pro Ala Asn Gln Asp Thr Ile Tyr Glu Gly Ile Gly Gly Pro
        435                 440                 445
Arg Gly Glu Lys Gly Gln Lys Gly Glu Pro Ala Ile Ile Glu Pro Gly
    450                 455                 460
Met Leu Ile Glu Gly Pro Pro Gly Pro Glu Gly Pro Ala Gly Leu Pro
465                 470                 475                 480
Gly Pro Pro Gly Thr Met Gly Pro Thr Gly Gln Val Gly Asp Pro Gly
                485                 490                 495
Glu Arg Gly Pro Pro Gly Arg Pro Gly Leu Pro Gly Ala Asp Gly Leu
            500                 505                 510
Pro Gly Pro Pro Gly Thr Met Leu Met Leu Pro Phe Arg Phe Gly Gly
        515                 520                 525
Gly Gly Asp Ala Gly Ser Lys Gly Pro Met Val Ser Ala Gln Glu Ser
    530                 535                 540
Gln Ala Gln Ala Ile Leu Gln Gln Ala Arg Leu Ala Leu Arg Gly Pro
545                 550                 555                 560
Ala Gly Pro Met Gly Leu Thr Gly Arg Pro Gly Pro Val Gly Pro Pro
                565                 570                 575
Gly Ser Gly Gly Leu Lys Gly Glu Pro Gly Asp Val Gly Pro Gln Gly
            580                 585                 590
Pro Arg Gly Val Gln Gly Pro Pro Gly Pro Ala Gly Lys Pro Gly Arg
        595                 600                 605
Arg Gly Arg Ala Gly Ser Asp Gly Ala Arg Gly Met Pro Gly Gln Thr
    610                 615                 620
```

Gly Pro Lys Gly Asp Arg Gly Phe Asp Gly Leu Ala Gly Leu Pro Gly
625 630 635 640
Glu Lys Gly His Arg Gly Asp Pro Gly Pro Ser Gly Pro Pro Gly Pro
645 650 655
Pro Gly Asp Asp Gly Glu Arg Gly Asp Asp Gly Glu Val Gly Pro Arg
660 665 670
Gly Leu Pro Gly Glu Pro Gly Pro Arg Gly Leu Leu Gly Pro Lys Gly
675 680 685
Pro Pro Gly Pro Pro Gly Pro Pro Gly Val Thr Gly Met Asp Gly Gln
690 695 700
Pro Gly Pro Lys Gly Asn Val Gly Pro Gln Gly Glu Pro Gly Pro Pro
705 710 715 720
Gly Gln Gln Gly Asn Pro Gly Ala Gln Gly Leu Pro Gly Pro Gln Gly
725 730 735
Ala Ile Gly Pro Pro Gly Glu Lys Gly Pro Leu Gly Lys Pro Gly Leu
740 745 750
Pro Gly Met Pro Gly Ala Asp Gly Pro Pro Gly His Pro Gly Lys Glu
755 760 765
Gly Pro Pro Gly Glu Lys Gly Gly Gln Gly Pro Pro Gly Pro Gln Gly
770 775 780
Pro Ile Gly Tyr Pro Gly Pro Arg Gly Val Lys Gly Ala Asp Gly Ile
785 790 795 800
Arg Gly Leu Lys Gly Thr Lys Gly Glu Lys Gly Glu Asp Gly Phe Pro
805 810 815
Gly Phe Lys Gly Asp Met Gly Ile Lys Gly Asp Arg Gly Glu Ile Gly
820 825 830
Pro Pro Gly Pro Arg Gly Glu Asp Gly Pro Glu Gly Pro Lys Gly Arg
835 840 845
Gly Gly Pro Asn Gly Asp Pro Gly Pro Leu Gly Pro Pro Gly Glu Lys
850 855 860
Gly Lys Leu Gly Val Pro Gly Leu Pro Gly Tyr Pro Gly Arg Gln Gly
865 870 875 880
Pro Lys Gly Ser Ile Gly Phe Pro Gly Phe Pro Gly Ala Asn Gly Glu
885 890 895
Lys Gly Gly Arg Gly Thr Pro Gly Lys Pro Gly Pro Arg Gly Gln Arg
900 905 910
Gly Pro Thr Gly Pro Arg Gly Glu Arg Gly Pro Arg Gly Ile Thr Gly
915 920 925
Lys Pro Gly Pro Lys Gly Asn Ser Gly Gly Asp Gly Pro Ala Gly Pro
930 935 940
Pro Gly Glu Arg Gly Pro Asn Gly Pro Gln Gly Pro Thr Gly Phe Pro
945 950 955 960
Gly Pro Lys Gly Pro Pro Gly Pro Pro Gly Lys Asp Gly Leu Pro Gly
965 970 975
His Pro Gly Gln Arg Gly Glu Thr Gly Phe Gln Gly Lys Thr Gly Pro
980 985 990
Pro Gly Pro Pro Gly Val Val Gly Pro Gln Gly Pro Thr Gly Glu Thr
995 1000 1005
Gly Pro Met Gly Glu Arg Gly His Pro Gly Pro Pro Gly Pro Pro
1010 1015 1020
Gly Glu Gln Gly Leu Pro Gly Leu Ala Gly Lys Glu Gly Thr Lys
1025 1030 1035
Gly Asp Pro Gly Pro Ala Gly Leu Pro Gly Lys Asp Gly Pro Pro
1040 1045 1050
Gly Leu Arg Gly Phe Pro Gly Asp Arg Gly Leu Pro Gly Pro Val
1055 1060 1065
Gly Ala Leu Gly Leu Lys Gly Asn Glu Gly Pro Pro Gly Pro Pro
1070 1075 1080
Gly Pro Ala Gly Ser Pro Gly Glu Arg Gly Pro Ala Gly Ala Ala
1085 1090 1095
Gly Pro Ile Gly Ile Pro Gly Arg Pro Gly Pro Gln Gly Pro Pro
1100 1105 1110
Gly Pro Ala Gly Glu Lys Gly Ala Pro Gly Glu Lys Gly Pro Gln
1115 1120 1125
Gly Pro Ala Gly Arg Asp Gly Leu Gln Gly Pro Val Gly Leu Pro
1130 1135 1140
Gly Pro Ala Gly Pro Val Gly Pro Pro Gly Glu Asp Gly Asp Lys
1145 1150 1155
Gly Glu Ile Gly Glu Pro Gly Gln Lys Gly Ser Lys Gly Asp Lys

1160 1165 1170
Gly Glu Gln Gly Pro Pro Gly Pro Thr Gly Pro Gln Gly Pro Ile
1175 1180 1185
Gly Gln Pro Gly Pro Ser Gly Ala Asp Gly Glu Pro Gly Pro Arg
1190 1195 1200
Gly Gln Gln Gly Leu Phe Gly Gln Lys Gly Asp Glu Gly Pro Arg
1205 1210 1215
Gly Phe Pro Gly Pro Pro Gly Pro Val Gly Leu Gln Gly Leu Pro
1220 1225 1230
Gly Pro Pro Gly Glu Lys Gly Glu Thr Gly Asp Val Gly Gln Met
1235 1240 1245
Gly Pro Pro Gly Pro Pro Gly Pro Arg Gly Pro Ser Gly Ala Pro
1250 1255 1260
Gly Ala Asp Gly Pro Gln Gly Pro Pro Gly Gly Ile Gly Asn Pro
1265 1270 1275
Gly Ala Val Gly Glu Lys Gly Glu Pro Gly Glu Ala Gly Glu Pro
1280 1285 1290
Gly Pro Ser Gly Arg Ser Gly Pro Pro Gly Pro Lys Gly Glu Arg
1295 1300 1305
Gly Glu Lys Gly Glu Ser Gly Pro Ser Gly Ala Ala Gly Pro Pro
1310 1315 1320
Gly Pro Lys Gly Pro Pro Gly Asp Asp Gly Pro Lys Gly Ser Pro
1325 1330 1335
Gly Pro Val Gly Phe Pro Gly Asp Pro Gly Pro Pro Gly Glu Pro
1340 1345 1350
Gly Pro Ala Gly Gln Asp Gly Pro Pro Gly Asp Lys Gly Asp Asp
1355 1360 1365
Gly Glu Pro Gly Gln Thr Gly Ser Pro Gly Pro Thr Gly Glu Pro
1370 1375 1380
Gly Pro Ser Gly Pro Pro Gly Lys Arg Gly Pro Pro Gly Pro Ala
1385 1390 1395
Gly Pro Glu Gly Arg Gln Gly Glu Lys Gly Ala Lys Gly Glu Ala
1400 1405 1410
Gly Leu Glu Gly Pro Pro Gly Lys Thr Gly Pro Ile Gly Pro Gln
1415 1420 1425
Gly Ala Pro Gly Lys Pro Gly Pro Asp Gly Leu Arg Gly Ile Pro
1430 1435 1440
Gly Pro Val Gly Glu Gln Gly Leu Pro Gly Ser Pro Gly Pro Asp
1445 1450 1455
Gly Pro Pro Gly Pro Met Gly Pro Pro Gly Leu Pro Gly Leu Lys
1460 1465 1470
Gly Asp Ser Gly Pro Lys Gly Glu Lys Gly His Pro Gly Leu Ile
1475 1480 1485
Gly Leu Ile Gly Pro Pro Gly Glu Gln Gly Glu Lys Gly Asp Arg
1490 1495 1500
Gly Leu Pro Gly Pro Gln Gly Ser Ser Gly Pro Lys Gly Glu Gln
1505 1510 1515
Gly Ile Thr Gly Pro Ser Gly Pro Ile Gly Pro Pro Gly Pro Pro
1520 1525 1530
Gly Leu Pro Gly Pro Pro Gly Pro Lys Gly Ala Lys Gly Ser Ser
1535 1540 1545
Gly Pro Thr Gly Pro Lys Gly Glu Ala Gly His Pro Gly Pro Pro
1550 1555 1560
Gly Pro Pro Gly Pro Pro Gly Glu Val Ile Gln Pro Leu Pro Ile
1565 1570 1575
Gln Ala Ser Arg Thr Arg Arg Asn Ile Asp Ala Ser Gln Leu Leu
1580 1585 1590
Asp Asp Gly Asn Gly Glu Asn Tyr Val Asp Tyr Ala Asp Gly Met
1595 1600 1605
Glu Glu Ile Phe Gly Ser Leu Asn Ser Leu Lys Leu Glu Ile Glu
1610 1615 1620
Gln Met Lys Arg Pro Leu Gly Thr Gln Gln Asn Pro Ala Arg Thr
1625 1630 1635
Cys Lys Asp Leu Gln Leu Cys His Pro Asp Phe Pro Asp Gly Glu
1640 1645 1650
Tyr Trp Val Asp Pro Asn Gln Gly Cys Ser Arg Asp Ser Phe Lys
1655 1660 1665
Val Tyr Cys Asn Phe Thr Ala Gly Gly Ser Thr Cys Val Phe Pro
1670 1675 1680

Asp Lys Lys Ser Glu Gly Ala Arg Ile Thr Ser Trp Pro Lys Glu
1685 1690 1695
Asn Pro Gly Ser Trp Phe Ser Glu Phe Lys Arg Gly Lys Leu Leu
1700 1705 1710
Ser Tyr Val Asp Ala Glu Gly Asn Pro Val Gly Val Val Gln Met
1715 1720 1725
Thr Phe Leu Arg Leu Leu Ser Ala Ser Ala His Gln Asn Val Thr
1730 1735 1740
Tyr His Cys Tyr Gln Ser Val Ala Trp Gln Asp Ala Ala Thr Gly
1745 1750 1755
Ser Tyr Asp Lys Ala Leu Arg Phe Leu Gly Ser Asn Asp Glu Glu
1760 1765 1770
Met Ser Tyr Asp Asn Asn Pro Tyr Ile Arg Ala Leu Val Asp Gly
1775 1780 1785
Cys Ala Thr Lys Lys Gly Tyr Gln Lys Thr Val Leu Glu Ile Asp
1790 1795 1800
Thr Pro Lys Val Glu Gln Val Pro Ile Val Asp Ile Met Phe Asn
1805 1810 1815
Asp Phe Gly Glu Ala Ser Gln Lys Phe Gly Phe Glu Val Gly Pro
1820 1825 1830
Ala Cys Phe Met Gly
1835

<210> 267
<211> 109
<212> PRT
<213> Homo sapiens
<400> 267
Met Lys Phe Ile Ser Thr Ser Leu Leu Leu Met Leu Leu Val Ser Ser
1 5 10 15
Leu Ser Pro Val Gln Gly Val Leu Glu Val Tyr Tyr Thr Ser Leu Arg
20 25 30
Cys Arg Cys Val Gln Glu Ser Ser Val Phe Ile Pro Arg Arg Phe Ile
35 40 45
Asp Arg Ile Gln Ile Leu Pro Arg Gly Asn Gly Cys Pro Arg Lys Glu
50 55 60
Ile Ile Val Trp Lys Lys Asn Lys Ser Ile Val Cys Val Asp Pro Gln
65 70 75 80
Ala Glu Trp Ile Gln Arg Met Met Glu Val Leu Arg Lys Arg Ser Ser
85 90 95
Ser Thr Leu Pro Val Pro Val Phe Lys Arg Lys Ile Pro
100 105

<210> 268
<211> 504
<212> PRT
<213> Homo sapiens
<400> 268
Met Phe Pro Arg Glu Lys Thr Trp Asn Ile Ser Phe Ala Gly Cys Gly
1 5 10 15
Phe Leu Gly Val Tyr Tyr Val Gly Val Ala Ser Cys Leu Arg Glu His
20 25 30
Ala Pro Phe Leu Val Ala Asn Ala Thr His Ile Tyr Gly Ala Ser Ala
35 40 45
Gly Ala Leu Thr Ala Thr Ala Leu Val Thr Gly Val Cys Leu Gly Glu
50 55 60
Ala Gly Ala Lys Phe Ile Glu Val Ser Lys Glu Ala Arg Lys Arg Phe
65 70 75 80
Leu Gly Pro Leu His Pro Ser Phe Asn Leu Val Lys Ile Ile Arg Ser
85 90 95
Phe Leu Leu Lys Val Leu Pro Ala Asp Ser His Glu His Ala Ser Gly
100 105 110
Arg Leu Gly Ile Ser Leu Thr Arg Val Ser Asp Gly Glu Asn Val Ile
115 120 125
Ile Ser His Phe Asn Ser Lys Asp Glu Leu Ile Gln Ala Asn Val Cys
130 135 140
Ser Gly Phe Ile Pro Val Tyr Cys Gly Leu Ile Pro Pro Ser Leu Gln
145 150 155 160
Gly Val Arg Tyr Val Asp Gly Gly Ile Ser Asp Asn Leu Pro Leu Tyr

165 170 175
Glu Leu Lys Asn Thr Ile Thr Val Ser Pro Phe Ser Gly Glu Ser Asp
180 185 190
Ile Cys Pro Gln Asp Ser Ser Thr Asn Ile His Glu Leu Arg Val Thr
195 200 205
Asn Thr Ser Ile Gln Phe Asn Leu Arg Asn Leu Tyr Arg Leu Ser Lys
210 215 220
Ala Leu Phe Pro Pro Glu Pro Leu Val Leu Arg Glu Met Cys Lys Gln
225 230 235 240
Gly Tyr Arg Asp Gly Leu Arg Phe Leu Gln Arg Asn Gly Leu Leu Asn
245 250 255
Arg Pro Asn Pro Leu Leu Ala Leu Pro Pro Ala Arg Pro His Gly Pro
260 265 270
Glu Asp Lys Asp Gln Ala Val Glu Ser Ala Gln Ala Glu Asp Tyr Ser
275 280 285
Gln Leu Pro Gly Glu Asp His Ile Leu Glu His Leu Pro Ala Arg Leu
290 295 300
Asn Glu Ala Leu Leu Glu Ala Cys Val Glu Pro Thr Asp Leu Leu Thr
305 310 315 320
Thr Leu Ser Asn Met Leu Pro Val Arg Leu Ala Thr Ala Met Met Val
325 330 335
Pro Tyr Thr Leu Pro Leu Glu Ser Ala Leu Ser Phe Thr Ile Arg Leu
340 345 350
Leu Glu Trp Leu Pro Asp Val Pro Glu Asp Ile Arg Trp Met Lys Glu
355 360 365
Gln Thr Gly Ser Ile Cys Gln Tyr Leu Val Met Arg Ala Lys Arg Lys
370 375 380
Leu Gly Arg His Leu Pro Ser Arg Leu Pro Glu Gln Val Glu Leu Arg
385 390 395 400
Arg Val Gln Ser Leu Pro Ser Val Pro Leu Ser Cys Ala Ala Tyr Arg
405 410 415
Glu Ala Leu Pro Gly Trp Met Arg Asn Asn Leu Ser Leu Gly Asp Ala
420 425 430
Leu Ala Lys Trp Glu Glu Cys Gln Arg Gln Leu Leu Leu Gly Leu Phe
435 440 445
Cys Thr Asn Val Ala Phe Pro Pro Glu Ala Leu Arg Met Arg Ala Pro
450 455 460
Ala Asp Pro Ala Pro Ala Pro Ala Asp Pro Ala Ser Pro Gln His Gln
465 470 475 480
Pro Ala Gly Pro Ala Pro Leu Leu Ser Thr Pro Ala Pro Glu Ala Arg
485 490 495
Pro Val Ile Gly Ala Leu Gly Leu
500

<210> 269
<211> 1498
<212> PRT
<213> Homo sapiens
<400> 269
Met Val Ala Leu Arg Gly Leu Gly Ser Gly Leu Gln Pro Trp Cys Pro
1 5 10 15
Leu Asp Leu Arg Leu Glu Trp Val Asp Thr Val Trp Glu Leu Asp Phe
20 25 30
Thr Glu Thr Glu Pro Leu Asp Pro Ser Ile Glu Ala Glu Ile Ile Glu
35 40 45
Thr Gly Leu Ala Ala Phe Thr Lys Leu Tyr Glu Ser Leu Leu Pro Phe
50 55 60
Ala Thr Gly Glu His Gly Ser Met Glu Ser Ile Trp Thr Phe Phe Ile
65 70 75 80
Glu Asn Asn Val Ser His Ser Thr Leu Val Ala Leu Phe Tyr His Phe
85 90 95
Val Gln Ile Val His Lys Lys Asn Val Ser Val Gln Tyr Arg Glu Tyr
100 105 110
Gly Leu His Ala Ala Gly Leu Tyr Phe Leu Leu Leu Glu Val Pro Gly
115 120 125
Ser Val Ala Asn Gln Val Phe His Pro Val Met Phe Asp Lys Cys Ile
130 135 140
Gln Thr Leu Lys Lys Ser Trp Pro Gln Glu Ser Asn Leu Asn Arg Lys
145 150 155 160

```
Arg Lys Lys Glu Gln Pro Lys Ser Ser Gln Ala Asn Pro Gly Arg His
                165                 170                 175
Arg Lys Arg Gly Lys Pro Pro Arg Arg Glu Asp Ile Glu Met Asp Glu
            180                 185                 190
Ile Ile Glu Glu Gln Glu Asp Glu Asn Ile Cys Phe Ser Ala Arg Asp
        195                 200                 205
Leu Ser Gln Ile Arg Asn Ala Ile Phe His Leu Leu Lys Asn Phe Leu
    210                 215                 220
Arg Leu Leu Pro Lys Phe Ser Leu Lys Glu Lys Pro Gln Cys Val Gln
225                 230                 235                 240
Asn Cys Ile Glu Val Phe Val Ser Leu Thr Asn Phe Glu Pro Val Leu
                245                 250                 255
His Glu Cys His Val Thr Gln Ala Arg Ala Leu Asn Gln Ala Lys Tyr
            260                 265                 270
Ile Pro Glu Leu Ala Tyr Tyr Gly Leu Tyr Leu Leu Cys Ser Pro Ile
        275                 280                 285
His Gly Glu Gly Asp Lys Val Ile Ser Cys Val Phe His Gln Met Leu
    290                 295                 300
Ser Val Ile Leu Met Leu Glu Val Gly Glu Gly Ser His Arg Ala Pro
305                 310                 315                 320
Leu Ala Val Thr Ser Gln Val Ile Asn Cys Arg Asn Gln Ala Val Gln
                325                 330                 335
Phe Ile Ser Ala Leu Val Asp Glu Leu Lys Glu Ser Ile Phe Pro Val
            340                 345                 350
Val Arg Ile Leu Leu Gln His Ile Cys Ala Lys Val Val Asp Lys Ser
        355                 360                 365
Glu Tyr Arg Thr Phe Ala Ala Gln Ser Leu Val Gln Leu Leu Ser Lys
    370                 375                 380
Leu Pro Cys Gly Glu Tyr Ala Met Phe Ile Ala Trp Leu Tyr Lys Tyr
385                 390                 395                 400
Ser Arg Ser Ser Lys Ile Pro His Arg Val Phe Thr Leu Asp Val Val
                405                 410                 415
Leu Ala Leu Leu Glu Leu Pro Glu Arg Glu Val Asp Asn Thr Leu Ser
            420                 425                 430
Leu Glu His Gln Lys Phe Leu Lys His Lys Phe Leu Val Gln Glu Ile
        435                 440                 445
Met Phe Asp Arg Cys Leu Asp Lys Ala Pro Thr Val Arg Ser Lys Ala
    450                 455                 460
Leu Ser Ser Phe Ala His Cys Leu Glu Leu Thr Val Thr Ser Ala Ser
465                 470                 475                 480
Glu Ser Ile Leu Glu Leu Leu Ile Asn Ser Pro Thr Phe Ser Val Ile
                485                 490                 495
Glu Ser His Pro Gly Thr Leu Leu Arg Asn Ser Ser Ala Phe Ser Tyr
            500                 505                 510
Gln Arg Gln Thr Ser Asn Arg Ser Glu Pro Ser Gly Glu Ile Asn Ile
        515                 520                 525
Asp Ser Ser Gly Glu Thr Val Gly Ser Gly Glu Arg Cys Val Met Ala
    530                 535                 540
Met Leu Arg Arg Arg Ile Arg Asp Glu Lys Thr Asn Val Arg Lys Ser
545                 550                 555                 560
Ala Leu Gln Val Leu Val Ser Ile Leu Lys His Cys Asp Val Ser Gly
                565                 570                 575
Met Lys Glu Asp Leu Trp Ile Leu Gln Asp Gln Cys Arg Asp Pro Ala
            580                 585                 590
Val Ser Val Arg Lys Gln Ala Leu Gln Ser Leu Thr Glu Leu Leu Met
        595                 600                 605
Ala Gln Pro Arg Cys Val Gln Ile Gln Lys Ala Trp Leu Arg Gly Val
    610                 615                 620
Val Pro Val Val Met Asp Cys Glu Ser Thr Val Gln Glu Lys Ala Leu
625                 630                 635                 640
Glu Phe Leu Asp Gln Leu Leu Leu Gln Asn Ile Arg His His Ser His
                645                 650                 655
Phe His Ser Gly Asp Asp Ser Gln Val Leu Ala Trp Ala Leu Leu Thr
            660                 665                 670
Leu Leu Thr Thr Glu Ser Gln Glu Leu Ser Arg Tyr Leu Asn Lys Ala
        675                 680                 685
Phe His Ile Trp Ser Lys Lys Glu Lys Phe Ser Pro Thr Phe Ile Asn
    690                 695                 700
Asn Val Ile Ser His Thr Gly Thr Glu His Ser Ala Pro Ala Trp Met
```

```
705                 710                 715                 720
Leu Leu Ser Lys Ile Ala Gly Ser Ser Pro Arg Leu Asp Tyr Ser Arg
                725                 730                 735
Ile Ile Gln Ser Trp Glu Lys Ile Ser Ser Gln Gln Asn Pro Asn Ser
            740                 745                 750
Asn Thr Leu Gly His Ile Leu Cys Val Ile Gly His Ile Ala Lys His
        755                 760                 765
Leu Pro Lys Ser Thr Arg Asp Lys Val Thr Asp Ala Val Lys Cys Lys
    770                 775                 780
Leu Asn Gly Phe Gln Trp Ser Leu Glu Val Ile Ser Ser Ala Val Asp
785                 790                 795                 800
Ala Leu Gln Arg Leu Cys Arg Ala Ser Ala Glu Thr Pro Ala Glu Glu
                805                 810                 815
Gln Glu Leu Leu Thr Gln Val Cys Gly Asp Val Leu Ser Thr Cys Glu
            820                 825                 830
His Arg Leu Ser Asn Ile Val Leu Lys Glu Asn Gly Thr Gly Asn Met
        835                 840                 845
Asp Glu Asp Leu Leu Val Lys Tyr Ile Phe Thr Leu Gly Asp Ile Ala
    850                 855                 860
Gln Leu Cys Pro Ala Arg Val Glu Lys Arg Ile Phe Leu Leu Ile Gln
865                 870                 875                 880
Ser Val Leu Ala Ser Ser Ala Asp Ala Asp His Ser Pro Ser Ser Gln
                885                 890                 895
Gly Ser Ser Glu Ala Pro Ala Ser Gln Pro Pro Pro Gln Val Arg Gly
            900                 905                 910
Ser Val Met Pro Ser Val Ile Arg Ala His Ala Ile Ile Thr Leu Gly
        915                 920                 925
Lys Leu Cys Leu Gln His Glu Asp Leu Ala Lys Lys Ser Ile Pro Ala
    930                 935                 940
Leu Val Arg Glu Leu Glu Val Cys Glu Asp Val Ala Val Arg Asn Asn
945                 950                 955                 960
Val Ile Ile Val Met Cys Asp Leu Cys Ile Arg Tyr Thr Ile Met Val
                965                 970                 975
Asp Lys Tyr Ile Pro Asn Ile Ser Met Cys Leu Lys Asp Ser Asp Pro
            980                 985                 990
Phe Ile Arg Lys Gln Thr Leu Ile  Leu Leu Thr Asn Leu  Leu Gln Glu
        995                 1000                1005
Glu Phe  Val Lys Trp Lys Gly  Ser Leu Phe Phe Arg  Phe Val Ser
    1010                1015                1020
Thr Leu  Ile Asp Ser His Pro  Asp Ile Ala Ser Phe  Gly Glu Phe
    1025                1030                1035
Cys Leu  Ala His Leu Leu Leu  Lys Arg Asn Pro Val  Met Phe Phe
    1040                1045                1050
Gln His  Phe Ile Glu Cys Ile  Phe His Phe Asn Asn  Tyr Glu Lys
    1055                1060                1065
His Glu  Lys Tyr Asn Lys Phe  Pro Gln Ser Glu Arg  Glu Lys Arg
    1070                1075                1080
Leu Phe  Ser Leu Lys Gly Lys  Ser Asn Lys Glu Arg  Arg Met Lys
    1085                1090                1095
Ile Tyr  Lys Phe Leu Leu Glu  His Phe Thr Asp Glu  Gln Arg Phe
    1100                1105                1110
Asn Ile  Thr Ser Lys Ile Cys  Leu Ser Ile Leu Ala  Cys Phe Ala
    1115                1120                1125
Asp Gly  Ile Leu Pro Leu Asp  Leu Asp Ala Ser Glu  Leu Leu Ser
    1130                1135                1140
Asp Thr  Phe Glu Val Leu Ser  Ser Lys Glu Ile Lys  Leu Leu Ala
    1145                1150                1155
Met Arg  Ser Lys Pro Asp Lys  Asp Leu Leu Met Glu  Glu Asp Asp
    1160                1165                1170
Met Ala  Leu Ala Asn Val Val  Met Gln Glu Ala Gln  Lys Lys Leu
    1175                1180                1185
Ile Ser  Gln Val Gln Lys Arg  Asn Phe Ile Glu Asn  Ile Ile Pro
    1190                1195                1200
Ile Ile  Ile Ser Leu Lys Thr  Val Leu Glu Lys Asn  Lys Ile Pro
    1205                1210                1215
Ala Leu  Arg Glu Leu Met His  Tyr Leu Arg Glu Val  Met Gln Asp
    1220                1225                1230
Tyr Arg  Asp Glu Leu Lys Asp  Phe Phe Ala Val Asp  Lys Gln Leu
    1235                1240                1245
```

Ala Ser Glu Leu Glu Tyr Asp Met Lys Lys Tyr Gln Glu Gln Leu
1250 1255 1260
Val Gln Glu Gln Glu Leu Ala Lys His Ala Asp Val Ala Gly Thr
1265 1270 1275
Ala Gly Gly Ala Glu Val Ala Pro Val Ala Gln Val Ala Leu Cys
1280 1285 1290
Leu Glu Thr Val Pro Val Pro Ala Gly Gln Glu Asn Pro Ala Met
1295 1300 1305
Ser Pro Ala Val Ser Gln Pro Cys Thr Pro Arg Ala Ser Ala Gly
1310 1315 1320
His Val Ala Val Ser Ser Pro Thr Pro Glu Thr Gly Pro Leu Gln
1325 1330 1335
Arg Leu Leu Pro Lys Ala Arg Pro Met Ser Leu Ser Thr Ile Ala
1340 1345 1350
Ile Leu Asn Ser Val Lys Lys Ala Val Glu Ser Lys Ser Arg His
1355 1360 1365
Arg Ser Arg Ser Leu Gly Val Leu Pro Phe Thr Leu Asn Ser Gly
1370 1375 1380
Ser Pro Glu Lys Thr Cys Ser Gln Val Ser Ser Tyr Ser Leu Glu
1385 1390 1395
Gln Glu Ser Asn Gly Glu Ile Glu His Val Thr Lys Arg Ala Ile
1400 1405 1410
Ser Thr Pro Glu Lys Ser Ile Ser Asp Val Thr Phe Gly Ala Gly
1415 1420 1425
Val Ser Tyr Ile Gly Thr Pro Arg Thr Pro Ser Ser Ala Lys Glu
1430 1435 1440
Lys Ile Glu Gly Arg Ser Gln Gly Asn Asp Ile Leu Cys Leu Ser
1445 1450 1455
Leu Pro Asp Lys Pro Pro Pro Gln Pro Gln Gln Trp Asn Val Arg
1460 1465 1470
Ser Pro Ala Arg Asn Lys Asp Thr Pro Ala Cys Ser Arg Arg Ser
1475 1480 1485
Leu Arg Lys Thr Pro Leu Lys Thr Ala Asn
1490 1495

<210> 270
<211> 551
<212> PRT
<213> Homo sapiens
<400> 270
Met Pro Thr Pro Gly Ala Ala Ser Leu Leu Ser Ala Val Gln Ala Gln
1 5 10 15
Glu Arg Arg Leu Tyr Val Pro Leu Pro Gly Gly Phe Ser Val Pro Pro
20 25 30
Val Thr Pro Met Pro His Cys Leu Leu Asp Pro Lys Glu Asp Leu Lys
35 40 45
Gly Cys Pro Gln Ala Cys Ser Gly Cys Ser Met Gly Thr Pro Ala Pro
50 55 60
Ser Tyr Leu Val Gln Gly Ala Gln Gly Met Pro Leu Arg Lys Ala Ala
65 70 75 80
Ser Leu Ala Thr Lys Leu Glu Glu Met Gly Leu Phe Gly Gln Gln Cys
85 90 95
Leu Pro Ala Met Gly Tyr Thr Cys Arg Cys Arg Glu Pro Leu Leu Leu
100 105 110
Pro Ser His Leu Ile Gly Val Gly Phe Pro Gln Ala Pro Ala Pro Gln
115 120 125
Leu Leu Ser Ala Leu Leu Leu Leu Pro Ala Pro Pro Ser Pro Ala Gln
130 135 140
Ser Glu Val His Pro Pro Val Arg Gly Ala Glu Gln Cys Cys Lys Leu
145 150 155 160
Ser Ala Pro Glu Tyr Pro Gly Asp Leu Leu Asn Cys Thr Phe Ser Phe
165 170 175
Cys Lys Pro Glu Leu Ser Leu Cys Thr Ser Asn Leu Leu Pro Gly Asn
180 185 190
Ala Ala Val Thr Glu Gln Ser Gly Lys Val Leu Asp Asn Thr Leu Gly
195 200 205
Gly Asn Phe Ser Leu Lys Cys Ile Gly Ala Glu Thr Arg Thr Pro Cys
210 215 220
Gln Ala Ala Leu Trp Val Phe Glu Ala Lys Val Ser Arg Leu Met Lys

225 230 235 240
Ser Ala Ser Cys Gln Arg Gln Arg Ile His Trp Gln Gln Gly Ala Leu
245 250 255
Ala Ala Val Arg Gln Gly Asp Leu Gly Leu Lys Tyr Lys Glu Ile Ser
260 265 270
Cys Lys Asp Ala Gly Val Phe His Gly Thr Ser Ser Ser Gly Lys Leu
275 280 285
His Ser His Gly Lys Thr Leu Glu Pro Glu Ser Gly Lys Ala Ile Arg
290 295 300
Ala Trp Ile Leu Glu Ile Leu Ser Ser Cys Lys Pro Gly Ser Leu Val
305 310 315 320
Gln Met Ala Lys Asn Met Thr Met Asp Arg Ala Cys Ala Tyr Leu Val
325 330 335
Thr Cys Arg Glu Ala Thr Leu Trp Trp Gly Val Val Ala Gly Ser Leu
340 345 350
Val Tyr Glu His Gly Asn Gln Lys Pro Ile Pro Val Thr Val Trp Arg
355 360 365
Gly Gly Glu Arg Arg Gly Met Val Pro Thr Lys Lys Asp Thr Ser Thr
370 375 380
Ser Val Leu Phe Ile Gln Ile His Val Leu Thr Phe Ser Ala Glu Asn
385 390 395 400
Lys Ile Gln Ser Asn Cys Asn Ser Ser Ser Cys Ile Arg Pro Thr Gly
405 410 415
Arg Arg Glu Arg Asp Val Thr Asn Gln Thr His Ala Ser Gly Lys Arg
420 425 430
Cys Val Ala Gly Leu Ala Pro Glu Gly Ser Asp Glu Gly Leu Leu Pro
435 440 445
Ala Thr Leu Pro Cys Leu Gly Phe Pro Lys Leu Leu Ser Ala Glu Glu
450 455 460
Arg Ala Asn His Gly Gln Pro Arg Asn Thr Arg Pro Cys Gln Leu Asp
465 470 475 480
Leu Asp Cys Ala Ile Phe Gly His Phe His Thr Leu Pro Asn Lys Leu
485 490 495
Leu Leu Val Ser Val Ser Glu Ile Leu Thr Ser Glu Ile Leu Thr Leu
500 505 510
Ala Asn Leu Leu Gln Gly Cys Gln His Glu Pro Glu Arg Tyr Gly His
515 520 525
Val Thr Asp Pro Glu His Lys Thr Thr Ser Ala Phe Thr Val Ser Lys
530 535 540
Ala Ser Arg Trp Arg Lys Asn
545 550

<210> 271
<211> 2779
<212> PRT
<213> Homo sapiens
<400> 271
Met Gln Gly Asp Leu Lys Thr Thr Asp Ile Ser Ile Glu Pro Pro Ser
1 5 10 15
Ala Gln Leu Glu Val Gln Ala Gly Gln Val Asp Leu Lys Leu Pro Glu
20 25 30
Gly His Val Pro Glu Gly Ala Gly Leu Lys Gly His Leu Pro Lys Leu
35 40 45
Gln Met Pro Ser Phe Lys Met Pro Lys Val Asp Arg Lys Gly Pro Gln
50 55 60
Ile Asp Val Lys Gly Pro Lys Leu Asp Leu Lys Gly Pro Lys Thr Asp
65 70 75 80
Val Thr Ala Pro Asp Val Glu Val Ser Gln Pro Gly Met Glu Val Asp
85 90 95
Val Glu Ala Pro Gly Ala Lys Leu Asp Gly Ala Arg Leu Glu Gly Asp
100 105 110
Leu Ser Leu Ala Asp Lys Asp Val Thr Ala Lys Asp Ser Lys Phe Lys
115 120 125
Met Pro Lys Phe Lys Met Pro Ser Phe Gly Val Ser Ala Pro Gly Lys
130 135 140
Ser Ile Glu Val Leu Val Asp Val Ser Ala Pro Lys Val Glu Ala Asp
145 150 155 160
Leu Ser Leu Pro Ser Met Gln Gly Asp Leu Lys Asn Thr Asp Ile Ser
165 170 175

Ile Glu Pro Pro Ser Ala Gln Leu Glu Val Gln Ala Gly Gln Val Asp
180 185 190
Val Lys Leu Pro Glu Gly His Val Leu Glu Gly Ala Gly Leu Lys Gly
195 200 205
His Leu Pro Lys Leu Gln Met Pro Ser Phe Lys Met Pro Lys Val Asp
210 215 220
Arg Lys Gly Pro Gln Ile Asp Ile Lys Gly Pro Lys Leu Asp Leu Lys
225 230 235 240
Gly Pro Lys Met Asp Val Thr Ala Pro Asp Val Glu Val Ser Gln Pro
245 250 255
Ser Met Glu Val Asp Val Glu Ala Pro Gly Ala Lys Leu Asp Gly Ala
260 265 270
Arg Leu Glu Gly Asp Leu Ser Leu Ala Asp Lys Asp Val Thr Ala Lys
275 280 285
Asp Ser Lys Phe Lys Met Pro Lys Phe Lys Met Pro Ser Tyr Arg Ala
290 295 300
Ser Ala Pro Gly Lys Ser Ile Gln Ala Ser Val Asp Val Ser Ala Pro
305 310 315 320
Lys Ala Glu Ala Asp Val Ser Leu Pro Ser Met Gln Gly Asp Leu Lys
325 330 335
Thr Thr Asp Leu Ser Ile Gln Leu Pro Ser Val Asp Leu Glu Val Gln
340 345 350
Ala Gly Gln Val Asp Val Lys Leu Pro Glu Gly His Val Pro Glu Gly
355 360 365
Ala Gly Leu Lys Gly His Leu Pro Lys Val Glu Met Pro Ser Phe Lys
370 375 380
Met Pro Lys Val Asp Leu Lys Ser Pro Gln Val Asp Ile Lys Gly Pro
385 390 395 400
Lys Leu Asp Leu Lys Val Pro Lys Ala Glu Val Thr Val Pro Asp Val
405 410 415
Glu Val Ser Leu Pro Ser Val Glu Val Asp Val Gln Ala Pro Arg Ala
420 425 430
Lys Leu Asp Gly Ala Arg Leu Glu Gly Asp Leu Ser Leu Ala Glu Lys
435 440 445
Asp Val Thr Ala Lys Asp Ser Lys Phe Lys Met Pro Lys Phe Lys Met
450 455 460
Pro Ser Phe Gly Val Ser Ala Pro Gly Arg Ser Ile Glu Ala Ser Leu
465 470 475 480
Asp Val Ser Ala Pro Lys Val Glu Ala Asp Val Ser Leu Ser Ser Met
485 490 495
Gln Gly Asp Leu Lys Ala Thr Asp Leu Ser Ile Gln Pro Pro Ser Ala
500 505 510
Asp Leu Glu Val Gln Ala Val Gln Val Asp Val Glu Leu Leu Glu Gly
515 520 525
Pro Val Pro Glu Gly Ala Gly Leu Lys Gly His Leu Pro Lys Val Glu
530 535 540
Met Pro Ser Leu Lys Thr Pro Lys Val Asp Leu Lys Gly Pro Gln Ile
545 550 555 560
Asp Val Lys Gly Pro Lys Leu Asp Leu Lys Gly Pro Lys Ala Glu Val
565 570 575
Arg Val Pro Asp Val Glu Val Ser Leu Pro Ser Val Glu Val Asp Val
580 585 590
Gln Ala Pro Lys Ala Lys Leu Asp Ala Gly Arg Leu Glu Gly Asp Leu
595 600 605
Ser Leu Ala Asp Lys Asp Val Thr Ala Lys Asp Ser Lys Phe Lys Met
610 615 620
Pro Lys Phe Lys Met Pro Ser Phe Arg Val Ser Ala Pro Gly Lys Ser
625 630 635 640
Met Glu Ala Ser Val Asp Val Ser Ala Pro Lys Val Glu Ala Asp Val
645 650 655
Ser Leu Pro Ser Met Gln Gly Asp Leu Lys Thr Thr Asp Leu Ser Ile
660 665 670
Gln Pro Pro Ser Ala Asp Leu Lys Val Gln Ala Gly Gln Met Asp Val
675 680 685
Lys Leu Pro Glu Gly Gln Val Pro Glu Gly Ala Gly Leu Lys Glu His
690 695 700
Leu Pro Lys Val Glu Met Pro Ser Leu Lys Met Pro Lys Val Asp Leu
705 710 715 720
Lys Gly Pro Gln Val Asp Ile Lys Gly Pro Lys Leu Asp Leu Lys Val

```
                725                 730                 735
Ser Lys Ala Glu Val Thr Ala Pro Asp Val Glu Val Ser Leu Pro Ser
            740                 745                 750
Val Glu Val Asp Val Gln Ala Pro Arg Ala Lys Leu Asp Ser Ala Gln
        755                 760                 765
Leu Glu Gly Asp Leu Ser Leu Ala Asp Lys Asp Val Thr Ala Lys Asp
    770                 775                 780
Ser Lys Phe Lys Met Pro Lys Phe Lys Met Pro Ser Phe Gly Val Ser
785                 790                 795                 800
Ala Pro Gly Lys Ser Ile Glu Ala Ser Val His Val Ser Ala Pro Lys
                805                 810                 815
Val Glu Ala Asp Val Ser Leu Pro Ser Met Gln Gly Asp Leu Lys Thr
            820                 825                 830
Thr Asp Leu Ser Ile Gln Pro His Ser Ala Asp Leu Thr Val Gln Ala
        835                 840                 845
Arg Gln Val Asp Met Lys Leu Leu Glu Gly His Val Pro Glu Glu Ala
    850                 855                 860
Gly Leu Lys Gly His Leu Pro Lys Val Gln Met Pro Ser Phe Lys Met
865                 870                 875                 880
Pro Lys Val Asp Leu Lys Gly Pro Glu Ile Asp Ile Lys Gly Pro Lys
                885                 890                 895
Leu Asp Leu Lys Asp Pro Lys Val Glu Val Thr Ala Pro Asp Val Glu
            900                 905                 910
Val Ser Leu Pro Ser Val Glu Val Asp Val Glu Ala Pro Gly Ala Lys
        915                 920                 925
Leu Asp Gly Ala Arg Leu Glu Gly Asp Leu Ser Leu Ala Asp Lys Asp
    930                 935                 940
Met Thr Ala Lys Asp Ser Lys Phe Lys Met Pro Lys Phe Lys Met Pro
945                 950                 955                 960
Ser Phe Gly Val Ser Ala Pro Gly Lys Ser Met Glu Ala Ser Val Asp
                965                 970                 975
Val Thr Ala Pro Lys Val Glu Ala Asp Val Ser Leu Pro Ser Met Gln
            980                 985                 990
Gly Asp Leu Lys Ala Thr Asp Leu  Ser Val Gln Pro Pro  Ser Ala Asp
        995                 1000                1005
Leu Glu  Val Gln Ala Gly Gln  Val Asp Val Lys Leu  Pro Glu Gly
    1010                1015                1020
Pro Val  Pro Glu Gly Ala Ser  Leu Lys Gly His Leu  Pro Lys Val
    1025                1030                1035
Gln Met  Pro Ser Phe Lys Met  Pro Lys Val Asp Leu  Lys Gly Pro
    1040                1045                1050
Gln Ile  Asp Val Lys Gly Pro  Lys Leu Asp Leu Lys  Gly Pro Lys
    1055                1060                1065
Ala Glu  Val Thr Ala Pro Asp  Val Lys Met Ser Leu  Ser Ser Met
    1070                1075                1080
Glu Val  Asp Val Gln Ala Pro  Arg Ala Lys Leu Asp  Gly Val Gln
    1085                1090                1095
Leu Glu  Gly Asp Leu Ser Leu  Ala Asp Lys Asp Val  Thr Ala Lys
    1100                1105                1110
Asp Ser  Lys Phe Lys Met Pro  Lys Phe Lys Met Pro  Ser Phe Gly
    1115                1120                1125
Val Ser  Ala Pro Gly Lys Ser  Met Glu Ala Ser Val  Asp Val Ser
    1130                1135                1140
Glu Leu  Lys Ala Lys Ala Asp  Val Ser Leu Pro Ser  Met Gln Gly
    1145                1150                1155
Asp Leu  Lys Thr Thr Asp Leu  Ser Ile Gln Ser Pro  Ser Ala Asp
    1160                1165                1170
Leu Glu  Val Gln Ala Gly Gln  Val Asp Val Lys Leu  Pro Glu Gly
    1175                1180                1185
Pro Leu  Pro Lys Gly Ala Gly  Leu Lys Gly His Leu  Pro Lys Val
    1190                1195                1200
Gln Met  Pro Cys Leu Lys Met  Pro Lys Val Ala Leu  Lys Gly Pro
    1205                1210                1215
Gln Val  Asp Val Lys Gly Pro  Lys Leu Asp Leu Lys  Gly Pro Lys
    1220                1225                1230
Ala Asp  Val Met Thr Pro Val  Val Glu Val Ser Leu  Pro Ser Met
    1235                1240                1245
Glu Val  Asp Val Glu Ala Pro  Gly Ala Lys Leu Asp  Ser Val Arg
    1250                1255                1260
```

Leu Glu Gly Asp Leu Ser Leu Ala Asp Lys Asp Met Thr Ala Lys
1265 1270 1275
Asp Ser Lys Phe Lys Met Pro Lys Phe Lys Met Pro Ser Phe Gly
1280 1285 1290
Val Ser Ala Pro Gly Lys Ser Ile Glu Ala Ser Leu Asp Val Ser
1295 1300 1305
Ala Leu Lys Val Glu Ala Asp Val Ser Leu Pro Ser Met Gln Gly
1310 1315 1320
Asp Leu Lys Thr Thr His Leu Ser Ile Gln Pro Pro Ser Ala Asp
1325 1330 1335
Leu Glu Val Gln Ala Gly Gln Glu Asp Val Lys Leu Pro Glu Gly
1340 1345 1350
Pro Val His Glu Gly Ala Gly Leu Lys Gly His Leu Pro Lys Leu
1355 1360 1365
Gln Met Pro Ser Phe Lys Val Pro Lys Val Asp Leu Lys Gly Pro
1370 1375 1380
Gln Ile Asp Val Asn Val Pro Lys Leu Asp Leu Lys Gly Pro Lys
1385 1390 1395
Val Glu Val Thr Ser Pro Asn Leu Asp Val Ser Leu Pro Ser Met
1400 1405 1410
Glu Val Asp Ile Gln Ala Pro Gly Ala Lys Leu Asp Ser Thr Arg
1415 1420 1425
Leu Glu Gly Asp Leu Ser Leu Ala Asp Lys Asp Val Thr Ala Lys
1430 1435 1440
Asp Ser Lys Phe Lys Met Pro Lys Phe Lys Met Pro Ser Phe Gly
1445 1450 1455
Met Leu Ser Pro Gly Lys Ser Ile Glu Val Ser Val Asp Val Ser
1460 1465 1470
Ala Pro Lys Met Glu Ala Asp Met Ser Ile Pro Ser Met Gln Gly
1475 1480 1485
Asp Leu Lys Thr Thr Asp Leu Arg Ile Gln Ala Pro Ser Ala Asp
1490 1495 1500
Leu Glu Val Gln Ala Gly Gln Val Asp Leu Lys Leu Pro Glu Gly
1505 1510 1515
His Met Pro Glu Val Ala Gly Leu Lys Gly His Leu Pro Lys Val
1520 1525 1530
Glu Met Pro Ser Phe Lys Met Pro Lys Val Asp Leu Lys Gly Pro
1535 1540 1545
Gln Val Asp Val Lys Gly Pro Lys Leu Asp Leu Lys Gly Pro Lys
1550 1555 1560
Ala Glu Val Met Ala Pro Asp Val Glu Val Ser Leu Pro Ser Val
1565 1570 1575
Glu Thr Asp Val Gln Ala Pro Gly Ser Met Leu Asp Gly Ala Arg
1580 1585 1590
Leu Glu Gly Asp Leu Ser Leu Ala His Glu Asp Val Ala Gly Lys
1595 1600 1605
Asp Ser Lys Phe Gln Gly Pro Lys Leu Ser Thr Ser Gly Phe Glu
1610 1615 1620
Trp Ser Ser Lys Lys Val Ser Met Ser Ser Ser Glu Ile Glu Gly
1625 1630 1635
Asn Val Thr Phe His Glu Lys Thr Ser Thr Phe Pro Ile Val Glu
1640 1645 1650
Ser Val Val His Glu Gly Asp Leu His Asp Pro Ser Arg Asp Gly
1655 1660 1665
Asn Leu Gly Leu Ala Val Gly Glu Val Gly Met Asp Ser Lys Phe
1670 1675 1680
Lys Lys Leu His Phe Lys Val Pro Lys Val Ser Phe Ser Ser Thr
1685 1690 1695
Lys Thr Pro Lys Asp Ser Leu Val Pro Gly Ala Lys Ser Ser Ile
1700 1705 1710
Gly Leu Ser Thr Ile Pro Leu Ser Ser Ser Glu Cys Ser Ser Phe
1715 1720 1725
Glu Leu Gln Gln Val Ser Ala Cys Ser Glu Pro Ser Met Gln Met
1730 1735 1740
Pro Lys Val Gly Phe Ala Gly Phe Pro Ser Ser Arg Leu Asp Leu
1745 1750 1755
Thr Gly Pro His Phe Glu Ser Ser Ile Leu Ser Pro Cys Glu Asp
1760 1765 1770
Val Thr Leu Thr Lys Tyr Gln Val Thr Val Pro Arg Ala Ala Leu

1775 1780 1785
Ala Pro Glu Leu Ala Leu Glu Ile Pro Ser Gly Ser Gln Ala Asp
1790 1795 1800
Ile Pro Leu Pro Lys Thr Glu Cys Ser Thr Asp Leu Gln Pro Pro
1805 1810 1815
Glu Gly Val Pro Thr Ser Gln Ala Glu Ser His Ser Gly Pro Leu
1820 1825 1830
Asn Ser Met Ile Pro Val Ser Leu Gly Gln Val Ser Phe Pro Lys
1835 1840 1845
Phe Tyr Lys Pro Lys Phe Val Phe Ser Val Pro Gln Met Ala Val
1850 1855 1860
Pro Glu Gly Asp Leu His Ala Ala Val Gly Ala Pro Val Met Ser
1865 1870 1875
Pro Leu Ser Pro Gly Glu Arg Val Gln Cys Pro Leu Pro Ser Thr
1880 1885 1890
Gln Leu Pro Ser Pro Gly Thr Cys Val Ser Gln Gly Pro Glu Glu
1895 1900 1905
Leu Val Ala Ser Leu Gln Thr Ser Val Val Ala Pro Gly Glu Ala
1910 1915 1920
Pro Ser Glu Asp Ala Asp His Glu Gly Lys Gly Ser Pro Leu Lys
1925 1930 1935
Met Pro Lys Ile Lys Leu Pro Ser Phe Arg Trp Ser Pro Lys Lys
1940 1945 1950
Glu Thr Gly Pro Lys Val Asp Pro Glu Cys Ser Val Glu Asp Ser
1955 1960 1965
Lys Leu Ser Leu Val Leu Asp Lys Asp Glu Val Ala Pro Gln Ser
1970 1975 1980
Ala Ile His Met Asp Leu Pro Pro Glu Arg Asp Gly Glu Lys Gly
1985 1990 1995
Arg Ser Thr Lys Pro Gly Phe Ala Met Pro Lys Leu Ala Leu Pro
2000 2005 2010
Lys Met Lys Ala Ser Lys Ser Gly Val Ser Leu Pro Gln Arg Asp
2015 2020 2025
Val Asp Pro Ser Leu Ser Ser Ala Thr Ala Gly Gly Ser Phe Gln
2030 2035 2040
Asp Thr Glu Lys Ala Ser Ser Asp Gly Gly Arg Gly Gly Leu Gly
2045 2050 2055
Ala Thr Ala Ser Ala Thr Gly Ser Glu Gly Val Asn Leu His Arg
2060 2065 2070
Pro Gln Val His Ile Pro Ser Leu Gly Phe Ala Lys Pro Asp Leu
2075 2080 2085
Arg Ser Ser Lys Ala Lys Val Glu Val Ser Gln Pro Glu Ala Asp
2090 2095 2100
Leu Pro Leu Pro Lys His Asp Leu Ser Thr Glu Gly Asp Ser Arg
2105 2110 2115
Gly Cys Gly Leu Gly Asp Val Pro Val Ser Gln Pro Cys Gly Glu
2120 2125 2130
Gly Ile Ala Pro Thr Pro Glu Asp Pro Leu Gln Pro Ser Cys Arg
2135 2140 2145
Lys Pro Asp Ala Glu Val Leu Thr Val Glu Ser Pro Glu Glu Glu
2150 2155 2160
Ala Met Thr Lys Tyr Ser Gln Glu Ser Trp Phe Lys Met Pro Lys
2165 2170 2175
Phe Arg Met Pro Ser Leu Arg Arg Ser Phe Arg Asp Arg Gly Gly
2180 2185 2190
Ala Gly Lys Leu Glu Val Ala Gln Thr Gln Ala Pro Ala Ala Thr
2195 2200 2205
Gly Gly Glu Ala Ala Ala Lys Val Lys Glu Phe Leu Val Ser Gly
2210 2215 2220
Ser Asn Val Glu Ala Ala Met Ser Leu Gln Leu Pro Glu Ala Asp
2225 2230 2235
Ala Glu Val Thr Ala Ser Glu Ser Lys Ser Ser Thr Asp Ile Leu
2240 2245 2250
Arg Cys Asp Leu Asp Ser Thr Gly Leu Lys Leu His Leu Ser Thr
2255 2260 2265
Ala Gly Met Thr Gly Asp Glu Leu Ser Thr Ser Glu Val Arg Ile
2270 2275 2280
His Pro Ser Lys Gly Pro Leu Pro Phe Gln Met Pro Gly Met Arg
2285 2290 2295

Leu Pro Glu Thr Gln Val Leu Pro Gly Glu Ile Asp Glu Thr Pro
2300 2305 2310
Leu Ser Lys Pro Gly His Asp Leu Ala Ser Met Glu Asp Lys Thr
2315 2320 2325
Glu Lys Trp Ser Ser Gln Pro Glu Gly Pro Leu Lys Leu Lys Ala
2330 2335 2340
Ser Ser Thr Asp Met Pro Ser Gln Ile Ser Val Val Asn Val Asp
2345 2350 2355
Gln Leu Trp Glu Asp Ser Val Leu Thr Val Lys Phe Pro Lys Leu
2360 2365 2370
Met Val Pro Arg Phe Ser Phe Pro Ala Pro Ser Ser Glu Asp Asp
2375 2380 2385
Val Phe Ile Pro Thr Val Arg Glu Val Gln Cys Pro Glu Ala Asn
2390 2395 2400
Ile Asp Thr Ala Leu Cys Lys Glu Ser Pro Gly Leu Trp Gly Ala
2405 2410 2415
Ser Ile Leu Lys Ala Gly Ala Gly Val Pro Gly Glu Gln Pro Val
2420 2425 2430
Asp Leu Asn Leu Pro Leu Glu Ala Pro Pro Ile Ser Lys Val Arg
2435 2440 2445
Val His Ile Gln Gly Ala Gln Val Glu Ser Gln Glu Val Thr Ile
2450 2455 2460
His Ser Ile Val Thr Pro Glu Phe Val Asp Leu Ser Val Pro Arg
2465 2470 2475
Thr Phe Ser Thr Gln Ile Val Arg Glu Ser Glu Ile Pro Thr Ser
2480 2485 2490
Glu Ile Gln Thr Pro Ser Tyr Gly Phe Ser Leu Leu Lys Val Lys
2495 2500 2505
Ile Pro Glu Pro His Thr Gln Ala Arg Val Tyr Thr Thr Met Thr
2510 2515 2520
Gln His Ser Arg Thr Gln Glu Gly Thr Glu Glu Ala Pro Ile Gln
2525 2530 2535
Ala Thr Pro Gly Val Asp Ser Ile Ser Gly Asp Leu Gln Pro Asp
2540 2545 2550
Thr Gly Glu Pro Phe Glu Met Ile Ser Ser Ser Val Asn Val Leu
2555 2560 2565
Gly Gln Gln Thr Leu Thr Phe Glu Val Pro Ser Gly His Gln Leu
2570 2575 2580
Ala Asp Ser Cys Ser Asp Glu Glu Pro Ala Glu Ile Leu Glu Phe
2585 2590 2595
Pro Pro Asp Asp Ser Gln Glu Ala Thr Thr Pro Leu Ala Asp Glu
2600 2605 2610
Gly Arg Ala Pro Lys Asp Lys Pro Glu Ser Lys Lys Ser Gly Leu
2615 2620 2625
Leu Trp Phe Trp Leu Pro Asn Ile Gly Phe Ser Ser Ser Val Asp
2630 2635 2640
Glu Thr Gly Val Asp Ser Lys Asn Asp Val Gln Arg Ser Ala Pro
2645 2650 2655
Ile Gln Thr Gln Pro Glu Ala Arg Pro Glu Ala Glu Leu Pro Lys
2660 2665 2670
Lys Gln Glu Lys Ala Gly Trp Phe Arg Phe Pro Lys Leu Gly Phe
2675 2680 2685
Ser Ser Ser Pro Thr Lys Lys Ser Lys Ser Thr Glu Asp Gly Ala
2690 2695 2700
Glu Leu Glu Glu Gln Lys Leu Gln Glu Glu Thr Ile Thr Phe Phe
2705 2710 2715
Asp Ala Arg Glu Ser Phe Ser Pro Glu Glu Lys Glu Glu Gly Glu
2720 2725 2730
Leu Ile Gly Pro Val Gly Thr Gly Leu Asp Ser Arg Val Met Val
2735 2740 2745
Thr Ser Ala Ala Arg Thr Glu Leu Ile Leu Pro Glu Gln Asp Arg
2750 2755 2760
Lys Ala Asp Asp Glu Ser Lys Gly Ser Gly Leu Gly Pro Asn Glu
2765 2770 2775
Gly

<210> 272
<211> 512

<212> PRT
<213> Homo sapiens
<400> 272

Met Asp Phe Glu Ser Gly Gln Val Asp Pro Leu Ala Ser Val Ile Leu
1 5 10 15
Pro Pro Asn Leu Leu Glu Asn Leu Ser Pro Glu Asp Ser Val Leu Val
20 25 30
Arg Arg Ala Gln Phe Thr Phe Phe Asn Lys Thr Gly Leu Phe Gln Asp
35 40 45
Val Gly Pro Gln Arg Lys Thr Leu Val Ser Tyr Val Met Ala Cys Ser
50 55 60
Ile Gly Asn Ile Thr Ile Gln Asn Leu Lys Asp Pro Val Gln Ile Lys
65 70 75 80
Ile Lys His Thr Arg Thr Gln Glu Val His His Pro Ile Cys Ala Phe
85 90 95
Trp Asp Leu Asn Lys Asn Lys Ser Phe Gly Gly Trp Asn Thr Ser Gly
100 105 110
Cys Val Ala His Arg Asp Ser Asp Ala Ser Glu Thr Val Cys Leu Cys
115 120 125
Asn His Phe Thr His Phe Gly Val Leu Met Asp Leu Pro Arg Ser Ala
130 135 140
Ser Gln Leu Asp Ala Arg Asn Thr Lys Val Leu Thr Phe Ile Ser Tyr
145 150 155 160
Ile Gly Cys Gly Ile Ser Ala Ile Phe Ser Ala Ala Thr Leu Leu Thr
165 170 175
Tyr Val Ala Phe Glu Lys Leu Arg Arg Asp Tyr Pro Ser Lys Ile Leu
180 185 190
Met Asn Leu Ser Thr Ala Leu Leu Phe Leu Asn Leu Leu Phe Leu Leu
195 200 205
Asp Gly Trp Ile Thr Ser Phe Asn Val Asp Gly Leu Cys Ile Ala Val
210 215 220
Ala Val Leu Leu His Phe Phe Leu Leu Ala Thr Phe Thr Trp Met Gly
225 230 235 240
Leu Glu Ala Ile His Met Tyr Ile Ala Leu Val Lys Val Phe Asn Thr
245 250 255
Tyr Ile Arg Arg Tyr Ile Leu Lys Phe Cys Ile Ile Gly Trp Gly Leu
260 265 270
Pro Ala Leu Val Val Ser Val Val Leu Ala Ser Arg Asn Asn Asn Glu
275 280 285
Val Tyr Gly Lys Glu Ser Tyr Gly Lys Glu Lys Gly Asp Glu Phe Cys
290 295 300
Trp Ile Gln Asp Pro Val Ile Phe Tyr Val Thr Cys Ala Gly Tyr Phe
305 310 315 320
Gly Val Met Phe Phe Leu Asn Ile Ala Met Phe Ile Val Val Met Val
325 330 335
Gln Ile Cys Gly Arg Asn Gly Lys Arg Ser Asn Arg Thr Leu Arg Glu
340 345 350
Glu Val Leu Arg Asn Leu Arg Ser Val Val Ser Leu Thr Phe Leu Leu
355 360 365
Gly Met Thr Trp Gly Phe Ala Phe Phe Ala Trp Gly Pro Leu Asn Ile
370 375 380
Pro Phe Met Tyr Leu Phe Ser Ile Phe Asn Ser Leu Gln Gly Leu Phe
385 390 395 400
Ile Phe Ile Phe His Cys Ala Met Lys Glu Asn Val Gln Lys Gln Trp
405 410 415
Arg Arg His Leu Cys Cys Gly Arg Phe Arg Leu Ala Asp Asn Ser Asp
420 425 430
Trp Ser Lys Thr Ala Thr Asn Ile Ile Lys Lys Ser Ser Asp Asn Leu
435 440 445
Gly Lys Ser Leu Ser Ser Ser Ser Ile Gly Ser Asn Ser Thr Tyr Leu
450 455 460
Thr Ser Lys Ser Lys Ser Ser Ser Thr Thr Tyr Phe Lys Arg Asn Ser
465 470 475 480
His Thr Asp Asn Val Ser Tyr Glu His Ser Phe Asn Lys Ser Gly Ser
485 490 495
Leu Arg Gln Cys Phe His Gly Gln Val Leu Val Lys Thr Gly Pro Cys
500 505 510

<210> 273

<211> 355
<212> PRT
<213> Homo sapiens
<400> 273

Met Ala Ala Pro Ala Phe Glu Pro Gly Arg Gln Ser Asp Leu Leu Val
1 5 10 15
Lys Leu Asn Arg Leu Met Glu Arg Cys Leu Arg Asn Ser Lys Cys Ile
20 25 30
Asp Thr Glu Ser Leu Cys Val Val Ala Gly Glu Lys Val Trp Gln Ile
35 40 45
Arg Val Asp Leu His Leu Leu Asn His Asp Gly Asn Ile Ile Asp Ala
50 55 60
Ala Ser Ile Ala Ala Ile Val Ala Leu Cys His Phe Arg Arg Pro Asp
65 70 75 80
Val Ser Val Gln Gly Asp Glu Val Thr Leu Tyr Thr Pro Glu Glu Arg
85 90 95
Asp Pro Val Pro Leu Ser Ile His His Met Pro Ile Cys Val Ser Phe
100 105 110
Ala Phe Phe Gln Gln Gly Thr Tyr Leu Leu Val Asp Pro Asn Glu Arg
115 120 125
Glu Glu Arg Val Met Asp Gly Leu Leu Val Ile Ala Met Asn Lys His
130 135 140
Arg Glu Ile Cys Thr Ile Gln Ser Ser Gly Gly Ile Met Leu Leu Lys
145 150 155 160
Asp Gln Val Leu Arg Cys Ser Lys Ile Ala Gly Val Lys Val Ala Glu
165 170 175
Ile Thr Glu Leu Ile Leu Lys Ala Leu Glu Asn Asp Gln Lys Val Arg
180 185 190
Lys Glu Gly Gly Lys Phe Gly Phe Ala Glu Ser Ile Ala Asn Gln Arg
195 200 205
Ile Thr Ala Phe Lys Met Glu Lys Ala Pro Ile Asp Thr Ser Asp Val
210 215 220
Glu Glu Lys Ala Glu Glu Ile Ile Ala Glu Ala Glu Pro Pro Ser Glu
225 230 235 240
Val Val Ser Thr Pro Val Leu Trp Thr Pro Gly Thr Ala Gln Ile Gly
245 250 255
Glu Gly Val Glu Asn Ser Trp Gly Asp Leu Glu Asp Ser Glu Lys Glu
260 265 270
Asp Asp Glu Gly Gly Gly Asp Gln Ala Ile Ile Leu Asp Gly Ile Lys
275 280 285
Met Asp Thr Gly Val Glu Val Ser Asp Ile Gly Ser Gln Asp Ala Pro
290 295 300
Ile Ile Leu Ser Asp Ser Glu Glu Glu Glu Met Ile Ile Leu Glu Pro
305 310 315 320
Asp Lys Asn Pro Lys Lys Ile Arg Thr Gln Thr Thr Ser Ala Lys Gln
325 330 335
Glu Lys Ala Pro Ser Lys Lys Pro Val Lys Arg Arg Lys Lys Lys Arg
340 345 350
Ala Ala Asn
355

<210> 274
<211> 940
<212> PRT
<213> Homo sapiens
<400> 274

Met Ile Leu Glu Gln Tyr Val Val Val Ser Asn Tyr Lys Lys Gln Glu
1 5 10 15
Asn Ser Glu Leu Ser Leu Gln Ala Gly Glu Val Val Asp Val Ile Glu
20 25 30
Lys Asn Glu Ser Gly Trp Trp Phe Val Ser Thr Ser Glu Glu Gln Gly
35 40 45
Trp Val Pro Ala Thr Tyr Leu Glu Ala Gln Asn Gly Thr Arg Asp Asp
50 55 60
Ser Asp Ile Asn Thr Ser Lys Thr Gly Glu Glu Glu Lys Tyr Val Thr
65 70 75 80
Val Gln Pro Tyr Thr Ser Gln Ser Lys Asp Glu Ile Gly Phe Glu Lys
85 90 95
Gly Val Thr Val Glu Val Ile Arg Lys Asn Leu Glu Gly Trp Trp Tyr

```
                    100                 105                 110
Ile Arg Tyr Leu Gly Lys Glu Gly Trp Ala Pro Ala Ser Tyr Leu Lys
            115                 120                 125
Lys Ala Lys Asp Asp Leu Pro Thr Arg Lys Lys Asn Leu Ala Gly Pro
    130                 135                 140
Val Glu Ile Ile Gly Asn Ile Met Glu Ile Ser Asn Leu Leu Asn Lys
145                 150                 155                 160
Lys Ala Ser Gly Asp Lys Glu Thr Pro Pro Ala Glu Gly Glu Gly His
                165                 170                 175
Glu Ala Pro Ile Ala Lys Lys Glu Ile Ser Leu Pro Ile Leu Cys Asn
            180                 185                 190
Ala Ser Asn Gly Ser Ala Val Gly Val Pro Asp Arg Thr Val Ser Arg
        195                 200                 205
Leu Ala Gln Gly Ser Pro Ala Val Ala Arg Ile Ala Pro Gln Arg Ala
    210                 215                 220
Gln Ile Ser Ser Pro Asn Leu Arg Thr Arg Pro Pro Pro Arg Arg Glu
225                 230                 235                 240
Ser Ser Leu Gly Phe Gln Leu Pro Lys Pro Pro Glu Pro Pro Ser Val
                245                 250                 255
Glu Val Glu Tyr Tyr Thr Ile Ala Glu Phe Gln Ser Cys Ile Ser Asp
            260                 265                 270
Gly Ile Ser Phe Arg Gly Gly Gln Lys Ala Glu Val Ile Asp Lys Asn
        275                 280                 285
Ser Gly Gly Trp Trp Tyr Val Gln Ile Gly Glu Lys Glu Gly Trp Ala
    290                 295                 300
Pro Ala Ser Tyr Ile Asp Lys Arg Lys Lys Pro Asn Leu Ser Arg Arg
305                 310                 315                 320
Thr Ser Thr Leu Thr Arg Pro Lys Val Pro Pro Pro Ala Pro Pro Ser
                325                 330                 335
Lys Pro Lys Glu Ala Glu Glu Gly Pro Thr Gly Ala Ser Glu Ser Gln
            340                 345                 350
Asp Ser Pro Arg Lys Leu Lys Tyr Glu Glu Pro Glu Tyr Asp Ile Pro
        355                 360                 365
Ala Phe Gly Phe Asp Ser Glu Pro Glu Leu Ser Glu Glu Pro Val Glu
    370                 375                 380
Asp Arg Ala Ser Gly Glu Arg Arg Pro Ala Gln Pro His Arg Pro Ser
385                 390                 395                 400
Pro Ala Ser Ser Leu Gln Arg Ala Arg Phe Lys Val Gly Glu Ser Ser
                405                 410                 415
Glu Asp Val Ala Leu Glu Glu Glu Thr Ile Tyr Glu Asn Glu Gly Phe
            420                 425                 430
Arg Pro Tyr Ala Glu Asp Thr Leu Ser Ala Arg Gly Ser Ser Gly Asp
        435                 440                 445
Ser Asp Ser Pro Gly Ser Ser Ser Leu Ser Leu Thr Arg Lys Asn Ser
    450                 455                 460
Pro Lys Ser Gly Ser Pro Lys Ser Ser Ser Leu Leu Lys Leu Lys Ala
465                 470                 475                 480
Glu Lys Asn Ala Gln Ala Glu Met Gly Lys Asn His Ser Ser Ala Ser
                485                 490                 495
Phe Ser Ser Ser Ile Thr Ile Asn Thr Thr Cys Cys Ser Ser Ser Ser
            500                 505                 510
Ser Ser Ser Ser Ser Leu Ser Lys Thr Ser Gly Asp Leu Lys Pro Arg
        515                 520                 525
Ser Ala Ser Asp Ala Gly Ile Arg Gly Thr Pro Lys Val Arg Ala Lys
    530                 535                 540
Lys Asp Ala Asp Ala Asn Ala Gly Leu Thr Ser Cys Pro Arg Ala Lys
545                 550                 555                 560
Pro Ser Val Arg Pro Lys Pro Phe Leu Asn Arg Ala Glu Ser Gln Ser
                565                 570                 575
Gln Glu Lys Met Asp Ile Ser Thr Leu Arg Arg Gln Leu Arg Pro Thr
            580                 585                 590
Gly Gln Leu Arg Gly Gly Leu Lys Gly Ser Lys Ser Glu Asp Ser Glu
        595                 600                 605
Leu Pro Pro Gln Thr Ala Ser Glu Ala Pro Ser Glu Gly Ser Arg Arg
    610                 615                 620
Ser Ser Ser Asp Leu Ile Thr Leu Pro Ala Thr Thr Pro Pro Cys Pro
625                 630                 635                 640
Thr Lys Lys Glu Trp Glu Gly Pro Ala Thr Ser Tyr Met Thr Cys Ser
                645                 650                 655
```

Ala Tyr Gln Lys Val Gln Asp Ser Glu Ile Ser Phe Pro Ala Gly Val
660 665 670
Glu Val Gln Val Leu Glu Lys Gln Glu Ser Gly Trp Trp Tyr Val Arg
675 680 685
Phe Gly Glu Leu Glu Gly Trp Ala Pro Ser His Tyr Leu Val Leu Asp
690 695 700
Glu Asn Glu Gln Pro Asp Pro Ser Gly Lys Glu Leu Asp Thr Val Pro
705 710 715 720
Ala Lys Gly Arg Gln Asn Glu Gly Lys Ser Asp Ser Leu Glu Lys Ile
725 730 735
Glu Arg Arg Val Gln Ala Leu Asn Thr Val Asn Gln Ser Lys Lys Ala
740 745 750
Thr Pro Pro Ile Pro Ser Lys Pro Pro Gly Gly Phe Gly Lys Thr Ser
755 760 765
Gly Thr Pro Ala Val Lys Met Arg Asn Gly Val Arg Gln Val Ala Val
770 775 780
Arg Pro Gln Ser Val Phe Val Ser Pro Pro Pro Lys Asp Asn Asn Leu
785 790 795 800
Ser Cys Ala Leu Arg Arg Asn Glu Ser Leu Thr Ala Thr Asp Gly Leu
805 810 815
Arg Gly Val Arg Arg Asn Ser Ser Phe Ser Thr Ala Arg Ser Ala Ala
820 825 830
Ala Glu Ala Lys Gly Arg Leu Ala Glu Arg Ala Ala Ser Gln Gly Ser
835 840 845
Asp Ser Pro Leu Leu Pro Ala Gln Arg Asn Ser Ile Pro Val Ser Pro
850 855 860
Val Arg Pro Lys Pro Ile Glu Lys Ser Gln Phe Ile His Asn Asn Leu
865 870 875 880
Lys Asp Val Tyr Val Ser Ile Ala Asp Tyr Glu Gly Asp Glu Glu Thr
885 890 895
Ala Gly Phe Gln Glu Gly Val Ser Met Glu Val Leu Glu Arg Asn Pro
900 905 910
Asn Gly Trp Trp Tyr Cys Gln Ile Leu Asp Gly Val Lys Pro Phe Lys
915 920 925
Gly Trp Val Pro Ser Asn Tyr Leu Glu Lys Lys Asn
930 935 940

<210> 275
<211> 871
<212> PRT
<213> Homo sapiens
<400> 275
Met Lys Tyr Ser Cys Cys Ala Leu Val Leu Ala Val Leu Gly Thr Glu
1 5 10 15
Leu Leu Gly Ser Leu Cys Ser Thr Val Arg Ser Pro Arg Phe Arg Gly
20 25 30
Arg Ile Gln Gln Glu Arg Lys Asn Ile Arg Pro Asn Ile Ile Leu Val
35 40 45
Pro Thr Asp Asp Gln Asp Val Glu Leu Gly Ser Leu Gln Val Met Asn
50 55 60
Lys Thr Arg Lys Ile Met Glu His Gly Gly Ala Thr Phe Ile Asn Ala
65 70 75 80
Phe Val Thr Thr Pro Met Cys Cys Pro Ser Arg Ser Ser Met Leu Thr
85 90 95
Gly Lys Tyr Val His Asn His Asn Val Tyr Thr Asn Asn Glu Asn Cys
100 105 110
Ser Ser Pro Ser Trp Gln Ala Met His Glu Pro Arg Thr Phe Ala Val
115 120 125
Tyr Leu Asn Asn Thr Gly Tyr Arg Thr Ala Phe Phe Gly Lys Tyr Leu
130 135 140
Asn Glu Tyr Asn Gly Ser Tyr Ile Pro Pro Gly Trp Arg Glu Trp Leu
145 150 155 160
Gly Leu Ile Lys Asn Ser Arg Phe Tyr Asn Tyr Thr Val Cys Arg Asn
165 170 175
Gly Ile Lys Glu Lys His Gly Phe Asp Tyr Ala Lys Asp Tyr Phe Thr
180 185 190
Asp Leu Ile Thr Asn Glu Ser Ile Asn Tyr Phe Lys Met Ser Lys Arg
195 200 205
Met Tyr Pro His Arg Pro Val Met Met Val Ile Ser His Ala Ala Pro

```
        210                 215                 220
His Gly Pro Glu Asp Ser Ala Pro Gln Phe Ser Lys Leu Tyr Pro Asn
225                 230                 235                 240
Ala Ser Gln His Ile Thr Pro Ser Tyr Asn Tyr Ala Pro Asn Met Asp
                245                 250                 255
Lys His Trp Ile Met Gln Tyr Thr Gly Pro Met Leu Pro Ile His Met
            260                 265                 270
Glu Phe Thr Asn Ile Leu Gln Arg Lys Arg Leu Gln Thr Leu Met Ser
        275                 280                 285
Val Asp Asp Ser Val Glu Arg Leu Tyr Asn Met Leu Val Glu Thr Gly
    290                 295                 300
Glu Leu Glu Asn Thr Tyr Ile Ile Tyr Thr Ala Asp His Gly Tyr His
305                 310                 315                 320
Ile Gly Gln Phe Gly Leu Val Lys Gly Lys Ser Met Pro Tyr Asp Phe
                325                 330                 335
Asp Ile Arg Val Pro Phe Phe Ile Arg Gly Pro Ser Val Glu Pro Gly
            340                 345                 350
Ser Ile Val Pro Gln Ile Val Leu Asn Ile Asp Leu Ala Pro Thr Ile
        355                 360                 365
Leu Asp Ile Ala Gly Leu Asp Thr Pro Pro Asp Val Asp Gly Lys Ser
    370                 375                 380
Val Leu Lys Leu Leu Asp Pro Glu Lys Pro Gly Asn Arg Phe Arg Thr
385                 390                 395                 400
Asn Lys Lys Ala Lys Ile Trp Arg Asp Thr Phe Leu Val Glu Arg Gly
                405                 410                 415
Lys Phe Leu Arg Lys Lys Glu Glu Ser Ser Lys Asn Ile Gln Gln Ser
            420                 425                 430
Asn His Leu Pro Lys Tyr Glu Arg Val Lys Glu Leu Cys Gln Gln Ala
        435                 440                 445
Arg Tyr Gln Thr Ala Cys Glu Gln Pro Gly Gln Lys Trp Gln Cys Ile
    450                 455                 460
Glu Asp Thr Ser Gly Lys Leu Arg Ile His Lys Cys Lys Gly Pro Ser
465                 470                 475                 480
Asp Leu Leu Thr Val Arg Gln Ser Thr Arg Asn Leu Tyr Ala Arg Gly
                485                 490                 495
Phe His Asp Lys Asp Lys Glu Cys Ser Cys Arg Glu Ser Gly Tyr Arg
            500                 505                 510
Ala Ser Arg Ser Gln Arg Lys Ser Gln Arg Gln Phe Leu Arg Asn Gln
        515                 520                 525
Gly Thr Pro Lys Tyr Lys Pro Arg Phe Val His Thr Arg Gln Thr Arg
    530                 535                 540
Ser Leu Ser Val Glu Phe Glu Gly Glu Ile Tyr Asp Ile Asn Leu Glu
545                 550                 555                 560
Glu Glu Glu Glu Leu Gln Val Leu Gln Pro Arg Asn Ile Ala Lys Arg
                565                 570                 575
His Asp Glu Gly His Lys Gly Pro Arg Asp Leu Gln Ala Ser Ser Gly
            580                 585                 590
Gly Asn Arg Gly Arg Met Leu Ala Asp Ser Ser Asn Ala Val Gly Pro
        595                 600                 605
Pro Thr Thr Val Arg Val Thr His Lys Cys Phe Ile Leu Pro Asn Asp
    610                 615                 620
Ser Ile His Cys Glu Arg Glu Leu Tyr Gln Ser Ala Arg Ala Trp Lys
625                 630                 635                 640
Asp His Lys Ala Tyr Ile Asp Lys Glu Ile Glu Ala Leu Gln Asp Lys
                645                 650                 655
Ile Lys Asn Leu Arg Glu Val Arg Gly His Leu Lys Arg Arg Lys Pro
            660                 665                 670
Glu Glu Cys Ser Cys Ser Lys Gln Ser Tyr Tyr Asn Lys Glu Lys Gly
        675                 680                 685
Val Lys Lys Gln Glu Lys Leu Lys Ser His Leu His Pro Phe Lys Glu
    690                 695                 700
Ala Ala Gln Glu Val Asp Ser Lys Leu Gln Leu Phe Lys Glu Asn Asn
705                 710                 715                 720
Arg Arg Arg Lys Lys Glu Arg Lys Glu Lys Arg Arg Gln Arg Lys Gly
                725                 730                 735
Glu Glu Cys Ser Leu Pro Gly Leu Thr Cys Phe Thr His Asp Asn Asn
            740                 745                 750
His Trp Gln Thr Ala Pro Phe Trp Asn Leu Gly Ser Phe Cys Ala Cys
        755                 760                 765
```

Thr Ser Ser Asn Asn Asn Thr Tyr Trp Cys Leu Arg Thr Val Asn Glu
    770                 775                 780
Thr His Asn Phe Leu Phe Cys Glu Phe Ala Thr Gly Phe Leu Glu Tyr
785                 790                 795                 800
Phe Asp Met Asn Thr Asp Pro Tyr Gln Leu Thr Asn Thr Val His Thr
                805                 810                 815
Val Glu Arg Gly Ile Leu Asn Gln Leu His Val Gln Leu Met Glu Leu
            820                 825                 830
Arg Ser Cys Gln Gly Tyr Lys Gln Cys Asn Pro Arg Pro Lys Asn Leu
        835                 840                 845
Asp Val Gly Asn Lys Asp Gly Gly Ser Tyr Asp Leu His Arg Gly Gln
    850                 855                 860
Leu Trp Asp Gly Trp Glu Gly
865                 870

<210> 276
<211> 1426
<212> PRT
<213> Homo sapiens
<400> 276
Met Asn Asp Trp His Arg Ile Phe Thr Gln Asn Val Leu Val Pro Pro
1               5                   10                  15
His Pro Gln Arg Ala Arg Gln Pro Trp Lys Glu Ser Thr Ala Phe Gln
            20                  25                  30
Cys Val Leu Lys Trp Leu Asp Gly Pro Val Ile Arg Gln Gly Val Leu
        35                  40                  45
Glu Val Leu Ser Glu Val Glu Cys His Leu Arg Val Ser Phe Phe Asp
    50                  55                  60
Val Thr Tyr Arg His Phe Phe Gly Arg Thr Trp Lys Thr Thr Val Lys
65                  70                  75                  80
Pro Thr Lys Arg Pro Pro Ser Arg Ile Val Phe Asn Glu Pro Leu Tyr
                85                  90                  95
Phe His Thr Ser Leu Asn His Pro His Ile Val Ala Val Val Glu Val
            100                 105                 110
Val Ala Glu Gly Lys Lys Arg Asp Gly Ser Leu Gln Thr Leu Ser Cys
        115                 120                 125
Gly Phe Gly Ile Leu Arg Ile Phe Ser Asn Gln Pro Asp Ser Pro Ile
    130                 135                 140
Ser Ala Ser Gln Asp Lys Arg Leu Arg Leu Tyr His Gly Thr Pro Arg
145                 150                 155                 160
Ala Leu Leu His Pro Leu Leu Gln Asp Pro Ala Glu Gln Asn Arg His
                165                 170                 175
Met Thr Leu Ile Glu Asn Cys Ser Leu Gln Tyr Thr Leu Lys Pro His
            180                 185                 190
Pro Ala Leu Glu Pro Ala Phe His Leu Leu Pro Glu Asn Leu Leu Val
        195                 200                 205
Ser Gly Leu Gln Gln Ile Pro Gly Leu Leu Pro Ala His Gly Glu Ser
    210                 215                 220
Gly Asp Ala Leu Arg Lys Pro Arg Leu Gln Lys Pro Ile Thr Gly His
225                 230                 235                 240
Leu Asp Asp Leu Phe Phe Thr Leu Tyr Pro Ser Leu Glu Lys Phe Glu
                245                 250                 255
Glu Glu Leu Leu Glu Leu His Val Gln Asp His Phe Gln Glu Gly Cys
            260                 265                 270
Gly Pro Leu Asp Gly Gly Ala Leu Glu Ile Leu Glu Arg Arg Leu Arg
        275                 280                 285
Val Gly Val His Asn Gly Leu Gly Phe Val Gln Arg Pro Gln Val Val
    290                 295                 300
Val Leu Val Pro Glu Met Asp Val Ala Leu Thr Arg Ser Ala Ser Phe
305                 310                 315                 320
Ser Arg Lys Val Val Ser Ser Ser Lys Thr Ser Ser Gly Ser Gln Ala
                325                 330                 335
Leu Val Leu Arg Ser Arg Leu Arg Leu Pro Glu Met Val Gly His Pro
            340                 345                 350
Ala Phe Ala Val Ile Phe Gln Leu Glu Tyr Val Phe Ser Ser Pro Ala
        355                 360                 365
Gly Val Asp Gly Asn Ala Ala Ser Val Thr Ser Leu Ser Asn Leu Ala
    370                 375                 380
Cys Met His Met Val Arg Trp Ala Val Trp Asn Pro Leu Leu Glu Ala

```
385                 390                 395                 400
Asp Ser Gly Arg Val Thr Leu Pro Leu Gln Gly Gly Ile Gln Pro Asn
                405                 410                 415
Pro Ser His Cys Leu Val Tyr Lys Val Pro Ser Ala Ser Met Ser Ser
            420                 425                 430
Glu Glu Val Lys Gln Val Glu Ser Gly Thr Leu Arg Phe Gln Phe Ser
        435                 440                 445
Leu Gly Ser Glu Glu His Leu Asp Ala Pro Thr Glu Pro Val Ser Gly
    450                 455                 460
Pro Lys Val Glu Arg Arg Pro Ser Arg Lys Pro Pro Thr Ser Pro Ser
465                 470                 475                 480
Ser Pro Pro Ala Pro Val Pro Arg Val Leu Ala Ala Pro Gln Asn Ser
                485                 490                 495
Pro Val Gly Pro Gly Leu Ser Ile Ser Gln Leu Ala Ala Ser Pro Arg
            500                 505                 510
Ser Pro Thr Gln His Cys Leu Ala Arg Pro Thr Ser Gln Leu Pro His
        515                 520                 525
Gly Ser Gln Ala Ser Pro Ala Gln Ala Gln Glu Phe Pro Leu Glu Ala
    530                 535                 540
Gly Ile Ser His Leu Glu Ala Asp Leu Ser Gln Thr Ser Leu Val Leu
545                 550                 555                 560
Glu Thr Ser Ile Ala Glu Gln Leu Gln Glu Leu Pro Phe Thr Pro Leu
                565                 570                 575
His Ala Pro Ile Val Val Gly Thr Gln Thr Arg Ser Ser Ala Gly Gln
            580                 585                 590
Pro Ser Arg Ala Ser Met Val Leu Leu Gln Ser Ser Gly Phe Pro Glu
        595                 600                 605
Ile Leu Asp Ala Asn Lys Gln Pro Ala Glu Ala Val Ser Ala Thr Glu
    610                 615                 620
Pro Val Thr Phe Asn Pro Gln Lys Glu Glu Ser Asp Cys Leu Gln Ser
625                 630                 635                 640
Asn Glu Met Val Leu Gln Phe Leu Ala Phe Ser Arg Val Ala Gln Asp
                645                 650                 655
Cys Arg Gly Thr Ser Trp Pro Lys Thr Val Tyr Phe Thr Phe Gln Phe
            660                 665                 670
Tyr Arg Phe Pro Pro Ala Thr Thr Pro Arg Leu Gln Leu Val Gln Leu
        675                 680                 685
Asp Glu Ala Gly Gln Pro Ser Ser Gly Ala Leu Thr His Ile Leu Val
    690                 695                 700
Pro Val Ser Arg Asp Gly Thr Phe Asp Ala Gly Ser Pro Gly Phe Gln
705                 710                 715                 720
Leu Arg Tyr Met Val Gly Pro Gly Phe Leu Lys Pro Gly Glu Arg Arg
                725                 730                 735
Cys Phe Ala Arg Tyr Leu Ala Val Gln Thr Leu Gln Ile Asp Val Trp
            740                 745                 750
Asp Gly Asp Ser Leu Leu Leu Ile Gly Ser Ala Ala Val Gln Met Lys
        755                 760                 765
His Leu Leu Arg Gln Gly Arg Pro Ala Val Gln Ala Ser His Glu Leu
    770                 775                 780
Glu Val Val Ala Thr Glu Tyr Glu Gln Asp Asn Met Val Val Ser Gly
785                 790                 795                 800
Asp Met Leu Gly Phe Gly Arg Val Lys Pro Ile Gly Val His Ser Val
                805                 810                 815
Val Lys Gly Arg Leu His Leu Thr Leu Ala Asn Val Gly His Pro Cys
            820                 825                 830
Glu Gln Lys Val Arg Gly Cys Ser Thr Leu Pro Pro Ser Arg Ser Arg
        835                 840                 845
Val Ile Ser Asn Asp Gly Ala Ser Arg Phe Ser Gly Gly Ser Leu Leu
    850                 855                 860
Thr Thr Gly Ser Ser Arg Arg Lys His Val Val Gln Ala Gln Lys Leu
865                 870                 875                 880
Ala Asp Val Asp Ser Glu Leu Ala Ala Met Leu Leu Thr His Ala Arg
                885                 890                 895
Gln Gly Lys Gly Pro Gln Asp Val Ser Arg Glu Ser Asp Ala Thr Arg
            900                 905                 910
Arg Arg Lys Leu Glu Arg Met Arg Ser Val Arg Leu Gln Glu Ala Gly
        915                 920                 925
Gly Asp Leu Gly Arg Arg Gly Thr Ser Val Leu Ala Gln Gln Ser Val
    930                 935                 940
```

```
Arg Thr Gln His Leu Arg Asp Leu Gln Val Ile Ala Ala Tyr Arg Glu
945                 950                 955                 960
Arg Thr Lys Ala Glu Ser Ile Ala Ser Leu Leu Ser Leu Ala Ile Thr
                965                 970                 975
Thr Glu His Thr Leu His Ala Thr Leu Gly Val Ala Glu Phe Phe Glu
            980                 985                 990
Phe Val Leu Lys Asn Pro His Asn  Thr Gln His Thr Val  Thr Val Glu
        995                 1000                1005
Ile Asp  Asn Pro Glu Leu Ser  Val Ile Val Asp Ser  Gln Glu Trp
    1010                 1015                 1020
Arg Asp  Phe Lys Gly Ala Ala  Gly Leu His Thr Pro  Val Glu Glu
    1025                 1030                 1035
Asp Met  Phe His Leu Arg Gly  Ser Leu Ala Pro Gln  Leu Tyr Leu
    1040                 1045                 1050
Arg Pro  His Glu Thr Ala His  Val Pro Phe Lys Phe  Gln Ser Phe
    1055                 1060                 1065
Ser Ala  Gly Gln Leu Ala Met  Val Gln Ala Ser Pro  Gly Leu Ser
    1070                 1075                 1080
Asn Glu  Lys Gly Met Asp Ala  Val Ser Pro Trp Lys  Ser Ser Ala
    1085                 1090                 1095
Val Pro  Thr Lys His Ala Lys  Val Leu Phe Arg Ala  Ser Gly Gly
    1100                 1105                 1110
Lys Pro  Ile Ala Val Leu Cys  Leu Thr Val Glu Leu  Gln Pro His
    1115                 1120                 1125
Val Val  Asp Gln Val Phe Arg  Phe Tyr His Pro Glu  Leu Ser Phe
    1130                 1135                 1140
Leu Lys  Lys Ala Ile Arg Leu  Pro Pro Trp His Thr  Phe Pro Gly
    1145                 1150                 1155
Ala Pro  Val Gly Met Leu Gly  Glu Asp Pro Pro Val  His Val Arg
    1160                 1165                 1170
Cys Ser  Asp Pro Asn Val Ile  Cys Glu Thr Gln Asn  Val Gly Pro
    1175                 1180                 1185
Gly Glu  Pro Arg Asp Ile Phe  Leu Lys Val Ala Ser  Gly Pro Ser
    1190                 1195                 1200
Pro Glu  Ile Lys Asp Phe Phe  Val Ile Ile Tyr Ser  Asp Arg Trp
    1205                 1210                 1215
Leu Ala  Thr Pro Thr Gln Thr  Trp Gln Val Tyr Leu  His Ser Leu
    1220                 1225                 1230
Gln Arg  Val Asp Val Ser Cys  Val Ala Gly Gln Leu  Thr Arg Leu
    1235                 1240                 1245
Ser Leu  Val Leu Arg Gly Thr  Gln Thr Val Arg Lys  Val Arg Ala
    1250                 1255                 1260
Phe Thr  Ser His Pro Gln Glu  Leu Lys Thr Asp Pro  Lys Gly Val
    1265                 1270                 1275
Phe Val  Leu Pro Pro Arg Gly  Val Gln Asp Leu His  Val Gly Val
    1280                 1285                 1290
Arg Pro  Leu Arg Ala Gly Ser  Arg Phe Val His Leu  Asn Leu Val
    1295                 1300                 1305
Asp Val  Asp Cys His Gln Leu  Val Ala Ser Trp Leu  Val Cys Leu
    1310                 1315                 1320
Cys Cys  Arg Gln Pro Leu Ile  Ser Lys Ala Phe Glu  Ile Met Leu
    1325                 1330                 1335
Ala Ala  Gly Glu Gly Lys Gly  Val Asn Lys Arg Ile  Thr Tyr Thr
    1340                 1345                 1350
Asn Pro  Tyr Pro Ser Arg Arg  Thr Phe His Leu His  Ser Asp His
    1355                 1360                 1365
Pro Glu  Leu Leu Arg Phe Arg  Glu Asp Ser Phe Gln  Val Gly Gly
    1370                 1375                 1380
Gly Glu  Thr Tyr Thr Ile Gly  Leu Gln Phe Ala Pro  Ser Gln Arg
    1385                 1390                 1395
Val Gly  Glu Glu Glu Ile Leu  Ile Tyr Ile Asn Asp  His Glu Asp
    1400                 1405                 1410
Lys Asn  Glu Glu Ala Phe Cys  Val Lys Val Ile Tyr  Gln
    1415                 1420                 1425
```

```
<210>  277
<211>  146
<212>  PRT
<213>  Homo sapiens
```

<400> 277
Met Met Trp Gln Lys Tyr Ala Gly Ser Arg Arg Ser Met Pro Leu Gly
1 5 10 15
Val Arg Ile Leu Phe His Gly Val Phe Tyr Ala Gly Gly Phe Ala Ile
20 25 30
Val Tyr Tyr Leu Ile Gln Lys Phe His Ser Arg Ala Leu Tyr Tyr Lys
35 40 45
Leu Ala Val Glu Gln Leu Gln Ser His Pro Glu Ala Gln Glu Ala Leu
50 55 60
Gly Pro Pro Leu Asn Ile His Tyr Leu Lys Leu Ile Asp Arg Glu Asn
65 70 75 80
Phe Val Asp Ile Val Asp Ala Lys Leu Lys Ile Pro Val Ser Gly Ser
85 90 95
Lys Ser Glu Gly Leu Leu Tyr Val His Ser Ser Arg Gly Gly Pro Phe
100 105 110
Gln Arg Trp His Leu Asp Glu Val Phe Leu Glu Leu Lys Asp Gly Gln
115 120 125
Gln Ile Pro Val Phe Lys Leu Ser Gly Glu Asn Gly Asp Glu Val Lys
130 135 140
Lys Glu
145

<210> 278
<211> 402
<212> PRT
<213> Homo sapiens
<400> 278
Met Lys Glu Thr Arg Gly Tyr Gly Gly Asp Ala Pro Phe Cys Thr Arg
1 5 10 15
Leu Asn His Ser Tyr Thr Gly Met Trp Ala Pro Glu Arg Ser Ala Glu
20 25 30
Ala Arg Gly Asn Leu Thr Arg Pro Pro Gly Ser Gly Glu Asp Cys Gly
35 40 45
Ser Val Ser Val Ala Phe Pro Ile Thr Met Leu Leu Thr Gly Phe Val
50 55 60
Gly Asn Ala Leu Ala Met Leu Leu Val Ser Arg Ser Tyr Arg Arg Arg
65 70 75 80
Glu Ser Lys Arg Lys Lys Ser Phe Leu Leu Cys Ile Gly Trp Leu Ala
85 90 95
Leu Thr Asp Leu Val Gly Gln Leu Leu Thr Thr Pro Val Val Ile Val
100 105 110
Val Tyr Leu Ser Lys Gln Arg Trp Glu His Ile Asp Pro Ser Gly Arg
115 120 125
Leu Cys Thr Phe Phe Gly Leu Thr Met Thr Val Phe Gly Leu Ser Ser
130 135 140
Leu Phe Ile Ala Ser Ala Met Ala Val Glu Arg Ala Leu Ala Ile Arg
145 150 155 160
Ala Pro His Trp Tyr Ala Ser His Met Lys Thr Arg Ala Thr Arg Ala
165 170 175
Val Leu Leu Gly Val Trp Leu Ala Val Leu Ala Phe Ala Leu Leu Pro
180 185 190
Val Leu Gly Val Gly Gln Tyr Thr Val Gln Trp Pro Gly Thr Trp Cys
195 200 205
Phe Ile Ser Thr Gly Arg Gly Gly Asn Gly Thr Ser Ser Ser His Asn
210 215 220
Trp Gly Asn Leu Phe Phe Ala Ser Ala Phe Ala Phe Leu Gly Leu Leu
225 230 235 240
Ala Leu Thr Val Thr Phe Ser Cys Asn Leu Ala Thr Ile Lys Ala Leu
245 250 255
Val Ser Arg Cys Arg Ala Lys Ala Thr Ala Ser Gln Ser Ser Ala Gln
260 265 270
Trp Gly Arg Ile Thr Thr Glu Thr Ala Ile Gln Leu Met Gly Ile Met
275 280 285
Cys Val Leu Ser Val Cys Trp Ser Pro Leu Leu Ile Met Met Leu Lys
290 295 300
Met Ile Phe Asn Gln Thr Ser Val Glu His Cys Lys Thr His Thr Glu
305 310 315 320
Lys Gln Lys Glu Cys Asn Phe Phe Leu Ile Ala Val Arg Leu Ala Ser
325 330 335

Leu Asn Gln Ile Leu Asp Pro Trp Val Tyr Leu Leu Leu Arg Lys Ile
340 345 350
Leu Leu Arg Lys Phe Cys Gln Met Arg Lys Arg Arg Leu Arg Glu Gln
355 360 365
Glu Met Gly Pro Asp Gly Arg Cys Phe Cys His Ala Trp Arg Gln Val
370 375 380
Pro Arg Thr Trp Cys Ser Ser His Asp Arg Glu Pro Cys Ser Val Gln
385 390 395 400
Leu Ser

<210> 279
<211> 1741
<212> PRT
<213> Homo sapiens
<400> 279
Met Arg Leu Leu Trp Gly Leu Ile Trp Ala Ser Ser Phe Phe Thr Leu
1 5 10 15
Ser Leu Gln Lys Pro Arg Leu Leu Leu Phe Ser Pro Ser Val Val His
20 25 30
Leu Gly Val Pro Leu Ser Val Gly Val Gln Leu Gln Asp Val Pro Arg
35 40 45
Gly Gln Val Val Lys Gly Ser Val Phe Leu Arg Asn Pro Ser Arg Asn
50 55 60
Asn Val Pro Cys Ser Pro Lys Val Asp Phe Thr Leu Ser Ser Glu Arg
65 70 75 80
Asp Phe Ala Leu Leu Ser Leu Gln Val Pro Leu Lys Asp Ala Lys Ser
85 90 95
Cys Gly Leu His Gln Leu Leu Arg Gly Pro Glu Val Gln Leu Val Ala
100 105 110
His Ser Pro Trp Leu Lys Asp Ser Leu Ser Arg Thr Thr Asn Ile Gln
115 120 125
Gly Ile Asn Leu Leu Phe Ser Ser Arg Arg Gly His Leu Phe Leu Gln
130 135 140
Thr Asp Gln Pro Ile Tyr Asn Pro Gly Gln Arg Val Arg Tyr Arg Val
145 150 155 160
Phe Ala Leu Asp Gln Lys Met Arg Pro Ser Thr Asp Thr Ile Thr Val
165 170 175
Met Val Glu Asn Ser His Gly Leu Arg Val Arg Lys Lys Glu Val Tyr
180 185 190
Met Pro Ser Ser Ile Phe Gln Asp Asp Phe Val Ile Pro Asp Ile Ser
195 200 205
Glu Pro Gly Thr Trp Lys Ile Ser Ala Arg Phe Ser Asp Gly Leu Glu
210 215 220
Ser Asn Ser Ser Thr Gln Phe Glu Val Lys Lys Tyr Val Leu Pro Asn
225 230 235 240
Phe Glu Val Lys Ile Thr Pro Gly Lys Pro Tyr Ile Leu Thr Val Pro
245 250 255
Gly His Leu Asp Glu Met Gln Leu Asp Ile Gln Ala Arg Tyr Ile Tyr
260 265 270
Gly Lys Pro Val Gln Gly Val Ala Tyr Val Arg Phe Gly Leu Leu Asp
275 280 285
Glu Asp Gly Lys Lys Thr Phe Phe Arg Gly Leu Glu Ser Gln Thr Lys
290 295 300
Leu Val Asn Gly Gln Ser His Ile Ser Leu Ser Lys Ala Glu Phe Gln
305 310 315 320
Asp Ala Leu Glu Lys Leu Asn Met Gly Ile Thr Asp Leu Gln Gly Leu
325 330 335
Arg Leu Tyr Val Ala Ala Ala Ile Ile Glu Ser Pro Gly Gly Glu Met
340 345 350
Glu Glu Ala Glu Leu Thr Ser Trp Tyr Phe Val Ser Ser Pro Phe Ser
355 360 365
Leu Asp Leu Ser Lys Thr Lys Arg His Leu Val Pro Gly Ala Pro Phe
370 375 380
Leu Leu Gln Ala Leu Val Arg Glu Met Ser Gly Ser Pro Ala Ser Gly
385 390 395 400
Ile Pro Val Lys Val Ser Ala Thr Val Ser Ser Pro Gly Ser Val Pro
405 410 415
Glu Ala Gln Asp Ile Gln Gln Asn Thr Asp Gly Ser Gly Gln Val Ser

```
                420                 425                 430
Ile Pro Ile Ile Ile Pro Gln Thr Ile Ser Glu Leu Gln Leu Ser Val
            435                 440                 445
Ser Ala Gly Ser Pro His Pro Ala Ile Ala Arg Leu Thr Val Ala Ala
    450                 455                 460
Pro Pro Ser Gly Gly Pro Gly Phe Leu Ser Ile Glu Arg Pro Asp Ser
465                 470                 475                 480
Arg Pro Pro Arg Val Gly Asp Thr Leu Asn Leu Asn Leu Arg Ala Val
                485                 490                 495
Gly Ser Gly Ala Thr Phe Ser His Tyr Tyr Tyr Met Ile Leu Ser Arg
                500                 505                 510
Gly Gln Ile Val Phe Met Asn Arg Glu Pro Lys Arg Thr Leu Thr Ser
            515                 520                 525
Val Ser Val Phe Val Asp His His Leu Ala Pro Ser Phe Tyr Phe Val
    530                 535                 540
Ala Phe Tyr Tyr His Gly Asp His Pro Val Ala Asn Ser Leu Arg Val
545                 550                 555                 560
Asp Val Gln Ala Gly Ala Cys Glu Gly Lys Leu Glu Leu Ser Val Asp
                565                 570                 575
Gly Ala Lys Gln Tyr Arg Asn Gly Glu Ser Val Lys Leu His Leu Glu
                580                 585                 590
Thr Asp Ser Leu Ala Leu Val Ala Leu Gly Ala Leu Asp Thr Ala Leu
            595                 600                 605
Tyr Ala Ala Gly Ser Lys Ser His Lys Pro Leu Asn Met Gly Lys Val
    610                 615                 620
Phe Glu Ala Met Asn Ser Tyr Asp Leu Gly Cys Gly Pro Gly Gly Gly
625                 630                 635                 640
Asp Ser Ala Leu Gln Val Phe Gln Ala Ala Gly Leu Ala Phe Ser Asp
                645                 650                 655
Gly Asp Gln Trp Thr Leu Ser Arg Lys Arg Leu Ser Cys Pro Lys Glu
                660                 665                 670
Lys Thr Thr Arg Lys Lys Arg Asn Val Asn Phe Gln Lys Ala Ile Asn
            675                 680                 685
Glu Lys Leu Gly Gln Tyr Ala Ser Pro Thr Ala Lys Arg Cys Cys Gln
    690                 695                 700
Asp Gly Val Thr Arg Leu Pro Met Met Arg Ser Cys Glu Gln Arg Ala
705                 710                 715                 720
Ala Arg Val Gln Gln Pro Asp Cys Arg Glu Pro Phe Leu Ser Cys Cys
                725                 730                 735
Gln Phe Ala Glu Ser Leu Arg Lys Lys Ser Arg Asp Lys Gly Gln Ala
                740                 745                 750
Gly Leu Gln Arg Ala Leu Glu Ile Leu Gln Glu Glu Asp Leu Ile Asp
            755                 760                 765
Glu Asp Asp Ile Pro Val Arg Ser Phe Phe Pro Glu Asn Trp Leu Trp
    770                 775                 780
Arg Val Glu Thr Val Asp Arg Phe Gln Ile Leu Thr Leu Trp Leu Pro
785                 790                 795                 800
Asp Ser Leu Thr Thr Trp Glu Ile His Gly Leu Ser Leu Ser Lys Thr
                805                 810                 815
Lys Gly Leu Cys Val Ala Thr Pro Val Gln Leu Arg Val Phe Arg Glu
                820                 825                 830
Phe His Leu His Leu Arg Leu Pro Met Ser Val Arg Arg Phe Glu Gln
            835                 840                 845
Leu Glu Leu Arg Pro Val Leu Tyr Asn Tyr Leu Asp Lys Asn Leu Thr
    850                 855                 860
Val Ser Val His Val Ser Pro Val Glu Gly Leu Cys Leu Ala Gly Gly
865                 870                 875                 880
Gly Gly Leu Ala Gln Gln Val Leu Val Pro Ala Gly Ser Ala Arg Pro
                885                 890                 895
Val Ala Phe Ser Val Val Pro Thr Ala Ala Ala Ala Val Ser Leu Lys
                900                 905                 910
Val Val Ala Arg Gly Ser Phe Glu Phe Pro Val Gly Asp Ala Val Ser
            915                 920                 925
Lys Val Leu Gln Ile Glu Lys Glu Gly Ala Ile His Arg Glu Glu Leu
    930                 935                 940
Val Tyr Glu Leu Asn Pro Leu Asp His Arg Gly Arg Thr Leu Glu Ile
945                 950                 955                 960
Pro Gly Asn Ser Asp Pro Asn Met Ile Pro Asp Gly Asp Phe Asn Ser
                965                 970                 975
```

Tyr Val Arg Val Thr Ala Ser Asp Pro Leu Asp Thr Leu Gly Ser Glu
980 985 990
Gly Ala Leu Ser Pro Gly Gly Val Ala Ser Leu Leu Arg Leu Pro Arg
995 1000 1005
Gly Cys Gly Glu Gln Thr Met Ile Tyr Leu Ala Pro Thr Leu Ala
1010 1015 1020
Ala Ser Arg Tyr Leu Asp Lys Thr Glu Gln Trp Ser Thr Leu Pro
1025 1030 1035
Pro Glu Thr Lys Asp His Ala Val Asp Leu Ile Gln Lys Gly Tyr
1040 1045 1050
Met Arg Ile Gln Gln Phe Arg Lys Ala Asp Gly Ser Tyr Ala Ala
1055 1060 1065
Trp Leu Ser Arg Asp Ser Ser Thr Trp Leu Thr Ala Phe Val Leu
1070 1075 1080
Lys Val Leu Ser Leu Ala Gln Glu Gln Val Gly Gly Ser Pro Glu
1085 1090 1095
Lys Leu Gln Glu Thr Ser Asn Trp Leu Leu Ser Gln Gln Gln Ala
1100 1105 1110
Asp Gly Ser Phe Gln Asp Pro Cys Pro Val Leu Asp Arg Ser Met
1115 1120 1125
Gln Gly Gly Leu Val Gly Asn Asp Glu Thr Val Ala Leu Thr Ala
1130 1135 1140
Phe Val Thr Ile Ala Leu His His Gly Leu Ala Val Phe Gln Asp
1145 1150 1155
Glu Gly Ala Glu Pro Leu Lys Gln Arg Val Glu Ala Ser Ile Ser
1160 1165 1170
Lys Ala Asn Ser Phe Leu Gly Glu Lys Ala Ser Ala Gly Leu Leu
1175 1180 1185
Gly Ala His Ala Ala Ala Ile Thr Ala Tyr Ala Leu Ser Leu Thr
1190 1195 1200
Lys Ala Pro Val Asp Leu Leu Gly Val Ala His Asn Asn Leu Met
1205 1210 1215
Ala Met Ala Gln Glu Thr Gly Asp Asn Leu Tyr Trp Gly Ser Val
1220 1225 1230
Thr Gly Ser Gln Ser Asn Ala Val Ser Pro Thr Pro Ala Pro Arg
1235 1240 1245
Asn Pro Ser Asp Pro Met Pro Gln Ala Pro Ala Leu Trp Ile Glu
1250 1255 1260
Thr Thr Ala Tyr Ala Leu Leu His Leu Leu Leu His Glu Gly Lys
1265 1270 1275
Ala Glu Met Ala Asp Gln Ala Ser Ala Trp Leu Thr Arg Gln Gly
1280 1285 1290
Ser Phe Gln Gly Gly Phe Arg Ser Thr Gln Asp Thr Val Ile Ala
1295 1300 1305
Leu Asp Ala Leu Ser Ala Tyr Trp Ile Ala Ser His Thr Thr Glu
1310 1315 1320
Glu Arg Gly Leu Asn Val Thr Leu Ser Ser Thr Gly Arg Asn Gly
1325 1330 1335
Phe Lys Ser His Ala Leu Gln Leu Asn Asn Arg Gln Ile Arg Gly
1340 1345 1350
Leu Glu Glu Glu Leu Gln Phe Ser Leu Gly Ser Lys Ile Asn Val
1355 1360 1365
Lys Val Gly Gly Asn Ser Lys Gly Thr Leu Lys Val Leu Arg Thr
1370 1375 1380
Tyr Asn Val Leu Asp Met Lys Asn Thr Thr Cys Gln Asp Leu Gln
1385 1390 1395
Ile Glu Val Thr Val Lys Gly His Val Glu Tyr Thr Met Glu Ala
1400 1405 1410
Asn Glu Asp Tyr Glu Tyr Asp Glu Leu Pro Ala Lys Asp Asp Pro
1415 1420 1425
Asp Ala Pro Leu Gln Pro Val Thr Pro Leu Gln Leu Phe Glu Gly
1430 1435 1440
Arg Arg Asn Arg Arg Arg Arg Glu Ala Pro Lys Val Val Glu Glu
1445 1450 1455
Gln Glu Ser Arg Val His Tyr Thr Val Cys Ile Trp Arg Asn Gly
1460 1465 1470
Lys Val Gly Leu Ser Gly Met Ala Ile Ala Asp Val Thr Leu Leu
1475 1480 1485
Ser Gly Phe His Ala Leu Arg Ala Asp Leu Glu Lys Leu Thr Ser

1490 1495 1500
Leu Ser Asp Arg Tyr Val Ser His Phe Glu Thr Glu Gly Pro His
1505 1510 1515
Val Leu Leu Tyr Phe Asp Ser Val Pro Thr Ser Arg Glu Cys Val
1520 1525 1530
Gly Phe Glu Ala Val Gln Glu Val Pro Val Gly Leu Val Gln Pro
1535 1540 1545
Ala Ser Ala Thr Leu Tyr Asp Tyr Tyr Asn Pro Glu Arg Arg Cys
1550 1555 1560
Ser Val Phe Tyr Gly Ala Pro Ser Lys Ser Arg Leu Leu Ala Thr
1565 1570 1575
Leu Cys Ser Ala Glu Val Cys Gln Cys Ala Glu Gly Lys Cys Pro
1580 1585 1590
Arg Gln Arg Arg Ala Leu Glu Arg Gly Leu Gln Asp Glu Asp Gly
1595 1600 1605
Tyr Arg Met Lys Phe Ala Cys Tyr Tyr Pro Arg Val Glu Tyr Gly
1610 1615 1620
Phe Gln Val Lys Val Leu Arg Glu Asp Ser Arg Ala Ala Phe Arg
1625 1630 1635
Leu Phe Glu Thr Lys Ile Thr Gln Val Leu His Phe Thr Lys Asp
1640 1645 1650
Val Lys Ala Ala Ala Asn Gln Met Arg Asn Phe Leu Val Arg Ala
1655 1660 1665
Ser Cys Arg Leu Arg Leu Glu Pro Gly Lys Glu Tyr Leu Ile Met
1670 1675 1680
Gly Leu Asp Gly Ala Thr Tyr Asp Leu Glu Gly His Pro Gln Tyr
1685 1690 1695
Leu Leu Asp Ser Asn Ser Trp Ile Glu Glu Met Pro Ser Glu Arg
1700 1705 1710
Leu Cys Arg Ser Thr Arg Gln Arg Ala Ala Cys Ala Gln Leu Asn
1715 1720 1725
Asp Phe Leu Gln Glu Tyr Gly Thr Gln Gly Cys Gln Val
1730 1735 1740

<210> 280
<211> 2150
<212> PRT
<213> Homo sapiens
<400> 280
Met Tyr Pro Asn Trp Gly Arg Tyr Gly Gly Ser Ser His Tyr Pro Pro
1 5 10 15
Pro Pro Val Pro Pro Pro Pro Pro Val Ala Leu Pro Glu Ala Ser Pro
20 25 30
Gly Pro Gly Tyr Ser Ser Ser Thr Thr Pro Ala Ala Pro Ser Ser Ser
35 40 45
Gly Phe Met Ser Phe Arg Glu Gln His Leu Ala Gln Leu Gln Gln Leu
50 55 60
Gln Gln Met His Gln Lys Gln Met Gln Cys Val Leu Gln Pro His His
65 70 75 80
Leu Pro Pro Pro Pro Leu Pro Pro Pro Pro Val Met Pro Gly Gly Gly
85 90 95
Tyr Gly Asp Trp Gln Pro Pro Pro Pro Pro Met Pro Pro Pro Pro Gly
100 105 110
Pro Ala Leu Ser Tyr Gln Lys Gln Gln Gln Tyr Lys His Gln Met Leu
115 120 125
His His Gln Arg Asp Gly Pro Pro Gly Leu Val Pro Met Glu Leu Glu
130 135 140
Ser Pro Pro Glu Ser Pro Pro Val Pro Pro Gly Ser Tyr Met Pro Pro
145 150 155 160
Ser Gln Ser Tyr Met Pro Pro Pro Gln Pro Pro Pro Ser Tyr Tyr Pro
165 170 175
Pro Thr Ser Ser Gln Pro Tyr Leu Pro Pro Ala Gln Pro Ser Pro Ser
180 185 190
Gln Ser Pro Pro Ser Gln Ser Tyr Leu Ala Pro Thr Pro Ser Tyr Ser
195 200 205
Ser Ser Ser Ser Ser Ser Gln Ser Tyr Leu Ser His Ser Gln Ser Tyr
210 215 220
Leu Pro Ser Ser Gln Ala Ser Pro Ser Arg Pro Ser Gln Gly His Ser
225 230 235 240

Lys Ser Gln Leu Leu Ala Pro Pro Pro Pro Ser Ala Pro Pro Gly Asn
245 250 255
Lys Thr Thr Val Gln Gln Glu Pro Leu Glu Ser Gly Ala Lys Asn Lys
260 265 270
Ser Thr Glu Gln Gln Gln Ala Ala Pro Glu Pro Asp Pro Ser Thr Met
275 280 285
Thr Pro Gln Glu Gln Gln Gln Tyr Trp Tyr Arg Gln His Leu Leu Ser
290 295 300
Leu Gln Gln Arg Thr Lys Val His Leu Pro Gly His Lys Lys Gly Pro
305 310 315 320
Val Val Ala Lys Asp Thr Pro Glu Pro Val Lys Glu Glu Val Thr Val
325 330 335
Pro Ala Thr Ser Gln Val Pro Glu Ser Pro Ser Ser Glu Glu Pro Pro
340 345 350
Leu Pro Pro Pro Asn Glu Glu Val Pro Pro Pro Leu Pro Pro Glu Glu
355 360 365
Pro Gln Ser Glu Asp Pro Glu Glu Asp Ala Arg Leu Lys Gln Leu Gln
370 375 380
Ala Ala Ala Ala His Trp Gln Gln His Gln Gln His Arg Val Gly Phe
385 390 395 400
Gln Tyr Gln Gly Ile Met Gln Lys His Thr Gln Leu Gln Gln Ile Leu
405 410 415
Gln Gln Tyr Gln Gln Ile Ile Gln Pro Pro Pro His Ile Gln Thr Met
420 425 430
Ser Val Asp Met Gln Leu Arg His Tyr Glu Met Gln Gln Gln Gln Phe
435 440 445
Gln His Leu Tyr Gln Glu Trp Glu Arg Glu Phe Gln Leu Trp Glu Glu
450 455 460
Gln Leu His Ser Tyr Pro His Lys Asp Gln Leu Gln Glu Tyr Glu Lys
465 470 475 480
Gln Trp Lys Thr Trp Gln Gly His Met Lys Ala Thr Gln Ser Tyr Leu
485 490 495
Gln Glu Lys Val Asn Ser Phe Gln Asn Met Lys Asn Gln Tyr Met Gly
500 505 510
Asn Met Ser Met Pro Pro Pro Phe Val Pro Tyr Ser Gln Met Pro Pro
515 520 525
Pro Leu Pro Thr Met Pro Pro Pro Val Leu Pro Pro Ser Leu Pro Pro
530 535 540
Pro Val Met Pro Pro Ala Leu Pro Ala Thr Val Pro Pro Pro Gly Met
545 550 555 560
Pro Pro Pro Val Met Pro Pro Ser Leu Pro Thr Ser Val Pro Pro Pro
565 570 575
Gly Met Pro Pro Ser Leu Ser Ser Ala Gly Pro Pro Pro Val Leu Pro
580 585 590
Pro Pro Ser Leu Ser Ser Ala Gly Pro Pro Pro Val Leu Pro Pro Pro
595 600 605
Ser Leu Ser Ser Thr Ala Pro Pro Pro Val Met Pro Leu Pro Pro Leu
610 615 620
Ser Ser Ala Thr Pro Pro Pro Gly Ile Pro Pro Pro Gly Val Pro Gln
625 630 635 640
Gly Ile Pro Pro Gln Leu Thr Ala Ala Pro Val Pro Pro Ala Ser Ser
645 650 655
Ser Gln Ser Ser Gln Val Pro Glu Lys Pro Arg Pro Ala Leu Leu Pro
660 665 670
Thr Pro Val Ser Phe Gly Ser Ala Pro Pro Thr Thr Tyr His Pro Pro
675 680 685
Leu Gln Ser Ala Gly Pro Ser Glu Gln Val Asn Ser Lys Ala Pro Leu
690 695 700
Ser Lys Ser Ala Leu Pro Tyr Ser Ser Phe Ser Ser Asp Gln Gly Leu
705 710 715 720
Gly Glu Ser Ser Ala Ala Pro Ser Gln Pro Ile Thr Ala Val Lys Asp
725 730 735
Met Pro Val Arg Ser Gly Gly Leu Leu Pro Asp Pro Pro Arg Ser Ser
740 745 750
Tyr Leu Glu Ser Pro Arg Gly Pro Arg Phe Asp Gly Pro Arg Arg Phe
755 760 765
Glu Asp Leu Gly Ser Arg Cys Glu Gly Pro Arg Pro Lys Gly Pro Arg
770 775 780
Phe Glu Gly Asn Arg Pro Asp Gly Pro Arg Pro Arg Tyr Glu Gly His

785 790 795 800
Pro Ala Glu Gly Thr Lys Ser Lys Trp Gly Met Ile Pro Arg Gly Pro
805 810 815
Ala Ser Gln Phe Tyr Ile Thr Pro Ser Thr Ser Leu Ser Pro Arg Gln
820 825 830
Ser Gly Pro Gln Trp Lys Gly Pro Lys Pro Ala Phe Gly Gln Gln His
835 840 845
Gln Gln Gln Pro Lys Ser Gln Ala Glu Pro Leu Ser Gly Asn Lys Glu
850 855 860
Pro Leu Ala Asp Thr Ser Ser Asn Gln Gln Lys Asn Phe Lys Met Gln
865 870 875 880
Ser Ala Ala Phe Ser Ile Ala Ala Asp Val Lys Asp Val Lys Ala Ala
885 890 895
Gln Ser Asn Glu Asn Leu Ser Asp Ser Gln Gln Glu Pro Pro Lys Ser
900 905 910
Glu Val Ser Glu Gly Pro Val Glu Pro Ser Asn Trp Asp Gln Asn Val
915 920 925
Gln Ser Met Glu Thr Gln Ile Asp Lys Ala Gln Ala Val Thr Gln Pro
930 935 940
Val Pro Leu Ala Asn Lys Pro Val Pro Ala Gln Ser Thr Phe Pro Ser
945 950 955 960
Lys Thr Gly Gly Met Glu Gly Gly Thr Ala Val Ala Thr Ser Ser Leu
965 970 975
Thr Ala Asp Asn Asp Phe Lys Pro Val Gly Ile Gly Leu Pro His Ser
980 985 990
Glu Asn Asn Gln Asp Lys Gly Leu Pro Arg Pro Asp Asn Arg Asp Asn
995 1000 1005
Arg Leu Glu Gly Asn Arg Gly Asn Ser Ser Ser Tyr Arg Gly Pro
1010 1015 1020
Gly Gln Ser Arg Met Glu Asp Thr Arg Asp Lys Gly Leu Val Asn
1025 1030 1035
Arg Gly Arg Gly Gln Ala Ile Ser Arg Gly Pro Gly Leu Val Lys
1040 1045 1050
Gln Glu Asp Phe Arg Asp Lys Met Met Gly Arg Arg Glu Asp Ser
1055 1060 1065
Arg Glu Lys Met Asn Arg Gly Glu Gly Ser Arg Asp Arg Gly Leu
1070 1075 1080
Val Arg Pro Gly Ser Ser Arg Glu Lys Val Pro Gly Gly Leu Gln
1085 1090 1095
Gly Ser Gln Asp Arg Gly Ala Ala Gly Ser Arg Glu Arg Gly Pro
1100 1105 1110
Pro Arg Arg Ala Gly Ser Gln Glu Arg Gly Pro Leu Arg Arg Ala
1115 1120 1125
Gly Ser Arg Glu Arg Ile Pro Pro Arg Arg Ala Gly Ser Arg Glu
1130 1135 1140
Arg Gly Pro Pro Arg Gly Pro Gly Ser Arg Glu Arg Gly Leu Gly
1145 1150 1155
Arg Ser Asp Phe Gly Arg Asp Arg Gly Pro Phe Arg Pro Glu Pro
1160 1165 1170
Gly Asp Gly Gly Glu Lys Met Tyr Pro Tyr His Arg Asp Glu Pro
1175 1180 1185
Pro Arg Ala Pro Trp Asn His Gly Glu Glu Arg Gly His Glu Glu
1190 1195 1200
Phe Pro Leu Asp Gly Arg Asn Ala Pro Met Glu Arg Glu Arg Leu
1205 1210 1215
Asp Asp Trp Asp Arg Glu Arg Tyr Trp Arg Glu Cys Glu Arg Asp
1220 1225 1230
Tyr Gln Asp Asp Thr Leu Glu Leu Tyr Asn Arg Glu Asp Arg Phe
1235 1240 1245
Ser Ala Pro Pro Ser Arg Ser His Asp Gly Asp Arg Arg Gly Pro
1250 1255 1260
Trp Trp Asp Asp Trp Glu Arg Asp Gln Asp Met Asp Glu Asp Tyr
1265 1270 1275
Asn Arg Glu Met Glu Arg Asp Met Asp Arg Asp Val Asp Arg Ile
1280 1285 1290
Ser Arg Pro Met Asp Met Tyr Asp Arg Ser Leu Asp Asn Glu Trp
1295 1300 1305
Asp Arg Asp Tyr Gly Arg Pro Leu Asp Glu Gln Glu Ser Gln Phe
1310 1315 1320

Arg Glu Arg Asp Ile Pro Ser Leu Pro Pro Leu Pro Pro Leu Pro
1325 1330 1335
Pro Leu Pro Pro Leu Asp Arg Tyr Arg Asp Asp Arg Trp Arg Glu
1340 1345 1350
Glu Arg Asn Arg Glu His Gly Tyr Asp Arg Asp Phe Arg Asp Arg
1355 1360 1365
Gly Glu Leu Arg Ile Arg Glu Tyr Pro Glu Arg Gly Asp Thr Trp
1370 1375 1380
Arg Glu Lys Arg Asp Tyr Val Pro Asp Arg Met Asp Trp Glu Arg
1385 1390 1395
Glu Arg Leu Ser Asp Arg Trp Tyr Pro Ser Asp Val Asp Arg His
1400 1405 1410
Ser Pro Met Ala Glu His Met Pro Ser Ser His His Ser Ser Glu
1415 1420 1425
Met Met Gly Ser Asp Ala Ser Leu Asp Ser Asp Gln Gly Leu Gly
1430 1435 1440
Gly Val Met Val Leu Ser Gln Arg Gln His Glu Ile Ile Leu Lys
1445 1450 1455
Ala Ala Gln Glu Leu Lys Met Leu Arg Glu Gln Lys Glu Gln Leu
1460 1465 1470
Gln Lys Met Lys Asp Phe Gly Ser Glu Pro Gln Met Ala Asp His
1475 1480 1485
Leu Pro Pro Gln Glu Ser Arg Leu Gln Asn Thr Ser Ser Arg Pro
1490 1495 1500
Gly Met Tyr Pro Pro Pro Gly Ser Tyr Arg Pro Pro Pro Pro Met
1505 1510 1515
Gly Lys Pro Pro Gly Ser Ile Val Arg Pro Ser Ala Pro Pro Ala
1520 1525 1530
Arg Ser Ser Val Pro Val Thr Arg Pro Pro Val Pro Ile Pro Pro
1535 1540 1545
Pro Pro Pro Pro Pro Pro Leu Pro Pro Pro Pro Pro Val Ile Lys
1550 1555 1560
Pro Gln Thr Ser Ala Val Glu Gln Glu Arg Trp Asp Glu Asp Ser
1565 1570 1575
Phe Tyr Gly Leu Trp Asp Thr Asn Asp Glu Gln Gly Leu Asn Ser
1580 1585 1590
Glu Phe Lys Ser Glu Thr Ala Ala Ile Pro Ser Ala Pro Val Leu
1595 1600 1605
Pro Pro Pro Pro Val His Ser Ser Ile Pro Pro Pro Gly Pro Val
1610 1615 1620
Pro Met Gly Met Pro Pro Met Ser Lys Pro Pro Pro Val Gln Gln
1625 1630 1635
Thr Val Asp Tyr Gly His Gly Arg Asp Ile Ser Thr Asn Lys Val
1640 1645 1650
Glu Gln Ile Pro Tyr Gly Glu Arg Ile Thr Leu Arg Pro Asp Pro
1655 1660 1665
Leu Pro Glu Arg Ser Thr Phe Glu Thr Glu His Ala Gly Gln Arg
1670 1675 1680
Asp Arg Tyr Asp Arg Glu Arg Asp Arg Glu Pro Tyr Phe Asp Arg
1685 1690 1695
Gln Ser Asn Val Ile Ala Asp His Arg Asp Phe Lys Arg Asp Arg
1700 1705 1710
Glu Thr His Arg Asp Arg Asp Arg Asp Arg Gly Val Ile Asp Tyr
1715 1720 1725
Asp Arg Asp Arg Phe Asp Arg Glu Arg Arg Pro Arg Asp Asp Arg
1730 1735 1740
Ala Gln Ser Tyr Arg Asp Lys Lys Asp His Ser Ser Ser Arg Arg
1745 1750 1755
Gly Gly Phe Asp Arg Pro Ser Tyr Asp Arg Lys Ser Asp Arg Pro
1760 1765 1770
Val Tyr Glu Gly Pro Ser Met Phe Gly Gly Glu Arg Arg Thr Tyr
1775 1780 1785
Pro Glu Glu Arg Met Pro Leu Pro Ala Pro Ser Leu Ser His Gln
1790 1795 1800
Pro Pro Pro Ala Pro Arg Val Glu Lys Lys Pro Glu Ser Lys Asn
1805 1810 1815
Val Asp Asp Ile Leu Lys Pro Pro Gly Arg Glu Ser Arg Pro Glu
1820 1825 1830
Arg Ile Val Val Ile Met Arg Gly Leu Pro Gly Ser Gly Lys Thr

```
    1835                1840                1845
His Val  Ala Lys Leu Ile Arg  Asp Lys Glu Val Glu  Phe Gly Gly
    1850                1855                1860
Pro Ala  Pro Arg Val Leu Ser  Leu Asp Asp Tyr Phe  Ile Thr Glu
    1865                1870                1875
Val Glu  Lys Glu Glu Lys Asp  Pro Asp Ser Gly Lys  Lys Val Lys
    1880                1885                1890
Lys Lys  Val Met Glu Tyr Glu  Tyr Glu Ala Glu Met  Glu Glu Thr
    1895                1900                1905
Tyr Arg  Thr Ser Met Phe Lys  Thr Phe Lys Lys Thr  Leu Asp Asp
    1910                1915                1920
Gly Phe  Phe Pro Phe Ile Ile  Leu Asp Ala Ile Asn  Asp Arg Val
    1925                1930                1935
Arg His  Phe Asp Gln Phe Trp  Ser Ala Ala Lys Thr  Lys Gly Phe
    1940                1945                1950
Glu Val  Tyr Leu Ala Glu Met  Ser Ala Asp Asn Gln  Thr Cys Gly
    1955                1960                1965
Lys Arg  Asn Ile His Gly Arg  Lys Leu Lys Glu Ile  Asn Lys Met
    1970                1975                1980
Ala Asp  His Trp Glu Thr Ala  Pro Arg His Met Met  Arg Leu Asp
    1985                1990                1995
Ile Arg  Ser Leu Leu Gln Asp  Ala Ala Ile Glu Glu  Val Glu Met
    2000                2005                2010
Glu Asp  Phe Asp Ala Asn Ile  Glu Glu Gln Lys Glu  Glu Lys Lys
    2015                2020                2025
Asp Ala  Glu Glu Glu Glu Ser  Glu Leu Gly Tyr Ile  Pro Lys Ser
    2030                2035                2040
Lys Trp  Glu Met Asp Thr Ser  Glu Ala Lys Leu Asp  Lys Leu Asp
    2045                2050                2055
Gly Leu  Arg Thr Gly Thr Lys  Arg Lys Arg Asp Trp  Glu Ala Ile
    2060                2065                2070
Ala Ser  Arg Met Glu Asp Tyr  Leu Gln Leu Pro Asp  Asp Tyr Asp
    2075                2080                2085
Thr Arg  Ala Ser Glu Ala Lys  Ala Ser Arg Ser Phe  Phe Val Cys
    2090                2095                2100
His Leu  Ala Ser Thr Phe Gln  Phe Leu Phe Cys Phe  Tyr Cys Phe
    2105                2110                2115
Ser Phe  Phe Asp Ala Glu Glu  Glu Glu Ser Glu Leu  Val Gly Asp
    2120                2125                2130
Arg Pro  Thr Thr Leu Asn Ser  Val Ser Leu Leu Lys  Phe Leu Lys
    2135                2140                2145
Lys Val
    2150
```

<210> 281
<211> 979
<212> PRT
<213> Homo sapiens
<400> 281

```
Met Ser Val Leu Gly Glu Tyr Glu Arg His Cys Asp Ser Ile Asn Ser
1               5                   10                  15
Asp Phe Gly Ser Glu Ser Gly Gly Cys Gly Asp Ser Ser Pro Gly Pro
            20                  25                  30
Ser Ala Ser Gln Gly Pro Arg Ala Gly Gly Gly Ala Ala Glu Gln Glu
        35                  40                  45
Glu Leu His Tyr Ile Pro Ile Arg Val Leu Gly Arg Gly Ala Phe Gly
    50                  55                  60
Glu Ala Thr Leu Tyr Arg Arg Thr Glu Asp Asp Ser Leu Val Val Trp
65                  70                  75                  80
Lys Glu Val Asp Leu Thr Arg Leu Ser Glu Lys Glu Arg Arg Asp Ala
                85                  90                  95
Leu Asn Glu Ile Val Ile Leu Ala Leu Leu Gln His Asp Asn Ile Ile
            100                 105                 110
Ala Tyr Tyr Asn His Phe Met Asp Asn Thr Thr Leu Leu Ile Glu Leu
        115                 120                 125
Glu Tyr Cys Asn Gly Gly Asn Leu Tyr Asp Lys Ile Leu Arg Gln Lys
    130                 135                 140
Asp Lys Leu Phe Glu Glu Glu Met Val Val Trp Tyr Leu Phe Gln Ile
145                 150                 155                 160
```

```
Val Ser Ala Val Ser Cys Ile His Lys Ala Gly Ile Leu His Arg Asp
                165                 170                 175
Ile Lys Thr Leu Asn Ile Phe Leu Thr Lys Ala Asn Leu Ile Lys Leu
            180                 185                 190
Gly Asp Tyr Gly Leu Ala Lys Lys Leu Asn Ser Glu Tyr Ser Met Ala
        195                 200                 205
Glu Thr Leu Val Gly Thr Pro Tyr Tyr Met Ser Pro Glu Leu Cys Gln
    210                 215                 220
Gly Val Lys Tyr Asn Phe Lys Ser Asp Ile Trp Ala Val Gly Cys Val
225                 230                 235                 240
Ile Phe Glu Leu Leu Thr Leu Lys Arg Thr Phe Asp Ala Thr Asn Pro
                245                 250                 255
Leu Asn Leu Cys Val Lys Ile Val Gln Gly Ile Arg Ala Met Glu Val
            260                 265                 270
Asp Ser Ser Gln Tyr Ser Leu Glu Leu Ile Gln Met Val His Ser Cys
        275                 280                 285
Leu Asp Gln Asp Pro Glu Gln Arg Pro Thr Ala Asp Glu Leu Leu Asp
    290                 295                 300
Arg Pro Leu Leu Arg Lys Arg Arg Arg Glu Met Glu Glu Lys Val Thr
305                 310                 315                 320
Leu Leu Asn Ala Pro Thr Lys Arg Pro Arg Ser Ser Thr Val Thr Glu
                325                 330                 335
Ala Pro Ile Ala Val Val Thr Ser Arg Thr Ser Glu Val Tyr Ile Trp
            340                 345                 350
Gly Gly Gly Lys Ser Thr Pro Gln Lys Leu Asp Val Ile Lys Ser Gly
        355                 360                 365
Cys Ser Ala Arg Gln Val Cys Ala Gly Asn Thr His Phe Ala Val Val
    370                 375                 380
Thr Val Glu Lys Glu Leu Tyr Thr Trp Val Asn Met Gln Gly Gly Thr
385                 390                 395                 400
Lys Leu His Gly Gln Leu Gly His Gly Asp Lys Ala Ser Tyr Arg Gln
                405                 410                 415
Pro Lys His Val Glu Lys Leu Gln Gly Lys Ala Ile His Gln Val Ser
            420                 425                 430
Cys Gly Asp Asp Phe Thr Val Cys Val Thr Asp Glu Gly Gln Leu Tyr
        435                 440                 445
Ala Phe Gly Ser Asp Tyr Tyr Gly Cys Met Gly Val Asp Lys Val Ala
    450                 455                 460
Gly Pro Glu Val Leu Glu Pro Met Gln Leu Asn Phe Phe Leu Ser Asn
465                 470                 475                 480
Pro Val Glu Gln Val Ser Cys Gly Asp Asn His Val Val Val Leu Thr
                485                 490                 495
Arg Asn Lys Glu Val Tyr Ser Trp Gly Cys Gly Glu Tyr Gly Arg Leu
            500                 505                 510
Gly Leu Asp Ser Glu Glu Asp Tyr Tyr Thr Pro Gln Lys Val Asp Val
        515                 520                 525
Pro Lys Ala Leu Ile Ile Val Ala Val Gln Cys Gly Cys Asp Gly Thr
    530                 535                 540
Phe Leu Leu Thr Gln Ser Gly Lys Val Leu Ala Cys Gly Leu Asn Glu
545                 550                 555                 560
Phe Asn Lys Leu Gly Leu Asn Gln Cys Met Ser Gly Ile Ile Asn His
                565                 570                 575
Glu Ala Tyr His Glu Val Pro Tyr Thr Thr Ser Phe Thr Leu Ala Lys
            580                 585                 590
Gln Leu Ser Phe Tyr Lys Ile Arg Thr Ile Ala Pro Gly Lys Thr His
        595                 600                 605
Thr Ala Ala Ile Asp Glu Arg Gly Arg Leu Leu Thr Phe Gly Cys Asn
    610                 615                 620
Lys Cys Gly Gln Leu Gly Val Gly Asn Tyr Lys Lys Arg Leu Gly Ile
625                 630                 635                 640
Asn Leu Leu Gly Gly Pro Leu Gly Gly Lys Gln Val Ile Arg Val Ser
                645                 650                 655
Cys Gly Asp Glu Phe Thr Ile Ala Ala Thr Asp Asp Asn His Ile Phe
            660                 665                 670
Ala Trp Gly Asn Gly Gly Asn Gly Arg Leu Ala Met Thr Pro Thr Glu
        675                 680                 685
Arg Pro His Gly Ser Asp Ile Cys Thr Ser Trp Pro Arg Pro Ile Phe
    690                 695                 700
Gly Ser Leu His His Val Pro Asp Leu Ser Cys Arg Gly Trp His Thr
```

705 710 715 720
Ile Leu Ile Val Glu Lys Val Leu Asn Ser Lys Thr Ile Arg Ser Asn
725 730 735
Ser Ser Gly Leu Ser Ile Gly Thr Val Phe Gln Ser Ser Ser Pro Gly
740 745 750
Gly Gly Gly Gly Gly Gly Gly Gly Glu Glu Glu Asp Ser Gln Gln Glu
755 760 765
Ser Glu Thr Pro Asp Pro Ser Gly Gly Phe Arg Gly Thr Met Glu Ala
770 775 780
Asp Arg Gly Met Glu Gly Leu Ile Ser Pro Thr Glu Ala Met Gly Asn
785 790 795 800
Ser Asn Gly Ala Ser Ser Ser Cys Pro Gly Trp Leu Arg Lys Glu Leu
805 810 815
Glu Asn Ala Glu Phe Ile Pro Met Pro Asp Ser Pro Ser Pro Leu Ser
820 825 830
Ala Ala Phe Ser Glu Ser Glu Lys Asp Thr Leu Pro Tyr Glu Glu Leu
835 840 845
Gln Gly Leu Lys Val Ala Ser Glu Ala Pro Leu Glu His Lys Pro Gln
850 855 860
Val Glu Ala Ser Ser Pro Arg Leu Asn Pro Ala Val Thr Cys Ala Gly
865 870 875 880
Lys Gly Thr Pro Leu Thr Pro Pro Ala Cys Ala Cys Ser Ser Leu Gln
885 890 895
Val Glu Val Glu Arg Leu Gln Gly Leu Val Leu Lys Cys Leu Ala Glu
900 905 910
Gln Gln Lys Leu Gln Gln Glu Asn Leu Gln Ile Phe Thr Gln Leu Gln
915 920 925
Lys Leu Asn Lys Lys Leu Glu Gly Gly Gln Gln Val Gly Met His Ser
930 935 940
Lys Gly Thr Gln Thr Ala Lys Glu Glu Met Glu Met Asp Pro Lys Pro
945 950 955 960
Asp Leu Asp Ser Asp Ser Gly Cys Leu Leu Gly Thr Asp Ser Cys Arg
965 970 975
Pro Ser Leu

<210> 282
<211> 107
<212> PRT
<213> Homo sapiens
<400> 282
Met Arg Phe Phe Leu Phe Phe Phe Phe Phe Leu Arg Trp Ser Leu Val
1 5 10 15
Ser Pro Arg Leu Glu Cys Ser Gly Leu Val Leu Ala His Cys Asn Leu
20 25 30
His Leu Leu Gly Ser Arg Glu Ser Pro Ala Ser Ala Ser Arg Val Ala
35 40 45
Gly Ile Thr Gly Val Ser His His Thr Gln Thr His Leu Met Ser Phe
50 55 60
Asp Val Lys Ala Cys Asn Ser Asn Pro Tyr Val Trp Asn Ile Cys Ile
65 70 75 80
Thr Ser Glu Phe Phe His Leu Val Asn Ser Cys Thr Ser Leu Ser Cys
85 90 95
Pro Phe Phe Pro Pro Leu Ala Val Ser Tyr Cys
100 105

<210> 283
<211> 533
<212> PRT
<213> Homo sapiens
<400> 283
Met Thr Lys Lys Arg Ile Ala Val Ile Gly Gly Gly Val Ser Gly Leu
1 5 10 15
Ser Ser Ile Lys Cys Cys Val Glu Glu Gly Leu Glu Pro Val Cys Phe
20 25 30
Glu Arg Thr Asp Asp Ile Gly Gly Leu Trp Arg Phe Gln Glu Asn Pro
35 40 45
Glu Glu Gly Arg Ala Ser Ile Tyr Lys Ser Val Ile Ile Asn Thr Ser
50 55 60

Lys Glu Met Met Cys Phe Ser Asp Tyr Pro Ile Pro Asp His Tyr Pro
65 70 75 80
Asn Phe Met His Asn Ala Gln Val Leu Glu Tyr Phe Arg Met Tyr Ala
85 90 95
Lys Glu Phe Asp Leu Leu Lys Tyr Ile Arg Phe Lys Thr Thr Val Cys
100 105 110
Ser Val Lys Lys Gln Pro Asp Phe Ala Thr Ser Gly Gln Trp Glu Val
115 120 125
Val Thr Glu Ser Glu Gly Lys Lys Glu Met Asn Val Phe Asp Gly Val
130 135 140
Met Val Cys Thr Gly His His Thr Asn Ala His Leu Pro Leu Glu Ser
145 150 155 160
Phe Pro Gly Ile Glu Lys Phe Lys Gly Gln Tyr Phe His Ser Arg Asp
165 170 175
Tyr Lys Asn Pro Glu Gly Phe Thr Gly Lys Arg Val Ile Ile Ile Gly
180 185 190
Ile Gly Asn Ser Gly Gly Asp Leu Ala Val Glu Ile Ser Gln Thr Ala
195 200 205
Lys Gln Val Phe Leu Ser Thr Arg Arg Gly Ala Trp Ile Leu Asn Arg
210 215 220
Val Gly Asp Tyr Gly Tyr Pro Ala Asp Val Leu Phe Ser Ser Arg Leu
225 230 235 240
Thr His Phe Ile Trp Lys Ile Cys Gly Gln Ser Leu Ala Asn Lys Tyr
245 250 255
Leu Glu Lys Lys Ile Asn Gln Arg Phe Asp His Glu Met Phe Gly Leu
260 265 270
Lys Pro Lys His Arg Ala Leu Ser Gln His Pro Thr Leu Asn Asp Asp
275 280 285
Leu Pro Asn Arg Ile Ile Ser Gly Leu Val Lys Val Lys Gly Asn Val
290 295 300
Lys Glu Phe Thr Glu Thr Ala Ala Ile Phe Glu Asp Gly Ser Arg Glu
305 310 315 320
Asp Asp Ile Asp Ala Val Ile Phe Ala Thr Gly Tyr Ser Phe Asp Phe
325 330 335
Pro Phe Leu Glu Asp Ser Val Lys Val Val Lys Asn Lys Ile Pro Leu
340 345 350
Tyr Lys Lys Val Phe Pro Pro Asn Leu Glu Arg Pro Thr Leu Ala Ile
355 360 365
Ile Gly Leu Ile Gln Pro Leu Gly Ala Ile Met Pro Ile Ser Glu Leu
370 375 380
Gln Gly Arg Trp Ala Thr Gln Val Phe Lys Gly Leu Lys Thr Leu Pro
385 390 395 400
Ser Gln Ser Glu Met Met Ala Glu Ile Ser Lys Ala Gln Glu Glu Ile
405 410 415
Asp Lys Arg Tyr Val Glu Ser Gln Arg His Thr Ile Gln Gly Asp Tyr
420 425 430
Ile Asp Thr Met Glu Glu Leu Ala Asp Leu Val Gly Val Arg Pro Asn
435 440 445
Leu Leu Ser Leu Ala Phe Thr Asp Pro Lys Leu Ala Leu His Leu Leu
450 455 460
Leu Gly Pro Cys Thr Pro Ile His Tyr Arg Val Gln Gly Pro Gly Lys
465 470 475 480
Trp Asp Gly Ala Arg Lys Ala Ile Leu Thr Thr Asp Asp Arg Ile Arg
485 490 495
Lys Pro Leu Met Thr Arg Val Val Glu Arg Ser Ser Ser Met Thr Ser
500 505 510
Thr Met Thr Ile Gly Lys Phe Met Leu Ala Leu Ala Phe Phe Ala Ile
515 520 525
Ile Ile Ala Tyr Phe
530

<210> 284
<211> 757
<212> PRT
<213> Homo sapiens
<400> 284
Met Val Pro Asp Thr Ala Cys Val Leu Leu Leu Thr Leu Ala Ala Leu
1 5 10 15
Gly Ala Ser Gly Gln Gly Gln Ser Pro Leu Gly Ser Asp Leu Gly Pro

```
            20                  25                  30
Gln Met Leu Arg Glu Leu Gln Glu Thr Asn Ala Ala Leu Gln Asp Val
        35                  40                  45
Arg Asp Trp Leu Arg Gln Gln Val Arg Glu Ile Thr Phe Leu Lys Asn
    50                  55                  60
Thr Val Met Glu Cys Asp Ala Cys Gly Met Gln Gln Ser Val Arg Thr
65                  70                  75                  80
Gly Leu Pro Ser Val Arg Pro Leu Leu His Cys Ala Pro Gly Phe Cys
                85                  90                  95
Phe Pro Gly Val Ala Cys Ile Gln Thr Glu Ser Gly Gly Arg Cys Gly
            100                 105                 110
Pro Cys Pro Ala Gly Phe Thr Gly Asn Gly Ser His Cys Thr Asp Val
        115                 120                 125
Asn Glu Cys Asn Ala His Pro Cys Phe Pro Arg Val Arg Cys Ile Asn
    130                 135                 140
Thr Ser Pro Gly Phe Arg Cys Glu Ala Cys Pro Pro Gly Tyr Ser Gly
145                 150                 155                 160
Pro Thr His Gln Gly Val Gly Leu Ala Phe Ala Lys Ala Asn Lys Gln
                165                 170                 175
Val Cys Thr Asp Ile Asn Glu Cys Glu Thr Gly Gln His Asn Cys Val
            180                 185                 190
Pro Asn Ser Val Cys Ile Asn Thr Arg Gly Ser Phe Gln Cys Gly Pro
        195                 200                 205
Cys Gln Pro Gly Phe Val Gly Asp Gln Ala Ser Gly Cys Gln Arg Gly
    210                 215                 220
Ala Gln Arg Phe Cys Pro Asp Gly Ser Pro Ser Glu Cys His Glu His
225                 230                 235                 240
Ala Asp Cys Val Leu Glu Arg Asp Gly Ser Arg Ser Cys Val Cys Arg
                245                 250                 255
Val Gly Trp Ala Gly Asn Gly Ile Leu Cys Gly Arg Asp Thr Asp Leu
            260                 265                 270
Asp Gly Phe Pro Asp Glu Lys Leu Arg Cys Pro Glu Pro Gln Cys Arg
        275                 280                 285
Lys Asp Asn Cys Val Thr Val Pro Asn Ser Gly Gln Glu Asp Val Asp
    290                 295                 300
Arg Asp Gly Ile Gly Asp Ala Cys Asp Pro Asp Ala Asp Gly Asp Gly
305                 310                 315                 320
Val Pro Asn Glu Lys Asp Asn Cys Pro Leu Val Arg Asn Pro Asp Gln
                325                 330                 335
Arg Asn Thr Asp Glu Asp Lys Trp Gly Asp Ala Cys Asp Asn Cys Arg
            340                 345                 350
Ser Gln Lys Asn Asp Asp Gln Lys Asp Thr Asp Gln Asp Gly Arg Gly
        355                 360                 365
Asp Ala Cys Asp Asp Asp Ile Asp Gly Asp Arg Ile Arg Asn Gln Ala
    370                 375                 380
Asp Asn Cys Pro Arg Val Pro Asn Ser Asp Gln Lys Asp Ser Asp Gly
385                 390                 395                 400
Asp Gly Ile Gly Asp Ala Cys Asp Asn Cys Pro Gln Lys Ser Asn Pro
                405                 410                 415
Asp Gln Ala Asp Val Asp His Asp Phe Val Gly Asp Ala Cys Asp Ser
            420                 425                 430
Asp Gln Asp Gln Asp Gly Asp Gly His Gln Asp Ser Arg Asp Asn Cys
        435                 440                 445
Pro Thr Val Pro Asn Ser Ala Gln Glu Asp Ser Asp His Asp Gly Gln
    450                 455                 460
Gly Asp Ala Cys Asp Asp Asp Asp Asp Asn Asp Gly Val Pro Asp Ser
465                 470                 475                 480
Arg Asp Asn Cys Arg Leu Val Pro Asn Pro Gly Gln Glu Asp Ala Asp
                485                 490                 495
Arg Asp Gly Val Gly Asp Val Cys Gln Asp Asp Phe Asp Ala Asp Lys
            500                 505                 510
Val Val Asp Lys Ile Asp Val Cys Pro Glu Asn Ala Glu Val Thr Leu
        515                 520                 525
Thr Asp Phe Arg Ala Phe Gln Thr Val Val Leu Asp Pro Glu Gly Asp
    530                 535                 540
Ala Gln Ile Asp Pro Asn Trp Val Val Leu Asn Gln Gly Arg Glu Ile
545                 550                 555                 560
Val Gln Thr Met Asn Ser Asp Pro Gly Leu Ala Val Gly Tyr Thr Ala
                565                 570                 575
```

Phe Asn Gly Val Asp Phe Glu Gly Thr Phe His Val Asn Thr Val Thr
580 585 590
Asp Asp Asp Tyr Ala Gly Phe Ile Phe Gly Tyr Gln Asp Ser Ser Ser
595 600 605
Phe Tyr Val Val Met Trp Lys Gln Met Glu Gln Thr Tyr Trp Gln Ala
610 615 620
Asn Pro Phe Arg Ala Val Ala Glu Pro Gly Ile Gln Leu Lys Ala Val
625 630 635 640
Lys Ser Ser Thr Gly Pro Gly Glu Gln Leu Arg Asn Ala Leu Trp His
645 650 655
Thr Gly Asp Thr Glu Ser Gln Val Arg Leu Leu Trp Lys Asp Pro Arg
660 665 670
Asn Val Gly Trp Lys Asp Lys Lys Ser Tyr Arg Trp Phe Leu Gln His
675 680 685
Arg Pro Gln Val Gly Tyr Ile Arg Val Arg Phe Tyr Glu Gly Pro Glu
690 695 700
Leu Val Ala Asp Ser Asn Val Val Leu Asp Thr Thr Met Arg Gly Gly
705 710 715 720
Arg Leu Gly Val Phe Cys Phe Ser Gln Glu Asn Ile Ile Trp Ala Asn
725 730 735
Leu Arg Tyr Arg Cys Asn Asp Thr Ile Pro Glu Asp Tyr Glu Thr His
740 745 750
Gln Leu Arg Gln Ala
755

<210> 285
<211> 3396
<212> PRT
<213> Homo sapiens
<400> 285
Met Phe Ile Asn Ile Lys Ser Ile Leu Trp Met Cys Ser Thr Leu Ile
1 5 10 15
Val Thr His Ala Leu His Lys Val Lys Val Gly Lys Ser Pro Pro Val
20 25 30
Arg Gly Ser Leu Ser Gly Lys Val Ser Leu Pro Cys His Phe Ser Thr
35 40 45
Met Pro Thr Leu Pro Pro Ser Tyr Asn Thr Ser Glu Phe Leu Arg Ile
50 55 60
Lys Trp Ser Lys Ile Glu Val Asp Lys Asn Gly Lys Asp Leu Lys Glu
65 70 75 80
Thr Thr Val Leu Val Ala Gln Asn Gly Asn Ile Lys Ile Gly Gln Asp
85 90 95
Tyr Lys Gly Arg Val Ser Val Pro Thr His Pro Glu Ala Val Gly Asp
100 105 110
Ala Ser Leu Thr Val Val Lys Leu Leu Ala Ser Asp Ala Gly Leu Tyr
115 120 125
Arg Cys Asp Val Met Tyr Gly Ile Glu Asp Thr Gln Asp Thr Val Ser
130 135 140
Leu Thr Val Asp Gly Val Val Phe His Tyr Arg Ala Ala Thr Ser Arg
145 150 155 160
Tyr Thr Leu Asn Phe Glu Ala Ala Gln Lys Ala Cys Leu Asp Val Gly
165 170 175
Ala Val Ile Ala Thr Pro Glu Gln Leu Phe Ala Ala Tyr Glu Asp Gly
180 185 190
Phe Glu Gln Cys Asp Ala Gly Trp Leu Ala Asp Gln Thr Val Arg Tyr
195 200 205
Pro Ile Arg Ala Pro Arg Val Gly Cys Tyr Gly Asp Lys Met Gly Lys
210 215 220
Ala Gly Val Arg Thr Tyr Gly Phe Arg Ser Pro Gln Glu Thr Tyr Asp
225 230 235 240
Val Tyr Cys Tyr Val Asp His Leu Asp Gly Asp Val Phe His Leu Thr
245 250 255
Val Pro Ser Lys Phe Thr Phe Glu Glu Ala Ala Lys Glu Cys Glu Asn
260 265 270
Gln Asp Ala Arg Leu Ala Thr Val Gly Glu Leu Gln Ala Ala Trp Arg
275 280 285
Asn Gly Phe Asp Gln Cys Asp Tyr Gly Trp Leu Ser Asp Ala Ser Val
290 295 300
Arg His Pro Val Thr Val Ala Arg Ala Gln Cys Gly Gly Gly Leu Leu

```
305                 310                 315                 320
Gly Val Arg Thr Leu Tyr Arg Phe Glu Asn Gln Thr Gly Phe Pro Pro
                325                 330                 335
Pro Asp Ser Arg Phe Asp Ala Tyr Cys Phe Lys Pro Lys Glu Ala Thr
            340                 345                 350
Thr Ile Asp Leu Ser Ile Leu Ala Glu Thr Ala Ser Pro Ser Leu Ser
        355                 360                 365
Lys Glu Pro Gln Met Val Ser Asp Arg Thr Thr Pro Ile Ile Pro Leu
    370                 375                 380
Val Asp Glu Leu Pro Val Ile Pro Thr Glu Phe Pro Pro Val Gly Asn
385                 390                 395                 400
Ile Val Ser Phe Glu Gln Lys Ala Thr Val Gln Pro Gln Ala Ile Thr
                405                 410                 415
Asp Ser Leu Ala Thr Lys Leu Pro Thr Pro Thr Gly Ser Thr Lys Lys
            420                 425                 430
Pro Trp Asp Met Asp Asp Tyr Ser Pro Ser Ala Ser Gly Pro Leu Gly
        435                 440                 445
Lys Leu Asp Ile Ser Glu Ile Lys Glu Glu Val Leu Gln Ser Thr Thr
    450                 455                 460
Gly Val Ser His Tyr Ala Thr Asp Ser Trp Asp Gly Val Val Glu Asp
465                 470                 475                 480
Lys Gln Thr Gln Glu Ser Val Thr Gln Ile Glu Gln Ile Glu Val Gly
                485                 490                 495
Pro Leu Val Thr Ser Met Glu Ile Leu Lys His Ile Pro Ser Lys Glu
            500                 505                 510
Phe Pro Val Thr Glu Thr Pro Leu Val Thr Ala Arg Met Ile Leu Glu
        515                 520                 525
Ser Lys Thr Glu Lys Lys Met Val Ser Thr Val Ser Glu Leu Val Thr
    530                 535                 540
Thr Gly His Tyr Gly Phe Thr Leu Gly Glu Glu Asp Asp Glu Asp Arg
545                 550                 555                 560
Thr Leu Thr Val Gly Ser Asp Glu Ser Thr Leu Ile Phe Asp Gln Ile
                565                 570                 575
Pro Glu Val Ile Thr Val Ser Lys Thr Ser Glu Asp Thr Ile His Thr
            580                 585                 590
His Leu Glu Asp Leu Glu Ser Val Ser Ala Ser Thr Thr Val Ser Pro
        595                 600                 605
Leu Ile Met Pro Asp Asn Asn Gly Ser Ser Met Asp Asp Trp Glu Glu
    610                 615                 620
Arg Gln Thr Ser Gly Arg Ile Thr Glu Glu Phe Leu Gly Lys Tyr Leu
625                 630                 635                 640
Ser Thr Thr Pro Phe Pro Ser Gln His Arg Thr Glu Ile Glu Leu Phe
                645                 650                 655
Pro Tyr Ser Gly Asp Lys Ile Leu Val Glu Gly Ile Ser Thr Val Ile
            660                 665                 670
Tyr Pro Ser Leu Gln Thr Glu Met Thr His Arg Arg Glu Arg Thr Glu
        675                 680                 685
Thr Leu Ile Pro Glu Met Arg Thr Asp Thr Tyr Thr Asp Glu Ile Gln
    690                 695                 700
Glu Glu Ile Thr Lys Ser Pro Phe Met Gly Lys Thr Glu Glu Glu Val
705                 710                 715                 720
Phe Ser Gly Met Lys Leu Ser Thr Ser Leu Ser Glu Pro Ile His Val
                725                 730                 735
Thr Glu Ser Ser Val Glu Met Thr Lys Ser Phe Asp Phe Pro Thr Leu
            740                 745                 750
Ile Thr Lys Leu Ser Ala Glu Pro Thr Glu Val Arg Asp Met Glu Glu
        755                 760                 765
Asp Phe Thr Ala Thr Pro Gly Thr Thr Lys Tyr Asp Glu Asn Ile Thr
    770                 775                 780
Thr Val Leu Leu Ala His Gly Thr Leu Ser Val Glu Ala Ala Thr Val
785                 790                 795                 800
Ser Lys Trp Ser Trp Asp Glu Asp Asn Thr Thr Ser Lys Pro Leu Glu
                805                 810                 815
Ser Thr Glu Pro Ser Ala Ser Ser Lys Leu Pro Pro Ala Leu Leu Thr
            820                 825                 830
Thr Val Gly Met Asn Gly Lys Asp Lys Asp Ile Pro Ser Phe Thr Glu
        835                 840                 845
Asp Gly Ala Asp Glu Phe Thr Leu Ile Pro Asp Ser Thr Gln Lys Gln
    850                 855                 860
```

Leu Glu Glu Val Thr Asp Glu Asp Ile Ala Ala His Gly Lys Phe Thr
865 870 875 880
Ile Arg Phe Gln Pro Thr Thr Ser Thr Gly Ile Ala Glu Lys Ser Thr
885 890 895
Leu Arg Asp Ser Thr Thr Glu Glu Lys Val Pro Pro Ile Thr Ser Thr
900 905 910
Glu Gly Gln Val Tyr Ala Thr Met Glu Gly Ser Ala Leu Gly Glu Val
915 920 925
Glu Asp Val Asp Leu Ser Lys Pro Val Ser Thr Val Pro Gln Phe Ala
930 935 940
His Thr Ser Glu Val Glu Gly Leu Ala Phe Val Ser Tyr Ser Ser Thr
945 950 955 960
Gln Glu Pro Thr Thr Tyr Val Asp Ser Ser His Thr Ile Pro Leu Ser
965 970 975
Val Ile Pro Lys Thr Asp Trp Gly Val Leu Val Pro Ser Val Pro Ser
980 985 990
Glu Asp Glu Val Leu Gly Glu Pro Ser Gln Asp Ile Leu Val Ile Asp
995 1000 1005
Gln Thr Arg Leu Glu Ala Thr Ile Ser Pro Glu Thr Met Arg Thr
1010 1015 1020
Thr Lys Ile Thr Glu Gly Thr Thr Gln Glu Glu Phe Pro Trp Lys
1025 1030 1035
Glu Gln Thr Ala Glu Lys Pro Val Pro Ala Leu Ser Ser Thr Ala
1040 1045 1050
Trp Thr Pro Lys Glu Ala Val Thr Pro Leu Asp Glu Gln Glu Gly
1055 1060 1065
Asp Gly Ser Ala Tyr Thr Val Ser Glu Asp Glu Leu Leu Thr Gly
1070 1075 1080
Ser Glu Arg Val Pro Val Leu Glu Thr Thr Pro Val Gly Lys Ile
1085 1090 1095
Asp His Ser Val Ser Tyr Pro Pro Gly Ala Val Thr Glu His Lys
1100 1105 1110
Val Lys Thr Asp Glu Val Val Thr Leu Thr Pro Arg Ile Gly Pro
1115 1120 1125
Lys Val Ser Leu Ser Pro Gly Pro Glu Gln Lys Tyr Glu Thr Glu
1130 1135 1140
Gly Ser Ser Thr Thr Gly Phe Thr Ser Ser Leu Ser Pro Phe Ser
1145 1150 1155
Thr His Ile Thr Gln Leu Met Glu Glu Thr Thr Thr Glu Lys Thr
1160 1165 1170
Ser Leu Glu Asp Ile Asp Leu Gly Ser Gly Leu Phe Glu Lys Pro
1175 1180 1185
Lys Ala Thr Glu Leu Ile Glu Phe Ser Thr Ile Lys Val Thr Val
1190 1195 1200
Pro Ser Asp Ile Thr Thr Ala Phe Ser Ser Val Asp Arg Leu His
1205 1210 1215
Thr Thr Ser Ala Phe Lys Pro Ser Ser Ala Ile Thr Lys Lys Pro
1220 1225 1230
Pro Leu Ile Asp Arg Glu Pro Gly Glu Glu Thr Thr Ser Asp Met
1235 1240 1245
Val Ile Ile Gly Glu Ser Thr Ser His Val Pro Pro Thr Thr Leu
1250 1255 1260
Glu Asp Ile Val Ala Lys Glu Thr Glu Thr Asp Ile Asp Arg Glu
1265 1270 1275
Tyr Phe Thr Thr Ser Ser Pro Pro Ala Thr Gln Pro Thr Arg Pro
1280 1285 1290
Pro Thr Val Glu Asp Lys Glu Ala Phe Gly Pro Gln Ala Leu Ser
1295 1300 1305
Thr Pro Gln Pro Pro Ala Ser Thr Lys Phe His Pro Asp Ile Asn
1310 1315 1320
Val Tyr Ile Ile Glu Val Arg Glu Asn Lys Thr Gly Arg Met Ser
1325 1330 1335
Asp Leu Ser Val Ile Gly His Pro Ile Asp Ser Glu Ser Lys Glu
1340 1345 1350
Asp Glu Pro Cys Ser Glu Glu Thr Asp Pro Val His Asp Leu Met
1355 1360 1365
Ala Glu Ile Leu Pro Glu Phe Pro Asp Ile Ile Glu Ile Asp Leu
1370 1375 1380
Tyr His Ser Glu Glu Asn Glu Glu Glu Glu Glu Glu Cys Ala Asn

1385 1390 1395
Ala Thr Asp Val Thr Thr Thr Pro Ser Val Gln Tyr Ile Asn Gly
1400 1405 1410
Lys His Leu Val Thr Thr Val Pro Lys Asp Pro Glu Ala Ala Glu
1415 1420 1425
Ala Arg Arg Gly Gln Phe Glu Ser Val Ala Pro Ser Gln Asn Phe
1430 1435 1440
Ser Asp Ser Ser Glu Ser Asp Thr His Pro Phe Val Ile Ala Lys
1445 1450 1455
Thr Glu Leu Ser Thr Ala Val Gln Pro Asn Glu Ser Thr Glu Thr
1460 1465 1470
Thr Glu Ser Leu Glu Val Thr Trp Lys Pro Glu Thr Tyr Pro Glu
1475 1480 1485
Thr Ser Glu His Phe Ser Gly Gly Glu Pro Asp Val Phe Pro Thr
1490 1495 1500
Val Pro Phe His Glu Glu Phe Glu Ser Gly Thr Ala Lys Lys Gly
1505 1510 1515
Ala Glu Ser Val Thr Glu Arg Asp Thr Glu Val Gly His Gln Ala
1520 1525 1530
His Glu His Thr Glu Pro Val Ser Leu Phe Pro Glu Glu Ser Ser
1535 1540 1545
Gly Glu Ile Ala Ile Asp Gln Glu Ser Gln Lys Ile Ala Phe Ala
1550 1555 1560
Arg Ala Thr Glu Val Thr Phe Gly Glu Glu Val Glu Lys Ser Thr
1565 1570 1575
Ser Val Thr Tyr Thr Pro Thr Ile Val Pro Ser Ser Ala Ser Ala
1580 1585 1590
Tyr Val Ser Glu Glu Glu Ala Val Thr Leu Ile Gly Asn Pro Trp
1595 1600 1605
Pro Asp Asp Leu Leu Ser Thr Lys Glu Ser Trp Val Glu Ala Thr
1610 1615 1620
Pro Arg Gln Val Val Glu Leu Ser Gly Ser Ser Ser Ile Pro Ile
1625 1630 1635
Thr Glu Gly Ser Gly Glu Ala Glu Glu Asp Glu Asp Thr Met Phe
1640 1645 1650
Thr Met Val Thr Asp Leu Ser Gln Arg Asn Thr Thr Asp Thr Leu
1655 1660 1665
Ile Thr Leu Asp Thr Ser Arg Ile Ile Thr Glu Ser Phe Phe Glu
1670 1675 1680
Val Pro Ala Thr Thr Ile Tyr Pro Val Ser Glu Gln Pro Ser Ala
1685 1690 1695
Lys Val Val Pro Thr Lys Phe Val Ser Glu Thr Asp Thr Ser Glu
1700 1705 1710
Trp Ile Ser Ser Thr Thr Val Glu Glu Lys Lys Arg Lys Glu Glu
1715 1720 1725
Glu Gly Thr Thr Gly Thr Ala Ser Thr Phe Glu Val Tyr Ser Ser
1730 1735 1740
Thr Gln Arg Ser Asp Gln Leu Ile Leu Pro Phe Glu Leu Glu Ser
1745 1750 1755
Pro Asn Val Ala Thr Ser Ser Asp Ser Gly Thr Arg Lys Ser Phe
1760 1765 1770
Met Ser Leu Thr Thr Pro Thr Gln Ser Glu Arg Glu Met Thr Asp
1775 1780 1785
Ser Thr Pro Val Phe Thr Glu Thr Asn Thr Leu Glu Asn Leu Gly
1790 1795 1800
Ala Gln Thr Thr Glu His Ser Ser Ile His Gln Pro Gly Val Gln
1805 1810 1815
Glu Gly Leu Thr Thr Leu Pro Arg Ser Pro Ala Ser Val Phe Met
1820 1825 1830
Glu Gln Gly Ser Gly Glu Ala Ala Ala Asp Pro Glu Thr Thr Thr
1835 1840 1845
Val Ser Ser Phe Ser Leu Asn Val Glu Tyr Ala Ile Gln Ala Glu
1850 1855 1860
Lys Glu Val Ala Gly Thr Leu Ser Pro His Val Glu Thr Thr Phe
1865 1870 1875
Ser Thr Glu Pro Thr Gly Leu Val Leu Ser Thr Val Met Asp Arg
1880 1885 1890
Val Val Ala Glu Asn Ile Thr Gln Thr Ser Arg Glu Ile Val Ile
1895 1900 1905

Ser Glu Arg Leu Gly Glu Pro Asn Tyr Gly Ala Glu Ile Arg Gly
1910 1915 1920
Phe Ser Thr Gly Phe Pro Leu Glu Glu Asp Phe Ser Gly Asp Phe
1925 1930 1935
Arg Glu Tyr Ser Thr Val Ser His Pro Ile Ala Lys Glu Glu Thr
1940 1945 1950
Val Met Met Glu Gly Ser Gly Asp Ala Ala Phe Arg Asp Thr Gln
1955 1960 1965
Thr Ser Pro Ser Thr Val Pro Thr Ser Val His Ile Ser His Ile
1970 1975 1980
Ser Asp Ser Glu Gly Pro Ser Ser Thr Met Val Ser Thr Ser Ala
1985 1990 1995
Phe Pro Trp Glu Glu Phe Thr Ser Ser Ala Glu Gly Ser Gly Glu
2000 2005 2010
Gln Leu Val Thr Val Ser Ser Ser Val Val Pro Val Leu Pro Ser
2015 2020 2025
Ala Val Gln Lys Phe Ser Gly Thr Ala Ser Ser Ile Ile Asp Glu
2030 2035 2040
Gly Leu Gly Glu Val Gly Thr Val Asn Glu Ile Asp Arg Arg Ser
2045 2050 2055
Thr Ile Leu Pro Thr Ala Glu Val Glu Gly Thr Lys Ala Pro Val
2060 2065 2070
Glu Lys Glu Glu Val Lys Val Ser Gly Thr Val Ser Thr Asn Phe
2075 2080 2085
Pro Gln Thr Ile Glu Pro Ala Lys Leu Trp Ser Arg Gln Glu Val
2090 2095 2100
Asn Pro Val Arg Gln Glu Ile Glu Ser Glu Thr Thr Ser Glu Glu
2105 2110 2115
Gln Ile Gln Glu Glu Lys Ser Phe Glu Ser Pro Gln Asn Ser Pro
2120 2125 2130
Ala Thr Glu Gln Thr Ile Phe Asp Ser Gln Thr Phe Thr Glu Thr
2135 2140 2145
Glu Leu Lys Thr Thr Asp Tyr Ser Val Leu Thr Thr Lys Lys Thr
2150 2155 2160
Tyr Ser Asp Asp Lys Glu Met Lys Glu Glu Asp Thr Ser Leu Val
2165 2170 2175
Asn Met Ser Thr Pro Asp Pro Asp Ala Asn Gly Leu Glu Ser Tyr
2180 2185 2190
Thr Thr Leu Pro Glu Ala Thr Glu Lys Ser His Phe Phe Leu Ala
2195 2200 2205
Thr Ala Leu Val Thr Glu Ser Ile Pro Ala Glu His Val Val Thr
2210 2215 2220
Asp Ser Pro Ile Lys Lys Glu Glu Ser Thr Lys His Phe Pro Lys
2225 2230 2235
Gly Met Arg Pro Thr Ile Gln Glu Ser Asp Thr Glu Leu Leu Phe
2240 2245 2250
Ser Gly Leu Gly Ser Gly Glu Glu Val Leu Pro Thr Leu Pro Thr
2255 2260 2265
Glu Ser Val Asn Phe Thr Glu Val Glu Gln Ile Asn Asn Thr Leu
2270 2275 2280
Tyr Pro His Thr Ser Gln Val Glu Ser Thr Ser Ser Asp Lys Ile
2285 2290 2295
Glu Asp Phe Asn Arg Met Glu Asn Val Ala Lys Glu Val Gly Pro
2300 2305 2310
Leu Val Ser Gln Thr Asp Ile Phe Glu Gly Ser Gly Ser Val Thr
2315 2320 2325
Ser Thr Thr Leu Ile Glu Ile Leu Ser Asp Thr Gly Ala Glu Gly
2330 2335 2340
Pro Thr Val Ala Pro Leu Pro Phe Ser Thr Asp Ile Gly His Pro
2345 2350 2355
Gln Asn Gln Thr Val Arg Trp Ala Glu Glu Ile Gln Thr Ser Arg
2360 2365 2370
Pro Gln Thr Ile Thr Glu Gln Asp Ser Asn Lys Asn Ser Ser Thr
2375 2380 2385
Ala Glu Ile Asn Glu Thr Thr Thr Ser Ser Thr Asp Phe Leu Ala
2390 2395 2400
Arg Ala Tyr Gly Phe Glu Met Ala Lys Glu Phe Val Thr Ser Ala
2405 2410 2415
Pro Lys Pro Ser Asp Leu Tyr Tyr Glu Pro Ser Gly Glu Gly Ser

2420 2425 2430
Gly Glu Val Asp Ile Val Asp Ser Phe His Thr Ser Ala Thr Thr
2435 2440 2445
Gln Ala Thr Arg Gln Glu Ser Ser Thr Thr Phe Val Ser Asp Gly
2450 2455 2460
Ser Leu Glu Lys His Pro Glu Val Pro Ser Ala Lys Ala Val Thr
2465 2470 2475
Ala Asp Gly Phe Pro Thr Val Ser Val Met Leu Pro Leu His Ser
2480 2485 2490
Glu Gln Asn Lys Ser Ser Pro Asp Pro Thr Ser Thr Leu Ser Asn
2495 2500 2505
Thr Val Ser Tyr Glu Arg Ser Thr Asp Gly Ser Phe Gln Asp Arg
2510 2515 2520
Phe Arg Glu Phe Glu Asp Ser Thr Leu Lys Pro Asn Arg Lys Lys
2525 2530 2535
Pro Thr Glu Asn Ile Ile Ile Asp Leu Asp Lys Glu Asp Lys Asp
2540 2545 2550
Leu Ile Leu Thr Ile Thr Glu Ser Thr Ile Leu Glu Ile Leu Pro
2555 2560 2565
Glu Leu Thr Ser Asp Lys Asn Thr Ile Ile Asp Ile Asp His Thr
2570 2575 2580
Lys Pro Val Tyr Glu Asp Ile Leu Gly Met Gln Thr Asp Ile Asp
2585 2590 2595
Thr Glu Val Pro Ser Glu Pro His Asp Ser Asn Asp Glu Ser Asn
2600 2605 2610
Asp Asp Ser Thr Gln Val Gln Glu Ile Tyr Glu Ala Ala Val Asn
2615 2620 2625
Leu Ser Leu Thr Glu Glu Thr Phe Glu Gly Ser Ala Asp Val Leu
2630 2635 2640
Ala Ser Tyr Thr Gln Ala Thr His Asp Glu Ser Met Thr Tyr Glu
2645 2650 2655
Asp Arg Ser Gln Leu Asp His Met Gly Phe His Phe Thr Thr Gly
2660 2665 2670
Ile Pro Ala Pro Ser Thr Glu Thr Glu Leu Asp Val Leu Leu Pro
2675 2680 2685
Thr Ala Thr Ser Leu Pro Ile Pro Arg Lys Ser Ala Thr Val Ile
2690 2695 2700
Pro Glu Ile Glu Gly Ile Lys Ala Glu Ala Lys Ala Leu Asp Asp
2705 2710 2715
Met Phe Glu Ser Ser Thr Leu Ser Asp Gly Gln Ala Ile Ala Asp
2720 2725 2730
Gln Ser Glu Ile Ile Pro Thr Leu Gly Gln Phe Glu Arg Thr Gln
2735 2740 2745
Glu Glu Tyr Glu Asp Lys Lys His Ala Gly Pro Ser Phe Gln Pro
2750 2755 2760
Glu Phe Ser Ser Gly Ala Glu Glu Ala Leu Val Asp His Thr Pro
2765 2770 2775
Tyr Leu Ser Ile Ala Thr Thr His Leu Met Asp Gln Ser Val Thr
2780 2785 2790
Glu Val Pro Asp Val Met Glu Gly Ser Asn Pro Pro Tyr Tyr Thr
2795 2800 2805
Asp Thr Thr Leu Ala Val Ser Thr Phe Ala Lys Leu Ser Ser Gln
2810 2815 2820
Thr Pro Ser Ser Pro Leu Thr Ile Tyr Ser Gly Ser Glu Ala Ser
2825 2830 2835
Gly His Thr Glu Ile Pro Gln Pro Ser Ala Leu Pro Gly Ile Asp
2840 2845 2850
Val Gly Ser Ser Val Met Ser Pro Gln Asp Ser Phe Lys Glu Ile
2855 2860 2865
His Val Asn Ile Glu Ala Thr Phe Lys Pro Ser Ser Glu Glu Tyr
2870 2875 2880
Leu His Ile Thr Glu Pro Pro Ser Leu Ser Pro Asp Thr Lys Leu
2885 2890 2895
Glu Pro Ser Glu Asp Asp Gly Lys Pro Glu Leu Leu Glu Glu Met
2900 2905 2910
Glu Ala Ser Pro Thr Glu Leu Ile Ala Val Glu Gly Thr Glu Ile
2915 2920 2925
Leu Gln Asp Phe Gln Asn Lys Thr Asp Gly Gln Val Ser Gly Glu
2930 2935 2940

Ala Ile Lys Met Phe Pro Thr Ile Lys Thr Pro Glu Ala Gly Thr
2945 2950 2955
Val Ile Thr Thr Ala Asp Glu Ile Glu Leu Glu Gly Ala Thr Gln
2960 2965 2970
Trp Pro His Ser Thr Ser Ala Ser Ala Thr Tyr Gly Val Glu Ala
2975 2980 2985
Gly Val Val Pro Trp Leu Ser Pro Gln Thr Ser Glu Arg Pro Thr
2990 2995 3000
Leu Ser Ser Ser Pro Glu Ile Asn Pro Glu Thr Gln Ala Ala Leu
3005 3010 3015
Ile Arg Gly Gln Asp Ser Thr Ile Ala Ala Ser Glu Gln Gln Val
3020 3025 3030
Ala Ala Arg Ile Leu Asp Ser Asn Asp Gln Ala Thr Val Asn Pro
3035 3040 3045
Val Glu Phe Asn Thr Glu Val Ala Thr Pro Pro Phe Ser Leu Leu
3050 3055 3060
Glu Thr Ser Asn Glu Thr Asp Phe Leu Ile Gly Ile Asn Glu Glu
3065 3070 3075
Ser Val Glu Gly Thr Ala Ile Tyr Leu Pro Gly Pro Asp Arg Cys
3080 3085 3090
Lys Met Asn Pro Cys Leu Asn Gly Gly Thr Cys Tyr Pro Thr Glu
3095 3100 3105
Thr Ser Tyr Val Cys Thr Cys Val Pro Gly Tyr Ser Gly Asp Gln
3110 3115 3120
Cys Glu Leu Asp Phe Asp Glu Cys His Ser Asn Pro Cys Arg Asn
3125 3130 3135
Gly Ala Thr Cys Val Asp Gly Phe Asn Thr Phe Arg Cys Leu Cys
3140 3145 3150
Leu Pro Ser Tyr Val Gly Ala Leu Cys Glu Gln Asp Thr Glu Thr
3155 3160 3165
Cys Asp Tyr Gly Trp His Lys Phe Gln Gly Gln Cys Tyr Lys Tyr
3170 3175 3180
Phe Ala His Arg Arg Thr Trp Asp Ala Ala Glu Arg Glu Cys Arg
3185 3190 3195
Leu Gln Gly Ala His Leu Thr Ser Ile Leu Ser His Glu Glu Gln
3200 3205 3210
Met Phe Val Asn Arg Val Gly His Asp Tyr Gln Trp Ile Gly Leu
3215 3220 3225
Asn Asp Lys Met Phe Glu His Asp Phe Arg Trp Thr Asp Gly Ser
3230 3235 3240
Thr Leu Gln Tyr Glu Asn Trp Arg Pro Asn Gln Pro Asp Ser Phe
3245 3250 3255
Phe Ser Ala Gly Glu Asp Cys Val Val Ile Ile Trp His Glu Asn
3260 3265 3270
Gly Gln Trp Asn Asp Val Pro Cys Asn Tyr His Leu Thr Tyr Thr
3275 3280 3285
Cys Lys Lys Gly Thr Val Ala Cys Gly Gln Pro Pro Val Val Glu
3290 3295 3300
Asn Ala Lys Thr Phe Gly Lys Met Lys Pro Arg Tyr Glu Ile Asn
3305 3310 3315
Ser Leu Ile Arg Tyr His Cys Lys Asp Gly Phe Ile Gln Arg His
3320 3325 3330
Leu Pro Thr Ile Arg Cys Leu Gly Asn Gly Arg Trp Ala Ile Pro
3335 3340 3345
Lys Ile Thr Cys Met Asn Pro Ser Ala Tyr Gln Arg Thr Tyr Ser
3350 3355 3360
Met Lys Tyr Phe Lys Asn Ser Ser Ser Ala Lys Asp Asn Ser Ile
3365 3370 3375
Asn Thr Ser Lys His Asp His Arg Trp Ser Arg Arg Trp Gln Glu
3380 3385 3390
Ser Arg Arg
3395

<210> 286
<211> 148
<212> PRT
<213> Homo sapiens
<400> 286
Met Met His Leu Leu Asn Ser Gln Gly Trp Asn Glu Pro Ala Gly Pro

```
1               5                   10                  15
Pro Glu Ser Trp Ser Gly Val Gln Ser Ser Val Phe Leu Ser Val Tyr
            20                  25                  30
Ser Ser Leu Thr Val Pro Arg Pro Ser Gly Val Gly Ala Gly Ser Gln
        35                  40                  45
Cys Trp Arg Arg Asn Asn Lys Ser Gln Leu Glu Pro Leu Phe Leu Lys
    50                  55                  60
Ser Ala Tyr Cys Ala Gln Ile Leu Phe Lys His Trp Thr Trp Ile Leu
65                  70                  75                  80
Ser Leu Ala Leu Ser Thr Pro Ala Val Gly Val Pro Pro Leu Pro Thr
                85                  90                  95
Cys Asp Gly Val Gln Arg His Leu Leu Phe Cys Met Val Phe Asn Arg
            100                 105                 110
Leu Gly Val Leu Phe Ile Ser Ser Asn Phe Val Gln Glu Leu Met Ala
        115                 120                 125
Cys Leu Gly Leu Ser Ser Leu Asn Gln Arg Lys Trp Lys Pro Phe Pro
    130                 135                 140
Cys Cys Ser Pro
145

<210>  287
<211>  449
<212>  PRT
<213>  Homo sapiens
<400>  287
Met Leu Trp Lys Leu Val Glu Asn Val Lys Tyr Glu Asp Ile Tyr Glu
1               5                   10                  15
Asp Arg His Asp Gly Val Pro Ser His Ser Ser Arg Leu Ser Gln Leu
            20                  25                  30
Gly Ser Val Ser Gln Gly Pro Tyr Ser Ser Ala Pro Pro Leu Ser His
        35                  40                  45
Thr Pro Ser Ser Asp Phe Gln Pro Pro Tyr Phe Pro Pro Pro Tyr Gln
    50                  55                  60
Pro Leu Pro Tyr His Gln Ser Gln Asp Pro Tyr Ser His Val Asn Asp
65                  70                  75                  80
Pro Tyr Ser Leu Asn Pro Leu His Gln Pro Gln Gln His Pro Trp Gly
                85                  90                  95
Gln Arg Gln Arg Gln Glu Val Gly Ser Glu Ala Gly Ser Leu Leu Pro
            100                 105                 110
Gln Pro Arg Ala Ala Leu Pro Gln Leu Ser Gly Leu Asp Pro Arg Arg
        115                 120                 125
Asp Tyr His Ser Val Arg Arg Pro Asp Val Leu Leu His Ser Ala His
    130                 135                 140
His Gly Leu Asp Ala Gly Met Gly Asp Ser Leu Ser Leu His Gly Leu
145                 150                 155                 160
Gly His Pro Gly Met Glu Asp Val Gln Ser Val Glu Asp Ala Asn Asn
                165                 170                 175
Ser Gly Met Asn Leu Leu Asp Gln Ser Val Ile Lys Lys Val Pro Val
            180                 185                 190
Pro Pro Lys Ser Val Thr Ser Leu Met Met Asn Lys Asp Gly Phe Leu
        195                 200                 205
Gly Gly Met Ser Val Asn Thr Gly Glu Val Phe Cys Ser Val Pro Gly
    210                 215                 220
Arg Leu Ser Leu Leu Ser Ser Thr Ser Lys Tyr Lys Val Thr Val Gly
225                 230                 235                 240
Glu Val Gln Arg Arg Leu Ser Pro Pro Glu Cys Leu Asn Ala Ser Leu
                245                 250                 255
Leu Gly Gly Val Leu Arg Arg Ala Lys Ser Lys Asn Gly Gly Arg Ser
            260                 265                 270
Leu Arg Glu Arg Leu Glu Lys Ile Gly Leu Asn Leu Pro Ala Gly Arg
        275                 280                 285
Arg Lys Ala Ala Asn Val Thr Leu Leu Thr Ser Leu Val Glu Gly Glu
    290                 295                 300
Ala Val His Leu Ala Arg Asp Phe Gly Tyr Ile Cys Glu Thr Glu Phe
305                 310                 315                 320
Pro Ala Lys Ala Val Ser Glu Tyr Leu Asn Arg Gln His Thr Asp Pro
                325                 330                 335
Ser Asp Leu His Ser Arg Lys Asn Met Leu Leu Ala Thr Lys Gln Leu
            340                 345                 350
```

Cys Lys Glu Phe Thr Asp Leu Leu Ala Gln Asp Arg Thr Pro Ile Gly
355 360 365
Asn Ser Arg Pro Ser Pro Ile Leu Glu Pro Gly Ile Gln Ser Cys Leu
370 375 380
Thr His Phe Ser Leu Ile Thr His Gly Phe Gly Ala Pro Ala Ile Cys
385 390 395 400
Ala Ala Leu Thr Ala Leu Gln Asn Tyr Leu Thr Glu Ala Leu Lys Gly
405 410 415
Met Asp Lys Met Phe Leu Asn Asn Thr Thr Thr Asn Arg His Thr Ser
420 425 430
Gly Glu Gly Pro Gly Ser Lys Thr Gly Asp Lys Glu Glu Lys His Arg
435 440 445
Lys

<210> 288
<211> 114
<212> PRT
<213> Homo sapiens
<400> 288
Met Leu Leu Ser Val Met Ala Tyr Asp Arg Phe Val Ala Ile Cys His
1 5 10 15
Pro Leu Tyr His Ser Ala Ile Met Asn Pro Cys Phe Cys Gly Phe Leu
20 25 30
Leu Leu Leu Ser Phe Phe Phe Phe Leu Ser Leu Leu Asp Thr Gln Leu
35 40 45
His Asn Leu Ile Ala Leu Gln Met Thr Cys Phe Lys Asp Val Glu Ile
50 55 60
Pro Asn Phe Phe Cys Asp Pro Ser Gln Leu Pro His Leu Ala Cys Cys
65 70 75 80
Asp Thr Phe Thr Asn Asn Ile Ile Val Tyr Phe Pro Ala Val Ile Phe
85 90 95
Val Phe Leu Pro Ile Ser Gly Thr Leu Phe Ser Leu Lys Leu Phe Pro
100 105 110
Pro Phe

<210> 289
<211> 195
<212> PRT
<213> Homo sapiens
<400> 289
Met Met Ala Ile Arg Glu Leu Lys Val Cys Leu Leu Gly Asp Thr Gly
1 5 10 15
Val Gly Lys Ser Ser Ile Val Cys Arg Phe Val Gln Asp His Phe Asp
20 25 30
His Asn Ile Ser Pro Thr Ile Gly Ala Ser Phe Met Thr Lys Thr Val
35 40 45
Pro Cys Gly Asn Glu Leu His Lys Phe Leu Ile Trp Asp Thr Ala Gly
50 55 60
Gln Glu Arg Phe His Ser Leu Ala Pro Met Tyr Tyr Arg Gly Ser Ala
65 70 75 80
Ala Ala Val Ile Val Tyr Asp Ile Thr Lys Gln Asp Ser Phe Tyr Thr
85 90 95
Leu Lys Lys Trp Val Lys Glu Leu Lys Glu His Gly Pro Glu Asn Ile
100 105 110
Val Met Ala Ile Ala Gly Asn Lys Cys Asp Leu Ser Asp Ile Arg Glu
115 120 125
Val Pro Leu Lys Asp Ala Lys Glu Tyr Ala Glu Ser Ile Gly Ala Ile
130 135 140
Val Val Glu Thr Ser Ala Lys Asn Ala Ile Asn Ile Glu Glu Leu Phe
145 150 155 160
Gln Gly Ile Ser Arg Gln Ile Pro Pro Leu Asp Pro His Glu Asn Gly
165 170 175
Asn Asn Gly Thr Ile Lys Val Glu Lys Pro Thr Met Gln Ala Ser Arg
180 185 190
Arg Cys Cys
195

<210> 290
<211> 270
<212> PRT
<213> Homo sapiens
<400> 290

Met Ala Ala Ser Ser Ser Gly Glu Lys Glu Lys Glu Arg Leu Gly Gly
1 5 10 15
Gly Leu Gly Val Ala Gly Gly Asn Ser Thr Arg Glu Arg Leu Leu Ser
20 25 30
Ala Leu Glu Asp Leu Glu Val Leu Ser Arg Glu Leu Ile Glu Met Leu
35 40 45
Ala Ile Ser Arg Asn Gln Lys Leu Leu Gln Ala Gly Glu Glu Asn Gln
50 55 60
Val Leu Glu Leu Leu Ile His Arg Asp Gly Glu Phe Gln Glu Leu Met
65 70 75 80
Lys Leu Ala Leu Asn Gln Gly Lys Ile His His Glu Met Gln Val Leu
85 90 95
Glu Lys Glu Val Glu Lys Arg Asp Ser Asp Ile Gln Gln Leu Gln Lys
100 105 110
Gln Leu Lys Glu Ala Glu Gln Ile Leu Ala Thr Ala Val Tyr Gln Ala
115 120 125
Lys Glu Lys Leu Lys Ser Ile Glu Lys Ala Arg Lys Gly Ala Ile Ser
130 135 140
Ser Glu Glu Ile Ile Lys Tyr Ala His Arg Ile Ser Ala Ser Asn Ala
145 150 155 160
Val Cys Ala Pro Leu Thr Trp Val Pro Gly Asp Pro Arg Arg Pro Tyr
165 170 175
Pro Thr Asp Leu Glu Met Arg Ser Gly Leu Leu Gly Gln Met Asn Asn
180 185 190
Pro Ser Thr Asn Gly Val Asn Gly His Leu Pro Gly Asp Ala Leu Ala
195 200 205
Ala Gly Arg Leu Pro Asp Val Leu Ala Pro Gln Tyr Pro Trp Gln Ser
210 215 220
Asn Asp Met Ser Met Asn Met Leu Pro Pro Asn His Ser Ser Asp Phe
225 230 235 240
Leu Leu Glu Pro Pro Gly His Asn Lys Glu Asn Glu Asp Asp Val Glu
245 250 255
Ile Met Ser Thr Asp Ser Ser Ser Ser Ser Ser Glu Ser Asp
260 265 270

<210> 291
<211> 228
<212> PRT
<213> Homo sapiens
<400> 291

Met Leu Ser Arg Cys Arg Ser Gly Leu Leu His Val Leu Gly Leu Ser
1 5 10 15
Phe Leu Leu Gln Thr Arg Arg Pro Ile Leu Leu Cys Ser Pro Arg Leu
20 25 30
Met Lys Pro Leu Val Val Phe Val Leu Gly Gly Pro Gly Ala Gly Lys
35 40 45
Gly Thr Gln Cys Ala Arg Ile Val Glu Lys Tyr Gly Tyr Thr His Leu
50 55 60
Ser Ala Gly Glu Leu Leu Arg Asp Glu Arg Lys Asn Pro Asp Ser Gln
65 70 75 80
Tyr Gly Glu Leu Ile Glu Lys Tyr Ile Lys Glu Gly Lys Ile Val Pro
85 90 95
Val Glu Ile Thr Ile Ser Leu Leu Lys Arg Glu Met Asp Gln Thr Met
100 105 110
Ala Ala Asn Ala Gln Lys Asn Lys Phe Leu Ile Asp Gly Phe Pro Arg
115 120 125
Asn Gln Asp Asn Leu Gln Gly Trp Asn Lys Thr Met Asp Gly Lys Ala
130 135 140
Asp Val Ser Phe Val Leu Phe Phe Asp Cys Asn Asn Glu Ile Cys Ile
145 150 155 160
Glu Arg Cys Leu Glu Arg Gly Lys Ser Ser Gly Arg Ser Asp Asp Asn
165 170 175
Arg Glu Ser Leu Glu Lys Arg Ile Gln Thr Tyr Leu Gln Ser Thr Lys
180 185 190

Pro Ile Ile Asp Leu Tyr Glu Glu Met Gly Lys Val Lys Lys Ile Asp
195 200 205
Ala Ser Lys Ser Val Asp Glu Val Phe Asp Glu Val Val Gln Ile Phe
210 215 220
Asp Lys Glu Gly
225

<210> 292
<211> 130
<212> PRT
<213> Homo sapiens
<400> 292
Met Ala Phe Cys Leu Arg Ala Ser Leu Gly Pro Asp Cys Cys Cys Trp
1 5 10 15
Tyr Gln Ser Pro Glu Thr Leu Pro Pro Trp Ser Pro Cys Met Pro Ser
20 25 30
Cys Ser Tyr Cys Leu Val Ala Leu Lys Gln His Ile Ser Thr Val Pro
35 40 45
Gly Arg Ile Val Gly Gly Phe Thr Thr Pro Ala Phe Leu Thr Ser Ser
50 55 60
Ser Ala Gln His Trp Val Pro Leu Pro Ser Phe Pro Ala Gly Ala Ser
65 70 75 80
Trp Ala Thr Val Ser Gly Ser Gly Pro Ala Arg Val Val Leu Leu Phe
85 90 95
Leu Leu Leu Leu Gly Asn His Thr Trp Gln Val Lys Cys Pro Lys His
100 105 110
Leu Val Ser Leu Pro Lys Asn Lys Pro Phe Leu Cys Thr Ser Leu Glu
115 120 125
Ala Arg
130

<210> 293
<211> 460
<212> PRT
<213> Homo sapiens
<400> 293
Met Ala Ser Leu Leu Gln Ser Asp Arg Val Leu Tyr Leu Val Gln Gly
1 5 10 15
Glu Lys Lys Val Arg Ala Pro Leu Ser Gln Leu Tyr Phe Cys Arg Tyr
20 25 30
Cys Ser Glu Leu Arg Ser Leu Glu Cys Val Ser His Glu Val Asp Ser
35 40 45
His Tyr Cys Pro Ser Cys Leu Glu Asn Met Pro Ser Ala Glu Ala Lys
50 55 60
Leu Lys Lys Asn Arg Cys Ala Asn Cys Phe Asp Cys Pro Gly Cys Met
65 70 75 80
His Thr Leu Ser Thr Arg Ala Thr Ser Ile Ser Thr Gln Leu Pro Asp
85 90 95
Asp Pro Ala Lys Thr Thr Met Lys Lys Ala Tyr Tyr Leu Ala Cys Gly
100 105 110
Phe Cys Arg Trp Thr Ser Arg Asp Val Gly Met Ala Asp Lys Ser Val
115 120 125
Ala Ser Gly Gly Trp Gln Glu Pro Glu Asn Pro His Thr Gln Arg Met
130 135 140
Asn Lys Leu Ile Glu Tyr Tyr Gln Gln Leu Ala Gln Lys Glu Lys Val
145 150 155 160
Glu Arg Asp Arg Lys Lys Leu Ala Arg Arg Arg Asn Tyr Met Pro Leu
165 170 175
Ala Phe Ser Asp Lys Tyr Gly Leu Gly Thr Arg Leu Gln Arg Pro Arg
180 185 190
Ala Gly Ala Ser Ile Ser Thr Leu Ala Gly Leu Ser Leu Lys Glu Gly
195 200 205
Glu Asp Gln Lys Glu Ile Lys Ile Glu Pro Ala Gln Ala Val Asp Glu
210 215 220
Val Glu Pro Leu Pro Glu Asp Tyr Tyr Thr Arg Pro Val Asn Leu Thr
225 230 235 240
Glu Val Thr Thr Leu Gln Gln Arg Leu Leu Gln Pro Asp Phe Gln Pro
245 250 255
Val Cys Ala Ser Gln Leu Tyr Pro Arg His Lys His Leu Leu Ile Lys

```
            260                 265                 270
Arg Ser Leu Arg Cys Arg Lys Cys Glu His Asn Leu Ser Lys Pro Glu
        275                 280                 285
Phe Asn Pro Thr Ser Ile Lys Phe Lys Ile Gln Leu Val Ala Val Asn
    290                 295                 300
Tyr Ile Pro Glu Val Arg Ile Met Ser Ile Pro Asn Leu Arg Tyr Met
305                 310                 315                 320
Lys Glu Ser Gln Val Leu Leu Thr Leu Thr Asn Pro Val Glu Asn Leu
                325                 330                 335
Thr His Val Thr Leu Phe Glu Cys Glu Glu Gly Asp Pro Asp Asp Ile
            340                 345                 350
Asn Ser Thr Ala Lys Val Val Val Pro Pro Lys Glu Leu Val Leu Ala
        355                 360                 365
Gly Lys Asp Ala Ala Ala Glu Tyr Asp Glu Leu Ala Glu Pro Gln Asp
    370                 375                 380
Phe Gln Asp Asp Pro Asp Ile Ile Ala Phe Arg Lys Ala Asn Lys Val
385                 390                 395                 400
Gly Ile Phe Ile Lys Val Thr Pro Gln Arg Glu Glu Gly Glu Val Thr
                405                 410                 415
Val Cys Phe Lys Met Lys His Asp Phe Lys Asn Leu Ala Ala Pro Ile
            420                 425                 430
Arg Pro Ile Glu Glu Ser Asp Gln Gly Thr Glu Val Ile Trp Leu Thr
        435                 440                 445
Gln His Val Glu Leu Ser Leu Gly Pro Leu Leu Pro
    450                 455                 460

<210>  294
<211>  524
<212>  PRT
<213>  Homo sapiens
<400>  294
Met Thr Ala Glu Asp Ser Thr Ala Ala Met Ser Ser Asp Ser Ala Ala
1               5                   10                  15
Gly Ser Ser Ala Lys Val Pro Glu Gly Val Ala Gly Ala Pro Asn Glu
            20                  25                  30
Ala Ala Leu Leu Ala Leu Met Glu Arg Thr Gly Tyr Ser Met Val Gln
        35                  40                  45
Glu Asn Gly Gln Arg Lys Tyr Gly Gly Pro Pro Pro Gly Trp Glu Gly
    50                  55                  60
Pro His Pro Gln Arg Gly Cys Glu Val Phe Val Gly Lys Ile Pro Arg
65                  70                  75                  80
Asp Val Tyr Glu Asp Glu Leu Val Pro Val Phe Glu Ala Val Gly Arg
                85                  90                  95
Thr Tyr Glu Leu Arg Leu Met Met Asp Phe Asp Gly Lys Asn Arg Gly
            100                 105                 110
Tyr Ala Phe Val Met Tyr Cys His Lys His Glu Ala Lys Arg Ala Val
        115                 120                 125
Arg Glu Leu Asn Asn Tyr Glu Ile Arg Pro Gly Arg Leu Leu Gly Val
    130                 135                 140
Cys Cys Ser Val Asp Asn Cys Arg Leu Phe Ile Gly Gly Ile Pro Lys
145                 150                 155                 160
Met Lys Lys Arg Glu Glu Ile Leu Glu Glu Ile Ala Lys Val Thr Glu
                165                 170                 175
Gly Val Leu Asp Val Ile Val Tyr Ala Ser Ala Ala Asp Lys Met Lys
            180                 185                 190
Asn Arg Gly Phe Ala Phe Val Glu Tyr Glu Ser His Arg Ala Ala Ala
        195                 200                 205
Met Ala Arg Arg Lys Leu Met Pro Gly Arg Ile Gln Leu Trp Gly His
    210                 215                 220
Gln Ile Ala Val Asp Trp Ala Glu Pro Glu Ile Asp Val Asp Glu Asp
225                 230                 235                 240
Val Met Glu Thr Val Lys Ile Leu Tyr Val Arg Asn Leu Met Ile Glu
                245                 250                 255
Thr Thr Glu Asp Thr Ile Lys Lys Ser Phe Gly Gln Phe Asn Pro Gly
            260                 265                 270
Cys Val Glu Arg Val Lys Lys Ile Arg Asp Tyr Ala Phe Val His Phe
        275                 280                 285
Thr Ser Arg Glu Asp Ala Val His Ala Met Asn Asn Leu Asn Gly Thr
    290                 295                 300
```

Glu Leu Glu Gly Ser Cys Leu Glu Val Thr Leu Ala Lys Pro Val Asp
305 310 315 320
Lys Glu Gln Tyr Ser Arg Tyr Gln Lys Ala Ala Arg Gly Gly Gly Ala
325 330 335
Ala Glu Ala Ala Gln Gln Pro Ser Tyr Val Tyr Ser Cys Asp Pro Tyr
340 345 350
Thr Leu Ala Tyr Tyr Gly Tyr Pro Tyr Asn Ala Leu Ile Gly Pro Asn
355 360 365
Arg Asp Tyr Phe Val Lys Val Ala Ile Pro Ala Ile Gly Ala Gln Tyr
370 375 380
Ser Met Phe Pro Ala Ala Pro Ala Pro Lys Met Ile Glu Asp Gly Lys
385 390 395 400
Ile His Thr Val Glu His Met Ile Ser Pro Ile Ala Val Gln Pro Asp
405 410 415
Pro Ala Ser Ala Ala Ala Ala Ala Ala Ala Ala Ala Ala Ala Ala Ala
420 425 430
Ala Val Ile Pro Thr Val Ser Thr Pro Pro Pro Phe Gln Gly Arg Pro
435 440 445
Ile Thr Pro Val Tyr Thr Val Ala Pro Asn Val Gln Arg Ile Pro Thr
450 455 460
Ala Gly Ile Tyr Gly Ala Ser Tyr Val Pro Phe Ala Ala Pro Ala Thr
465 470 475 480
Ala Thr Ile Ala Thr Leu Gln Lys Asn Ala Ala Ala Ala Ala Ala Val
485 490 495
Tyr Gly Gly Tyr Ala Gly Tyr Ile Pro Gln Ala Phe Pro Ala Ala Ala
500 505 510
Ile Gln Val Pro Ile Pro Asp Val Tyr Gln Thr Tyr
515 520

<210> 295
<211> 1232
<212> PRT
<213> Homo sapiens
<400> 295
Met Lys Glu Glu Val Lys Gly Ile Pro Val Arg Val Ala Leu Arg Cys
1 5 10 15
Arg Pro Leu Val Pro Lys Glu Ile Ser Glu Gly Cys Gln Met Cys Leu
20 25 30
Ser Phe Val Pro Gly Glu Pro Gln Val Val Val Gly Thr Asp Lys Ser
35 40 45
Phe Thr Tyr Asp Phe Val Phe Asp Pro Ser Thr Glu Gln Glu Glu Val
50 55 60
Phe Asn Thr Ala Val Ala Pro Leu Ile Lys Gly Val Phe Lys Gly Tyr
65 70 75 80
Asn Ala Thr Val Leu Ala Tyr Gly Gln Thr Gly Ser Gly Lys Thr Tyr
85 90 95
Ser Met Gly Gly Ala Tyr Thr Ala Glu Gln Glu Asn Glu Pro Thr Val
100 105 110
Gly Val Ile Pro Arg Val Ile Gln Leu Leu Phe Lys Glu Ile Asp Lys
115 120 125
Lys Ser Asp Phe Glu Phe Thr Leu Lys Val Ser Tyr Leu Glu Ile Tyr
130 135 140
Asn Glu Glu Ile Leu Asp Leu Leu Cys Pro Ser Arg Glu Lys Ala Gln
145 150 155 160
Ile Asn Ile Arg Glu Asp Pro Lys Glu Gly Ile Lys Ile Val Gly Leu
165 170 175
Thr Glu Lys Thr Val Leu Val Ala Leu Asp Thr Val Ser Cys Leu Glu
180 185 190
Gln Gly Asn Asn Ser Arg Thr Val Ala Ser Thr Ala Met Asn Ser Gln
195 200 205
Ser Ser Arg Ser His Ala Ile Phe Thr Ile Ser Leu Glu Gln Gly Lys
210 215 220
Lys Ser Asp Lys Asn Ser Ser Phe Arg Ser Lys Leu His Leu Val Asp
225 230 235 240
Leu Ala Gly Ser Glu Arg Gln Lys Lys Thr Lys Ala Glu Gly Asp Arg
245 250 255
Leu Lys Glu Gly Ile Asn Ile Asn Arg Gly Leu Leu Cys Leu Gly Asn
260 265 270
Val Ile Ser Ala Leu Gly Asp Asp Lys Lys Gly Gly Phe Ala Pro Tyr

```
                275                 280                 285
Arg Asp Ser Lys Leu Thr Arg Leu Leu Gln Asp Ser Leu Gly Gly Asn
    290                 295                 300
Ser His Thr Leu Met Ile Ala Cys Val Ser Pro Ala Asp Ser Asn Leu
305                 310                 315                 320
Glu Glu Thr Leu Asn Thr Leu Arg Tyr Ala Asp Arg Ala Arg Lys Ile
                325                 330                 335
Lys Asn Lys Pro Ile Val Asn Ile Asp Pro Gln Thr Ala Glu Leu Asn
            340                 345                 350
His Leu Lys Gln Gln Val Gln Gln Leu Gln Val Leu Leu Leu Gln Ala
        355                 360                 365
His Gly Gly Thr Leu Pro Gly Ser Ile Thr Val Glu Pro Ser Glu Asn
    370                 375                 380
Leu Gln Ser Leu Met Glu Lys Asn Gln Ser Leu Val Glu Glu Asn Glu
385                 390                 395                 400
Lys Leu Ser Arg Gly Leu Ser Glu Ala Ala Gly Gln Thr Ala Gln Met
                405                 410                 415
Leu Glu Arg Ile Ile Trp Thr Glu Gln Ala Asn Glu Lys Met Asn Ala
            420                 425                 430
Lys Leu Glu Glu Leu Arg Gln His Ala Ala Cys Lys Leu Asp Leu Gln
        435                 440                 445
Lys Leu Val Glu Thr Leu Glu Asp Gln Glu Leu Lys Glu Asn Val Glu
    450                 455                 460
Ile Ile Cys Asn Leu Gln Gln Leu Ile Thr Gln Leu Ser Asp Glu Thr
465                 470                 475                 480
Val Ala Cys Met Ala Ala Ala Ile Asp Thr Ala Val Glu Gln Glu Ala
                485                 490                 495
Gln Val Glu Thr Ser Pro Glu Thr Ser Arg Ser Ser Asp Ala Phe Thr
            500                 505                 510
Thr Gln His Ala Leu Arg Gln Ala Gln Met Ser Lys Glu Leu Val Glu
        515                 520                 525
Leu Asn Lys Ala Leu Ala Leu Lys Glu Ala Leu Ala Arg Lys Met Thr
    530                 535                 540
Gln Asn Asp Ser Gln Leu Gln Pro Ile Gln Tyr Gln Tyr Gln Asp Asn
545                 550                 555                 560
Ile Lys Glu Pro Glu Leu Glu Val Ile Asn Leu Gln Lys Glu Lys Glu
                565                 570                 575
Glu Leu Val Leu Glu Leu Gln Thr Ala Lys Lys Asp Ala Asn Gln Ala
            580                 585                 590
Lys Leu Ser Glu Arg Arg Arg Lys Arg Leu Gln Glu Leu Glu Gly Gln
        595                 600                 605
Ile Ala Asp Leu Lys Lys Lys Leu Asn Glu Gln Ser Lys Leu Leu Lys
    610                 615                 620
Leu Lys Glu Ser Thr Glu Arg Thr Val Ser Lys Leu Asn Gln Glu Ile
625                 630                 635                 640
Arg Met Met Lys Asn Gln Arg Val Gln Leu Met Arg Gln Met Lys Glu
                645                 650                 655
Asp Ala Glu Lys Phe Arg Gln Trp Lys Gln Lys Arg Asp Lys Glu Val
            660                 665                 670
Ile Gln Leu Lys Glu Arg Asp Arg Lys Arg Gln Tyr Glu Leu Leu Lys
        675                 680                 685
Leu Glu Arg Asn Phe Gln Lys Gln Ser Asn Val Leu Arg Arg Lys Thr
    690                 695                 700
Glu Glu Ala Ala Ala Ala Asn Lys Arg Leu Lys Asp Ala Leu Gln Lys
705                 710                 715                 720
Gln Arg Glu Val Ala Asp Lys Arg Lys Glu Thr Gln Ser Arg Gly Met
                725                 730                 735
Glu Gly Thr Ala Ala Arg Val Lys Asn Trp Leu Gly Asn Glu Ile Glu
            740                 745                 750
Val Met Val Ser Thr Glu Glu Ala Lys Arg His Leu Asn Asp Leu Leu
        755                 760                 765
Glu Asp Arg Lys Ile Leu Ala Gln Asp Val Ala Gln Leu Lys Glu Lys
    770                 775                 780
Lys Glu Ser Gly Glu Asn Pro Pro Pro Lys Leu Arg Arg Arg Thr Phe
785                 790                 795                 800
Ser Leu Thr Glu Val Arg Gly Gln Val Ser Glu Ser Glu Asp Ser Ile
                805                 810                 815
Thr Lys Gln Ile Glu Ser Leu Glu Thr Glu Met Glu Phe Arg Ser Ala
            820                 825                 830
```

Gln Ile Ala Asp Leu Gln Gln Lys Leu Leu Asp Ala Glu Ser Glu Asp
835 840 845
Arg Pro Lys Gln Arg Trp Glu Asn Ile Ala Thr Ile Leu Glu Ala Lys
850 855 860
Cys Ala Leu Lys Tyr Leu Ile Gly Glu Leu Val Ser Ser Lys Ile Gln
865 870 875 880
Val Ser Lys Leu Glu Ser Ser Leu Lys Gln Ser Lys Thr Ser Cys Ala
885 890 895
Asp Met Gln Lys Met Leu Phe Glu Glu Arg Asn His Phe Ala Glu Ile
900 905 910
Glu Thr Glu Leu Gln Ala Glu Leu Val Arg Met Glu Gln Gln His Gln
915 920 925
Glu Lys Val Leu Tyr Leu Leu Ser Gln Leu Gln Gln Ser Gln Met Ala
930 935 940
Glu Lys Gln Leu Glu Glu Ser Val Ser Glu Lys Glu Gln Gln Leu Leu
945 950 955 960
Ser Thr Leu Lys Cys Gln Asp Glu Glu Leu Glu Lys Met Arg Glu Val
965 970 975
Cys Glu Gln Asn Gln Gln Leu Leu Arg Glu Asn Glu Ile Ile Lys Gln
980 985 990
Lys Leu Thr Leu Leu Gln Val Ala Ser Arg Gln Lys His Leu Pro Lys
995 1000 1005
Asp Thr Leu Leu Ser Pro Asp Ser Ser Phe Glu Tyr Val Gln Pro
1010 1015 1020
Lys Pro Lys Pro Ser Arg Val Lys Glu Lys Phe Leu Glu Gln Ser
1025 1030 1035
Met Asp Ile Glu Asp Leu Lys Tyr Cys Ser Glu His Ser Val Asn
1040 1045 1050
Glu His Glu Asp Gly Asp Gly Asp Asp Asp Glu Gly Asp Asp Glu
1055 1060 1065
Glu Trp Lys Pro Thr Lys Leu Val Asn Val Ser Arg Lys Asn Ile
1070 1075 1080
Gln Gly Cys Ser Cys Lys Gly Trp Cys Gly Asn Lys Gln Cys Gly
1085 1090 1095
Cys Arg Lys Gln Lys Ser Asp Cys Gly Val Asp Cys Cys Cys Asp
1100 1105 1110
Pro Thr Lys Cys Arg Asn Arg Gln Gln Gly Lys Asp Ser Leu Gly
1115 1120 1125
Thr Val Glu Arg Thr Gln Asp Ser Glu Ser Ser Phe Lys Leu Glu
1130 1135 1140
Asp Pro Thr Glu Val Thr Pro Gly Leu Ser Phe Phe Asn Pro Val
1145 1150 1155
Cys Ala Thr Pro Asn Ser Lys Ile Leu Lys Glu Met Cys Asp Val
1160 1165 1170
Glu Gln Val Leu Ser Lys Lys Thr Pro Pro Ala Pro Ser Pro Phe
1175 1180 1185
Asp Leu Pro Glu Leu Lys His Val Ala Thr Glu Tyr Gln Glu Asn
1190 1195 1200
Lys Ala Pro Gly Lys Lys Lys Lys Arg Ala Leu Ala Ser Asn Thr
1205 1210 1215
Ser Phe Phe Ser Gly Cys Ser Pro Ile Glu Glu Glu Ala His
1220 1225 1230

<210> 296
<211> 230
<212> PRT
<213> Homo sapiens
<400> 296
Met Ala Gln Gly Leu Ile Glu Val Glu Arg Lys Phe Leu Pro Gly Pro
1 5 10 15
Gly Thr Glu Glu Arg Leu Gln Glu Leu Gly Gly Thr Leu Glu Tyr Arg
20 25 30
Val Thr Phe Arg Asp Thr Tyr Tyr Asp Thr Pro Glu Leu Ser Leu Met
35 40 45
Gln Ala Asp His Trp Leu Arg Arg Arg Glu Asp Ser Gly Trp Glu Leu
50 55 60
Lys Cys Pro Gly Ala Ala Gly Val Leu Gly Pro His Thr Glu Tyr Lys
65 70 75 80
Glu Leu Thr Ala Glu Pro Thr Ile Val Ala Gln Leu Cys Lys Val Leu

```
                85                  90                  95
Arg Ala Asp Gly Leu Gly Ala Gly Asp Val Ala Ala Val Leu Gly Pro
            100                 105                 110
Leu Gly Leu Gln Glu Val Ala Ser Phe Val Thr Lys Arg Ser Ala Trp
        115                 120                 125
Lys Leu Val Leu Leu Gly Ala Asp Glu Glu Glu Pro Gln Leu Arg Val
    130                 135                 140
Asp Leu Asp Thr Ala Asp Phe Gly Tyr Ala Val Gly Glu Val Glu Ala
145                 150                 155                 160
Leu Val His Glu Glu Ala Glu Val Pro Thr Ala Leu Glu Lys Ile His
                165                 170                 175
Arg Leu Ser Ser Met Leu Gly Val Pro Ala Gln Glu Thr Ala Pro Ala
            180                 185                 190
Lys Leu Ile Val Tyr Leu Gln Arg Phe Arg Pro Gln Asp Tyr Gln Arg
        195                 200                 205
Leu Leu Glu Val Asn Ser Ser Arg Glu Arg Pro Gln Glu Thr Glu Asp
    210                 215                 220
Pro Asp His Cys Leu Gly
225                 230
```

<210> 297
<211> 329
<212> PRT
<213> Homo sapiens
<400> 297

```
Met Ser Gly Val Val Pro Thr Ala Pro Glu Gln Pro Ala Gly Glu Met
1               5                   10                  15
Glu Asn Gln Thr Lys Pro Pro Asp Pro Arg Pro Asp Ala Pro Pro Glu
            20                  25                  30
Tyr Ser Ser His Phe Leu Pro Gly Pro Pro Gly Thr Ala Val Pro Pro
        35                  40                  45
Pro Thr Gly Tyr Pro Gly Gly Leu Pro Met Gly Tyr Tyr Ser Pro Gln
    50                  55                  60
Gln Pro Ser Thr Phe Pro Leu Tyr Gln Pro Val Gly Gly Ile His Pro
65                  70                  75                  80
Val Arg Tyr Gln Pro Gly Lys Tyr Pro Met Pro Asn Gln Ser Val Pro
                85                  90                  95
Ile Thr Trp Met Pro Gly Pro Thr Pro Met Ala Asn Cys Pro Pro Gly
            100                 105                 110
Leu Glu Tyr Leu Val Gln Leu Asp Asn Ile His Val Leu Gln His Phe
        115                 120                 125
Glu Pro Leu Glu Met Met Thr Cys Phe Glu Thr Asn Asn Arg Tyr Asp
    130                 135                 140
Ile Lys Asn Asn Ser Asp Gln Met Val Tyr Val Val Thr Glu Asp Thr
145                 150                 155                 160
Asp Asp Phe Thr Arg Asn Ala Tyr Arg Thr Leu Arg Pro Phe Val Leu
                165                 170                 175
Arg Val Thr Asp Cys Met Gly Arg Glu Ile Met Thr Met Gln Arg Pro
            180                 185                 190
Phe Arg Cys Thr Cys Cys Cys Phe Cys Cys Pro Ser Ala Arg Gln Glu
        195                 200                 205
Leu Glu Val Gln Cys Pro Pro Gly Val Thr Ile Gly Phe Val Ala Glu
    210                 215                 220
His Trp Asn Leu Cys Arg Ala Val Tyr Ser Ile Gln Asn Glu Lys Lys
225                 230                 235                 240
Glu Asn Val Met Arg Val Arg Gly Pro Cys Ser Thr Tyr Gly Cys Gly
                245                 250                 255
Ser Asp Ser Val Phe Glu Val Lys Ser Leu Asp Gly Ile Ser Asn Ile
            260                 265                 270
Gly Ser Ile Ile Arg Lys Trp Asn Gly Leu Leu Ser Ala Met Ala Asp
        275                 280                 285
Ala Asp His Phe Asp Ile His Phe Pro Leu Asp Leu Asp Val Lys Met
    290                 295                 300
Lys Ala Met Ile Phe Gly Ala Cys Phe Leu Ile Asp Phe Met Tyr Phe
305                 310                 315                 320
Glu Arg Ser Pro Pro Gln Arg Ser Arg
                325
```

<210> 298

<211> 323
<212> PRT
<213> Homo sapiens
<400> 298
Met Gly Gly Gln Val Ser Ala Ser Asn Ser Phe Ser Arg Leu His Cys
1 5 10 15
Arg Asn Ala Asn Glu Asp Trp Met Ser Ala Leu Cys Pro Arg Leu Trp
20 25 30
Asp Val Pro Leu His His Leu Ser Ile Pro Gly Ser His Asp Thr Met
35 40 45
Thr Tyr Cys Leu Asn Lys Lys Ser Pro Ile Ser His Glu Glu Ser Arg
50 55 60
Leu Leu Gln Leu Leu Asn Lys Ala Leu Pro Cys Ile Thr Arg Pro Val
65 70 75 80
Val Leu Lys Trp Ser Val Thr Gln Ala Leu Asp Val Thr Glu Gln Leu
85 90 95
Asp Ala Gly Val Arg Tyr Leu Asp Leu Arg Ile Ala His Met Leu Glu
100 105 110
Gly Ser Glu Lys Asn Leu His Phe Val His Met Val Tyr Thr Thr Ala
115 120 125
Leu Val Glu Asp Thr Leu Thr Glu Ile Ser Glu Trp Leu Glu Arg His
130 135 140
Pro Arg Glu Val Val Ile Leu Ala Cys Arg Asn Phe Glu Gly Leu Ser
145 150 155 160
Glu Asp Leu His Glu Tyr Leu Val Ala Cys Ile Lys Asn Ile Phe Gly
165 170 175
Asp Met Leu Cys Pro Arg Gly Glu Val Pro Thr Leu Arg Gln Leu Trp
180 185 190
Ser Arg Gly Gln Gln Val Ile Val Ser Tyr Glu Asp Glu Ser Ser Leu
195 200 205
Arg Arg His His Glu Leu Trp Pro Gly Val Pro Tyr Trp Trp Gly Asn
210 215 220
Arg Val Lys Thr Glu Ala Leu Ile Arg Tyr Leu Glu Thr Met Lys Ser
225 230 235 240
Cys Gly Arg Pro Gly Gly Leu Phe Val Ala Gly Ile Asn Leu Thr Glu
245 250 255
Asn Leu Gln Tyr Val Leu Ala His Pro Ser Glu Ser Leu Glu Lys Met
260 265 270
Thr Leu Pro Asn Leu Pro Arg Leu Ser Ala Trp Val Arg Glu Gln Cys
275 280 285
Pro Gly Pro Gly Ser Arg Cys Thr Asn Ile Ile Ala Gly Asp Phe Ile
290 295 300
Gly Ala Asp Gly Phe Val Ser Asp Val Ile Ala Leu Asn Gln Lys Leu
305 310 315 320
Leu Trp Cys

<210> 299
<211> 103
<212> PRT
<213> Homo sapiens
<400> 299
Met Thr Thr Glu Ile Gly Trp Trp Lys Leu Thr Phe Leu Arg Lys Lys
1 5 10 15
Lys Ser Thr Pro Lys Val Leu Tyr Glu Ile Pro Asp Thr Tyr Ala Gln
20 25 30
Thr Glu Gly Asp Ala Glu Pro Pro Arg Pro Asp Ala Gly Gly Pro Asn
35 40 45
Ser Asp Phe Asn Thr Arg Leu Glu Lys Ile Val Asp Lys Ser Thr Lys
50 55 60
Gly Lys His Val Lys Val Ser Asn Ser Gly Arg Phe Lys Glu Lys Lys
65 70 75 80
Lys Val Arg Ala Thr Leu Ala Glu Asn Pro Asn Leu Phe Asp Asp His
85 90 95
Glu Glu Gly Arg Ser Ser Lys
100

<210> 300
<211> 999

<212> PRT
<213> Homo sapiens
<400> 300

```
Met Gly Val Ala Gly Arg Asn Arg Pro Gly Ala Ala Trp Ala Val Leu
1               5                   10                  15
Leu Leu Leu Leu Leu Leu Pro Pro Leu Leu Leu Ala Gly Ala Val
            20                  25                  30
Pro Pro Gly Arg Gly Arg Ala Ala Gly Pro Gln Glu Asp Val Asp Glu
        35                  40                  45
Cys Ala Gln Gly Leu Asp Asp Cys His Ala Asp Ala Leu Cys Gln Asn
    50                  55                  60
Thr Pro Thr Ser Tyr Lys Cys Ser Cys Lys Pro Gly Tyr Gln Gly Glu
65                  70                  75                  80
Gly Arg Gln Cys Glu Asp Ile Asp Glu Cys Gly Asn Glu Leu Asn Gly
                85                  90                  95
Gly Cys Val His Asp Cys Leu Asn Ile Pro Gly Asn Tyr Arg Cys Thr
            100                 105                 110
Cys Phe Asp Gly Phe Met Leu Ala His Asp Gly His Asn Cys Leu Asp
        115                 120                 125
Val Asp Glu Cys Leu Glu Asn Asn Gly Gly Cys Gln His Thr Cys Val
    130                 135                 140
Asn Val Met Gly Ser Tyr Glu Cys Cys Cys Lys Glu Gly Phe Phe Leu
145                 150                 155                 160
Ser Asp Asn Gln His Thr Cys Ile His Arg Ser Glu Glu Gly Leu Ser
                165                 170                 175
Cys Met Asn Lys Asp His Gly Cys Ser His Ile Cys Lys Glu Ala Pro
            180                 185                 190
Arg Gly Ser Val Ala Cys Glu Cys Arg Pro Gly Phe Glu Leu Ala Lys
        195                 200                 205
Asn Gln Arg Asp Cys Ile Leu Thr Cys Asn His Gly Asn Gly Gly Cys
    210                 215                 220
Gln His Ser Cys Asp Asp Thr Ala Asp Gly Pro Glu Cys Ser Cys His
225                 230                 235                 240
Pro Gln Tyr Lys Met His Thr Asp Gly Arg Ser Cys Leu Glu Arg Glu
                245                 250                 255
Asp Thr Val Leu Glu Val Thr Glu Ser Asn Thr Thr Ser Val Val Asp
            260                 265                 270
Gly Asp Lys Arg Val Lys Arg Arg Leu Leu Met Glu Thr Cys Ala Val
        275                 280                 285
Asn Asn Gly Gly Cys Asp Arg Thr Cys Lys Asp Thr Ser Thr Gly Val
    290                 295                 300
His Cys Ser Cys Pro Val Gly Phe Thr Leu Gln Leu Asp Gly Lys Thr
305                 310                 315                 320
Cys Lys Asp Ile Asp Glu Cys Gln Thr Arg Asn Gly Gly Cys Asp His
                325                 330                 335
Phe Cys Lys Asn Ile Val Gly Ser Phe Asp Cys Gly Cys Lys Lys Gly
            340                 345                 350
Phe Lys Leu Leu Thr Asp Glu Lys Ser Cys Gln Asp Val Asp Glu Cys
        355                 360                 365
Ser Leu Asp Arg Thr Cys Asp His Ser Cys Ile Asn His Pro Gly Thr
    370                 375                 380
Phe Ala Cys Ala Cys Asn Arg Gly Tyr Thr Leu Tyr Gly Phe Thr His
385                 390                 395                 400
Cys Gly Asp Thr Asn Glu Cys Ser Ile Asn Asn Gly Gly Cys Gln Gln
                405                 410                 415
Val Cys Val Asn Thr Val Gly Ser Tyr Glu Cys Gln Cys His Pro Gly
            420                 425                 430
Tyr Lys Leu His Trp Asn Lys Lys Asp Cys Val Glu Val Lys Gly Leu
        435                 440                 445
Leu Pro Thr Ser Val Ser Pro Arg Val Ser Leu His Cys Gly Lys Ser
    450                 455                 460
Gly Gly Gly Asp Gly Cys Phe Leu Arg Cys His Ser Gly Ile His Leu
465                 470                 475                 480
Ser Ser Asp Val Thr Thr Ile Arg Thr Ser Val Thr Phe Lys Leu Asn
                485                 490                 495
Glu Gly Lys Cys Ser Leu Lys Asn Ala Glu Leu Phe Pro Glu Gly Leu
            500                 505                 510
Arg Pro Ala Leu Pro Glu Lys His Ser Ser Val Lys Glu Ser Phe Arg
        515                 520                 525
```

Tyr Val Asn Leu Thr Cys Ser Ser Gly Lys Gln Val Pro Gly Ala Pro
530 535 540
Gly Arg Pro Ser Thr Pro Lys Glu Met Phe Ile Thr Val Glu Phe Glu
545 550 555 560
Leu Glu Thr Asn Gln Lys Glu Val Thr Ala Ser Cys Asp Leu Ser Cys
565 570 575
Ile Val Lys Arg Thr Glu Lys Arg Leu Arg Lys Ala Ile Arg Thr Leu
580 585 590
Arg Lys Ala Val His Arg Glu Gln Phe His Leu Gln Leu Ser Gly Met
595 600 605
Asn Leu Asp Val Ala Lys Lys Pro Pro Arg Thr Ser Glu Arg Gln Ala
610 615 620
Glu Ser Cys Gly Val Gly Gln Gly His Ala Glu Asn Gln Cys Val Ser
625 630 635 640
Cys Arg Ala Gly Thr Tyr Tyr Asp Gly Ala Arg Glu Arg Cys Ile Leu
645 650 655
Cys Pro Asn Gly Thr Phe Gln Asn Glu Glu Gly Gln Met Thr Cys Glu
660 665 670
Pro Cys Pro Arg Pro Gly Asn Ser Gly Ala Leu Lys Thr Pro Glu Ala
675 680 685
Trp Asn Met Ser Glu Cys Gly Gly Leu Cys Gln Pro Gly Glu Tyr Ser
690 695 700
Ala Asp Gly Phe Ala Pro Cys Gln Leu Cys Ala Leu Gly Thr Phe Gln
705 710 715 720
Pro Glu Ala Gly Arg Thr Ser Cys Phe Pro Cys Gly Gly Gly Leu Ala
725 730 735
Thr Lys His Gln Gly Ala Thr Ser Phe Gln Asp Cys Glu Thr Arg Val
740 745 750
Gln Cys Ser Pro Gly His Phe Tyr Asn Thr Thr Thr His Arg Cys Ile
755 760 765
Arg Cys Pro Val Gly Thr Tyr Gln Pro Glu Phe Gly Lys Asn Asn Cys
770 775 780
Val Ser Cys Pro Gly Asn Thr Thr Thr Asp Phe Asp Gly Ser Thr Asn
785 790 795 800
Ile Thr Gln Cys Lys Asn Arg Arg Cys Gly Gly Glu Leu Gly Asp Phe
805 810 815
Thr Gly Tyr Ile Glu Ser Pro Asn Tyr Pro Gly Asn Tyr Pro Ala Asn
820 825 830
Thr Glu Cys Thr Trp Thr Ile Asn Pro Pro Pro Lys Arg Arg Ile Leu
835 840 845
Ile Val Val Pro Glu Ile Phe Leu Pro Ile Glu Asp Asp Cys Gly Asp
850 855 860
Tyr Leu Val Met Arg Lys Thr Ser Ser Ser Asn Ser Val Thr Thr Tyr
865 870 875 880
Glu Thr Cys Gln Thr Tyr Glu Arg Pro Ile Ala Phe Thr Ser Arg Ser
885 890 895
Lys Lys Leu Trp Ile Gln Phe Lys Ser Asn Glu Gly Asn Ser Ala Arg
900 905 910
Gly Phe Gln Val Pro Tyr Val Thr Tyr Asp Glu Asp Tyr Gln Glu Leu
915 920 925
Ile Glu Asp Ile Val Arg Asp Gly Arg Leu Tyr Ala Ser Glu Asn His
930 935 940
Gln Glu Ile Leu Lys Asp Lys Lys Leu Ile Lys Ala Leu Phe Asp Val
945 950 955 960
Leu Ala His Pro Gln Asn Tyr Phe Lys Tyr Thr Ala Gln Glu Ser Arg
965 970 975
Glu Met Phe Pro Arg Ser Phe Ile Arg Leu Leu Arg Ser Lys Val Ser
980 985 990
Arg Phe Leu Arg Pro Tyr Lys
995

<210> 301
<211> 340
<212> PRT
<213> Homo sapiens
<400> 301
Met Cys Ala Gln Tyr Cys Ile Ser Phe Ala Asp Val Glu Lys Ala His
1 5 10 15
Ile Asn Ile Arg Asp Ser Ile His Leu Thr Pro Val Leu Thr Ser Ser

```
                20                  25                  30
Ile Leu Asn Gln Leu Thr Gly Arg Asn Leu Phe Phe Lys Cys Glu Leu
        35                  40                  45
Phe Gln Lys Thr Gly Ser Phe Lys Ile Arg Gly Ala Leu Asn Ala Val
    50                  55                  60
Arg Ser Leu Val Pro Asp Ala Leu Glu Arg Lys Pro Lys Ala Val Val
65                  70                  75                  80
Thr His Ser Ser Gly Asn His Gly Gln Ala Leu Thr Tyr Ala Ala Lys
                85                  90                  95
Leu Glu Gly Ile Pro Ala Tyr Ile Val Val Pro Gln Thr Ala Pro Asp
            100                 105                 110
Cys Lys Lys Leu Ala Ile Gln Ala Tyr Gly Ala Ser Ile Val Tyr Cys
        115                 120                 125
Glu Pro Ser Asp Glu Ser Arg Glu Asn Val Ala Lys Arg Val Thr Glu
    130                 135                 140
Glu Thr Glu Gly Ile Met Val His Pro Asn Gln Glu Pro Ala Val Ile
145                 150                 155                 160
Ala Gly Gln Gly Thr Ile Ala Leu Glu Val Leu Asn Gln Val Pro Leu
                165                 170                 175
Val Asp Ala Leu Val Val Pro Val Gly Gly Gly Gly Met Leu Ala Gly
            180                 185                 190
Ile Ala Ile Thr Val Lys Ala Leu Lys Pro Ser Val Lys Val Tyr Ala
        195                 200                 205
Ala Glu Pro Ser Asn Ala Asp Asp Cys Tyr Gln Ser Lys Leu Lys Gly
    210                 215                 220
Lys Leu Met Pro Asn Leu Tyr Pro Pro Glu Thr Ile Ala Asp Gly Val
225                 230                 235                 240
Lys Ser Ser Ile Gly Leu Asn Thr Trp Pro Ile Ile Arg Asp Leu Val
                245                 250                 255
Asp Asp Ile Phe Thr Val Thr Glu Asp Glu Ile Lys Cys Ala Thr Gln
            260                 265                 270
Leu Val Trp Glu Arg Met Lys Leu Leu Ile Glu Pro Thr Ala Gly Val
        275                 280                 285
Gly Val Ala Ala Val Leu Ser Gln His Phe Gln Thr Val Ser Pro Glu
    290                 295                 300
Val Lys Asn Ile Cys Ile Val Leu Ser Gly Gly Asn Val Asp Leu Thr
305                 310                 315                 320
Ser Ser Ile Thr Trp Val Lys Gln Ala Glu Arg Pro Ala Ser Tyr Gln
                325                 330                 335
Ser Val Ser Val
            340
```

```
<210>  302
<211>  218
<212>  PRT
<213>  Homo sapiens
<400>  302
Met Asn Arg Leu Phe Gly Lys Ala Lys Pro Lys Ala Pro Arg Pro Ser
1               5                   10                  15
Leu Thr Asp Cys Ile Gly Thr Val Asp Ser Arg Ala Glu Ser Ile Asp
            20                  25                  30
Lys Lys Ile Ser Arg Leu Asp Ala Glu Leu Val Lys Tyr Lys Asp Gln
        35                  40                  45
Ile Lys Lys Met Arg Glu Gly Pro Ala Lys Asn Met Val Lys Gln Lys
    50                  55                  60
Ala Leu Arg Val Leu Lys Gln Lys Arg Met Tyr Glu Gln Gln Arg Asp
65                  70                  75                  80
Asn Leu Ala Asn Ser His Ser Thr Trp Thr Gly His Tyr Thr Ile Gln
                85                  90                  95
Ser Leu Lys Asp Thr Lys Thr Thr Val Asp Ala Met Lys Leu Gly Val
            100                 105                 110
Lys Glu Met Lys Lys Ala Tyr Lys Pro Val Lys Ile Asp Gln Ile Glu
        115                 120                 125
Asp Leu Gln Asp Gln Leu Glu Asp Met Met Glu Asp Ala Asn Glu Ile
    130                 135                 140
Gln Glu Ala Leu Ser Arg Ser Tyr Gly Thr Pro Glu Leu Asp Glu Asp
145                 150                 155                 160
Asp Leu Glu Ala Glu Leu Asp Ala Leu Gly Asp Glu Leu Leu Ala Asp
                165                 170                 175
```

Glu Asp Ser Ser Tyr Leu Asp Glu Ala Ala Ser Ala Pro Ala Ile Pro
180 185 190
Glu Gly Val Pro Thr Asp Thr Lys Asn Lys Asp Gly Val Leu Val Asp
195 200 205
Glu Phe Gly Leu Pro Gln Ile Pro Ala Ser
210 215

<210> 303
<211> 635
<212> PRT
<213> Homo sapiens
<400> 303
Met Ala Pro Pro Leu Leu Leu Leu Leu Leu Ala Ser Gly Ala Ala Ala
1 5 10 15
Cys Pro Leu Pro Cys Val Cys Gln Asn Leu Ser Glu Ser Leu Ser Thr
20 25 30
Leu Cys Ala His Arg Gly Leu Leu Phe Val Pro Pro Asn Val Asp Arg
35 40 45
Arg Thr Val Glu Leu Arg Leu Ala Asp Asn Phe Ile Gln Ala Leu Gly
50 55 60
Pro Pro Asp Phe Arg Asn Met Thr Gly Leu Val Asp Leu Thr Leu Ser
65 70 75 80
Arg Asn Ala Ile Thr Arg Ile Gly Ala Arg Ala Phe Gly Asp Leu Glu
85 90 95
Ser Leu Arg Ser Leu His Leu Asp Gly Asn Arg Leu Val Glu Leu Gly
100 105 110
Thr Gly Ser Leu Arg Gly Pro Val Asn Leu Gln His Leu Ile Leu Ser
115 120 125
Gly Asn Gln Leu Gly Arg Ile Ala Pro Gly Ala Phe Asp Asp Phe Leu
130 135 140
Glu Ser Leu Glu Asp Leu Asp Leu Ser Tyr Asn Asn Leu Arg Gln Val
145 150 155 160
Pro Trp Ala Gly Ile Gly Ala Met Pro Ala Leu His Thr Leu Asn Leu
165 170 175
Asp His Asn Leu Ile Asp Ala Leu Pro Pro Gly Ala Phe Ala Gln Leu
180 185 190
Gly Gln Leu Ser Arg Leu Asp Leu Thr Ser Asn Arg Leu Ala Thr Leu
195 200 205
Ala Pro Asp Pro Leu Phe Ser Arg Gly Arg Asp Ala Glu Ala Ser Pro
210 215 220
Ala Pro Leu Val Leu Ser Phe Ser Gly Asn Pro Leu His Cys Asn Cys
225 230 235 240
Glu Leu Leu Trp Leu Arg Arg Leu Ala Arg Pro Asp Asp Leu Glu Thr
245 250 255
Cys Ala Ser Pro Pro Gly Leu Ala Gly Arg Tyr Phe Trp Ala Val Pro
260 265 270
Glu Gly Glu Phe Ser Cys Glu Pro Pro Leu Ile Ala Arg His Thr Gln
275 280 285
Arg Leu Trp Val Leu Glu Gly Gln Arg Ala Thr Leu Arg Cys Arg Ala
290 295 300
Leu Gly Asp Pro Ala Pro Thr Met His Trp Val Gly Pro Asp Asp Arg
305 310 315 320
Leu Val Gly Asn Ser Ser Arg Ala Arg Ala Phe Pro Asn Gly Thr Leu
325 330 335
Glu Ile Gly Val Thr Gly Ala Gly Asp Ala Gly Gly Tyr Thr Cys Ile
340 345 350
Ala Thr Asn Pro Ala Gly Glu Ala Thr Ala Arg Val Glu Leu Arg Val
355 360 365
Leu Ala Leu Pro His Gly Gly Asn Ser Ser Ala Glu Gly Gly Arg Pro
370 375 380
Gly Pro Ser Asp Ile Ala Ala Ser Ala Arg Thr Ala Ala Glu Gly Glu
385 390 395 400
Gly Thr Leu Glu Ser Glu Pro Ala Val Gln Val Thr Glu Val Thr Ala
405 410 415
Thr Ser Gly Leu Val Ser Trp Gly Pro Gly Arg Pro Ala Asp Pro Val
420 425 430
Trp Met Phe Gln Ile Gln Tyr Asn Ser Ser Glu Asp Glu Thr Leu Ile
435 440 445
Tyr Arg Ile Val Pro Ala Ser Ser His His Phe Leu Leu Lys His Leu

450 455 460
Val Pro Gly Ala Asp Tyr Asp Leu Cys Leu Leu Ala Leu Ser Pro Ala
465 470 475 480
Ala Gly Pro Ser Asp Leu Thr Ala Thr Arg Leu Leu Gly Cys Ala His
485 490 495
Phe Ser Thr Leu Pro Ala Ser Pro Leu Cys His Ala Leu Gln Ala His
500 505 510
Val Leu Gly Gly Thr Leu Thr Val Ala Val Gly Gly Val Leu Val Ala
515 520 525
Ala Leu Leu Val Phe Thr Val Ala Leu Leu Val Arg Gly Arg Gly Ala
530 535 540
Gly Asn Gly Arg Leu Pro Leu Lys Leu Ser His Val Gln Ser Gln Thr
545 550 555 560
Asn Gly Gly Pro Ser Pro Thr Pro Lys Ala His Pro Pro Arg Ser Pro
565 570 575
Pro Pro Arg Pro Gln Arg Ser Cys Ser Leu Asp Leu Gly Asp Ala Gly
580 585 590
Cys Tyr Gly Tyr Ala Arg Arg Leu Gly Gly Ala Trp Ala Arg Arg Ser
595 600 605
His Ser Val His Gly Gly Leu Leu Gly Ala Gly Cys Arg Gly Val Gly
610 615 620
Gly Ser Ala Glu Arg Leu Glu Glu Ser Val Val
625 630 635

<210> 304
<211> 498
<212> PRT
<213> Homo sapiens
<400> 304
Met Asp Val Thr Asp His Tyr Glu Asp Val Arg Lys Ile Tyr Asp Asp
1 5 10 15
Phe Leu Lys Asn Ser Asn Met Leu Asp Leu Ile Asp Val Tyr Gln Lys
20 25 30
Cys Arg Ala Leu Thr Ser Asn Cys Glu Asn Tyr Asn Thr Val Ser Pro
35 40 45
Ser Gln Leu Leu Asp Phe Leu Ser Gly Lys Gln Tyr Ala Val Gly Asp
50 55 60
Glu Thr Asp Leu Ser Ile Pro Thr Ser Pro Thr Ser Lys Tyr Asn Arg
65 70 75 80
Asp Asn Glu Lys Val Gln Leu Leu Ala Arg Lys Ile Ile Phe Ser Tyr
85 90 95
Leu Asn Leu Leu Val Asn Ser Lys Asn Asp Leu Ala Val Ala Tyr Ile
100 105 110
Leu Asn Ile Pro Asp Arg Gly Leu Gly Arg Glu Ala Phe Thr Asp Leu
115 120 125
Lys His Ala Ala Arg Glu Lys Gln Met Ser Ile Phe Leu Val Ala Thr
130 135 140
Ser Phe Ile Arg Thr Ile Glu Leu Gly Gly Lys Gly Tyr Ala Pro Pro
145 150 155 160
Pro Ser Asp Pro Leu Arg Thr His Val Lys Gly Leu Ser Asn Phe Ile
165 170 175
Asn Phe Ile Asp Lys Leu Asp Glu Ile Leu Gly Glu Ile Pro Asn Pro
180 185 190
Ser Ile Ala Gly Gly Gln Ile Leu Ser Val Ile Lys Met Gln Leu Ile
195 200 205
Lys Gly Gln Asn Ser Arg Asp Pro Phe Cys Lys Ala Ile Glu Glu Val
210 215 220
Ala Gln Asp Leu Asp Leu Arg Ile Lys Asn Ile Ile Asn Ser Gln Glu
225 230 235 240
Gly Val Val Ala Leu Ser Thr Thr Asp Ile Ser Pro Ala Arg Pro Lys
245 250 255
Ser His Ala Ile Asn His Gly Thr Ala Tyr Cys Gly Arg Asp Thr Val
260 265 270
Lys Ala Leu Leu Val Leu Leu Asp Glu Glu Ala Ala Asn Ala Pro Thr
275 280 285
Lys Asn Lys Ala Glu Leu Leu Tyr Asp Glu Glu Asp Thr Ile His His
290 295 300
His Gly Thr Ser Ile Leu Thr Leu Phe Arg Ser Pro Thr Gln Val Asn
305 310 315 320

Asn Leu Ile Lys Pro Leu Arg Glu Arg Ile Cys Val Ser Met Gln Glu
325 330 335
Lys Lys Ile Lys Met Lys Gln Thr Leu Ile Arg Ser Gln Phe Ala Cys
340 345 350
Thr Tyr Lys Asp Asp Tyr Met Ile Ser Lys Asp Asn Trp Asn Asn Val
355 360 365
Asn Leu Ala Ser Lys Pro Leu Cys Val Leu Tyr Met Glu Asn Asp Leu
370 375 380
Ser Glu Gly Val Asn Pro Ser Val Gly Arg Ser Thr Ile Gly Thr Ser
385 390 395 400
Phe Gly Asn Val His Leu Asp Arg Ser Lys Asn Glu Lys Val Ser Arg
405 410 415
Lys Ser Thr Ser Gln Thr Gly Asn Lys Ser Ser Lys Arg Lys Gln Val
420 425 430
Asp Leu Asp Gly Glu Asn Ile Leu Cys Asp Asn Arg Asn Glu Pro Pro
435 440 445
Gln His Lys Asn Ala Lys Ile Pro Lys Lys Ser Asn Asp Ser Gln Asn
450 455 460
Arg Leu Tyr Gly Lys Leu Ala Lys Val Ala Lys Ser Asn Lys Cys Thr
465 470 475 480
Ala Lys Asp Lys Leu Ile Ser Gly Gln Ala Lys Leu Thr Gln Phe Phe
485 490 495
Arg Leu

<210> 305
<211> 172
<212> PRT
<213> Homo sapiens
<400> 305
Met Arg Asp Ile Ala Ile Leu Lys Glu Lys Gln Glu Lys Glu Ile Gln
1 5 10 15
Thr Leu Gln Glu Glu Thr Lys Lys Val Gln Ala Glu Thr Ala Ser Lys
20 25 30
Thr Arg Glu Val Gln Ala Gln Leu Leu Gln Glu Lys Arg Leu Leu Glu
35 40 45
Lys Gln Leu Ser Glu Pro Asp Arg Arg Leu Leu Gly Lys Arg Lys Arg
50 55 60
Arg Glu Leu Asn Met Lys Ala Gln Ala Leu Lys Leu Ala Ala Lys Arg
65 70 75 80
Phe Ile Phe Glu Tyr Ser Cys Gly Ile Asn Arg Glu Asn Gln Gln Phe
85 90 95
Lys Lys Glu Leu Leu Gln Leu Ile Glu Gln Ala Gln Lys Leu Thr Ala
100 105 110
Thr Gln Ser His Leu Glu Asn Arg Lys Gln Gln Leu Gln Gln Glu Gln
115 120 125
Trp Tyr Leu Glu Ser Leu Ile Gln Ala Arg Gln Arg Leu Gln Gly Ser
130 135 140
His Asn Gln Cys Leu Asn Arg Gln Asp Val Pro Lys Thr Thr Pro Ser
145 150 155 160
Leu Pro Gln Gly Thr Lys Ser Arg Ile Asn Pro Lys
165 170

<210> 306
<211> 330
<212> PRT
<213> Homo sapiens
<400> 306
Met Arg Arg Pro Ser Val Arg Ala Ala Gly Leu Val Leu Cys Thr Leu
1 5 10 15
Cys Tyr Leu Leu Val Gly Ala Ala Val Phe Asp Ala Leu Glu Ser Glu
20 25 30
Ala Glu Ser Gly Arg Gln Arg Leu Leu Val Gln Lys Arg Gly Ala Leu
35 40 45
Arg Arg Lys Phe Gly Phe Ser Ala Glu Asp Tyr Arg Glu Leu Glu Arg
50 55 60
Leu Ala Leu Gln Ala Glu Pro His Arg Ala Gly Arg Gln Trp Lys Phe
65 70 75 80
Pro Gly Ser Phe Tyr Phe Ala Ile Thr Val Ile Thr Thr Ile Gly Tyr

```
                    85                  90                  95
Gly His Ala Ala Pro Gly Thr Asp Ser Gly Lys Val Phe Cys Met Phe
                100                 105                 110
Tyr Ala Leu Leu Gly Ile Pro Leu Thr Leu Val Thr Phe Gln Ser Leu
            115                 120                 125
Gly Glu Arg Leu Asn Ala Val Val Arg Arg Leu Leu Leu Ala Ala Lys
    130                 135                 140
Cys Cys Leu Gly Leu Arg Trp Thr Cys Val Ser Thr Glu Asn Leu Val
145                 150                 155                 160
Val Ala Gly Leu Leu Ala Cys Ala Ala Thr Leu Ala Leu Gly Ala Val
                165                 170                 175
Ala Phe Ser His Phe Glu Gly Trp Thr Phe Phe His Ala Tyr Tyr Tyr
            180                 185                 190
Cys Phe Ile Thr Leu Thr Thr Ile Gly Phe Gly Asp Phe Val Ala Leu
        195                 200                 205
Gln Ser Gly Glu Ala Leu Gln Arg Lys Leu Pro Tyr Val Ala Phe Ser
    210                 215                 220
Phe Leu Tyr Ile Leu Leu Gly Leu Thr Val Ile Gly Ala Phe Leu Asn
225                 230                 235                 240
Leu Val Val Leu Arg Phe Leu Val Ala Ser Ala Asp Trp Pro Glu Arg
                245                 250                 255
Ala Ala Arg Pro Pro Ser Pro Arg Pro Pro Gly Ala Pro Glu Ser Arg
            260                 265                 270
Gly Leu Trp Leu Pro Arg Arg Pro Ala Arg Ser Val Gly Ser Ala Ser
        275                 280                 285
Val Phe Cys His Val His Lys Leu Glu Arg Cys Ala Arg Asp Asn Leu
    290                 295                 300
Gly Phe Ser Pro Pro Ser Ser Pro Gly Val Val Arg Gly Gly Gln Ala
305                 310                 315                 320
Pro Arg Pro Gly Ala Arg Trp Lys Ser Ile
                325                 330

<210>  307
<211>  741
<212>  PRT
<213>  Homo sapiens
<400>  307
Met Glu Ser Arg Asp His Asn Asn Pro Gln Glu Gly Pro Thr Ser Ser
1               5                   10                  15
Ser Gly Arg Arg Ala Ala Val Glu Asp Asn His Leu Leu Ile Lys Ala
            20                  25                  30
Val Gln Asn Glu Asp Val Asp Leu Val Gln Gln Leu Leu Glu Gly Gly
        35                  40                  45
Ala Asn Val Asn Phe Gln Glu Glu Glu Gly Gly Trp Thr Pro Leu His
    50                  55                  60
Asn Ala Val Gln Met Ser Arg Glu Asp Ile Val Glu Leu Leu Leu Arg
65                  70                  75                  80
His Gly Ala Asp Pro Val Leu Arg Lys Lys Asn Gly Ala Thr Pro Phe
                85                  90                  95
Ile Leu Ala Ala Ile Ala Gly Ser Val Lys Leu Leu Lys Leu Phe Leu
            100                 105                 110
Ser Lys Gly Ala Asp Val Asn Glu Cys Asp Phe Tyr Gly Phe Thr Ala
        115                 120                 125
Phe Met Glu Ala Ala Val Tyr Gly Lys Val Lys Ala Leu Lys Phe Leu
    130                 135                 140
Tyr Lys Arg Gly Ala Asn Val Asn Leu Arg Arg Lys Thr Lys Glu Asp
145                 150                 155                 160
Gln Glu Arg Leu Arg Lys Gly Gly Ala Thr Ala Leu Met Asp Ala Ala
                165                 170                 175
Glu Lys Gly His Val Glu Val Leu Lys Ile Leu Leu Asp Glu Met Gly
            180                 185                 190
Ala Asp Val Asn Ala Cys Asp Asn Met Gly Arg Asn Ala Leu Ile His
        195                 200                 205
Ala Leu Leu Ser Ser Asp Asp Ser Asp Val Glu Ala Ile Thr His Leu
    210                 215                 220
Leu Leu Asp His Gly Ala Asp Val Asn Val Arg Gly Glu Arg Gly Lys
225                 230                 235                 240
Thr Pro Leu Ile Leu Ala Val Glu Lys Lys His Leu Gly Leu Val Gln
                245                 250                 255
```

Arg Leu Leu Glu Gln Glu His Ile Glu Ile Asn Asp Thr Asp Ser Asp
260 265 270
Gly Lys Thr Ala Leu Leu Leu Ala Val Glu Leu Lys Leu Lys Lys Ile
275 280 285
Ala Glu Leu Leu Cys Lys Arg Gly Ala Ser Thr Asp Cys Gly Asp Leu
290 295 300
Val Met Thr Ala Arg Arg Asn Tyr Asp His Ser Leu Val Lys Val Leu
305 310 315 320
Leu Ser His Gly Ala Lys Glu Asp Phe His Pro Pro Ala Glu Asp Trp
325 330 335
Lys Pro Gln Ser Ser His Trp Gly Ala Ala Leu Lys Asp Leu His Arg
340 345 350
Ile Tyr Arg Pro Met Ile Gly Lys Leu Lys Phe Phe Ile Asp Glu Lys
355 360 365
Tyr Lys Ile Ala Asp Thr Ser Glu Gly Gly Ile Tyr Leu Gly Phe Tyr
370 375 380
Glu Lys Gln Glu Val Ala Val Lys Thr Phe Cys Glu Gly Ser Pro Arg
385 390 395 400
Ala Gln Arg Glu Val Ser Cys Leu Gln Ser Ser Arg Glu Asn Ser His
405 410 415
Leu Val Thr Phe Tyr Gly Ser Glu Ser His Arg Gly His Leu Phe Val
420 425 430
Cys Val Thr Leu Cys Glu Gln Thr Leu Glu Ala Cys Leu Asp Val His
435 440 445
Arg Gly Glu Asp Val Glu Asn Glu Glu Asp Glu Phe Ala Arg Asn Val
450 455 460
Leu Ser Ser Ile Phe Lys Ala Val Gln Glu Leu His Leu Ser Cys Gly
465 470 475 480
Tyr Thr His Gln Asp Leu Gln Pro Gln Asn Ile Leu Ile Asp Ser Lys
485 490 495
Lys Ala Ala His Leu Ala Asp Phe Asp Lys Ser Ile Lys Trp Ala Gly
500 505 510
Asp Pro Gln Glu Val Lys Arg Asp Leu Glu Asp Leu Gly Arg Leu Val
515 520 525
Leu Tyr Val Val Lys Lys Gly Ser Ile Ser Phe Glu Asp Leu Lys Ala
530 535 540
Gln Ser Asn Glu Glu Val Val Gln Leu Ser Pro Asp Glu Glu Thr Lys
545 550 555 560
Asp Leu Ile His Arg Leu Phe His Pro Gly Glu His Val Arg Asp Cys
565 570 575
Leu Ser Asp Leu Leu Gly His Pro Phe Phe Trp Thr Trp Glu Ser Arg
580 585 590
Tyr Arg Thr Leu Arg Asn Val Gly Asn Glu Ser Asp Ile Lys Thr Arg
595 600 605
Lys Ser Glu Ser Glu Ile Leu Arg Leu Leu Gln Pro Gly Pro Ser Glu
610 615 620
His Ser Lys Ser Phe Asp Lys Trp Thr Thr Lys Ile Asn Glu Cys Val
625 630 635 640
Met Lys Lys Met Asn Lys Phe Tyr Glu Lys Arg Gly Asn Phe Tyr Gln
645 650 655
Asn Thr Val Gly Asp Leu Leu Lys Phe Ile Arg Asn Leu Gly Glu His
660 665 670
Ile Asp Glu Glu Lys His Lys Lys Met Lys Leu Lys Ile Gly Asp Pro
675 680 685
Ser Leu Tyr Phe Gln Lys Thr Phe Pro Asp Leu Val Ile Tyr Val Tyr
690 695 700
Thr Lys Leu Gln Asn Thr Glu Tyr Arg Lys His Phe Pro Gln Thr His
705 710 715 720
Ser Pro Asn Lys Pro Gln Cys Asp Gly Ala Gly Gly Ala Ser Gly Leu
725 730 735
Ala Ser Pro Gly Cys
740

<210> 308
<211> 651
<212> PRT
<213> Homo sapiens
<400> 308
Met Ser Gly Val Arg Ala Val Arg Ile Ser Ile Glu Ser Ala Cys Glu

```
1               5                   10                  15
Lys Gln Val His Glu Val Gly Leu Asp Gly Thr Glu Thr Tyr Leu Pro
            20                  25                  30
Pro Leu Ser Met Ser Gln Asn Leu Ala Arg Leu Ala Gln Arg Ile Asp
        35                  40                  45
Phe Ser Gln Gly Ser Gly Ser Glu Glu Glu Glu Ala Ala Gly Thr Glu
    50                  55                  60
Gly Asp Ala Gln Glu Trp Pro Gly Ala Gly Ser Ser Ala Asp Gln Asp
65                  70                  75                  80
Asp Glu Glu Gly Val Val Lys Phe Gln Pro Ser Leu Trp Pro Trp Asp
                85                  90                  95
Ser Val Arg Asn Asn Leu Arg Ser Ala Leu Thr Glu Met Cys Val Leu
            100                 105                 110
Tyr Asp Val Leu Ser Ile Val Arg Asp Lys Lys Phe Met Thr Leu Asp
        115                 120                 125
Pro Val Ser Gln Asp Ala Leu Pro Pro Lys Gln Asn Pro Gln Thr Leu
    130                 135                 140
Gln Leu Ile Ser Lys Lys Lys Ser Leu Ala Gly Ala Ala Gln Ile Leu
145                 150                 155                 160
Leu Lys Gly Ala Glu Arg Leu Thr Lys Ser Val Thr Glu Asn Gln Glu
                165                 170                 175
Asn Lys Leu Gln Arg Asp Phe Asn Ser Glu Leu Leu Arg Leu Arg Gln
            180                 185                 190
His Trp Lys Leu Arg Lys Val Gly Asp Lys Ile Leu Gly Asp Leu Ser
        195                 200                 205
Tyr Arg Ser Ala Gly Ser Leu Phe Pro His His Gly Thr Phe Glu Val
    210                 215                 220
Ile Lys Asn Thr Asp Leu Asp Leu Asp Lys Lys Ile Pro Glu Asp Tyr
225                 230                 235                 240
Cys Pro Leu Asp Val Gln Ile Pro Ser Asp Leu Glu Gly Ser Ala Tyr
                245                 250                 255
Ile Lys Val Ser Ile Gln Lys Gln Ala Pro Asp Ile Gly Asp Leu Gly
            260                 265                 270
Thr Val Asn Leu Phe Lys Arg Pro Leu Pro Lys Ser Lys Pro Gly Ser
        275                 280                 285
Pro His Trp Gln Thr Lys Leu Glu Ala Ala Gln Asn Val Leu Leu Cys
    290                 295                 300
Lys Glu Ile Phe Ala Gln Leu Ser Arg Glu Ala Val Gln Ile Lys Ser
305                 310                 315                 320
Gln Val Pro His Ile Val Val Lys Asn Gln Ile Ile Ser Gln Pro Phe
                325                 330                 335
Pro Ser Leu Gln Leu Ser Ile Ser Leu Cys His Ser Ser Asn Asp Lys
            340                 345                 350
Lys Ser Gln Lys Phe Ala Thr Glu Lys Gln Cys Pro Glu Asp His Leu
        355                 360                 365
Tyr Val Leu Glu His Asn Leu His Leu Leu Ile Arg Glu Phe His Lys
    370                 375                 380
Gln Thr Leu Ser Ser Ile Met Met Pro His Pro Ala Ser Ala Pro Phe
385                 390                 395                 400
Gly His Lys Arg Met Arg Leu Ser Gly Pro Gln Ala Phe Asp Lys Asn
                405                 410                 415
Glu Ile Asn Ser Leu Gln Ser Ser Glu Gly Leu Leu Glu Lys Ile Ile
            420                 425                 430
Lys Gln Ala Lys His Ile Phe Leu Arg Ser Arg Ala Ala Ala Thr Ile
        435                 440                 445
Asp Ser Leu Ala Ser Arg Ile Glu Asp Pro Gln Ile Gln Ala His Trp
    450                 455                 460
Ser Asn Ile Asn Asp Val Tyr Glu Ser Ser Val Lys Val Leu Ile Thr
465                 470                 475                 480
Ser Gln Gly Tyr Glu Gln Ile Cys Lys Ser Ile Gln Leu Gln Leu Asn
                485                 490                 495
Ile Gly Val Glu Gln Ile Arg Val Val His Arg Asp Gly Arg Val Ile
            500                 505                 510
Thr Leu Ser Tyr Gln Glu Gln Glu Leu Gln Asp Phe Leu Leu Ser Gln
        515                 520                 525
Met Ser Gln His Gln Val His Ala Val Gln Gln Leu Ala Lys Val Met
    530                 535                 540
Gly Trp Gln Val Leu Ser Phe Ser Asn His Val Gly Leu Gly Pro Ile
545                 550                 555                 560
```

Glu Ser Ile Gly Asn Ala Ser Ala Ile Thr Val Ala Ser Pro Ser Gly
565 570 575
Asp Tyr Ala Ile Ser Val Arg Asn Gly Pro Glu Ser Gly Ser Lys Ile
580 585 590
Met Val Gln Phe Pro Arg Asn Gln Cys Lys Asp Leu Pro Lys Ser Asp
595 600 605
Val Leu Gln Asp Asn Lys Trp Ser His Leu Arg Gly Pro Phe Lys Glu
610 615 620
Val Gln Trp Asn Lys Met Glu Gly Arg Asn Phe Val Tyr Lys Met Glu
625 630 635 640
Leu Leu Met Ser Ala Leu Ser Pro Cys Leu Leu
645 650

<210> 309
<211> 1496
<212> PRT
<213> Homo sapiens
<400> 309
Met Met Ala Asn Trp Ala Glu Ala Arg Pro Leu Leu Ile Leu Ile Val
1 5 10 15
Leu Leu Gly Gln Phe Val Ser Ile Lys Ala Gln Glu Glu Asp Glu Asp
20 25 30
Glu Gly Tyr Gly Glu Glu Ile Ala Cys Thr Gln Asn Gly Gln Met Tyr
35 40 45
Leu Asn Arg Asp Ile Trp Lys Pro Ala Pro Cys Gln Ile Cys Val Cys
50 55 60
Asp Asn Gly Ala Ile Leu Cys Asp Lys Ile Glu Cys Gln Asp Val Leu
65 70 75 80
Asp Cys Ala Asp Pro Val Thr Pro Pro Gly Glu Cys Cys Pro Val Cys
85 90 95
Ser Gln Thr Pro Gly Gly Gly Asn Thr Asn Phe Gly Arg Gly Arg Lys
100 105 110
Gly Gln Lys Gly Glu Pro Gly Leu Val Pro Val Val Thr Gly Ile Arg
115 120 125
Gly Arg Pro Gly Pro Ala Gly Pro Pro Gly Ser Gln Gly Pro Arg Gly
130 135 140
Glu Arg Gly Pro Lys Gly Arg Pro Gly Pro Arg Gly Pro Gln Gly Ile
145 150 155 160
Asp Gly Glu Pro Gly Val Pro Gly Gln Pro Gly Ala Pro Gly Pro Pro
165 170 175
Gly His Pro Ser His Pro Gly Pro Asp Gly Leu Ser Arg Pro Phe Ser
180 185 190
Ala Gln Met Ala Gly Leu Asp Glu Lys Ser Gly Leu Gly Ser Gln Val
195 200 205
Gly Leu Met Pro Gly Ser Val Gly Pro Val Gly Pro Arg Gly Pro Gln
210 215 220
Gly Leu Gln Gly Gln Gln Gly Gly Ala Gly Pro Thr Gly Pro Pro Gly
225 230 235 240
Glu Pro Gly Asp Pro Gly Pro Met Gly Pro Ile Gly Ser Arg Gly Pro
245 250 255
Glu Gly Pro Pro Gly Lys Pro Gly Glu Asp Gly Glu Pro Gly Arg Asn
260 265 270
Gly Asn Pro Gly Glu Val Gly Phe Ala Gly Ser Pro Gly Ala Arg Gly
275 280 285
Phe Pro Gly Ala Pro Gly Leu Pro Gly Leu Lys Gly His Arg Gly His
290 295 300
Lys Gly Leu Glu Gly Pro Lys Gly Glu Val Gly Ala Pro Gly Ser Lys
305 310 315 320
Gly Glu Ala Gly Pro Thr Gly Pro Met Gly Ala Met Gly Pro Leu Gly
325 330 335
Pro Arg Gly Met Pro Gly Glu Arg Gly Arg Leu Gly Pro Gln Gly Ala
340 345 350
Pro Gly Gln Arg Gly Ala His Gly Met Pro Gly Lys Pro Gly Pro Met
355 360 365
Gly Pro Leu Gly Ile Pro Gly Ser Ser Gly Phe Pro Gly Asn Pro Gly
370 375 380
Met Lys Gly Glu Ala Gly Pro Thr Gly Ala Arg Gly Pro Glu Gly Pro
385 390 395 400
Gln Gly Gln Arg Gly Glu Thr Gly Pro Pro Gly Pro Val Gly Ser Pro

```
                405                 410                 415
Gly Leu Pro Gly Ala Ile Gly Thr Asp Gly Thr Pro Gly Pro Lys Gly
            420                 425                 430
Pro Thr Gly Ser Pro Gly Thr Ser Gly Pro Pro Gly Ser Ala Gly Pro
        435                 440                 445
Pro Gly Ser Pro Gly Pro Gln Gly Ser Thr Gly Pro Gln Gly Asn Ser
    450                 455                 460
Gly Leu Pro Gly Asp Pro Gly Phe Lys Gly Glu Ala Gly Pro Lys Gly
465                 470                 475                 480
Glu Pro Gly Pro His Gly Ile Gln Gly Pro Ile Gly Pro Pro Gly Glu
                485                 490                 495
Glu Gly Lys Arg Gly Pro Arg Gly Asp Pro Gly Thr Leu Gly Pro Pro
            500                 505                 510
Gly Pro Val Gly Glu Arg Gly Ala Pro Gly Asn Arg Gly Phe Pro Gly
        515                 520                 525
Ser Asp Gly Leu Pro Gly Pro Lys Gly Ala Gln Gly Glu Arg Gly Pro
    530                 535                 540
Val Gly Ser Ser Gly Pro Lys Gly Ser Gln Gly Asp Pro Gly Arg Pro
545                 550                 555                 560
Gly Glu Pro Gly Leu Pro Gly Ala Arg Gly Leu Thr Gly Asn Pro Gly
                565                 570                 575
Val Gln Gly Pro Glu Gly Lys Leu Gly Pro Leu Gly Ala Pro Gly Glu
            580                 585                 590
Asp Gly Arg Pro Gly Pro Pro Gly Ser Ile Gly Ile Lys Gly Gln Pro
        595                 600                 605
Gly Thr Met Gly Leu Pro Gly Pro Lys Gly Ser Asn Gly Asp Pro Gly
    610                 615                 620
Lys Pro Gly Glu Ala Gly Asn Pro Gly Val Pro Gly Gln Arg Gly Ala
625                 630                 635                 640
Pro Gly Lys Asp Gly Lys Val Gly Pro Tyr Gly Pro Pro Gly Pro Pro
                645                 650                 655
Gly Leu Arg Gly Glu Arg Gly Glu Gln Gly Pro Pro Gly Pro Thr Gly
            660                 665                 670
Phe Gln Gly His Pro Gly Pro Pro Gly Pro Pro Gly Glu Gly Gly Lys
        675                 680                 685
Pro Gly Asp Gln Gly Val Pro Gly Gly Pro Gly Ala Val Gly Pro Leu
    690                 695                 700
Gly Pro Arg Gly Glu Arg Gly Asn Pro Gly Glu Arg Gly Glu Pro Gly
705                 710                 715                 720
Ile Thr Gly Leu Pro Gly Glu Lys Gly Met Ala Gly Gly His Gly Pro
                725                 730                 735
Asp Gly Pro Lys Gly Ser Pro Gly Pro Ser Gly Thr Pro Gly Asp Thr
            740                 745                 750
Gly Pro Pro Gly Leu Gln Gly Met Pro Gly Glu Arg Gly Ile Ala Gly
        755                 760                 765
Thr Pro Gly Pro Lys Gly Asp Arg Gly Gly Ile Gly Glu Lys Gly Ala
    770                 775                 780
Glu Gly Thr Ala Gly Asn Asp Gly Ala Gly Gly Leu Pro Gly Pro Leu
785                 790                 795                 800
Gly Pro Pro Gly Pro Ala Gly Leu Leu Gly Glu Lys Gly Glu Pro Gly
                805                 810                 815
Pro Arg Gly Leu Val Gly Pro Pro Gly Ser Arg Gly Asn Pro Gly Ser
            820                 825                 830
Arg Gly Glu Asn Gly Pro Thr Gly Ala Val Gly Phe Ala Gly Pro Gln
        835                 840                 845
Gly Ser Asp Gly Gln Pro Gly Val Lys Gly Glu Pro Gly Glu Pro Gly
    850                 855                 860
Gln Lys Gly Asp Ala Gly Ser Pro Gly Pro Gln Gly Leu Ala Gly Ser
865                 870                 875                 880
Pro Gly Pro His Gly Pro Asn Gly Val Pro Gly Leu Lys Gly Gly Arg
                885                 890                 895
Gly Thr Gln Gly Pro Pro Gly Ala Thr Gly Phe Pro Gly Ser Ala Gly
            900                 905                 910
Arg Val Gly Pro Pro Gly Pro Ala Gly Ala Pro Gly Pro Ala Gly Pro
        915                 920                 925
Leu Gly Glu Pro Gly Lys Glu Gly Pro Pro Gly Pro Arg Gly Asp Pro
    930                 935                 940
Gly Ser His Gly Arg Val Gly Val Arg Gly Pro Ala Gly Pro Pro Gly
945                 950                 955                 960
```

Gly Pro Gly Asp Lys Gly Asp Pro Gly Glu Asp Gly Gln Pro Gly Pro
965 970 975
Asp Gly Pro Pro Gly Pro Ala Gly Thr Thr Gly Gln Arg Gly Ile Val
980 985 990
Gly Met Pro Gly Gln Arg Gly Glu Arg Gly Met Pro Gly Leu Pro Gly
995 1000 1005
Pro Ala Gly Thr Pro Gly Lys Val Gly Pro Thr Gly Ala Thr Gly
1010 1015 1020
Asp Lys Gly Pro Pro Gly Pro Val Gly Pro Pro Gly Ser Asn Gly
1025 1030 1035
Pro Val Gly Glu Pro Gly Pro Glu Gly Pro Ala Gly Asn Asp Gly
1040 1045 1050
Thr Pro Gly Arg Asp Gly Ala Val Gly Glu Arg Gly Asp Arg Gly
1055 1060 1065
Asp Pro Gly Pro Ala Gly Leu Pro Gly Ser Gln Gly Ala Pro Gly
1070 1075 1080
Thr Pro Gly Pro Val Gly Ala Pro Gly Asp Ala Gly Gln Arg Gly
1085 1090 1095
Asp Pro Gly Ser Arg Gly Pro Ile Gly His Leu Gly Arg Ala Gly
1100 1105 1110
Lys Arg Gly Leu Pro Gly Pro Gln Gly Pro Arg Gly Asp Lys Gly
1115 1120 1125
Asp His Gly Asp Arg Gly Asp Arg Gly Gln Lys Gly His Arg Gly
1130 1135 1140
Phe Thr Gly Leu Gln Gly Leu Pro Gly Pro Pro Gly Pro Asn Gly
1145 1150 1155
Glu Gln Gly Ser Ala Gly Ile Pro Gly Pro Phe Gly Pro Arg Gly
1160 1165 1170
Pro Pro Gly Pro Val Gly Pro Ser Gly Lys Glu Gly Asn Pro Gly
1175 1180 1185
Pro Leu Gly Pro Leu Gly Pro Pro Gly Val Arg Gly Ser Val Gly
1190 1195 1200
Glu Ala Gly Pro Glu Gly Pro Pro Gly Glu Pro Gly Pro Pro Gly
1205 1210 1215
Pro Pro Gly Pro Pro Gly His Leu Thr Ala Ala Leu Gly Asp Ile
1220 1225 1230
Met Gly His Tyr Asp Glu Ser Met Pro Asp Pro Leu Pro Glu Phe
1235 1240 1245
Thr Glu Asp Gln Ala Ala Pro Asp Asp Lys Asn Lys Thr Asp Pro
1250 1255 1260
Gly Val His Ala Thr Leu Lys Ser Leu Ser Ser Gln Ile Glu Thr
1265 1270 1275
Met Arg Ser Pro Asp Gly Ser Lys Lys His Pro Ala Arg Thr Cys
1280 1285 1290
Asp Asp Leu Lys Leu Cys His Ser Ala Lys Gln Ser Gly Glu Tyr
1295 1300 1305
Trp Ile Asp Pro Asn Gln Gly Ser Val Glu Asp Ala Ile Lys Val
1310 1315 1320
Tyr Cys Asn Met Glu Thr Gly Glu Thr Cys Ile Ser Ala Asn Pro
1325 1330 1335
Ser Ser Val Pro Arg Lys Thr Trp Trp Ala Ser Lys Ser Pro Asp
1340 1345 1350
Asn Lys Pro Val Trp Tyr Gly Leu Asp Met Asn Arg Gly Ser Gln
1355 1360 1365
Phe Ala Tyr Gly Asp His Gln Ser Pro Asn Thr Ala Ile Thr Gln
1370 1375 1380
Met Thr Phe Leu Arg Leu Leu Ser Lys Glu Ala Ser Gln Asn Ile
1385 1390 1395
Thr Tyr Ile Cys Lys Asn Ser Val Gly Tyr Met Asp Asp Gln Ala
1400 1405 1410
Lys Asn Leu Lys Lys Ala Val Val Leu Lys Gly Ala Asn Asp Leu
1415 1420 1425
Asp Ile Lys Ala Glu Gly Asn Ile Arg Phe Arg Tyr Ile Val Leu
1430 1435 1440
Gln Asp Thr Cys Ser Lys Arg Asn Gly Asn Val Gly Lys Thr Val
1445 1450 1455
Phe Glu Tyr Arg Thr Gln Asn Val Ala Arg Leu Pro Ile Ile Asp
1460 1465 1470
Leu Ala Pro Val Asp Val Gly Gly Thr Asp Gln Glu Phe Gly Val

1475 1480 1485
Glu Ile Gly Pro Val Cys Phe Val
1490 1495

<210> 310
<211> 52
<212> PRT
<213> Homo sapiens
<400> 310
Met Lys Arg Lys Thr Lys Thr Pro Ser Glu Gly Gly Gln Val Leu Ala
1 5 10 15
Glu Gln Arg Gly Ser Arg Ser Cys Gln Arg Leu Thr Ala Arg Lys Ala
20 25 30
Leu Pro Ile Leu Val Phe Thr Ile Glu Thr Ala Thr Ala Cys Thr Asp
35 40 45
His Phe Gly Ser
50

<210> 311
<211> 330
<212> PRT
<213> Homo sapiens
<400> 311
Met Ala Glu Glu Asp Leu Ala Pro Gly Lys Ser Ser Val Ala Val Asn
1 5 10 15
Asn Cys Ile Arg Gln Leu Ser Tyr Cys Lys Asn Asp Ile Arg Asp Thr
20 25 30
Val Gly Ile Trp Gly Glu Gly Lys Asp Met Tyr Leu Ile Leu Glu Asn
35 40 45
Asp Met Leu Ser Leu Val Asp Pro Met Asp Arg Ser Val Leu His Ser
50 55 60
Gln Pro Ile Val Ser Ile Arg Val Trp Gly Val Gly Arg Asp Asn Gly
65 70 75 80
Arg Asp Phe Ala Tyr Val Ala Arg Asp Lys Asp Thr Arg Ile Leu Lys
85 90 95
Cys His Val Phe Arg Cys Asp Thr Pro Ala Lys Ala Ile Ala Thr Ser
100 105 110
Leu His Glu Ile Cys Ser Lys Ile Met Ala Glu Arg Lys Asn Ala Lys
115 120 125
Ala Leu Ala Cys Ser Ser Leu Gln Glu Arg Ala Asn Val Asn Leu Asp
130 135 140
Val Pro Leu Gln Val Asp Phe Pro Thr Pro Lys Thr Glu Leu Val Gln
145 150 155 160
Lys Phe His Val Gln Tyr Leu Gly Met Leu Pro Val Asp Lys Pro Val
165 170 175
Gly Met Asp Ile Leu Asn Ser Ala Ile Glu Asn Leu Met Thr Ser Ser
180 185 190
Asn Lys Glu Asp Trp Leu Ser Val Asn Met Asn Val Ala Asp Ala Thr
195 200 205
Val Thr Val Ile Ser Glu Lys Asn Glu Glu Glu Val Leu Val Glu Cys
210 215 220
Arg Val Arg Phe Leu Ser Phe Met Gly Val Gly Lys Asp Val His Thr
225 230 235 240
Phe Ala Phe Ile Met Asp Thr Gly Asn Gln Arg Phe Glu Cys His Val
245 250 255
Phe Trp Cys Glu Pro Asn Ala Gly Asn Val Ser Glu Ala Val Gln Ala
260 265 270
Ala Cys Met Leu Arg Tyr Gln Lys Cys Leu Val Ala Arg Pro Pro Ser
275 280 285
Gln Lys Val Arg Pro Pro Pro Pro Pro Ala Asp Ser Val Thr Arg Arg
290 295 300
Val Thr Thr Asn Val Lys Arg Gly Val Leu Ser Leu Ile Asp Thr Leu
305 310 315 320
Lys Gln Lys Arg Pro Val Thr Glu Met Pro
325 330

<210> 312
<211> 115
<212> PRT

<213> Homo sapiens
<400> 312
Met Ser Arg His Ser Arg Leu Gln Arg Gln Val Leu Ser Leu Tyr Arg
1 5 10 15
Asp Leu Leu Arg Ala Gly Arg Gly Lys Pro Gly Ala Glu Ala Arg Val
20 25 30
Arg Ala Glu Phe Arg Gln His Ala Gly Leu Pro Arg Ser Asp Val Leu
35 40 45
Arg Ile Glu Tyr Leu Tyr Arg Arg Gly Arg Arg Gln Leu Gln Leu Leu
50 55 60
Arg Ser Gly His Ala Thr Ala Met Gly Ala Phe Val Arg Pro Arg Ala
65 70 75 80
Pro Thr Gly Glu Pro Gly Gly Val Gly Cys Gln Pro Asp Asp Gly Asp
85 90 95
Ser Pro Arg Asn Pro His Asp Ser Thr Gly Ala Pro Glu Thr Arg Pro
100 105 110
Asp Gly Arg
115

<210> 313
<211> 321
<212> PRT
<213> Homo sapiens
<400> 313
Met Lys Glu Asp Cys Leu Pro Ser Ser His Val Pro Ile Ser Asp Ser
1 5 10 15
Lys Ser Ile Gln Lys Ser Glu Leu Leu Gly Leu Leu Lys Thr Tyr Asn
20 25 30
Cys Tyr His Glu Gly Lys Ser Phe Gln Leu Arg His Arg Glu Glu Glu
35 40 45
Gly Thr Leu Ile Ile Glu Gly Leu Leu Asn Ile Ala Trp Gly Leu Arg
50 55 60
Arg Pro Ile Arg Leu Gln Met Gln Asp Asp Arg Glu Gln Val His Leu
65 70 75 80
Pro Ser Thr Ser Trp Met Pro Arg Arg Pro Ser Cys Pro Leu Lys Glu
85 90 95
Pro Ser Pro Gln Asn Gly Asn Ile Thr Ala Gln Gly Pro Ser Ile Gln
100 105 110
Pro Val His Lys Ala Glu Ser Ser Thr Asp Ser Ser Gly Pro Leu Glu
115 120 125
Glu Ala Glu Glu Ala Pro Gln Leu Met Arg Thr Lys Ser Asp Ala Ser
130 135 140
Cys Met Ser Gln Arg Arg Pro Lys Cys Arg Ala Pro Gly Glu Ala Gln
145 150 155 160
Arg Ile Arg Arg His Arg Phe Ser Ile Asn Gly His Phe Tyr Asn His
165 170 175
Lys Thr Ser Val Phe Thr Pro Ala Tyr Gly Ser Val Thr Asn Val Arg
180 185 190
Val Asn Ser Thr Met Thr Thr Leu Gln Val Leu Thr Leu Leu Leu Asn
195 200 205
Lys Phe Arg Val Glu Asp Gly Pro Ser Glu Phe Ala Leu Tyr Ile Val
210 215 220
His Glu Ser Gly Glu Arg Thr Lys Leu Lys Asp Cys Glu Tyr Pro Leu
225 230 235 240
Ile Ser Arg Ile Leu His Gly Pro Cys Glu Lys Ile Ala Arg Ile Phe
245 250 255
Leu Met Glu Ala Asp Leu Gly Val Glu Val Pro His Glu Val Ala Gln
260 265 270
Tyr Ile Lys Phe Glu Met Pro Val Leu Asp Ser Phe Val Glu Lys Leu
275 280 285
Lys Glu Glu Glu Glu Arg Glu Ile Ile Lys Leu Thr Met Lys Phe Gln
290 295 300
Ala Leu Arg Leu Thr Met Leu Gln Arg Leu Glu Gln Leu Val Glu Ala
305 310 315 320
Lys

<210> 314
<211> 490

<212> PRT
<213> Homo sapiens
<400> 314

Met Trp Pro Gln Asp Pro Ser Arg Lys Glu Val Leu Arg Phe Ala Val
1 5 10 15
Ser Cys Arg Ile Leu Thr Leu Met Leu Gln Ala Leu Phe Asn Ala Ile
20 25 30
Ile Pro Asp His His Ala Glu Ala Phe Ser Pro Pro Arg Leu Ala Pro
35 40 45
Ser Gly Phe Val Asp Gln Leu Val Glu Gly Leu Leu Gly Gly Leu Ser
50 55 60
His Trp Asp Ala Glu His Phe Leu Phe Ile Ala Glu His Gly Tyr Leu
65 70 75 80
Tyr Glu His Asn Phe Ala Phe Phe Pro Gly Phe Pro Leu Ala Leu Leu
85 90 95
Val Gly Thr Glu Leu Leu Arg Pro Leu Arg Gly Leu Leu Ser Leu Arg
100 105 110
Ser Cys Leu Leu Ile Ser Val Ala Ser Leu Asn Phe Leu Phe Phe Met
115 120 125
Leu Ala Ala Val Ala Leu His Asp Leu Gly Cys Leu Val Leu His Cys
130 135 140
Pro His Gln Ser Phe Tyr Ala Ala Leu Leu Phe Cys Leu Ser Pro Ala
145 150 155 160
Asn Val Phe Leu Ala Ala Gly Tyr Ser Glu Ala Leu Phe Ala Leu Leu
165 170 175
Thr Phe Ser Ala Met Gly Gln Leu Glu Arg Gly Arg Val Trp Thr Ser
180 185 190
Val Leu Leu Phe Ala Phe Ala Thr Gly Val Arg Ser Asn Gly Leu Val
195 200 205
Ser Val Gly Phe Leu Met His Ser Gln Cys Gln Gly Phe Phe Ser Ser
210 215 220
Leu Thr Met Leu Asn Pro Leu Arg Gln Leu Phe Lys Leu Met Ala Ser
225 230 235 240
Leu Phe Leu Ser Val Phe Thr Leu Gly Leu Pro Phe Ala Leu Phe Gln
245 250 255
Tyr Tyr Ala Tyr Thr Gln Phe Cys Leu Pro Gly Ser Ala Arg Pro Ile
260 265 270
Pro Glu Pro Leu Val Gln Leu Ala Val Asp Lys Gly Tyr Arg Ile Ala
275 280 285
Glu Gly Asn Glu Pro Pro Trp Cys Phe Trp Asp Val Pro Leu Ile Tyr
290 295 300
Ser Tyr Ile Gln Asp Val Tyr Trp Asn Val Gly Phe Leu Lys Tyr Tyr
305 310 315 320
Glu Leu Lys Gln Val Pro Asn Phe Leu Leu Ala Ala Pro Val Ala Ile
325 330 335
Leu Val Ala Trp Ala Thr Trp Thr Tyr Val Thr Thr His Pro Trp Leu
340 345 350
Cys Leu Thr Leu Gly Leu Gln Arg Ser Lys Asn Asn Lys Thr Leu Glu
355 360 365
Lys Pro Asp Leu Gly Phe Leu Ser Pro Gln Val Phe Val Tyr Val Val
370 375 380
His Ala Ala Val Leu Leu Leu Phe Gly Gly Leu Cys Met His Val Gln
385 390 395 400
Val Leu Thr Arg Phe Leu Gly Ser Ser Thr Pro Ile Met Tyr Trp Phe
405 410 415
Pro Ala His Leu Leu Gln Asp Gln Glu Pro Leu Leu Arg Ser Leu Lys
420 425 430
Thr Val Pro Trp Lys Pro Leu Ala Glu Asp Ser Pro Pro Gly Gln Lys
435 440 445
Val Pro Arg Asn Pro Ile Met Gly Leu Leu Tyr His Trp Lys Thr Cys
450 455 460
Ser Pro Val Thr Arg Tyr Ile Leu Gly Tyr Phe Leu Thr Tyr Trp Leu
465 470 475 480
Leu Gly Leu Leu Leu His Cys Asn Phe Leu
485 490

<210> 315
<211> 688
<212> PRT

<213> Homo sapiens
<400> 315

```
Met Ser Ser Arg Thr Val Leu Ala Pro Gly Asn Asp Arg Asn Ser Asp
1               5                   10                  15
Thr His Gly Thr Leu Gly Ser Gly Arg Ser Ser Asp Lys Gly Pro Ser
            20                  25                  30
Trp Ser Ser Arg Ser Leu Gly Ala Arg Cys Arg Asn Ser Ile Ala Ser
        35                  40                  45
Cys Pro Glu Glu Gln Pro His Val Gly Asn Tyr Arg Leu Leu Arg Thr
    50                  55                  60
Ile Gly Lys Gly Asn Phe Ala Lys Val Lys Leu Ala Arg His Ile Leu
65                  70                  75                  80
Thr Gly Arg Glu Val Ala Ile Lys Ile Ile Asp Lys Thr Gln Leu Asn
                85                  90                  95
Pro Ser Ser Leu Gln Lys Leu Phe Arg Glu Val Arg Ile Met Lys Gly
            100                 105                 110
Leu Asn His Pro Asn Ile Val Lys Leu Phe Glu Val Ile Glu Thr Glu
        115                 120                 125
Lys Thr Leu Tyr Leu Val Met Glu Tyr Ala Ser Ala Gly Glu Val Phe
    130                 135                 140
Asp Tyr Leu Val Ser His Gly Arg Met Lys Glu Lys Glu Ala Arg Ala
145                 150                 155                 160
Lys Phe Arg Gln Ile Val Ser Ala Val His Tyr Cys His Gln Lys Asn
                165                 170                 175
Ile Val His Arg Asp Leu Lys Ala Glu Asn Leu Leu Leu Asp Ala Glu
            180                 185                 190
Ala Asn Ile Lys Ile Ala Asp Phe Gly Phe Ser Asn Glu Phe Thr Leu
        195                 200                 205
Gly Ser Lys Leu Asp Thr Phe Cys Gly Ser Pro Pro Tyr Ala Ala Pro
    210                 215                 220
Glu Leu Phe Gln Gly Lys Lys Tyr Asp Gly Pro Glu Val Asp Ile Trp
225                 230                 235                 240
Ser Leu Gly Val Ile Leu Tyr Thr Leu Val Ser Gly Ser Leu Pro Phe
                245                 250                 255
Asp Gly His Asn Leu Lys Glu Leu Arg Glu Arg Val Leu Arg Gly Lys
            260                 265                 270
Tyr Arg Val Pro Phe Tyr Met Ser Thr Asp Cys Glu Ser Ile Leu Arg
        275                 280                 285
Arg Phe Leu Val Leu Asn Pro Ala Lys Arg Cys Thr Leu Glu Gln Ile
    290                 295                 300
Met Lys Asp Lys Trp Ile Asn Ile Gly Tyr Glu Gly Glu Glu Leu Lys
305                 310                 315                 320
Pro Tyr Thr Glu Pro Glu Glu Asp Phe Gly Asp Thr Lys Arg Ile Glu
                325                 330                 335
Val Met Val Gly Met Gly Tyr Thr Arg Glu Glu Ile Lys Glu Ser Leu
            340                 345                 350
Thr Ser Gln Lys Tyr Asn Glu Val Thr Ala Thr Tyr Leu Leu Leu Gly
        355                 360                 365
Arg Lys Thr Glu Glu Gly Gly Asp Arg Gly Ala Pro Gly Leu Ala Leu
    370                 375                 380
Ala Arg Val Arg Ala Pro Ser Asp Thr Thr Asn Gly Thr Ser Ser Ser
385                 390                 395                 400
Lys Gly Thr Ser His Ser Lys Gly Gln Arg Ser Ser Ser Ser Thr Tyr
                405                 410                 415
His Arg Gln Arg Arg His Ser Asp Phe Cys Gly Pro Ser Pro Ala Pro
            420                 425                 430
Leu His Pro Lys Arg Ser Pro Thr Ser Thr Gly Glu Ala Glu Leu Lys
        435                 440                 445
Glu Glu Arg Leu Pro Gly Arg Lys Ala Ser Cys Ser Thr Ala Gly Ser
    450                 455                 460
Gly Ser Arg Gly Leu Pro Pro Ser Ser Pro Met Val Ser Ser Ala His
465                 470                 475                 480
Asn Pro Asn Lys Ala Glu Ile Pro Glu Arg Arg Lys Asp Ser Thr Ser
                485                 490                 495
Thr Pro Asn Asn Leu Pro Pro Ser Met Met Thr Arg Arg Asn Thr Tyr
            500                 505                 510
Val Cys Thr Glu Arg Pro Gly Ala Glu Arg Pro Ser Leu Leu Pro Asn
        515                 520                 525
Gly Lys Glu Asn Ser Ser Gly Thr Pro Arg Val Pro Pro Ala Ser Pro
```

530 535 540
Ser Ser His Ser Leu Ala Pro Pro Ser Gly Glu Arg Ser Arg Leu Ala
545 550 555 560
Arg Gly Ser Thr Ile Arg Ser Thr Phe His Gly Gly Gln Val Arg Asp
565 570 575
Arg Arg Ala Gly Gly Gly Gly Gly Gly Gly Val Gln Asn Gly Pro Pro
580 585 590
Ala Ser Pro Thr Leu Ala His Glu Ala Ala Pro Leu Pro Ala Gly Arg
595 600 605
Pro Arg Pro Thr Thr Asn Leu Phe Thr Lys Leu Thr Ser Lys Leu Thr
610 615 620
Arg Arg Val Thr Leu Asp Pro Ser Lys Arg Gln Asn Ser Asn Arg Cys
625 630 635 640
Val Ser Gly Ala Ser Leu Pro Gln Gly Ser Lys Ile Arg Ser Gln Thr
645 650 655
Asn Leu Arg Glu Ser Gly Asp Leu Arg Ser Gln Val Ala Ile Tyr Leu
660 665 670
Gly Ile Lys Arg Lys Pro Pro Pro Gly Cys Ser Asp Ser Pro Gly Val
675 680 685

<210> 316
<211> 338
<212> PRT
<213> Homo sapiens
<400> 316
Met Ser Leu Ser Ala Phe Thr Leu Phe Leu Ala Leu Ile Gly Gly Thr
1 5 10 15
Ser Gly Gln Tyr Tyr Asp Tyr Asp Phe Pro Leu Ser Ile Tyr Gly Gln
20 25 30
Ser Ser Pro Asn Cys Ala Pro Glu Cys Asn Cys Pro Glu Ser Tyr Pro
35 40 45
Ser Ala Met Tyr Cys Asp Glu Leu Lys Leu Lys Ser Val Pro Met Val
50 55 60
Pro Pro Gly Ile Lys Tyr Leu Tyr Leu Arg Asn Asn Gln Ile Asp His
65 70 75 80
Ile Asp Glu Lys Ala Phe Glu Asn Val Thr Asp Leu Gln Trp Leu Ile
85 90 95
Leu Asp His Asn Leu Leu Glu Asn Ser Lys Ile Lys Gly Arg Val Phe
100 105 110
Ser Lys Leu Lys Gln Leu Lys Lys Leu His Ile Asn His Asn Asn Leu
115 120 125
Thr Glu Ser Val Gly Pro Leu Pro Lys Ser Leu Glu Asp Leu Gln Leu
130 135 140
Thr His Asn Lys Ile Thr Lys Leu Gly Ser Phe Glu Gly Leu Val Asn
145 150 155 160
Leu Thr Phe Ile His Leu Gln His Asn Arg Leu Lys Glu Asp Ala Val
165 170 175
Ser Ala Ala Phe Lys Gly Leu Lys Ser Leu Glu Tyr Leu Asp Leu Ser
180 185 190
Phe Asn Gln Ile Ala Arg Leu Pro Ser Gly Leu Pro Val Ser Leu Leu
195 200 205
Thr Leu Tyr Leu Asp Asn Asn Lys Ile Ser Asn Ile Pro Asp Glu Tyr
210 215 220
Phe Lys Arg Phe Asn Ala Leu Gln Tyr Leu Arg Leu Ser His Asn Glu
225 230 235 240
Leu Ala Asp Ser Gly Ile Pro Gly Asn Ser Phe Asn Val Ser Ser Leu
245 250 255
Val Glu Leu Asp Leu Ser Tyr Asn Lys Leu Lys Asn Ile Pro Thr Val
260 265 270
Asn Glu Asn Leu Glu Asn Tyr Tyr Leu Glu Val Asn Gln Leu Glu Lys
275 280 285
Phe Asp Ile Lys Ser Phe Cys Lys Ile Leu Gly Pro Leu Ser Tyr Ser
290 295 300
Lys Ile Lys His Leu Arg Leu Asp Gly Asn Arg Ile Ser Glu Thr Ser
305 310 315 320
Leu Pro Pro Asp Met Tyr Glu Cys Leu Arg Val Ala Asn Glu Val Thr
325 330 335
Leu Asn

<210> 317
<211> 1466
<212> PRT
<213> Homo sapiens
<400> 317

```
Met Met Ser Phe Val Gln Lys Gly Ser Trp Leu Leu Leu Ala Leu Leu
1               5                   10                  15
His Pro Thr Ile Ile Leu Ala Gln Gln Glu Ala Val Glu Gly Gly Cys
            20                  25                  30
Ser His Leu Gly Gln Ser Tyr Ala Asp Arg Asp Val Trp Lys Pro Glu
        35                  40                  45
Pro Cys Gln Ile Cys Val Cys Asp Ser Gly Ser Val Leu Cys Asp Asp
    50                  55                  60
Ile Ile Cys Asp Asp Gln Glu Leu Asp Cys Pro Asn Pro Glu Ile Pro
65                  70                  75                  80
Phe Gly Glu Cys Cys Ala Val Cys Pro Gln Pro Pro Thr Ala Pro Thr
                85                  90                  95
Arg Pro Pro Asn Gly Gln Gly Pro Gln Gly Pro Lys Gly Asp Pro Gly
            100                 105                 110
Pro Pro Gly Ile Pro Gly Arg Asn Gly Asp Pro Gly Ile Pro Gly Gln
        115                 120                 125
Pro Gly Ser Pro Gly Ser Pro Gly Pro Pro Gly Ile Cys Glu Ser Cys
    130                 135                 140
Pro Thr Gly Pro Gln Asn Tyr Ser Pro Gln Tyr Asp Ser Tyr Asp Val
145                 150                 155                 160
Lys Ser Gly Val Ala Val Gly Gly Leu Ala Gly Tyr Pro Gly Pro Ala
                165                 170                 175
Gly Pro Pro Gly Pro Pro Gly Pro Pro Gly Thr Ser Gly His Pro Gly
            180                 185                 190
Ser Pro Gly Ser Pro Gly Tyr Gln Gly Pro Pro Gly Glu Pro Gly Gln
        195                 200                 205
Ala Gly Pro Ser Gly Pro Pro Gly Pro Pro Gly Ala Ile Gly Pro Ser
    210                 215                 220
Gly Pro Ala Gly Lys Asp Gly Glu Ser Gly Arg Pro Gly Arg Pro Gly
225                 230                 235                 240
Glu Arg Gly Leu Pro Gly Pro Pro Gly Ile Lys Gly Pro Ala Gly Ile
                245                 250                 255
Pro Gly Phe Pro Gly Met Lys Gly His Arg Gly Phe Asp Gly Arg Asn
            260                 265                 270
Gly Glu Lys Gly Glu Thr Gly Ala Pro Gly Leu Lys Gly Glu Asn Gly
        275                 280                 285
Leu Pro Gly Glu Asn Gly Ala Pro Gly Pro Met Gly Pro Arg Gly Ala
    290                 295                 300
Pro Gly Glu Arg Gly Arg Pro Gly Leu Pro Gly Ala Ala Gly Ala Arg
305                 310                 315                 320
Gly Asn Asp Gly Ala Arg Gly Ser Asp Gly Gln Pro Gly Pro Pro Gly
                325                 330                 335
Pro Pro Gly Thr Ala Gly Phe Pro Gly Ser Pro Gly Ala Lys Gly Glu
            340                 345                 350
Val Gly Pro Ala Gly Ser Pro Gly Ser Asn Gly Ala Pro Gly Gln Arg
        355                 360                 365
Gly Glu Pro Gly Pro Gln Gly His Ala Gly Ala Gln Gly Pro Pro Gly
    370                 375                 380
Pro Pro Gly Ile Asn Gly Ser Pro Gly Gly Lys Gly Glu Met Gly Pro
385                 390                 395                 400
Ala Gly Ile Pro Gly Ala Pro Gly Leu Met Gly Ala Arg Gly Pro Pro
                405                 410                 415
Gly Pro Ala Gly Ala Asn Gly Ala Pro Gly Leu Arg Gly Gly Ala Gly
            420                 425                 430
Glu Pro Gly Lys Asn Gly Ala Lys Gly Glu Pro Gly Pro Arg Gly Glu
        435                 440                 445
Arg Gly Glu Ala Gly Ile Pro Gly Val Pro Gly Ala Lys Gly Glu Asp
    450                 455                 460
Gly Lys Asp Gly Ser Pro Gly Glu Pro Gly Ala Asn Gly Leu Pro Gly
465                 470                 475                 480
Ala Ala Gly Glu Arg Gly Ala Pro Gly Phe Arg Gly Pro Ala Gly Pro
                485                 490                 495
Asn Gly Ile Pro Gly Glu Lys Gly Pro Ala Gly Glu Arg Gly Ala Pro
```

```
                    500                          505                          510
Gly Pro Ala Gly Pro Arg Gly Ala Ala Gly Glu Pro Gly Arg Asp Gly
                515                          520                          525
Val Pro Gly Gly Pro Gly Met Arg Gly Met Pro Gly Ser Pro Gly Gly
        530                          535                          540
Pro Gly Ser Asp Gly Lys Pro Gly Pro Pro Gly Ser Gln Gly Glu Ser
545                          550                          555                          560
Gly Arg Pro Gly Pro Pro Gly Pro Ser Gly Pro Arg Gly Gln Pro Gly
                        565                          570                          575
Val Met Gly Phe Pro Gly Pro Lys Gly Asn Asp Gly Ala Pro Gly Lys
                    580                          585                          590
Asn Gly Glu Arg Gly Gly Pro Gly Gly Pro Gly Pro Gln Gly Pro Pro
                595                          600                          605
Gly Lys Asn Gly Glu Thr Gly Pro Gln Gly Pro Pro Gly Pro Thr Gly
        610                          615                          620
Pro Gly Gly Asp Lys Gly Asp Thr Gly Pro Pro Gly Pro Gln Gly Leu
625                          630                          635                          640
Gln Gly Leu Pro Gly Thr Gly Gly Pro Pro Gly Glu Asn Gly Lys Pro
                        645                          650                          655
Gly Glu Pro Gly Pro Lys Gly Asp Ala Gly Ala Pro Gly Ala Pro Gly
                    660                          665                          670
Gly Lys Gly Asp Ala Gly Ala Pro Gly Glu Arg Gly Pro Pro Gly Leu
                675                          680                          685
Ala Gly Ala Pro Gly Leu Arg Gly Gly Ala Gly Pro Pro Gly Pro Glu
        690                          695                          700
Gly Gly Lys Gly Ala Ala Gly Pro Pro Gly Pro Pro Gly Ala Ala Gly
705                          710                          715                          720
Thr Pro Gly Leu Gln Gly Met Pro Gly Glu Arg Gly Gly Leu Gly Ser
                        725                          730                          735
Pro Gly Pro Lys Gly Asp Lys Gly Glu Pro Gly Gly Pro Gly Ala Asp
                    740                          745                          750
Gly Val Pro Gly Lys Asp Gly Pro Arg Gly Pro Thr Gly Pro Ile Gly
                755                          760                          765
Pro Pro Gly Pro Ala Gly Gln Pro Gly Asp Lys Gly Glu Gly Gly Ala
        770                          775                          780
Pro Gly Leu Pro Gly Ile Ala Gly Pro Arg Gly Ser Pro Gly Glu Arg
785                          790                          795                          800
Gly Glu Thr Gly Pro Pro Gly Pro Ala Gly Phe Pro Gly Ala Pro Gly
                        805                          810                          815
Gln Asn Gly Glu Pro Gly Gly Lys Gly Glu Arg Gly Ala Pro Gly Glu
                    820                          825                          830
Lys Gly Glu Gly Gly Pro Pro Gly Val Ala Gly Pro Pro Gly Gly Ser
                835                          840                          845
Gly Pro Ala Gly Pro Pro Gly Pro Gln Gly Val Lys Gly Glu Arg Gly
        850                          855                          860
Ser Pro Gly Gly Pro Gly Ala Ala Gly Phe Pro Gly Ala Arg Gly Leu
865                          870                          875                          880
Pro Gly Pro Pro Gly Ser Asn Gly Asn Pro Gly Pro Pro Gly Pro Ser
                        885                          890                          895
Gly Ser Pro Gly Lys Asp Gly Pro Pro Gly Pro Ala Gly Asn Thr Gly
                    900                          905                          910
Ala Pro Gly Ser Pro Gly Val Ser Gly Pro Lys Gly Asp Ala Gly Gln
                915                          920                          925
Pro Gly Glu Lys Gly Ser Pro Gly Ala Gln Gly Pro Pro Gly Ala Pro
        930                          935                          940
Gly Pro Leu Gly Ile Ala Gly Ile Thr Gly Ala Arg Gly Leu Ala Gly
945                          950                          955                          960
Pro Pro Gly Met Pro Gly Pro Arg Gly Ser Pro Gly Pro Gln Gly Val
                        965                          970                          975
Lys Gly Glu Ser Gly Lys Pro Gly Ala Asn Gly Leu Ser Gly Glu Arg
                    980                          985                          990
Gly Pro Pro Gly Pro Gln Gly Leu  Pro Gly Leu Ala Gly  Thr Ala Gly
                995                          1000                         1005
Glu Pro  Gly Arg Asp Gly Asn  Pro Gly Ser Asp Gly  Leu Pro Gly
        1010                         1015                         1020
Arg Asp  Gly Ser Pro Gly Gly  Lys Gly Asp Arg Gly  Glu Asn Gly
        1025                         1030                         1035
Ser Pro  Gly Ala Pro Gly Ala  Pro Gly His Pro Gly  Pro Pro Gly
        1040                         1045                         1050
```

Pro Val Gly Pro Ala Gly Lys Ser Gly Asp Arg Gly Glu Ser Gly
1055 1060 1065
Pro Ala Gly Pro Ala Gly Ala Pro Gly Pro Ala Gly Ser Arg Gly
1070 1075 1080
Ala Pro Gly Pro Gln Gly Pro Arg Gly Asp Lys Gly Glu Thr Gly
1085 1090 1095
Glu Arg Gly Ala Ala Gly Ile Lys Gly His Arg Gly Phe Pro Gly
1100 1105 1110
Asn Pro Gly Ala Pro Gly Ser Pro Gly Pro Ala Gly Gln Gln Gly
1115 1120 1125
Ala Ile Gly Ser Pro Gly Pro Ala Gly Pro Arg Gly Pro Val Gly
1130 1135 1140
Pro Ser Gly Pro Pro Gly Lys Asp Gly Thr Ser Gly His Pro Gly
1145 1150 1155
Pro Ile Gly Pro Pro Gly Pro Arg Gly Asn Arg Gly Glu Arg Gly
1160 1165 1170
Ser Glu Gly Ser Pro Gly His Pro Gly Gln Pro Gly Pro Pro Gly
1175 1180 1185
Pro Pro Gly Ala Pro Gly Pro Cys Cys Gly Gly Val Gly Ala Ala
1190 1195 1200
Ala Ile Ala Gly Ile Gly Gly Glu Lys Ala Gly Gly Phe Ala Pro
1205 1210 1215
Tyr Tyr Gly Asp Glu Pro Met Asp Phe Lys Ile Asn Thr Asp Glu
1220 1225 1230
Ile Met Thr Ser Leu Lys Ser Val Asn Gly Gln Ile Glu Ser Leu
1235 1240 1245
Ile Ser Pro Asp Gly Ser Arg Lys Asn Pro Ala Arg Asn Cys Arg
1250 1255 1260
Asp Leu Lys Phe Cys His Pro Glu Leu Lys Ser Gly Glu Tyr Trp
1265 1270 1275
Val Asp Pro Asn Gln Gly Cys Lys Leu Asp Ala Ile Lys Val Phe
1280 1285 1290
Cys Asn Met Glu Thr Gly Glu Thr Cys Ile Ser Ala Asn Pro Leu
1295 1300 1305
Asn Val Pro Arg Lys His Trp Trp Thr Asp Ser Ser Ala Glu Lys
1310 1315 1320
Lys His Val Trp Phe Gly Glu Ser Met Asp Gly Gly Phe Gln Phe
1325 1330 1335
Ser Tyr Gly Asn Pro Glu Leu Pro Glu Asp Val Leu Asp Val Gln
1340 1345 1350
Leu Ala Phe Leu Arg Leu Leu Ser Ser Arg Ala Ser Gln Asn Ile
1355 1360 1365
Thr Tyr His Cys Lys Asn Ser Ile Ala Tyr Met Asp Gln Ala Ser
1370 1375 1380
Gly Asn Val Lys Lys Ala Leu Lys Leu Met Gly Ser Asn Glu Gly
1385 1390 1395
Glu Phe Lys Ala Glu Gly Asn Ser Lys Phe Thr Tyr Thr Val Leu
1400 1405 1410
Glu Asp Gly Cys Thr Lys His Thr Gly Glu Trp Ser Lys Thr Val
1415 1420 1425
Phe Glu Tyr Arg Thr Arg Lys Ala Val Arg Leu Pro Ile Val Asp
1430 1435 1440
Ile Ala Pro Tyr Asp Ile Gly Gly Pro Asp Gln Glu Phe Gly Val
1445 1450 1455
Asp Val Gly Pro Val Cys Phe Leu
1460 1465

<210> 318
<211> 1464
<212> PRT
<213> Homo sapiens
<400> 318
Met Phe Ser Phe Val Asp Leu Arg Leu Leu Leu Leu Leu Ala Ala Thr
1 5 10 15
Ala Leu Leu Thr His Gly Gln Glu Glu Gly Gln Val Glu Gly Gln Asp
20 25 30
Glu Asp Ile Pro Pro Ile Thr Cys Val Gln Asn Gly Leu Arg Tyr His
35 40 45
Asp Arg Asp Val Trp Lys Pro Glu Pro Cys Arg Ile Cys Val Cys Asp

```
    50                  55                  60
Asn Gly Lys Val Leu Cys Asp Asp Val Ile Cys Asp Glu Thr Lys Asn
65                  70                  75                  80
Cys Pro Gly Ala Glu Val Pro Glu Gly Glu Cys Cys Pro Val Cys Pro
                85                  90                  95
Asp Gly Ser Glu Ser Pro Thr Asp Gln Glu Thr Thr Gly Val Glu Gly
            100                 105                 110
Pro Lys Gly Asp Thr Gly Pro Arg Gly Pro Arg Gly Pro Ala Gly Pro
        115                 120                 125
Pro Gly Arg Asp Gly Ile Pro Gly Gln Pro Gly Leu Pro Gly Pro Pro
    130                 135                 140
Gly Pro Pro Gly Pro Pro Gly Pro Pro Gly Leu Gly Gly Asn Phe Ala
145                 150                 155                 160
Pro Gln Leu Ser Tyr Gly Tyr Asp Glu Lys Ser Thr Gly Gly Ile Ser
                165                 170                 175
Val Pro Gly Pro Met Gly Pro Ser Gly Pro Arg Gly Leu Pro Gly Pro
            180                 185                 190
Pro Gly Ala Pro Gly Pro Gln Gly Phe Gln Gly Pro Pro Gly Glu Pro
        195                 200                 205
Gly Glu Pro Gly Ala Ser Gly Pro Met Gly Pro Arg Gly Pro Pro Gly
    210                 215                 220
Pro Pro Gly Lys Asn Gly Asp Asp Gly Glu Ala Gly Lys Pro Gly Arg
225                 230                 235                 240
Pro Gly Glu Arg Gly Pro Pro Gly Pro Gln Gly Ala Arg Gly Leu Pro
                245                 250                 255
Gly Thr Ala Gly Leu Pro Gly Met Lys Gly His Arg Gly Phe Ser Gly
            260                 265                 270
Leu Asp Gly Ala Lys Gly Asp Ala Gly Pro Ala Gly Pro Lys Gly Glu
        275                 280                 285
Pro Gly Ser Pro Gly Glu Asn Gly Ala Pro Gly Gln Met Gly Pro Arg
    290                 295                 300
Gly Leu Pro Gly Glu Arg Gly Arg Pro Gly Ala Pro Gly Pro Ala Gly
305                 310                 315                 320
Ala Arg Gly Asn Asp Gly Ala Thr Gly Ala Ala Gly Pro Pro Gly Pro
                325                 330                 335
Thr Gly Pro Ala Gly Pro Pro Gly Phe Pro Gly Ala Val Gly Ala Lys
            340                 345                 350
Gly Glu Ala Gly Pro Gln Gly Pro Arg Gly Ser Glu Gly Pro Gln Gly
        355                 360                 365
Val Arg Gly Glu Pro Gly Pro Pro Gly Pro Ala Gly Ala Ala Gly Pro
    370                 375                 380
Ala Gly Asn Pro Gly Ala Asp Gly Gln Pro Gly Ala Lys Gly Ala Asn
385                 390                 395                 400
Gly Ala Pro Gly Ile Ala Gly Ala Pro Gly Phe Pro Gly Ala Arg Gly
                405                 410                 415
Pro Ser Gly Pro Gln Gly Pro Gly Gly Pro Pro Gly Pro Lys Gly Asn
            420                 425                 430
Ser Gly Glu Pro Gly Ala Pro Gly Ser Lys Gly Asp Thr Gly Ala Lys
        435                 440                 445
Gly Glu Pro Gly Pro Val Gly Val Gln Gly Pro Pro Gly Pro Ala Gly
    450                 455                 460
Glu Glu Gly Lys Arg Gly Ala Arg Gly Glu Pro Gly Pro Thr Gly Leu
465                 470                 475                 480
Pro Gly Pro Pro Gly Glu Arg Gly Gly Pro Gly Ser Arg Gly Phe Pro
                485                 490                 495
Gly Ala Asp Gly Val Ala Gly Pro Lys Gly Pro Ala Gly Glu Arg Gly
            500                 505                 510
Ser Pro Gly Pro Ala Gly Pro Lys Gly Ser Pro Gly Glu Ala Gly Arg
        515                 520                 525
Pro Gly Glu Ala Gly Leu Pro Gly Ala Lys Gly Leu Thr Gly Ser Pro
    530                 535                 540
Gly Ser Pro Gly Pro Asp Gly Lys Thr Gly Pro Pro Gly Pro Ala Gly
545                 550                 555                 560
Gln Asp Gly Arg Pro Gly Pro Pro Gly Pro Pro Gly Ala Arg Gly Gln
                565                 570                 575
Ala Gly Val Met Gly Phe Pro Gly Pro Lys Gly Ala Ala Gly Glu Pro
            580                 585                 590
Gly Lys Ala Gly Glu Arg Gly Val Pro Gly Pro Pro Gly Ala Val Gly
        595                 600                 605
```

Pro Ala Gly Lys Asp Gly Glu Ala Gly Ala Gln Gly Pro Pro Gly Pro
610 615 620
Ala Gly Pro Ala Gly Glu Arg Gly Glu Gln Gly Pro Ala Gly Ser Pro
625 630 635 640
Gly Phe Gln Gly Leu Pro Gly Pro Ala Gly Pro Pro Gly Glu Ala Gly
645 650 655
Lys Pro Gly Glu Gln Gly Val Pro Gly Asp Leu Gly Ala Pro Gly Pro
660 665 670
Ser Gly Ala Arg Gly Glu Arg Gly Phe Pro Gly Glu Arg Gly Val Gln
675 680 685
Gly Pro Pro Gly Pro Ala Gly Pro Arg Gly Ala Asn Gly Ala Pro Gly
690 695 700
Asn Asp Gly Ala Lys Gly Asp Ala Gly Ala Pro Gly Ala Pro Gly Ser
705 710 715 720
Gln Gly Ala Pro Gly Leu Gln Gly Met Pro Gly Glu Arg Gly Ala Ala
725 730 735
Gly Leu Pro Gly Pro Lys Gly Asp Arg Gly Asp Ala Gly Pro Lys Gly
740 745 750
Ala Asp Gly Ser Pro Gly Lys Asp Gly Val Arg Gly Leu Thr Gly Pro
755 760 765
Ile Gly Pro Pro Gly Pro Ala Gly Ala Pro Gly Asp Lys Gly Glu Ser
770 775 780
Gly Pro Ser Gly Pro Ala Gly Pro Thr Gly Ala Arg Gly Ala Pro Gly
785 790 795 800
Asp Arg Gly Glu Pro Gly Pro Pro Gly Pro Ala Gly Phe Ala Gly Pro
805 810 815
Pro Gly Ala Asp Gly Gln Pro Gly Ala Lys Gly Glu Pro Gly Asp Ala
820 825 830
Gly Ala Lys Gly Asp Ala Gly Pro Pro Gly Pro Ala Gly Pro Ala Gly
835 840 845
Pro Pro Gly Pro Ile Gly Asn Val Gly Ala Pro Gly Ala Lys Gly Ala
850 855 860
Arg Gly Ser Ala Gly Pro Pro Gly Ala Thr Gly Phe Pro Gly Ala Ala
865 870 875 880
Gly Arg Val Gly Pro Pro Gly Pro Ser Gly Asn Ala Gly Pro Pro Gly
885 890 895
Pro Pro Gly Pro Ala Gly Lys Glu Gly Gly Lys Gly Pro Arg Gly Glu
900 905 910
Thr Gly Pro Ala Gly Arg Pro Gly Glu Val Gly Pro Pro Gly Pro Pro
915 920 925
Gly Pro Ala Gly Glu Lys Gly Ser Pro Gly Ala Asp Gly Pro Ala Gly
930 935 940
Ala Pro Gly Thr Pro Gly Pro Gln Gly Ile Ala Gly Gln Arg Gly Val
945 950 955 960
Val Gly Leu Pro Gly Gln Arg Gly Glu Arg Gly Phe Pro Gly Leu Pro
965 970 975
Gly Pro Ser Gly Glu Pro Gly Lys Gln Gly Pro Ser Gly Ala Ser Gly
980 985 990
Glu Arg Gly Pro Pro Gly Pro Met Gly Pro Pro Gly Leu Ala Gly Pro
995 1000 1005
Pro Gly Glu Ser Gly Arg Glu Gly Ala Pro Ala Ala Glu Gly Ser
1010 1015 1020
Pro Gly Arg Asp Gly Ser Pro Gly Ala Lys Gly Asp Arg Gly Glu
1025 1030 1035
Thr Gly Pro Ala Gly Pro Pro Gly Ala Pro Gly Ala Pro Gly Ala
1040 1045 1050
Pro Gly Pro Val Gly Pro Ala Gly Lys Ser Gly Asp Arg Gly Glu
1055 1060 1065
Thr Gly Pro Ala Gly Pro Ala Gly Pro Val Gly Pro Val Gly Ala
1070 1075 1080
Arg Gly Pro Ala Gly Pro Gln Gly Pro Arg Gly Asp Lys Gly Glu
1085 1090 1095
Thr Gly Glu Gln Gly Asp Arg Gly Ile Lys Gly His Arg Gly Phe
1100 1105 1110
Ser Gly Leu Gln Gly Pro Pro Gly Pro Pro Gly Ser Pro Gly Glu
1115 1120 1125
Gln Gly Pro Ser Gly Ala Ser Gly Pro Ala Gly Pro Arg Gly Pro
1130 1135 1140
Pro Gly Ser Ala Gly Ala Pro Gly Lys Asp Gly Leu Asn Gly Leu

1145 1150 1155
Pro Gly Pro Ile Gly Pro Pro Gly Pro Arg Gly Arg Thr Gly Asp
1160 1165 1170
Ala Gly Pro Val Gly Pro Pro Gly Pro Pro Gly Pro Pro Gly Pro
1175 1180 1185
Pro Gly Pro Pro Ser Ala Gly Phe Asp Phe Ser Phe Leu Pro Gln
1190 1195 1200
Pro Pro Gln Glu Lys Ala His Asp Gly Gly Arg Tyr Tyr Arg Ala
1205 1210 1215
Asp Asp Ala Asn Val Val Arg Asp Arg Asp Leu Glu Val Asp Thr
1220 1225 1230
Thr Leu Lys Ser Leu Ser Gln Gln Ile Glu Asn Ile Arg Ser Pro
1235 1240 1245
Glu Gly Ser Arg Lys Asn Pro Ala Arg Thr Cys Arg Asp Leu Lys
1250 1255 1260
Met Cys His Ser Asp Trp Lys Ser Gly Glu Tyr Trp Ile Asp Pro
1265 1270 1275
Asn Gln Gly Cys Asn Leu Asp Ala Ile Lys Val Phe Cys Asn Met
1280 1285 1290
Glu Thr Gly Glu Thr Cys Val Tyr Pro Thr Gln Pro Ser Val Ala
1295 1300 1305
Gln Lys Asn Trp Tyr Ile Ser Lys Asn Pro Lys Asp Lys Arg His
1310 1315 1320
Val Trp Phe Gly Glu Ser Met Thr Asp Gly Phe Gln Phe Glu Tyr
1325 1330 1335
Gly Gly Gln Gly Ser Asp Pro Ala Asp Val Ala Ile Gln Leu Thr
1340 1345 1350
Phe Leu Arg Leu Met Ser Thr Glu Ala Ser Gln Asn Ile Thr Tyr
1355 1360 1365
His Cys Lys Asn Ser Val Ala Tyr Met Asp Gln Gln Thr Gly Asn
1370 1375 1380
Leu Lys Lys Ala Leu Leu Leu Lys Gly Ser Asn Glu Ile Glu Ile
1385 1390 1395
Arg Ala Glu Gly Asn Ser Arg Phe Thr Tyr Ser Val Thr Val Asp
1400 1405 1410
Gly Cys Thr Ser His Thr Gly Ala Trp Gly Lys Thr Val Ile Glu
1415 1420 1425
Tyr Lys Thr Thr Lys Ser Ser Arg Leu Pro Ile Ile Asp Val Ala
1430 1435 1440
Pro Leu Asp Val Gly Ala Pro Asp Gln Glu Phe Gly Phe Asp Val
1445 1450 1455
Gly Pro Val Cys Phe Leu
1460

<210> 319
<211> 764
<212> PRT
<213> Homo sapiens
<400> 319
Met Gly Ser Asn Leu Ser Pro Gln Leu Cys Leu Met Pro Phe Ile Leu
1 5 10 15
Gly Leu Leu Ser Gly Gly Val Thr Thr Thr Pro Trp Ser Leu Ala Gln
20 25 30
Pro Gln Gly Ser Cys Ser Leu Glu Gly Val Glu Ile Lys Gly Gly Ser
35 40 45
Phe Arg Leu Leu Gln Glu Gly Gln Ala Leu Glu Tyr Val Cys Pro Ser
50 55 60
Gly Phe Tyr Pro Tyr Pro Val Gln Thr Arg Thr Cys Arg Ser Thr Gly
65 70 75 80
Ser Trp Ser Thr Leu Lys Thr Gln Asp Gln Lys Thr Val Arg Lys Ala
85 90 95
Glu Cys Arg Ala Ile His Cys Pro Arg Pro His Asp Phe Glu Asn Gly
100 105 110
Glu Tyr Trp Pro Arg Ser Pro Tyr Tyr Asn Val Ser Asp Glu Ile Ser
115 120 125
Phe His Cys Tyr Asp Gly Tyr Thr Leu Arg Gly Ser Ala Asn Arg Thr
130 135 140
Cys Gln Val Asn Gly Arg Trp Ser Gly Gln Thr Ala Ile Cys Asp Asn
145 150 155 160

Gly Ala Gly Tyr Cys Ser Asn Pro Gly Ile Pro Ile Gly Thr Arg Lys
165 170 175
Val Gly Ser Gln Tyr Arg Leu Glu Asp Ser Val Thr Tyr His Cys Ser
180 185 190
Arg Gly Leu Thr Leu Arg Gly Ser Gln Arg Arg Thr Cys Gln Glu Gly
195 200 205
Gly Ser Trp Ser Gly Thr Glu Pro Ser Cys Gln Asp Ser Phe Met Tyr
210 215 220
Asp Thr Pro Gln Glu Val Ala Glu Ala Phe Leu Ser Ser Leu Thr Glu
225 230 235 240
Thr Ile Glu Gly Val Asp Ala Glu Asp Gly His Gly Pro Gly Glu Gln
245 250 255
Gln Lys Arg Lys Ile Val Leu Asp Pro Ser Gly Ser Met Asn Ile Tyr
260 265 270
Leu Val Leu Asp Gly Ser Asp Ser Ile Gly Ala Ser Asn Phe Thr Gly
275 280 285
Ala Lys Lys Cys Leu Val Asn Leu Ile Glu Lys Val Ala Ser Tyr Gly
290 295 300
Val Lys Pro Arg Tyr Gly Leu Val Thr Tyr Ala Thr Tyr Pro Lys Ile
305 310 315 320
Trp Val Lys Val Ser Glu Ala Asp Ser Ser Asn Ala Asp Trp Val Thr
325 330 335
Lys Gln Leu Asn Glu Ile Asn Tyr Glu Asp His Lys Leu Lys Ser Gly
340 345 350
Thr Asn Thr Lys Lys Ala Leu Gln Ala Val Tyr Ser Met Met Ser Trp
355 360 365
Pro Asp Asp Val Pro Pro Glu Gly Trp Asn Arg Thr Arg His Val Ile
370 375 380
Ile Leu Met Thr Asp Gly Leu His Asn Met Gly Gly Asp Pro Ile Thr
385 390 395 400
Val Ile Asp Glu Ile Arg Asp Leu Leu Tyr Ile Gly Lys Asp Arg Lys
405 410 415
Asn Pro Arg Glu Asp Tyr Leu Asp Val Tyr Val Phe Gly Val Gly Pro
420 425 430
Leu Val Asn Gln Val Asn Ile Asn Ala Leu Ala Ser Lys Lys Asp Asn
435 440 445
Glu Gln His Val Phe Lys Val Lys Asp Met Glu Asn Leu Glu Asp Val
450 455 460
Phe Tyr Gln Met Ile Asp Glu Ser Gln Ser Leu Ser Leu Cys Gly Met
465 470 475 480
Val Trp Glu His Arg Lys Gly Thr Asp Tyr His Lys Gln Pro Trp Gln
485 490 495
Ala Lys Ile Ser Val Ile Arg Pro Ser Lys Gly His Glu Ser Cys Met
500 505 510
Gly Ala Val Val Ser Glu Tyr Phe Val Leu Thr Ala Ala His Cys Phe
515 520 525
Thr Val Asp Asp Lys Glu His Ser Ile Lys Val Ser Val Gly Gly Glu
530 535 540
Lys Arg Asp Leu Glu Ile Glu Val Val Leu Phe His Pro Asn Tyr Asn
545 550 555 560
Ile Asn Gly Lys Lys Glu Ala Gly Ile Pro Glu Phe Tyr Asp Tyr Asp
565 570 575
Val Ala Leu Ile Lys Leu Lys Asn Lys Leu Lys Tyr Gly Gln Thr Ile
580 585 590
Arg Pro Ile Cys Leu Pro Cys Thr Glu Gly Thr Thr Arg Ala Leu Arg
595 600 605
Leu Pro Pro Thr Thr Thr Cys Gln Gln Gln Lys Glu Glu Leu Leu Pro
610 615 620
Ala Gln Asp Ile Lys Ala Leu Phe Val Ser Glu Glu Glu Lys Lys Leu
625 630 635 640
Thr Arg Lys Glu Val Tyr Ile Lys Asn Gly Asp Lys Lys Gly Ser Cys
645 650 655
Glu Arg Asp Ala Gln Tyr Ala Pro Gly Tyr Asp Lys Val Lys Asp Ile
660 665 670
Ser Glu Val Val Thr Pro Arg Phe Leu Cys Thr Gly Gly Val Ser Pro
675 680 685
Tyr Ala Asp Pro Asn Thr Cys Arg Gly Asp Ser Gly Gly Pro Leu Ile
690 695 700
Val His Lys Arg Ser Arg Phe Ile Gln Val Gly Val Ile Ser Trp Gly

705 710 715 720
Val Val Asp Val Cys Lys Asn Gln Lys Arg Gln Lys Gln Val Pro Ala
725 730 735
His Ala Arg Asp Phe His Ile Asn Leu Phe Gln Val Leu Pro Trp Leu
740 745 750
Lys Glu Lys Leu Gln Asp Glu Asp Leu Gly Phe Leu
755 760

<210> 320
<211> 909
<212> PRT
<213> Homo sapiens
<400> 320
Met Ala Ala Arg Pro Leu Pro Val Ser Pro Ala Arg Ala Leu Leu Leu
1 5 10 15
Ala Leu Ala Gly Ala Leu Leu Ala Pro Cys Glu Ala Arg Gly Val Ser
20 25 30
Leu Trp Asn Gln Gly Arg Ala Asp Glu Val Val Ser Ala Ser Val Arg
35 40 45
Ser Gly Asp Leu Trp Ile Pro Val Lys Ser Phe Asp Ser Lys Asn His
50 55 60
Pro Glu Val Leu Asn Ile Arg Leu Gln Arg Glu Ser Lys Glu Leu Ile
65 70 75 80
Ile Asn Leu Glu Arg Asn Glu Gly Leu Ile Ala Ser Ser Phe Thr Glu
85 90 95
Thr His Tyr Leu Gln Asp Gly Thr Asp Val Ser Leu Ala Arg Asn Tyr
100 105 110
Thr Val Ile Leu Gly His Cys Tyr Tyr His Gly His Val Arg Gly Tyr
115 120 125
Ser Asp Ser Ala Val Ser Leu Ser Thr Cys Ser Gly Leu Arg Gly Leu
130 135 140
Ile Val Phe Glu Asn Glu Ser Tyr Val Leu Glu Pro Met Lys Ser Ala
145 150 155 160
Thr Asn Arg Tyr Lys Leu Phe Pro Ala Lys Lys Leu Lys Ser Val Arg
165 170 175
Gly Ser Cys Gly Ser His His Asn Thr Pro Asn Leu Ala Ala Lys Asn
180 185 190
Val Phe Pro Pro Pro Ser Gln Thr Trp Ala Arg Arg His Lys Arg Glu
195 200 205
Thr Leu Lys Ala Thr Lys Tyr Val Glu Leu Val Ile Val Ala Asp Asn
210 215 220
Arg Glu Phe Gln Arg Gln Gly Lys Asp Leu Glu Lys Val Lys Gln Arg
225 230 235 240
Leu Ile Glu Ile Ala Asn His Val Asp Lys Phe Tyr Arg Pro Leu Asn
245 250 255
Ile Arg Ile Val Leu Val Gly Val Glu Val Trp Asn Asp Met Asp Lys
260 265 270
Cys Ser Val Ser Gln Asp Pro Phe Thr Ser Leu His Glu Phe Leu Asp
275 280 285
Trp Arg Lys Met Lys Leu Leu Pro Arg Lys Ser His Asp Asn Ala Gln
290 295 300
Leu Val Ser Gly Val Tyr Phe Gln Gly Thr Thr Ile Gly Met Ala Pro
305 310 315 320
Ile Met Ser Met Cys Thr Ala Asp Gln Ser Gly Gly Ile Val Met Asp
325 330 335
His Ser Asp Asn Pro Leu Gly Ala Ala Val Thr Leu Ala His Glu Leu
340 345 350
Gly His Asn Phe Gly Met Asn His Asp Thr Leu Asp Arg Gly Cys Ser
355 360 365
Cys Gln Met Ala Val Glu Lys Gly Gly Cys Ile Met Asn Ala Ser Thr
370 375 380
Gly Tyr Pro Phe Pro Met Val Phe Ser Ser Cys Ser Arg Lys Asp Leu
385 390 395 400
Glu Thr Ser Leu Glu Lys Gly Met Gly Val Cys Leu Phe Asn Leu Pro
405 410 415
Glu Val Arg Glu Ser Phe Gly Gly Gln Lys Cys Gly Asn Arg Phe Val
420 425 430
Glu Glu Gly Glu Glu Cys Asp Cys Gly Glu Pro Glu Glu Cys Met Asn
435 440 445

Arg Cys Cys Asn Ala Thr Thr Cys Thr Leu Lys Pro Asp Ala Val Cys
450 455 460
Ala His Gly Leu Cys Cys Glu Asp Cys Gln Leu Lys Pro Ala Gly Thr
465 470 475 480
Ala Cys Arg Asp Ser Ser Asn Ser Cys Asp Leu Pro Glu Phe Cys Thr
485 490 495
Gly Ala Ser Pro His Cys Pro Ala Asn Val Tyr Leu His Asp Gly His
500 505 510
Ser Cys Gln Asp Val Asp Gly Tyr Cys Tyr Asn Gly Ile Cys Gln Thr
515 520 525
His Glu Gln Gln Cys Val Thr Leu Trp Gly Pro Gly Ala Lys Pro Ala
530 535 540
Pro Gly Ile Cys Phe Glu Arg Val Asn Ser Ala Gly Asp Pro Tyr Gly
545 550 555 560
Asn Cys Gly Lys Val Ser Lys Ser Ser Phe Ala Lys Cys Glu Met Arg
565 570 575
Asp Ala Lys Cys Gly Lys Ile Gln Cys Gln Gly Gly Ala Ser Arg Pro
580 585 590
Val Ile Gly Thr Asn Ala Val Ser Ile Glu Thr Asn Ile Pro Leu Gln
595 600 605
Gln Gly Gly Arg Ile Leu Cys Arg Gly Thr His Val Tyr Leu Gly Asp
610 615 620
Asp Met Pro Asp Pro Gly Leu Val Leu Ala Gly Thr Lys Cys Ala Asp
625 630 635 640
Gly Lys Ile Cys Leu Asn Arg Gln Cys Gln Asn Ile Ser Val Phe Gly
645 650 655
Val His Glu Cys Ala Met Gln Cys His Gly Arg Gly Val Cys Asn Asn
660 665 670
Arg Lys Asn Cys His Cys Glu Ala His Trp Ala Pro Pro Phe Cys Asp
675 680 685
Lys Phe Gly Phe Gly Gly Ser Thr Asp Ser Gly Pro Ile Arg Gln Ala
690 695 700
Asp Asn Gln Gly Leu Thr Ile Gly Ile Leu Val Thr Ile Leu Cys Leu
705 710 715 720
Leu Ala Ala Gly Phe Val Val Tyr Leu Lys Arg Lys Thr Leu Ile Arg
725 730 735
Leu Leu Phe Thr Asn Lys Lys Thr Thr Ile Glu Lys Leu Arg Cys Val
740 745 750
Arg Pro Ser Arg Pro Pro Arg Gly Phe Gln Pro Cys Gln Ala His Leu
755 760 765
Gly His Leu Gly Lys Gly Leu Met Arg Lys Pro Pro Asp Ser Tyr Pro
770 775 780
Pro Lys Asp Asn Pro Arg Arg Leu Leu Gln Cys Gln Asn Val Asp Ile
785 790 795 800
Ser Arg Pro Leu Asn Gly Leu Asn Val Pro Gln Pro Gln Ser Thr Gln
805 810 815
Arg Val Leu Pro Pro Leu His Arg Ala Pro Arg Ala Pro Ser Val Pro
820 825 830
Ala Arg Pro Leu Pro Ala Lys Pro Ala Leu Arg Gln Ala Gln Gly Thr
835 840 845
Cys Lys Pro Asn Pro Pro Gln Lys Pro Leu Pro Ala Asp Pro Leu Ala
850 855 860
Arg Thr Thr Arg Leu Thr His Ala Leu Ala Arg Thr Pro Gly Gln Trp
865 870 875 880
Glu Thr Gly Leu Arg Leu Ala Pro Leu Arg Pro Ala Pro Gln Tyr Pro
885 890 895
His Gln Val Pro Arg Ser Thr His Thr Ala Tyr Ile Lys
900 905

<210> 321
<211> 574
<212> PRT
<213> Homo sapiens
<400> 321
Met Ala Leu Ala Arg Gly Ser Arg Gln Leu Gly Ala Leu Val Trp Gly
1 5 10 15
Ala Cys Leu Cys Val Leu Val His Gly Gln Gln Ala Gln Pro Gly Gln
20 25 30
Gly Ser Asp Pro Ala Arg Trp Arg Gln Leu Ile Gln Trp Glu Asn Asn

```
            35                  40                  45
Gly Gln Val Tyr Ser Leu Leu Asn Ser Gly Ser Glu Tyr Val Pro Ala
    50                  55                  60
Gly Pro Gln Arg Ser Glu Ser Ser Ser Arg Val Leu Leu Ala Gly Ala
65                  70                  75                  80
Pro Gln Ala Gln Gln Arg Arg Ser His Gly Ser Pro Arg Arg Arg Gln
                85                  90                  95
Ala Pro Ser Leu Pro Leu Pro Gly Arg Val Gly Ser Asp Thr Val Arg
            100                 105                 110
Gly Gln Ala Arg His Pro Phe Gly Phe Gly Gln Val Pro Asp Asn Trp
        115                 120                 125
Arg Glu Val Ala Val Gly Asp Ser Thr Gly Met Ala Leu Ala Arg Thr
    130                 135                 140
Ser Val Ser Gln Gln Arg His Gly Gly Ser Ala Ser Ser Val Ser Ala
145                 150                 155                 160
Ser Ala Phe Ala Ser Thr Tyr Arg Gln Gln Pro Ser Tyr Pro Gln Gln
                165                 170                 175
Phe Pro Tyr Pro Gln Ala Pro Phe Val Ser Gln Tyr Glu Asn Tyr Asp
            180                 185                 190
Pro Ala Ser Arg Thr Tyr Asp Gln Gly Phe Val Tyr Tyr Arg Pro Ala
        195                 200                 205
Gly Gly Gly Val Gly Ala Gly Ala Ala Ala Val Ala Ser Ala Gly Val
    210                 215                 220
Ile Tyr Pro Tyr Gln Pro Arg Ala Arg Tyr Glu Glu Tyr Gly Gly Gly
225                 230                 235                 240
Glu Glu Leu Pro Glu Tyr Pro Pro Gln Gly Phe Tyr Pro Ala Pro Glu
                245                 250                 255
Arg Pro Tyr Val Pro Pro Pro Pro Pro Pro Pro Asp Gly Leu Asp Arg
            260                 265                 270
Arg Tyr Ser His Ser Leu Tyr Ser Glu Gly Thr Pro Gly Phe Glu Gln
        275                 280                 285
Ala Tyr Pro Asp Pro Gly Pro Glu Ala Ala Gln Ala His Gly Gly Asp
    290                 295                 300
Pro Arg Leu Gly Trp Tyr Pro Pro Tyr Ala Asn Pro Pro Pro Glu Ala
305                 310                 315                 320
Tyr Gly Pro Pro Arg Ala Leu Glu Pro Pro Tyr Leu Pro Val Arg Ser
                325                 330                 335
Ser Asp Thr Pro Pro Pro Gly Gly Glu Arg Asn Gly Ala Gln Gln Gly
            340                 345                 350
Arg Leu Ser Val Gly Ser Val Tyr Arg Pro Asn Gln Asn Gly Arg Gly
        355                 360                 365
Leu Pro Asp Leu Val Pro Asp Pro Asn Tyr Val Gln Ala Ser Thr Tyr
    370                 375                 380
Val Gln Arg Ala His Leu Tyr Ser Leu Arg Cys Ala Ala Glu Glu Lys
385                 390                 395                 400
Cys Leu Ala Ser Thr Ala Tyr Ala Pro Glu Ala Thr Asp Tyr Asp Val
                405                 410                 415
Arg Val Leu Leu Arg Phe Pro Gln Arg Val Lys Asn Gln Gly Thr Ala
            420                 425                 430
Asp Phe Leu Pro Asn Arg Pro Arg His Thr Trp Glu Trp His Ser Cys
        435                 440                 445
His Gln His Tyr His Ser Met Asp Glu Phe Ser His Tyr Asp Leu Leu
    450                 455                 460
Asp Ala Ala Thr Gly Lys Lys Val Ala Glu Gly His Lys Ala Ser Phe
465                 470                 475                 480
Cys Leu Glu Asp Ser Thr Cys Asp Phe Gly Asn Leu Lys Arg Tyr Ala
                485                 490                 495
Cys Thr Ser His Thr Gln Gly Leu Ser Pro Gly Cys Tyr Asp Thr Tyr
            500                 505                 510
Asn Ala Asp Ile Asp Cys Gln Trp Ile Asp Ile Thr Asp Val Gln Pro
        515                 520                 525
Gly Asn Tyr Ile Leu Lys Val His Val Asn Pro Lys Tyr Ile Val Leu
    530                 535                 540
Glu Ser Asp Phe Thr Asn Asn Val Val Arg Cys Asn Ile His Tyr Thr
545                 550                 555                 560
Gly Arg Tyr Val Ser Ala Thr Asn Cys Lys Ile Val Gln Ser
                565                 570
```

<210> 322

<211> 344
<212> PRT
<213> Homo sapiens
<400> 322

```
Met Gly Pro Pro Ser Ala Pro Pro Cys Arg Leu His Val Pro Trp Lys
1               5                   10                  15
Glu Val Leu Leu Thr Ala Ser Leu Leu Thr Phe Trp Asn Pro Pro Thr
            20                  25                  30
Thr Ala Lys Leu Thr Ile Glu Ser Thr Pro Phe Asn Val Ala Glu Gly
        35                  40                  45
Lys Glu Val Leu Leu Leu Ala His Asn Leu Pro Gln Asn Arg Ile Gly
    50                  55                  60
Tyr Ser Trp Tyr Lys Gly Glu Arg Val Asp Gly Asn Ser Leu Ile Val
65                  70                  75                  80
Gly Tyr Val Ile Gly Thr Gln Gln Ala Thr Pro Gly Pro Ala Tyr Ser
                85                  90                  95
Gly Arg Glu Thr Ile Tyr Pro Asn Ala Ser Leu Leu Ile Gln Asn Val
            100                 105                 110
Thr Gln Asn Asp Thr Gly Phe Tyr Thr Leu Gln Val Ile Lys Ser Asp
        115                 120                 125
Leu Val Asn Glu Glu Ala Thr Gly Gln Phe His Val Tyr Pro Glu Leu
    130                 135                 140
Pro Lys Pro Ser Ile Ser Ser Asn Asn Ser Asn Pro Val Glu Asp Lys
145                 150                 155                 160
Asp Ala Val Ala Phe Thr Cys Glu Pro Glu Val Gln Asn Thr Thr Tyr
                165                 170                 175
Leu Trp Trp Val Asn Gly Gln Ser Leu Pro Val Ser Pro Arg Leu Gln
            180                 185                 190
Leu Ser Asn Gly Asn Met Thr Leu Thr Leu Leu Ser Val Lys Arg Asn
        195                 200                 205
Asp Ala Gly Ser Tyr Glu Cys Glu Ile Gln Asn Pro Ala Ser Ala Asn
    210                 215                 220
Arg Ser Asp Pro Val Thr Leu Asn Val Leu Tyr Gly Pro Asp Val Pro
225                 230                 235                 240
Thr Ile Ser Pro Ser Lys Ala Asn Tyr Arg Pro Gly Glu Asn Leu Asn
                245                 250                 255
Leu Ser Cys His Ala Ala Ser Asn Pro Pro Ala Gln Tyr Ser Trp Phe
            260                 265                 270
Ile Asn Gly Thr Phe Gln Gln Ser Thr Gln Glu Leu Phe Ile Pro Asn
        275                 280                 285
Ile Thr Val Asn Asn Ser Gly Ser Tyr Met Cys Gln Ala His Asn Ser
    290                 295                 300
Ala Thr Gly Leu Asn Arg Thr Thr Val Thr Met Ile Thr Val Ser Gly
305                 310                 315                 320
Ser Ala Pro Val Leu Ser Ala Val Ala Thr Val Gly Ile Thr Ile Gly
                325                 330                 335
Val Leu Ala Arg Val Ala Leu Ile
            340
```

<210> 323
<211> 488
<212> PRT
<213> Homo sapiens
<400> 323

```
Met Ala Pro Ala Ala Trp Leu Arg Ser Ala Ala Ala Arg Ala Leu Leu
1               5                   10                  15
Pro Pro Met Leu Leu Leu Leu Leu Gln Pro Pro Pro Leu Leu Ala Arg
            20                  25                  30
Ala Leu Pro Pro Asp Val His His Leu His Ala Glu Arg Arg Gly Pro
        35                  40                  45
Gln Pro Trp His Ala Ala Leu Pro Ser Ser Pro Ala Pro Ala Pro Ala
    50                  55                  60
Thr Gln Glu Ala Pro Arg Pro Ala Ser Ser Leu Arg Pro Pro Arg Cys
65                  70                  75                  80
Gly Val Pro Asp Pro Ser Asp Gly Leu Ser Ala Arg Asn Arg Gln Lys
                85                  90                  95
Arg Phe Val Leu Ser Gly Gly Arg Trp Glu Lys Thr Asp Leu Thr Tyr
            100                 105                 110
Arg Ile Leu Arg Phe Pro Trp Gln Leu Val Gln Glu Gln Val Arg Gln
```

115 120 125
Thr Met Ala Glu Ala Leu Lys Val Trp Ser Asp Val Thr Pro Leu Thr
130 135 140
Phe Thr Glu Val His Glu Gly Arg Ala Asp Ile Met Ile Asp Phe Ala
145 150 155 160
Arg Tyr Trp His Gly Asp Asp Leu Pro Phe Asp Gly Pro Gly Gly Ile
165 170 175
Leu Ala His Ala Phe Phe Pro Lys Thr His Arg Glu Gly Asp Val His
180 185 190
Phe Asp Tyr Asp Glu Thr Trp Thr Ile Gly Asp Asp Gln Gly Thr Asp
195 200 205
Leu Leu Gln Val Ala Ala His Glu Phe Gly His Val Leu Gly Leu Gln
210 215 220
His Thr Thr Ala Ala Lys Ala Leu Met Ser Ala Phe Tyr Thr Phe Arg
225 230 235 240
Tyr Pro Leu Ser Leu Ser Pro Asp Asp Cys Arg Gly Val Gln His Leu
245 250 255
Tyr Gly Gln Pro Trp Pro Thr Val Thr Ser Arg Thr Pro Ala Leu Gly
260 265 270
Pro Gln Ala Gly Ile Asp Thr Asn Glu Ile Ala Pro Leu Glu Pro Asp
275 280 285
Ala Pro Pro Asp Ala Cys Glu Ala Ser Phe Asp Ala Val Ser Thr Ile
290 295 300
Arg Gly Glu Leu Phe Phe Phe Lys Ala Gly Phe Val Trp Arg Leu Arg
305 310 315 320
Gly Gly Gln Leu Gln Pro Gly Tyr Pro Ala Leu Ala Ser Arg His Trp
325 330 335
Gln Gly Leu Pro Ser Pro Val Asp Ala Ala Phe Glu Asp Ala Gln Gly
340 345 350
His Ile Trp Phe Phe Gln Gly Ala Gln Tyr Trp Val Tyr Asp Gly Glu
355 360 365
Lys Pro Val Leu Gly Pro Ala Pro Leu Thr Glu Leu Gly Leu Val Arg
370 375 380
Phe Pro Val His Ala Ala Leu Val Trp Gly Pro Glu Lys Asn Lys Ile
385 390 395 400
Tyr Phe Phe Arg Gly Arg Asp Tyr Trp Arg Phe His Pro Ser Thr Arg
405 410 415
Arg Val Asp Ser Pro Val Pro Arg Arg Ala Thr Asp Trp Arg Gly Val
420 425 430
Pro Ser Glu Ile Asp Ala Ala Phe Gln Asp Ala Asp Gly Tyr Ala Tyr
435 440 445
Phe Leu Arg Gly Arg Leu Tyr Trp Lys Phe Asp Pro Val Lys Val Lys
450 455 460
Ala Leu Glu Gly Phe Pro Arg Leu Val Gly Pro Asp Phe Phe Gly Cys
465 470 475 480
Ala Glu Pro Ala Asn Thr Phe Leu
485

<210> 324
<211> 469
<212> PRT
<213> Homo sapiens
<400> 324
Met His Ser Phe Pro Pro Leu Leu Leu Leu Leu Phe Trp Gly Val Val
1 5 10 15
Ser His Ser Phe Pro Ala Thr Leu Glu Thr Gln Glu Gln Asp Val Asp
20 25 30
Leu Val Gln Lys Tyr Leu Glu Lys Tyr Tyr Asn Leu Lys Asn Asp Gly
35 40 45
Arg Gln Val Glu Lys Arg Arg Asn Ser Gly Pro Val Val Glu Lys Leu
50 55 60
Lys Gln Met Gln Glu Phe Phe Gly Leu Lys Val Thr Gly Lys Pro Asp
65 70 75 80
Ala Glu Thr Leu Lys Val Met Lys Gln Pro Arg Cys Gly Val Pro Asp
85 90 95
Val Ala Gln Phe Val Leu Thr Glu Gly Asn Pro Arg Trp Glu Gln Thr
100 105 110
His Leu Thr Tyr Arg Ile Glu Asn Tyr Thr Pro Asp Leu Pro Arg Ala
115 120 125

Asp Val Asp His Ala Ile Glu Lys Ala Phe Gln Leu Trp Ser Asn Val
130 135 140
Thr Pro Leu Thr Phe Thr Lys Val Ser Glu Gly Gln Ala Asp Ile Met
145 150 155 160
Ile Ser Phe Val Arg Gly Asp His Arg Asp Asn Ser Pro Phe Asp Gly
165 170 175
Pro Gly Gly Asn Leu Ala His Ala Phe Gln Pro Gly Pro Gly Ile Gly
180 185 190
Gly Asp Ala His Phe Asp Glu Asp Glu Arg Trp Thr Asn Asn Phe Arg
195 200 205
Glu Tyr Asn Leu His Arg Val Ala Ala His Glu Leu Gly His Ser Leu
210 215 220
Gly Leu Ser His Ser Thr Asp Ile Gly Ala Leu Met Tyr Pro Ser Tyr
225 230 235 240
Thr Phe Ser Gly Asp Val Gln Leu Ala Gln Asp Asp Ile Asp Gly Ile
245 250 255
Gln Ala Ile Tyr Gly Arg Ser Gln Asn Pro Val Gln Pro Ile Gly Pro
260 265 270
Gln Thr Pro Lys Ala Cys Asp Ser Lys Leu Thr Phe Asp Ala Ile Thr
275 280 285
Thr Ile Arg Gly Glu Val Met Phe Phe Lys Asp Arg Phe Tyr Met Arg
290 295 300
Thr Asn Pro Phe Tyr Pro Glu Val Glu Leu Asn Phe Ile Ser Val Phe
305 310 315 320
Trp Pro Gln Leu Pro Asn Gly Leu Glu Ala Ala Tyr Glu Phe Ala Asp
325 330 335
Arg Asp Glu Val Arg Phe Phe Lys Gly Asn Lys Tyr Trp Ala Val Gln
340 345 350
Gly Gln Asn Val Leu His Gly Tyr Pro Lys Asp Ile Tyr Ser Ser Phe
355 360 365
Gly Phe Pro Arg Thr Val Lys His Ile Asp Ala Ala Leu Ser Glu Glu
370 375 380
Asn Thr Gly Lys Thr Tyr Phe Phe Val Ala Asn Lys Tyr Trp Arg Tyr
385 390 395 400
Asp Glu Tyr Lys Arg Ser Met Asp Pro Gly Tyr Pro Lys Met Ile Ala
405 410 415
His Asp Phe Pro Gly Ile Gly His Lys Val Asp Ala Val Phe Met Lys
420 425 430
Asp Gly Phe Phe Tyr Phe Phe His Gly Thr Arg Gln Tyr Lys Phe Asp
435 440 445
Pro Lys Thr Lys Arg Ile Leu Thr Leu Gln Lys Ala Asn Ser Trp Phe
450 455 460
Asn Cys Arg Lys Asn
465

<210> 325
<211> 471
<212> PRT
<213> Homo sapiens
<400> 325
Met His Pro Gly Val Leu Ala Ala Phe Leu Phe Leu Ser Trp Thr His
1 5 10 15
Cys Arg Ala Leu Pro Leu Pro Ser Gly Gly Asp Glu Asp Asp Leu Ser
20 25 30
Glu Glu Asp Leu Gln Phe Ala Glu Arg Tyr Leu Arg Ser Tyr Tyr His
35 40 45
Pro Thr Asn Leu Ala Gly Ile Leu Lys Glu Asn Ala Ala Ser Ser Met
50 55 60
Thr Glu Arg Leu Arg Glu Met Gln Ser Phe Phe Gly Leu Glu Val Thr
65 70 75 80
Gly Lys Leu Asp Asp Asn Thr Leu Asp Val Met Lys Lys Pro Arg Cys
85 90 95
Gly Val Pro Asp Val Gly Glu Tyr Asn Val Phe Pro Arg Thr Leu Lys
100 105 110
Trp Ser Lys Met Asn Leu Thr Tyr Arg Ile Val Asn Tyr Thr Pro Asp
115 120 125
Met Thr His Ser Glu Val Glu Lys Ala Phe Lys Lys Ala Phe Lys Val
130 135 140
Trp Ser Asp Val Thr Pro Leu Asn Phe Thr Arg Leu His Asp Gly Ile

145 150 155 160
Ala Asp Ile Met Ile Ser Phe Gly Ile Lys Glu His Gly Asp Phe Tyr
165 170 175
Pro Phe Asp Gly Pro Ser Gly Leu Leu Ala His Ala Phe Pro Pro Gly
180 185 190
Pro Asn Tyr Gly Gly Asp Ala His Phe Asp Asp Asp Glu Thr Trp Thr
195 200 205
Ser Ser Ser Lys Gly Tyr Asn Leu Phe Leu Val Ala Ala His Glu Phe
210 215 220
Gly His Ser Leu Gly Leu Asp His Ser Lys Asp Pro Gly Ala Leu Met
225 230 235 240
Phe Pro Ile Tyr Thr Tyr Thr Gly Lys Ser His Phe Met Leu Pro Asp
245 250 255
Asp Asp Val Gln Gly Ile Gln Ser Leu Tyr Gly Pro Gly Asp Glu Asp
260 265 270
Pro Asn Pro Lys His Pro Lys Thr Pro Asp Lys Cys Asp Pro Ser Leu
275 280 285
Ser Leu Asp Ala Ile Thr Ser Leu Arg Gly Glu Thr Met Ile Phe Lys
290 295 300
Asp Arg Phe Phe Trp Arg Leu His Pro Gln Gln Val Asp Ala Glu Leu
305 310 315 320
Phe Leu Thr Lys Ser Phe Trp Pro Glu Leu Pro Asn Arg Ile Asp Ala
325 330 335
Ala Tyr Glu His Pro Ser His Asp Leu Ile Phe Ile Phe Arg Gly Arg
340 345 350
Lys Phe Trp Ala Leu Asn Gly Tyr Asp Ile Leu Glu Gly Tyr Pro Lys
355 360 365
Lys Ile Ser Glu Leu Gly Leu Pro Lys Glu Val Lys Lys Ile Ser Ala
370 375 380
Ala Val His Phe Glu Asp Thr Gly Lys Thr Leu Leu Phe Ser Gly Asn
385 390 395 400
Gln Val Trp Arg Tyr Asp Asp Thr Asn His Ile Met Asp Lys Asp Tyr
405 410 415
Pro Arg Leu Ile Glu Glu Asp Phe Pro Gly Ile Gly Asp Lys Val Asp
420 425 430
Ala Val Tyr Glu Lys Asn Gly Tyr Ile Tyr Phe Phe Asn Gly Pro Ile
435 440 445
Gln Phe Glu Tyr Ser Ile Trp Ser Asn Arg Ile Val Arg Val Met Pro
450 455 460
Ala Asn Ser Ile Leu Trp Cys
465 470

<210> 326
<211> 418
<212> PRT
<213> Homo sapiens
<400> 326
Met Arg Ser Leu Leu Leu Leu Ser Ala Phe Cys Leu Leu Glu Ala Ala
1 5 10 15
Leu Ala Ala Glu Val Lys Lys Pro Ala Ala Ala Ala Ala Pro Gly Thr
20 25 30
Ala Glu Lys Leu Ser Pro Lys Ala Ala Thr Leu Ala Glu Arg Ser Ala
35 40 45
Gly Leu Ala Phe Ser Leu Tyr Gln Ala Met Ala Lys Asp Gln Ala Val
50 55 60
Glu Asn Ile Leu Val Ser Pro Val Val Val Ala Ser Ser Leu Gly Leu
65 70 75 80
Val Ser Leu Gly Gly Lys Ala Thr Thr Ala Ser Gln Ala Lys Ala Val
85 90 95
Leu Ser Ala Glu Gln Leu Arg Asp Glu Glu Val His Ala Gly Leu Gly
100 105 110
Glu Leu Leu Arg Ser Leu Ser Asn Ser Thr Ala Arg Asn Val Thr Trp
115 120 125
Lys Leu Gly Ser Arg Leu Tyr Gly Pro Ser Ser Val Ser Phe Ala Asp
130 135 140
Asp Phe Val Arg Ser Ser Lys Gln His Tyr Asn Cys Glu His Ser Lys
145 150 155 160
Ile Asn Phe Arg Asp Lys Arg Ser Ala Leu Gln Ser Ile Asn Glu Trp
165 170 175

Ala Ala Gln Thr Thr Asp Gly Lys Leu Pro Glu Val Thr Lys Asp Val
180 185 190
Glu Arg Thr Asp Gly Ala Leu Leu Val Asn Ala Met Phe Phe Lys Pro
195 200 205
His Trp Asp Glu Lys Phe His His Lys Met Val Asp Asn Arg Gly Phe
210 215 220
Met Val Thr Arg Ser Tyr Thr Val Gly Val Met Met Met His Arg Thr
225 230 235 240
Gly Leu Tyr Asn Tyr Tyr Asp Asp Glu Lys Glu Lys Leu Gln Ile Val
245 250 255
Glu Met Pro Leu Ala His Lys Leu Ser Ser Leu Ile Ile Leu Met Pro
260 265 270
His His Val Glu Pro Leu Glu Arg Leu Glu Lys Leu Leu Thr Lys Glu
275 280 285
Gln Leu Lys Ile Trp Met Gly Lys Met Gln Lys Lys Ala Val Ala Ile
290 295 300
Ser Leu Pro Lys Gly Val Val Glu Val Thr His Asp Leu Gln Lys His
305 310 315 320
Leu Ala Gly Leu Gly Leu Thr Glu Ala Ile Asp Lys Asn Lys Ala Asp
325 330 335
Leu Ser Arg Met Ser Gly Lys Lys Asp Leu Tyr Leu Ala Ser Val Phe
340 345 350
His Ala Thr Ala Phe Glu Leu Asp Thr Asp Gly Asn Pro Phe Asp Gln
355 360 365
Asp Ile Tyr Gly Arg Glu Glu Leu Arg Ser Pro Lys Leu Phe Tyr Ala
370 375 380
Asp His Pro Phe Ile Phe Leu Val Arg Asp Thr Gln Ser Gly Ser Leu
385 390 395 400
Leu Phe Ile Gly Arg Leu Val Arg Pro Lys Gly Asp Lys Met Arg Asp
405 410 415
Glu Leu

<210> 327
<211> 314
<212> PRT
<213> Homo sapiens
<400> 327
Met Asp Ala Phe Lys Gly Gly Met Ser Leu Glu Arg Leu Pro Glu Gly
1 5 10 15
Leu Arg Pro Pro Pro Pro Pro Pro His Asp Met Gly Pro Ala Phe His
20 25 30
Leu Ala Arg Pro Ala Asp Pro Arg Glu Pro Leu Glu Asn Ser Ala Ser
35 40 45
Glu Ser Ser Asp Thr Glu Leu Pro Glu Lys Glu Arg Gly Gly Glu Pro
50 55 60
Lys Gly Pro Glu Asp Ser Gly Ala Gly Gly Thr Gly Cys Gly Gly Ala
65 70 75 80
Asp Asp Pro Ala Lys Lys Lys Lys Gln Arg Arg Gln Arg Thr His Phe
85 90 95
Thr Ser Gln Gln Leu Gln Glu Leu Glu Ala Thr Phe Gln Arg Asn Arg
100 105 110
Tyr Pro Asp Met Ser Met Arg Glu Glu Ile Ala Val Trp Thr Asn Leu
115 120 125
Thr Glu Pro Arg Val Arg Val Trp Phe Lys Asn Arg Arg Ala Lys Trp
130 135 140
Arg Lys Arg Glu Arg Asn Gln Gln Leu Asp Leu Cys Lys Gly Gly Tyr
145 150 155 160
Val Pro Gln Phe Ser Gly Leu Val Gln Pro Tyr Glu Asp Val Tyr Ala
165 170 175
Ala Gly Tyr Ser Tyr Asn Asn Trp Ala Ala Lys Ser Leu Ala Pro Ala
180 185 190
Pro Leu Ser Thr Lys Ser Phe Thr Phe Phe Asn Ser Met Ser Pro Leu
195 200 205
Ser Ser Gln Ser Met Phe Ser Ala Pro Ser Ser Ile Ser Ser Met Thr
210 215 220
Met Pro Ser Ser Met Gly Pro Gly Ala Val Pro Gly Met Pro Asn Ser
225 230 235 240
Gly Leu Asn Asn Ile Asn Asn Leu Thr Gly Ser Ser Leu Asn Ser Ala

245 250 255
Met Ser Pro Gly Ala Cys Pro Tyr Gly Thr Pro Ala Ser Pro Tyr Ser
260 265 270
Val Tyr Arg Asp Thr Cys Asn Ser Ser Leu Ala Ser Leu Arg Leu Lys
275 280 285
Ser Lys Gln His Ser Ser Phe Gly Tyr Gly Ala Leu Gln Gly Pro Ala
290 295 300
Ser Gly Leu Asn Ala Cys Gln Tyr Asn Ser
305 310

<210> 328
<211> 2828
<212> PRT
<213> Homo sapiens
<400> 328
Met Pro Lys Arg Ala His Trp Gly Ala Leu Ser Val Val Leu Ile Leu
1 5 10 15
Leu Trp Gly His Pro Arg Val Ala Leu Ala Cys Pro His Pro Cys Ala
20 25 30
Cys Tyr Val Pro Ser Glu Val His Cys Thr Phe Arg Ser Leu Ala Ser
35 40 45
Val Pro Ala Gly Ile Ala Arg His Val Glu Arg Ile Asn Leu Gly Phe
50 55 60
Asn Ser Ile Gln Ala Leu Ser Glu Thr Ser Phe Ala Gly Leu Thr Lys
65 70 75 80
Leu Glu Leu Leu Met Ile His Gly Asn Glu Ile Pro Ser Ile Pro Asp
85 90 95
Gly Ala Leu Arg Asp Leu Ser Ser Leu Gln Val Phe Lys Phe Ser Tyr
100 105 110
Asn Lys Leu Arg Val Ile Thr Gly Gln Thr Leu Gln Gly Leu Ser Asn
115 120 125
Leu Met Arg Leu His Ile Asp His Asn Lys Ile Glu Phe Ile His Pro
130 135 140
Gln Ala Phe Asn Gly Leu Thr Ser Leu Arg Leu Leu His Leu Glu Gly
145 150 155 160
Asn Leu Leu His Gln Leu His Pro Ser Thr Phe Ser Thr Phe Thr Phe
165 170 175
Leu Asp Tyr Phe Arg Leu Ser Thr Ile Arg His Leu Tyr Leu Ala Glu
180 185 190
Asn Met Val Arg Thr Leu Pro Ala Ser Met Leu Arg Asn Met Pro Leu
195 200 205
Leu Glu Asn Leu Tyr Leu Gln Gly Asn Pro Trp Thr Cys Asp Cys Glu
210 215 220
Met Arg Trp Phe Leu Glu Trp Asp Ala Lys Ser Arg Gly Ile Leu Lys
225 230 235 240
Cys Lys Lys Asp Lys Ala Tyr Glu Gly Gly Gln Leu Cys Ala Met Cys
245 250 255
Phe Ser Pro Lys Lys Leu Tyr Lys His Glu Ile His Lys Leu Lys Asp
260 265 270
Met Thr Cys Leu Lys Pro Ser Ile Glu Ser Pro Leu Arg Gln Asn Arg
275 280 285
Ser Arg Ser Ile Glu Glu Glu Gln Glu Gln Glu Glu Asp Gly Gly Ser
290 295 300
Gln Leu Ile Leu Glu Lys Phe Gln Leu Pro Gln Trp Ser Ile Ser Leu
305 310 315 320
Asn Met Thr Asp Glu His Gly Asn Met Val Asn Leu Val Cys Asp Ile
325 330 335
Lys Lys Pro Met Asp Val Tyr Lys Ile His Leu Asn Gln Thr Asp Pro
340 345 350
Pro Asp Ile Asp Ile Asn Ala Thr Val Ala Leu Asp Phe Glu Cys Pro
355 360 365
Met Thr Arg Glu Asn Tyr Glu Lys Leu Trp Lys Leu Ile Ala Tyr Tyr
370 375 380
Ser Glu Val Pro Val Lys Leu His Arg Glu Leu Met Leu Ser Lys Asp
385 390 395 400
Pro Arg Val Ser Tyr Gln Tyr Arg Gln Asp Ala Asp Glu Glu Ala Leu
405 410 415
Tyr Tyr Thr Gly Val Arg Ala Gln Ile Leu Ala Glu Pro Glu Trp Val
420 425 430

Met Gln Pro Ser Ile Asp Ile Gln Leu Asn Arg Arg Gln Ser Thr Ala
435 440 445
Lys Lys Val Leu Leu Ser Tyr Tyr Thr Gln Tyr Ser Gln Thr Ile Ser
450 455 460
Thr Lys Asp Thr Arg Gln Ala Arg Gly Arg Ser Trp Val Met Ile Glu
465 470 475 480
Pro Ser Gly Ala Val Gln Arg Asp Gln Thr Val Leu Glu Gly Gly Pro
485 490 495
Cys Gln Leu Ser Cys Asn Val Lys Ala Ser Glu Ser Pro Ser Ile Phe
500 505 510
Trp Val Leu Pro Asp Gly Ser Ile Leu Lys Ala Pro Met Asp Asp Pro
515 520 525
Asp Ser Lys Phe Ser Ile Leu Ser Ser Gly Trp Leu Arg Ile Lys Ser
530 535 540
Met Glu Pro Ser Asp Ser Gly Leu Tyr Gln Cys Ile Ala Gln Val Arg
545 550 555 560
Asp Glu Met Asp Arg Met Val Tyr Arg Val Leu Val Gln Ser Pro Ser
565 570 575
Thr Gln Pro Ala Glu Lys Asp Thr Val Thr Ile Gly Lys Asn Pro Gly
580 585 590
Glu Ser Val Thr Leu Pro Cys Asn Ala Leu Ala Ile Pro Glu Ala His
595 600 605
Leu Ser Trp Ile Leu Pro Asn Arg Arg Ile Ile Asn Asp Leu Ala Asn
610 615 620
Thr Ser His Val Tyr Met Leu Pro Asn Gly Thr Leu Ser Ile Pro Lys
625 630 635 640
Val Gln Val Ser Asp Ser Gly Tyr Tyr Arg Cys Val Ala Val Asn Gln
645 650 655
Gln Gly Ala Asp His Phe Thr Val Gly Ile Thr Val Thr Lys Lys Gly
660 665 670
Ser Gly Leu Pro Ser Lys Arg Gly Arg Arg Pro Gly Ala Lys Ala Leu
675 680 685
Ser Arg Val Arg Glu Asp Ile Val Glu Asp Glu Gly Gly Ser Gly Met
690 695 700
Gly Asp Glu Glu Asn Thr Ser Arg Arg Leu Leu His Pro Lys Asp Gln
705 710 715 720
Glu Val Phe Leu Lys Thr Lys Asp Asp Ala Ile Asn Gly Asp Lys Lys
725 730 735
Ala Lys Lys Gly Arg Arg Lys Leu Lys Leu Trp Lys His Ser Glu Lys
740 745 750
Glu Pro Glu Thr Asn Val Ala Glu Gly Arg Arg Val Phe Glu Ser Arg
755 760 765
Arg Arg Ile Asn Met Ala Asn Lys Gln Ile Asn Pro Glu Arg Trp Ala
770 775 780
Asp Ile Leu Ala Lys Val Arg Gly Lys Asn Leu Pro Lys Gly Thr Glu
785 790 795 800
Val Pro Pro Leu Ile Lys Thr Thr Ser Pro Pro Ser Leu Ser Leu Glu
805 810 815
Val Thr Pro Pro Phe Pro Ala Val Ser Pro Pro Ser Ala Ser Pro Val
820 825 830
Gln Thr Val Thr Ser Ala Glu Glu Ser Ser Ala Asp Val Pro Leu Leu
835 840 845
Gly Glu Glu Glu His Val Leu Gly Thr Ile Ser Ser Ala Ser Met Gly
850 855 860
Leu Glu His Asn His Asn Gly Val Ile Leu Val Glu Pro Glu Val Thr
865 870 875 880
Ser Thr Pro Leu Glu Glu Val Val Asp Asp Leu Ser Glu Lys Thr Glu
885 890 895
Glu Ile Thr Ser Thr Glu Gly Asp Leu Lys Gly Thr Ala Ala Pro Thr
900 905 910
Leu Ile Ser Glu Pro Tyr Glu Pro Ser Pro Thr Leu His Thr Leu Asp
915 920 925
Thr Val Tyr Glu Lys Pro Thr His Glu Glu Thr Ala Thr Glu Gly Trp
930 935 940
Ser Ala Ala Asp Val Gly Ser Ser Pro Glu Pro Thr Ser Ser Glu Tyr
945 950 955 960
Glu Pro Pro Leu Asp Ala Val Ser Leu Ala Glu Ser Glu Pro Met Gln
965 970 975
Tyr Phe Asp Pro Asp Leu Glu Thr Lys Ser Gln Pro Asp Glu Asp Lys

980 985 990
Met Lys Glu Asp Thr Phe Ala His Leu Thr Pro Thr Pro Thr Ile Trp
995 1000 1005
Val Asn Asp Ser Ser Thr Ser Gln Leu Phe Glu Asp Ser Thr Ile
1010 1015 1020
Gly Glu Pro Gly Val Pro Gly Gln Ser His Leu Gln Gly Leu Thr
1025 1030 1035
Asp Asn Ile His Leu Val Lys Ser Ser Leu Ser Thr Gln Asp Thr
1040 1045 1050
Leu Leu Ile Lys Lys Gly Met Lys Glu Met Ser Gln Thr Leu Gln
1055 1060 1065
Gly Gly Asn Met Leu Glu Gly Asp Pro Thr His Ser Arg Ser Ser
1070 1075 1080
Glu Ser Glu Gly Gln Glu Ser Lys Ser Ile Thr Leu Pro Asp Ser
1085 1090 1095
Thr Leu Gly Ile Met Ser Ser Met Ser Pro Val Lys Lys Pro Ala
1100 1105 1110
Glu Thr Thr Val Gly Thr Leu Leu Asp Lys Asp Thr Thr Thr Val
1115 1120 1125
Thr Thr Thr Pro Arg Gln Lys Val Ala Pro Ser Ser Thr Met Ser
1130 1135 1140
Thr His Pro Ser Arg Arg Arg Pro Asn Gly Arg Arg Arg Leu Arg
1145 1150 1155
Pro Asn Lys Phe Arg His Arg His Lys Gln Thr Pro Pro Thr Thr
1160 1165 1170
Phe Ala Pro Ser Glu Thr Phe Ser Thr Gln Pro Thr Gln Ala Pro
1175 1180 1185
Asp Ile Lys Ile Ser Ser Gln Val Glu Ser Ser Leu Val Pro Thr
1190 1195 1200
Ala Trp Val Asp Asn Thr Val Asn Thr Pro Lys Gln Leu Glu Met
1205 1210 1215
Glu Lys Asn Ala Glu Pro Thr Ser Lys Gly Thr Pro Arg Arg Lys
1220 1225 1230
His Gly Lys Arg Pro Asn Lys His Arg Tyr Thr Pro Ser Thr Val
1235 1240 1245
Ser Ser Arg Ala Ser Gly Ser Lys Pro Ser Pro Ser Pro Glu Asn
1250 1255 1260
Lys His Arg Asn Ile Val Thr Pro Ser Ser Glu Thr Ile Leu Leu
1265 1270 1275
Pro Arg Thr Val Ser Leu Lys Thr Glu Gly Pro Tyr Asp Ser Leu
1280 1285 1290
Asp Tyr Met Thr Thr Thr Arg Lys Ile Tyr Ser Ser Tyr Pro Lys
1295 1300 1305
Val Gln Glu Thr Leu Pro Val Thr Tyr Lys Pro Thr Ser Asp Gly
1310 1315 1320
Lys Glu Ile Lys Asp Asp Val Ala Thr Asn Val Asp Lys His Lys
1325 1330 1335
Ser Asp Ile Leu Val Thr Gly Glu Ser Ile Thr Asn Ala Ile Pro
1340 1345 1350
Thr Ser Arg Ser Leu Val Ser Thr Met Gly Glu Phe Lys Glu Glu
1355 1360 1365
Ser Ser Pro Val Gly Phe Pro Gly Thr Pro Thr Trp Asn Pro Ser
1370 1375 1380
Arg Thr Ala Gln Pro Gly Arg Leu Gln Thr Asp Ile Pro Val Thr
1385 1390 1395
Thr Ser Gly Glu Asn Leu Thr Asp Pro Pro Leu Leu Lys Glu Leu
1400 1405 1410
Glu Asp Val Asp Phe Thr Ser Glu Phe Leu Ser Ser Leu Thr Val
1415 1420 1425
Ser Thr Pro Phe His Gln Glu Glu Ala Gly Ser Ser Thr Thr Leu
1430 1435 1440
Ser Ser Ile Lys Val Glu Val Ala Ser Ser Gln Ala Glu Thr Thr
1445 1450 1455
Thr Leu Asp Gln Asp His Leu Glu Thr Thr Val Ala Ile Leu Leu
1460 1465 1470
Ser Glu Thr Arg Pro Gln Asn His Thr Pro Thr Ala Ala Arg Met
1475 1480 1485
Lys Glu Pro Ala Ser Ser Ser Pro Ser Thr Ile Leu Met Ser Leu
1490 1495 1500

Gly Gln Thr Thr Thr Thr Lys Pro Ala Leu Pro Ser Pro Arg Ile
1505 1510 1515
Ser Gln Ala Ser Arg Asp Ser Lys Glu Asn Val Phe Leu Asn Tyr
1520 1525 1530
Val Gly Asn Pro Glu Thr Glu Ala Thr Pro Val Asn Asn Glu Gly
1535 1540 1545
Thr Gln His Met Ser Gly Pro Asn Glu Leu Ser Thr Pro Ser Ser
1550 1555 1560
Asp Arg Asp Ala Phe Asn Leu Ser Thr Lys Leu Glu Leu Glu Lys
1565 1570 1575
Gln Val Phe Gly Ser Arg Ser Leu Pro Arg Gly Pro Asp Ser Gln
1580 1585 1590
Arg Gln Asp Gly Arg Val His Ala Ser His Gln Leu Thr Arg Val
1595 1600 1605
Pro Ala Lys Pro Ile Leu Pro Thr Ala Thr Val Arg Leu Pro Glu
1610 1615 1620
Met Ser Thr Gln Ser Ala Ser Arg Tyr Phe Val Thr Ser Gln Ser
1625 1630 1635
Pro Arg His Trp Thr Asn Lys Pro Glu Ile Thr Thr Tyr Pro Ser
1640 1645 1650
Gly Ala Leu Pro Glu Asn Lys Gln Phe Thr Thr Pro Arg Leu Ser
1655 1660 1665
Ser Thr Thr Ile Pro Leu Pro Leu His Met Ser Lys Pro Ser Ile
1670 1675 1680
Pro Ser Lys Phe Thr Asp Arg Arg Thr Asp Gln Phe Asn Gly Tyr
1685 1690 1695
Ser Lys Val Phe Gly Asn Asn Asn Ile Pro Glu Ala Arg Asn Pro
1700 1705 1710
Val Gly Lys Pro Pro Ser Pro Arg Ile Pro His Tyr Ser Asn Gly
1715 1720 1725
Arg Leu Pro Phe Phe Thr Asn Lys Thr Leu Ser Phe Pro Gln Leu
1730 1735 1740
Gly Val Thr Arg Arg Pro Gln Ile Pro Thr Ser Pro Ala Pro Val
1745 1750 1755
Met Arg Glu Arg Lys Val Ile Pro Gly Ser Tyr Asn Arg Ile His
1760 1765 1770
Ser His Ser Thr Phe His Leu Asp Phe Gly Pro Pro Ala Pro Pro
1775 1780 1785
Leu Leu His Thr Pro Gln Thr Thr Gly Ser Pro Ser Thr Asn Leu
1790 1795 1800
Gln Asn Ile Pro Met Val Ser Ser Thr Gln Ser Ser Ile Ser Phe
1805 1810 1815
Ile Thr Ser Ser Val Gln Ser Ser Gly Ser Phe His Gln Ser Ser
1820 1825 1830
Ser Lys Phe Phe Ala Gly Gly Pro Pro Ala Ser Lys Phe Trp Ser
1835 1840 1845
Leu Gly Glu Lys Pro Gln Ile Leu Thr Lys Ser Pro Gln Thr Val
1850 1855 1860
Ser Val Thr Ala Glu Thr Asp Thr Val Phe Pro Cys Glu Ala Thr
1865 1870 1875
Gly Lys Pro Lys Pro Phe Val Thr Trp Thr Lys Val Ser Thr Gly
1880 1885 1890
Ala Leu Met Thr Pro Asn Thr Arg Ile Gln Arg Phe Glu Val Leu
1895 1900 1905
Lys Asn Gly Thr Leu Val Ile Arg Lys Val Gln Val Gln Asp Arg
1910 1915 1920
Gly Gln Tyr Met Cys Thr Ala Ser Asn Leu His Gly Leu Asp Arg
1925 1930 1935
Met Val Val Leu Leu Ser Val Thr Val Gln Gln Pro Gln Ile Leu
1940 1945 1950
Ala Ser His Tyr Gln Asp Val Thr Val Tyr Leu Gly Asp Thr Ile
1955 1960 1965
Ala Met Glu Cys Leu Ala Lys Gly Thr Pro Ala Pro Gln Ile Ser
1970 1975 1980
Trp Ile Phe Pro Asp Arg Arg Val Trp Gln Thr Val Ser Pro Val
1985 1990 1995
Glu Ser Arg Ile Thr Leu His Glu Asn Arg Thr Leu Ser Ile Lys
2000 2005 2010
Glu Ala Ser Phe Ser Asp Arg Gly Val Tyr Lys Cys Val Ala Ser

2015 2020 2025
Asn Ala Ala Gly Ala Asp Ser Leu Ala Ile Arg Leu His Val Ala
2030 2035 2040
Ala Leu Pro Pro Val Ile His Gln Glu Lys Leu Glu Asn Ile Ser
2045 2050 2055
Leu Pro Pro Gly Leu Ser Ile His Ile His Cys Thr Ala Lys Ala
2060 2065 2070
Ala Pro Leu Pro Ser Val Arg Trp Val Leu Gly Asp Gly Thr Gln
2075 2080 2085
Ile Arg Pro Ser Gln Phe Leu His Gly Asn Leu Phe Val Phe Pro
2090 2095 2100
Asn Gly Thr Leu Tyr Ile Arg Asn Leu Ala Pro Lys Asp Ser Gly
2105 2110 2115
Arg Tyr Glu Cys Val Ala Ala Asn Leu Val Gly Ser Ala Arg Arg
2120 2125 2130
Thr Val Gln Leu Asn Val Gln Arg Ala Ala Ala Asn Ala Arg Ile
2135 2140 2145
Thr Gly Thr Ser Pro Arg Arg Thr Asp Val Arg Tyr Gly Gly Thr
2150 2155 2160
Leu Lys Leu Asp Cys Ser Ala Ser Gly Asp Pro Trp Pro Arg Ile
2165 2170 2175
Leu Trp Arg Leu Pro Ser Lys Arg Met Ile Asp Ala Leu Phe Ser
2180 2185 2190
Phe Asp Ser Arg Ile Lys Val Phe Ala Asn Gly Thr Leu Val Val
2195 2200 2205
Lys Ser Val Thr Asp Lys Asp Ala Gly Asp Tyr Leu Cys Val Ala
2210 2215 2220
Arg Asn Lys Val Gly Asp Asp Tyr Val Val Leu Lys Val Asp Val
2225 2230 2235
Val Met Lys Pro Ala Lys Ile Glu His Lys Glu Glu Asn Asp His
2240 2245 2250
Lys Val Phe Tyr Gly Gly Asp Leu Lys Val Asp Cys Val Ala Thr
2255 2260 2265
Gly Leu Pro Asn Pro Glu Ile Ser Trp Ser Leu Pro Asp Gly Ser
2270 2275 2280
Leu Val Asn Ser Phe Met Gln Ser Asp Asp Ser Gly Gly Arg Thr
2285 2290 2295
Lys Arg Tyr Val Val Phe Asn Asn Gly Thr Leu Tyr Phe Asn Glu
2300 2305 2310
Val Gly Met Arg Glu Glu Gly Asp Tyr Thr Cys Phe Ala Glu Asn
2315 2320 2325
Gln Val Gly Lys Asp Glu Met Arg Val Arg Val Lys Val Val Thr
2330 2335 2340
Ala Pro Ala Thr Ile Arg Asn Lys Thr Tyr Leu Ala Val Gln Val
2345 2350 2355
Pro Tyr Gly Asp Val Val Thr Val Ala Cys Glu Ala Lys Gly Glu
2360 2365 2370
Pro Met Pro Lys Val Thr Trp Leu Ser Pro Thr Asn Lys Val Ile
2375 2380 2385
Pro Thr Ser Ser Glu Lys Tyr Gln Ile Tyr Gln Asp Gly Thr Leu
2390 2395 2400
Leu Ile Gln Lys Ala Gln Arg Ser Asp Ser Gly Asn Tyr Thr Cys
2405 2410 2415
Leu Val Arg Asn Ser Ala Gly Glu Asp Arg Lys Thr Val Trp Ile
2420 2425 2430
His Val Asn Val Gln Pro Pro Lys Ile Asn Gly Asn Pro Asn Pro
2435 2440 2445
Ile Thr Thr Val Arg Glu Ile Ala Ala Gly Gly Ser Arg Lys Leu
2450 2455 2460
Ile Asp Cys Lys Ala Glu Gly Ile Pro Thr Pro Arg Val Leu Trp
2465 2470 2475
Ala Phe Pro Glu Gly Val Val Leu Pro Ala Pro Tyr Tyr Gly Asn
2480 2485 2490
Arg Ile Thr Val His Gly Asn Gly Ser Leu Asp Ile Arg Ser Leu
2495 2500 2505
Arg Lys Ser Asp Ser Val Gln Leu Val Cys Met Ala Arg Asn Glu
2510 2515 2520
Gly Gly Glu Ala Arg Leu Ile Val Gln Leu Thr Val Leu Glu Pro
2525 2530 2535

Met Glu Lys Pro Ile Phe His Asp Pro Ile Ser Glu Lys Ile Thr
2540 2545 2550
Ala Met Ala Gly His Thr Ile Ser Leu Asn Cys Ser Ala Ala Gly
2555 2560 2565
Thr Pro Thr Pro Ser Leu Val Trp Val Leu Pro Asn Gly Thr Asp
2570 2575 2580
Leu Gln Ser Gly Gln Gln Leu Gln Arg Phe Tyr His Lys Ala Asp
2585 2590 2595
Gly Met Leu His Ile Ser Gly Leu Ser Ser Val Asp Ala Gly Ala
2600 2605 2610
Tyr Arg Cys Val Ala Arg Asn Ala Ala Gly His Thr Glu Arg Leu
2615 2620 2625
Val Ser Leu Lys Val Gly Leu Lys Pro Glu Ala Asn Lys Gln Tyr
2630 2635 2640
His Asn Leu Val Ser Ile Ile Asn Gly Glu Thr Leu Lys Leu Pro
2645 2650 2655
Cys Thr Pro Pro Gly Ala Gly Gln Gly Arg Phe Ser Trp Thr Leu
2660 2665 2670
Pro Asn Gly Met His Leu Glu Gly Pro Gln Thr Leu Gly Arg Val
2675 2680 2685
Ser Leu Leu Asp Asn Gly Thr Leu Thr Val Arg Glu Ala Ser Val
2690 2695 2700
Phe Asp Arg Gly Thr Tyr Val Cys Arg Met Glu Thr Glu Tyr Gly
2705 2710 2715
Pro Ser Val Thr Ser Ile Pro Val Ile Val Ile Ala Tyr Pro Pro
2720 2725 2730
Arg Ile Thr Ser Glu Pro Thr Pro Val Ile Tyr Thr Arg Pro Gly
2735 2740 2745
Asn Thr Val Lys Leu Asn Cys Met Ala Met Gly Ile Pro Lys Ala
2750 2755 2760
Asp Ile Thr Trp Glu Leu Pro Asp Lys Ser His Leu Lys Ala Gly
2765 2770 2775
Val Gln Ala Arg Leu Tyr Gly Asn Arg Phe Leu His Pro Gln Gly
2780 2785 2790
Ser Leu Thr Ile Gln His Ala Thr Gln Arg Asp Ala Gly Phe Tyr
2795 2800 2805
Lys Cys Met Ala Lys Asn Ile Leu Gly Ser Asp Ser Lys Thr Thr
2810 2815 2820
Tyr Ile His Val Phe
2825

<210> 329
<211> 426
<212> PRT
<213> Homo sapiens
<400> 329
Met Pro Leu Leu Trp Leu Arg Gly Phe Leu Leu Ala Ser Cys Trp Ile
1 5 10 15
Ile Val Arg Ser Ser Pro Thr Pro Gly Ser Glu Gly His Ser Ala Ala
20 25 30
Pro Asp Cys Pro Ser Cys Ala Leu Ala Ala Leu Pro Lys Asp Val Pro
35 40 45
Asn Ser Gln Pro Glu Met Val Glu Ala Val Lys Lys His Ile Leu Asn
50 55 60
Met Leu His Leu Lys Lys Arg Pro Asp Val Thr Gln Pro Val Pro Lys
65 70 75 80
Ala Ala Leu Leu Asn Ala Ile Arg Lys Leu His Val Gly Lys Val Gly
85 90 95
Glu Asn Gly Tyr Val Glu Ile Glu Asp Asp Ile Gly Arg Arg Ala Glu
100 105 110
Met Asn Glu Leu Met Glu Gln Thr Ser Glu Ile Ile Thr Phe Ala Glu
115 120 125
Ser Gly Thr Ala Arg Lys Thr Leu His Phe Glu Ile Ser Lys Glu Gly
130 135 140
Ser Asp Leu Ser Val Val Glu Arg Ala Glu Val Trp Leu Phe Leu Lys
145 150 155 160
Val Pro Lys Ala Asn Arg Thr Arg Thr Lys Val Thr Ile Arg Leu Phe
165 170 175
Gln Gln Gln Lys His Pro Gln Gly Ser Leu Asp Thr Gly Glu Glu Ala

180 185 190
Glu Glu Val Gly Leu Lys Gly Glu Arg Ser Glu Leu Leu Leu Ser Glu
195 200 205
Lys Val Val Asp Ala Arg Lys Ser Thr Trp His Val Phe Pro Val Ser
210 215 220
Ser Ser Ile Gln Arg Leu Leu Asp Gln Gly Lys Ser Ser Leu Asp Val
225 230 235 240
Arg Ile Ala Cys Glu Gln Cys Gln Glu Ser Gly Ala Ser Leu Val Leu
245 250 255
Leu Gly Lys Lys Lys Lys Lys Glu Glu Glu Gly Glu Gly Lys Lys Lys
260 265 270
Gly Gly Gly Glu Gly Gly Ala Gly Ala Asp Glu Glu Lys Glu Gln Ser
275 280 285
His Arg Pro Phe Leu Met Leu Gln Ala Arg Gln Ser Glu Asp His Pro
290 295 300
His Arg Arg Arg Arg Arg Gly Leu Glu Cys Asp Gly Lys Val Asn Ile
305 310 315 320
Cys Cys Lys Lys Gln Phe Phe Val Ser Phe Lys Asp Ile Gly Trp Asn
325 330 335
Asp Trp Ile Ile Ala Pro Ser Gly Tyr His Ala Asn Tyr Cys Glu Gly
340 345 350
Glu Cys Pro Ser His Ile Ala Gly Thr Ser Gly Ser Ser Leu Ser Phe
355 360 365
His Ser Thr Val Ile Asn His Tyr Arg Met Arg Gly His Ser Pro Phe
370 375 380
Ala Asn Leu Lys Ser Cys Cys Val Pro Thr Lys Leu Arg Pro Met Ser
385 390 395 400
Met Leu Tyr Tyr Asp Asp Gly Gln Asn Ile Ile Lys Lys Asp Ile Gln
405 410 415
Asn Met Ile Val Glu Glu Cys Gly Cys Ser
420 425

<210> 330
<211> 6792
<212> PRT
<213> Homo sapiens
<400> 330
Met Phe Ile Asn Ile Lys Ser Ile Leu Trp Met Cys Ser Thr Leu Ile
1 5 10 15
Val Thr His Ala Leu His Lys Val Lys Val Gly Lys Ser Pro Pro Val
20 25 30
Arg Gly Ser Leu Ser Gly Lys Val Ser Leu Pro Cys His Phe Ser Thr
35 40 45
Met Pro Thr Leu Pro Pro Ser Tyr Asn Thr Ser Glu Phe Leu Arg Ile
50 55 60
Lys Trp Ser Lys Ile Glu Val Asp Lys Asn Gly Lys Asp Leu Lys Glu
65 70 75 80
Thr Thr Val Leu Val Ala Gln Asn Gly Asn Ile Lys Ile Gly Gln Asp
85 90 95
Tyr Lys Gly Arg Val Ser Val Pro Thr His Pro Glu Ala Val Gly Asp
100 105 110
Ala Ser Leu Thr Val Val Lys Leu Leu Ala Ser Asp Ala Gly Leu Tyr
115 120 125
Arg Cys Asp Val Met Tyr Gly Ile Glu Asp Thr Gln Asp Thr Val Ser
130 135 140
Leu Thr Val Asp Gly Val Val Phe His Tyr Arg Ala Ala Thr Ser Arg
145 150 155 160
Tyr Thr Leu Asn Phe Glu Ala Ala Gln Lys Ala Cys Leu Asp Val Gly
165 170 175
Ala Val Ile Ala Thr Pro Glu Gln Leu Phe Ala Ala Tyr Glu Asp Gly
180 185 190
Phe Glu Gln Cys Asp Ala Gly Trp Leu Ala Asp Gln Thr Val Arg Tyr
195 200 205
Pro Ile Arg Ala Pro Arg Val Gly Cys Tyr Gly Asp Lys Met Gly Lys
210 215 220
Ala Gly Val Arg Thr Tyr Gly Phe Arg Ser Pro Gln Glu Thr Tyr Asp
225 230 235 240
Val Tyr Cys Tyr Val Asp His Leu Asp Gly Asp Val Phe His Leu Thr
245 250 255

```
Val Pro Ser Lys Phe Thr Phe Glu Glu Ala Ala Lys Glu Cys Glu Asn
            260                 265                 270
Gln Asp Ala Arg Leu Ala Thr Val Gly Glu Leu Gln Ala Ala Trp Arg
        275                 280                 285
Asn Gly Phe Asp Gln Cys Asp Tyr Gly Trp Leu Ser Asp Ala Ser Val
    290                 295                 300
Arg His Pro Val Thr Val Ala Arg Ala Gln Cys Gly Gly Gly Leu Leu
305                 310                 315                 320
Gly Val Arg Thr Leu Tyr Arg Phe Glu Asn Gln Thr Gly Phe Pro Pro
                325                 330                 335
Pro Asp Ser Arg Phe Asp Ala Tyr Cys Phe Lys Pro Lys Glu Ala Thr
            340                 345                 350
Thr Ile Asp Leu Ser Ile Leu Ala Glu Thr Ala Ser Pro Ser Leu Ser
        355                 360                 365
Lys Glu Pro Gln Met Val Ser Asp Arg Thr Thr Pro Ile Ile Pro Leu
    370                 375                 380
Val Asp Glu Leu Pro Val Ile Pro Thr Glu Phe Pro Pro Val Gly Asn
385                 390                 395                 400
Ile Val Ser Phe Glu Gln Lys Ala Thr Val Gln Pro Gln Ala Ile Thr
                405                 410                 415
Asp Ser Leu Ala Thr Lys Leu Pro Thr Pro Thr Gly Ser Thr Lys Lys
            420                 425                 430
Pro Trp Asp Met Asp Asp Tyr Ser Pro Ser Ala Ser Gly Pro Leu Gly
        435                 440                 445
Lys Leu Asp Ile Ser Glu Ile Lys Glu Glu Val Leu Gln Ser Thr Thr
    450                 455                 460
Gly Val Ser His Tyr Ala Thr Asp Ser Trp Asp Gly Val Val Glu Asp
465                 470                 475                 480
Lys Gln Thr Gln Glu Ser Val Thr Gln Ile Glu Gln Ile Glu Val Gly
                485                 490                 495
Pro Leu Val Thr Ser Met Glu Ile Leu Lys His Ile Pro Ser Lys Glu
            500                 505                 510
Phe Pro Val Thr Glu Thr Pro Leu Val Thr Ala Arg Met Ile Leu Glu
        515                 520                 525
Ser Lys Thr Glu Lys Lys Met Val Ser Thr Val Ser Glu Leu Val Thr
    530                 535                 540
Thr Gly His Tyr Gly Phe Thr Leu Gly Glu Glu Asp Asp Glu Asp Arg
545                 550                 555                 560
Thr Leu Thr Val Gly Ser Asp Glu Ser Thr Leu Ile Phe Asp Gln Ile
                565                 570                 575
Pro Glu Val Ile Thr Val Ser Lys Thr Ser Glu Asp Thr Ile His Thr
            580                 585                 590
His Leu Glu Asp Leu Glu Ser Val Ser Ala Ser Thr Thr Val Ser Pro
        595                 600                 605
Leu Ile Met Pro Asp Asn Asn Gly Ser Ser Met Asp Asp Trp Glu Glu
    610                 615                 620
Arg Gln Thr Ser Gly Arg Ile Thr Glu Glu Phe Leu Gly Lys Tyr Leu
625                 630                 635                 640
Ser Thr Thr Pro Phe Pro Ser Gln His Arg Thr Glu Ile Glu Leu Phe
                645                 650                 655
Pro Tyr Ser Gly Asp Lys Ile Leu Val Glu Gly Ile Ser Thr Val Ile
            660                 665                 670
Tyr Pro Ser Leu Gln Thr Glu Met Thr His Arg Arg Glu Arg Thr Glu
        675                 680                 685
Thr Leu Ile Pro Glu Met Arg Thr Asp Thr Tyr Thr Asp Glu Ile Gln
    690                 695                 700
Glu Glu Ile Thr Lys Ser Pro Phe Met Gly Lys Thr Glu Glu Glu Val
705                 710                 715                 720
Phe Ser Gly Met Lys Leu Ser Thr Ser Leu Ser Glu Pro Ile His Val
                725                 730                 735
Thr Glu Ser Ser Val Glu Met Thr Lys Ser Phe Asp Phe Pro Thr Leu
            740                 745                 750
Ile Thr Lys Leu Ser Ala Glu Pro Thr Glu Val Arg Asp Met Glu Glu
        755                 760                 765
Asp Phe Thr Ala Thr Pro Gly Thr Thr Lys Tyr Asp Glu Asn Ile Thr
    770                 775                 780
Thr Val Leu Leu Ala His Gly Thr Leu Ser Val Glu Ala Ala Thr Val
785                 790                 795                 800
Ser Lys Trp Ser Trp Asp Glu Asp Asn Thr Thr Ser Lys Pro Leu Glu
```

```
                805                     810                     815
Ser Thr Glu Pro Ser Ala Ser Ser Lys Leu Pro Pro Ala Leu Leu Thr
            820                     825                     830
Thr Val Gly Met Asn Gly Lys Asp Lys Asp Ile Pro Ser Phe Thr Glu
        835                     840                     845
Asp Gly Ala Asp Glu Phe Thr Leu Ile Pro Asp Ser Thr Gln Lys Gln
    850                     855                     860
Leu Glu Glu Val Thr Asp Glu Asp Ile Ala Ala His Gly Lys Phe Thr
865                     870                     875                     880
Ile Arg Phe Gln Pro Thr Thr Ser Thr Gly Ile Ala Glu Lys Ser Thr
                885                     890                     895
Leu Arg Asp Ser Thr Thr Glu Glu Lys Val Pro Pro Ile Thr Ser Thr
            900                     905                     910
Glu Gly Gln Val Tyr Ala Thr Met Glu Gly Ser Ala Leu Gly Glu Val
        915                     920                     925
Glu Asp Val Asp Leu Ser Lys Pro Val Ser Thr Val Pro Gln Phe Ala
    930                     935                     940
His Thr Ser Glu Val Glu Gly Leu Ala Phe Val Ser Tyr Ser Ser Thr
945                     950                     955                     960
Gln Glu Pro Thr Thr Tyr Val Asp Ser Ser His Thr Ile Pro Leu Ser
                965                     970                     975
Val Ile Pro Lys Thr Asp Trp Gly Val Leu Val Pro Ser Val Pro Ser
            980                     985                     990
Glu Asp Glu Val Leu Gly Glu Pro  Ser Gln Asp Ile Leu  Val Ile Asp
        995                     1000                    1005
Gln Thr  Arg Leu Glu Ala Thr  Ile Ser Pro Glu Thr  Met Arg Thr
    1010                    1015                    1020
Thr Lys  Ile Thr Glu Gly Thr  Thr Gln Glu Glu Phe  Pro Trp Lys
    1025                    1030                    1035
Glu Gln  Thr Ala Glu Lys Pro  Val Pro Ala Leu Ser  Ser Thr Ala
    1040                    1045                    1050
Trp Thr  Pro Lys Glu Ala Val  Thr Pro Leu Asp Glu  Gln Glu Gly
    1055                    1060                    1065
Asp Gly  Ser Ala Tyr Thr Val  Ser Glu Asp Glu Leu  Leu Thr Gly
    1070                    1075                    1080
Ser Glu  Arg Val Pro Val Leu  Glu Thr Thr Pro Val  Gly Lys Ile
    1085                    1090                    1095
Asp His  Ser Val Ser Tyr Pro  Pro Gly Ala Val Thr  Glu His Lys
    1100                    1105                    1110
Val Lys  Thr Asp Glu Val Val  Thr Leu Thr Pro Arg  Ile Gly Pro
    1115                    1120                    1125
Lys Val  Ser Leu Ser Pro Gly  Pro Glu Gln Lys Tyr  Glu Thr Glu
    1130                    1135                    1140
Gly Ser  Ser Thr Thr Gly Phe  Thr Ser Ser Leu Ser  Pro Phe Ser
    1145                    1150                    1155
Thr His  Ile Thr Gln Leu Met  Glu Glu Thr Thr Thr  Glu Lys Thr
    1160                    1165                    1170
Ser Leu  Glu Asp Ile Asp Leu  Gly Ser Gly Leu Phe  Glu Lys Pro
    1175                    1180                    1185
Lys Ala  Thr Glu Leu Ile Glu  Phe Ser Thr Ile Lys  Val Thr Val
    1190                    1195                    1200
Pro Ser  Asp Ile Thr Thr Ala  Phe Ser Ser Val Asp  Arg Leu His
    1205                    1210                    1215
Thr Thr  Ser Ala Phe Lys Pro  Ser Ser Ala Ile Thr  Lys Lys Pro
    1220                    1225                    1230
Pro Leu  Ile Asp Arg Glu Pro  Gly Glu Glu Thr Thr  Ser Asp Met
    1235                    1240                    1245
Val Ile  Ile Gly Glu Ser Thr  Ser His Val Pro Pro  Thr Thr Leu
    1250                    1255                    1260
Glu Asp  Ile Val Ala Lys Glu  Thr Glu Thr Asp Ile  Asp Arg Glu
    1265                    1270                    1275
Tyr Phe  Thr Thr Ser Ser Pro  Pro Ala Thr Gln Pro  Thr Arg Pro
    1280                    1285                    1290
Pro Thr  Val Glu Asp Lys Glu  Ala Phe Gly Pro Gln  Ala Leu Ser
    1295                    1300                    1305
Thr Pro  Gln Pro Pro Ala Ser  Thr Lys Phe His Pro  Asp Ile Asn
    1310                    1315                    1320
Val Tyr  Ile Ile Glu Val Arg  Glu Asn Lys Thr Gly  Arg Met Ser
    1325                    1330                    1335
```

Asp Leu Ser Val Ile Gly His Pro Ile Asp Ser Glu Ser Lys Glu
1340 1345 1350
Asp Glu Pro Cys Ser Glu Glu Thr Asp Pro Val His Asp Leu Met
1355 1360 1365
Ala Glu Ile Leu Pro Glu Phe Pro Asp Ile Ile Glu Ile Asp Leu
1370 1375 1380
Tyr His Ser Glu Glu Asn Glu Glu Glu Glu Glu Glu Cys Ala Asn
1385 1390 1395
Ala Thr Asp Val Thr Thr Thr Pro Ser Val Gln Tyr Ile Asn Gly
1400 1405 1410
Lys His Leu Val Thr Thr Val Pro Lys Asp Pro Glu Ala Ala Glu
1415 1420 1425
Ala Arg Arg Gly Gln Phe Glu Ser Val Ala Pro Ser Gln Asn Phe
1430 1435 1440
Ser Asp Ser Ser Glu Ser Asp Thr His Pro Phe Val Ile Ala Lys
1445 1450 1455
Thr Glu Leu Ser Thr Ala Val Gln Pro Asn Glu Ser Thr Glu Thr
1460 1465 1470
Thr Glu Ser Leu Glu Val Thr Trp Lys Pro Glu Thr Tyr Pro Glu
1475 1480 1485
Thr Ser Glu His Phe Ser Gly Gly Glu Pro Asp Val Phe Pro Thr
1490 1495 1500
Val Pro Phe His Glu Glu Phe Glu Ser Gly Thr Ala Lys Lys Gly
1505 1510 1515
Ala Glu Ser Val Thr Glu Arg Asp Thr Glu Val Gly His Gln Ala
1520 1525 1530
His Glu His Thr Glu Pro Val Ser Leu Phe Pro Glu Glu Ser Ser
1535 1540 1545
Gly Glu Ile Ala Ile Asp Gln Glu Ser Gln Lys Ile Ala Phe Ala
1550 1555 1560
Arg Ala Thr Glu Val Thr Phe Gly Glu Glu Val Glu Lys Ser Thr
1565 1570 1575
Ser Val Thr Tyr Thr Pro Thr Ile Val Pro Ser Ser Ala Ser Ala
1580 1585 1590
Tyr Val Ser Glu Glu Glu Ala Val Thr Leu Ile Gly Asn Pro Trp
1595 1600 1605
Pro Asp Asp Leu Leu Ser Thr Lys Glu Ser Trp Val Glu Ala Thr
1610 1615 1620
Pro Arg Gln Val Val Glu Leu Ser Gly Ser Ser Ser Ile Pro Ile
1625 1630 1635
Thr Glu Gly Ser Gly Glu Ala Glu Glu Asp Glu Asp Thr Met Phe
1640 1645 1650
Thr Met Val Thr Asp Leu Ser Gln Arg Asn Thr Thr Asp Thr Leu
1655 1660 1665
Ile Thr Leu Asp Thr Ser Arg Ile Ile Thr Glu Ser Phe Phe Glu
1670 1675 1680
Val Pro Ala Thr Thr Ile Tyr Pro Val Ser Glu Gln Pro Ser Ala
1685 1690 1695
Lys Val Val Pro Thr Lys Phe Val Ser Glu Thr Asp Thr Ser Glu
1700 1705 1710
Trp Ile Ser Ser Thr Thr Val Glu Glu Lys Lys Arg Lys Glu Glu
1715 1720 1725
Glu Gly Thr Thr Gly Thr Ala Ser Thr Phe Glu Val Tyr Ser Ser
1730 1735 1740
Thr Gln Arg Ser Asp Gln Leu Ile Leu Pro Phe Glu Leu Glu Ser
1745 1750 1755
Pro Asn Val Ala Thr Ser Ser Asp Ser Gly Thr Arg Lys Ser Phe
1760 1765 1770
Met Ser Leu Thr Thr Pro Thr Gln Ser Glu Arg Glu Met Thr Asp
1775 1780 1785
Ser Thr Pro Val Phe Thr Glu Thr Asn Thr Leu Glu Asn Leu Gly
1790 1795 1800
Ala Gln Thr Thr Glu His Ser Ser Ile His Gln Pro Gly Val Gln
1805 1810 1815
Glu Gly Leu Thr Thr Leu Pro Arg Ser Pro Ala Ser Val Phe Met
1820 1825 1830
Glu Gln Gly Ser Gly Glu Ala Ala Ala Asp Pro Glu Thr Thr Thr
1835 1840 1845
Val Ser Ser Phe Ser Leu Asn Val Glu Tyr Ala Ile Gln Ala Glu

1850 1855 1860
Lys Glu Val Ala Gly Thr Leu Ser Pro His Val Glu Thr Thr Phe
1865 1870 1875
Ser Thr Glu Pro Thr Gly Leu Val Leu Ser Thr Val Met Asp Arg
1880 1885 1890
Val Val Ala Glu Asn Ile Thr Gln Thr Ser Arg Glu Ile Val Ile
1895 1900 1905
Ser Glu Arg Leu Gly Glu Pro Asn Tyr Gly Ala Glu Ile Arg Gly
1910 1915 1920
Phe Ser Thr Gly Phe Pro Leu Glu Glu Asp Phe Ser Gly Asp Phe
1925 1930 1935
Arg Glu Tyr Ser Thr Val Ser His Pro Ile Ala Lys Glu Glu Thr
1940 1945 1950
Val Met Met Glu Gly Ser Gly Asp Ala Ala Phe Arg Asp Thr Gln
1955 1960 1965
Thr Ser Pro Ser Thr Val Pro Thr Ser Val His Ile Ser His Ile
1970 1975 1980
Ser Asp Ser Glu Gly Pro Ser Ser Thr Met Val Ser Thr Ser Ala
1985 1990 1995
Phe Pro Trp Glu Glu Phe Thr Ser Ser Ala Glu Gly Ser Gly Glu
2000 2005 2010
Gln Leu Val Thr Val Ser Ser Ser Val Val Pro Val Leu Pro Ser
2015 2020 2025
Ala Val Gln Lys Phe Ser Gly Thr Ala Ser Ser Ile Ile Asp Glu
2030 2035 2040
Gly Leu Gly Glu Val Gly Thr Val Asn Glu Ile Asp Arg Arg Ser
2045 2050 2055
Thr Ile Leu Pro Thr Ala Glu Val Glu Gly Thr Lys Ala Pro Val
2060 2065 2070
Glu Lys Glu Glu Val Lys Val Ser Gly Thr Val Ser Thr Asn Phe
2075 2080 2085
Pro Gln Thr Ile Glu Pro Ala Lys Leu Trp Ser Arg Gln Glu Val
2090 2095 2100
Asn Pro Val Arg Gln Glu Ile Glu Ser Glu Thr Thr Ser Glu Glu
2105 2110 2115
Gln Ile Gln Glu Glu Lys Ser Phe Glu Ser Pro Gln Asn Ser Pro
2120 2125 2130
Ala Thr Glu Gln Thr Ile Phe Asp Ser Gln Thr Phe Thr Glu Thr
2135 2140 2145
Glu Leu Lys Thr Thr Asp Tyr Ser Val Leu Thr Thr Lys Lys Thr
2150 2155 2160
Tyr Ser Asp Asp Lys Glu Met Lys Glu Glu Asp Thr Ser Leu Val
2165 2170 2175
Asn Met Ser Thr Pro Asp Pro Asp Ala Asn Gly Leu Glu Ser Tyr
2180 2185 2190
Thr Thr Leu Pro Glu Ala Thr Glu Lys Ser His Phe Phe Leu Ala
2195 2200 2205
Thr Ala Leu Val Thr Glu Ser Ile Pro Ala Glu His Val Val Thr
2210 2215 2220
Asp Ser Pro Ile Lys Lys Glu Glu Ser Thr Lys His Phe Pro Lys
2225 2230 2235
Gly Met Arg Pro Thr Ile Gln Glu Ser Asp Thr Glu Leu Leu Phe
2240 2245 2250
Ser Gly Leu Gly Ser Gly Glu Glu Val Leu Pro Thr Leu Pro Thr
2255 2260 2265
Glu Ser Val Asn Phe Thr Glu Val Glu Gln Ile Asn Asn Thr Leu
2270 2275 2280
Tyr Pro His Thr Ser Gln Val Glu Ser Thr Ser Ser Asp Lys Ile
2285 2290 2295
Glu Asp Phe Asn Arg Met Glu Asn Val Ala Lys Glu Val Gly Pro
2300 2305 2310
Leu Val Ser Gln Thr Asp Ile Phe Glu Gly Ser Gly Ser Val Thr
2315 2320 2325
Ser Thr Thr Leu Ile Glu Ile Leu Ser Asp Thr Gly Ala Glu Gly
2330 2335 2340
Pro Thr Val Ala Pro Leu Pro Phe Ser Thr Asp Ile Gly His Pro
2345 2350 2355
Gln Asn Gln Thr Val Arg Trp Ala Glu Glu Ile Gln Thr Ser Arg
2360 2365 2370

Pro Gln Thr Ile Thr Glu Gln Asp Ser Asn Lys Asn Ser Ser Thr
2375 2380 2385
Ala Glu Ile Asn Glu Thr Thr Thr Ser Ser Thr Asp Phe Leu Ala
2390 2395 2400
Arg Ala Tyr Gly Phe Glu Met Ala Lys Glu Phe Val Thr Ser Ala
2405 2410 2415
Pro Lys Pro Ser Asp Leu Tyr Tyr Glu Pro Ser Gly Glu Gly Ser
2420 2425 2430
Gly Glu Val Asp Ile Val Asp Ser Phe His Thr Ser Ala Thr Thr
2435 2440 2445
Gln Ala Thr Arg Gln Glu Ser Ser Thr Thr Phe Val Ser Asp Gly
2450 2455 2460
Ser Leu Glu Lys His Pro Glu Val Pro Ser Ala Lys Ala Val Thr
2465 2470 2475
Ala Asp Gly Phe Pro Thr Val Ser Val Met Leu Pro Leu His Ser
2480 2485 2490
Glu Gln Asn Lys Ser Ser Pro Asp Pro Thr Ser Thr Leu Ser Asn
2495 2500 2505
Thr Val Ser Tyr Glu Arg Ser Thr Asp Gly Ser Phe Gln Asp Arg
2510 2515 2520
Phe Arg Glu Phe Glu Asp Ser Thr Leu Lys Pro Asn Arg Lys Lys
2525 2530 2535
Pro Thr Glu Asn Ile Ile Ile Asp Leu Asp Lys Glu Asp Lys Asp
2540 2545 2550
Leu Ile Leu Thr Ile Thr Glu Ser Thr Ile Leu Glu Ile Leu Pro
2555 2560 2565
Glu Leu Thr Ser Asp Lys Asn Thr Ile Ile Asp Ile Asp His Thr
2570 2575 2580
Lys Pro Val Tyr Glu Asp Ile Leu Gly Met Gln Thr Asp Ile Asp
2585 2590 2595
Thr Glu Val Pro Ser Glu Pro His Asp Ser Asn Asp Glu Ser Asn
2600 2605 2610
Asp Asp Ser Thr Gln Val Gln Glu Ile Tyr Glu Ala Ala Val Asn
2615 2620 2625
Leu Ser Leu Thr Glu Glu Thr Phe Glu Gly Ser Ala Asp Val Leu
2630 2635 2640
Ala Ser Tyr Thr Gln Ala Thr His Asp Glu Ser Met Thr Tyr Glu
2645 2650 2655
Asp Arg Ser Gln Leu Asp His Met Gly Phe His Phe Thr Thr Gly
2660 2665 2670
Ile Pro Ala Pro Ser Thr Glu Thr Glu Leu Asp Val Leu Leu Pro
2675 2680 2685
Thr Ala Thr Ser Leu Pro Ile Pro Arg Lys Ser Ala Thr Val Ile
2690 2695 2700
Pro Glu Ile Glu Gly Ile Lys Ala Glu Ala Lys Ala Leu Asp Asp
2705 2710 2715
Met Phe Glu Ser Ser Thr Leu Ser Asp Gly Gln Ala Ile Ala Asp
2720 2725 2730
Gln Ser Glu Ile Ile Pro Thr Leu Gly Gln Phe Glu Arg Thr Gln
2735 2740 2745
Glu Glu Tyr Glu Asp Lys Lys His Ala Gly Pro Ser Phe Gln Pro
2750 2755 2760
Glu Phe Ser Ser Gly Ala Glu Glu Ala Leu Val Asp His Thr Pro
2765 2770 2775
Tyr Leu Ser Ile Ala Thr Thr His Leu Met Asp Gln Ser Val Thr
2780 2785 2790
Glu Val Pro Asp Val Met Glu Gly Ser Asn Pro Pro Tyr Tyr Thr
2795 2800 2805
Asp Thr Thr Leu Ala Val Ser Thr Phe Ala Lys Leu Ser Ser Gln
2810 2815 2820
Thr Pro Ser Ser Pro Leu Thr Ile Tyr Ser Gly Ser Glu Ala Ser
2825 2830 2835
Gly His Thr Glu Ile Pro Gln Pro Ser Ala Leu Pro Gly Ile Asp
2840 2845 2850
Val Gly Ser Ser Val Met Ser Pro Gln Asp Ser Phe Lys Glu Ile
2855 2860 2865
His Val Asn Ile Glu Ala Thr Phe Lys Pro Ser Ser Glu Glu Tyr
2870 2875 2880
Leu His Ile Thr Glu Pro Pro Ser Leu Ser Pro Asp Thr Lys Leu

2885 2890 2895
Glu Pro Ser Glu Asp Asp Gly Lys Pro Glu Leu Leu Glu Glu Met
2900 2905 2910
Glu Ala Ser Pro Thr Glu Leu Ile Ala Val Glu Gly Thr Glu Ile
2915 2920 2925
Leu Gln Asp Phe Gln Asn Lys Thr Asp Gly Gln Val Ser Gly Glu
2930 2935 2940
Ala Ile Lys Met Phe Pro Thr Ile Lys Thr Pro Glu Ala Gly Thr
2945 2950 2955
Val Ile Thr Thr Ala Asp Glu Ile Glu Leu Glu Gly Ala Thr Gln
2960 2965 2970
Trp Pro His Ser Thr Ser Ala Ser Ala Thr Tyr Gly Val Glu Ala
2975 2980 2985
Gly Val Val Pro Trp Leu Ser Pro Gln Thr Ser Glu Arg Pro Thr
2990 2995 3000
Leu Ser Ser Ser Pro Glu Ile Asn Pro Glu Thr Gln Ala Ala Leu
3005 3010 3015
Ile Arg Gly Gln Asp Ser Thr Ile Ala Ala Ser Glu Gln Gln Val
3020 3025 3030
Ala Ala Arg Ile Leu Asp Ser Asn Asp Gln Ala Thr Val Asn Pro
3035 3040 3045
Val Glu Phe Asn Thr Glu Val Ala Thr Pro Pro Phe Ser Leu Leu
3050 3055 3060
Glu Thr Ser Asn Glu Thr Asp Phe Leu Ile Gly Ile Asn Glu Glu
3065 3070 3075
Ser Val Glu Gly Thr Ala Ile Tyr Leu Pro Gly Pro Asp Arg Cys
3080 3085 3090
Lys Met Asn Pro Cys Leu Asn Gly Gly Thr Cys Tyr Pro Thr Glu
3095 3100 3105
Thr Ser Tyr Val Cys Thr Cys Val Pro Gly Tyr Ser Gly Asp Gln
3110 3115 3120
Cys Glu Leu Asp Phe Asp Glu Cys His Ser Asn Pro Cys Arg Asn
3125 3130 3135
Gly Ala Thr Cys Val Asp Gly Phe Asn Thr Phe Arg Cys Leu Cys
3140 3145 3150
Leu Pro Ser Tyr Val Gly Ala Leu Cys Glu Gln Asp Thr Glu Thr
3155 3160 3165
Cys Asp Tyr Gly Trp His Lys Phe Gln Gly Gln Cys Tyr Lys Tyr
3170 3175 3180
Phe Ala His Arg Arg Thr Trp Asp Ala Ala Glu Arg Glu Cys Arg
3185 3190 3195
Leu Gln Gly Ala His Leu Thr Ser Ile Leu Ser His Glu Glu Gln
3200 3205 3210
Met Phe Val Asn Arg Val Gly His Asp Tyr Gln Trp Ile Gly Leu
3215 3220 3225
Asn Asp Lys Met Phe Glu His Asp Phe Arg Trp Thr Asp Gly Ser
3230 3235 3240
Thr Leu Gln Tyr Glu Asn Trp Arg Pro Asn Gln Pro Asp Ser Phe
3245 3250 3255
Phe Ser Ala Gly Glu Asp Cys Val Val Ile Ile Trp His Glu Asn
3260 3265 3270
Gly Gln Trp Asn Asp Val Pro Cys Asn Tyr His Leu Thr Tyr Thr
3275 3280 3285
Cys Lys Lys Gly Thr Val Ala Cys Gly Gln Pro Pro Val Val Glu
3290 3295 3300
Asn Ala Lys Thr Phe Gly Lys Met Lys Pro Arg Tyr Glu Ile Asn
3305 3310 3315
Ser Leu Ile Arg Tyr His Cys Lys Asp Gly Phe Ile Gln Arg His
3320 3325 3330
Leu Pro Thr Ile Arg Cys Leu Gly Asn Gly Arg Trp Ala Ile Pro
3335 3340 3345
Lys Ile Thr Cys Met Asn Pro Ser Ala Tyr Gln Arg Thr Tyr Ser
3350 3355 3360
Met Lys Tyr Phe Lys Asn Ser Ser Ser Ala Lys Asp Asn Ser Ile
3365 3370 3375
Asn Thr Ser Lys His Asp His Arg Trp Ser Arg Arg Trp Gln Glu
3380 3385 3390
Ser Arg Arg Met Phe Ile Asn Ile Lys Ser Ile Leu Trp Met Cys
3395 3400 3405

Ser Thr Leu Ile Val Thr His Ala Leu His Lys Val Lys Val Gly
3410 3415 3420
Lys Ser Pro Pro Val Arg Gly Ser Leu Ser Gly Lys Val Ser Leu
3425 3430 3435
Pro Cys His Phe Ser Thr Met Pro Thr Leu Pro Pro Ser Tyr Asn
3440 3445 3450
Thr Ser Glu Phe Leu Arg Ile Lys Trp Ser Lys Ile Glu Val Asp
3455 3460 3465
Lys Asn Gly Lys Asp Leu Lys Glu Thr Thr Val Leu Val Ala Gln
3470 3475 3480
Asn Gly Asn Ile Lys Ile Gly Gln Asp Tyr Lys Gly Arg Val Ser
3485 3490 3495
Val Pro Thr His Pro Glu Ala Val Gly Asp Ala Ser Leu Thr Val
3500 3505 3510
Val Lys Leu Leu Ala Ser Asp Ala Gly Leu Tyr Arg Cys Asp Val
3515 3520 3525
Met Tyr Gly Ile Glu Asp Thr Gln Asp Thr Val Ser Leu Thr Val
3530 3535 3540
Asp Gly Val Val Phe His Tyr Arg Ala Ala Thr Ser Arg Tyr Thr
3545 3550 3555
Leu Asn Phe Glu Ala Ala Gln Lys Ala Cys Leu Asp Val Gly Ala
3560 3565 3570
Val Ile Ala Thr Pro Glu Gln Leu Phe Ala Ala Tyr Glu Asp Gly
3575 3580 3585
Phe Glu Gln Cys Asp Ala Gly Trp Leu Ala Asp Gln Thr Val Arg
3590 3595 3600
Tyr Pro Ile Arg Ala Pro Arg Val Gly Cys Tyr Gly Asp Lys Met
3605 3610 3615
Gly Lys Ala Gly Val Arg Thr Tyr Gly Phe Arg Ser Pro Gln Glu
3620 3625 3630
Thr Tyr Asp Val Tyr Cys Tyr Val Asp His Leu Asp Gly Asp Val
3635 3640 3645
Phe His Leu Thr Val Pro Ser Lys Phe Thr Phe Glu Glu Ala Ala
3650 3655 3660
Lys Glu Cys Glu Asn Gln Asp Ala Arg Leu Ala Thr Val Gly Glu
3665 3670 3675
Leu Gln Ala Ala Trp Arg Asn Gly Phe Asp Gln Cys Asp Tyr Gly
3680 3685 3690
Trp Leu Ser Asp Ala Ser Val Arg His Pro Val Thr Val Ala Arg
3695 3700 3705
Ala Gln Cys Gly Gly Gly Leu Leu Gly Val Arg Thr Leu Tyr Arg
3710 3715 3720
Phe Glu Asn Gln Thr Gly Phe Pro Pro Pro Asp Ser Arg Phe Asp
3725 3730 3735
Ala Tyr Cys Phe Lys Pro Lys Glu Ala Thr Thr Ile Asp Leu Ser
3740 3745 3750
Ile Leu Ala Glu Thr Ala Ser Pro Ser Leu Ser Lys Glu Pro Gln
3755 3760 3765
Met Val Ser Asp Arg Thr Thr Pro Ile Ile Pro Leu Val Asp Glu
3770 3775 3780
Leu Pro Val Ile Pro Thr Glu Phe Pro Pro Val Gly Asn Ile Val
3785 3790 3795
Ser Phe Glu Gln Lys Ala Thr Val Gln Pro Gln Ala Ile Thr Asp
3800 3805 3810
Ser Leu Ala Thr Lys Leu Pro Thr Pro Thr Gly Ser Thr Lys Lys
3815 3820 3825
Pro Trp Asp Met Asp Asp Tyr Ser Pro Ser Ala Ser Gly Pro Leu
3830 3835 3840
Gly Lys Leu Asp Ile Ser Glu Ile Lys Glu Glu Val Leu Gln Ser
3845 3850 3855
Thr Thr Gly Val Ser His Tyr Ala Thr Asp Ser Trp Asp Gly Val
3860 3865 3870
Val Glu Asp Lys Gln Thr Gln Glu Ser Val Thr Gln Ile Glu Gln
3875 3880 3885
Ile Glu Val Gly Pro Leu Val Thr Ser Met Glu Ile Leu Lys His
3890 3895 3900
Ile Pro Ser Lys Glu Phe Pro Val Thr Glu Thr Pro Leu Val Thr
3905 3910 3915
Ala Arg Met Ile Leu Glu Ser Lys Thr Glu Lys Lys Met Val Ser

3920 3925 3930
Thr Val Ser Glu Leu Val Thr Thr Gly His Tyr Gly Phe Thr Leu
3935 3940 3945
Gly Glu Glu Asp Asp Glu Asp Arg Thr Leu Thr Val Gly Ser Asp
3950 3955 3960
Glu Ser Thr Leu Ile Phe Asp Gln Ile Pro Glu Val Ile Thr Val
3965 3970 3975
Ser Lys Thr Ser Glu Asp Thr Ile His Thr His Leu Glu Asp Leu
3980 3985 3990
Glu Ser Val Ser Ala Ser Thr Thr Val Ser Pro Leu Ile Met Pro
3995 4000 4005
Asp Asn Asn Gly Ser Ser Met Asp Asp Trp Glu Glu Arg Gln Thr
4010 4015 4020
Ser Gly Arg Ile Thr Glu Glu Phe Leu Gly Lys Tyr Leu Ser Thr
4025 4030 4035
Thr Pro Phe Pro Ser Gln His Arg Thr Glu Ile Glu Leu Phe Pro
4040 4045 4050
Tyr Ser Gly Asp Lys Ile Leu Val Glu Gly Ile Ser Thr Val Ile
4055 4060 4065
Tyr Pro Ser Leu Gln Thr Glu Met Thr His Arg Arg Glu Arg Thr
4070 4075 4080
Glu Thr Leu Ile Pro Glu Met Arg Thr Asp Thr Tyr Thr Asp Glu
4085 4090 4095
Ile Gln Glu Glu Ile Thr Lys Ser Pro Phe Met Gly Lys Thr Glu
4100 4105 4110
Glu Glu Val Phe Ser Gly Met Lys Leu Ser Thr Ser Leu Ser Glu
4115 4120 4125
Pro Ile His Val Thr Glu Ser Ser Val Glu Met Thr Lys Ser Phe
4130 4135 4140
Asp Phe Pro Thr Leu Ile Thr Lys Leu Ser Ala Glu Pro Thr Glu
4145 4150 4155
Val Arg Asp Met Glu Glu Asp Phe Thr Ala Thr Pro Gly Thr Thr
4160 4165 4170
Lys Tyr Asp Glu Asn Ile Thr Thr Val Leu Leu Ala His Gly Thr
4175 4180 4185
Leu Ser Val Glu Ala Ala Thr Val Ser Lys Trp Ser Trp Asp Glu
4190 4195 4200
Asp Asn Thr Thr Ser Lys Pro Leu Glu Ser Thr Glu Pro Ser Ala
4205 4210 4215
Ser Ser Lys Leu Pro Pro Ala Leu Leu Thr Thr Val Gly Met Asn
4220 4225 4230
Gly Lys Asp Lys Asp Ile Pro Ser Phe Thr Glu Asp Gly Ala Asp
4235 4240 4245
Glu Phe Thr Leu Ile Pro Asp Ser Thr Gln Lys Gln Leu Glu Glu
4250 4255 4260
Val Thr Asp Glu Asp Ile Ala Ala His Gly Lys Phe Thr Ile Arg
4265 4270 4275
Phe Gln Pro Thr Thr Ser Thr Gly Ile Ala Glu Lys Ser Thr Leu
4280 4285 4290
Arg Asp Ser Thr Thr Glu Glu Lys Val Pro Pro Ile Thr Ser Thr
4295 4300 4305
Glu Gly Gln Val Tyr Ala Thr Met Glu Gly Ser Ala Leu Gly Glu
4310 4315 4320
Val Glu Asp Val Asp Leu Ser Lys Pro Val Ser Thr Val Pro Gln
4325 4330 4335
Phe Ala His Thr Ser Glu Val Glu Gly Leu Ala Phe Val Ser Tyr
4340 4345 4350
Ser Ser Thr Gln Glu Pro Thr Thr Tyr Val Asp Ser Ser His Thr
4355 4360 4365
Ile Pro Leu Ser Val Ile Pro Lys Thr Asp Trp Gly Val Leu Val
4370 4375 4380
Pro Ser Val Pro Ser Glu Asp Glu Val Leu Gly Glu Pro Ser Gln
4385 4390 4395
Asp Ile Leu Val Ile Asp Gln Thr Arg Leu Glu Ala Thr Ile Ser
4400 4405 4410
Pro Glu Thr Met Arg Thr Thr Lys Ile Thr Glu Gly Thr Thr Gln
4415 4420 4425
Glu Glu Phe Pro Trp Lys Glu Gln Thr Ala Glu Lys Pro Val Pro
4430 4435 4440

Ala Leu Ser Ser Thr Ala Trp Thr Pro Lys Glu Ala Val Thr Pro
4445 4450 4455
Leu Asp Glu Gln Glu Gly Asp Gly Ser Ala Tyr Thr Val Ser Glu
4460 4465 4470
Asp Glu Leu Leu Thr Gly Ser Glu Arg Val Pro Val Leu Glu Thr
4475 4480 4485
Thr Pro Val Gly Lys Ile Asp His Ser Val Ser Tyr Pro Pro Gly
4490 4495 4500
Ala Val Thr Glu His Lys Val Lys Thr Asp Glu Val Val Thr Leu
4505 4510 4515
Thr Pro Arg Ile Gly Pro Lys Val Ser Leu Ser Pro Gly Pro Glu
4520 4525 4530
Gln Lys Tyr Glu Thr Glu Gly Ser Ser Thr Thr Gly Phe Thr Ser
4535 4540 4545
Ser Leu Ser Pro Phe Ser Thr His Ile Thr Gln Leu Met Glu Glu
4550 4555 4560
Thr Thr Thr Glu Lys Thr Ser Leu Glu Asp Ile Asp Leu Gly Ser
4565 4570 4575
Gly Leu Phe Glu Lys Pro Lys Ala Thr Glu Leu Ile Glu Phe Ser
4580 4585 4590
Thr Ile Lys Val Thr Val Pro Ser Asp Ile Thr Thr Ala Phe Ser
4595 4600 4605
Ser Val Asp Arg Leu His Thr Thr Ser Ala Phe Lys Pro Ser Ser
4610 4615 4620
Ala Ile Thr Lys Lys Pro Pro Leu Ile Asp Arg Glu Pro Gly Glu
4625 4630 4635
Glu Thr Thr Ser Asp Met Val Ile Ile Gly Glu Ser Thr Ser His
4640 4645 4650
Val Pro Pro Thr Thr Leu Glu Asp Ile Val Ala Lys Glu Thr Glu
4655 4660 4665
Thr Asp Ile Asp Arg Glu Tyr Phe Thr Thr Ser Ser Pro Pro Ala
4670 4675 4680
Thr Gln Pro Thr Arg Pro Pro Thr Val Glu Asp Lys Glu Ala Phe
4685 4690 4695
Gly Pro Gln Ala Leu Ser Thr Pro Gln Pro Pro Ala Ser Thr Lys
4700 4705 4710
Phe His Pro Asp Ile Asn Val Tyr Ile Ile Glu Val Arg Glu Asn
4715 4720 4725
Lys Thr Gly Arg Met Ser Asp Leu Ser Val Ile Gly His Pro Ile
4730 4735 4740
Asp Ser Glu Ser Lys Glu Asp Glu Pro Cys Ser Glu Glu Thr Asp
4745 4750 4755
Pro Val His Asp Leu Met Ala Glu Ile Leu Pro Glu Phe Pro Asp
4760 4765 4770
Ile Ile Glu Ile Asp Leu Tyr His Ser Glu Glu Asn Glu Glu Glu
4775 4780 4785
Glu Glu Glu Cys Ala Asn Ala Thr Asp Val Thr Thr Thr Pro Ser
4790 4795 4800
Val Gln Tyr Ile Asn Gly Lys His Leu Val Thr Thr Val Pro Lys
4805 4810 4815
Asp Pro Glu Ala Ala Glu Ala Arg Arg Gly Gln Phe Glu Ser Val
4820 4825 4830
Ala Pro Ser Gln Asn Phe Ser Asp Ser Ser Glu Ser Asp Thr His
4835 4840 4845
Pro Phe Val Ile Ala Lys Thr Glu Leu Ser Thr Ala Val Gln Pro
4850 4855 4860
Asn Glu Ser Thr Glu Thr Thr Glu Ser Leu Glu Val Thr Trp Lys
4865 4870 4875
Pro Glu Thr Tyr Pro Glu Thr Ser Glu His Phe Ser Gly Gly Glu
4880 4885 4890
Pro Asp Val Phe Pro Thr Val Pro Phe His Glu Glu Phe Glu Ser
4895 4900 4905
Gly Thr Ala Lys Lys Gly Ala Glu Ser Val Thr Glu Arg Asp Thr
4910 4915 4920
Glu Val Gly His Gln Ala His Glu His Thr Glu Pro Val Ser Leu
4925 4930 4935
Phe Pro Glu Glu Ser Ser Gly Glu Ile Ala Ile Asp Gln Glu Ser
4940 4945 4950
Gln Lys Ile Ala Phe Ala Arg Ala Thr Glu Val Thr Phe Gly Glu

4955 4960 4965
Glu Val Glu Lys Ser Thr Ser Val Thr Tyr Thr Pro Thr Ile Val
4970 4975 4980
Pro Ser Ser Ala Ser Ala Tyr Val Ser Glu Glu Glu Ala Val Thr
4985 4990 4995
Leu Ile Gly Asn Pro Trp Pro Asp Asp Leu Leu Ser Thr Lys Glu
5000 5005 5010
Ser Trp Val Glu Ala Thr Pro Arg Gln Val Val Glu Leu Ser Gly
5015 5020 5025
Ser Ser Ser Ile Pro Ile Thr Glu Gly Ser Gly Glu Ala Glu Glu
5030 5035 5040
Asp Glu Asp Thr Met Phe Thr Met Val Thr Asp Leu Ser Gln Arg
5045 5050 5055
Asn Thr Thr Asp Thr Leu Ile Thr Leu Asp Thr Ser Arg Ile Ile
5060 5065 5070
Thr Glu Ser Phe Phe Glu Val Pro Ala Thr Thr Ile Tyr Pro Val
5075 5080 5085
Ser Glu Gln Pro Ser Ala Lys Val Val Pro Thr Lys Phe Val Ser
5090 5095 5100
Glu Thr Asp Thr Ser Glu Trp Ile Ser Ser Thr Thr Val Glu Glu
5105 5110 5115
Lys Lys Arg Lys Glu Glu Glu Gly Thr Thr Gly Thr Ala Ser Thr
5120 5125 5130
Phe Glu Val Tyr Ser Ser Thr Gln Arg Ser Asp Gln Leu Ile Leu
5135 5140 5145
Pro Phe Glu Leu Glu Ser Pro Asn Val Ala Thr Ser Ser Asp Ser
5150 5155 5160
Gly Thr Arg Lys Ser Phe Met Ser Leu Thr Thr Pro Thr Gln Ser
5165 5170 5175
Glu Arg Glu Met Thr Asp Ser Thr Pro Val Phe Thr Glu Thr Asn
5180 5185 5190
Thr Leu Glu Asn Leu Gly Ala Gln Thr Thr Glu His Ser Ser Ile
5195 5200 5205
His Gln Pro Gly Val Gln Glu Gly Leu Thr Thr Leu Pro Arg Ser
5210 5215 5220
Pro Ala Ser Val Phe Met Glu Gln Gly Ser Gly Glu Ala Ala Ala
5225 5230 5235
Asp Pro Glu Thr Thr Thr Val Ser Ser Phe Ser Leu Asn Val Glu
5240 5245 5250
Tyr Ala Ile Gln Ala Glu Lys Glu Val Ala Gly Thr Leu Ser Pro
5255 5260 5265
His Val Glu Thr Thr Phe Ser Thr Glu Pro Thr Gly Leu Val Leu
5270 5275 5280
Ser Thr Val Met Asp Arg Val Val Ala Glu Asn Ile Thr Gln Thr
5285 5290 5295
Ser Arg Glu Ile Val Ile Ser Glu Arg Leu Gly Glu Pro Asn Tyr
5300 5305 5310
Gly Ala Glu Ile Arg Gly Phe Ser Thr Gly Phe Pro Leu Glu Glu
5315 5320 5325
Asp Phe Ser Gly Asp Phe Arg Glu Tyr Ser Thr Val Ser His Pro
5330 5335 5340
Ile Ala Lys Glu Glu Thr Val Met Met Glu Gly Ser Gly Asp Ala
5345 5350 5355
Ala Phe Arg Asp Thr Gln Thr Ser Pro Ser Thr Val Pro Thr Ser
5360 5365 5370
Val His Ile Ser His Ile Ser Asp Ser Glu Gly Pro Ser Ser Thr
5375 5380 5385
Met Val Ser Thr Ser Ala Phe Pro Trp Glu Glu Phe Thr Ser Ser
5390 5395 5400
Ala Glu Gly Ser Gly Glu Gln Leu Val Thr Val Ser Ser Ser Val
5405 5410 5415
Val Pro Val Leu Pro Ser Ala Val Gln Lys Phe Ser Gly Thr Ala
5420 5425 5430
Ser Ser Ile Ile Asp Glu Gly Leu Gly Glu Val Gly Thr Val Asn
5435 5440 5445
Glu Ile Asp Arg Arg Ser Thr Ile Leu Pro Thr Ala Glu Val Glu
5450 5455 5460
Gly Thr Lys Ala Pro Val Glu Lys Glu Glu Val Lys Val Ser Gly
5465 5470 5475

Thr Val Ser Thr Asn Phe Pro Gln Thr Ile Glu Pro Ala Lys Leu
5480 5485 5490
Trp Ser Arg Gln Glu Val Asn Pro Val Arg Gln Glu Ile Glu Ser
5495 5500 5505
Glu Thr Thr Ser Glu Glu Gln Ile Gln Glu Glu Lys Ser Phe Glu
5510 5515 5520
Ser Pro Gln Asn Ser Pro Ala Thr Glu Gln Thr Ile Phe Asp Ser
5525 5530 5535
Gln Thr Phe Thr Glu Thr Glu Leu Lys Thr Thr Asp Tyr Ser Val
5540 5545 5550
Leu Thr Thr Lys Lys Thr Tyr Ser Asp Asp Lys Glu Met Lys Glu
5555 5560 5565
Glu Asp Thr Ser Leu Val Asn Met Ser Thr Pro Asp Pro Asp Ala
5570 5575 5580
Asn Gly Leu Glu Ser Tyr Thr Thr Leu Pro Glu Ala Thr Glu Lys
5585 5590 5595
Ser His Phe Phe Leu Ala Thr Ala Leu Val Thr Glu Ser Ile Pro
5600 5605 5610
Ala Glu His Val Val Thr Asp Ser Pro Ile Lys Lys Glu Glu Ser
5615 5620 5625
Thr Lys His Phe Pro Lys Gly Met Arg Pro Thr Ile Gln Glu Ser
5630 5635 5640
Asp Thr Glu Leu Leu Phe Ser Gly Leu Gly Ser Gly Glu Glu Val
5645 5650 5655
Leu Pro Thr Leu Pro Thr Glu Ser Val Asn Phe Thr Glu Val Glu
5660 5665 5670
Gln Ile Asn Asn Thr Leu Tyr Pro His Thr Ser Gln Val Glu Ser
5675 5680 5685
Thr Ser Ser Asp Lys Ile Glu Asp Phe Asn Arg Met Glu Asn Val
5690 5695 5700
Ala Lys Glu Val Gly Pro Leu Val Ser Gln Thr Asp Ile Phe Glu
5705 5710 5715
Gly Ser Gly Ser Val Thr Ser Thr Thr Leu Ile Glu Ile Leu Ser
5720 5725 5730
Asp Thr Gly Ala Glu Gly Pro Thr Val Ala Pro Leu Pro Phe Ser
5735 5740 5745
Thr Asp Ile Gly His Pro Gln Asn Gln Thr Val Arg Trp Ala Glu
5750 5755 5760
Glu Ile Gln Thr Ser Arg Pro Gln Thr Ile Thr Glu Gln Asp Ser
5765 5770 5775
Asn Lys Asn Ser Ser Thr Ala Glu Ile Asn Glu Thr Thr Thr Ser
5780 5785 5790
Ser Thr Asp Phe Leu Ala Arg Ala Tyr Gly Phe Glu Met Ala Lys
5795 5800 5805
Glu Phe Val Thr Ser Ala Pro Lys Pro Ser Asp Leu Tyr Tyr Glu
5810 5815 5820
Pro Ser Gly Glu Gly Ser Gly Glu Val Asp Ile Val Asp Ser Phe
5825 5830 5835
His Thr Ser Ala Thr Thr Gln Ala Thr Arg Gln Glu Ser Ser Thr
5840 5845 5850
Thr Phe Val Ser Asp Gly Ser Leu Glu Lys His Pro Glu Val Pro
5855 5860 5865
Ser Ala Lys Ala Val Thr Ala Asp Gly Phe Pro Thr Val Ser Val
5870 5875 5880
Met Leu Pro Leu His Ser Glu Gln Asn Lys Ser Ser Pro Asp Pro
5885 5890 5895
Thr Ser Thr Leu Ser Asn Thr Val Ser Tyr Glu Arg Ser Thr Asp
5900 5905 5910
Gly Ser Phe Gln Asp Arg Phe Arg Glu Phe Glu Asp Ser Thr Leu
5915 5920 5925
Lys Pro Asn Arg Lys Lys Pro Thr Glu Asn Ile Ile Ile Asp Leu
5930 5935 5940
Asp Lys Glu Asp Lys Asp Leu Ile Leu Thr Ile Thr Glu Ser Thr
5945 5950 5955
Ile Leu Glu Ile Leu Pro Glu Leu Thr Ser Asp Lys Asn Thr Ile
5960 5965 5970
Ile Asp Ile Asp His Thr Lys Pro Val Tyr Glu Asp Ile Leu Gly
5975 5980 5985
Met Gln Thr Asp Ile Asp Thr Glu Val Pro Ser Glu Pro His Asp

5990 5995 6000
Ser Asn Asp Glu Ser Asn Asp Asp Ser Thr Gln Val Gln Glu Ile
6005 6010 6015
Tyr Glu Ala Ala Val Asn Leu Ser Leu Thr Glu Glu Thr Phe Glu
6020 6025 6030
Gly Ser Ala Asp Val Leu Ala Ser Tyr Thr Gln Ala Thr His Asp
6035 6040 6045
Glu Ser Met Thr Tyr Glu Asp Arg Ser Gln Leu Asp His Met Gly
6050 6055 6060
Phe His Phe Thr Thr Gly Ile Pro Ala Pro Ser Thr Glu Thr Glu
6065 6070 6075
Leu Asp Val Leu Leu Pro Thr Ala Thr Ser Leu Pro Ile Pro Arg
6080 6085 6090
Lys Ser Ala Thr Val Ile Pro Glu Ile Glu Gly Ile Lys Ala Glu
6095 6100 6105
Ala Lys Ala Leu Asp Asp Met Phe Glu Ser Ser Thr Leu Ser Asp
6110 6115 6120
Gly Gln Ala Ile Ala Asp Gln Ser Glu Ile Ile Pro Thr Leu Gly
6125 6130 6135
Gln Phe Glu Arg Thr Gln Glu Glu Tyr Glu Asp Lys Lys His Ala
6140 6145 6150
Gly Pro Ser Phe Gln Pro Glu Phe Ser Ser Gly Ala Glu Glu Ala
6155 6160 6165
Leu Val Asp His Thr Pro Tyr Leu Ser Ile Ala Thr Thr His Leu
6170 6175 6180
Met Asp Gln Ser Val Thr Glu Val Pro Asp Val Met Glu Gly Ser
6185 6190 6195
Asn Pro Pro Tyr Tyr Thr Asp Thr Thr Leu Ala Val Ser Thr Phe
6200 6205 6210
Ala Lys Leu Ser Ser Gln Thr Pro Ser Ser Pro Leu Thr Ile Tyr
6215 6220 6225
Ser Gly Ser Glu Ala Ser Gly His Thr Glu Ile Pro Gln Pro Ser
6230 6235 6240
Ala Leu Pro Gly Ile Asp Val Gly Ser Ser Val Met Ser Pro Gln
6245 6250 6255
Asp Ser Phe Lys Glu Ile His Val Asn Ile Glu Ala Thr Phe Lys
6260 6265 6270
Pro Ser Ser Glu Glu Tyr Leu His Ile Thr Glu Pro Pro Ser Leu
6275 6280 6285
Ser Pro Asp Thr Lys Leu Glu Pro Ser Glu Asp Asp Gly Lys Pro
6290 6295 6300
Glu Leu Leu Glu Glu Met Glu Ala Ser Pro Thr Glu Leu Ile Ala
6305 6310 6315
Val Glu Gly Thr Glu Ile Leu Gln Asp Phe Gln Asn Lys Thr Asp
6320 6325 6330
Gly Gln Val Ser Gly Glu Ala Ile Lys Met Phe Pro Thr Ile Lys
6335 6340 6345
Thr Pro Glu Ala Gly Thr Val Ile Thr Thr Ala Asp Glu Ile Glu
6350 6355 6360
Leu Glu Gly Ala Thr Gln Trp Pro His Ser Thr Ser Ala Ser Ala
6365 6370 6375
Thr Tyr Gly Val Glu Ala Gly Val Val Pro Trp Leu Ser Pro Gln
6380 6385 6390
Thr Ser Glu Arg Pro Thr Leu Ser Ser Ser Pro Glu Ile Asn Pro
6395 6400 6405
Glu Thr Gln Ala Ala Leu Ile Arg Gly Gln Asp Ser Thr Ile Ala
6410 6415 6420
Ala Ser Glu Gln Gln Val Ala Ala Arg Ile Leu Asp Ser Asn Asp
6425 6430 6435
Gln Ala Thr Val Asn Pro Val Glu Phe Asn Thr Glu Val Ala Thr
6440 6445 6450
Pro Pro Phe Ser Leu Leu Glu Thr Ser Asn Glu Thr Asp Phe Leu
6455 6460 6465
Ile Gly Ile Asn Glu Glu Ser Val Glu Gly Thr Ala Ile Tyr Leu
6470 6475 6480
Pro Gly Pro Asp Arg Cys Lys Met Asn Pro Cys Leu Asn Gly Gly
6485 6490 6495
Thr Cys Tyr Pro Thr Glu Thr Ser Tyr Val Cys Thr Cys Val Pro
6500 6505 6510

```
Gly Tyr  Ser Gly Asp Gln Cys  Glu Leu Asp Phe Asp  Glu Cys His
    6515                 6520                 6525
Ser Asn  Pro Cys Arg Asn Gly  Ala Thr Cys Val Asp  Gly Phe Asn
    6530                 6535                 6540
Thr Phe  Arg Cys Leu Cys Leu  Pro Ser Tyr Val Gly  Ala Leu Cys
    6545                 6550                 6555
Glu Gln  Asp Thr Glu Thr Cys  Asp Tyr Gly Trp His  Lys Phe Gln
    6560                 6565                 6570
Gly Gln  Cys Tyr Lys Tyr Phe  Ala His Arg Arg Thr  Trp Asp Ala
    6575                 6580                 6585
Ala Glu  Arg Glu Cys Arg Leu  Gln Gly Ala His Leu  Thr Ser Ile
    6590                 6595                 6600
Leu Ser  His Glu Glu Gln Met  Phe Val Asn Arg Val  Gly His Asp
    6605                 6610                 6615
Tyr Gln  Trp Ile Gly Leu Asn  Asp Lys Met Phe Glu  His Asp Phe
    6620                 6625                 6630
Arg Trp  Thr Asp Gly Ser Thr  Leu Gln Tyr Glu Asn  Trp Arg Pro
    6635                 6640                 6645
Asn Gln  Pro Asp Ser Phe Phe  Ser Ala Gly Glu Asp  Cys Val Val
    6650                 6655                 6660
Ile Ile  Trp His Glu Asn Gly  Gln Trp Asn Asp Val  Pro Cys Asn
    6665                 6670                 6675
Tyr His  Leu Thr Tyr Thr Cys  Lys Lys Gly Thr Val  Ala Cys Gly
    6680                 6685                 6690
Gln Pro  Pro Val Val Glu Asn  Ala Lys Thr Phe Gly  Lys Met Lys
    6695                 6700                 6705
Pro Arg  Tyr Glu Ile Asn Ser  Leu Ile Arg Tyr His  Cys Lys Asp
    6710                 6715                 6720
Gly Phe  Ile Gln Arg His Leu  Pro Thr Ile Arg Cys  Leu Gly Asn
    6725                 6730                 6735
Gly Arg  Trp Ala Ile Pro Lys  Ile Thr Cys Met Asn  Pro Ser Ala
    6740                 6745                 6750
Tyr Gln  Arg Thr Tyr Ser Met  Lys Tyr Phe Lys Asn  Ser Ser Ser
    6755                 6760                 6765
Ala Lys  Asp Asn Ser Ile Asn  Thr Ser Lys His Asp  His Arg Trp
    6770                 6775                 6780
Ser Arg  Arg Trp Gln Glu Ser  Arg Arg
    6785                 6790
```

<210> 331
<211> 24
<212> DNA
<213> Homo sapiens
<400> 331
tcctgcccta agagccatag ggaa 24

<210> 332
<211> 23
<212> DNA
<213> Homo sapiens
<400> 332
gagcttctga attgccaatt gtg 23

<210> 333
<211> 26
<212> DNA
<213> Homo sapiens
<400> 333
gagtctgttc atctgtacca gtgaca 26

<210> 334
<211> 32
<212> DNA
<213> Homo sapiens
<400> 334
ctgcagctga caaaattcct gggttactag gt 32

<210> 335
<211> 21

<212> DNA
<213> Homo sapiens
<400> 335
gcattcttag aacgcggttc a 21

<210> 336
<211> 21
<212> DNA
<213> Homo sapiens
<400> 336
ttggatgcaa tcagcttctg a 21

<210> 337
<211> 25
<212> DNA
<213> Homo sapiens
<400> 337
tctcgcttcc gcttcgcagt ttttg 25

<210> 338
<211> 19
<212> DNA
<213> Homo sapiens
<400> 338
gcaggtagtt gccgaagca 19

<210> 339
<211> 22
<212> DNA
<213> Homo sapiens
<400> 339
tggaggagtc tcgtcacttt ca 22

<210> 340
<211> 26
<212> DNA
<213> Homo sapiens
<400> 340
tctccattag gcacattcag tccact 26

<210> 341
<211> 21
<212> DNA
<213> Homo sapiens
<400> 341
gggtgggaag aaagaatgca a 21

<210> 342
<211> 19
<212> DNA
<213> Homo sapiens
<400> 342
acccaggaag cccctcatc 19

<210> 343
<211> 27
<212> DNA
<213> Homo sapiens
<400> 343
ccaaaggaac caaatcagaa cagctca 27

<210> 344
<211> 22
<212> DNA
<213> Homo sapiens
<400> 344
ccaaggacaa cagtggtgaa aa 22

<210> 345

<211> 26
<212> DNA
<213> Homo sapiens
<400> 345
gaaattggtg agactgtcaa attcag 26

<210> 346
<211> 27
<212> DNA
<213> Homo sapiens
<400> 346
agtcgccaca gatgtaccca ctagccc 27

<210> 347
<211> 24
<212> DNA
<213> Homo sapiens
<400> 347
ttctgtatac gcagctcagt ttcc 24

<210> 348
<211> 20
<212> DNA
<213> Homo sapiens
<400> 348
cttccgctga ctcacagcaa 20

<210> 349
<211> 26
<212> DNA
<213> Homo sapiens
<400> 349
aagagcttga cccaagtccg agccat 26

<210> 350
<211> 21
<212> DNA
<213> Homo sapiens
<400> 350
cactcatgcc aggacattgg t 21

<210> 351
<211> 25
<212> DNA
<213> Homo sapiens
<400> 351
caaacttaag ctccccagag tacat 25

<210> 352
<211> 32
<212> DNA
<213> Homo sapiens
<400> 352
caagaaaacc acctaaatat gaaagattca tc 32

<210> 353
<211> 21
<212> DNA
<213> Homo sapiens
<400> 353
tgctttgttg gagatggctt t 21

<210> 354
<211> 19
<212> DNA
<213> Homo sapiens
<400> 354
ttgaaacgca agcccattg 19

<210> 355
<211> 29
<212> DNA
<213> Homo sapiens
<400> 355
aacttaacta aattcatcgc catcaagaa 29

<210> 356
<211> 21
<212> DNA
<213> Homo sapiens
<400> 356
tggccaagaa tgtggtgaaa g 21

<210> 357
<211> 20
<212> DNA
<213> Homo sapiens
<400> 357
tcaggagttt gctgcttgca 20

<210> 358
<211> 27
<212> DNA
<213> Homo sapiens
<400> 358
agaagatccc cccagtcaac accaacc 27

<210> 359
<211> 25
<212> DNA
<213> Homo sapiens
<400> 359
tccttgagct agacctctcc tacaa 25

<210> 360
<211> 24
<212> DNA
<213> Homo sapiens
<400> 360
actcattgat cctattgcct tgga 24

<210> 361
<211> 22
<212> DNA
<213> Homo sapiens
<400> 361
catccaacgc cgtcgctgtg ac 22

<210> 362
<211> 21
<212> DNA
<213> Homo sapiens
<400> 362
tgtctttgcc aatgtcgctt a 21

<210> 363
<211> 21
<212> DNA
<213> Homo sapiens
<400> 363
aacagaaatg ggcatgatcc a 21

<210> 364
<211> 20
<212> DNA
<213> Homo sapiens
<400> 364
tcggatgccc agctcagccg 20

<210> 365
<211> 20
<212> DNA
<213> Homo sapiens
<400> 365
tgcgtcaacg tgctcaagag 20

<210> 366
<211> 22
<212> DNA
<213> Homo sapiens
<400> 366
ggcacttgat ggtcagcagt ac 22

<210> 367
<211> 28
<212> DNA
<213> Homo sapiens
<400> 367
aaagtgagac cctccacctt gtccaggt 28

<210> 368
<211> 21
<212> DNA
<213> Homo sapiens
<400> 368
gctgatccag cctcactatg c 21

<210> 369
<211> 21
<212> DNA
<213> Homo sapiens
<400> 369
agatccttgc agcaccagtt g 21

<210> 370
<211> 21
<212> DNA
<213> Homo sapiens
<400> 370
catgccatgc agtgcgttca g 21

<210> 371
<211> 22
<212> DNA
<213> Homo sapiens
<400> 371
gtgtgccctg ctatgagaaa ca 22

<210> 372
<211> 17
<212> DNA
<213> Homo sapiens
<400> 372
cccctcccgt ggtgatg 17

<210> 373
<211> 29
<212> DNA
<213> Homo sapiens
<400> 373
agccaggaga cgtaccttta ccacataga 29

<210> 374
<211> 20
<212> DNA
<213> Homo sapiens
<400> 374

gggaggacaa cagcgatgag 20

<210> 375
<211> 19
<212> DNA
<213> Homo sapiens
<400> 375
ccccgtagag gctgtcgtt 19

<210> 376
<211> 28
<212> DNA
<213> Homo sapiens
<400> 376
cacagctacg gtgacatttc tgccactg 28

<210> 377
<211> 24
<212> DNA
<213> Homo sapiens
<400> 377
aaatccgttc agtgatcaga caga 24

<210> 378
<211> 26
<212> DNA
<213> Homo sapiens
<400> 378
cagatcaaat gaacaagaaa cttcca 26

<210> 379
<211> 30
<212> DNA
<213> Homo sapiens
<400> 379
tctcagaatg cacaaaaaga aagtgacgca 30

<210> 380
<211> 20
<212> DNA
<213> Homo sapiens
<400> 380
tgaacatgga cggcaaagag 20

<210> 381
<211> 21
<212> DNA
<213> Homo sapiens
<400> 381
ccaatcacag catgggttca g 21

<210> 382
<211> 20
<212> DNA
<213> Homo sapiens
<400> 382
caggtggaaa aggccaaggt 20

<210> 383
<211> 21
<212> DNA
<213> Homo sapiens
<400> 383
aagagcatcc agcaacaacc a 21

<210> 384
<211> 16
<212> DNA
<213> Homo sapiens

<400> 384
ccaagcgccg tggcca 16

<210> 385
<211> 29
<212> DNA
<213> Homo sapiens
<400> 385
tctataccat ccttattcaa aacttgcat 29

<210> 386
<211> 19
<212> DNA
<213> Homo sapiens
<400> 386
gtgccaccaa cccattttg 19

<210> 387
<211> 29
<212> DNA
<213> Homo sapiens
<400> 387
gtatttccta aatggtacct gtatatgca 29

<210> 388
<211> 28
<212> DNA
<213> Homo sapiens
<400> 388
acaggcaata tcaatcttcc tccgggct 28

<210> 389
<211> 20
<212> DNA
<213> Homo sapiens
<400> 389
ccccaggcgc taatagatca 20

<210> 390
<211> 21
<212> DNA
<213> Homo sapiens
<400> 390
ctgctccaac atttggtttc c 21

<210> 391
<211> 19
<212> DNA
<213> Homo sapiens
<400> 391
atgccctttt gccgatgca 19

<210> 392
<211> 24
<212> DNA
<213> Homo sapiens
<400> 392
gccaaattgt gtttgatgga ttaa 24

<210> 393
<211> 27
<212> DNA
<213> Homo sapiens
<400> 393
gacaaaaccg agtcacatca gtaatag 27

<210> 394
<211> 27
<212> DNA

<213> Homo sapiens
<400> 394
tccccctgaa aagtgagcag caacgta 27

<210> 395
<211> 25
<212> DNA
<213> Homo sapiens
<400> 395
cagatttgag ctatcagacc aacaa 25

<210> 396
<211> 25
<212> DNA
<213> Homo sapiens
<400> 396
aaattcaaga gaggcttcac atacg 25

<210> 397
<211> 24
<212> DNA
<213> Homo sapiens
<400> 397
cgttctgcaa gcatcagttc tacg 24

<210> 398
<211> 27
<212> DNA
<213> Homo sapiens
<400> 398
agagaaaaac aaaacccccta agagact 27

<210> 399
<211> 24
<212> DNA
<213> Homo sapiens
<400> 399
gcacaaggaa atcttgttga tgat 24

<210> 400
<211> 23
<212> DNA
<213> Homo sapiens
<400> 400
ccacagcatg aagggcaacc agc 23

<210> 401
<211> 19
<212> DNA
<213> Homo sapiens
<400> 401
gccctgtcac gctgtacga 19

<210> 402
<211> 22
<212> DNA
<213> Homo sapiens
<400> 402
tcttgtcttt gcggtatttc ca 22

<210> 403
<211> 24
<212> DNA
<213> Homo sapiens
<400> 403
ttgatagaaa cgctgtgagc tcga 24

<210> 404
<211> 22

<212> DNA
<213> Homo sapiens
<400> 404
agctcatcaa ggcaattggt tt 22

<210> 405
<211> 28
<212> DNA
<213> Homo sapiens
<400> 405
acaagatcat gcaagttatc aagaagtt 28

<210> 406
<211> 27
<212> DNA
<213> Homo sapiens
<400> 406
atgaaggtac agcccaagca ccttggg 27

<210> 407
<211> 25
<212> DNA
<213> Homo sapiens
<400> 407
gctgtgaatt tactggacag attcc 25

<210> 408
<211> 24
<212> DNA
<213> Homo sapiens
<400> 408
aaataaaagc agctcagtcc aaca 24

<210> 409
<211> 30
<212> DNA
<213> Homo sapiens
<400> 409
atcctggcac agatttcagc tcctactcca 30

<210> 410
<211> 19
<212> DNA
<213> Homo sapiens
<400> 410
gaaggaggct ggccacagt 19

<210> 411
<211> 20
<212> DNA
<213> Homo sapiens
<400> 411
ttgaacgcag gaccttccat 20

<210> 412
<211> 38
<212> DNA
<213> Homo sapiens
<400> 412
tgtacagaat atatccacat ccgtccacaa taaatcct 38

<210> 413
<211> 27
<212> DNA
<213> Homo sapiens
<400> 413
taaaacagaa ggaaactaat ggacctt 27

<210> 414

<211> 25
<212> DNA
<213> Homo sapiens
<400> 414
cagtccactt ccatatgtgt tgttc 25

<210> 415
<211> 21
<212> DNA
<213> Homo sapiens
<400> 415
cacttcacgc aatgcttggc a 21

<210> 416
<211> 19
<212> DNA
<213> Homo sapiens
<400> 416
tgcgtgactt gccatgaga 19

<210> 417
<211> 24
<212> DNA
<213> Homo sapiens
<400> 417
ggtaagtgat tcctccagat gtga 24

<210> 418
<211> 24
<212> DNA
<213> Homo sapiens
<400> 418
caaacagccc attaagttcc ctgg 24

<210> 419
<211> 25
<212> DNA
<213> Homo sapiens
<400> 419
cagaaggtaa agccatgttt tgact 25

<210> 420
<211> 21
<212> DNA
<213> Homo sapiens
<400> 420
gggacagatg gacaggaagg t 21

<210> 421
<211> 27
<212> DNA
<213> Homo sapiens
<400> 421
ctgatgtgga tgacccagag gcattcc 27

<210> 422
<211> 22
<212> DNA
<213> Homo sapiens
<400> 422
accgacaagt caagtgcttc tg 22

<210> 423
<211> 24
<212> DNA
<213> Homo sapiens
<400> 423
ggttgtctta tggcatccag ttaa 24

<210> 424
<211> 28
<212> DNA
<213> Homo sapiens
<400> 424
tttctgattc tgcatgagaa ccttcgca 28

<210> 425
<211> 19
<212> DNA
<213> Homo sapiens
<400> 425
gagagcggag ggcagaaga 19

<210> 426
<211> 19
<212> DNA
<213> Homo sapiens
<400> 426
gagagcggag ggcagaaga 19

<210> 427
<211> 22
<212> DNA
<213> Homo sapiens
<400> 427
ccccacccct ctgctggtcc tg 22

<210> 428
<211> 20
<212> DNA
<213> Homo sapiens
<400> 428
ccagatgcct tggtccaaag 20

<210> 429
<211> 23
<212> DNA
<213> Homo sapiens
<400> 429
gcagcttata gcaccaacac gtt 23

<210> 430
<211> 32
<212> DNA
<213> Homo sapiens
<400> 430
cccaaagttt cataaagccc ctaagctcat ga 32

<210> 431
<211> 25
<212> DNA
<213> Homo sapiens
<400> 431
aatggaaaac aacctctgag tttga 25

<210> 432
<211> 22
<212> DNA
<213> Homo sapiens
<400> 432
tgtgggcaaa gagttgatga aa 22

<210> 433
<211> 21
<212> DNA
<213> Homo sapiens
<400> 433
cttcgtgagt gtcccgtgca c 21

<210> 434
<211> 21
<212> DNA
<213> Homo sapiens
<400> 434
ctcgtacctc agcatgccat t 21

<210> 435
<211> 20
<212> DNA
<213> Homo sapiens
<400> 435
gtgccgaggc gtagatgaag 20

<210> 436
<211> 25
<212> DNA
<213> Homo sapiens
<400> 436
acgtgcagtc aggtgtcttc ataca 25

<210> 437
<211> 20
<212> DNA
<213> Homo sapiens
<400> 437
catcggagtc ggagcttagg 20

<210> 438
<211> 20
<212> DNA
<213> Homo sapiens
<400> 438
ctcgccattc gactcttgct 20

<210> 439
<211> 26
<212> DNA
<213> Homo sapiens
<400> 439
aattctaatg tagcaaaacg taacca 26

<210> 440
<211> 25
<212> DNA
<213> Homo sapiens
<400> 440
tgaaacgatt agctgtagcc aaatt 25

<210> 441
<211> 29
<212> DNA
<213> Homo sapiens
<400> 441
cagtagattt accacacata ttgcatttt 29

<210> 442
<211> 39
<212> DNA
<213> Homo sapiens
<400> 442
aacatagttt tcctatttca ggcagagtgc ggtatattc 39

<210> 443
<211> 29
<212> DNA
<213> Homo sapiens
<400> 443

ggtgtacaag tcgtttttgg tataacttc 29

<210> 444
<211> 27
<212> DNA
<213> Homo sapiens
<400> 444
gctaagtgag taggaaacag tgtttcc 27

<210> 445
<211> 26
<212> DNA
<213> Homo sapiens
<400> 445
tgtttctaca cctgtgctat ggactc 26

<210> 446
<211> 23
<212> DNA
<213> Homo sapiens
<400> 446
tgaagcagaa cctccttcag aag 23

<210> 447
<211> 19
<212> DNA
<213> Homo sapiens
<400> 447
tccaatttgg gcagttcca 19

<210> 448
<211> 32
<212> DNA
<213> Homo sapiens
<400> 448
attatccttc gaaaaacatc cacagcagtc tg 32

<210> 449
<211> 25
<212> DNA
<213> Homo sapiens
<400> 449
agctttttg gaatcttctg ctaaa 25

<210> 450
<211> 19
<212> DNA
<213> Homo sapiens
<400> 450
gccccgtcca ttttttctg 19

<210> 451
<211> 28
<212> DNA
<213> Homo sapiens
<400> 451
cagactagcc atgacttgaa tgccagca 28

<210> 452
<211> 29
<212> DNA
<213> Homo sapiens
<400> 452
aaagagcgta tgaaaagtac gttagactt 29

<210> 453
<211> 27
<212> DNA
<213> Homo sapiens

<400> 453
tgacaaacac gacataaata acacaca 27

<210> 454
<211> 29
<212> DNA
<213> Homo sapiens
<400> 454
ttcccctgaa ggatgctaag gaatacgct 29

<210> 455
<211> 24
<212> DNA
<213> Homo sapiens
<400> 455
aacaagtgcg acctctcaga tatt 24

<210> 456
<211> 20
<212> DNA
<213> Homo sapiens
<400> 456
aaccacgatg gcacctatgg 20

<210> 457
<211> 26
<212> DNA
<213> Homo sapiens
<400> 457
cacctcatct aatataaaaa aggcaa 26

<210> 458
<211> 22
<212> DNA
<213> Homo sapiens
<400> 458
tcaatacctg cagctggtga at 22

<210> 459
<211> 28
<212> DNA
<213> Homo sapiens
<400> 459
ggtgcatact gactagcatt aaaatttt 28

<210> 460
<211> 27
<212> DNA
<213> Homo sapiens
<400> 460
tcatcccctg actgtgtgaa aaaagta 27

<210> 461
<211> 25
<212> DNA
<213> Homo sapiens
<400> 461
gaccaaatgt aattcggatc agatc 25

<210> 462
<211> 27
<212> DNA
<213> Homo sapiens
<400> 462
gaaactctgt gacaatcctt cactaga 27

<210> 463
<211> 25
<212> DNA

<213> Homo sapiens
<400> 463
ttttgaaaga tctccaccac aacgt 25

<210> 464
<211> 24
<212> DNA
<213> Homo sapiens
<400> 464
cttgcttcct cattgacttc atgt 24

<210> 465
<211> 24
<212> DNA
<213> Homo sapiens
<400> 465
ggcttgctgt gtctctctat cttg 24

<210> 466
<211> 17
<212> DNA
<213> Homo sapiens
<400> 466
ccgccgcgtc ccgaact 17

<210> 467
<211> 19
<212> DNA
<213> Homo sapiens
<400> 467
gtgctgacgg gacccttct 19

<210> 468
<211> 21
<212> DNA
<213> Homo sapiens
<400> 468
acgagagcga aactccattt g 21

<210> 469
<211> 23
<212> DNA
<213> Homo sapiens
<400> 469
ccctgacttc cgcaacatga cgg 23

<210> 470
<211> 21
<212> DNA
<213> Homo sapiens
<400> 470
gctggctgac aacttcatcc a 21

<210> 471
<211> 21
<212> DNA
<213> Homo sapiens
<400> 471
gatggcattg cgagacagtg t 21

<210> 472
<211> 1270
<212> DNA
<213> Homo sapiens
<400> 472
atttctaaa agggacagag agcaccctgc tacatttcct aatcaagaag ttggcgtgca 60
gctgggagag ctagactaag ttggtcatga tgcagaagct actcaaatgc agtcggcttg 120
tcctggctct tgccctcatc ctggttctgg aatcctcagt tcaaggttat cctacgcgga 180
gagccaggta ccaatgggtg cgctgcaatc cagacagtaa ttctgcaaac tgccttgaag 240

```
aaaaaggacc aatgttcgaa ctacttccag gtgaatccaa caagatcccc cgtctgagga    300
ctgacctttt tccaaagacg agaatccagg acttgaatcg tatcttccca ctttctgagg    360
actactctgg atcaggcttc ggctccggct ccggctctgg atcaggatct gggagtggct    420
tcctaacgga aatggaacag gattaccaac tagtagacga aagtgatgct ttccatgaca    480
accttaggtc tcttgacagg aatctgccct cagacagcca ggacttgggt caacatggat    540
tagaagagga ttttatgtta taaaagagga ttttcccacc ttgacaccag gcaatgtagt    600
tagcatattt tatgtaccat ggttatatga ttaatcttgg gacaaagaat tttatagaaa    660
tttttaaaca tctgaaaaag aagcttaagt tttatcatcc tttttttttct catgaattct    720
taaaggatta tgctttaatg ctgttatcta ttttattgtt cttgaaaata cctgcatttt    780
ttggtatcat gttcaaccaa catcattatg aaattaatta gattcccatg gccataaaat    840
ggctttaaag aatatatata tatttttaaa gtagcttgag aagcaaattg gcaggtaata    900
tttcatacct aaattaagac tctgacttgg attgtgaatt ataatgatat gccccttttc    960
ttataaaaac aaaaaaaaaa ataatgaaac acagtgaatt tgtagagtgg gggtatttga   1020
catattttac agggtggagt gtactatata ctattacctt tgaatgtgtt tgcagagcta   1080
gtggatgtgt ttgtctacaa gtatgattgc tgttacataa caccccaaat taactcccaa   1140
attaaaacac agttgtgctg tcaatacctc atactgcttt accttttttt cctggatatc   1200
tgtgtatttt caaatgttac tatatattaa agcagaaata taaccaaagg ttaaaaaaaa   1260
aaaaaaaaaa                                                          1270

<210>  473
<211>  2881
<212>  DNA
<213>  Homo sapiens
<400>  473
acagaagtgc tagaagccag tgctcgtgaa ctaaggagaa aaagaacaga caagggaaca     60
gcctggacat ggcatcagag atccacatga caggcccaat gtgcctcatt gagaacacta    120
atgggcgact gatggcgaat ccagaagctc tgaagatcct ttctgccatt acacagccta    180
tggtggtggt ggcaattgtg ggcctctacc gcacaggcaa atcctacctg atgaacaagc    240
tggctggaaa gaaaaagggc ttctctctgg gctccacggt gcagtctcac actaaaggaa    300
tctggatgtg gtgtgtgccc caccccaaga agccaggcca catcctagtt ctgctggaca    360
ccgagggtct gggagatgta gagaagggtg acaaccagaa tgactcctgg atcttcgccc    420
tggccgtcct cctgagcagc accttcgtgt acaatagcat aggaaccatc aaccagcagg    480
ctatggacca actgtactat gtgacagagc tgacacatag aatccgatca aaatcctcac    540
ctgatgagaa tgagaatgag gttgaggatt cagctgactt tgtgagcttc ttcccagact    600
ttgtgtggac actgagagat ttctccctgg acttggaagc agatggacaa cccctcacac    660
cagatgagta cctgacatac tccctgaagc tgaagaaagg taccagtcaa aaagatgaaa    720
cttttaacct gcccagactc tgtatccgga aattcttccc aaagaaaaaa tgctttgtct    780
ttgatcggcc cgttcaccgc aggaagcttg cccagctcga gaaactacaa gatgaagagc    840
tggaccccga atttgtgcaa caagtagcag acttctgttc ctacatcttt agtaattcca    900
aaactaaaac tctttcagga ggcatccagg tcaacgggcc tcgtctagag agcctggtgc    960
tgacctacgt caatgccatc agcagtgggg atctgccgtg catggagaac gcagtcctgg   1020
ccttggccca gatagagaac tcagctgcag tgcaaaaggc tattgcccac tatgaacagc   1080
agatgggcca gaaggtgcag ctgcccacag aaagcctcca ggagctgctg gacctgcaca   1140
gggacagtga gagagaggcc attgaagtct tcatcaggag ttccttcaaa gatgtggacc   1200
atctatttca aaaggagtta gcggcccagc tagaaaaaaa gcgggatgac ttttgtaaac   1260
agaatcagga agcatcatca gatcgttgct caggtttact tcaggtcatt ttcagtcctc   1320
tagaagaaga agtgaaggcg ggaatttatt cgaaaccagg gggctatcgt ctctttgttc   1380
agaagctaca agacctgaag aaaaagtact atgaggaacc gaggaagggg atacaggctg   1440
aagagattct gcagacatac ttgaaatcca aggagtctat gactgatgca attctccaga   1500
cagaccagac tctcacagaa aaagaaaagg agattgaagt ggaacgtgtg aaagctgagt   1560
ctgcacaggc ttcagcaaaa atgttgcagg aaatgcaaag aaagaatgag cagatgatgg   1620
aacagaagga gaggagttat caggaacact tgaaacaact gactgagaag atggagaacg   1680
acagggtcca gttgctgaaa gagcaagaga ggaccctcgc tcttaaactt caggaacagg   1740
agcaactact aaaagaggga tttcaaaaag aaagcagaat aatgaaaaat gagatacagg   1800
atctccagac gaaaatgaga cgacgaaagg catgtaccat aagctaaaga ccagagcctt   1860
cctgtcaccc ctaaccaagg cataattgaa acaattttag aatttggaac aagcgtcact   1920
acatttgata ataattagat cttgcatcat aacaccaaaa gtttataaag gcatgtggta   1980
caatgatcaa aatcatgttt tttcttaaaa aaaaaaaaaa gactgtaaat tgtgcaacaa   2040
agatgcattt acctctgtat caactcagga aatctcataa gctggtacca ctcaggagaa   2100
gtttattctt ccagatgacc agcagtagac aaatggatac tgagcagagt cttaggtaaa   2160
agtcttggga aatatttggg cattggtctg gccaagtcta caatgtccca atatcaagga   2220
caaccaccct agcttcttag tgaagacaat gtacagttat ccattagatc aagactacac   2280
ggtctatgag caataatgtg atttctggac attgcccatg tataatcctc actgatgatt   2340
tcaagctaaa gcaaaccacc ttatacagag atctagaatc tctttatgtt ctccagagga   2400
aggtggaaga aaccatgggc aggagtagga attgagtgat aaacaattgg gctaatgaag   2460
aaaacttctc ttattgttca gttcatccag attataactt caatggaaca ctttagacca   2520
ttagacaatt gacactggat taaacaaatt cacataatgc caaatacaca atgtatttat   2580
agcaacgtat aatttgcaaa gatggacttt aaaagatgct gtgtaactaa actgaaataa   2640
ttcaattact tattatttag aatgttaaag cttatgatag tcttttctaa ttcttaacac   2700
```

tcatacttga aatctttccg agtttcccca gaagagaata tgggattttt tttgacattt 2760
ttgacccatt taataatgct cttgtgttta cctagtatat gtagactttg tcttatgtgt 2820
caaaagtcct aggaaagtgg ttgatgtttc ttatagcaat taaaaattat ttttgaactg 2880
a 2881

<210> 474
<211> 2790
<212> DNA
<213> Homo sapiens
<400> 474
ctctgggtac caactctatt gcgcagctcg ctgccgtgcg tttaacccag gcgaggagga 60
ggaggagaaa attcccccag attcgggcag acacctccgg cccacacagc cgttcacccc 120
ccgtcttttc agtcctggaa aggaattcgg ggctttcatt gtctaacgtc gacagtctat 180
atttctgctc ttcagtctgt ccttaggatg aagctctaac tgaactgaag taaggagaaa 240
cagccttgaa tctttggagg gtctgtcttc cttttgggct ctgtgcaact gcagctacag 300
tggaaaaaag caaactgctc ttgatcccag gccctgccta agcctcagca gaacttgtaa 360
gcctaaactg aagagcctca cccggacgag caggcatccc ttaaccttaa gcaatccagt 420
tccacgccct ggatcagtga ataaccccag ctgcaccatg agccgcgttc gggatgctgg 480
ctgtgtagcg gcggggatag tgatagggggc tggtgcctgg tactgtgtct acaaatacac 540
caggggggaga gaccagacca agaagagaat ggccaagccc aaaaaccggg ctgtggctgg 600
gactggagcc agggctagag ctgggctaag agccggattc acaatcgacc ttgggtcagg 660
attcagtccc ccaaccccag tccgtgctga agcagaggac agggcccagg atgaagcctc 720
tgctctggac acagttggag ctgaggcagt ggccccagct gcatccagcg ctgaggctca 780
gagtggggca ggcagtcagg cccaagaggc agatggagcc ggggttgggc ctaaggccga 840
atcagtagtt ggggctgcaa tggcttctgc aatagcacca cctcccgggg tgacagaggc 900
ccttggggct gcagaagccc ctgcaatggc aggggctccc aaagtggcag aagctcccag 960
agaagcggag acttccaggg cagcggtgcc tcctgggaca gtggtgccta ccgaagcggc 1020
agcacccact gaggtgaccg agggtcctgg ggtagcagca cctaccaagg tagctgaagc 1080
tcccgggggtg gcatcgccta ccgaggcagc tgaggctcct gtgcccgcaa cgcctactgg 1140
ggctgcagca cctactgggg ctgcagagtc tcctggaact tctggttccc ctagaacagc 1200
ggtggttcct ggaacatcag ctgccaagaa agcaacccct ggggctcaca ctggggctat 1260
accgaaagcc acatcagcga ctggagcggt acccaaaggt ggaggcaagg gtgtaaccag 1320
gtcccggaat gggggcaagg gcaagggcaa gaaaagcaaa gttgaagtag acgaactggg 1380
gatgggcttc cgtcctggag atggggctgc agcagctgct gcagcctctg ctaatggcgg 1440
acaggctttc ctggcagagg tccctgattc tgaggaaggg gagtccgggt ggactgacac 1500
agagtcagat tcagactctg agcccgagac ccagcgcaga gggaggggaa gaagacccgt 1560
tgccatgcag aagcgcccct ttccttatga aattgatgag attctgggtg tccgcgatct 1620
caggaaggtc cttgccttgc ttcagaaatc tgatgatcct ttcatccaac aggtagcttt 1680
gctcactctg agcaacaatg ccaattattc atgcaatcaa gagacaatcc gcaaattggg 1740
aggcctccca attattgcaa acatgatcaa caaaactgat ccacacatta aggaaaaagc 1800
cttaatggcc atgaataacc tgagtgagaa ttatgaaaat cagggccggc ttcaggtgta 1860
catgaataaa gtgatggatg atatcatggc ctctaacctg aactcagcag ttcaagtagt 1920
tggactaaaa tttctaacaa acatgactat tactaatgac taccagcacc tgcttgtcaa 1980
ttccattgca aactttttcc gtttgctatc tcagggaggt ggaaaaatca aggttgagat 2040
tttgaaaatc ctttcgaatt ttgctgaaaa tccagatatg ttgaagaaac ttctcagtac 2100
ccaagtgcca gcatcattta gttccctcta taattcttac gtggaatcag aaatccttat 2160
taatgccctt actctatttg agattatcta tgacaatctc agagcagaag tgtttaacta 2220
tagagaattc aataaaggtt cccttttttta cttatgcact acatctggag tgtgtgttaa 2280
gaaaattaga gccttagcaa atcaccatga cctcttagtg aaagtgaaag ttataaaact 2340
agtgaacaaa ttctgattgg ttatgtaccg tcaaaagact tgaagaaatt tcatgatttt 2400
gcagtgtgga agcgttgaaa attgaaagtt actgcttttc cacttgctca tatagtaaag 2460
ggatcctttc agctgccagt gttgaataat gtatcatcca gagtgatgtt atctgtgaca 2520
gtcaccagct ttaagctgaa ccatttatg aataccaaat aaatagacct cttgtactga 2580
aaacatattt gtgactttaa tcgtgctgct tggatagaaa tatttttact ggttcttctg 2640
aattgacagt aaacctgtcc attatgaatg gcctactgtt ctattatttg ttttgacttg 2700
aatttatcca ccaaagactt catttgtgta tcatcaataa agttgtatgt ttcaactgac 2760
aaaaaaaaaa aaaaaaaaaa aaaaaaaaaa 2790

<210> 475
<211> 1504
<212> DNA
<213> Homo sapiens
<400> 475
aatagccccg gatatctgtg ttaccagcct tgtctcggcc acctcaagga taatcactaa 60
attctgccga aaggactgag gaacggtgcc tggaaaaggg caagaatatc acggcatggg 120
catgagtagc ttgaaactgc tgaagtatgt cctgtttttc ttcaacttgc tcttttggat 180
ctgtggctgc tgcattttgg gctttgggat ctacctgctg atccacaaca acttcggagt 240
gctcttccat aacctcccct ccctcacgct gggcaatgtg tttgtcatcg tgggctctat 300
tatcatggta gttgccttcc tgggctgcat gggctctatc aaggaaaaca agtgtctgct 360

tatgtcgttc ttcatcctgc tgctgattat cctccttgct gaggtgacct tggccatcct 420
gctctttgta tatgaacaga agctgaatga gtatgtggct aagggtctga ccgacagcat 480
ccaccgttac cactcagaca atagcaccaa ggcagcgtgg gactccatcc agtcatttct 540
gcagtgttgt ggtataaatg gcacgagtga ttggaccagt ggcccaccag catcttgccc 600
ctcagatcga aaagtggagg gttgctatgc gaaagcaaga ctgtggtttc attccaattt 660
cctgtatatc ggaatcatca ccatctgtgt atgtgtgatt gaggtgttgg ggatgtcctt 720
tgcactgacc ctgaactgcc agattgacaa aaccagccag accatagggc tatgatctgc 780
agtagttctg tggtgaagag acttgtttca tctccggaaa tgcaaaacca tttatagcat 840
gaagccctac atgatcactg caggatgatc ctcctcccat cctttccctt tttaggtccc 900
tgtcttatac aaccagagaa gtgggtgttg gccaggcaca tcccatctca ggcagcaaga 960
caatctttca ctcactgacg gcagcagcca tgtctctcaa agtggtgaaa ctaatatctg 1020
agcatctttt agacaagaga ggcaaagaca aactggattt aatggcccaa catcaaaggg 1080
tgaacccagg atatgaattt ttgcatcttc ccattgtcga attagtctcc agcctctaaa 1140
taatgcccag tcttctcccc aaagtcaagc aagagactag ttgaagggag ttctggggcc 1200
aggctcactg gaccattgtc acaaccctct gtttctcttt gactaagtgc cctggctaca 1260
ggaattacac agttctcttt ctccaaaggg caagatctca tttcaatttc tttattagag 1320
ggccttattg atgtgttcta agtctttcca gaaaaaaact atccagtgat ttatatcctg 1380
atttcaacca gtcacttagc tgataatcac agtaagaaga cttctggtat tatctctcta 1440
tcagataaga ttttgttaat gtactatttt actcttcaat aaataaaaca gtttattatc 1500
tcaa 1504

<210> 476
<211> 3119
<212> DNA
<213> Homo sapiens
<400> 476
cgcggtatct gcatcgggcc tcactggctt caggagctga ataccctccc aggcacacac 60
aggtgggaca caaataaggg ttttggaacc actattttct catcacgaca gcaacttaaa 120
atgcctggga agatggtcgt gatccttgga gcctcaaata tactttggat aatgtttgca 180
gcttctcaag cttttaaaat cgagaccacc ccagaatcta gatatcttgc tcagattggt 240
gactccgtct cattgacttg cagcaccaca ggctgtgagt ccccattttt ctcttggaga 300
acccagatag atagtccact gaatgggaag gtgacgaatg aggggaccac atctacgctg 360
acaatgaatc ctgttagttt tgggaacgaa cactcttacc tgtgcacagc aacttgtgaa 420
tctaggaaat tggaaaaagg aatccaggtg gagatctact cttttcctaa ggatccagag 480
attcatttga gtggccctct ggaggctggg aagccgatca cagtcaagtg ttcagttgct 540
gatgtatacc catttgacag gctggagata gacttactga aaggagatca tctcatgaag 600
agtcaggaat ttctggagga tgcagacagg aagtccctgg aaaccaagag tttggaagta 660
acctttactc ctgtcattga ggatattgga aaagttcttg tttgccgagc taaattacac 720
attgatgaaa tggattctgt gcccacagta aggcaggctg taaaagaatt gcaagtctac 780
atatcaccca agaatacagt tatttctgtg aatccatcca caaagctgca agaaggtggc 840
tctgtgacca tgacctgttc cagcgagggt ctaccagctc cagagatttt ctggagtaag 900
aaattagata atgggaatct acagcacctt tctggaaatg caactctcac cttaattgct 960
atgaggatgg aagattctgg aatttatgtg tgtgaaggag ttaatttgat tgggaaaaac 1020
agaaaagagg tggaattaat tgttcaagag aaaccattta ctgttgagat ctcccctgga 1080
ccccggattg ctgctcagat tggagactca gtcatgttga catgtagtgt catgggctgt 1140
gaatccccat ctttctcctg gagaacccag atagacagcc ctctgagcgg gaaggtgagg 1200
agtgagggga ccaattccac gctgaccctg agccctgtga gttttgagaa cgaacactct 1260
tatctgtgca cagtgacttg tggacataag aaactggaaa agggaatcca ggtggagctc 1320
tactcattcc ctagagatcc agaaatcgag atgagtggtg gcctcgtgaa tgggagctct 1380
gtcactgtaa gctgcaaggt tcctagcgtg taccccottg accggctgga gattgaatta 1440
cttaaggggg agactattct ggagaatata gagtttttgg aggatacgga tatgaaatct 1500
ctagagaaca aaagtttgga aatgaccttc atccctacca ttgaagatac tggaaaagct 1560
cttgtttgtc aggctaagtt acatattgat gacatggaat tcgaacccaa acaaaggcag 1620
agtacgcaaa cactttatgt caatgttgcc cccagagata caaccgtctt ggtcagccct 1680
tcctccatcc tggaggaagg cagttctgtg aatatgacat gcttgagcca gggctttcct 1740
gctccgaaaa tcctgtggag caggcagctc cctaacgggg agctacagcc tctttctgag 1800
aatgcaactc tcaccttaat ttctacaaaa atggaagatt ctggggttta tttatgtgaa 1860
ggaattaacc aggctggaag aagcagaaag gaagtggaat taattatcca agttactcca 1920
aaagacataa aacttacagc ttttccttct gagagtgtca aagaaggaga cactgtcatc 1980
atctcttgta catgtggaaa tgttccagaa acatggataa tcctgaagaa aaaagcggag 2040
acaggagaca cagtactaaa atctatagat ggcgcctata ccatccgaaa ggcccagttg 2100
aaggatgcgg gagtatatga atgtgaatct aaaaacaaag ttggctcaca attaagaagt 2160
ttaacacttg atgttcaagg aagagaaaac aacaaagact atttttctcc tgagcttctc 2220
gtgctctatt ttgcatcctc cttaataata cctgccattg gaatgataat ttactttgca 2280
agaaaagcca acatgaaggg gtcatatagt cttgtagaag cacagaaatc aaaagtgtag 2340
ctaatgcttg atatgttcaa ctggagacac tatttatctg tgcaaatcct tgatactgct 2400
catcattcct tgagaaaaac aatgagctga gaggcagact tccctgaatg tattgaactt 2460
ggaaagaaat gcccatctat gtcccttgct gtgagcaaga agtcaaagta aaacttgctg 2520
cctgaagaac agtaactgcc atcaagatga gagaactgga ggagttcctt gatctgtata 2580

```
tacaataaca taatttgtac atatgtaaaa taaaattatg ccatagcaag attgcttaaa   2640
atagcaacac tctatattta gattgttaaa ataactagtg ttgcttggac tattataatt   2700
taatgcatgt taggaaaatt tcacattaat atttgctgac agctgacctt tgtcatcttt   2760
cttctatttt attccctttc acaaaatttt attcctatat agtttattga caataatttc   2820
aggttttgta aagatgccgg gttttatatt tttatagaca aataataagc aaagggagca   2880
ctgggttgac tttcaggtac taaatacctc aacctatggt ataatggttg actgggtttc   2940
tctgtatagt actggcatgg tacggagatg tttcacgaag tttgttcatc agactcctgt   3000
gcaactttcc caatgtggcc taaaaatgca acttcttttt attttctttt gtaaatgttt   3060
aggttttttt gtatagtaaa gtgataattt ctggaattag aaaaaaaaaa aaaaaaaaa    3119
```

<210> 477
<211> 2796
<212> DNA
<213> Homo sapiens
<400> 477

```
cctcccctgg ggaggctcgc gttcccgctg ctcgcgcctg ccgcccgccg gcctcaggaa     60
cgcgccctct cgccgcgcgc gccctcgcag tcaccgccac ccaccagctc cggcaccaac    120
agcagcgccg ctgccaccgc ccaccttctg ccgccgccac cacagccacc ttctcctcct    180
ccgctgtcct ctcccgtcct cgcctctgtc gactatcagg tgaactttga accaggatgg    240
ctgagccccg ccaggagttc gaagtgatgg aagatcacgc tgggacgtac gggttggggg    300
acaggaaaga tcaggggggc tacaccatgc accaagacca agagggtgac acggacgctg    360
gcctgaaaga atctcccctg cagaccccca ctgaggacgg atctgaggaa ccgggctctg    420
aaacctctga tgctaagagc actccaacag cggaagatgt gacagcaccc ttagtggatg    480
agggagctcc cggcaagcag gctgccgcgc agccccacac ggagatccca gaaggaacca    540
cagctgaaga agcaggcatt ggagacaccc ccagcctgga agacgaagct gctggtcacg    600
tgacccaagc tcgcatggtc agtaaaagca aagacgggac tggaagcgat gacaaaaaag    660
ccaagggggc tgatggtaaa acgaagatcg ccacaccgcg gggagcagcc cctccaggcc    720
agaagggcca ggccaacgcc accaggattc cagcaaaaac cccgcccgct ccaaagacac    780
cacccagctc tggtgaacct ccaaaatcag gggatcgcag cggctacagc agccccggct    840
ccccaggcac tcccggcagc cgctcccgca ccccgtccct tccaacccca cccacccggg    900
agcccaagaa ggtggcagtg gtccgtactc cacccaagtc gccgtcttcc gccaagagcc    960
gcctgcagac agcccccgtg cccatgccag acctgaagaa tgtcaagtcc aagatcggct   1020
ccactgagaa cctgaagcac cagccgggag gcgggaaggt gcagataatt aataagaagc   1080
tggatcttag caacgtccag tccaagtgtg gctcaaagga taatatcaaa cacgtcccgg   1140
gaggcggcag tgtgcaaata gtctacaaac cagttgacct gagcaaggtg acctccaagt   1200
gtggctcatt aggcaacatc catcataaac caggaggtgg ccaggtggaa gtaaaatctg   1260
agaagcttga cttcaaggac agagtccagt cgaagattgg gtccctggac aatatcaccc   1320
acgtccctgg cggaggaaat aaaaagattg aaacccacaa gctgaccttc cgcgagaacg   1380
ccaaagccaa gacagaccac ggggcggaga tcgtgtacaa gtcgccagtg gtgtctgggg   1440
acacgtctcc acggcatctc agcaatgtct cctccaccgg cagcatcgac atggtagact   1500
cgccccagct cgccacgcta gctgacgagg tgtctgcctc cctggccaag cagggtttgt   1560
gatcaggccc ctggggcggt caataattgt ggagaggaga gaatgagaga gtgtggaaaa   1620
aaaaagaata atgacccggc ccccgccctc tgcccccagc tgctcctcgc agttcggtta   1680
attggttaat cacttaacct gcttttgtca ctcggctttg gctcgggact tcaaaatcag   1740
tgatgggagt aagagcaaat ttcatctttc caaattgatg ggtgggctag taataaaata   1800
tttaaaaaaa aacattcaaa aacatggcca catccaacat ttcctcaggc aattcctttt   1860
gattcttttt tcttcccccct ccatgtagaa gagggagaag gagaggctct gaaagctgct   1920
tctgggggat ttcaagggac tgggggtgcc aaccacctct ggccctgttg tgggggttgt   1980
cacagaggca gtggcagcaa caaaggattt gaaaactttg gtgtgttcgt ggagccacag   2040
gcagacgatg tcaaccttgt gtgagtgtga cgggggttgg ggtggggcgg gaggccacgg   2100
gggaggccga ggcaggggct gggcagaggg gaggaggaag cacaagaagt gggagtggga   2160
gaggaagcca cgtgctggag agtagacatc cccctccttg ccgctgggag agccaaggcc   2220
tatgccacct gcagcgtctg agcggccgcc tgtccttggt ggccgggggt gggggcctgc   2280
tgtgggtcag tgtgccaccc tctgcagggc agcctgtggg agaagggaca gcgggttaaa   2340
aagagaaggc aagcctggca ggagggttgg cacttcgatg atgacctcct tagaaagact   2400
gaccttgatg tcttgagagc gctggcctct tcctccctcc ctgcagggta gggcgcctga   2460
gcctaggcgg ttccctctgc tccacagaaa ccctgtttta ttgagttctg aaggttggaa   2520
ctgctgccat gattttggcc actttgcaga cctgggactt tagggctaac cagttctctt   2580
tgtaaggact tgtgcctctt gggagacgtc cacccgtttc caagcctggg ccactggcat   2640
ctctggagtg tgtgggggtc tgggaggcag gtcccgagcc ccctgtcctt cccacggcca   2700
ctgcagtcac cccgtctgcg ccgctgtgct gttgtctgcc gtgagagccc aatcactgcc   2760
tatacccctc atcacacgtc acaatgtccc gaattc                             2796
```

<210> 478
<211> 2984
<212> DNA
<213> Homo sapiens
<400> 478

```
taactgagcg aggagcaatt gattaatagc tcggcgaggg gactcactga ctgttataat     60
```

```
aacactacac cagcaactcc tggcttccca gcagccggaa cacagacagg agagagtcag    120
tggcaaatag acatttttct tatttcttaa aaaacagcaa cttgtttgct acttttattt    180
ctgttgattt ttttttcttg gtgtgtgtgg tggttgtttt taagtgtgga gggcaaaagg    240
agataccatc ccaggctcag tccaacccct ctccaaaacg gcttttctga cactccaggt    300
agcgagggag ttgggtctcc aggttgtgcg aggagcaaat gatgaccgcc aaggccgtag    360
acaaaatccc agtaactctc agtggttttg tgcaccagct gtctgacaac atctacccgg    420
tggaggacct cgccgccacg tcggtgacca tctttcccaa tgccgaactg ggaggcccct    480
ttgaccagat gaacggagtg gccggagatg gcatgatcaa cattgacatg actggagaga    540
agaggtcgtt ggatctccca tatcccagca gctttgctcc cgtctctgca cctagaaacc    600
agaccttcac ttacatgggc aagttctcca ttgaccctca gtaccctggt gccagctgct    660
acccagaagg cataatcaat attgtgagtg caggcatctt gcaaggggtc acttccccag    720
cttcaaccac agcctcatcc agcgtcacct ctgcctcccc caacccactg gccacaggac    780
ccctgggtgt gtgcaccatg tcccagaccc agcctgacct ggaccacctg tactctccgc    840
caccgcctcc tcctccttat tctggctgtg caggagacct ctaccaggac ccttctgcgt    900
tcctgtcagc agccaccacc tccacctctt cctctctggc ctacccacca cctccttcct    960
atccatcccc caagccagcc acggacccag gtctcttccc aatgatccca gactatcctg   1020
gattctttcc atctcagtgc cagagagacc tacatggtac agctggccca gaccgtaagc   1080
cctttccctg cccactggac accctgcggg tgccccctcc actcactcca ctctctacaa   1140
tccgtaactt taccctgggg ggccccagtg ctggggtgac cggaccaggg gccagtggag   1200
gcagcgaggg accccggctg cctggtagca gctcagcagc agcagcagcc gccgccgccg   1260
ccgcctataa cccacaccac ctgccactgc ggcccattct gaggcctcgc aagtacccca   1320
acagacccag caagacgccg gtgcacgaga ggccctaccc gtgcccagca gaaggctgcg   1380
accggcggtt ctcccgctct gacgagctga cacggcacat ccgaatccac actgggcata   1440
agcccttcca gtgtcggatc tgcatgcgca acttcagccg cagtgaccac ctcaccaccc   1500
atatccgcac ccacaccggt gagaagccct tcgcctgtga ctactgtggc cgaaagtttg   1560
cccggagtga tgagaggaag cgccacacca agatccacct gagacagaaa gagcggaaaa   1620
gcagtgcccc ctctgcatcg gtgccagccc cctctacagc ctcctgctct ggggggcgtgc   1680
agcctggggg taccctgtgc agcagtaaca gcagcagtct tggcggaggg ccgctcgccc   1740
cttgctcctc tcggacccgg acaccttgag atgagactca ggctgataca ccagctccca   1800
aaggtcccgg aggccctttg tccactggag ctgcacaaca aacactacca cccctttcctg   1860
tccctctctc cctttgttgg gcaaagggct ttggtggagc tagcactgcc cccttttccac   1920
ctagaagcag gttcttccta aaacttagcc cattctagtc tctcttaggt gagttgacta   1980
tcaacccaag gcaaagggga ggctcagaag gaggtggtgt ggggatcccc tggccaagag   2040
ggctgaggtc tgaccctgct ttaaagggtt gtttgactag gttttgctac cccacttccc   2100
cttattttga cccatcacag gtttttgacc ctggatgtca gagttgatct aagacgtttt   2160
ctacaatagg ttgggagatg ctgatccctt caagtgggga cagcaaaaag acaagcaaaa   2220
ctgatgtgca ctttatggct tgggactgat ttgggggaca ttgtacagtg agtgaagtat   2280
agcctttatg ccacactctg tggccctaaa atggtgaatc agagcatatc tagttgtctc   2340
aacccttgaa gcaatatgta ttatatactc agagaacaga agtgcaatgt gatgggagga   2400
acgtagcaat atctgctcct tttcgagttg tttgagaaat gtaggctatt ttttcagtgt   2460
atatccactc agattttgtg tatttttgat gtacccacac tgttctctaa attctgaatc   2520
tttgggaaaa aatgtaaagc atttatgatc tcagaggtta acttatttaa gggggatgta   2580
catattctct gaaactagga tgcatgcaat tgtgttggaa gtgtccttgg tcgccttgtg   2640
tgatgtagac aaatgttaca aggctgcatg taaatgggtt gccttattat ggagaaaaaa   2700
atcactccct gagtttagta tggctgtata tttatgccta ttaatatttg gaattttttt   2760
tagaaagtat atttttgtat gctttgtttt gtgacttaaa agtgttacct ttgtagtcaa   2820
atttcagata agaatgtaca taatgttacc ggagctgatt tgtttggtca ttagctctta   2880
atagttgtga aaaaataaat ctattctaac gcaaaaccac taactgaagt tcagatataa   2940
tggatggttt gtgactatag tgtaaataaa tacttttcaa caat                      2984
```

<210> 479
<211> 1712
<212> DNA
<213> Homo sapiens
<400> 479

```
aaggtcaatg atagcatctg cctagagtca aacctccgtg cttctcagac agtgcctttt     60
caccatgagt gggtgcccat ttttaggaaa caactttgga tatactttta aaaaactccc    120
cgtagaaggc agcgaagaag acaaatcaca aactggtgtg aatagagcca gcaaaggagg    180
tcttatctat gggaactacc tgcatttgga aaaagttttg aatgcacaag aactgcaaag    240
tgaaacaaaa ggaaataaaa tccatgatga acatcttttt atcataactc atcaagctta    300
tgaactctgg tttaagcaaa tcctctggga gttggattct gttcgagaga tctttcagaa    360
tggccatgtc agagatgaaa ggaacatgct taaggttgtt tctcggatgc accgagtgtc    420
agtgatcctg aaactgctgg tgcagcagtt ttccattctg gagacgatga cagccttgga    480
cttcaatgac ttcagagagt acttatctcc agcatcaggc ttccagagtt tgcaattccg    540
actattagaa aacaagatag gtgttcttca gaacatgaga gtcccttata acagaagaca    600
ttatcgtgat aacttcaaag gagaagaaaa tgaactgcta cttaaatctg agcaggaaaa    660
gacacttctg gaattagtgg aggcatggct ggaaagaact ccaggtttag agccacatgg    720
atttaacttc tggggaaagc ttgaaaaaaa tatcaccaga ggcctggaag aggaattcat    780
aaggattcag gctaaagaag agtctgaaga aaaagaggaa caggtggctg aatttcagaa    840
```

```
gcaaaaagag gtgctactgt ccttatttga tgagaaacgt catgaacatc tccttagtaa     900
aggtgaaaga cggctgtcat acagagcact tcagggagca ttgatgatat atttttacag     960
ggaagagcct aggttccagg tgccttttca gttgctgact tctcttatgg acatagattc    1020
actgatgacc aaatggagat ataaccatgt gtgcatggtg cacagaatgc tgggcagcaa    1080
agctggcacc ggtggttcct caggctatca ctacctgcga tcaactgtga gtgataggta    1140
caaggtattt gtagatttat ttaatctttc aacatacctg attccccgac actggatacc    1200
gaagatgaac ccaaccattc acaaatttct atatacagca gaatactgtg atagctccta    1260
cttcagcagt gatgaatcag attaaaatcg tctgcaaaat ctatgaagaa tactggtttc    1320
acagcctatt ttttattttc tatggatttt cataaataca gtttgaatat atgtatgcat    1380
atattgttca gcaccacgat gctctgattt aattctagaa acaatttgat tacctcttgt    1440
ttgtgacaag actaagcatt aagatgagaa agaatacatt taaatagtaa cattgtacat    1500
agggtgtttt cctattaaaa atcagtttcc cctgagactt aatgtaacca cttaatgtaa    1560
tcactatctc attgtttcat ctttataaac ttgtaaactt catctatttc aaatatttta    1620
tgcagtacat tatattattc tgtacaaagg ctttcaaaca aaatttttaa aataataaag    1680
tattaatctt tcaaaaaaaa aaaaaaaaaa aa                                  1712

<210>  480
<211>  2175
<212>  DNA
<213>  Homo sapiens
<400>  480
gagaaattgg agaagataaa actggacact ggggagacca caacttcatg ctgcgtggga      60
tctcccagct acctgcagtg gccaccatgt cttgggtcct gctgcctgta ctttggctca     120
ttgttcaaac tcaagcaata gccataaagc aaacacctga attaacgctc catgaaatag     180
tttgtcctaa aaaacttcac attttacaca aaagagagat caagaacaac cagacagaaa     240
agcatggcaa agaggaaagg tatgaacctg aagttcaata tcagatgatc ttaaatggag     300
aagaaatcat tctctcccta caaaaaacca agcacctcct ggggccagac tacactgaaa     360
cattgtactc acccagagga gaggaaatta ccacgaaacc tgagaacatg gaacactgtt     420
actataaagg aaacatccta aatgaaaaga attctgttgc cagcatcagt acttgtgacg     480
ggttgagagg atacttcaca catcatcacc aaagatacca gataaaacct ctgaaaagca     540
cagacgagaa agaacatgcc gtctttacat ctaaccagga ggaacaagac ccagctaacc     600
acacatgtgg tgtgaagagc actgacggga aacaaggccc aattcgaatc tctagatcac     660
tcaaaagccc agagaaagaa gactttcttc gggcacagaa atacattgat ctctatttgg     720
tgctggataa tgccttttat aagaactata atgagaatct aactctgata agaagctttg     780
tgtttgatgt gatgaaccta ctcaatgtga tatataacac catagatgtt caagtggcct     840
tggtaggtat ggaaatctgg tctgatgggg ataagataaa ggtggtgccc agcgcaagca     900
ccacgtttga caacttcctg agatggcaca gttctaacct ggggaaaaag atccacgacc     960
atgctcagct tctcagcggg attagcttca acaatcgacg tgtgggactg gcagcttcaa    1020
attccttgtg ttccccatct tcggttgctg ttattgaggc taaaaaaaag aataatgtgg    1080
ctcttgtagg agtgatgtca catgagctgg gccatgtcct tggtatgcct gatgttccat    1140
tcaacaccaa gtgtccctct ggcagttgtg tgatgaatca gtatctgagt tcaaaattcc    1200
caaaggattt cagtacatct tgccgtgcac attttgaaag ataccttttta tctcagaaac   1260
caaagtgcct gctgcaagca cctattccta caaatataat gacaacacca gtgtgtggga    1320
accaccttct agaagtggga gaagactgtg attgtggctc tcctaaggag tgtaccaatc    1380
tctgctgtga agccctaacg tgtaaactga agcctggaac tgattgcgga ggagatgctc    1440
caaaccatac cacagagtga atccaaaagt ctgcttcact gagatgctac cttgccagga    1500
caagaaccaa gaactctaac tgtcccagga atcttgtgaa ttttcaccca taatggtctt    1560
tcacttgtca ttctactttc tatattgtta tcagtccagg aaacaggtaa acagatgtaa    1620
ttagagacat tggctctttg tttaggccta atctttcttt ttactttttt ttttcttttt    1680
tcttttttttt taaagatcat gaatttgtga cttagttctg ccctttggag aacaaaagaa   1740
agcagtcttc catcaaatca ccttaaaatg cacggctaaa ctattcagag ttaacactcc    1800
agaattgtta aattacaagt actatgcttt aatgcttctt tcatcttact agtatggcct    1860
ataaaaaaaa taataccact tgatgggtga aggctttggc aatagaaaga agaatagaat    1920
tcaggtttta tgttattcct ctgtgttcac ttcgccttgc tcttgaaagt gcagtatttt    1980
tctacatcat gtcgagaatg attcaatgta aatatttttc attttatcat gtatatccta    2040
tacacacatc tccttcatca tcatatatga agtttatttt gagaagtcta cattgcttac    2100
attttaattg agccagcaaa gaaggcttaa tgatttattg aaccataatg tcaataaaaa    2160
cacaactttt gaggc                                                     2175

<210>  481
<211>  1805
<212>  DNA
<213>  Homo sapiens
<400>  481
tgtttacttt ggttgtccct tctggcatgg tgcatatgtt atgggaagag ggattataat      60
ttggtgctgt ttgtagagat gacaacactg ataaaatcca ctcattgctg gtcccagcac     120
acctggaaag ttctgcaagg cctcagctac agaaagccca gagacagaaa gtaaactctt     180
tcatgaccct tgatcatcag atcatcaatc caactcttaa atggtcacaa cctgcagtgc     240
caagtggtgg gcctcttgtg cagcatgcac acacaactct ggacagtgat gctggcctca     300
```

cagaaaaccc actcaccaag ttactagcta ttgggaaaga agatgacaat gcacaatggc 360
atatggagga cgttattgag gatataatcg gtatggaatc aagttttaaa gaggaaggag 420
cagactctcc tctgctaatg caaagaacat tatctggaag tattttggat gtgtatagcg 480
gtgaacaagg aatttcacca attaacatgg ggcttacaag tgcttcttgt ccaagtagtc 540
taccaatgaa aagagaaatt acagaaactg acactagagc tttagcaaaa gagagacaaa 600
aaaaggacaa ccacaacctc attgaaagaa gaagaaggta taatattaat taccgaatca 660
aggagcttgg cactcttatt ccaaagtcta atgatcctga tatgcgctgg aacaaaggaa 720
ccattctaaa agcatcagtg gagtacatca agtggctaca aaaagaacaa cagagagccc 780
gagaattgga acacagacag aagaaattag agcaggctaa caggcgactt ctacttcgga 840
ttcaggaact agaaattcag gctcgtactc atggtctgcc aaccctggct tcacttggca 900
cggttgattt aggtgctcat gtcaccaaac agcagagcca tcctgagcag aattcagtag 960
actattgcca acaactgact gtgtctcagg ggccaagccc tgagctctgt gatcaagcta 1020
tagccttttc tgatcctttg tcatacttca cagatttatc atttagtgct gcattgaaag 1080
aggaacaaag attggatggc atgctattgg atgacacaat ctctccattt ggaacagatc 1140
ctctgctatc tgccacttcc cctgcagttt ccaaagaaag cagtaggaga agtagcttta 1200
gctcagatga tggtgatgaa ttataagaaa taaacagacc caattcatca actggaaagc 1260
aattctatgc tggtgctatg caattatgct ctgtgtttca tatgttgctt tggcttattt 1320
tttttcttaa aggaatgtgt tgttcatgaa aaactgatag aagcaacaga agaattcgca 1380
ggaagaaaaa tcatagtgtt aatgaattat tgagggcgaa aaaaaggtgt tttcttcttt 1440
gactacggag tccaaatcca cttaaattct gttttcctga aaagaggtac agcataagaa 1500
atagctcttt attgatgttt taaaagcagc aacttggtgg tgtactactg gaactaatga 1560
ctgcaaagtg ttaaacgact gaaatataca aacagtctct tagttactca tttccatctt 1620
ctcttcaact ttcacatcag tcttccggaa tcaagatcaa catatcaggt ggtcattgcc 1680
tttctccatt gtctagtaga catgtctaaa gttcaaactt tataggataa ataaatgtat 1740
aatagattat ctgtcacttg tggttgaaag gcaaatctac aataaatgtg agaattttcc 1800
acaat 1805

<210> 482
<211> 895
<212> DNA
<213> Homo sapiens
<400> 482
gccatcttgc gtccccgcgt gtgtgcgcct aatctcaggt ggtccacccg agacccottg 60
agcaccaacc ctagtccccc gcgcggcccc ttattcgctc cgacaagatg aaagaaacaa 120
tcatgaacca ggaaaaactc gccaaactgc aggcacaagt gcgcattggt gggaaaggaa 180
ctgctcgcag aaagaagaag gtggttcata gaacagccac agcagatgac aaaaaacttc 240
agttctcctt aaagaagtta ggggtaaaca atatctctgg tattgaagag gtgaatatgt 300
ttacaaacca aggaacagtg atccacttta acaaccctaa agttcaggca tctctggcag 360
cgaacacttt caccattaca ggccatgctg agacaaagca gctgacagaa atgctaccca 420
gcatcttaaa ccagcttggt gcggatagtc tgactagttt aaggagactg gccgaagctc 480
tgcccaaaca atctgtggat ggaaaagcac cacttgctac tggagaggat gatgatgatg 540
aagttccaga tcttgtggag aattttgatg aggcttccaa gaatgaggca aactgaattg 600
agtcaacttc tgaagataaa acctgaagaa gttactggga gctgctattt tatattatga 660
ctgcttttta agaaattttt gtttatggat ctgataaaat ctagatctct aatattttta 720
agcccaagcc ccttggacac tgcagctctt ttcagttttt gcttatacac aattcattct 780
ttgcagctaa ttaagccgaa gaagcctggg aatcaagttt gaaacaaaga ttaataaagt 840
tctttgccta gtaaaaaaaa aaaaaaaaaa aaaaataaaa aaaaaaaaaa aaaaa 895

<210> 483
<211> 9432
<212> DNA
<213> Homo sapiens
<400> 483
gccgtggctc cggtagcagc aagttcgaac cccgctcccg ctccgcttcg gttctcgctc 60
cttcggccct tgggcctcca aacaccagtc cccggcagct cgttgcgcat tgcgctctcc 120
ccgccaccag gatgccggta accgagaagg atctagctga ggacgcgcct tggaagaaga 180
tccagcagaa cacgttcaca cgctggtgca acgagcacct caagtgcgtg aacaaacgca 240
tcggcaacct gcagaccgac ctgagcgacg ggctgcggct catcgcgctg ctcgaggtgc 300
tcagccagaa gcgcatgtac cgcaagtacc atcagcggcc cacctttcgc cagatgcagc 360
tcgagaatgt gtccgtggcg ctcgagttcc tggaccgtga gagcatcaag ctcgtgtcca 420
tcgatagcaa agccattgtg gatgggaacc tgaagctcat cttgggtctg gtgtggacgc 480
tgatcctcca ctactccatc tccatgcccg tgtgggagga tgaaggggat gatgatgcca 540
agaagcagac gccaaagcag aggctgctgg ggtggattca gaacaagatc ccctacttgc 600
ccatcaccaa ctttaaccag aactggcaag acggcaaagc cctgggagcc ctggtagaca 660
gctgtgctcc aggtctgtgc ccagactggg aatcctggga cccgcagaag cctgtggata 720
atgcacgaga agccatgcag caggcagatg actggctggg tgtcccacag gtcatcactc 780
ctgaagaaat cattcacccg gatgtggacg agcactcagt tatgacttac ctgtcccagt 840
tccccaaagc caagctcaag ccgggggctc ctctcaaacc caaactcaac ccgaagaaag 900
ccagggccta tggcagagga atcgagccca ctggaaacat ggtgaagcag ccagccaagt 960

tcactgtgga caccatcagc gccgggcaag gagacgtgat ggtgtttgtt gaggacccag 1020
aagggaacaa agaggaggca caagtgaccc ctgacagcga caagaacaag acatactctg 1080
tggagtatct gcccaaggtc accgggctac acaaagtcac agtcctcttt gcaggacagc 1140
acatctccaa gagcccattt gaagtgagtg ttgacaaggc ccagggagat gccagtaaag 1200
tcactgcaaa aggtccaggg ttggaagctg tagggaacat cgccaataag cccacctact 1260
ttgacatcta tacggcagga gctggtgtgg gtgacattgg tgtggaggtg gaagatcccc 1320
aggggaagaa caccgtggag ttgctcgtgg aagacaaagg aaaccaggtg tatcgatgtg 1380
tgtacaaacc catgcagcct ggccctcacg tggtcaagat cttctttgct ggggacacta 1440
ttcctaagag tcccttcgtt gtgcaggttg gggaagcctg caatccaaat gcctgccggg 1500
ccagtggccg aggcctacaa cccaaaggcg tccgtatccg ggagaccaca gatttcaagg 1560
ttgacaccaa agctgcagga agtggggagc tcggggtaac catgaagggt cctaagggtc 1620
tggaggagct ggtgaagcag aaagactttc tggatggggt ctacgcattc gagtattacc 1680
ccagcacccc ggggagatac agcattgcca tcacatgggg gggacaccac attccaaaga 1740
gcccctttga agttcaagtt ggccctgaag cgggtatgca gaaagtccgt gcttggggcc 1800
ctgggctcca tggtgggatt gtcgggcggt cagcggactt cgtggtagaa tccattggct 1860
ctgaagtggg gtctctgggg tttgccattg aaggcccctc tcaggcaaag attgagtaca 1920
acgaccagaa tgatggatcg tgtgatgtca aatactggcc caaggagcct ggcgaatatg 1980
ctgttcacat catgtgtgac gacgaagaca tcaaggacag cccgtacatg gccttcatcc 2040
acccagccac gggaggctac aaccctgatc tggttcgagc atacgggcca ggtttggaga 2100
aatctggatg cattgtcaac aacctggccg agttcactgt ggatcctaag gatgctggaa 2160
aagctccctt aaagatattt gctcaggatg gggaaggcca acgcattgac atccagatga 2220
agaaccggat ggacggcaca tatgcatgct catacacccc ggtgaaggcc atcaagcaca 2280
ccattgctgt ggtctgggga ggcgtgaaca tcccgcacag cccctacagg gtcaacatcg 2340
ggcaaggtag ccatcctcag aaggtcaaag tgtttgggcc aggtgtggag agaagtggtc 2400
tgaaggcaaa tgaacctaca cacttcacgg tggactgtac tgaggctggg gaaggtgatg 2460
tcagtgttgg cattaagtgt gatgcccggg tgttaagtga agatgaggaa gacgtggatt 2520
ttgacattat tcacaatgcc aatgatacgt tcacagtcaa atatgtgcct cctgctgctg 2580
ggcgatacac tatcaaagtt ctctttgcat ctcaggaaat ccccgccagc cctttcagag 2640
tcaaagttga cccttcccac gatgccagca aagtgaaggc agaaggccca gggctcagca 2700
aagcaggtgt ggaaaatggg aaaccgaccc acttcactgt ctacaccaag gggggctgga 2760
aagccccgct caacgtgcag ttcaacagcc ctcttcctgg cgatgcagtg aaggatttgg 2820
atatcatcga taattatgac tactctcaca cggttaaata tacacccacc caacagggca 2880
acatgcaggt tctggtgact tacggtggcg atcccatccc taaaagccct ttcactgtgg 2940
gtgttgctgc accgctggat ctgagcaaga taaaactcaa tgggctggaa aacagggtgg 3000
aagttgggaa ggatcaggag ttcaccgttg ataccagggg ggcaggaggc caggggaagc 3060
tggacgtgac aatcctcagc cctctcggga aggtcgtgcc atgcctagtg acacctgtga 3120
caggccggga gaacagcacg gccaagttca tccctcggga ggaggggctg tatgctgtag 3180
acgtgaccta cgatggacac cctgtgcccg ggagccccta cacagtggag gcctcgctgc 3240
caccagatcc cagcaaggtg aaggcccacg gtcccggcct cgaaggtggt ctcgtgggca 3300
agcctgccga gttcaccatc gataccaaag gagctggtac tggaggtctg ggcttaacgg 3360
tggaaggtcc gtgcgaggcc aaaatcgagt gctccgacaa tggtgatggg acctgctccg 3420
tctcttacct tcccacaaaa cccggggagt acttcgtcaa catcctcttt gaagaagtcc 3480
acatacctgg gtctcccttc aaagctgaca ttgaaatgcc ctttgacccc tctaaagtcg 3540
tggcatcggg gccaggtctc gagcacggga aggtgggtga agctggcctc cttagcgtca 3600
actgctcgga agcgggaccg ggggccctgg gcctggaagc tgtctcggac tcgggaacaa 3660
aagccgaagt cagtattcag aacaacaaag atggcaccta cgcggtgacc tacgtgcccc 3720
tgacggccgg catgtacacg ttgaccatga agtatggtgg cgaactcgtg ccacacttcc 3780
ccgcccgggt caaggtggag cccgccgtgg acaccagcag gatcaaagtc tttggaccag 3840
gaatagaagg gaaagatgtg ttccgggaag ctaccaccga ctttacagtt gactctcggc 3900
cgctgaccca ggttgggggt gaccacatca aggcccacat tgccaacccc tcaggggcct 3960
ccaccgagtg ctttgtcaca gacaatgcgg atgggaccta ccaggtggaa tacacaccct 4020
ttgagaaagg tctccatgta gtggaggtga catatgatga cgtgcctatc ccaaacagtc 4080
ccttcaaggt ggctgtcact gaaggctgcc agccatctag ggtgcaagcc caaggacctg 4140
gattgaaaga ggcctttacc aacaagccca atgtcttcac cgtggttacc agaggcgcag 4200
gaattggtgg gcttggcata actgttgagg gaccatcaga gtcgaagata aattgcagag 4260
acaacaagga tggcagctgc agtgctgagt acattccttt cgcgccgggg gattacgatg 4320
ttaatatcac atatggagga gcccacatcc ctggcagccc cttcagggtt cctgtgaagg 4380
atgttgtgga ccccagcaag gtcaagattg ccggccccgg gctgggctca ggcgtccgag 4440
cccgtgtcct gcagtccttc acggtggaca gcagcaaggc tggcctggct ccgctggaag 4500
tgaggttct gggcccacga ggcttggtgg agccagtgaa catggtggac aatggagatg 4560
gcacacacac agtaacctac accccatctc aggagggacc ttacatggtc tcagttaaat 4620
atgctgatga agagattcct cgcagtccct tcaaggtcaa ggtccttccc acatatgatg 4680
ccagcaaagt gactgccagt ggccccggcc ttagttccta tggtgtgcct gccagtctac 4740
ctgtggactt tgcaattgat gcccgagatg ccggggaagg cctgcttgct gttcaaataa 4800
cggaccaaga aggaaaaccc aaaagagcca ttgtccatga caataaagat ggcacgtatg 4860
ctgtcaccta catccccgac aagactgggc gctatatgat tggagtcacc tacgggggtg 4920
acgacatccc actttctcct tatcgcatcc gagccacaca gacgggtgat gccagcaagt 4980
gcctggccac gggtcctgga atcgcctcca ctgtgaaaac tggcgaagaa gtaggctttg 5040
tggttgatgc caagactgcc gggaagggta aagtgacctg cacggttctg accccagatg 5100

gcactgaggc cgaggccgat gtcattgaga atgaagatgg aacctatgac atcttctaca 5160
cagctgccaa gccgggcaca tatgtgatct atgtgcgctt cggtggtgtt gatattccta 5220
acagcccctt cactgtcatg gccacagatg gggaagtcac agccgtggag gaggcaccgg 5280
taaatgcatg tccccctgga ttcaggccct gggtgaccga agaggcctat gtcccagtga 5340
gtgacatgaa cggcctggga tttaagcctt ttgacctggt cattccgttt gctgtcagga 5400
aaggagaaat cactggagag gtccacatgc cttctgggaa gacagccaca cctgagattg 5460
tggacaacaa ggacggcacg gtcactgtta gatatgcccc cactgaggtc gggctccatg 5520
agatgcacat caaatacatg ggcagccaca tccctgagag cccactccag ttctacgtga 5580
actaccccaa cagtggaagt gtttctgcat acggtccagg cctcgtgtat ggagtggcca 5640
acaaaactgc caccttcacc atcgtcacag aggatgcagg agaaggtggt ctggacttgg 5700
ctattgaggg cccctcaaaa gcagaaatca gctgcattga caataaagat gggacatgca 5760
cagtgaccta cctgccgact ctgccaggcg actacagcat tctggtcaag tacaatgaca 5820
agcacatccc tggcagcccc ttcacagcca agatcacaga tgacagcagg cggtgctccc 5880
aggtgaagtt gggctcagcc gctgacttcc tgctcgacat cagtgagact gacctcagca 5940
gcctgacggc cagcattaag gccccatctg gccgagacga gccctgtctc ctgaagaggc 6000
tgcccaacaa ccacattggc atctccttca tcccccggga agtgggcgaa catctggtca 6060
gcatcaagaa aaatggcaac catgtggcca acagccccgt gtctatcatg gtggtccagt 6120
cggagattgg tgacgcccgc cgagccaaag tctatggccg cggcctgtca gaaggccgga 6180
ctttcgagat gtctgacttc atcgtggaca caagggatgc aggttatggt ggcatatcct 6240
tggcggtgga aggccccagc aaagtggaca tccagacgga ggacctggaa gatggcacct 6300
gcaaagtctc ctacttccct accgtgcctg ggtttatat cgtctccacc aaattcgctg 6360
acgagcacgt gcctgggagc ccatttaccg tgaagatcag tggggaggga agagtcaaag 6420
agagcatcac ccgcaccagt cgggccccgt ccgtggccac tgtcgggagc atttgtgacc 6480
tgaacctgaa aatcccagaa atcaacagca gtgatatgtc ggcccacgtc accagcccct 6540
ctggccgtgt gactgaggca gagattgtgc ccatggggaa gaactcacac tgcgtccggt 6600
ttgtgcccca ggagatgggc gtgcacacgg tcagcgtcaa gtaccgtggg cagcacgtca 6660
ccggcagccc cttccagttc accgtggggc cacttggtga aggaggcgcc cacaaggtgc 6720
gggcaggagg ccctggcctg gagagaggag aagcgggagt cccagctgag ttcagcattt 6780
ggacccggga agcaggcgct ggaggcctct ccatcgctgt tgagggcccc agtaaggccg 6840
agattacatt cgatgaccat aaaaatgggt cgtgcggtgt atcttatatt gcccaagagc 6900
ctggtaacta cgaggtgtcc atcaagttca atgatgagca catcccggaa agcccctacc 6960
tggtgccggt catcgcaccc tccgacgacg cccgccgcct cactgttatg agccttcagg 7020
aatcgggatt aaaagttaac cagccagcat cctttgctat aaggttgaat ggcgcaaaag 7080
gcaagattga tgcaaaggtg cacagcccct ctggagccgt ggaggagtgc cacgtgtctg 7140
agctggagcc agataagtat gctgttcgct tcatccctca tgagaatggt gtccacacca 7200
tcgatgtcaa gttcaatggg agccacgtgg ttggaagccc cttcaaagtg cgcgttgggg 7260
agcctggaca agcggggaac cctgccctgg tgtccgccta tggcacggga ctcgaagggg 7320
gcaccacagg tatccagtcg gaattcttta ttaacaccac ccgagcaggt ccagggacat 7380
tatccgtcac catcgaaggc ccatccaagg ttaaaatgga ttgccaggaa acacctgaag 7440
ggtacaaagt catgtacacc cccatggctc ctggtaacta cctgatcagt gtcaaatacg 7500
gtgggcccaa ccacatcgtg ggcagtccct tcaaggccaa ggtgacaggc cagcgtctag 7560
ttagccctgg ctcagccaac gagacctcat ccatcctggt ggagtcagtg accaggtcgt 7620
ctacagagac ctgctatagc gccattccca aggcatcctc ggacgccagc aaggtgacct 7680
ctaagggggc agggctctca aaggcctttg tgggccagaa gagttccttc ctggtggact 7740
gcagcaaagc tggctccaac atgctgctga tcggggtcca tgggcccacc accccctgcg 7800
aggaggtctc catgaagcat gtaggcaacc agcaatacaa cgtcacatac gtcgtcaagg 7860
agaggggcga ttatgtgctg gctgtgaagt gggggggagga acacatccct ggcagccctt 7920
ttcatgtcac agtgccttaa aacagttttc tcaaatcctg gagagagttc ttgtggttgc 7980
ttttgttgct tgtttgtaat tcattttata caaagccctc cagcctgttt gtggggctga 8040
aaccccatcc ctaaaatatt gctgttgtaa aatgccttca gaaataagtc ctagactgga 8100
ctcttgaggg acatattgga gaatcttaag aaatgcaagc ttgttcaggg ggctgagaag 8160
atcctgagta cactaggtgc aaaccagaac tcttggtgga acagaccagc cactgcagca 8220
gacagaccag gaacacaatg agactgacat ttcaaaaaaa caaaactggc tagcctgagc 8280
tgctggttca ctcttcagca tttatgaaac aaggctaggg gaagatgggc agagaaaaag 8340
gggacaccta gtttggttgt catttggcaa aggagatgac ttaaaatccg cttaatctct 8400
tccagtgtcc gtgttaatgt atttggctat tagatcacta gcactgcttt accgctcctc 8460
atcgccaaca cccccatgct ctgtggcctt cttacacttc tcagagggca gagtggcagc 8520
cgggcaccct acagaaactc agagggcaga gtggcagcca ggcccacatg tctctcaagt 8580
acctgtcccc tcgctctggt gattatttct tgcagaatca ccacacgaga ccatcccggc 8640
agtcatggtt ttgctttagt tttccaagtc cgtttcagtc ccttccttgg tctgaagaaa 8700
ttctgcagtg gcgagcagtt tcccacttgc caaagatccc ttttaaccaa cactagccct 8760
tgtttttaac acacgctcca gcccttcatc agcctgggca gtcttaccaa aatgtttaaa 8820
gtgatctcag aggggcccat ggattaacgc cctcatccca aggtccgtcc catgacataa 8880
cactccacac ccgccccagc caacttcatg ggtcactttt tctggaaaat aatgatctgt 8940
acagacagga cagaatgaaa ctcctgcggc tctttggcct gaaagttggg aatggttggg 9000
ggagagaagg gcagcagctt attggtggtc ttttcaccat tggcagaaac agtgagagct 9060
gtgtggtgca gaaatccaga aatgaggtgt agggaatttt gcctgccttc ctgcagacct 9120
gagctggctt tggaatgagg ttaaagtgtc agggacgttg cctgagccca aatgtgtagt 9180
gtggtctggg caggcagacc tttaggtttt gctgcttagt cctgaggaag tggccactct 9240

```
tgtggcaggt gtagtatctg gggcgagtgt tggggggtaaa agcccaccct acagaaagtg   9300
gaacagcccg gacctgatgt gaaaggacca cgggtgttgt aagctgggac cggaaccaaa   9360
ctggaatcaa acgcgactgt aaattgtatc ttataactta ttaaataaaa cattgctccg   9420
taaaaaaaaa aa                                                       9432

<210>  484
<211>  5010
<212>  DNA
<213>  Homo sapiens
<400>  484
ggcggctcgg gacggaggac gcgctagtgt gagtgcgggc ttctagaact acaccgaccc     60
tcgtgtcctc ccttcatcct gcggggctgg ctggagcggc cgctccggtg ctgtccagca    120
gccataggga gccgcacggg gagcgggaaa gcggtcgcgg ccccaggcgg ggcggccggg    180
atggagcggg gccgcgagcc tgtggggaag gggctgtggc ggcgcctcga gcggctgcag    240
gttcttctgt gtggcagttc agaatgatgg atcaagctag atcagcattc tctaacttgt    300
ttggtggaga accattgtca tatacccggt tcagcctggc tcggcaagta gatggcgata    360
acagtcatgt ggagatgaaa cttgctgtag atgaagaaga aaatgctgac aataacacaa    420
aggccaatgt cacaaaacca aaaaggtgta gtggaagtat ctgctatggg actattgctg    480
tgatcgtctt tttcttgatt ggatttatga ttggctactt gggctattgt aaaggggtag    540
aaccaaaaac tgagtgtgag agactggcag gaaccgagtc tccagtgagg gaggagccag    600
gagaggactt ccctgcagca cgtcgcttat attgggatga cctgaagaga aagttgtcgg    660
agaaactgga cagcacagac ttcaccagca ccatcaagct gctgaatgaa aattcatatg    720
tccctcgtga ggctggatct caaaaagatg aaaatcttgc gttgtatgtt gaaaatcaat    780
ttcgtgaatt taaactcagc aaagtctggc gtgatcaaca ttttgttaag attcaggtca    840
aagacagcgc tcaaaactcg gtgatcatag ttgataagaa cggtagactt gtttacctgg    900
tggagaatcc tggggggttat gtggcgtata gtaaggctgc aacagttact ggtaaactgg    960
tccatgctaa ttttggtact aaaaaagatt ttgaggattt atacactcct gtgaatggat   1020
ctatagtgat tgtcagagca gggaaaatca cctttgcaga aaaggttgca aatgctgaaa   1080
gcttaaatgc aattggtgtg ttgatataca tggaccagac taaatttccc attgttaacg   1140
cagaactttc attctttgga catgctcatc tggggacagg tgacccttac acacctggat   1200
tcccttcctt caatcacact cagtttccac catctcggtc atcaggattg cctaatatac   1260
ctgtccagac aatctccaga gctgctgcag aaaagctgtt tgggaatatg gaaggagact   1320
gtccctctga ctggaaaaca gactctacat gtaggatggt aacctcagaa agcaagaatg   1380
tgaagctcac tgtgagcaat gtgctgaaag agataaaaat tcttaacatc tttggagtta   1440
ttaaaggctt tgtagaacca gatcactatg ttgtagttgg ggcccagaga gatgcatggg   1500
gccctggagc tgcaaaatcc ggtgtaggca cagctctcct attgaaactt gcccagatgt   1560
tctcagatat ggtcttaaaa gatgggtttc agcccagcag aagcattatc tttgccagtt   1620
ggagtgctgg agactttgga tcggttggtg ccactgaatg gctagaggga tacctttcgt   1680
ccctgcattt aaaggctttc acttatatta atctggataa agcggttctt ggtaccagca   1740
acttcaaggt ttctgccagc ccactgttgt atacgcttat tgagaaaaca atgcaaaatg   1800
tgaagcatcc ggttactggg caatttctat atcaggacag caactgggcc agcaaagttg   1860
agaaactcac tttagacaat gctgctttcc ctttccttgc atattctgga atcccagcag   1920
tttctttctg tttttgcgag gacacagatt atccttattt gggtaccacc atggacacct   1980
ataaggaact gattgagagg attcctgagt tgaacaaagt ggcacgagca gctgcagagg   2040
tcgctggtca gttcgtgatt aaactaaccc atgatgttga attgaacctg gactatgaga   2100
ggtacaacag ccaactgctt tcatttgtga gggatctgaa ccaatacaga gcagacataa   2160
aggaaatggg cctgagttta cagtggctgt attctgctcg tggagacttc ttccgtgcta   2220
cttccagact aacaacagat ttcgggaatg ctgagaaaac agacagattt gtcatgaaga   2280
aactcaatga tcgtgtcatg agagtggagt atcacttcct ctctccctac gtatctccaa   2340
aagagtctcc tttccgacat gtcttctggg gctccggctc tcacacgctg ccagctttac   2400
tggagaactt gaaactgcgt aaacaaaata acggtgcttt taatgaaacg ctgttcagaa   2460
accagttggc tctagctact tggactattc agggagctgc aaatgccctc tctggtgacg   2520
tttgggacat tgacaatgag ttttaaatgt gatacccata gcttccatga gaacagcagg   2580
gtagtctggt ttctagactt gtgctgatcg tgctaaattt tcagtagggc tacaaaacct   2640
gatgttaaaa ttccatccca tcatcttggt actactagat gtctttaggc agcagctttt   2700
aatacagggt agataacctg tacttcaagt taaagtgaat aaccacttaa aaaatgtcca   2760
tgatggaata ttcccctatc tctagaattt taagtgcttt gtaatgggaa ctgcctcttt   2820
cctgttgttg ttaatgaaaa tgtcagaaac cagttatgtg aatgatctct ctgaatccta   2880
agggctggtc tctgctgaag gttgtaagtg gttcgcttac tttgagtgat cctccaactt   2940
catttgatgc taaataggag ataccaggtt gaaagacctc tccaaatgag atctaagcct   3000
ttccataagg aatgtagcag gtttcctcat tcctgaaaga aacagttaac tttcagaaga   3060
gatgggcttg ttttcttgcc aatgaggtct gaaatggagg tccttctgct ggataaaatg   3120
aggttcaact gttgattgca ggaataaggc cttaatatgt taacctcagt gtcatttatg   3180
aaaagagggg accagaagcc aaagacttag tatattttct tttcctctgt cccttccccc   3240
ataagcctcc atttagttct ttgttatttt tgtttcttcc aaagcacatt gaaagagaac   3300
cagtttcagg tgtttagttg cagactcagt ttgtcagact ttaaagaata atatgctgcc   3360
aaattttggc caaagtgtta atcttagggg agagctttct gtccttttgg cactgagata   3420
tttattgttt atttatcagt gacagagttc actataaatg gtgttttttt aatagaatat   3480
aattatcgga agcagtgcct tccataatta tgacagttat actgtcggtt ttttttaaat   3540
```

aaaagcagca tctgctaata aaacccaaca gatactggaa gttttgcatt tatggtcaac 3600
acttaagggt tttagaaaac agccgtcagc caaatgtaat tgaataaagt tgaagctaag 3660
atttagagat gaattaaatt taattagggg ttgctaagaa gcgagcactg accagataag 3720
aatgctggtt ttcctaaatg cagtgaattg tgaccaagtt ataaatcaat gtcacttaaa 3780
ggctgtggta gtactcctgc aaaattttat agctcagttt atccaaggtg taactctaat 3840
tcccatttgc aaaatttcca gtacctttgt cacaatccta acacattatc gggagcagtg 3900
tcttccataa tgtataaaga acaaggtagt ttttacctac cacagtgtct gtatcggaga 3960
cagtgatctc catatgttac actaagggtg taagtaatta tcgggaacag tgtttcccat 4020
aattttcttc atgcaatgac atcttcaaag cttgaagatc gttagtatct aacatgtatc 4080
ccaactccta taattcccta tcttttagtt ttagttgcag aaacattttg tggtcattaa 4140
gcattgggtg ggtaaattca accactgtaa aatgaaatta ctacaaaatt tgaaatttag 4200
cttgggtttt tgttaccttt atggtttctc caggtcctct acttaatgag atagcagcat 4260
acatttataa tgtttgctat tgacaagtca ttttaattta tcacattatt tgcatgttac 4320
ctcctataaa cttagtgcgg acaagtttta atccagaatt gaccttttga cttaaagcag 4380
agggactttg tatagaaggt ttgggggctg tggggaagga gagtcccctg aaggtctgac 4440
acgtctgcct acccattcgt ggtgatcaat taaatgtagg tatgaataag ttcgaagctc 4500
cgtgagtgaa ccatcatata aacgtgtagt acagctgttt gtcatagggc agttggaaac 4560
ggcctcctag ggaaaagttc atagggtctc ttcaggttct tagtgtcact tacctagatt 4620
tacagcctca cttgaatgtg tcactactca cagtctcttt aatcttcagt tttatcttta 4680
atctcctctt ttatcttgga ctgacattta gcgtagctaa gtgaaaaggt catagctgag 4740
attcctggtt cgggtgttac gcacacgtac ttaaatgaaa gcatgtggca tgttcatcgt 4800
ataacacaat atgaatacag ggcatgcatt ttgcagcagt gagtctcttc agaaaaccct 4860
tttctacagt tagggttgag ttacttccta tcaagccagt acgtgctaac aggctcaata 4920
ttcctgaatg aaatatcaga ctagtgacaa gctcctggtc ttgagatgtc ttctcgttaa 4980
ggagtagggc cttttggagg taaaggtata 5010

<210> 485
<211> 1836
<212> DNA
<213> Homo sapiens
<400> 485
atggcggcct cagcaaaaaa gaagaataag aaggggaaga ctatctccct aacagacttt 60
ctggctgagg atgggggtac tggtggagga agcacctatg tttccaaacc agtcagctgg 120
gctgatgaaa cggatgacct ggaaggagat gtttcgacca cttggcacag taacgatgac 180
gatgtgtata gggcgcctcc aattgaccgt tccatccttc ccactgctcc acgggctgct 240
cgggaaccca atatcgaccg gagccgtctt cccaaatcgc caccctacac tgcttttcta 300
ggaaacctac cctatgatgt tacagaagag tcaattaagg aattctttcg aggattaaat 360
atcagtgcag tgcgtttacc acgtgaaccc agcaatccag agaggttgaa aggttttggt 420
tatgctgaat ttgaggacct ggattccctg ctcagtgccc tgagtctcaa tgaagagtct 480
ctaggtaaca ggagaattcg agtggacgtt gctgatcaag cacaggataa agacagggat 540
gatcgttctt ttggccgtga tagaaatcgg gattctgaca aaacagatac agactggagg 600
gctcgtcctg ctacagacag ctttgatgac tacccaccta gaagaggtga tgatagcttt 660
ggagacaagt atcgagatcg ttatgattca gaccggtatc gggatgggta tcgggatggg 720
tatcgggatg gcccacgccg ggatatggat cgatatggtg gccgggatcg ctatgatgac 780
cgaggcagca gagactatga tagaggctat gattccccgga taggcagtgg cagaagagca 840
tttggcagtg ggtatcgcag ggatgatgac tacagaggag gcggggaccg ctatgaagac 900
cgatatgaca gacgggatga tcggtcgtgg agctccagag atgattactc tcgggatgat 960
tataggcgtg atgatagagg tccccccccaa agacccaaac tgaatctaaa gcctcggagt 1020
actcctgaag aagatgattc ctctgctagt acctcccagt ccactcgagc tgcttctatc 1080
tttggagggg caaagcctgt tgacacagct gctagagaaa gagaagtaga agaacggcta 1140
cagaaggaac aagagaagtt gcagcgtcag tggaatgagc caaaactaga acgacggcct 1200
cgggagagac acccaagctg gcgaagtgaa gaaactcagg aacgggaacg gtcgaggaca 1260
ggaagtgagt catcacaaac tgggacctcc accacatcta gcagaaatgc acgaaggaga 1320
gagagtgaga agtctctaga aaatgaaaca ctcaataagg aggaagattg ccactctcca 1380
acttctaaac ctcccaaacc tgatcagccc ctaaaggtaa tgccagcccc tccaccaaag 1440
gagaatgctt gggtgaagcg aagttctaac cctcctgctc gatctcagag ctcagacaca 1500
gagcagcagt cccctacaag tggtgggggga aaagtagctc cagctcaacc atctgaggaa 1560
ggaccaggaa ggaaagatga aaataaagta gatgggatga atgccccaaa aggccaaact 1620
gggaactcta gccgtggtcc aggagacgga gggaacagag accactggaa ggagtcagat 1680
aggaaagatg gcaaaaagga tcaagactcc agatctgcac ctgagccaaa gaaacctgag 1740
gaaaatccag cttctaagtt cagttctgca agcaagtatg ctgctctctc tgttgatggt 1800
gaagatgaaa atgagggaga agattatgcc gaatag 1836

<210> 486
<211> 1866
<212> DNA
<213> Homo sapiens
<400> 486
cgttcctcgg cgccgccggg gccccagagg gcagcggcag caacagcagc agcagcagca 60

gcgggagtgg agatggcggc ggcggcggct caggggggcg ggggcgggga gccccgtaga 120
accgaggggg tcggcccggg ggtcccgggg gaggtggaga tggtgaaggg gcagccgttc 180
gacgtgggcc cgcgctacac gcagttgcag tacatcggcg agggcgcgta cggcatggtc 240
agctcggcct atgaccacgt gcgcaagact cgcgtggcca tcaagaagat cagccccttc 300
gaacatcaga cctactgcca gcgcacgctc cgggagatcc agatcctgct gcgcttccgc 360
catgagaatg tcatcggcat ccgagacatt ctgcgggcgt ccaccctgga agccatgaga 420
gatgtctaca ttgtgcagga cctgatggag actgacctgt acaagttgct gaaaagccag 480
cagctgagca atgaccatat ctgctacttc ctctaccaga tcctgcgggg cctcaagtac 540
atccactccg ccaacgtgct ccaccgagat ctaaagccct ccaacctgct cagcaacacc 600
acctgcgacc ttaagatttg tgatttcggc ctggcccgga ttgccgatcc tgagcatgac 660
cacaccggct tcctgacgga gtatgtggct acgcgctggt accgggcccc agagatcatg 720
ctgaactcca agggctatac caagtccatc gacatctggt ctgtgggctg cattctggct 780
gagatgctct ctaaccggcc catcttccct ggcaagcact acctggatca gctcaaccac 840
attctgggca tcctgggctc cccatcccag gaggacctga attgtatcat caacatgaag 900
gcccgaaact acctacagtc tctgccctcc aagaccaagg tggcttgggc caagcttttc 960
cccaagtcag actccaaagc ccttgacctg ctggaccgga tgttaacctt taaccccaat 1020
aaacggatca cagtggagga agcgctggct cacccctacc tggagcagta ctatgacccg 1080
acggatgagc cagtggccga ggagcccttc accttcgcca tggagctgga tgacctacct 1140
aaggagcggc tgaaggagct catcttccag gagacagcac gcttccagcc cggagtgctg 1200
gaggccccct agcccagaca gacatctctg caccctgggg cctggacctg cctcctgcct 1260
gcccctctcc cgccagactg ttagaaaatg gacactgtgc ccagcccgga ccttggcagc 1320
ccaggccggg gtggagcatg ggcctggcca cctctctcct ttgctgaggc ctccagcttc 1380
aggcaggcca aggccttctc ctccccaccc gccctcccca cggggcctcg ggagctcagg 1440
tggccccagt tcaatctccc gctgctgctg ctgctgcgcc cttaccttcc ccagcgtccc 1500
agtctctggc agttctggaa tggaagggtt ctggctgccc caacctgctg aagggcagag 1560
gtggagggtg gggggcgctg agtagggact cagggccatg cctgcccccc tcatctcatt 1620
caaaccccac cctagtttcc ctgaaggaac attccttagt ctcaagggct agcatccctg 1680
aggagccagg ccgggccgaa tcccctccct gtcaaagctg tcacttcgcg tgccctcgct 1740
gcttctgtgt gtggtgagca gaagtggagc tggggggcgt ggagagcccg gcgcccctgc 1800
cacctccctg acccgtctaa tatataaata tagagatgtg tctatggctg aaaaaaaaaa 1860
aaaaaa 1866

<210> 487
<211> 3033
<212> DNA
<213> Homo sapiens
<400> 487

agcagtcccc gggcttgcgc ggcgcggaga gggaggcgcc ccgcggcggg gcccccactc 60
ggtcagcccg cgcgtcggtc cccgcgcggc cgccatgacc tggagcgcca cggcccgggg 120
cgcccaccag cccgacaaca ccgccttcac tcagcagcgc ctcccccgcct ggcagccgct 180
gctgtcggcc agcatcgcgc tgccgctctt cttctgcgcg ggcctggcct tcatcggcct 240
gggcctgggc ctctactact cctccaacgg catcaaggag ctggagtacg actatacagg 300
cgacccgggc accggtaact gctcggtgtg cgccgcggct ggccagggcc gggcgctgcc 360
gcccccctgc tcgtgcgcct ggtacttctc gctgcccgag ctcttccagg gcccagtgta 420
cctctactac gagctgacca acttctacca gaacaaccgg cgctacggcg tgtcccgcga 480
cgacgcgcag ctgagcggac tccccagcgc gctgcgccac cctgtcaacg agtgcgcccc 540
ctaccagcgc agcgcggccg gcctgcccat cgcgccctgc ggcgccatcg ccaacagcct 600
cttcaacgac tccttctcgc tttggcacca gcgccagccc ggcgggccct acgtcgaggt 660
gccgctcgac cgctccggca tcgcctggtg gaccgactac cacgtcaagt tccgcaaccc 720
gccgctggtc aacggcagcc tggcgttggc cttccagggc acggcgcccc cgcccaactg 780
gcgccggcca gtctacgagc tcagccccga ccccaacaac accggcttca tcaatcagga 840
cttcgtggtg tggatgcgca cggcggcgct gcccacgttc cgcaaactgt acgcgcgcat 900
ccgccagggc aactactcgg ccgggctgcc gcgggggcgcc taccgcgtca acatcaccta 960
caactacccg gtgcgcgcgt tcggcggcca caagctcctc atcttcagca gcatctcgtg 1020
gatgggtggc aagaacccct tcctgggcat cgcctacctg gtcgtcggct ccctctgcat 1080
cctcaccggc tttgtcatgc tggtcgtcta cattcgctac caggaccagg acgacgacga 1140
cgaggagtga ttccggcttt ccagggatcc ctcctgcttc ttgccaaaac tcttgcaagg 1200
tgcttttggc atctcctcct cgcccgtcac ccaattccaa cctcgcctag cttttcctcc 1260
ctttgtgaat gaggggacca gataagggaa ttaccccccct tgctcttggg gggctgctag 1320
actgtcttgc cgcggggagg gatgttgact gcagagtgaa acatccttgc aaactcttcc 1380
cacctccttc acgacactga gttgccatgt gaggttcttc aagtctgaga gtggaaggga 1440
tccctatgga gactcctatt aaacccctat tagaggaaga gattgagaga cctagcaatg 1500
tgaagtaaca aagatcaggc agctgcaagt gactcctgaa tcttgagtcc agggctttcg 1560
ccactacagt acagtggttt tcttttcttt ggtcggggag agtgggctgg aatggagagt 1620
gaggcccaca aattacctgc agagacgtgg aggcgtgagg gagaacatgc ttgttaaata 1680
tgcaggtaga ttaggagaca ccaaacagag attcagacac agtaaggctg ggatgagatc 1740
ctcgaagctg tgttttaaca aactccactg gagagtccca tattccctca aatttgggaa 1800
tcacgaccct gaaccaggtt gggcctgaag cagtcaactg aattcacttt ttcggatagt 1860
aatttgttcc caggggcagt gacaaccatg atgttccagg tttggtctgg cactctgcct 1920

```
tgaacgtagg aagctcttga ttttattgat taacatcaat cagtatgttt aaagttattc    1980
taagaagcaa tagtttattt ttaaaaacct tgtatagcaa aataacttaa aaccctttgt    2040
gatatcatct taccagttta tttggtaaaa acaaacagtt atttggtatt tgtcagaatt    2100
cttcagtgcc tgctattaca gctattttcc aattactaac ttgattatac tcactcaagg    2160
cagtgcaaga tcttgaagta ctttttagca gttaagtaat attgaattgt attgaatagt    2220
ttacatagtt tattctagtc tttgaaaatt actgaacatg gacaatgtgc atgtcattga    2280
catctgcctt agaacttctg ggacaatcct gattcgagag attctatccc attatttaca    2340
tataccaaaa atactttgtt aatttaatgt gttggcttcc caactcctga acacgacaca    2400
attttattat tagattttgt atggtgattt taggctatga aaacatgatc attatatgta    2460
tatagataca tttttatttg ttacaaatgt ttgagcagct cactagccca cccctcctct    2520
atttgggta agagaattta ctaccttttt taactatgta gttgagagca acatgtattt    2580
tgttattttt agaatggtca gtatattgct ataaaatttt aaatgagact atgaaagtta    2640
aagtattctg attctggtta aattaacgaa tatggttcca ggccctgttc tctgggtttt    2700
tgagagagaa taaaggttat gtttgtctta cctttgttat cgagtttgct gaattctttt    2760
gaacgatgat cttaaaggca caaacaccac cagccacttt gctaatttct taatagcaga    2820
tttacattgc agcaagaaaa ccatctttta tagtaacatt cagttaaaat gaactcaatt    2880
cattgttaac ttcctaaaac agaatttgaa ctttatcaac ctcaacgtgt atataaacta    2940
gatagtcctc aatactttat caacctcaac atgtatataa actagatagt cctcaaatac    3000
tgtttgaatt taataaatgt caatttaaaa att                                  3033
```

<210> 488
<211> 3533
<212> DNA
<213> Homo sapiens
<400> 488

```
agcggaggag ccgggcgcgc ctgccacgca aaactaccgg gctggcaggg cggcgggcgc      60
ggtgcgcgat cccgggtggc ggcggcaacg gcggtggtga cggcggcgac tgcagcggcc     120
ggctctcacc tctcccctgt gcacccgcat ctcgccgcgc cgccgagcag ccagcagtcc     180
ccgggtcgcc cagcccacgc gcgcacggcc gagcccagcg cacaatagcg gcgacagcca     240
tggggaaacc ggcgaggaaa ggatgcgagt ggaagcgctt cctgaagaat aactgggtgt     300
tgctgtccac cgtggccgcg gtggtgctag gcattaccac aggagtcttg gttcgagaac     360
acagcaacct ctcaactcta gagaaattct actttgcttt tcctggagaa attctaatgc     420
ggatgctgaa actcatcatt ttgccattaa ttatatccag catgattaca ggtgttgctg     480
cactggattc caacgtatcc ggaaaaattg gtgtgcgcgc tgtcgtgtat tatttctgta     540
ccactctcat tgctgttatt ctaggtattg tgctggtggt gagcatcaag cctggtgtca     600
cccagaaagt gggtgaaatt gcgaggacag gcagcacccc tgaagtcagt acggtggatg     660
ccatgttaga tctcatcagg aatatgttcc ctgagaatct tgtccaggcc tgttttcagc     720
agtacaaaac taagcgtgaa gaagtgaagc ctcccagcga tccagagatg aacatgacag     780
aagagtcctt cacagctgtc atgacaactg caatttccaa gaacaaaaca aaggaataca     840
aaattgttgg catgtattca gatggcataa acgtcctggg cttgattgtc ttttgccttg     900
tctttggact tgtcattgga aaaatgggag aaaagggaca aattctggtg gatttcttca     960
atgctttgag tgatgcaacc atgaaaatcg ttcagatcat catgtgttat atgccactag    1020
gtattttgtt cctgattgct gggaagatca tagaagttga agactgggaa atattccgca    1080
agctgggcct ttacatggcc acagtcctga ctgggcttgc aatccactcc attgtaattc    1140
tcccgctgat atatttcata gtcgtacgaa agaacccttt ccgatttgcc atgggaatgg    1200
cccaggctct cctgacagct ctcatgatct cttccagttc agcaacactg cctgtcacct    1260
tccgctgtgc tgaagaaaat aaccaggtgg acaagaggat cactcgattc gtgttacccg    1320
ttggtgcaac aatcaacatg gatgggactg cgctctatga agcagtggca gcggtgttta    1380
ttgcacagtt gaatgacctg gacttgggca ttgggcagat catcaccatc agtatcacgg    1440
ccacatctgc cagcatcgga gctgctggcg tgccccaggc tggcctggtg accatggtga    1500
ttgtgctgag tgccgtgggc ctgcccgccg aggatgtcac cctgatcatt gctgtcgact    1560
ggctcctgga ccggttcagg accatggtca acgtccttgg tgatgctttt gggacgggca    1620
ttgtggaaaa gctctccaag aaggagctgg agcagatgga tgtttcatct gaagtcaaca    1680
ttgtgaatcc ctttgccttg gaatccacaa tccttgacaa cgaagactca gacaccaaga    1740
agtcttatgt caatggaggc tttgcagtag acaagtctga caccatctca ttcacccaga    1800
cctcacagtt ctagggcccc tggctgcaga tgactggaaa caaggaagga catttccgtg    1860
agagtcatct caaacactgc ttaaggaaaa gagaaacact aatggccaag tgtacatttg    1920
atttgatata cagacctcca gattattttc tatatttgga ttcacagcct ttgcgctctg    1980
ggttttggga tttgggtgtg ggtaagttg aagggaaatc aatttaaagg aaagttctat    2040
tatctgggtt ttagaaattc tataagagac aaagtttgga agtacataaa gtaataactg    2100
ttagaattag gtaatggata tgaaagagaa aatgctttct catgcataga caagtgtttt    2160
gggtttttaa aaaaaatatt ctgtcattgg ttacaaattt ttactcaggc tttctattgg    2220
catggatttc ctttgacctc tcactttttt ataaattata atgcatctaa accacctgtc    2280
cccagttaat gtgccaaaat gtcaattttt aacttatctc cagccaattt caaagaaaac    2340
agaccagcat agttctgcaa taacagtttt aagatgggca tagggtttgg aagaaagaga    2400
gaaggattct tttttcaatg tactgtattg ggacgctggt aactgttaac ccagtgttca    2460
gcatagagct atatatatat atatatgtat atatttatta ttttcatata atttgccaga    2520
cagagatcag aattgaaccg tcaatgtgaa ataaagagtt ctccttgtac ttgaataata    2580
accacgattc caacccaggt ctgctttggg gcttatcaga actcctttct aaggagcact    2640
```

```
agaatgagaa atcatgttgt tcgatcgttt cacatctgta tatcagctct aaagcagaga   2700
tgtattatgg tgatactcca aggtggcata gccattcatt tacaacttcc agatttgagc   2760
tgcctggagg gaatccatat cagctctgca taagattata tacaaagctg tcactcacaa   2820
aaggctggat gtgctttcat ccaactggaa ggctttattc ttccaagttc attcatactc   2880
aaagaggcca gtactttgcc atccttgcac tttctgttat cagggcccaa ataacagtgg   2940
caagctacca actaagttgt attttaataa agattccatg ggttgaacaa gccacgttgc   3000
agaaaaagag cttcccctaa cctgggttgt tgcagagtaa atcccacgac ataagctggt   3060
atcactggtt cgggggaaat agttccattc tatgactctt gtctcctcct ccaggaggac   3120
tgttctaact agtaatcttg gccctattca ttacatcctc tgcttgtcat tctgctaatt   3180
tatgaagata gtttattata gtctgtactt cagttctcat cttgtaaata atgcttaaca   3240
taaacttgta cttacactga aatccaaaat agtcatgttt ctgcagtatt ctgtagccaa   3300
cttaaacctg tgctttcatg tttaagaaat gagaaattgt gccaaagata gcagaagagt   3360
agataagtgc tcagtattga cgacctacat ctgaaatcta caacataatg atactgaatt   3420
gttatgtaaa catcataaat agtaaataat gattcaatgt gaattttaaa atgcaaatat   3480
tgctattgtt tataggaaat aaatctaaat ataaacgaaa aaaaaaaaaa aaa          3533
```

<210> 489
<211> 3263
<212> DNA
<213> Homo sapiens
<400> 489

```
ggcatcactc gagcccaggt cccagtcatc accactcaca gcgctcggcg ttcaggaaga     60
ggagcaccag cggagcgcgg ctgcttcagc ggcgggcggg cgccagaaag gccccgatcg    120
aaaagcctgg gagggccgcc gaactacccc cggagggagg agccagtccg aacccaaggc    180
gccaccgccg cagaagcgga gcgaggcagc attcgcctcc atggcccact cgccggtggc    240
tgtccaagtg cctgggatgc agaataacat agctgatcca gaagaactgt tcacaaaatt    300
agagcgcatt gggaaaggct catttgggga agttttcaaa ggaattgata accgtaccca    360
gcaagtcgtt gctattaaaa tcatagacct tgaggaagcc gaagatgaaa tagaagacat    420
tcagcaagaa ataactgtct tgagtcaatg tgacagctca tatgtaacaa aatactatgg    480
gtcatattta aaggggtcta aattatggat aataatggaa tacctgggcg gtggttcagc    540
actggatctt cttcgagctg gtccatttga tgagttccag attgctacca tgctaaagga    600
aattttaaaa ggtctggact atctgcattc agaaaagaaa attcaccgag acataaaagc    660
tgccaatgtc ttgctctcag aacaaggaga tgttaaactt gctgattttg gagttgctgg    720
tcagctgaca gatacacaga ttaaaagaaa tacctttgtg ggaactccat ttggatggc     780
tcctgaagtt attcaacagt cagcttatga ctcaaaagct gacatttggt cattgggaat    840
tactgctatt gaactagcca agggagagcc acctaactcc gatatgcatc caatgagagt    900
tctgtttctt attcccaaaa acaatcctcc aactcttgtt ggagacttta ctaagtcttt    960
taaggagttt attgatgctt gcctgaacaa agatccatca tttcgtccta cagcaaaaga   1020
acttctgaaa cacaaattca ttgtaaaaaa ttcaaagaag acttcttatc tgactgaact   1080
gatagatcgt tttaagagat ggaaggcaga aggacacagt gatgatgaat ctgattccga   1140
gggctctgat tcggaatcta ccagcaggga aaacaatact catcctgaat ggagctttac   1200
caccgtacga aagaagcctg atccaaagaa agtacagaat ggggcagagc aagatcttgt   1260
gcaaaccctg agttgtttgt ctatgataat cacacctgca tttgctgaac ttaaacagca   1320
ggacgagaat aacgctagca ggaatcaggc gattgaagaa ctcgagaaaa gtattgctgt   1380
ggctgaagcc gcctgtcccg gcatcacaga taaaatggtg aagaaactaa ttgaaaaatt   1440
tcaaaagtgt tcagcagacg aatcccccta agaaacttat tattggcttc tgtttcatat   1500
ggacccagag agccccacca aacctacgtc aagattaaca atgcttaacc catgagctcc   1560
atgtgccttt tggatctttg caacactgaa gatttggaag aagctattaa actattttgt   1620
gatggcgttt atcattttat attttgaaag gattattttg taaggaataa cttttaatac   1680
tatagtttca cctgtattct agtaaatgtt gagacaccgt tttgctttta agtatcccta   1740
tttcttaagt tacgaggatg aatacctttc acattttgat ctttagttga ctctacagtc   1800
atgaaacata caggtctttc aaagtcattc tcaatattca gcttttgtaa attatcaagc   1860
ttcaaaaagc tttttttttta aaaaaaaaaa catgcatatt ctaaaaatga ctattggtgg   1920
ggaggtgtaa ataagtcata ccttcttaaa acagaaaatt taagtaaagt cttttaaatg   1980
aaacctgtaa aagtattgac tcttctacca agttggtatg atattccagg cagctcaatg   2040
attatcacat ttgagaccct gtgtttgaag catttacagg caatgtacag caacagaggt   2100
acctcttggt gtatagtatt tacattctct tttaggtaga agaggcaatt ttacccttat   2160
ttcacatggt tagaaattta aagcaagatc atttacccaa ggataggtgt ttggtaatgt   2220
tgaaggagtt agtctggctt catgttttac atcttcaact aaaatcccat actatctgct   2280
tggatttgga gagccaaaaa ataaagctga ttgtcatgtg attaaatatc tgatcaacag   2340
gtatgaatat aacttaaatc agcatatttt tgccatggta ataaattgtc ctataaacta   2400
tttatatatt tttgttcttc ataattatca ctaataagca tcagtttgtt gtttttaaaa   2460
ggatatttaa gtgagcattt tctagttcat atgaaaataa ccatagtaca ggatgatttc   2520
tgtccacaca aaggttaaat tagattgcac agttaatttt cacttatatt tatggtacta   2580
ttatgtgggt gatgcctttt tcttttaagc ccagtacata tattatgcct gcctaagttc   2640
tgaactgggg ctgtatttca gtagttgtag aattattgat atttagtttt gatagctaat   2700
gtttaattgt ttggatctgc acagtttggt ttttgcacaa aagtcattta aaaaaatctg   2760
agtaattgtc aaatattaaa agaaagatat tcttcctgta aggaatacag tttttagtca   2820
aagtggccat tacatcctct ttttaattta cataatacag atacttgaga aagttgttgt   2880
```

ggtgttgtat gccaagaaaa ttctttttat tggtgcctat attgtaacaa ttatttttaa 2940
tgcattgtat tttgaagtaa cggttcagtt aaatttttca cctgctgtgt aactgaagca 3000
caattacagt ttataatcat ctgtagaagt ctggagataa ttttgcaact catgttatgg 3060
gttaaatgaa tatttttgta aaagtaaaag caacaaattt ataaattgat tatttgaaac 3120
tttacaacac aattgcatcc caaatacaaa ttgtattgct tattcattat agctattcgt 3180
cctgtaatct gtttctaggt gaagcatact ccagtgtttt aggggttttg aaaataaata 3240
tttaaatttc acagtcaaaa aaa 3263

<210> 490
<211> 3197
<212> DNA
<213> Homo sapiens
<400> 490
ggaccacagc tcctcccgtg catccactcg gcctgggagg ttctggattt tggctgtcga 60
gggagtttgc ctgcctctcc agagaaagat ggtcatgagg cccctgtgga gtctgcttct 120
ctgggaagcc ctacttccca ttacagttac tggtgcccaa gtgctgagca aagtcggggg 180
ctcggtgctg ctggtggcag cgcgtccccc tggcttccaa gtccgtgagg ctatctggcg 240
atctctctgg ccttcagaag agctcctggc cacgtttttc cgaggctccc tggagactct 300
gtaccattcc cgcttcctgg gccgagccca gctacacagc aacctcagcc tggagctcgg 360
gccgctggag tctggagaca gcggcaactt ctccgtgttg atggtggaca caaggggcca 420
gccctggacc cagaccctcc agctcaaggt gtacgatgca gtgcccaggc ccgtggtaca 480
agtgttcatt gctgtagaaa gggatgctca gccctccaag acctgccagg ttttcttgtc 540
ctgttgggcc cccaacatca gcgaaataac ctatagctgg cgacgggaga caaccatgga 600
ctttggtatg gaaccacaca gcctcttcac agacggacag gtgctgagca tttccctggg 660
accaggagac agagatgtgg cctattcctg cattgtctcc aaccctgtca gctgggactt 720
ggccacagtc acgccctggg atagctgtca tcatgaggca gcaccaggga aggcctccta 780
caaagatgtg ctgctggtgg tggtgcctgt ctcgctgctc ctgatgctgg ttactctctt 840
ctctgcctgg cactggtgcc cctgctcagg gaaaaagaaa aaggatgtcc atgctgacag 900
agtgggtcca gagacagaga acccccttgt gcaggatctg ccataaagga caatatgaac 960
tgatgcctgg actatcagta accccactgc acaggcacac gatgctctgg gacataactg 1020
gtgcctggaa atcaccatgg tcctcatatc tcccatggga atcctgtcct gcctcgaagg 1080
agcagcctgg gcagccatca caccacgagg acaggaagca ccagcacgtt tcacacctcc 1140
cccttccctc tcccatcttc tcatatcctg gctcttctct gggcaagatg agccaagcag 1200
aacattccat ccaggacact ggaagttctc caggatccag atccatgggg acattaatag 1260
tccaaggcat tccctccccc accactattc ataaagtatt aaccaactgg caccaaggaa 1320
ttgcctccag cctgagtcct aggctctaaa agatattaca tatttgaact aatagaggaa 1380
ctctgagtca cccatgccag catcagcttc agccccagac cctgcagttt gagatctgat 1440
gcttcctgag ggccaaggca ttgctgtaag aaaaggtcta gaaataggtg aaagtgagag 1500
gtgggggaca ggggtttctc tttctggcct aaggactttc aggtaatcag agttcatggg 1560
ccctcaaagg taaattgcag ttgtagacac cgaggatggt tgacaaccca tggttgagat 1620
gggcaccgtt ttgcaggaaa caccatatta atagacatcc tcaccatctc catccgctct 1680
cacgcctcct gcaggatctg ggagtgaggg tggagagtct ttcctcacgc tccagcacag 1740
tggccaggaa aagaaatact gaatttgccc cagccaacag gacgttcttg cacaacttca 1800
agaaaagcag ctcagctcag gatgagtctt cctgcctgaa actgagagag tgaagaacca 1860
taaaacgcta tgcagaagga acattatgga gagaaagggt actgaggcac tctagaatct 1920
gccacattca ttttcaaatg caaatgcaga agacttacct tagttcaagg ggaggggaca 1980
aagaccccac agcccaacag caggactgta gaggtcactc tgactccatc aaacttttta 2040
ttgtggccat cttaggaaaa tacattctgc ccctgaatga ttctgtctag aaaagctctg 2100
gagtattgat cactactgga aaaacactta aggagctaaa cttaccttcg gggattatta 2160
gctgataagg ttcacagttt ctctcaccca ggtgtaactg gatttttttct ggggcctcaa 2220
tccagtcttg ataacagcga ggaaagaggt attgaagaaa caggggtggg tttgaagtac 2280
tattttcccc agggtggctt caatctcccc acctaggatg tcagccctgt ccaaggacct 2340
tccctcttct cccccagttc cctgggcaat cacttcacct tggacaaagg atcagcacag 2400
ctggcctcca gatccacatc accactcttc cactcgattg ttcccagatc ctccctgcct 2460
ggcctgctca gaggttccct gttggtaacc tggctttatc aaattctcat ccctttccca 2520
cacccacttc tctcctatca ccttccccca agattacctg aacagggtcc atggccactc 2580
aacctgtcag cttgcaccat ccccacctgc cacctacagt caggccacat gcctggtcac 2640
tgaatcatgc aaaactggcc tcagtcccta aaaatgatgt ggaaaggaaa gcccaggatc 2700
tgacaatgag ccctggtgga tttgtgggga aaaaatacac agcactcccc acctttcttt 2760
cgttcatctc cagggcccca cctcagatca aagcagctct ggatgagatg ggacctgcag 2820
ctctccctcc acaaggtgac tcttagcaac ctcatttcga cagtggtttg tagcgtggtg 2880
caccagggcc ttgttgaaca gatccacact gctctaataa agttcccatc cttaatgact 2940
cacttgtcaa ctagtggact aattaaccct ccaccaaaaa aacacaaagt gcttctgtga 3000
gaccaatttt gtgctaatga gcattgagac tgatgctttg taagtcacac cacaacaaat 3060
attgattgag ggcgctgcat gtgctgggta catttcttgg cacttgggaa tcagtagtca 3120
agcgaaaccc ttgcctttga gagtttatgg tctggataat ataaataaac aagtaagcat 3180
aaaaaaaaaa aaaaaaa 3197

<210> 491

<211> 1646
<212> DNA
<213> Homo sapiens
<400> 491
acagaataat ggcgtctcgt agccccaggc gacagcgtgg aggggcgggt ctgtcgattg 60
gatgaacgca gctgagatta ctcccagcca ctaaggacga agaggtgggg cggtggcgtc 120
ccacgcctcg tgcgacagtg ggcggggctt tgttgcctga gtaaccgtat gatggtggtg 180
gtggtggtgt cttcctgtct caacgatacc tattttctag tgctgagatc ctgagacaat 240
gaatcatagt gaaagattcg ttttcattgc agagtggtat gatccaaatg cttcacttct 300
tcgacgttat gagcttttat tttacccagg ggatggatct gttgaaatgc atgatgtaaa 360
gaatcatcgc acctttttaa agcggaccaa atatgataac ctgcacttgg aagatttatt 420
tataggcaac aaagtgaatg tcttttctcg acaactggta ttaattgact atggggatca 480
atatacagct cgccagctgg gcagtaggaa agaaaaaacg ctagccctaa ttaaaccaga 540
tgcaatatca aaggctggag aaataattga aataataaac aaagctggat ttactataac 600
caaactcaaa atgatgatgc tttcaaggaa agaagcattg gattttcatg tagatcacca 660
gtcaagaccc tttttcaatg agctgatcca gtttattaca actggtccta ttattgccat 720
ggagatttta agagatgatg ctatatgtga atggaaaaga ctgctgggac ctgcaaactc 780
tggagtggca cgcacagatg cttctgaaag cattagagcc ctctttggaa cagatggcat 840
aagaaatgca gcgcatggcc ctgattcttt tgcttctgcg gccagagaaa tggagttgtt 900
ttttccttca agtggaggtt gtgggccggc aaacactgct aaatttacta attgtacctg 960
ttgcattgtt aaaccccatg ctgtcagtga aggactgttg ggaaagatcc tgatggctat 1020
ccgagatgca ggttttgaaa tctcagctat gcagatgttc aatatggatc gggttaatgt 1080
tgaggaattc tatgaagttt ataaaggagt agtgaccgaa tatcatgaca tggtgacaga 1140
aatgtattct ggcccttgtg tagcaatgga gattcaacag aataatgcta caaagacatt 1200
tcgagaattt tgtggacctg ctgatcctga aattgcccgg catttacgcc ctggaactct 1260
cagagcaatc tttggtaaaa ctaagatcca gaatgctgtt cactgtactg atctgccaga 1320
ggatggccta ttagaggttc aatacttctt caagatcttg gataattagt ggtgtggaaa 1380
gtaaagaagt cacaggttgg gacatttaga caagagtgaa tcacacacga ggaatgtgtt 1440
cattctttta ttgtccgttg ttttaacctg actgaataca agatcaacaa gagcactgta 1500
ctcctggcaa ttattacata tgttagaaca tggattttgc actgtagaca acatttaaca 1560
ccagtctatg ggtactgca ttgcttttta taaagttcaa aataaagatt tattttcaaa 1620
caaaaaaaaa aaaaaaaaaa aaaaaa 1646

<210> 492
<211> 158
<212> PRT
<213> Homo sapiens
<400> 492
Met Met Gln Lys Leu Leu Lys Cys Ser Arg Leu Val Leu Ala Leu Ala
1 5 10 15
Leu Ile Leu Val Leu Glu Ser Ser Val Gln Gly Tyr Pro Thr Arg Arg
20 25 30
Ala Arg Tyr Gln Trp Val Arg Cys Asn Pro Asp Ser Asn Ser Ala Asn
35 40 45
Cys Leu Glu Glu Lys Gly Pro Met Phe Glu Leu Leu Pro Gly Glu Ser
50 55 60
Asn Lys Ile Pro Arg Leu Arg Thr Asp Leu Phe Pro Lys Thr Arg Ile
65 70 75 80
Gln Asp Leu Asn Arg Ile Phe Pro Leu Ser Glu Asp Tyr Ser Gly Ser
85 90 95
Gly Phe Gly Ser Gly Ser Gly Ser Gly Ser Gly Ser Gly Ser Gly Phe
100 105 110
Leu Thr Glu Met Glu Gln Asp Tyr Gln Leu Val Asp Glu Ser Asp Ala
115 120 125
Phe His Asp Asn Leu Arg Ser Leu Asp Arg Asn Leu Pro Ser Asp Ser
130 135 140
Gln Asp Leu Gly Gln His Gly Leu Glu Glu Asp Phe Met Leu
145 150 155

<210> 493
<211> 592
<212> PRT
<213> Homo sapiens
<400> 493
Met Ala Ser Glu Ile His Met Thr Gly Pro Met Cys Leu Ile Glu Asn
1 5 10 15
Thr Asn Gly Arg Leu Met Ala Asn Pro Glu Ala Leu Lys Ile Leu Ser
20 25 30
Ala Ile Thr Gln Pro Met Val Val Val Ala Ile Val Gly Leu Tyr Arg

```
        35                  40                  45
Thr Gly Lys Ser Tyr Leu Met Asn Lys Leu Ala Gly Lys Lys Lys Gly
    50                  55                  60
Phe Ser Leu Gly Ser Thr Val Gln Ser His Thr Lys Gly Ile Trp Met
65                  70                  75                  80
Trp Cys Val Pro His Pro Lys Lys Pro Gly His Ile Leu Val Leu Leu
                85                  90                  95
Asp Thr Glu Gly Leu Gly Asp Val Glu Lys Gly Asp Asn Gln Asn Asp
            100                 105                 110
Ser Trp Ile Phe Ala Leu Ala Val Leu Leu Ser Ser Thr Phe Val Tyr
        115                 120                 125
Asn Ser Ile Gly Thr Ile Asn Gln Gln Ala Met Asp Gln Leu Tyr Tyr
    130                 135                 140
Val Thr Glu Leu Thr His Arg Ile Arg Ser Lys Ser Ser Pro Asp Glu
145                 150                 155                 160
Asn Glu Asn Glu Val Glu Asp Ser Ala Asp Phe Val Ser Phe Phe Pro
                165                 170                 175
Asp Phe Val Trp Thr Leu Arg Asp Phe Ser Leu Asp Leu Glu Ala Asp
            180                 185                 190
Gly Gln Pro Leu Thr Pro Asp Glu Tyr Leu Thr Tyr Ser Leu Lys Leu
        195                 200                 205
Lys Lys Gly Thr Ser Gln Lys Asp Glu Thr Phe Asn Leu Pro Arg Leu
    210                 215                 220
Cys Ile Arg Lys Phe Phe Pro Lys Lys Lys Cys Phe Val Phe Asp Arg
225                 230                 235                 240
Pro Val His Arg Arg Lys Leu Ala Gln Leu Glu Lys Leu Gln Asp Glu
                245                 250                 255
Glu Leu Asp Pro Glu Phe Val Gln Gln Val Ala Asp Phe Cys Ser Tyr
            260                 265                 270
Ile Phe Ser Asn Ser Lys Thr Lys Thr Leu Ser Gly Gly Ile Gln Val
        275                 280                 285
Asn Gly Pro Arg Leu Glu Ser Leu Val Leu Thr Tyr Val Asn Ala Ile
    290                 295                 300
Ser Ser Gly Asp Leu Pro Cys Met Glu Asn Ala Val Leu Ala Leu Ala
305                 310                 315                 320
Gln Ile Glu Asn Ser Ala Ala Val Gln Lys Ala Ile Ala His Tyr Glu
                325                 330                 335
Gln Gln Met Gly Gln Lys Val Gln Leu Pro Thr Glu Ser Leu Gln Glu
            340                 345                 350
Leu Leu Asp Leu His Arg Asp Ser Glu Arg Glu Ala Ile Glu Val Phe
        355                 360                 365
Ile Arg Ser Ser Phe Lys Asp Val Asp His Leu Phe Gln Lys Glu Leu
    370                 375                 380
Ala Ala Gln Leu Glu Lys Lys Arg Asp Asp Phe Cys Lys Gln Asn Gln
385                 390                 395                 400
Glu Ala Ser Ser Asp Arg Cys Ser Gly Leu Leu Gln Val Ile Phe Ser
                405                 410                 415
Pro Leu Glu Glu Glu Val Lys Ala Gly Ile Tyr Ser Lys Pro Gly Gly
            420                 425                 430
Tyr Arg Leu Phe Val Gln Lys Leu Gln Asp Leu Lys Lys Lys Tyr Tyr
        435                 440                 445
Glu Glu Pro Arg Lys Gly Ile Gln Ala Glu Glu Ile Leu Gln Thr Tyr
    450                 455                 460
Leu Lys Ser Lys Glu Ser Met Thr Asp Ala Ile Leu Gln Thr Asp Gln
465                 470                 475                 480
Thr Leu Thr Glu Lys Glu Lys Glu Ile Glu Val Glu Arg Val Lys Ala
                485                 490                 495
Glu Ser Ala Gln Ala Ser Ala Lys Met Leu Gln Glu Met Gln Arg Lys
            500                 505                 510
Asn Glu Gln Met Met Glu Gln Lys Glu Arg Ser Tyr Gln Glu His Leu
        515                 520                 525
Lys Gln Leu Thr Glu Lys Met Glu Asn Asp Arg Val Gln Leu Leu Lys
    530                 535                 540
Glu Gln Glu Arg Thr Leu Ala Leu Lys Leu Gln Glu Gln Glu Gln Leu
545                 550                 555                 560
Leu Lys Glu Gly Phe Gln Lys Glu Ser Arg Ile Met Lys Asn Glu Ile
                565                 570                 575
Gln Asp Leu Gln Thr Lys Met Arg Arg Arg Lys Ala Cys Thr Ile Ser
            580                 585                 590
```

<210> 494
<211> 632
<212> PRT
<213> Homo sapiens
<400> 494

```
Met Ser Arg Val Arg Asp Ala Gly Cys Val Ala Ala Gly Ile Val Ile
1               5                   10                  15
Gly Ala Gly Ala Trp Tyr Cys Val Tyr Lys Tyr Thr Arg Gly Arg Asp
            20                  25                  30
Gln Thr Lys Lys Arg Met Ala Lys Pro Lys Asn Arg Ala Val Ala Gly
        35                  40                  45
Thr Gly Ala Arg Ala Arg Ala Gly Leu Arg Ala Gly Phe Thr Ile Asp
    50                  55                  60
Leu Gly Ser Gly Phe Ser Pro Pro Thr Pro Val Arg Ala Glu Ala Glu
65                  70                  75                  80
Asp Arg Ala Gln Asp Glu Ala Ser Ala Leu Asp Thr Val Gly Ala Glu
                85                  90                  95
Ala Val Ala Pro Ala Ala Ser Ser Ala Glu Ala Gln Ser Gly Ala Gly
            100                 105                 110
Ser Gln Ala Gln Glu Ala Asp Gly Ala Gly Val Gly Pro Lys Ala Glu
        115                 120                 125
Ser Val Val Gly Ala Ala Met Ala Ser Ala Ile Ala Pro Pro Pro Gly
    130                 135                 140
Val Thr Glu Ala Leu Gly Ala Ala Glu Ala Pro Ala Met Ala Gly Ala
145                 150                 155                 160
Pro Lys Val Ala Glu Ala Pro Arg Glu Ala Glu Thr Ser Arg Ala Ala
                165                 170                 175
Val Pro Pro Gly Thr Val Val Pro Thr Glu Ala Ala Ala Pro Thr Glu
            180                 185                 190
Val Thr Glu Gly Pro Gly Val Ala Ala Pro Thr Lys Val Ala Glu Ala
        195                 200                 205
Pro Gly Val Ala Ser Pro Thr Glu Ala Ala Glu Ala Pro Val Pro Ala
    210                 215                 220
Thr Pro Thr Gly Ala Ala Ala Pro Thr Gly Ala Ala Glu Ser Pro Gly
225                 230                 235                 240
Thr Ser Gly Ser Pro Arg Thr Ala Val Val Pro Gly Thr Ser Ala Ala
                245                 250                 255
Lys Lys Ala Thr Pro Gly Ala His Thr Gly Ala Ile Pro Lys Ala Thr
            260                 265                 270
Ser Ala Thr Gly Ala Val Pro Lys Gly Gly Gly Lys Gly Val Thr Arg
        275                 280                 285
Ser Arg Asn Gly Gly Lys Gly Lys Gly Lys Lys Ser Lys Val Glu Val
    290                 295                 300
Asp Glu Leu Gly Met Gly Phe Arg Pro Gly Asp Gly Ala Ala Ala Ala
305                 310                 315                 320
Ala Ala Ala Ser Ala Asn Gly Gly Gln Ala Phe Leu Ala Glu Val Pro
                325                 330                 335
Asp Ser Glu Glu Gly Glu Ser Gly Trp Thr Asp Thr Glu Ser Asp Ser
            340                 345                 350
Asp Ser Glu Pro Glu Thr Gln Arg Arg Gly Arg Gly Arg Arg Pro Val
        355                 360                 365
Ala Met Gln Lys Arg Pro Phe Pro Tyr Glu Ile Asp Glu Ile Leu Gly
    370                 375                 380
Val Arg Asp Leu Arg Lys Val Leu Ala Leu Leu Gln Lys Ser Asp Asp
385                 390                 395                 400
Pro Phe Ile Gln Gln Val Ala Leu Leu Thr Leu Ser Asn Asn Ala Asn
                405                 410                 415
Tyr Ser Cys Asn Gln Glu Thr Ile Arg Lys Leu Gly Gly Leu Pro Ile
            420                 425                 430
Ile Ala Asn Met Ile Asn Lys Thr Asp Pro His Ile Lys Glu Lys Ala
        435                 440                 445
Leu Met Ala Met Asn Asn Leu Ser Glu Asn Tyr Glu Asn Gln Gly Arg
    450                 455                 460
Leu Gln Val Tyr Met Asn Lys Val Met Asp Asp Ile Met Ala Ser Asn
465                 470                 475                 480
Leu Asn Ser Ala Val Gln Val Val Gly Leu Lys Phe Leu Thr Asn Met
                485                 490                 495
Thr Ile Thr Asn Asp Tyr Gln His Leu Leu Val Asn Ser Ile Ala Asn
```

```
            500                 505                 510
Phe Phe Arg Leu Leu Ser Gln Gly Gly Gly Lys Ile Lys Val Glu Ile
        515                 520                 525
Leu Lys Ile Leu Ser Asn Phe Ala Glu Asn Pro Asp Met Leu Lys Lys
    530                 535                 540
Leu Leu Ser Thr Gln Val Pro Ala Ser Phe Ser Ser Leu Tyr Asn Ser
545                 550                 555                 560
Tyr Val Glu Ser Glu Ile Leu Ile Asn Ala Leu Thr Leu Phe Glu Ile
                565                 570                 575
Ile Tyr Asp Asn Leu Arg Ala Glu Val Phe Asn Tyr Arg Glu Phe Asn
            580                 585                 590
Lys Gly Ser Leu Phe Tyr Leu Cys Thr Thr Ser Gly Val Cys Val Lys
        595                 600                 605
Lys Ile Arg Ala Leu Ala Asn His His Asp Leu Leu Val Lys Val Lys
    610                 615                 620
Val Ile Lys Leu Val Asn Lys Phe
625                 630

<210>  495
<211>  219
<212>  PRT
<213>  Homo sapiens
<400>  495
Met Gly Met Ser Ser Leu Lys Leu Leu Lys Tyr Val Leu Phe Phe Phe
1               5                   10                  15
Asn Leu Leu Phe Trp Ile Cys Gly Cys Cys Ile Leu Gly Phe Gly Ile
            20                  25                  30
Tyr Leu Leu Ile His Asn Asn Phe Gly Val Leu Phe His Asn Leu Pro
        35                  40                  45
Ser Leu Thr Leu Gly Asn Val Phe Val Ile Val Gly Ser Ile Ile Met
    50                  55                  60
Val Val Ala Phe Leu Gly Cys Met Gly Ser Ile Lys Glu Asn Lys Cys
65                  70                  75                  80
Leu Leu Met Ser Phe Phe Ile Leu Leu Leu Ile Ile Leu Leu Ala Glu
                85                  90                  95
Val Thr Leu Ala Ile Leu Leu Phe Val Tyr Glu Gln Lys Leu Asn Glu
            100                 105                 110
Tyr Val Ala Lys Gly Leu Thr Asp Ser Ile His Arg Tyr His Ser Asp
        115                 120                 125
Asn Ser Thr Lys Ala Ala Trp Asp Ser Ile Gln Ser Phe Leu Gln Cys
    130                 135                 140
Cys Gly Ile Asn Gly Thr Ser Asp Trp Thr Ser Gly Pro Pro Ala Ser
145                 150                 155                 160
Cys Pro Ser Asp Arg Lys Val Glu Gly Cys Tyr Ala Lys Ala Arg Leu
                165                 170                 175
Trp Phe His Ser Asn Phe Leu Tyr Ile Gly Ile Ile Thr Ile Cys Val
            180                 185                 190
Cys Val Ile Glu Val Leu Gly Met Ser Phe Ala Leu Thr Leu Asn Cys
        195                 200                 205
Gln Ile Asp Lys Thr Ser Gln Thr Ile Gly Leu
    210                 215

<210>  496
<211>  739
<212>  PRT
<213>  Homo sapiens
<400>  496
Met Pro Gly Lys Met Val Val Ile Leu Gly Ala Ser Asn Ile Leu Trp
1               5                   10                  15
Ile Met Phe Ala Ala Ser Gln Ala Phe Lys Ile Glu Thr Thr Pro Glu
            20                  25                  30
Ser Arg Tyr Leu Ala Gln Ile Gly Asp Ser Val Ser Leu Thr Cys Ser
        35                  40                  45
Thr Thr Gly Cys Glu Ser Pro Phe Phe Ser Trp Arg Thr Gln Ile Asp
    50                  55                  60
Ser Pro Leu Asn Gly Lys Val Thr Asn Glu Gly Thr Thr Ser Thr Leu
65                  70                  75                  80
Thr Met Asn Pro Val Ser Phe Gly Asn Glu His Ser Tyr Leu Cys Thr
                85                  90                  95
```

Ala Thr Cys Glu Ser Arg Lys Leu Glu Lys Gly Ile Gln Val Glu Ile
100 105 110
Tyr Ser Phe Pro Lys Asp Pro Glu Ile His Leu Ser Gly Pro Leu Glu
115 120 125
Ala Gly Lys Pro Ile Thr Val Lys Cys Ser Val Ala Asp Val Tyr Pro
130 135 140
Phe Asp Arg Leu Glu Ile Asp Leu Leu Lys Gly Asp His Leu Met Lys
145 150 155 160
Ser Gln Glu Phe Leu Glu Asp Ala Asp Arg Lys Ser Leu Glu Thr Lys
165 170 175
Ser Leu Glu Val Thr Phe Thr Pro Val Ile Glu Asp Ile Gly Lys Val
180 185 190
Leu Val Cys Arg Ala Lys Leu His Ile Asp Glu Met Asp Ser Val Pro
195 200 205
Thr Val Arg Gln Ala Val Lys Glu Leu Gln Val Tyr Ile Ser Pro Lys
210 215 220
Asn Thr Val Ile Ser Val Asn Pro Ser Thr Lys Leu Gln Glu Gly Gly
225 230 235 240
Ser Val Thr Met Thr Cys Ser Ser Glu Gly Leu Pro Ala Pro Glu Ile
245 250 255
Phe Trp Ser Lys Lys Leu Asp Asn Gly Asn Leu Gln His Leu Ser Gly
260 265 270
Asn Ala Thr Leu Thr Leu Ile Ala Met Arg Met Glu Asp Ser Gly Ile
275 280 285
Tyr Val Cys Glu Gly Val Asn Leu Ile Gly Lys Asn Arg Lys Glu Val
290 295 300
Glu Leu Ile Val Gln Glu Lys Pro Phe Thr Val Glu Ile Ser Pro Gly
305 310 315 320
Pro Arg Ile Ala Ala Gln Ile Gly Asp Ser Val Met Leu Thr Cys Ser
325 330 335
Val Met Gly Cys Glu Ser Pro Ser Phe Ser Trp Arg Thr Gln Ile Asp
340 345 350
Ser Pro Leu Ser Gly Lys Val Arg Ser Glu Gly Thr Asn Ser Thr Leu
355 360 365
Thr Leu Ser Pro Val Ser Phe Glu Asn Glu His Ser Tyr Leu Cys Thr
370 375 380
Val Thr Cys Gly His Lys Lys Leu Glu Lys Gly Ile Gln Val Glu Leu
385 390 395 400
Tyr Ser Phe Pro Arg Asp Pro Glu Ile Glu Met Ser Gly Gly Leu Val
405 410 415
Asn Gly Ser Ser Val Thr Val Ser Cys Lys Val Pro Ser Val Tyr Pro
420 425 430
Leu Asp Arg Leu Glu Ile Glu Leu Leu Lys Gly Glu Thr Ile Leu Glu
435 440 445
Asn Ile Glu Phe Leu Glu Asp Thr Asp Met Lys Ser Leu Glu Asn Lys
450 455 460
Ser Leu Glu Met Thr Phe Ile Pro Thr Ile Glu Asp Thr Gly Lys Ala
465 470 475 480
Leu Val Cys Gln Ala Lys Leu His Ile Asp Asp Met Glu Phe Glu Pro
485 490 495
Lys Gln Arg Gln Ser Thr Gln Thr Leu Tyr Val Asn Val Ala Pro Arg
500 505 510
Asp Thr Thr Val Leu Val Ser Pro Ser Ser Ile Leu Glu Glu Gly Ser
515 520 525
Ser Val Asn Met Thr Cys Leu Ser Gln Gly Phe Pro Ala Pro Lys Ile
530 535 540
Leu Trp Ser Arg Gln Leu Pro Asn Gly Glu Leu Gln Pro Leu Ser Glu
545 550 555 560
Asn Ala Thr Leu Thr Leu Ile Ser Thr Lys Met Glu Asp Ser Gly Val
565 570 575
Tyr Leu Cys Glu Gly Ile Asn Gln Ala Gly Arg Ser Arg Lys Glu Val
580 585 590
Glu Leu Ile Ile Gln Val Thr Pro Lys Asp Ile Lys Leu Thr Ala Phe
595 600 605
Pro Ser Glu Ser Val Lys Glu Gly Asp Thr Val Ile Ile Ser Cys Thr
610 615 620
Cys Gly Asn Val Pro Glu Thr Trp Ile Ile Leu Lys Lys Lys Ala Glu
625 630 635 640
Thr Gly Asp Thr Val Leu Lys Ser Ile Asp Gly Ala Tyr Thr Ile Arg

```
                645                 650                 655
Lys Ala Gln Leu Lys Asp Ala Gly Val Tyr Glu Cys Glu Ser Lys Asn
            660                 665                 670
Lys Val Gly Ser Gln Leu Arg Ser Leu Thr Leu Asp Val Gln Gly Arg
        675                 680                 685
Glu Asn Asn Lys Asp Tyr Phe Ser Pro Glu Leu Leu Val Leu Tyr Phe
    690                 695                 700
Ala Ser Ser Leu Ile Ile Pro Ala Ile Gly Met Ile Ile Tyr Phe Ala
705                 710                 715                 720
Arg Lys Ala Asn Met Lys Gly Ser Tyr Ser Leu Val Glu Ala Gln Lys
                725                 730                 735
Ser Lys Val


<210>  497
<211>  441
<212>  PRT
<213>  Homo sapiens
<400>  497
Met Ala Glu Pro Arg Gln Glu Phe Glu Val Met Glu Asp His Ala Gly
1               5                   10                  15
Thr Tyr Gly Leu Gly Asp Arg Lys Asp Gln Gly Gly Tyr Thr Met His
            20                  25                  30
Gln Asp Gln Glu Gly Asp Thr Asp Ala Gly Leu Lys Glu Ser Pro Leu
        35                  40                  45
Gln Thr Pro Thr Glu Asp Gly Ser Glu Glu Pro Gly Ser Glu Thr Ser
    50                  55                  60
Asp Ala Lys Ser Thr Pro Thr Ala Glu Asp Val Thr Ala Pro Leu Val
65                  70                  75                  80
Asp Glu Gly Ala Pro Gly Lys Gln Ala Ala Ala Gln Pro His Thr Glu
                85                  90                  95
Ile Pro Glu Gly Thr Thr Ala Glu Glu Ala Gly Ile Gly Asp Thr Pro
            100                 105                 110
Ser Leu Glu Asp Glu Ala Ala Gly His Val Thr Gln Ala Arg Met Val
        115                 120                 125
Ser Lys Ser Lys Asp Gly Thr Gly Ser Asp Asp Lys Lys Ala Lys Gly
    130                 135                 140
Ala Asp Gly Lys Thr Lys Ile Ala Thr Pro Arg Gly Ala Ala Pro Pro
145                 150                 155                 160
Gly Gln Lys Gly Gln Ala Asn Ala Thr Arg Ile Pro Ala Lys Thr Pro
                165                 170                 175
Pro Ala Pro Lys Thr Pro Pro Ser Ser Gly Glu Pro Pro Lys Ser Gly
            180                 185                 190
Asp Arg Ser Gly Tyr Ser Ser Pro Gly Ser Pro Gly Thr Pro Gly Ser
        195                 200                 205
Arg Ser Arg Thr Pro Ser Leu Pro Thr Pro Pro Thr Arg Glu Pro Lys
    210                 215                 220
Lys Val Ala Val Val Arg Thr Pro Pro Lys Ser Pro Ser Ser Ala Lys
225                 230                 235                 240
Ser Arg Leu Gln Thr Ala Pro Val Pro Met Pro Asp Leu Lys Asn Val
                245                 250                 255
Lys Ser Lys Ile Gly Ser Thr Glu Asn Leu Lys His Gln Pro Gly Gly
            260                 265                 270
Gly Lys Val Gln Ile Ile Asn Lys Lys Leu Asp Leu Ser Asn Val Gln
        275                 280                 285
Ser Lys Cys Gly Ser Lys Asp Asn Ile Lys His Val Pro Gly Gly Gly
    290                 295                 300
Ser Val Gln Ile Val Tyr Lys Pro Val Asp Leu Ser Lys Val Thr Ser
305                 310                 315                 320
Lys Cys Gly Ser Leu Gly Asn Ile His His Lys Pro Gly Gly Gly Gln
                325                 330                 335
Val Glu Val Lys Ser Glu Lys Leu Asp Phe Lys Asp Arg Val Gln Ser
            340                 345                 350
Lys Ile Gly Ser Leu Asp Asn Ile Thr His Val Pro Gly Gly Gly Asn
        355                 360                 365
Lys Lys Ile Glu Thr His Lys Leu Thr Phe Arg Glu Asn Ala Lys Ala
    370                 375                 380
Lys Thr Asp His Gly Ala Glu Ile Val Tyr Lys Ser Pro Val Val Ser
385                 390                 395                 400
```

Gly Asp Thr Ser Pro Arg His Leu Ser Asn Val Ser Ser Thr Gly Ser
405 410 415
Ile Asp Met Val Asp Ser Pro Gln Leu Ala Thr Leu Ala Asp Glu Val
420 425 430
Ser Ala Ser Leu Ala Lys Gln Gly Leu
435 440

<210> 498
<211> 476
<212> PRT
<213> Homo sapiens
<400> 498
Met Met Thr Ala Lys Ala Val Asp Lys Ile Pro Val Thr Leu Ser Gly
1 5 10 15
Phe Val His Gln Leu Ser Asp Asn Ile Tyr Pro Val Glu Asp Leu Ala
20 25 30
Ala Thr Ser Val Thr Ile Phe Pro Asn Ala Glu Leu Gly Gly Pro Phe
35 40 45
Asp Gln Met Asn Gly Val Ala Gly Asp Gly Met Ile Asn Ile Asp Met
50 55 60
Thr Gly Glu Lys Arg Ser Leu Asp Leu Pro Tyr Pro Ser Ser Phe Ala
65 70 75 80
Pro Val Ser Ala Pro Arg Asn Gln Thr Phe Thr Tyr Met Gly Lys Phe
85 90 95
Ser Ile Asp Pro Gln Tyr Pro Gly Ala Ser Cys Tyr Pro Glu Gly Ile
100 105 110
Ile Asn Ile Val Ser Ala Gly Ile Leu Gln Gly Val Thr Ser Pro Ala
115 120 125
Ser Thr Thr Ala Ser Ser Ser Val Thr Ser Ala Ser Pro Asn Pro Leu
130 135 140
Ala Thr Gly Pro Leu Gly Val Cys Thr Met Ser Gln Thr Gln Pro Asp
145 150 155 160
Leu Asp His Leu Tyr Ser Pro Pro Pro Pro Pro Pro Pro Tyr Ser Gly
165 170 175
Cys Ala Gly Asp Leu Tyr Gln Asp Pro Ser Ala Phe Leu Ser Ala Ala
180 185 190
Thr Thr Ser Thr Ser Ser Ser Leu Ala Tyr Pro Pro Pro Pro Ser Tyr
195 200 205
Pro Ser Pro Lys Pro Ala Thr Asp Pro Gly Leu Phe Pro Met Ile Pro
210 215 220
Asp Tyr Pro Gly Phe Phe Pro Ser Gln Cys Gln Arg Asp Leu His Gly
225 230 235 240
Thr Ala Gly Pro Asp Arg Lys Pro Phe Pro Cys Pro Leu Asp Thr Leu
245 250 255
Arg Val Pro Pro Pro Leu Thr Pro Leu Ser Thr Ile Arg Asn Phe Thr
260 265 270
Leu Gly Gly Pro Ser Ala Gly Val Thr Gly Pro Gly Ala Ser Gly Gly
275 280 285
Ser Glu Gly Pro Arg Leu Pro Gly Ser Ser Ser Ala Ala Ala Ala Ala
290 295 300
Ala Ala Ala Ala Ala Tyr Asn Pro His His Leu Pro Leu Arg Pro Ile
305 310 315 320
Leu Arg Pro Arg Lys Tyr Pro Asn Arg Pro Ser Lys Thr Pro Val His
325 330 335
Glu Arg Pro Tyr Pro Cys Pro Ala Glu Gly Cys Asp Arg Arg Phe Ser
340 345 350
Arg Ser Asp Glu Leu Thr Arg His Ile Arg Ile His Thr Gly His Lys
355 360 365
Pro Phe Gln Cys Arg Ile Cys Met Arg Asn Phe Ser Arg Ser Asp His
370 375 380
Leu Thr Thr His Ile Arg Thr His Thr Gly Glu Lys Pro Phe Ala Cys
385 390 395 400
Asp Tyr Cys Gly Arg Lys Phe Ala Arg Ser Asp Glu Arg Lys Arg His
405 410 415
Thr Lys Ile His Leu Arg Gln Lys Glu Arg Lys Ser Ser Ala Pro Ser
420 425 430
Ala Ser Val Pro Ala Pro Ser Thr Ala Ser Cys Ser Gly Gly Val Gln
435 440 445
Pro Gly Gly Thr Leu Cys Ser Ser Asn Ser Ser Ser Leu Gly Gly Gly

450 455 460
Pro Leu Ala Pro Cys Ser Ser Arg Thr Arg Thr Pro
465 470 475

<210> 499
<211> 406
<212> PRT
<213> Homo sapiens
<400> 499
Met Ser Gly Cys Pro Phe Leu Gly Asn Asn Phe Gly Tyr Thr Phe Lys
1 5 10 15
Lys Leu Pro Val Glu Gly Ser Glu Glu Asp Lys Ser Gln Thr Gly Val
20 25 30
Asn Arg Ala Ser Lys Gly Gly Leu Ile Tyr Gly Asn Tyr Leu His Leu
35 40 45
Glu Lys Val Leu Asn Ala Gln Glu Leu Gln Ser Glu Thr Lys Gly Asn
50 55 60
Lys Ile His Asp Glu His Leu Phe Ile Ile Thr His Gln Ala Tyr Glu
65 70 75 80
Leu Trp Phe Lys Gln Ile Leu Trp Glu Leu Asp Ser Val Arg Glu Ile
85 90 95
Phe Gln Asn Gly His Val Arg Asp Glu Arg Asn Met Leu Lys Val Val
100 105 110
Ser Arg Met His Arg Val Ser Val Ile Leu Lys Leu Leu Val Gln Gln
115 120 125
Phe Ser Ile Leu Glu Thr Met Thr Ala Leu Asp Phe Asn Asp Phe Arg
130 135 140
Glu Tyr Leu Ser Pro Ala Ser Gly Phe Gln Ser Leu Gln Phe Arg Leu
145 150 155 160
Leu Glu Asn Lys Ile Gly Val Leu Gln Asn Met Arg Val Pro Tyr Asn
165 170 175
Arg Arg His Tyr Arg Asp Asn Phe Lys Gly Glu Glu Asn Glu Leu Leu
180 185 190
Leu Lys Ser Glu Gln Glu Lys Thr Leu Leu Glu Leu Val Glu Ala Trp
195 200 205
Leu Glu Arg Thr Pro Gly Leu Glu Pro His Gly Phe Asn Phe Trp Gly
210 215 220
Lys Leu Glu Lys Asn Ile Thr Arg Gly Leu Glu Glu Glu Phe Ile Arg
225 230 235 240
Ile Gln Ala Lys Glu Glu Ser Glu Glu Lys Glu Glu Gln Val Ala Glu
245 250 255
Phe Gln Lys Gln Lys Glu Val Leu Leu Ser Leu Phe Asp Glu Lys Arg
260 265 270
His Glu His Leu Leu Ser Lys Gly Glu Arg Arg Leu Ser Tyr Arg Ala
275 280 285
Leu Gln Gly Ala Leu Met Ile Tyr Phe Tyr Arg Glu Glu Pro Arg Phe
290 295 300
Gln Val Pro Phe Gln Leu Leu Thr Ser Leu Met Asp Ile Asp Ser Leu
305 310 315 320
Met Thr Lys Trp Arg Tyr Asn His Val Cys Met Val His Arg Met Leu
325 330 335
Gly Ser Lys Ala Gly Thr Gly Gly Ser Ser Gly Tyr His Tyr Leu Arg
340 345 350
Ser Thr Val Ser Asp Arg Tyr Lys Val Phe Val Asp Leu Phe Asn Leu
355 360 365
Ser Thr Tyr Leu Ile Pro Arg His Trp Ile Pro Lys Met Asn Pro Thr
370 375 380
Ile His Lys Phe Leu Tyr Thr Ala Glu Tyr Cys Asp Ser Ser Tyr Phe
385 390 395 400
Ser Ser Asp Glu Ser Asp
405

<210> 500
<211> 470
<212> PRT
<213> Homo sapiens
<400> 500
Met Leu Arg Gly Ile Ser Gln Leu Pro Ala Val Ala Thr Met Ser Trp
1 5 10 15

Val Leu Leu Pro Val Leu Trp Leu Ile Val Gln Thr Gln Ala Ile Ala
20 25 30
Ile Lys Gln Thr Pro Glu Leu Thr Leu His Glu Ile Val Cys Pro Lys
35 40 45
Lys Leu His Ile Leu His Lys Arg Glu Ile Lys Asn Asn Gln Thr Glu
50 55 60
Lys His Gly Lys Glu Glu Arg Tyr Glu Pro Glu Val Gln Tyr Gln Met
65 70 75 80
Ile Leu Asn Gly Glu Glu Ile Ile Leu Ser Leu Gln Lys Thr Lys His
85 90 95
Leu Leu Gly Pro Asp Tyr Thr Glu Thr Leu Tyr Ser Pro Arg Gly Glu
100 105 110
Glu Ile Thr Thr Lys Pro Glu Asn Met Glu His Cys Tyr Tyr Lys Gly
115 120 125
Asn Ile Leu Asn Glu Lys Asn Ser Val Ala Ser Ile Ser Thr Cys Asp
130 135 140
Gly Leu Arg Gly Tyr Phe Thr His His His Gln Arg Tyr Gln Ile Lys
145 150 155 160
Pro Leu Lys Ser Thr Asp Glu Lys Glu His Ala Val Phe Thr Ser Asn
165 170 175
Gln Glu Glu Gln Asp Pro Ala Asn His Thr Cys Gly Val Lys Ser Thr
180 185 190
Asp Gly Lys Gln Gly Pro Ile Arg Ile Ser Arg Ser Leu Lys Ser Pro
195 200 205
Glu Lys Glu Asp Phe Leu Arg Ala Gln Lys Tyr Ile Asp Leu Tyr Leu
210 215 220
Val Leu Asp Asn Ala Phe Tyr Lys Asn Tyr Asn Glu Asn Leu Thr Leu
225 230 235 240
Ile Arg Ser Phe Val Phe Asp Val Met Asn Leu Leu Asn Val Ile Tyr
245 250 255
Asn Thr Ile Asp Val Gln Val Ala Leu Val Gly Met Glu Ile Trp Ser
260 265 270
Asp Gly Asp Lys Ile Lys Val Val Pro Ser Ala Ser Thr Thr Phe Asp
275 280 285
Asn Phe Leu Arg Trp His Ser Ser Asn Leu Gly Lys Lys Ile His Asp
290 295 300
His Ala Gln Leu Leu Ser Gly Ile Ser Phe Asn Asn Arg Arg Val Gly
305 310 315 320
Leu Ala Ala Ser Asn Ser Leu Cys Ser Pro Ser Ser Val Ala Val Ile
325 330 335
Glu Ala Lys Lys Lys Asn Asn Val Ala Leu Val Gly Val Met Ser His
340 345 350
Glu Leu Gly His Val Leu Gly Met Pro Asp Val Pro Phe Asn Thr Lys
355 360 365
Cys Pro Ser Gly Ser Cys Val Met Asn Gln Tyr Leu Ser Ser Lys Phe
370 375 380
Pro Lys Asp Phe Ser Thr Ser Cys Arg Ala His Phe Glu Arg Tyr Leu
385 390 395 400
Leu Ser Gln Lys Pro Lys Cys Leu Leu Gln Ala Pro Ile Pro Thr Asn
405 410 415
Ile Met Thr Thr Pro Val Cys Gly Asn His Leu Leu Glu Val Gly Glu
420 425 430
Asp Cys Asp Cys Gly Ser Pro Lys Glu Cys Thr Asn Leu Cys Cys Glu
435 440 445
Ala Leu Thr Cys Lys Leu Lys Pro Gly Thr Asp Cys Gly Gly Asp Ala
450 455 460
Pro Asn His Thr Thr Glu
465 470

<210> 501
<211> 347
<212> PRT
<213> Homo sapiens
<400> 501
Met Thr Leu Asp His Gln Ile Ile Asn Pro Thr Leu Lys Trp Ser Gln
1 5 10 15
Pro Ala Val Pro Ser Gly Gly Pro Leu Val Gln His Ala His Thr Thr
20 25 30
Leu Asp Ser Asp Ala Gly Leu Thr Glu Asn Pro Leu Thr Lys Leu Leu

```
        35                  40                  45
Ala Ile Gly Lys Glu Asp Asp Asn Ala Gln Trp His Met Glu Asp Val
    50                  55                  60
Ile Glu Asp Ile Ile Gly Met Glu Ser Ser Phe Lys Glu Glu Gly Ala
65                  70                  75                  80
Asp Ser Pro Leu Leu Met Gln Arg Thr Leu Ser Gly Ser Ile Leu Asp
                85                  90                  95
Val Tyr Ser Gly Glu Gln Gly Ile Ser Pro Ile Asn Met Gly Leu Thr
            100                 105                 110
Ser Ala Ser Cys Pro Ser Ser Leu Pro Met Lys Arg Glu Ile Thr Glu
        115                 120                 125
Thr Asp Thr Arg Ala Leu Ala Lys Glu Arg Gln Lys Lys Asp Asn His
    130                 135                 140
Asn Leu Ile Glu Arg Arg Arg Arg Tyr Asn Ile Asn Tyr Arg Ile Lys
145                 150                 155                 160
Glu Leu Gly Thr Leu Ile Pro Lys Ser Asn Asp Pro Asp Met Arg Trp
                165                 170                 175
Asn Lys Gly Thr Ile Leu Lys Ala Ser Val Glu Tyr Ile Lys Trp Leu
            180                 185                 190
Gln Lys Glu Gln Gln Arg Ala Arg Glu Leu Glu His Arg Gln Lys Lys
        195                 200                 205
Leu Glu Gln Ala Asn Arg Arg Leu Leu Leu Arg Ile Gln Glu Leu Glu
    210                 215                 220
Ile Gln Ala Arg Thr His Gly Leu Pro Thr Leu Ala Ser Leu Gly Thr
225                 230                 235                 240
Val Asp Leu Gly Ala His Val Thr Lys Gln Gln Ser His Pro Glu Gln
                245                 250                 255
Asn Ser Val Asp Tyr Cys Gln Gln Leu Thr Val Ser Gln Gly Pro Ser
            260                 265                 270
Pro Glu Leu Cys Asp Gln Ala Ile Ala Phe Ser Asp Pro Leu Ser Tyr
        275                 280                 285
Phe Thr Asp Leu Ser Phe Ser Ala Ala Leu Lys Glu Glu Gln Arg Leu
    290                 295                 300
Asp Gly Met Leu Leu Asp Asp Thr Ile Ser Pro Phe Gly Thr Asp Pro
305                 310                 315                 320
Leu Leu Ser Ala Thr Ser Pro Ala Val Ser Lys Glu Ser Ser Arg Arg
                325                 330                 335
Ser Ser Phe Ser Ser Asp Asp Gly Asp Glu Leu
            340                 345
```

<210> 502
<211> 162
<212> PRT
<213> Homo sapiens
<400> 502

```
Met Lys Glu Thr Ile Met Asn Gln Glu Lys Leu Ala Lys Leu Gln Ala
1               5                   10                  15
Gln Val Arg Ile Gly Gly Lys Gly Thr Ala Arg Arg Lys Lys Lys Val
            20                  25                  30
Val His Arg Thr Ala Thr Ala Asp Asp Lys Lys Leu Gln Phe Ser Leu
        35                  40                  45
Lys Lys Leu Gly Val Asn Asn Ile Ser Gly Ile Glu Glu Val Asn Met
    50                  55                  60
Phe Thr Asn Gln Gly Thr Val Ile His Phe Asn Asn Pro Lys Val Gln
65                  70                  75                  80
Ala Ser Leu Ala Ala Asn Thr Phe Thr Ile Thr Gly His Ala Glu Thr
                85                  90                  95
Lys Gln Leu Thr Glu Met Leu Pro Ser Ile Leu Asn Gln Leu Gly Ala
            100                 105                 110
Asp Ser Leu Thr Ser Leu Arg Arg Leu Ala Glu Ala Leu Pro Lys Gln
        115                 120                 125
Ser Val Asp Gly Lys Ala Pro Leu Ala Thr Gly Glu Asp Asp Asp Asp
    130                 135                 140
Glu Val Pro Asp Leu Val Glu Asn Phe Asp Glu Ala Ser Lys Asn Glu
145                 150                 155                 160
Ala Asn
```

<210> 503

<211> 2602
<212> PRT
<213> Homo sapiens
<400> 503

```
Met Pro Val Thr Glu Lys Asp Leu Ala Glu Asp Ala Pro Trp Lys Lys
1               5                   10                  15
Ile Gln Gln Asn Thr Phe Thr Arg Trp Cys Asn Glu His Leu Lys Cys
            20                  25                  30
Val Asn Lys Arg Ile Gly Asn Leu Gln Thr Asp Leu Ser Asp Gly Leu
        35                  40                  45
Arg Leu Ile Ala Leu Leu Glu Val Leu Ser Gln Lys Arg Met Tyr Arg
    50                  55                  60
Lys Tyr His Gln Arg Pro Thr Phe Arg Gln Met Gln Leu Glu Asn Val
65                  70                  75                  80
Ser Val Ala Leu Glu Phe Leu Asp Arg Glu Ser Ile Lys Leu Val Ser
                85                  90                  95
Ile Asp Ser Lys Ala Ile Val Asp Gly Asn Leu Lys Leu Ile Leu Gly
            100                 105                 110
Leu Val Trp Thr Leu Ile Leu His Tyr Ser Ile Ser Met Pro Val Trp
        115                 120                 125
Glu Asp Glu Gly Asp Asp Asp Ala Lys Lys Gln Thr Pro Lys Gln Arg
    130                 135                 140
Leu Leu Gly Trp Ile Gln Asn Lys Ile Pro Tyr Leu Pro Ile Thr Asn
145                 150                 155                 160
Phe Asn Gln Asn Trp Gln Asp Gly Lys Ala Leu Gly Ala Leu Val Asp
                165                 170                 175
Ser Cys Ala Pro Gly Leu Cys Pro Asp Trp Glu Ser Trp Asp Pro Gln
            180                 185                 190
Lys Pro Val Asp Asn Ala Arg Glu Ala Met Gln Gln Ala Asp Asp Trp
        195                 200                 205
Leu Gly Val Pro Gln Val Ile Thr Pro Glu Glu Ile Ile His Pro Asp
    210                 215                 220
Val Asp Glu His Ser Val Met Thr Tyr Leu Ser Gln Phe Pro Lys Ala
225                 230                 235                 240
Lys Leu Lys Pro Gly Ala Pro Leu Lys Pro Lys Leu Asn Pro Lys Lys
                245                 250                 255
Ala Arg Ala Tyr Gly Arg Gly Ile Glu Pro Thr Gly Asn Met Val Lys
            260                 265                 270
Gln Pro Ala Lys Phe Thr Val Asp Thr Ile Ser Ala Gly Gln Gly Asp
        275                 280                 285
Val Met Val Phe Val Glu Asp Pro Glu Gly Asn Lys Glu Glu Ala Gln
    290                 295                 300
Val Thr Pro Asp Ser Asp Lys Asn Lys Thr Tyr Ser Val Glu Tyr Leu
305                 310                 315                 320
Pro Lys Val Thr Gly Leu His Lys Val Thr Val Leu Phe Ala Gly Gln
                325                 330                 335
His Ile Ser Lys Ser Pro Phe Glu Val Ser Val Asp Lys Ala Gln Gly
            340                 345                 350
Asp Ala Ser Lys Val Thr Ala Lys Gly Pro Gly Leu Glu Ala Val Gly
        355                 360                 365
Asn Ile Ala Asn Lys Pro Thr Tyr Phe Asp Ile Tyr Thr Ala Gly Ala
    370                 375                 380
Gly Val Gly Asp Ile Gly Val Glu Val Glu Asp Pro Gln Gly Lys Asn
385                 390                 395                 400
Thr Val Glu Leu Leu Val Glu Asp Lys Gly Asn Gln Val Tyr Arg Cys
                405                 410                 415
Val Tyr Lys Pro Met Gln Pro Gly Pro His Val Val Lys Ile Phe Phe
            420                 425                 430
Ala Gly Asp Thr Ile Pro Lys Ser Pro Phe Val Val Gln Val Gly Glu
        435                 440                 445
Ala Cys Asn Pro Asn Ala Cys Arg Ala Ser Gly Arg Gly Leu Gln Pro
    450                 455                 460
Lys Gly Val Arg Ile Arg Glu Thr Thr Asp Phe Lys Val Asp Thr Lys
465                 470                 475                 480
Ala Ala Gly Ser Gly Glu Leu Gly Val Thr Met Lys Gly Pro Lys Gly
                485                 490                 495
Leu Glu Glu Leu Val Lys Gln Lys Asp Phe Leu Asp Gly Val Tyr Ala
            500                 505                 510
Phe Glu Tyr Tyr Pro Ser Thr Pro Gly Arg Tyr Ser Ile Ala Ile Thr
```

```
                515                     520                     525
Trp Gly Gly His His Ile Pro Lys Ser Pro Phe Glu Val Gln Val Gly
    530                     535                     540
Pro Glu Ala Gly Met Gln Lys Val Arg Ala Trp Gly Pro Gly Leu His
545                     550                     555                     560
Gly Gly Ile Val Gly Arg Ser Ala Asp Phe Val Val Glu Ser Ile Gly
                565                     570                     575
Ser Glu Val Gly Ser Leu Gly Phe Ala Ile Glu Gly Pro Ser Gln Ala
            580                     585                     590
Lys Ile Glu Tyr Asn Asp Gln Asn Asp Gly Ser Cys Asp Val Lys Tyr
        595                     600                     605
Trp Pro Lys Glu Pro Gly Glu Tyr Ala Val His Ile Met Cys Asp Asp
    610                     615                     620
Glu Asp Ile Lys Asp Ser Pro Tyr Met Ala Phe Ile His Pro Ala Thr
625                     630                     635                     640
Gly Gly Tyr Asn Pro Asp Leu Val Arg Ala Tyr Gly Pro Gly Leu Glu
                645                     650                     655
Lys Ser Gly Cys Ile Val Asn Asn Leu Ala Glu Phe Thr Val Asp Pro
            660                     665                     670
Lys Asp Ala Gly Lys Ala Pro Leu Lys Ile Phe Ala Gln Asp Gly Glu
        675                     680                     685
Gly Gln Arg Ile Asp Ile Gln Met Lys Asn Arg Met Asp Gly Thr Tyr
    690                     695                     700
Ala Cys Ser Tyr Thr Pro Val Lys Ala Ile Lys His Thr Ile Ala Val
705                     710                     715                     720
Val Trp Gly Gly Val Asn Ile Pro His Ser Pro Tyr Arg Val Asn Ile
                725                     730                     735
Gly Gln Gly Ser His Pro Gln Lys Val Lys Val Phe Gly Pro Gly Val
            740                     745                     750
Glu Arg Ser Gly Leu Lys Ala Asn Glu Pro Thr His Phe Thr Val Asp
        755                     760                     765
Cys Thr Glu Ala Gly Glu Gly Asp Val Ser Val Gly Ile Lys Cys Asp
    770                     775                     780
Ala Arg Val Leu Ser Glu Asp Glu Glu Asp Val Asp Phe Asp Ile Ile
785                     790                     795                     800
His Asn Ala Asn Asp Thr Phe Thr Val Lys Tyr Val Pro Pro Ala Ala
                805                     810                     815
Gly Arg Tyr Thr Ile Lys Val Leu Phe Ala Ser Gln Glu Ile Pro Ala
            820                     825                     830
Ser Pro Phe Arg Val Lys Val Asp Pro Ser His Asp Ala Ser Lys Val
        835                     840                     845
Lys Ala Glu Gly Pro Gly Leu Ser Lys Ala Gly Val Glu Asn Gly Lys
    850                     855                     860
Pro Thr His Phe Thr Val Tyr Thr Lys Gly Ala Gly Lys Ala Pro Leu
865                     870                     875                     880
Asn Val Gln Phe Asn Ser Pro Leu Pro Gly Asp Ala Val Lys Asp Leu
                885                     890                     895
Asp Ile Ile Asp Asn Tyr Asp Tyr Ser His Thr Val Lys Tyr Thr Pro
            900                     905                     910
Thr Gln Gln Gly Asn Met Gln Val Leu Val Thr Tyr Gly Gly Asp Pro
        915                     920                     925
Ile Pro Lys Ser Pro Phe Thr Val Gly Val Ala Ala Pro Leu Asp Leu
    930                     935                     940
Ser Lys Ile Lys Leu Asn Gly Leu Glu Asn Arg Val Glu Val Gly Lys
945                     950                     955                     960
Asp Gln Glu Phe Thr Val Asp Thr Arg Gly Ala Gly Gly Gln Gly Lys
                965                     970                     975
Leu Asp Val Thr Ile Leu Ser Pro Ser Arg Lys Val Val Pro Cys Leu
            980                     985                     990
Val Thr Pro Val Thr Gly Arg Glu  Asn Ser Thr Ala Lys  Phe Ile Pro
        995                     1000                    1005
Arg Glu  Glu Gly Leu Tyr Ala  Val Asp Val Thr Tyr  Asp Gly His
    1010                    1015                    1020
Pro Val  Pro Gly Ser Pro Tyr  Thr Val Glu Ala Ser  Leu Pro Pro
    1025                    1030                    1035
Asp Pro  Ser Lys Val Lys Ala  His Gly Pro Gly Leu  Glu Gly Gly
    1040                    1045                    1050
Leu Val  Gly Lys Pro Ala Glu  Phe Thr Ile Asp Thr  Lys Gly Ala
    1055                    1060                    1065
```

```
Gly Thr Gly Gly Leu Gly Leu Thr Val Glu Gly Pro Cys Glu Ala
    1070                1075                1080
Lys Ile Glu Cys Ser Asp Asn Gly Asp Gly Thr Cys Ser Val Ser
    1085                1090                1095
Tyr Leu Pro Thr Lys Pro Gly Glu Tyr Phe Val Asn Ile Leu Phe
    1100                1105                1110
Glu Glu Val His Ile Pro Gly Ser Pro Phe Lys Ala Asp Ile Glu
    1115                1120                1125
Met Pro Phe Asp Pro Ser Lys Val Val Ala Ser Gly Pro Gly Leu
    1130                1135                1140
Glu His Gly Lys Val Gly Glu Ala Gly Leu Leu Ser Val Asn Cys
    1145                1150                1155
Ser Glu Ala Gly Pro Gly Ala Leu Gly Leu Glu Ala Val Ser Asp
    1160                1165                1170
Ser Gly Thr Lys Ala Glu Val Ser Ile Gln Asn Asn Lys Asp Gly
    1175                1180                1185
Thr Tyr Ala Val Thr Tyr Val Pro Leu Thr Ala Gly Met Tyr Thr
    1190                1195                1200
Leu Thr Met Lys Tyr Gly Gly Glu Leu Val Pro His Phe Pro Ala
    1205                1210                1215
Arg Val Lys Val Glu Pro Ala Val Asp Thr Ser Arg Ile Lys Val
    1220                1225                1230
Phe Gly Pro Gly Ile Glu Gly Lys Asp Val Phe Arg Glu Ala Thr
    1235                1240                1245
Thr Asp Phe Thr Val Asp Ser Arg Pro Leu Thr Gln Val Gly Gly
    1250                1255                1260
Asp His Ile Lys Ala His Ile Ala Asn Pro Ser Gly Ala Ser Thr
    1265                1270                1275
Glu Cys Phe Val Thr Asp Asn Ala Asp Gly Thr Tyr Gln Val Glu
    1280                1285                1290
Tyr Thr Pro Phe Glu Lys Gly Leu His Val Val Glu Val Thr Tyr
    1295                1300                1305
Asp Asp Val Pro Ile Pro Asn Ser Pro Phe Lys Val Ala Val Thr
    1310                1315                1320
Glu Gly Cys Gln Pro Ser Arg Val Gln Ala Gln Gly Pro Gly Leu
    1325                1330                1335
Lys Glu Ala Phe Thr Asn Lys Pro Asn Val Phe Thr Val Val Thr
    1340                1345                1350
Arg Gly Ala Gly Ile Gly Gly Leu Gly Ile Thr Val Glu Gly Pro
    1355                1360                1365
Ser Glu Ser Lys Ile Asn Cys Arg Asp Asn Lys Asp Gly Ser Cys
    1370                1375                1380
Ser Ala Glu Tyr Ile Pro Phe Ala Pro Gly Asp Tyr Asp Val Asn
    1385                1390                1395
Ile Thr Tyr Gly Gly Ala His Ile Pro Gly Ser Pro Phe Arg Val
    1400                1405                1410
Pro Val Lys Asp Val Val Asp Pro Ser Lys Val Lys Ile Ala Gly
    1415                1420                1425
Pro Gly Leu Gly Ser Gly Val Arg Ala Arg Val Leu Gln Ser Phe
    1430                1435                1440
Thr Val Asp Ser Ser Lys Ala Gly Leu Ala Pro Leu Glu Val Arg
    1445                1450                1455
Val Leu Gly Pro Arg Gly Leu Val Glu Pro Val Asn Met Val Asp
    1460                1465                1470
Asn Gly Asp Gly Thr His Thr Val Thr Tyr Thr Pro Ser Gln Glu
    1475                1480                1485
Gly Pro Tyr Met Val Ser Val Lys Tyr Ala Asp Glu Glu Ile Pro
    1490                1495                1500
Arg Ser Pro Phe Lys Val Lys Val Leu Pro Thr Tyr Asp Ala Ser
    1505                1510                1515
Lys Val Thr Ala Ser Gly Pro Gly Leu Ser Ser Tyr Gly Val Pro
    1520                1525                1530
Ala Ser Leu Pro Val Asp Phe Ala Ile Asp Ala Arg Asp Ala Gly
    1535                1540                1545
Glu Gly Leu Leu Ala Val Gln Ile Thr Asp Gln Glu Gly Lys Pro
    1550                1555                1560
Lys Arg Ala Ile Val His Asp Asn Lys Asp Gly Thr Tyr Ala Val
    1565                1570                1575
Thr Tyr Ile Pro Asp Lys Thr Gly Arg Tyr Met Ile Gly Val Thr
```

1580 1585 1590
Tyr Gly Gly Asp Asp Ile Pro Leu Ser Pro Tyr Arg Ile Arg Ala
1595 1600 1605
Thr Gln Thr Gly Asp Ala Ser Lys Cys Leu Ala Thr Gly Pro Gly
1610 1615 1620
Ile Ala Ser Thr Val Lys Thr Gly Glu Glu Val Gly Phe Val Val
1625 1630 1635
Asp Ala Lys Thr Ala Gly Lys Gly Lys Val Thr Cys Thr Val Leu
1640 1645 1650
Thr Pro Asp Gly Thr Glu Ala Glu Ala Asp Val Ile Glu Asn Glu
1655 1660 1665
Asp Gly Thr Tyr Asp Ile Phe Tyr Thr Ala Ala Lys Pro Gly Thr
1670 1675 1680
Tyr Val Ile Tyr Val Arg Phe Gly Gly Val Asp Ile Pro Asn Ser
1685 1690 1695
Pro Phe Thr Val Met Ala Thr Asp Gly Glu Val Thr Ala Val Glu
1700 1705 1710
Glu Ala Pro Val Asn Ala Cys Pro Pro Gly Phe Arg Pro Trp Val
1715 1720 1725
Thr Glu Glu Ala Tyr Val Pro Val Ser Asp Met Asn Gly Leu Gly
1730 1735 1740
Phe Lys Pro Phe Asp Leu Val Ile Pro Phe Ala Val Arg Lys Gly
1745 1750 1755
Glu Ile Thr Gly Glu Val His Met Pro Ser Gly Lys Thr Ala Thr
1760 1765 1770
Pro Glu Ile Val Asp Asn Lys Asp Gly Thr Val Thr Val Arg Tyr
1775 1780 1785
Ala Pro Thr Glu Val Gly Leu His Glu Met His Ile Lys Tyr Met
1790 1795 1800
Gly Ser His Ile Pro Glu Ser Pro Leu Gln Phe Tyr Val Asn Tyr
1805 1810 1815
Pro Asn Ser Gly Ser Val Ser Ala Tyr Gly Pro Gly Leu Val Tyr
1820 1825 1830
Gly Val Ala Asn Lys Thr Ala Thr Phe Thr Ile Val Thr Glu Asp
1835 1840 1845
Ala Gly Glu Gly Gly Leu Asp Leu Ala Ile Glu Gly Pro Ser Lys
1850 1855 1860
Ala Glu Ile Ser Cys Ile Asp Asn Lys Asp Gly Thr Cys Thr Val
1865 1870 1875
Thr Tyr Leu Pro Thr Leu Pro Gly Asp Tyr Ser Ile Leu Val Lys
1880 1885 1890
Tyr Asn Asp Lys His Ile Pro Gly Ser Pro Phe Thr Ala Lys Ile
1895 1900 1905
Thr Asp Asp Ser Arg Arg Cys Ser Gln Val Lys Leu Gly Ser Ala
1910 1915 1920
Ala Asp Phe Leu Leu Asp Ile Ser Glu Thr Asp Leu Ser Ser Leu
1925 1930 1935
Thr Ala Ser Ile Lys Ala Pro Ser Gly Arg Asp Glu Pro Cys Leu
1940 1945 1950
Leu Lys Arg Leu Pro Asn Asn His Ile Gly Ile Ser Phe Ile Pro
1955 1960 1965
Arg Glu Val Gly Glu His Leu Val Ser Ile Lys Lys Asn Gly Asn
1970 1975 1980
His Val Ala Asn Ser Pro Val Ser Ile Met Val Val Gln Ser Glu
1985 1990 1995
Ile Gly Asp Ala Arg Arg Ala Lys Val Tyr Gly Arg Gly Leu Ser
2000 2005 2010
Glu Gly Arg Thr Phe Glu Met Ser Asp Phe Ile Val Asp Thr Arg
2015 2020 2025
Asp Ala Gly Tyr Gly Gly Ile Ser Leu Ala Val Glu Gly Pro Ser
2030 2035 2040
Lys Val Asp Ile Gln Thr Glu Asp Leu Glu Asp Gly Thr Cys Lys
2045 2050 2055
Val Ser Tyr Phe Pro Thr Val Pro Gly Val Tyr Ile Val Ser Thr
2060 2065 2070
Lys Phe Ala Asp Glu His Val Pro Gly Ser Pro Phe Thr Val Lys
2075 2080 2085
Ile Ser Gly Glu Gly Arg Val Lys Glu Ser Ile Thr Arg Thr Ser
2090 2095 2100

Arg Ala Pro Ser Val Ala Thr Val Gly Ser Ile Cys Asp Leu Asn
2105 2110 2115
Leu Lys Ile Pro Glu Ile Asn Ser Ser Asp Met Ser Ala His Val
2120 2125 2130
Thr Ser Pro Ser Gly Arg Val Thr Glu Ala Glu Ile Val Pro Met
2135 2140 2145
Gly Lys Asn Ser His Cys Val Arg Phe Val Pro Gln Glu Met Gly
2150 2155 2160
Val His Thr Val Ser Val Lys Tyr Arg Gly Gln His Val Thr Gly
2165 2170 2175
Ser Pro Phe Gln Phe Thr Val Gly Pro Leu Gly Glu Gly Gly Ala
2180 2185 2190
His Lys Val Arg Ala Gly Gly Pro Gly Leu Glu Arg Gly Glu Ala
2195 2200 2205
Gly Val Pro Ala Glu Phe Ser Ile Trp Thr Arg Glu Ala Gly Ala
2210 2215 2220
Gly Gly Leu Ser Ile Ala Val Glu Gly Pro Ser Lys Ala Glu Ile
2225 2230 2235
Thr Phe Asp Asp His Lys Asn Gly Ser Cys Gly Val Ser Tyr Ile
2240 2245 2250
Ala Gln Glu Pro Gly Asn Tyr Glu Val Ser Ile Lys Phe Asn Asp
2255 2260 2265
Glu His Ile Pro Glu Ser Pro Tyr Leu Val Pro Val Ile Ala Pro
2270 2275 2280
Ser Asp Asp Ala Arg Arg Leu Thr Val Met Ser Leu Gln Glu Ser
2285 2290 2295
Gly Leu Lys Val Asn Gln Pro Ala Ser Phe Ala Ile Arg Leu Asn
2300 2305 2310
Gly Ala Lys Gly Lys Ile Asp Ala Lys Val His Ser Pro Ser Gly
2315 2320 2325
Ala Val Glu Glu Cys His Val Ser Glu Leu Glu Pro Asp Lys Tyr
2330 2335 2340
Ala Val Arg Phe Ile Pro His Glu Asn Gly Val His Thr Ile Asp
2345 2350 2355
Val Lys Phe Asn Gly Ser His Val Val Gly Ser Pro Phe Lys Val
2360 2365 2370
Arg Val Gly Glu Pro Gly Gln Ala Gly Asn Pro Ala Leu Val Ser
2375 2380 2385
Ala Tyr Gly Thr Gly Leu Glu Gly Gly Thr Thr Gly Ile Gln Ser
2390 2395 2400
Glu Phe Phe Ile Asn Thr Thr Arg Ala Gly Pro Gly Thr Leu Ser
2405 2410 2415
Val Thr Ile Glu Gly Pro Ser Lys Val Lys Met Asp Cys Gln Glu
2420 2425 2430
Thr Pro Glu Gly Tyr Lys Val Met Tyr Thr Pro Met Ala Pro Gly
2435 2440 2445
Asn Tyr Leu Ile Ser Val Lys Tyr Gly Gly Pro Asn His Ile Val
2450 2455 2460
Gly Ser Pro Phe Lys Ala Lys Val Thr Gly Gln Arg Leu Val Ser
2465 2470 2475
Pro Gly Ser Ala Asn Glu Thr Ser Ser Ile Leu Val Glu Ser Val
2480 2485 2490
Thr Arg Ser Ser Thr Glu Thr Cys Tyr Ser Ala Ile Pro Lys Ala
2495 2500 2505
Ser Ser Asp Ala Ser Lys Val Thr Ser Lys Gly Ala Gly Leu Ser
2510 2515 2520
Lys Ala Phe Val Gly Gln Lys Ser Ser Phe Leu Val Asp Cys Ser
2525 2530 2535
Lys Ala Gly Ser Asn Met Leu Leu Ile Gly Val His Gly Pro Thr
2540 2545 2550
Thr Pro Cys Glu Glu Val Ser Met Lys His Val Gly Asn Gln Gln
2555 2560 2565
Tyr Asn Val Thr Tyr Val Val Lys Glu Arg Gly Asp Tyr Val Leu
2570 2575 2580
Ala Val Lys Trp Gly Glu Glu His Ile Pro Gly Ser Pro Phe His
2585 2590 2595
Val Thr Val Pro
2600

<210> 504
<211> 760
<212> PRT
<213> Homo sapiens
<400> 504

```
Met Met Asp Gln Ala Arg Ser Ala Phe Ser Asn Leu Phe Gly Gly Glu
1               5                   10                  15
Pro Leu Ser Tyr Thr Arg Phe Ser Leu Ala Arg Gln Val Asp Gly Asp
            20                  25                  30
Asn Ser His Val Glu Met Lys Leu Ala Val Asp Glu Glu Glu Asn Ala
        35                  40                  45
Asp Asn Asn Thr Lys Ala Asn Val Thr Lys Pro Lys Arg Cys Ser Gly
    50                  55                  60
Ser Ile Cys Tyr Gly Thr Ile Ala Val Ile Val Phe Phe Leu Ile Gly
65                  70                  75                  80
Phe Met Ile Gly Tyr Leu Gly Tyr Cys Lys Gly Val Glu Pro Lys Thr
                85                  90                  95
Glu Cys Glu Arg Leu Ala Gly Thr Glu Ser Pro Val Arg Glu Glu Pro
            100                 105                 110
Gly Glu Asp Phe Pro Ala Ala Arg Arg Leu Tyr Trp Asp Asp Leu Lys
        115                 120                 125
Arg Lys Leu Ser Glu Lys Leu Asp Ser Thr Asp Phe Thr Ser Thr Ile
    130                 135                 140
Lys Leu Leu Asn Glu Asn Ser Tyr Val Pro Arg Glu Ala Gly Ser Gln
145                 150                 155                 160
Lys Asp Glu Asn Leu Ala Leu Tyr Val Glu Asn Gln Phe Arg Glu Phe
                165                 170                 175
Lys Leu Ser Lys Val Trp Arg Asp Gln His Phe Val Lys Ile Gln Val
            180                 185                 190
Lys Asp Ser Ala Gln Asn Ser Val Ile Ile Val Asp Lys Asn Gly Arg
        195                 200                 205
Leu Val Tyr Leu Val Glu Asn Pro Gly Gly Tyr Val Ala Tyr Ser Lys
    210                 215                 220
Ala Ala Thr Val Thr Gly Lys Leu Val His Ala Asn Phe Gly Thr Lys
225                 230                 235                 240
Lys Asp Phe Glu Asp Leu Tyr Thr Pro Val Asn Gly Ser Ile Val Ile
                245                 250                 255
Val Arg Ala Gly Lys Ile Thr Phe Ala Glu Lys Val Ala Asn Ala Glu
            260                 265                 270
Ser Leu Asn Ala Ile Gly Val Leu Ile Tyr Met Asp Gln Thr Lys Phe
        275                 280                 285
Pro Ile Val Asn Ala Glu Leu Ser Phe Phe Gly His Ala His Leu Gly
    290                 295                 300
Thr Gly Asp Pro Tyr Thr Pro Gly Phe Pro Ser Phe Asn His Thr Gln
305                 310                 315                 320
Phe Pro Pro Ser Arg Ser Ser Gly Leu Pro Asn Ile Pro Val Gln Thr
                325                 330                 335
Ile Ser Arg Ala Ala Ala Glu Lys Leu Phe Gly Asn Met Glu Gly Asp
            340                 345                 350
Cys Pro Ser Asp Trp Lys Thr Asp Ser Thr Cys Arg Met Val Thr Ser
        355                 360                 365
Glu Ser Lys Asn Val Lys Leu Thr Val Ser Asn Val Leu Lys Glu Ile
    370                 375                 380
Lys Ile Leu Asn Ile Phe Gly Val Ile Lys Gly Phe Val Glu Pro Asp
385                 390                 395                 400
His Tyr Val Val Val Gly Ala Gln Arg Asp Ala Trp Gly Pro Gly Ala
                405                 410                 415
Ala Lys Ser Gly Val Gly Thr Ala Leu Leu Leu Lys Leu Ala Gln Met
            420                 425                 430
Phe Ser Asp Met Val Leu Lys Asp Gly Phe Gln Pro Ser Arg Ser Ile
        435                 440                 445
Ile Phe Ala Ser Trp Ser Ala Gly Asp Phe Gly Ser Val Gly Ala Thr
    450                 455                 460
Glu Trp Leu Glu Gly Tyr Leu Ser Ser Leu His Leu Lys Ala Phe Thr
465                 470                 475                 480
Tyr Ile Asn Leu Asp Lys Ala Val Leu Gly Thr Ser Asn Phe Lys Val
                485                 490                 495
Ser Ala Ser Pro Leu Leu Tyr Thr Leu Ile Glu Lys Thr Met Gln Asn
            500                 505                 510
```

Val Lys His Pro Val Thr Gly Gln Phe Leu Tyr Gln Asp Ser Asn Trp
515 520 525
Ala Ser Lys Val Glu Lys Leu Thr Leu Asp Asn Ala Ala Phe Pro Phe
530 535 540
Leu Ala Tyr Ser Gly Ile Pro Ala Val Ser Phe Cys Phe Cys Glu Asp
545 550 555 560
Thr Asp Tyr Pro Tyr Leu Gly Thr Thr Met Asp Thr Tyr Lys Glu Leu
565 570 575
Ile Glu Arg Ile Pro Glu Leu Asn Lys Val Ala Arg Ala Ala Ala Glu
580 585 590
Val Ala Gly Gln Phe Val Ile Lys Leu Thr His Asp Val Glu Leu Asn
595 600 605
Leu Asp Tyr Glu Arg Tyr Asn Ser Gln Leu Leu Ser Phe Val Arg Asp
610 615 620
Leu Asn Gln Tyr Arg Ala Asp Ile Lys Glu Met Gly Leu Ser Leu Gln
625 630 635 640
Trp Leu Tyr Ser Ala Arg Gly Asp Phe Phe Arg Ala Thr Ser Arg Leu
645 650 655
Thr Thr Asp Phe Gly Asn Ala Glu Lys Thr Asp Arg Phe Val Met Lys
660 665 670
Lys Leu Asn Asp Arg Val Met Arg Val Glu Tyr His Phe Leu Ser Pro
675 680 685
Tyr Val Ser Pro Lys Glu Ser Pro Phe Arg His Val Phe Trp Gly Ser
690 695 700
Gly Ser His Thr Leu Pro Ala Leu Leu Glu Asn Leu Lys Leu Arg Lys
705 710 715 720
Gln Asn Asn Gly Ala Phe Asn Glu Thr Leu Phe Arg Asn Gln Leu Ala
725 730 735
Leu Ala Thr Trp Thr Ile Gln Gly Ala Ala Asn Ala Leu Ser Gly Asp
740 745 750
Val Trp Asp Ile Asp Asn Glu Phe
755 760

<210> 505
<211> 611
<212> PRT
<213> Homo sapiens
<400> 505
Met Ala Ala Ser Ala Lys Lys Lys Asn Lys Lys Gly Lys Thr Ile Ser
1 5 10 15
Leu Thr Asp Phe Leu Ala Glu Asp Gly Gly Thr Gly Gly Gly Ser Thr
20 25 30
Tyr Val Ser Lys Pro Val Ser Trp Ala Asp Glu Thr Asp Asp Leu Glu
35 40 45
Gly Asp Val Ser Thr Thr Trp His Ser Asn Asp Asp Asp Val Tyr Arg
50 55 60
Ala Pro Pro Ile Asp Arg Ser Ile Leu Pro Thr Ala Pro Arg Ala Ala
65 70 75 80
Arg Glu Pro Asn Ile Asp Arg Ser Arg Leu Pro Lys Ser Pro Pro Tyr
85 90 95
Thr Ala Phe Leu Gly Asn Leu Pro Tyr Asp Val Thr Glu Glu Ser Ile
100 105 110
Lys Glu Phe Phe Arg Gly Leu Asn Ile Ser Ala Val Arg Leu Pro Arg
115 120 125
Glu Pro Ser Asn Pro Glu Arg Leu Lys Gly Phe Gly Tyr Ala Glu Phe
130 135 140
Glu Asp Leu Asp Ser Leu Leu Ser Ala Leu Ser Leu Asn Glu Glu Ser
145 150 155 160
Leu Gly Asn Arg Arg Ile Arg Val Asp Val Ala Asp Gln Ala Gln Asp
165 170 175
Lys Asp Arg Asp Asp Arg Ser Phe Gly Arg Asp Arg Asn Arg Asp Ser
180 185 190
Asp Lys Thr Asp Thr Asp Trp Arg Ala Arg Pro Ala Thr Asp Ser Phe
195 200 205
Asp Asp Tyr Pro Pro Arg Arg Gly Asp Asp Ser Phe Gly Asp Lys Tyr
210 215 220
Arg Asp Arg Tyr Asp Ser Asp Arg Tyr Arg Asp Gly Tyr Arg Asp Gly
225 230 235 240
Tyr Arg Asp Gly Pro Arg Arg Asp Met Asp Arg Tyr Gly Gly Arg Asp

245 250 255
Arg Tyr Asp Asp Arg Gly Ser Arg Asp Tyr Asp Arg Gly Tyr Asp Ser
260 265 270
Arg Ile Gly Ser Gly Arg Arg Ala Phe Gly Ser Gly Tyr Arg Arg Asp
275 280 285
Asp Asp Tyr Arg Gly Gly Gly Asp Arg Tyr Glu Asp Arg Tyr Asp Arg
290 295 300
Arg Asp Asp Arg Ser Trp Ser Ser Arg Asp Asp Tyr Ser Arg Asp Asp
305 310 315 320
Tyr Arg Arg Asp Asp Arg Gly Pro Pro Gln Arg Pro Lys Leu Asn Leu
325 330 335
Lys Pro Arg Ser Thr Pro Glu Glu Asp Asp Ser Ser Ala Ser Thr Ser
340 345 350
Gln Ser Thr Arg Ala Ala Ser Ile Phe Gly Gly Ala Lys Pro Val Asp
355 360 365
Thr Ala Ala Arg Glu Arg Glu Val Glu Glu Arg Leu Gln Lys Glu Gln
370 375 380
Glu Lys Leu Gln Arg Gln Trp Asn Glu Pro Lys Leu Glu Arg Arg Pro
385 390 395 400
Arg Glu Arg His Pro Ser Trp Arg Ser Glu Glu Thr Gln Glu Arg Glu
405 410 415
Arg Ser Arg Thr Gly Ser Glu Ser Ser Gln Thr Gly Thr Ser Thr Thr
420 425 430
Ser Ser Arg Asn Ala Arg Arg Arg Glu Ser Glu Lys Ser Leu Glu Asn
435 440 445
Glu Thr Leu Asn Lys Glu Glu Asp Cys His Ser Pro Thr Ser Lys Pro
450 455 460
Pro Lys Pro Asp Gln Pro Leu Lys Val Met Pro Ala Pro Pro Pro Lys
465 470 475 480
Glu Asn Ala Trp Val Lys Arg Ser Ser Asn Pro Pro Ala Arg Ser Gln
485 490 495
Ser Ser Asp Thr Glu Gln Gln Ser Pro Thr Ser Gly Gly Gly Lys Val
500 505 510
Ala Pro Ala Gln Pro Ser Glu Glu Gly Pro Gly Arg Lys Asp Glu Asn
515 520 525
Lys Val Asp Gly Met Asn Ala Pro Lys Gly Gln Thr Gly Asn Ser Ser
530 535 540
Arg Gly Pro Gly Asp Gly Gly Asn Arg Asp His Trp Lys Glu Ser Asp
545 550 555 560
Arg Lys Asp Gly Lys Lys Asp Gln Asp Ser Arg Ser Ala Pro Glu Pro
565 570 575
Lys Lys Pro Glu Glu Asn Pro Ala Ser Lys Phe Ser Ser Ala Ser Lys
580 585 590
Tyr Ala Ala Leu Ser Val Asp Gly Glu Asp Glu Asn Glu Gly Glu Asp
595 600 605
Tyr Ala Glu
610

<210> 506
<211> 379
<212> PRT
<213> Homo sapiens
<400> 506
Met Ala Ala Ala Ala Ala Gln Gly Gly Gly Gly Gly Glu Pro Arg Arg
1 5 10 15
Thr Glu Gly Val Gly Pro Gly Val Pro Gly Glu Val Glu Met Val Lys
20 25 30
Gly Gln Pro Phe Asp Val Gly Pro Arg Tyr Thr Gln Leu Gln Tyr Ile
35 40 45
Gly Glu Gly Ala Tyr Gly Met Val Ser Ser Ala Tyr Asp His Val Arg
50 55 60
Lys Thr Arg Val Ala Ile Lys Lys Ile Ser Pro Phe Glu His Gln Thr
65 70 75 80
Tyr Cys Gln Arg Thr Leu Arg Glu Ile Gln Ile Leu Leu Arg Phe Arg
85 90 95
His Glu Asn Val Ile Gly Ile Arg Asp Ile Leu Arg Ala Ser Thr Leu
100 105 110
Glu Ala Met Arg Asp Val Tyr Ile Val Gln Asp Leu Met Glu Thr Asp
115 120 125

Leu Tyr Lys Leu Leu Lys Ser Gln Gln Leu Ser Asn Asp His Ile Cys
130 135 140
Tyr Phe Leu Tyr Gln Ile Leu Arg Gly Leu Lys Tyr Ile His Ser Ala
145 150 155 160
Asn Val Leu His Arg Asp Leu Lys Pro Ser Asn Leu Leu Ser Asn Thr
165 170 175
Thr Cys Asp Leu Lys Ile Cys Asp Phe Gly Leu Ala Arg Ile Ala Asp
180 185 190
Pro Glu His Asp His Thr Gly Phe Leu Thr Glu Tyr Val Ala Thr Arg
195 200 205
Trp Tyr Arg Ala Pro Glu Ile Met Leu Asn Ser Lys Gly Tyr Thr Lys
210 215 220
Ser Ile Asp Ile Trp Ser Val Gly Cys Ile Leu Ala Glu Met Leu Ser
225 230 235 240
Asn Arg Pro Ile Phe Pro Gly Lys His Tyr Leu Asp Gln Leu Asn His
245 250 255
Ile Leu Gly Ile Leu Gly Ser Pro Ser Gln Glu Asp Leu Asn Cys Ile
260 265 270
Ile Asn Met Lys Ala Arg Asn Tyr Leu Gln Ser Leu Pro Ser Lys Thr
275 280 285
Lys Val Ala Trp Ala Lys Leu Phe Pro Lys Ser Asp Ser Lys Ala Leu
290 295 300
Asp Leu Leu Asp Arg Met Leu Thr Phe Asn Pro Asn Lys Arg Ile Thr
305 310 315 320
Val Glu Glu Ala Leu Ala His Pro Tyr Leu Glu Gln Tyr Tyr Asp Pro
325 330 335
Thr Asp Glu Pro Val Ala Glu Glu Pro Phe Thr Phe Ala Met Glu Leu
340 345 350
Asp Asp Leu Pro Lys Glu Arg Leu Lys Glu Leu Ile Phe Gln Glu Thr
355 360 365
Ala Arg Phe Gln Pro Gly Val Leu Glu Ala Pro
370 375

<210> 507
<211> 351
<212> PRT
<213> Homo sapiens
<400> 507
Met Thr Trp Ser Ala Thr Ala Arg Gly Ala His Gln Pro Asp Asn Thr
1 5 10 15
Ala Phe Thr Gln Gln Arg Leu Pro Ala Trp Gln Pro Leu Leu Ser Ala
20 25 30
Ser Ile Ala Leu Pro Leu Phe Phe Cys Ala Gly Leu Ala Phe Ile Gly
35 40 45
Leu Gly Leu Gly Leu Tyr Tyr Ser Ser Asn Gly Ile Lys Glu Leu Glu
50 55 60
Tyr Asp Tyr Thr Gly Asp Pro Gly Thr Gly Asn Cys Ser Val Cys Ala
65 70 75 80
Ala Ala Gly Gln Gly Arg Ala Leu Pro Pro Pro Cys Ser Cys Ala Trp
85 90 95
Tyr Phe Ser Leu Pro Glu Leu Phe Gln Gly Pro Val Tyr Leu Tyr Tyr
100 105 110
Glu Leu Thr Asn Phe Tyr Gln Asn Asn Arg Arg Tyr Gly Val Ser Arg
115 120 125
Asp Asp Ala Gln Leu Ser Gly Leu Pro Ser Ala Leu Arg His Pro Val
130 135 140
Asn Glu Cys Ala Pro Tyr Gln Arg Ser Ala Ala Gly Leu Pro Ile Ala
145 150 155 160
Pro Cys Gly Ala Ile Ala Asn Ser Leu Phe Asn Asp Ser Phe Ser Leu
165 170 175
Trp His Gln Arg Gln Pro Gly Gly Pro Tyr Val Glu Val Pro Leu Asp
180 185 190
Arg Ser Gly Ile Ala Trp Trp Thr Asp Tyr His Val Lys Phe Arg Asn
195 200 205
Pro Pro Leu Val Asn Gly Ser Leu Ala Leu Ala Phe Gln Gly Thr Ala
210 215 220
Pro Pro Pro Asn Trp Arg Arg Pro Val Tyr Glu Leu Ser Pro Asp Pro
225 230 235 240
Asn Asn Thr Gly Phe Ile Asn Gln Asp Phe Val Val Trp Met Arg Thr

```
                245                 250                 255
Ala Ala Leu Pro Thr Phe Arg Lys Leu Tyr Ala Arg Ile Arg Gln Gly
            260                 265                 270
Asn Tyr Ser Ala Gly Leu Pro Arg Gly Ala Tyr Arg Val Asn Ile Thr
        275                 280                 285
Tyr Asn Tyr Pro Val Arg Ala Phe Gly Gly His Lys Leu Leu Ile Phe
    290                 295                 300
Ser Ser Ile Ser Trp Met Gly Gly Lys Asn Pro Phe Leu Gly Ile Ala
305                 310                 315                 320
Tyr Leu Val Val Gly Ser Leu Cys Ile Leu Thr Gly Phe Val Met Leu
                325                 330                 335
Val Val Tyr Ile Arg Tyr Gln Asp Gln Asp Asp Asp Asp Glu Glu
            340                 345                 350

<210>  508
<211>  524
<212>  PRT
<213>  Homo sapiens
<400>  508
Met Gly Lys Pro Ala Arg Lys Gly Cys Glu Trp Lys Arg Phe Leu Lys
1               5                   10                  15
Asn Asn Trp Val Leu Leu Ser Thr Val Ala Ala Val Val Leu Gly Ile
            20                  25                  30
Thr Thr Gly Val Leu Val Arg Glu His Ser Asn Leu Ser Thr Leu Glu
        35                  40                  45
Lys Phe Tyr Phe Ala Phe Pro Gly Glu Ile Leu Met Arg Met Leu Lys
    50                  55                  60
Leu Ile Ile Leu Pro Leu Ile Ile Ser Ser Met Ile Thr Gly Val Ala
65                  70                  75                  80
Ala Leu Asp Ser Asn Val Ser Gly Lys Ile Gly Val Arg Ala Val Val
                85                  90                  95
Tyr Tyr Phe Cys Thr Thr Leu Ile Ala Val Ile Leu Gly Ile Val Leu
            100                 105                 110
Val Val Ser Ile Lys Pro Gly Val Thr Gln Lys Val Gly Glu Ile Ala
        115                 120                 125
Arg Thr Gly Ser Thr Pro Glu Val Ser Thr Val Asp Ala Met Leu Asp
    130                 135                 140
Leu Ile Arg Asn Met Phe Pro Glu Asn Leu Val Gln Ala Cys Phe Gln
145                 150                 155                 160
Gln Tyr Lys Thr Lys Arg Glu Glu Val Lys Pro Pro Ser Asp Pro Glu
                165                 170                 175
Met Asn Met Thr Glu Glu Ser Phe Thr Ala Val Met Thr Thr Ala Ile
            180                 185                 190
Ser Lys Asn Lys Thr Lys Glu Tyr Lys Ile Val Gly Met Tyr Ser Asp
        195                 200                 205
Gly Ile Asn Val Leu Gly Leu Ile Val Phe Cys Leu Val Phe Gly Leu
    210                 215                 220
Val Ile Gly Lys Met Gly Glu Lys Gly Gln Ile Leu Val Asp Phe Phe
225                 230                 235                 240
Asn Ala Leu Ser Asp Ala Thr Met Lys Ile Val Gln Ile Ile Met Cys
                245                 250                 255
Tyr Met Pro Leu Gly Ile Leu Phe Leu Ile Ala Gly Lys Ile Ile Glu
            260                 265                 270
Val Glu Asp Trp Glu Ile Phe Arg Lys Leu Gly Leu Tyr Met Ala Thr
        275                 280                 285
Val Leu Thr Gly Leu Ala Ile His Ser Ile Val Ile Leu Pro Leu Ile
    290                 295                 300
Tyr Phe Ile Val Val Arg Lys Asn Pro Phe Arg Phe Ala Met Gly Met
305                 310                 315                 320
Ala Gln Ala Leu Leu Thr Ala Leu Met Ile Ser Ser Ser Ser Ala Thr
                325                 330                 335
Leu Pro Val Thr Phe Arg Cys Ala Glu Glu Asn Asn Gln Val Asp Lys
            340                 345                 350
Arg Ile Thr Arg Phe Val Leu Pro Val Gly Ala Thr Ile Asn Met Asp
        355                 360                 365
Gly Thr Ala Leu Tyr Glu Ala Val Ala Ala Val Phe Ile Ala Gln Leu
    370                 375                 380
Asn Asp Leu Asp Leu Gly Ile Gly Gln Ile Ile Thr Ile Ser Ile Thr
385                 390                 395                 400
```

```
Ala Thr Ser Ala Ser Ile Gly Ala Ala Gly Val Pro Gln Ala Gly Leu
                405                 410                 415
Val Thr Met Val Ile Val Leu Ser Ala Val Gly Leu Pro Ala Glu Asp
            420                 425                 430
Val Thr Leu Ile Ile Ala Val Asp Trp Leu Leu Asp Arg Phe Arg Thr
        435                 440                 445
Met Val Asn Val Leu Gly Asp Ala Phe Gly Thr Gly Ile Val Glu Lys
    450                 455                 460
Leu Ser Lys Lys Glu Leu Glu Gln Met Asp Val Ser Ser Glu Val Asn
465                 470                 475                 480
Ile Val Asn Pro Phe Ala Leu Glu Ser Thr Ile Leu Asp Asn Glu Asp
                485                 490                 495
Ser Asp Thr Lys Lys Ser Tyr Val Asn Gly Gly Phe Ala Val Asp Lys
            500                 505                 510
Ser Asp Thr Ile Ser Phe Thr Gln Thr Ser Gln Phe
        515                 520

<210>  509
<211>  416
<212>  PRT
<213>  Homo sapiens
<400>  509
Met Ala His Ser Pro Val Ala Val Gln Val Pro Gly Met Gln Asn Asn
1               5                   10                  15
Ile Ala Asp Pro Glu Glu Leu Phe Thr Lys Leu Glu Arg Ile Gly Lys
            20                  25                  30
Gly Ser Phe Gly Glu Val Phe Lys Gly Ile Asp Asn Arg Thr Gln Gln
        35                  40                  45
Val Val Ala Ile Lys Ile Ile Asp Leu Glu Glu Ala Glu Asp Glu Ile
    50                  55                  60
Glu Asp Ile Gln Gln Glu Ile Thr Val Leu Ser Gln Cys Asp Ser Ser
65                  70                  75                  80
Tyr Val Thr Lys Tyr Tyr Gly Ser Tyr Leu Lys Gly Ser Lys Leu Trp
                85                  90                  95
Ile Ile Met Glu Tyr Leu Gly Gly Gly Ser Ala Leu Asp Leu Leu Arg
            100                 105                 110
Ala Gly Pro Phe Asp Glu Phe Gln Ile Ala Thr Met Leu Lys Glu Ile
        115                 120                 125
Leu Lys Gly Leu Asp Tyr Leu His Ser Glu Lys Lys Ile His Arg Asp
    130                 135                 140
Ile Lys Ala Ala Asn Val Leu Leu Ser Glu Gln Gly Asp Val Lys Leu
145                 150                 155                 160
Ala Asp Phe Gly Val Ala Gly Gln Leu Thr Asp Thr Gln Ile Lys Arg
                165                 170                 175
Asn Thr Phe Val Gly Thr Pro Phe Trp Met Ala Pro Glu Val Ile Gln
            180                 185                 190
Gln Ser Ala Tyr Asp Ser Lys Ala Asp Ile Trp Ser Leu Gly Ile Thr
        195                 200                 205
Ala Ile Glu Leu Ala Lys Gly Glu Pro Pro Asn Ser Asp Met His Pro
    210                 215                 220
Met Arg Val Leu Phe Leu Ile Pro Lys Asn Asn Pro Pro Thr Leu Val
225                 230                 235                 240
Gly Asp Phe Thr Lys Ser Phe Lys Glu Phe Ile Asp Ala Cys Leu Asn
                245                 250                 255
Lys Asp Pro Ser Phe Arg Pro Thr Ala Lys Glu Leu Leu Lys His Lys
            260                 265                 270
Phe Ile Val Lys Asn Ser Lys Lys Thr Ser Tyr Leu Thr Glu Leu Ile
        275                 280                 285
Asp Arg Phe Lys Arg Trp Lys Ala Glu Gly His Ser Asp Asp Glu Ser
    290                 295                 300
Asp Ser Glu Gly Ser Asp Ser Glu Ser Thr Ser Arg Glu Asn Asn Thr
305                 310                 315                 320
His Pro Glu Trp Ser Phe Thr Thr Val Arg Lys Lys Pro Asp Pro Lys
                325                 330                 335
Lys Val Gln Asn Gly Ala Glu Gln Asp Leu Val Gln Thr Leu Ser Cys
            340                 345                 350
Leu Ser Met Ile Ile Thr Pro Ala Phe Ala Glu Leu Lys Gln Gln Asp
        355                 360                 365
Glu Asn Asn Ala Ser Arg Asn Gln Ala Ile Glu Glu Leu Glu Lys Ser
```

370 375 380
Ile Ala Val Ala Glu Ala Ala Cys Pro Gly Ile Thr Asp Lys Met Val
385 390 395 400
Lys Lys Leu Ile Glu Lys Phe Gln Lys Cys Ser Ala Asp Glu Ser Pro
405 410 415

<210> 510
<211> 285
<212> PRT
<213> Homo sapiens
<400> 510
Met Val Met Arg Pro Leu Trp Ser Leu Leu Leu Trp Glu Ala Leu Leu
1 5 10 15
Pro Ile Thr Val Thr Gly Ala Gln Val Leu Ser Lys Val Gly Gly Ser
20 25 30
Val Leu Leu Val Ala Ala Arg Pro Pro Gly Phe Gln Val Arg Glu Ala
35 40 45
Ile Trp Arg Ser Leu Trp Pro Ser Glu Glu Leu Leu Ala Thr Phe Phe
50 55 60
Arg Gly Ser Leu Glu Thr Leu Tyr His Ser Arg Phe Leu Gly Arg Ala
65 70 75 80
Gln Leu His Ser Asn Leu Ser Leu Glu Leu Gly Pro Leu Glu Ser Gly
85 90 95
Asp Ser Gly Asn Phe Ser Val Leu Met Val Asp Thr Arg Gly Gln Pro
100 105 110
Trp Thr Gln Thr Leu Gln Leu Lys Val Tyr Asp Ala Val Pro Arg Pro
115 120 125
Val Val Gln Val Phe Ile Ala Val Glu Arg Asp Ala Gln Pro Ser Lys
130 135 140
Thr Cys Gln Val Phe Leu Ser Cys Trp Ala Pro Asn Ile Ser Glu Ile
145 150 155 160
Thr Tyr Ser Trp Arg Arg Glu Thr Thr Met Asp Phe Gly Met Glu Pro
165 170 175
His Ser Leu Phe Thr Asp Gly Gln Val Leu Ser Ile Ser Leu Gly Pro
180 185 190
Gly Asp Arg Asp Val Ala Tyr Ser Cys Ile Val Ser Asn Pro Val Ser
195 200 205
Trp Asp Leu Ala Thr Val Thr Pro Trp Asp Ser Cys His His Glu Ala
210 215 220
Ala Pro Gly Lys Ala Ser Tyr Lys Asp Val Leu Leu Val Val Val Pro
225 230 235 240
Val Ser Leu Leu Leu Met Leu Val Thr Leu Phe Ser Ala Trp His Trp
245 250 255
Cys Pro Cys Ser Gly Lys Lys Lys Lys Asp Val His Ala Asp Arg Val
260 265 270
Gly Pro Glu Thr Glu Asn Pro Leu Val Gln Asp Leu Pro
275 280 285

<210> 511
<211> 376
<212> PRT
<213> Homo sapiens
<400> 511
Met Asn His Ser Glu Arg Phe Val Phe Ile Ala Glu Trp Tyr Asp Pro
1 5 10 15
Asn Ala Ser Leu Leu Arg Arg Tyr Glu Leu Leu Phe Tyr Pro Gly Asp
20 25 30
Gly Ser Val Glu Met His Asp Val Lys Asn His Arg Thr Phe Leu Lys
35 40 45
Arg Thr Lys Tyr Asp Asn Leu His Leu Glu Asp Leu Phe Ile Gly Asn
50 55 60
Lys Val Asn Val Phe Ser Arg Gln Leu Val Leu Ile Asp Tyr Gly Asp
65 70 75 80
Gln Tyr Thr Ala Arg Gln Leu Gly Ser Arg Lys Glu Lys Thr Leu Ala
85 90 95
Leu Ile Lys Pro Asp Ala Ile Ser Lys Ala Gly Glu Ile Ile Glu Ile
100 105 110
Ile Asn Lys Ala Gly Phe Thr Ile Thr Lys Leu Lys Met Met Met Leu
115 120 125

Ser Arg Lys Glu Ala Leu Asp Phe His Val Asp His Gln Ser Arg Pro
    130                 135                 140
Phe Phe Asn Glu Leu Ile Gln Phe Ile Thr Thr Gly Pro Ile Ile Ala
145                 150                 155                 160
Met Glu Ile Leu Arg Asp Asp Ala Ile Cys Glu Trp Lys Arg Leu Leu
                165                 170                 175
Gly Pro Ala Asn Ser Gly Val Ala Arg Thr Asp Ala Ser Glu Ser Ile
            180                 185                 190
Arg Ala Leu Phe Gly Thr Asp Gly Ile Arg Asn Ala Ala His Gly Pro
        195                 200                 205
Asp Ser Phe Ala Ser Ala Ala Arg Glu Met Glu Leu Phe Phe Pro Ser
    210                 215                 220
Ser Gly Gly Cys Gly Pro Ala Asn Thr Ala Lys Phe Thr Asn Cys Thr
225                 230                 235                 240
Cys Cys Ile Val Lys Pro His Ala Val Ser Glu Gly Leu Leu Gly Lys
                245                 250                 255
Ile Leu Met Ala Ile Arg Asp Ala Gly Phe Glu Ile Ser Ala Met Gln
            260                 265                 270
Met Phe Asn Met Asp Arg Val Asn Val Glu Glu Phe Tyr Glu Val Tyr
        275                 280                 285
Lys Gly Val Val Thr Glu Tyr His Asp Met Val Thr Glu Met Tyr Ser
    290                 295                 300
Gly Pro Cys Val Ala Met Glu Ile Gln Gln Asn Asn Ala Thr Lys Thr
305                 310                 315                 320
Phe Arg Glu Phe Cys Gly Pro Ala Asp Pro Glu Ile Ala Arg His Leu
                325                 330                 335
Arg Pro Gly Thr Leu Arg Ala Ile Phe Gly Lys Thr Lys Ile Gln Asn
            340                 345                 350
Ala Val His Cys Thr Asp Leu Pro Glu Asp Gly Leu Leu Glu Val Gln
        355                 360                 365
Tyr Phe Phe Lys Ile Leu Asp Asn
    370                 375

<210> 512
<211> 27
<212> DNA
<213> Homo sapiens
<400> 512
ccctcatcct ggttctggaa tcctcag 27

<210> 513
<211> 19
<212> DNA
<213> Homo sapiens
<400> 513
tcggcttgtc ctggctctt 19

<210> 514
<211> 20
<212> DNA
<213> Homo sapiens
<400> 514
tggctctccg cgtaggataa 20

<210> 515
<211> 20
<212> DNA
<213> Homo sapiens
<400> 515
cttggccaga ccaatgccca 20

<210> 516
<211> 28
<212> DNA
<213> Homo sapiens
<400> 516
cagagtctta ggtaaaagtc ttgggaaa 28

<210> 517

<211> 25
<212> DNA
<213> Homo sapiens
<400> 517
tgtccttgat attgggacat tgtag 25

<210> 518
<211> 37
<212> DNA
<213> Homo sapiens
<400> 518
ttttactggt tcttctgaat tgacagtaaa cctgtcc 37

<210> 519
<211> 24
<212> DNA
<213> Homo sapiens
<400> 519
aatcgtgctg cttggataga aata 24

<210> 520
<211> 29
<212> DNA
<213> Homo sapiens
<400> 520
caaataatag aacagtaggc cattcataa 29

<210> 521
<211> 27
<212> DNA
<213> Homo sapiens
<400> 521
tttcgcatag caaccctcca cttttcg 27

<210> 522
<211> 19
<212> DNA
<213> Homo sapiens
<400> 522
cagcatcttg cccctcaga 19

<210> 523
<211> 24
<212> DNA
<213> Homo sapiens
<400> 523
aattggaatg aaaccacagt cttg 24

<210> 524
<211> 23
<212> DNA
<213> Homo sapiens
<400> 524
aaatgcccat ctatgtccct tgc 23

<210> 525
<211> 24
<212> DNA
<213> Homo sapiens
<400> 525
tccctgaatg tattgaactt ggaa 24

<210> 526
<211> 24
<212> DNA
<213> Homo sapiens
<400> 526
ttcaggcagc aagttttact ttga 24

<210> 527
<211> 32
<212> DNA
<213> Homo sapiens
<400> 527
atggcagcag ttccaacctt cagaactcaa ta 32

<210> 528
<211> 20
<212> DNA
<213> Homo sapiens
<400> 528
ccctctgctc cacagaaacc 20

<210> 529
<211> 21
<212> DNA
<213> Homo sapiens
<400> 529
ggtctgcaaa gtggccaaaa t 21

<210> 530
<211> 22
<212> DNA
<213> Homo sapiens
<400> 530
tcccaagcca taaagtgcac at 22

<210> 531
<211> 22
<212> DNA
<213> Homo sapiens
<400> 531
ggacagcaaa aagacaagca aa 22

<210> 532
<211> 22
<212> DNA
<213> Homo sapiens
<400> 532
ctgtacaatg tcccccaaat ca 22

<210> 533
<211> 30
<212> DNA
<213> Homo sapiens
<400> 533
attcactgat gaccaaatgg agatataacc 30

<210> 534
<211> 26
<212> DNA
<213> Homo sapiens
<400> 534
cagttgctga cttctcttat ggacat 26

<210> 535
<211> 21
<212> DNA
<213> Homo sapiens
<400> 535
attctgtgca ccatgcacac a 21

<210> 536
<211> 28
<212> DNA
<213> Homo sapiens
<400> 536
agtatctgag ttcaaaattc ccaaagga 28

<210> 537
<211> 20
<212> DNA
<213> Homo sapiens
<400> 537
tccctctggc agttgtgtga 20

<210> 538
<211> 21
<212> DNA
<213> Homo sapiens
<400> 538
tgcacggcaa gatgtactga a 21

<210> 539
<211> 27
<212> DNA
<213> Homo sapiens
<400> 539
cagcgcatat caggatcatt agacttt 27

<210> 540
<211> 22
<212> DNA
<213> Homo sapiens
<400> 540
aatcaaggag cttggcactc tt 22

<210> 541
<211> 26
<212> DNA
<213> Homo sapiens
<400> 541
gatgctttta gaatggttcc tttgtt 26

<210> 542
<211> 26
<212> DNA
<213> Homo sapiens
<400> 542
ttcggccagt ctccttaaac tagtca 26

<210> 543
<211> 20
<212> DNA
<213> Homo sapiens
<400> 543
accagcttgg tgcggatagt 20

<210> 544
<211> 22
<212> DNA
<213> Homo sapiens
<400> 544
gtgcttttcc atccacagat tg 22

<210> 545
<211> 25
<212> DNA
<213> Homo sapiens
<400> 545
cagcaaagct ggctccaaca tgctg 25

<210> 546
<211> 20
<212> DNA
<213> Homo sapiens
<400> 546

tgtgggccag aagagttcct 20

<210> 547
<211> 17
<212> DNA
<213> Homo sapiens
<400> 547
cccatggacc ccgatca 17

<210> 548
<211> 32
<212> DNA
<213> Homo sapiens
<400> 548
agctccgtga gtgaaccatc attataaacg tg 32

<210> 549
<211> 20
<212> DNA
<213> Homo sapiens
<400> 549
gcctacccat tcgtggtgat 20

<210> 550
<211> 19
<212> DNA
<213> Homo sapiens
<400> 550
tccctaggag gccgtttcc 19

<210> 551
<211> 23
<212> DNA
<213> Homo sapiens
<400> 551
cccaccactt gtaggggact gct 23

<210> 552
<211> 23
<212> DNA
<213> Homo sapiens
<400> 552
ctcgatctca gagctcagac aca 23

<210> 553
<211> 28
<212> DNA
<213> Homo sapiens
<400> 553
gcattcatcc catctacttt attttcat 28

<210> 554
<211> 22
<212> DNA
<213> Homo sapiens
<400> 554
cagtggccga ggagcccttc ac 22

<210> 555
<211> 23
<212> DNA
<213> Homo sapiens
<400> 555
agtactatga cccgacggat gag 23

<210> 556
<211> 21
<212> DNA
<213> Homo sapiens

<400> 556
tcagccgctc cttaggtagg t 21

<210> 557
<211> 24
<212> DNA
<213> Homo sapiens
<400> 557
caccagccac tttgctaatt tctt 24

<210> 558
<211> 24
<212> DNA
<213> Homo sapiens
<400> 558
gaacgatgat cttaaaggca caaa 24

<210> 559
<211> 25
<212> DNA
<213> Homo sapiens
<400> 559
tcttgctgca atgtaaatct gctat 25

<210> 560
<211> 26
<212> DNA
<213> Homo sapiens
<400> 560
agaaaaagag cttcccctaa cctggg 26

<210> 561
<211> 20
<212> DNA
<213> Homo sapiens
<400> 561
tgggttgaac aagccacgtt 20

<210> 562
<211> 23
<212> DNA
<213> Homo sapiens
<400> 562
gtcgtgggat ttactctgca aca 23

<210> 563
<211> 30
<212> DNA
<213> Homo sapiens
<400> 563
taagtatccc tatttcttaa gttacgagga 30

<210> 564
<211> 23
<212> DNA
<213> Homo sapiens
<400> 564
aatgttgaga caccgttttg ctt 23

<210> 565
<211> 31
<212> DNA
<213> Homo sapiens
<400> 565
gtagagtcaa ctaaagatca aaatgtgaaa g 31

<210> 566
<211> 25
<212> DNA

<213> Homo sapiens
<400> 566
atcaccttcc cccaagatta cctga 25

<210> 567
<211> 19
<212> DNA
<213> Homo sapiens
<400> 567
ccctttccca cacccactt 19

<210> 568
<211> 19
<212> DNA
<213> Homo sapiens
<400> 568
gggatggtgc aagctgaca 19

<210> 569
<211> 26
<212> DNA
<213> Homo sapiens
<400> 569
ttgaaatctc agctatgcag atgttc 26

<210> 570
<211> 21
<212> DNA
<213> Homo sapiens
<400> 570
tcctgatggc tatccgagat g 21

<210> 571
<211> 21
<212> DNA
<213> Homo sapiens
<400> 571
cctcaacatt aacccgatcc a 21

## Description

### TECHNICAL FIELD OF THE INVENTION

The present invention relates to methods and compositions for the prediction of therapy outcome (e.g. tumor response to therapy), diagnosis, prognosis, prevention and treatment of neoplastic diseases. Cancer cells display a specific pattern of gene expression related to their morphological type, state of progression, acquirement of genomic alterations, point mutations in critical genes such as gatekeepers and tumor suppressors or due to the dependency of external signals such as growth factors, hormones or other secondary messengers.

The invention discloses genes which show an altered expression in a particular neoplastic tissue compared to the corresponding healthy tissue or to other neoplastic lesions unresponsive to a given chemotherapy. They are useful as diagnostic markers and could be also regarded as therapeutically targets. Methods are disclosed for predicting, diagnosing and prognosing as well as preventing and treating neoplastic disease. The genes disclosed in this invention have been identified in breast cancers but are predictable of outcome to a certain therapy regimen and therefor they are also relevant for other types of cancers in tissues other than breast.

### BACKGROUND OF THE INVENTION AND PRIOR ART

Cancer is the second leading cause of death in the United States after cardiovascular disease. One in three Americans will develop cancer in his or her lifetime, and one of every four Americans will die of cancer. More specifically breast cancer claims the lives of approximately 40,000 women and is diagnosed in approximately 200,000 women annually in the United States alone. Cancer are classified based on different parameters, such as tumor size, invasion status, involvement of lymph notes, metastasis, histolopathology, imunohistochemical markers, and molecular markers (WHO. International Classification of diseases (1); Sabin and Wittekind, 1997 (2)). With the recent advances in gene chip technology, researchers are increasingly focusing on the categorization of tumors based on the distinct expression of marker genes Sorlie et al., 2001 (3): van 't Veer et al., 2002 (4).

Chemotherapy remains a mainstay in therapeutic regimens offered to patients with breast cancer, particularly those who have cancer that has metastasized from its site of origin (Perez, 1999, (5)). There are several chemotherapeutic agents that have demonstrated activity in the treatment of breast cancer and research is continuously in an attempt to determine optimal drugs and regimens. However, different patients tend to respond differently to the same therapeutic regimen. Currently, the individuals response to certain therapy can only be assessed statistically, based on data of former clinical studies. There are still a great number of patients who will not benefit from a systemic chemotherapy. Especially, breast cancers are very heterogeneous in their aggressiveness and treatment response. They contain different genetic mutations and variations affecting growths characteristic and sensitivity to several drugs. Identification of each tumor's molecular fingerprint, then, could help to segregate patients who have particularly aggressive tumors or who need to be treated with specific beneficial therapies. As research involving genetics and associated responses to treatment matures, standard practice will undoubtedly become more individualized, enabling physicians to provide specific treatment regimens matched with a tumor's genetic profiles to ensure optimal outcomes.

### SUMMARY OF THE INVENTION

The present invention relates to the identification of 185 human genes being differentially expressed in neoplastic tissue resulting in an altered clinical behavior of a neoplastic lesion. The differential expression of these 185 genes is not limited to a specific neoplastic lesion in a certain tissue of the human body.

In preferred embodiments of this invention the neoplastic lesion, of which these 185 genes are altered in their expression is a cancer of the human breast. This cancer is not limited to females and may also be diagnosed and analyzed in males.

The invention relates to various methods, reagents and kits for diagnosing, staging, prognosis, monitoring and therapy of breast cancer. "Breast cancer" as used herein includes carcinomas, (e.g., carcinoma in situ, invasive carcinoma, metastatic carcinoma) and pre-malignant conditions, neomorphic changes independent of their histological origin (e.g. ductal, lobular, medullary, mixed origin). The compositions, methods, and kits of the present invention comprise comparing the level of mRNA expression of a single or plurality (e.g. 2, 5, 10, or 50 or more) of genes (hereinafter "marker genes", listed in Table 1a and 1b, SEQ ID NO:1 to 165 and 472 to 491, the respective polypeptide sequences coded by them are numerated SEQ ID NO:166 to 330 and 492 to 511, see also Table 1a and 1b) in a patient sample, and the average level of expression of the marker gene(s) in a sample from a control subject (e.g., a human subject without breast cancer). A preferred sub-set of marker genes representing a specific test composition or kit is listed in Table 2.

The invention relates further to various compositions, methods, reagents and kits, for prediction of clinically measurable tumor therapy response to a given breast cancer therapy. The compositions, methods, and kits of the present invention comprise comparing the level of mRNA expression of a single or plurality (e.g. 2, 5, 10, or 50 or more) of breast cancer marker genes in an unclassified patient sample, and the average level of expression of the marker gene(s) in a sample cohort comprising patient responding in different intensity to an administered breast cancer therapy. In preferred embodiments of this invention the specific expression of the marker genes can be utilized for discrimination of responders and non-responders to an anthracycline based (e.g. polychemotherapies with epirubicin or doxorubicin) chemo-therapeutic intervention.

In further preferred embodiments, the control level of mRNA expression is the average level of expression of the marker gene(s) in samples from several (e.g., 2, 3, 4, 5, 8, 10, 12, 15, 20, 30 or 50) control subjects. These control subjects may either be not affected by breast cancer or be identified and classified by their clinical response prior to the determination of their individual expression profile.

As elaborated below, a significant change in the level of expression of one or more of the marker genes (set of marker genes) in the patient sample relative to the control level provides significant information regarding the patient's breast cancer status and responsiveness to chemotherapy. In the compositions, methods, and kits of the present invention the marker genes listed in Table 1a and 1b may also be used in combination with well known breast cancer marker genes (e.g. CEA, mammaglobin, or CA 15-3)

According to the invention, the marker gene(s) and marker gene sets are selected such that the positive predictive value of the compositions, methods, and kits of the invention is at least about 10%, preferably about 25%, more preferably about 50% and most preferably about 90%. Also preferred for use in the compositions, methods, and kits of the invention are marker gene(s) and sets that are differentially expressed, as compared to normal breast cells, by at least the minimal mean differential expression factor presented in Table 3, in at least about 20%, more preferably about 50% and most preferably about 75% of any of the following conditions: stage 0 breast cancer patients, stage I breast cancer patients, stage II breast cancer patients, stage III breast cancer patients, stage IV breast cancer patients, grade I breast cancer patients, grade II breast cancer patients, grade III breast cancer patients, malignant breast cancer patients, patients with primary carcinomas of the breast, and all other types of cancers, malignancies and transformations associated with the breast.

The detection of marker gene expression is not limited to the detection within a primary, secondary or metastatic lesion of breast cancer patients, and may also be detected in lymphnodes affected by breast cancer cells or minimal residual disease cells either locally deposited (e.g. bone marrow, liver, kidney) or freely floating throughout the patients body.

In one embodiment of the compositions, methods, reagents and kits of the present invention, the sample to be analyzed is tissue material from neoplastic lesion taken by aspiration or punctuation, excision or by any other surgical method leading to biopsy or resected cellular material. In one embodiment of the compositions, methods, and kits of the present invention, the sample comprises cells obtained from the patient. The cells may be found in a breast cell "smear" collected, for example, by a nipple aspiration, ductal lavarge, fine needle biopsy or from provoked or spontaneous nipple discharge. In another embodiment, the sample is a body fluid. Such fluids include, for example, blood fluids, lymph, ascitic fluids, gynecological fluids, or urine but not limited to these fluids.

In accordance with the compositions, methods, and kits of the present invention the determination of gene expression is not limited to any specific method or to the detection of mRNA. The presence and/or level of expression of the marker gene in a sample can be assessed, for example, by measuring and/or quantifying of:

1) a protein encoded by the marker gene in Table 1a and 1b (SEQ ID NO:1 to 165 and 472 to 491)or a polypeptide comprising a polypeptide selected from SEQ ID NO: 166 to 330 and 492 to 511 or a polypeptide resulting from processing or degradation of the protein (e.g. using a reagent, such as an antibody, an antibody derivative, or an antibody fragment, which binds specifically with the protein or polypeptide)

2) a metabolite which is produced directly (i.e., catalyzed) or indirectly by a protein encoded by the marker gene in Table 1a and 1b (SEQ ID NO: 1 to 165 and 472 to 491)or by a polypeptide comprising a polypeptide selected from SEQ ID NO:166 to 330 and 492 to 511

3) a RNA transcript (e.g., mRNA, hnRNA) encoded by the marker gene in Table 1a and 1b, or a fragment of the RNA transcript (e.g. by contacting a mixture of RNA transcripts obtained from the sample or cDNA prepared from the transcripts with a substrate having nucleic acid comprising a sequence of one or more of the marker genes listed within Table 1a and 1b fixed thereto at selected positions). The mRNA expression of these genes can be detected e.g. with DNA-microarrays as provided by Affymetrix Inc. or other manufacturers. U.S. Pat. No. 5,556,752. In a further embodiment the expression of these genes can be detected with bead based direct fluorescent readout techniques such as provided by Luminex Inc. PCT No. WO 97/14028.

In one aspect, the present invention provides a composition, method, and kit of assessing whether a patient is afflicted with breast cancer (e.g., new detection or "screening", detection of recurrence, reflex testing, especially in patients having an enhanced risk of developing breast cancer (e.g., patients having a familial history of breast cancer and patients identified as having a mutant oncogene). For this purpose the composition, method, and kit comprises comparing:

a) the level of expression of a single or plurality of marker genes in a patient sample, wherein at least one (e.g. 2, 5, 10, or 50 or more) of the marker genes is selected from the marker genes of Table 1a and 1b and

b) the normal level of expression of the marker gene in a control subject without breast cancer.

A significant increase as well as decrease in the level of expression of the selected marker genes (e.g. 2, 5, 10, or 50 or more) in the patient sample relative to each marker gene's normal level of expression is an indication that the patient is afflicted with breast cancer.

The composition, method, and kit of the present invention is also useful for prognosing the progression or the outcome of the malignant neoplasia. For this purpose the composition, method, and kit comprises comparing

a) the level of expression of a single or plurality of marker genes in a patient sample, wherein at least one (e.g. 2, 5, 10, or 50 or more) of the marker genes is selected from the marker genes of Table 1a and 1b

b) a control pattern of expression of these marker genes.

The composition, method, and kit of the present invention is particularly useful for identifying patients who will respond to a certain chemotherapy. For this purpose the composition, method, and kit comprises comparing

a) the level of expression of a single or plurality of marker genes in a patient sample, wherein at least one (e.g. 2, 5, 10, or 50 or more) of the marker genes is selected from the marker genes of Table 1a and 1b and

b) the level of expression of the marker gene in a control subject. The control subject may either be not affected by breast cancer or be identified and classified by their clinical response to the particular chemotherapy.

In another aspect, the invention provides a composition, method, and kit of assessing the efficacy of a therapy for inhibiting breast cancer in a patient. This composition, method, and kit comprises comparing:

a) expression of a single or plurality of marker genes in a first, sample obtained from the patient prior to any treatment of the patient, wherein at least one of the marker genes is selected from the marker genes listed within Table 1a and 1b and

b) expression of the marker gene in a second sample obtained from the patient following at least one dose of the therapy.

It will be appreciated that in this composition, method, and kit the "therapy" may be any therapy for treating breast cancer including, but not limited to, chemotherapy, anti-hormonal therapy, directed antibody therapy, radiation therapy and surgical removal of tissue, e.g., a breast tumor. Thus, the compositions, methods, and kits of the invention may be used to evaluate a patient before, during and after therapy, for example, to evaluate the reduction in tumor burden.

In a further aspect, the present invention provides a composition, method, and kit for monitoring the progression of breast cancer in a patient. This composition, method, and kit comprising:

a) detecting in a patient sample at a first time point, the expression of a single or plurality of marker genes, wherein at least one of the marker genes is selected from the marker genes listed in Table 1a and 1b

b) repeating step a) at a subsequent time point in time; and

c) comparing the level of expression of each marker gene detected in steps a) and b), and therefrom monitoring the progression of breast cancer in the patient.

In another aspect, the invention provides a composition, method, and kit for *in vitro* selection of a therapy regime (e.g. the kind of chemotherapeutical argents) for inhibiting breast cancer in a patient. This composition, method, and kit comprises the steps of:

a) obtaining a sample comprising cancer cells from the patient;

b) separately maintaining aliquots of the sample in the presence of a diverse test compositions;

c) comparing expression of a single or plurality of marker genes, selected from the marker genes listed in Table 1a and 1b;

in each of the aliquots; and

d) selecting one of the test compositions which induces a lower level of expression of genes from SEQ ID 11, 17, 22, 25, 31, 36, 48, 49, 57, 83, 107, 108, 112, and 159 and/or a higher level of expression of genes from SEQ ID 24, 47, 54, 58, 59, 60, 67, 79, 80, 88, 114, 118, 135, and 141 in the aliquot containing that test composition, relative to the level of expression of each marker gene in the aliquots containing the other test compositions.

The invention further provides a composition, method, and kit of assessing the carcinogenic potential of a certain biological or chemical compound. This composition, method, and kit comprises the steps of:

a) maintaining separate aliquots of breast cells in the presence and absence of the test compound; and

b) comparing expression of a singe or plurality of marker genes in each of the aliquots,

wherein at least one of the genes is selected from the marker genes listed within Table 1a and 1b, A significant increase in the level of expression of genes from SEQC ID 19, 23, 36, 45, 62, 74, 81, 96, 103, 106, 107, 112, 113, and 132 and/or a significant decrease of genes from SEQ ID 22, 25, 31, 40, 43, 47, 55, 57, 59, 60, 108, 119, 121, 124, 154, 156, 157, 158, 159, 160, 162, and 164 in the aliquot maintained in the presence of (or exposed to) the test compound, relative to the level of expression of each marker gene in the aliquot maintained in the absence of the test compound, is an indication that the test compound possesses breast carcinogenic potential.

The invention further provides a composition, method, and kit of treating a patient afflicted with breast cancer. This composition, method, and kit comprises providing to cells of the patient an antisense oligonucleotide complementary to a polynucleotide sequence of a marker gene listed within Table 1a and 1b

The invention additionally provides a composition, method, and kit of inhibiting breast cancer cells in a patient at risk for developing breast cancer. This composition, method, and kit comprises inhibiting expression of a marker gene listed in Table 1a and 1b.

In yet another embodiment the invention provides compositions, methods, and kits of screening for agents which regulate the activity of a polypeptide comprising a polypeptide selected from SEQ ID NO: 166 to 330 and 492 to 511 . A test compound is contacted with the particular polypeptide. Binding of the test compound to the polypeptide is detected. A test compound which binds to the polypeptide is thereby identified as a potential therapeutic agent for the treatment of malignant neoplasia and more particularly breast cancer.

In even another embodiment the invention provides another composition, method, and kit of screening for agents which regulate the activity of a polypeptide comprising a polypeptide selected from SEQ ID NO: 166 to 330 and 492 to 511. A test compound is contacted with the particular polypeptide. A biological activity mediated by the polypeptide is detected. A test compound which decreases the biological activity is thereby identified as a potential therapeutic agent for decreasing the activity of the particular polypeptide in malignant neoplasia and especially in breast cancer A test compound which increases the biological activity is thereby identified as a potential therapeutic agent for increasing the activity of the particular polypeptide in malignant neoplasia and especially in breast cancer

The invention thus provides polypeptides selected from one of the polypeptides with SEQ ID NO: 166 to 330 and 492 to 511 which can be used to identify compounds which may act, for example, as regulators or modulators such as agonists and antagonists, partial agonists, inverse agonists, activators, co-activators and inhibitors of the polypeptide comprising a polypeptide selected from SEQ ID NO: 166 to 330 and 492 to 511 Accordingly, the invention provides reagents and compositions, methods, and kits for regulating a polypeptide comprising a polypeptide selected from SEQ ID NO: 166 to 330 and 492 to 511 in malignant neoplasia and more particularly breast cancer. The regulation can be an up- or down regulation. Reagents that modulate the expression, stability or amount of a polynucleotide listed in Table 1a and 1b (SEQ ID NO: 1 to 165 and 472 to 491 or the activity of the polypeptide comprising a polypeptide selected from SEQ ID NO: 166 to 330 and 492 to 511 can be a protein, a peptide, a peptidomimetic, a nucleic acid, a nucleic acid analogue (e.g. peptide nucleic acid, locked nucleic acid) or a small molecule. Compositions, methods, and kits that modulate the expression, stability or amount of a polynucleotide comprising a polynucleotide selected from SEQ ID NO:

1 to 165 and 472 to 491 (listed in Table 1a and 1b) or the activity of the polypeptide comprising a polypeptide selected from SEQ ID NO: 166 to 330 and 492 to 511 (Table 1) can be gene replacement therapies, antisense, ribozyme and triplex nucleic acid approaches.

The invention further provides a composition, method, and kit of making an isolated hybridoma which produces an antibody useful for assessing whether a patient is afflicted with breast cancer. The composition, method, and kit comprises isolating a protein encoded by a marker gene listed within Table 1a and 1b or a polypeptide fragment of the protein, immunizing a mammal using the isolated protein or polypeptide fragment, isolating splenocytes from the immunized mammal, fusing the isolated splenocytes with an immortalized cell line to form hybridomas, and screening individual hybridomas for production of an antibody which specifically binds with the protein or polypeptide fragment to isolate the hybridoma. The invention also includes an antibody produced by this method. Such antibodies specifically bind to a full-length or partial polypeptide comprising a polypeptide selected from SEQ ID NO: 166 to 330 and 492 to 511 (listed in Table 1a and 1b) for use in prediction, prevention, diagnosis, prognosis and treatment of malignant neoplasia and breast cancer in particular.

Yet another embodiment of the invention is the use of a reagent which specifically binds to a polynucleotide comprising a polynucleotide selected from SEQ ID NO: 1 to 165 and 472 to 491 or to a polypeptide comprising a polypeptide selected from SEQ ID NO: 166 to 330 and 492 to 511 (listed in Table 1a and 1b) in the preparation of a medicament for the treatment of malignant neoplasia and breast cancer in particular.

Still another embodiment is the use of a reagent that modulates the activity or stability of a polypeptide comprising a polypeptide selected from SEQ ID NO: 166 to 330 and 492 to 511 (Table 1a and 1b) or the expression, amount or stability of a polynucleotide comprising a polynucleotide selected from SEQ ID NO: 1 to 165 and 472 to 491 (Table 1a and 1b) in the preparation of a medicament for the treatment of malignant neoplasia and breast cancer in particular.

Still another embodiment of the invention is a pharmaceutical composition which includes a reagent which specifically binds to a polynucleotide comprising a polynucleotide selected from SEQ ID NO: 1 to165 (Table1) or a polypeptide comprising a polypeptide selected from SEQ ID NO: 166 to 300 , and a pharmaceutically acceptable carrier.

A further embodiment of the invention is a pharmaceutical composition comprising a polynucleotide including a sequence which hybridizes under stringent conditions to a polynucleotide comprising a polynucleotide selected from SEQ ID NO: 1 to 165 and 472 to 491 and encoding a polypeptide exhibiting the same biological function as given for the respective polynucleotide in Table 1a and 1b or 4, or encoding a polypeptide comprising a polypeptide selected from SEQ ID NO: 166 to 330 and 492 to 511. Pharmaceutical compositions, useful in the present invention may further include fusion proteins comprising a polypeptide comprising a polynucleotide selected from SEQ ID NO: 1 to 165 and 472 to 491, or a fragment thereof, antibodies, or antibody fragments

The invention also provides various kits. Such kit comprises reagents for assessing expression of a single or a plurality of genes selected from the marker genes listed in Table 1a and 1b or selected from the sub-set of genes listed in Table 2.

In one aspect, the invention provides a kit for assessing whether a patient is afflicted with breast cancer.

In another aspect, the invention provides a kit for assessing the suitability of each of a plurality of compounds for inhibiting a breast cancer in a patient. The kit comprises reagents for assessing expression of a marker gene listed within Table 1a and 1b, or reagents for assessing the expression of each marker gene of a marker gene set listed in Table 2. The kit may also comprise a plurality of compounds.

In an additional aspect, the invention provides a kit for assessing the presence of breast cancer cells. This kit comprises an antibody, wherein the antibody binds specifically with a protein encoded by a marker gene listed within Table 1a and 1b or polypeptide fragment of the protein. The kit may also comprise a plurality of antibodies, wherein the plurality binds specifically with-the protein encoded by each marker gene of a marker gene set listed in Table 2.

In yet another aspect, the invention provides a kit for assessing the presence of breast cancer cells, wherein the kit comprises a nucleic acid probe. The probe hybridizes specifically with a RNA transcript of a marker gene listed within Table 1a and 1b or cDNA of the transcript. The kit may also comprise a plurality of probes, wherein each of the probes hybridizes specifically with a RNA transcript of one of the marker genes of a marker gene set listed in Table 2.

It will be appreciated that the compositions, methods, and kits of the present invention may also include known cancer marker genes including known breast cancer marker genes. It will further be appreciated that the compositions, methods, and kits may be used to identify cancers other than breast cancer.

## DETAILED DESCRIPTION OF THE INVENTION

### *DEFINITIONS*

"Differential expression", or "expression" as used herein, refers to both quantitative as well as qualitative differences in the genes' expression patterns depending on differential development, different genetic background of tumor cells and/or reaction to the tissue environment of the tumor. Differentially expressed genes may represent "marker genes," and/or "target genes". The expression pattern of a differentially expressed gene disclosed herein may be utilized as part of a prognostic or diagnostic breast cancer evaluation., Alternatively, a differentially expressed gene disclosed herein may be used in methods for identifying reagents and compounds and uses of these reagents and compounds for the treatment of breast cancer as well as methods of treatment. The differential regulation of the gene is not limited to a specific cancer cell type or clone, but rather displays the interplay of cancer cells, muscle cells, stromal cells, connective tissue cells, other epithelial cells, endothelial cells and blood vesses1 as well as cells of the immune system (e.g. lymphocytes, macrophages, killer cells).

"Biological activity" or "bioactivity" or "activity" or "biological function", which are used interchangeably, herein mean an effector or antigenic function that is directly or indirectly performed by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers a biological molecule, e.g., a nucleic acid, peptide, hormone, etc., whose presence or concentration can be detected and correlated with a known condition, such as a disease state.

The term "marker gene," as used herein, refers to a differentially expressed gene which expression pattern may be utilized as part of predictive, prognostic or diagnostic process in malignant neoplasia or breast cancer evaluation, or which, alternatively, may be used in methods for identifying compounds useful for the treatment or prevention of malignant neoplasia and breast cancer in particular. A marker gene may also have the characteristics of a target gene.

"Target gene", as used herein, refers to a differentially expressed gene involved in breast cancer in a manner by which modulation of the level of target gene expression or of target gene product activity may act to ameliorate symptoms of malignant neoplasia and breast cancer in particular. A target gene may also have the characteristics of a marker gene.

The term "neoplastic lesion" or " neoplastic disease" or "neoplasia" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin). The term "cancer" is not limited to any stage, grade, histomorphological feature, invasiveness, agressivity or malignancie of an affected tissue or cell aggregation. In particular stage 0 breast cancer, stage I breast cancer, stage II breast cancer, stage III breast cancer, stage IV breast cancer, grade I breast cancer, grade II breast cancer, grade III breast cancer, malignant breast cancer, primary carcinomas of the breast, and all other types of cancers, malignancies and transformations associated with the breast are included. The terms "neoplastic lesion" or " neoplastic disease" or "neoplasia" or "cancer" are not limited to any tissue or cell type they also include primary, secondary or metastatic lesion of cancer patients, and also comprises lymphnodes affected by cancer cells or minimal residual disease cells either locally deposited (e.g. bone marrow, liver, kidney) or freely floating throughout the patients body.

The term "biological sample", as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, cell-containing bodyfluids, free floating nucleic acids, urine, peritoneal fluid, and pleural fluid, or cells therefrom. Biological samples may also include sections of tissues such as frozen or fixed sections taken for histological purposes. A biological sample to be analyzed is tissue material from neoplastic lesion taken by aspiration or punctuation, excision or by any other surgical method leading to biopsy or resected cellular material. Such biological sample may comprises cells obtained from a patient. The cells may be found in a breast cell "smear" collected, for example, by a nipple aspiration, ductal lavarge, fine needle biopsy or from provoked or spontaneous nipple discharge. In another embodiment, the sample is a body fluid. Such fluids include, for example, blood fluids, lymph, ascitic fluids, gynecological fluids, or urine but not limited to these fluids.

The term "therapy modality", "therapy mode", "regimen" or "chemo regimen" as well as "therapy regime" refers to a timely sequential or simultaneous administration of anti tumor, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in dose of the single agent, timeframe of application and frequency of administration within a defined therapy window. Currently various combinations of various drugs and/or physical methods, and various schedules are under investigation.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays

wherein the probes are oligonucleotides are referred to as "oligonucleotide arrays" or "oligonucleotide chips." A "microarray," herein also refers to a "biochip" or "biological chip", an array of regions having a density of discrete regions of at least about 100/$cm^2$, and preferably at least about 1000/$cm^2$. The regions in a microarray have typical dimensions, e.g., diameters, in the range of between about 10-250 $\mu$m, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

The term "agonist", as used herein, is meant to refer to an agent that mimics or upregulates (e.g., potentiates or supplements) the bioactivity of a protein. An agonist can be a wild-type protein or derivative thereof having at least one bioactivity of the wild-type protein. An agonist can also be a compound that upregulates expression of a gene or which increases at least one bioactivity of a protein. An agonist can also be a compound which increases the interaction of a polypeptide with another molecule, e.g., a target peptide or nucleic acid.

The term "antagonist" as used herein is meant to refer to an agent that downregulates (e.g., suppresses or inhibits) at least one bioactivity of a protein. An antagonist can be a compound which inhibits or decreases the interaction between a protein and another molecule, e.g., a target peptide, a ligand or an enzyme substrate. An antagonist can also be a compound that downregulates expression of a gene or which reduces the amount of expressed protein present.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The terms "modulated" or "modulation" or "regulated" or "regulation" and "differentially regulated" as used herein refer to both upregulation (i.e., activation or stimulation (e.g., by agonizing or potentiating) and down regulation [i.e., inhibition or suppression (e.g., by antagonizing, decreasing or inhibiting)].

"Transcriptional regulatory unit" refers to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally occurring forms of the polypeptide.

The term "derivative" refers to the chemical modification of a polypeptide sequence, or a polynucleotide sequence. Chemical modifications of a polynucleotide sequence can include, for example, replacement of hydrogen by an alkyl, acyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

The term "nucleotide analog" refers to oligomers or polymers being at least in one feature different from naturally occurring nucleotides, oligonucleotides or polynucleotides, but exhibiting functional features of the respective naturally occurring nucleotides (e.g. base paring, hybridization, coding information) and that can be used for said compositions. The nucleotide analogs can consist of non-naturally occurring bases or polymer backbones, examples of which are LNAs, PNAs and Morpholinos. The nucleotide analog has at least one molecule different from its naturally occurring counterpart or equivalent.

"BREAST CANCER GENES" or "BREAST CANCER GENE" as used herein refers to the polynucleotides of SEQ ID NO:1 to 165 and 472 to 491 (listed in Table 1a and 1b), as well as derivatives, fragments, analogs and homologues thereof, the polypeptides encoded thereby, (SEQ ID NO:166 to 330 and 492 to 511, see Table 1) as well as derivatives, fragments, analogs and homologues thereof and the corresponding genomic transcription units which can be derived or identified with standard techniques well known in the art using the information disclosed in Tables 1 to 5. The Genename, Reference Sequence, unique Gene-identifier, and the Locuslink ID numbers of the polynucleotide sequences of the SEQ ID NO: 1 to 65 and the polypeptides of the SEQ ID NO: 166 to 330 and 492 to 511 are shown in Table 1a and 1b, the gene description, gene function and subcellelar localization is given in Tables 4a and 4b.

The term "chromosomal region" as used herein refers to a consecutive DNA stretch on a chromosome which can be defined by cytogenetic or other genetic markers such as e.g. restriction length polymorphisms (RFLPs), single nucleotide polymorphisms (SNPs), expressed sequence tags (ESTs), sequence tagged sites (STSs), microsatellites, variable number of tandem repeats (VNTRs) and genes. Typically a chromosomal region consists of up to 2 Megabases (MB), up to 4 MB, up to 6 MB, up to 8 MB, up to 10 MB, up to 20 MB or even more MB.

The term "kit" as used herein refers to any manufacture (e.g. a diagnostic or research product) comprising at least one reagent, e.g. a probe, for specifically detecting the expression of at least one marker gene disclosed in the invention, in particular of those genes listed in Table 2, whereas the manufacture is being sold, distributed, and/or promoted as a unit for performing the methods of the present invention. The genes, primer and probes listed in Table 2 and 5 or any combination of at least two of them, regard as one single test for the purposes, methods and disclosures of this invention. Also reagents (e.g. immunoassays) to detect the presence, the stability, activity, complexity of the respective marker gene products comprising polypeptides selected from SEQ ID NO:166 to 330 and 492 to 511 regard as components of the kit. In addition, any combination of nucleic acid and protein detection as disclosed in the invention are regard as a kit.

The present invention provides polynucleotide sequences and proteins encoded thereby, as well as probes derived from the polynucleotide sequences, antibodies directed to the encoded proteins, and predictive, preventive, diagnostic, prognostic and therapeutic uses for individuals which are at risk for or which have malignant neoplasia and breast cancer in particular. The sequences disclosure herein have been found to be differentially expressed in samples from breast cancer.

The present invention is based on the identification of 185 genes that are differentially regulated (up- or down regulated) in tumor biopsies of patients with clinical evidence of breast cancer.. The characterization of the co-expression of some of these genes provides newly identified roles in breast cancer. The gene names, the database accession numbers (Genename, Reference Sequence, unique Gene-identifier, and the Locuslink ID numbers) as well as the putative or known functions of the encoded proteins and their subcellular localization are given in Tables 1 to 4a and 4b. The primer sequences used for the gene amplification and hybridization probes are shown in Table 5.

The present invention relates to:

1. A method for characterizing (preferably *ex vivo*) the state of a neoplastic disease in a subject, comprising

(i) determining the pattern of expression levels of at least 6, 8, 10, 15, 20, 30, or 47 marker genes, comprised in a group of marker genes consisting of SEQ ID NO: 1 to 165 and 472 to 491, in a biological sample from said subject,

(ii) comparing the pattern of expression levels determined in (i) with one or several reference pattern(s) of expression levels,

(iii) characterizing the state of said neoplastic disease in said subject from the outcome of the comparison in step (ii).

2. A method for detection, diagnosis, screening, monitoring, and/or prognosis of a neoplastic disease in a subject, (preferably *ex vivo*) comprising

(i) determining the pattern of expression levels of at least 1, 2, 3, 5, 10, 15, 20, 30, or 47 marker genes, comprised in a group of marker genes consisting of SEQ ID NOs:1 to 17, 19 to 33, 35 to 50, 52 to 64, 66 to 85, 88 to 91, and 93 to 165 and 472 to 491 in biological samples from said subject,

(ii) comparing the pattern of expression levels determined in (i) with one or several reference pattern(s) of expression levels,

(iii) detecting, diagnosing, screening, monitoring, and/or prognosing said neoplastic disease in said subject from the outcome of the comparison in step (ii).

Determination of an expression level can comprise a quantitatification of the expression level and/or a purely qualitative determination of the expression level.

A "pattern of expression levels" of a single gene is to be understood as the expression level of said gene as determined by suitable methods.

Nucleic acid molecules, referred to with a specific SEQ ID NO, within the meaning of the invention, are to be understood as comprising also variants of said nucleic acid molecules, which can be derived from the original nucleic acid molecules by deletion, insertion or transposition of nucleotides, provided said variants still have an 80, 90, 95, or 99% sequence identity towards the original sequence. Preferrably the variants still have the same biological activity and/or function as have the original molecules.

It is obvious to the person skilled in the art that a reference to a nucleotide sequence is meant to comprise the reference to the associated protein sequence which is coded by said nucleotide sequence.

"% identity" of a first sequence towards a second sequence, within the meaning of the invention, means the % identity which is calculated as follows: First the optimal global alignment between the two sequences is determined with the CLUSTALW algorithm [Thomson JD, Higgins DG, Gibson TJ. 1994. ClustalW: Improving the sensitivity of progressive multiple sequence alignment through sequence weighting, positions-specific gap penalties and weight matrix choice. Nucleic Acids Res., 22: 4673-4680], Version 1.8, applying the following command line syntax: ./clustalw - infile=./infile.txt -output= -outorder=aligned -pwmatrix=gonnet -pwdnamatrix=clustalw -pwgapopen=10.0 -pwgapex=0.1 -matrix=gonnet -gapopen=10.0 -gapext=0.05 -gapdist=8- hgapresidues=GPSNDQERK -maxdiv=40. Implementations of the CLUSTAL W algorithm are readily available at numerous sites on the internet, including, e.g., http://www.ebi.ac.uk. Thereafter, the number of matches in the alignment is determined by counting the number of identical nucleotides (or amino acid residues) in aligned positions. Finally, the total number of matches is divided by the number of nucleotides (or amino acid residues) of the longer of the two sequences, and multiplied by 100 to yield the % identity of the first sequence towards the second sequence.

3. A method of count 1 or 2, wherein said method comprises multiple determinations of a pattern of expression levels, at different points in time, thereby allowing to monitor the development of said neoplastic disease in said subject.

4. A method of count 1, wherein said method comprises an estimation of the likelihood of success of a given mode of treatment for said neoplastic disease in said subject.

5. A method of count 1, wherein said method comprises an assessment of whether the subject is expected to respond or whether the subject is expected not to a given mode of treatment for said neoplastic disease.

The terms "to respond" or "not to respond" are to be understood in a qualitative and/or in a quantitative fashion. "To respond" and "not to respond" is to be assessed with regard to a suitable reference responses, such as, e.g., responses shown by "responders" and "not-responders" to a certain mode of treatment or modality of treatment.

6. A method of count 4 or 5, wherein a predictive algorithm is used.

Predictive algorithms, which are well known to a person skilled in the art of data analysis, are to be understood as being any kind of predictive algorithm known in the art. Preferred examples of such algorithms are, e.g., the SVM algorithm disclosed in Example 4.

7. A method of count 6, wherein the predictive algorithm is a Support Vector Machine.

Support Vector Machines are algorithms, well known to the person skilled in the art of data analysis. A Support Vector Machine algorithm is disclosed in Example 4.

8. A method of any of counts 4 to 7, wherein said given mode of treatment

(i) acts on cell proliferation, and/or

(ii) acts on cell survival, and/or

(iii) acts on cell motility, and/or

(iv) is an anthracycline based mode of treatment, and/or

(v) comprises administration of epirubicin and/or cyclophoshamid.

9. A method of treatment for a subject afflicted with a neoplastic disease, comprising

(i) identifying a promising mode of treatment with the method of count 4 or 5,

(ii) treating said neoplastic disease in said patient by the mode of treatment identified in step (i).

10. A method of screening for subjects afflicted with a neoplastic disease, wherein the method of count 1 or 2 is applied to a plurality of subjects.

11. A method of screening for substances and/or therapy modalities having curative effect on a neoplastic disease comprising

(i) obtaining a biological sample from a subject afflicted with said neoplastic disease,

(ii) assessing, from said biological sample, using the method of count 4 or 5, whether said subject is expected to respond to a given mode of treatment for said neoplastic disease,

(iii) if said subject is expected to respond to said given mode of treatment, incubating said biological sample with said substance under said therapy modalities,

(iv) observing changes in said biological sample triggered by said test substance under said therapy modalities,

(v) selecting or rejecting said test substance and/or said therapy modalities, based on the observation of changes in said biological sample under (iv).

Selecting specific biological samples of, e.g., good responders to a given threapy can help to identify novel substances and/or therapy modalities for the treatment of said specific neoplastic disease.

12. A method of screening for compounds having curative effect on a neoplastic disease comprising

(i) incubating biological samples or extracts of these with a test substance,

(ii) determining the pattern of expression levels of at least 1, 2, 3, 5, 10, 15, 20, 30, or 47 marker genes, comprised in a group of marker genes consisting of SEQ ID NO:1 to 17, 19 to 33, 35 to 50, 52 to 64, 66 to 85, 88 to 91, and 93 to 165 and 472 to 491 in said biological sample,

(iii) comparing the pattern of expression levels determined in (ii) with one or several reference pattern(s),

(iv) selecting or rejecting said test substance, based on the comparison performed under (iii).

13. A method of any of counts 1 to 12 wherein said marker genes are comprised in a group of marker genes listed in Table 2.

Marker genes listed in Table 2 are shown to be particularly informative with respect to assessing the propability of success of a certain mode of treatment for a given neoplastic disease. Marker genes of Table 2 are preferred marker genes, according to the invention.

14. A method of any of counts 1 to 13, wherein the expression level is determined

(i) with a hybridization based method, or

(ii) with a hybridization based method utilizing arrayed probes, or

(iii) with a hybridization based method utilizing individually labeled probes, or

(iv) by real time real time PCR, or

(v) by assessing the expression of polypeptides, proteins or derivatives thereof, or

(vi) by assessing the amount of polypeptides, proteins or derivatives thereof.

15. A method of any of counts 1 to 14, wherein the neoplastic disease is breast cancer.

The methods of the invention are preferably performed *ex vivo.* More preferably, methods of the invention are performed ex vivo on samples that are already available or can be obtained without intervention of a physician or other medically trained personnel.

16. A kit comprising at least 6, 8, 10, 15, 20, 30, or 47 primer pairs and probes suitable for marker genes comprised in a group of marker genes consisting of

(i) SEQ ID NO:1 to SEQ ID NO:165, or
(ii) the marker genes listed in Table 2.

17. A kit comprising at least 6, 8, 10, 15, 20, 30, or 47 sets of individually labeled probes, each having a sequence comprised in a group of sequences consisting of SEQ ID NO:331 to SEQ ID NO:471.

18. A kit comprising at least 6, 8, 10, 15, 20, 30, or 47 sets of arrayed probes, each having a sequence comprised in a group of sequences consisting of SEQ ID NO:331 to SEQ ID NO:471.

*Biological relevance of the genes which are part of the invention*

Some of the genes listed in Table 1a and 1b represent biological, cellular processes and are characterized by similar regulation of genes. By the way of illustration but limited to the following examples a few characteristic genes from Table are described in later by greater detail:

MAD2L 1

The initiation of chromosome segregation at anaphase is linked by the spindle assembly checkpoint to the completion of chromosome-microtubule attachment during metaphase. To determine the function of the Mad2 protein during normal cell division, knock out experiments in mice were performed. These cells were unable to arrest in response to spindle disruption. At embryonic day 6.5, the cells of the epiblast began rapid cell division, and the absence of a checkpoint resulted in widespread chromosome missegregation and apoptosis. In contrast, the postmitotic trophoblast giant cells survived without Mad2. Thus, the spindle assembly checkpoint is required for accurate chromosome segregation in mitotic mouse cells and for embryonic viability, even in the absence of spindle damage.

Meiosis I nondisjunction in spindle checkpoint mutants could be prevented by delaying the onset of anaphase. In a recombinant-defective mutant, the checkpoint delayed the biochemical events of anaphase I, suggesting that chromosomes that are attached to microtubules but are not under tension can activate the spindle checkpoint. Spindle checkpoint mutants reduced the accuracy of chromosome segregation in meiosis I much more than that in meiosis II, suggesting that checkpoint defects may contribute to Down syndrome and possibly to the "chaotic" polyploidy observed in cancer.

IGFBP4

Seven structurally distinct insulin-like growth factor binding proteins have been isolated and their cDNAs cloned: IGFBP1, IGFBP2, IGFBP3, IGFBP4, IGFBP5, IGFBP6, and IGFBP7. The proteins display strong sequence homologies, suggesting that they are encoded by a closely related family of genes. The IGFBPs contain 3 structurally distinct domains each comprising approximately one-third of the molecule. The N-terminal domain 1 and the C-terminal domain 3 of the 6 human IGFBPs show moderate to high levels of sequence identity including 12 and 6 invariant cysteine residues in domains 1 and 3, respectively (IGFBP6 contains 10 cysteine residues in domain 1), and are thought to be the IGF binding domains. Domain 2 is defined primarily by a lack of sequence identity among the 6 IGFBPs and by a lack of cysteine residues, though it does contain 2 cysteines in IGFBP4. Domain 3 is homologous to the thyroglobulin type I repeat unit. Studies suggested that the primary effect of the proteins is the attenuation of IGF activity and suggested that they contribute to the control of IGF-mediated cell growth and metabolism

DDB2

In human cells, efficient global genomic repair of DNA damage induced by ultraviolet radiation requires the p53 tumor suppressor. The p48 gene is required for expression of an ultraviolet radiation-damaged DNA-binding activity and is disrupted by mutations in the subset of xeroderma pigmentosum group E cells that lack this activity, DDB-negative XPE. p48 mRNA levels are strongly depend on basal p53 expression and increase further after DNA damage in a p53-dependent manner. Furthermore, like p53 -/- cells, xeroderma pigmentosum group E cells are deficient in global genomic repair. These results identified p48 as a link between p53 and the nucleotide excision-repair apparatus.

UV-damaged DNA-binding activity (UV-DDB) is deficient in cell lines and primary tissues from rodents. Transfection of p48 conferred UV-DDB to hamster cells and enhanced removal of cyclobutane pyrimidine dimers (CPDs) from genomic DNA and from the nontranscribed strand of an expressed gene. Expression of p48 suppressed UV-induced mutations arising from the nontranscribed strand but had no effect on cellular UV sensitivity. The results defined the role of p48 in DNA repair, demonstrated the importance of CPDs in mutagenesis, and suggested how rodent models can be improved to better reflect cancer susceptibility in humans.

HSPA2

Several heat-shock protein genes are located in the major histocompatibility complex on chromosome 6, e.g., HSPA1 . However HSPA2 is located on 14q22-q24 . isolated The clone for HSPA2 is characterized by a single open reading frame of 1,917 basepairs that encodes a 639-amino acid protein with a predicted molecular weight of 70,030 Da. Analysis of the sequence indicated that HSPA2 is the human homolog of the murine Hsp70-2 gene with 91.7% identity in the nucleotide coding sequence and 98.2% in the corresponding amino acid sequence. HSPA2 has less amino acid homology to the other members of the human HSP70 gene family. HSPA2 is constitutively expressed in most tissues, with very high levels in testis and skeletal muscle. HSPA2 is expressed abundantly in muscle, heart, esophagus, and brain, and to a lesser extent in testis. A female homozygous knockout mice for Hsp70-2 undergo normal meiosis and is fertile. In contrast, homozygous male knockout mice lacked postmeiotic spermatids and mature sperm and were infertile. Hsp70-2 is normally associated with synaptonemal complexes in the nuclei of meiotic spermatocytes. In the male knockouts, these structures were abnormal by late prophase. One can observe also a large increase in spermatocyte apoptosis.

*Polynucleotides*

A "BREAST CANCER GENE" polynucleotide can be single- or double-stranded and comprises a coding sequence or the complement of a coding sequence for a "BREAST CANCER GENE" polypeptide. Degenerate nucleotide sequences encoding human "BREAST CANCER GENE" polypeptides, as well as homologous nucleotide sequences which are at least about 50, 55, 60, 65, 70, preferably about 75, 90, 96, or 98% identical to the nucleotide sequences of SEQ ID NO: 1 to 165 and 472 to 491 also are "BREAST CANCER GENE" polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologues, and variants of "BREAST CANCER GENE" polynucleotides which encode biologically active "BREAST CANCER GENE" polypeptides also are "BREAST CANCER GENE" polynucleotides.

*Preparation of Polynucleotides*

A naturally occurring "BREAST CANCER GENE" polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated "BREAST CANCER GENE" polynucleotides. For example, restriction enzymes and probes can be used to isolate polynucleotide fragments which comprises "BREAST CANCER GENE" nucleotide sequences. Isolated polynucleotides are in preparations which are free or at least 70, 80, or 90% free of other molecules. "BREAST CANCER GENE" cDNA molecules can be made with standard molecular biology techniques, using "BREAST CANCER GENE" mRNA as a template. Any RNA isolation technique which does not select against the isolation of mRNA may be utilized for the purification of such RNA samples. See, for example, Sambrook et al., 1989, (6); and Ausubel, F. M. et al., 1989, (7), both of which are incorporated herein by reference in their entirety. Additionally, large numbers of tissue samples may readily be processed using techniques well known to those of skill in the art, such as, for example, the single-step RNA isolation process of Chomczynski, P. (1989, U.S. Pat. No. 4,843,155), which is incorporated herein by reference in its entirety.

"BREAST CANCER GENE" cDNA molecules can thereafter be replicated using molecular biology techniques known in the art and disclosed in manuals such as Sambrook et al., 1989, (6) . An amplification technique, such as PCR, can be used to obtain additional copies of polynucleotides of the invention, using either human genomic DNA or cDNA as a template.

Alternatively, synthetic chemistry techniques can be used to synthesizes "BREAST CANCER GENE" polynucleotides. The degeneracy of the genetic code allows alternate nucleotide sequences to be synthesized which will encode a "BREAST CANCER GENE" polypeptide or a biologically active variant thereof.

*Identification of differential expression*

Transcripts within the collected RNA samples which represent RNA produced by differentially expressed genes may be identified by utilizing a variety of methods which are ell known to those of skill in the art. For example, differential screening [Tedder, T. F. et al., 1988, (8)], subtractive hybridization [Hedrick, S. M. et al., 1984, (9); Lee, S. W. et al., 1984, (10)], and, preferably, differential display (Liang, P., and Pardee, A. B., 1993, U.S. Pat. No. 5,262,311, which is incorporated herein by reference in its entirety), may be utilized to identify polynucleotide sequences derived from genes that are differentially expressed.

Differential screening involves the duplicate screening of a cDNA library in which one copy of the library is screened with a total cell cDNA probe corresponding to the mRNA population of one cell type while a duplicate copy of the cDNA library is screened with a total cDNA probe corresponding to the mRNA population of a second cell type. For example, one cDNA probe may correspond to a total cell cDNA probe of a cell type derived from a control subject, while the second cDNA probe may correspond to a total cell cDNA probe of the same cell type derived from an experimental subject. Those clones which hybridize to one probe but not to the other potentially represent clones derived from genes differentially expressed in the cell type of interest in control versus experimental subjects.

Subtractive hybridization techniques generally involve the isolation of mRNA taken from two different sources, e.g., control and experimental tissue, the hybridization of the mRNA or single-stranded cDNA reverse-transcribed from the isolated mRNA, and the removal of all hybridized, and therefore double-stranded, sequences. The remaining non-hybridized, single-stranded cDNAs, potentially represent clones derived from genes that are differentially expressed in the two mRNA sources. Such single-stranded cDNAs are then used as the starting material for the construction of a library comprising clones derived from differentially expressed genes.

The differential display technique describes a procedure, utilizing the well known polymerase chain reaction (PCR; the experimental embodiment set forth in Mullis, K. B., 1987, U.S. Pat. No. 4,683,202) which allows for the identification of sequences derived from genes which are differentially expressed. First, isolated RNA is reverse-transcribed into single-stranded cDNA, utilizing standard techniques which are well known to those of skill in the art. Primers for the reverse transcriptase reaction may include, but are not limited to, oligo dT-containing primers, preferably of the reverse primer type of oligonucleotide described below. Next, this technique uses pairs of PCR primers, as described below, which allow for the amplification of clones representing a random subset of the RNA transcripts present within any given cell. Utilizing different pairs of primers allows each of the mRNA transcripts present in a cell to be amplified. Among such amplified transcripts may be identified those which have been produced from differentially expressed genes.

The reverse oligonucleotide primer of the primer pairs may contain an oligo dT stretch of nucleotides, preferably eleven nucleotides long, at its 5' end, which hybridizes to the poly(A) tail of mRNA or to the complement of a cDNA reverse transcribed from an mRNA poly(A) tail. Second, in order to increase the specificity of the reverse primer, the primer may contain one or more, preferably two, additional nucleotides at its 3' end. Because, statistically, only a subset of the mRNA derived sequences present in the sample of interest will hybridize to such primers, the additional nucleotides allow the primers to amplify only a subset of the mRNA derived sequences present in the sample of interest. This is preferred in that it allows more accurate and complete visualization and characterization of each of the bands representing amplified sequences.

The forward primer may contain a nucleotide sequence expected, statistically, to have the ability to hybridize to cDNA sequences derived from the tissues of interest. The nucleotide sequence may be an arbitrary one, and the length of the forward oligonucleotide primer may range from about 9 to about 13 nucleotides, with about 10 nucleotides being preferred. Arbitrary primer sequences cause the lengths of the amplified partial cDNAs produced to be variable, thus allowing different clones to be separated by using standard denaturing sequencing gel electrophoresis. PCR reaction conditions should be chosen which optimize amplified product yield and specificity, and, additionally, produce amplified products of lengths which may be resolved utilizing standard gel electrophoresis techniques. Such reaction conditions are well known to those of skill in the art, and important reaction parameters include, for example, length and nucleotide sequence of oligonucleotide primers as discussed above, and annealing and elongation step temperatures and reaction times. The pattern of clones resulting from the reverse transcription and amplification of the mRNA of two different cell types is displayed via sequencing gel electrophoresis and compared. Differences in the two banding patterns indicate potentially differentially expressed genes.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' nontranscribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer; ABI), and the entire process from loading of samples to computer analysis and electronic data display can be computer controlled. Capillary electrophoresis is especially preferable for the sequencing of small pieces of DNA which might be present in limited amounts in a particular sample.

Once potentially differentially expressed gene sequences have been identified via bulk techniques such as, for example, those described above, the differential expression of such putatively differentially expressed genes should be corroborated. Corroboration may be accomplished via, for example, such well known techniques as Northern analysis and/or RT-PCR. Upon corroboration, the differentially expressed genes may be further characterized, and may be identified as target and/or marker genes, as discussed, below.

Also, amplified sequences of differentially expressed genes obtained through, for example, differential display may be used to isolate full length clones of the corresponding gene. The full length coding portion of the gene may readily be isolated, without undue experimentation, by molecular biological techniques well known in the art. For example, the isolated differentially expressed amplified fragment may be labeled and used to screen a cDNA library. Alternatively, the labeled fragment may be used to screen a genomic library.

An analysis of the tissue distribution of the mRNA produced by the identified genes may be conducted, utilizing standard techniques well known to those of skill in the art. Such techniques may include, for example, Northern analyses and RT-PCR. Such analyses provide information as to whether the identified genes are expressed in tissues expected to contribute to breast cancer. Such analyses may also provide quantitative information regarding steady state mRNA regulation, yielding data concerning which of the identified genes exhibits a high level of regulation in, preferably, tissues which may be expected to contribute to breast cancer.

Such analyses may also be performed on an isolated cell population of a particular cell type derived from a given tissue. Additionally, standard in situ hybridization techniques may be utilized to provide information regarding which cells within a given tissue express the identified gene. Such analyses may provide information regarding the biological function of an identified gene relative to breast cancer in instances wherein only a subset of the cells within the tissue is thought to be relevant to breast cancer.

*Extending Polynucleotides*

In one embodiment of such a procedure for the identification and cloning of full length gene sequences, RNA may be isolated, following standard procedures, from an appropriate tissue or cellular source. A reverse transcription reaction may then be performed on the RNA using an oligonucleotide primer complimentary to the mRNA that corresponds to the amplified fragment, for the priming of first strand synthesis. Because the primer is anti-parallel to the mRNA, extension will proceed toward the 5' end of the mRNA. The resulting RNA hybrid may then be "tailed" with guanines using a standard terminal transferase reaction, the hybrid may be digested with RNase H, and second strand synthesis may then be primed with a poly-C primer. Using the two primers, the 5' portion of the gene is amplified using PCR. Sequences obtained may then be isolated and recombined with previously isolated sequences to generate a full-length cDNA of the differentially expressed genes of the invention. For a review of cloning strategies and recombinant DNA techniques, see e.g., Sambrook et al., (6); and Ausubel et al., (7).

Various PCR-based methods can be used to extend the polynucleotide sequences disclosed herein to detect upstream sequences such as promoters and regulatory elements. For example, restriction site PCR uses universal primers to retrieve unknown sequence adjacent to a known locus [Sarkar, 1993, (11)]. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region [Triglia et al., 1988 ,(12)]. Primers can be designed using commercially available software, such as OLIGO 4.06 Primer Analysis software (National Biosciences Inc., Plymouth, Minn.), to be e.g. 2230 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA [Lagerstrom et al., 1991, (13))]. In this method, multiple restriction enzyme digestions and ligations also can be used to place an engineered double-stranded sequence into an unknown fragment of the DNA molecule before performing PCR.

Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

The sequences of the identified genes may be used, utilizing standard techniques, to place the genes onto genetic maps, e.g., mouse [Copeland & Jenkins, 1991, (14)] and human genetic maps [Cohen, et al., 1993 ,(15)]. Such mapping information may yield information regarding the genes' importance to human disease by, for example, identifying genes which map near genetic regions to which known genetic breast cancer tendencies map.

*Identification of Polynucleotide Variants and Homologues or splice Variants*

Variants and homologues of the "BREAST CANCER GENE" polynucleotides described above also are "BREAST CANCER GENE" polynucleotides. Typically, homologous "BREAST CANCER GENE" polynucleotide sequences can be identified by hybridization of candidate polynucleotides to known "BREAST CANCER GENE" polynucleotides under stringent conditions, as is known in the art. For example, using the following wash conditions: 2X SSC (0.3 M NaCl, 0.03 M sodium citrate, pH 7.0), 0.1% SDS, room temperature twice, 30 minutes each; then 2X SSC, 0.1% SDS, 50 EC once, 30 minutes; then 2X SSC, room temperature twice, 10 minutes each homologous sequences can be identified which contain at most about 25-30% basepair mismatches. More preferably, homologous polynucleotide strands contain 15-25% basepair mismatches, even more preferably 5-15% basepair mismatches.

Species homologues of the "BREAST CANCER GENE" polynucleotides disclosed herein also can be identified by making suitable probes or primers and screening cDNA expression libraries from other species, such as mice, monkeys, or yeast. Human variants of "BREAST CANCER GENE" polynucleotides can be identified, for example, by screening human cDNA expression libraries. It is well known that the $T_m$ of a double-stranded DNA decreases by 1-1.5˚C with every 1% decrease in homology [Bonner et al., 1973, (16)]. Variants of human "BREAST CANCER GENE" polynucleotides or "BREAST CANCER GENE" polynucleotides of other species can therefore be identified by hybridizing a putative homologous "BREAST CANCER GENE" polynucleotide with a polynucleotide having a nucleotide sequence of one of the sequences of the SEQ ID NO: 1 to 165 and 472 to 491 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to "BREAST CANCER GENE" polynucleotides or their complements following stringent hybridization and/or wash conditions also are "BREAST CANCER GENE" polynucleotides. Stringent wash conditions are well known and understood in the art and are disclosed, for example, in Sambrook et al., (6). Typically, for stringent hybridization conditions a combination of temperature and salt concentration should be chosen that is approximately 12to20˚C below the calculated $T_m$ of the hybrid under study. The $T_m$ of a hybrid between a "BREAST CANCER GENE" polynucleotide having a nucleotide sequence of one of the sequences of the SEQ ID NO: 1 to 165 and 472 to 491 or the complement thereof and a polynucleotide sequence which is at least about 50, preferably about 75, 90, 96, or 98% identical to one of those nucleotide sequences can be calculated, for example, using the equation below [Bolton and McCarthy, 1962, (17):

$$T_m = 81.5°C - 16.6(\log_{10}[Na^+]) + 0.41(\%G + C) - 0.63(\%formamide) - 600/l),$$

where 1= the length of the hybrid in basepairs.

Stringent wash conditions include, for example, 4X SSC at 65˚C, or 50% formamide, 4X SSC at 28˚C, or 0.5X SSC, 0.1% SDS at 65˚C. Highly stringent wash conditions include, for example, 0.2X SSC at 65˚C.

The biological function of the identified genes may be more directly assessed by utilizing relevant in vivo and in vitro systems. In vivo systems may include, but are not limited to, animal systems which naturally exhibit breast cancer predisposition, or ones which have been engineered to exhibit such symptoms, including but not limited to oncogene overexpression (e.g. HER2/neu, ras, raf, or EGFR) malignant neoplasia mouse.

Splice variants derived from the same genomic region, encoded by the same pre mRNA can be identified by hybridization conditions described above for homology search. The specific characteristics of variant proteins encoded by splice variants of the same pre transcript may differ and can also be assayed as disclosed. A "BREAST CANCER GENE" polynucleotide having a nucleotide sequence of one of the sequences of the SEQ ID NO: 1 to 165 and 472 to 491 or the complement thereof may therefor differ in parts of the entire sequence. The prediction of splicing events and the identification of the utilized acceptor and donor sites within the pre mRNA can be computed (e.g. Software Package GRAIL or GenomeSCAN) and verified by PCR method by those with skill in the art.

*Antisense oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 6 nucleotides in length, but can be at least 7, 8, 10, 12, 15, 20, 25, 30, 35, 40, 45, or 50 or more nucleotides long. Longer sequences also can be used. Antisense oligonucleotide molecules can be provided in a DNA construct and introduced into a cell as described above to alter the level of "BREAST CANCER GENE" gene products in the cell.

Antisense oligonucleotides can be deoxyribonucleotides, ribonucleotides, peptide nucleic acids (PNAs; described in U.S. Pat. No. 5,714,331), locked nucleic acids (LNAs; described in WO 99/12826), or a combination of them. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphor-amidates, phosphate esters, carbamates, acetamidate, carboxymethyl esters, carbonates, and phosphate triesters[Brown, 1994, (55); Sonveaux, 1994, (56) and Uhlmann et al., 1990, (57)].

Modifications of "BREAST CANCER GENE" expression can be obtained by designing antisense oligonucleotides which will form duplexes to the control, 5', or regulatory regions of the "BREAST CANCER GENE". Oligonucleotides derived from the transcription initiation site, e.g., between positions 10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Gee et al., 1994, (58)]. An antisense oligonucleotide also can be designed to block translation of mRNA by preventing the transcript from binding to ribosomes.

Precise complementarity is not required for successful complex formation between an antisense oligonucleotide and the complementary sequence of a "BREAST CANCER GENE" polynucleotide. Antisense oligonucleotides which comprise, for example, 2, 3, 4, or 5 or more stretches of contiguous nucleotides which are precisely complementary to a "BREAST CANCER GENE" polynucleotide, each separated by a stretch of contiguous nucleotides which are not complementary to adjacent "BREAST CANCER GENE" nucleotides, can provide sufficient targeting specificity for "BREAST CANCER GENE" mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular "BREAST CANCER GENE" polynucleotide sequence.

Antisense oligonucleotides can be modified without affecting their ability to hybridize to a "BREAST CANCER GENE" polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5' substituted oligonucleotide in which the 3' hydroxyl group or the 5' phosphate group are substituted, also can be employed in a modified antisense oligonucleotide. These modified oligonucleotides can be prepared by methods well known in the art [Agrawal et al., 1992, (59); Uhlmann et al., 1987, (57) and Uhlmann et al., 2000 (60)].

*Ribozymes*

Ribozymes are RNA molecules with catalytic activity [Cech, 1987, (61); Cech, 1990, (62) and Couture & Stinchcomb, 1996, (63)]. Ribozymes can be used to inhibit gene function by cleaving an RNA sequence, as is known in the art (e.g., Haseloff et al., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The transcribed sequence of a "BREAST CANCER GENE" can be used to generate ribozymes which will specifically bind to mRNA transcribed from a "BREAST CANCER GENE" genomic locus. Methods of designing and constructing ribozymes which can cleave other RNA molecules in trans in a highly sequence specific manner have been developed and described in the art [Haseloff et al., 1988, (64)]. For example, the cleavage activity of ribozymes can be targeted to specific RNAs by engineering a discrete "hybridization" region into the ribozyme. The hybridization region contains a sequence complementary to the target RNA and thus specifically hybridizes with the target [see, for example, Gerlach et al., EP 0 321201].

Specific ribozyme cleavage sites within a "BREAST CANCER GENE" RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate "BREAST CANCER GENE" RNA targets also can be evaluated by testing accessibility to hybridization with complementary oligonucleotides using ribonuclease protection assays. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease "BREAST CANCER GENE" expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S Pat. No. 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Polypeptides*

"BREAST CANCER GENE" polypeptides according to the invention comprise an polypeptide selected from SEQ ID NO: 166 to 330 and 492 to 511 or encoded by any of the polynucleotide sequences of the SEQ ID NO: 1 to 165 and 472 to 491 or derivatives, fragments, analogues and homologues thereof. A BREAST CANCER GENE" polypeptide of the invention therefore can be a portion, a full-length, or a fusion protein comprising all or a portion of a "BREAST CANCER GENE" polypeptide.

*Protein Purification*

"BREAST CANCER GENE" polypeptides can be purified from any cell which expresses the responding protein, including host cells which have been transfected with "BREAST CANCER GENE" expression constructs.. A purified "BREAST CANCER GENE" polypeptide is separated from other compounds which are normally associate with the "BREAST CANCER GENE" polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis. A preparation of purified "BREAST CANCER GENE" polypeptides is at least 80% pure; preferably, the preparations are 90%, 95%, or 99% pure. Purity of the preparations can be assessed by any means known in the art, such as SDS-polyacrylamide gel electrophoresis.

*Obtaining Polypeptides*

"BREAST CANCER GENE" polypeptides can be obtained, for example, by purification from human cells, by expression of "BREAST CANCER GENE" polynucleotides, or by direct chemical synthesis.

*Biologically Active Variants*

"BREAST CANCER GENE" polypeptide variants which are biologically active, i.e., retain an "BREAST CANCER GENE" activity, can be also regarded as "BREAST CANCER GENE" polypeptides. Preferably, naturally or non-naturally occurring "BREAST CANCER GENE" polypeptide variants have amino acid sequences which are at least about 60, 65, or 70, preferably about 75, 80, 85, 90, 92, 94, 96, or 98% identical to any of the amino acid sequences of the polypeptides of SEQ ID NO: 166 to 330 and 492 to 511 or the polypeptides encoded by any of the polynucleotides of SEQ ID NO: 1 to 165 and 472 to 491 or a fragment thereof. Percent identity between a putative "BREAST CANCER GENE" polypeptide variant and of the polypeptides of SEQ ID NO: 166 to 330 and 492 to 511 polypeptides encoded by any of the polynucleotides of SEQ ID NO: 1 to 165 and 472 to 491 or a fragment thereof is determined by conventional methods. [See, for example, Altschul *et al.*, 1986, (19) and Henikoff & Henikoff, 1992, (20)]. Briefly, two amino acid sequences are aligned to optimize the alignment scores using a gap opening penalty of 10, a gap extension penalty of 1, and the "BLOSUM62" scoring matrix of Henikoff & Henikoff, 1992 (20).

Those skilled in the art appreciate that there are many established algorithms available to align two amino acid sequences. The "FASTA" similarity search algorithm of Pearson & Lipman is a suitable protein alignment method for examining the level of identity shared by an amino acid sequence disclosed herein and the amino acid sequence of a putative variant [Pearson & Lipman, 1988, (21), and Pearson, 1990, (22)]. Briefly, FASTA first characterizes sequence similarity by identifying regions shared by the query sequence (e.g., SEQ ID NO: 1 to 165 and 472 to 491) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm [Needleman & Wunsch, 1970, (23), and Sellers, 1974, (24)], which allows for amino acid insertions and deletions. Preferred parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, (22).

FASTA can also be used to determine the sequence identity of nucleic acid molecules using a ratio as disclosed above. For nucleotide sequence comparisons, the ktup value can range between one to six, preferably from three to six, most preferably three, with other parameters set as default.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a "BREAST CANCER GENE" polypeptide can be found using computer programs well known in the art, such as DNASTAR software. Whether an amino acid change results in a biologically active "BREAST CANCER GENE" polypeptide can readily be determined by assaying for "BREAST CANCER GENE" activity, as described for example, in the specific Examples, below. Larger insertions or deletions can also be caused by alternative splicing. Protein domains can be inserted or deleted without altering the main activity of the protein.

*Fusion Proteins*

Fusion proteins are useful for generating antibodies against "BREAST CANCER GENE" polypeptide amino acid sequences and for use in various assay systems. For example, fusion proteins can be used to identify proteins which interact with portions of a "BREAST CANCER GENE" polypeptide. Protein affinity chromatography or library-based assays for protein-protein interactions, such as the yeast two-hybrid or phage display systems, can be used for this purpose. Such methods are well known in the art and also can be used as drug screens.

A "BREAST CANCER GENE" polypeptide fusion protein comprises two polypeptide segments fused together by means of a peptide bond. The first polypeptide segment comprises at least 25, 50, 75, 100, 150, 200, 300, 400, 500, 600, 700 or 750 contiguous amino acids of an amino acid sequence encoded by any polynucleotide sequences of the SEQ ID NO: 1 to 165 and 472 to 491 or of a biologically active variant, such as those described above. The first polypeptide segment also can comprise full-length "BREAST CANCER GENE".

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S- tag, Lex a DNA binding domain (DBD) fusions, GAL4 DNA binding domain fusions, and herpes simplex virus (HSV) BP16 protein fusions. A fusion protein also can be engineered to contain a cleavage site located between the "BREAST CANCER GENE" polypeptide-encoding sequence and the heterologous protein sequence, so that the "BREAST CANCER GENE" polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from any of the polynucleotide sequences of the SEQ ID NO: 1 to 165 and 472 to 491 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologues*

Species homologues of human a "BREAST CANCER GENE" polypeptide can be obtained using "BREAST CANCER GENE" polynucleotides (described below) to make suitable probes or primers for screening cDNA expression libraries from other species, such as mice, monkeys, or yeast, identifying cDNAs which encode homologues of a "BREAST CANCER GENE" polypeptide, and expressing the cDNAs as is known in the art.

*Expression of Polynucleotides*

To express a "BREAST CANCER GENE" polynucleotide, the polynucleotide can be inserted into an expression vector which contains the necessary elements for the transcription and translation of the inserted coding sequence. Methods which are well known to those skilled in the art can be used to construct expression vectors containing sequences encoding "BREAST CANCER GENE" polypeptides and appropriate transcriptional and translational control elements. These methods include in vitro recombinant DNA techniques, synthetic techniques, and in vivo genetic recombination. Such techniques are described, for example, in Sambrook et al., (6) and in Ausubel et al., (7).

A variety of expression vector/host systems can be utilized to contain and express sequences encoding a "BREAST CANCER GENE" polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those regions of the vector enhancers, promoters, 5' and 3' untranslated regions which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORTI plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a "BREAST CANCER GENE" polypeptide, vectors based on SV40 or EBV can be used with an appropriate selectable marker.

*Bacterial and Yeast Expression Systems*

In bacterial systems, a number of expression vectors can be selected depending upon the use intended for the "BREAST CANCER GENE" polypeptide. For example, when a large quantity of the "BREAST CANCER GENE" polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the "BREAST CANCER GENE" polypeptide can be ligated into the vector in frame with sequences for the amino terminal Met and the subsequent 7 residues of β-galactosidase so that a hybrid protein is produced. pIN vectors [Van Heeke & Schuster, (113)] or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast Saccharomyces cerevisiae, a number of vectors containing constitutive or inducible promoters such as alpha factor, alcohol oxidase, and PGH can be used. For reviews, see Ausubel et al., (7) and Grant et al., (114).

*Plant and Insect Expression Systems*

If plant expression vectors are used, the expression of sequences encoding "BREAST CANCER GENE" polypeptides can be driven by any of a number of promoters. For example, viral promoters such as the 35S and 19S promoters of CaMV can be used alone or in combination with the omega leader sequence from TMV [Takamatsu, 1987, (25)]. Alternatively, plant promoters such as the small subunit of RUBISCO or heat shock promoters can be used [Coruzzi et al., 1984, (26); Broglie et al., 1984, (27); Winter et al., 1991, (28)]. These constructs can be introduced into plant cells by direct DNA transformation or by pathogen-mediated transfection. Such techniques are described in a number of generally available reviews.

An insect system also can be used to express a "BREAST CANCER GENE" polypeptide. For example, in one such system Autographa californica nuclear polyhedrosis virus (AcNPV) is used as a vector to express foreign genes in Spodoptera frugiperda cells or in Trichoplusia larvae. Sequences encoding "BREAST CANCER GENE" polypeptides can be cloned into a nonessential region of the virus, such as the polyhedrin gene, and placed under control of the polyhedrin promoter. Successful insertion of "BREAST CANCER GENE" polypeptides will render the polyhedrin gene inactive and produce recombinant virus lacking coat protein. The recombinant viruses can then be used to infect S. frugiperda cells or Trichoplusia larvae in which "BREAST CANCER GENE" polypeptides can be expressed [Engelhard et al., 1994, (29)].

*Mammalian Expression Systems*

A number of viral-based expression systems can be used to express "BREAST CANCER GENE" polypeptides in mammalian host cells. For example, if an adenovirus is used as an expression vector, sequences encoding "BREAST CANCER GENE" polypeptides can be ligated into an adenovirus transcription/translation complex comprising the late promoter and tripartite leader sequence. Insertion in a nonessential E1 or E3 region of the viral genome can be used to obtain a viable virus which is capable of expressing a "BREAST CANCER GENE" polypeptide in infected host cells [Logan & Shenk, 1984, (30)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding "BREAST CANCER GENE" polypeptides. Such signals include the ATG initiation codon and adjacent sequences. In cases where sequences encoding a "BREAST CANCER GENE" polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used [Scharf et al., 1994, (31)].

*Host Cells*

A host cell strain can be chosen for its ability to modulate the expression of the inserted sequences or to process the expressed "BREAST CANCER GENE" polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for Post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express "BREAST CANCER GENE" polypeptides can be transformed using expression vectors which can contain viral origins of replication and/or endogenous expression elements and a selectable marker gene on the same or on a separate vector. Following the introduction of the vector, cells can be allowed to grow for 12 days in an enriched medium before they are switched to a selective medium. The purpose of the selectable marker is to confer resistance to selection, and its presence allows growth and recovery of cells which successfully express the introduced "BREAST CANCER GENE" sequences. Resistant clones of stably transformed cells can be proliferated using tissue culture techniques appropriate to the cell type [Freshney et al., 1986, (32).

Any number of selection systems can be used to recover transformed cell lines. These include, but are not limited to, the herpes simplex virus thymidine kinase (Wigler et al., 1977, (33)] and adenine phosphoribosyltransferase [Lowy et al., 1980, (34)] genes which can be employed in tk$^-$ or aprt$^-$ cells, respectively. Also, antimetabolite, antibiotic, or herbicide resistance can be used as the basis for selection. For example, dhfr confers resistance to methotrexate [Wigler et al., 1980, (35)], npt confers resistance to the aminoglycosides, neomycin and G418 [Colbere-Garapin et al., 1981, (36)], and als and pat confer resistance to chlorsulfuron and phosphinotricin acetyltransferase, respectively. Additional selectable genes have been described. For example, trpB allows cells to utilize indole in place of tryptophan, or hisD, which allows cells to utilize histinol in place of histidine [Hartman & Mulligan, 1988 ,(37)]. Visible markers such as anthocyanins, ß-glucuronidase and its substrate GUS, and luciferase and its substrate luciferin, can be used to identify transformants and to quantify the amount of transient or stable protein expression attributable to a specific vector system [Rhodes et al., 1995, (38)].

*Detecting Expression and gene product*

Although the presence of marker gene expression suggests that the "BREAST CANCER GENE" polynucleotide is also present, its presence and expression may need to be confirmed. For example, if a sequence encoding a "BREAST CANCER GENE" polypeptide is inserted within a marker gene sequence, transformed cells containing sequences which encode a "BREAST CANCER GENE" polypeptide can be identified by the absence of marker gene function. Alternatively, a marker gene can be placed in tandem with a sequence encoding a "BREAST CANCER GENE" polypeptide under the control of a single promoter. Expression of the marker gene in response to induction or selection usually indicates expression of the "BREAST CANCER GENE" polynucleotide.

Alternatively, host cells which contain a "BREAST CANCER GENE" polynucleotide and which express a "BREAST CANCER GENE" polypeptide can be identified by a variety of procedures known to those of skill in the art. These procedures include, but are not limited to, DNA-DNA or DNA-RNA hybridization and protein bioassay or immunoassay techniques which include membrane, solution, or chip-based technologies for the detection and/or quantification of polynucleotide or protein. For example, the presence of a polynucleotide sequence encoding a "BREAST CANCER GENE" polypeptide can be detected by DNA-DNA or DNA-RNA hybridization or amplification using probes or fragments or fragments of polynucleotides encoding a "BREAST CANCER GENE" polypeptide. Nucleic acid amplification-based assays involve the use of oligonucleotides selected from sequences encoding a "BREAST CANCER GENE" polypeptide to detect transformants which contain a "BREAST CANCER GENE" polynucleotide.

A variety of protocols for detecting and measuring the expression of a "BREAST CANCER GENE" polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a "BREAST CANCER GENE" polypeptide can be used, or a competitive binding assay can be employed. These and other assays are described in Hampton et al., (39) and Maddox et al., 40).

A wide variety of labels and conjugation techniques are known by those skilled in the art and can be used in various nucleic acid and amino acid assays. Means for producing labeled hybridization or PCR probes for detecting sequences related to polynucleotides encoding "BREAST CANCER GENE" polypeptides include oligo labeling, nick translation, end-labeling, or PCR amplification using a labeled nucleotide. Alternatively, sequences encoding a "BREAST CANCER GENE" polypeptide can be cloned into a vector for the production of an mRNA probe. Such vectors are known in the art, are commercially available, and can be used to synthesize RNA probes in vitro by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a "BREAST CANCER GENE" polypeptide can be cultured under conditions suitable for the expression and recovery of the protein from cell culture. The polypeptide produced by a transformed cell can be secreted or stored intracellular depending on the sequence and/or the vector used. As will be understood by those of skill in the art, expression vectors containing polynucleotides which encode "BREAST CANCER GENE" polypeptides can be designed to contain signal sequences which direct secretion of soluble "BREAST CANCER GENE" polypeptides through a prokaryotic or eukaryotic cell membrane or which direct the membrane insertion of membrane-bound "BREAST CANCER GENE" polypeptide.

As discussed above, other constructions can be used to join a sequence encoding a "BREAST CANCER GENE" polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the "BREAST CANCER GENE" polypeptide also can be used to facilitate purification. One such expression vector provides for expression of a fusion protein containing a "BREAST CANCER GENE" polypeptide and 6 histidine residues preceding a thioredoxin or an enterokinase cleavage site. The histidine residues facilitate purification by IMAC (immobilized metal ion affinity chromatography [Porath et al., 1992, (41)], while the enterokinase cleavage site provides a means for purifying the "BREAST CANCER GENE" polypeptide from the fusion protein. Vectors which contain fusion proteins are disclosed in Kroll et al., (42).

*Chemical Synthesis*

Sequences encoding a "BREAST CANCER GENE" polypeptide can be synthesized, in whole or in part, using chemical methods well known in the art (see Caruthers et al., (43) and Horn et al., (44). Alternatively, a "BREAST CANCER GENE" polypeptide itself can be produced using chemical methods to synthesize its amino acid sequence, such as by direct peptide synthesis using solid-phase techniques [Merrifield, 1963, (45) and Roberge et al., 1995, (46)]. Protein synthesis can be performed using manual techniques or by automation. Automated synthesis can be achieved, for example, using Applied Biosystems 431A Peptide Synthesizer (Perkin Elmer). Optionally, fragments of "BREAST CANCER GENE" polypeptides can be separately synthesized and combined using chemical methods to produce a full-length molecule.

The newly synthesized peptide can be substantially purified by preparative high performance liquid chromatography [Creighton, 1983, (47)]. The composition of a synthetic "BREAST CANCER GENE" polypeptide can be confirmed by amino acid analysis or sequencing (e.g., the Edman degradation procedure; see Creighton, (47). Additionally, any portion of the amino acid sequence of the "BREAST CANCER GENE" polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce "BREAST CANCER GENE" polypeptide-encoding nucleotide sequences possessing non-natural occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter "BREAST CANCER GENE" polypeptide-encoding sequences for a variety of reasons, including but not limited to, alterations which modify the cloning, processing, and/or expression of the polypeptide or mRNA product. DNA shuffling by random fragmentation and PCR re-assembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Predictive, Diagnostic and Prognostic Assays*

The present invention provides compositions, methods, and kits for determining whether a subject is at risk for developing malignant neoplasia and breast cancer in particular by detecting the disclosed biomarkers, i.e., the disclosed polynucleotide markers comprising any of the polynucleotides sequences of the SEQ ID NO 1 to 165 and 472 to 491 and/or the polypeptide markers encoded thereby or polypeptide markers comprising any of the polypeptide sequences of the SEQ ID NO: 166 to 330 and 492 to 511 for malignant neoplasia and breast cancer in particular.

In clinical applications, biological samples can be screened for the presence and/or absence of the biomarkers identified herein. Such samples are for example needle biopsy cores, surgical resection samples, or body fluids like serum, thin needle nipple aspirates and urine. For example, these methods include obtaining a biopsy, which is optionally fractionated by cryostat sectioning to enrich diseases cells to about 80% of the total cell population. In certain embodiments, polynucleotides extracted from these samples may be amplified using techniques well known in the art. The expression levels of selected markers detected would be compared with statistically valid groups of diseased and healthy samples.

In one embodiment the compositions, methods, and kits comprises determining whether a subject has an abnormal mRNA and/or protein level of the disclosed markers, such as by Northern blot analysis, reverse transcription-polymerase chain reaction (RT-PCR), in situ hybridization, immunoprecipitation, Western blot hybridization, or immunohistochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of malignant neoplasia such as breast cancer.

In another embodiment the compositions, methods, and kits comprises determining whether a subject has an abnormal DNA content of said genes or said genomic loci, such as by Southern blot analysis, dot blot analysis, Fluorescence or Colorimetric In Situ Hybridization, Comparative Genomic Hybridization or quantitative PCR. In general these assays comprise the usage of probes from representative genomic regions. The probes contain at least parts of said genomic regions or sequences complementary or analogous to said regions. In particular intra- or intergenic regions of said genes or genomic regions. The probes can consist of nucleotide sequences or sequences of analogous functions (e.g. PNAs, Morpholino oligomers) being able to bind to target regions by hybridization. In general genomic regions being altered in said patient samples are compared with unaffected control samples (normal tissue from the same or different patients, surrounding unaffected tissue, peripheral blood) or with genomic regions of the same sample that don't have said alterations and can therefore serve as internal controls. In a preferred embodiment regions located on the same chromosome are used. Alternatively, gonosomal regions and /or regions with defined varying amount in the sample are used. In one favored embodiment the DNA content, structure, composition or modification is compared that lie within distinct genomic regions. Especially favored are methods that detect the DNA content of said samples, where the amount of target regions are altered by amplification and or deletions. In another embodiment the target regions are analyzed for the presence of polymorphisms (e.g. Single Nucleotide Polymorphisms or mutations) that affect or predispose the cells in said samples with regard to clinical aspects, being of diagnostic, prognostic or therapeutic value. Preferably, the identification of sequence variations is used to define haplotypes that result in characteristic behavior of said samples with said clinical aspects.

In one embodiment, the compositions, methods, and kits for the prediction, diagnosis or prognosis of malignant neoplasia and breast cancer in particular are done by the detection of:

(a) a polynucleotide selected from the polynucleotides of the SEQ ID NO: 1 to 165 and 472 to 491;

(b) a polynucleotide which hybridizes under stringent conditions to a polynucleotide specified in (a) encoding a polypeptide exhibiting the same biological function as specified for the respective sequence in Table 1a and 1b or 4a and 4b;

(c) a polynucleotide the sequence of which deviates from the polynucleotide specified in (a) and (b) due to the generation of the genetic code encoding a polypeptide exhibiting the same biological function as specified for the polypeptides of SEQ ID NO: 166 to 330 and 492 to 511

(d) a polynucleotide which represents a specific fragment, derivative or allelic variation of a polynucleotide sequence specified in (a) to (c) encoding a polypeptide exhibiting the same biological function as specified for the respective sequence in Table 1a and 1b or 4a and 4b;

in a biological sample comprising the following steps: hybridizing any polynucleotide or analogous oligomer specified in (a) to (d) to a polynucleotide material of a biological sample, thereby forming a hybridization complex; and detecting said hybridization complex.

In another embodiment the method for the prediction, diagnosis or prognosis of malignant neoplasia is done as just described but, wherein before hybridization, the polynucleotide material of the biological sample is amplified.

In another embodiment the method for the diagnosis or prognosis of malignant neoplasia and breast cancer in particular is done by the detection of:

(a) a polynucleotide selected from the polynucleotides of the SEQ ID NO: 166 to 330 and 492 to 511;

(b) a polynucleotide which hybridizes under stringent conditions to a polynucleotide specified in (a) encoding a polypeptide exhibiting the same biological function as specified for the respective sequence in Table 1a and 1b or 4a and 4b;

(c) a polynucleotide the sequence of which deviates from the polynucleotide specified in (a) and (b) due to the generation of the genetic code encoding a polypeptide exhibiting the same biological function as specified for the respective sequence in Table 1a and 1b or 4a and 4b;

(d) a polynucleotide which represents a specific fragment, derivative or allelic variation of a polynucleotide sequence specified in (a) to (c) encoding a polypeptide exhibiting the same biological function as specified for the respective sequence in Table 1a and 1b or 4a and 4b;

(e) a polypeptide encoded by a polynucleotide sequence specified in (a) to (d)

(f) a polypeptide comprising any polypeptide of SEQ ID NO: 166 to 330 and 492 to 511 (g)

comprising the steps of contacting a biological sample with a reagent which specifically interacts with the polynucleotide specified in (a) to (d) or the polypeptide specified in (e).

*1. DNA array technology*

In one embodiment, the present Invention also provides a method wherein polynucleotide probes are immobilized an a DNA chip in an organized array. Oligonucleotides can be bound to a solid Support by a variety of processes, including lithography. For example a chip can hold up to 410.000 oligonucleotides (GeneChip, Affymetrix). The present invention provides significant advantages over the available tests for malignant neoplasia, such as breast cancer, because it increases the reliability of the test by providing an array of polynucleotide markers an a single chip.

The method includes obtaining a biologocal sample which can be a biopsy of an affected person, which is optionally fractionated by cryostat sectioning to enrich diseased cells to about 80% of the total cell population and the use of body fluids such as serum or urine, serum or cell containing liquids (e.g. derived from fine needle aspirates). The DNA or RNA is then extracted, amplified, and analyzed with a DNA chip to determine the presence of absence of the marker polynucleotide sequences. In one embodiment, the polynucleotide probes are spotted onto a substrate in a two-dimensional matrix or array. samples of polynucleotides can be labeled and then hybridized to the probes. Double-stranded polynucleotides, comprising the labeled sample polynucleotides bound to probe polynucleotides, can be detected once the unbound portion of the sample is washed away.

The probe polynucleotides can be spotted on substrates including glass, nitrocellulose, etc. The probes can be bound to the substrate by either covalent bonds or by non-specific interactions, such as hydrophobic interactions. The sample polynucleotides can be labeled using radioactive labels, fluorophores, chromophores, etc. Techniques for constructing arrays and methods of using these arrays are described in EP0 799 897; WO 97/29212; WO 97/27317; EP 0 785 280; WO 97/02357; U.S. Pat. No. 5,593,839; U.S. Pat. No. 5,578,832; EP 0 728 520; U.S. Pat. No. 5,599,695; EP 0 721 016; U.S. Pat. No. 5,556,752; WO 95/22058; and U.S. Pat. No. 5,631,734. Further, arrays can be used to examine differential expression of genes and can be used to determine gene function. For example, arrays of the instant polynucleotide sequences can be used to determine if any of the polynucleotide sequences are differentially expressed between normal cells and diseased cells, for example. High expression of a particular message in a diseased sample, which is not observed in a corresponding normal sample, can indicate a breast cancer specific protein.

Accordingly, in one aspect, the invention provides probes and primers that are specific to the polynucleotide sequences of SEQ ID NO: 1 to 165 and 472 to 491.

In one embodiment, the composition, method, and kit comprise using a polynucleotide probe to determine the presence of malignant or breast cancer cells in particular in a tissue from a patient. Specifically, the method comprises:

1) providing a polynucleotide probe comprising a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a polynucleotide selected from the polynucleotides of SEQ ID NO: 1 to 165 and 472 to 491 or a sequence complementary thereto;

2) obtaining a tissue sample from a patient with malignant neoplasia;

3) providing a second tissue sample from a patient with no malignant neoplasia;

4) contacting the polynucleotide probe under stringent conditions with RNA of each of said first and second tissue samples (e.g., in a Northern blot or in situ hybridization assay); and

5) comparing (a) the amount of hybridization of the probe with RNA of the first tissue sample, with (b) the amount of hybridization of the probe with RNA of the second tissue sample;

wherein a statistically significant difference in the amount of hybridization with the RNA of the first tissue sample as compared to the amount of hybridization with the RNA of the second tissue sample is indicative of malignant neoplasia and breast cancer in particular in the first tissue sample.

*2. Data analysis methods*

Comparison of the expression levels of one or more "BREAST CANCER GENES" with reference expression levels, e.g., expression levels in diseased cells of breast cancer or in normal counterpart cells, is preferably conducted using computer systems. In one embodiment, expression levels are obtained in two cells and these two sets of expression levels are introduced into a computer system for comparison. In a preferred embodiment, one set of expression levels is entered into a computer system for comparison with values that are already present in the computer system, or in computer-readable form that is then entered into the computer system.

In one embodiment, the invention provides a computer readable form of the gene expression profile data of the invention, or of values corresponding to the level of expression of at least one "BREAST CANCER GENE" in a diseased cell. The values can be mRNA expression levels obtained from experiments, e.g., microarray analysis. The values can also be mRNA levels normalised relative to a reference gene whose expression is constant in numerous cells under numerous conditions, e.g., GAPDH. In other embodiments, the values in the computer are ratios of, or differences between, normalized or non-normalized mRNA levels in different samples.

The gene expression profile data can be in the form of a table, such as an Excel table. The data can be alone, or it can be part of a larger database, e.g., comprising other expression profiles. For example, the expression profile data of the invention can be part of a public database. The computer readable form can be in a computer. In another embodiment, the invention provides a computer displaying the gene expression profile data.

In one embodiment, the invention provides a method for determining the similarity between the level of expression of one or more "BREAST CANCER GENES" in a first cell, e.g., a cell of a subject, and that in a second cell, comprising obtaining the level of expression of one or more "BREAST CANCER GENES" in a first cell and entering these values into a computer comprising a database including records comprising values corresponding to levels of expression of one or more "BREAST CANCER GENES" in a second cell, and processor instructions, e.g., a user interface, capable of receiving a selection of one or more values for comparison purposes with data that is stored in the computer. The computer may further comprise a means for converting the comparison data into a diagram or chart or other type of output.

In another embodiment, values representing expression levels of "BREAST CANCER GENES" are entered into a computer system, comprising one or more databases with reference expression levels obtained from more than one cell. For example, the computer comprises expression data of diseased and normal cells. Instructions are provided to the computer, and the computer is capable of comparing the data entered with the data in the computer to determine whether the data entered is more similar to that of a normal cell or of a diseased cell.

In another embodiment, the computer comprises values of expression levels in cells of subjects at different stages of breast cancer, and the computer is capable of comparing expression data entered into the computer with the data stored, and produce results indicating to which of the expression profiles in the computer, the one entered is most similar, such as to determine the stage of breast cancer in the subject.

In yet another embodiment, the reference expression profiles in the computer are expression profiles from cells of breast cancer of one or more subjects, which cells are treated *in vivo* or *in vitro* with a drug used for therapy of breast cancer. Upon entering of expression data of a cell of a subject treated *in vitro* or *in vivo* with the drug, the computer is instructed to compare the data entered to the data in the computer, and to provide results indicating whether the expression data input into the computer are more similar to those of a cell of a subject that is responsive to the drug or more similar to those of a cell of a subject that is not responsive to the drug. Thus, the results indicate whether the subject is likely to respond to the treatment with the drug or unlikely to respond to it.

In one embodiment, the invention provides a system that comprises a means for receiving gene expression data for one or a plurality of genes; a means for comparing the gene expression data from each of said one or plurality of genes to a common reference frame; and a means for presenting the results of the comparison. This system may further comprise a means for clustering the data.

In another embodiment, the invention provides a computer program for analyzing gene expression data comprising (i) a computer code that receives as input gene expression data for a plurality of genes and (ii) a computer code that compares said gene expression data from each of said plurality of genes to a common reference frame.

The invention also provides a machine-readable or computer-readable medium including program instructions for performing the following steps: (i) comparing a plurality of values corresponding to expression levels of one or more genes characteristic of breast cancer in a query cell with a database including records comprising reference expression or expression profile data of one or more reference cells and an annotation of the type of cell; and (ii) indicating to which cell the query cell is most similar based on similarities of expression profiles. The reference cells can be cells from subjects at different stages of breast cancer. The reference cells can also be cells from subjects responding or not responding to a particular drug treatment and optionally incubated *in vitro* or *in vivo* with the drug.

The reference cells may also be cells from subjects responding or not responding to several different treatments, and the computer system indicates a preferred treatment for the subject. Accordingly, the invention provides a method for selecting a therapy for a patient having breast cancer, the method comprising: (i) providing the level of expression of one or more genes characteristic of breast cancer in a diseased cell of the patient; (ii) providing a plurality of reference profiles, each associated with a therapy, wherein the subject expression profile and each reference profile has a plurality of values, each value representing the level of expression of a gene characteristic of breast cancer; and (iii) selecting the reference profile most similar to the subject expression profile, to thereby select a therapy for said patient. In a preferred embodiment step (iii) is performed by a computer. The most similar reference profile may be selected by weighing a comparison value of the plurality using a weight value associated with the corresponding expression data.

The relative abundance of an mRNA in two biological samples can be scored as a perturbation and its magnitude determined (i.e., the abundance is different in the two sources of mRNA tested), or as not perturbed (i.e., the relative abundance is the same). In various embodiments, a difference between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant) is scored as a perturbation. Perturbations can be used by a computer for calculating and expression comparisons.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

The computer readable medium may further comprise a pointer to a descriptor of a stage of breast cancer or to a treatment for breast cancer.

In operation, the means for receiving gene expression data, the means for comparing the gene expression data, the means for presenting, the means for normalizing, and the means for clustering within the context of the systems of the present invention can involve a programmed computer with the respective functionalities described herein, implemented in hardware or hardware and software; a logic circuit or other component of a programmed computer that performs the operations specifically identified herein, dictated by a computer program; or a computer memory encoded with executable instructions representing a computer program that can cause a computer to function in the particular fashion described herein.

Those skilled in the art will understand that the systems and methods of the present invention may be applied to a variety of systems, including IBM-compatible personal computers running MS-DOS or Microsoft Windows.

The computer may have internal components linked to external components. The internal components may include a processor element interconnected with a main memory. The computer system can be an Intel Pentium®-based processor of 200 MHz or greater clock rate and with 32 MB or more of main memory. The external component may comprise a mass storage, which can be one or more hard disks (which are typically packaged together with the processor and memory).

Such hard disks are typically of 1 GB or greater storage capacity. Other external components include a user interface device, which can be a monitor, together with an inputing device, which can be a "mouse", or other graphic input devices, and/or a keyboard. A printing device can also be attached to the computer.

Typically, the computer system is also linked to a network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the instant invention. These software components collectively cause the computer system to function according to the methods of this invention. These software components are typically stored on a mass storage. A software component represents the operating system, which is responsible for managing the computer system and its network interconnections. This operating system can be, for example, of the Microsoft Windows' family, such as Windows 95, Windows 98, or Windows NT. A software component represents common languages and functions conveniently present on this system to assist programs implementing the methods specific to this invention. Many high or low level computer languages can be used to program the analytic methods of this invention. Instructions can be interpreted during run-time or compiled. Preferred languages include C/C++, and JAVA®. Most preferably, the methods of this invention are programmed in mathematical software packages which allow symbolic entry of equations and high-level specification of processing, including algorithms to be used, thereby freeing a user of the need to procedurally program individual equations or algorithms. Such packages include Matlab from Mathworks (Natick, Mass.), Mathematica from Wolfram Research (Champaign, Ill.), or S-Plus from Math Soft (Cambridge, Mass.). Accordingly, a software component represents the analytic methods of this invention as programmed in a procedural language or symbolic package. In a preferred embodiment, the computer system also contains a database comprising values representing levels of expression of one or more genes characteristic of breast cancer. The database may contain one or more expression profiles of genes characteristic of breast cancer in different cells.

In an exemplary implementation, to practice the methods of the present invention, a user first loads expression profile data into the computer system. These data can be directly entered by the user from a monitor and keyboard, or from other computer systems linked by a network connection, or on removable storage media such as a CD-ROM or floppy disk or through the network. Next the user causes execution of expression profile analysis software which performs the steps of comparing and, e.g., clustering co-varying genes into groups of genes.

In another exemplary implementation, expression profiles are compared using a method described in U.S. Patent No. 6,203,987. A user first loads expression profile data into the computer system. Geneset profile definitions are loaded into the memory from the storage media or from a remote computer, preferably from a dynamic geneset database system, through the network. Next the user causes execution of projection software which performs the steps of converting expression profile to projected expression profiles. The projected expression profiles are then displayed.

In yet another exemplary implementation, a user first leads a projected profile into the memory. The user then causes the loading of a reference profile into the memory. Next, the user causes the execution of comparison software which performs the steps of objectively comparing the profiles.

*3. Detection of variant polynucleotide sequence*

In yet another embodiment, the invention provides methods for determining whether a subject is at risk for developing a disease, such as a predisposition to develop malignant neoplasia, for example breast cancer, associated with an aberrant activity of any one of the polypeptides encoded by any of the polynucleotides of the SEQ ID NO: 1 to 165 and 472 to 491, wherein the aberrant activity of the polypeptide is characterized by detecting the presence or absence of a genetic lesion characterized by at least one of these:

(i) an alteration affecting the integrity of a gene encoding a marker polypeptides, or

(ii) the misexpression of the encoding polynucleotide.

To illustrate, such genetic lesions can be detected by ascertaining the existence of at least one of these:

I. a deletion of one or more nucleotides from the polynucleotide sequence

II. an addition of one or more nucleotides to the polynucleotide sequence

III. a substitution of one or more nucleotides of the polynucleotide sequence

IV. a gross chromosomal rearrangement of the polynucleotide sequence

V. a gross alteration in the level of a messenger RNA transcript of the polynucleotide sequence

VI. aberrant modification of the polynucleotide sequence, such as of the methylation pattern of the genomic DNA

VII. the presence of a non-wild type splicing pattern of a messenger RNA transcript of the gene

VIII. a non-wild type level of the marker polypeptide

IX. allelic loss of the gene

X. inappropriate post-translational modification of the marker polypeptide

The present invention provides assay techniques for detecting mutations in the encoding polynucleotide sequence. These methods include, but are not limited to, methods involving sequence analysis, Southern blot hybridization, restriction enzyme site mapping, and methods involving detection of absence of nucleotide pairing between the polynucleotide to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g. individuals which developed a specific disease, such as breast cancer. A polymorphic region can be located in any region of a gene, e.g., exons, in coding or non coding regions of exons, introns, and promoter region.

In an exemplary embodiment, there is provided a polynucleotide composition comprising a polynucleotide probe including a region of nucleotide sequence which is capable of hybridising to a sense or antisense sequence of a gene or naturally occurring mutants thereof, or 5' or 3' flanking sequences or intronic sequences naturally associated with the subject genes or naturally occurring mutants thereof. The polynucleotide of a cell is rendered accessible for hybridization, the probe is contacted with the polynucleotide of the sample, and the hybridization of the probe to the sample polynucleotide is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridising specifically to allelic variants are attached to a solid phase support, e.g., a "chip". Mutation detection analysis using these chips comprising oligonucleotides, also termed "DNA probe arrays" is described e.g., in Cronin et al. (48). In one embodiment, a chip comprises all the allelic variants of at least one polymorphic region of a gene. The solid phase support is then contacted with a test polynucleotide and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) [Landegran et al., 1988, (49) and Nakazawa et al., 1994 (50)], the latter of which can be particularly useful for detecting point mutations in the gene; Abravaya et al., 1995 ,(51)]. In a merely illustrative embodiment, the method includes the steps of (i) collecting a sample of cells from a patient, (ii) isolating polynucleotide (e.g., genomic, mRNA or both) from the cells of the sample, (iii) contacting the polynucleotide sample with one or more primers which specifically hybridize to a polynucleotide sequence under conditions such that hybridization and amplification of the polynucleotide (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication [Guatelli, J.C. et al., 1990, (52)], transcriptional amplification system [Kwoh, D.Y. et al., 1989, (53)], Q-Beta replicase [Lizardi, P.M. et al., 1988 ,(54)], or any other polynucleotide amplification method, followed by the detection of the amplified molecules using techniques well known to those of skill in the art. These detection schemes are especially useful for the detection of polynucleotide molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover; the use of sequence specific ribozymes (see, for example, U.S. Patent No. 5,498,531) can be used to score for the presence of specific mutations by development or loss of a ribozyme cleavage site.

*4. In situ hybridization*

In one aspect, the method comprises *in situ* hybridization with a probe derived from a given marker polynucleotide, which sequence is selected from any of the polynucleotide sequences of the SEQ ID NO: 1 to 165 and 472 to 491 or a sequence complementary thereto. The method comprises contacting the labeled hybridization probe with a sample of a given type of tissue from a patient potentially having malignant neoplasia and breast cancer in particular as well as normal tissue from a person with no malignant neoplasia, and determining whether the probe labels tissue of the patient to a degree significantly different (e.g., by at least a factor of two, or at least a factor of five, or at least a factor of twenty, or at least a factor of fifty) than the degree to which normal tissue is labelled.

*Polypeptide detection*

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

*Antibodies*

Any type of antibody known in the art can be generated to bind specifically to an epitope of a "BREAST CANCER GENE" polypeptide. An antibody as used herein includes intact immunoglobulin molecules, as well as fragments thereof, such as Fab, $F(ab)_2$, and Fv, which are capable of binding an epitope of a "BREAST CANCER GENE" polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a "BREAST CANCER GENE" polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a "BREAST CANCER GENE" polypeptide provides a detection signal at least 5-, 10-, or 20-fold higher than a detection signal provided with other proteins when used in an immunochemical assay. Preferably, antibodies which specifically bind to "BREAST CANCER GENE" polypeptides do not detect other proteins in immunochemical assays and can immunoprecipitate a "BREAST CANCER GENE" polypeptide from solution.

"BREAST CANCER GENE" polypeptides can be used to immunize a mammal, such as a mouse, rat, rabbit, guinea pig, monkey, or human, to produce polyclonal antibodies. If desired, a "BREAST CANCER GENE" polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG (bacilli Calmette-Guerin) and Corynebacterium parvum are especially useful.

Monoclonal antibodies which specifically bind to a "BREAST CANCER GENE" polypeptide can be prepared using any technique which provides for the production of antibody molecules by continuous cell lines in culture. These techniques include, but are not limited to, the hybridoma technique, the human B cell hybridoma technique, and the EBV hybridoma technique [Kohler et al., 1985, (65); Kozbor et al., 1985, (66); Cote et al., 1983, (67) and Cole et al., 1984, (68)].

In addition, techniques developed for the production of chimeric antibodies, the splicing of mouse antibody genes to human antibody genes to obtain a molecule with appropriate antigen specificity and biological activity, can be used [Morrison et al., 1984, (69); Neuberger et al., 1984, (70); Takeda et al., 1985, (71)]. Monoclonal and other antibodies also can be humanized to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a "BREAST CANCER GENE" polypeptide can contain antigen binding sites which are either partially or fully humanized, as disclosed in U.S. Patent 5,565,332.

Alternatively, techniques described for the production of single chain antibodies can be adapted using methods known in the art to produce single chain antibodies which specifically bind to "BREAST CANCER GENE" polypeptides. Antibodies with related specificity, but of distinct idiotypic composition, can be generated by chain shuffling from random combinatorial immunoglobulin libraries [Burton, 1991, (72)].

Single-chain antibodies also can be constructed using a DNA amplification method, such as PCR, using hybridoma cDNA as a template [Thirion et al., 1996, (73)]. Single-chain antibodies can be mono- or bispecific, and can be bivalent or tetravalent. Construction of tetravalent, bispecific single-chain antibodies is taught, for example, in Coloma & Morrison, (74). Construction of bivalent, bispecific single-chain antibodies is taught in Mallender & Voss, (75).

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology [Verhaar et al., 1995, (76); Nicholls et al., 1993, (77)].

Antibodies which specifically bind to "BREAST CANCER GENE" polypeptides also can be produced by inducing in vivo production in the lymphocyte population or by screening immunoglobulin libraries or panels of highly specific binding reagents as disclosed in the literature [Orlandi et al., 1989, (789) and Winter et al., 1991, (79)].

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the antibodies described in WO 94/13804, also can be prepared.

Antibodies according to the invention can be purified by methods well known in the art. For example, antibodies can be affinity purified by passage over a column to which a "BREAST CANCER GENE" polypeptide is bound. The bound antibodies can then be eluted from the column using a buffer with a high salt concentration.

Immunoassays are commonly used to quantify the levels of proteins in cell samples, and many other immunoassay techniques are known in the art. The invention is not limited to a particular assay procedure, and therefore is intended to include both homogeneous and heterogeneous procedures. Exemplary immunoassays which can be conducted according to the invention include fluorescence polarisation immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

Other methods to quantify the level of a particular protein, or a protein fragment, or modified protein in a particular sample are based on flow-cytometric methods. Flow cytometry allows the identification of proteins on the cell surface as well as of intracellular proteins using fluorochrome labeled, protein specific antibodies or non-labeled antibodies in combination with fluorochrome labeled secondary antibodies. General techniques to be used in performing flow cytometric assays noted above are known to those of ordinary skill in the art. A special method based on the same principles is the microsphere-based flow cytometric. Microsphere beads are labeled with precise quantities of fluorescent dye and particular antibodies. Such techniques are provided by Luminex Inc. WO 97/14028. In another embodiment the level of a particular protein or a protein fragment, or modified protein in a particular sample may be determined by 2D gel-electrophoresis and/or mass spectrometry. Determination of protein nature, sequence, molecular mass as well charge can be achieved in one detection step. Mass spectrometry can be performed with methods known to those with skills in the art as MALDI, TOF, or combinations of these.

In another embodiment, the level of the encoded product, i.e., the product encoded by any of the polynucleotide sequences of the SEQ ID NO: 1 to 165 and 472 to 491 or a sequence complementary thereto, in a biological fluid (e.g., blood or urine) of a patient may be determined as a way of monitoring the level of expression of the marker polynucleotide sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of the disorder, e.g., plaque formation. The level of the marker polypeptide can be used predictively to evaluate whether a sample of cells contains cells which are, or are predisposed towards becoming, plaque associated cells. The observation of marker polypeptide level can be utilized in decisions regarding, e.g., the use of more stringent therapies.

As set out above, one aspect of the present invention relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantify the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

*Immunohistochemistry*

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissues samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a Label for subsequent detection of binding. samples are incubated for a time Sufficient for formation of the immunocomplexes. Binding of the antibody is then detected by virtue of a Label conjugated to this antibody. Where the antibody is unlabelled, a second labeled antibody may be employed, e.g., which is specific for the isotype of the anti-marker polypeptide antibody. Examples of labels which may be employed include radionuclides, fluorescence, chemoluminescence, and enzymes.

Where enzymes are employed, the Substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a Single cell by correlating the amount of marker polypeptide in a cell-free extract produced from a predetermined number of cells.

In yet another embodiment, the invention contemplates using a panel of antibodies which are generated against the marker polypeptides of this invention, which polypeptides are encoded by any of the polynucleotide sequences of the SEQ ID NO: 1 to 165 and 472 to 491. Such a panel of antibodies may be used as a reliable diagnostic probe for breast cancer. The assay of the present invention comprises contacting a biopsy sample containing cells, e.g., macrophages, with a panel of antibodies to one or more of the encoded products to determine the presence or absence of the marker polypeptides.

The diagnostic methods of the subject invention may also be employed as follow-up to treatment, e.g., quantification of the level of marker polypeptides may be indicative of the effectiveness of current or previously employed therapies for malignant neoplasia and breast cancer in particular as well as the effect of these therapies upon patient prognosis.

The diagnostic assays described above can be adapted to be used as prognostic assays, as well. Such an application takes advantage of the sensitivity of the assays of the Invention to events which take place at characteristic stages in the progression of plaque generation in case of malignant neoplasia. For example, a given marker gene may be up- or down-regulated at a very early stage, perhaps before the cell is developing into a foam cell, while another marker gene may be characteristically up or down regulated only at a much later stage. Such a method could involve the steps of contacting the mRNA of a test cell with a polynucleotide probe derived from a given marker polynucleotide which is expressed at different characteristic levels in breast cancer tissue cells at different stages of malignant neoplasia progression, and determining the approximate amount of hybridization of the probe to the mRNA of the cell, such amount being an indication of the level of expression of the gene in the cell, and thus an indication of the stage of disease progression of the cell; alternatively, the assay can be carried out with an antibody specific for the gene product of the given marker polynucleotide, contacted with the proteins of the test cell. A battery of such tests will disclose not only the existence of a certain neoplastic lesion, but also will allow the clinician to select the mode of treatment most appropriate for the disease, and to predict the likelihood of success of that treatment.

The methods of the invention can also be used to follow the clinical course of a given breast cancer predisposition. For example, the assay of the Invention can be applied to a blood sample from a patient; following treatment of the patient for BREAST CANCER, another blood sample is taken and the test repeated. Successful treatment will result in removal of demonstrate differential expression, characteristic of the breast cancer tissue cells, perhaps approaching or even surpassing normal levels.

*Polypeptide activity*

In one embodiment the present invention provides a method for screening potentially therapeutic agents which modulate the activity of one or more "BREAST CANCER GENE" polypeptides, such that if the activity of the polypeptide is increased as a result of the upregulation of the "BREAST CANCER GENE" in a subject having or at risk for malignant neoplasia and breast cancer in particular, the therapeutic substance will decrease the activity of the polypeptide relative to the activity of the some polypeptide in a subject not having or not at risk for malignant neoplasia or breast cancer in particular but not treated with the therapeutic agent. Likewise, if the activity of the polypeptide as a result of the downregulation of the "BREAST CANCER GENE" is decreased in a subject having or at risk for malignant neoplasia or breast cancer in particular, the therapeutic agent will increase the activity of the polypeptide relative to the activity of the same polypeptide in a subject not having or not at risk for malignant neoplasia or breast cancer in particular, but not treated with the therapeutic agent.

The activity of the "BREAST CANCER GENE" polypeptides indicated in Table 2 or 3 may be measured by any means known to those of skill in the art, and which are particular for the type of activity performed by the particular polypeptide. Examples of specific assays which may be used to measure the activity of particular polynucleotides are shown below.

a) G protein coupled receptors

In one embodiment, the "BREAST CANCER GENE" polynucleotide may encode a G protein coupled receptor. In one embodiment, the present invention provides a method of screening potential modulators (inhibitors or activators) of the G protein coupled receptor by measuring changes in the activity of the receptor in the presence of a candidate modulator.

1) *$G_i$-coupled receptors*

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37˚C in a humidified atmosphere with 10% $CO_2$ and are routinely split at a ratio of 1:10 every 2 or 3 days. Test cultures are seeded into 384 - well plates at an appropriate density (e.g. 2000 cells / well in 35 μl cell culture medium) in DMEM/F12 with FBS, and are grown for 48 hours (range: ~ 24 - 60 hours, depending on cell line). Growth medium is then exchanged against serum free medium (SFM; e.g. Ultra-CHO), containing 0,1% BSA. Test compounds dissolved in DMSO are diluted in SFM and transferred to the test cultures (maximal final concentration 10 μmolar), followed by addition of forskolin (~ 1 μmolar, final conc.) in SFM + 0,1% BSA 10 minutes later. In case of antagonist screening both, an appropriate concentration of agonist, and forskolin are added. The plates are incubated at 37˚C in 10% $CO_2$ for 3 hours. Then the supernatant is removed, cells are lysed with lysis reagent (25 mmolar phosphate-buffer, pH 7,8, containing 2 mmolar DDT, 10% glycerol and 3% Triton X100). The luciferase reaction is started by addition of substrate-buffer (e.g. luciferase assay reagent, Promega) and luminescence is immediately determined (e.g. Berthold luminometer or Hamamatzu camera system).

*2) $G_s$-coupled receptors*

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37˚C in a humidified atmosphere with 10% $CO_2$ and are routinely split at a ratio of 1:10 every 2 or 3 days. Test cultures are seeded into 384 - well plates at an appropriate density (e.g. 1000 or 2000 cells / well in 35 μl cell culture medium) in DMEM/F12 with FBS, and are grown for 48 hours (range: ~ 24 - 60 hours, depending on cell line). The assay is started by addition of test-compounds in serum free medium (SFM; e.g. Ultra-CHO) containing 0,1% BSA: Test compounds are dissolved in DMSO, diluted in SFM and transferred to the test cultures (maximal final concentration 10 μmolar, DMSO conc. < 0,6 %). In case of antagonist screening an appropriate concentration of agonist is added 5-10 minutes later. The plates are incubated at 37˚C in 10% $CO_2$ for 3 hours. Then the cells are lysed with 10 μl lysis reagent per well (25 mmolar phosphate-buffer, pH 7,8 , containing 2 mmolar DDT, 10% glycerol and 3% Triton X100) and the luciferase reaction is started by addition of 20 μl substrate-buffer per well (e.g. luciferase assay reagent, Promega). Measurement of luminescence is started immediately (e.g. Berthold luminometer or Hamamatzu camera system).

3) $G_g$ -coupled receptors

Cells (such as CHO cells or primary cells) are stably transfected with the relevant receptor. Cells expressing functional receptor protein are grown in 50% Dulbecco's modified Eagle medium /50% F12 (DMEM/F12) supplemented with 10% FBS, at 37˚C in a humidified atmosphere with 5% $CO_2$ and are routinely split at a cell line dependent ratio every 3 or 4 days. Test cultures are seeded into 384 - well plates at an appropriate density (e.g. 2000 cells / well in 35 μl cell culture medium) in DMEMIF12 with FBS, and are grown for 48 hours (range: ~ 24 - 60 hours, depending on cell line). Growth medium is then exchanged against physiological salt solution (e.g. Tyrode solution). Test compounds dissolved in DMSO are diluted in Tyrode solution containing 0.1% BSA and transferred to the test cultures (maximal final concentration 10 μmolar). After addition of the receptor specific agonist the resulting Gq-mediated intracellular calcium increase is measured using appropriate read-out systems (e.g. calcium-sensitive dyes).

b) Ion channels

Ion channels are integral membrane proteins involved in electrical signaling, transmembrane signal transduction, and electrolyte and solute transport. By forming macromolecular pores through the membrane lipid bilayer, ion channels account for the flow of specific ion species driven by the electrochemical potential gradient for the permeating ion. At the single molecule level, individual channels undergo conformational transitions ("gating") between the 'open' (ion conducting) and 'closed' (non conducting) state. Typical single channel openings last for a few milliseconds and result in elementary transmembrane currents in the range of $10^{-9}$ - $10^{-12}$ Ampere. Channel gating is controlled by various chemical and/or biophysical parameters, such as neurotransmitters and intracellular second messengers ('ligand-gated' channels) or membrane potential ('voltage-gated' channels). Ion channels are functionally characterized by their ion selectivity, gating properties, and regulation by hormones and pharmacological agents. Because of their central role in signaling and transport processes, ion channels present ideal targets for pharmacological therapeutics in various pathophysiological settings.

In one embodiment, the "BREAST CANCER GENE" may encode an ion channel. In one embodiment, the present invention provides a method of screening potential activators or inhibitors of channels activity of the "BREAST CANCER GENE" polypeptide. Screening for compounds interaction with ion channels to either inhibit or promote their activity can be based on (1.) binding and (2.) functional assays in living cells [Hille (112)].

1. For ligand-gated channels, e.g. ionotropic neurotransmitter/hormone receptors, assays can be designed detecting binding to the target by competition between the compound and a labeled ligand.

2. Ion channel function can be tested functionally in living cells. Target proteins are either expressed endogenously in appropriate reporter cells or are introduced recombinantly. Channel activity can be monitored by (2.1) concentration changes of the permeating ion (most prominently $Ca^{2+}$ ions), (2.2) by changes in the transmembrane electrical potential gradient, and (2.3) by measuring a cellular response (e.g. expression of a reporter gene, secretion of a neurotransmitter) triggered or modulated by the target activity.

2.1 Channel activity results in transmembrane ion fluxes. Thus activation of ionic channels can be monitored by the resulting changes in intracellular ion concentrations using luminescent or fluorescent indicators. Because of its wide dynamic range and availability of suitable indicators this applies particularly to changes in intracellular $Ca^{2+}$ ion concentration ($[Ca^{2+}]_i$). $[Ca^{2+}]_i$ can be measured, for example, by aequorin luminescence or fluorescence dye technology (e.g. using Fluo-3, Indo-1, Fura-2). Cellular assays can be designed where either the $Ca^{2+}$ flux through the target channel itself is measured directly or where modulation of the target channel affects membrane potential and thereby the activity of co-expressed voltage-gated $Ca^{2+}$ channels.

2.2 Ion channel currents result in changes of electrical membrane potential ($V_m$) which can be monitored directly using potentiometric fluorescent probes. These electrically charged indicators (e.g. the anionic oxonol dye $DiBAC_4(3)$) redistribute between extra- and intracellular compartment in response to voltage changes. The equilibrium distribution is governed by the Nernst-equation. Thus changes in membrane potential results in concomitant changes in cellular fluorescence. Again, changes in $V_m$ might be caused directly by the activity of the target ion channel or through amplification and/or prolongation of the signal by channels co-expressed in the same cell.

2.3 Target channel activity can cause cellular $Ca^{2+}$ entry either directly or through activation of additional $Ca^{2+}$ channel (see 2.1). The resulting intracellular $Ca^{2+}$ signals regulate a variety of cellular responses, e.g. secretion or gene transcription. Therefore modulation of the target channel can be detected by monitoring secretion of a known hormone/transmitter from the target-expressing cell or through expression of a reporter gene (e.g. luciferase) controlled by an $Ca^{2+}$-responsive promoter element (e.g. cyclic AMP/ $Ca^{2+}$-responsive elements; CRE).

c) DNA-binding proteins and transcription factors

In one embodiment, the "BREAST CANCER GENE" may encode a DNA-binding protein or a transcription factor. The activity of such a DNA-binding protein or a transcription factor may be measured, for example, by a promoter assay which measures the ability of the DNA-binding protein or the transcription factor to initiate transcription of a test sequence linked to a particular promoter. In one embodiment, the present invention provides a method of screening test compounds for its ability to modulate the activity of such a DNA-binding protein or a transcription factor by measuring the changes in the expression of a test gene which is regulated by a promoter which is responsive to the transcription factor.

*Promotor assays*

A promoter assay was set up with a human hepatocellular carcinoma cell HepG2 that was stably transfected with a luciferase gene under the control of a gene of interest (e.g. thyroid hormone) regulated promoter. The vector 2xIROluc, which was used for transfection, carries a thyroid hormone responsive element (TRE) of two 12 bp inverted palindromes separated by an 8 bp spacer in front of a tk minimal promoter and the luciferase gene. Test cultures were seeded in 96 well plates in serum - free Eagle's Minimal Essential Medium supplemented with glutamine, tricine, sodium pyruvate, non - essential amino acids, insulin, selen, transferrin, and were cultivated in a humidified atmosphere at 10 % $CO_2$ at 37˚C. After 48 hours of incubation serial dilutions of test compounds or reference compounds (L-T3, L-T4 e.g.) and co-stimulator if appropriate (final concentration 1 nM) were added to the cell cultures and incubation was continued for the optimal time (e.g. another 4-72 hours). The cells were then lysed by addition of buffer containing Triton X100 and luciferin and the luminescence of luciferase induced by T3 or other compounds was measured in a luminometer. For each concentration of a test compound replicates of 4 were tested. $EC_{50}$ - values for each test compound were calculated by use of the Graph Pad Prism Scientific software.

*Screening Methods*

The invention provides assays for screening test compounds which bind to or modulate the activity of a "BREAST CANCER GENE" polypeptide or a "BREAST CANCER GENE" polynucleotide. A test compound preferably binds to a "BREAST CANCER GENE" polypeptide or polynucleotide. More preferably, a test compound decreases or increases "BREAST CANCER GENE" activity by at least about 10, preferably about 50, more preferably about 75, 90, or 100% relative to the absence of the test compound.

*Test Compounds*

Test compounds can be pharmacological agents already known in the art or can be compounds previously unknown to have any pharmacological activity. The compounds can be naturally occurring or designed in the laboratory. They can be isolated from microorganisms, animals, or plants, and can be produced recombinant, or synthesised by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the one-bead one-compound library method, and synthetic library methods using affinity chromatography selection. The biological library approach is limited to polypeptide libraries, while the other four approaches are applicable to polypeptide, non-peptide oligomer, or small molecule libraries of compounds. [For review see Lam, 1997, (80)].

Methods for the synthesis of molecular libraries are well known in the art [see, for example, DeWitt et al., 1993, (81); Erb et al., 1994, (82); Zuckermann et al., 1994, (83); Cho et al., 1993, (84); Carell et al., 1994, (85) and Gallop et al., 1994, (86). Libraries of compounds can be presented in solution [see, e.g., Houghten, 1992, (87)], or on beads [Lam, 1991, (88)], DNA-chips [Fodor, 1993, (89)], bacteria or spores (Ladner, U.S. Patent 5,223,409), plasmids [Cull et al., 1992, (901)], or phage [Scott & Smith, 1990, (91); Devlin, 1990, (92); Cwirla et al., 1990, (93); Felici, 1991, (94)].

<u>High Throughput Screening</u>

Test compounds can be screened for the ability to bind to "BREAST CANCER GENE" polypeptides or polynucleotides or to affect "BREAST CANCER GENE" activity or "BREAST CANCER GENE" expression using high throughput screening. Using high throughput screening, many discrete compounds can be tested in parallel so that large numbers of test compounds can be quickly screened. The most widely established techniques utilize 96-well, 384-well or 1536-well microtiter plates. The wells of the microtiter plates typically require assay volumes that range from 5 to 500 $\mu$l. In addition to the plates, many instruments, materials, pipettors, robotics, plate washers, and plate readers are commercially available to fit the microwell formats.

Alternatively, free format assays, or assays that have no physical barrier between samples, can be used. For example, an assay using pigment cells (melanocytes) in a simple homogeneous assay for combinatorial peptide libraries is described by Jayawickreme et al., (95). The cells are placed under agarose in culture dishes, then beads that carry combinatorial compounds are placed on the surface of the agarose. The combinatorial compounds are partially released the compounds from the beads. Active compounds can be visualised as dark pigment areas because, as the compounds diffuse locally into the gel matrix, the active compounds cause the cells to change colors.

Another example of a free format assay is described by Chelsky, (96). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV light. Compounds that inhibited the enzyme were observed as local zones of inhibition having less color change.

In another example, combinatorial libraries were screened for compounds that had cytotoxic effects on cancer cells growing in agar [Salmon et al., 1996, (97)].

Another high throughput screening method is described in Beutel et al., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies, for example, the ATP/GTP binding site of the enzyme or the active site of a "BREAST CANCER GENE" polypeptide, such that normal biological activity is prevented. Examples of such small molecules include, but are not limited to, small peptides or peptide-like molecules.

In binding assays, either the test compound or a "BREAST CANCER GENE" polypeptide can comprise a detectable label, such as a fluorescent, radioisotopic, chemiluminescent, or enzymatic label, such as horseradish peroxidase, alkaline phosphatase, or luciferase. Detection of a test compound which is bound to a "BREAST CANCER GENE" polypeptide can then be accomplished, for example, by direct counting of radioemmission, by scintillation counting, or by determining conversion of an appropriate substrate to a detectable product.

Alternatively, binding of a test compound to a "BREAST CANCER GENE" polypeptide can be determined without labeling either of the interactants. For example, a microphysiometer can be used to detect binding of a test compound with a "BREAST CANCER GENE" polypeptide. A microphysiometer (e.g., CytosensorJ) is an analytical instrument that measures the rate at which a cell acidifies its environment using a light-addressable potentiometric sensor (LAPS). Changes in this acidification rate can be used as an indicator of the interaction between a test compound and a "BREAST CANCER GENE" polypeptide [McConnell et al., 1992, (98)].

Determining the ability of a test compound to bind to a "BREAST CANCER GENE" polypeptide also can be accomplished using a technology such as real-time Bimolecular Interaction Analysis (BIA) [Sjolander & Urbaniczky, 1991, (99), and Szabo et al., 1995, (100)]. BIA is a technology for studying biospecific interactions in real time, without labeling any of the interactants (e.g., BIAcore™). Changes in the optical phenomenon surface plasmon resonance (SPR) can be used as an indication of real-time reactions between biological molecules.

In yet another aspect of the invention, a "BREAST CANCER GENE" polypeptide can be used as a "bait protein" in a two-hybrid assay or three-hybrid assay [see, e.g., U.S. Patent 5,283,317; Zervos et al., 1993, (101); Madura et al., 1993, (102); Bartel et al., 1993, (1034); Iwabuchi et al., 1993, (104) and Brent WO 94/10300], to identify other proteins which bind to or interact with the "BREAST CANCER GENE" polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a "BREAST CANCER GENE" polypeptide can be fused to a polynucleotide encoding the DNA binding domain of a known transcription factor (e.g., GAL4). In the other construct a DNA sequence that encodes an unidentified protein ("prey" or "sample") can be fused to a polynucleotide that codes for the activation domain of the known transcription factor. If the "bait" and the "prey" proteins are able to interact in vivo to form an protein- dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the "BREAST CANCER GENE" polypeptide.

It may be desirable to immobilize either a "BREAST CANCER GENE" polypeptide (or polynucleotide) or the test compound to facilitate separation of bound from unbound forms of one or both of the interactants, as well as to accommodate automation of the assay. Thus, either a "BREAST CANCER GENE" polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach a "BREAST CANCER GENE" polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a "BREAST CANCER GENE" polypeptide (or polynucleotide) can be accomplished in any vessel suitable for containing the reactants. Examples of such vessels include microtiter plates, test tubes, and microcentrifuge tubes.

In one embodiment, a "BREAST CANCER GENE" polypeptide is a fusion protein comprising a domain that allows the "BREAST CANCER GENE" polypeptide to be bound to a solid support. For example, glutathione S-transferase fusion proteins can be adsorbed onto glutathione sepharose beads (Sigma Chemical, St. Louis, Mo.) or glutathione derivatized microtiter plates, which are then combined with the test compound or the test compound and the nonadsorbed "BREAST CANCER GENE" polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilising proteins or polynucleotides on a solid support also can be used in the screening assays of the invention. For example, either a "BREAST CANCER GENE" polypeptide (or polynucleotide) or a test compound can be immobilized utilizing conjugation of biotin and streptavidin. Biotinylated "BREAST CANCER GENE" polypeptides (or polynucleotides) or test compounds can be prepared from biotin NHS (N-hydroxysuccinimide) using techniques well known in the art (e.g., biotinylation kit, Pierce Chemicals, Rockford, Ill.) and immobilized in the wells of streptavidin-coated 96 well plates (Pierce Chemical).

Alternatively, antibodies which specifically bind to a "BREAST CANCER GENE" polypeptide, polynucleotide, or a test compound, but which do not interfere with a desired binding site, such as the ATP/GTP binding site or the active site of the "BREAST CANCER GENE" polypeptide, can be derivatised to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to a "BREAST CANCER GENE" polypeptide or test compound, enzyme-linked assays which rely on detecting an activity of a "BREAST CANCER GENE" polypeptide, and SDS gel electrophoresis under non-reducing conditions.

Screening for test compounds which bind to a "BREAST CANCER GENE" polypeptide or polynucleotide also can be carried out in an intact cell. Any cell which comprises a "BREAST CANCER GENE" polypeptide or polynucleotide can be used in a cell-based assay system. A "BREAST CANCER GENE" polynucleotide can be naturally occurring in the cell or can be introduced using techniques such as those described above. Binding of the test compound to a "BREAST CANCER GENE" polypeptide or polynucleotide is determined as described above.

*Modulation of Gene Expression*

In another embodiment, test compounds which increase or decrease "BREAST CANCER GENE" expression are identified. A "BREAST CANCER GENE" polynucleotide is contacted with a test compound in an approriate expression test system as described below or in a cell system, and the expression of an RNA or polypeptide product of the "BREAST CANCER GENE" polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of "BREAST CANCER GENE" mRNA or polypeptide expression in the cells can be determined by methods well known in the art for detecting mRNA or polypeptide. Either qualitative or quantitative methods can be used. The presence of polypeptide products of a "BREAST CANCER GENE" polynucleotide can be determined, for example, using a variety of techniques known in the art, including immunochemical methods such as radioimmunoassay, Western blotting, and immunohistochemistry. Alternatively, polypeptide synthesis can be determined in vivo, in a cell culture, or in an in vitro translation system by detecting incorporation of labeled amino acids into a "BREAST CANCER GENE" polypeptide.

Such screening can be carried out either in a cell-free assay system or in an intact cell. Any cell which expresses a "BREAST CANCER GENE" polynucleotide can be used in a cell-based assay system. A "BREAST CANCER GENE" polynucleotide can be naturally occurring in the cell or can be introduced using techniques such as those described above. Either a primary culture or an established cell line, such as CHO or human embryonic kidney 293 cells, can be used.

*Therapeutic Indications and Methods*

Therapies for treatment of breast cancer primarily relied upon effective chemotherapeutic drugs for intervention on the cell proliferation, cell growth or angiogenesis. The advent of genomics-driven molecular target identification has opened up the possibility of identifying new breast cancer-specific targets for therapeutic intervention that will provide safer, more effective treatments for malignant neoplasia patients and breast cancer patients in particular. Thus, newly discovered breast cancer-associated genes and their products can be used as tools to develop innovative therapies. The identification of the Her2/neu receptor kinase presents exciting new opportunities for treatment of a certain subset of tumor patients as described before. Genes playing important roles in any of the physiological processes outlined above can be characterized as breast cancer targets. Genes or gene fragments identified through genomics can readily be expressed in one or more heterologous expression systems to produce functional recombinant proteins. These proteins are characterized in vitro for their biochemical properties and then used as tools in high-throughput molecular screening programs to identify chemical modulators of their biochemical activities. Modulators of target gene expression or protein activity can be identified in this manner and subsequently tested in cellular and in vivo disease models for therapeutic activity. Optimization of lead compounds with iterative testing in biological models and detailed pharmacokinetic and toxicological analyses form the basis for drug development and subsequent testing in humans.

This invention further pertains to the use of novel agents identified by the screening assays described above. Accordingly, it is within the scope of this invention to use a test compound identified as described herein in an appropriate animal model. For example, an agent identified as described herein (e.g., a modulating agent, an antisense polynucleotide molecule, a specific antibody, ribozyme, or a human "BREAST CANCER GENE" polypeptide binding molecule) can be used in an animal model to determine the efficacy, toxicity, or side effects of treatment with such an agent. Alternatively, an agent identified as described herein, can be used in an animal model to determine the mechanism of action of such an agent. Furthermore, this invention pertains to uses of novel agents identified by the above described screening assays for treatments as described herein.

A reagent which affects human "BREAST CANCER GENE" activity can be administered to a human cell, either in vitro or in vivo, to reduce or increase human "BREAST CANCER GENE" activity. The reagent preferably binds to an expression product of a human "BREAST CANCER GENE". If the expression product is a protein, the reagent is preferably an antibody. For treatment of human cells ex vivo, an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 $\mu$g of DNA per 16 nmol of liposome delivered to about $10^6$ cells, more preferably about 1.0 $\mu$g of DNA per 16 nmol of liposome delivered to about $10^6$ cells, and even more preferably about 2.0 $\mu$g of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present invention include those liposomes usually used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell type, such as a cell-specific ligand exposed on the outer surface of the liposome.

Complexing a liposome with a reagent such as an antisense oligonucleotide or ribozyme can be achieved using methods which are standard in the art (see, for example, U.S. Patent 5,705,151).

Preferably, from about 0.1 $\mu$g to about 10 $\mu$g of polynucleotide is combined with about 8 nmol of liposomes, more preferably from about 0.5 $\mu$g to about 5 $\mu$g of polynucleotides are combined with about 8 nmol liposomes, and even more preferably about 1.0 $\mu$g of polynucleotides is combined with about 8 nmol liposomes.

In another embodiment, antibodies can be delivered to specific tissues in vivo using receptor-mediated targeted delivery. Receptor-mediated DNA delivery techniques are taught in, for example, Findeis et al., 1993, (105); Chiou et al., 1994, (106); Wu & Wu, 1988, (107); Wu et al., 1994, (108); Zenke et al., 1990, (109); Wu et al., 1991, (110).

*Determination of a Therapeutically Effective Dose*

The determination of a therapeutically effective dose is well within the capability of those skilled in the art. A therapeutically effective dose refers to that amount of active ingredient which increases or decreases human "BREAST CANCER GENE" activity relative to the human "BREAST CANCER GENE" activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either ex vivo or in vivo using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, a gene gun, and DEAE- or calcium phosphate-mediated transfection.

Effective in vivo dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective in vivo dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a "BREAST CANCER GENE" gene or the activity of a "BREAST CANCER GENE" polypeptide by at least about 10, preferably about 50, more preferably about 75, 90, or 100% relative to the absence of the reagent. The effectiveness of the mechanism chosen to decrease the level of expression of a "BREAST CANCER GENE" gene or the activity of a "BREAST CANCER GENE" polypeptide can be assessed using methods well known in the art, such as hybridization of nucleotide probes to "BREAST CANCER GENE"-specific mRNA, quantitative RT-PCR, immunologic detection of a "BREAST CANCER GENE" polypeptide, or measurement of "BREAST CANCER GENE" activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, birds and mammals such as dogs, cats, cows, pigs, sheep, goats, horses, rabbits, monkeys, and most preferably, humans.

All patents and patent applications cited in this disclosure are expressly incorporated herein by reference. The above disclosure generally describes the present invention. A more complete understanding can be obtained by reference to the following specific examples which are provided for purposes of illustration only and are not intended to limit the scope of the invention.

*Pharmaceutical Compositions*

The invention also provides pharmaceutical compositions which can be administered to a patient to achieve a therapeutic effect. Pharmaceutical compositions of the invention can comprise, for example, a "BREAST CANCER GENE" polypeptide, "BREAST CANCER GENE" polynucleotide, ribozymes or antisense oligonucleotides, antibodies which specifically bind to a "BREAST CANCER GENE" polypeptide, or mimetics, agonists, antagonists, or inhibitors of a "BREAST CANCER GENE" polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intraarterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintergrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, e.g., by means of conventional mixing, dissolving, granulating, dragee making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 150 mM histidine, 0.1%2% sucrose, and 27% mannitol, at a pH range of 4.5 to 5.5, that is combined with buffer prior to use.

Further details on techniques for formulation and administration can be found in the latest edition of REMINGTON'S PHARMACEUTICAL SCIENCES (111). After pharmaceutical compositions have been prepared, they can be placed in an appropriate container and labeled for treatment of an indicated condition. Such labeling would include amount, frequency, and method of administration.

One strategy for identifying genes that are involved in breast cancer is to detect genes that are expressed differentially under conditions associated with the disease versus non-disease or in the context of therapy response conditions. The sub-sections below describe a number of experimental systems which can be used to detect such differentially expressed genes. In general, these experimental systems include at least one experimental condition in which subjects or samples are treated in a manner associated with breast cancer, in addition to at least one experimental control condition lacking such disease associated treatment or does not respond to such treatment. Differentially expressed genes are detected, as described below, by comparing the pattern of gene expression between the experimental and control conditions.

Once a particular gene has been identified through the use of one such experiment, its expression pattern may be further characterized by studying its expression in a different experiment and the findings may be validated by an independent technique. Such use of multiple experiments may be useful in distinguishing the roles and relative importance of particular genes in breast cancer and the treatment thereof. A combined approach, comparing gene expression pattern in cells derived from breast cancer patients to those of *in vitro* cell culture models can give substantial hints on the pathways involved in development and/or progression of breast cancer. It can also elucidate the role of such genes in the development of resistance or insensitivity to certain therapeutic agents (e.g. chemotherapeutic drugs).

Among the experiments which may be utilized for the identification ,of differentially expressed genes involved in malignant neoplasia and breast cancer in paticular, are experiments designed to analyze those genes which are involved in signal transduction. Such experiments may serve to identify genes involved in the proliferation of cells.

Below are methods described for the identification of genes which are involved in breast cancer. Such represent genes which are differentially expressed in breast cancer conditions relative to their expression in normal, or non-breast cancer conditions or upon experimental manipulation based on clinical observations. Such differentially expressed genes represent "target" and/or "marker" genes. Methods for the further characterization of such differentially expressed genes, and for their identification as target and/or marker genes, are presented below.

Alternatively, a differentially expressed gene may have its expression modulated, i.e., quantitatively increased or decreased, in normal versus breast cancer states, or under control versus experimental conditions. The degree to which expression differs in normal versus breast cancer or control versus experimental states need only be large enough to be visualized via standard characterization techniques, such as, for example, the differential display technique described below. Other such standard characterization techniques by which expression differences may be visualized include but are not limited to quantitative RT-PCR and Northern analyses, which are well known to those of skill in the art.

In Addition to the experiments described above the following describes algorithms and statistical analyses which can be utilized for data evaluation and for the classification as well as response prediction for a sofar not classsified biological sample in the context of control samples. Predictive algorithms and equations described below have already shown their power to subdivide individual cancers.

**EXAMPLE 1**

*Expression profiling utilizing quantitative kinetic RT-PCR*

For a detailed analysis of gene expression by quantitative PCR methods, one will utilize primers flanking the genomic region of interest and a fluorescent labeled probe hybridizing in-between. Using the PRISM 7700 Sequence Detection System of PE Applied Biosystems (Perkin Elmer, Foster City, CA, USA) with the technique of a fluorogenic probe, consisting of an oligonucleotide labeled with both a fluorescent reporter dye and a quencher dye, one can perform such a expression measurement. Amplification of the probe-specific product causes cleavage of the probe, generating an increase in reporter fluorescence. Primers and probes were selected using the Primer Express software and localized mostly in the 3' region of the coding sequence or in the 3' untranslated region (see Table 5 for primer- and probe-sequences). All primer pairs were checked for specificity by conventional PCR reactions and gel electrophoresis. To standardize the amount of sample RNA, GAPDH was selected as a reference, since it was not differentially regulated in the samples analyzed. To performe such an expression analysis of genes within a biological samples the respective primer/probes are prepared by mixing 25 $\mu$l of the 100 $\mu$M stock solution "Upper Primer", 25 $\mu$l of the 100 $\mu$M stock solution "Lower Primer" with 12,5 $\mu$l of the 100 $\mu$M stock solution TaqMan-probe (FANVTamra) and adjusted to 500 $\mu$l with aqua dest (Primer/probe-mix). For each reaction 1,25 $\mu$l cDNA of the patient samples were mixed with 8,75 $\mu$l nuclease-free water and added to one well of a 96 Well-Optical Reaction Plate (Applied Biosystems Part No. 4306737). 1,5 $\mu$l of the Primer/Probe-mix described above, 12,5$\mu$l Taq Man Universal-PCR-mix (2x) (Applied Biosystems Part No. 4318157) and 1 $\mu$l Water are then added. The 96 well plates are closed with 8 Caps/Strips (Applied Biosystems Part Number 4323032) and centrifuged for 3 minutes. Measurements of the PCR reaction are done according to the instructions of the manufacturer with a TaqMan 7900 HT from Applied Biosystems (No. 20114) under appropriate conditions (2 min. 50˚C, 10 min. 95˚C, 0.15min. 95˚C, 1 min. 60˚C; 40 cycles). Prior to the maesurement of so far unclassified biological samples control expreiments will e.g. cell lines, healthy control samples, samples of defined therapy response could be used for standardization of the experimental conditions.

TaqMan validation experiments were performed showing that the efficiencies of the target and the control amplifications are approximately equal which is a prerequisite for the relative quantification of gene expression by the comparative $\Delta\Delta C_T$ method, known to those with skills in the art. Herefor the SoftwareSDS 2.0 from Applied Biosystems can be used according to the respective instructions. CT-values are then further analyzed with appropriate software (Microsoft Excel™) of statistical software packages (SAS).

As well as the technology described above, provided by Perkin Elmer, one may use other technique implementations like Lightcycler ™ from Roche Inc. or iCycler from Stratagene Inc.capable of real time detection of an RT-PCR reaction.

**EXAMPLE 2**

*Expression profiling utilizing DNA microarrays*

Expression profiling can bee carried out using the Affymetrix Array Technology. By hybridization of mRNA to such a DNA-array or DNA-Chip, it is possible to identify the expression value of each transcripts due to signal intensity at certain position of the array. Usually these DNA-arrays are produced by spotting of cDNA, oligonucleotides or subcloned DNA fragments. In case of Affymetrix technology app. 400.000 individual oligonucleotide sequences were synthesized on the surface of a silicon wafer at distinct positions. The minimal length of oligomers is 12 nucleotides, preferable 25 nucleotides or full length of the questioned transcript. Expression profiling may also be carried out by hybridization to nylon or nitro-cellulose membrane bound DNA or oligonucleotides. Detection of signals derived from hybridization may be obtained by either colorimetric, fluorescent, electrochemical, electronic, optic or by radioactive readout. Detailed description of array construction have been mentioned above and in other patents cited. To determine the quantitative and qualitative changes in the chromosomal region to analyze, RNA from tumor tissue which is suspected to contain such genomic alterations has to be compared to RNA extracted from benign tissue (e.g. epithelial breast tissue, or micro dissected ductal tissue) on the basis of expression profiles for the whole transcriptome. With minor modifications, the sample preparation protocol followed the Affymetrix GeneChip Expression Analysis Manual (Santa Clara, CA). Total RNA extraction and isolation from tumor or benign tissues, biopsies, cell isolates or cell containing body fluids can be performed by using TRIzol (Life Technologies, Rockville, MD) and Oligotex mRNA Midi kit (Qiagen, Hilden, Germany), and an ethanol precipitation step should be carried out to bring the concentration to 1 mg/ml. Using 5-10 mg of mRNA to create double stranded cDNA by the SuperScript system (Life Technologies). First strand cDNA synthesis was primed with a T7-(dT24) oligonucleotide. The cDNA can be extracted with phenol/chloroform and precipitated with ethanol to a final concentration of 1mg /ml. From the generated cDNA, cRNA can be synthesized using Enzo's (Enzo Diagnostics Inc., Farmingdale, NY) *in vitro* Transcription Kit. Within the same step the cRNA can be labeled with biotin nucleotides Bio-11-CTP and Bio-16-UTP (Enzo Diagnostics Inc., Farmingdale, NY) . After labeling and cleanup (Qiagen, Hilden (Germany) the cRNA then should be fragmented in an appropriated fragmentation buffer (e.g., 40 mM Tris-Acetate, pH 8.1, 100 mM KOAc, 30 mM MgOAc, for 35 minutes at 94 ˚C). As per the Affymetrix protocol, fragmented cRNA should be hybridized on the HG_U133 arrays A and B, comprising app. 40.000 probed transcripts each, for 24 hours at 60 rpm in a 45 ˚C hybridization oven. After Hybridization step the chip surfaces have to be washed and stained with streptavidin phycoerythrin (SAPE; Molecular Probes, Eugene, OR) in Affymetrix fluidics stations. To amplify staining, a second labeling step can be introduced, which is recommended but not compulsive. Here one should add SAPE solution twice with an antistreptavidin biotinylated antibody. Hybridization to the probe arrays may be detected by fluorometric scanning (Hewlett Packard Gene Array Scanner; Hewlett Packard Corporation, Palo Alto, CA).

After hybridization and scanning, the microarray images can be analyzed for quality control, looking for major chip defects or abnormalities in hybridization signal. Therefor either Affymetrix GeneChip MAS 5.0 Software or other microarray image analysis software can be utilized. Primary data analysis should be carried out by software provided by the manufacturer..

In case of the genes analyses in one embodiment of this invention the primary data have been analyzed by further bioinformatic tools and additional filter criteria. The bioinformatic analysis is described in detail below.

EXAMPLE 3

*Data analysis from expression profiling experiments*

According to Affymetrix measurement technique (Affymetrix GeneChip Expression Analysis Manual, Santa Clara, CA) a single gene expression measurement on one chip yields the average difference value and the absolute call. Each chip contains 16-20 oligonucleotide probe pairs per gene or cDNA clone. These probe pairs include perfectly matched sets and mismatched sets, both of which are necessary for the calculation of the average difference, or expression value, a measure of the intensity difference for each probe pair, calculated by subtracting the intensity of the mismatch from the intensity of the perfect match. This takes into consideration variability in hybridization among probe pairs and other hybridization artifacts that could affect the fluorescence intensities. The average difference is a numeric value supposed to represent the expression value of that gene. The absolute call can take the values 'A' (absent), 'M' (marginal), or 'P' (present) and denotes the quality of a single hybridization. We used both the quantitative information given by the average difference and the qualitative information given by the absolute call to identify the genes which are differentially expressed in biological samples from individuals with breast cancer versus biological samples from the normal population. With other algorithms than the Affymetrix one we have obtained different numerical values representing the same expression values and expression differences upon comparison.

The differential expression E in one of the breast cancer groups compared to the normal population is calculated as follows. Given n average difference values $d_1, d_2, ..., d_n$ in the breast cancer population and m average difference values $c_1, c_2, ..., c_m$ in the population of normal individuals, it is computed by the equation:

$$E \equiv \exp\left(\frac{1}{m}\sum_{i=1}^{m}\ln(c_i)-\frac{1}{n}\sum_{i=1}^{n}\ln(d_i)\right) \text{ (equation 1)}$$

If $d_j$<50 or $c_i$<50 for one or more values of i and j, these particular values $c_i$ and/or $d_j$ are set to an "artificial" expression value of 50. These particular computation of E allows for a correct comparison to TaqMan results.

A gene is called up-regulated in breast cancer versus normal if E ≥ minimal change factor given in Table 3 and if the number of absolute calls equal to 'P' in the breast cancer population is greater than n/2. The minimal fold change factors in Table 3 are given for those patient populations responding to a given chemotherapy (CR), non responding to a administered chemotherapy (NC) or those tissues without any pathological signs of a tumor (NB). Fold changes greater than 1 refers to an increase in gene expression in the first names tissue sample compared to the second. This regulation factors are mean values and may differ individually, here the combined profiles of all 185 genes listed in Table 1a and 1b in a cluster analysis or a principle component analysis will indicate the classification group for such sample.

According to the above, a gene is called down-regulated in breast cancer versus normal if E≤ minimal change factor given in Table 3 and if the number of absolute calls equal to 'P' in the breast cancer population is greater than n/2. Values smaller than 1 describe an decreased expression of the given gene.

The minimal fold change factors given in Table 3 indicate also the relative up- and downregulation of those gene indicative of tumor presence. These genes do show in the comparison of any tumor tissue to the normal healthy counterpart (NT) the highest increase or decrease factors (e.g. SEQ ID: 43, 55, 65, or 162)

The final list of differentially regulated genes consists of all up-regulated and all down-regulated genes in biological samples from individuals with breast cancer versus biological samples from the normal population or of an individual response pattern. Those genes on this list which are interesting for a diagnostic or pharmaceutical application were finally validated by quantitative real time RT-PCR (see Example 1). If a good correlation between the expression values/behavior of a transcript could be observed with both techniques, such a gene is listed in Tables 1 to 5.

**EXAMPLE 4**

Analysis of differential gene expression patterns using support vector machines

Support vector machines (SVM) are well suited for two-class or multi-class pattern recognition (Weston and Watkins, 1999 (115); Vapnik, 1995 (116); Vapnik, 1998 (117); Burges, 1998 (118).

For the two-class classification problem, (e.g. tumor tissue vs. non tumor tissue, or therapy response vs. non response) assume that we have a set of samples, i.e., a series of input vectors

$$\overrightarrow{\mathbf{x}_i} \in \mathbf{R}^d \text{ (i = 1, 2, ..., m)}$$

with corresponding labels

$$y_i \in \{+1,-1\} \text{ (i = 1, 2, ..., m).}$$

Here, +1 and -1 indicate the two classes. To classify gene expression patterns of marker genes from Table 1a and 1b or 2 for describing the current tumor status or probable response to a therapeutic agent, the input vector dimension is equal to the number of different oligonucleotide types present on the oligonucleotide array or a subset hereof, and each input vector unit stands for the hybridization value of one specific oligonucleotide type.

The goal is to construct a binary classifier or derive a decision function from the available samples which has a small probability of misclassifying a future sample.

An SVM implements the following idea: it maps the input vectors

$$\overrightarrow{\mathbf{x}_i} \in \mathbf{R}^d$$

into a high-dimensional feature space

$$\Phi(\vec{\mathbf{x}}) \in H$$

and constructs an Optimal Separating Hyperplane (OSH), which maximizes the margin, the distance between the hyperplane and the nearest data points of each class in the space *H*. By choosing OSH from among the many that can separate the positive from the negative examples in the feature space, SVMs are avoiding the risk of overfitting.

Different mappings construct different SVMs. The mapping

$$\Phi : \mathbf{R}^d \mapsto H$$

is performed by a kernel function

$$K(\overrightarrow{x_i}, \overrightarrow{x_j})$$

which defines an inner product in the space *H*.

The decision function implemented by SVM can be written as (Burges, 1998 (118):

$$f(\vec{x}) = \mathrm{sgn}\left(\sum_{i=1}^{m} y_i \alpha_i \cdot K\left(\vec{\mathbf{x}}, \overrightarrow{\mathbf{x}_i}\right) + b\right) \quad \text{(equation 2)}$$

where the coefficients $\alpha_i$ are obtained by solving the following convex Quadratic Programming (QP) problem:

Maximize

$$\sum_{i=1}^{m} \alpha_i - \frac{1}{2} \sum_{i=1}^{m} \sum_{j=1}^{m} \alpha_i \alpha_j \cdot y_i y_j \cdot K\left(\vec{\mathbf{x}}_i, \vec{\mathbf{x}}_j\right)$$

subject to

$$0 \le \alpha_i \le C \quad \text{(equation 3)}$$

and

$$\sum_{i=1}^{m} \alpha_i y_i = 0$$

The regularity parameter *C* (equation 3) controls the trade off between margin and misclassification error. The $\overleftarrow{x}j$ are called Support Vectors only if the corresponding $\alpha_i > 0$.

Two of the kernel functions used in the current example:

$$K\left(\overrightarrow{\mathbf{x}_i}, \overrightarrow{\mathbf{x}_j}\right) = \left(\overrightarrow{\mathbf{x}_i} \cdot \overrightarrow{\mathbf{x}_j} + 1\right)^d \qquad \text{(equation 4)}$$

$$K\left(\overrightarrow{\mathbf{x}_i}, \overrightarrow{\mathbf{x}_j}\right) = e^{\left(-r\left\|\overrightarrow{\mathbf{x}_i} - \overrightarrow{\mathbf{x}_j}\right\|^2\right)} . \qquad \text{(equation 5)}$$

where the first one (equation 4) is called the polynomial kernel function of degree *d* which will eventually revert to the linear function when *d* = 1, the latter (equation 5) is called the Radial Basic Function (RBF) kernel.

For a given data set, only the kernel function and the regularity parameter C must be selected to specify one SVM. An SVM has many attractive features. For instance, the solution of the QP problem is globally optimised while with neural networks the gradient based training algorithms only guarantee finding a local minima. In addition, SVM can handle large feature spaces, can effectively avoid overfitting (see above) by controlling the margin, can automatically identify a small subset made up of informative points, i.e., the Support Vectors, etc.

The classification of biological sample and thereby the identification of an neoplastic lesion as well as the response of such lesion to therapeutic agents based on gene expression data is a multi-class classification problem. The class number k is equal to the number tumor subcalsses (e.g. histological features, TNM stage, grade, hormonal status) and is equal to response subgroupe to a certain therapeutic agent (e.g. pathologicaly confirmed complete remission, good remission, partial remission, or no remission, as well as progressive disease) which shall be predicted, i.e., which are present in the training data set. Due to the limited number of different classes in the present sample set, we decided to handle the multi-class classification by reducing the multi-classification to a series of binary classifications. For a *k*-class classification, *k* SVMs are constructed. The *i*th SVM will be trained with all of the samples in the ith class with positive labels and all other samples with negative labels. Finally an unknown sample is classified into the class that corresponds to the SVM with the highest output value. This method is used to construct a prediction/classification system for gene expression patterns of differentially expressed marker genes as given in Table 1a and 1b and 2.

Each data point generated by a microarray hybridization experiment or by real time RT-PCR (cf. example 1 and 2) corresponds to and is determined by the number of mRNA copies present in the analysed sample, i.e., from an experiment with *n* oligonucleotide types on a polynucleotide array, a series of *n* expression-level values is obtained. These *n* values are typically stored in a metrics file which is the result of the analysis of a "cel file" by the Affymetrix® Microarray Suite or software described above. The data from a series of m metrics files (representing m expression analyses) are taken to build an expression matrix, in which each of the *m* rows consists of an n-element expression vector for a single experiment. In order to normalise the expression values of the m experiments, we define $x_{i,j}$ to be the sum of the logarithms of the expression level $a_{ij}$ for gene *j* (whose mRNA hybridizes with the oligonucleotide type *j*' present on the microarray, or gives a valid $\Delta\Delta C_T$ intesity), normalized so that the expression vector $\overleftarrow{\mathbf{x}}_i$ has the Euclidean length 1:

$$x_{j,i} = \frac{\ln(a_{i,j})}{\sqrt{\sum_{k=1}^{n} \ln(a_{i,k})^2}} \qquad \text{(equation 6)}$$

Initial analyses are carried out using a set of 20000-element expression vectors for 150 experiments as described in example 1 and 2 (100 experiments in the training set and 50 in the test set).

Using the knowledge that the 150 experiments represent three different response classes and two different tumor states as well as the information of tumor and non-tumor tissue, we trained the SVMs described above with the training set to recognize those response classes and disease states. The test set was used to assess the prediction accuracy. Here we have preformed crossvalidations utilizing the "leave one out" method and for more stringent testing a four to five fold validation (leave 25% out) with n iterations (n>100).

In such crossvalidations and classification experiments the predictive power of a subset of marker genes chosen from Table 1a and 1b (e.g. SEQ ID: 27, 38, 55, 81, 97, 98) has been tested. The average cross validation error rate was 8.333 % with affinity levels as follows:

| Tissue sample | True response | Predicted CR | Predicted NC |
|---|---|---|---|
| Sample_1 | CR | 0.9141 | -0.9141 |
| Sample_2 | CR | 1.281 | -1.281 |
| Sample_3 | CR | 1.149 | -1.149 |
| Sample_4 | CR | 0.3987 | -0.3987 |
| Sample_5 | CR | 0.2182 | -0.2182 |
| Sample_6 | CR | 0.7127 | -0.7127 |
| Sample_7 | NC | -1.124 | 1.124 |
| Sample_8 | NC | -1.492 | 1.492 |
| Sample_9 | NC | -1.896 | 1.896 |
| Sample_10 | NC | 0.475 | -0.475 |
| Sample_11 | NC | -1.962 | 1.962 |
| Sample_12 | NC | -0.7557 | 0.7557 |

The misclassification of one sample can be compensated by addition of more marker genes from Table 1a and 1b. These data show the minimal number of marker genes that could be combined for a predictive assay or kit.

## EXAMPLE 5

In order to optimize prediction of non responding tumor samples one may use this class from the trainings cohort and run multiple statistical tests, suitable for group comparison such as t-test or Wilcoxon. As listed in Table 6 one can identify such genes with a differential expression in the non responding tumor tissue and a significance level (p-value) below 0.05. In Table 6 20 genes are selected fulfilling the criterion of low p-value and high expressional fold change between the two classes.

One may combine the gene list selected as most preffered given in Table 2 with those genes from Table 1b and performe classification expriments for any sofar unclassified sample and predict response to chemotherapy.

While as those algorithms described in Example 4 can be implemented in a certain kernel to classify samples according to their specific gene expression into two classes another approach can be taken to predict class membership by implementation of a k-NN classification. The method of k-Nearest Neighbors (k-NN), proposed by T. M. Cover and P. E. Hart, an important approach to nonparametric classification, is quite easy and efficient. Partly because of its perfect mathematical theory, NN method develops into several variations. As we know, if we have infinitely many sample points, then the density estimates converge to the actual density function. The classifier becomes the Bayesian classifier if the large-scale sample is provided. But in practice, given a small sample, the Bayesian classifier usually fails in the estimation of the Bayes error especially in a high-dimensional space, which is called the disaster of dimension. Therefore, the method of k-NN has a great pity that the sample space must be large enough.

In k-nearest-neighbor classification, the training data set is used to classify each member of a "target" data set. The structure of the data is that there is a classification (categorical) variable of interest (e.g. "responder" (CR) or "non-responder" (NC)), and a number of additional predictor variables (gene expression values). Generally speaking, the algorithm is as follows:

1. For each sample in the data set to be classified, locate the k nearest neighbors of the training data set. A Euclidean Distance measure can be used to calculate how close each member of the training set is to the target sample that is being examined.

2. Examine the k nearest neighbors - which classification do most of them belong to? Assign this category to the sample being examined.

3. Repeat this procedure for the remaining samples in the target set.

Of course the computing time goes up as k goes up, but the advantage is that higher values of k provide smoothing that reduces vulnerability to noise in the training data. In practical applications, typically, k is in units or tens rather than in hundreds or thousands.

The "nearest neighbors" are determined if given the considered the vector and the distance measurement. Given a training set of expression values for a certain number of samples

$T = \{(\mathbf{x}1, y1), (x2, y2), \cdots, (xm, ym)\}$, to determine the class of the input vector x.

The most special case is the k-NN method, while k= 1, which just searches the one nearest neighbor:

$$j = argmin //\mathbf{x} - \mathbf{x}i//$$

then, (x, *yj*) is the solution.

For estimation on the error rate of this classification the following considerations could be made:

A training set $T = \{(x1, y1), (x2, y2),\cdots, (xm, ym)\}$ is called (*k, d*%)-*stable* if the error rate of *k*-NN method is *d*%, where *d*% is the empirical error rate from independent experiments. If the clustering of data are quite distinct (the class distance is the crucial standard of classification), then the *k* must be small. The key idea is we prefer the least *k* in the case that *d*% is bigger the threshold value.

The *k*-NN method gathers the nearest *k* neighbors and let them vote - the class of most neighbors wins. Theoretically, the more neighbors we consider, the smaller error rate it takes place. The general case is a little more complex. But by imagination, it is true to be the more

*k* the lower upper bound asymptotic to PBayes(e) if *N* is fixed.

One can use such algorithm to classify and cross validate a given cohort of samples based on the genes presented by this invention in Tables 1a and 1b. Most preferably the classification shall be performed based on the expression levels of the genes presented in Table 1b in combination with the genes from Table 2. With k = 3 and > 100 iteration one can get classifications as depicted below for a cross-validation experiment with the three classes "normal breast tissue" (not affected by cancer), non responding tumor (NC), and responding tumor (CR). Affinities ranging from -1 to 1 for a given class.

| Tissue sample | True response | Predicted breast | normal | Predicted-NC | Predicted-CR | Remarks |
|---|---|---|---|---|---|---|
| "normal" tissue | | | 1 | -0.5 | -0.5 | |
| Sample_1 | CR | | -0.4994 | -0.5 | 0.9994 | |
| Sample_2 | CR | | -0.4988 | -0.5 | 0.9988 | |
| Sample_3 | CR | | -0.4988 | -0.5 | 0.9988 | |
| Sample_4 | CR | | -0.5 | -0.5 | 1 | |
| Sample_5 | CR | | -0.4988 | -0.5 | 0.9988 | |
| Sample_6 | CR | | -0.5 | -0.5 | 1 | |
| Sample_7 | CR | | -0.5 | -0.4988 | 0.9988 | |
| Sample_8 | CR | | -0.4883 | -0.4649 | 0.9532 | |
| Sample_9 | NC | | -0.497 | 0.997 | -0.5 | |
| Sample_10 | NC | | -0.4969 | 0.9969 | -0.5 | |
| Sample_11 | NC | | -0.4975 | 0.9975 | -0.5 | |
| Sample_12 | NC | | -0.4982 | 0.9982 | -0.5 | |
| Sample_13 | NC | | 1 | -0.5 | -0.5 | low tumor % |
| Sample_14 | NC | | -0.5 | -0.4988 | 0.9988 | false |
| Sample_15 | NC | | -0.4976 | 0.9976 | -0.5 | |
| Sample_16 | NC | | -0.4976 | 0.9976 | -0.5 | |

The misclassification of one sample can be compensated by addition of more marker genes from Table 1a. These data show the minimal number of marker genes that could be combined for a predictive assay or kit.

**EXAMPLE 6**

In order to get the most accurate prediction for response to chemotherapy based on the expression levels of genes listed in Tables 1a and Table 1b. One can implement a step wise classification model identifying first those individuals (tumor tissues) with the highes affinity (e.g. by k-NN classification) to the class of responding tumors (CR). If an sofar unclassified tumor sample did not belong to the class of CR on may performe a second classification step for this sample unsing the expression levels of the genes from Table 1a (e.g. SEQ ID Nos: 2, 8, 9, 21, 24, 35, 53, 54, 57, 64, 80, 87, 89, 95, 97, 118 and 146) which will give in a k-NN classification a better separation of the non responding tumors from those which will respond partially. For this second classification step only the predefined classes NC and PR should be utilized.

**References**

*Patents cited*

U.S. Pat. No. 4,843,155Chomczynski, P.
U.S. Pat. No. 5,262,31Liang, P., and Pardee, A. B., 1993
U.S. Pat. No. 4,683,202Mullis, K. B., 1987
U.S. Pat. No. 5,593,839
U.S. Pat. No. 5,578,832
U.S. Pat. No. 5,556,752
U.S. Pat. No. 5,631,734
U.S. Pat. No. 5,599,695
U.S. Pat. No. 4,683,195
U.S. Pat. No. 5,498,531
U.S. Pat. No. 5,714,331
U.S. Pat. No. 5,641,673Haseloff et al.,
U.S. Pat. No. 5,223,409Lander, E.,
U.S. Pat. No. 5,976,813Beutel et al.
U.S. Pat. No. 5,283,317
U.S. Pat No. 6,203,987

WO 97/29212
WO 97/27317
WO 95/22058
WO 99/12826
WO 97/02357
WO 94/13804
WO 94/10300
EP 0 785 280
EP 0 799 897
EP 0 728 520
EP 0 721 016
EP 0 321 201
GB2188638B


*Other references cited*

(1) Publications cited:WHO. International Classification of Diseases, 10th edition (ICD-10). WHO
(2) Sabin, L.H., Wittekind, C. (eds): TNM Classification of Malignant Tumors. Wiley, New York, 1997
(3) Sorlie et al., Proc Natl Acad Sci U S A. 2001 Sep 11;98(19):10869-74 (3);
(4) van't Veer et al., Nature. 2002 Jan 31;415(6871):530-6. (4).
(5) Perez, E.A.: Current Managment of Metastatic Breast Cancer. Semin. Oncol., 1999; 26 (Suppl.12): 1-10
(6) Sambrook et al., MOLECULAR CLONING: A LABORATORY MANUAL, 2d ed., 1989

(7) Ausubel et al., CURRENT PROTOCOLS IN MOLECULAR BIOLOGY, John Wiley & Sons, New York, N.Y., 1989.
(8) Tedder, T. F. et al., Proc. Natl. Acad. Sci. U.S.A. 85:208-212, 1988
(9) Hedrick, S. M. et al., Nature 308:149-153, 1984
(10) Lee, S. W. et al., Proc. Natl. Acad. Sci. U.S.A. 88:4225, 1984
(11) Sarkar, PCR Methods Applic. 2, 318-322, 1993
(12) Triglia et al., Nucleic Acids Res. 16, 81-86, 1988
(13) Lagerstrom et al., PCR Methods Applic. 1, 111-119, 1991
(14) Copeland & Jenkins, Trends in Genetics 7: 113-118, 1991
(15) Cohen, et al., Nature 366: 698-701, 1993
(16) Bonner et al., J. Mol. Biol. 81, 123 1973
(17) Bolton and McCarthy, Proc. Natl. Acad. Sci. U.S.A. 48, 1390 1962
(19) Altschul et al., Bull. Math. Bio. 48:603, 1986,
(20) Henikoff & Henikoff, Proc. Natl. Acad. Sci. USA 89:10915, 1992
(21) Pearson & Lipman, Proc. Nat'1 Acad. Sci. USA 85:2444, 1988
(22) Pearson et al., Meth. Enzymol. 183:63, 1990
(23) Needleman & Wunsch, J. Mol. Biol.48:444, 1970
(24) Sellers, SIAM J. Appl. Math.Xno:787, 1974
(25) Takamatsu, EMBO J. 6, 307-311, 1987
(26) Coruzzi et al., EMBO J. 3, 1671-1680, 1984
(27) Broglie et al., Science 224, 838-843, 1984
(28) Winter et al., Results Probl. Cell Differ. 17, 85-105, 1991 1
(29) Engelhard et al., Proc. Nat. Acad. Sci. 91, 3224-3227, 1994
(30) Logan & Shenk, Proc. Natl. Acad. Sci. 81, 3655-3659, 1984
(31) Scharf et al., Results Probl. Cell Differ. 20, 125-162, 1994
(32) Freshney R.I., ed., ANIMAL CELL CULTURE , 1986
(33) Wigler et al., Cell 11, 223-232, 1977
(34) Lowy et al., Cell 22, 817-823, 1980
(35) Wigler et al., Proc. Natl. Acad. Sci. 77, 3567-3570, 1980
(36) Colbere-Garapin et al., J. Mol. Biol. 150, 114, 1981
(37) Hartman & Mulligan, Proc. Natl. Acad. Sci. 85, 8047-8051, 1988
(38) Rhodes et al., Methods Mol. Biol. 55, 121-131, 1995
(39) Hampton et al., SEROLOGICAL METHODS: A LABORATORY MANUAL, APS Press, St. Paul, Minn., 1990
(40) Maddox et al., J. Exp. Med. 158, 1211-1216, 1983
(41) Porath et al., Prot. Exp. Purif. 3, Xno3-281, 1992
(42) Kroll et al., DNA Cell Biol. 12, 441-453, 1993
(43) Caruthers et al., Nucl. Acids Res. Symp. Ser. 215-223, 1980
(44) Horn et al. Nucl. Acids Res. Symp. Ser. 225-232, 1980
(45) Merrifield, J. Am. Chem. Soc. 85, 2149-2154, 1963
(46) Roberge et al., Science Xno9, 202-204, 1995
(47) Creighton, PROTEINS: STRUCTURES AND MOLECULAR PRINCIPLES, WH and Co., New York, N.Y., 1983
(48) Cronin et al., Human Mutation 7:244, 1996
(49) Landegran et al., Science 241:1077-1080, 1988
(50) Nakazawa et al., PNAS 91:360-364, 1994
(51) Abravaya et al., Nuc Acid Res 23:675-682, 1995
(52) Guatelli, J.C. et al., Proc. Natl. Acad. Sci. USA 87:1874-1878, 1990
(53) Kwoh, D.Y. et al., Proc. Natl. Acad. Sci. USA 86:1173 -1177, 1989
(54) Lizardi, P.M. et al., Bio/Technology 6:1197, 1988
(55) Brown, Meth. Mol. Biol. 20, 18, 1994
(56) Sonveaux, Meth. Mol. Biol. Xno, 1-72, 1994
(57) Uhlmann et al., Chem. Rev. 90, 543-583, 1990
(58) Gee et al., in Huber & Carr, MOLECULAR AND IMMUNOLOGIC APPROACHES, Publishing Co., Mt. Kisco, N.Y., 1994
(59) Agrawal et al., Trends Biotechnol. 10, 152-158, 1992
(60) Uhlmann et al., Tetrahedron. Lett. 215, 3539-3542, 1987
(61) Cech, Science 236, 1532-1539, 1987
(62) Cech, Ann. Rev. Biochem. 59, 543-568, 1990
(63) Couture & Stinchcomb, Trends Genet. 12, 510-515, 1996
(64) Haseloff et al. Nature 334, 585-591, 1988

(65) Kohler et al., Nature 256, 495-497, 1985
(66) Kozbor et al., J. Immunol. Methods 81, 3142, 1985
(67) Cote et al., Proc. Natl. Acad. Sci. 80, 20Xno-2030, 1983
(68) Cole et al., Mol. Cell Biol. 62, 109-120, 1984
(69) Morrison et al., Proc. Natl. Acad. Sci. 81, 6851-6855, 1984
(70) Neuberger et al., Nature 312, 604-608, 1984
(71) Takeda et al., Nature 314, 452-454, 1985
(72) Burton, Proc. Natl. Acad. Sci. 88, 11120-11123, 1991
(73) Thirion et al., Eur. J. Cancer Prev. 5, 507-11, 1996
(74) Coloma & Morrison, Nat. Biotechnol. 15, 159-63, 1997
(75) Mallender & Voss, J. Biol. Chem. Xno9, 199-206, 1994
(76) Verhaar et al., Int. J. Cancer 61, 497-501, 1995
(77) Nicholls et al., J. Immunol. Meth. 165, 81-91, 1993
(78) Orlandi et al., Proc. Natl. Acad. Sci. 86, 3833-3837, 1989
(79) Winter et al., Nature 349, 293-299, 1991
(80) Lam, Anticancer Drug Des. 12, 145, 1997
(81) DeWitt et al., Proc. Natl. Acad. Sci. U.S.A. 90, 6909, 1993
(82) Erb et al. Proc. Natl. Acad. Sci. U.S.A. 91, 11422, 1994
(83) Zuckermann et al., J. Med. Chem. 37, Xno78, 1994
(84) Cho et al., Science Xno1, 1303, 1993
(85) Carell et al., Angew. Chem. Int. Ed. Engl. 33, 2059 & 2061, 1994
(86) Gallop et al., J. Med. Chem. 37, 1233, 1994
(87) Houghten, BioTechniques 13, 412-421, 1992
(88) Lam, Nature 354, 8284, 1991
(89) Fodor, Nature 364, 555-556, 1993
(90) Cull et al., Proc. Natl. Acad. Sci. U.S.A. 89, 1865-1869, 1992
(91) Scott & Smith, Science 249, 386-390, 1990
(92) Devlin, Science 249, 404-406, 1990
(93) Cwirla et al., Proc. Natl. Acad. Sci. 97, 6378-6382, 1990
(94) Felici, J. Mol. Biol. 222, 301-310, 1991
(95) Jayawickreme et al., Proc. Natl. Acad. Sci. U.S.A. 19, 1614-1618, 1994
(96) Chelsky, Strategies for Screening Combinatorial Libraries 1995
(97) Salmon et al., Molecular Diversity 2, 57-63, 1996
(98) McConnell et al., Science 257, 1906-1912, 1992
(99) Sjolander & Urbaniczky, Anal. Chem. 63, 2338-2345, 1991
(100) Szabo et al., Curr. Opin. Struct. Biol. 5, 699-705, 1995
(101) Zervos et al., Cell 72, 223-232, 1993
(102) Madura et al., J. Biol. Chem. Xno8, 12046-12054, 1993
(103) Bartel et al., BioTechniques 14, 920-924, 1993
(104) Iwabuchi et al., Oncogene 8, 1693-1696, 1993
(105) Findeis et al. Trends in Biotechnol. 11, 202-205, 1993
(106) Chiou et al., GENE THERAPEUTICS: METHODS AND APPLICATIONS OF DIRECT GENE TRANSFER J.A. Wolff, ed., 1994
(107) Wu & Wu, J. Biol. Chem. Xno3, 621-24, 1988
(108) Wu et al., J. Biol. Chem. Xno9, 542-46, 1994
(109) Zenke et al., Proc. Natl. Acad. Sci. U.S.A. 87, 3655-59, 1990
(110) Wu et al., J. Biol. Chem. Xno6, 338-42, 1991
(111) REMINGTON'S PHARMACEUTICAL SCIENCES Maack Publishing Co., Easton, Pa.
(112) Hille, Excitable Membranes, Sunderland, MA, Sinauer Associates, Inc.
(113) Van Heeke & Schuster, J. Biol. Chem. 264, 5503-5509, 1989
(114) Grant et al., Methods Enzymol. 153, 516-544, 1987
(115) Weston and Watkins, Proceedings of the Seventh European Symposium On Artificial Neural Networks, 1999
(116) Vapnik, The Nature of Statistical Learning Theory, 1995, Springer, New York
(117) Vapnik, Statistical Learning Theory, 1998, Wiley, New York
(118) Burges, Data Mining and Knowledge Discovery, 2(2):955-974, 1998

**Table 1a: List of 165 genes which are differentially expressed in responders compared to non-responders or normal healthy tissue. Reference is given to the SEQ ID NOs of the sequence listing.**

| SEQ ID NO: (DNA Sequence) | SEQ ID NO: Gene_ Symbol (Protein Sequence) | Ref. Sequences [A] | Gene_ID | Locus_Link_ID |
|---|---|---|---|---|
| 1 | 166 CTSB | NM_001908 | 4503138 | 1508 |
| 2 | 167 SSR1 | NM_003144 | 14781630 | 6745 |
| 3 | 168 STX8 | NM_002803 | 4506208 | 5701 |
| 4 | 169 KPNA2 | NM_002266 | 4504896 | 3838 |
| 5 | 170 CSE1L | NM_001316 | 18591914 | 1434 |
| 6 | 171 RHEB2 | NM_005614 | 18600748 | 6009 |
| 7 | 172 DKC1 | NM_001363 | 15011921 | 1736 |
| 8 | 1731GFBP4 | NM_001552 | 10835020 | 3487 |
| 9 | 174 SMC1L1 | NM_006306 | - | 8243 |
| 10 | 175 PWP1 | NM_007062 | 5902033 | 11137 |
| 11 | 176 HDAC2 | NM_001527 | 4557640 | 3066 |
| 12 | 177 PRKAB1 | NM_006253 | 18602783 | 5564 |
| 13 | 178 IMPDH2 | NM_000884 | 4504688 | 3615 |
| 14 | 179 UBE2A | NM_003336 | 4507768 | 7319 |
| 15 | 180 YR-29 | NM_014886 | 7662676 | 10412 |
| 16 | 181 MUF1 | NM_006369 | 5453747 | 10489 |
| 17 | 182 MYO10 | NM_012334 | 11037056 | 4651 |
| 18 | 183 EGFR | NM_005228 | 4885198 | 1956 |
| 19 | 184 IFRD1 | NM_001550 | 4504606 | 3475 |
| 20 | 185 CD2BP2 | NM_006110 | 5174408 | 10421 |
| 21 | 186 ARL3 | NM_004311 | 4757773 | 403 |
| 22 | 187 CCNB2 | NM_004701 | 10938017 | 9133 |
| 23 | 188 FMOD | NM_002023 | 18548671 | 2331 |
| 24 | 189 SLC7A8 | NM_012244 | 14751202 | 23428 |
| 25 | 190 E2-EPF | NM_014501 | 7657045 | 27338 |
| 26 | 191 AGT | NM_000029 | 4557286 | 183 |
| 27 | 192 FHL2 | NM_001450 | 4503722 | 2274 |
| 28 | 193 LDLC | NM_007357 | 6678675 | 22796 |
| 29 | 194 MGC16824 | NM_020314 | 10092674 | 57020 |
| 30 | 195 UGDH | NM_003359 | 4507812 | 7358 |
| 31 | 196 MAD2L1 | NM_002358 | 6466452 | 4085 |
| 32 | 197 DDB2 | NM_000107 | 4557514 | 1643 |
| 33 | 198 OS4 | NM_005730 | 5031964 | 10106 |
| 34 | 199 BCL2 | NM_000633 | 13646672 | 596 |
| 35 | 200 SEMA3C | NM_006379 | 5454047 | 10512 |
| 36 | 201 DTR | NM_001945 | 4503412 | 1839 |
| 37 | 202 GARP | NM_005512 | 5031706 | 2615 |
| 38 | 203 ACK1 | NM_005781 | 8922074 | 10188 |
| 39 | 204 EDG2 | NM_001401 | 16950637 | 1902 |
| 40 | 205 RARRES3 | NM_004585 | 8051633 | 5920 |
| 41 | 206 CCNH | NM_001239 | 17738313 | 902 |
| 42 | 207 PREP | NM_002726 | 4506042 | 5550 |
| 43 | 208 COL11A1 | NM_001854 | 18548530 | 1301 |
| 44 | 209 GALC | NM_000153 | 4557612 | 2581 |
| 45 | 210 HMGCS2 | NM_005518 | 5031750 | 3158 |
| 46 | 211 ZNF274 | NM_016324 | 7706506 | 10782 |
| 47 | 212 TFF1 | NM_003225 | 4507450 | 7031 |

(continued)

| SEQ ID NO: (DNA Sequence) | SEQ ID NO: Gene_ Symbol (Protein Sequence) | Ref. Sequences [A] | Gene_ID | Locus_Link_ID |
|---|---|---|---|---|
| 48 | 213 RAD51 | NM_002875 | 4506388 | 5888 |
| 49 | 214 ASNS | NM_001673 | 4502258 | 440 |
| 50 | 215 PCMT1 | NM_005389 | 4885538 | 5110 |
| 51 | 216 ESR1 | NM_000125 | 4503602 | 2099 |
| 52 | 217 ACAT1 | NM_000019 | 4557236 | 38 |
| 53 | 218 XPA | NM_000380 | 4507936 | 7507 |
| 54 | 219 LAF4 | NM_002285 | 4504938 | 3899 |
| 55 | 220 COL10A1 | NM_000493 | 18105031 | 1300 |
| 56 | 221 KIAA 1041 | NM_014947 | 15299048 | 22887 |
| 57 | 222 PLA2G7 | NM_005084 | 4826883 | 7941 |
| 58 | 223 GRP | NM_002091 | 4504158 | 2922 |
| 59 | 224 CYP2B6 | NM_000767 | 14550410 | 1555 |
| 60 | 225 CHAD | NM_001267 | 4502798 | 1101 |
| 61 | 226 GALNT10 | NM_017540 | 9055207 | 55568 |
| 62 | 227 GADD45B | NM_015675 | 9945331 | 4616 |
| 63 | 228 WBSCR20 | NM_017528 | 8923713 | 114049 |
| 64 | 229 BTBD2 | NM_017797 | 8923361 | 55643 |
| 65 | 230 PGR | NM_000926 | 4505766 | 5241 |
| 66 | 231 TBPL1 | NM_004865 | 4759233 | 9519 |
| 67 | 232 C4B | NM_000592 | 14577918 | 721 |
| 68 | 233 CCNG1 | NM_004060 | - | 900 |
| 69 | 234 PDHB | NM_000925 | 4505686 | 5162 |
| 70 | 235 HNRPDL | NM_005463 | 14110410 | 9987 |
| 71 | 236 TAF11 | NM_005643 | 5032150 | 6882 |
| 72 | 237 AMACR | NM_014324 | 14725899 | 23600 |
| 73 | 238 EMD | NM_000117 | 4557552 | 2010 |
| 74 | 239 NR2F1 | NM_005654 | 5032172 | 7025 |
| 75 | 240 HSF2 | NM_004506 | 6806888 | 3298 |
| 76 | 241 SPG4 | NM_014946 | - | 6683 |
| 77 | 242 TRIP11 | NM_004239 | 10863904 | 9321 |
| 78 | 243 OCLN | NM_002538 | 9257230 | 4950 |
| 79 | 244 CACNA1D | NM_000720 | - | 776 |
| 80 | 245 CYP2B7 | NR_001278 | 14550410 | 1556 |
| 81 | 246 FHL1 | NM_001449 | 4503720 | 2273 |
| 82 | 247 MSX2 | NM_002449 | 18560141 | 4488 |
| 83 | 248 PAI-RBP1 | NM_015640 | 7661625 | 26135 |
| 84 | 249 CLDN14 | NM_012130 | 18593128 | 23562 |
| 85 | 250 ITPK1 | NM_014216 | 18583687 | 3705 |
| 86 | 251 ERBB2 | NM_004448 | 4758297 | 2064 |
| 87 | 252 TP53 | NM_000546 | 8400737 | 7157 |
| 88 | 253 HSPA2 | NM_021979 | 13676856 | 3306 |
| 89 | 254 LIG1 | NM_015541 | 18554950 | 26018 |
| 90 | 255 GSS | NM_000178 | 4504168 | 2937 |
| 91 | 256 PRO1843 | NM_018507 | 8924082 | 55378 |
| 92 | 257 MKI67 | NM_002417 | 4505188 | 4288 |
| 93 | 258 BIK | NM_001197 | 7262371 | 638 |
| 94 | 259 KIAA0225 | D86978 | 18566873 | 23165 |
| 95 | 260 TNRC15 | AB014542 | 18550089 | 26058 |

(continued)

| SEQ ID NO: (DNA Sequence) | SEQ ID NO: Gene_ Symbol (Protein Sequence) | Ref. Sequences [A] | Gene_ID | Locus_Link_ID |
|---|---|---|---|---|
| 96 | 261 SFRS5 | NM_006925 | 5902077 | 6430 |
| 97 | 262 RPL17 | NM_000985 | 14591906 | 6139 |
| 98 | 263 GNG12 | NM_018841 | - | 55970 |
| 99 | 264 LAP1B | NM_015602 | 17488747 | 26092 |
| 100 | 265 LOC253782 | AL080192 | - | 253782 |
| 101 | 266 COL5A1 | NM_000093 | 18571690 | 1289 |
| 102 | 267 CXCL13 | NM_006419 | 5453576 | 10563 |
| 103 | 268 TTS-2.2 | AF055000 | 3231586CB1 | 57104 |
| 104 | 269 KIAA0056 | D29954 | 18578675 | 23310 |
| 105 | 270 FLJ22642 | AI700633 | - | - |
| 106 | 271 LOC113146 | W28438 | 15300131 | 113146 |
| 107 | 272 GPR126 | NM_020455 | 18562351 | 57211 |
| 108 | 273 PMSCL1 | NM_005033 | 4826921 | 5393 |
| 109 | 274 KIAA0418 | NM_014631 | 7662103 | - |
| 110 | 275 SULF1 | NM_015170 | 18571189 | 23213 |
| 111 | 276 KIAA0673 | NM_015102 | 14720169 | 261734 |
| 112 | 277 FLJ10803 | NM_018224 | - | 55744 |
| 113 | 278 DKFZp586M0723 | AL050227 | - | - |
| 114 | 279 C4A | NM_007293 | 14577920 | 720 |
| 115 | 280 ZAP3 | L40403 | 18597333 | 56252 |
| 116 | 281 NEK9 | NM_033116 | 14916458 | 91754 |
| 117 | 282 FLJ13125 | AK023187 | 14726621- | |
| 118 | 283 FMO5 | NM_001461 | 4503760 | 2330 |
| 119 | 284 COMP | NM_000095 | 4557482 | 1311 |
| 120 | 285 CSPG2 | NM_004385 | 4758081 | 1462 |
| 121 | 286 LOC151996 | AA418080 | 18554956 | - |
| 122 | 287 TFAP2B | NM_003221 | 4507442 | 7021 |
| 123 | 288 OR7E38P | AF065854 | 18544324 | 10821 |
| 124 | 289 RAB31 | NM_006868 | 5803130 | 11031 |
| 125 | 290 HSPC126 | NM_014166 | 14759175 | 29079 |
| 126 | 291 UMP-CMPK | NM_016308 | 7706496 | 51727 |
| 127 | 292 FLJ22195 | NM_022758 | 12232426 | 64771 |
| 128 | 293 DCTN4 | NM_016221 | 14733974 | 51164 |
| 129 | 294 FLJ20273 | NM_019027 | 9506670 | 54502 |
| 130 | 295 KIF4A | NM_012310 | 14765683 | 24137 |
| 131 | 296 THTP | NM_024328 | 13236576 | 79178 |
| 132 | 297 PLSCR4 | NM_020353 | 9966818 | 57088 |
| 133 | 298 FLJ11323 | NM_018390 | 8922994 | 55344 |
| 134 | 299 MGC11242 | NM_024320 | 13236560 | 79170 |
| 135 | 300 CEGP1 | NM_020974 | 10190747 | 57758 |
| 136 | 301 SRR | NM_021947 | 8922495 | 63826 |
| 137 | 302 HSPC177 | NM_015961 | 7705488 | 51510 |
| 138 | 303 MGC3103 | NM_024036 | 13128987 | 78999 |
| 139 | 304 FLJ20641 | NM_017915 | 8923595 | 55010 |
| 140 | 305 FLJ13646 | NM_024584 | 13375767 | 79635 |
| 141 | 306 KCNK15 | NM_022358 | 16507967 | 60598 |
| 142 | 307 RNASEL | NM_021133 | 10863928 | 6041 |

(continued)

| SEQ ID NO: (DNA Sequence) | SEQ ID NO: Gene_ Symbol (Protein Sequence) | Ref. Sequences [A] | Gene_ID | Locus_Link_ID |
|---|---|---|---|---|
| 143 | 308 CRSP6 | NM_004268 | 18577903 | 9440 |
| 144 | 309 COL5A2 | NM_000393 | 16554580 | 1290 |
| 145 | 310 LOC51218 | NM_016417 | 9994192 | 51218 |
| 146 | 311 APBB2 | NM_173075 | 18557629 | 323 |
| 147 | 312 yy15c12.s1 | N31716 | - | - |
| 148 | 313 AD037 | NM_032023 | 14042936 | 83937 |
| 149 | 314 FLJ20477 | AA203365 | 8923441 | - |
| 150 | 315 MARKL1 | NM_031417 | 13899224 | 57787 |
| 151 | 316 LUM | NM_002345 | 4505046 | 4060 |
| 152 | 317 COL3A1 | NM_000090 | 15149480 | 1281 |
| 153 | 318 COL1A1 | NM_000088 | 18587373 | 1277 |
| 154 | 319 BF | NM_001710 | 14550403 | 629 |
| 155 | 320 ADAM12 | NM_003474 | 13259517 | 8038 |
| 156 | 321 LOXL1 | NM_005576 | 5031882 | 4016 |
| 157 | 322 CEACAM6 | NM_002483 | 4505340 | 4680 |
| 158 | 323 MMP11 | NM_005940 | 13027795 | 4320 |
| 159 | 324 MMP1 | NM_002421 | 13027798 | 4312 |
| 160 | 325 MMP13 | NM_002427 | 13027796 | 4322 |
| 161 | 326 SERPINH1 | NM_001235 | 4757923 | 872 |
| 162 | 327 PITX1 | NM_002653 | 4505824 | 5307 |
| 163 | 328 RAD52 | NM_015419 | 18390318 | 25878 |
| 164 | 329 INHBA | NM_002192 | 4504698 | 3624 |
| 165 | 330 CSPG2 | NM_004385 | 4758081 | 1462 |

**Table 1b: List of 20 genes which are differentially expressed in non-responding tumors compared to tumors with at least a minor therapy assosiated regression or normal healthy tissue. Reference is given to the SEQ ID NOs of the sequence listing.**

| SEQ ID NO: (DNA Sequence) | SEQ ID NO: Gene_ Symbol (Protein Sequence) | Ref. Sequences [A] | UniGene_ID | Locus_Link_I D |
|---|---|---|---|---|
| 472 | 492 PRG1 | NM_002727 | 1908 | 5552 |
| 473 | 493 GBP1 | NM_002053 | 62661 | 2633 |
| 474 | 494 ALEX2 | NM_014782 | 48924 | 9823 |
| 475 | 495 CD53 | NM_000560 | 82212 | 963 |
| 476 | 496 VCAM1 | NM_001078 | 109225 | 7412 |
| 477 | 497 MAPT | NM_005910 | 101174 | 4137 |
| 478 | 498 EGR2 | NM_000399 | 1395 | 1959 |
| 479 | 499 TDO2 | NM_005651 | 183671 | 6999 |
| 480 | 500 ADAMDEC1 | NM_014479 | 145296 | 27299 |
| 481 | 501 TFEC | NM_012252 | 113274 | 22797 |
| 482 | 502 BTF3 | NM_001207 | 101025 | 689 |
| 483 | 503 FLNB | NM_001457 | 81008 | 2317 |
| 484 | 504 TFRC | NM_003234 | 77356 | 7037 |
| 485 | 505 EIF4B | NM_001417 | 93379 | 1975 |
| 486 | 506 MAPK3 | - | 861 | 5595 |
| 487 | 507 LOC161291 | - | 85335 | 161291 |
| 488 | 508 SLC1A1 | NM_004170 | 91139 | 6505 |
| 489 | 509 MST4 | NM_016542 | 23643 | 51765 |

(continued)

| SEQ ID NO: (DNA Sequence) | SEQ ID NO: Gene_ Symbol (Protein Sequence) | Ref. Sequences [A] | UniGene_ID | Locus_Link_I D |
|---|---|---|---|---|
| 490 | 510 BLAME | NM_014036 | 20450 | 56833 |
| 491 | 511 NME7 | NM_013330 | 274479 | 29922 |

**<u>Table 2:</u> List of 47 preferred genes which differentially expressed in responders compared to non responders or normal healthy tissue. Listed genes are preferred genes, e.g., for use in the assessment whether or not a subject is expected to respond or not to respond to a given mode of treatment.**

| SEQ ID NO: (DNA Sequence) | SEQ ID NO: Gene Symbol (Protein Sequence) | Ref. Sequences [A] | Gene_ID | Locus Link I D |
|---|---|---|---|---|
| 4 | 169 KPNA2 | NM_002266 | 4504896 | 3838 |
| 5 | 170 CSE1L | NM_001316 | 18591914 | 1434 |
| 6 | 171 RHEB2 | NM_005614 | 18600748 | 6009 |
| 7 | 172 DKC1 | NM_001363 | 15011921 | 1736 |
| 8 | 173 IGFBP4 | NM_001552 | 10835020 | 3487 |
| 11 | 176 HDAC2 | NM_001527 | 4557640 | 3066 |
| 12 | 177 PRKAB1 | NM_006253 | 18602783 | 5564 |
| 13 | 178 IMPDH2 | NM_000884 | 4504688 | 3615 |
| 15 | 180 YR-29 | NM_014886 | 7662676 | 10412 |
| 22 | 187 CCNB2 | NM_004701 | 10938017 | 9133 |
| 23 | 188 FMOD | NM_002023 | 18548671 | 2331 |
| 24 | 189 SLC7A8 | NM_012244 | 14751202 | 23428 |
| 25 | 190 E2-EPF | NM_014501 | 7657045 | 27338 |
| 26 | 191 AGT | NM_000029 | 4557286 | 183 |
| 27 | 192 FHL2 | NM_001450 | 4503722 | 2274 |
| 29 | 194 MGC16824 | NM_02031 | 10092674 | 57020 |
| 31 | 196 MAD2L1 | NM_002358 | 6466452 | 4085 |
| 32 | 197 DDB2 | NM_000107 | 4557514 | 1643 |
| 40 | 205 RARRES3 | NM_004585 | 8051633 | 5920 |
| 43 | 208 COL11A1 | NM_001854 | 18548530 | 1301 |
| 50 | 215 PCMT1 | NM_005389 | 4885538 | 5110 |
| 51 | 216 ESR1 | NM_000125 | 4503602 | 2099 |
| 55 | 220 COL10A1 | NM_000493 | 18105031 | 1300 |
| 58 | 223 GRP | NM_002091 | 4504158 | 2922 |
| 61 | 226 GALNT10 | NM_017540 | 9055207 | 55568 |
| 65 | 230 PGR | NM_000926 | 4505766 | 5241 |
| 68 | 233 CCNG1 | NM_004060 | - | 900 |
| 69 | 234 PDHB | NM_000925 | 4505686 | 5162 |
| 74 | 239 NR2F1 | NM_005654 | 5032172 | 7025 |
| 81 | 246 FHL1 | NM_001449 | 4503720 | 2273 |
| 82 | 247 MSX2 | NM_002449 | 18560141 | 4488 |
| 83 | 248 PAI-RBP1 | NM_015640 | 7661625 | 26135 |
| 92 | 257 MKI67 | NM_002417 | 4505188 | 4288 |
| 98 | 263 GNG12 | NM_018841 | - | 55970 |
| 100 | 265 LOC253782 | AL080192- | - | - 253782 |
| 101 | 266 COL5A1 | NM_000093 | 18571690 | 1289 |
| 104 | 269 KIAA0056 | D29954 | 18578675 | 23310 |
| 105 | 270 FLJ22642 | A1700633 | - | - |
| 106 | 271 LOC113146 | W28438 | 15300131 | 113146 |

(continued)

| SEQ ID NO: (DNA Sequence) | SEQ ID NO: Gene Symbol (Protein Sequence) | Ref. Sequences [A] | Gene_ID | Locus Link I D |
|---|---|---|---|---|
| 108 | 273 PMSCL1 | NM_005033 | 4826921 | 5393 |
| 113 | 278 DKFZp586M 0723 | AL050227 | - | - |
| 124 | 289 RAB31 | NM_006868 | 5803130 | 11031 |
| 128 | 293 DCTN4 | NM_016221 | 14733974 | 51164 |
| 132 | 297 PLSCR4 | NM_020353 | 9966818 | 57088 |
| 129 | 294 FLJ20273 | NM_019027 | 9506670 | 54502 |
| 133 | 298 FLJ11323 | NM_018390 | 8922994 | 55344 |
| 138 | 303 MGC3103 | NM_024036 | 13128987 | 78999 |

**Table 3: Relative expression of 165 genes in complete responders as compared to non-responders and normal tissue. (CR - complete responder to therapy; NC - no change in tumor state; NT - normal healthy tissue)**

| SEQ ID NO: (DNA Sequence) | SEQ ID NO: Gene_ Symbol (Protein Sequence) | CR_vs._NC | CR_vs_NT | NC_vs_NT |
|---|---|---|---|---|
| 1 | 166 CTSB | 1.69033759 | 2.53990608 | 1.50260284 |
| 2 | 167 SSR1 | 1.69676002 | 1.56735024 | 0.92373125 |
| 3 | 168 STX8 | 1.42795315 | 1.65931125 | 1.16202079 |
| 4 | 169 KPNA2 | 2.10809096 | 2.08540708 | 0.98923961 |
| 5 | 170 CSE1L | 2.00249838 | 2.79008752 | 1.39330326 |
| 6 | 171 RHEB2 | 1.84519193 | 1.60184035 | 0.86811584 |
| 7 | 172 DKC1 | 2.25597289 | 2.3855889 | 1.0574546 |
| 8 | 173 IGFBP4 | 0.27862606 | 0.38691248 | 1.38864428 |
| 9 | 174 SMC1L1 | 1.69816116 | 1.71849631 | 1.01197481 |
| 10 | 175 PWP1 | 0.64477544 | 0.59496475 | 0.92274723 |
| 11 | 176 HDAC2 | 3.14799689 | 2.11008385 | 0.67029413 |
| 12 | 177 PRKAB1 | 0.52384682 | 0.56333165 | 1.07537477 |
| 13 | 178 IMPDH2 | 0.43342682 | 0.53415121 | 1.23239078 |
| 14 | 179 UBE2A | 1.56667644 | 1.8748269 | 1.19669056 |
| 15 | 180 YR-29 | 0.51635771 | 0.3928245 | 0.7607604 |
| 16 | 181 MUF1 | 1.48621121 | 1.67042393 | 1.12394787 |
| 17 | 182 MYO10 | 2.64854259 | 1.9657171 | 0.74218822 |
| 18 | 183 EGFR | 1.84523855 | 0.3988927 | 0.21617406 |
| 19 | 184 IFRD1 | 2.34518159 | 0.67841153 | 0.28927889 |
| 20 | 185 CD2BP2 | 0.40973605 | 0.74398402 | 1.81576414 |
| 21 | 186 ARL3 | 0.46877208 | 0.81409499 | 1.73665419 |
| 22 | 187 CCNB2 | 2.94729142 | 5.81162556 | 1.97185304 |
| 23 | 188 FMOD | 0.33346407 | 0.24429053 | 0.73258426 |
| 24 | 189 SLC7A8 | 0.23327957 | 0.68038164 | 2.91659333 |
| 25 | 190 E2-EPF | 2.50218494 | 4.49667635 | 1.79709992 |
| 26 | 191 AGT | 0.38629467 | 0.52277847 | 1.35331525 |
| 27 | 192 FHL2 | 0.31699809 | 0.39190285 | 1.23629407 |
| 28 | 193 LDLC | 0.56234146 | 0.88888889 | 1.58069244 |
| 29 | 194 MGC16824 | 0.51520913 | 0.67362665 | 1.30748198 |
| 30 | 195 UGDH | 0.4487715 | 0.59229116 | 1.31980566 |
| 31 | 196 MAD2L1 | 4.48217081 | 6.89647789 | 1.53864683 |
| 32 | 197 DDB2 | 0.37904516 | 0.3243275 | 0.85564341 |

(continued)

**NC - no change in tumor state; NT - normal healthy tissue)**

| SEQ ID NO: (DNA Sequence) | SEQ ID NO: Gene_ Symbol (Protein Sequence) | CR_vs._NC | CR_vs_NT | NC_vs_NT |
|---|---|---|---|---|
| 33 | 198 OS4 | 0.64290847 | 0.50896135 | 0.79165444 |
| 34 | 199 BCL2 | 0.37660415 | 0.26111358 | 0.69333698 |
| 35 | 200 SEMA3C | 0.5199821 | 0.48877024 | 0.93997512 |
| 36 | 201 DTR | 7.22480411 | 0.4189956 | 0.05799404 |
| 37 | 202 GARP | 0.47456604 | 0.3525155 | 0.74281654 |
| 38 | 203 ACK1 | 0.52564876 | 0.49278642 | 0.93748232 |
| 39 | 204 EDG2 | 0.71655585 | 0.46969319 | 0.6554872 |
| 40 | 205 RARRES3 | 0.24142196 | 1.41881212 | 5.87689745 |
| 41 | 206 CCNH | 0.55809994 | 0.42039831 | 0.75326706 |
| 42 | 207 PREP | 1.84855753 | 1.63361667 | 0.88372509 |
| 43 | 208 COL11A1 | 0.6377322 | 30.5047541 | 47.8331723 |
| 44 | 209 GALC | 0.50650838 | 0.63980608 | 1.26316978 |
| 45 | 210 HMGCS2 | 0.04797018 | 0.03074921 | 0.64100686 |
| 46 | 211 ZNF274 | 1.70500973 | 0.86640362 | 0.50815172 |
| 47 | 212 TFF1 | 0.0321807 | 0.2064045 | 6.41392222 |
| 48 | 213 RAD51 | 3.1036169 | 2.89007176 | 0.93119475 |
| 49 | 214 ASNS | 3.60284107 | 2.12910917 | 0.59095284 |
| 50 | 215 PCMT1 | 2.46691568 | 1.76150989 | 0.71405355 |
| 51 | 216 ESR1 | 0.12287491 | 0.2490413 | 2.02678727 |
| 52 | 217 ACAT1 | 0.51017664 | 0.39593742 | 0.7760791 |
| 53 | 218 XPA | 0.51539825 | 0.52117332 | 1.01120505 |
| 54 | 219 LAF4 | 0.23519327 | 0.35275966 | 1.49987143 |
| 55 | 220 COL10A1 | 0.38555774 | 9.32859382 | 24.1950629 |
| 56 | 221 KIAA1041 | 1.44589009 | 1.01679685 | 0.70323246 |
| 57 | 222 PLA2G7 | 4.23491725 | 4.95203213 | 1.16933386 |
| 58 | 223 GRP | 0.12594309 | 0.25636115 | 2.03553163 |
| 59 | 224 CYP2B6 | 0.01213194 | 0.12755005 | 10.513574 |
| 60 | 225 CHAD | 0.02707726 | 0.17583189 | 6.49371152 |
| 61 | 226 GALNT10 | 0.32020561 | 0.93356021 | 2.91550231 |
| 62 | 227 GADD45B | 0.51944741 | 0.22157381 | 0.42655678 |
| 63 | 228 WBSCR20 | 1.61337697 | 2.19652173 | 1.36144358 |
| 64 | 229 BTBD2 | 0.59662324 | 1.02610179 | 1.71984885 |
| 65 | 230 PGR | 0.06700908 | 0.12481888 | 1.86271582 |
| 66 | 231 TBPL1 | 1.71529386 | 1.53220024 | 0.89325816 |
| 67 | 232 C4B | 0.12173232 | 0.37926849 | 3.11559395 |
| 68 | 233 CCNG1 | 0.46882525 | 0.37588048 | 0.80174965 |
| 69 | 234 PDHB | 0.48347992 | 0.82135629 | 1.69884261 |
| 70 | 235 HNRPDL | 0.62657647 | 0.54249869 | 0.86581401 |
| 71 | 236 TAF11 | 1.83477376 | 1.42164687 | 0.77483497 |
| 72 | 237 AMACR | 0.61312794 | 0.84739097 | 1.38207854 |
| 73 | 238 EMD | 1.6831552 | 1.40144514 | 0.83262978 |
| 74 | 239 NR2F1 | 0.2644964 | 0.09725355 | 0.36769327 |
| 75 | 240 HSF2 | 1.72328808 | 1.03289666 | 0.5993755 |
| 76 | 241 SPG4 | 2.02820496 | 1.22197745 | 0.60249209 |
| 77 | 242 TRIP11 | 0.63637488 | 0.86619209 | 1.36113495 |
| 78 | 243 OCLN | 0.47955471 | 0.70987061 | 1.48027033 |
| 79 | 244 CACNA1D | 0.16768932 | 0.44304396 | 2.64205236 |

(continued)

**NC - no change in tumor state; NT - normal healthy tissue)**

| SEQ ID NO: (DNA Sequence) | SEQ ID NO: Gene_ Symbol (Protein Sequence) | CR_vs._NC | CR_vs_NT | NC_vs_NT |
|---|---|---|---|---|
| 80 | 245 CYP2B7 | 0.01399196 | 0.13737489 | 9.81812983 |
| 81 | 246 FHL1 | 0.30932043 | 0.03099618 | 0.10020734 |
| 82 | 247 MSX2 | 0.26991798 | 0.51082405 | 1.89251586 |
| 83 | 248 PAI-RBP1 | 2.81808253 | 1.95566986 | 0.69397182 |
| 84 | 249 CLDN14 | 0.34578658 | 0.30319698 | 0.87683272 |
| 85 | 250 ITPK1 | 0.59689657 | 0.52128465 | 0.87332492 |
| 86 | 251 ERBB2 | 1.86323083 | 7.16756759 | 3.84684897 |
| 87 | 252 TP53 | 0.51575976 | 1.18684511 | 2.30115879 |
| 88 | 253 HSPA2 | 0.09735986 | 0.34190488 | 3.51176445 |
| 89 | 254 LIG1 | 0.3244685 | 0.36453228 | 1.12347509 |
| 90 | 255 GSS | 0.58258632 | 0.84095907 | 1.44349265 |
| 91 | 256 PRO1843 | 0.57531505 | 0.51177072 | 0.88954864 |
| 92 | 257 MKI67 | 2.0943328 | 2.19410145 | 1.04763744 |
| 93 | 258 BIK | 0.50587875 | 1.55537704 | 3.0746044 |
| 94 | 259 KIAA0225 | 2.13074615 | 2.13861404 | 1.00369255 |
| 95 | 260 TNRC15 | 0.63566173 | 0.69130642 | 1.0875382 |
| 96 | 261 SFRS5 | 0.55670226 | 0.25236203 | 0.45331597 |
| 97 | 262 RPL17 | 0.67408803 | 0.65848911 | 0.97685923 |
| 98 | 263 GNG12 | 0.39809519 | 0.35596632 | 0.89417388 |
| 99 | 264 LAP1 B | 0.59182478 | 0.87189088 | 1.47322468 |
| 100 | 265 LOC253782 | 0.33656287 | 1.0069827 | 2.99196016 |
| 101 | 266 COL5A1 | 0.48612506 | 1.91919073 | 3.94793618 |
| 102 | 267 CXCL13 | 1.09334867 | 2.55193586 | 2.33405493 |
| 103 | 268 TTS-2.2 | 0.52779839 | 0.24321886 | 0.46081774 |
| 104 | 269 KIAA0056 | 2.15880901 | 2.32531026 | 1.07712643 |
| 105 | 270 FLJ22642 | 0.50735263 | 0.47592636 | 0.93805833 |
| 106 | 271 LOC113146 | 0.4322237 | 0.20955508 | 0.48483016 |
| 107 | 272 GPR126 | 2.97045989 | 1.28374752 | 0.4321713 |
| 108 | 273 PMSCL1 | 3.85379762 | 5.25959238 | 1.36478168 |
| 109 | 274 KIAA0418 | 0.63562548 | 0.58234822 | 0.91618138 |
| 110 | 275 SULF1 | 1.05390365 | 3.85641652 | 3.65917372 |
| 111 | 276 KIAA0673 | 0.57391504 | 0.57797443 | 1.00707314 |
| 112 | 277 FLJ10803 | 2.8794926 | 0.80518888 | 0.27962874 |
| 113 | 278 DKFZp586M0723 | 0.13647343 | 0.11662161 | 0.85453708 |
| 114 | 279 C4A | 0.17445163 | 0.36240753 | 2.07740986 |
| 115 | 280 ZAP3 | 0.60561667 | 0.54605096 | 0.90164454 |
| 116 | 281 NEK9 | 0.42385526 | 0.71295236 | 1.6820656 |
| 117 | 282 FLJ13125 | 1.7458421 | 1.35110145 | 0.77389671 |
| 118 | 283 FMO5 | 0.08559415 | 0.30218827 | 3.53047791 |
| 119 | 284 COMP | 0.2912537 | 4.73047702 | 16.2417748 |
| 120 | 285 CSPG2 | 0.59090269 | 1.88790387 | 3.19494885 |
| 121 | 286 LOC151996 | 0.41338598 | 2.34521857 | 5.67319337 |
| 122 | 287 TFAP2B | 0.43320817 | 1.34577659 | 3.10653554 |
| 123 | 288 OR7E38P | 2.4721374 | 2.04397969 | 0.82680667 |
| 124 | 289 RAB31 | 0.40394741 | 2.19420728 | 5.43191319 |
| 125 | 290 HSPC126 | 1.62954666 | 1.26787014 | 0.77805083 |

(continued)

**NC - no change in tumor state; NT - normal healthy tissue)**

| SEQ ID NO: (DNA Sequence) | SEQ ID NO: Gene_ Symbol (Protein Sequence) | CR_vs._NC | CR_vs_NT | NC_vs_NT |
|---|---|---|---|---|
| 126 | 291 UMP-CMPK | 1.92778452 | 1.24300347 | 0.64478341 |
| 127 | 292 FLJ22195 | 1.43061659 | 1.51916101 | 1.06189249 |
| 128 | 293 DCTN4 | 0.50788607 | 0.54260141 | 1.06835262 |
| 129 | 294 FLJ20273 | 0.38803157 | 0.89334309 | 2.30224333 |
| 130 | 295 KIF4A | 2.22685745 | 3.35533346 | 1.50675718 |
| 131 | 296 THTP | 0.58831486 | 0.8535722 | 1.45087649 |
| 132 | 297 PLSCR4 | 0.3444877 | 0.14809284 | 0.42989295 |
| 133 | 298 FLJ11323 | 2.11180669 | 1.12860006 | 0.53442394 |
| 134 | 299 MGC11242 | 0.39970231 | 0.96317642 | 2.40973447 |
| 135 | 300 CEGP1 | 0.06321053 | 0.22757341 | 3.6002451 |
| 136 | 301 SRR | 0.43030252 | 0.50748029 | 1.17935701 |
| 137 | 302 HSPC177 | 0.54280584 | 0.75044087 | 1.38252174 |
| 138 | 303 MGC3103 | 2.49147139 | 2.67377209 | 1.0731699 |
| 139 | 304 FLJ20641 | 2.19559981 | 2.13795703 | 0.97374623 |
| 140 | 305 FLJ13646 | 0.50690215 | 0.68417519 | 1.34971847 |
| 141 | 306 KCNK15 | 0.08400027 | 0.30393847 | 3.6183034 |
| 142 | 307 RNASEL | 0.43951061 | 0.48409168 | 1.10143344 |
| 143 | 308 CRSP6 | 1.57038515 | 1.63575579 | 1.04162714 |
| 144 | 309 COL5A2 | 0.44650047 | 1.59810403 | 3.57917657 |
| 145 | 310 LOC51218 | 0.59078156 | 1.08711676 | 1.84013321 |
| 146 | 311 APBB2 | 0.34810181 | 0.3281072 | 0.94256105 |
| 147 | 312 yy15c12.s1 | 1.37222353 | 1.42335867 | 1.03726444 |
| 148 | 313 AD037 | 2.09401866 | 1.44748322 | 0.69124657 |
| 149 | 314 FLJ20477 | 0.52024352 | 0.42892996 | 0.82447919 |
| 150 | 315 MARKL1 | 1.86975496 | 1.64523021 | 0.87991755 |
| 151 | 316 LUM | 0.81501967 | 1.26269875 | 1.54928623 |
| 152 | 317 COL3A1 | 0.60780953 | 1.3093042 | 2.15413568 |
| 153 | 318 COL1A1 | 0.55118736 | 1.72152105 | 3.1232956 |
| 154 | 319 BF | 0.23831298 | 1.7123556 | 7.18532235 |
| 155 | 320 ADAM12 | 0.53384591 | 0.70372001 | 1.31820811 |
| 156 | 321 LOXL1 | 0.48175564 | 1.99702419 | 4.14530526 |
| 157 | 322 CEACAM6 | 0.57151883 | 7.72858988 | 13.5228963 |
| 158 | 323 MMP11 | 0.75362281 | 6.87206597 | 9.11870749 |
| 159 | 324 MMP1 | 26.1407301 | 117.806871 | 4.50664042 |
| 160 | 325 MMP13 | 0.24808412 | 2.09572957 | 8.4476569 |
| 161 | 326 SERPINH1 | 1.28483815 | 2.27223116 | 1.76849603 |
| 162 | 327 PITX1 | 1.54911156 | 16.9745142 | 10.9575802 |
| 163 | 328 RAD52 | 0.66443667 | 1.71706792 | 2.58424617 |
| 164 | 329 INHBA | 0.72936034 | 4.21043511 | 5.77277773 |
| 165 | 330 CSPG2 | 0.77410378 | 1.86511138 | 2.40938157 |

<u>**Table 4a**</u>

**Putative biological function of 165 marker genes**

| SEQ ID NO: (DNA Sequence) | SEQ ID NO: Gene_Symbol (Protein Sequence) | Gene Description |
|---|---|---|
| 1 | 166 CTSB | wu69b10.x1 cathepsin B |

(continued)

**Putative biological function of 165 marker genes**

| SEQ ID NO: (DNA Sequence) | SEQ ID NO: Gene_Symbol (Protein Sequence) | Gene Description |
|---|---|---|
| 2 | 167 SSR1 | SSR alpha subunit signal sequence receptor alpha (translocon-associated protein alpha) SSR alpha subunit signal sequence receptor, alpha (translocon-associated |
| 3 | 168 STX8 | MSS1 proteasome (prosome macropain) 26S subunit ATPase 2 mammalian suppressor of sgv1; transactivation factor proteasome (prosome, macropain) 26S subunit, ATPase, 2 |
| 4 | 169 KPNA2 | nuclear localization sequence receptor hSRP1 alpha karyopherin alpha 2 (RAG cohort 1 importin alpha 1) karyopherin alpha~2 (RAG cohort 1, importin alpha 1) |
| 5 | 170 CSE1 | brain cellular apoptosis susceptibility protein (CSE1) brain cellular apoptosis susceptibility protein (CSE1) d chromosome segregation 1 (yeast homolog)-like CSE1 chromosome segregation 1-like (yeast) |
| 6 | 171 RHEB2 | D78132 ras-related GTP-binding protein Ras homolog enriched in brain 2 Rheb; ras-related GTP-binding protein Ras homologue enriched in brain; similar to rat Rheb gene ras-related GTP-binding protein |
| 7 | 172 DKC1 | Cbf5p homolog (CBF5) dyskeratosis congenita 1 dyskerin nucleolar protein; similar to yeast Cbf5p Cbf5p homolog |
| 8 | 173 IGFBP4 | df29g03.y1 insulin-like growth factor-binding protein 4 insulin-like growth factor binding protein 4 |
| 9 | 174 SMC1L1 | KIAA0178 gene SMC1 (structural maintenance of chromosomes 1 yeast)-like 1 KIAA0178 similar to mitosis-specific chromosome segregation protein SMC1 of S.cerevisiae. SMC1 structural maintenance of chromosomes 1-like 1 (yeast) |
| 10 | 175 PWP1 | IEF SSP 9502 nuclear phosphoprotein similar to S. cerevisiae PWP1 |
| 11 | 176 HDAC2 | transcriptional regulator homolog RPD3 histone deacetylase 2 similar to yeast RPD3, encoded by GenBank Accession Number X78454 transcriptional regulator homolog RPD3 |
| 12 | 177 PRKAB1 | 5-AMP-activated protein kinase beta-1 protein kinase AMP-activated beta 1 non-catalytic subunit protein kinase, AMP-activated, beta 1 non-catalytic subunit |

(continued)

**Putative biological function of 165 marker genes**

| SEQ ID NO: (DNA Sequence) | SEQ ID NO: Gene_Symbol (Protein Sequence) | Gene Description |
|---|---|---|
| 13 | 178 IMPDH2 | (clone FFE-7) type II inosine monophosphate dehydrogenase (IMPDH2) gene exons 1-13 IMP (inosine monophosphate) dehydrogenase 2 NAD-dependent; differentiation; inosine monophosphate dehydrogenase; inosine-5'-monophosphate dehydrogenase; nucleotide biosynthesis; proliferation associated gene IMP (inosine monophosphate) dehydrogenase 2 |
| 14 | 179 UBE2A | HUMHHR6A HHR6A (yeast RAD 6 homologue) ubiquitin-conjugating enzyme E2A (RAD6 homolog) |
| 15 | 180 YR-29 | hypothetical protein clone YR-29 hypothetical protein |
| 16 | 181 MUF1 | MUF1 protein MUF1 protein |
| 17 | 182 MYO10 | KIAA0799 protein myosin X hg01449 cDNA clone for KIAA0799 has a 1204-bp insertion at position 373 of the sequence of KIAA0799. KIAA0799 protein |
| 18 | 183 EGFR | HSEGFPRE precursor of epidermal growth factor receptor epidermal growth factor receptor (avian erythroblastic leukemia viral (v-erb-b) oncogene homolog) epidermal growth factor receptor; signal peptide epidermal growth factor receptor epidermal growth factor receptor (erythroblastic leukemia |
| 19 | 184 IFRD1 | BAC clone RG163K11 from 7q31 interferon-related developmental regulator 1 nucleophosmin 1 (nucleolar phosphoprotein B23 numatrin) pseudogene 14 HTG similar to mouse interferon-related protein PC4; 96% identical to P19182 (PID:g135861); H_ RG163K11.1 |
| 20 | 185 CD2BP2 | zk74b08.r1 CD2 antigen (cytoplasmic tail)-binding protein 2 CD2 antigen (cytoplasmic tail) binding protein 2 |
| 21 | 186 ARL3 | 48c8 ADP-ribosylation factor-like 3 EST |
| 22 | 187 CCNB2 | DKFZp434B174 (from clone DKFZp434B174) cyclin B2 cyclins B2 hypothetical protein |
| 23 | 188 FMOD | fibromodulin fibromodulin precursor fibromodulin Encodes only the most carboxy terminal 58 amino acids of fibromodulin. fibromodulin |

(continued)

**Putative biological function of 165 marker genes**

| SEQ ID NO: (DNA Sequence) | SEQ ID NO: Gene_Symbol (Protein Sequence) | Gene Description |
|---|---|---|
| 24 | 189 SLC7A8 | SLC7A8 protein solute carrier family 7 (cationic amino acid transporter y+ system) member 8 solute carrier family 7 (cationic amino acid transporter, |
| 25 | 190 E2-EPF | HUME2EPI ubiquitin carrier protein (E2-EPF) ubiquitin carrier protein |
| 26 | 191 AGT | G angiotensinogen serine (or cysteine) proteinase inhibitor clade A (alpha-1 antiproteinase antitrypsin) member 8 angiotensinogen (serine (or cysteine) proteinase inhibitor, |
| 27 | 192 FHL2 | heart protein (FHL-2) four and a half LIM domains 2 |
| 28 | 193 LDLC | LDLC low density lipoprotein receptor defect C complementing |
| 29 | 194 MGC16824 | hypothetical protein |
| 30 | 195 UGDH | UDP-glucose dehydrogenase (UGDH) UDP-glucose dehydrogenase UDPGDH; NAD+-linked oxidoreductase UDP-glucose dehydrogenase |
| 31 | 196 MAD2L1 | MAD2 protein MAD2 (mitotic arrest deficient yeast homolog)-like 1 MAD2 gene MAD2-like 1 MAD2 mitotic arrest deficient-like 1 (yeast) |
| 32 | 197 DDB2 | HSU18300 damage-specific DNA binding protein p48 subunit (DDB2) damage-specific DNA binding protein 2 (48kD) damage-specific DNA binding protein p48 subunit; implicated in Xeroderma pigmentosum group E DDBb p48 |
| 33 | 198 OS4 | OS-4 protein (OS-4) conserved gene amplified in osteosarcoma |
| 34 | 199 BCL2 | HUMBCL2A B-cell leukemia lymphoma 2 (bcl-2) proto-oncogene encoding bcl-2-alpha protein B-cell leukemialymphoma 2 (bcl-2) proto-oncogene encoding bcl-2-alpha proteind B-cell CLUlymphoma 2 alternative splicing; bcl-2-alpha protein; proto-oncogene bcl2-alpha protein B-cell lymphoma protein 2 beta |
| 35 | 200 SEMA3C | AB000220 semaphorin E sema domain immunoglobulin domain (lg) short basic domain secreted (semaphorin) 3C semaphorin E sema domain, immunoglobulin domain (lg), short basic domain, secreted, (semaphorin) 3C |

(continued)

**Putative biological function of 165 marker genes**

| SEQ ID NO: (DNA Sequence) | SEQ ID NO: Gene_Symbol (Protein Sequence) | Gene Description |
|---|---|---|
| 36 | 201 DTR | heparin-binding EGF-like growth factor diphtheria toxin receptor (heparin-binding epidermal growth factor-like growth factor) heparin-binding EGF-like growth factor putative diphtheria toxin receptor (heparin-binding epidermal growth factor-like growth factor) |
| 37 | 202 GARP | garp gene glycoprotein A repetitions predominantprecursor glycoprotein A repetitions predominant GARP gene; leucine-rich repeat containing protein glycoprotein A repetitions predominant precursor |
| 38 | 203 ACK1 | HUMNRTYKIN activated p21cdc42Hs kinase (ack) activated p21cdc42Hs kinase putative activated p21cdc42Hs kinase |
| 39 | 204 EDG2 | wc44d05.x1 endothelial differentiation lysophosphatidic acid G-protein-coupled receptor 2 EST |
| 40 | 205 RARRES3 | retinoic acid receptor responder 3 (RARRES3) retinoic acid receptor responder (tazarotene induced) 3 putative class II tumor suppressor; growth inhibitory protein; tazarotene induced retinoic acid receptor responder 3 |
| 41 | 206 CCNH | HSU11791 cyclin H cyclin H cyclin H |
| 42 | 207 PREP | prolyl oligopeptidase prolyl endopeptidase prolyl oligopeptidase prolyl endopeptidase |
| 43 | 208 COL11A1 | alpha-1 type XI collagen (COL11A1) collagen type XI alpha 1 alpha-1 type XI collagen; collagen; type XI collagen alpha-1 (type XI) collagen precursor collagen, type XI, alpha 1 |
| 44 | 209 GALC | DNAgalactocerebrosidase galactosylceramidase (Krabbe disease) GALC galactocerebrosidase |
| 45 | 210 HMGCS2 | 3-hydroxy-3-methylglutaryl coenzyme A synthase 3-hydroxy-3-methylglutaryl-Coenzyme A synthase 2 (mitochondrial) hydroxymethyl-CoA synthetase 3-hydroxy-3-methylglutaryl-Coenzyme A synthase 2 (mitochondrial) |
| 46 | 211 ZNF274 | zinc finger protein zfp2 (zf2) KRAB zinc finger protein HFB101 L zinc finger protein 274 |
| 47 | 212 TFF1 | EST186646 trefoil factor 1 (breast cancer estrogen-inducible sequence expressed in) EST trefoil factor 1 (breast cancer, estrogen-inducible sequence |

(continued)

**Putative biological function of 165 marker genes**

| SEQ ID NO: (DNA Sequence) | SEQ ID NO: Gene_Symbol (Protein Sequence) | Gene Description |
|---|---|---|
| 48 | 213 RAD51 | DKFZp564H1178_s1 RAD51 (S. cerevisiae) homolog (E coli RecA homolog) EST RAD51 homolog (RecA homolog, E. coli) (S. cerevisiae) |
| 49 | 214 ASNS | asparagine synthetase asparagine synthetase asparagine synthetase |
| 50 | 215 PCMT1 | carboxyl methyltransferase protein-L-isoaspartate (D-aspartate) O-methyltransferase carboxyl methyltransferase protein-L-isoaspartate (D-aspartate) O-methyltransferase |
| 51 | 216 ESR1 | HSERR oestrogen receptor estrogen receptor 1 estrogen receptor; receptor; steroid hormone receptor oestrogen receptor |
| 52 | 217 ACAT1 | MAT genemitochondrial acetoacetyl-CoA thiolase acetyl-Coenzyme A acetyltransferase 1 (acetoacetyl Coenzyme A thiolase) (ACAT1) nuclear gene encoding mitochondrial prote |
| 53 | 218 XPA | HUMXPAC XPAC protein xeroderma pigmentosum complementation group A XPAC protein xeroderma pigmentosum, complementation group A |
| 54 | 219 LAF4 | lymphoid nuclear protein (LAF-4) lymphoid nuclear protein related to AF4 |
| 55 | 220 COL10A1 | COL10A1 genecollagen (alpha-1 type X) collagen type X alpha 1 (Schmid metaphyseal chondrodysplasia) collagen, type X, alpha 1 (Schmid metaphyseal chondrodysplasia) |
| 56 | 221 KIAA1041 | KIAA1041 protein KIAA1041 protein KIAA1041 protein |
| 57 | 222 PLA2G7 | LDL-phospholipase A2 phospholipase A2 group VII (platelet-activating factor acetylhydrolase plasma) PAF-acetylhydrolase phospholipase A2, group VII (platelet-activating factor acetylhydrolase, plasma) |
| 58 | 223 GRP | HUMGRP5E gastrin-releasing peptide gastrin-releasing peptide gastrin-releasing peptide pre-progastrin releasing peptide gastrin-releasing peptide |

(continued)

**Putative biological function of 165 marker genes**

| SEQ ID NO: (DNA Sequence) | SEQ ID NO: Gene_Symbol (Protein Sequence) | Gene Description |
|---|---|---|
| 59 | 224 CYP2B6 | HUMCYP2BB cytochrome P450-IIB (hIIB1) cytochrome P450 subfamily IIB (phenobarbital-inducible) polypeptide 6 cytochrome P450 subfamily IIB (phenobarbital-inducible) cytochrome P450; cytochrome P450 IIB cytochrome P450-IIB cytochrome P450, subfamily IIB (phenobarbital-inducible) |
| 60 | 225 CHAD | chondroadherin gene 5flanking region and chondroadherin precursor cartilage leucine-rich repeat protein chondroadherin |
| 61 | 226 GALNT10 | DKFZp586H0623 (from clone DKFZp586H0623) hypothetical protein DKFZp586H0623 (DKF hypothetical protein DKFZp586H0623 similarity to N-acetylgalactosaminyltransferase,; The frame shift was determined manually hypothetical protein putative UDP-GalNAc:polypeptide N-acetylgalactosaminyltransferase |
| 62 | 227 GADD45B | growth arrest and DNA-damage-inducible protein GADD45beta growth arrest and DNA-damage-inducible beta growth arrest and DNA-damage-inducible, beta |
| 63 | 228 WBSCR20 | wh80b02.x1 putative methyltransferase |
| 64 | 229 BTBD2 | zd42a12.s1 BTB (POZ) domain containing 2 hypothetical protein FLJ20386 EST |
| 65 | 230 PGR | progesterone receptor |
| 66 | 231 TBPL1 | DNA sequence from clone 73H22 on chromosome 6q23 TBP-like 1 HTG; CpG Island dJ73H22.1 (TBP-like protein) |
| 67 | 232 C4B | RP1 and complement C4B precursor (C4B) genes complement component 4B |
| 68 | 233 CCNG1 | cyclin G1 clone MGC:6 |
| 69 | 234 PDHB | pyruvate dehydrogenase (EC 1.2.4.1) beta subunit gene exons 1-10 pyruvate dehydrogenase E1-beta subunit d pyruvate dehydrogenase (lipoamide) beta |
| 70 | 235 HNRPDL | A+U-rich element RNA binding factor for A+U-rich element RNA binding factord heterogeneous nuclear ribonucleoprotein D-like |
| 71 | 236 TAF11 | wr91e02.x1 TATA box binding protein (TBP)-associated factor RNA polymerase II I 28kD TAF11 RNA polymerase II, TATA box binding protein (TBP)-associated |

(continued)

**Putative biological function of 165 marker genes**

| SEQ ID NO: (DNA Sequence) | SEQ ID NO: Gene_Symbol (Protein Sequence) | Gene Description |
|---|---|---|
| 72 | 237 AMACR | 2-methylacyl-CoA racemase alpha-methylacyl-CoA racemase d alpha-methylacyl-CoA racemase |
| 73 | 238 EMD | EDMD gene emerin (Emery-Dreifuss muscular dystrophy) clone MGC:21 emerin (Emery-Dreifuss muscular dystrophy) EDMD gene; emerin emerin |
| 74 | 239 NR2F1 | V-Erba Related Ear-3 Protein nuclear receptor subfamily 2 group F member 1 nuclear receptor subfamily 2, group F, member 1 |
| 75 | 240 HSF2 | HUMHSF2 heat shock factor 2 (HSF2) heat shock factor 2 (HSF2) d heat shock transcription factor 2 heat shock factor 2 HSF2 |
| 76 | 241 SPG4 | KIAA1083 protein spastic paraplegia 4 (autosomal dominant spastin) KIAA1083 protein spastic paraplegia 4 (autosomal dominant; spastin) |
| 77 | 242 TRIP11 | Golgi-associated microtubule-binding protein (GMAP-210) thyroid hormone receptor interactor 11 GMAP-210 gene; Golgi-associated microtubule-binding protein Golgi-associated microtubule-binding protein |
| 78 | 243 OCLN | wr26e08.x1 tight junction protein occludin d occludin EST |
| 79 | 244 CACNA1D | wt59c07.x1 calcium channel voltage-dependent L type alpha 1D subunit ESTs calcium channel, voltage-dependent, L type, alpha 1D |
| 80 | 245 CYP2B7 | cytochrome P450-IIB (hIIB3) ds cytochrome P450, subfamily IIB (phenobarbital-inducible), |
| 81 | 246 FHL1 | LIM protein SLIMMER LIM protein SLIMMER d four and a half LIM domains 1 skeletal and cardiac muscle SLIM isoform LIM protein SLIMMER |
| 82 | 247 MSX2 | MSX-2 msh (Drosophila) homeo box homolog 2 msh homeo box homolog 2 (Drosophila) |
| 83 | 248 PAI-RBP1 | DKFZp564M2423 (from clone DKFZp564M2423) Similar to DKFZP564M2423 protein clone MGC: 13 DKFZP564M2423 protein |
| 84 | 249 CLDN14 | CLDN14 gene claudin 14 (CLDN14) d claudin 14 claudin-14; CLDN14 gene claudin-14 |

(continued)

**Putative biological function of 165 marker genes**

| SEQ ID NO: (DNA Sequence) | SEQ ID NO: Gene_Symbol (Protein Sequence) | Gene Description |
|---|---|---|
| 85 | 250 ITPK1 | inositol 1 3 4-trisphosphate 5 6-kinase inositol 1 3 4-trisphosphate 56-kinase d inositol 1 3 4-triphosphate 5/6 kinase inositol 1,3,4-triphosphate 5/6 kinase |
| 86 | 251 ERBB2 | tyrosine kinase-type receptor (HER2) v-erb-b2 avian erythroblastic leukemia viral oncogene homolog 2 (neuroglioblastoma derived oncogene homolog) v-erb-b2 avian erythroblastic leukemia viral oncogene homolog 2 (neuro/glioblastoma derived oncogene homolog) tyrosine kinase HER2 receptor v-erb-b2 avian erythroblastic leukemia viral oncogene homolog 2 (neuro/ glioblastoma derived oncogene homolog) |
| 87 | 252 TP53 | HSP53 p53 cellular tumor antigen p53 cellular tumor antigen d tumor protein p53 (Li-Fraumeni syndrome) antigen; tumor antigen p53 tumor antigen (aa 1-?) tumor protein p53 |
| 88 | 253 HSPA2 | HUMHSPA2A heat shock protein HSPA2 gene heat shock protein d heat shock 70kD protein 2 |
| 89 | 254 LIG1 | DKFZp434N0910_s1 for membrane glycoprotein LIG-1d DKFZP586O1624 protein EST |
| 90 | 255 GSS | wt55b10.x1 (clone pGSH1) glutathione synthetase (gsh-s) d glutathione synthetase |
| 91 | 256 PRO1843 | initiation factor 4B eukaryotic translation initiation factor 4B |
| 92 | 257 MKI67 | HSMKI67 mki67a (long type)antigen of monoclonal antibody Ki-67 antigen identified by monoclonal antibody Ki-67 |
| 93 | 258 BIK | HSU34584 Bcl-2 interacting killer (BIK) BCL2-interacting killer (apoptosis-inducing) Bik (Bcl-2 interacting killer); Bcl-2 homology 3 (BH3) domain Bik interacts with the survival proteins Bcl-2, Bcl-xL, EBV-BHRF1 and adenovirus E1B 19kD; This protein is identical with that described by Robin Brown and colleagues (personal communication) which is a Human NBK apoptotic inducer protein, encoded by GenB Bik |
| 94 | 259 KIAA0225 | KIAA0225 gene KIAA0225 protein |
| 95 | 260 TNRC15 | KIAA0642 protein trinucleotide repeat containing 15 |
| 96 | 261 SFRS5 | zc81g05.s1 splicing factor arginineserine-rich 5 ESTs |

(continued)

**Putative biological function of 165 marker genes**

| SEQ ID NO: (DNA Sequence) | SEQ ID NO: Gene_Symbol (Protein Sequence) | Gene Description |
|---|---|---|
| 97 | 262 RPL17 | L23 putative ribosomal protein ribosomal protein L17 ribosomal protein putative ribosomal protein (AA 1-184) ribosomal protein L17 |
| 98 | 263 GNG12 | DKFZp586B0918 (from clone DKFZp586B0918) DKFZp586B0918 Zp586B0918) |
| 99 | 264 LAP1B | UI-H-BI0-aao-g-10-0-Ul.s1 FLJ11551 fis clone HEMBA1002999 moderately similar to Rattus norvegicus lamina associated polypeptide 1C (LAP1C) mRN DKFZP586G011 protein |
| 100 | 265 LOC253782 | DKFZp434B102 (from clone DKFZp434B102) : FLJ21238 fis clone COL01115 Homo sapiens mRNA; cDNA DKFZp434B102 (from clone DKFZp434B102) |
| 101 | 266 COL5A1 | pro-alpha-1 (V) collagen collagen type V alpha 1 |
| 102 | 267 CXCL13 | B lymphocyte chemoattractant BLC small inducible cytokine B subfamily (Cys-X-Cys motif) member 13 (B-cell chemoattractant) small inducible cytokine B subfamily (Cys-X-Cys motif), |
| 103 | 268 TTS-2.2 | clone 24519 unknown transport-secretion protein 2.2 |
| 104 | 269 KIAA0056 | KIAA0056 gene KIAA0056 protein |
| 105 | 270 FLJ22642 | we38g03.x1 : FLJ22642 fis clone HSI06970 EST |
| 106 | 271 LOC113146 | 47g10 ESTs |

(continued)

**Putative biological function of 165 marker genes**

| SEQ ID NO: (DNA Sequence) | SEQ ID NO: Gene_Symbol (Protein Sequence) | Gene Description |
|---|---|---|
| 107 | 272 GPR126 | DNA sequence from clone 287G14 on chromosome 6q23.1-24.3. Contains a novel seven transmembrane domain protein gene and an exon similar to parts of BMP and Tolloid genes. Contains ESTs an STS and GSSs DNA sequence from clone 287G14 on chromosome 6q23.1-24.3. Contains a novel seven transmembrane domain protein gene and an exon similar to parts of BMP and Tolloid genes. Contains ESTs an STS and GS Human DNA sequence from clone 287G14 on chromosome 6q23.1-24.3. Contains a novel seven transmembrane domain protein gene and an exon similar to parts of BMP and Tolloid genes. Contains ESTs an STS and GSSs HTG; BMP; seven transmembrane domain; Tolloid supported by GENSCAN and FGENES dJ287G14.1 (exon of a yet unidentified gene, or part of a pseudogene?; similar to parts of BMP and Tolloid proteins) |
| 108 | 273 PMSCL1 | tx67e10.x1 polymyositisscleroderma autoantigen 1 (75kD) EST Weakly similar to JH0446 75K autoantigen -human☐ [H.sapiens] |
| 109 | 274 KIAA0418 | wi34b03.x1 KIAA0418 gene product EST |
| 110 | 275 SULF1 | KIAA1077 protein KIAA1077 protein KIAA1077 protein |
| 111 | 276 KIAA0673 | KIAA0673 protein for KIAA0673 proteind KIAA0673 protein KIAA0673 protein |
| 112 | 277 FLJ10803 | ni36d11.s1 hypothetical protein FLJ10803 ESTs |
| 113 | 278 DKFZp586M07 23 | DKFZp586M0723 (from clone DKFZp586M0723) DKFZp586M0723 Zp586M0723) |
| 114 | 279 C4A | RP1 and complement C4B precursor (C4B) genes complement component C4A d complement component 4B complement component 4A |
| 115 | 280 ZAP3 | (clone zap3) of cds and unknown ge ZAP3 protein ORF; putative |
| 116 | 281 NEK9 | Untitled hypothetical protein MGC16714 |
| 117 | 282 FLJ13125 | FLJ13125 fis clone NT2RP3002877 |

(continued)

**Putative biological function of 165 marker genes**

| SEQ ID NO: (DNA Sequence) | SEQ ID NO: Gene_Symbol (Protein Sequence) | Gene Description |
|---|---|---|
| 118 | 283 FMO5 | flavin-containing monooxygenase 5 (FMO5) FLJ12110 fis clone MAMMA1000020 highly similar to for flavin-containing monooxygenase 5 (FMO5 flavin containing monooxygenase 5 flavin-containing monooxygenase 5 flavin containing monooxygenase 5 |
| 119 | 284 COMP | germline oligomeric matrix protein (COMP) cartilage oligomeric matrix protein (pseudoachondroplasia epiphyseal dysplasia 1 multiple) cartilage oligomeric matrix protein (pseudoachondroplasia, |
| 120 | 285 CSPG2 | pgH3 proteoglycan PG-M(V3) chondroitin sulfate proteoglycan 2 (versican) PG-M; proteoglycan PG-M (V3); large chondroitin sulfate proteoglycan; pgH3; major extracellular matrix molecule proteoglycan PG-M(V3) |
| 121 | 286 LOC151996 | zv97h07.s1 FLJ12280 fis clone MAMMA1001744 EST |
| 122 | 287 TFAP2B | transcription factor AP-2 beta (activating enhancer-binding protein 2 beta) transcription factor AP-2 beta (activating enhancer binding |
| 123 | 288 OR7E38P | OR7E12P pseudogene complete sequence olfactory receptor family 7 subfamily E member 38 pseudogene olfactory receptor family 7 subfamily E member 12 pseudogene olfactory receptor |
| 124 | 289 RAB31 | low-Mr GTP-binding protein (RAB31) RAB31 member RAS oncogene family Low Mr GTP-binding protein of the Rab subfamily low-Mr GTP-binding protein Rab31 RAB31, member RAS oncogene family |
| 125 | 290 HSPC126 | wq62d04.×1 HSPC126 protein |
| 126 | 291 UMP-CMPK | ws85a09.×1 UMP-CMP kinase EST |
| 127 | 292 FLJ22195 | DKFZp762L203_s1 hypothetical protein FLJ22195 Homo sapiens cDNA: FLJ22195 fis clone HRC01166 |
| 128 | 293 DCTN4 | wz58c04.x1 dynactin p62 subunit dynactin 4 (p62) |
| 129 | 294 FLJ20273 | nh92d01.s1 hypothetical protein EST |
| 130 | 295 KIF4A | zh97c02.s1 kinesin family member 4A EST |
| 131 | 296 THTP | yi24d06.r1 hypothetical protein MGC2652 ESTs |

(continued)

**Putative biological function of 165 marker genes**

| SEQ ID NO: (DNA Sequence) | SEQ ID NO: Gene_Symbol (Protein Sequence) | Gene Description |
|---|---|---|
| 132 | 297 PLSCR4 | wk77f02.x1 phospholipid scramblase 4 EST |
| 133 | 298 FLJ11323 | ac16g07.s1 hypothetical protein FLJ11323 EST |
| 134 | 299 MGC11242 | zh46f04.r1 hypothetical protein MGC11242 ESTs |
| 135 | 300 CEGP1 | wv11f12.x1 CEGP1 protein |
| 136 | 301 SRR | wq60g02.x1 serine racemase Homo sapiens cDNA FLJ13107 fis clone NT2RP3002501 weakly similar to THREONINE DEHYDRATASE CATABOLIC (EC 4.2.1.16) EST |
| 137 | 302 HSPC177 | wn81b08.x1 hypothetical protein CGI-34 protein hypothetical protein HSPC177 |
| 138 | 303 MGC3103 | ws44f11.x1 hypothetical protein MGC3103 ESTs |
| 139 | 304 FLJ20641 | qi31h03.x1 hypothetical protein FLJ20641 |
| 140 | 305 FLJ13646 | tg49h03.x1 hypothetical protein FLJ13646 Homo sapiens cDNA FLJ13646 fis clone PLACE1011325 EST |
| 141 | 306 KCNK15 | two pore potassium channel KT3.3 |
| 142 | 307 RNASEL | ribonuclease L (2 5-oligoisoadenylate synthetase-dependent) ribonuclease L (2',5'-oligoisoadenylate synthetase-dependent) |
| 143 | 308 CRSP6 | C05931 cofactor required for Sp1 transcriptional activation subunit 6 (77kD) EST cofactor required for Sp1 transcriptional activation, |
| 144 | 309 COL5A2 | yl92e08.r1 collagen type V alpha 2 TRIAD3 protein EST |
| 145 | 310 LOC51218 | wr52b07.x1 clone FLB4739 EST |
| 146 | 311 APBB2 | DKFZp434E033 (from clone DKFZp434E033) FE65-like protein (hFE65L) Homo sapiens mRNA; cDNA DKFZp434E033 (from clone DKFZp434E033) amyloid beta (A4) precursor protein-binding, family B, |
| 147 | 312 yy15c12.s1 | yy15c12.s1 ESTs |
| 148 | 313 AD037 | FE65-LIKE 2 AD037 protein |
| 149 | 314 FLJ20477 | zx56a06.r1 hypothetical protein FLJ20477 EST |
| 150 | 315 MARKL1 | DKFZp761B169_s1 ESTs MAP/ microtubule affinity-regulating kinase like 1 |
| 151 | 316 LUM | lumican lumican lumican |

(continued)

**Putative biological function of 165 marker genes**

| SEQ ID NO: (DNA Sequence) | SEQ ID NO: Gene_Symbol (Protein Sequence) | Gene Description |
|---|---|---|
| 152 | 317 COL3A1 | pro-alpha-1 type 3 collagen collagen type III alpha 1 (Ehlers-Danlos syndrome type IV autosomal dominant) COL3A1 gene; collagen; collagen alpha 1 type III; collagen type III prepro-alpha-1 type 3 collagen |
| 153 | 318 COL1A1 | prepro-alpha1(I) collagen proalpha 1 (I) chain of type I procollagen (partial collagen type I alpha 1 alpha1 (I)-collagen collagen, type I, alpha 1 |
| 154 | 319 BF | complement factor B B-factor properdin complement factor; complement factor B B-factor, properdin |
| 155 | 320 ADAM12 | meltrin-L precursor (ADAM12) a disintegrin and metalloproteinase domain 12 (meltrin alpha) (ADAM12) transcript variant a disintegrin and metalloproteinase domain 12 (meltrin alpha) |
| 156 | 321 LOXL1 | lysyl oxidase-like protein gene lysyl oxidase-like 1 lysyl oxidase-like 1 |
| 157 | 322 CEACAM6 | nonspecific crossreacting antigen carcinoembryonic antigen-related cell adhesion molecule 6 (non-specific cross reacting antigen) clone MGC:104 nonspecific cross-reacting antigen ORF1 non-specific cross reacting antigen |
| 158 | 323 MMP11 | stromelysin-3 matrix metalloproteinase 11 (stromelysin 3) |
| 159 | 324 MMP1 | skin collagenase matrix metalloproteinase 1 (interstitial collagenase) |
| 160 | 325 MMP13 | collagenase 3 matrix metalloproteinase 13 (collagenase 3) |
| 161 | 326 SERPINH1 | colligin (a collagen-binding protein) serine (or cysteine) proteinase inhibitor clade H (heat shock protein 47) member 1 collagen-binding protein; colligin colligin serine (or cysteine) proteinase inhibitor, clade H (heat |
| 162 | 327 PITX1 | hindlimb expressed homeobox protein backfoot (Bft) paired-like homeodomain transcription factor 1 paired-like homeodomain transcription factor 1 |
| 163 | 328 RAD52 homolog | DKFZp56411922 (from clone DKFZp56411922) adlican d DKFZP564I1922 protein similarity to perlecan hypothetical protein |

(continued)

**Putative biological function of 165 marker genes**

| SEQ ID NO: (DNA Sequence) | SEQ ID NO: Gene_Symbol (Protein Sequence) | Gene Description |
|---|---|---|
| 164 | 329 INHBA | erythroid differentiation protein (EDF) inhibin beta A (activin A activin AB alpha polypeptide) inhibin, beta A (activin A, activin AB alpha polypeptide) |
| 165 | 330 CSPG2 | the chondroitin sulphate proteoglycan versican V1 splice-variant precursor peptide chondroitin sulfate proteoglycan 2 (versican) |

**Table 4b**

**Putative biological function of 20 nonresponder marker genes**

| SEQ ID NO: (DNA Sequence) | SEQ ID NO: Gene_Symbol (Protein Sequence) | Gene Description |
|---|---|---|
| 472 | 492 PRG1 | hematopoetic proteoglycan core protein proteoglycan 1 secretory granule haematopoetic proteoglycan core protein hematopoetic proteoglycan core protein (AA 1 - 158) proteoglycan 1, secretory granule |
| 473 | 493 GBP1 | guanylate binding protein isoform I (GBP-2) guanylate binding protein 1 interferon-inducible 67kD guanylate binding protein isoform I guanylate binding protein 1, interferon-inducible, 67kD |
| 474 | 494 ALEX2 | KIAA0512 protein KIAA0512 gene product ALEX2 KIAA0512 gene product KIAA0512 protein KIAA0512 gene product armadillo repeat protein ALEX2 |
| 475 | 495 CD53 | CD53 glycoprotein CD53 antigen |
| 476 | 496 VCAM1 | vascular cell adhesion molecule-1 (VCAM1) gene vascular cell adhesion molecule 1 |
| 477 | 497 MAPT | HUMTAUA microtubule-associated protein tau microtubule-associated protein tau epitope microtubule-associated protein tau microtubule-associated protein tau, isoform 2 |
| 478 | 498 EGR2 | early growth response 2 protein (EGR2) early growth response 2 (Krox-20 (Drosophila) homolog) EGR2 gene; early growth response protein early growth response 2 protein early growth response 2 (Krox-20 homolog, Drosophila) |
| 479 | 499 TDO2 | tryptophan oxygenase (TDO) tryptophan 2 3-dioxygenase tryptophan 2,3-dioxygenase |

(continued)

**Putative biological function of 20 nonresponder marker genes**

| SEQ ID NO: (DNA Sequence) | SEQ ID NO: Gene_Symbol (Protein Sequence) | Gene Description |
|---|---|---|
| 480 | 500 ADAMDEC1 | disintegrin-protease disintegrin protease disintegrin; protease disintegrin protease |
| 481 | 501 TFEC | TFEC isoform (or TFECL) transcription factor EC TFEC TFEC isoform (or TFECL) |
| 482 | 502 BTF3 | Transcription Factor Btf3b basic transcription factor 3 |
| 483 | 503 FLNB | yi17d08.r1 filamin B beta (actin-binding protein-278) Homo sapiens mRNA; cDNA DKFZp586J021 (from clone DKFZp586J021) EST filamin B, beta (actin binding protein 278) |
| 484 | 504 TFRC | transferrin receptor transferrin receptor (p90 CD71) clone MGC:31 transferrin receptor (p90 CD71) transferrin receptor put. transferrin receptor (aa 1-760) transferrin receptor (p90, CD71) |
| 485 | 505 EIF4B | eukaryotic translation initiation factor 4B |
| 486 | 506 MAPK3 | HSERK1 ERK1 protein serine threonine kinase ERK1 for protein serinethreonine kinas mitogen-activated protein kinase 3 erk1 gene; protein-serine/threonine kinase protein serine/threonine kinase |
| 487 | 507 LOC161291 | DKFZp564D1462 (from clone DKFZp564D1462) DKFZp564D1462 Zp564D1462) |
| 488 | 508 SLC1A1 | High affinity glutamate transporter, important for reuptake of glutamate and has a role in excitatory neurotransmission |
| 489 | 509 MST4 | serinethreonine protein kinase MASK (LOC51765), mRNA. |
| 490 | 510 BLAME | BCM-like membrane protein precursor (SBBI42), mRNA. |
| 491 | 511 NME7 | NME7 |

**Table 5a: Primer and Probe sequences**

| SEQ ID NO: (DNA) | SEQ ID NO: (Probe) | SEQ ID NO: (Primer 1) | SEQ ID NO: Gene_Symbol (Primer 2) | Probe | Forward Primer | Reverse Primer |
|---|---|---|---|---|---|---|
| 4 | 331 | 332 | 333 KPNA2 | TCCTGCCCTAAGAGCCATAGGGAA | GAGCTTCTGAATTGCCAATTGTG | GAGTCTGTTCATCTGTACCAGTGAC A |
| 5 | 334 | 335 | 336 CSE1L | CTGCAGCTGACAAAATTCCTGGGTTACT AGGT | GCATTCTTAGAACGCGGTTCA | TTGGATGCAATCAGCTTCTGA |
| 6 | 448 | 449 | 450 RHEB2 | ATTATCCTTCGAAAAACATCCACAGCAGT CTG | AGCTTTTTTGGAATCTTCTGCTAAA | GCCCCGTCCATTTTTTCTG |
| 7 | 337 | 338 | 339 DKC1 | TCTCGCTTCCGCTTCGCAGTTTTTG | GCAGGTAGTTGCCGAAGCA | TGGAGGAGTCTCGTCACTTTCA |
| 8 | 340 | 341 | 342 IGFBP4 | TCTCCATTAGGCACATTCAGTCCACT | GGGTGGGAAGAAAGAATGCAA | ACCCAGGAAGCCCCTCATC |
| 11 | 343 | 344 | 345 HDAC2 | CCAAAGGAACCAAATCAGAACAGCTCA | CCAAGGACAACAGTGGTGAAAA | GAAATTGGTGAGACTGTCAAATTCA G |
| 12 | 346 | 347 | 348 PRKAB1 | AGTCGCCACAGATGTACCCACTAGCCC | TTCTGTATACGCAGCTCAGTTTCC | CTTCCGCTGACTCACAGCAA |
| 13 | 349 | 350 | 351 IMPDH2 | AAGAGCTTGACCCAAGTCCGAGCCAT | CACTCATGCCAGGACATTGGT | CAAACTTAAGCTCCCCAGAGTACA T |
| 15 | 352 | 353 | 354 YR-29 | CAAGAAAACCACCTAAATATGAAAGATTC ATC | TGCTTTGTTGGAGATGGCTTT | TTGAAACGCAAGCCCATTG |
| 22 | 355 | 356 | 357 CCNB2 | AACTTAACTAAATTCATCGCCATCAAGA A | TGGCCAAGAATGTGGTGAAAG | TCAGGAGTTTGCTGCTTGCA |
| 23 | 358 | 359 | 360 FMOD | AGAAGATCCCCCCAGTCAACACCAACC | TCCTTGAGCTAGACCTCTCCTACA A | ACTCATTGATCCTATTGCCTTGGA |
| 24 | 361 | 362 | 363 SLC7A8 | CATCCAACGCCGTCGCTGTGAC | TGTCTTTGCCAATGTCGCTTA | AACAGAAATGGGCATGATCCA |
| 25 | 364 | 365 | 366 E2-EPF | TCGGATGCCCAGCTCAGCCG | TGCGTCAACGTGCTCAAGAG | GGCACTTGATGGTCAGCAGTAC |
| 26 | 367 | 368 | 369 AGT | AAAGTGAGACCCTCCACCTTGTCCAGG T | GCTGATCCAGCCTCACTATGC | AGATCCTTGCAGCACCAGTTG |
| 27 | 370 | 371 | 372 FHL2 | CATGCCATGCAGTGCGTTCAG | GTGTGCCCTGCTATGAGAAACA | CCCCTCCCGTGGTGATG |

(continued)

| SEQ ID NO: (DNA) | SEQ ID NO: (Probe) | SEQ ID NO: (Primer 1) | SEQ ID NO: Gene_Symbol (Primer 2) | Probe | Forward Primer | Reverse Primer |
|---|---|---|---|---|---|---|
| 29 | 373 | 374 | 375 MGC16824 | AGCCAGGAGACGTACCTTTACCACATAGA | GGGAGGACAACAGCGATGAG | CCCCGTAGAGGCTGTCGTT |
| 31 | 376 | 377 | 378 MAD2L1 | CACAGCTACGGTGACATTTCTGCCACTG | AAATCCGTTCAGTGATCAGACAGA | CAGATCAAATGAACAAGAAACTTCCA |
| 32 | 379 | 380 | 381 DDB2 | TCTCAGAATGCACAAAAAGAAAGTGACGCA | TGAACATGGACGGCAAAGAG | CCAATCACAGCATGGGTTCAG |
| 40 | 382 | 383 | 384 RARRES3 | CCAAGCGCCGTGGCCA | CAGGTGGAAAAGGCCAAGGT | AAGAGCATCCAGCAACAACCA |
| 43 | 385 | 386 | 387 COL11A1 | TCTATACCATCCTTATTCAAAACTTGCAT | GTGCCACCAACCCATTTTG | GTATTTCCTAAATGGTACCTGTATATGCA |
| 50 | 388 | 389 | 390 PCMT1 | ACAGGCAATATCAATCTTCCTCCGGGCT | CCCCAGGCGCTAATAGATCA | CTGCTCCAACATTTGGTTTCC |
| 51 | 391 | 392 | 393 ESR1 | ATGCCCTTTTGCCGATGCA | GCCAAATTGTGTTTGATGGATTAA | GACAAAACCGAGTCACATCAGTAATAG |
| 55 | 394 | 395 | 396 COL10A1 | TCCCCCTGAAAAGTGAGCAGCAACGTA | CAGATTTGAGCTATCAGACCAACAA | AAATTCAAGAGAGGCTTCACATACG |
| 58 | 397 | 398 | 399 GRP | CGTTCTGCAAGCATCAGTTCTACG | AGAGAAAAACAAAACCCCTAAGAGACT | GCACAAGGAAATCTTGTTGATGAT |
| 61 | 400 | 401 | 402 GALNT10 | CCACAGCATGAAGGGCAACCAGC | GCCCTGTCACGCTGTACGA | TCTTGTCTTTGCGGTATTTCCA |
| 65 | 403 | 404 | 405 PGR | TTGATAGAAACGCTGTGAGCTCGA | AGCTCATCAAGGCAATTGGTTT | ACAAGATCATGCAAGTTATCAAGAAGTT |
| 68 | 406 | 407 | 408 CCNG1 | ATGAAGGTACAGCCCAAGCACCTTGGG | GCTGTGAATTTACTGGACAGATT | AAATAAAAGCAGCTCAGTCCAACA |
| 69 | 409 | 410 | 411 PDHB | ATCCTGGCACAGATTTCAGCTCCTACTCCA | GAAGGAGGCTGGCCACAGT | TTGAACGCAGGACCTTCCAT |
| 74 | 412 | 413 | 414 NR2F1 | TGTACAGAATATATCCACATCCGTCCACAATAAATCCT | TAAAACAGAAGGAAACTAATGGACCTT | CAGTCCACTTCCATATGTGTTGTTC |

(continued)

| SEQ ID NO: (DNA) | SEQ ID NO: (Probe) | SEQ ID NO: (Primer 1) | SEQ ID NO: Gene_Symbol (Primer 2) | Probe | Forward Primer | Reverse Primer |
|---|---|---|---|---|---|---|
| 81 | 415 | 416 | 417 FHL1 | CACTTCACGCAATGCTTGGCA | TGCGTGACTTGCCATGAGA | GGTAAGTGATTCCTCCAGATGTGA |
| 82 | 418 | 419 | 420 MSX2 | CAAACAGCCCATTAAGTTCCCTGG | CAGAAGGTAAAGCCATGTTTTGACT | GGGACAGATGGACAGGAAGGT |
| 83 | 421 | 422 | 423 PAI-RBP1 | CTGATGTGGATGACCCAGAGGCATTCC | ACCGACAAGTCAAGTGCTTCTG | GGTTGTCTTATGGCATCCAGTTAA |
| 92 | 424 | 425 | 426 MKI67 | TTTCTGATTCTGCATGAGAACCTTCGCA | GAGAGCGGAGGGCAGAAGA | GAGAGCGGAGGGCAGAAGA |
| 98 | 427 | 428 | 429 GNG12 | CCCCACCCCTCTGCTGGTCCTG | CCAGATGCCTTGGTCCAAAG | GCAGCTTATAGCACCAACACGTT |
| 100 | 430 | 431 | 432 LOC253782 | CCCAAAGTTTCATAAAGCCCCTAAGCTCATGA | AATGGAAAACAACCTCTGAGTTTGA | TGTGGGCAAAGAGTTGATGAAA |
| 101 | 433 | 434 | 435 COL5A1 | CTTCGTGAGTGTCCCGTGCAC | CTCGTACCTCAGCATGCCATT | GTGCCGAGGCGTAGATGAAG |
| 104 | 436 | 437 | 438 KIAA0056 | ACGTGCAGTCAGGTGTCTTCATACA | CATCGGAGTCGGAGCTTAGG | CTCGCCATTCGACTCTTGCT |
| 105 | 439 | 440 | 441 FLJ22642 | AATTCTAATGTAGCAAAACGTAACCA | TGAAACGATTAGCTGTAGCCAAATT | CAGTAGATTTACCACACATATTGCATTTT |
| 106 | 442 | 443 | 444 LOC113146 | AACATAGTTTTCCTATTTCAGGCAGAGTGCGGTATATTC | GGTGTACAAGTCGTTTTTGGTATAACTTC | GCTAAGTGAGTAGGAAACAGTGTTTCC |
| 108 | 445 | 446 | 447 PMSCL1 | TGTTTCTACACCTGTGCTATGGACTC | TGAAGCAGAACCTCCTTCAGAAG | TCCAATTTGGGCAGTTCCA |
| 113 | 451 | 452 | 453 DKFZp586M072 3 | CAGACTAGCCATGACTTGAATGCCAGCA | AAAGAGCGTATGAAAAGTACGTTAGACTT | TGACAAACACGACATAAATAACACACA |
| 124 | 454 | 455 | 456 RAB31 | TTCCCCTGAAGGATGCTAAGGAATACGCT | AACAAGTGCGACCTCTCAGATATT | AACCACGATGGCACCTATGG |
| 128 | 457 | 458 | 459 DCTN4 | CACCTCATCTAATATAAAAAAGGCAA | TCAATACCTGCAGCTGGTGAAT | GGTGCATACTGACTAGCATTAAAATTTT |
| 129 | 460 | 461 | 462 FLJ20273 | TCATCCCCTGACTGTGTGAAAAAAGTA | GACCAAATGTAATTCGGATCAGATC | GAAACTCTGTGACAATCCTTCACTAGA |
| 132 | 463 | 464 | 465 PLSCR4 | TTTTGAAAGATCTCCACCACAACGT | CTTGCTTCCTCATTGACTTCATGT | GGCTTGCTGTGTCTCTCTATCTTG |

(continued)

| SEQ ID NO: (DNA) | SEQ ID NO: (Probe) | SEQ ID NO: (Primer 1) | SEQ ID NO: Gene_Symbol (Primer 2) | **Probe** | **Forward Primer** | **Reverse Primer** |
|---|---|---|---|---|---|---|
| 133 | 466 | 467 | 468 FLJ11323 | CCGCCGCGTCCCGAACT | GTGCTGACGGGACCCTTCT | ACGAGAGCGAAACTCCATTTG |
| 138 | 469 | 470 | 471 MGC3103 | CCCTGACTTCCGCAACATGACGG | GCTGGCTGACAACTTCATCCA | GATGGCATTGCGAGACAGTGT |

**Table 5b: Primer and Probe sequences**

| SEQ ID NO: (DNA) | SEQ ID NO: (Probe) | SEQ ID NO: (Primer 1) | SEQ ID NO: Gene_ Symbol (Primer 2) | Probe | Forward Primer | Reverse Primer |
|---|---|---|---|---|---|---|
| 472 | 512 | 513 | 514 PRG1 | CCCTCATCCTGGTTCTGGAATCCTCAG | TCGGCTTGTCCTGGCTCTT | TGGCTCTCCGCGTAGGATAA |
| 473 | 515 | 516 | 517 GBP1 | CTTGGCCAGACCAATGCCCA | CAGAGTCTTAGGTAAAAGTCTTGG GAAA | TGTCCTTGATATTGGGACATTGTAG |
| 474 | 518 | 519 | 520 ALEX2 | TTTTACTGGTTCTTCTGAATTGACAGTAA ACCTGTCC | AATCGTGCTGCTTGGATAGAAATA | CAAATAATAGAACAGTAGGCCATTC ATAA |
| 475 | 521 | 522 | 523 CD53 | TTTCGCATAGCAACCCTCCACTTTTCG | CAGCATCTTGCCCCTCAGA | AATTGGAATGAAACCACAGTCTTG |
| 476 | 524 | 525 | 526 VCAM1 | AAATGCCCATCTATGTCCCTTGC | TCCCTGAATGTATTGAACTTGGAA | TTCAGGCAGCAAGTTTTACTTTGA |
| 477 | 527 | 528 | 529 MAPT | ATGGCAGCAGTTCCAACCTTCAGAACTC AATA | CCCTCTGCTCCACAGAAACC | GGTCTGCAAAGTGGCCAAAAT |
| 478 | 530 | 531 | 532 EGR2 | TCCCAAGCCATAAAGTGCACAT | GGACAGCAAAAAGACAAGCAAA | CTGTACAATGTCCCCCAAATCA |
| 479 | 533 | 534 | 535 TDO2 | ATTCACTGATGACCAAATGGAGATATAAC C | CAGTTGCTGACTTCTCTTATGGACA T | ATTCTGTGCACCATGCACACA |
| 480 | 536 | 537 | 538 ADAMDEC 1 | AGTATCTGAGTTCAAAATTCCCAAAGG A | TCCCTCTGGCAGTTGTGTGA | TGCACGGCAAGATGTACTGAA- |
| 481 | 539 | 540 | 541 TFEC | CAGCGCATATCAGGATCATTAGACTTT | AATCAAGGAGCTTGGCACTCTT | GATGCTTTTAGAATGGTTCCTTTGT T |
| 482 | 542 | 543 | 544 BTF3 | TTCGGCCAGTCTCCTTAAACTAGTCA | ACCAGCTTGGTGCGGATAGT | GTGCTTTTCCATCCACAGATTG |
| 483 | 545 | 546 | 547 FLNB | CAGCAAAGCTGGCTCCAACATGCTG | TGTGGGCCAGAAGAGTTCCT | CCCATGGACCCCGATCA |
| 484 | 548 | 549 | 550 TFRC | AGCTCCGTGAGTGAACCATCATTATAAAC GTG | GCCTACCCATTCGTGGTGAT | TCCCTAGGAGGCCGTTTCC |
| 485 | 551 | 552 | 553 EIF4B | CCCACCACTTGTAGGGGACTGCT | CTCGATCTCAGAGCTCAGACACA | GCATTCATCCCATCTACTTTATTTTC AT |
| 486 | 554 | 555 | 556 MAPK3 | CAGTGGCCGAGGAGCCCTTCAC | AGTACTATGACCCGACGGATGAG | TCAGCCGCTCCTTAGGTAGGT |

(continued)

| SEQ ID NO: (DNA) | SEQ ID NO: (Probe) | SEQ ID NO: (Primer 1) | SEQ ID NO: Gene_ Symbol (Primer 2) | Probe | Forward Primer | Reverse Primer |
|---|---|---|---|---|---|---|
| 487 | 557 | 558 | 559 LOC161291 | CACCAGCCACTTTGCTAATTTCTT | GAACGATGATCTTAAAGGCACAAA | TCTTGCTGCAATGTAAATCTGCTAT |
| 488 | 560 | 561 | 562 SLC1A1 | AGAAAAAGAGCTTCCCCTAACCTGGG | TGGGTTGAACAAGCCACGTT | GTCGTGGGATTTACTCTGCAACA |
| 489 | 563 | 564 | 565 MST4 | TAAGTATCCCTATTTCTTAAGTTACGAGGA | AATGTTGAGACACCGTTTTGCTT | GTAGAGTCAACTAAAGATCAAAATGTGAAAG |
| 490 | 566 | 567 | 568 BLAME | ATCACCTTCCCCCAAGATTACCTGA | CCCTTTCCCACACCCACTT | GGGATGGTGCAAGCTGACA |
| 491 | 569 | 570 | 571 NME7 | TTGAAATCTCAGCTATGCAGATGTTC | TCCTGATGGCTATCCGAGATG | CCTCAACATTAACCCGATCCA |

**Table 6: Statistical relevance of 20 genes differentially in non-responders (NC) as compared to responding tumors . (CR - complete responder to therapy)**

| SEQ ID NO: (DNA Sequence) | SEQ ID NO: Gene_ Symbol (Protein Sequence) | T-Test p-value | Welch-Test p-value | Wilcoxon p-value |
|---|---|---|---|---|
| 472 | 492 PRG1 | 0.0002116 | 0.0002631 | 0.0003108 |
| 473 | 493 GBP1 | 0.0020070 | 0.0023060 | 0.0029530 |
| 474 | 494 ALEX2 | 0.0003502 | 0.0012570 | 0.0001554 |
| 475 | 495 CD53 | 0.0019770 | 0.0039540 | 0.0018650 |
| 476 | 496 VCAM1 | 0.0010630 | 0.0010690 | 0.0018650 |
| 477 | 497 MAPT | 0.0005838 | 0.0007540 | 0.0001554 |
| 478 | 498 EGR2 | 0.0008870 | 0.0009158 | 0.0006216 |
| 479 | 499 TD02 | 0.0084350 | 0.0105000 | 0.0018650 |
| 480 | 500 ADAMDEC1 | 0.0018700 | 0.0021870 | 0.0029530 |
| 481 | 501 TFEC | 0.0085550 | 0.0155500 | 0.0010880 |
| 482 | 502 BTF3 | 0.0001140 | 0.0001471 | 0.0003108 |
| 483 | 503 FLNB | 0.0006050 | 0.0007720 | 0.0018650 |
| 484 | 504 TFRC | 0.0005408 | 0.0010110 | 0.0010880 |
| 485 | 505 EIF4B | 0.0013130 | 0.0013330 | 0.0006216 |
| 486 | 506 MAPK3 | 0.0001388 | 0.0003527 | 0.0006216 |
| 487 | 507 LOC161291 | 0.0015790 | 0.0031610 | 0.0006216 |
| 488 | 508 SLC1A1 | 0.0000179 | 0.0000389 | 0.0001554 |
| 489 | 509 MST4 | 0.0000888 | 0.0000904 | 0.0001554 |
| 490 | 510 BLAME | 0.0048620 | 0.0081110 | 0.0029530 |
| 491 | 511 NME7 | 0.0020950 | 0.0021980 | 0.0006216 |

## Claims

**1.** Method for characterizing the state of a neoplastic disease in a subject, comprising

(i) determining the pattern of expression levels of at least 6, 8, 10, 15, 20, 30, or 47 marker genes, comprised in a group of marker genes consisting of SEQ ID NO: 1 to 165, in a biological sample from said subject,
(ii) comparing the pattern of expression levels determined in (i) with one or several reference pattern(s) of expression levels,
(iii) characterizing the state of said neoplastic disease in said subject from the outcome of the comparison in step (ii).

**2.** Method for characterizing the state of a neoplastic disease in a subject, comprising

(i) determining the pattern of expression levels of at least 6, 8, 10, 15, 20, 30, 47 or 67 marker genes, comprised in a group of marker genes consisting of SEQ ID NO: 1 to 165 and 472 to 491, in a biological sample from said subject,
(ii) comparing the pattern of expression levels determined in (i) with one or several reference pattern(s) of expression levels,
(iii) characterizing the state of said neoplastic disease in said subject from the outcome of the comparison in step (ii).

**3.** Method for detection, diagnosis, screening, monitoring, and/or prognosis of a neoplastic disease in a subject, comprising

(i) determining the pattern of expression levels of at least 1, 2, 3, 5, 10, 15, 20, 30, or 47 marker genes, comprised in a group of marker genes consisting of SEQ ID NOs:1 to 17, 19 to 33, 35 to 50, 52 to 64, 66 to 85, 88 to 91, and 93 to 165 in biological samples from said subject,
(ii) comparing the pattern of expression levels determined in (i) with one or several reference pattern(s) of expression levels,
(iii) detecting, diagnosing, screening, monitoring, and/or prognosing said neoplastic disease in said subject from the outcome of the comparison in step (ii).

**4.** Method for detection, diagnosis, screening, monitoring, and/or prognosis of a neoplastic disease in a subject, comprising

(i) determining the pattern of expression levels of at least 1, 2, 3, 5, 10, 15, 20, 30, 47, or 67 marker genes, comprised in a group of marker genes consisting of SEQ ID NOs:1 to 17, 19 to 33, 35 to 50, 52 to 64, 66 to 85, 88 to 91, and 93 to 165 and 472 to 491 in biological samples from said subject,
(ii) comparing the pattern of expression levels determined in (i) with one or several reference pattern(s) of expression levels,
(iii) detecting, diagnosing, screening, monitoring, and/or prognosing said neoplastic disease in said subject from the outcome of the comparison in step (ii).

**5.** Method of any of claims 1 to 4, wherein said method comprises multiple determinations of a pattern of expression levels, at different points in time, thereby allowing to monitor the development of said neoplastic disease in said subject.

**6.** Method of claim 1 or 2, wherein said method comprises an estimation of the likelihood of success of a given mode of treatment for said neoplastic disease in said subject.

**7.** Method of claim 1 or 2, wherein said method comprises an assessment of whether or not the subject is expected to respond to a given mode of treatment for said neoplastic disease.

**8.** Method of claim 6 or 7, wherein a predictive algorithm is used.

**9.** Method of claim 8, wherein the predictive algorithm is a Support Vector Machine.

**10.** Method of any of claims 6 to 9, wherein said given mode of treatment

(i) acts on cell proliferation, and/or
(ii) acts on cell survival, and/or
(iii) acts on cell motility, and/or
(iv) is an anthracycline based mode of treatment, and/or
(v) comprises administration of epirubicin and/or cyclophoshamid.

**11.** Method of treatment for a subject afflicted with a neoplastic disease, comprising

(i) identifying the most promising mode of treatment with the method of claim 6 or 7,
(ii) treating said neoplastic disease in said patient by the mode of treatment identified in step (i).

**12.** Method of screening for subjects afflicted with a neoplastic disease, wherein a method of any of claims 1 to 4 is applied to a plurality of subjects.

**13.** Method of screening for substances and/or therapy modalities having curative effect on a neoplastic disease comprising

(i) obtaining a biological sample from a subject afflicted with said neoplastic disease,
(ii) assessing, from said biological sample, using the method of claim 6 or 7, whether said subject is expected to respond to a given mode of treatment for said neoplastic disease,

(iii) if said subject is expected to respond to said given mode of treatment, incubating said biological sample with said substance under said therapy modalities,
(iv) observing changes in said biological sample triggered by said test substance under said therapy modalities,
(v) selecting or rejecting said test substance and/or said therapy modalities, based on the observation of changes in said biological sample under (iv).

**14.** Method of screening for compounds having curative effect on a neoplastic disease comprising

(i) incubating biological samples or extracts of these with a test substance,
(ii) determining the pattern of expression levels of at least 1, 2, 3, 5, 10, 15, 20, 30, or 47 marker genes, comprised in a group of marker genes consisting of SEQ ID NO:1 to 17, 19 to 33, 35 to 50, 52 to 64, 66 to 85, 88 to 91, and 93 to 165 in said biological sample,
(iii) comparing the pattern of expression levels determined in (ii) with one or several reference pattern(s),
(iv) selecting or rejecting said test substance, based on the comparison performed under (iii).

**15.** Method of screening for compounds having curative effect on a neoplastic disease comprising

(i) incubating biological samples or extracts of these with a test substance,
(ii) determining the pattern of expression levels of at least 1, 2, 3, 5, 10, 15, 20, 30, 47, or 67 marker genes, comprised in a group of marker genes consisting of SEQ ID NO:1 to 17, 19 to 33, 35 to 50, 52 to 64, 66 to 85, 88 to 91, and 93 to 165 and 472 to 491 in said biological sample,
(iii) comparing the pattern of expression levels determined in (ii) with one or several reference pattern(s),
(iv) selecting or rejecting said test substance, based on the comparison performed under (iii).

**16.** Method of any of claims 1 to 15 wherein said marker genes are comprised in a group of marker genes listed in Table 2.

**17.** Method of any of claims 1 to 16, wherein the expression level is determined

(i) with a hybridization based method, or
(ii) with a hybridization based method utilizing arrayed probes, or
(iii) with a hybridization based method utilizing individually labeled probes, or
(iv) by real time real time PCR, or
(v) by assessing the expression of polypeptides, proteins or derivatives thereof, or
(vi) by assessing the amount of polypeptides, proteins or derivatives thereof.

**18.** Method of any of claims 1 to 17, wherein the neoplastic disease is breast cancer.

**19.** A kit comprising at least 6, 8, 10, 15, 20, 30, or 47 primer pairs and probes suitable for marker genes comprised in a group of marker genes consisting of

(i) SEQ ID NO: 1 to SEQ ID NO: 165, or
(iii) the marker genes listed in Table 2.

**20.** A kit comprising at least 6, 8, 10, 15, 20, 30, 47, or 67 primer pairs and probes suitable for marker genes comprised in a group of marker genes consisting of

(i) SEQ ID NO : 1 to SEQ ID NO:165, and/or
(ii) SEQ ID NO:472 to SEQ ID NO:491, or
(iii) the marker genes listed in Table 2.

**21.** A kit comprising at least 6, 8, 10, 15, 20, 30, or 47 individually labeled probes, each having a sequence comprised in a group of sequences consisting of SEQ ID NO:331 to SEQ ID NO:471.

**22.** A kit comprising at least 6, 8, 10, 15, 20, 30, 47 or 67 individually labeled probes, each having a sequence comprised in a group of sequences consisting of SEQ ID NO:331 to SEQ ID NO:471 and SEQ ID NO:512 to 571.

**23.** A kit comprising at least 6, 8, 10, 15, 20, 30, or 47 arrayed probes, each having a sequence comprised in a group of sequences consisting of SEQ ID NO:331 to SEQ ID NO:471.

**24.** A kit comprising at least 6, 8, 10, 15, 20, 30, 47 or 67 arrayed probes, each having a sequence comprised in a group of sequences consisting of SEQ ID NO:331 to SEQ ID NO:471 and SEQ ID NO:512 to 571.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5556752 A
- WO 9714028 A
- US 4843155 A
- US 5262311 A
- US 4683202 A, Mullis, K. B
- US 5714331 A
- WO 9912826 A
- US 5641673 A
- EP 0321201 A
- EP 0799897 A
- WO 9729212 A
- WO 9727317 A
- EP 0785280 A
- WO 9702357 A
- US 5593839 A
- US 5578832 A
- EP 0728520 A
- US 5599695 A
- EP 0721016 A
- WO 9522058 A
- US 5631734 A
- US 6203987 B
- US 4683195 A
- US 5498531 A
- GB 2188638 B
- US 5565332 A
- WO 9303151 A
- WO 9413804 A
- US 5223409 A
- US 5976813 A, Beutel
- US 5283317 A
- WO 9410300 A, Brent
- US 5705151 A
- US 526231 A, Liang, P., and Pardee, A. B


### Non-patent literature cited in the description


- **THOMSON JD ; HIGGINS DG ; GIBSON TJ.** ClustalW: Improving the sensitivity of progressive multiple sequence alignment through sequence weighting, positions-specific gap penalties and weight matrix choice. *Nucleic Acids Res.,* 1994, vol. 22, 4673-4680
- WHO. International Classification of Diseases. WHO
- TNM Classification of Malignant Tumors. Wiley, 1997
- **SORLIE et al.** *Proc Natl Acad Sci U S A.,* 11 September 2001, vol. 98 (19), 10869-74
- **VAN'T VEER et al.** *Nature,* 31 January 2002, vol. 415 (6871), 530-6
- **PEREZ, E.A.** *Current Managment of Metastatic Breast Cancer. Semin. Oncol,* 1999, vol. 26 (12), 1-10
- **SAMBROOK et al.** MOLECULAR CLONING: A LABORATORY MANUAL. 1989
- **AUSUBEL et al.** CURRENT PROTOCOLS IN MOLECULAR BIOLOGY. John Wiley & Sons, 1989
- **TEDDER, T. F. et al.** *Proc. Natl. Acad. Sci. U.S.A.,* 1988, vol. 85, 208-212
- **HEDRICK, S. M et al.** *Nature,* 1984, vol. 308, 149-153
- **LEE, S. W et al.** *Proc. Natl. Acad. Sci. U.S.A.,* 1984, vol. 88, 4225
- **SARKAR.** *PCR Methods Applic,* 1993, vol. 2, 318-322
- **TRIGLIA et al.** *Nucleic Acids Res.,* 1988, vol. 16, 81-86
- **LAGERSTROM et al.** *PCR Methods Applic,* 1991, vol. 1, 111-119
- **COPELAND ; JENKINS.** *Trends in Genetics,* 1991, vol. 7, 113-118
- **COHEN et al.** *Nature,* 1993, vol. 366, 698-701
- **BONNER et al.** *J. Mol. Biol.,* 1973, vol. 81, 123
- **BOLTON ; MCCARTHY.** *Proc. Natl. Acad. Sci. U.S.A.,* 1962, vol. 48, 1390
- **ALTSCHUL et al.** *Bull. Math. Bio.,* 1986, vol. 48, 603
- **HENIKOFF ; HENIKOFF.** *Proc. Natl. Acad. Sci. USA,* 1992, vol. 89, 10915
- **PEARSON ; LIPMAN.** *Proc. Nat'1 Acad. Sci. USA,* 1988, vol. 85, 2444
- **PEARSON et al.** *Meth. Enzymol.,* 1990, vol. 183, 63
- **NEEDLEMAN ; WUNSCH.** *J. Mol. Biol.,* 1970, vol. 48, 444
- **SELLERS.** *SIAM J. Appl. Math.,* 1974, vol. X (787
- **TAKAMATSU.** *EMBO J.,* 1987, vol. 6, 307-311
- **CORUZZI et al.** *EMBO J.,* 1984, vol. 3, 1671-1680

- **BROGLIE et al.** *Science,* 1984, vol. 224, 838-843
- **WINTER et al.** *Results Probl. Cell Differ.,* 1991, vol. 17, 85-105
- **ENGELHARD et al.** *Proc. Nat. Acad. Sci.,* 1994, vol. 91, 3224-3227
- **LOGAN ; SHENK.** *Proc. Natl. Acad. Sci.,* 1984, vol. 81, 3655-3659
- **SCHARF et al.** *Results Probl. Cell Differ.,* 1994, vol. 20, 125-162
- ANIMAL CELL CULTURE. 1986
- **WIGLER et al.** *Cell,* 1977, vol. 11, 223-232
- **LOWY et al.** *Cell,* 1980, vol. 22, 817-823
- **WIGLER et al.** *Proc. Natl. Acad. Sci.,* 1980, vol. 77, 3567-3570
- **COLBERE-GARAPIN et al.** *J. Mol. Biol.,* 1981, vol. 150, 114
- **HARTMAN ; MULLIGAN.** *Proc. Natl. Acad. Sci.,* 1988, vol. 85, 8047-8051
- **RHODES et al.** *Methods Mol. Biol.,* 1995, vol. 55, 121-131
- **HAMPTON et al.** SEROLOGICAL METHODS: A LABORATORY MANUAL. APS Press, 1990
- **MADDOX et al.** *J. Exp. Med.,* 1983, vol. 158, 1211-1216
- **PORATH et al.** *Prot. Exp. Purif.,* 1992, vol. X (3-281
- **KROLL et al.** *DNA Cell Biol.,* 1993, vol. 12, 441-453
- **CARUTHERS et al.** *Nucl. Acids Res. Symp. Ser.,* 1980, 215-223
- **HORN et al.** *Nucl. Acids Res. Symp. Ser.,* 1980, 225-232
- **MERRIFIELD.** *J. Am. Chem. Soc.,* 1963, vol. 85, 2149-2154
- **ROBERGE et al.** *Science,* 1995, vol. X, 202-204
- **CREIGHTON.** PROTEINS: STRUCTURES AND MOLECULAR PRINCIPLES. WH and Co, 1983
- **CRONIN et al.** *Human Mutation,* 1996, vol. 7, 244
- **LANDEGRAN et al.** *Science,* 1988, vol. 241, 1077-1080
- **NAKAZAWA et al.** *PNAS,* 1994, vol. 91, 360-364
- **ABRAVAYA et al.** *Nuc Acid Res,* 1995, vol. 23, 675-682
- **GUATELLI, J.C. et al.** *Proc. Natl. Acad. Sci. USA,* 1990, vol. 87, 1874-1878
- **KWOH, D.Y et al.** *Proc. Natl. Acad. Sci. USA,* 1989, vol. 86, 1173-1177
- **LIZARDI, P.M. et al.** *Bio/Technology,* 1988, vol. 6, 1197
- **BROWN.** *Meth. Mol. Biol.,* 1994, vol. 20, 18
- **SONVEAUX.** *Meth. Mol. Biol.,* 1994, vol. X (1-72
- **UHLMANN et al.** *Chem. Rev.,* 1990, vol. 90, 543-583
- **GEE et al.** MOLECULAR AND IMMUNOLOGIC APPROACHES. Publishing Co, 1994
- **AGRAWAL et al.** *Trends Biotechnol.,* 1992, vol. 10, 152-158
- **UHLMANN et al.** *Tetrahedron. Lett.,* 1987, vol. 215, 3539-3542
- **CECH.** *Science,* 1987, vol. 236, 1532-1539
- **CECH.** *Biochem.,* 1990, vol. 59, 543-568
- **COUTURE ; STINCHCOMB.** *Trends Genet.,* 1996, vol. 12, 510-515
- **HASELOFF et al.** *Nature,* 1988, vol. 334, 585-591
- **KOHLER et al.** *Nature,* 1985, vol. 256, 495-497
- **KOZBOR et al.** *J. Immunol. Methods,* 1985, vol. 81, 3142
- **COTE et al.** *Proc. Natl. Acad. Sci.,* 1983, vol. 20X (2030
- **COLE et al.** *Mol. Cell Biol.,* 1984, vol. 62, 109-120
- **MORRISON et al.** *Proc. Natl. Acad. Sci.,* 1984, vol. 81, 6851-6855
- **NEUBERGER et al.** *Nature,* 1984, vol. 312, 604-608
- **TAKEDA et al.** *Nature,* 1985, vol. 314, 452-454
- **BURTON.** *Proc. Natl. Acad. Sci.,* 1991, vol. 88, 11120-11123
- **THIRION et al.** *Eur. J. Cancer Prev.,* 1996, vol. 5, 507-11
- **COLOMA ; MORRISON.** *Nat. Biotechnol.,* 1997, vol. 15, 159-63
- **MALLENDER ; VOSS.** *J. Biol. Chem.,* 1994, vol. X (9), 199-206
- **VERHAAR et al.** *Int. J. Cancer,* 1995, vol. 61, 497-501
- **NICHOLLS et al.** *J. Immunol. Meth.,* 1993, vol. 165, 81-91
- **ORLANDI et al.** *Proc. Natl. Acad. Sci.,* 1989, vol. 86, 3833-3837
- **WINTER et al.** *Nature,* 1991, vol. 349, 293-299
- **LAM.** *Anticancer Drug Des.,* 1997, vol. 12, 145
- **DEWITT et al.** *Proc. Natl. Acad. Sci. U.S.A.,* 1993, vol. 90, 6909
- **ERB et al.** *Proc. Natl. Acad. Sci. U.S.A.,* 1994, vol. 91, 11422
- **ZUCKERMANN et al.** *J. Med. Chem.,* 1994, vol. X (78
- **CHO et al.** *Science,* 1993, vol. X (1), 1303
- **CARELL et al.** *Angew. Chem. Int. Ed. Engl.,* 1994, vol. 33, 2059, 2061
- **GALLOP et al.** *J. Med. Chem.,* 1994, vol. 37, 1233

- **HOUGHTEN.** *BioTechniques,* 1992, vol. 13, 412-421
- **LAM.** *Nature,* 1991, vol. 354, 8284
- **FODOR.** *Nature,* 1993, vol. 364, 555-556
- **CULL et al.** *Proc. Natl. Acad. Sci. U.S.A.,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 97, 6378-6382
- **FELICI.** *J. Mol. Biol.,* 1991, vol. 222, 301-310
- **JAYAWICKREME et al.** *Proc. Natl. Acad. Sci. U.S.A.,* 1994, vol. 19, 1614-1618
- **CHELSKY.** *Strategies for Screening Combinatorial Libraries,* 1995
- **SALMON et al.** *Molecular Diversity,* 1996, vol. 2, 57-63
- **MCCONNELL et al.** *Science,* 1992, vol. 257, 1906-1912
- **SJOLANDER ; URBANICZKY.** *Anal. Chem.,* 1991, vol. 63, 2338-2345
- **SZABO et al.** *Curr. Opin. Struct. Biol.,* 1995, vol. 5, 699-705
- **ZERVOS et al.** *Cell,* 1993, vol. 72, 223-232
- **MADURA et al.** *J. Biol. Chem.,* 1993, vol. X (8), 12046-12054
- **BARTEL et al.** *BioTechniques,* 1993, vol. 14, 920-924
- **IWABUCHI et al.** *Oncogene,* 1993, vol. 8, 1693-1696
- **FINDEIS et al.** *Trends in Biotechnol,* 1993, vol. 11, 202-205
- **CHIOU et al.** GENE THERAPEUTICS: METHODS AND APPLICATIONS OF DIRECT GENE TRANSFER. 1994
- **WU ; WU.** *J. Biol. Chem.,* 1988, vol. X (3), 621-24
- **WU et al.** *J. Biol. Chem.,* 1994, vol. X (9), 542-46
- **ZENKE et al.** *Proc. Natl. Acad. Sci. U.S.A.,* 1990, vol. 87, 3655-59
- **WU et al.** *J. Biol. Chem.,* 1991, vol. X (6), 338-42
- REMINGTON'S PHARMACEUTICAL SCIENCES. Maack Publishing Co
- **HILLE.** Excitable Membranes. Sinauer Associates, Inc
- **VAN HEEKE ; SCHUSTER.** *J. Biol. Chem.,* 1989, vol. 264, 5503-5509
- **GRANT et al.** *Methods Enzymol.,* 1987, vol. 153, 516-544
- **WESTON ; WATKINS.** Proceedings of the Seventh European Symposium On Artificial Neural Networks. 1999
- **VAPNIK.** The Nature of Statistical Learning Theory. Springer, 1995
- **VAPNIK.** Statistical Learning Theory. Wiley, 1998
- **BURGES.** *Data Mining and Knowledge Discovery,* 1998, vol. 2 (2), 955-974